# EUROPEAN PATENT SPECIFICATION

(11) **EP 4 291 558 B1**
(45) Date of publication and mention of the grant of the patent: **11.11.2026**
(21) Application number: 22706272.6
(22) Date of filing: 10.02.2022
(51) Int. Cl.: C07D 417/10, C07D 417/14, C07D 513/04, C07D 519/00, A61P 35/00, A61K 31/554

(54) **BICYCLIC TETRAHYDROAZEPINE DERIVATIVES FOR THE TREATMENT OF CANCER**
BICYCLISCHE TETRAHYDROAZEPINDERIVATE ZUR BEHANDLUNG VON KREBS
DÉRIVÉS BICYCLIQUES DE TÉTRAHYDROAZÉPINE POUR LE TRAITEMENT DU CANCER

(30) Priority: 12.02.2021 EP 21156877; 21.07.2021 EP 21186928
(43) Date of publication of application: 20.12.2023
(73) Proprietor: F. Hoffmann-La Roche AG, 4070 Basel (CH)
(72) Inventor: BRITSCHGI, Adrian, 4070 Basel (CH); HUTTER, Roman, 4070 Basel (CH); KUEHNE, Holger, 4070 Basel (CH); KUHN, Bernd, 4070 Basel (CH); LUEBBERS, Thomas, 4070 Basel (CH); MARTIN, Laetitia Janine, 4070 Basel (CH); MUELLER, Barbara Johanna, 4070 Basel (CH); WICHMANN, Juergen, 4070 Basel (CH); BRANDSTAETTER, Marco, 4070 Basel (CH)
(74) Representative: Belkacemi, Doria
(86) International application number: PCT/EP2022/053257
(87) International publication number: WO 2022/171745

(56) References cited:
- WO-A1-03/031376
- WO-A1-2014/023708
- WO-A1-2016/139181
- VEZENKOV LUBOMIR L. ET AL: "Structure-Activity Relationships of JMV4463, a Vectorized Cathepsin?D Inhibitor with Antiproliferative Properties: The Unique Role of the AMPA-Based Vector", CHEMMEDCHEM COMMUNICATIONS, vol. 11, no. 3, 7 December 2015 (2015-12-07), DE, pages 302 - 308, XP055908068, ISSN: 1860-7179, DOI: 10.1002/cmdc.201500457

## Description

### Field of the invention

The present invention relates to bicyclic tetrahydroazepine compounds which inhibit Diacylglycerol kinases (DGK) α and ζ and are useful as T-Cell signal 2 enhancers, their manufacture and pharmaceutical compositions comprising said compounds. The present compounds may be useful as immunotherapeutic agents for the treatment of human diseases. More specifically, the present compounds can be used alone or in combination with other immunotherapeutic agents in order to boost anti-cancer immunity.

### Background of the invention

Cancer immunity is a multistep process that is regulated by a series of negative immune checkpoint and positive co-stimulatory receptors and related intracellular signaling cascades that when effectively triggered can achieve antitumor response (Mellman, I., et al. (2011) Cancer Immunotherapy Comes of Age, Nature 480(7378), 480-489). Indeed, PD1/PDL1 targeting and other immune-checkpoint inhibitors have revolutionized cancer immunotherapy, but still more than 70% of patients do not benefit from immune-checkpoint inhibition. Similarly, for T-cell bispecific antibodies, even in the most promising indication (Non-Hodgkin lymphoma), these T-cell binders (TCBs) achieve complete remissions in less than 50% of patients. T-cell exhaustion seems to play an important role in many of these examples of primary or secondary resistance to cancer immunotherapy. A possible reason for this lack of efficacy is that T-cell activation occurs via targeting and crosslinking of CD3 (signal 1), but co-stimulation e.g. via CD28 or 4-1BB (signal 2) is missing. This hypothesis was verified clinically for CAR T-cell therapy where it was shown that only after the incorporation of co-stimulatory domains, clinically relevant efficacy was observed.

Diacylglycerol kinases (DGKs) are lipid kinases that catalyze the conversion of Diacylglycerol (DAG) to phosphatidic acid (PA), thus limiting DAG-regulated and promoting PA-dependent functions (Merida, I., Avila-Flores, A., and Merino, E. 2008: Diacylglycerol kinases: at the hub of cell signalling. Biochem. J. 409 (1), 1-18). The DGK family consist of ten isoforms that can be grouped into five subtypes based on the presence of different regulatory domains within their structure. Beyond that, the lack of structural data as of now still hinders a more thorough understanding of the DGKs mode of action. Also information on certain prokaryotic DGK and other lipid kinases like sphingosine kinase and phosphatidylinositol-3-kinase (PI3K) has provided only limited insight into the DGK catalytic mechanisms which seems to be distinct from classical kinases (Arranz-Nicolás, J. and Mérida, I., 2020. Biological regulation of diacylglycerol kinases in normal and neoplastic tissues: New opportunities for cancer immunotherapy, Advances in Biological Regulation, Volume 75; Ma, Q., Gabelli, S.B., Raben, D.M., 2019: Diacylglycerol kinases: relationship to other lipid kinases. Adv Biol Regul 71, 104-110).

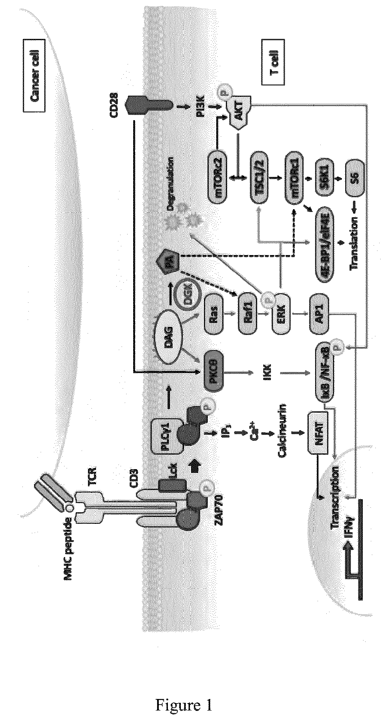

Although several isoforms within the DGK family have been described to play a role in cancer, the α and ζ isoforms are the ones that have been most deeply studied in this regard. As PA producers, both enzymes have been implicated in various processes promoting tumor growth and metastasis. On the other hand, as DAG consumers, DGKα and ζ have been extensively characterized as negative regulators of T cell responses (Riese, M.J., Moon, E.K., Johnson, B.D., Albelda, S.M., 2016. Diacylglycerol kinases (DGKs): novel targets for improving T cell activity in cancer. Front Cell Dev Biol 4,108; Noessner, E., 2017. DGK-alpha: a checkpoint in cancer-mediated immuno-inhibition and target for immunotherapy. Front Cell Dev Biol 5, 16; Sakane, F., Mizuno, S., Komenoi, S., 2016. Diacylglycerol kinases as emerging potential drug targets for a variety of diseases: an update. Front Cell Dev Biol 4, 82; Arranz-Nicolás, J. and Mérida, I., 2020. Biological regulation of diacylglycerol kinases in normal and neoplastic tissues: New opportunities for cancer immunotherapy, Advances in Biological Regulation, Volume 75) These two isozymes DGKα and DGKζ are active downstream of CD28 and other costimulatory receptors as well as the T cell receptor (TCR), and their function is to limit the amount of DAG generated - and ultimately T-cell activation (Merida, I., Andrada, E., Gharbi, S.I., Avila-Flores, A., 2015. Redundant and specialized roles for diacylglycerol kinases alpha and zeta in the control of T cell functions. Sci. Signal. 8 (374); Shulga, Y.V., Topham, M.K., Epand, R.M., 2011. Regulation and functions of diacylglycerol kinases. Chem. Rev. 111 (10), 6186-6208.) A summary of representative DGK-regulated signaling pathways is shown in Figure 1 (Sim, J.A.; Kim, J.; Yang, D. Beyond Lipid Signaling: Pleiotropic Effects of Diacylglycerol Kinases in Cellular Signaling. Int. J. Mol. Sci. 2020, 21, 6861): Activated PLC1 cleaves PIP2 in the plasma membrane to generate two secondary messengers, DAG and IP3. DAG activates PKC, Ras/MEK/ERK/AP-1 and NF-kB, while IP3 is involved in the activation of intracellular Ca2+ flux. The upregulated Ca2+ signaling in turn activates the transcription factor NFAT. In short, DAG production and levels determine the duration and intensity of the Ras/MEK/ERK and PKC-dependent signaling pathways, and they are central to T-cell activation. Thus, DGKs serve as intracellular checkpoints and inhibition of DGKs is expected to enhance T cell signaling pathways and T cell activation.

Experimental evidence suggests that enhanced DGK function and / or expression in tumor infiltrating T-cells (TILs) limits tumor destruction. Experiments with CAR T cells directed against human mesothelioma engrafted into nude mice demonstrated that tumor-infiltrating CAR T cells express elevated concentrations of surface inhibitory receptors, as well as the inhibitory enzymes SHIP-1, DGKα and DGKζ (Moon et al., 2014). Further, high DGKα expression was also observed in TIL isolated from human renal tumors (Prinz et al., 2012). In mouse mesoCAR T cells, dual deletion of DGKα and DGKζ results in enhanced cytokine expression and cytotoxicity against tumor cells (Riese et al., 2013). Similar results have been reported for human CAR T cells in which both DGKα and DGKζ expression were silenced using CRISPR/ Cas9 (Jung et al., 2018). All these studies support a rationale for targeting DGKα/ζ in the development of anti-cancer therapies (Arranz-Nicolás, J. and Mérida, I., 2020. Biological regulation of diacylglycerol kinases in normal and neoplastic tissues: New opportunities for cancer immunotherapy, Advances in Biological Regulation, Volume 75; Riese, M.J., Moon, E.K., Johnson, B.D., Albelda, S.M., 2016. Diacylglycerol kinases (DGKs): novel targets for improving T cell activity in cancer. Front Cell Dev Biol 4, 108.). Knock out mouse models provide further evidence: Mice lacking either DGKα or DGKζ showed a hyper-responsive T cell phenotype and improved anti-tumor immune activity (Riese, M.J., Grewal, J., Das, J., Zou, T., Patil, V., Chakraborty, A.K., Koretzky, G.A., 2011. Decreased diacylglycerol metabolism enhances ERK activation and augments CD8+ T cell functional responses. J. Biol. Chem. 286 (7), 5254-5265; Zha, Y., Marks, R., Ho, A.W., Peterson, A.C., Janardhan, S., Brown, I., Praveen, K., Stang, S., Stone, J.C., Gajewski, T.F., 2006. T cell anergy is reversed by active Ras and is regulated by diacylglycerol kinase-alpha. Nat. Immunol. 7 (11), 1166-1173; Olenchock, B.A., Guo, R., Carpenter, J.H., Jordan, M., Topham, M.K., Koretzky, G.A., Zhong, X.P., 2006a. Disruption of diacylglycerol metabolism impairs the induction of T cell anergy. Nat. Immunol. 7 (11), 1174-1181.)

Taken together, there is substantial evidence that DGKα and DGKζ are high value targets for cancer immunotherapy. At the same time, there is a lack of compounds with the ability to potently inhibit both DGKα and DGKζ with good selectivity over other diacylglycerol kinases, protein kinases, and/or other lipid kinases.

This invention describes such dual DGKa/z inhibitors with excellent selectivity over other protein kinases, across safety / off-target panels and vs. other lipid kinases. These compounds potently activate suboptimally stimulated T-cells and thereby act as intracellular enhancers of co-stimulatory signaling cascades. These DGKa/z inhibitors have the potential to increase proliferation, cytotoxicity and the life span of targeted T-cells which may result in improved anticancer activity of CPIs, CD3 engaging T-cell bispecifics and CAR T-cells. Further, by engaging a signaling node central to both TCR and co-stimulatory receptors, it is plausible that these molecules enhance both signals 1 and 2 and thus single agent activity can be achieved, e.g. in inflamed tumors.

There is an ongoing need for new compounds capable of activating and proliferating T-cells, thus enabling the treatment, prevention and/or delay of progression of cancer. It is, therefore, an object of this invention to provide compounds useful as T-cell signal 2 enhancers for the treatment or prevention or amelioration of such diseases with improved therapeutic properties, in particular improved pharmacokinetic properties.

### Summary of the invention

A first object of the present invention is a compound of formula (I) 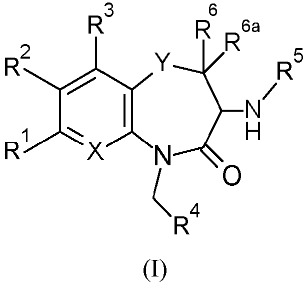 or a pharmaceutically acceptable salt thereof, wherein:
X is C(R⁷) or N;
Y is S, S(O), S(O)₂, S(O)N(R^{y}),
R¹ is 5-membered heteroaryl, wherein R¹ is optionally substituted with one or more R¹⁰ which can be the same or different;
R², R³ and R⁷ are each independently selected from hydrogen, halogen, C₁₋₆-alkyl, C₂₋₆-alkynyl, hydroxy, cyano, halo-C₁₋₆-alkyl, N(R⁸R^{8a}), C₁₋₆-alkoxy, C₃₋₇-cycloalkyl and 3-10 membered heterocyclyl;
R⁴ is selected from C₅₋₁₄-aryl and 5-14 membered heteroaryl, wherein R⁴ is optionally substituted with one or more R¹¹ which can be the same or different;
R⁵ is selected from hydrogen, C₁₋₆-alkyl, -C(O)(R⁹), amino, amino-C₁₋₆-alkyl and C₃₋₇-cycloalkyl,
wherein said C₃₋₇-cycloalkyl is optionally substituted with one or more C₁₋₆-alkyl, amino, amino-C₁₋₆-alkyl-;
R⁶ and R^{6a} are each independently selected from hydrogen and C₁-₆-alkyl;
R⁸ and R^{8a} are each independently selected from hydrogen and C₁₋₆-alkyl;
R⁹ is selected from C₁₋₆-alkyl, hydroxy-C₁₋₆-alkyl, amino-C₁₋₆-alkyl- and -(C₁₋₆-alkyl)-N(R^{9a}R^{9b}), wherein said amino-C₁₋₆-alkyl is optionally substituted with one or more hydroxy, hydroxy-C₁₋₆-alkyl-;
R^{9a} and R^{9b} are each independently selected from hydrogen and C₁₋₆-alkyl, wherein said C₁₋₆-alkyl is optionally substituted with one or more hydroxy;
or R^{9a} and R^{9b}, taken together with the nitrogen atom to which they are attached, form a 3-10 membered heterocyclyl;
R¹⁰ is selected from:
   i) C₁₋₁₀-alkyl, optionally substituted with one or more halogen, C₂₋₆-alkynyl, halo-C₁₋₆-alkyl, amino, hydroxy, 5-6 membered heteroaryl, C₁₋₆-haloalkoxy, C₁₋₆-alkoxy, 3-10 membered cycloalkyl, C₁₋₃-alkyl, -N(R^{10a}R^{10b}), -S(O)₂(C₁₋₆-alkyl), - S(O)2(C₁₋₆-cycloalkyl), 3-10 membered heterocyclyl, phenyl, cyano, - C(O)N(R^{10c}R^{10d}), wherein 3-10 membered heterocyclyl, 3-10 membered cycloalkyl, and phenyl are optionally substituted with one or more C₁₋₁₀-alkyl, C₁₋₁₀-alkoxy, -S(O)₂(C₁₋₆-alkyl), oxo, halogen, C₂₋₆-alkynyl, 3-10 membered cycloalkyl;
   ii) C₃₋₁₀-cycloalkyl, optionally substituted with one or more halogen, C₁₋₁₀-alkyl, - S(O)₂(C₁₋₆-alkyl), cyano, C₁₋₆ haloalkyl, C₁₋₆-alkoxy, hydroxy, oxo, amino, - C(O)N(R^{10c}R^{10d}), =N(OH), hydroxy-C₁₋₆-alkyl;
   iii) 3-10 membered heterocyclyl, optionally substituted with one or more halogen, C₁₋₁₀-alkyl, -S(O)₂(C₁₋₆-alkyl), -C₁₋₁₀-alkyl-C₁₋₄-alkoxy, amino, -C(O)N(R^{10h}R¹⁰ⁱ), C₁₋₆-haloalkyl, C₁₋₆-alkoxy, cyano, hydroxy-C₁₋₁₀-alkyl, oxo, -C(O)(C₁₋₆-alkyl), - C(O)O-(R^{10q}), C₃₋₁₀-cycloalkyl;
   iv) phenyl, optionally substituted with one or more halogen, C₁₋₁₀-alkyl, -S(O)₂(C₁₋₆-alkyl);
   v) -N(R^{10e}R^{10f});
   vi) -OR^{10g};
   vii)-C(O)NR^{10h}R¹⁰ⁱ;
   viii) heteroaryl, optionally substituted with one or more C₁₋₁₀-alkyl, halogen, - SO₂(C₁₋₆-alkyl); and
   ix) oxo;
R^{10a} and R^{10b} are each independently selected from hydrogen, C₁₋₆-alkyl, halo-C₁₋₆-alkyl, hydroxy-C₁₋₆-alkyl, C₁₋₆-alkoxy,-C(O)(R^{10j}), amino-C₁₋₆-alkyl-, 3-10 membered heterocyclyl, - SO₂(R^{10k}), -C₁₋₆-alkyl-SO₂(R^{10k}) and -N(R^{10l}R^{10m}), wherein 3-10 membered heterocyclyl is optionally substituted with C₁₋₆-alkyl;
or R^{10a} and R^{10b}, taken together with the nitrogen atom to which they are attached, form a 3-10 membered heterocyclyl which is optionally substituted with one or more halogen, C₁₋₆-alkyl;
R^{10c} and R^{10d} are each independently selected from hydrogen and C₁₋₆-alkyl;
R^{10e} and R^{10f} are each independently selected from:
   i) hydrogen;
   ii) C₁₋₆-alkyl, optionally substituted with one or more cyano, in particular one cyano, halogen, hydroxy;
   iii) -C(O)R¹⁰ⁿ;
   iv) -C₁₋₁₀alkyl((O-C₁₋₁₀alkyl)ₘ), wherein m is an integer between 1 to 5 (more particularly m is 2 or 3), optionally substituted with one or more C₂₋₆-alkynyl;
   v) C₃₋₁₀-cycloalkyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, -C(O)O( R^{10o}), - C(O)(R^{10o}), -C(O)N(R^{10p})(R^{10p'}), -SO₂(C₁₋₆-alkyl), C2-6-alkynyl; and
   vi) 3-10 membered heterocyclyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, - C(O)O(R^{10o}), -C(O)(R^{10o}), -C(O)N(R^{10p})(R^{10p'}), -SO₂(C₁₋₆-alkyl), 3-10 membered cycloalkyl;
R^{10g} is selected from halo-C₁₋₆-alkyl, cyano, -C₁₋₁₀-alkyl-phenyl -C₁₋₆-alkyl-C₃₋₇-cycloalkyl and -C₁₋₆-alkoxy-halo-C₁₋₆-alkyl;
R^{10h} and R¹⁰ⁱ are each independently selected from hydrogen and C₁₋₆-alkyl, C₁₋₆-haloalkyl, wherein C₁₋₆-alkyl and C₁₋₆-haloalkyl are optionally substituted with one or more hydroxy,
or R^{10h} and R¹⁰ⁱ, taken together with the nitrogen atom to which they are attached, form a 3-10 membered heterocyclyl which is optionally substituted with one or more halogen, hydroxy, C₁₋₆-alkyl;
R^{10j} is selected from C₁₋₆-alkyl, halo-C₁₋₆-alkyl-, hydroxy-C₁₋₆-alkyl- and amino-C₁₋₆-alkyl-;
R^{10k} is selected from hydrogen, C₁₋₆-alkyl and halo-C₁₋₆-alkyl;
R^{10l} and R^{10m} are each independently selected from hydrogen and C₁₋₆-alkyl;
R¹⁰ⁿ is selected from C₁₋₆-alkyl, amino-C₁₋₆-alkyl-, halo-C₁₋₆-alkyl, C₃₋₇-cycloalkyl and 3-10 membered heterocyclyl, wherein said C₃₋₇-cycloalkyl and 3-10 membered heterocyclyl are optionally substituted with one or more halogen, C₁₋₆-alkyl;
R^{10o} is selected from C₁₋₆-alkyl and halo-C₁₋₆-alkyl;
R^{10p} and R^{10p'} are independently selected from hydrogen, C₁₋₆-alkyl and halo-C₁₋₆-alkyl;
R^{10q} is C₁₋₁₀-alkyl, C₁₋₆-haloalkyl, aryl, heteroaryl, wherein aryl and heteroaryl are optionally substituted with one or more halogen, C₁₋₁₀-alkyl;
R¹¹ is selected from:
   i) halogen;
   ii) hydroxy;
   iii) cyano;
   iv) C₁₋₁₀-alkyl, optionally substituted with one or more cyano, halogen, hydroxyl, C₃₋₇-cycloalkyl, amino, aryl, -O-aryl, 5-6 membered heteroaryl, 3-7 membered heterocyclyl, wherein 3-7 membered heterocyclyl and aryl are optionally substituted with one or more C₁₋₆-alkyl, -SO₂(C₁₋₆-alkyl), hydroxy, halogen, cyano;
   v) C₁₋₆-alkoxy, optionally substituted with one or more, particularly one, 3-10 membered heterocyclyl, halogen;
   vi) C₃₋₇-cycloalkyl;
   vii)3-10 membered heterocyclyl, optionally substituted with one or more halo-C₁₋₆-alkyl, hydroxy, C₁₋₆-alkyl, C₃₋₁₀-cycloalkyl, C₁₋₆-alkoxy, oxo;
   viii) 5-6 membered heteroaryl, optionally substituted with one or more C₁₋₆-alkyl, 3-10 membered cycloalkyl, halogen, halo-C₁₋₆-alkyl, C₁₋₆-alkoxy, C₁₋₆-haloalkoxy;
   ix) phenyl, optionally substituted with one or more halogen, cyano, C₁₋₆-alkoxy, C₁₋₆-haloalkyl, C₁₋₆-alkyl, C₁₋₆-haloalkoxy;
   x) -O(R^{11a});
   xi) -C(O)N(R^{11b}R^{11c});
   xii)-SO₂(R^{11d});
   xiii) -C(O)OR^{11e};
   xiv) -C(O)R^{11f};
   xv) oxo;
   xvi) -N(R^{11g}R^{11h}); and
   xvii) -S(R^{11k});
R^{11a} is selected from C₁₋₁₂-alkyl, halo-C₁₋₆-alkyl, amino-C₁₋₁₂-alkyl-, hydroxy-C₁₋₆-alkyl-, cyano, C₂₋₆-alkynyl, C₃₋₇-cycloalkyl, 3-10 membered heterocyclyl, 3-10 membered -(C₁₋₆-alkyl)heterocyclyl, 5-6 membered heteroaryl, phenyl, -C₁₋₆-alkyl-phenyl, -C₁₋₁₂-alkyl-C(O)N(R¹¹ⁱR^{11j}), -C₁₋₁₂-alkyl-NH-C(O)(C₁₋₆-alkyl), -C₁₋₁₂-alkoxy-NH-C(O)(C₁₋₆-alkyl), -C₁₋₆-alkyl-NH-C(O)(C₁₋₆-alkyl), -(CH₂CH₂O)ₙ-CH₂CH₂NH₂ and -(CH₂CH₂O)ₙ-CH₂CH₂₋NH-C(O)(C₁₋₆-alkyl);
wherein said C₁₋₁₂-alkyl, C₃₋₇-cycloalkyl, 3-10 membered heterocyclyl, 5-6 membered heteroaryl, phenyl and -C₁₋₆-alkyl-phenyl are optionally substituted with one or more halogen, C₁₋₆-alkyl, halo-C₁₋₆-alkyl, C₁₋₆-alkoxyl, C₁₋₆-haloalkoxyl, cyano;
n is an integer between 1 and 6, in particular wherein n is two or three;
R^{11b} and R^{11c} are each independently selected from hydrogen, C₁₋₆-alkyl and halo-C₁₋₆-alkyl,
or R^{11b} and R^{11c}, taken together with the nitrogen atom to which they are attached, form a 3-10 membered heterocyclyl;
R^{11d} is selected from hydrogen, C₁₋₆-alkyl, -N(R^{11l}R^{11m}), halo-C₁₋₆-alkyl and phenyl;
R^{11e} is selected from hydrogen and C₁₋₆-alkyl;
R^{11f} is selected from hydrogen, C₁₋₆-alkyl and phenyl;
R^{11g} and R^{11h} are each independently selected from hydrogen, C₁₋₆-alkyl, -(C₁₋₆-alkyl)phenyl, halo-C₁₋₆-alkyl, -SO₂(C₁₋₆-alkyl), -SO₂(halo-C₁₋₆-alkyl) and -SO(C₁₋₆-alkyl)₂,
R¹¹ⁱR^{11j} are each independently selected from hydrogen, C₁₋₆-alkyl and halo-C₁₋₆-alkyl,
or R¹¹ⁱR^{11j}, taken together with the nitrogen atom to which they are attached, form a 3-10 membered heterocyclyl;
R^{11k} is selected from hydrogen, C₁₋₆-alkyl and halo-C₁₋₆-alkyl;
R^{11l} and R^{11m} are each independently selected from hydrogen and C₁₋₆-alkyl,
or R^{11l} and R^{11m}, taken together with the nitrogen atom to which they are attached, form a 3-10 membered heterocyclyl;
R^{y} is selected from hydrogen and C₁₋₆-alkyl.

A second object of the present invention is a compound is of formula (I) 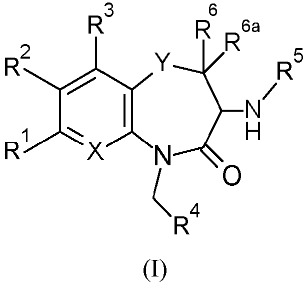 or a pharmaceutically acceptable salt thereof, wherein:
X is C(R⁷) or N;
Y is S, S(O), S(O)₂, S(O)N(R^{y}),
R¹ is 5-membered heteroaryl, wherein R¹ is optionally substituted with one or more R¹⁰ which can be the same or different;
R², R³ and R⁷ are each independently selected from hydrogen, halogen, C₁₋₆-alkyl, C₂₋₆-alkynyl, hydroxy, cyano, halo-C₁₋₆-alkyl, N(R⁸R^{8a}), C₁₋₆-alkoxy, C₃₋₇-cycloalkyl and 3-10 membered heterocyclyl;
R⁴ is selected from C₅₋₁₄-aryl and 5-14 membered heteroaryl, wherein R⁴ is optionally substituted with one or more R¹¹ which can be the same or different;
R⁵ is selected from hydrogen, C₁₋₆-alkyl, -C(O)(R⁹), amino, amino-C₁₋₆-alkyl and C₃₋₇-cycloalkyl,
wherein said C₃₋₇-cycloalkyl is optionally substituted with one or more C₁₋₆-alkyl, amino, amino-C₁₋₆-alkyl-;
R⁶ and R^{6a} are each independently selected from hydrogen and C₁-₆-alkyl;
R⁸ and R^{8a} are each independently selected from hydrogen and C₁₋₆-alkyl;
R⁹ is selected from C₁₋₆-alkyl, hydroxy-C₁₋₆-alkyl, amino-C₁₋₆-alkyl- and -(C₁₋₆-alkyl)-N(R^{9a}R^{9b}), wherein said amino-C₁₋₆-alkyl is optionally substituted with one or more hydroxy, hydroxy-C₁₋₆-alkyl;
R^{9a} and R^{9b} are each independently selected from hydrogen and C₁₋₆-alkyl, wherein said C₁₋₆-alkyl is optionally substituted with one or more hydroxy;
or R^{9a} and R^{9b}, taken together with the nitrogen atom to which they are attached, form a 3-10 membered heterocyclyl;
R¹⁰ is selected from:
   i) C₁₋₁₀-alkyl, optionally substituted with one or more halogen, C₂₋₆-alkynyl, halo-C₁₋₆-alkyl, amino, hydroxy, C₁₋₆-alkoxy, -N(R^{10a}R^{10b}), -S(O)₂(C₁₋₆-alkyl), - S(O)2(C1-6-cycloalkyl), 3-10 membered heterocyclyl, cyano, -C(O)N(R^{10c}R^{10d}), wherein 3-10 membered heterocyclyl is optionally substituted with one or more C₁₋₁₀-alkyl, C1-10-alkoxy , oxo, halogen;
   ii) C₁₋₁₀-haloalkyl, optionally substituted with one or more hydroxy, C₁₋₆-alkoxy, amino, phenyl, wherein phenyl is optionally substituted with one or more C₁₋₁₀-alkyl, halogen;
   iii) amino-C₁₋₁₀-alkyl- optionally substituted with one or more amino, halogen, C₁₋₆-haloalkyl, C₁₋₃-alkyl, cyano, 3-10 membered cycloalkyl, 3-10 membered heterocyclyl, C₁₋₆-haloalkoxy, C₁₋₆-alkoxy wherein 3-10 membered heterocyclyl and 3-10 membered cycloalkyl are optionally substituted with one or more halogen, C₂₋₆-alkynyl, 3-10 membered cycloalkyl;
   iv) hydroxy-C₁₋₁₀-alkyl-;
   v) C₁₋₆-alkoxy, optionally substituted with one or more cyano;
   vi) C₁₋₆-alkoxy-C₁₋₁₀-alkyl-;
   vii)C₃₋₁₀-cycloalkyl, optionally substituted with one or more halogen, C₁₋₁₀-alkyl, - S(O)₂(C₁₋₆-alkyl), cyano, C₁₋₆ haloalkyl, C₁₋₆-alkoxy, hydroxy, oxo, amino, - C(O)N(R^{10c}R^{10d}), =N(OH), hydroxy-C₁₋₆-alkyl;
   viii) 3-10 membered heterocyclyl, optionally substituted with one or more halogen, C₁₋₁₀-alkyl, -S(O)₂(C₁₋₆-alkyl), -C₁₋₁₀-alkyl-C₁₋₄-alkoxy, amino, - C(O)N(R^{10h}R¹⁰ⁱ), C₁₋₆-haloalkyl, C₁₋₆-alkoxy, cyano, hydroxy-C₁₋₁₀-alkyl, oxo, - C(O)(C₁₋₆-alkyl), -C(O)O-(R^{10q}), C₃₋₁₀-cycloalkyl;
   ix) -(C₁₋₆-alkyl)-C₃₋₇-cycloalkyl;
   x) 3-10 membered -(C₁₋₆-alkyl)-heterocyclyl, optionally substituted with one or more halogen, C₁₋₁₀-alkyl;
   xi) phenyl, optionally substituted with one or more halogen, C₁₋₁₀-alkyl, -S(O)₂(C₁₋₆-alkyl);
   xii)-(C₁₋₁₀-alkyl)-phenyl, optionally substituted with one or more halogen, wherein C₁₋₁₀-alkyl is optionally substituted with C₁₋₆-alkyl, cyano;
   xiii) 5-6 membered -(C₁₋₁₀-alkyl)-heteroaryl;
   xiv) -(alkoxy-C₁₋₁₀-alkyl)-phenyl;
   xv) -(amino-C₁-₁₀-alkyl)-phenyl;
   xvi) -C₁₋₆-alkyl-SO₂(C₁₋₆-alkyl);
   xvii) -N(R^{10e}R^{10f});
   xviii) -OR^{10g}; and
   xix) -C(O)NR^{10h}R¹⁰ⁱ;
   xx) heteroaryl, optionally substituted with one or more C₁₋₁₀-alkyl, halogen, -SO₂(C₁₋₆-alkyl); and
   xxi) oxo
R^{10a} and R^{10b} are each independently selected from hydrogen, C₁₋₆-alkyl, halo-C₁₋₆-alkyl, hydroxy-C₁₋₆-alkyl, C₁₋₆-alkoxy,-C(O)(R^{10j}), amino-C₁₋₆-alkyl-, 3-10 membered heterocyclyl, - SO₂(R^{10k}), -C₁₋₆-alkyl-SO₂(R^{10k}) and -N(R^{10l}R^{10m}), wherein 3-10 membered heterocyclyl is optionally substituted with C₁₋₆-alkyl;
or R^{10a} and R^{10b}, taken together with the nitrogen atom to which they are attached, form a 3-10 membered heterocyclyl which is optionally substituted with one or more halogen, C₁₋₆-alkyl;
R^{10c} and R^{10d} are each independently selected from hydrogen and C₁₋₆-alkyl;
R^{10e} and R^{10f} are each independently selected from:
   i) hydrogen;
   ii) C₁₋₆-alkyl, optionally substituted with one or more cyano, in particular one cyano;
   iii) halo-C₁₋₆-alkyl, wherein halo-C₁₋₆-alkyl is optionally substituted with one or more hydroxy;
   iv) hydroxy-C₁₋₆-alkyl;
   v) -C(O)R¹⁰ⁿ;
   vi) -C₁₋₁₀alkyl((O-C₁₋₁₀alkyl)ₘ), wherein m is an integer between 1 to 5 (more particularly m is 2 or 3), optionally substituted with one or more C₂₋₆-alkynyl;
   vii)C₃₋₁₀-cycloalkyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, -C(O)O( R^{10o}), - C(O)(R^{10o}), -C(O)N(R^{10p})(R^{10p'}), -SO₂(C₁₋₆-alkyl), C2-6-alkynyl;
   viii) 3-10 membered heterocyclyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, -C(O)O(R^{10o}), -C(O)(R^{10o}), -C(O)N(R^{10p})(R^{10p'}), -SO₂(C₁₋₆-alkyl), 3-10 membered cycloalkyl;
or R^{10e} and R^{10f}, taken together with the nitrogen atom to which they are attached, form a 3-10 membered heterocyclyl which is optionally substituted with one or more halogen, amino, C₁₋₆-alkyl, halo-C₁₋₆-alkyl, hydroxy-C₁₋₆-alkyl, C₁₋₆-alkoxy, C₃₋₇-cycloalkyl, -C(O)O(C₁₋₆-alkyl);
R^{10g} is selected from halo-C₁₋₆-alkyl, C₁₋₆-alkyl-C₃₋₇-cycloalkyl and C₁₋₆-alkoxy-halo-C₁₋₆-alkyl;
R^{10h} and R¹⁰ⁱ are each independently selected from hydrogen and C₁₋₆-alkyl, C₁₋₆-haloalkyl, wherein C₁₋₆-alkyl and C₁₋₆-haloalkyl are optionally substituted with one or more hydroxy,
or R^{10h} and R¹⁰ⁱ, taken together with the nitrogen atom to which they are attached, form a 3-10 membered heterocyclyl which is optionally substituted with one or more halogen, hydroxy, C₁₋₆-alkyl;
R¹⁰ is selected from C₁₋₆-alkyl, halo-C₁₋₆-alkyl, hydroxy-C₁₋₆-alkyl and amino-C₁₋₆-alkyl-;
R^{10k} is selected from hydrogen, C₁₋₆-alkyl and halo-C₁₋₆-alkyl;
R^{10l} and R^{10m} are each independently selected from hydrogen and C₁₋₆-alkyl;
R¹⁰ⁿ is selected from C₁₋₆-alkyl, amino-C₁₋₆-alkyl-, halo-C₁₋₆-alkyl, C₃₋₇-cycloalkyl and 3-10 membered heterocyclyl, wherein said C₃₋₇-cycloalkyl and 3-10 membered heterocyclyl are optionally substituted with one or more halogen, C₁₋₆-alkyl;
R^{10o} is selected from C₁₋₆-alkyl and halo-C₁₋₆-alkyl;
R^{10p} and R^{10p'} are independently selected from hydrogen, C₁₋₆-alkyl and halo-C₁₋₆-alkyl;
R^{10q} is C₁₋₁₀-alkyl, C₁₋₆-haloalkyl, aryl, heteroaryl, wherein aryl and heteroaryl are optionally substituted with one or more halogen, C₁₋₁₀-alkyl;
R¹¹ is selected from:
   i) halogen;
   ii) hydroxy;
   iii) cyano;
   iv) C₁₋₆-alkyl, optionally substituted with one or more cyano, aryl, haloaryl;
   v) C₁₋₆-alkoxy, optionally substituted with one or more, particularly one, 3-10 membered heterocyclyl;
   vi) halo-C₁₋₆-alkyl;
   vii)amino-C₁₋₁₀-alkyl-;
   viii) hydroxy-C₁₋₆-alkyl;
   ix) C₃₋₇-cycloalkyl;
   x) -C₁₋₆-alkyl-C₃₋₇-cycloalkyl;
   xi) 3-10 membered heterocyclyl, optionally substituted with one or more halo-C₁₋₆-alkyl, hydroxy, C₁₋₆-alkyl, C₃₋₁₀-cycloalkyl, C₁₋₆-alkoxy, oxo;
   xii)3-7 membered -(C₁₋₆-alkyl)-heterocyclyl, optionally substituted with one or more C₁₋₆-alkyl;
   xiii) 5-6 membered heteroaryl, optionally substituted with one or more C₁₋₆-alkyl, 3-10 membered cycloalkyl, halogen, halo-C₁₋₆-alkyl, C₁₋₆-alkoxy; C₁₋₆-haloalkoxy;
   xiv) phenyl, optionally substituted with one or more halogen, cyano, C₁₋₆-alkoxy, halo-C₁₋₆-alkyl, C₁₋₆-haloalkoxy, C₁₋₆-alkyl;
   xv) -(C₁₋₆-alkyl)-phenyl, optionally substituted with one or more -SO₂(C₁₋₆-alkyl), hydroxy, halogen, cyano;
   xvi) -(C₁₋₆-alkyl)-O-phenyl;
   xvii) 5-6 membered -(C₁₋₆-alkyl)-heteroaryl;
   xviii) -O(R^{11a});
   xix) -C(O)N(R^{11b}R^{11c});
   xx) -SO₂(R^{11d});
   xxi) -C(O)OR^{11e};
   xxii) -C(O)R^{11f};
   xxiii) oxo;
   xxiv) -N(R^{11g}R^{11h});
   xxv) -S(R^{11k}); and
   xxvi) C₁₋₆-haloalkoxyl;
R^{11a} is selected from C₁₋₁₂-alkyl, halo-C₁₋₆-alkyl, amino-C₁₋₁₂-alkyl-, hydroxy-C₁₋₆-alkyl, cyano, -C₁₋₆-alkyl, C₂₋₆-alkynyl, C₃₋₇-cycloalkyl, 3-10 membered heterocyclyl, 3-10 membered - (C₁₋₆-alkyl)heterocyclyl, 5-6 membered heteroaryl, phenyl, -C₁₋₆-alkyl-phenyl, -C₁₋₁₂-alkyl-C(O)N(R¹¹ⁱR^{11j}), -C₁₋₁₂-alkyl-NH-C(O)(C₁₋₆-alkyl), -C₁₋₁₂-alkoxy-NH-C(O)(C₁₋₆-alkyl), -C₁₋₆-alkyl-NH-C(O)(C₁₋₆-alkyl), -(CH₂CH₂O)ₙ-CH₂CH₂NH₂ and -(CH₂CH₂O)ₙ-CH₂CH₂₋NH-C(O)(C₁₋₆-alkyl);
wherein said C₁₋₁₂-alkyl, C₃₋₇-cycloalkyl, 3-10 membered heterocyclyl, 5-6 membered heteroaryl, phenyl and -C₁₋₆-alkyl-phenyl are optionally substituted with one or more halogen, C₁₋₆-alkyl, halo-C₁₋₆-alkyl, C₁₋₆-alkoxyl, C₁₋₆-haloalkoxyl, cyano;
n is an integer between 1 and 6;
R^{11b} and R^{11c} are each independently selected from hydrogen, C₁₋₆-alkyl and halo-C₁₋₆-alkyl,
or R^{11b} and R^{11c}, taken together with the nitrogen atom to which they are attached, form a 3-10 membered heterocyclyl;
R^{11d} is selected from hydrogen, C₁₋₆-alkyl, -N(R^{11l}R^{11m}), halo-C₁₋₆-alkyl and phenyl;
R^{11e} is selected from hydrogen and C₁₋₆-alkyl;
R^{11f} is selected from hydrogen, C₁₋₆-alkyl and phenyl;
R^{11g} and R^{11h} are each independently selected from hydrogen, C₁₋₆-alkyl, -(C₁₋₆-alkyl)phenyl. halo-C₁₋₆-alkyl, -SO₂(C₁₋₆-alkyl), -SO₂(halo-C₁₋₆-alkyl) and -SO(C₁₋₆-alkyl)₂,
or R^{11g} and R^{11h}, taken together with the nitrogen atom to which they are attached, form a 3-10 membered heterocyclyl optionally substituted with C₁₋₆-alkyl, C₁₋₆-alkoxy;
R¹¹ⁱR^{11j} are each independently selected from hydrogen, C₁₋₆-alkyl and halo-C₁₋₆-alkyl,
or R¹¹ⁱR^{11j}, taken together with the nitrogen atom to which they are attached, form a 3-10 membered heterocyclyl;
R^{11k} is selected from hydrogen, C₁₋₆-alkyl and halo-C₁₋₆-alkyl;
R^{11l} and R^{11m} are each independently selected from hydrogen and C₁₋₆-alkyl,
or R^{11l} and R^{11m}, taken together with the nitrogen atom to which they are attached, form a 3-10 membered heterocyclyl;
R^{y} is selected from hydrogen and C₁₋₆-alkyl.

A third object of the present invention is a compound of formula (I) 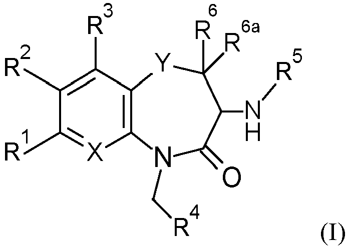 or a pharmaceutically acceptable salt thereof, wherein:
X is C(R⁷) or N;
Y is S, S(O), S(O)₂, S(O)N(R^{y}),
R¹ is 5-membered heteroaryl, wherein R¹ is optionally substituted with one or more R¹⁰ which can be the same or different;
R², R³ and R⁷ are each independently selected from hydrogen, halogen, C₁₋₆-alkyl, C₂₋₆-alkynyl, hydroxy, cyano, halo-C₁₋₆-alkyl, N(R⁸R^{8a}), C₁₋₆-alkoxy, C₃₋₇-cycloalkyl and 3-10 membered heterocyclyl;
R⁴ is selected from C₅₋₁₄-aryl and 5-14 membered heteroaryl, wherein R⁴ is optionally substituted with one or more R¹¹ which can be the same or different;
R⁵ is selected from hydrogen, C₁₋₆-alkyl, -C(O)(R⁹), amino, amino-C₁₋₆-alkyl and C₃₋₇-cycloalkyl,
wherein said C₃₋₇-cycloalkyl is optionally substituted with one or more C₁₋₆-alkyl, amino, amino-C₁₋₆-alkyl;
R⁶ and R^{6a} are each independently selected from hydrogen and C₁-₆-alkyl;
R⁸ and R^{8a} are each independently selected from hydrogen and C₁₋₆-alkyl;
R⁹ is selected from C₁₋₆-alkyl, hydroxy-C₁₋₆-alkyl, amino-C₁₋₆-alkyl and (C₁₋₆-alkyl)-N(R^{9a}R^{9b}), wherein said amino-C₁₋₆-alkyl is optionally substituted with one or more hydroxy, hydroxy-C₁₋₆-alkyl;
R^{9a} and R^{9b} are each independently selected from hydrogen and C₁₋₆-alkyl, wherein said C₁₋₆-alkyl is optionally substituted with one or more hydroxy;
or R^{9a} and R^{9b}, taken together with the nitrogen atom to which they are attached, form a 3-10 membered heterocyclyl;
R¹⁰ is selected from
   i) C₁₋₁₀-alkyl, optionally substituted with one or more halogen, C₂₋₆-alkynyl, halo-C₁₋₆-alkyl, amino, -N(R^{10a}R^{10b}), -S(O)₂(C₁₋₆-alkyl), 3-10 membered heterocyclyl, cyano, -C(O)N(R^{10c}R^{10d});
   ii) C₁₋₁₀-haloalkyl;
   iii) amino-C₁₋₁₀-alkyl;
   iv) hydroxy-C₁₋₁₀-alkyl;
   v) C₁₋₆-alkoxy;
   vi) C₁₋₆-alkoxy-C₁₋₁₀-alkyl;
   vii) C₃₋₁₀-cycloalkyl, optionally substituted with one or more halogen, C₁₋₁₀-alkyl, - S(O)₂(C₁₋₆-alkyl), hydroxy, oxo, amino, -C(O)N(R^{10c}R^{10d}), =N(OH), hydroxy-C₁₋₆-alkyl;
   viii) 3-10 membered heterocyclyl, optionally substituted with one or more halogen, C₁₋₁₀-alkyl, -S(O)₂(C₁₋₆-alkyl), cyano, hydroxy-C₁₋₁₀-alkyl, oxo, -C(O)(C₁₋₆-alkyl), - C(O)O-(C₁₋₆-alkyl), C₃₋₁₀-cycloalkyl;
   ix) (C₁₋₆-alkyl)-C₃₋₇-cycloalkyl;
   x) 3-10 membered (C₁₋₆-alkyl)-heterocyclyl, optionally substituted with one or more halogen, C₁₋₁₀-alkyl;
   xi) phenyl, optionally substituted with one or more halogen;
   xii) (C₁₋₁₀-alkyl)-phenyl, optionally substituted with one or more halogen, wherein C₁₋₁₀-alkyl is optionally substituted with C₁₋₆-alkyl, cyano;
   xiii) 5-6 membered (C₁₋₁₀-alkyl)-heteroaryl;
   xiv) (alkoxy-C₁₋₁₀-alkyl)-phenyl;
   xv) (amino-C₁-₁₀-alkyl)-phenyl;
   xvi) -C₁₋₆-alkyl-SO₂(C₁₋₆-alkyl);
   xvii) -N(R^{10e}R^{10f});
   xviii) -OR^{10g}; and
   xix) -C(O)NR^{10h}R¹⁰ⁱ;
R^{10a} and R^{10b} are each independently selected from hydrogen, C₁₋₆-alkyl, halo-C₁₋₆-alkyl, hydroxy-C₁₋₆-alkyl, C₁₋₆-alkoxy,-C(O)(R^{10j}), amino-C₁₋₆-alkyl, 3-10 membered heterocyclyl, - SO₂(R^{10k}), C₁₋₆-alkyl-SO₂(R^{10k}) and -N(R^{10l}R^{10m}), wherein 3-10 membered heterocyclyl is optionally substituted with C₁₋₆-alkyl; or R^{10a} and R^{10b}, taken together with the nitrogen atom to which they are attached, form a 3-10 membered heterocyclyl which is optionally substituted with one or more halogen, C₁₋₆-alkyl;
R^{10c} and R^{10d} are each independently selected from hydrogen and C₁₋₆-alkyl;
R^{10e} and R^{10f} are each independently selected from hydrogen, C₁₋₆-alkyl, halo-C₁₋₆-alkyl, hydroxy-C₁₋₆-alkyl, -C(O)R¹⁰ⁿ, C₃₋₁₀-cycloalkyl and 3-10 membered heterocyclyl, wherein said C₃₋₁₀-cycloalkyl and 3-10 membered heterocyclyl are optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, - C(O)O( R^{10o}), -C(O)(R^{10o}), -C(O)N(R^{10p})(R^{10p'}), -SO₂(C₁₋₆-alkyl); or R^{10e} and R^{10f}, taken together with the nitrogen atom to which they are attached, form a 3-10 membered heterocyclyl which is optionally substituted with one or more halogen, amino, C₁₋₆-alkyl, halo-C₁₋₆-alkyl, hydroxy-C₁₋₆-alkyl, C₁₋₆-alkoxy, C₃₋₇-cycloalkyl, -C(O)O(C₁₋₆-alkyl);
R^{10g} is selected from halo-C₁₋₆-alkyl, C₁₋₆-alkyl-C₃₋₇-cycloalkyl and C₁₋₆-alkoxy-halo-C₁₋₆-alkyl;
R^{10h} and R¹⁰ⁱ are each independently selected from hydrogen and C₁₋₆-alkyl, or R^{10h} and R¹⁰ⁱ, taken together with the nitrogen atom to which they are attached, form a 3-10 membered heterocyclyl which is optionally substituted with one or more halogen, hydroxy, C₁₋₆-alkyl;
R¹⁰ is selected from C₁₋₆-alkyl, halo-C₁₋₆-alkyl, hydroxy-C₁₋₆-alkyl and amino-C₁₋₆-alkyl;
R^{10k} is selected from hydrogen, C₁₋₆-alkyl and halo-C₁₋₆-alkyl;
R^{10l} and R^{10m} are each independently selected from hydrogen and C₁₋₆-alkyl;
R¹⁰ⁿ is selected from C₁₋₆-alkyl, amino-C₁₋₆-alkyl, halo-C₁₋₆-alkyl, C₃₋₇-cycloalkyl and 3-10 membered heterocyclyl, wherein said C₃₋₇-cycloalkyl and 3-10 membered heterocyclyl are optionally substituted with one or more halogen, C₁₋₆-alkyl;
R^{10o} is selected from C₁₋₆-alkyl and halo-C₁₋₆-alkyl;
R^{10p} and R^{10p'} are independently selected from hydrogen, C₁₋₆-alkyl and halo-C₁₋₆-alkyl;
R¹¹ is selected from
   i) halogen;
   ii) hydroxy;
   iii) cyano;
   iv) C₁₋₆-alkyl, optionally substituted with one or more cyano;
   v) C₁₋₆-alkoxy;
   vi) halo-C₁₋₆-alkyl;
   vii) amino-C₁₋₁₀-alkyl;
   viii) hydroxy-C₁₋₆-alkyl;
   ix) C₃₋₇-cycloalkyl;
   x) C₁₋₆-alkyl-C₃₋₇-cycloalkyl;
   xi) 3-10 membered heterocyclyl, optionally substituted with one or more halo-C₁₋₆-alkyl, hydroxy, C₁₋₆-alkyl, C₁₋₆-alkoxy, oxo;
   xii) 3-7 membered (C₁₋₆-alkyl)-heterocyclyl, optionally substituted with one or more C₁₋₆-alkyl;
   xiii) 5-6 membered heteroaryl, optionally substituted with one or more C₁₋₆-alkyl, halogen, halo-C₁₋₆-alkyl;
   xiv) phenyl, optionally substituted with one or more halogen, cyano;
   xv) (C₁₋₆-alkyl)-phenyl, optionally substituted with one or more -SO₂(C₁₋₆-alkyl), hydroxy, halogen, cyano;
   xvi) (C₁₋₆-alkyl)-O-phenyl;
   xvii) 5-6 membered (C₁₋₆-alkyl)-heteroaryl;
   xviii) -O(R^{11a});
   xix) -C(O)N(R^{11b}R^{11c});
   xx) -SO₂(R^{11d});
   xxi) -C(O)OR^{11e};
   xxii) -C(O)R^{11f};
   xxiii) oxo;
   xxiv) -N(R^{11g}R^{11h}); and
   xxv) -S(R^{11k});
R^{11a} is selected from C₁₋₁₂-alkyl, halo-C₁₋₆-alkyl, amino-C₁₋₁₂-alkyl, hydroxy-C₁₋₆-alkyl, cyano, - C₁₋₆-alkyl, C₂₋₆-alkynyl, C₃₋₇-cycloalkyl, 3-10 membered heterocyclyl, 5-6 membered heteroaryl, phenyl, -C₁₋₆-alkyl-phenyl, C₁₋₁₂-alkyl-C(O)N(R¹¹ⁱR^{11j}), -C₁₋₁₂-alkyl-NH-C(O)(C₁₋₆-alkyl), -C₁₋₁₂-alkoxy-NH-C(O)(C₁₋₆-alkyl), -C₁₋₆-alkyl-NH-C(O)(C₁₋₆-alkyl), -(CH₂CH₂O)ₙ-CH₂CH₂NH₂ and - (CH₂CH₂O)ₙ-CH₂CH₂₋NH-C(O)(C₁₋₆-alkyl);
wherein said C₁₋₁₂-alkyl, C₃₋₇-cycloalkyl, 3-10 membered heterocyclyl, 5-6 membered heteroaryl, phenyl and C₁₋₆-alkyl-phenyl are optionally substituted with one or more halogen, C₁₋₆-alkyl, halo-C₁₋₆-alkyl, cyano;
n is an integer between 1 and 6, in particular n is two or three;
R^{11b} and R^{11c} are each independently selected from hydrogen, C₁₋₆-alkyl and halo-C₁₋₆-alkyl, or R^{11b} and R^{11c}, taken together with the nitrogen atom to which they are attached, form a 3-10 membered heterocyclyl;
R^{11d} is selected from hydrogen, C₁₋₆-alkyl, -N(R^{11l}R^{11m}), halo-C₁₋₆-alkyl and phenyl;
R^{11e} is selected from hydrogen and C₁₋₆-alkyl;
R^{11f} is selected from hydrogen, C₁₋₆-alkyl and phenyl;
R^{11g} and R^{11h} are each independently selected from hydrogen, C₁₋₆-alkyl, halo-C₁₋₆-alkyl, SO₂(C_{1- 6}-alkyl), SO₂(halo-C₁₋₆-alkyl) and SO(C₁₋₆-alkyl)₂, or R^{11g} and R^{11h}, taken together with the nitrogen atom to which they are attached, form a 3-10 membered heterocyclyl optionally substituted with C₁₋₆-alkyl, C₁₋₆-alkoxy;
R¹¹ⁱR^{11j} are each independently selected from hydrogen, C₁₋₆-alkyl and halo-C₁₋₆-alkyl, or R¹¹ⁱR^{11j}, taken together with the nitrogen atom to which they are attached, form a 3-10 membered heterocyclyl;
R^{11k} is selected from hydrogen, C₁₋₆-alkyl and halo-C₁₋₆-alkyl;
R^{11l} and R^{11m} are each independently selected from hydrogen and C₁₋₆-alkyl, or R^{11l} and R^{11m}, taken together with the nitrogen atom to which they are attached, form a 3-10 membered heterocyclyl;
R^{y} is selected from hydrogen and C₁₋₆-alkyl.

A further object of the present invention is a process for the preparation of a compound as described herein, or a pharmaceutically acceptable salt thereof, comprising
a) reacting a compound of formula (IX) 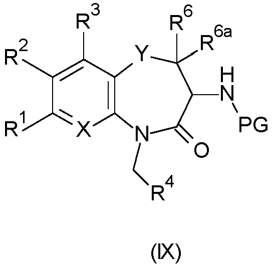 wherein X, Y, R¹, R², R³, R⁴, R⁶, R^{6a} are as described herein and PG is an amino protecting group, with a suitable deprotection agent to form said compound of formula (I), or a pharmaceutically acceptable salt thereof, wherein X, Y, R¹, R², R³, R⁴, R⁶, R^{6a} are as described herein and R⁵ is hydrogen; or
b) reacting a compound of formula (Ia) 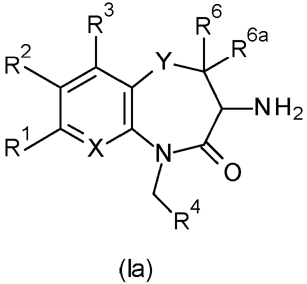 wherein X, Y, R¹, R², R³, R⁴, R⁶, R^{6a} are as described herein and R⁵ is hydrogen, with a carboxylic acid derivative of formula R⁹CO₂H wherein R⁹ is as described herein, in the presence of a base to form a compound of formula (I), or a pharmaceutically acceptable salt thereof, wherein R⁵ is -C(O)(R⁹).

A further object of the present invention is a compound of formula (I) as described herein, or a pharmaceutically acceptable salt thereof, for use as therapeutically active substance.

A further object of the present invention is a pharmaceutical composition comprising a compound of formula (I) as described herein, or a pharmaceutically acceptable salt thereof, and a pharmaceutically acceptable excipient.

A further object of the present invention is a compound of formula (I) as described herein, or a pharmaceutically acceptable salt thereof, for use in the treatment, prevention and/or delay of progression of cancer. A further object of the present invention is the use of a compound of formula (I) as described herein, or a pharmaceutically acceptable salt thereof, for the preparation of a medicament for the treatment, prevention and/or delay of progression of cancer.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below.

The nomenclature used in this application is based on IUPAC systematic nomenclature, unless indicated otherwise.

### Brief description of the figures

Figure 1 provides a summary of representative DGK-regulated signaling pathways (Sim, J.A.; Kim, J.; Yang, D. Beyond Lipid Signaling: Pleiotropic Effects of Diacylglycerol Kinases in Cellular Signaling. Int. J. Mol. Sci. 2020, 21, 6861)

### Detailed description of the invention

### Definitions

The following definitions of the general terms used in the present description apply irrespectively of whether the terms in question appear alone or incombination with other groups. The term "alkyl" refers to a saturated linear (i.e. unbranched) or branched univalent hydrocarbon chain or combination thereof, having the number of carbon atoms designated (*i.e.,* C₁-₁₀ means one to ten carbon atoms). Particular alkyl groups are those having 1 to 20 carbon atoms (a "C₁-₂₀ alkyl"), having 1 to 12 carbon atoms (a "C₁-₁₂ alkyl"), having 1 to 10 carbon atoms (a "C₁-₁₀ alkyl"), having 1 to 8 carbon atoms (a "C₁-₈ alkyl"), having 1 to 6 carbon atoms (a "C₁-₆ alkyl"), having 2 to 6 carbon atoms (a "C₂-₆ alkyl"), or having 1 to 4 carbon atoms (a "C₁₋₄ alkyl"). Examples of alkyl group include, but are not limited to, groups such as methyl, ethyl, n-propyl, isopropyl, n-butyl, t-butyl, isobutyl, sec-butyl, homologs and isomers of, for example, n-pentyl, n-hexyl, n-heptyl, n-octyl, and the like.

The term "alkynyl" refers to an unsaturated linear (i.e. unbranched) or branched univalent hydrocarbon chain or combination thereof, having at least one site of acetylenic unsaturation (*i.e.,* having at least one moiety of the formula C≡C) having the number of carbon atoms designated (*i.e.* C₂₋₁₀ means two to ten carbon atoms). Particular alkynyl groups are those having 2 to 20 carbon atoms (a "C₂₋₂₀ alkynyl"), having 2 to 8 carbon atoms (a "C₂₋₈ alkynyl"), having 2 to 6 carbon atoms (a "C₂₋₆ alkynyl"), having 2 to 4 carbon atoms (a "C₂₋₄ alkynyl"). Examples of alkynyl group include, but are not limited to, groups such as ethynyl (or acetylenyl), prop-1-ynyl, prop-2-ynyl (or propargyl), but-1-ynyl, but-2-ynyl, but-3-ynyl, homologs and isomers thereof, and the like.

The term "alkoxy" refers to an alkyl group, as previously defined, attached to the parent molecular moiety via an oxygen atom. Unless otherwise specified, the alkoxy group contains 1 to 12 carbon atoms ("C₁₋₁₂-alkoxy"), preferably 1 to 10 carbon atoms ("C₁₋₁₀-alkoxy"), more preferably 1 to 6 carbon atoms ("C₁₋₆-alkoxy"). In some preferred embodiments, the alkoxy group contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy.

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms, most preferably one hydrogen atom of the alkyl group have been replaced by an alkoxy group. Particularly preferred, yet non-limiting examples of alkoxyalkyl is methoxymethyl and 2-methoxyethyl.

The term "amino", alone or in combination with other groups, refers to NH₂.

The term "aminoalkyl" refers to an alkyl group wherein one or more of the hydrogen atoms of the alkyl group have been replaced by an amino moiety.

The term "aromatic" denotes the conventional idea of aromaticity as defined in the literature, in particular in IUPAC - Compendium of Chemical Terminology, 2^{nd} Edition, A. D. McNaught & A. Wilkinson (Eds). Blackwell Scientific Publications, Oxford (1997).

The term "cyano", alone or in combination with other groups, refers to CN (i.e. nitrile).

The term "cyanoalkyl" refers to an alkyl group wherein one or more of the hydrogen atoms of the alkyl group have been replaced by a cyano moiety.

The term "cycloalkyl" means a saturated or partially unsaturated carbocyclic moiety having mono-, bi- (including bridged bicyclic and cycloalkyl spiro moieties) or tricyclic rings and 3 to 10 carbon atoms i.e., (C₃-C₁₀)cycloalkyl) in the ring. The cycloalkyl moiety can optionally be substituted with one or more substituents. In particular aspects cycloalkyl contains from 3 to 8 carbon atoms (i.e., (C₃-C₈)cycloalkyl). In other particular aspects cycloalkyl contains from 3 to 6 carbon atoms (i.e., (C₃-C₆)cycloalkyl). Examples of cycloalkyl moieties include, but are not limited to, cyclopropyl, cyclobutyl, cyclopentyl, cyclohexyl, cycloheptyl, and partially unsaturated (cycloalkenyl) derivatives thereof (e.g. cyclopentenyl, cyclohexenyl, and cycloheptenyl), bicyclo[3.1.0]hexanyl, bicyclo[3.1.0]hexenyl, bicyclo[3.1.1]heptanyl, bicyclo[3.1.1]heptenyl and bicyclo[1.1.1]pentane. The cycloalkyl moiety can be attached in a "spiro-cycloalkyl" or "cycloalkyl spiro" fashion such as "spirocyclopropyl": 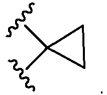 "Halo" or "Halogen" refers to fluoro, chloro, bromo and/or iodo. Where a residue is substituted with more than one halogen, it can be referred to by using a prefix corresponding to the number of halogen moieties attached, e.g., dihaloaryl, dihaloalkyl, trihaloaryl etc. refer to aryl and alkyl substituted with two ("di") or three ("tri") halo groups, which can be but are not necessarily the same halo; thus 4-chloro-3-fluorophenyl is within the scope of dihaloaryl. An alkyl group in which one or more hydrogen is replaced with a halo group is referred to as a "haloalkyl", for example, "C₁₋₆ haloalkyl." A preferred haloalkyl group is trifluoroalkyl (-CF₃). Similarly, "haloalkoxy" refers to an alkoxy group in which at least one halogen takes the place of each H in the hydrocarbon making up the alkyl moiety of the alkoxy group. An example of a haloalkoxy group is difluoromethoxy (-OCHF₂), trifluoromethoxy (-OCF₃).

The term "heteroaryl" refers to an aromatic heterocyclic mono-, bi- or tricyclic ring system of 5 to 14 ring atoms, preferably from 5 to 10 ring atoms, more preferably from 5 to 6 ring atoms, comprising 1, 2, 3 or 4 heteroatoms selected from N, O and S, the remaining ring atoms being carbon. In some aspects, monocyclic heteroaryl rings may be 5-6 membered. Bicyclic heteroaryl ring systems include fused bicyclics having two fused five-membered heteroaryl rings (denoted as 5-5), having a five-membered heteroaryl ring and a fused six-membered heteroaryl ring (denoted as 5-6 and 6-5), and having two fused six-membered heteroaryl rings (denoted as 6-6). The heteroaryl group can be optionally substituted as defined herein. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, isoxazolyl, benzofuranyl, isothiazolyl, benzothienyl, benzothiophenyl, indolyl, aza-indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, quinoxalinyl, pyrrolopyridinyl, furopyridinyl, thienopyridinyl, pyrrolopyridazinyl, pyrrolopyrimidinyl, pyrrolopyrazinyl, thienopyridazinyl, thienopyrimidinyl, thienopyrazinyl, furopyridazinyl, furopyrimidinyl, and furopyrazinyl. Most preferably, "5-membered heteroaryl" refers to the following groups: 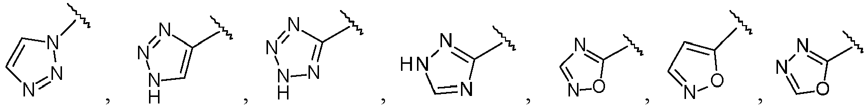 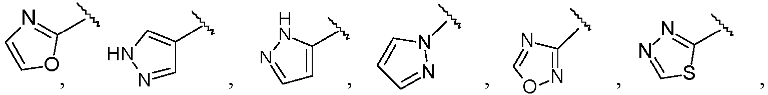 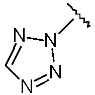 The terms "heterocycle" or "heterocyclyl" refer to a 3, 4, 5, 6, 7, 8, 9, 10-membered monocyclic, 7, 8, 9 and 10-membered bicyclic (including bridged bicyclic and cycloalkyl spiro moieties) or 10, 11, 12, 13, 14 and 15-membered bicyclic heterocyclic moiety that is saturated or partially unsaturated, and has one or more (e.g., 1, 2, 3 or 4 heteroatoms selected from oxygen, nitrogen and sulfur in the ring with the remaining ring atoms being carbon. In some aspects, the heterocycle is a heterocycloalkyl. In particular aspects heterocycle or heterocyclyl refers to a 4, 5, 6 or 7-membered heterocycle. When used in reference to a ring atom of a heterocycle, a nitrogen or sulfur may also be in an oxidized form, and a nitrogen may be substituted with one or more (C₁-C₆)alkyl or groups. The heterocycle can be attached to its pendant group at any heteroatom or carbon atom that results in a stable structure. Any of the heterocycle ring atoms can be optionally substituted with one or more substituents described herein. Examples of such saturated or partially unsaturated heterocycles include, without limitation, tetrahydrofuranyl, tetrahydrothienyl, pyrrolidinyl, pyrrolidonyl, piperidinyl, pyrrolinyl, tetrahydroquinolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, pyrrolidine 1-oxide, N-hydroxypiperidine, 1-methylpyrrolidine N-oxide, diazirinyl and quinuclidinyl. The term heterocycle also includes groups in which a heterocycle is fused to one or more aryl, heteroaryl, or cycloalkyl rings, such as indolinyl, 3H-indolyl, chromanyl, azabicyclo[2.2.1]heptanyl, azabicyclo[3.1.0]hexanyl, azabicyclo[3.1.1]heptanyl, octahydroindolyl, or tetrahydroquinolinyl.

The term "aryl" refers to a cyclic aromatic hydrocarbon moiety having a mono-, bi- or tricyclic aromatic ring of 5 to 14 carbon ring atoms ("C₅₋₁₄-aryl"). Bicyclic aryl ring systems include fused bicyclics having two fused five-membered aryl rings (denoted as 5-5), having a five-membered aryl ring and a fused six-membered aryl ring (denoted as 5-6 and as 6-5), and having two fused six-membered aryl rings (denoted as 6-6). The aryl group can be optionally substituted as defined herein. Examples of aryl moieties include, but are not limited to, phenyl, naphthyl, phenanthryl, fluorenyl, indenyl, pentalenyl, azulenyl, and the like. The term "aryl" also includes partially hydrogenated derivatives of the cyclic aromatic hydrocarbon moiety provided that at least one ring of the cyclic aromatic hydrocarbon moiety is aromatic, each being optionally substituted.

The term "haloaryl" refers to an aryl wherein at least one hydrogen has been substituted with an halogen.

The term "hydroxy", alone or in combination with other groups, refers to OH.

The term "hydroxyalkyl" refers to an alkyl group wherein one or more of the hydrogen atoms of the alkyl group have been replaced by a hydroxy moiety. Examples include alcohols and diols.

The term "oxo", alone or in combination with other groups, refers to =O.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein.

Particularly preferred pharmaceutically acceptable salts of compounds of formula (I) are the salts of hydrochloric acid, hydrobromic acid, sulfuric acid, phosphoric acid and methanesulfonic acid. The term "protecting group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

The terms "moiety" and "substituent" refer to an atom or group of chemically bonded atoms that is attached to another atom or molecule by one or more chemical bonds thereby forming part of a molecule. When indicating the number of substituents, the term "one or more" refers to the range from one substituent to the highest possible number of substitution, i.e. replacement of one hydrogen up to replacement of all hydrogens by substituents, in particular wherein "one or more" refers to one, two or three, most particularly "one or more" refers to one or two.

The term "optionally substituted" means unsubstituted or substituted. Generally these substituents can be the same or different.

"Optional" or "optionally" means that the subsequently described event or circumstance may but need not occur, and that the description includes instances where the event or circumstance occurs and instances in which it does not. For example, "aryl group optionally substituted with an alkyl group" means that the alkyl may but need not be present, and the description includes situations where the aryl group is substituted with an alkyl group and situations where the aryl group is not substituted with the alkyl group.

The term "substituted" refers to the replacement of at least one of hydrogen atoms of a compound or moiety with another substituent or moiety. Examples of such substituents include, without limitation, halogen, -OH, -CN, oxo, alkoxy, alkyl, alkylene, aryl, heteroaryl, haloalkyl, haloalkoxy, cycloalkyl and heterocycle. For example, the term "haloalkyl" refers to the fact that one or more hydrogen atoms of an alkyl (as defined below) is replaced by one or more halogen atoms (e.g., trifluoromethyl, difluoromethyl, fluoromethyl, chloromethyl, etc.). In one aspect, substituted as used herein can refer to replacement of at least one hydrogen atom of a compound or moiety described herein with halogen or alkyl.

The term "therapeutically inert carrier" denotes any ingredient having no therapeutic activity and being non-toxic such as disintegrators, binders, fillers, solvents, buffers, tonicity agents, stabilizers, antioxidants, surfactants or lubricants used in formulating pharmaceutical products.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

Whenever a chiral carbon is present in a chemical structure, it is intended that all stereoisomers associated with that chiral carbon are encompassed by the structure as pure stereoisomers as well as mixtures thereof.

All separate embodiments may be combined.

The term "ECₓ" is the half maximal effective concentration and denotes the plasma concentration of a particular compound required for obtaining x% of the maximum of a particular effect in vivo. Examples of "ECₓ" are EC₂₀, EC₅₀ and EC₁₀₀ denoting the plasma concentration of a particular compound required for obtaining 20%, 50% and 100%, respectively, of the maximum of a particular effect in vivo.

The term "prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "cancer" refers to a disease characterized by the presence of a neoplasm or tumor resulting from abnormal uncontrolled growth of cells (such cells being "cancer cells"). As used herein, the term cancer explicitly includes, but is not limited to, hepatocellular cancer, malignancies and hyperproliferative disorders of the colon (colon cancer), lung cancer, breast cancer, prostate cancer, melanoma, and ovarian cancer.

The following abbreviations are used in the present text:
BOP = benzotriazol-1-yloxytris(dimethylamino)phosphonium hexafluorophosphate, Brine = saturated aqueous NaCl solution, CAS = chemical abstracts registration number, CDI = 1,1'-Carbonyldiimidazole, DBU = 1,8-diazabicyclo[5,4,0]undec-7-ene, DCM = dichloromethane, DDQ = 2,3-dichloro-5,6-dicyano-1,4-benzoquinone, DMF = N,N-dimethylformamide, DIPEA = N,N-diisopropylethylamine, EDC = 1-ethyl-3-(3-dimethylaminopropyl)carbodiimide, ESI = electrospray ionization, EtOAc = ethyl acetate, EtOH = ethanol, h = hour(s), HATU = 1-[bis(dimethylamino)methylene]-1H-1,2,3-triazolo[4,5-b]pyridinium-3-oxide hexafluorophosphate, HBTU = O-benzotriazole-N,N,N',N'-tetramethyl-uronium-hexafluorophosphate, HFIP = hexafluoroisopropanol, HOBt = hydroxybenzotriazole, HPLC = high performance liquid chromatography, m-CPBA = meta-chloroperoxybenzoic acid, MeCN = acetonitrile, MeI = methyliodide, MeOH = methanol, min = minute(s), MS = mass spectrum, NBS = N-bromosuccinimide, PE = petroleum ether, PyBroP = bromo-tris-pyrrolidino-phosphonium hexafluorophosphate, RT = room temperature, TBAF = tetrabutylammonium fluoride, TBAOH = tetrabutylammonium hydroxide, TBDMS = tert-butyldimethylsilyl, TEA = triethylamine, TFA = trifluoroacetic acid, THF = tetrahydrofuran, TMSOTF = trifluoromethanesulfonic acid trimethylsilylester, TLC = thin layer chromatography

### Compounds of the invention

In a particular embodiment, the present invention comprises a compound of formula (I) 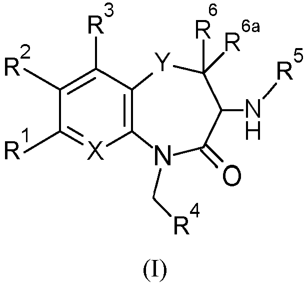 or a pharmaceutically acceptable salt thereof, wherein:
X is C(R⁷) or N;
Y is S, S(O), S(O)₂, S(O)N(R^{y}),
R¹ is 5-membered heteroaryl, wherein R¹ is optionally substituted with one or more R¹⁰ which can be the same or different;
R², R³ and R⁷ are each independently selected from hydrogen, halogen, C₁₋₆-alkyl, C₂₋₆-alkynyl, hydroxy, cyano, halo-C₁₋₆-alkyl, N(R⁸R^{8a}), C₁₋₆-alkoxy, C₃₋₇-cycloalkyl and 3-10 membered heterocyclyl;
R⁴ is selected from C₅₋₁₄-aryl and 5-14 membered heteroaryl, wherein R⁴ is optionally substituted with one or more R¹¹ which can be the same or different;
R⁵ is selected from hydrogen, C₁₋₆-alkyl, -C(O)(R⁹), amino, amino-C₁₋₆-alkyl and C₃₋₇-cycloalkyl,
wherein said C₃₋₇-cycloalkyl is optionally substituted with one or more C₁₋₆-alkyl, amino, amino-C₁₋₆-alkyl-;
R⁶ and R^{6a} are each independently selected from hydrogen and C₁-₆-alkyl;
R⁸ and R^{8a} are each independently selected from hydrogen and C₁₋₆-alkyl;
R⁹ is selected from C₁₋₆-alkyl, hydroxy-C₁₋₆-alkyl, amino-C₁₋₆-alkyl- and -(C₁₋₆-alkyl)-N(R^{9a}R^{9b}), wherein said amino-C₁₋₆-alkyl is optionally substituted with one or more hydroxy, hydroxy-C₁₋₆-alkyl-;
R^{9a} and R^{9b} are each independently selected from hydrogen and C₁₋₆-alkyl, wherein said C₁₋₆-alkyl is optionally substituted with one or more hydroxy;
or R^{9a} and R^{9b}, taken together with the nitrogen atom to which they are attached, form a 3-10 membered heterocyclyl;
R¹⁰ is selected from:
   x) C₁₋₁₀-alkyl, optionally substituted with one or more halogen, C₂₋₆-alkynyl, halo-C₁₋₆-alkyl, amino, hydroxy, 5-6 membered heteroaryl, C₁₋₆-haloalkoxy, C₁₋₆-alkoxy, 3-10 membered cycloalkyl, C₁₋₃-alkyl, -N(R^{10a}R^{10b}), -S(O)₂(C₁₋₆-alkyl), - S(O)2(C₁₋₆-cycloalkyl), 3-10 membered heterocyclyl, phenyl, cyano, - C(O)N(R^{10c}R^{10d}), wherein 3-10 membered heterocyclyl, 3-10 membered cycloalkyl, and phenyl are optionally substituted with one or more C₁₋₁₀-alkyl, C₁₋₁₀-alkoxy, -S(O)₂(C₁₋₆-alkyl), oxo, halogen, C₂₋₆-alkynyl, 3-10 membered cycloalkyl;
   xi) C₃₋₁₀-cycloalkyl, optionally substituted with one or more halogen, C₁₋₁₀-alkyl, - S(O)₂(C₁₋₆-alkyl), cyano, C₁₋₆ haloalkyl, C₁₋₆-alkoxy, hydroxy, oxo, amino, - C(O)N(R^{10c}R^{10d}), =N(OH), hydroxy-C₁₋₆-alkyl;
   xii)3-10 membered heterocyclyl, optionally substituted with one or more halogen, C₁₋₁₀-alkyl, -S(O)₂(C₁₋₆-alkyl), -C₁₋₁₀-alkyl-C₁₋₄-alkoxy, amino, -C(O)N(R^{10h}R¹⁰ⁱ), C₁₋₆-haloalkyl, C₁₋₆-alkoxy, cyano, hydroxy-C₁₋₁₀-alkyl, oxo, -C(O)(C₁₋₆-alkyl), - C(O)O-(R^{10q}), C₃₋₁₀-cycloalkyl;
   xiii) phenyl, optionally substituted with one or more halogen, C₁₋₁₀-alkyl, - S(O)₂(C₁₋₆-alkyl);
   xiv) -N(R^{10e}R^{10f});
   xv) -OR^{10g};
   xvi) -C(O)NR^{10h}R¹⁰ⁱ;
   xvii) heteroaryl, optionally substituted with one or more C₁₋₁₀-alkyl, halogen, - SO₂(C₁₋₆-alkyl); and
   xviii) oxo;
R^{10a} and R^{10b} are each independently selected from hydrogen, C₁₋₆-alkyl, halo-C₁₋₆-alkyl, hydroxy-C₁₋₆-alkyl, C₁₋₆-alkoxy,-C(O)(R^{10j}), amino-C₁₋₆-alkyl-, 3-10 membered heterocyclyl, - SO₂(R^{10k}), -C₁₋₆-alkyl-SO₂(R^{10k}) and -N(R^{10l}R^{10m}), wherein 3-10 membered heterocyclyl is optionally substituted with C₁₋₆-alkyl;
or R^{10a} and R^{10b}, taken together with the nitrogen atom to which they are attached, form a 3-10 membered heterocyclyl which is optionally substituted with one or more halogen, C₁₋₆-alkyl;
R^{10c} and R^{10d} are each independently selected from hydrogen and C₁₋₆-alkyl;
R^{10e} and R^{10f} are each independently selected from:
   vii)hydrogen;
   viii) C₁₋₆-alkyl, optionally substituted with one or more cyano, in particular one cyano, halogen, hydroxy;
   ix) -C(O)R¹⁰ⁿ;
   x) -C₁₋₁₀alkyl((O-C₁₋₁₀alkyl)ₘ), wherein m is an integer between 1 to 5 (more particularly m is 2 or 3), optionally substituted with one or more C₂₋₆-alkynyl;
   xi) C₃₋₁₀-cycloalkyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, -C(O)O( R^{10o}), - C(O)(R^{10o}), -C(O)N(R^{10p})(R^{10p'}), -SO₂(C₁₋₆-alkyl), C2-6-alkynyl; and
   xii)3-10 membered heterocyclyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, - C(O)O(R^{10o}), -C(O)(R^{10o}), -C(O)N(R^{10p})(R^{10p'}), -SO₂(C₁₋₆-alkyl), 3-10 membered cycloalkyl;
R^{10g} is selected from halo-C₁₋₆-alkyl, cyano, -C₁₋₁₀-alkyl-phenyl -C₁₋₆-alkyl-C₃₋₇-cycloalkyl and -C₁₋₆-alkoxy-halo-C₁₋₆-alkyl;
R^{10h} and R¹⁰ⁱ are each independently selected from hydrogen and C₁₋₆-alkyl, C₁₋₆-haloalkyl, wherein C₁₋₆-alkyl and C₁₋₆-haloalkyl are optionally substituted with one or more hydroxy,
or R^{10h} and R¹⁰ⁱ, taken together with the nitrogen atom to which they are attached, form a 3-10 membered heterocyclyl which is optionally substituted with one or more halogen, hydroxy, C₁₋₆-alkyl;
R^{10j} is selected from C₁₋₆-alkyl, halo-C₁₋₆-alkyl-, hydroxy-C₁₋₆-alkyl- and amino-C₁₋₆-alkyl-;
R^{10k} is selected from hydrogen, C₁₋₆-alkyl and halo-C₁₋₆-alkyl;
R^{10l} and R^{10m} are each independently selected from hydrogen and C₁₋₆-alkyl;
R¹⁰ⁿ is selected from C₁₋₆-alkyl, amino-C₁₋₆-alkyl-, halo-C₁₋₆-alkyl, C₃₋₇-cycloalkyl and 3-10 membered heterocyclyl, wherein said C₃₋₇-cycloalkyl and 3-10 membered heterocyclyl are optionally substituted with one or more halogen, C₁₋₆-alkyl;
R^{10o} is selected from C₁₋₆-alkyl and halo-C₁₋₆-alkyl;
R^{10p} and R^{10p'} are independently selected from hydrogen, C₁₋₆-alkyl and halo-C₁₋₆-alkyl;
R^{10q} is C₁₋₁₀-alkyl, C₁₋₆-haloalkyl, aryl, heteroaryl, wherein aryl and heteroaryl are optionally substituted with one or more halogen, C₁₋₁₀-alkyl;
R¹¹ is selected from:
   i) halogen;
   ii) hydroxy;
   iii) cyano;
   iv) C₁₋₁₀-alkyl, optionally substituted with one or more cyano, halogen, hydroxyl, C₃₋₇-cycloalkyl, amino, aryl (e.g. phenyl), -O-aryl (e.g. -O-phenyl), 5-6 membered heteroaryl, 3-7 membered heterocyclyl, wherein 3-7 membered heterocyclyl and aryl are optionally substituted with one or more C₁₋₆-alkyl, -SO₂(C₁₋₆-alkyl), hydroxy, halogen, cyano;
   v) C₁₋₆-alkoxy, optionally substituted with one or more, particularly one, 3-10 membered heterocyclyl, halogen;
   vi) C₃₋₇-cycloalkyl;
   vii)3-10 membered heterocyclyl, optionally substituted with one or more halo-C₁₋₆-alkyl, hydroxy, C₁₋₆-alkyl, C₃₋₁₀-cycloalkyl, C₁₋₆-alkoxy, oxo;
   viii) 5-6 membered heteroaryl, optionally substituted with one or more C₁₋₆-alkyl, 3-10 membered cycloalkyl, halogen, halo-C₁₋₆-alkyl, C₁₋₆-alkoxy, C₁₋₆-haloalkoxy;
   ix) phenyl, optionally substituted with one or more halogen, cyano, C₁₋₆-alkoxy, C₁₋₆-haloalkyl, C1-6-haloalkoxy, C₁₋₆-alkyl;
   x) -O(R^{11a});
   xi) -C(O)N(R^{11b}R^{11c});
   xii) -SO₂(R^{11d});
   xiii) -C(O)OR^{11e};
   xiv) -C(O)R^{11f};
   xv) oxo;
   xvi) -N(R^{11g}R^{11h}); and
   xvii) -S(R^{11k});
R^{11a} is selected from C₁₋₁₂-alkyl, halo-C₁₋₆-alkyl, amino-C₁₋₁₂-alkyl-, hydroxy-C₁₋₆-alkyl-, cyano, C₂₋₆-alkynyl, C₃₋₇-cycloalkyl, 3-10 membered heterocyclyl, 3-10 membered -(C₁₋₆-alkyl)heterocyclyl, 5-6 membered heteroaryl, phenyl, -C₁₋₆-alkyl-phenyl, -C₁₋₁₂-alkyl-C(O)N(R¹¹ⁱR^{11j}), -C₁₋₁₂-alkyl-NH-C(O)(C₁₋₆-alkyl), -C₁₋₁₂-alkoxy-NH-C(O)(C₁₋₆-alkyl), -C₁₋₆-alkyl-NH-C(O)(C₁₋₆-alkyl), -(CH₂CH₂O)ₙ-CH₂CH₂NH₂ and -(CH₂CH₂O)ₙ-CH₂CH₂₋NH-C(O)(C₁₋₆-alkyl);
wherein said C₁₋₁₂-alkyl, C₃₋₇-cycloalkyl, 3-10 membered heterocyclyl, 5-6 membered heteroaryl, phenyl and -C₁₋₆-alkyl-phenyl are optionally substituted with one or more halogen, C₁₋₆-alkyl, halo-C₁₋₆-alkyl, C₁₋₆-alkoxyl, C₁₋₆-haloalkoxyl, cyano;
n is an integer between 1 and 6, in particular n is two or three;
R^{11b} and R^{11c} are each independently selected from hydrogen, C₁₋₆-alkyl and halo-C₁₋₆-alkyl,
or R^{11b} and R^{11c}, taken together with the nitrogen atom to which they are attached, form a 3-10 membered heterocyclyl;
R^{11d} is selected from hydrogen, C₁₋₆-alkyl, -N(R^{11l}R^{11m}), halo-C₁₋₆-alkyl and phenyl;
R^{11e} is selected from hydrogen and C₁₋₆-alkyl;
R^{11f} is selected from hydrogen, C₁₋₆-alkyl and phenyl;
R^{11g} and R^{11h} are each independently selected from hydrogen, C₁₋₆-alkyl, -(C₁₋₆-alkyl)phenyl, halo-C₁₋₆-alkyl, -SO₂(C₁₋₆-alkyl), -SO₂(halo-C₁₋₆-alkyl) and -SO(C₁₋₆-alkyl)₂,
R¹¹ⁱR^{11j} are each independently selected from hydrogen, C₁₋₆-alkyl and halo-C₁₋₆-alkyl,
or R¹¹ⁱR^{11j}, taken together with the nitrogen atom to which they are attached, form a 3-10 membered heterocyclyl;
R^{11k} is selected from hydrogen, C₁₋₆-alkyl and halo-C₁₋₆-alkyl;
R^{11l} and R^{11m} are each independently selected from hydrogen and C₁₋₆-alkyl,
or R^{11l} and R^{11m}, taken together with the nitrogen atom to which they are attached, form a 3-10 membered heterocyclyl;
R^{y} is selected from hydrogen and C₁₋₆-alkyl.

In an other embodiment the present invention comprises a compound is of formula (I) 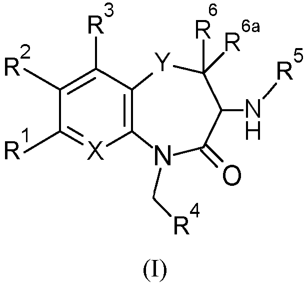 or a pharmaceutically acceptable salt thereof, wherein:
X is C(R⁷) or N;
Y is S, S(O), S(O)₂, S(O)N(R^{y}),
R¹ is 5-membered heteroaryl, wherein R¹ is optionally substituted with one or more R¹⁰ which can be the same or different;
R², R³ and R⁷ are each independently selected from hydrogen, halogen, C₁₋₆-alkyl, C₂₋₆-alkynyl, hydroxy, cyano, halo-C₁₋₆-alkyl, N(R⁸R^{8a}), C₁₋₆-alkoxy, C₃₋₇-cycloalkyl and 3-10 membered heterocyclyl;
R⁴ is selected from C₅₋₁₄-aryl and 5-14 membered heteroaryl, wherein R⁴ is optionally substituted with one or more R¹¹ which can be the same or different;
R⁵ is selected from hydrogen, C₁₋₆-alkyl, -C(O)(R⁹), amino, amino-C₁₋₆-alkyl and C₃₋₇-cycloalkyl,
wherein said C₃₋₇-cycloalkyl is optionally substituted with one or more C₁₋₆-alkyl, amino, amino-C₁₋₆-alkyl-;
R⁶ and R^{6a} are each independently selected from hydrogen and C₁₋₆-alkyl;
R⁸ and R^{8a} are each independently selected from hydrogen and C₁₋₆-alkyl;
R⁹ is selected from C₁₋₆-alkyl, hydroxy-C₁₋₆-alkyl, amino-C₁₋₆-alkyl- and -(C₁₋₆-alkyl)-N(R^{9a}R^{9b}), wherein said amino-C₁₋₆-alkyl is optionally substituted with one or more hydroxy, hydroxy-C₁₋₆-alkyl;
R^{9a} and R^{9b} are each independently selected from hydrogen and C₁₋₆-alkyl, wherein said C₁₋₆-alkyl is optionally substituted with one or more hydroxy;
or R^{9a} and R^{9b}, taken together with the nitrogen atom to which they are attached, form a 3-10 membered heterocyclyl;
R^{10j} is selected from:
   xxii) C₁₋₁₀-alkyl, optionally substituted with one or more halogen, C₂₋₆-alkynyl, halo-C₁₋₆-alkyl, amino, hydroxy, C₁₋₆-alkoxy, -N(R^{10a}R^{10b}), -S(O)₂(C₁₋₆-alkyl), - S(O)2(C1-6-cycloalkyl), 3-10 membered heterocyclyl, cyano, -C(O)N(R^{10c}R^{10d}), wherein 3-10 membered heterocyclyl is optionally substituted with one or more C₁₋₁₀-alkyl, C1-10-alkoxy , oxo, halogen;
   xxiii) C₁₋₁₀-haloalkyl, optionally substituted with one or more hydroxy, C₁₋₆-alkoxy, amino, phenyl, wherein phenyl is optionally substituted with one or more C₁₋₁₀-alkyl, halogen;
   xxiv) amino-C₁₋₁₀-alkyl- optionally substituted with one or more amino, halogen, C₁₋₆-haloalkyl, C₁₋₃-alkyl, cyano, 3-10 membered cycloalkyl, 3-10 membered heterocyclyl, C₁₋₆-haloalkoxy, C₁₋₆-alkoxy wherein 3-10 membered heterocyclyl and 3-10 membered cycloalkyl are optionally substituted with one or more halogen, C₂₋₆-alkynyl, 3-10 membered cycloalkyl;
   xxv) hydroxy-C₁₋₁₀-alkyl-;
   xxvi) C₁₋₆-alkoxy, optionally substituted with one or more cyano;
   xxvii) C₁₋₆-alkoxy-C₁₋₁₀-alkyl-;
   xxviii) C₃₋₁₀-cycloalkyl, optionally substituted with one or more halogen, C₁₋₁₀-alkyl, -S(O)₂(C₁₋₆-alkyl), cyano, C₁₋₆ haloalkyl, C₁₋₆-alkoxy, hydroxy, oxo, amino, -C(O)N(R^{10c}R^{10d}), =N(OH), hydroxy-C₁₋₆-alkyl;
   xxix) 3-10 membered heterocyclyl, optionally substituted with one or more halogen, C₁₋₁₀-alkyl, -S(O)₂(C₁₋₆-alkyl), -C₁₋₁₀-alkyl-C₁₋₄-alkoxy, amino, - C(O)N(R^{10h}R¹⁰ⁱ), C₁₋₆-haloalkyl, C₁₋₆-alkoxy, cyano, hydroxy-C₁₋₁₀-alkyl, oxo, - C(O)(C₁₋₆-alkyl), -C(O)O-(R^{10q}), C₃₋₁₀-cycloalkyl;
   xxx) -(C₁₋₆-alkyl)-C₃₋₇-cycloalkyl;
   xxxi) 3-10 membered -(C₁₋₆-alkyl)-heterocyclyl, optionally substituted with one or more halogen, C₁₋₁₀-alkyl;
   xxxii) phenyl, optionally substituted with one or more halogen, C₁₋₁₀-alkyl, - S(O)₂(C₁₋₆-alkyl);
   xxxiii) -(C₁₋₁₀-alkyl)-phenyl, optionally substituted with one or more halogen, wherein C₁₋₁₀-alkyl is optionally substituted with C₁₋₆-alkyl, cyano;
   xxxiv) 5-6 membered -(C₁₋₁₀-alkyl)-heteroaryl;
   xxxv) -(alkoxy-C₁₋₁₀-alkyl)-phenyl;
   xxxvi) -(amino-C₁-₁₀-alkyl)-phenyl;
   xxxvii) -C₁₋₆-alkyl-SO₂(C₁₋₆-alkyl);
   xxxviii) -N(R^{10e}R^{10f});
   xxxix) -OR^{10g}; and
   xl) -C(O)NR^{10h}R¹⁰ⁱ;
   xli)heteroaryl, optionally substituted with one or more C₁₋₁₀-alkyl, halogen, -SO₂(C_{1- 6}-alkyl); and
   xlii) oxo
R^{10a} and R^{10b} are each independently selected from hydrogen, C₁₋₆-alkyl, halo-C₁₋₆-alkyl, hydroxy-C₁₋₆-alkyl, C₁₋₆-alkoxy,-C(O)(R^{10j}), amino-C₁₋₆-alkyl-, 3-10 membered heterocyclyl, - SO₂(R^{10k}), -C₁₋₆-alkyl-SO₂(R^{10k}) and -N(R^{10l}R^{101m}), wherein 3-10 membered heterocyclyl is optionally substituted with C₁₋₆-alkyl;
or R^{10a} and R^{10b}, taken together with the nitrogen atom to which they are attached, form a 3-10 membered heterocyclyl which is optionally substituted with one or more halogen, C₁₋₆-alkyl;
R^{10c} and R^{10d} are each independently selected from hydrogen and C₁₋₆-alkyl;
R^{10e} and R^{10f} are each independently selected from:
   ix) hydrogen;
   x) C₁₋₆-alkyl, optionally substituted with one or more cyano, in particular one cyano;
   xi) halo-C₁₋₆-alkyl, wherein halo-C₁₋₆-alkyl is optionally substituted with one or more hydroxy;
   xii) hydroxy-C₁₋₆-alkyl;
   xiii) -C(O)R¹⁰ⁿ;
   xiv) -C₁₋₁₀alkyl((O-C₁₋₁₀alkyl)ₘ), wherein m is an integer between 1 to 5 (more particularly m is 2 or 3), optionally substituted with one or more C₂₋₆-alkynyl;
   xv) C₃₋₁₀-cycloalkyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, -C(O)O( R^{10o}), - C(O)(R^{10o}), -C(O)N(R^{10p})(R^{10p'}), -SO₂(C₁₋₆-alkyl), C2-6-alkynyl; and
   xvi) 3-10 membered heterocyclyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, -C(O)O(R^{10o}), -C(O)(R^{10o}), -C(O)N(R^{10p})(R^{10p'}), -SO₂(C₁₋₆-alkyl), 3-10 membered cycloalkyl;
or R^{10e} and R^{10f}, taken together with the nitrogen atom to which they are attached, form a 3-10 membered heterocyclyl which is optionally substituted with one or more halogen, amino, C₁₋₆-alkyl, halo-C₁₋₆-alkyl, hydroxy-C₁₋₆-alkyl, C₁₋₆-alkoxy, C₃₋₇-cycloalkyl, -C(O)O(C₁₋₆-alkyl);
R^{10g} is selected from halo-C₁₋₆-alkyl, C₁₋₆-alkyl-C₃₋₇-cycloalkyl and C₁₋₆-alkoxy-halo-C₁₋₆-alkyl;
R^{10h} and R¹⁰ⁱ are each independently selected from hydrogen and C₁₋₆-alkyl, C₁₋₆-haloalkyl, wherein C₁₋₆-alkyl and C₁₋₆-haloalkyl are optionally substituted with one or more hydroxy,
or R^{10h} and R¹⁰ⁱ, taken together with the nitrogen atom to which they are attached, form a 3-10 membered heterocyclyl which is optionally substituted with one or more halogen, hydroxy, C₁₋₆-alkyl;
R^{10j} is selected from C₁₋₆-alkyl, halo-C₁₋₆-alkyl, hydroxy-C₁₋₆-alkyl and amino-C₁₋₆-alkyl-;
R^{10k} is selected from hydrogen, C₁₋₆-alkyl and halo-C₁₋₆-alkyl;
R^{10l} and R^{10m} are each independently selected from hydrogen and C₁₋₆-alkyl;
R¹⁰ⁿ is selected from C₁₋₆-alkyl, amino-C₁₋₆-alkyl-, halo-C₁₋₆-alkyl, C₃₋₇-cycloalkyl and 3-10 membered heterocyclyl, wherein said C₃₋₇-cycloalkyl and 3-10 membered heterocyclyl are optionally substituted with one or more halogen, C₁₋₆-alkyl;
R^{10o} is selected from C₁₋₆-alkyl and halo-C₁₋₆-alkyl;
R^{10p} and R^{10p'} are independently selected from hydrogen, C₁₋₆-alkyl and halo-C₁₋₆-alkyl;
R^{10q} is C₁₋₁₀-alkyl, C₁₋₆-haloalkyl, aryl, heteroaryl, wherein aryl and heteroaryl are optionally substituted with one or more halogen, C₁₋₁₀-alkyl;
R¹¹ is selected from:
   i) halogen;
   ii) hydroxy;
   iii) cyano;
   iv) C₁₋₆-alkyl, optionally substituted with one or more cyano, aryl, haloaryl;
   v) C₁₋₆-alkoxy, optionally substituted with one or more, particularly one, 3-10 membered heterocyclyl;
   vi) halo-C₁₋₆-alkyl;
   vii) amino-C₁₋₁₀-alkyl-;
   viii) hydroxy-C₁₋₆-alkyl;
   ix) C₃₋₇-cycloalkyl;
   x) -C₁₋₆-alkyl-C₃₋₇-cycloalkyl;
   xi) 3-10 membered heterocyclyl, optionally substituted with one or more halo-C₁₋₆-alkyl, hydroxy, C₁₋₆-alkyl, C₃₋₁₀-cycloalkyl, C₁₋₆-alkoxy, oxo;
   xii)3-7 membered -(C₁₋₆-alkyl)-heterocyclyl, optionally substituted with one or more C₁₋₆-alkyl;
   xiii) 5-6 membered heteroaryl, optionally substituted with one or more C₁₋₆-alkyl, 3-10 membered cycloalkyl, halogen, halo-C₁₋₆-alkyl, C₁₋₆-alkoxy; C₁₋₆-haloalkoxy;
   xiv) phenyl, optionally substituted with one or more halogen, cyano, C₁₋₆-alkoxy, halo-C₁₋₆-alkyl, C₁₋₆-haloalkoxy, C₁₋₆-alkyl;
   xv) -(C₁₋₆-alkyl)-phenyl, optionally substituted with one or more -SO₂(C₁₋₆-alkyl), hydroxy, halogen, cyano;
   xvi) -(C₁₋₆-alkyl)-O-phenyl;
   xvii) 5-6 membered -(C₁₋₆-alkyl)-heteroaryl;
   xviii) -O(R^{11a});
   xix) -C(O)N(R^{11b}R^{11c});
   xx) -SO₂(R^{11d});
   xxi) -C(O)OR^{11e};
   xxii) -C(O)R^{11f};
   xxiii) oxo;
   xxiv) -N(R^{11g}R^{11h});
   xxv) -S(R^{11k}); and
   xxvi) C₁₋₆-haloalkoxyl;
R^{11a} is selected from C₁₋₁₂-alkyl, halo-C₁₋₆-alkyl, amino-C₁₋₁₂-alkyl-, hydroxy-C₁₋₆-alkyl, cyano, -C₁₋₆-alkyl, C₂₋₆-alkynyl, C₃₋₇-cycloalkyl, 3-10 membered heterocyclyl, 3-10 membered - (C₁₋₆-alkyl)heterocyclyl, 5-6 membered heteroaryl, phenyl, -C₁₋₆-alkyl-phenyl, -C₁₋₁₂-alkyl-C(O)N(R¹¹ⁱR^{11j}), -C₁₋₁₂-alkyl-NH-C(O)(C₁₋₆-alkyl), -C₁₋₁₂-alkoxy-NH-C(O)(C₁₋₆-alkyl), -C₁₋₆-alkyl-NH-C(O)(C₁₋₆-alkyl), -(CH₂CH₂O)ₙ-CH₂CH₂NH₂ and -(CH₂CH₂O)ₙ-CH₂CH₂₋NH-C(O)(C₁₋₆-alkyl);
wherein said C₁₋₁₂-alkyl, C₃₋₇-cycloalkyl, 3-10 membered heterocyclyl, 5-6 membered heteroaryl, phenyl and -C₁₋₆-alkyl-phenyl are optionally substituted with one or more halogen, C₁₋₆-alkyl, halo-C₁₋₆-alkyl, C₁₋₆-alkoxyl, C₁₋₆-haloalkoxyl, cyano;
n is an integer between 1 and 6;
R^{11b} and R^{11c} are each independently selected from hydrogen, C₁₋₆-alkyl and halo-C₁₋₆-alkyl,
or R^{11b} and R^{11c}, taken together with the nitrogen atom to which they are attached, form a 3-10 membered heterocyclyl;
R^{11d} is selected from hydrogen, C₁₋₆-alkyl, -N(R^{11l}R^{11m}), halo-C₁₋₆-alkyl and phenyl;
R^{11e} is selected fromhydrogen and C₁₋₆-alkyl;
R^{11f} is selected from hydrogen, C₁₋₆-alkyl and phenyl;
R^{11g} and R^{11h} are each independently selected from hydrogen, C₁₋₆-alkyl, -(C₁₋₆-alkyl)phenyl. halo-C₁₋₆-alkyl, -SO₂(C₁₋₆-alkyl), -SO₂(halo-C₁₋₆-alkyl) and -SO(C₁₋₆-alkyl)₂,
or R^{11g} and R^{11h}, taken together with the nitrogen atom to which they are attached, form a 3-10 membered heterocyclyl optionally substituted with C₁₋₆-alkyl, C₁₋₆-alkoxy;
R¹¹ⁱR^{11j} are each independently selected from hydrogen, C₁₋₆-alkyl and halo-C₁₋₆-alkyl,
or R¹¹ⁱR^{11j}, taken together with the nitrogen atom to which they are attached, form a 3-10 membered heterocyclyl;
R^{11k} is selected from hydrogen, C₁₋₆-alkyl and halo-C₁₋₆-alkyl;
R^{11l} and R^{11m} are each independently selected from hydrogen and C₁₋₆-alkyl,
or R^{11l} and R^{11m}, taken together with the nitrogen atom to which they are attached, form a 3-10 membered heterocyclyl;
R^{y} is selected from hydrogen and C₁₋₆-alkyl.

A particular embodiment of the present invention relates to a compound of formula (I) 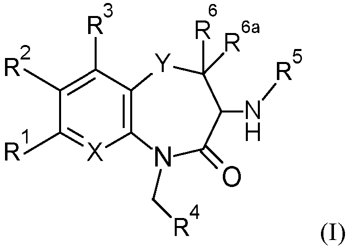 or a pharmaceutically acceptable salt thereof, wherein:
X is C(R⁷) or N;
Y is S, S(O), S(O)₂, S(O)N(R^{y}),
R¹ is 5-membered heteroaryl, wherein R¹ is optionally substituted with one or more R¹⁰ which can be the same or different;
R², R³ and R⁷ are each independently selected from hydrogen, halogen, C₁₋₆-alkyl, C₂₋₆-alkynyl, hydroxy, cyano, halo-C₁₋₆-alkyl, N(R⁸R^{8a}), C₁₋₆-alkoxy, C₃₋₇-cycloalkyl and 3-10 membered heterocyclyl;
R⁴ is selected from C₅₋₁₄-aryl and 5-14 membered heteroaryl, wherein R⁴ is optionally substituted with one or more R¹¹ which can be the same or different;
R⁵ is selected from hydrogen, C₁₋₆-alkyl, -C(O)(R⁹), amino, amino-C₁₋₆-alkyl and C₃₋₇-cycloalkyl,
wherein said C₃₋₇-cycloalkyl is optionally substituted with one or more C₁₋₆-alkyl, amino, amino-C₁₋₆-alkyl;
R⁶ and R^{6a} are each independently selected from hydrogen and C₁₋₆-alkyl;
R⁸ and R^{8a} are each independently selected from hydrogen and C₁₋₆-alkyl;
R⁹ is selected from C₁₋₆-alkyl, hydroxy-C₁₋₆-alkyl, amino-C₁₋₆-alkyl and (C₁₋₆-alkyl)-N(R^{9a}R^{9b}), wherein said amino-C₁₋₆-alkyl is optionally substituted with one or more hydroxy, hydroxy-C₁₋₆-alkyl;
R^{9a} and R^{9b} are each independently selected from hydrogen and C₁₋₆-alkyl, wherein said C₁₋₆-alkyl is optionally substituted with one or more hydroxy;
or R^{9a} and R^{9b}, taken together with the nitrogen atom to which they are attached, form a 3-10 membered heterocyclyl;
R^{10j} is selected from
   i) C₁₋₁₀-alkyl, optionally substituted with one or more halogen, C₂₋₆-alkynyl, halo-C₁₋₆-alkyl, amino, -N(R^{10a}R^{10b}), -S(O)₂(C₁₋₆-alkyl), 3-10 membered heterocyclyl, cyano, -C(O)N(R^{10c}R^{10d});
   ii) C₁₋₁₀-haloalkyl;
   iii) amino-C₁₋₁₀-alkyl;
   iv) hydroxy-C₁₋₁₀-alkyl;
   v) C₁₋₆-alkoxy;
   vi) C₁₋₆-alkoxy-C₁₋₁₀-alkyl;
   vii) C₃₋₁₀-cycloalkyl, optionally substituted with one or more halogen, C₁₋₁₀-alkyl, - S(O)₂(C₁₋₆-alkyl), hydroxy, oxo, amino, -C(O)N(R^{10c}R^{10d}), =N(OH), hydroxy-C₁₋₆-alkyl;
   viii) 3-10 membered heterocyclyl, optionally substituted with one or more halogen, C₁₋₁₀-alkyl, -S(O)₂(C₁₋₆-alkyl), cyano, hydroxy-C₁₋₁₀-alkyl, oxo, -C(O)(C₁₋₆-alkyl), - C(O)O-(C₁₋₆-alkyl), C₃₋₁₀-cycloalkyl;
   ix) (C₁₋₆-alkyl)-C₃₋₇-cycloalkyl;
   x) 3-10 membered (C₁₋₆-alkyl)-heterocyclyl, optionally substituted with one or more halogen, C₁₋₁₀-alkyl;
   xi) phenyl, optionally substituted with one or more halogen;
   xii) (C₁₋₁₀-alkyl)-phenyl, optionally substituted with one or more halogen, wherein C₁₋₁₀-alkyl is optionally substituted with C₁₋₆-alkyl, cyano;
   xiii) 5-6 membered (C₁₋₁₀-alkyl)-heteroaryl;
   xiv) (alkoxy-C₁₋₁₀-alkyl)-phenyl;
   xv) (amino-C₁₋₁₀-alkyl)-phenyl;
   xvi) -C₁₋₆-alkyl-SO₂(C₁₋₆-alkyl);
   xvii) -N(R^{10e}R^{10f});
   xviii) -OR^{10g}; and
   xix) -C(O)NR^{10h}R¹⁰ⁱ;
R^{10a} and R^{10b} are each independently selected from hydrogen, C₁₋₆-alkyl, halo-C₁₋₆-alkyl, hydroxy-C₁₋₆-alkyl, C₁₋₆-alkoxy,-C(O)(R^{10j}), amino-C₁₋₆-alkyl, 3-10 membered heterocyclyl, - SO₂(R^{10k}), C₁₋₆-alkyl-SO₂(R^{10k}) and -N(R^{10l}R^{10m}), wherein 3-10 membered heterocyclyl is optionally substituted with C₁₋₆-alkyl; or R^{10a} and R^{10b}, taken together with the nitrogen atom to which they are attached, form a 3-10 membered heterocyclyl which is optionally substituted with one or more halogen, C₁₋₆-alkyl;
R^{10c} and R^{10d} are each independently selected from hydrogen and C₁₋₆-alkyl;
R^{10e} and R^{10f} are each independently selected from hydrogen, C₁₋₆-alkyl, halo-C₁₋₆-alkyl, hydroxy-C₁₋₆-alkyl, -C(O)R¹⁰ⁿ, C₃₋₁₀-cycloalkyl and 3-10 membered heterocyclyl, wherein said C₃₋₁₀-cycloalkyl and 3-10 membered heterocyclyl are optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, - C(O)O( R^{10o}), -C(O)(R^{10o}), -C(O)N(R^{10p})(R^{10p'}), -SO₂(C₁₋₆-alkyl); or R^{10e} and R^{10f}, taken together with the nitrogen atom to which they are attached, form a 3-10 membered heterocyclyl which is optionally substituted with one or more halogen, amino, C₁₋₆-alkyl, halo-C₁₋₆-alkyl, hydroxy-C₁₋₆-alkyl, C₁₋₆-alkoxy, C₃₋₇-cycloalkyl, -C(O)O(C₁₋₆-alkyl);
R^{10g} is selected from halo-C₁₋₆-alkyl, C₁₋₆-alkyl-C₃₋₇-cycloalkyl and C₁₋₆-alkoxy-halo-C₁₋₆-alkyl;
R^{10h} and R¹⁰ⁱ are each independently selected from hydrogen and C₁₋₆-alkyl, or R^{10h} and R¹⁰ⁱ, taken together with the nitrogen atom to which they are attached, form a 3-10 membered heterocyclyl which is optionally substituted with one or more halogen, hydroxy, C₁₋₆-alkyl;
R^{10j} is selected from C₁₋₆-alkyl, halo-C₁₋₆-alkyl, hydroxy-C₁₋₆-alkyl and amino-C₁₋₆-alkyl;
R^{10k} is selected from hydrogen, C₁₋₆-alkyl and halo-C₁₋₆-alkyl;
R^{10l} and R^{10m} are each independently selected from hydrogen and C₁₋₆-alkyl;
R¹⁰ⁿ is selected from C₁₋₆-alkyl, amino-C₁₋₆-alkyl, halo-C₁₋₆-alkyl, C₃₋₇-cycloalkyl and 3-10 membered heterocyclyl, wherein said C₃₋₇-cycloalkyl and 3-10 membered heterocyclyl are optionally substituted with one or more halogen, C₁₋₆-alkyl;
R^{10o} is selected from C₁₋₆-alkyl and halo-C₁₋₆-alkyl;
R^{10p} and R^{10p'} are independently selected from hydrogen, C₁₋₆-alkyl and halo-C₁₋₆-alkyl;
R¹¹ is selected from
   i) halogen;
   ii) hydroxy;
   iii) cyano;
   iv) C₁₋₆-alkyl, optionally substituted with one or more cyano;
   v) C₁₋₆-alkoxy;
   vi) halo-C₁₋₆-alkyl;
   vii) amino-C₁₋₁₀-alkyl;
   viii) hydroxy-C₁₋₆-alkyl;
   ix) C₃₋₇-cycloalkyl;
   x) C₁₋₆-alkyl-C₃₋₇-cycloalkyl;
   xi) 3-10 membered heterocyclyl, optionally substituted with one or more halo-C₁₋₆-alkyl, hydroxy, C₁₋₆-alkyl, C₁₋₆-alkoxy, oxo;
   xii) 3-7 membered (C₁₋₆-alkyl)-heterocyclyl, optionally substituted with one or more C₁₋₆-alkyl;
   xiii) 5-6 membered heteroaryl, optionally substituted with one or more C₁₋₆-alkyl, halogen, halo-C₁₋₆-alkyl;
   xiv) phenyl, optionally substituted with one or more halogen, cyano;
   xv) (C₁₋₆-alkyl)-phenyl, optionally substituted with one or more -SO₂(C₁₋₆-alkyl), hydroxy, halogen, cyano;
   xvi) (C₁₋₆-alkyl)-O-phenyl;
   xvii) 5-6 membered (C₁₋₆-alkyl)-heteroaryl;
   xviii) -O(R^{11a});
   xix) -C(O)N(R^{11b}R^{11c});
   xx) -SO₂(R^{11d});
   xxi) -C(O)OR^{11e};
   xxii) -C(O)R^{11f};
   xxiii) oxo;
   xxiv) -N(R^{11g}R^{11h}); and
   xxv) -S(R^{11k});
R^{11a} is selected from C₁₋₁₂-alkyl, halo-C₁₋₆-alkyl, amino-C₁₋₁₂-alkyl, hydroxy-C₁₋₆-alkyl, cyano, - C₁₋₆-alkyl, C₂₋₆-alkynyl, C₃₋₇-cycloalkyl, 3-10 membered heterocyclyl, 5-6 membered heteroaryl, phenyl, -C₁₋₆-alkyl-phenyl, C₁₋₁₂-alkyl-C(O)N(R¹¹ⁱR^{11j}), -C₁₋₁₂-alkyl-NH-C(O)(C₁₋₆-alkyl), -C₁₋₁₂-alkoxy-NH-C(O)(C₁₋₆-alkyl), -C₁₋₆-alkyl-NH-C(O)(C₁₋₆-alkyl), -(CH₂CH₂O)ₙ-CH₂CH₂NH₂ and - (CH₂CH₂O)ₙ-CH₂CH₂₋NH-C(O)(C₁₋₆-alkyl);
wherein said C₁₋₁₂-alkyl, C₃₋₇-cycloalkyl, 3-10 membered heterocyclyl, 5-6 membered heteroaryl, phenyl and C₁₋₆-alkyl-phenyl are optionally substituted with one or more halogen, C₁₋₆-alkyl, halo-C₁₋₆-alkyl, cyano;
n is an integer between 1 and 6;
R^{11b} and R^{11c} are each independently selected from hydrogen, C₁₋₆-alkyl and halo-C₁₋₆-alkyl, or R^{11b} and R^{11c}, taken together with the nitrogen atom to which they are attached, form a 3-10 membered heterocyclyl;
R^{11d} is selected from hydrogen, C₁₋₆-alkyl, -N(R^{11l}R^{11m}), halo-C₁₋₆-alkyl and phenyl;
R^{11e} is selected from hydrogen and C₁₋₆-alkyl;
R^{11f} is selected from hydrogen, C₁₋₆-alkyl and phenyl;
R^{11g} and R^{11h} are each independently selected from hydrogen, C₁₋₆-alkyl, halo-C₁₋₆-alkyl, SO₂(C₁₋₆-alkyl), SO₂(halo-C₁₋₆-alkyl) and SO(C₁₋₆-alkyl)₂,
or R^{11g} and R^{11h}, taken together with the nitrogen atom to which they are attached, form a 3-10 membered heterocyclyl optionally substituted with C₁₋₆-alkyl, C₁₋₆-alkoxy;
R¹¹ⁱR^{11j} are each independently selected from hydrogen, C₁₋₆-alkyl and halo-C₁₋₆-alkyl, or R¹¹ⁱR^{11j}, taken together with the nitrogen atom to which they are attached, form a 3-10 membered heterocyclyl;
R^{11k} is selected from hydrogen, C₁₋₆-alkyl and halo-C₁₋₆-alkyl;
R^{11l} and R^{11m} are each independently selected from hydrogen and C₁₋₆-alkyl, or R^{11l} and R^{11m}, taken together with the nitrogen atom to which they are attached, form a 3-10 membered heterocyclyl;
R^{y} is selected from hydrogen and C₁₋₆-alkyl.

In one embodiment, there is provided a compound as described herein, or a pharmaceutically acceptable salt thereof, wherein the compound is a compound of formula (IA) 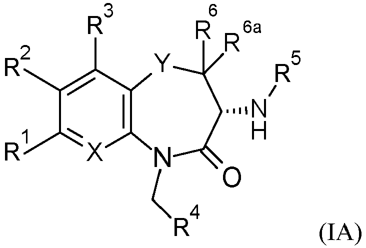 wherein X, Y, R¹, R², R³, R⁴, R⁵, R⁶, R^{6a} are as defined herein.

Further, it is to be understood that every embodiment relating to a specific X, Y, R¹, R², R³, R⁴, R⁵, R⁶, R^{6a} R⁷, R⁸, R^{8a}, R⁹, R^{9a}, R^{9b}, R¹⁰, R^{10a}, R^{10b}, R^{10c}, R^{10d}, R^{10e}, R^{10f}, R^{10g}, R^{10h}, R¹⁰ⁱ, R^{10j}, R^{10k}, R^{10l}, R^{10m}, R¹⁰ⁿ, R^{10o}, R^{10p}, R^{10p'}, R^{10q}, R¹¹, R^{11a}, R^{11b}, R^{11c}, R^{11d}, R^{11e}, R^{11f}, R^{11g}, R^{11h}, R¹¹ⁱ, R^{11j}, R^{11k}, R^{11l}, R^{11m}, and R^{y} as disclosed herein may be combined with any other embodiment relating to another X, Y, R¹, R², R³, R⁴, R⁵, R⁶, R^{6a}, R⁷, R⁸, R^{8a}, R⁹, R^{9a}, R^{9b}, R¹⁰, R^{10a}, R^{10b}, R^{10c}, R^{10d}, R^{10e}, R^{10f}, R^{10g}, R^{10h}, R¹⁰ⁱ, R^{10j}, R^{10k}, R^{10l}, R^{10m}, R¹⁰ⁿ, R^{10o}, R^{10p}, R^{10p'}, R^{10q}, R¹¹, R^{11a}, R^{11b}, R^{11c}, R^{11d}, R^{11e}, R^{11f}, R^{11g}, R^{11h}, R¹¹ⁱ, R^{11j}, R^{11k}, R^{11l}, R^{11m}, and R^{y} as disclosed herein.

In one embodiment, there is provided a compound of formula (I) as described herein, or a pharmaceutically acceptable salt thereof, wherein X is N or C(R⁷), wherein R⁷ is hydrogen or halogen.

In a particular embodiment, there is provided a compound of formula (I) as described herein, or a pharmaceutically acceptable salt thereof, wherein X is CH.

In one embodiment, there is provided a compound of formula (I) as described herein, or a pharmaceutically acceptable salt thereof, wherein Y is SO or S(O)₂.

In one embodiment, there is provided a compound of formula (I) as described herein, or a pharmaceutically acceptable salt thereof, wherein R¹ is substituted with at least one R¹⁰, more particularly with one R¹⁰.

In one embodiment, there is provided a compound of formula (I) as described herein,, or a pharmaceutically acceptable salt thereof, wherein R¹ is selected from 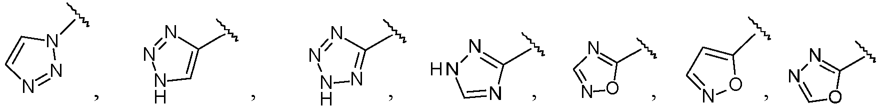 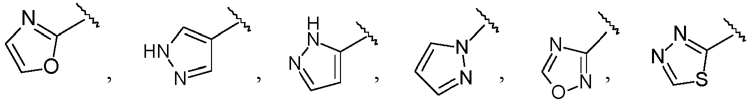 and

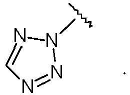

In one embodiment, there is provided a compound of formula (I) as described herein,, or a pharmaceutically acceptable salt thereof, wherein R¹ is selected from 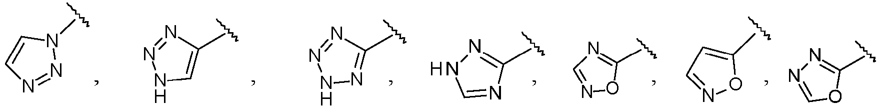 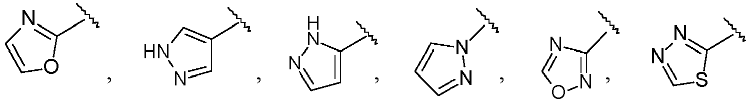 and 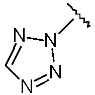 wherein R¹ is optionally substituted with one or more R¹⁰ which can be the same or different as defined herein, more particularly wherein R¹ is optionally substituted with one R¹⁰ . In a particular embodiment, there is provided a compound of formula (I) as described herein, or a pharmaceutically acceptable salt thereof, wherein R¹ is selected from

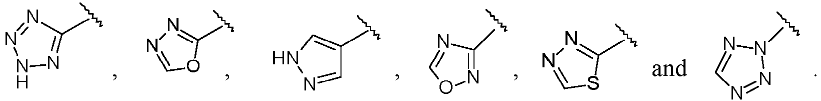

In one embodiment, there is provided a compound of formula (I) as described herein, or a pharmaceutically acceptable salt thereof, wherein R² is hydrogen, halogen, C₁₋₆-alkyl, hydroxy or N(R⁸R^{8a}).

In a more particular embodiment, there is provided a compound of formula (I) as described herein, or a pharmaceutically acceptable salt thereof, wherein wherein R¹ is selected from 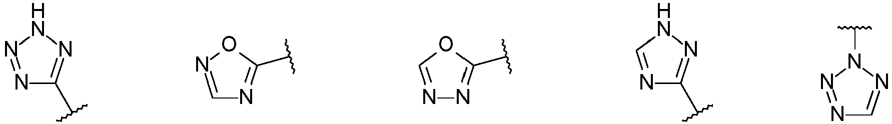 wherein R¹ is optionally substituted with one or more R¹⁰ which can be the same or different as defined herein, more particularly wherein R¹ is optionally substituted with one R¹⁰.

In a particular embodiment, there is provided a compound of formula (I) as described herein, or a pharmaceutically acceptable salt thereof, wherein R⁸ and R^{8a} are each independently selected from hydrogen and C₁₋₆-alkyl.

In another particularembodiment, there is provided a compound of formula (I) as described herein, or a pharmaceutically acceptable salt thereof, wherein R² is hydrogen, halogen or C₁₋₆-alkyl.

In a particular embodiment, there is provided a compound of formula (I) as described herein, or a pharmaceutically acceptable salt thereof, wherein R² is hydrogen, fluorine or methyl.

In a more particularly embodiment, there is provided a compound of formula (I) as described herein, or a pharmaceutically acceptable salt thereof, wherein R² is hydrogen, fluorine.

In one embodiment, there is provided a compound of formula (I) as described herein, or a pharmaceutically acceptable salt thereof, wherein R³ is hydrogen.

In one embodiment, there is provided a compound of formula (I) as described herein, or a pharmaceutically acceptable salt thereof, wherein R⁴ is selected from

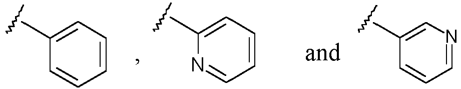

In one embodiment, there is provided a compound of formula (I) as described herein, or a pharmaceutically acceptable salt thereof, wherein R⁴ is selected from 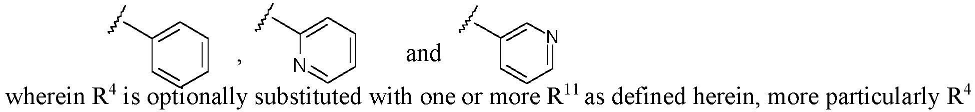 is optionally substituted with one R¹¹ .

In anotherembodiment, there is provided a compound of formula (I) as described herein, or a pharmaceutically acceptable salt thereof, wherein R⁴ is

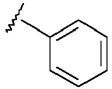

In yet anotherembodiment, there is provided a compound of formula (I) as described herein, or a pharmaceutically acceptable salt thereof, wherein R⁴ is 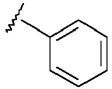 wherein R⁴ is optionally substituted with one or more R¹¹ as defined herein, more particularly R⁴ is optionally substituted with one R¹¹ .

In one embodiment, there is provided a compound of formula (I) as described herein, or a pharmaceutically acceptable salt thereof, wherein R⁵ is hydrogen or -C(O)(R⁹).

In a particular embodiment, there is provided a compound of formula (I) as described herein, or a pharmaceutically acceptable salt thereof, wherein R⁵ is hydrogen or -C(O)(amino-C₁₋₆-alkyl).

In a more particular embodiment, there is provided a compound of formula (I) as described herein, or a pharmaceutically acceptable salt thereof, wherein R⁵ is hydrogen or -C(O)(CH₂NH₂).

In one embodiment, there is provided a compound of formula (I) as described herein, or a pharmaceutically acceptable salt thereof, wherein R⁶ and R^{6a} are hydrogen.

In a particular embodiment, there is provided a compound of formula (I) as described herein, or a pharmaceutically acceptable salt thereof, wherein R¹⁰ is selected from:
i) C₁₋₁₀-alkyl, optionally substituted with one or more C₂₋₆-alkynyl, cyano;
ii) C₁₋₁₀-haloalkyl, optionally substituted with C₁₋₅-alkoxy, amino, phenyl, wherein phenyl is optionally substituted with one or more halogen;
iii) C₃₋₁₀-cycloalkyl, optionally substituted with one or more halogen;
iv) phenyl, optionally substituted with one or more halogen, C₁₋₁₀-alkyl;
v) heteroaryl, optionally substituted with one or more halogen, C₁₋₁₀-alkyl;
vi) 3-10 membered heterocyclyl, optionally substituted with one or more C₁₋₁₀-alkyl, C₃₋₁₀-cycloalkyl, C₁₋₆-haloalkyl, halogen, -C₁₋₁₀-alkyl-C₁₋₄-alkoxy, -C(O)O-C₁₋₅-alkyl;
vii)amino, optionally substituted with C₁₋₁₀-alkyl, C₁₋₁₀-haloalkyl, C₃₋₁₀-cycloalkyl, wherein C₃₋₁₀-cycloalkyl is optionally substituted with one or more C₁₋₅-alkyl, halogen.

In one embodiment, there is provided a compound of formula (I) as described herein, or a pharmaceutically acceptable salt thereof, wherein R¹⁰ is selected from C₁₋₁₀-alkyl, C₁₋₁₀-alkyl substituted with C₂₋₆-alkynyl, C₁₋₁₀-haloalkyl, amino-C₁₋₁₀-alkyl, hydroxy-C₁₋₁₀-alkyl, C₃₋₁₀-cycloalkyl, C₃₋₁₀-cycloalkyl substituted with one or more halogen, phenyl substituted with one or more halogen, (C₁₋₁₀-alkyl)-phenyl substituted with one or more halogen, (alkoxy-C₁₋₁₀-alkyl)-phenyl, 3-10 membered heterocyclyl, 3-10 membered heterocyclyl substituted with one or more C₁₋₁₀-alkyl and halogen, and -N(R^{10e}R^{10f}).

In a particular embodiment, there is provided a compound of formula (I) as described herein, or a pharmaceutically acceptable salt thereof, wherein R¹⁰ is selected from C₁₋₁₀-alkyl, C₁₋₁₀-alkyl substituted with C₂₋₆-alkynyl, C₁₋₁₀-haloalkyl, amino-C₁₋₁₀-alkyl-, hydroxy-C₁₋₁₀-alkyl, C₃₋₁₀-cycloalkyl, C₃₋₁₀-cycloalkyl substituted with one or more halogen, phenyl substituted with one or more halogen, -(C₁₋₁₀-alkyl)-phenyl substituted with one or more halogen, -(alkoxy-C₁₋₁₀-alkyl)-phenyl, 3-10 membered heterocyclyl, 3-10 membered heterocyclyl substituted with one or more C₁₋₁₀-alkyl and halogen, heteroaryl, heteroaryl substituted with one or more C₁₋₁₀-alkyl, heteroaryl substituted with one or more halogen, and -N(R^{10e}R^{10f}).

In one embodiment, there is provided a compound of formula (I) as described herein, or a pharmaceutically acceptable salt thereof, wherein R¹⁰ is selected from C₁₋₁₀-alkyl, C₁₋₁₀-haloalkyl, C₃₋₁₀-cycloalkyl substituted with one or more halogen, hydroxy-C₁₋₁₀-alkyl, C₁₋₁₀-alkyl substituted with C₂₋₆-alkynyl, 3-10 membered heterocyclyl, 3-10 membered heterocyclyl substituted with one or more C₁₋₁₀-alkyl and halogen, and -NH(C₃₋₇-cycloalkyl).

In a more particular embodiment, there is provided a compound of formula (I) as described herein, or a pharmaceutically acceptable salt thereof, wherein R¹⁰ is selected from tert-butyl, methyl 3-azabicyclo[3.1.1]heptane-3-carboxylate, 3-oxa-8-azabicyclo[3.2.1]octan-8-yl, 3,3-(difluorocyclobutyl)aminoyl, (trifluoromethyl)cyclopropyl, 2,2-difluoromorpholin-4-yl, 5,5-difluoro-1-methyl-3-piperidyl, 2,2,2-trifluoroethyl, (3,3-difluoro-1-methyl-cyclobutyl)aminoyl, o-tolyl, cyclobutylaminoyl, 2-methyl-propanenitrile, 6-fluoro-2-methyl-3-pyridyl, 1,2,2,2-tetrafluoro-1-methoxy-ethyl, 4-oxa-7-azaspiro[2.5]octan-7-yl, ethyl, 1-amino-2,2,2-trifluoro-1-methyl-ethyl, morpholinyl, 1-amino-2,2,2-trifluoro-1-(4-fluorophenyl)ethyl, methyl(2,2,2-trifluoroethyl)amino, 2,2-difluoromorpholin-4-yl, tert-butylaminoyl, cyclohexylaminoyl, methyl 4-methylpiperidine-1-carboxylate, 2-oxa-5-azabicyclo[4.1.0]heptan-5-yl, 2-(trifluoromethyl)morpholin-4-yl, 3,3-difluorocyclohexyl, 1,1-dimethylbut-3-ynyl, 3,3-difluorocyclobutyl, isobutyl, methyl 3,3-difluoropiperidine-1-carboxylate, difluorocyclopentyl, trifluoromethylmorpholinyl, 2,2-difluorospiro[3.3]heptan-6-yl, difluorocyclohexyl, 5,5-difluoro-1-(2-methoxyethyl)-3-piperidyl, 1-ethyl-5,5-difluoro-3-piperidyl, 2-cyclopropyltetrahydrofuran-2-yl, 3-fluoro-1-methyl-3-piperidyl, 6-fluoro-2-methyl-3-pyridyl, 2-fluoro-6-methyl-phenyl, fluorophenyl, 1,2,2,2-tetrafluoro-1-methoxy-ethyl. In a particular embodiment, there is provided a compound of formula (I) as described herein, or a pharmaceutically acceptable salt thereof, wherein R¹⁰ is selected from ethyl, tertbutyl, isopropyl, -CH₂CF₃, -C((CH₃)₂)F, -C((CH₃)₂)CH₂OH, -C((CH₃)₂)CH₂CCH₃, difluorocyclohexyl, difluorocyclobutyl, piperidyl substituted with fluorine and methyl, morpholinyl, and - NH(cyclopentyl).

In one embodiment, there is provided a compound of formula (I) as described herein, or a pharmaceutically acceptable salt thereof, wherein R¹¹ is selected from halogen, C₁₋₆-haloalkoxy, C₁₋₆-alkoxy, -O-R^{11a}, C₁₋₆-alkyl, C₁₋₆-alkyl substituted with cyano.

In one embodiment, there is provided a compound of formula (I) as described herein, or a pharmaceutically acceptable salt thereof, wherein R¹¹ is selected from halogen, C₁₋₆-haloalkoxy, C₁₋₆-alkoxy, -O-arly, -O-C₃₋₁₀-cycloalkyl, C₁₋₆-alkyl, C₁₋₆-alkyl substituted with cyano.

In one embodiment, there is provided a compound of formula (I) as described herein, or a pharmaceutically acceptable salt thereof, wherein R¹¹ is selected from halogen, -O(R^{11a}), cyano, amino-C₁₋₁₀-alkyl, -SO₂(C₁₋₆-alkyl) and 3-10 membered heterocyclyl substituted with one or more halo-C₁₋₆-alkyl.

In one embodiment, there is provided a compound of formula (I) as described herein, or a pharmaceutically acceptable salt thereof, wherein R¹¹ is selected from chloro, fluoro, trifluoromethoxy, 1,1,2,2-tetrafluoroethoxy, phenoxy, 2-methyl-propanenitrile, isopropoxy, methoxy, cyclopentoxy.

In one embodiment, there is provided a compound of formula (I) as described herein, or a pharmaceutically acceptable salt thereof, wherein R¹¹ is selected from halogen, -O(R^{11a}), cyano, amino-C₁₋₁₀-alkyl, -SO₂(C₁₋₆-alkyl) and 3-10 membered heterocyclyl substituted with one or more halo-C₁₋₆-alkyl.

In a particular embodiment, there is provided a compound of formula (I) as described herein, or a pharmaceutically acceptable salt thereof, wherein R^{11a} is selected from C₁₋₁₂-alkyl, halo-C₁₋₆-alkyl, 3-7 membered heterocycloalkyl and amino-C₁₋₁₂-alkyl.

In a more particular embodiment, there is provided a compound of formula (I) as described herein, or a pharmaceutically acceptable salt thereof, wherein R¹¹ is selected from halogen, -O(halo-C₁₋₆-alkyl), -O(C₁₋₆-alkyl), cyano and 3-10 membered heterocyclyl substituted with one or more halo-C₁₋₆-alkyl.

In an even more particular embodiment, there is provided a compound of formula (I) as described herein, or a pharmaceutically acceptable salt thereof, wherein R¹¹ is selected from chlorine, -OCF₃, -OCH₃, cyano and 3-trifluoromethyl-diazirin-3-yl.

In yet a more particular embodiment, there is provided a compound of formula (I) as described herein, or a pharmaceutically acceptable salt thereof, wherein
X is N or C(R⁷);
Y is S, SO or S(O)₂;
R¹ is selected from 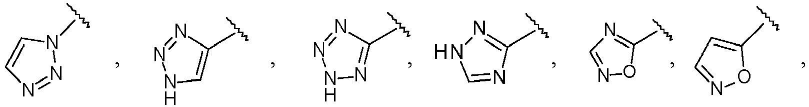 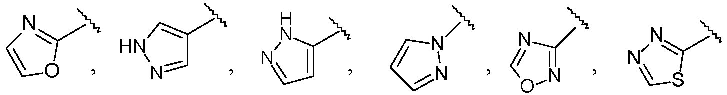 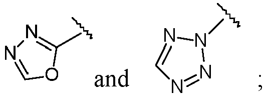
R² is hydrogen, halogen, C₁₋₆-alkyl, hydroxy or N(R⁸R^{8a});
R³ is hydrogen;
R⁴ is selected from 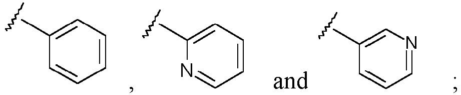
R⁵ is hydrogen or -C(O)(R⁹);
R⁶ and R^{6a} are hydrogen;
R⁷ is hydrogen or halogen;
R⁸ and R^{8a} are each independently selected from hydrogen and C₁₋₆-alkyl;
R⁹ is amino-C₁₋₆-alkyl;
R¹⁰ is selected from C₁₋₁₀-alkyl, C₁₋₁₀-alkyl substituted with C₂₋₆-alkynyl, C₁₋₁₀-haloalkyl, amino-C₁₋₁₀-alkyl, hydroxy-C₁₋₁₀-alkyl, C₃₋₁₀-cycloalkyl, C₃₋₁₀-cycloalkyl substituted with one or more halogen, phenyl substituted with one or more halogen, (C₁₋₁₀-alkyl)-phenyl substituted with one or more halogen, (alkoxy-C₁₋₁₀-alkyl)-phenyl, 3-10 membered heterocyclyl, 3-10 membered heterocyclyl substituted with one or more C₁₋₁₀-alkyl and halogen, and -N(R^{10e}R^{10f});
R^{10e} is hydrogen, R^{10f} is C₃₋₇-cycloalkyl;
R¹¹ is selected from halogen, -O(R^{11a}), cyano, amino-C₁₋₁₀-alkyl, -S(O₂)(C₁₋₆-alkyl), and 3-10 membered heterocyclyl substituted with one or more halo-C₁₋₆-alkyl;
R^{11a} is selected from C₁₋₁₂-alkyl, halo-C₁₋₆-alkyl, 3-7 membered heterocycloalkyl, and amino-C_{1- 12}-alkyl.

In another embodiment, there is provided a compound of formula (I) as described herein, or a pharmaceutically acceptable salt thereof, wherein
X is N or C(R⁷);
Y is S, SO or S(O)₂;
R¹ is selected from 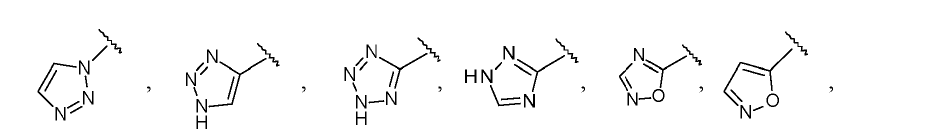 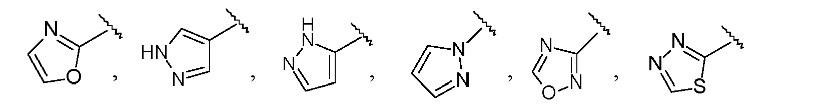 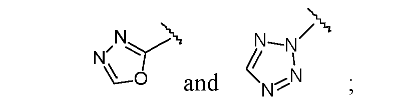 wherein R¹ is optionally substituted with one or more R¹⁰ which can be the same or different;
R² is hydrogen, halogen, C₁₋₆-alkyl, hydroxy or N(R⁸R^{8a});
R³ is hydrogen;
R⁴ is selected from 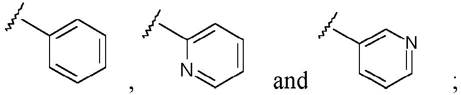
R⁵ is hydrogen or -C(O)(R⁹);
R⁶ and R^{6a} are hydrogen;
R⁷ is hydrogen or halogen;
R⁸ and R^{8a} are each independently selected from hydrogen and C₁₋₆-alkyl;
R⁹ is amino-C₁₋₆-alkyl;
R¹⁰ is selected from C₁₋₁₀-alkyl, C₁₋₁₀-alkyl substituted with C₂₋₆-alkynyl, C₁₋₁₀-haloalkyl, amino-C₁₋₁₀-alkyl, hydroxy-C₁₋₁₀-alkyl, C₃₋₁₀-cycloalkyl, C₃₋₁₀-cycloalkyl substituted with one or more halogen, phenyl substituted with one or more halogen, (C₁₋₁₀-alkyl)-phenyl substituted with one or more halogen, (alkoxy-C₁₋₁₀-alkyl)-phenyl, 3-10 membered heterocyclyl, 3-10 membered heterocyclyl substituted with one or more C₁₋₁₀-alkyl and halogen, and -N(R^{10e}R^{10f});
R¹⁰⁰ is hydrogen, R^{10f} is C₃₋₇-cycloalkyl;
R¹¹ is selected from halogen, -O(R^{11a}), cyano, amino-C₁₋₁₀-alkyl, -S(O₂)(C₁₋₆-alkyl), and 3-10 membered heterocyclyl substituted with one or more halo-C₁₋₆-alkyl;
R^{11a} is selected from C₁₋₁₂-alkyl, halo-C₁₋₆-alkyl, 3-7 membered heterocycloalkyl, and amino-C_{1- 12}-alkyl.

**In** a particular embodiment, there is provided a compound of formula (I) as described herein, or a pharmaceutically acceptable salt thereof, wherein
X is N or C(R⁷);
Y is S, SO or S(O)₂;
   R¹ is selected from 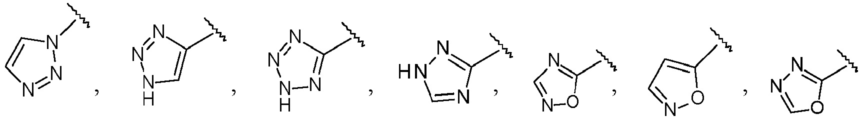 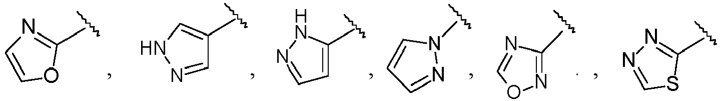 and 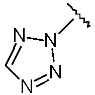
wherein R¹ is optionally substituted with one or more R¹⁰ which can be the same or different;
R² is hydrogen, halogen, C₁₋₆-alkyl, hydroxy or N(R⁸R^{8a});
R³ is hydrogen;
R⁴ is selected 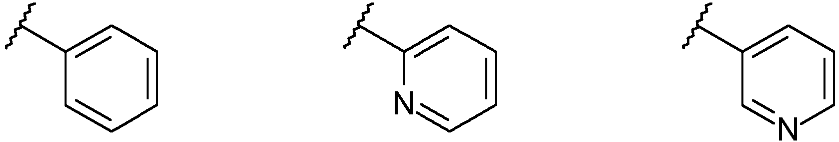
R⁵ is hydrogen or -C(O)(R⁹);
R⁶ and R^{6a} are hydrogen;
R⁷ is hydrogen or halogen;
R⁸ and R^{8a} are each independently selected from hydrogen and C₁₋₆-alkyl;
R⁹ is amino-C₁₋₆-alkyl;
R¹⁰ is selected from C₁₋₁₀-alkyl, C₁₋₁₀-alkyl substituted with C₂₋₆-alkynyl, C₁₋₁₀-haloalkyl, amino-C₁₋₁₀-alkyl, hydroxy-C₁₋₁₀-alkyl, C₃₋₁₀-cycloalkyl, C₃₋₁₀-cycloalkyl substituted with one or more halogen, phenyl substituted with one or more halogen, (C₁₋₁₀-alkyl)-phenyl substituted with one or more halogen, (alkoxy-C₁₋₁₀-alkyl)-phenyl, 3-10 membered heterocyclyl, 3-10 membered heterocyclyl substituted with one or more C₁₋₁₀-alkyl and halogen, -N(R^{10e}R^{10f});
R¹⁰⁰ is hydrogen, R^{10f} is C₃₋₇-cycloalkyl;
R¹¹ is selected from halogen, -O(R^{11a}), cyano, amino-C₁₋₁₀-alkyl, -S(O₂)(C₁₋₆-alkyl) and 3-10 membered heterocyclyl substituted with one or more halo-C₁₋₆-alkyl;
R^{11a} is selected from C₁₋₁₂-alkyl, halo-C₁₋₆-alkyl, 3-7 membered heterocycloalkyl, amino-C₁₋₁₂-alkyl.

In a a more particular embodiment, there is provided a compound of formula (I) as described herein, or a pharmaceutically acceptable salt thereof, wherein
X is CH;
Y is SO or S(O)₂;
R¹ is selected from 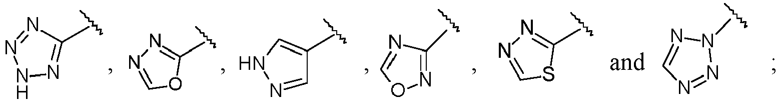
R² is hydrogen, halogen, C₁₋₆-alkyl;
R³ is hydrogen;
R⁴ is 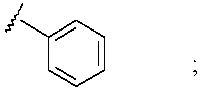
R¹ is hydrogen or -C(O)(amino-C₁₋₆-alkyl);
R⁶ and R^{6a} are hydrogen;
R¹⁰ is selected from C₁₋₁₀-alkyl, C₁₋₁₀-haloalkyl, C₃₋₁₀-cycloalkyl substituted with one or more halogen, hydroxy-C₁₋₁₀-alkyl, C₁₋₁₀-alkyl substituted with C₂₋₆-alkynyl, 3-10 membered heterocyclyl, 3-10 membered heterocyclyl substituted with one or more C₁₋₁₀-alkyl, halogen, and -NH(C₃₋₇-cycloalkyl);
R¹¹ is selected from halogen, -O(halo-C₁₋₆-alkyl), -O(C₁₋₆-alkyl), cyano, 3-10 membered heterocyclyl substituted with one or more halo-C₁₋₆-alkyl.

In another particular embodiment, there is provided a compound of formula (I) as described herein, or a pharmaceutically acceptable salt thereof, wherein:
X is CH;
Y is SO or S(O)₂;
R¹ is selected from 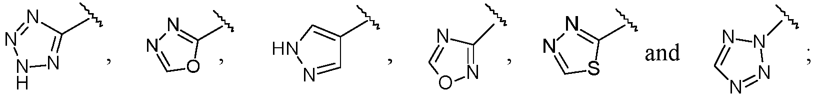
R² is hydrogen, fluorine or methyl;
R³ is hydrogen;
R⁴ is selected from 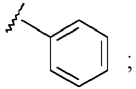
R⁵ is hydrogen or -C(O)(CH₂NH₂);
R⁶ and R^{6a} are hydrogen;
R¹⁰ is selected from ethyl, tertbutyl, isopropyl, -CH₂CF₃, -C((CH₃)₂)F, -C((CH₃)₂)CH₂OH, - C((CH₃)₂)CH₂CCH₃, difluorocyclohexyl, difluorocyclobutyl, piperidyl substituted with fluorine and methyl, morpholinyl, -NH(cyclopentyl);
R¹¹ is selected from chlorine, -OCF₃, -OCH₃, cyano, 3-trifluoromethyl-diazirin-3-yl.

In a more particularembodiment, there is provided a compound of formula (I) as described herein, or a pharmaceutically acceptable salt thereof, wherein
X is CH;
Y is SO or S(O)₂;
R¹ is selected 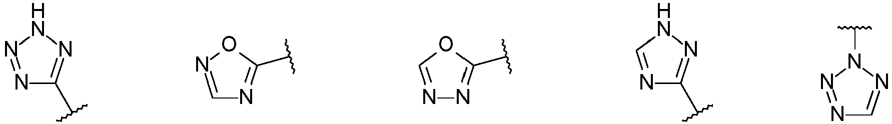 wherein R¹ is optionally substituted with one or more R¹⁰ which can be the same or different;
R² is hydrogen, halogen, C₁₋₆-alkyl;
R³ is hydrogen;
R⁴ is 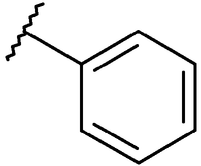
R⁵ is hydrogen or -C(O)(amino-C₁₋₆-alkyl);
R⁶ and R^{6a} are hydrogen;
R¹⁰ is selected from
C₁₋₁₀-alkyl, optionally substituted with one or more C₂₋₆-alkynyl, cyano;
C₁₋₁₀-haloalkyl, optionally substituted with C₁₋₅-alkoxy, amino, phenyl, wherein phenyl is optionally substituted with one or more halogen;
C₃₋₁₀-cycloalkyl, optionally substituted with one or more halogen;
phenyl, optionally substituted with one or more halogen, C₁₋₁₀-alkyl;
heteroaryl, optionally substituted with one or more halogen, C₁₋₁₀-alkyl;
3-10 membered heterocyclyl, optionally substituted with one or more C₁₋₁₀-alkyl, C₃₋₁₀-cycloalkyl, C₁₋₆-haloalkyl, halogen, -C₁₋₁₀-alkyl-C₁₋₄-alkoxy, -C(O)O-C₁₋₅-alkyl;
amino, optionally substituted with C₁₋₁₀-alkyl, C₁₋₁₀-haloalkyl, C₃₋₁₀-cycloalkyl, wherein C₃₋₁₀-cycloalkyl is optionally substituted with one or more C₁₋₅-alkyl, halogen;
R¹¹ is selected from halogen, -O(halo-C₁₋₆-alkyl), -O(C₁₋₆-alkyl), cyano, 3-10 membered heterocyclyl substituted with one or more halo-C₁₋₆-alkyl.

In anotherparticular embodiment, there is provided a compound of formula (I) as described herein, or a pharmaceutically acceptable salt thereof, wherein
X is CH;
Y is SO or S(O)₂;
R¹ is selected from 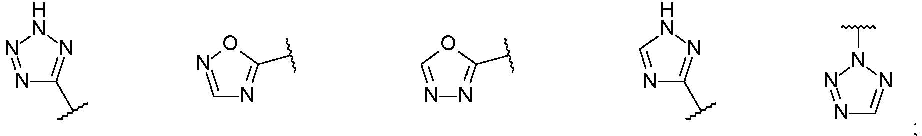
wherein R¹ is optionally substituted with one or more R¹⁰ which can be the same or different;
R² is hydrogen, fluorine or methyl;
R³ is hydrogen;
R⁴ is selected 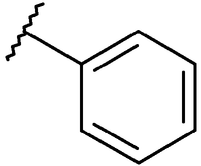
R⁵ is hydrogen or -C(O)(CH₂NH₂);
R⁶ and R^{6a} are hydrogen;
R¹⁰ is selected from tert-butyl, methyl 3-azabicyclo[3.1.1]heptane-3-carboxylate, 3-oxa-8-azabicyclo[3.2.1]octan-8-yl, 3,3-(difluorocyclobutyl)aminoyl, (trifluoromethyl)cyclopropyl, 2,2-difluoromorpholin-4-yl, 5,5-difluoro-1-methyl-3-piperidyl, 2,2,2-trifluoroethyl, (3,3-difluoro-1-methyl-cyclobutyl)aminoyl, o-tolyl, cyclobutylaminoyl, 2-methyl-propanenitrile, 6-fluoro-2-methyl-3-pyridyl, 1,2,2,2-tetrafluoro-1-methoxy-ethyl, 4-oxa-7-azaspiro[2.5]octan-7-yl, ethyl, 1-amino-2,2,2-trifluoro-1-methyl-ethyl, morpholinyl, 1-amino-2,2,2-trifluoro-1-(4-fluorophenyl)ethyl, methyl(2,2,2-trifluoroethyl)amino, 2,2-difluoromorpholin-4-yl, tertbutylaminoyl, cyclohexylaminoyl, methyl 4-methylpiperidine-1-carboxylate, 2-oxa-5-azabicyclo[4.1.0]heptan-5-yl, 2-(trifluoromethyl)morpholin-4-yl, 3,3-difluorocyclohexyl, 1,1-dimethylbut-3-ynyl, 3,3-difluorocyclobutyl, isobutyl, methyl 3,3-difluoropiperidine-1-carboxylate, difluorocyclopentyl, trifluoromethylmorpholinyl, 2,2-difluorospiro[3.3]heptan-6-yl, difluorocyclohexyl, 5,5-difluoro-1-(2-methoxyethyl)-3-piperidyl, 1-ethyl-5,5-difluoro-3-piperidyl, 2-cyclopropyltetrahydrofuran-2-yl, 3-fluoro-1-methyl-3-piperidyl, 6-fluoro-2-methyl-3-pyridyl, 2-fluoro-6-methyl-phenyl, fluorophenyl, 1,2,2,2-tetrafluoro-1-methoxy-ethyl;
R¹¹ is selected from chlorine, -OCF₃, -OCH₃, cyano, 3-trifluoromethyl-diazirin-3-yl.

In an yet more particular embodiment, there is provided a compound of formula (I) as described herein, selected from:
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1lambda4,5-benzothiazepin-4-one;
(3R)-3-amino-7-[5-(1-amino-2,2,2-trifluoro-ethyl)-1,2,4-oxadiazol-3-yl]-8-fluoro-5-[(4-isopropoxyphenyl)methyl]-1,1-dioxo-2,3-dihydro-1lambda6,5-benzothiazepin-4-one;
(3R)-3-amino-5-[(4-chlorophenyl)methyl]-7-[5-[3-fluoro-1-(2-methoxyethyl)-3-piperidyl]-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1lambda4,5-benzothiazepin-4-one;
(3R)-3-amino-7-(5-tert-butyl-1,2,4-oxadiazol-3-yl)-8-fluoro-5-[(4-fluorophenyl)methyl]-1,1-dioxo-2,3-dihydro-1lambda6,5-benzothiazepin-4-one;
(3R)-3-amino-5-[(4-chlorophenyl)methyl]-8-fluoro-7-(5-morpholino-1,2,4-oxadiazol-3-yl)-1-oxo-2,3-dihydro-1lambda4,5-benzothiazepin-4-one;
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1-oxo-2,3-dihydro-1lambda4,5-benzothiazepin-4-one;
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1-oxo-2,3-dihydro-1lambda4,5-benzothiazepin-4-one;
(3R)-3-amino-8-fluoro-5-[(6-isopropoxy-3-pyridyl)methyl]-7-[5-(4-oxa-7-azaspiro[2.5]octan-7-yl)-1,2,4-oxadiazol-3-yl]-1,1-dioxo-2,3-dihydro-1lambda6,5-benzothiazepin-4-one
(3R)-3-amino-5-[(4-chlorophenyl)methyl]-7-[5-[2-(difluoromethyl)morpholin-4-yl]-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1-oxo-7-[5-[2-(trifluoromethyl)morpholin-4-yl]-1,2,4-oxadiazol-3-yl]-2,3-dihydro-1lambda4,5-benzothiazepin-4-one;
(3R)-3-amino-5-[(4-chlorophenyl)methyl]-7-(5-morpholino-1,3,4-oxadiazol-2-yl)-1,1-dioxo-2,3-dihydro-1lambda6,5-benzothiazepin-4-one
(3R)-3-amino-5-[(4-chlorophenyl)methyl]-8-fluoro-1-oxo-7-[5-[2-(trifluoromethyl)morpholin-4-yl]-1,2,4-oxadiazol-3-yl]-2,3-dihydro-1lambda4,5-benzothiazepin-4-one;
(3R)-3-amino-5-[(4-chlorophenyl)methyl]-7-[5-(4-oxa-7-azaspiro[2.5]octan-7-yl)-1,3,4-oxadiazol-2-yl]-1,1-dioxo-2,3-dihydro-1lambda6,5-benzothiazepin-4-one;
methyl 3-[5-[(3R)-3-amino-5-[[4-(cyclopentoxy)phenyl]methyl]-1,1,4-trioxo-2,3-dihydro-1lambda6,5-benzothiazepin-7-yl]-1,3,4-oxadiazol-2-yl]pyrrolidine-1-carboxylate;
(3R)-3-amino-5-[(4-chlorophenyl)methyl]-1,1-dioxo-7-[5-[2-(trifluoromethyl)morpholin-4-yl]-1,3,4-oxadiazol-2-yl]-2,3-dihydro-1lambda6,5-benzothiazepin-4-one
(3R)-3-amino-8-fluoro-5-[(5-isopropoxy-2-pyridyl)methyl]-7-[5-(4-oxa-7-azaspiro[2.5]octan-7-yl)-1,2,4-oxadiazol-3-yl]-1,1-dioxo-2,3-dihydro-1lambda6,5-benzothiazepin-4-one;
2-amino-N-[(3R)-7-(2-tert-butyltetrazol-5-yl)-5-[(4-chlorophenyl)methyl]-8-fluoro-1,1,4-trioxo-2,3-dihydro-1lambda6,5-benzothiazepin-3-yl]acetamide
(3R)-3-amino-5-[(4-chlorophenyl)methyl]-7-[5-(6-methyl-3-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4,6-dimethyl-3-pyridyl)-1,2,4-oxadiazol-3-yl]-8-fluoro-1,1-dioxo-2,3-dihydro-1lambda6,5-benzothiazepin-4-one;
(3R)-3-amino-8-fluoro-7-[5-[2-(hydroxymethyl)tetrahydrofuran-2-yl]-1,2,4-oxadiazol-3-yl]-1,1-dioxo-5-[(4-phenoxyphenyl)methyl]-2,3-dihydro-1lambda6,5-benzothiazepin-4-one;
2-[5-[(3R)-3-amino-1,1,4-trioxo-5-[[4-(trifluoromethoxy)phenyl]methyl]-2,3-dihydro-1lambda6,5-benzothiazepin-7-yl]-1,3,4-oxadiazol-2-yl]-2-methyl-propanenitrile;
(3R)-3-amino-5-[(4-chlorophenyl)methyl]-8-fluoro-7-[5-(1-fluoro-1-methyl-ethyl)-1,2,4-oxadiazol-3-yl]-1,1-dioxo-2,3-dihydro-1lambda6,5-benzothiazepin-4-one;
(3R)-3-amino-8-fluoro-7-[5-(1-fluoro-1-methyl-ethyl)-1,2,4-oxadiazol-3-yl]-5-[(4-fluorophenyl)methyl]-1,1-dioxo-2,3-dihydro-1lambda6,5-benzothiazepin-4-one;
(3R)-3-amino-7-[5-(2,2-difluoromorpholin-4-yl)-1,3,4-oxadiazol-2-yl]-1,1-dioxo-5-[(4-phenoxyphenyl)methyl]-2,3-dihydro-1lambda6,5-benzothiazepin-4-one;
(3R)-3-amino-5-[(4-chlorophenyl)methyl]-7-[5-(2-oxa-5-azabicyclo[4.1.0]heptan-5-yl)-1,3,4-oxadiazol-2-yl]-1,1-dioxo-2,3-dihydro-1lambda6,5-benzothiazepin-4-one
(3R)-3-amino-7-[5-(3-aminooxetan-3-yl)-1,2,4-oxadiazol-3-yl]-8-fluoro-5-[[4-(4-methoxyphenyl)phenyl]methyl]-1,1-dioxo-2,3-dihydro-1lambda6,5-benzothiazepin-4-one
(3R)-3-amino-5-[(4-chlorophenyl)methyl]-8-fluoro-7-[5-(6-fluoro-2-methyl-3-pyridyl)-1,2,4-oxadiazol-3-yl]-1,1-dioxo-2,3-dihydro-1lambda6,5-benzothiazepin-4-one
(3R)-3-amino-5-[(4-chlorophenyl)methyl]-8-fluoro-7-[5-(2-methyl-4-methylsulfonyl-phenyl)-1,2,4-oxadiazol-3-yl]-1,1-dioxo-2,3-dihydro-1lambda6,5-benzothiazepin-4-one;
(3R)-3-amino-5-[(4-chlorophenyl)methyl]-8-fluoro-7-[5-(2-methyl-5-methylsulfonyl-phenyl)-1,2,4-oxadiazol-3-yl]-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-8-fluoro-7-[5-(4-methyl-3-pyridyl)-1,2,4-oxadiazol-3-yl]-1,1-dioxo-2,3-dihydro-1lambda6,5-benzothiazepin-4-one;
(3R)-3-amino-5-[(4-chlorophenyl)methyl]-7-[5-[(1R,5S)-3-oxa-8-azabicyclo[3.2.1]octan-8-yl]-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(2,2-dimethylmorpholin-4-yl)-1,2,4-oxadiazol-3-yl]-8-fluoro-1,1-dioxo-2,3-dihydro-1lambda6,5-benzothiazepin-4-one
(3R)-3-amino-5-[(4-chlorophenyl)methyl]-7-[5-(2-methyl-4-methylsulfonyl-phenyl)-1,3,4-oxadiazol-2-yl]-1,1-dioxo-2,3-dihydro-1lambda6,5-benzothiazepin-4-one;
methyl 3-[3-[(3R)-3-amino-5-[[4-(cyclopentoxy)phenyl]methyl]-8-fluoro-1,1,4-trioxo-2,3-dihydro-1lambda6,5-benzothiazepin-7-yl]-1,2,4-oxadiazol-5-yl]pyrrolidine-1-carboxylate
(3R)-3-amino-5-[(4-chlorophenyl)methyl]-7-[5-(2,2-difluoromorpholin-4-yl)-1,2,4-oxadiazol-3-yl]-8-fluoro-1,1-dioxo-2,3-dihydro-1lambda6,5-benzothiazepin-4-one
(3R)-3-amino-7-(5-tert-butyl-1,2,4-oxadiazol-3-yl)-5-[[4-(cyclopentoxy)phenyl]methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(5-methyl-1,2,4-oxadiazol-3-yl)-1,3,4-oxadiazol-2-yl]-1,1-dioxo-2,3-dihydro-1lambda6,5-benzothiazepin-4-one;
(3R)-3-amino-8-fluoro-7-[5-(3-fluoro-1-methyl-3-piperidyl)-1,2,4-oxadiazol-3-yl]-1,1-dioxo-5-[(4-phenoxyphenyl)methyl]-2,3-dihydro-1lambda6,5-benzothiazepin-4-one
(3R)-3-amino-5-[(4-chlorophenyl)methyl]-8-fluoro-7-[5-(6-fluoro-2-methyl-3-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4,6-dimethyl-3-pyridyl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-(4,4-difluoro-1-methoxy-cyclohexyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(2-fluoro-6-methyl-phenyl)-1,3,4-oxadiazol-2-yl]-1,1-dioxo-2,3-dihydro-1lambda6,5-benzothiazepin-4-one;1,1,1,3,3,3-hexafluoropropan-2-ol;
methyl 4-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-4-methoxy-piperidine-1-carboxylate;
(3R)-3-amino-5-[(4-chlorophenyl)methyl]-8-fluoro-7-[5-(5-methyl-1,3,4-oxadiazol-2-yl)-1,2,4-oxadiazol-3-yl]-1,1-dioxo-2,3-dihydro-1lambda6,5-benzothiazepin-4-one;
methyl N-[2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-2-methyl-1-(trifluoromethyl)propyl]carbamate;
(3R)-3-amino-7-[5-(2-amino-3,3,3-trifluoro-1,1-dimethyl-propyl)-1,3,4-oxadiazol-2-yl]-5-[(4-chlorophenyl)methyl]-8-fluoro-1,1-dioxo-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N-(3,3,3-trifluoro-2-hydroxy-2-methyl-propyl)-1,2,4-oxadiazole-5-carboxamide;
(3R)-3-amino-5-[(4-chlorophenyl)methyl]-8-fluoro-7-[5-[2-(hydroxymethyl)tetrahydrofuran-2-yl]-1,2,4-oxadiazol-3-yl]-1,1-dioxo-2,3-dihydro-1lambda6,5-benzothiazepin-4-one
(3R)-3-amino-8-fluoro-7-[5-(5-methyl-1,3,4-oxadiazol-2-yl)-1,2,4-oxadiazol-3-yl]-1,1-dioxo-5-[[4-(trifluoromethoxy)phenyl]methyl]-2,3-dihydro-1lambda6,5-benzothiazepin-4-one;
(3R)-3-amino-7-[5-[1-amino-2,2,2-trifluoro-1-(4-fluorophenyl)ethyl]-1,2,4-oxadiazol-3-yl]-5-[(4-chlorophenyl)methyl]-8-fluoro-1,1-dioxo-2,3-dihydro-1lambda6,5-benzothiazepin-4-one;
(3R)-3-amino-8-fluoro-7-[5-(5-methyl-1,3,4-oxadiazol-2-yl)-1,3,4-oxadiazol-2-yl]-1,1-dioxo-5-[[4-(trifluoromethoxy)phenyl]methyl]-2,3-dihydro-1lambda6,5-benzothiazepin-4-one;
(3R)-3-amino-5-[(4-chlorophenyl)methyl]-8-fluoro-7-[5-(3-oxa-8-azabicyclo[3.2.1]octan-8-yl)-1,2,4-oxadiazol-3-yl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[2-(trifluoromethyl)morpholin-4-yl]-1,2,4-oxadiazol-3-yl]-2,3-dihydro-1lambda6,5-benzothiazepin-4-one;
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1,1-dioxo-2,3-dihydro-1lambda6,5-benzothiazepin-4-one;
(3R)-3-amino-8-fluoro-7-[5-[2-(hydroxymethyl)tetrahydrofuran-2-yl]-1,2,4-oxadiazol-3-yl]-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-7-[5-(2-fluorophenyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4-fluorophenyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-1,1-dioxo-7-[5-(p-tolyl)-1,3,4-oxadiazol-2-yl]-2,3-dihydro-1lambda6,5-benzothiazepin-4-one;
(3R)-3-amino-5-[(4-chlorophenyl)methyl]-8-fluoro-7-[5-(2-fluorophenyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-7-[5-(m-tolyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1,1-dioxo-7-[5-(p-tolyl)-1,3,4-oxadiazol-2-yl]-2,3-dihydro-1lambda6,5-benzothiazepin-4-one
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-2-oxa-5-azabicyclo[2.2.1]heptane-5-carboxylate
(3R)-3-amino-7-[5-(1-amino-2,2,2-trifluoro-1-tetrahydropyran-4-yl-ethyl)-1,2,4-oxadiazol-3-yl]-5-[(4-chlorophenyl)methyl]-8-fluoro-1,1-dioxo-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N-(2,2-difluoropropyl)-1,2,4-oxadiazole-5-carboxamide;
(3R)-3-amino-7-[5-(1-amino-2,2,2-trifluoro-1-methyl-ethyl)-1,2,4-oxadiazol-3-yl]-8-fluoro-1,1-dioxo-5-[[4-(trifluoromethoxy)phenyl]methyl]-2,3-dihydro-1lambda6,5-benzothiazepin-4-one;
(3R)-3-amino-8-fluoro-1,1-dioxo-5-[[4-(trifluoromethoxy)phenyl]methyl]-7-[5-[1-(trifluoromethyl)cyclopropyl]-1,2,4-oxadiazol-3-yl]-2,3-dihydro-1lambda6,5-benzothiazepin-4-one
(3R)-3-amino-7-[5-(1-amino-2,2,2-trifluoro-1-methyl-ethyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[1-(trifluoromethyl)cyclopropyl]-1,3,4-oxadiazol-2-yl]-2,3-dihydro-1lambda6,5-benzothiazepin-4-one;
(3R)-3-amino-5-[(4-chlorophenyl)methyl]-1,1-dioxo-7-[5-[1-(trifluoromethyl)cyclopropyl]-1,3,4-oxadiazol-2-yl]-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N-(2,2,2-trifluoroethyl)-1,2,4-oxadiazole-5-carboxamide;
(3R)-3-amino-5-[(4-chlorophenyl)methyl]-1,1-dioxo-7-(5-phenyl-1,3,4-oxadiazol-2-yl)-2,3-dihydro-1lambda6,5-benzothiazepin-4-one
(3R)-3-amino-5-[(4-chlorophenyl)methyl]-8-fluoro-7-[5-(4-fluorophenyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-7-[5-(o-tolyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1,1-dioxo-7-(5-phenyl-1,3,4-oxadiazol-2-yl)-2,3-dihydro-1lambda6,5-benzothiazepin-4-one;
methyl 3-[5-[(3R)-3-amino-1,1,4-trioxo-5-[[4-(trifluoromethoxy)phenyl]methyl]-2,3-dihydro-1lambda6,5-benzothiazepin-7-yl]-1,3,4-oxadiazol-2-yl]pyrrolidine-1-carboxylate;
(3R)-3-amino-5-[(4-chlorophenyl)methyl]-8-fluoro-7-[5-(5-methyl-2-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(6-methyl-3-pyridyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1,1-dioxo-7-[5-[(3,3,3-trifluoro-2-hydroxy-2-methyl-propyl)amino]-1,3,4-oxadiazol-2-yl]-2,3-dihydro-1lambda6,5-benzothiazepin-4-one
(3R)-3-amino-5-[(4-chlorophenyl)methyl]-8-fluoro-7-[5-[[1-(2-hydroxyethyl)cyclohexyl]amino]-1,3,4-oxadiazol-2-yl]-1,1-dioxo-2,3-dihydro-1lambda6,5-benzothiazepin-4-one;
(3R)-3-amino-5-benzyl-8-fluoro-1,1-dioxo-7-[5-(1,2,2,2-tetrafluoro-1-methoxy-ethyl)-1,2,4-oxadiazol-3-yl]-2,3-dihydro-1lambda6,5-benzothiazepin-4-one;
(3R)-3-amino-7-[5-(3-aminooxetan-3-yl)-1,2,4-oxadiazol-3-yl]-8-fluoro-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-8-fluoro-7-[5-(6-fluoro-3-pyridyl)-1,3,4-oxadiazol-2-yl]-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[(4-phenoxyphenyl)methyl]-2,3-dihydro-1lambda6,5-benzothiazepin-4-one
(3R)-3-amino-5-[(4-chlorophenyl)methyl]-8-fluoro-7-[5-(5-fluoro-2-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2,2-difluoropropylamino)-1,3,4-oxadiazol-2-yl]-1,1-dioxo-2,3-dihydro-1lambda6,5-benzothiazepin-4-one;
2-[[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]amino]-2-methyl-propanenitrile
(3R)-3-amino-5-[(4-chlorophenyl)methyl]-7-[5-(1-methoxycyclopropyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-1,1-dioxo-7-[5-(2,2,2-trifluoro-1-methoxy-1-methyl-ethyl)-1,3,4-oxadiazol-2-yl]-2,3-dihydro-1lambda6,5-benzothiazepin-4-one;
(3R)-3-amino-5-[(4-chlorophenyl)methyl]-7-[5-(4-methoxytetrahydropyran-4-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-(1,1,2,2,2-pentafluoroethyl)-1,3,4-oxadiazol-2-yl]-2,3-dihydro-1lambda6,5-benzothiazepin-4-one
N-[11-[4-[[(3R)-3-amino-7-(5-tert-butyl-1,3,4-oxadiazol-2-yl)-8-fluoro-1,1,4-trioxo-2,3-dihydro-l1ambda6,5-benzothiazepin-5-yl]methyl]phenoxy]undecyl]-3-[2,2-difluoro-12-(1H-pyrrol-2-yl)-1-aza-3-azonia-2-boranuidatricyclo[7.3.0.03,7]dodeca-3,5,7,9,11-pent
(3R)-3-amino-5-[(4-chlorophenyl)methyl]-7-[5-(1-methoxy-1-methyl-ethyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[(1-ethynylcyclohexyl)amino]-1,3,4-oxadiazol-2-yl]-8-fluoro-1,1-dioxo-2,3-dihydro-1lambda6,5-benzothiazepin-4-one;
(3R)-3-amino-7-(5-tert-butyl-1,2,4-oxadiazol-3-yl)-5-[(4-chlorophenyl)methyl]-8-fluoro-1-imino-1-oxo-2,3-dihydro-1lambda6,5-benzothiazepin-4-one;
(3R)-3-amino-5-[(4-chlorophenyl)methyl]-7-[5-(9,9-difluoro-3-oxa-7-azabicyclo[3.3.1]nonan-7-yl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[4,4-difluoro-3-(hydroxymethyl)-1-piperidyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(3,3-dimethylmorpholin-4-yl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1lambda4,5-benzothiazepin-4-one
(3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1lambda4,5-benzothiazepin-4-one
methyl 4-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-4-cyano-piperidine-l-carboxylate
(3R)-3-amino-5-[(4-chlorophenyl)methyl]-7-[5-(3,3-difluoro-1,8-diazaspiro[4.5]decan-8-yl)-1,3,4-oxadiazol-2-yl]-8-fluoro-1,1-dioxo-2,3-dihydro-1lambda6,5-benzothiazepin-4-one;
(3R)-3-amino-7-(5-tert-butyl-1,2,4-oxadiazol-3-yl)-5-[(4-chlorophenyl)methyl]-8-fluoro-1-oxo-2,3 -dihydro-l1ambda4, 5-benzothiazepin-4-one;
(3R)-3-amino-7-(5-tert-butyl-1,2,4-oxadiazol-3-yl)-5-[(4-chlorophenyl)methyl]-8-fluoro-1-oxo-2,3 -dihydro-l1ambda4, 5-benzothiazepin-4-one;
(3R)-3-amino-5-[(4-chlorophenyl)methyl]-8-fluoro-7-[5-(1-methyl-1-pyrrolidin-1-yl-ethyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1-oxo-2,3-dihydro-1lambda4,5-benzothiazepin-4-one;
2-[4-[[(3R)-3-amino-7-(5-tert-butyl-1,3,4-oxadiazol-2-yl)-8-fluoro-1,1,4-trioxo-2,3-dihydro-l1ambda6,5-benzothiazepin-5-yl]methyl]phenyl]-2-methyl-propanenitrile
(3R)-3-amino-5-[(4-chlorophenyl)methyl]-7-[5-[(1S,5R)-6,6-difluoro-3-azabicyclo[3.1.1]heptan-3-yl]-1,3,4-oxadiazol-2-yl]-8-fluoro-1,1-dioxo-2,3-dihydro-1lambda6,5-benzothiazepin-4-one;
1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-4-methyl-piperidine-4-carbonitrile;
(3R)-3-amino-7-(5-tert-butyl-1,3,4-oxadiazol-2-yl)-8-fluoro-1,1-dioxo-5-[[4-(2,2,2-trifluoroethoxy)phenyl]methyl]-2,3-dihydro-1lambda6,5-benzothiazepin-4-one
(3R)-3-amino-5-[(4-chlorophenyl)methyl]-7-[5-[[1,1-dimethyl-4-(2-prop-2-ynoxyethoxy)butyl]amino]-1,2,4-oxadiazol-3-yl]-8-fluoro-1,1-dioxo-2,3-dihydro-1lambda6,5-benzothiazepin-4-one
3-[(3R)-3-amino-8-fluoro-1,1,4-trioxo-5-[[4-(1,1,2,2-tetrafluoroethoxy)phenyl]methyl]-2,3-dihydro-1lambda6,5-benzothiazepin-7-yl]-N,N-dimethyl-1,2,4-oxadiazole-5-carboxamide;
(3R)-3-amino-7-(5-tert-butyl-1,3,4-oxadiazol-2-yl)-5-[(6-chloropyridazin-3-yl)methyl]-8-fluoro-1,1-dioxo-2,3-dihydro-1lambda6,5-benzothiazepin-4-one
(3R)-3-amino-5-[(4-chlorophenyl)methyl]-8-fluoro-1,1-dioxo-7-[5-(2,2,2-trifluoroethylamino)-1,3,4-oxadiazol-2-yl]-2,3-dihydro-1lambda6,5-benzothiazepin-4-one;
(3R)-3-amino-5-[(4-chlorophenyl)methyl]-7-[5-[1,1-dimethyl-4-[2-(2-prop-2-ynoxyethoxy)ethoxy]butyl]-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-7-[2-(5,5-difluoro-1-methylsulfonyl-3-piperidyl)tetrazol-5-yl]-8-fluoro-1,1-dioxo-2,3-dihydro-1lambda6,5-benzothiazepin-4-one
methyl 5-[5-[(3R)-3-amino-5-[(4-chlorophenyl)methyl]-8-fluoro-1,1,4-trioxo-2,3-dihydro-1lambda6,5-benzothiazepin-7-yl]tetrazol-2-yl]-3,3-difluoro-piperidine-1-carboxylate
2-[[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]amino]-2-methyl-propanenitrile;
(3R)-3-amino-5-[(4-chlorophenyl)methyl]-8-fluoro-7-[5-[4-methyl-1-(2,2,2-trifluoroethyl)-4-piperidyl]-1,2,4-oxadiazol-3-yl]-1,1-dioxo-2,3-dihydro-1lambda6,5-benzothiazepin-4-one;
(3R)-3-amino-5-[(4-chlorophenyl)methyl]-7-[5-(2,2-difluoropropylamino)-1,3,4-oxadiazol-2-yl]-8-fluoro-1,1-dioxo-2,3-dihydro-1lambda6,5-benzothiazepin-4-one;
(3R)-3-amino-7-[5-(1-bicyclo[1.1.1]pentanylamino)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[(3,3,3-trifluoro-1-methyl-propyl)amino]-1,3,4-oxadiazol-2-yl]-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-1,1-dioxo-5-[(4-tetrahydropyran-4-yloxyphenyl)methyl]-2,3-dihydro-1lambda6,5-benzothiazepin-4-one
methyl 1-[3-[(3R)-3-amino-8-fluoro-1-imino-1,4-dioxo-5-[[4-(1,1,2,2-tetrafluoroethoxy)phenyl]methyl]-2,3-dihydro-1lambda6,5-benzothiazepin-7-yl]-1,2,4-oxadiazol-5-yl]-3-azabicyclo[3.1.1]heptane-3-carboxylate
methyl 1-[3-[(3R)-3-amino-8-fluoro-1,4-dioxo-5-[[4-(trifluoromethoxy)phenyl]methyl]-2,3-dihydro-1lambda4,5-benzothiazepin-7-yl]-1,2,4-oxadiazol-5-yl]-3-azabicyclo[3.1.1]heptane-3-carboxylate
(3R)-3-amino-5-[(4-chlorophenyl)methyl]-8-fluoro-1-imino-1-oxo-7-[5-(1,2,2,2-tetrafluoro-1-methoxy-ethyl)-1,2,4-oxadiazol-3-yl]-2,3-dihydro-1lambda6,5-benzothiazepin-4-one;
(3R)-3-amino-7-(2-tert-butyltetrazol-5-yl)-8-fluoro-1,1-dioxo-5-[[4-(tetrahydropyran-4-ylmethoxy)phenyl]methyl]-2,3-dihydro-1lambda6,5-benzothiazepin-4-one
(3R)-3-amino-7-(5-tert-butyl-1,2,4-oxadiazol-3-yl)-8-fluoro-1,1-dioxo-5-[[4-(tetrahydropyran-4-ylmethoxy)phenyl]methyl]-2,3-dihydro-1lambda6,5-benzothiazepin-4-one
(3R)-3-amino-7-(5-tert-butyl-1,3,4-oxadiazol-2-yl)-5-[[4-(cyclopropoxy)phenyl]methyl]-1,1-dioxo-2,3-dihydro-1lambda6,5-benzothiazepin-4-one
methyl 1-[3-[(3R)-3-amino-8-fluoro-1,4-dioxo-5-[[4-(trifluoromethoxy)phenyl]methyl]-2,3-dihydro-1lambda4,5-benzothiazepin-7-yl]-1,2,4-oxadiazol-5-yl]-3-azabicyclo[3.1.1]heptane-3-carboxylate
(3R)-7-[2-(1-acetyl-5,5-difluoro-3-piperidyl)tetrazol-5-yl]-3-amino-5-[(4-chlorophenyl)methyl]-8-fluoro-1,1-dioxo-2,3-dihydro-1lambda6,5-benzothiazepin-4-one
N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1lambda6,5-benzothiazepin-3-yl]acetamide;
(3R)-3-amino-5-[(4-chlorophenyl)methyl]-8-fluoro-1,1-dioxo-7-[5-(1,5,5-trimethyl-3-piperidyl)-1,2,4-oxadiazol-3-yl]-2,3-dihydro-1lambda6,5-benzothiazepin-4-one;
(3R)-3-amino-7-(5-tert-butyl-1,3,4-oxadiazol-2-yl)-5-[[4-(cyclohexoxy)phenyl]methyl]-1,1-dioxo-2,3-dihydro-1lambda6,5-benzothiazepin-4-one
methyl 1-[3-[(3R)-3-amino-8-fluoro-1,4-dioxo-5-[[4-(1,1,2,2-tetrafluoroethoxy)phenyl]methyl]-2,3-dihydro-l1ambda4,5-benzothiazepin-7-yl]-1,2,4-oxadiazol-5-yl]-3-azabicyclo[3.1.1]heptane-3-carboxylate
(3R)-3-amino-7-(2-tert-butyltetrazol-5-yl)-5-[(4-chlorophenyl)methyl]-8-fluoro-1-oxo-2,3-dihydro-1lambda4,5-benzothiazepin-4-one
(3R)-3-amino-7-(2-tert-butyltetrazol-5-yl)-5-[(4-chlorophenyl)methyl]-8-fluoro-1-oxo-2,3-dihydro-1lambda4,5-benzothiazepin-4-one
(3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1lambda4,5-benzothiazepin-4-one;
(3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1lambda4,5-benzothiazepin-4-one
(3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1lambda4,5-benzothiazepin-4-one;
(3R)-3-amino-5-[(4-chlorophenyl)methyl]-8-fluoro-7-[5-[[(1S)-1-methyl-2-(trifluoromethoxy)ethyl]amino]-1,3,4-oxadiazol-2-yl]-1,1-dioxo-2,3-dihydro-1lambda6,5-benzothiazepin-4-one;
(3R)-3-amino-5-benzyl-7-[2-(5,5-difluoro-1-methyl-3-piperidyl)tetrazol-5-yl]-8-fluoro-1,1-dioxo-2,3-dihydro-1lambda6,5-benzothiazepin-4-one
(3R)-3-amino-5-[(4-chlorophenyl)methyl]-7-[2-(5,5-difluoro-1-methyl-3-piperidyl)tetrazol-5-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-1,1-dioxo-5-[[4-(tetrahydropyran-4-ylmethoxy)phenyl]methyl]-2,3-dihydro-1lambda6,5-benzothiazepin-4-one
(3R)-3-amino-8-fluoro-1,1-dioxo-5-[[4-(trifluoromethoxy)phenyl]methyl]-7-[8-(trifluoromethyl)-5,6,7,8-tetrahydro-[1,2,4]triazolo[1,5-a]pyrazin-2-yl]-2,3-dihydro-1lambda6,5-benzothiazepin-4-one
(3R)-3-amino-8-fluoro-5-[(4-fluorophenyl)methyl]-1-oxo-7-[5-(1,2,2,2-tetrafluoro-1-methoxy-ethyl)-1,2,4-oxadiazol-3-yl]-2,3-dihydro-1lambda4,5-benzothiazepin-4-one;
(3R)-3-amino-8-fluoro-5-[(4-fluorophenyl)methyl]-1,1-dioxo-7-[5-(1,2,2,2-tetrafluoro-1-methoxy-ethyl)-1,2,4-oxadiazol-3-yl]-2,3-dihydro-1lambda6,5-benzothiazepin-4-one;
(3R)-3-amino-5-[(4-chlorophenyl)methyl]-8-fluoro-1,1-dioxo-7-[5-(2,2,2-trifluoro-1-hydroxy-1-methyl-ethyl)-1,2,4-oxadiazol-3-yl]-2,3-dihydro-1lambda6,5-benzothiazepin-4-one
(3R)-3-amino-7-(5-tert-butyl-1,3,4-oxadiazol-2-yl)-1,1-dioxo-5-[[4-[[5-(trifluoromethyl)-2-pyridyl]oxy]phenyl]methyl]-2,3-dihydro-l1ambda6,5-benzothiazepin-4-one
methyl 1-[3-[(3R)-3-amino-8-fluoro-1,1,4-trioxo-5-[[4-(1,1,2,2-tetrafluoroethoxy)phenyl]methyl]-2,3-dihydro-1lambda6,5-benzothiazepin-7-yl]-1,2,4-oxadiazol-5-yl]-3-azabicyclo[3.1.1]heptane-3-carboxylate
(3R)-3-amino-7-(5-tert-butyl-1,3,4-oxadiazol-2-yl)-5-[[4-(4-fluorophenoxy)phenyl]methyl]-1,1-dioxo-2,3-dihydro-1lambda6,5-benzothiazepin-4-one
methyl 1-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-3-azabicyclo[3.1.1]heptane-3-carboxylate
methyl 1-[3-[(3R)-3-amino-8-fluoro-1,1,4-trioxo-5-[[4-(trifluoromethoxy)phenylmethyl]-2,3-dihydro-1lambda6,5-benzothiazepin-7-yl]-1,2,4-oxadiazol-5-yl]-3-azabicyclo[3.1.1]heptane-3-carboxylate
(3R)-3-amino-5-[(4-chlorophenyl)methyl]-8-fluoro-1,1-dioxo-7-[5-[1-(trifluoromethyl)cyclopropyl]-1,2,4-oxadiazol-3-yl]-2,3-dihydro-1lambda6,5-benzothiazepin-4-one;
(3R)-3-amino-7-(2-tert-butyltetrazol-5-yl)-8-fluoro-5-[(4-fluorophenyl)methyl]-1,1-dioxo-2,3-dihydro-1lambda6,5-benzothiazepin-4-one
(3R)-3-amino-1-oxo-7-[5-(2,2,2-trifluoroethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-1-oxo-7-[5-(2,2,2-trifluoroethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-1-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-1-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-7-[5-(1-amino-2,2,2-trifluoro-ethyl)-1,2,4-oxadiazol-3-yl]-8-fluoro-1,1-dioxo-5-[[4-(trifluoromethoxy)phenyl]methyl]-2,3-dihydro-1lambda6,5-benzothiazepin-4-one;
isopropyl 1-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-3-azabicyclo[3.1.1]heptane-3-carboxylate
ethyl 1-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-3-azabicyclo[3.1.1]heptane-3-carboxylate
(3R)-3-amino-5-[(4-chlorophenyl)methyl]-7-[5-[1-(2,2-difluorocyclopropyl)ethylamino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[1-(2,2-difluorocyclopropyl)ethylamino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[1-(3,3-difluorocyclobutyl)ethylamino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[(3,3,3-trifluoro-1,1-dimethyl-propyl)amino]-1,3,4-oxadiazol-2-yl]-2,3-dihydro-l1ambda6,5-benzothiazepin-4-one;
(3R)-3-amino-5-[(4-chlorophenyl)methyl]-8-fluoro-7-[5-[1-methyl-1-(2,2,2-trifluoroethylamino)ethyl]-1,2,4-oxadiazol-3-yl]-1,1-dioxo-2,3-dihydro-1lambda6,5-benzothiazepin-4-one;
methyl 3-[3-[(3R)-3-amino-8-fluoro-5-[(4-isopropoxyphenyl)methyl]-1,1,4-trioxo-2,3-dihydro-1lambda6,5-benzothiazepin-7-yl]-1,2,4-oxadiazol-5-yl]pyrrolidine-1-carboxylate;
methyl 1-[5-[(3R)-3-amino-1,1,4-trioxo-5-[[4-(trifluoromethoxy)phenyl]methyl]-2,3-dihydro-1lambda6,5-benzothiazepin-7-yl]-1,3,4-oxadiazol-2-yl]-3-azabicyclo[3.1.1]heptane-3-carboxylate
(3R)-3-amino-7-(5-tert-butyl-1,3,4-oxadiazol-2-yl)-1,1-dioxo-5-[[4-(trifluoromethylsulfonyl)phenyl]methyl]-2,3-dihydro-1lambda6,5-benzothiazepin-4-one
(3R)-3-amino-7-[5-(5,5-difluoro-1-methyl-3-piperidyl)-1,2,4-oxadiazol-3-yl]-8-fluoro-5-[(4-isopropoxyphenyl)methyl]-1,1-dioxo-2,3-dihydro-1lambda6,5-benzothiazepin-4-one;
(3R)-3-amino-7-[5-(5,5-difluoro-1-methyl-3-piperidyl)-1,2,4-oxadiazol-3-yl]-8-fluoro-5-[(6-isopropoxy-3-pyridyl)methyl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[[3-(trifluoromethyl)cyclobutyl]amino]-1,3,4-oxadiazol-2-yl]-2,3-dihydro-1lambda6,5-benzothiazepin-4-one;
(3R)-3-amino-5-[(4-chlorophenyl)methyl]-8-fluoro-1,1-dioxo-7-[5-(1,2,2,2-tetrafluoroethyl)-1,2,4-oxadiazol-3-yl]-2,3-dihydro-l1ambda6,5-benzothiazepin-4-one;
methyl 1-[5-[(3R)-3-amino-1,1,4-trioxo-5-[[4-(1,1,2,2-tetrafluoroethoxy)phenyl]methyl]-2,3-dihydro-1lambda6,5-benzothiazepin-7-yl]-1,3,4-oxadiazol-2-yl]-3-azabicyclo[3.1.1]heptane-3-carboxylate
(3R)-3-amino-7-[5-[(3-chloro-1-bicyclo[1.1.1]pentanyl)amino]-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-[(3-fluoro-1-bicyclo[1.1.1]pentanyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-(1,1,2,2,2-pentafluoroethyl)-1,2,4-oxadiazol-3-yl]-2,3-dihydro-1lambda6,5-benzothiazepin-4-one
(3R)-3-amino-5-[(4-chlorophenyl)methyl]-7-[5-[(3,3-difluoro-1-methylcyclobutyl)amino]-1,2,4-oxadiazol-3-yl]-8-fluoro-1,1-dioxo-2,3-dihydro-1lambda6,5-benzothiazepin-4-one
(3R)-3-amino-5-[(4-chlorophenyl)methyl]-8-fluoro-7-[2-[(3R)-1-methylpyrrolidin-3-yl]tetrazol-5-yl]-1,1-dioxo-2,3-dihydro-1lambda6,5-benzothiazepin-4-one;
(3R)-3-amino-5-[(4-chlorophenyl)methyl]-8-fluoro-7-[2-[(3S)-1-methylpyrrolidin-3-yl]tetrazol-5-yl]-1,1-dioxo-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N,N-dimethyl-1,2,4-oxadiazole-5-carboxamide;
3-[(3R)-3-amino-8-fluoro-1,1,4-trioxo-5-[[4-(trifluoromethoxy)phenyl]methyl]-2,3-dihydro-1lambda6,5-benzothiazepin-7-yl]-N,N-dimethyl-1,2,4-oxadiazole-5-carboxamide;
(3R)-3-amino-5-[(4-chlorophenyl)methyl]-7-[1-(5,5-difluoro-1-methyl-3-piperidyl)pyrazol-4-yl]-8-fluoro-1,1-dioxo-2,3-dihydro-1lambda6,5-benzothiazepin-4-one;
(3R)-3-amino-7-[5-[(2-amino-3,3,3-trifluoro-propyl)amino]-1,2,4-oxadiazol-3-yl]-5-[(4-chlorophenyl)methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[(4-pyrrolidin-1-ylphenyl)methyl]-2,3-dihydro-1lambda6,5-benzothiazepin-4-one
(6-methyl-3-pyridyl) 4-[5-[(3R)-3-amino-5-[(4-chlorophenyl)methyl]-1,1,4-trioxo-2,3-dihydro-1lambda6,5-benzothiazepin-7-yl1,3,4-oxadiazol-2-yl]-4-methyl-piperidine-1-carboxylate;
(3R)-3-amino-7-[5-(5,5-difluoro-1-methyl-3-piperidyl)-1,2,4-oxadiazol-3-yl]-8-fluoro-5-[(5-isopropoxy-2-pyridyl)methyl]-1,1-dioxo-2,3-dihydro-1lambda6,5-benzothiazepin-4-one;
(3R)-3-amino-5-[(4-chlorophenyl)methyl]-7-[5-(1-ethyl-4-methyl-4-piperidyl)-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-(1,2,2,2-tetrafluoro-1-methoxy-ethyl)-1,2,4-oxadiazol-3-yl]-2,3-dihydro-1lambda6,5-benzothiazepin-4-one;
2-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-2-methyl-propanenitrile;
(3R)-3-amino-5-[(4-chlorophenyl)methyl]-7-[5-(5,5-dimethyl-3-piperidyl)-1,2,4-oxadiazol-3 -yl]-8-fluoro-1,1-dioxo-2,3-dihydro-1lambda6,5-benzothiazepin-4-one;
2,2,2-trifluoroethyl 4-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl-4-methyl-piperidine-1-carboxylate;
methyl 4-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-4-methyl-piperidine-1-carboxylate;
(3R)-3-amino-5-[(4-chlorophenyl)methyl]-8-fluoro-1,1-dioxo-7-[5-[[1-(2,2,2-trifluoroethyl)-3-piperidyl]amino]-1,3,4-oxadiazol-2-yl]-2,3-dihydro-1lambda6,5-benzothiazepin-4-one
(3R)-3-amino-7-[2-(2-amino-3,3,3-trifluoro-propyl)tetrazol-5-yl]-5-[(4-chlorophenyl)methyl]-8-fluoro-1,1-dioxo-2,3-dihydro-1lambda6,5-benzothiazepin-4-one;
4-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-N,4-dimethyl-piperidine-1-carboxamide;
(3R)-3-amino-5-[(4-chlorophenyl)methyl]-8-fluoro-7-[5-[4-methyl-1-(pyrrolidine-1-carbonyl)-4-piperidyl]-1,2,4-oxadiazol-3-yl]-1,1-dioxo-2,3-dihydro-1lambda6,5-benzothiazepin-4-one;
(3R)-3-amino-7-[5-(5,5-difluoro-1-methyl-3-piperidyl)-1,3,4-oxadiazol-2-yl]-1,1-dioxo-5-[[4-(trifluoromethoxy)phenyl]methyl]-2,3-dihydro-1lambda6,5-benzothiazepin-4-one;
(3R)-3-amino-5-[(4-chlorophenyl)methyl]-8-fluoro-1,1-dioxo-7-[5-[(2,2,2-trifluoro-1-methyl-ethyl)amino]-1,3,4-oxadiazol-2-yl]-2,3-dihydro-1lambda6,5-benzothiazepin-4-one;
(3R)-3-amino-7-[5-(1-amino-2,2,2-trifluoro-1-methyl-ethyl)-1,2,4-oxadiazol-3-yl]-5-[(4-chlorophenyl)methyl]-8-fluoro-1,1-dioxo-2,3-dihydro-1lambda6,5-benzothiazepin-4-one;
methyl 3-[5-[(3R)-3-amino-5-[(4-chlorophenyl)methyl]-8-fluoro-1,1,4-trioxo-2,3-dihydro-1lambda6,5-benzothiazepin-7-yl]tetrazol-2-yl]pyrrolidine-1-carboxylate
(3R)-3-amino-5-[(4-chlorophenyl)methyl]-7-[5-[(3,3-difluorocyclobutyl)amino]-1,2,4-oxadiazol-3 -yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-5-[[4-(4-cyclopropylpiperazin-1-yl)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[[4-(4-methoxy-1-piperidyl)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[[4-(cyclopropylmethoxy)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[[4-(2,2-difluoroethoxy)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[(4-but-2-ynoxyphenyl)methyl]-1,1-dioxo-2,3-dihydro-1lambda6,5-benzothiazepin-4-one
(3R)-3-amino-7-[5-(1-amino-2,2,2-trifluoro-1-methyl-ethyl)-1,3,4-oxadiazol-2-yl]-5-[(4-chlorophenyl)methyl]-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[[4-(2,2,2-trifluoroethoxy)phenyl]methyl]-2,3-dihydro-1lambda6,5-benzothiazepin-4-one
(3R)-3-amino-7-(5-tert-butyl-1,3,4-oxadiazol-2-yl)-5-[(4-morpholinophenyl)methyl]-1,1-dioxo-2,3-dihydro-1lambda6,5-benzothiazepin-4-one
(3R)-3-amino-5-[(4-chlorophenyl)methyl]-8-fluoro-1,1-dioxo-7-[5-[3-(trifluoromethyl)piperazin-1-yl]-1,2,4-oxadiazol-3-yl]-2,3-dihydro-1lambda6,5-benzothiazepin-4-one;2,2,2-trifluoroacetic acid
(3R)-3-amino-1,1-dioxo-7-[5-(1,1,2,2,2-pentafluoroethyl)-1,3,4-oxadiazol-2-yl]-5-[[4-(trifluoromethoxy)phenyl]methyl]-2,3-dihydro-l1ambda6,5-benzothiazepin-4-one;
(3R)-3-amino-5-[(4-chlorophenyl)methyl]-7-[5-[(3,3-difluorocyclohexyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(3,3-difluorocyclobutyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(3,3-difluoro-1-methylcyclobutyl)amino]-1,3,4-oxadiazol-2-yl]-8-fluoro-1,1-dioxo-2,3-dihydro-1lambda6,5-benzothiazepin-4-one;
2-[4-[[(3R)-3-amino-7-(5-tert-butyl-1,3,4-oxadiazol-2-yl)-1,1,4-trioxo-2,3-dihydro-1lambda6,5-benzothiazepin-5-yl]methyl]phenyl]-2-methyl-propanenitrile
(3R)-3-amino-5-[(4-chlorophenyl)methyl]-7-[5-(4,4-difluoro-1-piperidyl)-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-8-fluoro-7-(5-morpholino-1,2,4-oxadiazol-3-yl)-1,1-dioxo-2,3-dihydro-1lambda6,5-benzothiazepin-4-one;
(3R)-3-amino-5-[(4-chlorophenyl)methyl]-7-[5-(cyclobutylamino)-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1,1-dioxo-2,3-dihydro-1lambda6,5-benzothiazepin-4-one;
(3R)-7-[2-(1-acetyl-3-piperidyl)tetrazol-5-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[2-(1-methyl-3-piperidyl)tetrazol-5-yl]-1,1-dioxo-2,3-dihydro-1lambda6,5-benzothiazepin-4-one;
(3R)-3-amino-7-(3-tert-butyl-1,2,4-triazol-1-yl)-5-[(4-chlorophenyl)methyl]-8-fluoro-1,1-dioxo-2,3-dihydro-1lambda6,5-benzothiazepin-4-one;
(3R)-3-amino-7-[5-[1-methyl-1-(methylamino)ethyl]-1,3,4-oxadiazol-2-yl]-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-7-[5-[3-(dimethylamino)-1,1-dimethylpropyl]-1,3,4-oxadiazol-2-yl]-8-fluoro-1,1-dioxo-2,3-dihydro-1lambda6,5-benzothiazepin-4-one;
3-[(3R)-3-amino-1,1,4-trioxo-5-[[4-(trifluoromethoxy)phenyl]methyl]-2,3-dihydro-1lambda6,5-benzothiazepin-7-yl]-N-tert-butyl-1,2,4-oxadiazole-5-carboxamide
3-[(3R)-3-amino-8-fluoro-1,1,4-trioxo-5-[[4-(trifluoromethoxy)phenyl]methyl]-2,3-dihydro-1lambda6,5-benzothiazepin-7-yl]-N-tert-butyl-1,2,4-oxadiazole-5-carboxamide
3-[(3R)-3-amino-5-[(4-chlorophenyl)methyl]-8-fluoro-1,1,4-trioxo-2,3-dihydro-1lambda6,5-benzothiazepin-7-yl]-N-tert-butyl-1,2,4-oxadiazole-5-carboxamide
3-[(3R)-3-amino-5-[(4-chlorophenyl)methyl]-1,1,4-trioxo-2,3-dihydro-1lambda6,5-benzothiazepin-7-yl]-N-tert-butyl-1,2,4-oxadiazole-5-carboxamide
(3R)-3-amino-5-[(4-chlorophenyl)methyl]-7-[5-[(dimethylamino)methyl]-1,2,4-oxadiazol-3-yl]-8-fluoro-1,1-dioxo-2,3-dihydro-1lambda6,5-benzothiazepin-4-one
(3R)-3-amino-7-[5-(1-aminocyclopentyl)-1,3,4-oxadiazol-2-yl]-1,1-dioxo-5-[[4-(trifluoromethoxy)phenyl]methyl]-2,3-dihydro-l1ambda6,5-benzothiazepin-4-one
(3R)-3-amino-5-[(4-chlorophenyl)methyl]-7-[5-(3,3-difluorocyclohexyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-[cyclopropyl(methyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[methyl(2,2,2-trifluoroethyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[[3,3-difluoro-1-(methoxymethyl)cyclobutyl]amino]-1,3,4-oxadiazol-2-yl]-1,1-dioxo-2,3-dihydro-1lambda6,5-benzothiazepin-4-one;
(3R)-3-amino-7-[5-(4-aminotetrahydropyran-4-yl)-1,3,4-oxadiazol-2-yl]-1,1-dioxo-5-[[4-(trifluoromethoxy)phenyl]methyl]-2,3-dihydro-1lambda6,5-benzothiazepin-4-one
(3R)-3-amino-7-[5-(1-aminocyclohexyl)-1,3,4-oxadiazol-2-yl]-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-8-fluoro-7-[5-(1-hydroxy-1-methyl-ethyl)-1,3,4-oxadiazol-2-yl]-1,1-dioxo-2,3-dihydro-1lambda6,5-benzothiazepin-4-one;2,2,2-trifluoroacetic acid
(3R)-3-amino-7-(2-tert-butyltetrazol-5-yl)-1,1-dioxo-5-[[4-(trifluoromethoxy)phenyl]methyl]-2,3-dihydro-l1ambda6,5-benzothiazepin-4-one
(3R)-3-amino-7-(2-tert-butyltetrazol-5-yl)-8-fluoro-1,1-dioxo-5-[[4-(trifluoromethoxy)phenyl]methyl]-2,3-dihydro-l1ambda6,5-benzothiazepin-4-one
(3R)-3-amino-5-[(4-chlorophenyl)methyl]-8-fluoro-7-[2-(1-methylsulfonyl-3-piperidyl)tetrazol-5-yl]-1,1-dioxo-2,3-dihydro-1lambda6,5-benzothiazepin-4-one;
(3R)-3-amino-5-[(4-chlorophenyl)methyl]-7-[5-[(3,3-difluoro-1-methylcyclobutyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[(3,3-difluorocyclobutyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[(3,3-difluorocyclohexyl)amino]-1,3,4-oxadiazol-2-yl]-1,1-dioxo-2,3-dihydro-1lambda6,5-benzothiazepin-4-one;
(3R)-3-amino-7-(4-tert-butylimidazol-1-yl)-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(5-oxa-2-azaspiro[3.4]octan-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2-cyclopropyltetrahydrofuran-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2-cyclopropyltetrahydrofuran-2-yl)-1,3,4-oxadiazol-2-yl]-1,1-dioxo-2,3-dihydro-1lambda6,5-benzothiazepin-4-one
(3R)-3-amino-7-(2-tert-butyltetrazol-5-yl)-5-[(4-chlorophenyl)methyl]-1,1-dioxo-2,3-dihydro-1lambda6,5-benzothiazepin-4-one
(3R)-7-[5-(3-acetyl-3-azabicyclo[3.1.0]hexan-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
methyl 3-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]azetidine-1-carboxylate;2,2,2-trifluoroacetic acid
(3R)-3-amino-5-[(4-chlorophenyl)methyl]-7-[5-(2,2-difluoromorpholin-4-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-1,1-dioxo-7-[5-(1,2,2,2-tetrafluoroethyl)-1,3,4-oxadiazol-2-yl]-2,3-dihydro-1lambda6,5-benzothiazepin-4-one;
(3R)-3-amino-5-[(4-chlorophenyl)methyl]-8-fluoro-7-[5-(3-methylsulfonyl-3-azabicyclo[3.1.0]hexan-1-yl)-1,3,4-oxadiazol-2-yl]-1,1-dioxo-2,3-dihydro-1lambda6,5-benzothiazepin-4-one
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[3.1.0]hexane-3-carboxylate
(3R)-3-amino-5-[(4-chlorophenyl)methyl]-8-fluoro-7-[2-(1-methylsulfonylpyrrolidin-3-yl)tetrazol-5-yl]-1,1-dioxo-2,3-dihydro-1lambda6,5-benzothiazepin-4-one;
(3R)-3-amino-5-[(4-chlorophenyl)methyl]-7-[5-(2-hydroxyspiro[3.3]heptan-6-yl)-1,3,4-oxadiazol-2-yl]-1,1-dioxo-2,3-dihydro-1lambda6,5-benzothiazepin-4-one
(3R)-3-amino-7-[5-[3-(chloromethyl)-3-(hydroxymethyl)azetidin-1-yl]-1,3,4-oxadiazol-2-yl]-5-[(4-chlorophenyl)methyl]-1,1-dioxo-2,3-dihydro-1lambda6,5-benzothiazepin-4-one;
2-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-2-methoxy-acetonitrile;
(3R)-3-amino-1,1-dioxo-7-[5-(2,2,2-trifluoroethyl)-1,3,4-oxadiazol-2-yl]-5-[[4-(trifluoromethoxy)phenyl]methyl]-2,3-dihydro-l1ambda6,5-benzothiazepin-4-one;
(3R)-3-amino-5-[(4-chlorophenyl)methyl]-8-fluoro-7-[2-(1-methylsulfonyl-4-piperidyl)tetrazol-5-yl]-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[[4-(1,1,2,2-tetrafluoroethoxy)phenyl]methyl]-2,3-dihydro-l1ambda6,5-benzothiazepin-4-one
(3R)-3-amino-5-[(4-chlorophenyl)methyl]-8-fluoro-7-[5-(5-oxa-2-azaspiro[3.4]octan-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(6-oxa-2-azaspiro[3.5]nonan-2-yl)-1,3,4-oxadiazol-2-yl]-1,1-dioxo-2,3-dihydro-1lambda6,5-benzothiazepin-4-one;
(3R)-3-amino-7-(5-tert-butyl-1,3,4-oxadiazol-2-yl)-5-[[4-(cyclopentoxy)phenyl]methyl]-1,1-dioxo-2,3-dihydro-1lambda6,5-benzothiazepin-4-one
(3R)-3-amino-5-[(4-chlorophenyl)methyl]-1,1-dioxo-7-[5-(1,1,2,2,2-pentafluoroethyl)-1,3,4-oxadiazol-2-yl]-2,3-dihydro-1lambda6,5-benzothiazepin-4-one;
methyl 7-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-5-azaspiro[2.4]heptane-5-carboxylate;
2-[5-[(3R)-3-amino-8-fluoro-1,1,4-trioxo-5-[[4-(trifluoromethoxy)phenyl]methyl]-2,3-dihydro-1lambda6,5-benzothiazepin-7-yl]-1,3,4-oxadiazol-2-yl]-2-methyl-propanenitrile;
(3R)-3-amino-1,1-dioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1lambda6,5-benzothiazepin-4-one;
(3R)-3-amino-5-[(4-chlorophenyl)methyl]-1,1-dioxo-7-[5-(1,2,2,2-tetrafluoro-1-methoxy-ethyl)-1,3,4-oxadiazol-2-yl]-2,3-dihydro-1lambda6,5-benzothiazepin-4-one;
(3R)-3-amino-7-[5-[(1R,5S)-8-azabicyclo[3.2.1]octane-8-carbonyl]-1,3,4-oxadiazol-2-yl]-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(5-methylsulfonyl-5-azaspiro[2.4]heptan-7-yl)-1,3,4-oxadiazol-2-yl]-1,1-dioxo-2,3-dihydro-1lambda6,5-benzothiazepin-4-one;
(3R)-7-[5-(1-acetyl-4-piperidyl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-8-fluoro-7-(2-methyltetrazol-5-yl)-1,1-dioxo-2,3-dihydro-1lambda6,5-benzothiazepin-4-one;
(3R)-3-amino-5-[(4-chlorophenyl)methyl]-8-fluoro-7-[2-(1-methylpyrrolidin-3-yl)tetrazol-5-yl]-1,1-dioxo-2,3-dihydro-1lambda6,5-benzothiazepin-4-one;
(3R)-7-[2-(1-acetyl-4-piperidyl)tetrazol-5-yl]-3-amino-5-[(4-chlorophenyl)methyl]-8-fluoro-1,1-dioxo-2,3-dihydro-1lambda6,5-benzothiazepin-4-one;
(3R)-7-[5-(3-acetyl-3-azabicyclo[4.1.0]heptan-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(3-methylsulfonyl-3-azabicyclo[4.1.0]heptan-1-yl)-1,3,4-oxadiazol-2-yl]-1,1-dioxo-2,3-dihydro-1lambda6,5-benzothiazepin-4-one;
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[4.1.0]heptane-3-carboxylate;
(3R)-3-amino-5-[(4-chlorophenyl)methyl]-7-[5-(6,6-difluoro-2-azaspiro[3.3]heptan-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(isopropylamino)-1,3,4-oxadiazol-2-yl]-1,1-dioxo-2,3-dihydro-1lambda6,5-benzothiazepin-4-one;
(3R)-3-amino-7-[5-(2-azaspiro[3.3]heptan-2-yl)-1,3,4-oxadiazol-2-yl]-5-[(4-chlorophenyl)methyl]-1,1-dioxo-2,3-dihydro-1lambda6,5-benzothiazepin-4-one;
methyl 5-[[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]amino]-3,3-difluoro-piperidine-1-carboxylate;
(3R)-3-amino-5-[(4-chlorophenyl)methyl]-7-[5-(4,4-difluoro-1-piperidyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2,2-dimethylmorpholin-4-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(3-fluoro-1-methyl-3-piperidyl)-1,3,4-oxadiazol-2-yl]-1,1-dioxo-2,3-dihydro-1lambda6,5-benzothiazepin-4-one;
methyl 4-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]piperidine-1-carboxylate
(3R)-3-amino-7-[5-(1-amino-2,2,2-trifluoro-ethyl)-1,3,4-oxadiazol-2-yl]-8-fluoro-1,1-dioxo-5-[[4-(trifluoromethoxy)phenyl]methyl]-2,3-dihydro-1lambda6,5-benzothiazepin-4-one
methyl 3-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]pyrrolidine-1-carboxylate
(3R)-3-amino-5-[(4-chlorophenyl)methyl]-7-[5-(5,5-difluoro-1-methyl-3-piperidyl)-1,3,4-oxadiazol-2-yl]-8-methyl-1,1-dioxo-2,3-dihydro-1lambda6,5-benzothiazepin-4-one
(3R)-3-amino-5-[(4-chlorophenyl)methyl]-7-[5-(5,5-difluoro-1-methyl-3-piperidyl)-1,3,4-oxadiazol-2-yl]-1,1-dioxo-2,3-dihydro-1lambda6,5-benzothiazepin-4-one
2-[5-[(3R)-3-amino-5-[(4-chlorophenyl)methyl]-8-methyl-1,1,4-trioxo-2,3-dihydro-1lambda6,5-benzothiazepin-7-yl]-1,3,4-oxadiazol-2-yl]-2-methyl-propanenitrile;
(3R)-3-amino-5-[(4-chlorophenyl)methyl]-7-[5-[(5,5-difluoro-3-piperidyl)amino]-1,3,4-oxadiazol-2-yl]-8-fluoro-1,1-dioxo-2,3-dihydro-1lambda6,5-benzothiazepin-4-one;
(3R)-3-amino-7-[5-(3-azabicyclo[4.1.0]heptan-1-yl)-1,3,4-oxadiazol-2-yl]-5-[(4-chlorophenyl)methyl]-8-fluoro-1,1-dioxo-2,3-dihydro-1lambda6,5-benzothiazepin-4-one
(3R)-7-[5-(5-acetyl-5-azaspiro[2.4]heptan-7-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-7-[5-(3,3-difluoropiperidine-1-carbonyl)-1,3,4-oxadiazol-2-yl]-8-fluoro-1,1-dioxo-2,3-dihydro-l1ambda6,5-benzothiazepin-4-one
2-[5-[(3R)-3-amino-5-[(4-chlorophenyl)methyl]-8-methyl-1,1,4-trioxo-2,3-dihydro-1lambda6,5-benzothiazepin-7-yl]-1,3,4-oxadiazol-2-yl]propanenitrile;
(3R)-3-amino-5-[(4-chlorophenyl)methyl]-8-fluoro-7-[5-(3-methylsulfonyl-3-azabicyclo[3.1.1]heptan-1-yl)-1,3,4-oxadiazol-2-yl]-1,1-dioxo-2,3-dihydro-1lambda6,5-benzothiazepin-4-one
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[3.1.1]heptane-3-carboxylate;
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[3.1.1]heptane-3-carboxylate
2-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-2-methyl-propanenitrile;
(3R)-7-[5-(3-acetyl-3-azabicyclo[3.1.1]heptan-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
(3R)-3-amino-7-[5-(1-amino-2,2,2-trifluoro-ethyl)-1,3,4-oxadiazol-2-yl]-5-[(4-chlorophenyl)methyl]-8-fluoro-1,1-dioxo-2,3-dihydro-1lambda6,5-benzothiazepin-4-one;2,2,2-trifluoroacetic acid
(3R)-3-amino-7-[5-(1-amino-2,2,2-trifluoro-ethyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-[4-(dimethylamino)-1,1-dimethyl-butyl]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(3,3-difluorocyclopentyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(4,4-difluorocyclohexyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-(1,3-dimethylazetidin-3-yl)-1,3,4-oxadiazol-2-yl]-8-fluoro-1,1-dioxo-2,3-dihydro-1lambda6,5-benzothiazepin-4-one;
(3R)-3-amino-7-[5-(tert-butylamino)-1,3,4-oxadiazol-2-yl]-5-[(4-chlorophenyl)methyl]-8-fluoro-1,1-dioxo-2,3-dihydro-1lambda6,5-benzothiazepin-4-one;
1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]cyclopropanecarbonitrile;
2-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]propanenitrile;
methyl 3-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]piperidine-1-carboxylate
(3R)-3-amino-5-[(4-chlorophenyl)methyl]-7-[5-(3,3-difluoropyrrolidine-1-carbonyl)-1,3,4-oxadiazol-2-yl]-8-fluoro-1,1-dioxo-2,3-dihydro-l1ambda6,5-benzothiazepin-4-one
(3R)-3-amino-7-[5-(2-aminospiro[3.3]heptan-6-yl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(2-oxa-7-azaspiro[3.5]nonan-7-yl)-1,3,4-oxadiazol-2-yl]-1,1-dioxo-2,3-dihydro-1lambda6,5-benzothiazepin-4-one
4-[[(3R)-3-amino-8-fluoro-7-[5-(2-hydroxy-1,1-dimethyl-ethyl)-1,3,4-oxadiazol-2-yl]-1,1,4-trioxo-2,3-dihydro-1lambda6,5-benzothiazepin-5-yl]methyl]benzonitrile;
(3R)-3-amino-5-[(4-chlorophenyl)methyl]-7-[5-(5,5-difluoro-1-methyl-3-piperidyl)-1,2,4-oxadiazol-3-yl]-8-fluoro-1,1-dioxo-2,3-dihydro-1lambda6,5-benzothiazepin-4-one
(3R)-3-amino-7-[5-(5-amino-3,3-difluoro-1-piperidyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-7-[5-[1-(chloromethyl)-2-hydroxy-1-methyl-ethyl]-1,3,4-oxadiazol-2-yl]-5-[(4-chlorophenyl)methyl]-8-fluoro-1,1-dioxo-2,3-dihydro-1lambda6,5-benzothiazepin-4-one;
5-[(3R)-3-amino-5-[(4-chlorophenyl)methyl]-8-fluoro-1,1,4-trioxo-2,3-dihydro-1lambda6,5-benzothiazepin-7-yl]-N-isopropyl-N-methyl-1,3,4-oxadiazole-2-carboxamide
(3R)-3-amino-5-[(4-chlorophenyl)methyl]-7-[5-(1-cyclopropyl-5,5-difluoro-3-piperidyl)-1,3,4-oxadiazol-2-yl]-8-fluoro-1,1-dioxo-2,3-dihydro-1lambda6,5-benzothiazepin-4-one;
(3R)-3-amino-7-[5-[4-(chloromethyl)-4-(hydroxymethyl)-1-piperidyl]-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(4,4-difluoro-1-methyl-3-piperidyl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(2-methylsulfonylethyl)-1,3,4-oxadiazol-2-yl]-1,1-dioxo-2,3-dihydro-1lambda6,5-benzothiazepin-4-one;
5-[(3R)-3-amino-5-[(4-chlorophenyl)methyl]-8-fluoro-1,1,4-trioxo-2,3-dihydro-1lambda6,5-benzothiazepin-7-yl]-N-tert-butyl-1,3,4-oxadiazole-2-carboxamide
(3R)-3-amino-5-[(4-chlorophenyl)methyl]-8-fluoro-7-[5-(isopropoxymethyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-[5,5-difluoro-1-(2-methoxyethyl)-3-piperidyl]-1,3,4-oxadiazol-2-yl]-8-fluoro-1,1-dioxo-2,3-dihydro-1lambda6,5-benzothiazepin-4-one
5-[(3R)-3-amino-5-[(4-chlorophenyl)methyl]-8-fluoro-1,1,4-trioxo-2,3-dihydro-1lambda6,5-benzothiazepin-7-yl]-3-tert-butyl-1,3,4-oxadiazol-2-one;
(3R)-3-amino-5-[(4-chlorophenyl)methyl]-8-fluoro-7-[5-(2-methyltetrahydrofuran-2-yl)-1,3,4-oxadiazol-2-yl]-1,1-dioxo-2,3-dihydro-1lambda6,5-benzothiazepin-4-one;
(3R)-3-amino-7-[5-(4-amino-1,1-dimethyl-butyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(1,3-dimethyl-3-piperidyl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(1-ethyl-3-piperidyl)amino]-1,3,4-oxadiazol-2-yl]-8-fluoro-1,1-dioxo-2,3-dihydro-1lambda6,5-benzothiazepin-4-one
(3R)-3-amino-7-[5-(3-amino-4,4-difluoro-1-piperidyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-[2-(1,1-dioxo-1,4-thiazinan-4-yl)-1,1-dimethyl-ethyl]-1,3,4-oxadiazol-2-yl]-8-fluoro-1,1-dioxo-2,3-dihydro-1lambda6,5-benzothiazepin-4-one
5-[(3R)-3-amino-5-[(4-chlorophenyl)methyl]-8-fluoro-1,1,4-trioxo-2,3-dihydro-1lambda6,5-benzothiazepin-7-yl]-N,N-dimethyl-1,3,4-oxadiazole-2-carboxamide
(3R)-3-amino-5-[(4-chlorophenyl)methyl]-7-[5-(1-ethyl-5,5-difluoro-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(1-methylpyrrolidin-3-yl)-1,3,4-oxadiazol-2-yl]-1,1-dioxo-2,3-dihydro-1lambda6,5-benzothiazepin-4-one
methyl 5-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3,3-difluoro-piperidine-l-carboxylate
(3R)-3-amino-7-[5-(2-azaspiro[3.3]heptan-2-yl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(isopropylamino)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4,4-difluoro-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[(1-cyclopropyl-4-piperidyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-(2,2-dimethylmorpholin-4-yl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(4-oxa-7-azaspiro[2.5]octan-7-yl)-1,3,4-oxadiazol-2-yl]-1,1-dioxo-2,3-dihydro-1lambda6,5-benzothiazepin-4-one
(3R)-3-amino-7-(5-tert-butyl-1,3,4-oxadiazol-2-yl)-5-[[6-(cyclopentoxy)-3-pyridyl]methyl]-8-fluoro-1,1-dioxo-2,3-dihydro-1lambda6,5-benzothiazepin-4-one
(3R)-3-amino-5-[(4-chlorophenyl)methyl]-7-[5-[1,1-dimethyl-2-(2-oxo-1-piperidyl)ethyl]-1,3,4-oxadiazol-2-yl]-8-fluoro-1,1-dioxo-2,3-dihydro-1lambda6,5-benzothiazepin-4-one
2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-2-methyl-propanenitrile;
2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]propanenitrile;
(3R)-3-amino-5-[(4-chlorophenyl)methyl]-8-fluoro-7-[5-(3-methylazetidin-3-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(5,5-difluoro-1-methylsulfonyl-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[5,5-difluoro-1-(2-hydroxyethyl)-3-piperidyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[5-[1-(hydroxymethyl)cyclopropyl]-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(cyclobutylamino)-1,3,4-oxadiazol-2-yl]-8-fluoro-1,1-dioxo-2,3-dihydro-1lambda6,5-benzothiazepin-4-one;
(3R)-7-[5-(1-acetyl-5,5-difluoro-3-piperidyl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(5,5-difluoro-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-(3-ethyl-1,2,4-triazol-1-yl)-8-fluoro-1,1-dioxo-2,3-dihydro-1lambda6,5-benzothiazepin-4-one
(3R)-7-[5-(4-acetylpiperazin-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-(5-isobutyl-1,3,4-oxadiazol-2-yl)-1,1-dioxo-2,3-dihydro-1lambda6,5-benzothiazepin-4-one
(3R)-3-amino-5-[(4-chlorophenyl)methyl]-8-fluoro-7-[5-(4-methylpiperazin-1-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-[2-[2-hydroxyethyl(methyl)amino]-1,1-dimethyl-ethyl]-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-[1,1-dimethyl-2-(2-oxopyrrolidin-1-yl)ethyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(1-methyl-3-piperidyl)-1,3,4-oxadiazol-2-yl]-1,1-dioxo-2,3-dihydro-l1ambda6,5-benzothiazepin-4-one
(3R)-3-amino-5-[(4-chlorophenyl)methyl]-7-[5-[1,1-dimethyl-2-(4-methylpiperazin-1-yl)ethyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[1,1-dimethyl-2-(tetrahydrofuran-3-ylamino)ethyl]-1,3,4-oxadiazol-2-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-5-[(5-isopropoxy-2-pyridyl)methyl]-1,1-dioxo-2,3-dihydro-1lambda6,5-benzothiazepin-4-one;
(3R)-3-amino-5-[(4-chlorophenyl)methyl]-7-[5-[1,1-dimethyl-2-(oxetan-3-ylamino)ethyl]-1,3,4-oxadiazol-2-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-5-[(3,4-difluorophenyl)methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-5-[(4-chloro-3-fluorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(4,4-difluoro-1-piperidyl)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-7-[5-(1,1-dimethyl-2-morpholino-ethyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[2-(dimethylamino)-1,1-dimethyl-ethyl]-1,3,4-oxadiazol-2-yl]-8-fluoro-1,1-dioxo-2,3-dihydro-l1ambda6,5-benzothiazepin-4-one
(3R)-3-amino-5-[(4-chlorophenyl)methyl]-7-[5-(6,6-difluoro-2-azaspiro[3.3]heptan-2-yl)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-8-fluoro-5-[(6-isopropoxy-3-pyridyl)methyl]-1,1-dioxo-2,3-dihydro-1lambda6,5-benzothiazepin-4-one;
(3R)-3-amino-5-[(4-chlorophenyl)methyl]-7-[5-[2-(4,4-difluoro-1-piperidyl)-1,1-dimethyl-ethyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[2-(2-hydroxy-1,1-dimethylethyl)tetrazol-5-yl]-1,1-dioxo-2,3-dihydro-1lambda6,5-benzothiazepin-4-one;
(3R)-3-amino-5-[(4-chlorophenyl)methyl]-8-fluoro-7-[2-(2-hydroxy-2-methylpropyl)tetrazol-5-yl]-1,1-dioxo-2,3-dihydro-1lambda6,5-benzothiazepin-4-one;
(3R)-3-amino-5-[(4-chlorophenyl)methyl]-7-[5-(cyclopropylamino)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-7-[5-[1,1-dimethyl-2-(1-piperidyl)ethyl]-1,3,4-oxadiazol-2-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-5-[(4-chlorophenyl)methyl]-8-fluoro-1-oxo-2,3 -dihydro-l1ambda4, 5-benzothiazepin-4-one;
(3R)-3-amino-7-(5-tert-butyl-1,3,4-oxadiazol-2-yl)-5-[(4-chlorophenyl)methyl]-8-fluoro-1-oxo-2,3-dihydro-1lambda4,5-benzothiazepin-4-one
(3R)-3-amino-5-[(4-chlorophenyl)methyl]-7-[5-(2,2-difluorospiro[3.3]heptan-6-yl)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-7-[5-(dimethylamino)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-8-fluoro-7-[5-[(3-methylazetidin-1-yl)methyl]-1,3,4-oxadiazol-2-yl]-2,3-dihydro-1,5-benzothiazepin-4-one;1,1,1,3,3,3-hexafluoropropan-2-ol;
methyl 4-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-4-methyl-piperidine-1-carboxylate;
(3R)-3-amino-5-[(4-chlorophenyl)methyl]-8-fluoro-7-[5-(4-methyl-1-methylsulfonyl-4-piperidyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(cyclohexylamino)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-8-fluoro-7-[5-(1-methylcyclopropyl)-1,3,4-oxadiazol-2-yl]-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-7-[5-[3-(difluoromethyl)azetidin-3-yl]-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-7-[5-(2-amino-1,1-dimethyl-ethyl)-1,3,4-oxadiazol-2-yl]-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(6-oxaspiro[3.4]octan-2-yl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-l1ambda6,5-benzothiazepin-4-one
(3R)-3-amino-7-(5-tert-butyl-1,3,4-oxadiazol-2-yl)-8-fluoro-5-[(3-fluoro-4-methylsulfonyl-phenyl)methyl]-1,1-dioxo-2,3-dihydro-1lambda6,5-benzothiazepin-4-one;
(3R)-3-amino-7-(5-tert-butyl-1,3,4-oxadiazol-2-yl)-5-[(4-ethyloxazol-2-yl)methyl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-1-imino-1-oxo-5-[[4-(trifluoromethoxy)phenyl]methyl]-2,3-dihydro-1lambda6,5-benzothiazepin-4-one;
(3R)-3-amino-7-(5-tert-butyl-1,3,4-oxadiazol-2-yl)-8-fluoro-5-[(2-fluoro-4-methylsulfonyl-phenyl)methyl]-1,1-dioxo-2,3-dihydro-1lambda6,5-benzothiazepin-4-one;1,1,1,3,3,3-hexafluoropropan-2-ol;
2-[5-[(3R)-3-amino-5-[(4-chlorophenyl)methyl]-8-fluoro-1,1,4-trioxo-2,3-dihydro-l1ambda6,5-benzothiazepin-7-yl]-1,3,4-oxadiazol-2-yl]-N,N,2-trimethyl-propanamide;1,1,1,3,3,3-hexafluoropropan-2-ol;
(3R)-3-amino-5-[(4-chlorophenyl)methyl]-8-fluoro-7-[3-(2-hydroxy-1,1-dimethyl-ethyl)-1,2,4-oxadiazol-5-yl]-1,1-dioxo-2,3-dihydro-1lambda6,5-benzothiazepin-4-one;
(3R)-3-amino-7-(5-tert-butyl-1,3,4-oxadiazol-2-yl)-8-fluoro-5-[[4-(3-hydroxyoxetan-3-yl)phenyl]methyl]-1,1-dioxo-2,3-dihydro-1lambda6,5-benzothiazepin-4-one
(3R)-3-amino-5-[(4-chlorophenyl)methyl]-8-fluoro-7-[5-(2-hydroxy-1,1-dimethyl-ethyl)-1,2,4-oxadiazol-3-yl]-1,1-dioxo-2,3-dihydro-1lambda6,5-benzothiazepin-4-one;2,2,2-trifluoroacetic acid
(3R)-3-amino-5-[(4-chlorophenyl)methyl]-8-fluoro-7-[5-(2-hydroxy-1,1-dimethyl-ethyl)-1,2,4-oxadiazol-3-yl]-1,1-dioxo-2,3-dihydro-1lambda6,5-benzothiazepin-4-one
(3R)-3-amino-7-(5-tert-butyl-1,3,4-oxadiazol-2-yl)-8-fluoro-1,1-dioxo-5-[(4-phenoxyphenyl)methyl]-2,3-dihydro-1lambda6,5-benzothiazepin-4-one;
(3R)-3-amino-7-(5-tert-butyl-1,3,4-oxadiazol-2-yl)-8-fluoro-1,1-dioxo-5-[[4-(1,1,2,2-tetrafluoroethoxy)phenyl]methyl]-2,3-dihydro-1lambda6,5-benzothiazepin-4-one;
(3R)-3-amino-7-(5-tert-butyl-1,3,4-oxadiazol-2-yl)-8-fluoro-5-[[4-(2-hydroxyethoxy)phenylmethyl]-1,1-dioxo-2,3-dihydro-1lambda6,5-benzothiazepin-4-one;2,2,2-trifluoroacetic acid
(3R)-3-amino-7-(5-tert-butyl-1,3,4-oxadiazol-2-yl)-5-[(4-chlorophenyl)methyl]-8-fluoro-9-methyl-1,1-dioxo-2,3-dihydro-1lambda6,5-benzothiazepin-4-one
(3R)-3-amino-7-(5-tert-butyl-1,3,4-oxadiazol-2-yl)-5-[[1-(cyclopropylmethyl)pyrazol-4-yl]methyl]-8-fluoro-1,1-dioxo-2,3-dihydro-1lambda6,5-benzothiazepin-4-one;2,2,2-trifluoroacetic acid
(3R)-7-[5-(1-acetyl-4-methyl-4-piperidyl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-8-fluoro-1-methylimino-1-oxo-5-[[4-(trifluoromethoxy)phenyl]methyl]-2,3-dihydro-l1ambda6,5-benzothiazepin-4-one;
2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-N,2-dimethyl-propanamide;
(3R)-3-amino-7-(5-tert-butyl-1,3,4-oxadiazol-2-yl)-5-[(2-cyclopropylpyrimidin-5-yl)methyl]-8-fluoro-1,1-dioxo-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-5-[(6-chloro-3-pyridyl)methyl]-8-fluoro-1,1-dioxo-2,3-dihydro-1lambda6,5-benzothiazepin-4-one;1,1,1,3,3,3-hexafluoropropan-2-ol;
(2R,3R)-3-amino-7-(5-tert-butyl-1,3,4-oxadiazol-2-yl)-5-[(4-chlorophenyl)methyl]-8-fluoro-2-methyl-1,1-dioxo-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-5-[[5-[(4-chlorophenyl)methyl]-3-pyridyl]methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(4-methyl-4-piperidyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(4-methyltetrahydropyran-4-yl)-1,3,4-oxadiazol-2-yl]-1,1-dioxo-2,3-dihydro-1lambda6,5-benzothiazepin-4-one;2,2,2-trifluoroacetic acid
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1lambda4,5-benzothiazepin-4-one;
(3R)-3-amino-7-(5-tert-butyl-1,3,4-oxadiazol-2-yl)-8-fluoro-1-imino-1-oxo-5-[[4-(trifluoromethoxy)phenyl]methyl]-2,3-dihydro-l1ambda6,5-benzothiazepin-4-one;
(3R)-3-amino-7-(5-tert-butyl-1,3,4-oxadiazol-2-yl)-8-chloro-5-[(4-chlorophenyl)methyl]-1,1-dioxo-2,3-dihydro-1lambda6,5-benzothiazepin-4-one;
2-[4-[[(3R)-3-amino-7-(5-tert-butyl-1,3,4-oxadiazol-2-yl)-8-fluoro-1,1,4-trioxo-2,3-dihydro-1lambda6,5-benzothiazepin-5-yl]methyl]phenoxy]acetamide;1,1,1,3,3,3-hexafluoropropan-2-ol;
N-[11-[4-[[(3R)-3-amino-7-(5-tert-butyl-1,3,4-oxadiazol-2-yl)-8-fluoro-1,1,4-trioxo-2,3-dihydro-1lambda6,5-benzothiazepin-5-yl]methyl]phenoxy]undecyl]acetamide;
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]-2-(methylamino)propanamide
(2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]butanamide
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]-3-hydroxy-2-(methylamino)propanamide
(3R)-3-amino-7-(5-tert-butyl-1,3,4-oxadiazol-2-yl)-5-[[4-(cyclopentoxy)phenyl]methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-8-fluoro-5-[(4-isopropoxyphenyl)methyl]-1,1-dioxo-2,3-dihydro-1lambda6,5-benzothiazepin-4-one;
(3R)-3-amino-5-[(4-chlorophenyl)methyl]-1,1-dioxo-7-[5-[2,2,2-trideuterio-1,1-bis(trideuteriomethyl)ethyl]-1,3,4-oxadiazol-2-yl]-2,3-dihydro-1lambda6,5-benzothiazepin-4-one;
(3R)-3-amino-5-[(4-chlorophenyl)methyl]-7-[5-(3,3-difluorocyclohexyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-(4-ethyltriazol-1-yl)-8-fluoro-2,3-dihydro-1,5-benzothiazepin-4-one;
(3R)-3-amino-5-[(4-chlorophenyl)methyl]-7-[4-[(4-chlorophenyl)methyl]triazol-1-yl]-8-fluoro-2,3-dihydro-1,5-benzothiazepin-4-one;
(3R)-3-amino-5-[(4-chlorophenyl)methyl]-7-(4-ethyltriazol-1-yl)-8-fluoro-1,1-dioxo-2,3-dihydro-1λ⁶,5-benzothiazepin-4-one;
(3R)-3-amino-5-[(4-chlorophenyl)methyl]-7-[4-[(4-chlorophenyl)methyl]triazol-1-yl]-8-fluoro-1,1-dioxo-2,3-dihydro-1λ⁶5-benzothiazepin-4-one;
(3R)-3-amino-7-(4-tert-butyltriazol-1-yl)-5-[(4-chlorophenyl)methyl]-8-fluoro-1,1-dioxo-2,3-dihydro-1λ⁶ 5-benzothiazepin-4-one;
(3R)-3-amino-5-[(4-chlorophenyl)methyl]-7-(1-ethyltriazol-4-yl)-8-fluoro-1,1-dioxo-2,3-dihydro-1λ⁶,5-benzothiazepin-4-one;
(3R)-3-amino-5-[(4-chlorophenyl)methyl]-7-(2-ethyltetrazol-5-yl)-8-fluoro-1,1-dioxo-2,3-dihydro-1λ⁶,5-benzothiazepin-4-one;
(3R)-3-amino-7-(2-tert-butyltetrazol-5-yl)-5-[(4-chlorophenyl)methyl]-8-fluoro-1,1-dioxo-2,3-dihydro-1λ⁶,5--benzothiazepin-4-one;
(3R)-3-amino-5-[(4-chlorophenyl)methyl]-8-fluoro-1,1-dioxo-7-(1H-pyrazol-5-yl)-2,3-dihydro-1λ⁶,5-benzothiazepin-4-one;
(3R)-3-amino-5-[(4-chlorophenyl)methyl]-7-(5-ethyl-1H-1,2,4-triazol-3-yl)-8-fluoro-1,1-dioxo-2,3-dihydro-1λ⁶,5-benzothiazepin-4-one;
(3R)-3-amino-5-[(4-chlorophenyl)methyl]-1,1-dioxo-7-[3-(2,2,2-trifluoroethyl)-1,2,4-oxadiazol-5-yl]-2,3-dihydropyrido[3,2-b][1,4]thiazepin-4-one;
(3R)-3-amino-5-[(4-chlorophenyl)methyl]-7-(3-cyclopropyl-1,2,4-oxadiazol-5-yl)-8-fluoro-1,1-dioxo-2,3-dihydro-1λ⁶,5-benzothiazepin-4-one;
(3R)-3-Amino-5-[(4-chlorophenyl)methyl]-7-(3 -ethyl-1,2,4-oxadiazol-5-yl)-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-(3-propyl-1,2,4-oxadiazol-5-yl)-2,3-dihydro-1λ⁶,5-benzothiazepin-4-one;
(3R)-3-Amino-7-(3-tert-butyl-1,2,4-oxadiazol-5-yl)-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-7-[3-[(4-chlorophenyl)methyl]-1,2,4-oxadiazol-5-yl]-8-fluoro-1,1-dioxo-2,3-dihydro-1λ⁶,5-benzothiazepin-4-one;
(3R)-3-Amino-7-[3-(benzyloxymethyl)-1,2,4-oxadiazol-5-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-8-fluoro-1,1-dioxo-7-[3-(2,2,2-trifluoroethyl)-1,2,4-oxadiazol-5-yl]-2,3-dihydro-1λ⁶,5-benzothiazepin-4-one;
(3R)-3-amino-7-[3-(aminomethyl)-1,2,4-oxadiazol-5-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(3-ethyl-1,2,4-oxadiazol-5-yl)-1,1-dioxo-2,3-dihydro-1λ⁶,5-benzothiazepin-4-one;
(3R)-3-amino-7-(3-tert-butylisoxazol-5-yl)-5-[(4-chlorophenyl)methyl]-8-fluoro-1,1-dioxo-2,3-dihydro-1λ⁶,5-benzothiazepin-4-one;
(3R)-3-amino-7-[5-(2,2-difluorocyclohexyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-amino-7-(5-tert-butyl-1,3,4-oxadiazol-2-yl)-8-fluoro-5-[[4-(trifluoromethoxy)phenyl]methyl]-2,3-dihydro-1,5-benzothiazepin-4-one;
(3R)-3-Amino-7-[5-(3,3-difluorocyclohexyl)-1,3,4-oxadiazol-2-yl]-8-fluoro-1,1-dioxo-5-[4-(trifluoromethoxy)phenyl]methyl]-2,3-dihydro-1λ⁶,5-benzothiazepin-4-one;
(3R)-3-Amino-8-fluoro-1,1-dioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-4-one;
(3R)-3-Amino-7-(5-tert-butyl-1,3,4-oxadiazol-2-yl)-8-fluoro-1,1-dioxo-5-[[4-(trifluoromethoxy)phenyl]methyl]-2,3-dihydro-1λ⁶,5-benzothiazepin-4-one;
(2S)-N-[(3R)-8-fluoro-1,1,4-trioxo-7-[5-(2,2,2-trifluoroethyl)-1,3,4-oxadiazol-2-yl]-5-[4-(trifluoromethoxy)phenyl]methyl]-2,3-dihydro-1λ⁶,5-benzothiazepin-3-yl]-2-(methylamino)propanamide;
(3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-[5-(3,3,3-trifluoropropyl)-1,3,4-oxadiazol-2-yl]-2,3-dihydro-1λ⁶,5-benzothiazepin-4-one;
(3R)-3-Amino-5-[(4-chlorophenyl)methyl]-8-fluoro-1,1-dioxo-7-[5-(2,2,2-trifluoroethyl)-1,3,4-oxadiazol-2-yl]-2,3-dihydro-1λ⁶,5-benzothiazepin-4-one;
(3R)-3-Amino-7-[5-(1-amino-1-methyl-ethyl)-1,3,4-oxadiazol-2-yl]-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-8-fluoro-7-[5-(1-fluoro-1-methyl-ethyl)-1,3,4-oxadiazol-2-yl]-1,1-dioxo-2,3-dihydro-1λ⁶,5-benzothiazepin-4-one;
(3R)-3-Amino-5-[(4-chlorophenyl)methyl]-7-[5-(2,2-difluoroethyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-(5-ethyl-1,3,4-oxadiazol-2-yl)-8-fluoro-1,1-dioxo-2,3-dihydro-1λ⁶,5-benzothiazepin-4-one;
(3R)-3-Amino-5-[(4-chlorophenyl)methyl]-7-[5-(4,4-difluorocyclohexyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclohexyl)-1,3,4-oxadiazol-2-yl]-8-fluoro-1,1-dioxo-2,3-dihydro-1λ⁶,5-benzothiazepin-4-one:
(3R)-3-Amino-5-[(4-chlorophenyl)methyl]-7-[5-(3,3-difluorocyclopentyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclopentyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-[(3,3-difluorocyclobutyl)methyl]-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-[5-(tetrahydropyran-4-ylmethyl)-1,3,4-oxadiazol-2-yl]-2,3-dihydro-1λ⁶,5-benzothiazepin-4-one;
(3R)-3-Amino-5-[(4-chlorophenyl)methyl]-8-fluoro-1,1-dioxo-7-[5-(4,4,4-trifluorobutyl)-1,3,4-oxadiazol-2-yl]-2,3-dihydro-1λ⁶,5-benzothiazepin-4-one;
(3R)-3-Amino-5-[(4-chlorophenyl)methyl]-7-[5-(3,3-difluorocyclobutyl)-1,3,4-oxadiazol-2-yl]-8-fluoro-1,1-dioxo-2,3-dihydro-1λ^{6,}5-benzothiazepin-4-one;
(3R)-3-Amino-5-[(4-chlorophenyl)methyl]-7-[5-(2,2-difluorocyclobutyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-(5-tetrahydropyran-4-yl-1,3,4-oxadiazol-2-yl)-2,3-dihydro-1λ⁶,5-benzothiazepin-4-one;
(3R)-3-Amino-5-[(4-chlorophenyl)methyl]-8-fluoro-7-[5-(2-fluoroethyl)-1,3,4-oxadiazol-2-yl]-1,1-dioxo-2,3-dihydro-1λ⁶,5-benzothiazepin-4-one;
(3R)-3-Amino-5-[(4-chlorophenyl)methyl]-8-fluoro-7-(5-isopropyl-1,3,4-oxadiazol-2-yl)-1,1-dioxo-2,3-dihydro-1λ⁶,5-benzothiazepin-4-one;
(3R)-3-Amino-5-[(4-chlorophenyl)methyl]-7-(5-cyclopropyl-1,3,4-oxadiazol-2-yl)-8-fluoro-1,1-dioxo-2,3-dihydro-1λ⁶5-benzothiazepin-4-one;
(3R)-3-amino-5-[(4-chlorophenyl)methyl]-8-fluoro-7-[5-(2-hydroxy-1,1-dimethyl-ethyl)-1,3,4-oxadiazol-2-yl]-1,1-dioxo-2,3-dihydro-1λ⁶,5-benzothiazepin-4-one;
(3R)-3-amino-7-[5-(azetidin-1-yl)-1,3,4-oxadiazol-2-yl]-5-[(4-chlorophenyl)methyl]-8-fluoro-1,1-dioxo-2,3-dihydro-1λ⁶5-benzothiazepin-4-one;
(3R)-3-amino-5-[(4-chlorophenyl)methyl]-7-(4-ethyloxazol-2-yl)-8-fluoro-1,1-dioxo-2,3-dihydro-1λ⁶,5-benzothiazepin-4-one;
(3R)-3-amino-5-[(4-chlorophenyl)methyl]-6-fluoro-1,1-dioxo-7-[5-(2,2,2-trifluoroethyl)-1,3,4-oxadiazol-2-yl]-2,3-dihydro-1λ⁶,5-benzothiazepin-4-one;
(3R)-3-amino-7-(5-tert-butyl-1,3,4-oxadiazol-2-yl)-5-[(3-chlorophenyl)methyl]-8-fluoro-1,1-dioxo-2,3-dihydro-1λ⁶,5-benzothiazepin-4-one;
4-[[(3R)-3-amino-7-(5-tert-butyl-1,3,4-oxadiazol-2-yl)-8-fluoro-1,1,4-trioxo-2,3-dihydro-1λ⁶,5-benzothiazepin-5-yl]methyl]benzonitrile;
(3R)-3-amino-7-(5-tert-butyl-1,3,4-oxadiazol-2-yl)-8-fluoro-1, 1-dioxo-5-[(4-tetrahydrofuran-3-yloxyphenyl)methyl]-2,3-dihydro-1λ⁶,5-benzothiazepin-4-one;
(3R)-3-amino-7-(5-tert-butyl-1,3,4-oxadiazol-2-yl)-5-[[4-(2,2-difluoroethoxy)phenyl]methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-5-[[4-(difluoromethoxy)phenyl]methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-1,1-dioxo-5-(2-pyridylmethyl)-2,3-dihydro-1λ⁶,5-benzothiazepin-4-one;
(3R)-3-amino-7-(5-tert-butyl-1,3,4-oxadiazol-2-yl)-8-fluoro-5-[(6-methoxy-3-pyridyl)methyl]-1,1-dioxo-2,3-dihydro-1λ⁶,5-benzothiazepin-4-one;
(3R)-3-amino-5-[[4-(aminomethyl)phenyl]methyl]-7-(5-tert-butyl-1,3,4-oxadiazol-2-yl)-8-fluoro-1,1-dioxo-2,3-dihydro-1λ⁶,5-benzothiazepin-4-one;
(3R)-3-amino-7-(5-tert-butyl-1,3,4-oxadiazol-2-yl)-5-[(2-chlorophenyl)methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(5-methoxy-2-pyridyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-methylsulfonylphenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-methoxyphenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-fluorophenyl)methyl]-1,1-dioxo-2,3-dihydro-1λ⁶,5-benzothiazepin-4-one;
(3R)-3-amino-5-[[2-(aminomethyl)-4-chloro-phenyl]methyl]-7-(5-tert-butyl-1,3,4-oxadiazol-2-yl)-8-fluoro-1,1-dioxo-2,3-dihydro-1λ⁶,5-benzothiazepin-4-one;
(3R)-3-amino-5-[[4-(11-aminoundecoxy)phenyl]methyl]-7-(5-tert-butyl-1,3,4-oxadiazol-2-yl)-8-fluoro-1,1-dioxo-2,3-dihydro-1λ⁶,5-benzothiazepin-4-one;
N-[(3R)-7-(5-tert-butyl-1,3,4-oxadiazol-2-yl)-5-[(4-chlorophenyl)methyl]-8-fluoro-1,1,4-trioxo-2,3-dihydro-1 *λ⁶*,5,5-benzothiazepin-3-yl]acetamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-hydroxy-butanamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide;
(2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)butanamide;
3-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)acetamide;
2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]acetamide;
4-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide;
(3R)-3-amino-8-bromo-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-methyl-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-hydroxy-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-(dimethylamino)-1,1-dioxo-2,3-dihydro-1λ⁶,5-benzothiazepin-4-one;
(3R)-3,8-diamino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-(1-tert-butylpyrazol-4-yl)-5-[(4-chlorophenyl)methyl]-8-fluoro-1,1-dioxo-2,3-dihydro-1λ⁶,5-benzothiazepin-4-one;
((3R)-3-amino-5-[(4-chlorophenyl)methyl]-7-(1-ethylpyrazol-4-yl)-8-fluoro-1,1-dioxo-2,3-dihydro-1λ⁶,5-benzothiazepin-4-one;
(3R)-3-amino-5-[(4-chlorophenyl)methyl]-7-(1-ethylpyrazol-3-yl)-8-fluoro-1,1-dioxo-2,3-dihydro-1λ⁶,5-benzothiazepin-4-one;
(3R)-3-amino-7-(4-tert-butylpyrazol-1-yl)-5-[(4-chlorophenyl)methyl]-8-fluoro-1,1-dioxo-2,3-dihydro-1λ⁶,5-benzothiazepin-4-one;
(3R)-3-amino-7-(3-tert-butylpyrazol-1-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(4-ethylpyrazol-1-yl)-8-fluoro-1,1-dioxo-2,3-dihydro-1λ⁶,5-benzothiazepin-4-one;
(3R)-3-amino-5-[(4-chlorophenyl)methyl]-7-(1-ethyl-1,2,4-triazol-3-yl)-8-fluoro-1,1-dioxo-2,3-dihydro-1λ⁶,5-benzothiazepin-4-one;
(3R)-3-amino-7-(1-tert-butyl-1,2,4-triazol-3-yl)-5-[(4-chlorophenyl)methyl]-8-fluoro-1,1-dioxo-2,3-dihydro-1λ⁶,5-benzothiazepin-4-one;
(3R)-3-amino-7-(1-tert-butylpyrazol-3-yl)-5-[(4-chlorophenyl)methyl]-8-fluoro-1,1-dioxo-2,3-dihydro-1λ⁶,5-benzothiazepin-4-one;
(3R)-3-amino-7-(5-tert-butyl-1,2,4-oxadiazol-3-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(5-ethyl-1,2,4-oxadiazol-3-yl)-8-fluoro-1,1-dioxo-2,3-dihydro-1λ⁶,5-benzothiazepin-4-one;
(3R)-3-amino-7-[5-(1,1-dimethylbut-3-ynyl)-1,3,4-oxadiazol-2-yl]-8-fluoro-1,1-dioxo-5-[[4-[3-(trifluoromethyl)diazirin-3-yl]phenyl]methyl]-2,3-dihydro-1λ⁶,5-benzothiazepin-4-one;
(3R)-3-amino-7-(5-tert-butyl-1,3,4-oxadiazol-2-yl)-1,1-dioxo-5-[[4-(trifluoromethoxy)phenyl]methyl]-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-[5-(1,1-dimethylbut-3-ynyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
3R)-3-amino-5-[(4-chlorophenyl)methyl]-8-fluoro-7-(5-morpholino-1,3,4-oxadiazol-2-yl)-1,1-dioxo-2,3-dihydro-1λ⁶,5-benzothiazepin-4-one;
(3R)-3-amino-5-[(4-chlorophenyl)methyl]-7-[5-(cyclopentylamino)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-thiadiazol-2-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(5-ethyltetrazol-2-yl)-8-fluoro-1,1-dioxo-2,3-dihydro-1λ⁶,5-benzothiazepin-4-one;
or a pharmaceutically acceptable salt thereof.

In a particularembodiment, there is provided a compound of formula (I) as described herein, selected from:
(3R)-3-amino-5-[(4-chlorophenyl)methyl]-7-(4-ethyltriazol-1-yl)-8-fluoro-2,3-dihydro-1,5-benzothiazepin-4-one;
(3R)-3-amino-5-[(4-chlorophenyl)methyl]-7-[4-[(4-chlorophenyl)methyl]triazol-1-yl]-8-fluoro-2,3-dihydro-1, 5-benzothiazepin-4-one;
(3R)-3-amino-5-[(4-chlorophenyl)methyl]-7-(4-ethyltriazol-1-yl)-8-fluoro-1,1-dioxo-2,3-dihydro-1λ⁶,5-benzothiazepin-4-one;
(3R)-3-amino-5-[(4-chlorophenyl)methyl]-7-[4-[(4-chlorophenyl)methyl]triazol-1-yl]-8-fluoro-1,1-dioxo-2,3-dihydro-1λ⁶,5-benzothiazepin-4-one;
(3R)-3-amino-7-(4-tert-butyltriazol-1-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(1-ethyltriazol-4-yl)-8-fluoro-1,1-dioxo-2,3-dihydro-1λ⁶,5-benzothiazepin-4-one;
(3R)-3-amino-5-[(4-chlorophenyl)methyl]-7-(2-ethyltetrazol-5-yl)-8-fluoro-1,1-dioxo-2,3-dihydro-1λ⁶,5-benzothiazepin-4-one;
(3R)-3-amino-7-(2-tert-butyltetrazol-5-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-8-fluoro-1,1-dioxo-7-(1H-pyrazol-5-yl)-2,3-dihydro-1λ⁶,5-benzothiazepin-4-one;
(3R)-3-amino-5-[(4-chlorophenyl)methyl]-7-(5-ethyl-1H-1,2,4-triazol-3-yl)-8-fluoro-1,1-dioxo-2,3-dihydro-1λ⁶,5-benzothiazepin-4-one;
(3R)-3-amino-5-[(4-chlorophenyl)methyl]-1,1-dioxo-7-[3-(2,2,2-trifluoroethyl)-1,2,4-oxadiazol-5-yl]-2,3-dihydropyrido[3,2-b][1,4]thiazepin-4-one;
(3R)-3-amino-5-[(4-chlorophenyl)methyl]-7-(3-cyclopropyl-1,2,4-oxadiazol-5-yl)-8-fluoro-1,1-dioxo-2,3-dihydro-1λ⁶,5-benzothiazepin-4-one;
(3R)-3-Amino-5-[(4-chlorophenyl)methyl]-7-(3-ethyl-1,2,4-oxadiazol-5-yl)-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-(3-propyl-1,2,4-oxadiazol-5-yl)-2,3-dihydro-1λ⁶,5-benzothiazepin-4-one;
(3R)-3-Amino-7-(3-tert-butyl-1,2,4-oxadiazol-5-yl)-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-7-[3-[(4-chlorophenyl)methyl]-1,2,4-oxadiazol-5-yl]-8-fluoro-1,1-dioxo-2,3-dihydro-1λ⁶,5-benzothiazepin-4-one;
(3R)-3-Amino-7-[3-(benzyloxymethyl)-1,2,4-oxadiazol-5-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-8-fluoro-1,1-dioxo-7-[3-(2,2,2-trifluoroethyl)-1,2,4-oxadiazol-5-yl]-2,3-dihydro-1λ⁶,5-benzothiazepin-4-one;
(3R)-3-amino-7-[3-(aminomethyl)-1,2,4-oxadiazol-5-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(3-ethyl-1,2,4-oxadiazol-5-yl)-1,1-dioxo-2,3-dihydro-1λ⁶,5-benzothiazepin-4-one;
(3R)-3-amino-7-(3-tert-butylisoxazol-5-yl)-5-[(4-chlorophenyl)methyl]-8-fluoro-1,1-dioxo-2,3-dihydro-1λ⁶,5-benzothiazepin-4-one;
(3R)-3-amino-7-[5-(2,2-difluorocyclohexyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-amino-7-(5-tert-butyl-1,3,4-oxadiazol-2-yl)-8-fluoro-5-[[4-(trifluoromethoxy)phenyl]methyl]-2,3-dihydro-1,5-benzothiazepin-4-one;
(3R)-3-Amino-7-[5-(3,3-difluorocyclohexyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-Amino-8-fluoro-1,1-dioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-4-one;
(3R)-3-Amino-7-(5-tert-butyl-1,3,4-oxadiazol-2-yl)-8-fluoro-1,1-dioxo-5-[[4-(trifluoromethoxy)phenyl]methyl]-2,3-dihydro-1λ⁶,5-benzothiazepin-4-one;
(2S)-N-[(3R)-8-fluoro-1,1,4-trioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-3-yl]-2-(methylamino)propanamide;
(3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-[5-(3,3,3-trifluoropropyl)-1,3,4-oxadiazol-2-yl]-2,3-dihydro-1λ⁶,5-benzothiazepin-4-one;
(3R)-3-Amino-5-[(4-chlorophenyl)methyl]-8-fluoro-1,1-dioxo-7-[5-(2,2,2-trifluoroethyl)-1,3,4-oxadiazol-2-yl]-2,3-dihydro-1λ⁶,5-benzothiazepin-4-one;
(3R)-3-Amino-7-[5-(1-amino-1-methyl-ethyl)-1,3,4-oxadiazol-2-yl]-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-8-fluoro-7-[5-(1-fluoro-1-methyl-ethyl)-1,3,4-oxadiazol-2-yl]-1,1-dioxo-2,3-dihydro-1λ⁶,5-benzothiazepin-4-one;
(3R)-3-Amino-5-[(4-chlorophenyl)methyl]-7-[5-(2,2-difluoroethyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-(5-ethyl-1,3,4-oxadiazol-2-yl)-8-fluoro-1,1-dioxo-2,3-dihydro-1λ⁶,5-benzothiazepin-4-one;
(3R)-3-Amino-5-[(4-chlorophenyl)methyl]-7-[5-(4,4-difluorocyclohexyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclohexyl)-1,3,4-oxadiazol-2-yl]-8-fluoro-1,1-dioxo-2,3-dihydro-1λ⁶,5-benzothiazepin-4-one:
(3R)-3-Amino-5-[(4-chlorophenyl)methyl]-7-[5-(3,3-difluorocyclopentyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclopentyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-[(3,3-difluorocyclobutyl)methyl]-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-[5-(tetrahydropyran-4-ylmethyl)-1,3,4-oxadiazol-2-yl]-2,3-dihydro-1λ⁶,5-benzothiazepin-4-one;
(3R)-3-Amino-5-[(4-chlorophenyl)methyl]-8-fluoro-1,1-dioxo-7-[5-(4,4,4-trifluorobutyl)-1,3,4-oxadiazol-2-yl]-2,3-dihydro-1λ⁶,5-benzothiazepin-4-one;
(3R)-3-Amino-5-[(4-chlorophenyl)methyl]-7-[5-(3,3-difluorocyclobutyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclobutyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-(5-tetrahydropyran-4-yl-1,3,4-oxadiazol-2-yl)-2,3-dihydro-1λ⁶,5-benzothiazepin-4-one;
(3R)-3-Amino-5-[(4-chlorophenyl)methyl]-8-fluoro-7-[5-(2-fluoroethyl)-1,3,4-oxadiazol-2-yl]-1,1-dioxo-2,3-dihydro-1λ⁶,5-benzothiazepin-4-one;
(3R)-3-Amino-5-[(4-chlorophenyl)methyl]-8-fluoro-7-(5-isopropyl-1,3,4-oxadiazol-2-yl)-1,1-dioxo-2,3-dihydro-1λ⁶,5-benzothiazepin-4-one;
(3R)-3-Amino-5-[(4-chlorophenyl)methyl]-7-(5-cyclopropyl-1,3,4-oxadiazol-2-yl)-8-fluoro-1,1-dioxo-2,3-dihydro-1λ⁶,5-benzothiazepin-4-one;
(3R)-3-amino-5-[(4-chlorophenyl)methyl]-8-fluoro-7-[5-(2-hydroxy-1,1-dimethyl-ethyl)-1,3,4-oxadiazol-2-yl]-1,1-dioxo-2,3-dihydro-1λ⁶,5-benzothiazepin-4-one;
(3R)-3-amino-7-[5-(azetidin-1-yl)-1,3,4-oxadiazol-2-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(4-ethyloxazol-2-yl)-8-fluoro-1,1-dioxo-2,3-dihydro-1λ⁶,5-benzothiazepin-4-one;
(3R)-3-amino-5-[(4-chlorophenyl)methyl]-6-fluoro-1,1-dioxo-7-[5-(2,2,2-trifluoroethyl)-1,3,4-oxadiazol-2-yl]-2,3-dihydro-1λ⁶,5-benzothiazepin-4-one;
(3R)-3-amino-7-(5-tert-butyl-1,3,4-oxadiazol-2-yl)-5-[(3-chlorophenyl)methyl]-8-fluoro-1,1-dioxo-2,3-dihydro-1λ⁶,5-benzothiazepin-4-one;
4-[[(3R)-3-amino-7-(5-tert-butyl-1,3,4-oxadiazol-2-yl)-8-fluoro-1,1,4-trioxo-2,3-dihydro-1λ⁶,5-benzothiazepin-5-yl]methyl]benzonitrile;
(3R)-3-amino-7-(5-tert-butyl-1,3,4-oxadiazol-2-yl)-8-fluoro-1,1-dioxo-5-[(4-tetrahydrofuran-3-yloxyphenyl)methyl]-2,3-dihydro-1λ⁶,5-benzothiazepin-4-one;
(3R)-3-amino-7-(5-tert-butyl-1,3,4-oxadiazol-2-yl)-5-[[4-(2,2-difluoroethoxy)phenyl]methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-5-[[4-(difluoromethoxy)phenyl]methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-1,1-dioxo-5-(2-pyridylmethyl)-2,3-dihydro-1λ⁶,5-benzothiazepin-4-one;
(3R)-3-amino-7-(5-tert-butyl-1,3,4-oxadiazol-2-yl)-8-fluoro-5-[(6-methoxy-3-pyridyl)methyl]-1,1-dioxo-2,3-dihydro-1λ⁶,5-benzothiazepin-4-one;
(3R)-3-amino-5-[[4-(aminomethyl)phenyl]methyl]-7-(5-tert-butyl-1,3,4-oxadiazol-2-yl)-8-fluoro-1,1-dioxo-2,3-dihydro-1λ⁶,5-benzothiazepin-4-one;
(3R)-3-amino-7-(5-tert-butyl-1,3,4-oxadiazol-2-yl)-5-[(2-chlorophenyl)methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(5-methoxy-2-pyridyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-methylsulfonylphenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-methoxyphenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-fluorophenyl)methyl]-1,1-dioxo-2,3-dihydro-1λ⁶,5-benzothiazepin-4-one;
(3R)-3-amino-5-[[2-(aminomethyl)-4-chloro-phenyl]methyl]-7-(5-tert-butyl-1,3,4-oxadiazol-2-yl)-8-fluoro-1,1-dioxo-2,3-dihydro-1λ⁶,5-benzothiazepin-4-one;
(3R)-3-amino-5-[[4-(11-aminoundecoxy)phenyl]methyl]-7-(5-tert-butyl-1,3,4-oxadiazol-2-yl)-8-fluoro-1,1-dioxo-2,3-dihydro-1λ⁶,5-benzothiazepin-4-one;
N-[(3R)-7-(5-tert-butyl-1,3,4-oxadiazol-2-yl)-5-[(4-chlorophenyl)methyl]-8-fluoro-1,1,4-trioxo-2,3-dihydro-1 *λ*⁶,5,5-benzothiazepin-3-yl]acetamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-hydroxy-butanamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide;
(2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
(2S5)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)butanamide;
3-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)acetamide;
2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]acetamide;
4-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide;
(3R)-3-amino-8-bromo-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-methyl-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-hydroxy-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-(dimethylamino)-1,1-dioxo-2,3-dihydro-1λ⁶,5-benzothiazepin-4-one;
(3R)-3,8-diamino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-(1-tert-butylpyrazol-4-yl)-5-[(4-chlorophenyl)methyl]-8-fluoro-1,1-dioxo-2,3-dihydro-1λ⁶,5-benzothiazepin-4-one;
((3R)-3-amino-5-[(4-chlorophenyl)methyl]-7-(1-ethylpyrazol-4-yl)-8-fluoro-1,1-dioxo-2,3-dihydro-1λ⁶,5-benzothiazepin-4-one;
(3R)-3-amino-5-[(4-chlorophenyl)methyl]-7-(1-ethylpyrazol-3-yl)-8-fluoro-1,1-dioxo-2,3-dihydro-1λ⁶,5-benzothiazepin-4-one;
(3R)-3-amino-7-(4-tert-butylpyrazol-1-yl)-5-[(4-chlorophenyl)methyl]-8-fluoro-1,1-dioxo-2,3-dihydro-1λ⁶,5-benzothiazepin-4-one;
(3R)-3-amino-7-(3-tert-butylpyrazol-1-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(4-ethylpyrazol-1-yl)-8-fluoro-1,1-dioxo-2,3-dihydro-1λ⁶,5-benzothiazepin-4-one;
(3R)-3-amino-5-[(4-chlorophenyl)methyl]-7-(1-ethyl-1,2,4-triazol-3-yl)-8-fluoro-1,1-dioxo-2,3-dihydro-1λ⁶,5-benzothiazepin-4-one;
(3R)-3-amino-7-(1-tert-butyl-1,2,4-triazol-3-yl)-5-[(4-chlorophenyl)methyl]-8-fluoro-1,1-dioxo-2,3-dihydro-1λ⁶,5-benzothiazepin-4-one;
(3R)-3-amino-7-(1-tert-butylpyrazol-3-yl)-5-[(4-chlorophenyl)methyl]-8-fluoro-1,1-dioxo-2,3-dihydro-1λ⁶,5-benzothiazepin-4-one;
(3R)-3-amino-7-(5-tert-butyl-1,2,4-oxadiazol-3-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(5-ethyl-1,2,4-oxadiazol-3-yl)-8-fluoro-1,1-dioxo-2,3-dihydro-1λ⁶,5-benzothiazepin-4-one;
(3R)-3-amino-7-[5-(1,1-dimethylbut-3-ynyl)-1,3,4-oxadiazol-2-yl]-8-fluoro-1,1-dioxo-5-[[4-[3-(trifluoromethyl)diazirin-3-yl]phenyl]methyl]-2,3-dihydro-1λ⁶,5-benzothiazepin-4-one;
(3R)-3-amino-7-(5-tert-butyl-1,3,4-oxadiazol-2-yl)-1,1-dioxo-5-[[4-(trifluoromethoxy)phenyl]methyl]-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-[5-(1,1-dimethylbut-3-ynyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
3R)-3-amino-5-[(4-chlorophenyl)methyl]-8-fluoro-7-(5-morpholino-1,3,4-oxadiazol-2-yl)-1,1-dioxo-2,3-dihydro-1λ⁶,5-benzothiazepin-4-one;
(3R)-3-amino-5-[(4-chlorophenyl)methyl]-7-[5-(cyclopentylamino)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-thiadiazol-2-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(5-ethyltetrazol-2-yl)-8-fluoro-1,1-dioxo-2,3-dihydro-1λ⁶,5-benzothiazepin-4-one;
or a pharmaceutically acceptable salt thereof.

In a most particular embodiment, there is provided a compound of formula (I) as described herein, selected from:
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[3.1.1]heptane-3-carboxylate
2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]acetamide
(3R)-3-amino-5-[(4-chlorophenyl)methyl]-8-fluoro-7-[5-(3-oxa-8-azabicyclo[3.2.1]octan-8-yl)-1,2,4-oxadiazol-3-yl]-1,1-dioxo-2,3-dihydro-1lambda6,5-benzothiazepin-4-one;hydrochloride
2-amino-N-[(3R)-7-(2-tert-butyltetrazol-5-yl)-5-[(4-chlorophenyl)methyl]-8-fluoro-1,1,4-trioxo-2,3-dihydro-1lambda6,5-benzothiazepin-3-yl]acetamide
(3R)-3-amino-5-[(4-chlorophenyl)methyl]-7-[5-[(3,3-difluorocyclobutyl)amino]-1,2,4-oxadiazol-3-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-1-imino-1-oxo-5-[[4-(trifluoromethoxy)phenyl]methyl]-2,3-dihydro-1lambda6,5-benzothiazepin-4-one;hydrochloride
(2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]propanamide
(3R)-3-amino-7-(2-tert-butyltetrazol-5-yl)-5-[(4-chlorophenyl)methyl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-1,1-dioxo-5-[[4-(1,1,2,2-tetrafluoroethoxy)phenyl]methyl]-2,3-dihydro-1lambda6,5-benzothiazepin-4-one;hydrochloride
(3R)-3-amino-8-fluoro-1,1-dioxo-5-[[4-(trifluoromethoxy)phenyl]methyl]-7-[5-[1-(trifluoromethyl)cyclopropyl]-1,2,4-oxadiazol-3-yl]-2,3-dihydro-1lambda6,5-benzothiazepin-4-one
(3R)-3-amino-7-(5-tert-butyl-1,2,4-oxadiazol-3-yl)-5-[(4-chlorophenyl)methyl]-8-fluoro-1,1-dioxo-2,3-dihydro-1lambda6,5-benzothiazepin-4-one
(3R)-3-amino-7-[5-(2,2-difluoromorpholin-4-yl)-1,3,4-oxadiazol-2-yl]-1,1-dioxo-5-[(4-phenoxyphenyl)methyl]-2,3-dihydro-1lambda6,5-benzothiazepin-4-one;hydrochloride
(3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1lambda6,5-benzothiazepin-4-one;formic acid
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1lambda4,5-benzothiazepin-4-one;hydrochloride
(3R)-3-amino-5-[(4-chlorophenyl)methyl]-7-[5-[(3,3-difluorocyclobutyl)amino]-1,3,4-oxadiazol-2-yl]-8-fluoro-1,1-dioxo-2,3-dihydro-1lambda6,5-benzothiazepin-4-one;hydrochloride
(2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]butanamide
(3R)-3-amino-8-fluoro-1,1-dioxo-7-[5-(2,2,2-trifluoroethyl)-1,3,4-oxadiazol-2-yl]-5-[[4-(trifluoromethoxy)phenyl]methyl]-2,3-dihydro-1lambda6,5-benzothiazepin-4-one;hydrochloride
(3R)-3-amino-5-[(4-chlorophenyl)methyl]-7-[5-[(3,3-difluoro-1-methylcyclobutyl)amino]-1,3,4-oxadiazol-2-yl]-8-fluoro-1,1-dioxo-2,3-dihydro-1lambda6,5-benzothiazepin-4-one; hydrochloride
2-[4-[[(3R)-3-amino-7-(5-tert-butyl-1,3,4-oxadiazol-2-yl)-1,1,4-trioxo-2,3-dihydro-1lambda6,5-benzothiazepin-5-yl]methyl]phenyl]-2-methyl-propanenitrile
(3R)-3-amino-7-(3-tert-butyl-1,2,4-oxadiazol-5-yl)-5-[(4-chlorophenyl)methyl]-8-fluoro-1,1-dioxo-2,3-dihydro-1lambda6,5-benzothiazepin-4-one;hydrochloride
(3R)-3-amino-7-(5-tert-butyl-1,3,4-oxadiazol-2-yl)-5-[(4-chlorophenyl)methyl]-8-fluoro-1-oxo-2,3-dihydro-1lambda4,5-benzothiazepin-4-one
(3R)-3-amino-5-[(4-chlorophenyl)methyl]-7-[5-(5,5-difluoro-1-methyl-3-piperidyl)-1,2,4-oxadiazol-3-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1lambda4,5-benzothiazepin-4-one;hydrochloride
(3R)-3-amino-7-(5-tert-butyl-1,3,4-oxadiazol-2-yl)-8-fluoro-1,1-dioxo-5-[[4-(trifluoromethoxy)phenyl]methyl]-2,3-dihydro-1lambda6,5-benzothiazepin-4-one;hydrochloride
(3R)-3-amino-7-(2-tert-butyltetrazol-5-yl)-8-fluoro-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-8-fluoro-7-[5-(o-tolyl)-1,3,4-oxadiazol-2-yl]-1,1-dioxo-2,3-dihydro-1lambda6,5-benzothiazepin-4-one
(3R)-3-amino-7-(5-tert-butyl-1,3,4-oxadiazol-2-yl)-8-fluoro-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-7-[5-(cyclobutylamino)-1,3,4-oxadiazol-2-yl]-8-fluoro-1,1-dioxo-2,3-dihydro-1lambda6,5-benzothiazepin-4-one;hydrochloride
2-[5-[(3R)-3-amino-8-fluoro-1,1,4-trioxo-5-[[4-(trifluoromethoxy)phenyl]methyl]-2,3-dihydro-1lambda6,5-benzothiazepin-7-yl]-1,3,4-oxadiazol-2-yl]-2-methylpropanenitrile;hydrochloride
(3R)-3-amino-7-(5-tert-butyl-1,3,4-oxadiazol-2-yl)-8-fluoro-5-[(6-isopropoxy-3-pyridyl)methyl]-1,1-dioxo-2,3-dihydro-1lambda6,5-benzothiazepin-4-one;hydrochloride
(3R)-3-amino-5-[(4-chlorophenyl)methyl]-8-fluoro-1,1-dioxo-7-[3-(2,2,2-trifluoroethyl)-1,2,4-oxadiazol-5-yl]-2,3-dihydro-1lambda6,5-benzothiazepin-4-one;hydrochloride
(3R)-3-amino-5-[(4-chlorophenyl)methyl]-8-fluoro-7-[5-(6-fluoro-2-methyl-3-pyridyl)-1,2,4-oxadiazol-3-yl]-1,1-dioxo-2,3-dihydro-1lambda6,5-benzothiazepin-4-one
(3R)-3-amino-1,1-dioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1lambda6,5-benzothiazepin-4-one;hydrochloride
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1,1-dioxo-2,3-dihydro-1lambda6,5-benzothiazepin-4-one;hydrochloride
(3R)-3-amino-5-[(4-chlorophenyl)methyl]-7-(5-ethyl-1,3,4-oxadiazol-2-yl)-8-fluoro-1,1-dioxo-2,3-dihydro-1lambda6,5-benzothiazepin-4-one
(3R)-3-amino-7-[5-(1-amino-2,2,2-trifluoro-1-methyl-ethyl)-1,2,4-oxadiazol-3-yl]-8-fluoro-1,1-dioxo-5-[[4-(trifluoromethoxy)phenyl]methyl]-2,3-dihydro-1lambda6,5-benzothiazepin-4-one; hydrochloride
(3R)-3-amino-7-(5-tert-butyl-1,3,4-oxadiazol-2-yl)-8-fluoro-5-[(4-methoxyphenyl)methyl]-1,1-dioxo-2,3-dihydro-1lambda6,5-benzothiazepin-4-one;1,1,1,3,3,3-hexafluoropropan-2-ol;hydrochloride
(3R)-3-amino-5-[(4-chlorophenyl)methyl]-8-fluoro-7-(5-morpholino-1,2,4-oxadiazol-3-yl)-1,1-dioxo-2,3-dihydro-1lambda6,5-benzothiazepin-4-one;hydrochloride
(3R)-3-amino-7-[5-[1-amino-2,2,2-trifluoro-1-(4-fluorophenyl)ethyl]-1,2,4-oxadiazol-3-yl]-5-[(4-chlorophenyl)methyl]-8-fluoro-1,1-dioxo-2,3-dihydro-1lambda6,5-benzothiazepin-4-one;hydrochloride
(3R)-3-amino-5-[(4-chlorophenyl)methyl]-7-[5-[methyl(2,2,2-trifluoroethyl)amino]-1,3,4-oxadiazol-2-yl]-1,1-dioxo-2,3-dihydro-1lambda6,5-benzothiazepin-4-one;hydrochloride
(3R)-3-amino-5-[(4-chlorophenyl)methyl]-7-(5-ethyltetrazol-2-yl)-8-fluoro-1,1-dioxo-2,3-dihydro-1lambda6,5-benzothiazepin-4-one
(3R)-3-amino-5-[(4-chlorophenyl)methyl]-7-[5-(2,2-difluoromorpholin-4-yl)-1,2,4-oxadiazol-3-yl]-8-fluoro-1,1-dioxo-2,3-dihydro-1lambda6,5-benzothiazepin-4-one
(3R)-3-amino-7-[5-(tert-butylamino)-1,3,4-oxadiazol-2-yl]-5-[(4-chlorophenyl)methyl]-8-fluoro-1,1-dioxo-2,3-dihydro-1lambda6,5-benzothiazepin-4-one;hydrochloride
(3R)-3-amino-5-[(4-chlorophenyl)methyl]-8-fluoro-7-(5-morpholino-1,3,4-oxadiazol-2-yl)-1,1-dioxo-2,3-dihydro-1lambda6,5-benzothiazepin-4-one;1,1,1,3,3,3-hexafluoropropan-2-ol;hydrochloride
(3R)-3-amino-7-(5-tert-butyl-1,3,4-oxadiazol-2-yl)-5-[(4-chlorophenyl)methyl]-8-fluoro-1,1-dioxo-2,3-dihydro-1lambda6,5-benzothiazepin-4-one;hydrochloride
(3R)-3-amino-5-[(4-chlorophenyl)methyl]-7-[5-(cyclohexylamino)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;hydrochloride
(3R)-3-amino-7-(1-tert-butyl-1,2,4-triazol-3-yl)-5-[(4-chlorophenyl)methyl]-8-fluoro-1,1-dioxo-2,3-dihydro-1lambda6,5-benzothiazepin-4-one
methyl 4-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl-4-methyl-piperidine-1-carboxylate;hydrochloride
(3R)-3-amino-7-(5-tert-butyl-1,3,4-oxadiazol-2-yl)-8-fluoro-5-[[4-(trifluoromethoxy)phenyl]methyl]-2,3-dihydro-1,5-benzothiazepin-4-one;hydrochloride
(3R)-3-amino-5-[(4-chlorophenyl)methyl]-7-(3-ethyl-1,2,4-oxadiazol-5-yl)-8-fluoro-1,1-dioxo-2,3-dihydro-1lambda6,5-benzothiazepin-4-one;hydrochloride
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1lambda4,5-benzothiazepin-4-one; hydrochloride
(3R)-3-amino-7-(5-tert-butyl-1,3,4-oxadiazol-2-yl)-5-[(4-chloro-3-fluorophenyl)methyl]-8-fluoro-1,1-dioxo-2,3-dihydro-1lambda6,5-benzothiazepin-4-one;hydrochloride
(3R)-3-amino-7-(5-tert-butyl-1,3,4-oxadiazol-2-yl)-8-fluoro-5-[(4-isopropoxyphenyl)methyl]-1,1-dioxo-2,3-dihydro-1lambda6,5-benzothiazepin-4-one;hydrochloride
(3R)-3-amino-5-[(4-chlorophenyl)methyl]-1,1-dioxo-7-[5-[2-(trifluoromethyl)morpholin-4-yl]-1,3,4-oxadiazol-2-yl]-2,3-dihydro-1lambda6,5-benzothiazepin-4-one
(3R)-3-amino-7-[5-(3,3-difluorocyclohexyl)-1,3,4-oxadiazol-2-yl]-8-fluoro-1,1-dioxo-5-[4-(trifluoromethoxy)phenyl]methyl]-2,3-dihydro-1lambda6,5-benzothiazepin-4-one; 1,1,1,3,3,3-hexafluoropropan-2-ol
(3R)-3-amino-5-[(4-chlorophenyl)methyl]-7-[5-(2,2-difluoromorpholin-4-yl)-1,3,4-oxadiazol-2-yl]-1,1-dioxo-2,3-dihydro-1lambda6,5-benzothiazepin-4-one;hydrochloride
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1,1-dioxo-2,3-dihydro-1lambda6,5-benzothiazepin-4-one; hydrochloride
(3R)-3-amino-7-[5-(1,1-dimethylbut-3-ynyl)-1,3,4-oxadiazol-2-yl]-8-fluoro-1,1-dioxo-5-[4-(trifluoromethoxy)phenyl]methyl]-2,3-dihydro-1lambda6,5-benzothiazepin-4-one
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1-oxo-2,3-dihydro-1lambda4,5-benzothiazepin-4-one;hydrochloride
(3R)-3-amino-5-[(4-chlorophenyl)methyl]-7-[5-(3,3-difluorocyclobutyl)-1,3,4-oxadiazol-2-yl]-8-fluoro-1,1-dioxo-2,3-dihydro-1lambda6,5-benzothiazepin-4-one;hydrochloride
(3R)-3-amino-5-[(4-chlorophenyl)methyl]-8-fluoro-7-(5-isobutyl-1,3,4-oxadiazol-2-yl)-1,1-dioxo-2,3-dihydro-1lambda6,5-benzothiazepin-4-one
methyl 5-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3,3-difluoro-piperidine-1-carboxylate
(3R)-3-amino-5-[(4-chlorophenyl)methyl]-7-[5-(3,3-difluorocyclopentyl)-1,3,4-oxadiazol-2-yl]-8-fluoro-1,1-dioxo-2,3-dihydro-1lambda6,5-benzothiazepin-4-one;hydrochloride
(3R)-3-amino-5-[(4-chlorophenyl)methyl]-8-fluoro-1,1-dioxo-7-[5-[2-(trifluoromethyl)morpholin-4-yl]-1,2,4-oxadiazol-3-yl]-2,3-dihydro-1lambda6,5-benzothiazepin-4-one;hydrochloride
(3R)-3-amino-5-[(4-chlorophenyl)methyl]-7-[5-(2,2-difluorospiro[3.3]heptan-6-yl)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;hydrochloride
(3R)-3-amino-5-[(4-chlorophenyl)methyl]-7-[5-(3,3-difluorocyclohexyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(2,2-difluorocyclohexyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[5,5-difluoro-1-(2-methoxyethyl)-3-piperidyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(1-ethyl-5,5-difluoro-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(2-cyclopropyltetrahydrofuran-2-yl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(3-fluoro-1-methyl-3-piperidyl)-1,3,4-oxadiazol-2-yl]-1,1-dioxo-2,3-dihydro-1lambda6,5-benzothiazepin-4-one;hydrochloride
(3R)-3-amino-5-[(4-chlorophenyl)methyl]-8-fluoro-7-[5-(6-fluoro-2-methyl-3-pyridyl)-1,3,4-oxadiazol-2-yl]-1,1-dioxo-2,3-dihydro-1lambda6,5-benzothiazepin-4-one;hydrochloride
(3R)-3-amino-7-(5-tert-butyl-1,2,4-oxadiazol-3-yl)-5-[[4-(cyclopentoxy)phenyl]methyl]-8-fluoro-1,1-dioxo-2,3-dihydro-1lambda6,5-benzothiazepin-4-one;hydrochloride
(3R)-3-amino-5-[(4-chlorophenyl)methyl]-8-fluoro-7-[5-(2-fluoro-6-methyl-phenyl)-1,3,4-oxadiazol-2-yl]-1,1-dioxo-2,3-dihydro-1lambda6,5-benzothiazepin-4-one;1,1,1,3,3,3-hexafluoropropan-2-ol;hydrochloride
(3R)-3-amino-7-(5-tert-butyl-1,3,4-oxadiazol-2-yl)-5-[[4-(cyclopentoxy)phenyl]methyl]-8-fluoro-1,1-dioxo-2,3-dihydro-1lambda6,5-benzothiazepin-4-one;hydrochloride
(3R)-3-amino-7-(5-tert-butyl-1,3,4-oxadiazol-2-yl)-5-[[4-(cyclopentoxy)phenyl]methyl]-1,1-dioxo-2,3-dihydro-1lambda6,5-benzothiazepin-4-one
(3R)-3-amino-5-[(4-chlorophenyl)methyl]-8-fluoro-7-[5-(4-fluorophenyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1,1-dioxo-7-[5-(1,2,2,2-tetrafluoro-1-methoxy-ethyl)-1,2,4-oxadiazol-3-yl]-2,3-dihydro-1lambda6,5-benzothiazepin-4-one;hydrochloride
(3R)-3-amino-7-(5-tert-butyl-1,3,4-oxadiazol-2-yl)-8-fluoro-1,1-dioxo-5-[(4-phenoxyphenyl)methyl]-2,3-dihydro-1lambda6,5-benzothiazepin-4-one;hydrochloride
or a pharmaceutically acceptable salt thereof.

In a particular embodiment, there is provided a compound of formula (I) as described herein, selected from:
(3R)-3-amino-7-(2-tert-butyltetrazol-5-yl)-5-[(4-chlorophenyl)methyl]-8-fluoro-1,1-dioxo-2,3-dihydro-1λ⁶,5-benzothiazepin-4-one;
(3R)-3-Amino-8-fluoro-1,1-dioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-4-one;
(3R)-3-Amino-7-(5-tert-butyl-1,3,4-oxadiazol-2-yl)-8-fluoro-1,1-dioxo-5-[[4-(trifluoromethoxy)phenyl]methyl]-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-8-fluoro-7-[5-(1-fluoro-1-methyl-ethyl)-1,3,4-oxadiazol-2-yl]-1,1-dioxo-2,3-dihydro-1λ⁶,5-benzothiazepin-4-one;
(3R)-3-Amino-5-[(4-chlorophenyl)methyl]-7-[5-(4,4-difluorocyclohexyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclohexyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(3,3-difluorocyclobutyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-8-fluoro-7-(5-isopropyl-1,3,4-oxadiazol-2-yl)-1,1-dioxo-2,3-dihydro-1λ⁶,5-benzothiazepin-4-one;
(3R)-3-amino-5-[(4-chlorophenyl)methyl]-8-fluoro-7-[5-(2-hydroxy-1,1-dimethyl-ethyl)-1,3,4-oxadiazol-2-yl]-1,1-dioxo-2,3-dihydro-1λ⁶,5-benzothiazepin-4-one;
4-[[(3R)-3-amino-7-(5-tert-butyl-1,3,4-oxadiazol-2-yl)-8-fluoro-1,1,4-trioxo-2,3-dihydro-1λ⁶,5-benzothiazepin-5-yl]methyl]benzonitrile;
(3R)-3-amino-7-(5-tert-butyl-1,3,4-oxadiazol-2-yl)-8-fluoro-5-[(4-methoxyphenyl)methyl]-1,1-dioxo-2,3-dihydro-1λ⁶,5-benzothiazepin-4-one; 2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]acetamide;
(3R)-3-amino-7-(5-tert-butyl-1,3,4-oxadiazol-2-yl)-5-[(4-chlorophenyl)methyl]-8-methyl-1,1-dioxo-2,3-dihydro-1λ⁶,5-benzothiazepin-4-one;
(3R)-3-amino-7-(1-tert-butylpyrazol-4-yl)-5-[(4-chlorophenyl)methyl]-8-fluoro-1,1-dioxo-2,3-dihydro-1λ⁶,5-benzothiazepin-4-one;
(3R)-3-amino-7-(5-tert-butyl-1,2,4-oxadiazol-3-yl)-5-[(4-chlorophenyl)methyl]-8-fluoro-1,1-dioxo-2,3-dihydro-1λ⁶,5-benzothiazepin-4-one;
(3R)-3-amino-7-[5-(1,1-dimethylbut-3-ynyl)-1,3,4-oxadiazol-2-yl]-8-fluoro-1,1-dioxo-5-[[4-[3-(trifluoromethyl)diazirin-3-yl]phenyl]methyl]-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
(3R)-3-amino-5-[(4-chlorophenyl)methyl]-8-fluoro-7 -(5-morpholino-1,3,4-oxadiazol-2-yl)-1,1-dioxo-2,3-dihydro-1λ⁶,5-benzothiazepin-4-one;
(3R)-3-amino-5-[(4-chlorophenyl)methyl]-7-[5-(cyclopentylamino)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-thiadiazol-2-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(5-ethyltetrazol-2-yl)-8-fluoro-1,1-dioxo-2,3-dihydro-1λ⁶,5-benzothiazepin-4-one;
or a pharmaceutically acceptable salt thereof.

In the description herein, if there is a discrepancy between a depicted structure and a name given to that structure, then the depicted structure controls. Additionally, if the stereochemistry of a structure or a portion of a structure is not indicated with, for example, bold wedged, or dashed lines, the structure or portion of the structure is to be interpreted as encompassing all stereoisomers of it. In some cases, however, where more than one chiral center exists, the structures and names may be represented as single enantiomers to help describe the relative stereochemistry.

Unless otherwise indicated, the terms "a compound of the formula" or "a compound of formula" or "compounds of the formula" or "compounds of formula" refer to any compound selected from the genus of compounds as defined by the formula (including any pharmaceutically acceptable salt of any such compound if not otherwise noted).

Certain compounds may exhibit tautomerism. Tautomeric compounds can exist as two or more interconvertable species. Prototropic tautomers result from the migration of a covalently bonded hydrogen atom between two atoms. Tautomers generally exist in equilibrium and attempts to isolate an individual tautomers usually produce a mixture whose chemical and physical properties are consistent with a mixture of compounds. The position of the equilibrium is dependent on chemical features within the molecule. For example, in many aliphatic aldehydes and ketones, such as acetaldehyde, the keto form predominates while; in phenols, the enol form predominates. Common prototropic tautomers include keto/enol (-C(=O)-CH- ↔ -C(-OH)=CH-), amide/imidic acid (-C(=O)-NH- ↔ -C(-OH)=N-) and amidine (-C(=NR)-NH- ↔ - C(-NHR)=N-) tautomers. The latter two are particularly common in heteroaryl and heterocyclic rings and the present invention encompasses all tautomeric forms of the compounds. Furthermore, the invention includes all optical isomers, i.e. diastereoisomers, diastereomeric mixtures, racemic mixtures, all their corresponding enantiomers and/or tautomers as well as their solvates of the compounds of formula (I). The compounds of formula (I) may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90% of the desired isomer by weight, particularly > 95% of the desired isomer by weight, or more particularly > 99% of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H , ³H , ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of manufacturing

Processes for the manufacture of compounds of formula (I), or pharmaceutically acceptable salts thereof, as described herein are also an object of the invention.

The preparation of compounds of formula (I) of the present invention may be carried out in sequential or convergent synthetic routes. Syntheses of the invention are shown in the following general schemes. The skills required for carrying out the reaction and purification of the resulting products are known to those persons skilled in the art. The substituents and indices used in the following description of the processes have the significance given herein, unless indicated to the contrary.

If one of the starting materials, intermediates or compounds of formula (I) contain one or more functional groups which are not stable or are reactive under the reaction conditions of one or more reaction steps, appropriate protective groups (as described e.g., in "Protective Groups in Organic Chemistry" by T. W. Greene and P. G. M. Wutts, 5th Ed., 2014, John Wiley & Sons, N.Y.) can be introduced before the critical step applying methods well known in the art. Such protective groups can be removed at a later stage of the synthesis using standard methods described in the literature.

If starting materials or intermediates contain stereogenic centers, compounds of formula (I) can be obtained as mixtures of diastereomers or enantiomers, which can be separated by methods well known in the art e.g., chiral HPLC, chiral SFC or chiral crystallization. Racemic compounds can e.g., be separated into their antipodes via diastereomeric salts by crystallization with optically pure acids or by separation of the antipodes by specific chromatographic methods using either a chiral adsorbent or a chiral eluent. It is equally possible to separate starting materials and intermediates containing stereogenic centers to afford diastereomerically/enantiomerically enriched starting materials and intermediates. Using such diastereomerically/enantiomerically enriched starting materials and intermediates in the synthesis of compounds of formula (I) will typically lead to the respective diastereomerically/enantiomerically enriched compounds of formula (I).

A person skilled in the art will acknowledge that in the synthesis of compounds of formula (I) - insofar not desired otherwise - an "orthogonal protection group strategy" will be applied, allowing the cleavage of several protective groups one at a time each without affecting other protective groups in the molecule. The principle of orthogonal protection is well known in the art and has also been described in literature (e.g. Barany and R. B. Merrifield, J. Am. Chem. Soc. 1977, 99, 7363; H. Waldmann et al., Angew. Chem. Int. Ed. Engl. 1996, 35, 2056).

A person skilled in the art will acknowledge that the sequence of reactions may be varied depending on reactivity and nature of the intermediates.

In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 2nd Edition, Richard C. Larock. John Wiley & Sons, New York, NY. 1999*).* It was found convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 hours to several days will usually suffice to yield the described intermediates and compounds. The reaction sequence is not limited to the one displayed in the schemes, however, depending on the starting materials and their respective reactivity, the sequence of reaction steps can be freely altered.

If starting materials or intermediates are not commercially available or their synthesis not described in literature, they can be prepared in analogy to existing procedures for close analogues or as outlined in the experimental section.

In one embodiment, compounds of formula (I) as described herein, or a pharmaceutically acceptable salt thereof, may be prepared by a process comprising
a) reacting a compound of formula (IX) 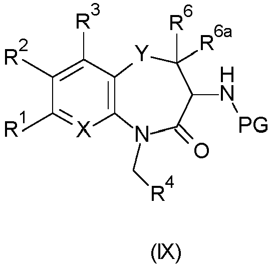 wherein X, Y, R¹, R², R³, R⁴, R⁶, R^{6a} are as described herein and PG is an amino protecting group, with a suitable deprotection agent to form said compound of formula (I), or a pharmaceutically acceptable salt thereof, wherein X, Y, R¹, R², R³, R⁴, R⁶, R^{6a} are as described herein and R⁵ is hydrogen; or
b) reacting a compound of formula (Ia) 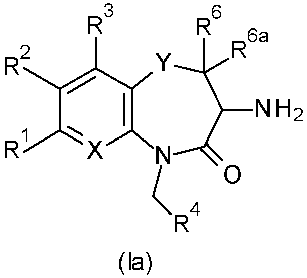 wherein X, Y, R¹, R², R³, R⁴, R⁶, R^{6a} are as described herein and R⁵ is hydrogen, with a carboxylic acid derivative of formula R⁹CO₂H wherein R⁹ is as described herein, in the presence of a base to form a compound of formula (I), or a pharmaceutically acceptable salt thereof, wherein R⁵ is -C(O)(R⁹).

The present compounds of formula (I), or their pharmaceutically acceptable salts, may be prepared by a process described below (Scheme 1), together with synthetic methods known in the art of organic chemistry, or modifications and derivatizations that are familiar to those of ordinary skilled in the art.

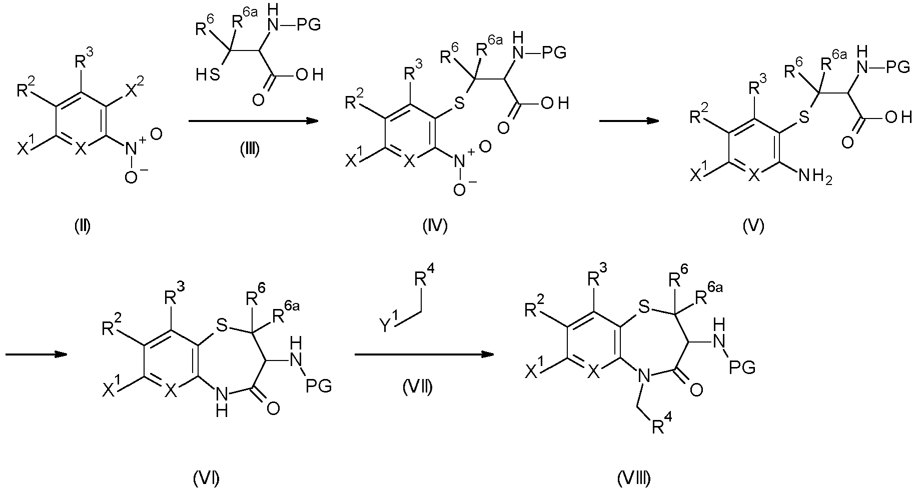

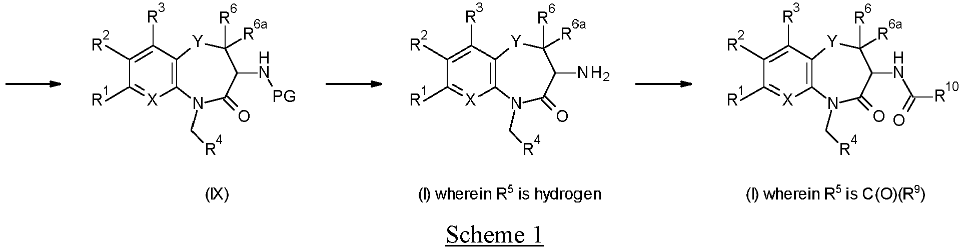

Suitable starting materials for the preparation of compounds of formula (I) are nitro compounds of formula (II) wherein X² is F or Cl and X¹ is either already R¹ or a group such as Br or -CO₂Alkyl which can later be elaborated into R¹. Compounds of formula (II) can be reacted with suitably protected cysteine derivatives (III) in the presence of a base such as DIPEA at elevated temperatures in a solvent such as 1,2-dichloroethane to obtain compounds of formula (IV). The preferred protecting group (PG) of the cysteine derivative (III) is Boc. The nitro group in formula (IV) compounds can be reduced using iron in the presence of either hydrogen chloride or ammonium chloride at elevated temperatures in a solvent mixture of water and ethanol to obtain compounds of formula (V). Alternatively, this conversion can be achieved by catalytic hydrogenation. Compounds of formula (V) can be cyclized to compounds of formula (VI) using standard amide coupling conditions. Preferably, this cyclization is conducted using 2,4,6-tripropyl-1,3,5,2,4,6-trioxatriphosphinane 2,4,6-trioxide (50% solution in EtOAc) and employing a base such as DIPEA in a solvent such as DMF at room temperature. Reaction of formula (VI) compounds with compounds of formula (VII) wherein Y¹ is Cl, Br, I or a sulfonate group in the presence of a base such as potassium carbonate and if necessary with an additive such as potassium iodide in a solvent such as DMSO or DMF at room temperature affords compounds of formula (VIII). For compounds of formula (VIII) wherein X¹ is Br or -CO₂Alkyl, these groups can be elaborated into substituents R¹ at this stage as described in the schemes below. Compounds of formula (VIII) can then be converted into compounds of formula (IX) wherein Y is S(O) or S(O)₂ by reaction with an appropriate amount of an oxidant such m-CPBA in a solvent such as DCM at room temperature. Alternatively, compounds of formula (VIII) can be converted into compounds of formula (IX) wherein Y is S(O)N(R^{y}) and R^{y} is hydrogen. Typical conditions include iodobenzene diacetate in the presence of ammonium carbonate in a solvent such as methanol at room temperature. Compounds of formula (IX) wherein Y is S(O)N(R^{y}) and R^{y} is C₁₋₆-alkyl can be obtained from compounds of formula (IX) wherein Y is S(O)N(R^{y}) and R^{y} is hydrogen by reaction with a C₁₋₆-alkylboronic acid in the presence of copper(II) acetate and pyridine as a base in a solvent such as dioxane at reflux temperature [Org. Biomol. Chem., 2017, 15, 8493]. Final deprotection provides compounds of formula (I). If the N-protecting group (PG) is Boc, typical conditions for this deprotection step include TFA in a solvent such as DCM at room temperature, hydrogen chloride in solvents such as dioxane or ethyl acetate at room temperature or hexafluoroisopropanol at reflux temperature. Alternatively, the protecting group (PG) of compounds of formula (VIII) can be cleaved accordingly to provide compounds of formula (I) wherein Y is S. Additionally, substituents R¹ and R⁴ might contain functional groups that could be either modified prior to the removal of the N-protecting group (PG) or that might require the use of suitable protecting groups during the synthesis. These protecting groups might be removed prior to the removal of the N-protecting group (PG) or they might be removed simultaneously using suitable methods [Peter G. M. Wuts, Greene's protective groups in organic synthesis, 5th edition, Hoboken, N.J.: Wiley-Interscience]. Compounds of formula (I) wherein R⁵ is hydrogen can be converted into formula (I) compounds wherein R⁵ is -C(O)(R⁹) by reaction with carboxylic acid derivatives R⁹CO₂H using standard amide coupling conditions such as HATU in the presence of a base such as DIPEA in a solvent such as THF at room temperature. If the substituent R⁹ contains a functional group that requires to be protected during this coupling step, the protecting group can be removed in an additional step using suitable conditions.

Alternatively, compounds of formula (I) wherein Y is S(O)₂ may be prepared as illustrated in scheme 2. 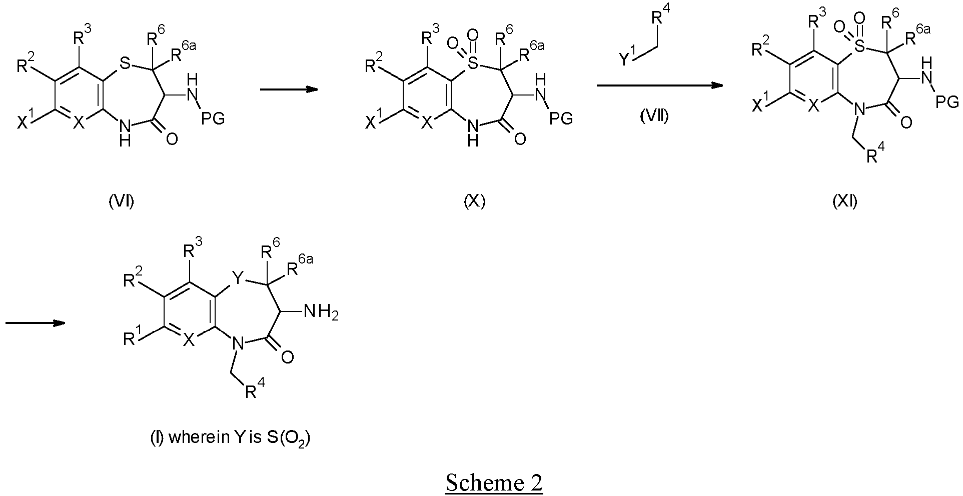 Compounds of formula (VI) can be converted into compounds of formula (X) upon reaction with an oxidant such as m-CPBA in a solvent such as DCM at room temperature. The reaction of formula (X) compounds with compounds of formula (VII) to afford compounds of formula (XI) and the subsequent conversion into compounds of formula (I) wherein Y is S(O₂) can be achieved using reaction conditions as described for the similar steps in scheme 1. If X¹ is Br or -CO₂Alkyl, these groups can be elaborated into substituents R¹ at any stage of the synthesis (for compounds of formula (VI), (X) or (XI)) using methods as described for the schemes below).

Compounds of formula (I) wherein the 5-membered heteroaryl R¹ is a 1,3,4-oxadiazolyl group may be prepared as illustrated in scheme 3.

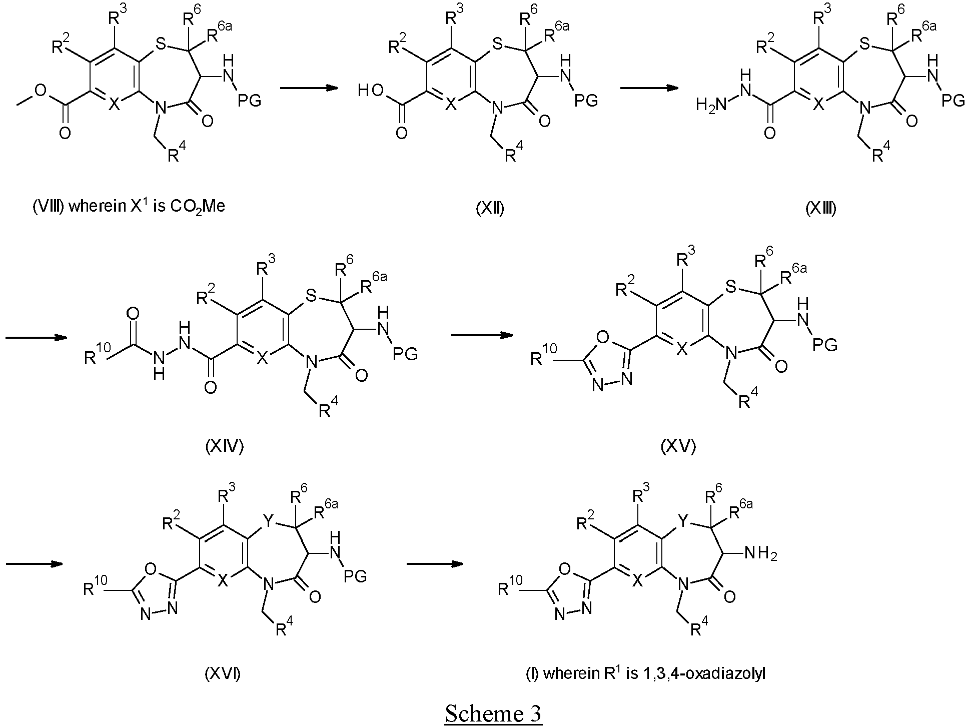

Compounds of formula (VIII) wherein X¹ is CO₂Me can be converted into compounds of formula (XII) by reaction with alkali hydroxides such as LiOH, NaOH or KOH in a mixture of solvents such as MeOH, THF and water at room temperature. Compounds of formula (XII) can be reacted with hydrazine hydrate after activation with suitable reagents such as CDI in a solvent such as THF at room temperature to obtain compounds of formula (XIII). Compounds of formula (XIII) can be reacted with carboxylic acids R¹⁰CO₂H using standard amide coupling conditions such as HATU in the presence of a base such as DIPEA in a solvent such as THF at room temperature. The coupling products of formula (XIV) can be cyclized to compounds of formula (XV) using a dehydrating reagent such as Burgess reagent at room temperature. The conversion of compounds of formula (XV) into compounds of formula (XVI) and the subsequent conversion into compounds of formula (I) can be achieved using reaction conditions as described for the similar steps in scheme 1.

Compounds of formula (I) wherein the 5-membered heteroaryl R¹ is a 1,2,4-oxadiazolyl group may be prepared as illustrated in scheme 4.

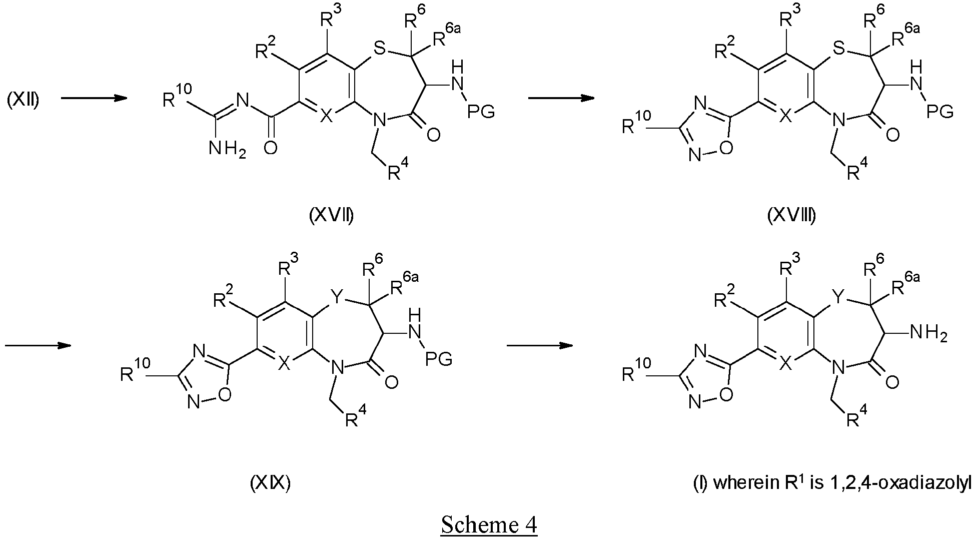

Compounds of formula (XII) can be reacted with amidines R¹⁰C(NH)NH₂ using standard amide coupling conditions such as HBTU in the presence of a base such as DIPEA in a solvent such as DMF at room temperature to obtain compounds of formula (XVII). Reaction of formula (XVII) compounds with NBS in the presence of DBU in EtOAc at room temperature can afford compounds of formula (XVIII) [Tetrahedron 74 (2018) 4613-4618]. The conversion of compounds of formula (XVIII) into compounds of formula (XIX) and the subsequent conversion into compounds of formula (I) can be achieved using reaction conditions as described for the similar steps in scheme 1.

Compounds of formula (I) wherein the 5-membered heteroaryl R¹ is a 1,2,3-triazolyl group may be prepared as illustrated in scheme 5.

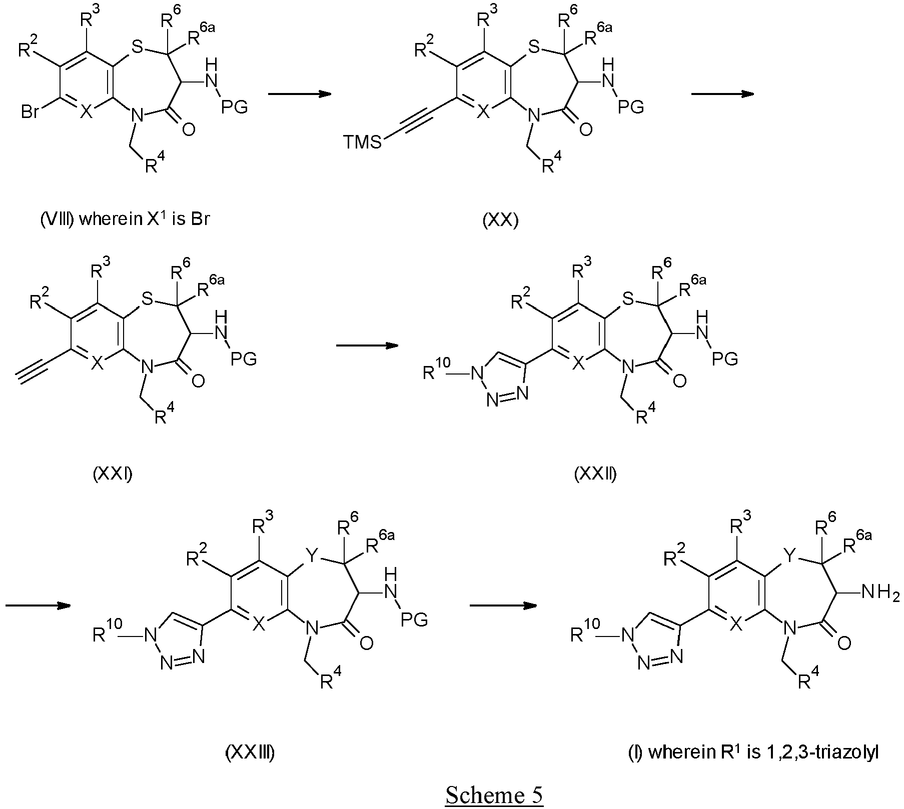

Compounds of formula (VIII) wherein X¹ is Br can be converted into compounds of formula (XX) by Sonogashira coupling with trimethylsilylacetylene. Typical conditions include bis(triphenylphosphine)palladium(II) chloride and copper(I) iodide as catalysts in the presence of a base such as DIPEA in a solvent such as THF at room temperature. Reaction of compounds of formula (XX) with KF in MeOH affords compounds of formula (XXI) which can be transformed into compounds of formula (XXII) by reaction with azides R¹²N₃ in the presence of CuSO₄•5H₂O and sodium ascorbate in a solvent mixture of MeOH and water. If necessary, azides R¹²N₃ can be prepared in situ from amines R¹²NH₂ and 1H-imidazole-1-sulfonyl azide hydrochloride. The conversion of compounds of formula (XXII) into compounds of formula (XXIII) and the subsequent conversion into compounds of formula (I) can be achieved using reaction conditions as described for the similar steps in scheme 1.

Compounds of formula (I) wherein the 5-membered heteroaryl R¹ is a pyrazolyl group may be prepared as illustrated in scheme 6.

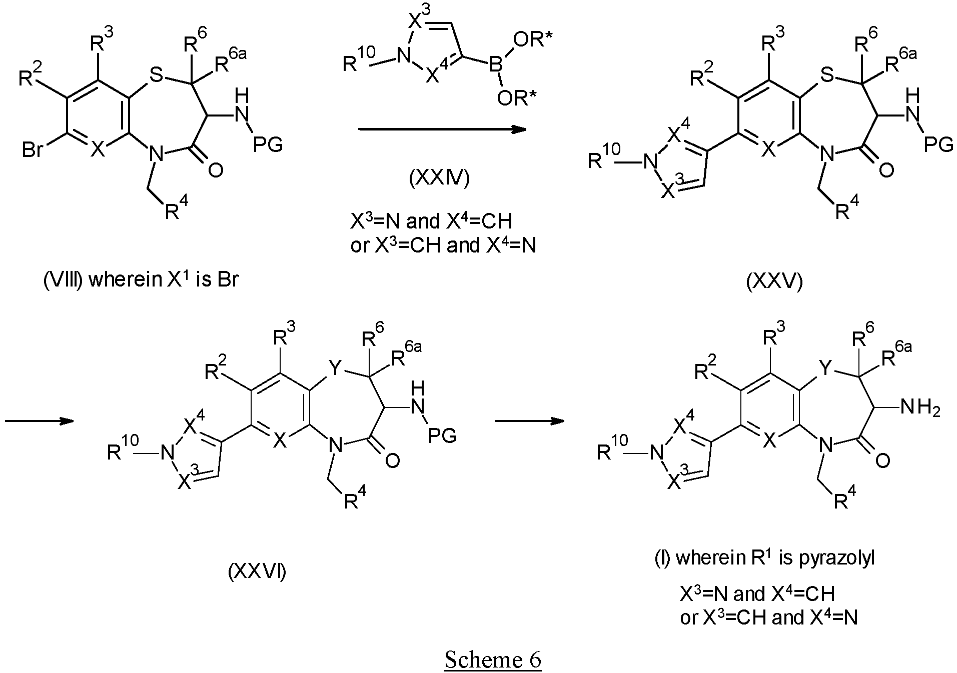

Compounds of formula (VIII) wherein X¹ is Br can be converted into compounds of formula (XXV) by Suzuki coupling with boronic acids or boronic acid esters (XXIV). Typical reaction conditions include the use of Pd(dppf)Cl₂ as catalyst in the presence of K₃PO₄ in a mixture of dioxane and water at reflux temperature. The conversion of compounds of formula (XXV) into compounds of formula (XXVI) and the subsequent conversion into compounds of formula (I) can be achieved using reaction conditions as described for the similar steps in scheme 1. Alternatively, compounds of formula (I) wherein the 5-membered heteroaryl R¹ is a pyrazolyl group may be prepared as illustrated in scheme 7.

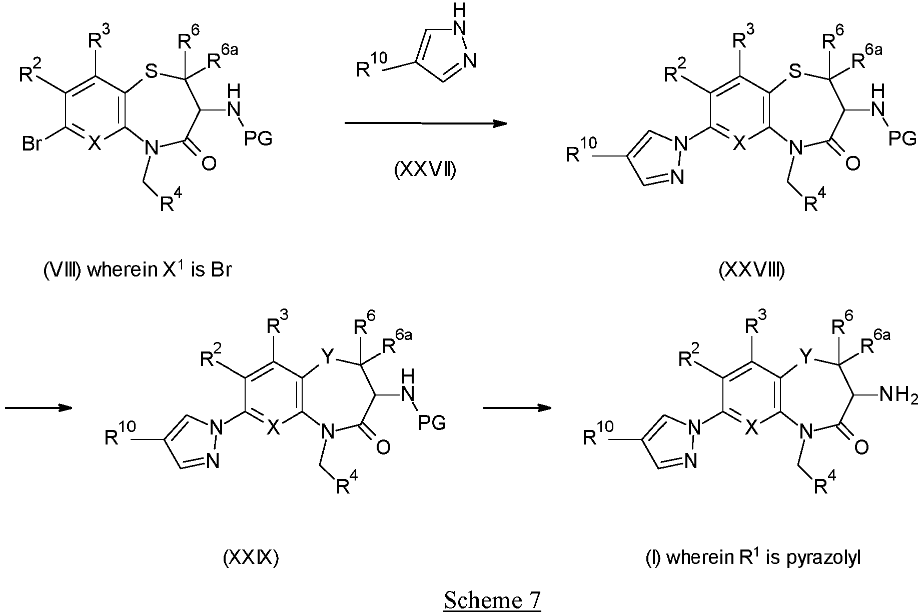

Compounds of formula (VIII) wherein X¹ is Br can be converted into compounds of formula (XXVIII) by reaction with pyrazoles (XXVII) in the presence of CuI, trans-N,N'-dimethylcyclohexane-1,2-diamine and potassium carbonate in toluene as a solvent at reflux temperature. The conversion of compounds of formula (XXVIII) into compounds of formula (XXIX) and the subsequent conversion into compounds of formula (I) can be achieved using reaction conditions as described for the similar steps in scheme 1. Alternatively, compounds of formula (I) wherein the 5-membered heteroaryl R¹ is a pyrazolyl group may also be prepared as illustrated in scheme 8.

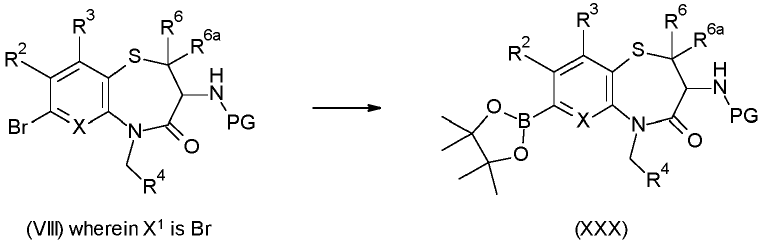

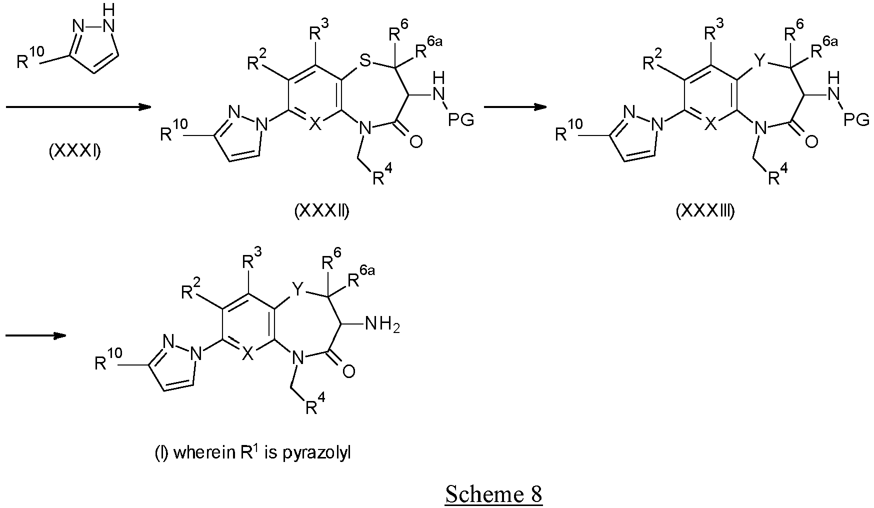

Compounds of formula (VIII) wherein X¹ is Br can be converted into compounds of formula (XXX) by reaction with 4,4,5,5-tetramethyl-2-(4,4,5,5-tetramethyl-1,3,2-dioxaborolan-2-yl)-1,3,2-dioxaborolane in the presence of Pd(dppf)Cl₂•CH₂Cl₂ and potassium acetate in dioxane at 80°C. Compounds of formula (XXX) can be transformed into compounds of formula (XXXII) by Chan-Lam coupling with pyrazoles (XXXI). Typical reaction conditions include Cu(OAc)₂ in the presence of TEA in acetonitrile at 30°C under an atmosphere of oxygen. The conversion of compounds of formula (XXXII) into compounds of formula (XXXIII) and the subsequent conversion into compounds of formula (I) can be achieved using reaction conditions as described for the similar steps in scheme 1. Compounds of formula (I) wherein the 5-membered heteroaryl R¹ is a 1,2,4-triazolyl group may be prepared as illustrated in scheme 9.

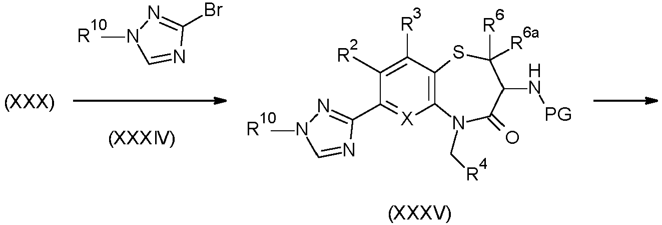

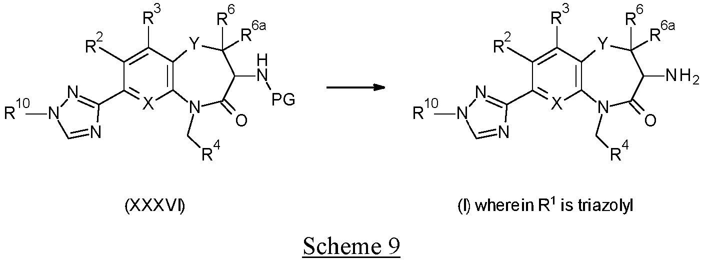

Suzuki coupling of compounds of formula (XXX) with triazolylbromides (XXXIV) provides compounds of formula (XXXV). Typical reaction conditions include the use of Pd(dppf)Cl₂ as catalyst in the presence of K₃PO₄ in a mixture of dioxane and water at reflux temperature. The conversion of compounds of formula (XXXV) into compounds of formula (XXXVI) and the subsequent conversion into compounds of formula (I) can be achieved using reaction conditions as described for the similar steps in scheme 1. Compounds of formula (I) wherein the 5-membered heteroaryl R¹ is a tetrazolyl group may be prepared as illustrated in scheme 10.

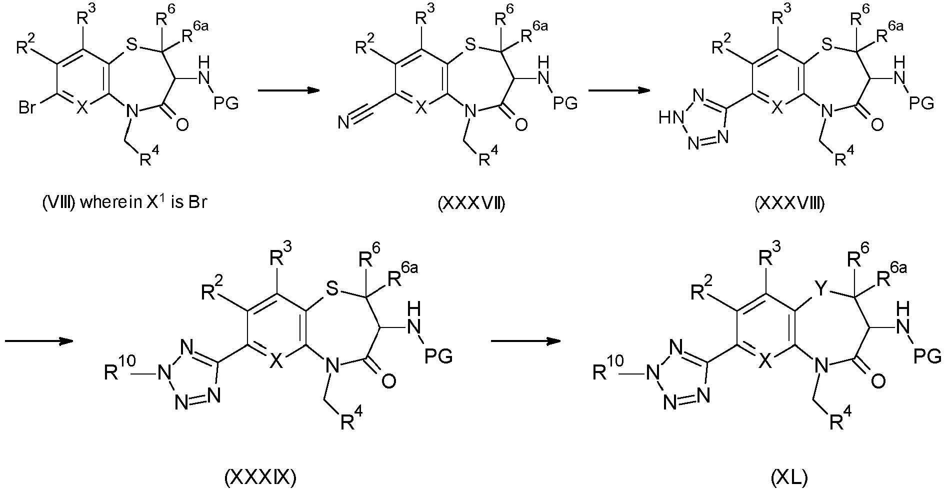

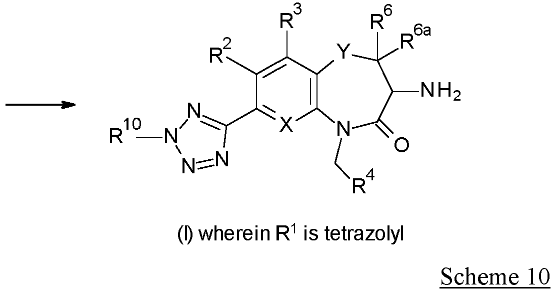

Compounds of formula (VIII) wherein X¹ is Br can be converted into compounds of formula (XXXVII) using a mixture of zinc cyanide, zinc powder, t-Bu₃P and Pd₂(dba)₃ in DMF at elevated temperature. The conversion of compounds of formula (XXXVII) to compounds of formula (XXXVIII) can be achieved by reaction with azide reagents such as azidotrimethylsilane in the presence of tetra-N-butylammonium fluoride trihydrate in toluene at elevated temperature. To obtain compounds of formula (XXXIX) from compounds of formula (XXXVIII), substituents R¹⁰ can be introduced by a variety of methods such as reaction with a reagent R¹⁰Y (wherein Y is Cl, Br or I) in the presence of a base such as potassium carbonate in a solvent such as DMF at room temperature. The conversion of compounds of formula (XXXIX) into compounds of formula (XL) and the subsequent conversion into compounds of formula (I) can be achieved using reaction conditions as described for the similar steps in scheme 1. Compounds of formula (I) wherein the 5-membered heteroaryl R¹ is a 1,2,3-triazolyl group may be prepared as illustrated in scheme 11.

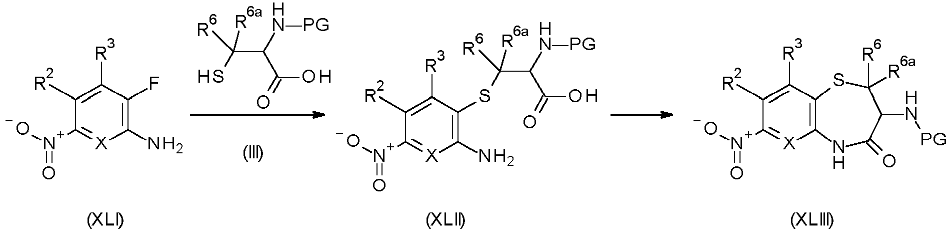

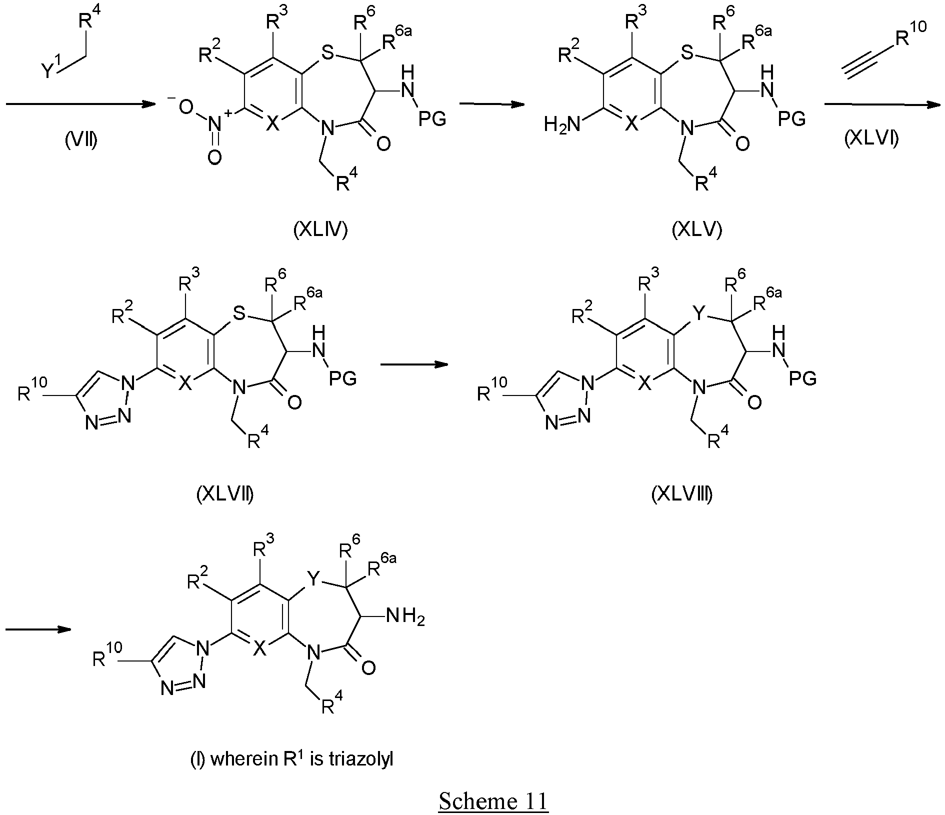

Suitable starting materials for the synthesis of compounds of formula (I) wherein the 5-membered heteroaryl R¹ is a 1,2,3-triazolyl group are fluoro-nitro-amines of formula (XLI). Reaction of compounds of formula (XLI) with cysteine derivatives (III) to obtain compounds of formula (XLII), subsequent cyclization to compounds of formula (XLIII) and reaction with compounds of formula (VII) to afford compounds of formula (XLIV) can be accomplished using conditions as described for the similar reaction steps in scheme 1. Conversion of nitro compounds of formula (XLIV) into anilines of formula (XLV) can be achieved using zinc powder in the presence of ammonium chloride in a solvent such as MeOH at elevated temperatures. Reaction of compounds of formula (XLV) with first isopentyl nitrite and azidotrimethylsilane and then with acetylenes of formula (XLVI) in the presence of copper(I) oxide in a solvent such as acetonitrile at room temperature affords compounds of formula (XLVII). The conversion of compounds of formula (XLVII) into compounds of formula (XLVIII) and the subsequent conversion into compounds of formula (I) can be achieved using reaction conditions as described for the similar steps in scheme 1.

Compounds of formula (I) wherein the 5-membered heteroaryl R¹ is an isoxazolyl group may be prepared as illustrated in scheme 12.

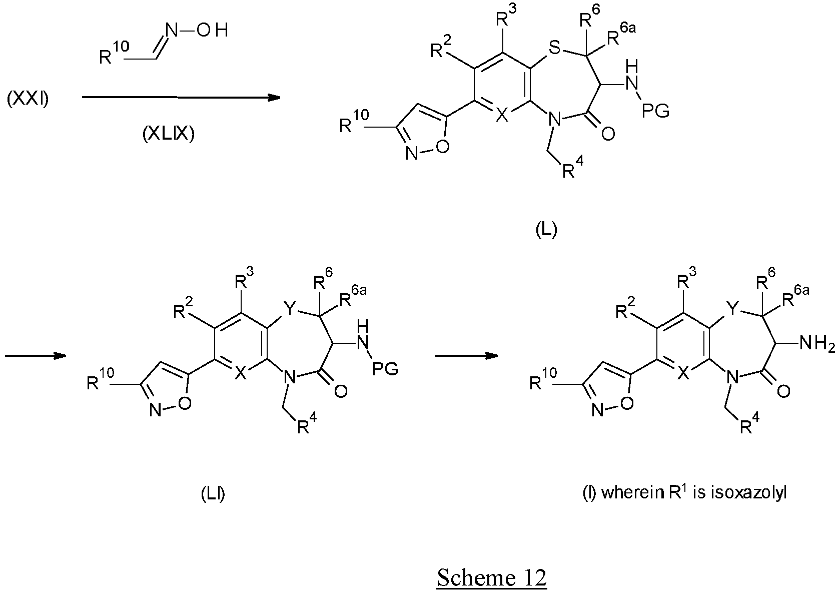

Acetylene compounds of formula (XXI) can be reacted with oximes of formula (XLIX) in the presence of aqueous sodium hypochlorite solution and TEA in a solvent such as DCM at 30°C to obtain compounds of formula (L). The conversion of compounds of formula (L) into compounds of formula (LI) and the subsequent conversion into compounds of formula (I) can be achieved using reaction conditions as described for the similar steps in scheme 1. Compounds of formula (I) wherein the 5-membered heteroaryl R¹ is a 1,2,4-triazolyl group and Y is S(O₂) may be prepared as illustrated in scheme 13.

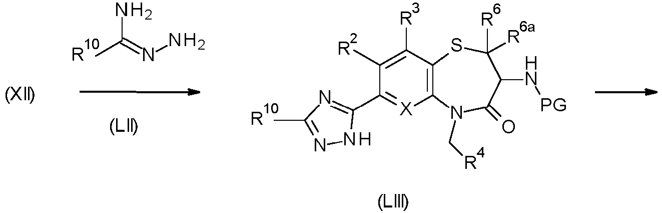

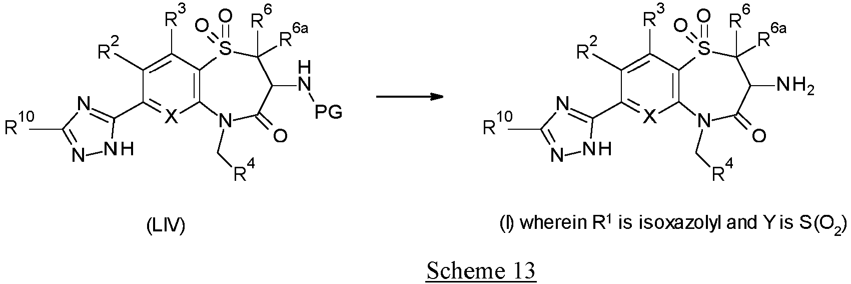

Carboxylic acids of formula (XII) can be reacted with amidrazones of formula (LII) in the presence of an activating reagent such as HATU and a base such as TEA in a solvent such as DMF to obtain the corresponding coupling products, which will cyclize to compounds of formula (LIII) upon heating. Oxidation of compounds of formula (LIII) with m-CPBA in a solvent such as DCM and subsequent cleavage of the N-protecting group (PG) affords compounds of formula (I).

Compounds of formula (I) wherein the 5-membered heteroaryl R¹ is a 1,2,4-oxadiazolyl group may be prepared as illustrated in scheme 14.

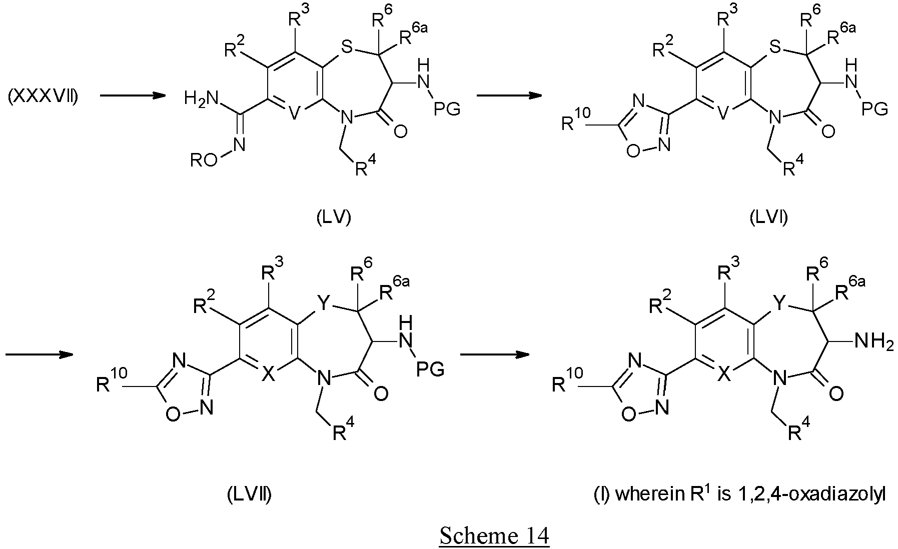

Nitrile compounds of formula (XXXVII) can be reacted with hydroxylamine hydrochloride in presence of a base such as potassium carbonate in a solvent such as ethanol at elevated temperatures to obtain amidoxime compounds of formula (LV wherein R is H). Reaction of compounds of formula (LV wherein R is H) with carboxylic acids R¹⁰CO₂H using standard amide coupling conditions with activating agents such CDI, EDC/HOBt or HATU in the presence of a base such as DIPEA in a solvent such as DMF, THF or acetonitrile, provides coupling intermediates (LV wherein R is -C(O)R¹⁰) which upon heating cyclize to the corresponding compounds of formula (LVI). Alternatively, coupling intermediates (LV wherein R is -C(O)R¹⁰) can be isolated and the cyclization step can be conducted either by heating in a solvent such as toluene or by reaction with TBAOH in a solvent such as THF. The conversion of compounds of formula (LVI) into compounds of formula (LVII) and the subsequent conversion into compounds of formula (I) can be achieved using reaction conditions as described for the similar steps in scheme 1. Compounds of formula (I) wherein the 5-membered heteroaryl R¹ is an oxazolyl group may be prepared as illustrated in scheme 15.

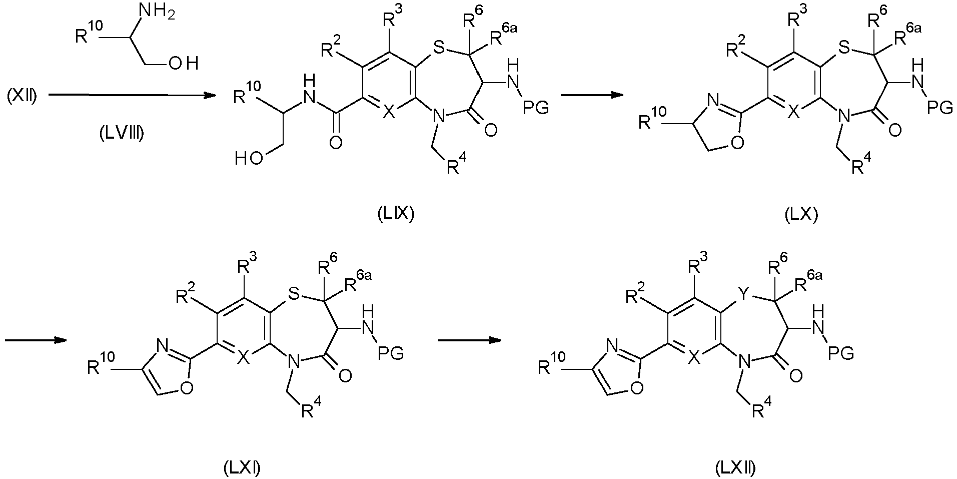

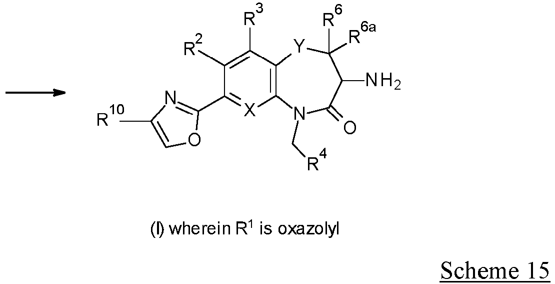

Compounds of formula (XII) can be reacted with amino alcohols of formula (LVIII) using standard amide coupling conditions such as HATU in the presence of a base such as DIPEA in a solvent such as DMF at room temperature to obtain compounds of formula (LIX). Cyclization of compounds of formula (LIX) to compounds of formula (LX) can be accomplished with a dehydrating reagent such as Burgess reagent in a solvent such as THF at elevated temperatures. Reaction of compounds of formula (LX) with DDQ in toluene at elevated temperatures provides compounds of formula (LXI). The conversion of compounds of formula (LXI) into compounds of formula (LXII) and the subsequent conversion into compounds of formula (I) can be achieved using reaction conditions as described for the similar steps in scheme 1. Compounds of formula (I) wherein the 5-membered heteroaryl R¹ is a 1,3,4-oxadiazolyl group, R¹⁰ is N(R^{10e}R^{10f}) and Y is S(O₂) may be prepared as illustrated in scheme 16.

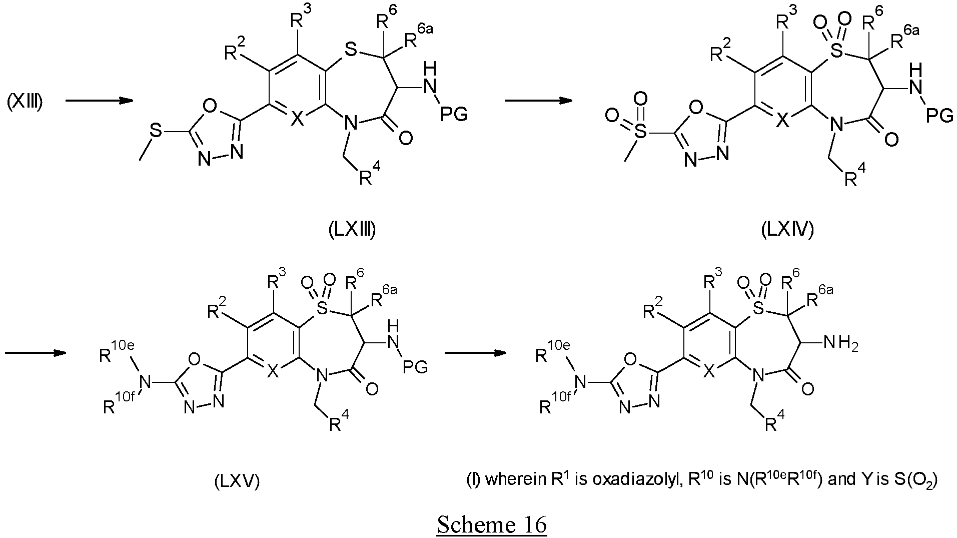

Compounds of formula (XIII) can be reacted first with CS₂ in DMF at elevated temperature and then with methyl iodide in the presence of TEA at room temperature to obtain compounds of formula (LXIII). Oxidation of compounds of formula (LXIII) with an oxidation agent such as KMnO₄ in a solvent mixture of water and acetic acid at a temperature between 0 and 5°C provides compounds of formula (LXIV). Compounds of formula (LXIV) can be transformed into compounds of formula (LXV) by reaction with amines HN(R^{10e}R^{10f}) in presence of a base such as potassium carbonate in a solvent such as DMF at room temperature. Cleavage of the N-protecting group (PG) affords compounds of formula (I). Alternatively, compounds of formula (I) wherein the 5-membered heteroaryl R¹ is a 1,3,4-oxadiazolyl group, R¹⁰ is N(R^{10e}R^{10f}) and Y is S(O₂) may be prepared as illustrated in scheme 17.

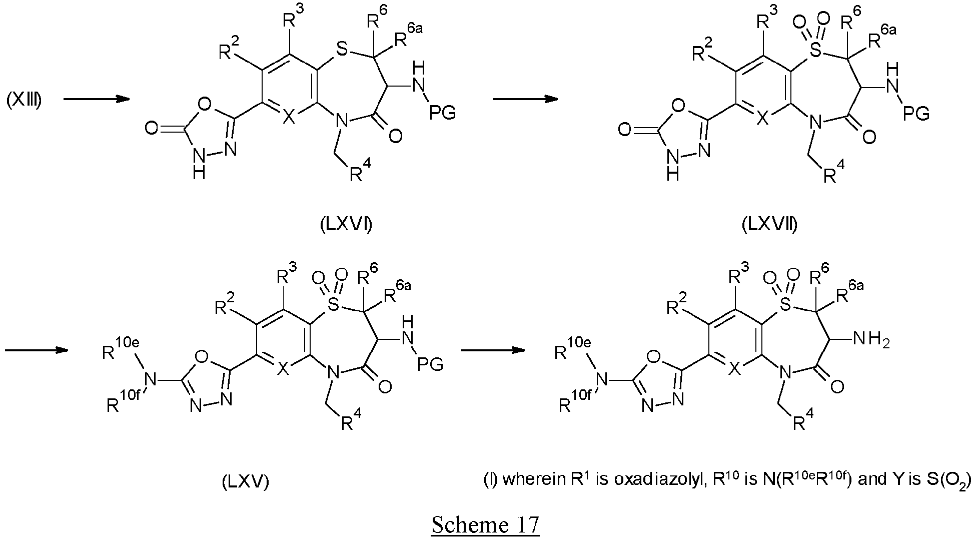

Compounds of formula (XIII) can be reacted with CDI in THF in the presence of a base such as TEA at room temperature to obtain compounds of formula (LXVI). Oxidation of compounds of formula (LXVI) with an oxidation agent such as m-CPBA in a solvent such as DCM at room temperature provides compounds of formula (LXVII). Compounds of formula (LXVII) can be transformed into compounds of formula (LXV) by reaction with amines HN(R^{10e}R^{10f}) in presence of DIPEA and BOP in a solvent such as DMF at room temperature or at elevated temperatures [Org. Lett., 2008, Vol. 10, 1755-1758]. Cleavage of the N-protecting group (PG) affords compounds of formula (I).

Compounds of formula (I) wherein the 5-membered heteroaryl R¹ is a 1,2,4-oxadiazolyl group, R¹⁰ is N(R^{10e}R^{10f}) and Y is S(O₂) may be prepared as illustrated in scheme 18.

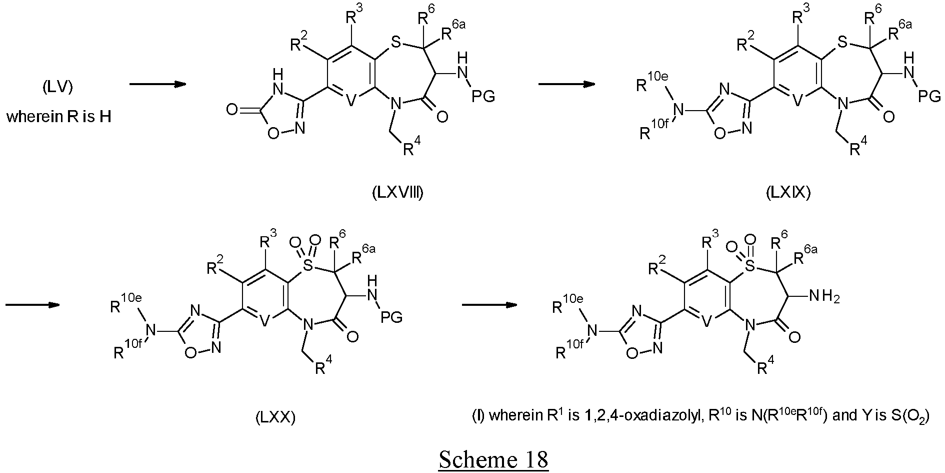

Compounds of formula (LV wherein R is H) can be reacted with CDI in THF in the presence of a base such as TEA at room temperature to obtain compounds of formula (LXVIII). Compounds of formula (LXVIII) can be transformed into compounds of formula (LXIX) by reaction with amines HN(R^{10e}R^{10f}) in presence of DIPEA and PyBroP in a solvent such as dioxane at elevated temperatures. Oxidation of compounds of formula (LXIX) with an oxidation agent such as m-CPBA in a solvent such as DCM at room temperature provides compounds of formula (LXX). Cleavage of the N-protecting group (PG) affords compounds of formula (I). Alternatively, compounds of formula (I) wherein the 5-membered heteroaryl R¹ is a 1,2,4-oxadiazolyl group, R¹⁰ is N(R^{10e}R^{10f}) and Y is S(O₂) may be prepared as illustrated in scheme 19.

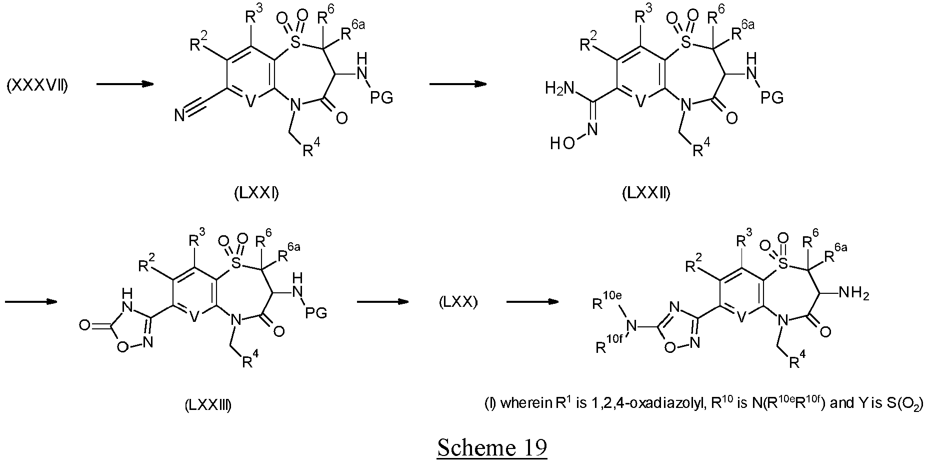

Reaction of compounds of formula (XXXVII) with an oxidation agent such as m-CPBA in a solvent such as DCM at room temperature provides compounds of formula (LXXI) which can be converted into compounds of formula (LXXII) by reaction with hydroxylamine hydrochloride in presence of a base such as sodium bicarbonate in a solvent such as methanol at elevated temperatures. The subsequent conversion of formula (LXXII) compounds into compounds of formula (I) can be achieved using reaction conditions as described for the similar steps in scheme 18.

Compounds of formula (I) wherein the 5-membered heteroaryl R¹ is a 1,3,4-oxadiazolyl group, R¹⁰ is N(R^{10e}R^{10f}) and Y is S(O₂) may be prepared as illustrated in scheme 20.

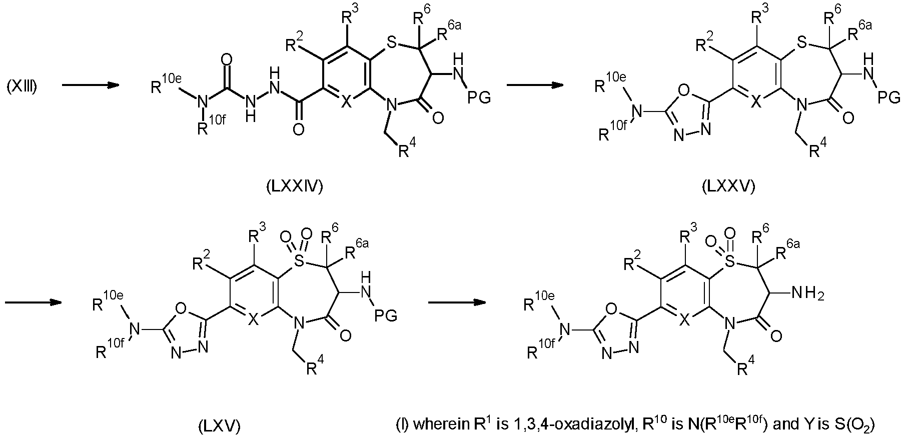

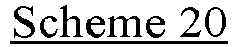

Reaction of amines HN(R^{10e})(R^{10f}) with triphosgene in presence of a base such as aqueous sodium bicarbonate in a solvent such as 1,2-dichloroethane and subsequent reaction with a hydrazide compound of formula (XIII) provides compounds of formula (LXXIV). Compounds of formula (LXXIV) can be cyclized to formula (LXXV) compounds by employing reagents such as p-toluenesulfonyl chloride and DIPEA at room temperature. Oxidation of compounds of formula (LXXV) to compounds of formula (LXV) and conversion to compounds of formula (I) can be achieved using reaction conditions as described for the similar steps in scheme 1.

### Pharnaceutical compositions and administration

Another object of the present invention is a pharmaceutical composition comprising a compound of formula (I) as described herein, or a pharmaceutically acceptable salt thereof, and a pharmaceutically acceptable excipient.

The compounds of formula (I) and their pharmaceutically acceptable salts can be used as medicaments, in the form of pharmaceutical preparations. The pharmaceutical preparations can be administered internally, such as orally (e.g. in the form of tablets, coated tablets, dragées, hard and soft gelatine capsules, solutions, emulsions or suspensions), nasally (e.g. in the form of nasal sprays) or rectally (e.g. in the form of suppositories). However, the administration can also be effected parenterally, such as intramuscularly or intravenously (e.g. in the form of injection solutions). The administration can also be effected topically, e.g. transdermal administration, or in form of eye drops or ear drops.

The compounds of formula (I) and their pharmaceutically acceptable salts can be processed with pharmaceutically inert, inorganic or organic carriers for the production of pharmaceutical preparations, such as tablets, coated tablets, dragées, hard gelatin capsules, injection solutions or topical formulations. Lactose, corn starch or derivatives thereof, talc, stearic acids or salts thereof, and the like can be used, for example, as such carriers for tablets, coated tablets, dragées and hard gelatin capsules.

Suitable carriers for soft gelatin capsules are, for example, vegetable oils, waxes, fats, semi-solid substances and liquid polyols and the like. Depending on the nature of the active substance no carriers are, however, usually required in the case of soft gelatin capsules.

Suitable carriers for the production of solutions and syrups are, for example, water, alcohols, polyols, saccharose, glucose, invert sugar, vegetable oil, etc.

Suitable carriers for injection solutions are, for example, water, alcohols, polyols, glycerol, vegetable oils, etc.

Suitable carriers for suppositories are, for example, natural or hardened oils, waxes, fats, semi-liquid or liquid polyols, etc.

Suitable carriers for topical ocular formulations are, for example, cyclodextrins, mannitol or many other carriers and excipients known in the art.

Moreover, the pharmaceutical preparations can contain preservatives, solubilizers, viscosity increasing substances, stabilizers, wetting agents, emulsifiers, sweeteners, colorants, flavorants, salts for varying the osmotic pressure, buffers, masking agents or antioxidants. They can also contain other therapeutically valuable substances.

Medicaments containing a compound of formula (I) or a pharmaceutically acceptable salt thereof and a pharmaceutically acceptable excipient are also an object of the present invention, as is a process for their production, which comprises bringing one or more compounds of formula (I) and/or pharmaceutically acceptable salts thereof and, if desired, one or more other therapeutically valuable substances into a galenical administration form together with one or more pharmaceutically acceptable excipients.

The dosage can vary within wide limits and will, of course, have to be adjusted to the individual requirements in each particular case. In general, in the case of oral administration a daily dosage of about 0.1 mg to 20 mg per kg body weight, preferably 0.5 mg to 4 mg per kg body weight (e.g. about 300 mg per person), divided into preferably 1-3 individual doses, which can consist, for example, of the same amounts, should be appropriate. In the case of topical administration, the formulation can contain 0.001% to 15% by weight of medicament and the required dose, which can be between 0.1 and 25 mg, and can be administered either by single dose per day or per week, or by multiple doses (2 to 4) per day, or by multiple doses per week. It will, however, be clear that the upper or lower limit given herein can be exceeded when this is shown to be indicated.

The pharmaceutical composition according to the invention may be prepared as follows.

### Preparation of pharmaceutical compositions comprising compounds of the invention

**Tablet Formulation (Wet Granulation)**

| **Ingredient** | **mg/tablet** | | | |
|---|---|---|---|---|
| | 5 | 25 | 100 | 500 |
| 1) Compound of formula (1) | 5 | 25 | 100 | 500 |
| 2) Lactose Anhydrous DTG | 125 | 105 | 30 | 150 |
| 3) Sta-Rx 1500 | 6 | 6 | 6 | 30 |
| 4) Microcrystalline Cellulose | 30 | 30 | 30 | 150 |
| 5) Magnesium Stearate | 1 | 1 | 1 | 1 |
| Total | 167 | 167 | 167 | 831 |

### Manufacturing Procedure:

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

**Capsule Formulation**

| **Ingredient** | **mg/capsule** | | | |
|---|---|---|---|---|
| | 5 | 25 | 100 | 500 |
| 1) Compound of formula (1) | 5 | 25 | 100 | 500 |
| 2) Hydrous Lactose | 159 | 123 | 148 | - |
| 3) Corn Starch | 25 | 35 | 40 | 70 |
| 4) Talc | 10 | 15 | 10 | 25 |
| 5) Magnesium Stearate | 1 | 2 | 2 | 5 |
| Total | 200 | 200 | 300 | 600 |

### Manufacturing Procedure:

1. Mix ingredients 1, 2 and 3 in a suitable mixer for 30 minutes.
2. Add ingredients 4 and 5 and mix for 3 minutes.
3. Fill into a suitable capsule.

**Injection Solutions**

| **Ingredient** | **mg/injection solution** |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure:

A compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Indications

The compounds of formula (I) can be used in an effective amount to treat a subject, in particular a human, affected by cancer.

In one aspect, the present invention provides a compound of formula (I) described herein, or a pharmaceutically acceptable thereof, for use as a therapeutically active substance.

In a further aspect, the present invention provides a compound of formula (I) as described herein, or a pharmaceutically acceptable thereof, for use in the treatment, prevention and/or delay of progression of cancer.

In a further aspect, the present invention provides the use of a compound of formula (I) as described herein, or a pharmaceutically acceptable salt thereof, for the treatment, prevention and/or delay of progression of cancer.

In a further aspect, the present invention provides the use of a compound of formula (I) as described herein, or a pharmaceutically acceptable salt thereof, for the preparation of a medicament for the treatment, prevention and/or delay of progression of cancer.

In a further aspect, the present invention provides a method for the treatment, prevention and/or delay of progression of cancer, which method comprises administering a therapeutically effective amount of a compound of formula (I) as described herein, or a pharmaceutically acceptable salt thereof.

By the term "treatment" or "treating" and grammatical variations thereof as used herein, is meant therapeutic therapy. In reference to a particular condition, treating means: (1) to ameliorate the condition or one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition or treatment thereof, or (4) to slow the progression of the condition or one or more of the biological manifestations of the condition. Prophylactic therapy using the methods and/or compositions of the invention is also contemplated. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing cancer, such as when a subject has a strong family history of cancer or when a subject has been exposed to a carcinogen.

As immunotherapeutic agents acting on immune cells rather than directly acting on the cancer cells, the present disclosure could also be foreseen for the use as anti-cancer vaccines. This also comprises approaches in which immune cells are cultured and manipulated *ex vivo* and the herein disclosed molecules are used as a way of conferring co-stimulation of the *ex vivo* manipulated cells.

In one embodiment, the cancer is a hematologic cancer such as lymphoma, a leukemia or a myeloma. A hematologic cancer contemplated herein includes, but is not limited to, one or more leukemias such as B-cell acute lymphoid leukemia ("BALL"), T-cell acute lymphoid leukemia ("TALL"), acute lymphoid leukemia (ALL); one or more chronic leukemias including but not limited to chronic myelogenous leukemia (CML) and chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, mucosa-associated lymphoid tissue (MALT) lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia, and "preleukemia," which are a diverse collection of hematological conditions united by ineffective production (or dysplasia) of myeloid blood cells.

In a further embodiment, the cancer is a non-hematologic cancer such as a sarcoma, a carcinoma, or a melanoma. A non-hematologic cancer contemplated herein includes, but is not limited to, a neuroblastoma, renal cell carcinoma, colon cancer, colorectal cancer, breast cancer, epithelial squamous cell cancer, melanoma, stomach cancer, brain cancer, lung cancer (e.g. non-small cell lung cancer - NSCLC), pancreatic cancer, cervical cancer, ovarian cancer, liver cancer, bladder cancer, prostate cancer, testicular cancer, thyroid cancer, uterine cancer, adrenal cancer and head and neck cancer.

### Co-Administration of Compounds of Formula (I) and Other Agents

The compounds of formula (I) or salts thereof or a compound disclosed herein or a pharmaceutically acceptable salt thereof may be employed alone or in combination with other agents for treatment. For example, the second agent of the pharmaceutical combination formulation or dosing regimen may have complementary activities to the compound of formula (I) such that they do not adversely affect each other. The compounds may be administered together in a unitary pharmaceutical composition or separately. In one embodiment a compound or a pharmaceutically acceptable salt can be co-administered with a cytotoxic agent to treat proliferative diseases and cancer.

The term "co-administering" refers to either simultaneous administration, or any manner of separate sequential administration, of a compound of formula (I) or a salt thereof or a compound disclosed herein or a pharmaceutically acceptable salt thereof and a further active pharmaceutical ingredient or ingredients, including cytotoxic agents and radiation treatment. If the administration is not simultaneous, the compounds are administered in a close time proximity to each other. Furthermore, it does not matter if the compounds are administered in the same dosage form, e.g. one compound may be administered topically and another compound may be administered orally.

Typically, any agent that has anti-cancer activity may be co-administered. Examples of such agents can be found in Cancer Principles and Practice of Oncology by V.T. Devita and S. Heilman (editors), 6th edition (February 15, 2001), Lippincott Williams & Wilkins Publishers. A person of ordinary skill in the art would be able to discern which combinations of agents would be useful based on the particular characteristics of the drugs and the disease involved.

In one aspect, the present invention provides a pharmaceutical composition described herein, further comprising an additional therapeutic agent.

In one embodiment, said additional therapeutic agent is a chemotherapeutic agent.

In one embodiment, said additional therapeutic agent is a cytotoxic agent.

In one embodiment, said additional therapeutic agent is an immuno-oncology agent.

The term "cytotoxic agent" as used herein refers to a substance that inhibits or prevents a cellular function and/or causes cell death or destruction. Cytotoxic agents include, but are not limited to, radioactive isotopes (At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents; growth inhibitory agents; enzymes and fragments thereof such as nucleolytic enzymes; and toxins such as small molecule toxins or enzymatically active toxins of bacterial, fungal, plant or animal origin, including fragments and/or variants thereof.

Exemplary cytotoxic agents can be selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine kinase angiogenesis inhibitors, immunotherapeutic agents, proapoptotic agents, inhibitors of LDH-A; inhibitors of fatty acid biosynthesis; cell cycle signaling inhibitors; HDAC inhibitors, proteasome inhibitors; and inhibitors of cancer metabolism.

"Chemotherapeutic agent" includes chemical compounds useful in the treatment of cancer. Examples of chemotherapeutic agents include erlotinib (TARCEVA^{®}, Genentech/OSI Pharm.), bortezomib (VELCADE^{®}, Millennium Pharm.), disulfiram , epigallocatechin gallate , salinosporamide A, carfilzomib, 17-AAG(geldanamycin), radicicol, lactate dehydrogenase A (LDH-A), fulvestrant (FASLODEX^{®}, AstraZeneca), sunitib (SUTENT^{®}, Pfizer/Sugen), letrozole (FEMARA^{®}, Novartis), imatinib mesylate (GLEEVEC^{®}., Novartis), finasunate (VATALANIB^{®}, Novartis), oxaliplatin (ELOXATIN^{®}, Sanofi), 5-FU (5-fluorouracil), leucovorin, Rapamycin (Sirolimus, RAPAMUNE^{®}, Wyeth), Lapatinib (TYKERB^{®}, GSK572016, Glaxo Smith Kline), Lonafamib (SCH 66336), sorafenib (NEXAVAR^{®}, Bayer Labs), gefitinib (IRESSA^{®}, AstraZeneca), AG1478, alkylating agents such as thiotepa and CYTOXAN^{®} cyclosphosphamide; alkyl sulfonates such as busulfan, improsulfan and piposulfan; Eiziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; callystatin; CC-1065 (including its adozelesin, carzelesin and bizelesin synthetic analogs); cryptophycins (particularly cryptophycin I and cryptophycin 8); adrenocorticosteroids (including prednisone and prednisolone); cyproterone acetate; 5a-reductases including finasteride and dutasteride); vorinostat, romidepsin, panobinostat, valproic acid, mocetinostat dolastatin; aldesleukin, talc duocarmycin (including the synthetic analogs, KW-2189 and CBI-TM I); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin γI and calicheamicin coll (Angew Chem. Inti. Ed. Engl. 1994 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL (paclitaxel; Bristol-Myers Squibb Oncology, Princeton, N.J.), ABRAXANE^{®} (Cremophor-free), albumin-engineered nanoparticle formulations of paclitaxel (American Pharmaceutical Partners, Schaumberg, 111.), and TAXOTERE^{®} (docetaxel, doxetaxel; Sanofi-Aventis); chloranmbucil; GEMZAR^{®} (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE^{®} (vinorelbine); novantrone; teniposide; edatrexate; daunomycin; aminopterin; capecitabine (XELODA^{®}); ibandronate; CPT-I I; topoisomerase inhibitor RFS 2000; difluoromethylomithine (DMFO); retinoids such as retinoic acid; and pharmaceutically acceptable salts, acids and derivatives of any of the above.

Chemotherapeutic agent also includes (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, iodoxyfene , 4-hydroxytamoxifen, trioxifene, keoxifene,LYl 17018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RIVISOR^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAXID^{®}; PROLEUKIN^{®}, rIL-2; a topoisomerase I inhibitor such as LURTOTECAN^{®}; ABARELIX^{®} rmRH; and (ix) pharmaceutically acceptable salts, acids and derivatives of any of the above.

Chemotherapeutic agent also includes antibodies such as alemtuzumab (Campath), bevacizumab (AVASTIN^{®}, Genentech); cetuximab (ERBITUX^{®}, Imclone); panitumumab (VECTIBIX^{®}, Amgen), rituximab (RITUXAN^{®}, Genentech/Biogen Idee), pertuzumab (OMNITARG^{®}, 2C4, Genentech), trastuzumab (HERCEPTIN^{®}, Genentech), tositumomab (Bexxar, Corixia), and the antibody drug conjugate, gemtuzumab ozogamicin (MYLOTARG^{®}, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, full- length IgGi λ antibody genetically modified to recognize interleukin-12 p40 protein.

Chemotherapeutic agent also includes "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind toEGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX^{®}) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as El.l, E2.4, E2.5, E6.2, E6.4, E2.ll, E6. 3 and E7.6. 3 and described in US 6,235,883; MDX-447 (Medarex Inc); and mAb 806 or humanized mAb 806 (Johns et al, J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). EGFR antagonists include small molecules such as compounds described in US Patent Nos: 5,616,582, 5,457,105,5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFRantagonists include OSI-774 (CP-358774, erlotinib, TARCEVA^{®} Genentech/OSI Pharmaceuticals); PD 183805 (Cl 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA^{®}) 4-(3'-Chloro-4'-fluoroanilino)-7-methoxy-6-(3-morpholinopropoxy)quinazoline, AstraZeneca); ZM 105180 ((6-amino-4-(3-methylphenylamino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4- [4- [(I -phenylethyl)amino] -1 H-pyrrolo[2,3 -d]pyrimidin-6-yl] -phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl] -2-butynamide); EKB-569 (N- [4- [(3 -chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB^{®}, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR- targeted drugs noted in the preceding paragraph; small molecule FIER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-I inhibitors such as antisense agent ISIS-5132 available from ISIS Pharmaceuticals which inhibit Raf-I signaling; non-HER targeted TK inhibitors such as imatinib mesylate (GLEEVEC^{®}, available from Glaxo SmithKline); multi-targeted tyrosine kinase inhibitors such as sunitinib (SUTENT^{®}, available from Pfizer); VEGF receptor tyrosine kinase inhibitors such as vatalanib (PTK787/ZK222584, available from Novartis/Schering AG); MAPK extracellular regulated kinase I inhibitor Cl-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Wamer-Lamber); antisense molecules (e.g. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI- 1033 (Pfizer); Affinitac (ISIS 3521; Isis/Lilly); imatinib mesylate (GLEEVEC^{®}); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-ICl I (Imclone), rapamycin (sirolimus, RAPAMUNE^{®}); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca).

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone- 17-butyrate, clobetasol-17-propionate, fluocortolone caproate, fluocortolone pivalate and fluprednidene acetate; immune selective anti-inflammatory peptides (ImSAIDs) such as phenylalanine-glutamine-glycine (PEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNFa) blockers such as etanercept (Enbrel), infliximab (Remicade), adalimumab (Humira), certolizumab pegol (Cimzia), golimumab (Simponi), Interleukin I (IL-I) blockers such as anakinra (Kineret), T cell costimulation blockers such as abatacept (Orencia), Interleukin 6 (IL-6) blockers such as tocilizumab (ACTEMERA^{®}); Interleukin 13 (IL-13) blockers such as lebrikizumab; Interferon alpha (IFN) blockers such as Rontalizumab; Beta 7 integrin blockers such as rhuMAb Beta7; IgE pathway blockers such as Anti-Ml prime;Secreted homotrimeric LTa3 and membrane bound heterotrimer LTa I/β2 blockers such as Anti-lymphotoxin alpha (LTa); radioactive isotopes (e.g., At²¹¹, I¹³¹, I^{125,} Y⁹⁰, Re¹⁸⁶, Re¹⁸⁸, Sm¹⁵³, Bi²¹², P³², Pb²¹² and radioactive isotopes of Lu); miscellaneous investigational agents such as thioplatin, PS-341, phenylbutyrate, ET-18- OCH3, or famesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol, MARINOL^{®}); beta-lapachone; lapachol; colchicines; betulinic acid; acetylcamptothecin, scopolectin, and 9-aminocamptothecin); podophyllotoxin; tegafur (UFTORAL^{®}); bexarotene (TARGRETIN^{®}); bisphosphonates such as clodronate (for example, BONEFOS^{®} or OSTAC^{®}), etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}), or risedronate (ACTQNEL^{®}); and epidermal growth factor receptor (EGF-R); vaccines such as THERATOPE^{®} vaccine; perifosine, COX-2 inhibitor (e.g. celecoxib or etoricoxib), proteosome inhibitor (e.g. PS341); CCI-779; tipifamib (R11577); orafenib, ABT510; Bcl-2 inhibitor such as oblimersen sodium (GENASENSE^{®}); pixantrone; famesyltransferase inhibitors such as lonafamib (SCH 6636, SARASAR^{™}); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN^{™}) combined with 5-FU and leucovorin.

In another embodiment, compounds of formula (I) can be co-formulated with an immuno-oncology agent. Immuno-oncology agents include, for example, a small molecule drug, antibody, or other biologic or small molecule. Examples of biologic immuno-oncology agents include, but are not limited to, cancer vaccines, antibodies, and cytokines. In one aspect, the antibody is a monoclonal antibody. In another aspect, the monoclonal antibody is humanized or human. In another aspect, the antibody is a bispecific antibody.

In one aspect, the immuno-oncology agent is (i) an agonist of a stimulatory (including a co-stimulatory) receptor or (ii) an antagonist of an inhibitory (including a co-inhibitory) signal on T cells, both of which result in amplifying antigen-specific T cell responses (often referred to as immune checkpoint regulators).

Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAILR2/DR5, TRAILR3, TRAILR4, OPG, RANK, RANKL, TWEAKR/Fnl4, TWEAK, BAFFR, EDAR, XEDAR, TACI, APRIL, BCMA, LTfiR, LIGHT, DcR3, HVEM, VEGI/TL1A, TRAMP/DR3, EDAR, EDA1, XEDAR, EDA2,TNFR1, Lymphotoxin α/TNPβ, TNFR2, TNF a, LT R, Lymphotoxin a 1β2, FAS, FASL, RELT, DR6, TROY, NGFR.

In one aspect, T cell responses can be stimulated by a combination of a compound of formula (I) and one or more of (i) an antagonist of a protein that inhibits T cell activation (e.g., immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4, and (ii) an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

Other agents that can be combined with compounds of formula (I) for the treatment of cancer include antagonists of inhibitory receptors on NK cells or agonists of activating receptors on NK cells. For example, compounds of formula (I) can be combined with antagonists of KIR, such as lirilumab.

Yet other agents for combination therapies include agents that inhibit or deplete macrophages or monocytes, including but not limited to CSF-1R antagonists such as CSF-1R antagonist antibodies including RG7155 or FPA-008.

In another aspect, compounds of formula (I) can be used with one or more of agonistic agents that ligate positive costimulatory receptors, blocking agents that attenuate signaling through inhibitory receptors, antagonists, and one or more agents that increase systemically the frequency of antitumor T cells, agents that overcome distinct immune suppressive pathways within the tumor microenvironment (e.g., block inhibitory receptor engagement (e.g., PD-Ll/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell anergy or exhaustion) and agents that trigger innate immune activation and/or inflammation at tumor sites.

In some embodiments, the immuno-oncology agent is a CTLA-4 antagonist, such as an antagonistic CTLA-4 antibody. Suitable CTLA-4 antibodies include, for example, YERVOY (ipilimumab) or tremelimumab. In another aspect, the immuno-oncology agent is a PD-1 antagonist, such as an antagonistic PD-1 antibody. Suitable PD-1 antibodies include, for example, OPDIVO (nivolumab), KEYTRUDA (pembrolizumab), or MEDI-0680 (AMP-514; WO2012/145493). The immuno-oncology agent may also include pidilizumab (CT-011), though its specificity for PD-1 binding has been questioned. Another approach to target the PD-1 receptor is the recombinant protein composed of the extracellular domain of PD-L2 (B7-DC) fused to the Fc portion of IgGl, called AMP-224

In another aspect, the immuno-oncology agent is a PD-L1 antagonist, such as an antagonistic PD-L1 antibody. Suitable PD-L1 antibodies include, for example, TECENTRIQ (atezolizumab) (RG7446; WO2010/077634), durvalumab (MEDI4736), BMS-936559 (WO2007/005874), and MSB0010718C (WO2013/79174).

In another aspect, the immuno-oncology agent is a LAG-3 antagonist, such as an antagonistic LAG-3 antibody. Suitable LAG3 antibodies include, for example, BMS-986016 (WO2010/19570, WO2014/08218), or IMP-731 or IMP-321 (WO2008/132601, WO2009/44273).

In another aspect, the immuno-oncology agent is a CD137 (4-1BB) agonist, such as an agonistic CD137 antibody. Suitable CD137 antibodies include, for example, urelumab and PF-05082566 (WO2012/32433).

In another aspect, the immuno-oncology agent is a GITR agonist, such as an agonistic GITR antibody. Suitable GITR antibodies include, for example, BMS-986153, BMS-986156, TRX-518 (WO2006/105021, WO2009/009116) and MK-4166 (WO2011/028683).

In another aspect, the immuno-oncology agent is an IDO antagonist. Suitable IDO antagonists include, for example, INCB-024360 (WO2006/122150, WO2007/75598, WO2008/36653, WO2008/36642), indoximod, or NLG-919 (WO2009/73620, WO2009/1156652, WO2011/56652, WO2012/142237).

In another aspect, the immuno-oncology agent is an OX40 agonist, such as an agonistic OX40 antibody. Suitable OX40 antibodies include, for example, MEDI-6383 or MEDI-6469. In another aspect, the immuno-oncology agent is an OX40L antagonist, such as an antagonistic OX40 antibody. Suitable OX40L antagonists include, for example, RG-7888 (WO06/029879).

In another aspect, the immuno-oncology agent is a CD40 agonist, such as an agonistic CD40 antibody. In yet another embodiment, the immuno-oncology agent is a CD40 antagonist, such as an antagonistic CD40 antibody. Suitable CD40 antibodies include, for example, lucatumumab or dacetuzumab.

In another aspect, the immuno-oncology agent is a CD27 agonist, such as an agonistic CD27 antibody. Suitable CD27 antibodies include, for example, varlilumab.

In another aspect, the immuno-oncology agent is MGA271 (to B7H3) (WO2011/109400).

### Examples

The invention will be more fully understood by reference to the following examples. The claims should not, however, be construed as limited to the scope of the examples.

### 1) Preparative examples

All reaction examples and intermediates were prepared under an argon atmosphere if not specified otherwise.

### 1.1) General procedures

### • Nucleophilic substitution: General procedure 1a

A suspension of an intermediate of formula (XLI) (5.7 mmol), (tert-butoxycarbonyl)-L-cysteine (III) (5.7 mmol) and potassium carbonate (17.2 mmol) was stirred in DMF (10 mL) at RT overnight. The reaction was filtered, the solid washed with 5 mL DMF. The desired product (XLII) was used crude in the next step or was purified by flash column chromatography on silica gel or by reverse phase preparative HPLC.

### • Nucleophilic substitution: General procedure 1b

A solution of an intermediate of formula (II) (23 mmol) and (tert-butoxycarbonyl)-L-cysteine (III) (23 mmol) in 1,2-dichloroethane (150 mL) was treated with DIPEA (68.9 mmol). The reaction mixture was heated to 80°C and stirred for 2 h. The reaction mixture was cooled to RT and it was washed once with 1N aqueous HCl solution and extracted twice with DCM. The combined organic layers were washed with saturated aqueous sodium chloride solution, dried over Na₂SO₄ filtrated and concentrated in vacuo. The desired product (IV) was used crude in the next step or was purified by flash column chromatography on silica gel or by reverse phase preparative HPLC.

### • Reduction of nitro-group: General procedure 2

To a solution of an intermediate of formula (IV) (23 mmol) in EtOH (112 mL) and water (18.7 mL) was added 1N aqueous hydrogen chloride solution (2.3 mL). The reaction mixture was heated to 50°C and iron (8.99 g, 161 mmol) was added to the hot and stirred solution. The temperature was raised to 80°C and stirred for 3.5 h. The reaction mixture was cooled to RT and filtered over celite, washed with ethyl acetate and concentrated under reduced pressure. The desired product (V) was used crude in the next step.

### • Cyclization: General procedure 3

To a solution of of an intermediate of formula (V) (5.74 mmol) in DMF (15 mL) were added DIPEA (1.85 g, 2.51 mL, 14.4 mmol, 2.5 eq) and 2,4,6-tripropyl-1,3,5,2,4,6-trioxatriphosphinane 2,4,6-trioxide 50% solution in EtOAc (7.31 g, 6.76 mL). The reaction mixture was stirred at RT for 3 h. The reaction mixture was quenched with water and extracted twice with DCM, washed with 1M aqueous NaOH solution, 1M aqueous HCl solution and sat. aqueous NaCl solution, dried over Na₂SO₄, filtered and concentrated in vacuo. The desired product (VI) was used crude in the next step or was purified by flash column chromatography on silica gel or by reverse phase preparative HPLC.

### • Alkylation: General procedure 4

To a solution of an intermediate of formula (VI) (2.74 mmol) in DMSO (10 mL) were added at RT potassium carbonate (1.14 g, 8.23 mmol), potassium iodide (228 mg, 1.37 mmol) and a reagent of formula (VII) (3.29 mmol). The reaction was stirred at RT for 2 h, quenched with water and extracted twice with DCM. The combined organic layers were washed with water, saturated aqueous sodium chloride solution, dried over Na₂SO₄, filtered and the solvent was evaporated under reduced pressure. The desired product (VIII) was used crude in the next step or was purified by flash column chromatography on silica gel or by reverse phase preparative HPLC.

### • Oxidation: General procedure 5

A solution of an intermediate of formula (VIII) (2.74 mmol) and m-CPBA (1.18 g, 6.85 mmol) in DCM (10 mL) was stirred at RT for 1 day. The reaction was diluted with ethyl acetate and THF, washed with 2N aqueous sodium hydroxide solution, 1N aqueous HCl solution and saturated aqueous sodium chloride solution, dried over Na₂SO₄, filtered and the solvent was removed under reduced pressure. The desired product (IX) was used crude in the next step or was purified by flash column chromatography on silica gel or by reverse phase preparative HPLC.

### • Boc de-protection: General procedure 6a

To a solution of an intermediate of formula (IX) (38.9 µmol) in DCM (1 mL) was added 4M HCl in dioxane (200 µl, 800 µmol, 20.6 eq) and the reaction was stirred at RT overnight . The solvent was evaporated and the residue was suspended in DCM and diethyl ether. The solid was filtered off, washed with diethyl ether and dried in vacuo to yield desired product (I).

### • Boc de-protection: General procedure 6b

To a solution of an intermediate of formula (IX) (0.250 mmol) in EtOAc (4 mL) was added HCl/EtOAc (4.0 mL, 16 mmol, 63 eq) at 0 °C. The reaction mixture was stirred at 20°C for 3 h and then concentrated in vacuo. The remaining residue was purified by prep-HPLC and dried by lyophilization to obtain the desired product (I).

### • Boc de-protection: General procedure 6c

A solution of an intermediate of formula (IX) (22.7 µmol) in 1,1,1,3,3,3-hexafluoropropan-2-ol (1.5 mL) was stirred at reflux for 5 days. The solvent was evaporated and the remaining residue was dried under high vacuum to yield desired product (I).

### • Boc de-protection: General procedure 6d

To a solution of an intermediate of formula (IX) (0.250 mmol) in 1,1,1,3,3,3-hexafluoropropan-2-ol (4 mL) was added HCl/dioxane or HCl/Et₂O (0.5 mmol, 2 eq) at 0 °C. The reaction mixture was stirred at 20°C for 2 h. The solvent was evaporated and the resulting solid taken up in DCM and concentrated again to remove trace 1,1,1,3,3,3-hexafluoropropan-2-ol. This process was repeated two times followed by drying under high vacuum to obtain the desired product (I).

### • Boc de-protection: General procedure 6e

A solution of an intermediate of formula (IX) (80 µmol) in DCM (2.5 mL) and TFA (0.5 mL) was stirred at room temperature for 1 hour. The solvent was evaporated and the remaining residue was dried under high vacuum to yield desired product (I) or was taken up in MeOH and purified by e.g. prep-HPLC.

### • Hydrazide Coupling: General procedure 7a

To a solution of an intermediate of formula (XIII) (0.3 mmol) in THF (3 ml) was added a carboxylic acid of formula R10CO₂H (0.45 mmol), DIPEA (0.6 mmol) and HATU (0.45 mmol).

The resulting solution was stirred for 4 h at RT. The reaction mixture was diluted with EtOAc and water. The layers were separated and the aqueous phase extracted twice with EtOAc. The combined organic layers were washed with brine and then dried over sodium sulfate, filtered and the solvent evaporated under reduced pressure. The desired product (XIV) was used crude in the next step or purified by flash column chromatography on silica gel or by reverse phase preparative HPLC.

### • Hydrazide Coupling: General procedure 7b

To a solution of an intermediate of formula (XIII) (0.5 mmol) in THF (5 ml) was added a carboxylic acid of formula R10CO₂H (0.5 mmol), DIPEA (1.5 mmol) and T₃P (50% in EtOAc, 1.5 mmol). The resulting solution was stirred at 60 °C for 2 h. The reaction was then cooled to RT and diluted with water. The mixture was extracted three times with EtOAc. The combined organic layers were washed with brine and then dried over sodium sulfate, filtered and the solvent evaporated under reduced pressure. The desired product (XIV) was used crude in the next step or purified by flash column chromatography on silica gel or by reverse phase preparative HPLC.

### • 1,3,4-Oxadiazole cyclization: General procedure 8a

To a solution of an intermediate of formula (XIV) (0.3 mmol) in THF (3 ml) was added Burgess reagent (0.9 mmol). The resulting solution was stirred at RT overnight. Water was added and the mixture was extracted three times with EtOAc. The combined organic layers were washed with brine and then dried over sodium sulfate, filtered and the solvent evaporated under reduced pressure. The desired product (XV) was used crude in the next step or purified by flash column chromatography on silica gel or by reverse phase preparative HPLC.

### • 1,3,4-Oxadiazole cyclization: General procedure 8b

To a solution of an intermediate of formula (XIV) (0.1 mmol) in acetonitrile (1.3 ml) was added p-toluenesulfonyl chloride (0.3 mmol) and DIPEA (0.2 mmol). The resulting solution was stirred at RT for 30 min. The reaction was diluted with water and extracted three times with EtOAc. The combined organic layers were washed with brine and then dried over sodium sulfate, filtered and the solvent evaporated under reduced pressure. The desired product (XV) was used crude in the next step or purified by flash column chromatography on silica gel or by reverse phase preparative HPLC.

### • direct 1,2,4-Oxadiazole formation from amidoxime: General procedure 9a

To a solution of a carboxylic acid of formula R₁₀CO₂H (2.4 mmol) in DMF (5 ml) was added CDI (2.64 mmol) and stirred for 60 min. Then, a solution of an intermediate of formula (LV, wherein R is H) (1.2 mmol) in DMF (5 ml) was added and the resulting mixture heated to 120 °C for 4 h. The reaction was allowed to cool RT and water and EtOAc were added. The layers were separated and the aqueous phase extracted twice with EtOAc. The combined organic layers were washed with 1N HCl, dried over anhydrous sodium sulfate, filtered and the solvent evaporated under reduced pressure. The desired product (LVI) was used crude in the next step or purified by flash column chromatography on silica gel or by reverse phase preparative HPLC.

### • direct 1,2,4-Oxadiazole formation from amidoxime: General procedure 9b

To a solution of of an intermediate of formula (LV, wherein R is H) (0.3 mmol) in THF (5 mL) were added a carboxylic acid of formula R10CO₂H (0.45 mmol), DIPEA (0.76 mmol) and 2,4,6-tripropyl-1,3,5,2,4,6-trioxatriphosphinane 2,4,6-trioxide 50% solution in EtOAc (0.6 mmol) and the reaction was stirred at RT for 16 h. The reaction mixture was quenched with water and extracted twice with EtOAc, washed with 1M aqueous NaOH solution, 1M aqueous HCl solution and brine, dried over sodium sulfate, filtered and concentrated in vacuo. The desired product (LVI) was used crude in the next step or was purified by flash column chromatography on silica gel or by reverse phase preparative HPLC.

### • direct 1,2,4-Oxadiazole formation from amidoxime: General procedure 9c

To solution of an intermediate of formula (LV, wherein R is H) (0.2 mmol) in DMF (1.5 ml) was added a carboxylic acid of formula R10CO₂H (0.24 mmol), EDC hydrochloride (0.4 mmol), DIPEA (0.6 mmol) and HOBt (0.3 mmol) and the resulting mixture was heated to 80 °C for 8 h. The reaction was allowed to cool RT and water and EtOAc were added. The layers were separated and the aqueous phase extracted twice with EtOAc. The combined organic layers were washed with brine, dried over anhydrous sodium sulfate, filtered and the solvent evaporated under reduced pressure. The desired product (LVI) was used crude in the next step or purified by flash column chromatography on silica gel or by reverse phase preparative HPLC.

### • Amidoxime Coupling with RCO₂H: General procedure 10a

To a solution of an intermediate of formula (LV, wherein R is H) (1.0 mmol) in THF (8.5 ml) was added a carboxylic acid of formula R10CO₂H (0.12 mmol), DIPEA (2.0 mmol) and HATU (0.15 mmol) and the reaction was stirred at RT for 4 h. Water and EtOAc were added and the layers were separated. The aqueous phase was extracted twice with EtOAc. The combined organic layers were washed with brine, dried over anhydrous sodium sulfate, filtered and the solvent evaporated under reduced pressure. The desired product (LV, wherein R is -CO(R10)) was used crude in the next step or purified by flash column chromatography on silica gel or by reverse phase preparative HPLC.

### • Amidoxime Coupling with RCO₂H: General procedure 10b

To solution of an intermediate of formula (LV, wherein R is H) (0.2 mmol) in DMF (1.5 ml) was added a carboxylic acid of formula R10CO₂H (0.24 mmol), EDC hydrochloride (0.4 mmol), DIPEA (0.6 mmol) and HOBt (0.3 mmol) and the resulting mixture was stirred at RT for 16 h. Water and EtOAc were added, the layers were separated and the aqueous phase extracted twice with EtOAc. The combined organic layers were washed with brine, dried over anhydrous sodium sulfate, filtered and the solvent evaporated under reduced pressure. The desired product (LV, wherein R is -CO(R10)) was used crude in the next step or purified by flash column chromatography on silica gel or by reverse phase preparative HPLC.

### • Amidoxime Coupling with RCO₂H: General procedure 10c

To a solution of a carboxylic acid of formula R10CO₂H (0.11 mmol) in acetonitrile (0.33 ml) was added CDI (0.12 mmol) and stirred at RT for 60 min. To this mixture was then added a solution of an intermediate of formula (LV, wherein R is H) (0.1 mmol) in acetonitrile (0.33 ml) and stirred for 60 min at RT. The reaction was diluted with DCM and water was added. The layers were separated and the aqueous phase extracted twice with DCM. The combined organic layers were washed with brine, dried over anhydrous sodium sulfate, filtered and the solvent evaporated under reduced pressure. The desired product (LV, wherein R is -CO(R10)) was used crude in the next step or purified by flash column chromatography on silica gel or by reverse phase preparative HPLC.

### • 1,2,4-Oxadiazole cyclization: General procedure 11a

A solution of an intermediate of formula (LV, wherein R is -CO(R10)) (0.15 mmol) in toluene (1 ml) was heated to 120 °C for 16 h. The solvent was then evaporated under reduced pressure. The desired product (LVI) was used crude in the next step or purified by flash column chromatography on silica gel or by reverse phase preparative HPLC.

### • 1,2,4-Oxadiazole cyclization: General procedure 11b

To a solution of an intermediate of formula (LV, wherein R is -CO(R10)) (0.12 mmol) in THF (1.2 ml) was added tetrabutylammonium hydroxide (0.06 mmol) and stirred at RT for 30 min. The reaction was diluted with EtOAc and washed with sat. aq. NaHCO3. The aqueous phase was then washed twice with EtOAc and the combined organic layers dried over anhydrous sodium sulfate, filtered and the solvent evaporated under reduced pressure. The desired product (LVI) was used crude in the next step or purified by flash column chromatography on silica gel or by reverse phase preparative HPLC.

### • Amidoxime formation: General procedure 12

To a solution of an intermediate of formula (XXXVII) (0.3 mmol) in EtOH (2.5 ml) was added solid NaHCO₃ (1.5 mmol) and hydroxylamine hydrochloride (0.6 mmol). The resulting suspension was heated to 80 °C for 90 min and then allowed to cool to RT. The suspension was filtered and the filter cake washed with EtOH and DCM. The filtrate was concentrated under reduced pressure and the remaining solid was dissolved in DCM and washed with water and brine, dried over anhydrous sodium sulfate, filtered and the solvent evaporated under reduced pressure. The desired product (LV) was used crude in the next step or purified by flash column chromatography on silica gel or by reverse phase preparative HPLC.

### • Saponification: General procedure 13

To a solution of an intermediate of formula (VII) (4 mmol) in THF (18 ml), MeOH (3 ml) and water (6 ml) was added LiOH hydrate (8 mmol) and stirred at RT for 2 h. 1N HCl was added and the resulting suspension extracted three times with EtOAc. The combined organic layers were washed with brine and then dried over sodium sulfate, filtered and the solvent evaporated under reduced pressure. The desired product (XII) was used crude in the next step or purified by flash column chromatography on silica gel or by reverse phase preparative HPLC.

### • Hydrazide formation: General procedure 14

To a solution of an intermediate of formula (XII) (4.5 mmol) in THF (20 ml) was added CDI (5.7 mmol) and stirred at RT for 90 min. To this solution was then added a mixture of hydrazine hydrate (13.5 mmol) in THF (3.3 ml) and stirred for 1 h. The reaction mixture was diluted with water and EtOAc. The layers were separated and the aqueous phase washed twice with EtOAc. The combined organic layers were washed with brine and then dried over sodium sulfate, filtered and the solvent evaporated under reduced pressure. The desired product (XIII) was used crude in the next step or purified by flash column chromatography on silica gel or by reverse phase preparative HPLC.

### • Urea formation: General procedure 15

To a solution of an amine of formula HN(R10eR10f) (0.1 mmol) in 1,2-dichloroethane (0.4 ml) was added sat. aqueous sodium bicarbonate (3.7 mmol) and triphosgene (0.1 mmol) at 0 °C. The cooling bath was removed and stirring was continued for 2 h. To this solution was then added an intermediate of formula (XIII) and stirred for 2 h at RT. Water and DCM were added and the layers were separated. The aqueous layer was extracted twice with DCM and the combined organic layers were dried over anhydrous sodium sulfate, filtered and the solvent evaporated under reduced pressure. The desired product (LXXIV) was used crude in the next step or purified bxy flash column chromatography on silica gel or by reverse phase preparative HPLC.

### • Amine coupling with 1,2,4-oxadiazolon: General procedure 16

To a solution of an intermediate of formula (LXVIII) (0.06 mmol) in 1,4-dioxane (0.6 ml) was added an amine of formula HN(R10eR10f) (0.12 mmol), DIPEA (0.18 mmol) and PyBroP (0.072 mmol). The mixture was heated to 50 °C for 90 min. After cooling to RT, EtOAc and water were added and the reaction stirred vigorously for 5 min. The layers were separated and the aqueous phase extracted twice with EtOAc. The combined organic layers were dried over anhydrous sodium sulfate, filtered and concentrated in vacuo. The desired product (LXIX) was used crude in the next step or purified bxy flash column chromatography on silica gel or by reverse phase preparative HPLC.

### 1.2) Syntheses of examples

### Example 1

### (3R)-3-amino-5-[(4-chlorophenyl)methyl]-7-(4-ethyltriazol-1-yl)-8-fluoro-2,3-dihydro-1,5-benzothiazepin-4-one

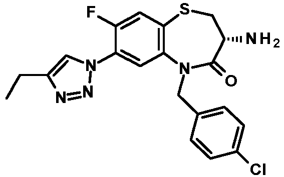

### Step a) (2R)-3-(2-amino-5-fluoro-4-nitro-phenyl)sulfanyl-2-(tert-butoxycarbonylamino)propanoic acid

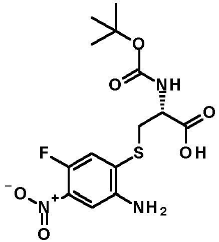

The title compound was prepared in analogy to general procedure **1a** using 2,4-difluoro-5-nitroaniline (CAS 123344-02-5) (1 g, 5.74 mmol, Eq: 1) and (tert-butoxycarbonyl)-L-cysteine (1.27 g, 5.74 mmol, Eq: 1) to yield the title compound (and 10% of a regioisomeric product) as a light yellow foam (109 % yield). The product was used crude in the next step. MS (ESI): 374.3 [M-H]⁻.

### Step b) tert-butyl N-[(3R)-8-fluoro-7-nitro-4-oxo-3,5-dihydro-2H-1,5-benzothiazepin-3-yl]carbamate

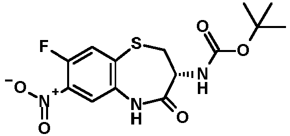

The title compound was prepared in analogy to general procedure 3 from (2R)-3-(2-amino-5-fluoro-4-nitro-phenyl)sulfanyl-2-(tert-butoxycarbonylamino)propanoic acid (2.15 g, 5.74 mmol, Eq: 1) and was obtained as an orange solid (0.98 g, 47% yield). MS (ESI): 356.3 [M-H]⁻.

### Step c) tert-butylN-[(3R)-5-[(4-chlorophenyl)methyl]-8-fluoro-7-nitro-4-oxo-2, 3-dihydro-1, 5-benzothiazepin-3-yl]carbamate

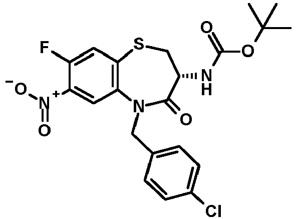

The title compound was prepared in analogy to general procedure 4 using 1-(bromomethyl)-4-chlorobenzene (676 mg, 3.29 mmol, Eq: 1.2) and tert-butyl N-[(3R)-8-fluoro-7-nitro-4-oxo-3,5-dihydro-2H-1,5-benzothiazepin-3-yl]carbamate (0.98 g, 2.74 mmol, Eq: 1) to yield the title compound as a light yellow foam (1.43 g, 97% yield, 90% purity). MS (ESI): 480.3 [M-H]⁻.

### Step d) tert-butyl N-[(3R)-7-amino-5-[(4-chlorophenyl)methyl]-8-fluoro-4-oxo-2,3-dihydro-1,5-benzothiazepin-3-yl]carbamate

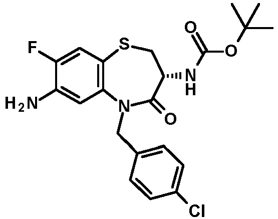

To a suspension of tert-butyl N-[(3R)-5-[(4-chlorophenyl)methyl]-8-fluoro-7-nitro-4-oxo-2,3-dihydro-1,5-benzothiazepin-3-yl]carbamate (4.53 g, 9.4 mmol, 1 eq) and ammonium chloride (1.01 g, 18.8 mmol, 2 eq) in MeOH (50 mL) was added zinc powder (6.15 g, 94 mmol, 10 eq) at 25 °C. The reaction mixture was purged with nitrogen three times and then heated to 70 °C and stirred for 2 h. The mixture was filtered and the filter cake was washed with hot MeOH (3 x 20 mL). The combined filtrates were concentrated in vacuo to obtain the crude product as a yellow oil. The crude product was purified by column chromatography on silica gel (EtOAc:PE = 1:4 to 100:0) to afford the desired title compound (3.29 g, 7.28 mmol, 77% yield) as a light yellow solid. MS (ESI): 396.0 [M-isobutene+H] ⁺.

### Step e) tert-butyl N-[(3R)-5-[(4-chlorophenyl)methyl]-7-(4-ethyltriazol-1-yl)-8-fluoro-4-oxo-2,3-dihydro-1, 5-benzothiazepin-3-yl]carbamate

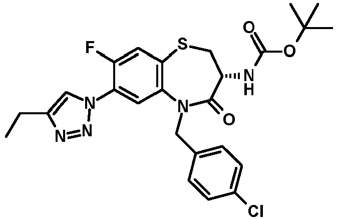

To a solution of tert-butyl tert-butyl N-[(3R)-7-amino-5-[(4-chlorophenyl)methyl]-8-fluoro-4-oxo-2,3-dihydro-1,5-benzothiazepin-3-yl]carbamate (300.0 mg, 0.660 mmol, 1 eq) in MeCN (6 mL) were added isopentyl nitrite (0.13 mL, 1 mmol, 1.5 eq) and azidotrimethylsilane (0.13 mL, 1 mmol, 1.5 eq) at 0°C. The reaction mixture was purged with nitrogen three times and then stirred for 2 h at 25°C. To the mixture were then added copper(I) oxide (9.5 mg, 0.070 mmol, 0.1 eq) and 1-butyne ( 98+% purity) (71.81 mg, 1.33 mmol, 2 eq) in MeCN (3 mL) at 25°C. The mixture was stirred for 2 h at 25°C, filtered, extracted with EtOAc (3 x 6 mL), washed with brine (8 mL) and dried with Na₂SO₄. After filtration, the organic layer was concentrated in vacuo to obtain the crude product (571 mg) as a brown oil. The crude product was purified by column chromatography on silica gel (EtOAc:PE = 1:9 to 2:3) to afford the desired title compound (254 mg, 0.48 mmol, 72% yield) as yellow oil. MS (ESI): 532.0 [M+H] ⁺.

### Step f) (3R)-3-amino-5-[(4-chlorophenyl)methyl]-7-(4-ethyltriazol-1-yl)-8-fluoro-2,3-dihydro-1,5-benzothiazepin-4-one

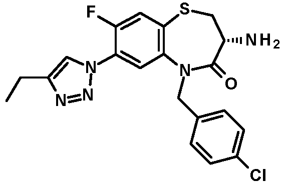

The title compound was prepared in analogy to general procedure 6b from tert-butyl N-[(3R)-5-[(4-chlorophenyl)methyl]-7-(4-ethyltriazol-1-yl)-8-fluoro-4-oxo-2,3-dihydro-1,5-benzothiazepin-3-yl]carbamate (55.0 mg, 0.1 mmol) and was obtained as a brown gum, as hydrochloride salt (48 mg, 0.1 mmol, 98% yield). MS (ESI): 432.0 [M+H] ⁺.

Example 2 of the following table was prepared in analogy to Example 1, using the appropriate alkyne building block.

*** obtained as a hydrochloride salt.**

| **Ex.** | **Structure** | **Systematic Name** | **Building Block** | **MS, ESI: m/z** |
|---|---|---|---|---|
| 2 | 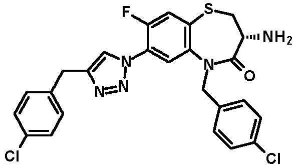 | (3R)-3-amino-5-[(4-chlorophenyl)methyl]-7-[4-[(4-chlorophenyl)methyl]triazol-1-yl]-8-fluoro-2,3-dihydro-1,5-benzothiazepin-4-one (*) | 1-Chloro-4-(2-propyn-1-yl)benzene | 528.0 [M+H]⁺ |
| | | | (CAS 70090-69-6) | |

### Example 3

### (3R)-3-amino-5-[(4-chlorophenyl)methyl]-7-(4-ethyltriazol-1-yl)-8-fluoro-1,1-dioxo-2,3-dihydro-1λ⁶,5-benzothiazepin-4-one

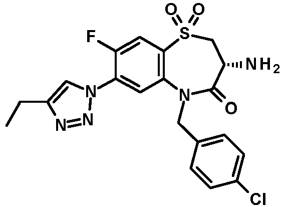

### Step a) tert-butyl N-[(3R)-5-[(4-chlorophenyl)methyl]-7-(4-ethyltriazol-1-yl)-8-fluoro-1,1,4-trioxo-2,3-dihydro-1λ⁶,5-benzothiazepin-3-yl]carbamate

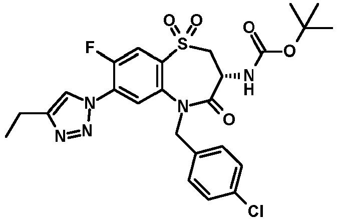

The title compound was prepared according to general procedure 5 from tert-butyl N-[(3R)-5-[(4-chlorophenyl)methyl]-7-(4-ethyltriazol-1-yl)-8-fluoro-4-oxo-2,3-dihydro-1,5-benzothiazepin-3-yl]carbamate (Example 1, step e) (199.0 mg, 0.370 mmol) and was obtained as yellow oil (220 mg) which was used in the next reaction step without further purification. MS (ESI): 564.0 [M+H] ⁺.

### Step b) (3R)-3-amino-5-[(4-chlorophenyl)methyl]-7-(4-ethyltriazol-1-yl)-8-fluoro-1,1-dioxo-2, 3-dihydro-1λ⁶,5-benzothiazepin-4-one

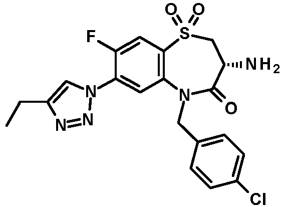

The title compound was prepared in analogy to general procedure 6b from tert-butyl N-[(3R)-5-[(4-chlorophenyl)methyl]-7-(4-ethyltriazol-1-yl)-8-fluoro-1,1,4-trioxo-2,3-dihydro-1λ⁶,5-benzothiazepin-3-yl]carbamate (220 mg, 0.39 mmol) and was obtained as a white solid and as a hydrochloride salt (75.3 mg, 0.150 mmol, 39% yield). MS (ESI): 464.0 [M+H] ⁺.

Examples 4 to 5 of the following table were prepared in analogy to Example 3, using the appropriate alkyne building blocks.

| **Ex.** | **Structure** | **Systematic Name** | **Building Blocks** | **MS, ESI: m/z** |
|---|---|---|---|---|
| 4 | 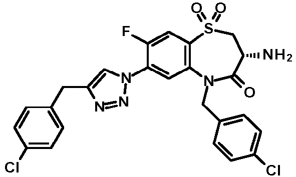 | (3R)-3-amino-5-[(4-chlorophenyl)methyl]-7-[4-[(4-chlorophenyl)methyl]triazol-1-yl]-8-fluoro-1,1-dioxo-2,3-dihydro-1λ⁶,5-benzothiazepin-4-one | 1-Chloro-4-(2-propyn-1-yl)benzene | 560.0 [M+H]⁺ |
| | | | (CAS 70090-69-6) | |
| | | (*) | | |
| 5 | 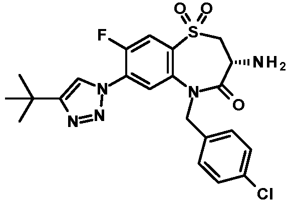 | (3R)-3-amino-7-(4-tert-butyltriazol-1-yl)-5-[(4-chlorophenyl)methyl]-8-fluoro-1,1-dioxo-2,3-dihydro-1λ⁶,5-benzothiazepin-4-one | 3,3-Dimethyl-1-butyne | 492.3 [M+H]⁺ |
| | | | (CAS 917-92-0) | |

| | | | | |
|---|---|---|---|---|
| * obtained as a hydrochloride salt | | | | |

### Example 6

### (3R)-3-amino-5-[(4-chlorophenyl)methyl]-7-(1-ethyltriazol-4-yl)-8-fluoro-1,1-dioxo-2,3-dihydro-λ⁶,5-benzothiazepin-4-one

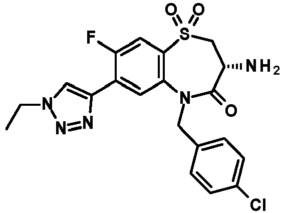

### Step a) tert-butyl N-[(3R)-7-amino-8-fluoro-4-oxo-3, 5-dihydro-2H-1, 5-benzothiazepin-3-yl]carbamate

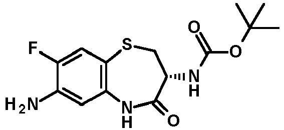

To a suspension of tert-butyl N-[(3R)-8-fluoro-7-nitro-4-oxo-3,5-dihydro-2H-1,5-benzothiazepin-3-yl]carbamate (Example 1, step b)) (6.0 g, 16.7 mmol, 1 eq) and ammonium chloride (2.05 g, 38.3 mmol, 2.29 eq) in MeOH (60 mL) was added zinc powder (7.0 g, 107.07 mmol, 6.38 eq) in batches at 25°C. Then the mixture was heated to 80°C and stirred for 6 h under an atmosphere of nitrogen. The reaction mixture was filtered and the filter cake was washed with hot MeOH (3 x 20 mL). The combined filtrates were concentrated in vacuo and the remaining residue was dissolved with EtOAc (20 mL). The organic layer was washed with brine, dried with Na₂SO₄ and concentrated in vacuo to afford the title compound (5.1 g, 15.58 mmol, 93% yield) as a brown solid. MS (ESI): 272.1 [M-isobutene-CO₂+H] ⁺.

### Step b) tert-butyl N-[(3R)-7-bromo-8-fluoro-4-oxo-3,5-dihydro-2H-1,5-benzothiazepin-3-yl]carbamate

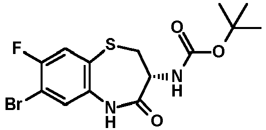

To a mixture of tert-butyl N-[(3R)-7-amino-8-fluoro-4-oxo-3,5-dihydro-2H-1,5-benzothiazepin-3-yl]carbamate (5.5 g, 10.9 mmol, 1 eq), copper(I) bromide (200 mg, 1.39 mmol, 0.13 eq) and copper(II) bromide (0.57 mL, 12.09 mmol, 1.11 eq) in MeCN (60 mL) was added dropwise tert-butyl nitrite (2.0 g, 19.39 mmol, 1.78 eq) at 0°C under an atmosphere of nitrogen. The mixture was stirred for 0.5 h at 0°C and then warmed to 25°C and stirred for 3.5 h. The reaction mixture was diluted with EtOAc (50 mL) and washed with brine. The organic layer was then dried with Na₂SO₄, filtered and concentrated in vacuo to afford a crude product which was purified by column chromatography on silica gel (4% to 25% EtOAc in PE) to obtain the title compound (1.9 g, 4.86 mmol, 44% yield) as yellow solid. MS (ESI): 337.0 [M-isobutene+H] ⁺.

### Step c) tert-butyl N-[(3R)-7-bromo-5-[(4-chlorophenyl)methyl]-8-fluoro-4-oxo-2,3-dihydro-1,5-benzothiazepin-3-yl[carbamate

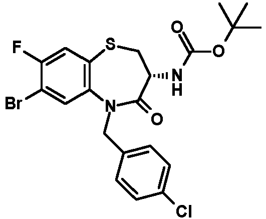

The title compound was prepared from tert-butyl N-[(3R)-7-bromo-8-fluoro-4-oxo-3,5-dihydro-2H-1,5-benzothiazepin-3-yl]carbamate (2000.0 mg, 5.11 mmol, 1 eq) and 1-(bromomethyl)-4-chlorobenzene (1157.89 mg, 5.64 mmol, 1.1 eq) in analogy to general procedure 4 and was obtained as orange solid (2000 mg, 3.88 mmol, 72% yield). MS (ESI): 461.1 [M-isobutene+H] ⁺.

### Step d) tert-butyl N-[(3R)-5-[(4-chlorophenyl)methyl]-8-fluoro-4-oxo-7-(2-trimethylsilylethynyl)-2,3-dihydro-1,5-benzothiazepin-3-yl]carbamate

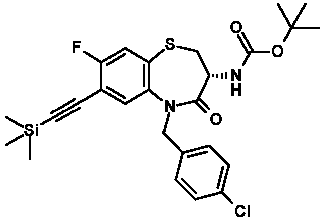

To a mixture of tert-butyl N-[(3R)-7-bromo-5-[(4-chlorophenyl)methyl]-8-fluoro-4-oxo-2,3-dihydro-1,5-benzothiazepin-3-yl]carbamate (200.0 mg, 0.390 mmol, 1 eq) in THF (2 mL) were added DIPEA(0.14 mL, 0.800 mmol, 2.08 eq), trimethylsilylacetylene (200.0 mg, 2.04 mmol, 5.25 eq), copper iodide (16.0 mg, 0.080 mmol, 0.220 eq) and bis(triphenylphosphine)palladium(II) chloride (52.0 mg, 0.070 mmol, 0.190 eq) under an atmosphere of nitrogen at 25 °C. The mixture was stirred for 16 h at 25°C and then concentrated. The remaining residue was purified by column chromatography on silica gel (10% to 50% EtOAc in PE) to afford the title compound (200 mg, 0.38 mmol, 97% yield) as a brown solid. MS (ESI): 477.1 [M-isobutene+H] ⁺.

### Step e) tert-butyl N-[(3R)-5-[(4-chlorophenyl)methyl]-7-ethynyl-8-fluoro-4-oxo-2,3-dihydro-1,5-benzothiazepin-3-yl]carbamate

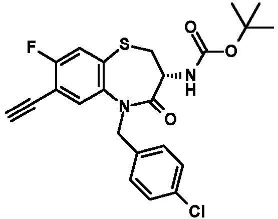

To a solution of tert-butyl N-[(3R)-5-[(4-chlorophenyl)methyl]-8-fluoro-4-oxo-7-(2-trimethylsilylethynyl)-2,3-dihydro-1,5-benzothiazepin-3-yl]carbamate (139.0 mg, 0.260 mmol, 1 eq) in MeOH (3 mL) was added potassium fluoride (55.08 mg, 0.950 mmol, 3.64 eq) at 15°C and the mixture was stirred at 15°C for 5 h. The mixture was concentrated in vacuo and the remaining residue was purified by preparative-TLC (PE:EtOAc = 3:1) to afford the title compound (96 mg, 0.21 mmol, 71% yield) as a dark brown oil. MS (ESI): 405.1 [M-isobutene+H] ⁺.

### Step f) tert-butylN-[(3R)-5-[(4-chlorophenyl)methyl]-7-(1-ethyltriazol-4-yl)-8-fluoro-4-oxo-2,3-dihydro-1, 5-benzothiazepin-3-yl]carbamate

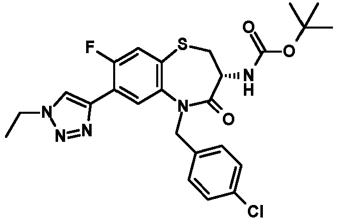

K₂CO₃ (70.0 mg, 0.51 mmol, 2.6 eq), CuSO₄•5H₂O (2.0 mg, 0.01 mmol, 0.04 eq) and 1H-imidazole-1-sulfonyl azide hydrochloride (70.0 mg, 0.330 mmol, 1.71 eq) were added to a solution of ethylamine (18.0 mg, 0.400 mmol, 2.04 eq) in MeOH (2 mL). The mixture was stirred for 14 h at 20°C and then tert-butyl N-[(3R)-5-[(4-chlorophenyl)methyl]-7-ethynyl-8-fluoro-4-oxo-2,3-dihydro-1,5-benzothiazepin-3-yl]carbamate (90.0 mg, 0.2 mmol, 1 eq), water (0.4 mL), CuSO₄•5H₂O (10.0 mg, 0.04 mmol, 0.21 eq) and sodium ascorbate (8.0 mg, 0.040 mmol, 0.210 eq) were added. The reaction mixture was stirred vigorously for 14 h at 20°C and poured into 1N NH₃•H₂O solution (5 mL). The mixture was extracted with EtOAc (3 x 5 mL) and the combined organic extracts were washed with brine (10 mL), dried with Na₂SO₄ and concentrated. The remaining residue was purified by column chromatography on silica gel (3-25% EtOAc in PE) to afford the title compound (100 mg, 0.19 mmol, 96% yield) as a brown oil. MS (ESI): 476.2 [M-isobutene+H] ⁺.

### Step g) tert-butylN-[(3R)-5-[(4-chlorophenyl)methyl]-7-(1-ethyltriazol-4-yl)-8-fluoro-1,1,4-trioxo-2, 3-dihydro-1λ⁶,5-benzothiazepin-3-yl]carbamate

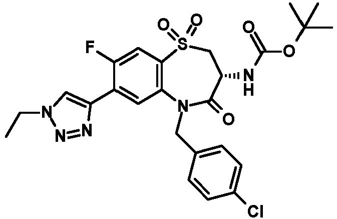

The title compound was prepared from tert-butyl N-[(3R)-5-[(4-chlorophenyl)methyl]-7-(1-ethyltriazol-4-yl)-8-fluoro-4-oxo-2,3-dihydro-1,5-benzothiazepin-3-yl]carbamate (70.0 mg, 0.130 mmol) in analogy to general procedure 5 to obtain a white solid (85 mg) containing the title compound which was used in the next reaction step without further purification. MS (ESI): 508.2 [M-isobutene+H] ⁺.

### Step h) (3R)-3-amino-5-[(4-chlorophenyl)methyl]-7-(1-ethyltriazol-4-yl)-8-fluoro-1,1-dioxo-2, 3-dihydro-1λ⁶,5-benzothiazepin-4-one

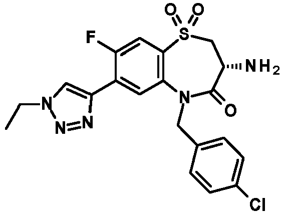

The title compound was prepared from tert-butyl N-[(3R)-5-[(4-chlorophenyl)methyl]-7-(1-ethyltriazol-4-yl)-8-fluoro-1,1,4-trioxo-2,3-dihydro-1λ⁶,5-benzothiazepin-3-yl]carbamate (85.0 mg, 0.150 mmol) in analogy to general procedure 6a and was obtained as white solid, as a hydrochloride salt (48.8 mg, 0.1 mmol, 65% yield). MS (ESI): 464.2 [M+H] ⁺.

### Example 7

### (3R)-3-amino-5-[(4-chlorophenyl)methyl]-7-(2-ethyltetrazol-5-yl)-8-fluoro-1,1-dioxo-2,3-dihydro-λ⁶,5-benzothiazepin-4-one

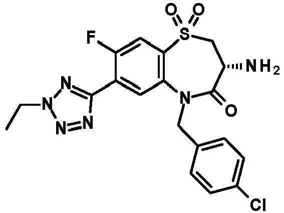

### Step a) tert-butyl N-[(3R)-5-[(4-chlorophenyl)methyl]-7-cyano-8-fluoro-4-oxo-2,3-dihydro-1,5-benzothiazepin-3-yl]carbamate

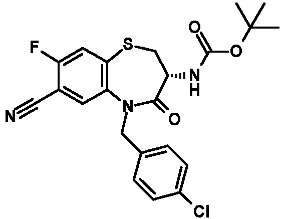

To a solution of tert-butyl tert-butyl N-[(3R)-7-bromo-5-[(4-chlorophenyl)methyl]-8-fluoro-4-oxo-2,3-dihydro-1,5-benzothiazepin-3-yl]carbamate (Example 6, step c) (300.0 mg, 0.58 mmol, 1 eq) in DMF (9 mL) were added Zn(CN)₂ (102.43 mg, 0.870 mmol, 1.5 eq), Zn powder (38.02 mg, 0.580 mmol, 1 eq), t-Bu₃P (0.12 mL, 0.120 mmol, 0.2 eq) and Pd₂(dba)₃ (53.26 mg, 0.060 mmol, 0.100 eq). The mixture was degassed with nitrogen and stirred at 50 °C for 16 h. The reaction was cooled to RT, EtOAc (30 mL) was added and the mixture was filtered through Celite (the celite was pre-washed with NaClO solution (20 mL) and 1 N HCl (15 mL)). The filtrate was washed with CaCl₂ solution (10 mL), water (2 x 5 mL) and brine (5 mL) and then it was dried with Na₂SO₄, filtered, and concentrated. The remaining residue was purified by column chromatography on silica gel (5 to 40% EtOAc in PE) to afford the title compound (260 mg, 0.56 mmol, 97% yield) as an off-white solid. MS (ESI): 406.1 [M-isobutene+H] ⁺.

### Step b) tert-butylN-[(3R)-5-[(4-chlorophenyl)methyl]-8-fluoro-4-oxo-7-(2H-tetrazol-5-yl)-2,3-dihydro-1, 5-benzothiazepin-3-yl]carbamate

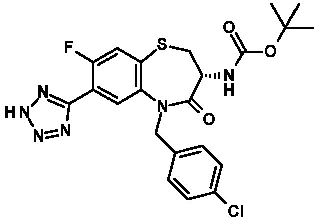

To a mixture of tert-butyl tert-butyl N-[(3R)-5-[(4-chlorophenyl)methyl]-7-cyano-8-fluoro-4-oxo-2,3-dihydro-1,5-benzothiazepin-3-yl]carbamate (250.0 mg, 0.540 mmol, 1 eq) in toluene (2.5 mL) were added tetra-N-butylammonium fluoride trihydrate (85.38 mg, 0.27 mmol, 0.5 eq) and azidotrimethylsilane (0.11 mL, 0.810 mmol, 1.5 eq) at 25°C. The mixture was purged with nitrogen for three times and then heated to 85°C and stirred for 16 h. The reaction was quenched by addition of saturated NH₄Cl solution (2 mL) and the mixture was then extracted with EtOAc (3 x 3 mL) and washed with brine (3 x 2 mL). The organic layer was concentrated in vacuo to afford the crude title compound (334 mg) as a brown oil which was used in next step without further purification. MS (ESI): 449 [M-isobutene+H] ⁺.

### Step c) tert-butyl N-[(3R)-5-[(4-chlorophenyl)methyl]-7-(2-ethyltetrazol-5-yl)-8-fluoro-4-oxo-2,3-dihydro-1,5-benzothiazepin-3-yl]carbamate

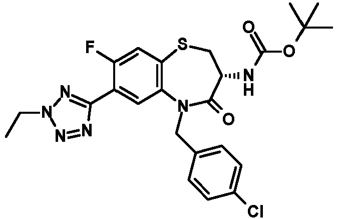

To a solution of tert-butyl N-[(3R)-5-[(4-chlorophenyl)methyl]-8-fluoro-4-oxo-7-(2H-tetrazol-5-yl)-2,3-dihydro-1,5-benzothiazepin-3-yl]carbamate (334.0 mg, 0.660 mmol, 1 eq) in DMF (3 mL) were added iodoethane (0.08 mL, 0.990 mmol, 1.5 eq) and potassium carbonate (137.12 mg, 0.990 mmol, 1.5 eq) at 25°C and the mixture was stirred for 16 h at 25°C. Then water (3 mL) was added and the mixture was extracted with EtOAc (3 x 3 mL). The organic extracts were washed with brine (3 x 8 mL), dried with anhydrous Na₂SO₄, filtered and concentrated in vacuo to afford a crude material (579 mg) as a yellow oil. The crude product was purified by column chromatography on silica gel (10% to 50% EtOAc in PE) to afford desired title compound (30 mg, 0.06 mmol, 8.5% yield) as a yellow oil. MS (ESI): 477 [M-isobutene+H] ⁺.

### Step d) tert-butyl N-[(3R)-5-[(4-chlorophenyl)methyl]-7-(2-ethyltetrazol-5-yl)-8-fluoro-1,1,4-trioxo-2,3-dihydro-1λ⁶,5-benzothiazepin-3-yl]carbamate

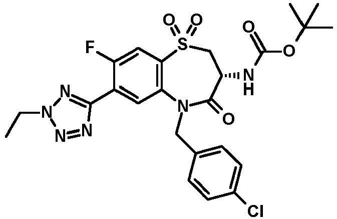

The title compound was prepared from tert-butyl N-[(3R)-5-[(4-chlorophenyl)methyl]-7-(2-ethyltetrazol-5-yl)-8-fluoro-4-oxo-2,3-dihydro-1,5-benzothiazepin-3-yl]carbamate (30.0 mg, 0.060 mmol) in analogy to general procedure 5 and was obtained as yellow oil (30 mg, 0.05 mmol, 94% yield). MS (ESI): 509 [M-isobutene+H]⁺.

### Step e) (3R)-3-amino-5-[(4-chlorophenyl)methyl]-7-(2-ethyltetrazol-5-yl)-8-fluoro-1,1-dioxo-2,3-dihydro-1λ⁶,5-benzothiazepin-4-one

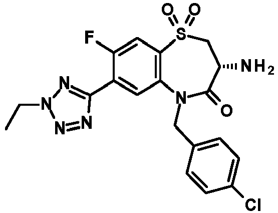

The title compound was prepared from tert-butyl N-[(3R)-5-[(4-chlorophenyl)methyl]-7-(2-ethyltetrazol-5-yl)-8-fluoro-1,1,4-trioxo-2,3-dihydro-1λ⁶,5-benzothiazepin-3-ylcarbamate (30.0 mg, 0.05 mmol) in analogy to general procedure **6b** and was obtained as white solid, as a hydrochloride salt (12.6 mg, 0.03 mmol, 47% yield). MS (ESI): 464.9 [M+H] ⁺.

### Example 8

### (3R)-3-amino-7-(2-tert-butyltetrazol-5-yl)-5-[(4-chlorophenyl)methyl]-8-fluoro-1,1-dioxo-2,3-dihydro-1λ⁶,5-benzothiazepin-4-one

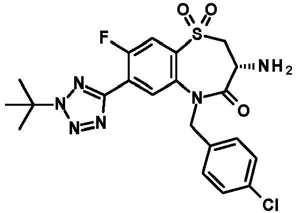

### Step a) tert-butyl N-[(3R)-5-[(4-chlorophenyl)methyl]-7-cyano-8-fluoro-1,1,4-trioxo-2, 3-dihydro-1λ⁶,5-benzothiazepin-3-yl]carbamate

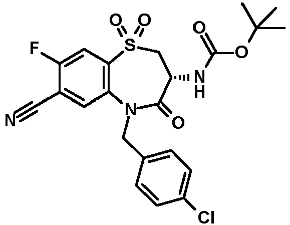

The title compound was prepared from tert-butyl N-[(3R)-5-[(4-chlorophenyl)methyl]-7-cyano-8-fluoro-4-oxo-2,3-dihydro-1,5-benzothiazepin-3-yl]carbamate (400.0 mg, 0.870 mmol, 1 eq) in analogy to general procedure 5 and was obtained as yellow solid (45 mg, 0.080 mmol, 65% yield). MS (ESI): 438.1 [M-isobutene+H] ⁺.

### Step b) tert-butyl N-[(3R)-5-[(4-chlorophenyl)methyl]-8-fluoro-1,1,4-trioxo-7-(2H-tetrazol-5-yl)-2,3-dihydro-1λ⁶,5-benzothiazepin-3-yl[carbamate

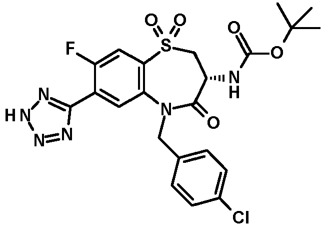

A solution of tert-butyl N-[(3R)-5-[(4-chlorophenyl)methyl]-7-cyano-8-fluoro-1,1,4-trioxo-2,3-dihydro-1λ⁶,5-benzothiazepin-3-yl]carbamate (440.0 mg, 0.890 mmol, 1 eq) and azidotrimethyltin (550.06 mg, 2.67 mmol, 3 eq) in p-xylene (10 mL) was stirred for 6 h under an atmosphere of nitrogen at 120°C. After cooling to ambient temperature, a solution of NaNO₂ (172 mg) in water (0.8 mL) was added to the mixture with stirring. Then the mixture was slowly acidified by addition of aqueous HCl (1 N) to adjust the pH to 3 and stirring was continued for 1 h until no more gas evolution was observed. After that, the mixture was basified with aqueous NaOH (1 N) to pH=8 and extracted with EtOAc (5 mL x 2). The combined organic layers were washed with brine (10 mL x 3), dried over anhydrous Na₂SO₄ and concentrated under vacuum. To the remaining residue mixture was added PE (20 mL) slowly until no more precipitation was observed. After filtration, the filter cake was dried under vacuum to afford the title compound (570 mg, 1.06 mmol, 104.87% yield) as a yellow solid. MS (ESI): 481.1 [M-isobutene+H] ⁺.

### Step c) (3R)-3-amino-7-(2-tert-butyltetrazol-5-yl)-5-[(4-chlorophenyl)methyl]-8-fluoro-1,1-dioxo-2, 3-dihydro-1λ⁶,5-benzothiazepin-4-one

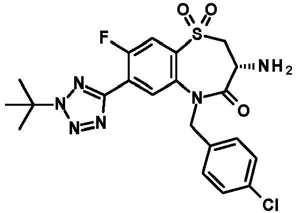

A solution of tert-butyl N-[(3R)-5-[(4-chlorophenyl)methyl]-8-fluoro-1,1,4-trioxo-7-(2H-tetrazol-5-yl)-2,3-dihydro-1λ⁶,5-benzothiazepin-3-yl]carbamate (200.0 mg, 0.370 mmol, 1 eq) and tBuOH (0.04 mL, 0.450 mmol, 1.2 eq) in H₂SO₄ (2.0 mL, 0.370 mmol, 1 eq) was stirred at 25°C for 2 h. The reaction mixture was added into saturated Na₂CO₃ aqueous (10 mL) drop-wise. The resulting mixture was extracted with EtOAc (5 mL x 2) and the combined organic phases were washed with brine (20 mL x 2), dried over anhydrous Na₂SO₄ and concentrated. The remaining residue was purified by prep-HPLC (column: Phenomenex Gemini-NX C18 75*30mm*3um; water (0.05% ammonia hydroxide v/v)-MeCN;B%: 33%-63%,11.5min) affording the title compound (39.5 mg, 0.080 mmol, 21.3% yield) as a white solid. MS (ESI): 493.2 [M+H] ⁺.

### Example 9

### (3R)-3-amino-5-[(4-chlorophenyl)methyl]-8-fluoro-1,1-dioxo-7-[2-[(3S)-1-methylpyrrolidin-3-yl]tetrazol-5-yl]-2,3-dihydro-1λ⁶,5-benzothiazepin-4-one

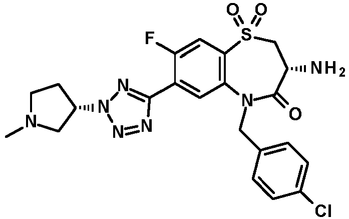

### Step a) tert-butyl N-[(3R)-5-[(4-chlorophenyl)methyl]-8-fluoro-1,1,4-trioxo-7-[2-[(3S)-1-methylpyrrolidin-3-yl]tetrazol-5-yl]-2,3-dihydro-1λ⁶,5-benzothiazepin-3-yl]carbamate

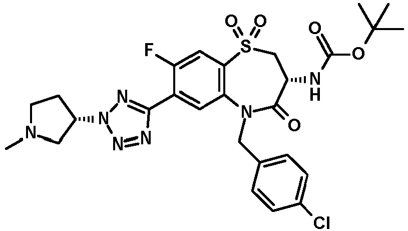

N-[(3R)-5-(4-chlorobenzyl)-8-fluoro-1,1,4-triketo-7-(2H-tetrazol-5-yl)-2,3-dihydro-1λ6,5-benzothiazepin-3-yl]carbamic acid tert-butyl ester (50 mg, 0.093 mmol, 1 eq) was stirred in toluene (1 mL) with (3R)-1-methylpyrrolidin-3-ol (11.3 mg, 12.2 uL, 0.11 mmol, 1.2 eq) (CAS 104641-60-3), DIAD (37.6 mg, 36.2 uL, 0.186 mmol, 2 eq) and Ph₃P (48.8 mg, 0.18 mmol, 2 eq) at 50°C for 2 hours. Another batch of reactants was added DIAD (37.6 mg, 36.2 uL, 0.18 mmol, 2 eq), Ph₃P (48.8 mg, 0.18 mmol, 2 eq), (3R)-1-methylpyrrolidin-3-ol (11.3 mg, 12.2 uL, 0.11 mmol, 1.2 eq) and stirred for another 2 hours at 50 °C. The reaction was cooled to RT and diluted with EtOAc and water was added. The phases were separated and the aq. phase washed twice with EtOAc. The combined orgamic phases were dried over Na₂SO₄, filtered and concentrated. The crude product was purified by prep-HPLC and then by column chromatography on silica gel (100 % EtOAc) to afford the title compound (31 mg, 53% yield) as white solid. MS (ESI): 618.3 [M-H]⁻.

### Step b) (3R)-3-amino-5-[(4-chlorophenyl)methyl]-8-fluoro-1,1-dioxo-7-[2-[(3S)-1-methylpyrrolidin-3-yl]tetrazol-5-yl]-2,3-dihydro-1λ⁶,5-benzothiazepin-4-one

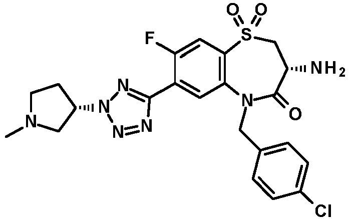

4 M HCl in dioxane (29.2 uL, 0.11 mmol, 2.5 eq) was stirred with tert-butyl N-[(3R)-5-[(4-chlorophenyl)methyl]-8-fluoro-1,1,4-trioxo-7-[2-[(3S)-1-methylpyrrolidin-3-yl]tetrazol-5-yl]-2,3-dihydro-1λ⁶,5-benzothiazepin-3-yl]carbamate (29 mg, 0.047 mmol, 1 eq) in 1,1,1,3,3,3-hexafluoro-2-propanol (2.34 mL) at room temperature for 75 min The solvent was evaporated and the crude resuspended in DCM and concentrated again. This process was repeated 2x and the resulting solid then dried in vacuo to afford the title compounds as a bis hydrochloride salt (27.5 mg, 99% yield) as white solid. MS (ESI): 260.7 [M/2+H]⁺.

Examples 10 to 15 of the following table were prepared in analogy to Example 9 in two steps, using the appropriate alcohol building block.

| **Ex.** | **Structure** | **Systematic Name** | **Building Block** | **MS, ESI: m/z** |
|---|---|---|---|---|
| 10 | 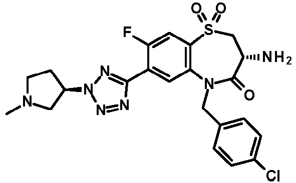 | (3R)-3-amino-5-[(4-chlorophenyl)methyl]-8-fluoro-1,1-dioxo-7-[2-[(3R)-1-methylpyrrolidin-3-yl]tetrazol-5-yl]-2,3-dihydro-1λ⁶,5-benzothiazepin-4-one (*) | (3S)-1-Methylpyrr olidin-3-ol (CAS 104641-59-0) | 260.7 [M/2+ H]⁺ |
| 11 | 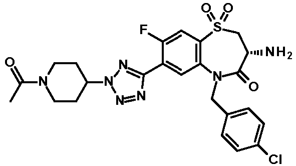 | (3R)-7-[2-(1-acetyl-4-piperidyl)tetrazol-5-yl]-3-amino-5-[(4-chlorophenyl)methyl]-8-fluoro-1,1-dioxo-2,3-dihydro-1λ⁶,5-benzothiazepin-4-one (**) | 1-(4-Hydroxypiper idin-1-yl)ethanone | 562.1 [M+H]⁺ |
| 12 | 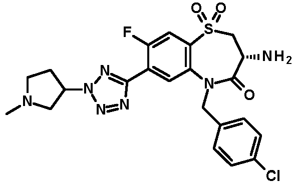 | (3R)-3-amino-5-[(4-chlorophenyl)methyl]-8-fluoro-7-[2-(1-methylpyrrolidin-3-yl)tetrazol-5-yl]-1,1-dioxo-2,3-dihydro-1λ⁶,5-benzothiazepin-4-one (**) | 1-Methyl-3-pyrrolidinol | 520.2 [M+H]⁺ |
| 13 | 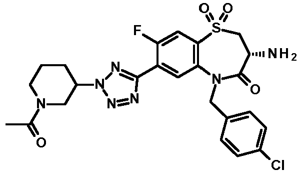 | (3R)-7-[2-(1-acetyl-3-piperidyl)tetrazol-5-yl]-3-amino-5-[(4-chlorophenyl)methyl]-8-fluoro-1,1-dioxo-2,3-dihydro-1λ⁶,5-benzothiazepin-4-one (*) | 1-(3-Hydroxypiper idin-1-yl)ethanone | 562.2 [M+H]⁺ |
| 14 | 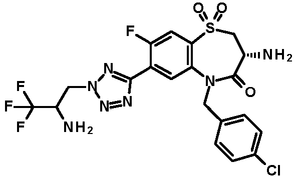 | (3R)-3-amino-7-[2-(2-amino-3,3,3-trifluoro-propyl)tetrazol-5-yl]-5-[(4-chlorophenyl)methyl]-8-fluoro-1,1-dioxo-2,3-dihydro-1λ⁶,5-benzothiazepin-4-one (*) | N-(2,2,2-Trifluoro-1-methylol-ethyl)carbamic acid tert-butyl ester | 548.1 [M+H]⁺ |
| 15 | 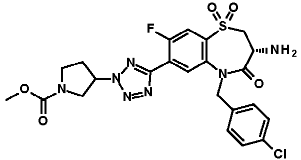 | methyl 3-[5-[(3R)-3-amino-5-[(4-chlorophenyl)methyl]-8-fluoro-1,1,4-trioxo-2,3-dihydro-1λ⁶,5-benzothiazepin-7-yl]tetrazol-2-yl]pyrrolidine-1-carboxylate (*) | 3-Hydroxypyrro lidine-1-carboxylic acid methyl ester | 564.2 [M+H]⁺ |

| | | | | |
|---|---|---|---|---|
| * obtained as a hydrochloride salt using general method 6a. ** obtained as a hydrochloride salt using general method 6b. | | | | |

### Example 16

### (3R)-3-amino-5-[(4-chlorophenyl)methyl]-8-fluoro-7-(2-methyltetrazol-5-yl)-1,1-dioxo-2,3-dihydro-λ⁶,5-benzothiazepin-4-one

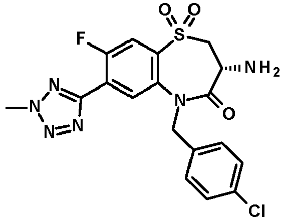

### Step a) tert-butyl N-[(3R)-5-[(4-chlorophenyl)methyl]-8-fluoro-7-(2-methyltetrazol-5-yl)-1,1,4-trioxo-2, 3-dihydro-1λ⁶,5-benzothiazepin-3-yl]carbamate

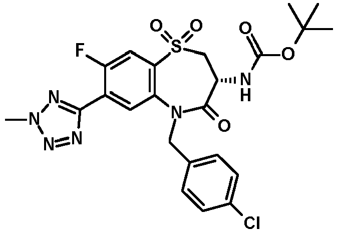

The title compound was prepared from tert-butyl N-[(3R)-5-[(4-chlorophenyl)methyl]-8-fluoro-1,1,4-trioxo-7-(2H-tetrazol-5-yl)-2,3-dihydro-1λ⁶,5-benzothiazepin-3-yl]carbamate (30.0 mg, 0.06 mmol) and methyliodide in analogy to Example 7, step c) and was obtained as yellow solid (24 mg, 0.04 mmol, 60% yield). MS (ESI): 495.1 [M-isobutene+H] ⁺.

### Step b) (3R)-3-amino-5-[(4-chlorophenyl)methyl]-8-fluoro-7-(2-methyltetrazol-5-yl)-1,1-dioxo-2,3-dihydro-1λ⁶,5-benzothiazepin-4-one

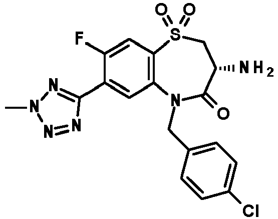

The title compound was prepared from tert-butyl N-[(3R)-5-[(4-chlorophenyl)methyl]-8-fluoro-7-(2-methyltetrazol-5-yl)-1,1,4-trioxo-2,3-dihydro-1λ⁶,5-benzothiazepin-3-yl]carbamate (60 mg, 0.11 mmol) in analogy to general procedure 6b and was obtained as white solid as a hydrochloride salt (24 mg, 0.05 mmol, 43% yield). MS (ESI): 451.1 [M+H] ⁺.

### Example 17

### (3R)-3-amino-5-[(4-chlorophenyl)methyl]-8-fluoro-7-[2-(1-methylsulfonyl-4-piperidyl)tetrazol-5-yl]-1,1-dioxo-2,3-dihydro-1λ⁶,5-benzothiazepin-4-one

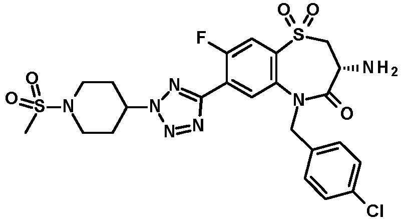

### Step a) tert-butyl 4-[5-[(3R)-3-(tert-butoxycarbonylamino)-5-[(4-chlorophenyl)methyl]-8-fluoro-1,1,4-trioxo-2,3-dihydro-1λ⁶,5-henzothiazepin-7-yl]tetrazol-2-yl]piperidine-1-carhoxylate

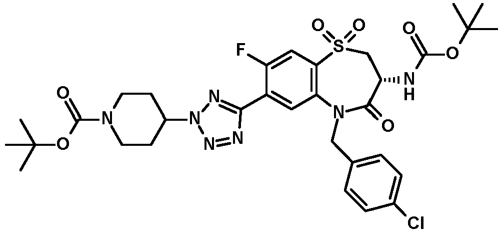

The title compound was prepared from tert-butyl N-[(3R)-5-[(4-chlorophenyl)methyl]-8-fluoro-1,1,4-tiioxo-7-(2H-tetrazol-5-yl)-2,3-dihydro-1λ6,5-benzothiazepin-3-yl]carbamate (100 mg, 0.19 mmol) and 1-boc-4-hydroxypiperidine in analogy to example 9, step a) and was obtained as white solid (130 mg, 0.18 mmol, 96% yield). MS (ESI): 620.2 [M-isobutene-CO₂+H]⁺.

### Step b) (3R)-3-amino-5-[(4-chlorophenyl)methyl]-8-fluoro-1,1-dioxo-7-[2-(4-piperidyl)tetrazol-5-yl]-2,3-dihydro-1λ⁶,5-benzothiazepin-4-one

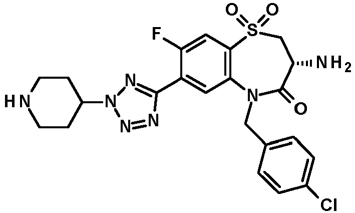

The title compound was prepared from tert-butyl 4-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate (130 mg, 0.18 mmol) in analogy to general procedure 6b and was obtained as white solid as a dihydrochloride salt (107 mg, 0.18 mmol, 99% yield). MS (ESI): 520.1 [M+H]⁺.

### Step c) (3R)-3-amino-5-[(4-chlorophenyl)methyl]-8-fluoro-7-[2-(1-methylsulfonyl-4-piperidyl) tetrazol-5-yl]-1,1-dioxo-2,3-dihydro-1λ⁶,5-benzothiazepin-4-one

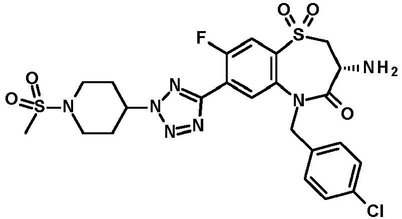

To a solution of (3R)-3-amino-5-[(4-chlorophenyl)methyl]-8-fluoro-1,1-dioxo-7-[2-(4-piperidyl)tetrazol-5-yl]-2,3-dihydro-1λ⁶,5-benzothiazepin-4-one dihydrochloride salt (100 mg, 0.17 mmol, 1 eq) in DCM (2 mL) was added NEt₃ (54.6 mg, 0.54 mmol, 3.2 eq) and methanesulfonyl chloride (0.02 mL, 0.20 mmol, 1.2 eq), the mixture was stirred at 10 °C for 0.5 h, concentrated and purified by prep-HPLC. The elution was lyophilized to yield the title compound (25.7 mg, 0.04 mmol, 24% yield) as a white solid as the hydrochloride salt. MS (ESI): 598.0 [M+H]⁺.

Example 18 of the following table was prepared in analogy to Example 17 in three steps, using the appropriate alcohol and sulfonylating building block.

*** obtained as a hydrochloride salt.**

| **Ex.** | **Structure** | **Systematic Name** | **Building Blocks** | **MS, ESI: m/z** |
|---|---|---|---|---|
| 18 | 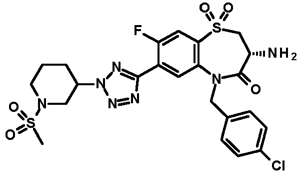 | (3R)-3-amino-5-[(4-chlorophenyl)methyl]-8-fluoro-7-[2-(1-methylsulfonyl-3-piperidyl)tetrazol-5-yl]-1,1-dioxo-2,3-dihydro-1λ⁶,5-benzothiazepin-4-one (*) | 1-Boc-3-hydroxypip eridine / methane sulfonyl chloride | 598.1 [M+H]⁺ |

### Example 19

### (3R)-3-amino-5-[(4-chlorophenyl)methyl]-8-fluoro-7-[2-(1-methylsulfonylpyrrolidin-3-yl)tetrazol-5-yl]-1,1-dioxo-2,3-dihydro-λ⁶,5-benzothiazepin-4-one

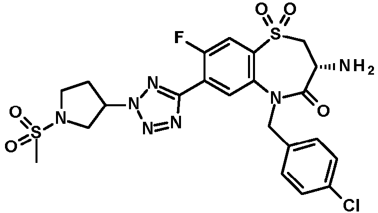

### Step a) benzyl 3-[5-[(3R)-3-(tert-butoxycarbonylamino)-5-[(4-chlorophenyl)methyl]-8-fluoro-1, 1, 4-trioxo-2, 3-dihydro-1λ⁶,5-benzothiazepin-7-yl]tetrazol-2-yl]pyrrolidine-1-carboxylate

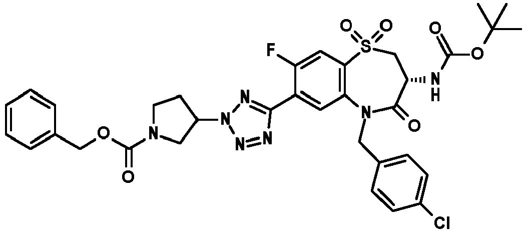

The title compound was prepared from tert-butyl N-[(3R)-5-[(4-chlorophenyl)methyl]-8-fluoro-1,1,4-tiioxo-7-(2H-tetrazol-5-yl)-2,3-dihydro-1λ6,5-benzothiazepin-3-yl]carbamate (50.0 mg, 0.09 mmol) and 1-Cbz-3-pyrrolidinol in analogy to example 9, step a) and was obtained as light brown solid (47 mg, 0.06 mmol, 62% yield). MS (ESI): 640.2 [M isobutene-CO₂+H]⁺.

### Step b) tert-butyl N-[(3R)-5-[(4-chlorophenyl)methyl]-8-fluoro-1, 1, 4-trioxo-7-(2-pyrrolidin-3-yltetrazol-5-yl)-2,3-dihydro-1λ⁶,5-benzothiazepin-3-yl]carbamate

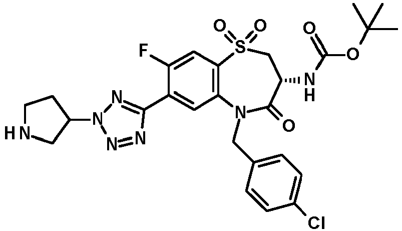

To a solution of benzyl 3-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]pyrrolidine-1-carboxylate (47.0 mg, 0.06 mmol, 1 eq) in MeOH (1 mL) was added Pd/C (0.03 mL, 0.13 mmol, 2 eq) the mixture was degassed with H₂ for 3 times, then stirred at 25 °C for 2 h. After filtration, the filtrate was concentrated to afford the crude title compound (28 mg, 0.05 mmol, 34% yield, 47% purity) as a light brown solid. MS (ESI): 606.2 [M+H]⁺.

### Step c) tert-butyl N-[(3R)-5-[(4-chlorophenyl)methyl]-8-fluoro-7-[2-(1-methylsulfonylpyrrolidin-3-yl)tetrazol-5-yl]-1,1,4-trioxo-2,3-dihydro-1λ⁶,5-henzothiazepin-3-yl]carbamate

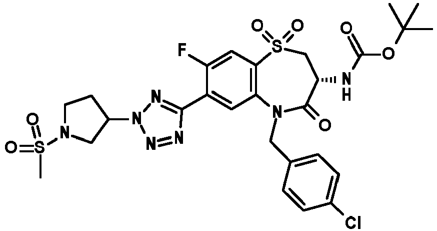

The title compound was prepared from tert-butyl N-[(3R)-5-[(4-chlorophenyl)methyl]-8-fluoro-1,1,4-trioxo-7-(2-pyrrolidin-3-yltetrazol-5-yl)-2,3-dihydro-1λ⁶,5-benzothiazepin-3-yl]carbamate (40 mg, 0.07 mmol) and methanesulfonyl chloride in analogy to example 17, step c) and was obtained as light yellow gum (24 mg, 0.04 mmol, 53% yield). MS (ESI): 584.2 [M-isobutene-CO₂+H]⁺.

### Step d) (3R)-3-amino-5-[(4-chlorophenyl)methyl]-8-fluoro-7-[2-(1-methylsulfonylpyrrolidin-3-yl)tetrazol-5-yl]-1,1-dioxo-2,3-dihydro-1λ⁶,5-benzothiazepin-4-one

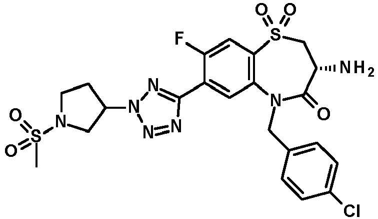

The title compound was prepared from tert-butyl N-[(3R)-5-[(4-chlorophenyl)methyl]-8-fluoro-7-[2-(1-methylsulfonylpyrrolidin-3-yl)tetrazol-5-yl]-1,1,4-trioxo-2,3-dihydro-1λ⁶,5-benzothiazepin-3-yl]carbamate (60.0 mg, 0.11 mmol) in analogy to general procedure **6a** and was obtained as white solid, as a hydrochloride salt (7.3 mg, 0.01 mmol, 32% yield). MS (ESI): 584.0 [M+H]⁺.

### Example 20

### (3R)-3-amino-5-[(4-chlorophenyl)methyl]-8-fluoro-7-[2-(1-methyl-3-piperidyl)tetrazol-5-yl]-1,1-dioxo-2,3-dihydro-1λ⁶ 5-benzothiazepin-4-one

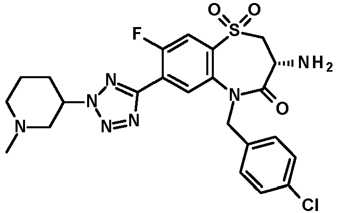

### Step a) benzyl 3-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate

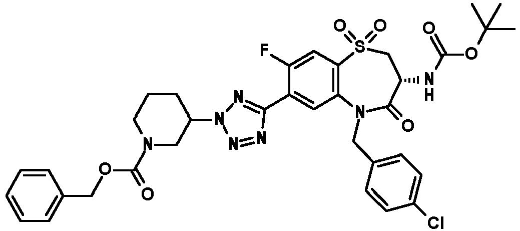

The title compound was prepared from tert-butyl N-[(3R)-5-[(4-chlorophenyl)methyl]-8-fluoro-1,1,4-trioxo-7-(2H-tetrazol-5-yl)-2,3-dihydro-1λ6,5-benzothiazepin-3-yl]carbamate (50 mg, 0.09 mmol) and benzyl 3-hydroxypiperidine-1-carboxylate in analogy to example 9, step a) and was obtained as white solid (20 mg, 0.03 mmol, 26% yield). MS (ESI): 754.2 [M +H]⁺.

### Step b) tert-butyl N-[(3R)-5-[(4-chlorophenyl)methyl]-8-fluoro-1,1,4-trioxo-7-[2-(3-piperidyl) tetrazol-5-yl]-2,3-dihydro-1λ⁶,5-benzothiazepin-3-yl]carbamate

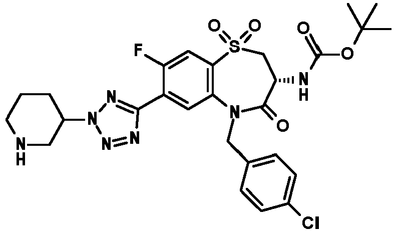

To a solution of benzyl 3-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate (20 mg, 0.03 mmol) in EtOH (3 mL) was added HCl\EtOAc (0.01 mL, 0.05 mmol, 2 eq) and Pd(OH)₂/C (wet. 10%) (10.0 mg), the mixture was degassed with H₂ for three times, then stirred at 25 °C for 8 h. Additional HCl\EtOAc (0.01 mL, 0.03 mmol, 1 eq) was added into the mixture, then degassed with H₂ for three times and stirred for another 6 h. The mixture was filtered by diatomite and washed with EtOH (5 mL), concentrated and purified by prep-HPLC to afford the title compound (3 mg, 16% yield) as a white solid. MS (ESI): 620.2 [M +H]⁺.

### Step c) tert-butyl N-[(3R)-5-[(4-chlorophenyl)methyl]-8-fluoro-7-[2-(1-methyl-3-piperidyl) tetrazol-5-yl]-1,1,4-trioxo-2,3-dihydro-1λ⁶,5-benzothiazepin-3-yl]carbamate

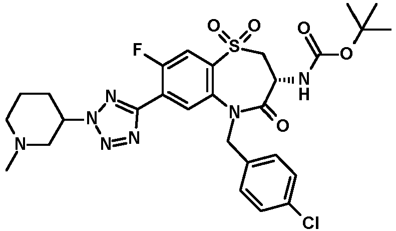

To a solution of tert-butyl N-[(3R)-5-[(4-chlorophenyl)methyl]-8-fluoro-1,1,4-trioxo-7-[2-(3-piperidyl)tetrazol-5-yl]-2,3-dihydro-1λ⁶,5-benzothiazepin-3-yl]carbamate (50 mg, 0.08 mmol, 1 eq) in MeOH (2 mL) was added formaldehyde (0.01 mL, 0.16 mmol, 2 eq) at 20°C. After stirred for 0.5 h, sodium cyanoborohydride (9.88 mg, 0.16 mmol, 2 eq) was added and the mixture was stirred for 16 h at 20 °C. The reaction mixture was quenched by aqueous 0.5 M HCl (1 mL), extracted with EtOAc (3 mL x 3). The combined organic phase was washed with brine (5 mL x 2), dried over anhydrous Na₂SO₄, concentrated under vacuo. The crude product which was purified by prep-HPLC. The eluent was concentrated under vacuo, the residue was freeze dried to afford the title compound (25 mg, 0.04 mmol, 50% yield) as a white solid which contained 51% de-Boc. MS (ESI): 634.1 [M +H]⁺.

### Step d) (3R)-3-amino-5-[(4-chlorophenyl)methyl]-8-fluoro-7-[2-(1-methyl-3-piperidyl)tetrazol-5-yl]-1,1-dioxo-2,3-dihydro-1λ⁶,5-benzothiazepin-4-one

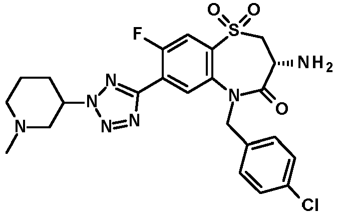

The title compound was prepared tert-butyl N-[(3R)-5-[(4-chlorophenyl)methyl]-8-fluoro-7-[2-(1-methyl-3-piperidyl)tetrazol-5-yl]-1,1,4-trioxo-2,3-dihydro-1λ⁶,5-benzothiazepin-3-yl]carbamate (25 mg, 0.04 mmol) in analogy to general procedure 6b and was obtained as white solid, as a dihydrochloride salt (8.4 mg, 0.01 mmol, 33% yield). MS (ESI): 534.2 [M+H]⁺.

### Example 21 and 22

### (3R)-3-amino-5-[(4-chlorophenyl)methyl]-7-[2-(5,5-difluoro-1-methyl-3-piperidyl)tetrazol-5-yl]-8-fluoro-1,1-dioxo-2,3-dihydro-1λ⁶,5-benzothiazepin-4-one and (3R)-3-amino-5-benzyl-7-[2-(5,5-difluoro-1-methyl-3-piperidyl)tetrazol-5-yl]-8-fluoro-1,1-dioxo-2,3-dihydro-λ⁶,5-benzothiazepin-4-one

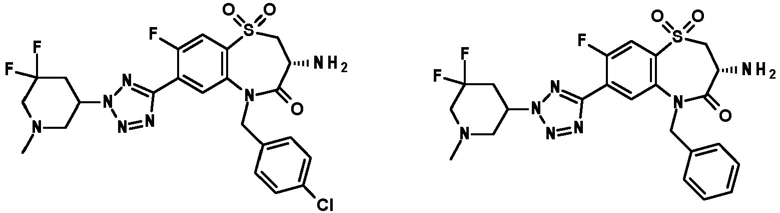

### Step a) benzyl 3,3-difluoro-5-[5-[(3R)-3-(tert-butoxycarbonylamino)-5-[(4-chlorophenyl)methyl]-8-fluoro-1, 1, 4-trioxo-2, 3-dihydro-1λ⁶,5-benzothiazepin-7-yl]tetrazol-2-yl]piperidine-1-carboxylate

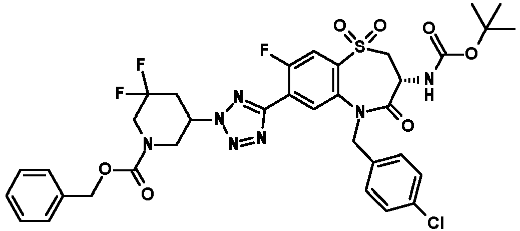

The title compound was prepared from tert-butyl N-[(3R)-5-[(4-chlorophenyl)methyl]-8-fluoro-1,1,4-tiioxo-7-(2H-tetrazol-5-yl)-2,3-dihydro-1λ6,5-benzothiazepin-3-yl]carbamate (50 mg, 0.09 mmol) and benzyl 3,3-difluoro-5-hydroxy-piperidine-1-carboxylate (30.3 mg, 0.11 mmol, 1.2 eq) in analogy to example 9, step a) and was obtained as white solid (32 mg, 0.04 mmol, 41% yield). MS (ESI): 690.2 [M-isobutene-CO₂+H]⁺.

### Step b) tert-butyl N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1λ⁶,5-benzothiazepin-3-yl]carbamate and tert-butyl N-[(3R)-5-benzyl-7-[2-(5,5-difluoro-3-piperidyl)tetrazol-5-yl]-8-fluoro-1,1,4-trioxo-2,3-dihydro-1λ⁶,5-benzothiazepin-3-yl]carbamate

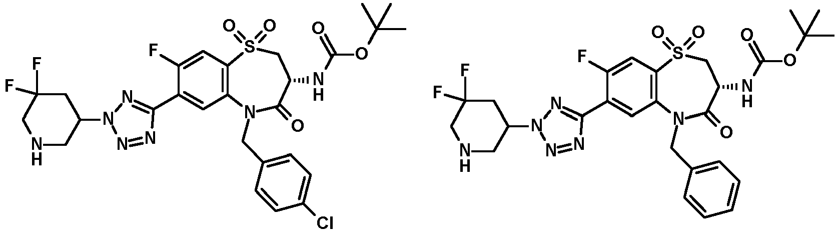

The title compound was prepared from benzyl 3,3-difluoro-5-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate (130 mg, 0.16 mmol) in analogy to example 19, step b) and was obtained as white solid (75 mg, 45% yield). MS (ESI): 600.1 [M-isobutene+H]⁺.

### Step c) tert-butyl N-[(3R)-5-[(4-chlorophenyl)methyl]-7-[2-(5, 5-difluoro-1-methyl-3-piperidyl) tetrazol-5-yl]-8-fluoro-1,1,4-trioxo-2,3-dihydro-1λ⁶,-yl]carbamate and tert-butyl N-[(3R)-5-benzyl-7-[2-(5,5-difluoro-1-methyl-3-piperidyl)tetrazol-5-yl]-8-fluoro-1,1,4-trioxo-2, 3-dihydro-1λ⁶,5-benzothiazepin-3-yl]carbamate

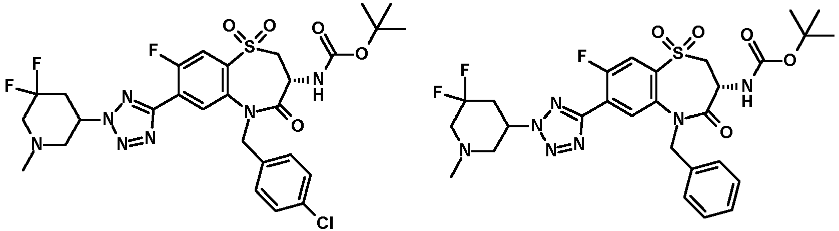

The title compounds were prepared from tert-butyl N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1λ⁶,5-benzothiazepin-3-yl]carbamate (24.0 mg, 0.04 mmol) and tert-butyl N-[(3R)-5-benzyl-7-[2-(5,5-difluoro-3-piperidyl)tetrazol-5-yl]-8-fluoro-1,1,4-trioxo-2,3-dihydro-1λ⁶,5-benzothiazepin-3-yl]carbamate (16.0 mg, 0.03 mmol) in analogy to example 20, step c) and were obtained as a white solid as a crude mixture (16 mg, 0.030 mmol, 68% yield and 21 mg, 0.030 mmol, 85% yield)). MS (ESI): 670.3 [M+H]⁺ and MS (ESI): 636.3 [M+H]⁺.

### Step d) (3R)-3-amino-5-[(4-chlorophenyl)methyl]-7-[2-(5,5-difluoro-1-methyl-3-piperidyl) tetrazol-5-yl]-8-fluoro-1,1-dioxo-2,3-dihydro-1λ⁶,5-benzothiazepin-4-one and (3R)-3-amino-5-benzyl-7-[2-(5,5-difluoro-1-methyl-3-piperidyl)tetrazol-5-yl]-8-fluoro-1,1-dioxo-2,3-dihydro-1λ⁶,5-benzothiazepin-4-one

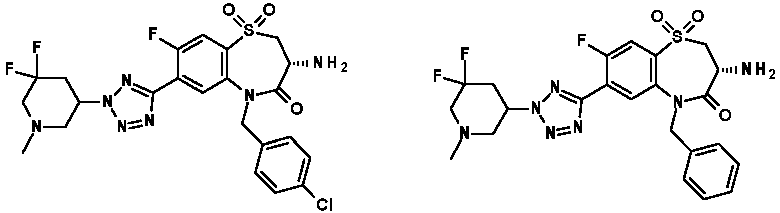

The title compounds were prepared from tert-butyl N-[(3R)-5-[(4-chlorophenyl)methyl]-7-[2-(5,5-difluoro-1-methyl-3-piperidyl)tetrazol-5-yl]-8-fluoro-1,1,4-trioxo-2,3-dihydro-1λ⁶,5-benzothiazepin-3-yl]carbamate (21.0 mg, 0.03 mmol) and tert-butyl N-[(3R)-5-benzyl-7-[2-(5,5-difluoro-1-methyl-3-piperidyl)tetrazol-5-yl]-8-fluoro-1,1,4-trioxo-2,3-dihydro-1λ⁶,5-benzothiazepin-3-yl]carbamate (16.0 mg, 0.03 mmol) in analogy to general procedure **6b** and (3R)-3-amino-5-[(4-chlorophenyl)methyl]-7-[2-(5,5-difluoro-1-methyl-3-piperidyl)tetrazol-5-yl]-8-fluoro-1,1-dioxo-2,3-dihydro-1λ⁶,5-benzothiazepin-4-one was obtained after prep-HPLC as white solid (4.5 mg, 25% yield) (MS (ESI): 570.2 [M+H]⁺) and (3R)-3-amino-5-benzyl-7-[2-(5,5-difluoro-1-methyl-3-piperidyl)tetrazol-5-yl]-8-fluoro-1,1-dioxo-2,3-dihydro-1λ⁶,5-benzothiazepin-4-one as white solid (4.4 mg, 25% yield) (MS (ESI): 536.3 [M+H]⁺).

### Example 23

### (3R)-3-amino-5-[(4-chlorophenyl)methyl]-7-[2-(5,5-difluoro-1-methylsulfonyl-3-piperidyl)tetrazol-5-yl]-8-fluoro-1,1-dioxo-2,3-dihydro-λk⁶,5-benzothiazepin-4-one

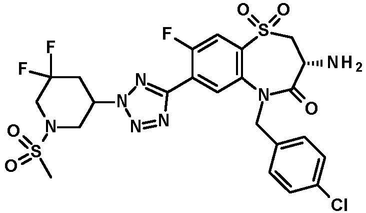

### Step a) tert-butyl N-[(3R)-5-[(4-chlorophenyl)methyl]-7-[2-(5,5-difluoro-1-methylsulfonyl-3-piperidyl)tetrazol-5-yl]-8-fluoro-1,1,4-trioxo-2,3-dihydro-1λ⁶,5-benzothiazepin-3-yl]carbamate

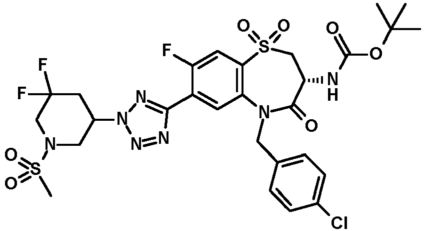

The title compound was prepared from tert-butyl N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1λ⁶,5-benzothiazepin-3-yl]carbamate (90 mg, 0.06 mmol) and methane sulfonylchloride in analogy to example 17, step c) and was obtained as light yellow gum (46 mg, 0.06 mmol, 96% yield). MS (ESI): 678.0 [M-isobutene+H]⁺.

### Step b) (3R)-3-amino-5-[(4-chlorophenyl)methyl]-7-[2-(5,5-difluoro-1-methylsulfonyl-3-piperidyl)tetrazol-5-yl]-8-fluoro-1,1-dioxo-2, 3-dihydro-1λ⁶,5-benzothiazepin-4-one

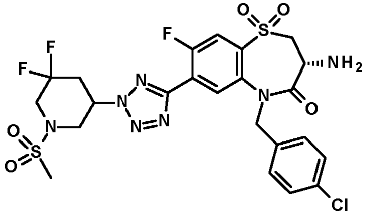

The title compound was prepared from tert-butyl N-[(3R)-5-[(4-chlorophenyl)methyl]-7-[2-(5,5-difluoro-1-methylsulfonyl-3-piperidyl)tetrazol-5-yl]-8-fluoro-1,1,4-trioxo-2,3-dihydro-1λ⁶,5-benzothiazepin-3-yl]carbamate (46.0 mg, 0.06 mmol) in analogy to general procedure 6b and was obtained as white solid (22.1 mg, 0.03 mmol, 54% yield) after prep-HPLC. MS (ESI): 634.1 [M+H]⁺.

### Example 24

### (3R)-7-[2-(1-acetyl-5,5-difluoro-3-piperidyl)tetrazol-5-yl]-3-amino-5-[(4-chlorophenyl)methyl]-8-fluoro-1,1-dioxo-2,3-dihydro-1λ⁶,5-benzothiazepin-4-one

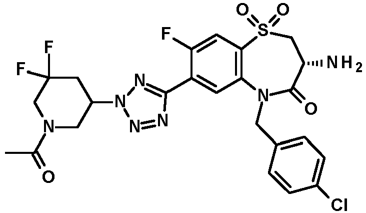

### Step a) tert-butyl N-[(3R)-7-[2-(1-acetyl-5,5-difluoro-3-piperidyl)tetrazol-5-yl]-5-[(4-chlorophenyl)methyl]-8-fluoro-1,1,4-trioxo-2,3-dihydro-1λ⁶,5-benzothiazepin-3-yl]carbamate

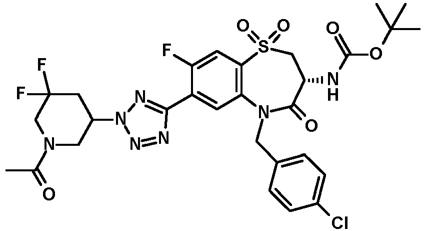

To a solution tert-butyl N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1λ⁶,5-benzothiazepin-3-yl]carbamate (75 mg, 0.11 mmol, 1 eq) in DCM (2 mL) was added acetic anhydride (0.01 mL, 0.11 mmol, 1 eq) at 0 °C. The mixture was stirred for 3 hr at 25 °C. The reaction solution was concentrated in vacuo and purified by prep-TLC (PE:EtOAc = 1:1) to afford the title compound (50 mg, 0.07 mmol, 41% yield) as a white solid. MS (ESI): 598.1 [M-isobutene-CO₂+H]⁺.

### Step b) (3R)-7-[2-(1-acetyl-5,5-difluoro-3-piperidyl)tetrazol-5-yl]-3-amino-5-[(4-chlorophenyl) methyl]-8-fluoro-1,1-dioxo-2, 3-dihydro-1λ⁶,5-benzothiazepin-4-one

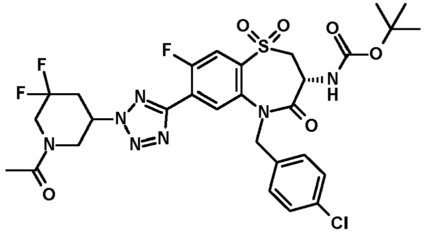

The title compound was prepared from tert-butyl N-[(3R)-7-[2-(1-acetyl-5,5-difluoro-3-piperidyl)tetrazol-5-yl]-5-[(4-chlorophenyl)methyl]-8-fluoro-1,1,4-trioxo-2,3-dihydro-1λ⁶,5-benzothiazepin-3-yl]carbamate (50 mg, 0.07 mmol) in analogy to general procedure **6b** and was obtained as white solid after prep-HPLC (10.9 mg, 0.02 mmol, 24% yield). MS (ESI): 598.2 [M+H]⁺.

### Example 25

### methyl 3,3-difluoro-5-[5-[(3R)-3-amino-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate

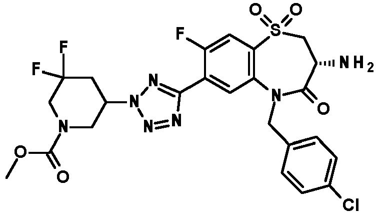

### Step a) methyl 3, 3-difluoro-5-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate

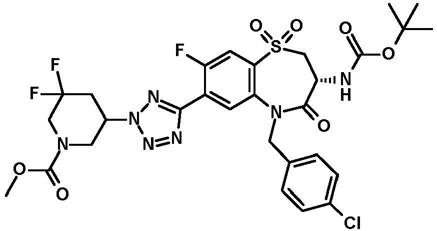

The title compound was prepared from tert-butyl N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1λ⁶,5-benzothiazepin-3-yl]carbamate (200 mg, 0.18 mmol) and methyl chloroformate in analogy to example 17, step c) and was obtained as a white solid (66 mg, 0.09 mmol, 46% yield) after prep-TLC. MS (ESI): 614.1 [M-isobutene-CO₂+H]⁺.

### Step b) methyl 3,3-difluoro-5-[5-[(3R)-3-amino-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate

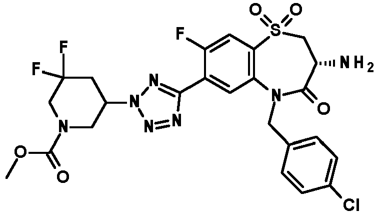

The title compound was prepared from methyl 3,3-difluoro-5-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate (66 mg, 0.09 mmol) in analogy to general procedure **6b** and was obtained as white solid (13 mg, 0.02 mmol, 22% yield) after prep-HPLC. MS (ESI): 614.1 [M+H]⁺.

### Example 26

### N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1λ⁶,5-benzothiazepin-3-yl]acetamide

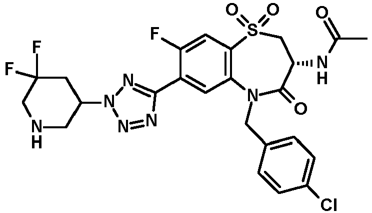

### Step a) (3R)-3-amino-5-[(4-chlorophenyl)methyl]-7-[2-(5,5-difluoro-3-piperidyl)tetrazol-5-yl]-8-fluoro-1,1-dioxo-2, 3-dihydro-1λ⁶,5-benzothiazepin-4-one

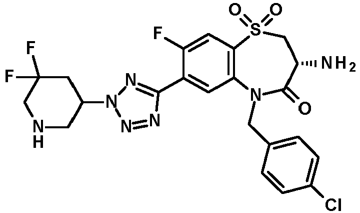

To a solution of benzyl 3,3-difluoro-5-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]tetrazol-2-yl]piperidine-1-carboxylate (300 mg, 0.38 mmol) in DCM (30 mL) was added trimethylsilyl iodide (0.14 mL, 0.95 mmol, 2.5 eq), the mixture was degassed with N₂ for three times, then stirred at 25 °C for 1 h. The mixture was quenched by addition of water (10 mL) and basified with saturated aqueous NaHCO₃ to pH = 8, then extracted with EtOAc (10 mL x 2). The combined organic phase was dried over anhydrous Na₂SO₄, concentrated to give crude product which was triturated with EtOAc:PE (2:1, 5 mL) and filtered, the cake was dried in vacuo to provide the title compound (88 mg, 0.16 mmol, 33% yield) as a white solid. MS (ESI): 556.2 [M+H]⁺.

### Step b) N-[(3R)-5-[(4-chlorophenyl)methyl]-7-[2-(5,5-difluoro-3-piperidyl)tetrazol-5-yl]-8-fluoro-1,1,4-trioxo-2, 3-dihydro-1λ⁶,5-benzothiazepin-3-yl]acetamide

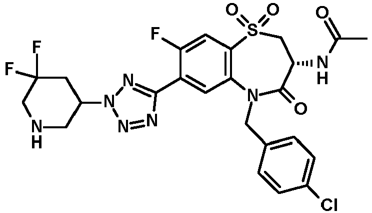

The title compound was prepared from (3R)-3-amino-5-[(4-chlorophenyl)methyl]-7-[2-(5,5-difluoro-3-piperidyl)tetrazol-5-yl]-8-fluoro-1,1-dioxo-2,3-dihydro-1λ⁶,5-benzothiazepin-4-one (88.0 mg, 0.13 mmol) in analogy to example 24, step a) and was obtained as white solid (21.3 mg, 25% yield) after prep-HPLC (water(0.05%HCl)-MeCN) as the hydrochloride salt. MS (ESI): 598.1 [M+H]⁺.

### Example 27 and 28

### (3R)-3-amino-5-[(4-chlorophenyl)methyl]-8-fluoro-7-[2-(2-hydroxy-2-methylpropyl)tetrazol-5-yl]-1,1-dioxo-2,3-dihydro-1λ⁶,5-benzothiazepin-4-one and (3R)-3-amino-5-[(4-chlorophenyl)methyl]-8-fluoro-7-[2-(2-hydroxy-1,1-dimethyl-ethyl)tetrazol-5-yl]-1,1-dioxo-2,3-dihydro-1λ⁶,5-benzothiazepin-4-one

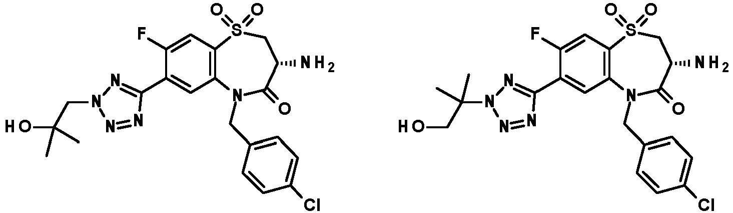

### Step a) tert-butyl N-[(3R)-5-[(4-chlorophenyl)methyl]-8-fluoro-7-[2-(2-hydroxy-2-methylpropyl)tetrazol-5-yl]-1,1, 4-trioxo-2, 3-dihydro-1λ ⁶,5-benzothiazepin-3-yl]carbamate and tert-butyl N-[(3R)-5-[(4-chlorophenyl)methyl]-8-fluoro-7-[2-(2-hydroxy-1,1-dimethyl-ethyl)tetrazol-5-ylj-1, 1, 4-trioxo-2, 3-dihydro-1λ ⁶,5-benzothiazepin-3-yl]carbamate

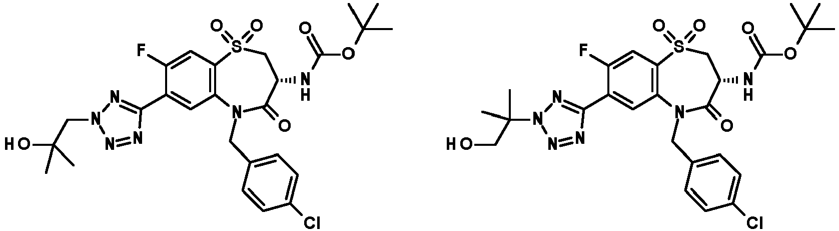

A suspension of tert-butyl N-[(3R)-5-[(4-chlorophenyl)methyl]-8-fluoro-1,1,4-trioxo-7-(2H-tetrazol-5-yl)-2,3-dihydro-1λ6,5-benzothiazepin-3-yl]carbamate (270 mg, 0.50 mmol), 2,2-dimethyloxirane (0.18 mL, 1.5 mmol, 3 eq) and CuS (53.9 mg, 0.55 mmol, 1.1 eq) in water (5 mL) was heated to 50°C and the mixture was stirred for 16 h. After cooled to ambient temperature, the mixture was filtered and the cake was washed with EtOAc (5 mL x 2). The water phase was extracted with EtOAc (5 mL x 2), the combined organic phase was dried over anhydrous Na₂SO₄, concentrated and purified by prep-TLC (PE:EA = 2:1) and subsequently by SFC (DAICEL CHIRALCEL OD, 0.1%NH₃ H2O MeOH) to provide the title compounds as a mixture (100 mg, 0.16 mmol, 32% yield) as a white solid. MS (ESI): 553.1 [M-isobutene+H]⁺.

### Step b) (3R)-3-amino-5-[(4-chlorophenyl)methyl]-8-fluoro-7-[2-(2-hydroxy-2-methyl-propyl) tetrazol-5-yl]-1,1-dioxo-2,3-dihydro-1λ⁶,5-benzothiazepin-4-one and (3R)-3-amino-5-[(4-chlorophenyl)methyl]-8-fluoro-7-[2-(2-hydroxy-1,1-dimethyl-ethyl)tetrazol-5-yl]-1,1-dioxo-2,3-dihydro-1λ⁶,5-benzothiazepin-4-one

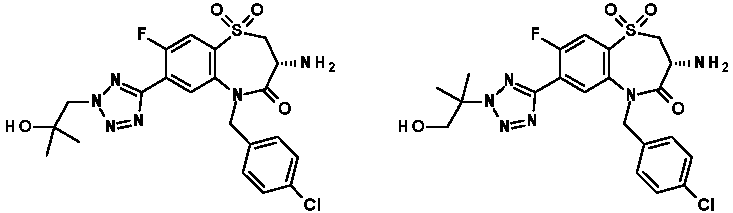

The title compounds were prepared from a mixture of tert-butyl N-[(3R)-5-[(4-chlorophenyl)methyl]-8-fluoro-7-[2-(2-hydroxy-2-methyl-propyl)tetrazol-5-yl]-1,1,4-trioxo-2,3-dihydro-1λ⁶,5-benzothiazepin-3-yl]carbamate (82.0 mg, 0.13 mmol) and tert-butyl N-[(3R)-5-[(4-chlorophenyl)methyl]-8-fluoro-7-[2-(2-hydroxy-1,1-dimethyl-ethyl)tetrazol-5-yl]-1,1,4-trioxo-2,3-dihydro-1λ⁶,5-benzothiazepin-3-yl]carbamate (15.0 mg, 0.02 mmol) in analogy to general procedure 6b. (3R)-3-amino-5-[(4-chlorophenyl)methyl]-8-fluoro-7-[2-(2-hydroxy-2-methylpropyl)tetrazol-5-yl]-1,1-dioxo-2,3-dihydro-1λ6,5-benzothiazepin-4-one (33.7 mg, 0.06 mmol, 74% yield) was obtained as a white solid as hydrochloride salt and (3R)-3-amino-5-[(4-chlorophenyl)methyl]-8-fluoro-7-[2-(2-hydroxy-1,1-dimethyl-ethyl)tetrazol-5-yl]-1,1-dioxo-2,3-dihydro-1λ6,5-benzothiazepin-4-one (4.6 mg, 0.01 mmol, 9% yield) was obtained as a white solid as hydrochloride salt after prep-HPLC (water 0.05% HCl-MeCN). MS (ESI): 509.1 [M+H]⁺.

### Example 29

### 2-amino-N-[(3R)-7-(2-tert-butyltetrazol-5-yl)-5-[(4-chlorophenyl)methyl]-8-fluoro-1,1,4-trioxo-2,3-dihydro-1lambda6,5-benzothiazepin-3-yl]acetamide

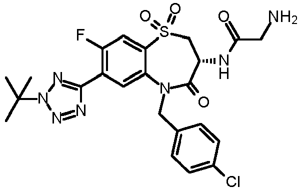

### Step a) tert-butylN-[2-[[(3R)-7-(2-tert-butyltetrazol-5-yl)-5-[(4-chlorophenyl)methyl]-8-fluoro-1,1,4-trioxo-2,3-dihydro-1λ⁶,5-benzothiazepin-3-yl]amino]-2-oxo-ethyl]carbamate

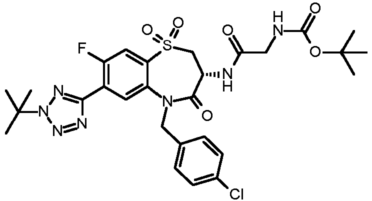

To a solution of Boc-glycine (10.6 mg, 0.061 mmol, 1.5 eq), DIPEA (15.7 mg, 21.2 uL, 0.12 mmol, 3 eq) and HATU (30.8 mg, 0.081 mmol, 2 eq) in DMF (0.3 mL) was added (3R)-3-amino-7-(2-tert-butyltetrazol-5-yl)-5-[(4-chlorophenyl)methyl]-8-fluoro-1,1-dioxo-2,3-dihydro-1λ⁶,5-benzothiazepin-4-one (Example 8, step c) (20 mg, 0.041 mmol, 1 eq) and the reaction mixture was stirred at 25 °C overnight. The reaction was purified by column chromatography on silica gel (heptane:EtOAc 1:0 to 0:1) to obtain the title compound (30 mg, 113% yield) as a white solid. MS (ESI): 594.3 [M-isobutene+H]⁺.

### Step b) 2-amino-N-[(3R)-7-(2-tert-butyltetrazol-5-yl)-5-[(4-chlorophenyl)methyl]-8-fluoro-1, 1,4-trioxo-2, 3-dihydro-1lambda6,5-benzothiazepin-3-yl]acetamide

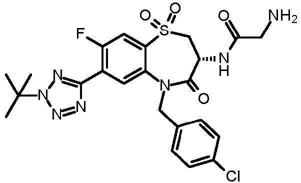

The title compound was prepared in analogy to general procedure 6d from tert-butyl N-[2-[[(3R)-7-(2-tert-butyltetrazol-5-yl)-5-[(4-chlorophenyl)methyl]-8-fluoro-1,1,4-trioxo-2,3-dihydro-1λ⁶,5-benzothiazepin-3-yl]amino]-2-oxo-ethyl]carbamate (30 mg, 0.046 mmol) and was obtained as a withe solid (14 mg, 55% yield). MS (ESI): 550.3 [M+H]⁺.

### Example 30

### (3R)-3-amino-7-(2-tert-butyltetrazol-5-yl)-8-fluoro-1,1-dioxo-5-[[4-(trifluoromethoxy)phenyl]methyl]-2,3-dihydro-1λ⁶,5-benzothiazepin-4-one

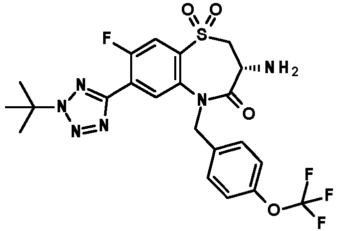

### Step a) tert-butylN-[(3R)-7-cyano-8-fluoro-4-oxo-5-[[4-(trifluoromethoxy)phenyl]methyl]-2, 3-dihydro-1, 5-benzothiazepin-3-yl]carbamate

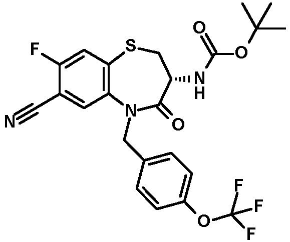

The title compound was prepared from N-[(3R)-7-cyano-8-fluoro-4-keto-3,5-dihydro-2H-1,5-benzothiazepin-3-yl]carbamic acid tert-butyl ester (600 mg, 1.78 mmol) and 1-(bromomethyl)-4-(trifluoromethoxy)benzene in DMF in analogy to general procedure **4** and was obtained as light yellow solid (752 mg, 82% yield). MS (ESI): 456.3 [M-isobutene+H]⁺.

### Step b) tert-butyl N-[(3R)-7-cyano-8-fluoro-1,1,4-trioxo-5-[[4-(trifluoromethoxy)phenyl]methyl]-2,3-dihydro-1λ⁶,5-benzothiazepin-3-yl]carbamate

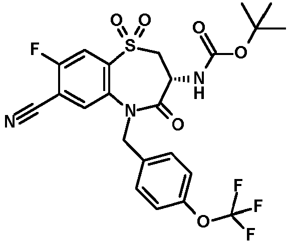

The title compound was prepared from tert-butyl N-[(3R)-7-cyano-8-fluoro-4-oxo-5-[[4-(trifluoromethoxy)phenyl]methyl]-2,3-dihydro-1,5-benzothiazepin-3-yl]carbamate (750 mg, 1.47 mmol) in analogy to general procedure **5** and was obtained as off-white solid (475 mg, 53% yield). MS (ESI): 488.2 [M-isobutene+H]⁺.

### Step c) tert-butylN-[(3R)-8-fluoro-1,1, 4-trioxo-7-(2H-tetrazol-5-yl)-5-[[4-(trifluoromethoxy)phenyl]methyl]-2,3-dihydro-1λ⁶,5-benzothiazepin-3-yl]carbamate

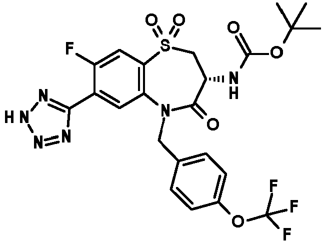

To a solution of tert-butyl N-[(3R)-7-cyano-8-fluoro-1,1,4-trioxo-5-[[4-(trifluoromethoxy)phenyl]methyl]-2,3-dihydro-1λ⁶,5-benzothiazepin-3-yl]carbamate (200 mg, 0.370 mmol) in toluene (4 mL) was added trimethylsilyl azide (169 mg, 1.47 mmol, 4 eq), and dibutyltin oxide (13.7 mg, 0.06 mmol, 0.15 eq), the mixture was degassed with N₂ for 2 mins, then stirred at 100 °C for 3 h. After cooled to room temperature, the mixture was quenched by water (5 mL), saturated aqueous KF (5 mL), and stirred for 1 h. Then the pH of the mixture was adjusted to over 9 and stirred for 10 mins. After separated, the aqueous phase was extracted with EtOAc (5 mL x 3), the combined organic phase was washed with brine (10 mL x 3), dried over anhydrous Na₂SO₄, concentrated to give crude product which was triturated with toluene (5 mL) and filtered to afford the title compound (240 mg, 0.41 mmol, 92% yield) as a light brown solid. MS (ESI): 531.4 [M-isobutene+H]⁺.

### Step d) (3R)-3-amino-7-(2-tert-butyltetrazol-5-yl)-8-fluoro-1,1-dioxo-5-[[4-(trifluoromethoxy)phenyl]methyl]-2,3-dihydro-1λ ⁶,5-benzothiazepin-4-one

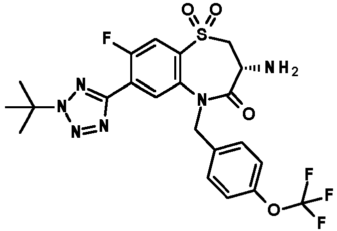

The title compound was prepared from tert-butyl N-[(3R)-8-fluoro-1,1,4-trioxo-7-(2H-tetrazol-5-yl)-5-[[4-(trifluoromethoxy)phenyl]methyl]-2,3-dihydro-1λ⁶,5-benzothiazepin-3-yl]carbamate (220 mg, 0.38 mmol) in analogy to Example 8, step c) and was obtained as white solid (41.7 mg, 0.08 mmol, 20% yield). MS (ESI): 543.1 [M+H] ⁺.

Example 31 of the following table was prepared in analogy to Example 30 in four steps, using the appropriate amide building block.

| **Ex.** | **Structure** | **Systematic Name** | **Building Blocks** | **MS, ESI: m/z** |
|---|---|---|---|---|
| 31 | 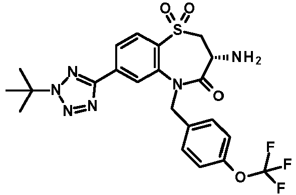 | (3R)-3-amino-7-(2-tert-butyltetrazol-5-yl)-1,1-dioxo-5-[[4-(trifluoromethoxy)phenyl]met hyl]-2,3-dihydro-1λ⁶,5-benzothiazepin-4-one | tert-Butyl N-[(3R)-7-cyano-4-oxo-3,5-dihydro-2H-1,5-benzothiaz epin-3-yl]carbamate | 525.1 [M+H]⁺ |

Example 32 of the following table was prepared in analogy to Example 30 in four steps, using the appropriate alkylating building block.

| **Ex.** | **Structure** | **Systematic Name** | **Building Blocks** | **MS, ESI: m/z** |
|---|---|---|---|---|
| 32 | 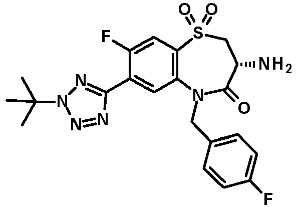 | (3R)-3-amino-7-(2-tert-butyltetrazol-5-yl)-8-fluoro-5-[(4-fluorophenyl)methyl]-1,1-dioxo-2,3-dihydro-1λ⁶,5-benzothiazepin-4-one | 4-Fluorobenzyl bromide | 477.3 [M+H]⁺ |

### Example 33

### (3R)-3-amino-7-(2-tert-butyltetrazol-5-yl)-8-fluoro-1,1-dioxo-5-[[4-(tetrahydropyran-4-ylmethoxy)phenyl]methyl]-2,3-dihydro-1λ⁶,5-benzothiazepin-4-one

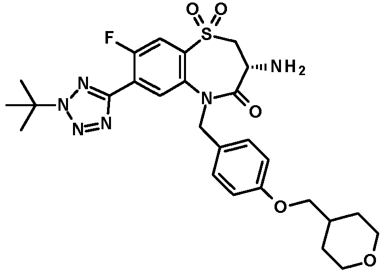

### Step a) tert-butyl N-[(3R)-7-cyano-8-fluoro-4-oxo-5-[[4-(tetrahydropyran-4-ylmethoxy)phenyl]methyl]-2,3-dihydro-1,5-benzothiazepin-3-yl]carbamate

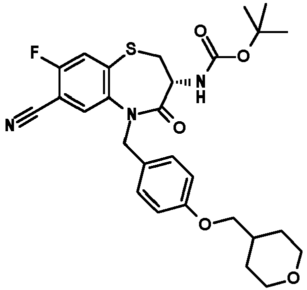

The title compound was prepared from N-[(3R)-7-cyano-8-fluoro-4-keto-3,5-dihydro-2H-1,5-benzothiazepin-3-yl]carbamic acid tert-butyl ester (250 mg, 0.74 mmol) and 4-[[4-(chloromethyl)phenoxy]methyl]tetrahydropyran (196 mg, 0.82 mmol,) in DMF in analogy to general procedure **4** and was obtained as light yellow solid (200 mg, 49% yield). MS (ESI): 564.2 [M+Na]⁺.

### Step b) tert-butyl N-[(3R)-7-cyano-8-fluoro-1, 1, 4-trioxo-5-[[4-(tetrahydropyran-4-ylmethoxy)phenyl]methyl]-2, 3-dihydro-1λ ⁶,5-benzothiazepin-3-yl]carbamate

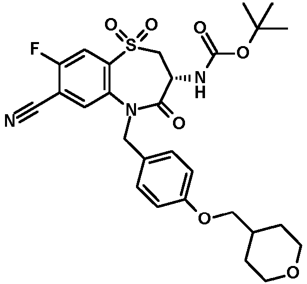

The title compound was prepared from tert-butyl N-[(3R)-7-cyano-8-fluoro-4-oxo-5-[[4-(tetrahydropyran-4-ylmethoxy)phenyl]methyl]-2,3-dihydro-1,5-benzothiazepin-3-yl]carbamate (190 mg, 0.35 mmol) in analogy to general procedure **5** and was obtained as-white solid (180 mg 89% yield). MS (ESI): 596.2 [M+Na]⁺.

### Step c) tert-butyl N-[(3R)-8-fluoro-1,1, 4-trioxo-5-[[4-(tetrahydropyran-4-ylmethoxy)phenyl] methyl]-7-(2H-tetrazol-5-yl)-2,3-dihydro-1λ⁶,5-benzothiazepin-3-yl]carbamate

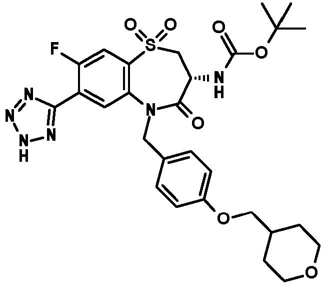

The title compound was prepared from tert-butyl N-[(3R)-7-cyano-8-fluoro-1,1,4-trioxo-5-[[4-(tetrahydropyran-4-ylmethoxy)phenyl]methyl]-2,3-dihydro-1λ⁶,5-benzothiazepin-3-yl]carbamate (180 mg, 0.31 mmol) in analogy to example 30, step c) and was obtained as-white solid (100 mg 51% yield). MS (ESI): 639.2 [M+Na]⁺.

### Step d) (3R)-3-amino-7-(2-tert-butyltetrazol-5-yl)-8-fluoro-1,1-dioxo-5-[[4-(tetrahydropyran-4-ylmethoxy)phenyl]methyl]-2, 3-dihydro-1λ⁶,5-benzothiazepin-4-one

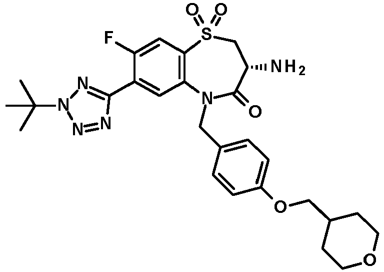

To tert-butyl N-[(3R)-8-fluoro-1,1,4-trioxo-5-[[4-(tetrahydropyran-4-ylmethoxy)phenyl]methyl]-7-(2H-tetrazol-5-yl)-2,3-dihydro-1λ⁶,5-benzothiazepin-3-yl]carbamate (70 mg, 0.14 mmol, 1 eq) in tert-butanol (2 mL) was added TFA (4.0 mL, 53 mmol, 397 eq) at 25°C, then heated to 30 °C for 2 h. The reaction was concentrated, purified by prep-HPLC (neutral) and lyophilized to afford the title compound (26.5 mg, 0.05 mmol, 33% yield) as a white solid. MS (ESI): 573.3 [M+H]⁺.

### Example 34 and Example 35

### (3R)-3-amino-7-(2-tert-butyltetrazol-5-yl)-5-[(4-chlorophenyl)methyl]-8-fluoro-1-oxo-2,3-dihydro-1λ⁴,5-benzothiazepin-4-one (Epimer A) and (3R)-3-amino-7-(2-tert-butyltetrazol-5-yl)-5-[(4-chlorophenyl)methyl]-8-fluoro-1-oxo-2,3-dihydro-1λ⁴,5-benzothiazepin-4-one (Epimer B)

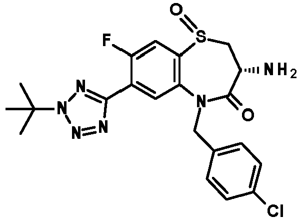

### Step a) tert-butyl N-[(3R)-7-(2-tert-butyltetrazol-S-yl)-5-[(4-chlorophenyl)methyl]-8-fluoro-4-oxo-2,3-dihydro-1,5-benzothiazepin-3-yl]carbamate

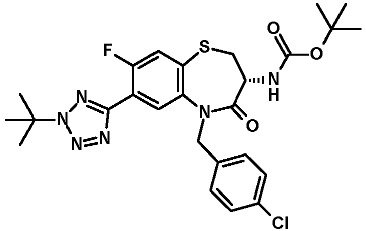

(3R)-3-amino-7-(2-tert-butyltetrazol-5-yl)-5-(4-chlorobenzyl)-8-fluoro-2,3-dihydro-1,5-benzothiazepin-4-one (105 mg, 0.23 mmol) was stirred in DCM (2.5 mL) at room temperature. (Boc)₂O (54.6 mg, 58.2 uL, 0.25 mmol, 1.1 eq) was added and the reaction was stirred overnight at room temperature. The crude material was purified by column chromatography on silica gel (heptane:EtOAc (0-100%)) affording the title compound (88 mg, 55% yield) as light yellow solid. MS (ESI): 505.2 [M-isobutene+H]⁺.

### Step b) (3R)-3-amino-7-(2-tert-butyltetrazol-5-yl)-5-[(4-chlorophenyl)methyl]-8-fluoro-1-oxo-2,3-dihydro-1λ⁶,5-benzothiazepin-4-one (Epimer A) and (3R)-3-amino-7-(2-tert-butyltetrazol-5-yl)-5-[(4-chlorophenyl)methyl]-8-fluoro-1-oxo-2,3-dihydro-1λ⁴,5-benzothiazepin-4-one (Epimer B)

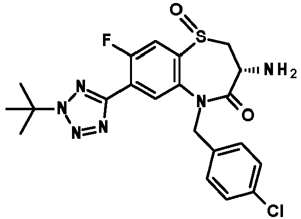

tert-butyl N-[(3R)-7-(2-tert-butyltetrazol-5-yl)-5-[(4-chlorophenyl)methyl]-8-fluoro-4-oxo-2,3-dihydro-1,5-benzothiazepin-3-yl]carbamate (40 mg, 0.071 mmol, 1 eq) was stirred with 3-chloroperoxybenzoic acid (15.9 mg, 0.071 mmol, 1 eq) in DCM (1 mL) at room temperature overnight. The solvent was evaporated. 1,1,1,3,3,3-hexafluoro-2-propanol (1.5 mL) was added followed by HCl in dioxane 4M (5 drops). The mixture was stirred for 3h at room temperature. The solvent was evaporated and the crude was purified by prep-HPLC affording the title compound Epimer A (9.1 mg, 26% yield) as white solid and Epimer B (12.5 mg, 36%, yield) as a white solid. MS (ESI): 477.1 [M+H]⁺.

### Example 36

### ((3R)-3-amino-7-(2-tert-butyltetrazol-5-yl)-5-[(4-chlorophenyl)methyl]-1,1-dioxo-2,3-dihydro-1λ⁶,5-benzothiazepin-4-one

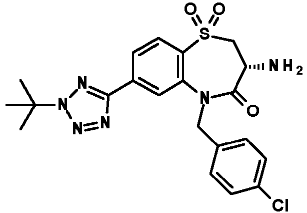

### Step a) (2R)-2-(tert-butoxycarbonylamino)-3-(4-cyano-2-nitro-phenyl)sulfanyl-propanoic acid

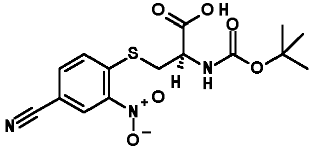

To a solution of 4-fluoro-3-nitrobenzonitrile (10.0 g, 60.2 mmol) in DCM (200 mL) was added (2R)-2-(tert-butoxycarbonylamino)-3-sulfanyl-propanoic acid (14.65 g, 66.2 mmol, 1.1 eq) and DIPEA (20.5 mL, 120.4 mmol, 2 eq), the mixture was stirred at 25 °C for 16 h. After concentrated under vacuo, the residue was diluted with EtOAc (200 mL) and washed with 1 N aqueous HCl (50 mL) and water (100 mL), then brine (50 mL x 2), dried over Na₂SO₄, filtered and concentrated to give the crude title compound (24 g, 65.3 mmol, 77% yield) as a yellow solid. MS (ESI): 390.1 [M+Na]⁺.

### Step b) (2R)-3-(2-amino-4-cyano-phenyl)sulfanyl-2-(tert-butoxycarbonylamino)propanoic acid

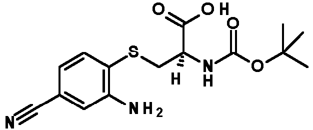

The title compound was prepared from (2R)-2-(tert-butoxycarbonylamino)-3-(4-cyano-2-nitrophenyl)sulfanyl-propanoic acid (10.0 g, 27.2 mmol) in analogy to general procedure **2** and was obtained as-brown solid (10 g, 87% yield). MS (ESI): 338.2 [M+H]⁺.

### Step c) tert-butylN-[(3R)-7-cyano-4-oxo-3, 5-dihydro-2H-1, 5-benzothiazepin-3-yl]carbamate

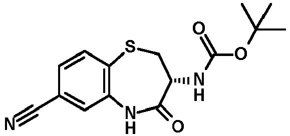

The title compound was prepared from (2R)-3-(2-amino-4-cyano-phenyl)sulfanyl-2-(tert-butoxycarbonylamino)propanoic acid (11.3 g, 33.5 mmol) in analogy to general procedure **3** and was obtained as-white solid (5 g, 45% yield). MS (ESI): 263.9 [M-isobutene+H]⁺.

### Step d) tert-butyl N-[(3R)-5-[(4-chlorophenyl)methyl]-7-cyano-4-oxo-2, 3-dihydro-1,5-benzothiazepin-3-yl]carbamate

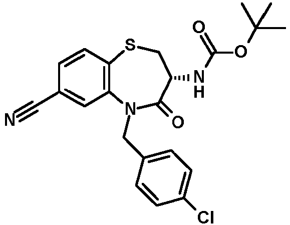

The crude title compound was prepared from tert-butyl N-[(3R)-7-cyano-4-oxo-3,5-dihydro-2H-1,5-benzothiazepin-3-yl]carbamate (1.0 g, 3.1 mmol) in analogy to general procedure **4** and was obtained as-white solid (2 g, 143% yield). MS (ESI): 388.1 [M-isobutene+H]⁺.

### Step e) tert-butyl N-[(3R)-5-[(4-chlorophenyl)methyl]-7-cyano-1,1, 4-trioxo-2, 3-dihydro-1λ⁶,5-benzothiazepin-3-yl]carbamate

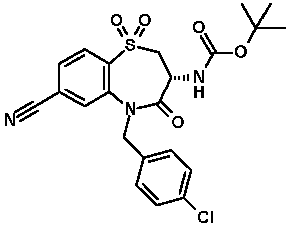

The title compound was prepared from tert-butyl N-[(3R)-5-[(4-chlorophenyl)methyl]-7-cyano-4-oxo-2,3-dihydro-1,5-benzothiazepin-3-yl]carbamate (2000 mg, 4.51 mmol) in analogy to general procedure 5 and was obtained as-white solid (1700 mg, 77% yield). MS (ESI): 498.4 [M+Na]⁺.

### Step f) tert-butyl N-[(3R)-5-[(4-chlorophenyl)methyl]-1,1, 4-trioxo-7-(2H-tetrazol-5-yl)-2,3-dihydro-1λ⁶,5-benzothiazepin-3-yl]carbamate

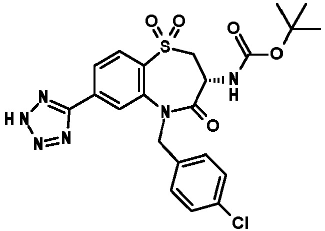

The title compound was prepared from tert-butyl N-[(3R)-5-[(4-chlorophenyl)methyl]-7-cyano-1,1,4-trioxo-2,3-dihydro-1λ⁶,5-benzothiazepin-3-yl]carbamate (1500 mg, 3.15 mmol) in analogy to example 30, step c) and was obtained as-white solid (940 mg, 55% yield) after prep-HPLC. MS (ESI): 463.1 [M-isobutene+H]⁺.

### Step g) ((3R)-3-amino-7-(2-tert-butyltetrazol-5-yl)-5-[(4-chlorophenyl)methyl]-1,1-dioxo-2,3-dihydro-1λ⁶,5-benzothiazepin-4-one

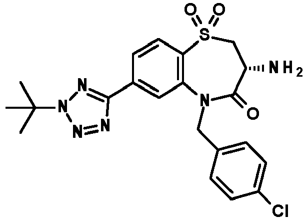

The title compound was prepared from tert-butyl N-[(3R)-5-[(4-chlorophenyl)methyl]-1,1,4-trioxo-7-(2H-tetrazol-5-yl)-2,3-dihydro-1λ⁶,5-benzothiazepin-3-yl]carbamate (100 mg, 0.19 mmol) in analogy to Example 8, step c) and was obtained as white solid (18.6 mg, 0.04 mmol, 20% yield) after prep-HPLC. MS (ESI): 475.1 [M+H]⁺.

### Example 37

### (3R)-3-amino-5-[(4-chlorophenyl)methyl]-8-fluoro-1,1-dioxo-7-(1H-pyrazol-5-yl)-2,3-dihydro-1λ⁶,5-benzothiazepin-4-one

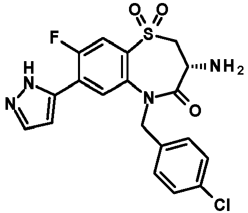

### Step a) tert-butyl N-[(3R)-7-bromo-5-[(4-chlorophenyl)methyl]-8-fluoro-1, 1, 4-trioxo-2, 3-dihydro-1λ⁶,5-benzothiazepin-3-yl]carbamate

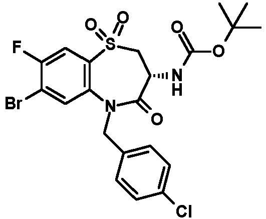

The title compound was prepared from tert-butyl N-[(3R)-7-bromo-5-[(4-chlorophenyl)methyl]-8-fluoro-4-oxo-2,3-dihydro-1,5-benzothiazepin-3-yl]carbamate (Example 6, step c) (550.0 mg, 1.07 mmol) in analogy to general procedure **5** and was obtained as light yellow solid (590 mg, 1.08 mmol, 96% yield). MS (ESI): 493.1 [M-isobutene+H] ⁺.

### Step b) tert-butyl N-[(3R)-5-[(4-chlorophenyl)methyl]-8-fluoro-1,1,4-trioxo-7-(2-tetrahydropyran-2-ylpyrazol-3-yl)-2,3-dihydro-1λ⁶,5-benzothiazepin-3-yl]carbamate

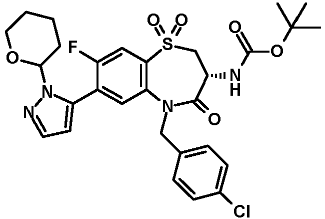

To a solution of tert-butyl N-[(3R)-7-bromo-5-[(4-chlorophenyl)methyl]-8-fluoro-1,1,4-trioxo-2,3-dihydro-1λ⁶,5-benzothiazepin-3-yl]carbamate (130.0 mg, 0.24 mmol, 1 eq) and 1-(tetrahydro-2H-pyran-2-yl)-5-(4,4,5,5-tetramethyl-1,3,2-dioxaborolan-2-yl)-1H-pyrazole (99.01 mg, 0.360 mmol, 1.5 eq) in 1,4-dioxane (2.6 mL) were added bis(triphenylphosphine)palladium dichloride (16.66 mg, 0.02 mmol, 0.1 eq) and sodium carbonate (60.62 mg, 0.570 mmol, 2.41 eq) (as a solution in water (0.650 mL)) at 25°C. The mixture was stirred at 80°C for 16 h. Then water (2 mL) was added and the mixture was extracted with EtOAc (3 x 2 mL). The combined extracts were washed with brine (3 x 6 mL) and dried with Na₂SO₄. After filtration, the organic layer was concentrated in vacuo to afford the crude product (230 mg) as a yellow oil. The crude product was purified by column chromatography on silica gel (10% to 25% EtOAc in PE) to obtain the desired title compound (160 mg, 0.26 mmol, 96% yield) as a white solid. MS (ESI): 479 [M-isobutene-dihydropyranyl+H] ⁺.

### Step c) (3R)-3-amino-5-[(4-chlorophenyl)methyl]-8-fluoro-1,1-dioxo-7-(1H-pyrazol-5-yl)-2,3-dihydro-1λ⁶,5-benzothiazepin-4-one

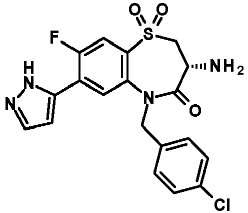

The title compound was prepared from tert-butyl N-[(3R)-5-[(4-chlorophenyl)methyl]-8-fluoro-1,1,4-trioxo-7-(2-tetrahydropyran-2-ylpyrazol-3-yl)-2,3-dihydro-1λ⁶,5-benzothiazepin-3-yl]carbamate (160.0 mg, 0.230 mmol) in analogy to general procedure **6b** and was obtained as white solid, as a hydrochloride salt (71.7 mg, 0.15 mmol, 66% yield). MS (ESI): 435.1 [M+H]⁺.

### Example 38

### (3R)-3-amino-5-[(4-chlorophenyl)methyl]-7-(5-ethyl-1H-1,2,4-triazol-3-yl)-8-fluoro-1,1-dioxo-2,3-dihydro-1λ⁶,5-benzothiazepin-4-one

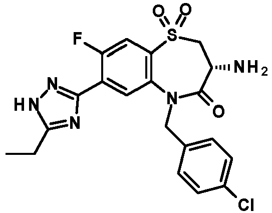

### Step a) tert-butyl N-[(3R)-5-[(4-chlorophenyl)methyl]-7-(5-ethyl-1H-1, 2, 4-triazol-3-yl)-8-fluoro-4-oxo-2, 3-dihydro-1, 5-benzothiazepin-3-yl]carbamate

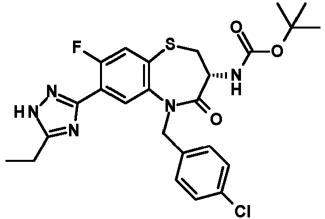

To a stirred solution of (3R)-3-(tert-butoxycarbonylamino)-5-[(4-chlorophenyl)methyl]-8-fluoro-4-oxo-2,3-dihydro-1,5-benzothiazepine-7-carboxylic acid (CAS 2002449-40-1) (200.0 mg, 0.420 mmol, 1 eq) in DMF (4 mL) were added NEt₃ (0.12 mL, 0.830 mmol, 2 eq) and HATU (173.92 mg, 0.460 mmol, 1.1 eq) and the mixture was stirred for 10 min at 20 °C. Then N-aminopropanamidine (40.25 mg, 0.420 mmol, 1 eq) was added and the reaction mixture was stirred at 20°C for 4 h. The mixture was diluted with H₂O (15 mL) and extracted with EtOAc (2 x 15 mL). The combined organic layers were washed with H₂O (5 mL), saturated CaCl₂ solution (5 mL) and brine (5 mL) and then dried with Na₂SO₄, filtered and concentrated in vacuo. The crude product was purified by column chromatography on silica gel (10% to 50% EtOAc in PE) to afford the title compound (135 mg, 0.250 mmol, 61% yield) as light yellow gum. MS (ESI): 532.2 [M+H]⁺.

### Step b) tert-butyl N-[(3R)-5-[(4-chlorophenyl)methyl]-7-(5-ethyl-1H-1, 2, 4-triazol-3-yl)-8-fluoro-1,1,4-trioxo-2, 3-dihydro-1λ⁶,5-benzothiazepin-3-yl]carbamate

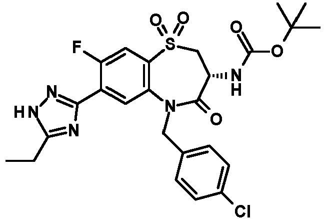

The title compound was prepared from tert-butyl N-[(3R)-5-[(4-chlorophenyl)methyl]-7-(5-ethyl-1H-1,2,4-triazol-3-yl)-8-fluoro-4-oxo-2,3-dihydro-1,5-benzothiazepin-3-yl]carbamate (65.0 mg, 0.120 mmol) in analogy to general procedure **5** and was obtained as light yellow gum (45 mg, 0.080 mmol, 65% yield). MS (ESI): 564.2 [M+H]⁺.

### Step c) (3R)-3-amino-5-[(4-chlorophenyl)methyl]-7-(5-ethyl-1H-1, 2, 4-triazol-3-yl)-8-fluoro-1,1-dioxo-2,3-dihydro-1λ⁶,5-benzothiazepin-4-one

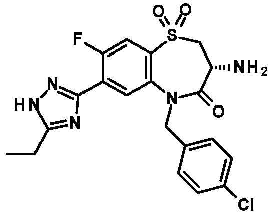

The title compound was prepared from tert-butyl N-[(3R)-5-[(4-chlorophenyl)methyl]-7-(5-ethyl-1H-1,2,4-triazol-3-yl)-8-fluoro-1,1,4-trioxo-2,3-dihydro-1λ⁶,5-benzothiazepin-3-yl]carbamate (45.0 mg, 0.080 mmol) in analogy to general procedure **6b** and was obtained as white solid, as a hydrochloride salt (13 mg, 0.030 mmol, 32% yield). MS (ESI): 463.9 [M+H]⁺.

### Example 39

### (3R)-3-amino-5-[(4-chlorophenyl)methyl]-1,1-dioxo-7-[3-(2,2,2-trifluoroethyl)-1,2,4-oxadiazol-5-yl]-2,3-dihydropyrido [3,2-b] [1,4]thiazepin-4-one

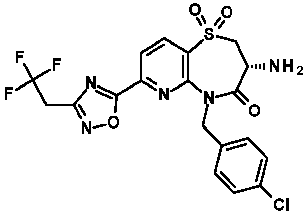

### Step a) (2R)-3-[(2-amino-6-methoxycarbonyl-3-pyridyl)sulfanyl]-2-(tert-butoxycarbonylamino)propanoic acid

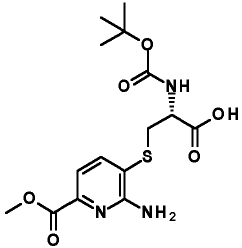

To a solution of methyl 6-amino-5-bromopyridine-2-carboxylate (CAS 178876-82-9) (4500 mg, 19.48 mmol, 1 eq) in toluene (54.15 mL) were added DIPEA (6.78 mL, 38.95 mmol, 2 eq), (2R)-2-(tert-butoxycarbonylamino)-3-sulfanyl-propanoic acid (4309.52 mg, 19.48 mmol, 1 eq), 9,9-dimethyl-4,5-bis(diphenylphosphino)xanthene (2253 mg, 3.9 mmol, 0.2 eq) and tris(dibenzylideneacetone)dipalladium(0) (1783 mg, 1.95 mmol, 0.1 eq) at 25°C. The reaction mixture was heated to 100°C and stirred for 1.5 h under an atmosphere of nitrogen. The reaction mixture was then filtered, the filter cake was washed with EtOAc (3 x 20 mL) and the volume of the filtrate was reduced in vacuo. The filtrate was extracted with H₂O (3 x 60 mL) and the combined aqueous layers were dried by lyophilization to obtain the crude title compound (7.84 g, 21.11 mmol, 97% yield) as light yellow solid which was used in next step without further purification. MS (ESI): 372 [M+H]⁺.

### Step b) methyl (3R)-3-(tert-butoxycarbonylamino)-4-oxo-3,5-dihydro-2H-pyrido[3,2-b][1,4]thiazepine-7-carboxylate

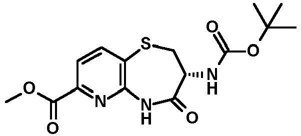

To a solution of (2R)-3-[(2-amino-6-methoxycarbonyl-3-pyridyl)sulfanyl]-2-(tert-butoxycarbonylamino)propanoic acid (7.48 g, 18.13 mmol, 1 eq) and DIPEA (6.31 mL, 36.25 mmol, 2 eq) in THF (74.8 mL) was added propylphosphonic anhydride solution (50% in EtOAC) (16.69 g, 36.25 mmol, 2 eq) at 0°C. The mixture was stirred for 4 h at 25°C and then concentrated in vacuo. The remaining residue was diluted with EtOAc (100 mL) and the organic layer was washed with brine (3 x 100 mL), dried with Na₂SO₄, filtered and concentrated in vacuo to obtain the crude product (4.92 g) as a yellow oil. The crude product was purified by column chromatography on silica gel (PE:EA = 10:1 to 3:1) to obtain the desired title compound (2.6 g, 7.36 mmol, 41% yield) as a white solid. MS (ESI): 354 [M+H] ⁺.

### Step c) methyl (3R)-3-(tert-butoxycarbonylamino)-5-[(4-chlorophenyl)methyl]-4-oxo-2, 3-dihydropyrido[3, 2-b][1,4]thiazepine-7-carboxylate

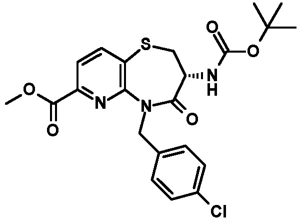

The title compound was prepared from methyl (3R)-3-(tert-butoxycarbonylamino)-4-oxo-3,5-dihydro-2H-pyrido[3,2-b][1,4]thiazepine-7-carboxylate (12.5 g, 35.3 mmol) and 1-(bromomethyl)-4-chlorobenzene (7.6 g, 37.0 mmol) in analogy to general procedure **4** and was obtained as white solid (14.0 g). MS (ESI): 478.2 [M+H]⁺.

### Step d) (3R)-3-(tert-butoxycarbonylamino)-5-[(4-chlorophenyl)methyl]-4-oxo-2, 3-dihydropyrido[3,2-b][1,4]thiazepine-7-carboxylic acid

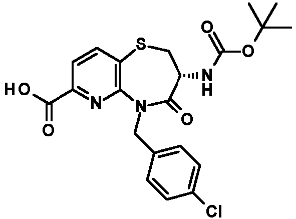

To a solution of methyl (3R)-3-(tert-butoxycarbonylamino)-5-[(4-chlorophenyl)methyl]-4-oxo-2,3-dihydropyrido[3,2-b][1,4]thiazepine-7-carboxylate (500.0 mg, 1.05 mmol, 1 eq) in THF (15 mL) was added a solution of LiOH•H2O (50.48 mg, 1.2 mmol, 1.15 eq) in water (5 mL) at 0°C. The reaction mixture was stirred for 0.5 h at 0°C. The reaction mixture was poured into a mixture of aqueous HCl (0.5 M, 3.6 mL) and EtOAc (20 mL) at 0°C with stirring. The resulting mixture was extracted with EtOAc (2 x 20 mL) and the combined organic phases were washed with brine (3 x 20 mL), dried with anhydrous Na₂SO₄, filtered and concentrated in vacuo to give thr title compound (480 mg, 1.03 mmol, 97% yield) as a light yellow solid which was used into next step without any further purification. MS (ESI): 408.0 [M-isobutene+H] ⁺.

### Step e) tert-butyl N-[(3R)-5-[(4-chlorophenyl)methyl]-4-oxo-7-[3-(2,2, 2-trifluoroethyl)-1,2,4-oxadiazol-5-yl]-2,3-dihydropyrido[3,2-b][1,4]thiazepin-3-yl]carbamate

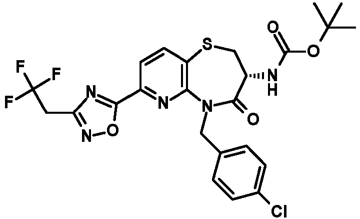

To a solution of (3R)-3-(tert-butoxycarbonylamino)-5-[(4-chlorophenyl)methyl]-4-oxo-2,3-dihydropyrido[3,2-b][1,4]thiazepine-7-carboxylic acid (220.0 mg, 0.470 mmol, 1 eq) in DMF (4 mL) was added CDI (84.58 mg, 0.520 mmol, 1.1 eq) at 25°C. After the mixture was stirred for 1 h at 25°C, a solution of 3,3,3-trifluoro-N'-hydroxy-propanamidine (CAS 1016726-53-6) (0.3 M in DMF, 8.4 mL, 2.52 mmol, 5.31 eq) was added into the mixture. Then the mixture was heated to 80°C and stirred for 16 h. After the mixture was cooled to RT, it was diluted with EtOAc (20 mL), then washed with brine (3 x 30 mL), dried with anhydrous Na₂SO₄, filtered and concentrated. The remaining crude product (300 mg) was purified by column chromatography on silica gel (PE:EtOAc = 5:1 to 1:1) to give the title compound (68 mg, 0.120 mmol, 22% yield) as a light brown gum. MS (ESI): 514.0 [M-isobuten+H] ⁺.

### Step f) tert-butyl N-[(3R)-5-[(4-chlorophenyl)methyl]-1,1,4-trioxo-7-[3-(2,2,2-trifluoroethyl)-1,2,4-oxadiazol-5-yl]-2,3-dihydropyrido[3,2-b][1,4]thiazepin-3yl]carbamate

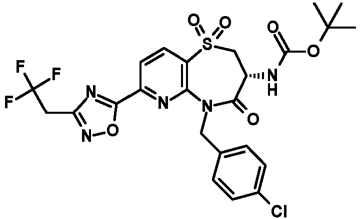

The title compound was prepared from tert-butyl N-[(3R)-5-[(4-chlorophenyl)methyl]-4-oxo-7-[3-(2,2,2-trifluoroethyl)-1,2,4-oxadiazol-5-yl]-2,3-dihydropyrido[3,2-b][1,4]thiazepin-3-yl]carbamate (68.0 mg, 0.120 mmol) in analogy to general procedure **5** and was obtained as light yellow solid (55 mg, 0.090 mmol, 67% yield). MS (ESI): 546 [M-isobutene+H] ⁺.

### Step g) (3R)-3-amino-5-[(4-chlorophenyl)methyl]-1,1-dioxo-7-[3-(2,2, 2-trifluoroethyl)-1, 2, 4-oxadiazol-5-yl]-2,3-dihydropyrido[3,2-b][1,4]thiazepin-4-one

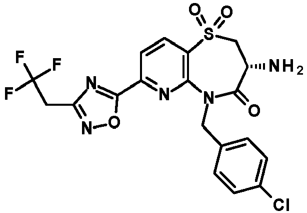

The title compound was prepared from tert-butyl N-[(3R)-5-[(4-chlorophenyl)methyl]-1,1,4-trioxo-7-[3-(2,2,2-trifluoroethyl)-1,2,4-oxadiazol-5-yl]-2,3-dihydropyrido[3,2-b][1,4]thiazepin-3-yl]carbamate (55.0 mg, 0.090 mmol) in analogy to general procedure **6b** and was obtained as a white solid (15.1 mg, 0.030 mmol, 32% yield). MS (ESI): 502.0 [M+H]⁺.

### Example 40

### (3R)-3-amino-5-[(4-chlorophenyl)methyl]-7-(3-cyclopropyl-1,2,4-oxadiazol-5-yl)-8-fluoro-1,1-dioxo-2,3-dihydro-1λ⁶,5-benzothiazepin-4-one

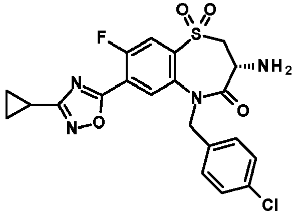

### Step a) tert-butyl N-[(3R)-5-[(4-chlorophenyl)methyl]-7-(cyclopropanecarboximidoylcarbamoyl)-8-fluoro-4-oxo-2,3-dihydro-1,5-benzothiazepin-3-yl]carbamate

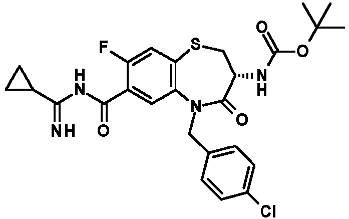

(3R)-3-(tert-butoxycarbonylamino)-5-[(4-chlorophenyl)methyl]-8-fluoro-4-oxo-2,3-dihydro-1,5-benzothiazepine-7-carboxylic acid (CAS 2002449-40-1) (50 mg, 104 µmol, Eq: 1) was combined with cyclopropanecarboximidamide hydrochloride (12.5 mg, 104 µmol, Eq: 1), HBTU (43.4 mg, 114 µmol, Eq: 1.1) and DIPEA (53.7 mg, 72.6 µl, 416 µmol, Eq: 4) in DMF (500 µL). The reaction was stirred at RT for 45 min. The reaction was quenched with water and was extracted with EtOAc. The organic layer was washed with brine, dried over magnesium sulfate, filtered and evaporated affording a brown oil (73 mg) containing the title. MS (ESI): 547.3 [M+H]⁺.

### Step b) tert-butyl N-[(3R)-5-[(4-chlorophenyl)methyl]-7-(3-cyclopropyl-1,2,4-oxadiazol-5-yl)-8-fluoro-4-oxo-2, 3-dihydro-1,5-benzothiazepin-3-yl]carbamate

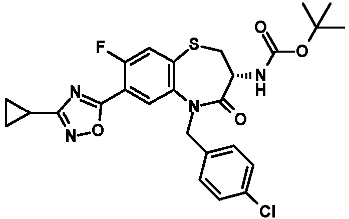

NBS (27.3 mg, 154 µmol, Eq: 1.2) and DBU (23.4 mg, 23.1 µl, 154 µmol, Eq: 1.2) were added to a solution of tert-butyl N-[(3R)-5-[(4-chlorophenyl)methyl]-7-(cyclopropanecarboximidoylcarbamoyl)-8-fluoro-4-oxo-2,3-dihydro-1,5-benzothiazepin-3-yl]carbamate (70 mg, 128 µmol, Eq: 1) in EtOAc (1 mL) and the reaction mixture was stirred at RT for 10 min. The reaction was quenched with water and was extracted with EtOAc. The organic layer was washed with brine, dried over magnesium sulfate, filtered and evaporated. The crude residue was purified by flash chromatography on silica gel (0-50% EtOAc in heptane) affording the title compound (37 mg, 67.9 µmol, 53% yield) as a white solid. MS (ESI): 489.2 [M-isobutene+H]⁺.

### Step c) tert-butyl N-[(3R)-5-[(4-chlorophenyl)methyl]-7-(3-cyclopropyl-1, 2, 4-oxadiazol-5-yl)-8-fluoro-1,1, 4-trioxo-2, 3-dihydro-1λ⁶,5-benzothiazepin-3-yl]carbamate

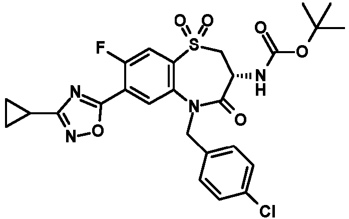

The title compound was prepared from tert-butyl N-[(3R)-5-[(4-chlorophenyl)methyl]-7-(3-cyclopropyl-1,2,4-oxadiazol-5-yl)-8-fluoro-4-oxo-2,3-dihydro-1,5-benzothiazepin-3-yl]carbamate (35 mg, 64.2 µmol, Eq: 1) in analogy to general procedure **5** to yield the title compound (33 mg, 57.2 µmol, 89% yield) as a white solid. MS (ESI): 521.2 [M-isobutene+H]⁺.

### Step d) (3R)-3-amino-5-[(4-chlorophenyl)methyl]-7-(3-cyclopropyl-1,2,4-oxadiazol-5-yl)-8-fluoro-1,1-dioxo-2,3-dihydro-1λ⁶,5-benzothiazepin-4-one

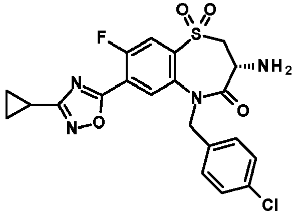

The title compound was prepared from tert-butyl N-[(3R)-5-[(4-chlorophenyl)methyl]-7-(3-cyclopropyl-1,2,4-oxadiazol-5-yl)-8-fluoro-1,1,4-trioxo-2,3-dihydro-1λ⁶,5-benzothiazepin-3-yl]carbamate (30 mg, 52 µmol, Eq: 1) in analogy to general procedure **6a** to yield the title compound (26 mg, 50.6 µmol, 97% yield) as a white solid, as a hydrochloride salt. MS (ESI): 477.1 [M+H]⁺.

Examples 41 to 45 of the following table were prepared in analogy to Example 40, using the appropriate amidine building block.

| **Ex.** | **Structure** | **Systematic Name** | **Building Block** | **MS, ESI: m/z** |
|---|---|---|---|---|
| 41 | 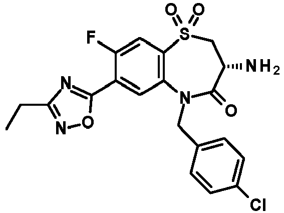 | (3R)-3-Amino-5-[(4-chlorophenyl)methyl]-7-(3-ethyl-1,2,4-oxadiazol-5-yl)-8-fluoro-1,1-dioxo-2,3-dihydro-1λ⁶,5-benzothiazepin-4-one | Propanami dine;hydro chloride | 465.2 [M+H]⁺ |
| | | | (CAS 3599-89-1) | |
| 42 | 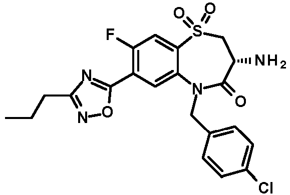 | (3R)-3-Amino-5-[(4-chlorophenyl)methyl]-8-fluoro-1,1-dioxo-7-(3-propyl-1,2,4-oxadiazol-5-yl)-2,3-dihydro-1λ⁶,5-benzothiazepin-4-one | Butanamid ine;hydroc hloride | 479.3 [M+H]⁺ |
| | | | (CAS 3020-81-3) | |
| 43 | 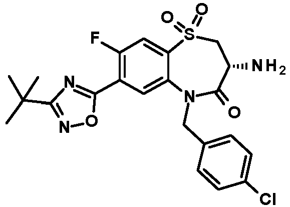 | (3R)-3-Amino-7-(3-tert-butyl-1,2,4-oxadiazol-5-yl)-5-[(4-chlorophenyl)methyl]-8-fluoro-1,1-dioxo-2,3-dihydro-1λ⁶,5-benzothiazepin-4-one | 2,2-Dimethylpr opanamidi ne;hydroc hloride | 493.2 [M+H]⁺ |
| | | | (CAS 18202-73-8) | |
| 44 | 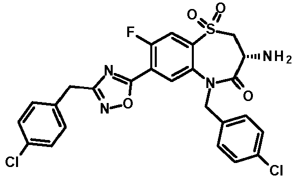 | (3R)-3-Amino-5-[(4-chlorophenyl)methyl]-7-[3-[(4-chlorophenyl)methyl]-1,2,4-oxadiazol-5-yl]-8-fluoro-1,1-dioxo-2,3-dihydro-1λ⁶,5-benzothiazepin-4-one | 2-(4-Chlorophe nyl)acetam idine;hydr ochloride | 561.2 [M+H]⁺ |
| | | | (CAS 6487-93-0) | |
| 45 | 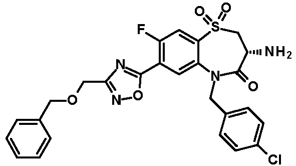 | (3R)-3-Amino-7-[3-(benzyloxymethyl)-1,2,4-oxadiazol-5-yl]-5-[(4-chlorophenyl)methyl]-8-fluoro-1,1-dioxo-2,3-dihydro-1λ⁶,5-benzothiazepin-4-one | 2-Benzyloxya cetamidine ;hydrochloride | 557.3 [M+H]⁺ |
| | | | (CAS 22018-43-5) | |

### Example 46

### (3R)-3-amino-5-[(4-chlorophenyl)methyl]-8-fluoro-7-[3-(2-hydroxy-1,1-dimethyl-ethyl)-1,2,4-oxadiazol-5-yl]-1,1-dioxo-2,3-dihydro-1λ⁶,5-benzothiazepin-4-one

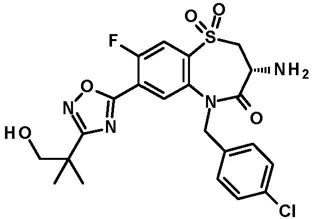

### Step a) N',3-dihydroxy-2, 2-dimethyl-propanamidine

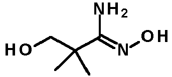

3-hydroxy-2,2-dimethyl-propionitrile (50 mg, 0.504 mmol) was combined with hydroxylamine hydrochloride (175 mg, 2.5 mmol, 5 eq) and Et₃N (306 mg, 421 uL, 3.0 mmol, 6 eq) in THF (0.5 mL). The reaction was heated to 80 °C and was stirred over night. EtOAc was added and the reaction was extracted with aq. sat. NaHCO₃. The organic layer was washed with water and brine, dried over magnesium sulfate, filtered and evaporated affording the crude title compound (27 mg, 40% yield) as colorless oil.

### Step b) tert-butyl N-[(3R)-5-[(4-chlorophenyl)methyl]-8-fluoro-7-[3-(2-hydroxy-1,1-dimethylethyl)-1,2, 4-oxadiazol-5-yl]-4-oxo-2,3-dihydro-1,5-benzothiazepin-3-yl]carbamate

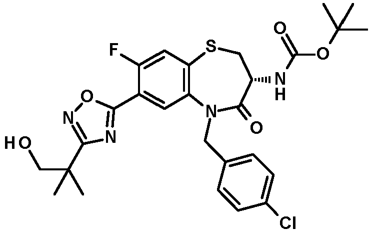

The title compound was prepared in analogy to general procedure **10b** from 3-hydroxy-2,2-dimethyl-propanehydroxamic acid (27 mg, 0.204 mmol) and (3R)-3-(tert-butoxycarbonylamino)-5-(4-chlorobenzyl)-8-fluoro-4-keto-2,3-dihydro-1,5-benzothiazepine-7-carboxylic acid (98.2 mg, 0.204 mmol) after column chromatography on silica gel (heptane:EtOAc = 1:0 to 1:1) as a white solid (22 mg, 18% yield). MS (ESI): 521.3 [M-isobutene+H]⁺.

### Step c) tert-butyl N-[(3R)-5-[(4-chlorophenyl)methyl]-8-fluoro-7-[3-(2-hydroxy-1,1-dimethylethyl)-1,2, 4-oxadiazol-5-yl]-1,1, 4-trioxo-2, 3-dihydro-1λ⁶,5-benzothiazepin-3-yl]carbamate

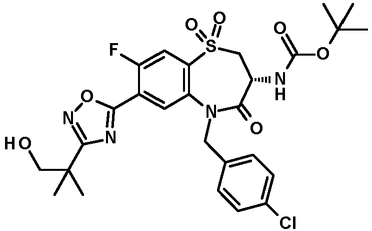

The title compound was prepared in analogy to general procedure **5** from tert-butyl N-[(3R)-5-[(4-chlorophenyl)methyl]-8-fluoro-7-[3-(2-hydroxy-1,1-dimethyl-ethyl)-1,2,4-oxadiazol-5-yl]-4-oxo-2,3-dihydro-1,5-benzothiazepin-3-yl]carbamate (22 mg, 0.037 mmol) after column chromatography on silica gel (heptane:EtOAc = 1:0 to 1:1) as a white solid (14 mg, 61% yield). MS (ESI): 553.2 [M-isobutene+H]⁺.

### Step d) rac-(3R)-3-amino-5-[(4-chlorophenyl)methyl]-8-fluoro-7-[3-(2-hydroxy-1,1-dimethylethyl)-1,2, 4-oxadiazol-5-yl]-1,1-dioxo-2,3-dihydro-1λ⁶,5-benzothiazepin-4-one

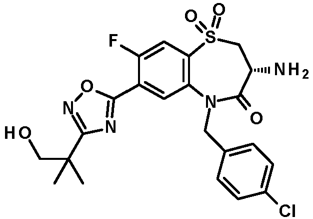

The title compound was prepared in analogy to general procedure **6d** from tert-butyl N-[(3R)-5-[(4-chlorophenyl)methyl]-8-fluoro-7-[3-(2-hydroxy-1,1-dimethyl-ethyl)-1,2,4-oxadiazol-5-yl]-1,1,4-trioxo-2,3-dihydro-1λ⁶,5-benzothiazepin-3-yl]carbamate (14 mg, 0.023 mmol) as a white solid as a hydrochloride salt (12 mg, 95% yield). MS (ESI): 509.2 [M+H]⁺.

### Example 47

### (3R)-3-amino-5-[(4-chlorophenyl)methyl]-8-fluoro-7-[5-(1-fluoro-1-methyl-ethyl)-1,2,4-oxadiazol-3-yl]-1,1-dioxo-2,3-dihydro-1λ⁶,5-benzothiazepin-4-one

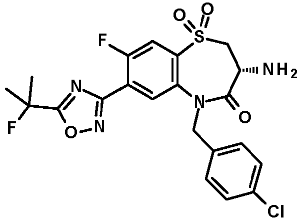

### Step a) [(Z)-[amino-[(3R)-3-(tert-butoxycarbonylamino)-5-[(4-chlorophenyl)methyl]-8-fluoro-1, 1, 4-trioxo-2, 3-dihydro-1λ⁶,5-benzothiazepin- 7-yl]methylene]amino] 2-fluoro-2-methylpropanoate

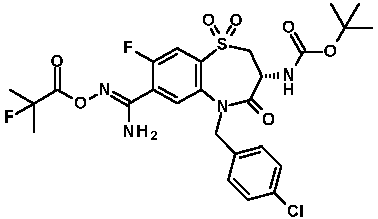

The title compound was prepared in analogy to general procedure **10c** from tert-butyl N-[(3R)-8-fluoro-7-[(Z)-N'-hydroxycarbamimidoyl]-1,1,4-trioxo-3,5-dihydro-2H-1λ6,5-benzothiazepin-3-yl]carbamate (130 mg, 0.32 mmol) and 2-fluoroisobutyric acid (68.5 mg, 0.65 mmol, 2 eq) as a yellow oil (70 mg, 0.14 mmol, 25% yield). MS (ESI): 636.9 [M+Na]⁺.

### Step b) tert-butyl N-[(3R)-5-[(4-chlorophenyl)methyl]-8-fluoro-7-[5-(1-fluoro-1-methyl-ethyl)-1,2, 4-oxadiazol-3-yl]-1,1, 4-trioxo-2, 3-dihydro-1λ⁶,5-benzothiazepin-3-yl]carbamate

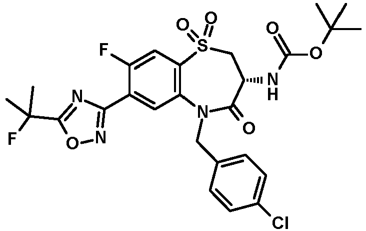

The title compound was prepared in analogy to general procedure **11a** from [[(3R)-3-(tert-butoxycarbonylamino)-5-[(4-chlorophenyl)methyl]-8-fluoro-1,1,4-trioxo-2,3-dihydro-1λ⁶,5-benzothiazepine-7-carbonyl]amino] 2-fluoro-2-methyl-propanoate (70 mg, 0.11 mmol) as a yellow oil (60 mg, 0.10 mmol, 37% yield). MS (ESI): 618.9 [M+Na]⁺.

### Step c) (3R)-3-amino-5-[(4-chlorophenyl)methyl]-8-fluoro-7-[5-(1-fluoro-1-methyl-ethyl)-1,2,4-oxadiazol-3-yl]-1,1-dioxo-2,3-dihydro-1λ⁶,5-benzothiazepin-4-one

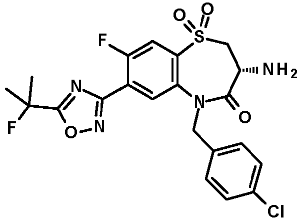

The title compound was prepared in analogy to general procedure **6b** tert-butyl N-[(3R)-5-[(4-chlorophenyl)methyl]-8-fluoro-7-[5-(1-fluoro-1-methyl-ethyl)-1,2,4-oxadiazol-3-yl]-1,1,4-trioxo-2,3-dihydro-1λ⁶,5-benzothiazepin-3-yl]carbamate (60 mg, 0.10 mmol) after prep-HPLC (HCl) as an orange solid as a hydrochloride salt (11.9 mg, 0.02 mmol, 21% yield). MS (ESI): 496.8 [M+H]⁺.

### Example 48

### (3R)-3-amino-7-[5-(1-amino-2,2,2-trifluoro-ethyl)-1,2,4-oxadiazol-3-yl]-8-fluoro-5-[(4-isopropoxyphenyl)methyl]-1,1-dioxo-2,3-dihydro-1λ⁶,5-benzothiazepin-4-one

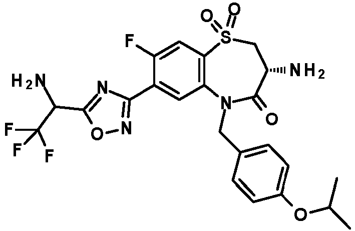

### Step a) [(Z)-[amino-[(3R)-3-(tert-butoxycarbonylamino)-8-fluoro-5-[(4-isopropoxyphenyl)methyl]-4-oxo-2,3-dihydro-1, 5-benzothiazepin-7-yl]methylene]amino] 2-(tert-butoxycarbonylamino)-3, 3, 3-trifluoro-propanoate

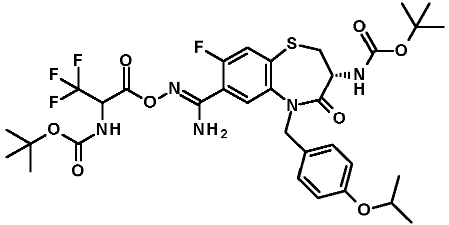

The title compound was prepared in analogy to general procedure **10a** from tert-butyl N-[(3R)-8-fluoro-7-[(Z)-N'-hydroxycarbamimidoyl]-5-[(4-isopropoxyphenyl)methyl]-4-oxo-2,3-dihydro-1,5-benzothiazepin-3-yl]carbamate (350 mg, 0.67 mmol,) and 2-(tert-butoxycarbonylamino)-3,3,3-trifluoro-propanoic acid (229 mg, 0.94 mmol, 1.4 eq) after prep-HPLC as a yellow oil (200 mg, 0.27 mmol, 37% yield). MS (ESI): 688.3 [M-isobutene+H]⁺.

### Step b) tert-butyl N-[(3R)-7-[5-[1-(tert-butoxycarbonylamino)-2, 2, 2-trifluoro-ethyl]-1, 2,4-oxadiazol-3-yl]-8-fluoro-5-[(4-isopropoxyphenyl)methyl]-4-oxo-2,3-dihydro-1,5-benzothiazepin-3-yl]carbamate

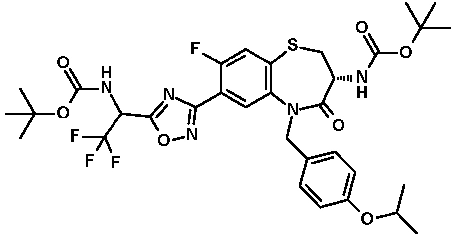

The title compound was prepared in analogy to general procedure **11a** from [[(3R)-3-(tert-butoxycarbonylamino)-8-fluoro-5-[(4-isopropoxyphenyl)methyl]-4-oxo-2,3-dihydro-1,5-benzothiazepine-7-carbonyl]amino] 2-(tert-butoxycarbonylamino)-3,3,3-trifluoro-propanoate (50 mg, 0.07 mmol) after prep-TLC (PE:EA = 3:1) as a white solid (70 mg, 0.10 mmol, 129% yield). MS (ESI): 726.4 [M+H]⁺.

### Step c) tert-butyl N-[(3R)-7-[5-[1-(tert-butoxycarbonylamino)-2,2, 2-trifluoro-ethyl]-1,2, 4-oxadiazol-3-yl]-8-fluoro-5-[(4-isopropoxyphenyl)methyl]-1,1, 4-trioxo-2, 3-dihydro-1λ⁶,5-benzothiazepin-3-yl]carbamate

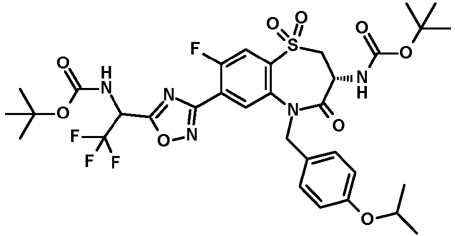

The title compound was prepared in analogy to general procedure **5** from tert-butyl N-[(3R)-7-[5-[1-(tert-butoxycarbonylamino)-2,2,2-trifluoro-ethyl]-1,2,4-oxadiazol-3-yl]-8-fluoro-5-[(4-isopropoxyphenyl)methyl]-4-oxo-2,3-dihydro-1,5-benzothiazepin-3-yl]carbamate (60 mg, 0.08 mmol) and was obtained after prep-TLC (PE:EA = 2:1) as a white solid (60 mg, 0.08 mmol, 87% yield). MS (ESI): 780.2 [M+Na]⁺.

### Step d) (3R)-3-amino-7-[5-(1-amino-2, 2, 2-trifluoro-ethyl)-1, 2, 4-oxadiazol-3-yl]-8-fluoro-5-[(4-isopropoxyphenyl)methyl]-1, 1-dioxo-2,3-dihydro-1λ⁶,5-benzothiazepin-4-one

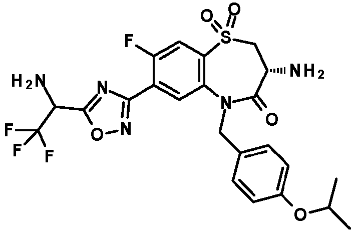

The title compound was prepared in analogy to general procedure **6b** from tert-butyl N-[(3R)-7-[5-[1-(tert-butoxycarbonylamino)-2,2,2-trifluoro-ethyl]-1,2,4-oxadiazol-3-yl]-8-fluoro-5-[(4-isopropoxyphenyl)methyl]-1,1,4-trioxo-2,3-dihydro-1λ⁶*,*5-benzothiazepin-3-yl]carbamate (54 mg, 0.07 mmol) and was obtained after prep-HPLC as a white solid as a hydrochloride salt (12.5 mg, 0.020 mmol, 27% yield). MS (ESI): 558.1 [M+H]⁺.

### Example 49

### (3R)-3-amino-5-[(4-chlorophenyl)methyl]-8-fluoro-1,1-dioxo-7-[3-(2,2,2-trifluoroethyl)-1,2,4-oxadiazol-5-yl]-2,3-dihydro-1λ⁶,5-benzothiazepin-4-one

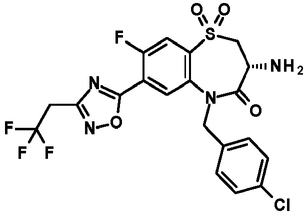

### Step a) tert-butyl N-[(3R)-5-[(4-chlorophenyl)methyl]-8-fluoro-4-oxo-7-[3-(2,2,2-trifluoroethyl)-1,2,4-oxadiazol-5-yl]-2,3-dihydro-1,5-benzothiazepin-3-yl]carbamate

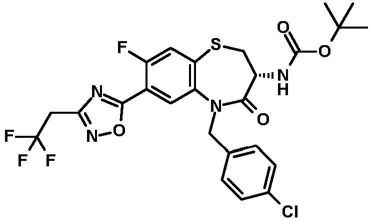

(3R)-3-(tert-butoxycarbonylamino)-5-[(4-chlorophenyl)methyl]-8-fluoro-4-oxo-2,3-dihydro-1,5-benzothiazepine-7-carboxylic acid (200 mg, 416 µmol, Eq: 1) was combined with (Z)-3,3,3-trifluoro-N'-hydroxypropanimidamide (59.1 mg, 416 µmol, Eq: 1), HATU (174 mg, 457 µmol, Eq: 1.1) and DIPEA (107 mg, 145 µl, 832 µmol, Eq: 2) in THF (4 mL) and the reaction was stirred at RT for 2 h to form the intermediate tert-butyl N-[(3R)-5-[(4-chlorophenyl)methyl]-8-fluoro-7-[[(Z)-N-hydroxy-C-(2,2,2-trifluoroethyl)carbonimidoyl]carbamoyl]-4-oxo-2,3-dihydro-1,5-benzothiazepin-3-yl]carbamate. Burgess Reagent (495 mg, 2.08 mmol, Eq: 5) was added, the reaction was heated to 100°C and was stirred over night. The solvent was evaporated. The crude residue was purified by chromatography on silica gel (0-30% EtOAc in heptane) affording the title compound (85 mg, 89.8 µmol, 22% yield) as a yellow oil. MS (ESI): 531.1 [M-isobutene+H]⁺.

### Step b) tert-butyl N-[(3R)-5-[(4-chlorophenyl)methyl]-8-fluoro-1,1, 4-trioxo-7-[3-(2,2,2-trifluoroethyl)-1,2, 4-oxadiazol-5-yl]-2,3-dihydro-1λ⁶,5-benzothiazepin-3-yl]carbamate

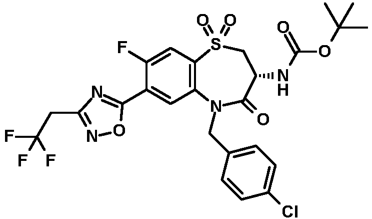

The title compound was prepared from tert-butyl N-[(3R)-5-[(4-chlorophenyl)methyl]-8-fluoro-4-oxo-7-[3-(2,2,2-trifluoroethyl)-1,2,4-oxadiazol-5-yl]-2,3-dihydro-1,5-benzothiazepin-3-yl]carbamate (85 mg, 89.8 µmol, Eq: 1) in analogy to general procedure **5** and was obtained as a white solid (84 mg, 89.6 µmol, 100% yield). MS (ESI): 563.1 [M-isobutene+H]⁺.

### Step c) (3R)-3-amino-5-[(4-chlorophenyl)methyl]-8-fluoro-1,1-dioxo-7-[3-(2,2,2-trifluoroethyl)-1,2, 4-oxadiazol-5-yl]-2,3-dihydro-1λ⁶,5-benzothiazepin-4-one

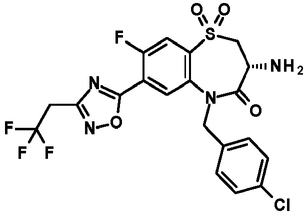

The title compound was prepared from tert-butyl N-[(3R)-5-[(4-chlorophenyl)methyl]-8-fluoro-1,1,4-trioxo-7-[3-(2,2,2-trifluoroethyl)-1,2,4-oxadiazol-5-yl]-2,3-dihydro-1λ⁶,5-benzothiazepin-3-yl]carbamate (80 mg, 85.3 µmol, Eq: 1) in analogy to general procedure **6a** and was obtained as a white solid, as a hydrochloride salt (43 mg, 82.9 µmol, 97% yield). MS (ESI): 519.1 [M+H]⁺.

Example 50 of the following table was prepared in analogy to Example 49, using the appropriate imidamide building block.

| **Ex.** | **Structure** | **Systematic Name** | **Building Block** | **MS, ESI: m/z** |
|---|---|---|---|---|
| 50 | 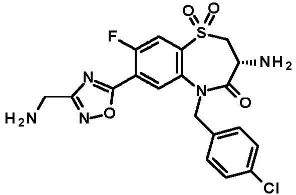 | (3R)-3-amino-7-[3-(aminomethyl)-1,2,4-oxadiazol-5-yl]-5-[(4-chlorophenyl)methyl]-8-fluoro-1,1-dioxo-2,3-dihydro-1λ⁶,5-benzothiazepin-4-one | tert-Butyl N-[(2Z)-2-amino-2-hydroxyimi no-ethyl]carb amate | 466.07 40 [M+H]⁺ |
| | | | (CAS 479079-15-7) | |

### Example 51

### (3R)-3-amino-5-[(4-chlorophenyl)methyl]-7-(3-ethyl-1,2,4-oxadiazol-5-yl)-1,1-dioxo-2,3-dihydro-1λ⁶,5-benzothiazepin-4-one

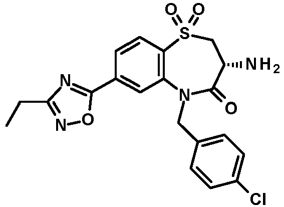

### Step a) tert-butyl N-[(3R)-5-[(4-chlorophenyl)methyl]-7-(3-ethyl-1, 2, 4-oxadiazol-5-yl)-4-oxo-2, 3-dihydro-1, 5-benzothiazepin-3-yl]carbamate

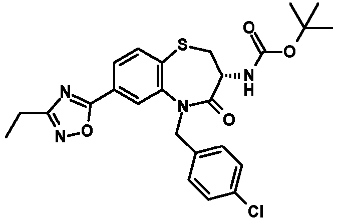

(3R)-3-(tert-butoxycarbonylamino)-5-[(4-chlorophenyl)methyl]-4-oxo-2,3-dihydro-1,5-benzothiazepine-7-carboxylic acid (CAS 2002449-61-6) (50 mg, 108 µmol, Eq: 1) was combined with propionimidamide hydrochloride (11.7 mg, 108 µmol, Eq: 1), HBTU (45.1 mg, 119 µmol, Eq: 1.1) and DIPEA (55.8 mg, 75.5 µL, 432 µmol, Eq: 4) in DMF (500 µL). The reaction mixture was stirred at RT for 45 min. The reaction was quenched with water and was extracted with EtOAc. The organic layer was washed with brine, dried over magnesium sulfate, filtered and evaporated affording the intermediate tert-butyl N-[(3R)-5-[(4-chlorophenyl)methyl]-4-oxo-7-(propanimidoylcarbamoyl)-2,3-dihydro-1,5-benzothiazepin-3-yl]carbamate as an orange oil. This material was dissolved in EtOAc (1 mL). NBS (23.1 mg, 130 µmol, Eq: 1.2) and DBU (19.7 mg, 19.5 µL, 130 µmol, Eq: 1.2) were added successively and the reaction was stirred at RT for 10 min. The reaction was quenched with water and was extracted with EtOAc. The organic layer was washed with brine, dried over magnesium sulfate, filtered and evaporated. The crude residue was purified by chromatography on silica gel (0-50% EtOAc in heptane) affording the title compound (30 mg, 56.5 µmol, 52% yield) as a light yellow oil. MS (ESI): 459.2 [M-isobutene+H] ⁺.

### Step b) tert-butyl N-[(3R)-5-[(4-chlorophenyl)methyl]-7-(3-ethyl-1,2,4-oxadiazol-5-yl)-1,1,4-trioxo-2, 3-dihydro-1λ⁶,5-benzothiazepin-3-yl]carbamate

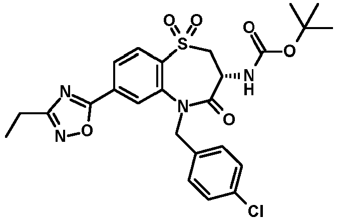

The title compound was prepared from tert-butyl N-[(3R)-5-[(4-chlorophenyl)methyl]-7-(3-ethyl-1,2,4-oxadiazol-5-yl)-4-oxo-2,3-dihydro-1,5-benzothiazepin-3-yl]carbamate (30 mg, 58.2 µmol, Eq: 1) in analogy to general procedure **5** and was obtained as a white solid (13 mg, 23.3 µmol, 40% yield). MS (ESI): 491.1 [M-isobutene+H]⁺.

### Step c) (3R)-3-amino-5-[(4-chlorophenyl)methyl]-7-(3-ethyl-1,2, 4-oxadiazol-5-yl)-1,1-dioxo-2,3-dihydro-1λ⁶,5-benzothiazepin-4-one

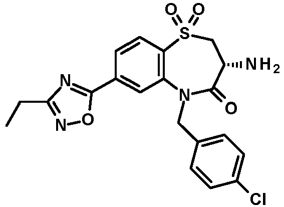

The title compound was prepared from tert-butyl N-[(3R)-5-[(4-chlorophenyl)methyl]-7-(3-ethyl-1,2,4-oxadiazol-5-yl)-1,1,4-trioxo-2,3-dihydro-1λ⁶,5-benzothiazepin-3-yl]carbamate (11 mg, 20.1 µmol, Eq: 1) in analogy to general procedure **6a** and was obtained as a white solid, as a hydrochloride salt (7 mg, 14.5 µmol, 72% yield). MS (ESI): 447.2 [M+H]⁺.

### Example 52

### (3R)-3-amino-7-(3-tert-butylisoxazol-5-yl)-5-[(4-chlorophenyl)methyl]-8-fluoro-1,1-dioxo-2,3-dihydro-1λ⁶,5-benzothiazepin-4-one

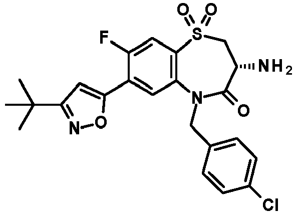

### Step a) tert-butyl N-[(3R)-7-(3-tert-butylisoxazol-5-yl)-5-[(4-chlorophenyl)methyl]-8-fluoro-4-oxo-2,3-dihydro-1,5-benzothiazepin-3-yl]carbamate

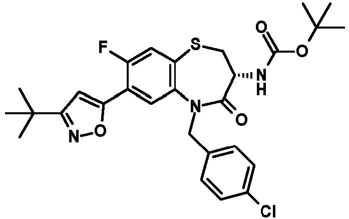

tert-butyl-N-[(3 R)-5-[(4-chlorophenyl)methyl]-7-ethynyl-8-fluoro-4-oxo-2,3-dihydro-1,5-benzothiazepin-3-yl]carbamate (Example 6, step e) (100 mg, 217 µmol,Eq: 1) was dissolved in DCM (3 mL) and the colorless solution was cooled down to 0°C. (1E)-2,2-dimethylpropanal oxime (35.1 mg, 40.8 µl, 347 µmol, Eq: 1.6), NEt₃ (439 mg, 605 µl, 4.34 mmol, Eq: 20) and sodium hypochlorite 12% in water (2.02 g, 1.67 ml, 3.25 mmol, Eq: 15) were added successively.

The reaction was heated to 30 °C and was stirred overnight. The solvent was evaporated. EtOAc was added and the mixture was washed with saturated aqueous NaHCO₃ solution, water and brine. The organic layer was dried over magnesium sulfate, filtered and evaporated. The crude residue was purified by chromatography on silica gel (0-30% EtOAc in heptane) affording the title compound (103 mg, 153 µmol, 70% yield) as a white solid. MS (ESI): 504.1 [M-isobutene+H]⁺.

### Step b) tert-butyl N-[(3R)-7-(3-tert-butylisoxazol-5-yl)-5-[(4-chlorophenyl)methyl]-8-fluoro-1,1,4-trioxo-2, 3-dihydro-1λ⁶,5-benzothiazepin-3-yl]carbamate

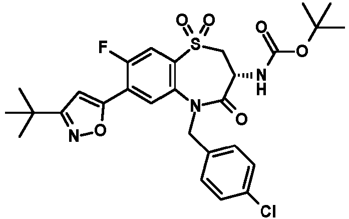

The title compound was prepared from tert-butyl N-[(3R)-7-(3-tert-butylisoxazol-5-yl)-5-[(4-chlorophenyl)methyl]-8-fluoro-4-oxo-2,3-dihydro-1,5-benzothiazepin-3-yl]carbamate (100 mg, 179 µmol, Eq: 1) in analogy to general procedure **5** and was obtained as a white solid (64 mg, 94 µmol, 53% yield). MS (ESI): 536.1 [M-isobutene+H]⁺.

### Step c) (3R)-3-amino-7-(3-tert-butylisoxazol-5-yl)-5-[(4-chlorophenyl)methyl]-8-fluoro-1,1-dioxo-2, 3-dihydro-1λ⁶,5-benzothiazepin-4-one

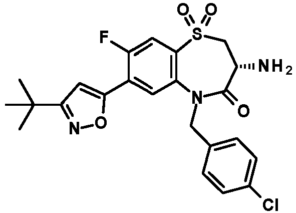

The title compound was prepared from tert-butyl N-[(3R)-7-(3-tert-butylisoxazol-5-yl)-5-[(4-chlorophenyl)methyl]-8-fluoro-1,1,4-trioxo-2,3-dihydro-1λ⁶,5-benzothiazepin-3-yl]carbamate (60 mg, 101 µmol, Eq: 1) in analogy to general procedure **6a** and was obtained as a white solid (21 mg, 42.7 µmol, 42% yield). MS (ESI): 492.1 [M+H]⁺.

### Example 53

### (3R)-3-amino-7-[5-(2,2-difluorocyclohexyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one

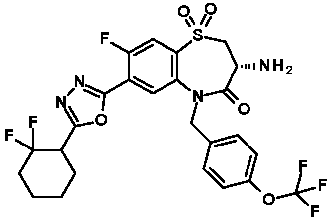

### Step a) methyl (3R)-3-(tert-butoxycarbonylamino)-8-fluoro-4-oxo-5-[[4-(trifluoromethoxy)phenyl]methyl]-2, 3-dihydro-1, 5-benzothiazepine-7-carboxylate

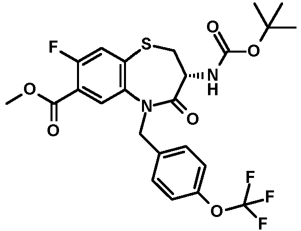

To a solution of methyl (R)-3-((tert-butoxycarbonyl)amino)-8-fluoro-4-oxo-2,3,4,5-tetrahydrobenzo[b][1,4]thiazepine-7-carboxylate (1.24 g, 3.35 mmol, Eq: 1) (CAS: 2002449-38-7) in DMSO (15 mL) were added 1-(bromomethyl)-4-(trifluoromethoxy)benzene (1.28 g, 803 µl, 5.02 mmol, Eq: 1.5), potassium carbonate (1.39 g, 10 mmol, Eq: 3) and potassium iodide (278 mg, 1.67 mmol, Eq: 0.5) and the reaction mixture was stirred at RT for 1 h. The reaction mixture was poured into water (100 mL) and the mixture was extracted with EtOAc (2 x 100 mL), dried over magnesium sulfate, filtered and evaporated. The crude residue was purified by chromatography on silica gel (0-40% EtOAc in heptane) affording the title compound (1.535 g, 2.26 mmol, 68% yield) as a white solid. MS (ESI): 489.0 [M-isobutene+H]⁺.

### Step b) (3R)-3-(tert-butoxycarbonylamino)-8-fluoro-4-oxo-5-[[4-(trifluoromethoxy)phenyl]methyl]-2,3-dihydro-1,5-benzothiazepine-7-carboxylic acid

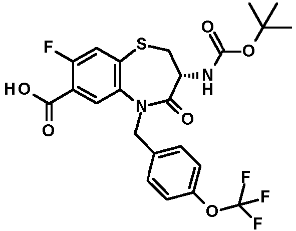

Methyl (3R)-3-(tert-butoxycarbonylamino)-8-fluoro-4-oxo-5-[[4-(trifluoromethoxy)phenyl]methyl]-2,3-dihydro-1,5-benzothiazepine-7-carboxylate (1.535 g, 2.82 mmol, Eq: 1) was dissolved in a mixture of THF (28 mL), MeOH (4 mL) and water (8 mL). Lithium hydroxide (135 mg, 5.64 mmol, Eq: 2) was added and the reaction mixture was stirred at RT for 2 h. The reaction mixture was washed with aqueous 1M HCl and brine. The organic layer was dried with magnesium sulfate. After filtration the solvent was evaporated affording the crude title compound (1.53 g, 2.81 mmol, 99% yield) as an off-white solid. MS (ESI): 529.1 [M-H]⁻.

### Step c) tert-butyl N-[(3R)-8-fluoro-7-(hydrazinecarbonyl)-4-oxo-5-[[4-(trifluoromethoxy)phenyl]methyl]-2, 3-dihydro-1, 5-benzothiazepin-3-yl]carbamate

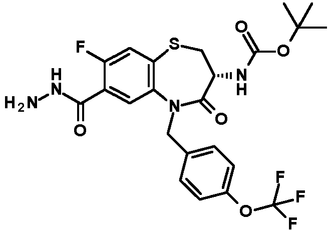

A mixture of (3R)-3-(tert-butoxycarbonylamino)-8-fluoro-4-oxo-5-[[4-(trifluoromethoxy)phenyl]methyl]-2,3-dihydro-1,5-benzothiazepine-7-carboxylic acid (250 mg, 471 µmol, Eq: 1) and CDI (99.3 mg, 613 µmol, Eq: 1.3) in THF (3 mL) was stirred for 30 min at RT and then a solution of hydrazine hydrate (70.8 mg, 68.6 µl, 1.41 mmol, Eq: 3) in THF (1 mL) was added. The reaction mixture was stirred at RT for 2 h. The reaction mixture was poured into water (20 mL) and was extracted with EtOAc (3 x 20 mL), dried over magnesium sulfate, filtered and evaporated. The crude material was purified by chromatography on silica gel (0% to 50% EtOAc in heptane) affording the title compound (167 mg, 307 µmol, 65% yield) as colorless solid. MS (ESI): 489.0 [M-isobutene+H]⁺.

### Step d) tert-butyl N-[(3R)-7-[5-(2,2-difluorocyclohexyl)-1,3, 4-oxadiazol-2-yl]-8-fluoro-4-oxo-5-[[4-(trifluoromethoxy)phenyl]methyl]-2,3-dihydro-1, 5-benzothiazepin-3-yl]carbamate

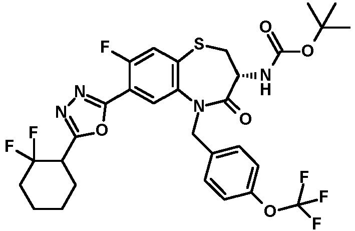

A mixture of tert-butyl N-[(3R)-8-fluoro-7-(hydrazinecarbonyl)-4-oxo-5-[[4-(trifluoromethoxy)phenyl]methyl]-2,3-dihydro-1,5-benzothiazepin-3-yl]carbamate (50 mg, 91.8 µmol, Eq: 1), 2,2-difluorocyclohexane-1-carboxylic acid (16.6 mg, 101 µmol, Eq: 1.1) in THF (1 mL), HATU (38.4 mg, 101 µmol, Eq: 1.1) and DIPEA (23.7 mg, 32.1 µl, 184 µmol, Eq: 2) was stirred at RT for 30 min. Burgess reagent (109 mg, 459 µmol, Eq: 5) was added and stirring was continued at RT overnight. The solvent was evaporated and the crude material was purified by column chromatography on silica gel (0-50% EtOAc in heptane), affording the title compound (33 mg, 41.1 µmol, 45% yield) as white solid. MS (ESI): 617.0 [M-isobutene+H]⁺.

### Step e) tert-butyl N-[(3R)-7-[5-(2, 2-difluorocyclohexyl)-1, 3, 4-oxadiazol-2-yl]-8-fluoro-1,1, 4-trioxo-5-[[4-(trifluoromethoxy)phenyl]methyl]-2, 3-dihydro-1λ⁶,5-benzothiazepin-3-yl]carbamate

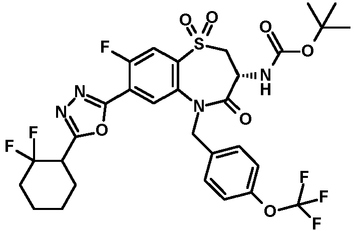

The title compound was prepared according to general method **5** from tert-butyl N-[(3R)-7-[5-(2,2-difluorocyclohexyl)-1,3,4-oxadiazol-2-yl]-8-fluoro-4-oxo-5-[[4-(trifluoromethoxy)phenyl]methyl]-2,3-dihydro-1,5-benzothiazepin-3-yl]carbamate (33 mg, 49.1 µmol) and was obtained as white solid (18.3 mg, 22.1 µmol, 45% yield). MS (ESI): 649.1 [M-isobuten+H]⁺.

### Step f) (3R)-3-amino-7-[5-(2, 2-difluorocyclohexyl)-1,3, 4-oxadiazol-2-yl]-8-fluoro-1,1-dioxo-5-[[4-(trifluoromethoxy)phenyl]methyl]-2, 3-dihydro-1λ⁶,5-benzothiazepin-4-one

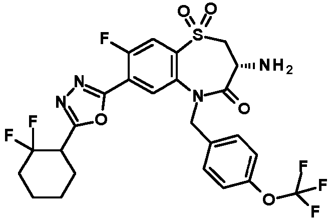

The title compound was prepared according to general method **6c** from tert-butyl N-[(3R)-7-[5-(2,2-difluorocyclohexyl)-1,3,4-oxadiazol-2-yl]-8-fluoro-1,1,4-trioxo-5-[[4-(trifluoromethoxy)phenyl]methyl]-2,3-dihydro-1λ⁶,5-benzothiazepin-3-yl]carbamate (16 mg, 22.7 µmol, Eq: 1) and was obtained as a light yellow solid, as a 1,1,1,3,3,3-hexafluoropropan-2-ol adduct. (17.5 mg, 20.6 µmol, 91% yield). MS (ESI): 605.4 [M+H]⁺.

### Example 54

### (3R)-3-amino-7-(5-tert-butyl-1,3,4-oxadiazol-2-yl)-8-fluoro-5-[[4-(trifluoromethoxy)phenyl]methyl]-2,3-dihydro-1,5-benzothiazepin-4-one

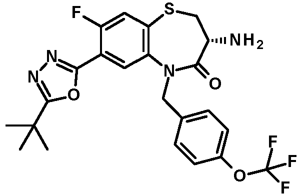

### Step a) tert-butyl N-[(3R)-7-(5-tert-butyl-1,3,4-oxadiazol-2-yl)-8-fluoro-4-oxo-5-[[4-(trifluoromethoxy)phenyl]methyl]-2,3-dihydro-1,5-benzothiazepin-3-yl]carbamate

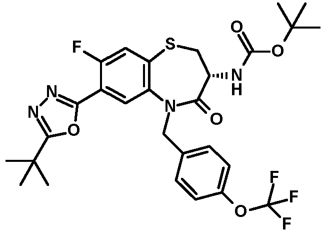

The title compound was prepared according to the method used in Example 53, step d) from (R)-3-((tert-butoxycarbonyl)amino)-8-fluoro-4-oxo-5-(4-(trifluoromethoxy)benzyl)-2,3,4,5-tetrahydrobenzo[b][1,4]thiazepine-7-carboxylic acid (50 mg, 94.3 µmol) and was obtained (45 mg, 73.7 µmol, 78% yield) as a light yellow oil. MS (ESI): 555.3 [M-isobutene+H]⁺.

### Step b) (3R)-3-amino-7-(5-tert-butyl-1,3,4-oxadiazol-2-yl)-8-fluoro-5-[[4-(trifluoromethoxy)phenyl]methyl]-2,3-dihydro-1,5-benzothiazepin-4-one

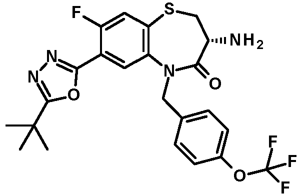

The title compound was prepared according to general method **6c** from tert-butyl N-[(3R)-7-(5-tert-butyl-1,3,4-oxadiazol-2-yl)-8-fluoro-4-oxo-5-[[4-(trifluoromethoxy)phenyl]methyl]-2,3-dihydro-1,5-benzothiazepin-3-yl]carbamate (12 mg, 19.7 µmol) with additional 500 µl HCl (4M in dioxane) and was obtained as a white solid, as a hydrochloride salt (11 mg). MS (ESI): 511.2 [M+H] ⁺.

Examples 55 to 60 of the following table were prepared in analogy to Example 53, using the appropriate carboxylic acids.

| **Ex.** | **Structure** | **Systematic Name** | **Building Blocks** | **MS, ESI: m/z** |
|---|---|---|---|---|
| 55 | 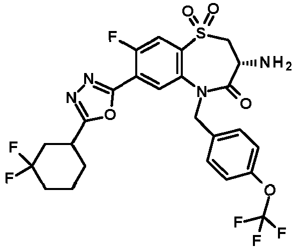 | (3R)-3-Amino-7-[5-(3,3-difluorocyclohexyl)-1,3,4-oxadiazol-2-yl]-8-fluoro-1,1-dioxo-5-[[4-(trifluoromethoxy)phenyl]met hyl]-2,3-dihydro-1λ⁶,5-benzothiazepin-4-one | 3,3-Difluorocy clohexane-1-carboxylic acid | 605.4 [M+H]⁺ |
| | | | (CAS 849669-20-1) | |
| | | (***) | | |
| 56 | 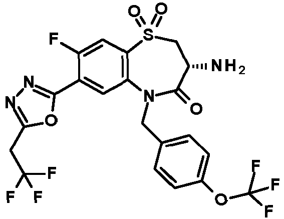 | (3R)-3-Amino-8-fluoro-1,1-dioxo-7-[5-(2,2,2-trifluoroethyl)-1,3,4-oxadiazol-2-yl]-5-[[4-(trifluoromethoxy)phenyl]met hyl]-2,3-dihydro-1λ⁶,5-benzothiazepin-4-one | 3,3,3-Trifluoropr opionic acid | 569.2 [M+H]⁺ |
| | | | (CAS 2516-99-6) | |
| | | (*) | | |
| 57 | 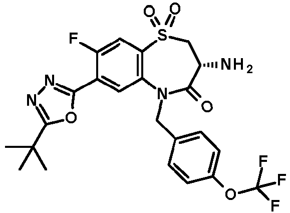 | (3R)-3-Amino-7-(5-tert-butyl-1,3,4-oxadiazol-2-yl)-8-fluoro-1,1-dioxo-5-[[4-(trifluoromethoxy)phenyl]met hyl]-2,3-dihydro-1λ⁶,5-benzothiazepin-4-one | 2,2-Dimethylpr opanoic acid | 543.2 [M+H]⁺ |
| | | | (CAS 75-98-9) | |
| | | (*) | | |
| 58 | 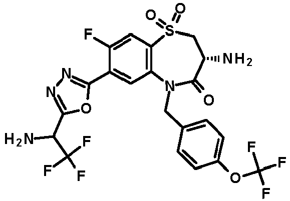 | (3R)-3-amino-7-[5-(1-amino-2,2,2-trifluoro-ethyl)-1,3,4-oxadiazol-2-yl]-8-fluoro-1,1-dioxo-5-[[4-(trifluoromethoxy)phenyl]met hyl]-2,3-dihydro-1λ⁶,5-benzothiazepin-4-one | 2-(tert-Butoxycar bonylamin o)-3,3,3-trifluoropropanoic acid (CAS 188030-43-5) | 584.1 [M+H]⁺ |
| 59 | 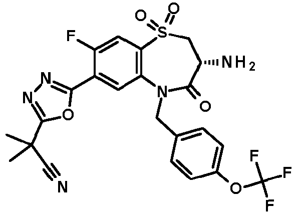 | (2-methyl-2-[5-[(3R)-3-amino-8-fluoro-1,1,4-trioxo-5-[[4-(trifluoromethoxy)phenyl]met hyl]-2,3-dihydro-1λ⁶,5-benzothiazepin-7-yl]-1,3,4-oxadiazol-2-yl]propanenitrile | 2-Cyano-2-methylpro panoic acid | 554.5 [M+H]⁺ |
| | | | (CAS 22426-30-8) | |
| | | (*) | | |
| 60 | 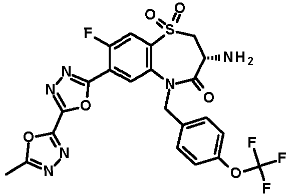 | (3R)-3-amino-8-fluoro-7-[5-(5-methyl-1,3,4-oxadiazol-2-yl)-1,3,4-oxadiazol-2-yl]-1,1-dioxo-5-[[4-(trifluoromethoxy)phenyl]met hyl]-2,3-dihydro-1λ⁶,5-benzothiazepin-4-one | 5-Methyl-1,3,4-oxadiazole -2-carbonyl chloride (CAS 889131-28-6) | 569.3 [M+H]⁺ |
| | | (*) | | |

| | | | | |
|---|---|---|---|---|
| * as a hydrochloride salt ** as a 1,1,1,3,3,3-hexafluoropropan-2-ol adduct *** as a hydrochloride salt and 1,1,1,3,3,3-hexafluoropropan-2-ol adduct | | | | |

### Example 61

### (2S)-N-[(3R)-8-fluoro-1,1,4-trioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-3-yl]-2-(methylamino)propanamide

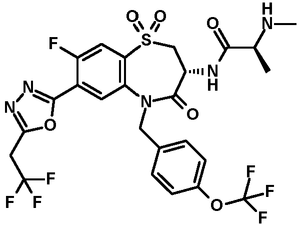

### Step a) tert-butyl N-[(1S)-2-[[(3R)-8-fluoro-1,1,4-trioxo-7-[5-(2,2,2-trifluoroethyl)-1,3,4-oxadiazol-2-yl]-5-[[4-(trifluoromethoxy)phenyl]methyl]-2, 3-dihydro-1λ⁶,5-benzothiazepin-3-yl]amino]-1-methyl-2-oxo-ethyl]-N-methyl-carbamate

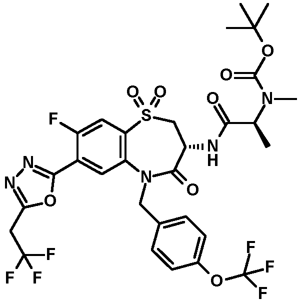

To a solution of (R)-3-amino-8-fluoro-7-(5-(2,2,2-trifluoroethyl)-1,3,4-oxadiazol-2-yl)-5-(4-(trifluoromethoxy)benzyl)-2,3-dihydrobenzo[b][1,4]thiazepin-4(5H)-one, 1,1-dioxide hydrochloride (Example 56) (20 mg, 33.1 µmol, Eq: 1) in DMF (200 µl) were added N-(tert-butoxycarbonyl)-N-methyl-L-alanine (10.1 mg, 49.6 µmol, Eq: 1.5), HATU (37.7 mg, 99.2 µmol, Eq: 3) and DIPEA (17.1 mg, 23.1 µl, 132 µmol, Eq: 4) and the yellow solution was stirred at RT for 30 min. The solvent was evaporated and the remaining residue was purified by chromatography on silica gel (0-100% EtOAc in heptane) affording the title compound (16.5 mg, 21.9 µmol, 66.2 % yield) as a white solid. MS (ESI): 752.3 [M-H]⁻.

### Step b) 2S)-N-[(3R)-8-fluoro-1,1, 4-trioxo-7-[5-(2, 2, 2-trifluoroethyl)-1, 3, 4-oxadiazol-2-yl]-5-[[4-(trifluoromethoxy)phenyl]methyl]-2, 3-dihydro-1λ⁶,5-benzothiazepin-3-yl]-2-(methylamino)propanamide

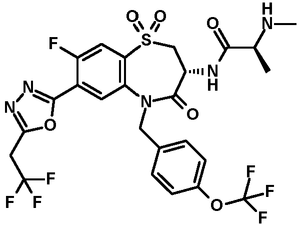

The title compound was prepared in analogy to method **6c** from tert-butyl N-[(1S)-2-[[(3R)-8-fluoro-1,1,4-trioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-3-yl]amino]-1-methyl-2-oxo-ethyl]-N-methyl-carbamate (16 mg, 21.2 µmol) with additional 4 drops of HCl (4M in dioxane) and was obtained as an off-white solid, as hydrochloride salt and 1,1,1,3,3,3-hexafluoropropan-2-ol adduct (17 mg, 19.8 µmol, 93% yield). MS (ESI): 654.5 [M+H] ⁺.

### Example 62

### (3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one (Epimers 1:1)

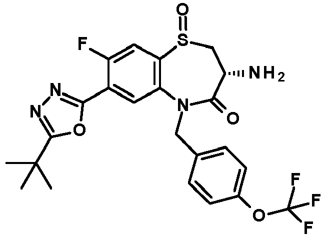

### Step a) tert-butyl N-[(3R)-7-(5-tert-butyl-1, 3, 4-oxadiazol-2-yl)-8-fluoro-1,4-dioxo-5-[[4-(trifluoromethoxy)phenyl]methyl]-2,3-dihydro-1λ⁴,5-benzothiazepin-3-yl]carbamate (Epimer A) and tert-butyl N-[(3R)-7-(5-tert-butyl-1,3,4-oxadiazol-2-yl)-8-fluoro-1, 4-dioxo-5-[[4-(trifluoromethoxy)phenyl]methyl]-2,3-dihydro-1λ⁴,5-benzothiazepin-3-yl]carbamate (Epimer B)

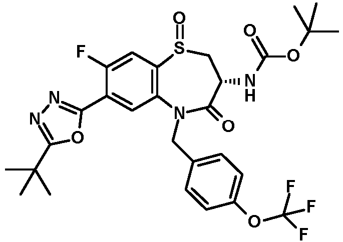

To a solution of tert-butyl N-[(3R)-7-(5-tert-butyl-1,3,4-oxadiazol-2-yl)-8-fluoro-4-oxo-5-[[4-(trifluoromethoxy)phenyl]methyl]-2,3-dihydro-1,5-benzothiazepin-3-yl]carbamate (100 mg, 0.16 mmol) in DCM (5 mL) was added m-CPBA (16.6 mg, 0.08 mmol, 0.5 eq). The mixture was stirred at 15 °C for 16 h, quenched by sat. aqueous Na₂SO₃ (5 mL), extracted DCM (10 mL), washed with H₂O (5 mL), brine (5 mL), dried over Na₂SO₄, filtered, concentrated and purified by column chromatography on silica gel (EtOAc:PE = 9:1 to 1:4) to afford the title compound, epimer A (32 mg, 0.05 mmol, 24% yield) and epimer B (35 mg, 0.06 mmol, 27% yield) as a light yellow gum. MS (ESI): 571.1 [M-isobutene+H] ⁺.

### Step b) (3R)-3-amino-7-(5-tert-butyl-1, 3, 4-oxadiazol-2-yl)-8-fluoro-1-oxo-5-[[4-(trifluoromethoxy)phenyl]methyl]-2,3-dihydro-1λ⁴,5-benzothiazepin-4-one (Epimers 1:1)

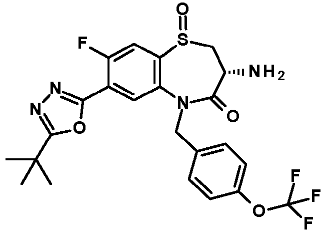

The title compound was prepared in analogy to method **6b** from tert-butyl N-[(3R)-7-(5-tert-butyl-1,3,4-oxadiazol-2-yl)-8-fluoro-1,4-dioxo-5-[[4-(trifluoromethoxy)phenyl]methyl]-2,3-dihydro-1λ⁴,5-benzothiazepin-3-yl]carbamate (100 mg, 0.16 mmol) and was obtained after prep-HPLC as a white solid (35 mg, 0.070 mmol, 41% yield). MS (ESI): 527.1 [M+H] ⁺.

### Example 63

### (3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one

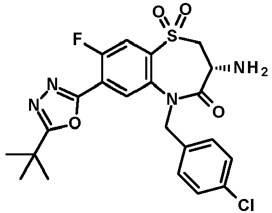

### Step a) tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate

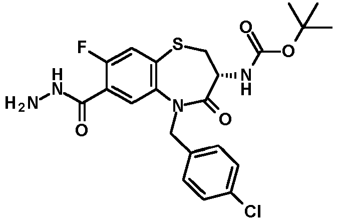

The title compound was prepared in analogy to the method used in Example 53, step c) from (R)-3-((tert-butoxycarbonyl)amino)-5-(4-chlorobenzyl)-8-fluoro-4-oxo-2,3,4,5-tetrahydrobenzo[b][1,4]thiazepine-7-carboxylic acid (CAS:2002449-40-1) (284 mg, 467 µmol, Eq) and was obtained as a light yellow solid (306 mg, 402 µmol, 86% yield). MS (ESI): 439.1 [M-isobutene+H]⁺.

### Step b) tert-butyl N-[(3R)-7-(5-tert-butyl-1,3, 4-oxadiazol-2-yl)-5-[(4-chlorophenyl)methyl]-8-fluoro-4-oxo-2,3-dihydro-1,5-benzothiazepin-3-yl]carbamate

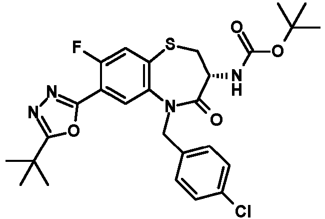

The title compound was prepared in analogy to the method used in Example 53, step d) from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (250 mg, 505 µmol) and was obtained as a light yellow solid (158 mg, 268 µmol, 53% yield). MS (ESI): 505.1 [M-isobutene+H]⁺.

### Step c) tert-butyl N-[(3R)-7-(5-tert-butyl-1,3,4-oxadiazol-2-yl)-5-[(4-chlorophenyl)methyl]-8-fluoro-1,1, 4-trioxo-2, 3-dihydro-1λ⁶,5-benzothiazepin-3-yl]carbamate

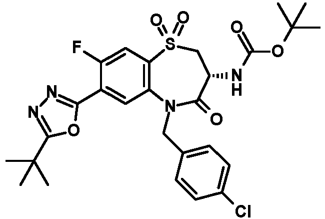

The title compound was prepared according to general method **5** from tert-butyl N-[(3R)-7-(5-tert-butyl-1,3,4-oxadiazol-2-yl)-5-[(4-chlorophenyl)methyl]-8-fluoro-4-oxo-2,3-dihydro-1,5-benzothiazepin-3-yl]carbamate (176 mg, 314 µmol) and was obtained as white solid (80 mg, 135 µmol, 43% yield). MS (ESI): 537.1 [M-isobuteneH]⁺.

### Step d) (3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one

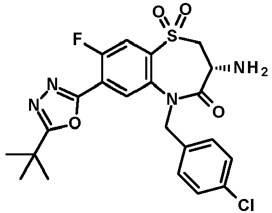

The title compound was prepared in analogy to the method used in **6a** from tert-butyl N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]carbamate (133 mg, 224 µmol) and was obtained as a light yellow solid (88 mg, 170 µmol, 76% yield). MS (ESI): 493.97 [M+H]⁺.

### Starting material for example 64

### 2,2-dimethyl-3-(2-oxopyrrolidin-1-yl)propanoic acid

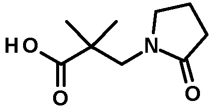

### Step a) benzyl 2,2-dimethyl-3-oxo-propanoate

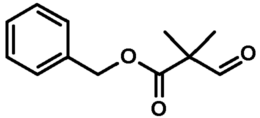

To a solution of oxalyl dichloride (0.25 mL, 2.9 mmol, 1.2 eq) in DCM (12 mL) was added DMSO (0.27 mL, 3.87 mmol, 1.6 eq) dropwise with stirring at -78 °C over 5 mins under N₂. After stirred for 10 mins at -78 °C, a solution of benzyl 3-hydroxy-2,2-dimethyl-propanoate (500 mg, 2.4 mmol, 1 eq) in DCM (9 mL) was added dropwise to the mixture over 15 mins keeping the temperature below -65 °C. After stirred for 40 mins at -78 °C, NEt₃ (0.95 mL, 6.79 mmol, 2.83 eq) was added dropwise into the mixture for 10 min below -65 °C. The reaction mixture was stirred at -78 °C for 30 min and then allowed to warm to 25 °C and stirred for 2 hrs. The reaction mixture was diluted with EtOAc (30 mL) and washed with brine (50 mLx 3), dried over anhydrous Na₂SO₄ and concentrated, purified by column chromatography on silica gel (PE:EtOAc = 1:0 to 3:2) to obtain the title compound (300 mg, 1.45 mmol, 52% yield) as a colorless oil. MS (ESI): 229.1 [M+Na]⁺.

### Step b) benzyl 2,2-dimethyl-3-(2-oxopyrrolidin-1-yl)propanoate

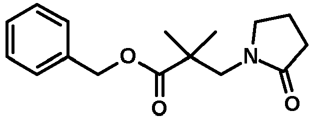

To a solution of benzyl 2,2-dimethyl-3-oxo-propanoate (400 mg, 1.94 mmol) in DCE (16 mL) was added 4-aminobutyric acid (0.31 mL, 3.1 mmol, 1.6 eq) and stirred at 25 °C for 30 min. To the reaction mixture was added sodium triacetoxyborohydride (739 mg, 3.49 mmol, 1.8 eq) and stirred at 25 °C for 16 hrs. The mixture was quenched by water (15 mL) and extracted with EtOAc (15 x 2) , the combined organic phase was washed with brine (30 mL x 2) concentrated under vacuum and purified by column chromatography on silica gel (PE:EA=15:1 to 2:1) to provide the title compound (280 mg, 1.0 mmol, 48% yield) as a colorless oil. MS (ESI): 276.2 [M+H]⁺.

### Step c) 2,2-dimethyl-3-(2-oxopyrrolidin-1-yl)propanoic acid

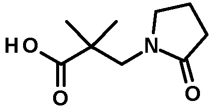

To a solution of benzyl 2,2-dimethyl-3-(2-oxopyrrolidin-1-yl)propanoate (180 mg, 0.65 mmol,) in methanol (6 mL) was added Pd/C (69.5 mg, 0.07 mmol, 0.1 eq) under N₂ atmosphere. After the mixture was degassed with H₂ balloon for 3 times, it was stirred at 25 °C for 16 hr. The reaction mixture was filtered and concentrated to obtain the title compound (90 mg, 0.49 mmol, 74% yield) as a grey solid which was used crude in the next step. MS (ESI): 186.0 [M+H]⁺.

### Starting material for example 65

### 1-(benzyloxymethyl)cyclopropanecarboxylic acid

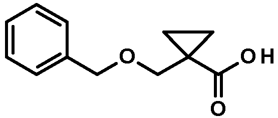

### Step a) ethyl 1-(benzyloxymethyl)cyclopropanecarboxylate

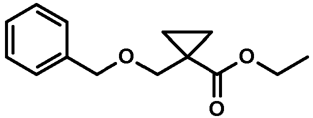

To a solution of 1-hydroxymethyl-cyclopropanecarboxylic acid ethyl ester (1500 mg, 10.4 mmol) in DMF (10 mL) was added NaH (499 mg, 12.5 mmol, 1.2 eq) at 0-10°C under N₂. After stirring at 25°C for 0.5 h benzyl bromide (1.48 mL, 12.5 mmol, 1.2 eq) in DMF (5 mL) was added at 0-10°C under N₂. The reaction was stirred at RT for 12 h, quenched with sat. aqueous NH₄Cl solution (20 mL) and extracted with EtOAc (50 mL x 3). The combined extracts were washed with brine (50 mL), dried over Na₂SO₄, filtered, concentrated under vacuum and purified by column chromatography on silica gel (PE:EA = 100:1) to give the title compound (1600 mg, 6.83 mmol, 65% yield) as colourless oil. MS (ESI): 235.1 [M+H]⁺.

### Step b) 1-(benzyloxymethyl)cyclopropanecarboxylic acid

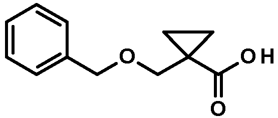

To a solution of ethyl 1-(benzyloxymethyl)cyclopropanecarboxylate (700 mg, 2.99 mmol) in THF (7 mL), MeOH (3.5 mL) and water (7 mL) was added LiOH·H₂O (376 mg, 8.96 mmol, 3 eq) at 25°C. The reaction was stirred at 25°C for 2 h, concentrated under vacuum and extracted with EtOAc (5 mLx 2). The aqueous phase was carefully acidified with 2 N aqueous HCl to pH = 3-4 and extracted with EtOAc (20 mL x 3). The organic layers were washed with brine, dried over Na₂SO₄ and concentrated under vacuum to afford the title compound (1000 mg, 4.85 mmol, 162% yield) as light yellow oil. MS (ESI): 229.1 [M+Na]⁺.

### Starting material for example 66

### 2,2-dimethyl-3-(2-oxo-1-piperidyl)propanoic acid

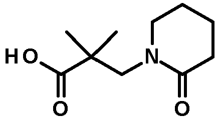

The title compound was prepared in analogy to starting material for starting material for Example 64 step b and c) from benzyl 2,2-dimethyl-3-oxo-propanoate (400 mg, 1.94 mmol) and 5-aminovaleric acid (0.35 mL, 3.1 mmol, 1.6 eq) and was obtained as a grey solid. MS (ESI): 200.0, [M-isobutene+H]⁺.

Examples 64 to 122 and Intermediate 120 of the following table were prepared in analogy to Example 63, using the appropriate carboxylic acid building block.

| **Ex.** | **Structure** | **Systematic Name** | **Building Block, General Methods** | **MS, ESI: m/z** |
|---|---|---|---|---|
| 64 | 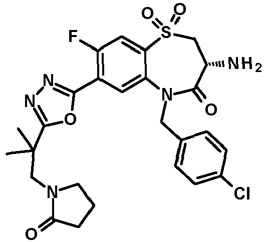 | (3R)-3-amino-5-[(4-chlorophenyl)methyl]-7-[5-[1,1-dimethyl-2-(2-oxopyrrolidin-1-yl)ethyl]-1,3,4-oxadiazol-2-yl]-8-fluoro-1,1-dioxo-2,3-dihydro-1λ⁶,5-benzothiazepin-4-one | 2,2-Dimethyl-3-(2-oxopyrroli din-1-yl)propano ic acid **7a, 8a, 5, 6b** | 576.3 [M+H]⁺ |
| | | (*****) | | |
| 65 | 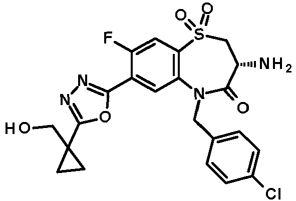 | (3R)-3-amino-5-[(4-chlorophenyl)methyl]-8-fluoro-7-[5-[1-(hydroxymethyl)cyclopropyl]-1,3,4-oxadiazol-2-yl]-1,1-dioxo-2,3-dihydro-1λ⁶,5-benzothiazepin-4-one | 1-(Benzyloxy methyl)cyc lopropane carboxylic acid, **7a, 8a, debenzylat ion, 5, 6e** | 507.2 [M+H]⁺ |
| | | (*****) | | |
| 66 | 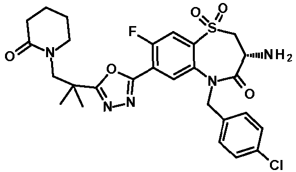 | (3R)-3-amino-5-[(4-chlorophenyl)methyl]-7-[5-[1,1-dimethyl-2-(2-oxo-1-piperidyl)ethyl]-1,3,4-oxadiazol-2-yl]-8-fluoro-1,1-dioxo-2,3-dihydro-1λ⁶,5-benzothiazepin-4-one | 2,2-Dimethyl-3-(2-oxo-1-piperidyl)p ropanoic acid **7a, 8b, 5, 6b** | 590.4 [M+H]⁺ |
| | | (*) | | |
| 67 | 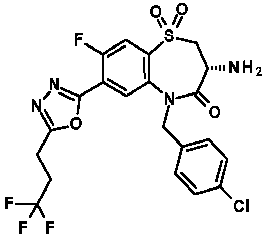 | (3R)-3-Amino-5-[(4-chlorophenyl)methyl]-8-fluoro-1,1-dioxo-7-[5-(3,3,3-trifluoropropyl)-1,3,4-oxadiazol-2-yl]-2,3-dihydro-1λ⁶,5-benzothiazepin-4-one | 4,4,4 Trifluorobu tyric acid | 533.2 [M+H]⁺ |
| | | | (CAS 406-93-9) | |
| | | (*) | **7a, 8a, 5, 6b** | |
| 68 | 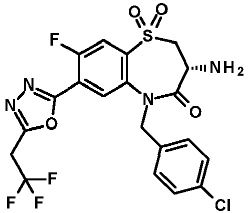 | (3R)-3-Amino-5-[(4-chlorophenyl)methyl]-8-fluoro-1,1-dioxo-7-[5-(2,2,2-trifluoroethyl)-1,3,4- | 3,3,3-Trifluoropr opionic acid | 519.2 [M+H]⁺ |
| | | oxadiazol-2-yl]-2,3-dihydro-1λ⁶,5-benzothiazepin-4-one | (CAS 2516-99-6) | |
| | | (*) | **7a, 8a, 5, 6b** | |
| 69 | 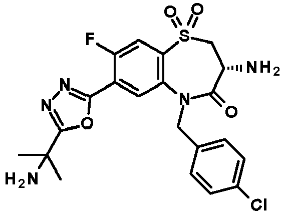 | (3R)-3-Amino-7-[5-(1-amino-1-methyl-ethyl)-1,3,4-oxadiazol-2-yl]-5-[(4-chlorophenyl)methyl]-8-fluoro-1,1-dioxo-2,3-dihydro-1λ⁶,5-benzothiazepin-4-one | Boc-2-Aminoisob utyric acid | 494.1 [M+H]⁺ |
| | | | (CAS 30992-29-1) | |
| | | | **7a, 8a, 5, 6b** | |
| 70 | 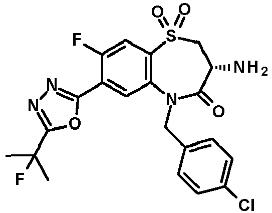 | (3R)-3-Amino-5-[(4-chlorophenyl)methyl]-8-fluoro-7-[5-(1-fluoro-1-methyl-ethyl)-1,3,4-oxadiazol-2-yl]-1,1-dioxo-2,3-dihydro-1λ⁶,5-benzothiazepin-4-one | 2-Fluoro-2-methylpro panoic acid | 497.1 [M+H]⁺ |
| | | | (CAS 63812-15-7) | |
| | | (****) | **7a, 8a, 5, 6b** | |
| 71 | 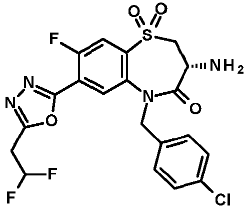 | (3R)-3-Amino-5-[(4-chlorophenyl)methyl]-7-[5-(2,2-difluoroethyl)-1,3,4-oxadiazol-2-yl]-8-fluoro-1,1-dioxo-2,3-dihydro-1λ⁶,5-benzothiazepin-4-one | 3,3-Difluoropr opanoic acid | 500.9 [M+H]⁺ |
| | | | (CAS 155142-69-1) | |
| | | | **7a, 8a, 5, 6b** | |
| 72 | 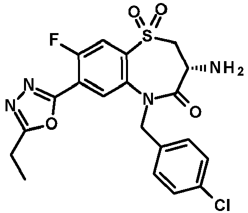 | (3R)-3-Amino-5-[(4-chlorophenyl)methyl]-7-(5-ethyl-1,3,4-oxadiazol-2-yl)-8-fluoro-1,1-dioxo-2,3-dihydro-1λ⁶,5-benzothiazepin-4-one | Propanoic acid | 565.2 [M+H]⁺ |
| | | | (CAS 79-09-4) | |
| | | | **7a, 8a, 5, 6b** | |
| 73 | 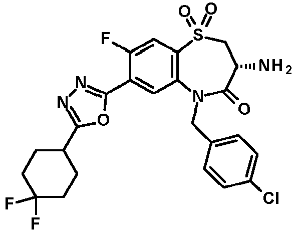 | (3R)-3-Amino-5-[(4-chlorophenyl)methyl]-7-[5-(4,4-difluorocyclohexyl)-1,3,4-oxadiazol-2-yl]-8-fluoro-1,1-dioxo-2,3-dihydro-1λ⁶,5-benzothiazepin-4-one (*) | 4,4-Difluorocy clohexane-1-carboxylic acid | 555.1 [M+H]⁺ |
| | | | (CAS 122665-97-8) | |
| | | | **7a, 8a, 5, 6b** | |
| 74 | 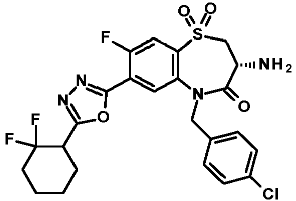 | (3R)-3-Amino-5-[(4-chlorophenyl)methyl]-7-[5-(2,2-difluorocyclohexyl)-1,3,4-oxadiazol-2-yl]-8-fluoro-1,1-dioxo-2,3-dihydro-1λ⁶,5-benzothiazepin-4-one | 2,2-Difluorocy clohexane-1-carboxylic acid | 555.1 [M+H]⁺ |
| | | | (CAS 1461714-25-9) | |
| | | | **7a, 8a, 5, 6b** | |
| 75 | 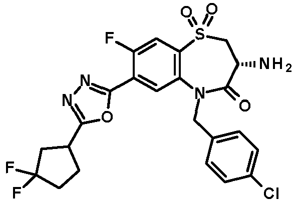 | (3R)-3-Amino-5-[(4-chlorophenyl)methyl]-7-[5-(3,3-difluorocyclopentyl)-1,3,4-oxadiazol-2-yl]-8-fluoro-1,1-dioxo-2,3-dihydro-1λ⁶ 5-benzothiazepin-4-one (*) | 3,3-Difluorocy clopentane carboxylic acid | 541.1 [M+H]⁺ |
| | | | (CAS 1260897-05-9) | |
| | | | **7a, 8a, 5, 6b** | |
| 76 | 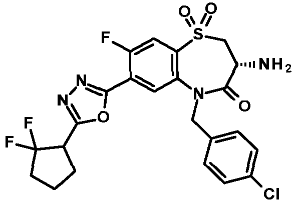 | (3R)-3-Amino-5-[(4-chlorophenyl)methyl]-7-[5-(2,2-difluorocyclopentyl)-1,3,4-oxadiazol-2-yl]-8-fluoro-1,1-dioxo-2,3-dihydro-1λ⁶,5-benzothiazepin-4-one | 2,2-Difluorocy clopentane -1-carboxylic acid | 541.1 [M+H]⁺ |
| | | | (CAS 1378525-26-8) | |
| | | | **7a, 8a, 5, 6b** | |
| 77 | 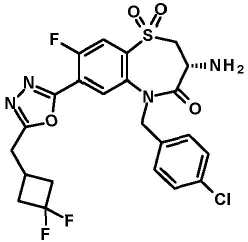 | (3R)-3-Amino-5-[(4-chlorophenyl)methyl]-7-[5-[(3,3-difluorocyclobutyl)methyl]-1,3,4-oxadiazol-2-yl]-8-fluoro-1,1-dioxo-2,3-dihydro-1λ⁶,5-benzothiazepin-4-one | 2-(3,3-Difluorocy clobutyl)ac etic acid | 541.1 [M+H]⁺ |
| | | | (CAS 1373503-48-0) | |
| | | (*) | **7a, 8a, 5, 6b** | |
| 78 | 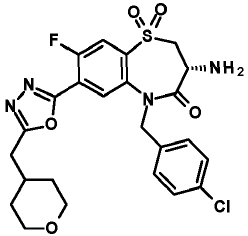 | (3R)-3-Amino-5-[(4-chlorophenyl)methyl]-8-fluoro-1,1-dioxo-7-[5-(tetrahydropyran-4-ylmethyl)-1,3,4-oxadiazol-2-yl]-2,3-dihydro-1λ⁶,5-benzothiazepin-4-one | Tetrahydro pyranyl-4-acetic acid | 535.1 [M+H]⁺ |
| | | | (CAS 85064-61-5) | |
| | | | **7a, 8a, 5, 6b** | |
| 79 | 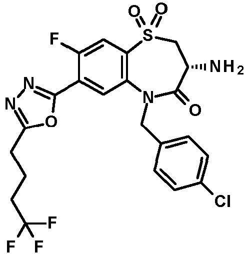 | (3R)-3-Amino-5-[(4-chlorophenyl)methyl]-8-fluoro-1,1-dioxo-7-[5-(4,4,4-trifluorobutyl)-1,3,4-oxadiazol-2-yl]-2,3-dihydro-1λ⁶,5-benzothiazepin-4-one | 5,5,5-Trifluorope ntanoic acid | 547.2 [M+H]⁺ |
| | | | (CAS 407-62-5) | |
| | | (*) | **7a, 8a, 5, 6b** | |
| 80 | 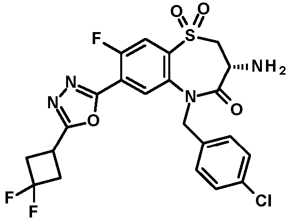 | (3R)-3-Amino-5-[(4-chlorophenyl)methyl]-7-[5-(3,3-difluorocyclobutyl)-1,3,4-oxadiazol-2-yl]-8-fluoro-1,1-dioxo-2,3-dihydro-1λ⁶,5-benzothiazepin-4-one | 3,3-Difluorocy clobutanec arboxylic acid | 527.1 [M+H]⁺ |
| | | | (CAS 107496-54-8) | |
| | | (*) | **7a, 8a, 5, 6b** | |
| 81 | 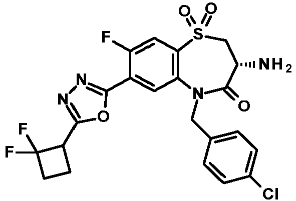 | (3R)-3-Amino-5-[(4-chlorophenyl)methyl]-7-[5-(2,2-difluorocyclobutyl)-1,3,4-oxadiazol-2-yl]-8-fluoro-1,1-dioxo-2,3-dihydro-1λ⁶,5-benzothiazepin-4-one | 2,2-Difluorocy clobutane-1-carboxylic acid | 527.1 [M+H]⁺ |
| | | | (CAS 1150518-74-3) | |
| | | | **7a, 8a, 5, 6b** | |
| 82 | 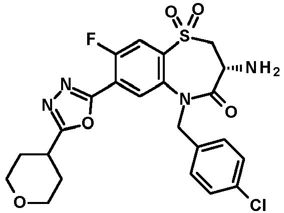 | (3R)-3-Amino-5-[(4-chlorophenyl)methyl]-8-fluoro-1,1-dioxo-7-(5-tetrahydropyran-4-yl-1,3,4-oxadiazol-2-yl)-2,3-dihydro-1λ⁶,5-benzothiazepin-4-one | Tetrahydro -2H-pyran-4-carboxylic acid | 521.1 [M+H]⁺ |
| | | | (5337-03-1) | |
| | | | **7a, 8a, 5, 6b** | |
| 83 | 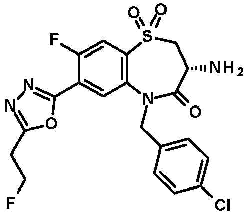 | (3R)-3-Amino-5-[(4-chlorophenyl)methyl]-8-fluoro-7-[5-(2-fluoroethyl)-1,3,4-oxadiazol-2-yl]-1,1-dioxo-2,3-dihydro-1λ⁶,5-benzothiazepin-4-one | 3-Fluoroprop anoic acid | 483.1 [M+H]⁺ |
| | | | (CAS 461-56-3) | |
| | | | **7a, 8a, 5, 6b** | |
| 84 | 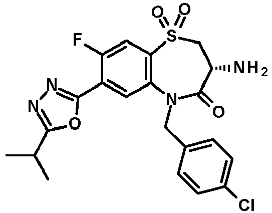 | (3R)-3-Amino-5-[(4-chlorophenyl)methyl]-8-fluoro-7-(5-isopropyl-1,3,4-oxadiazol-2-yl)-1,1-dioxo-2,3-dihydro-1λ⁶,5-benzothiazepin-4-one | Isobutyric acid | 479.1 [M+H]⁺ |
| | | | (CAS 79-31-2) | |
| | | | **7a, 8a, 5, 6b** | |
| | | (**) | | |
| 85 | 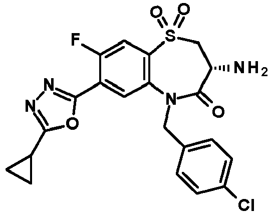 | (3R)-3-Amino-5-[(4-chlorophenyl)methyl]-7-(5-cyclopropyl-1,3,4-oxadiazol-2-yl)-8-fluoro-1,1-dioxo-2,3-dihydro-1λ⁶,5-benzothiazepin-4-one | Cyclopropa necarboxyl ic acid | 477.1 [M+H]⁺ |
| | | | (CAS 1759-53-1) | |
| | | | **7a, 8a, 5, 6b** | |
| 86 | y | (3R)-3-amino-5-[(4-chlorophenyl)methyl]-8-fluoro-7-[5-(2-hydroxy-1,1-dimethyl-ethyl)-1,3,4-oxadiazol-2-yl]-1,1-dioxo-2,3-dihydro-1λ⁶,5-benzothiazepin-4-one | 3-[tert-Butyl(dime thyl)silyl]o xypropanoi c acid | 509.0 [M+H]⁺ |
| | | | (CAS 528602-18-8) | |
| | | (**) | **7a, 8a, 5, 6b** | |
| 87 | 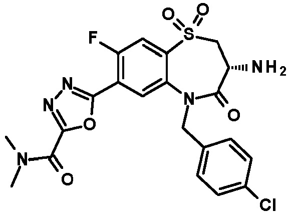 | N,N-dimethyl-5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazole-2-carboxamide | N,N-dimethylox amic acid | 509.1 [M+H]⁺ |
| | | | **7a, 8a, 5, 6b** | |
| | | (*****) | | |
| 88 | 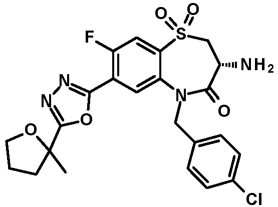 | (3R)-3-amino-5-[(4-chlorophenyl)methyl]-8-fluoro-7-[5-(2-methyltetrahydrofuran-2-yl)-1,3,4-oxadiazol-2-yl]-1,1-dioxo-2,3-dihydro-1λ⁶,5-benzothiazepin-4-on | 2-Methyltetr ahydrofura n-2-carboxylic acid **7a, 8a, 5, 6d** | 521.2 [M+H]⁺ |
| | | (*) | | |
| 89 | 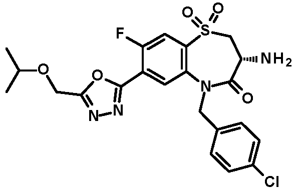 | (3R)-3-amino-5-[(4-chlorophenyl)methyl]-8-fluoro-7-[5-(isopropoxymethyl)-1,3,4-oxadiazol-2-yl]-1,1-dioxo-2,3-dihydro-1λ⁶,5-benzothiazepin-4-one | 2-Isopropoxy acetic acid **7a, 8a, 5, 6a** | 509.2 [M+H]⁺ |
| | | (*****) | | |
| 90 | 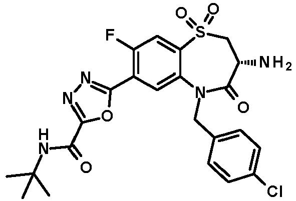 | N-tert-butyl-5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazole-2-carboxamide | 2-(tert-Butylamin o)-2-oxo-acetic acid **7a, 8a, 5, 6b** | 536.4 [M+H]⁺ |
| | | (*****) | | |
| 91 | 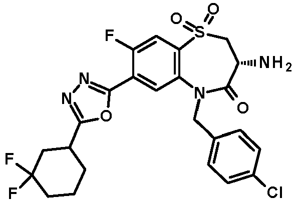 | (3R)-3-amino-5-[(4-chlorophenyl)methyl]-7-[5-(3,3-difluorocyclohexyl)-1,3,4-oxadiazol-2-yl]-8-fluoro-1,1-dioxo-2,3-dihydro-1λ⁶,5-benzothiazepin-4-one | 3,3-Difluorocy clohexane-1-carboxylic acid **7a, 8a, 5, 6c** | 555.1 [M+H]⁺ |
| | | (*) | | |
| 92 | 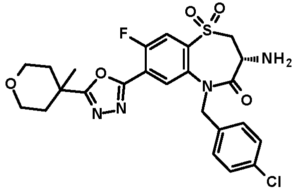 | (3R)-3-amino-5-[(4-chlorophenyl)methyl]-8-fluoro-7-[5-(4-methyltetrahydropyran-4-yl)-1,3,4-oxadiazol-2-yl]-1,1-dioxo-2,3-dihydro-1λ⁶,5-benzothiazepin-4-one | 4-Methyl-tetrahydro -pyran-4-carboxylic acid | 535.2 [M+H]⁺ |
| | | (******) | **7a, 8a, 5, 6b** | |
| 93 | 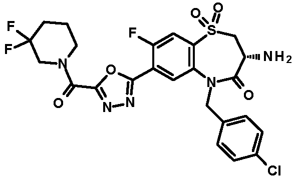 | (3R)-3-amino-5-[(4-chlorophenyl)methyl]-7-[5-(3,3-difluoropiperidine-1-carbonyl)-1,3,4-oxadiazol-2-yl]-8-fluoro-1,1-dioxo-2,3-dihydro-1λ⁶,5-benzothiazepin-4-one | 2-(3,3-Difluoro-1-piperidyl)-2-oxo-acetic acid **7a, 8a, 5, 6e** | 584.0 [M+H]⁺ |
| | | (*****) | | |
| 94 | 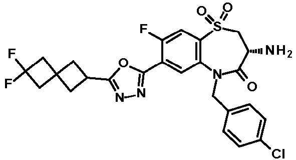 | (3R)-3-amino-5-[(4-chlorophenyl)methyl]-7-[5-(2,2-difluorospiro[3.3]heptan-6-yl)-1,3,4-oxadiazol-2-yl]-8-fluoro-1,1-dioxo-2,3-dihydro-1λ⁶,5-benzothiazepin-4-one | 2,2-Difluorospi ro[3.3]hep tane-6-carboxylic acid **7a, 8a, 5, 6a** | 567.2 [M+H]⁺ |
| | | (***) | | |
| 95 | 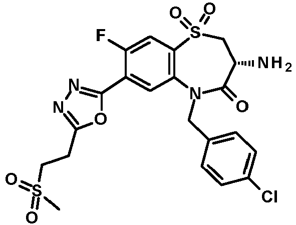 | (3R)-3-amino-5-[(4-chlorophenyl)methyl]-8-fluoro-7-[5-(2-methylsulfonylethyl)-1,3,4-oxadiazol-2-yl]-1,1-dioxo-2,3-dihydro-1λ⁶,5-benzothiazepin-4-one | 3-(Methylthi o)propanoi c acid **7a, 8a, 5, 6a** | 543.2 [M+H]⁺ |
| | | (*) | | |
| 96 | 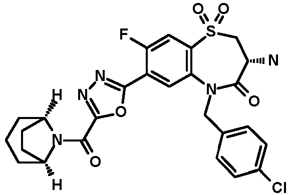 | (3R)-3-amino-5-[(4-chlorophenyl)methyl]-8-fluoro-1,1-dioxo-7-[5-[(1R,5S)-8-azabicyclo[3.2.1]octane-8-carbonyl]-1,3,4-oxadiazol-2-yl]-2,3-dihydro-1λ⁶,5-benzothiazepin-4-one | 2-(8-Azabicyclo[ 3.2.1]octa n-8-yl)-2-oxo-acetic acid **7a, 8a, 5, 6b** | 574.1 [M+H]⁺ |
| | | (*****) | | |
| 97 | 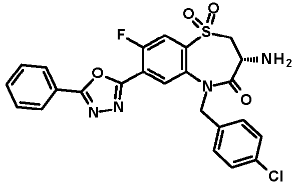 | (3R)-3-amino-5-[(4-chlorophenyl)methyl]-8-fluoro-1,1-dioxo-7-(5-phenyl-1,3,4-oxadiazol-2-yl)-2,3-dihydro-1λ⁶,5-benzothiazepin-4-one | Benzoic acid **7a, 8a, 5, 6b** | 513.0 [M+H]⁺ |
| | | (*****) | | |
| 98 | 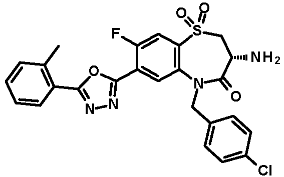 | (3R)-3-amino-5-[(4-chlorophenyl)methyl]-8-fluoro-7-[5-(o-tolyl)-1,3,4-oxadiazol-2-yl]-1,1-dioxo-2,3-dihydro-1λ⁶,5-benzothiazepin-4-one | o-Toluic acid **7a, 8a, 5, 6b** | 527.0 [M+H]⁺ |
| | | (*****) | | |
| 99 | 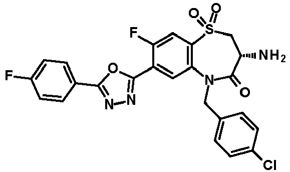 | (3R)-3-amino-5-[(4-chlorophenyl)methyl]-8-fluoro-7-[5-(4-fluorophenyl)-1,3,4-oxadiazol-2-yl]-1,1-dioxo-2,3-dihydro-1λ⁶,5-benzothiazepin-4-one | 4-Fluorobenz oic acid **7a, 8a, 5, 6b** | 530.8 [M+H]⁺ |
| | | (*****) | | |
| 100 | 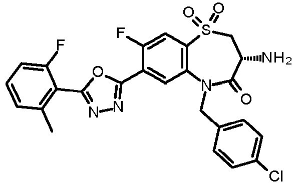 | (3R)-3-amino-5-[(4-chlorophenyl)methyl]-8-fluoro-7-[5-(2-fluoro-6-methyl-phenyl)-1,3,4-oxadiazol-2-yl]-1,1-dioxo-2,3-dihydro-1λ⁶,5-benzothiazepin-4-one | 2-Fluoro-6-methylbenzoic acid **7a, 8a, 5, 6d** | 545.3 [M+H]⁺ |
| | | (*) | | |
| 101 | 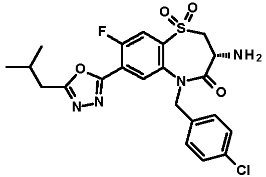 | (3R)-3-amino-5-[(4-chlorophenyl)methyl]-8-fluoro-7-(5-isobutyl-1,3,4-oxadiazol-2-yl)-1,1-dioxo-2,3-dihydro-1λ⁶,5-benzothiazepin-4-one | 3-Methylbut yric acid **7a, 8a, 5, 6d** | 493.2 [M+H]⁺ |
| | | (*) | | |
| 102 | 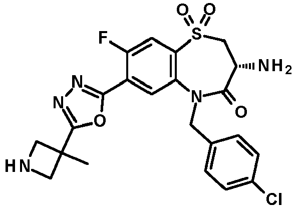 | (3R)-3-amino-5-[(4-chlorophenyl)methyl]-8-fluoro-7-[5-(3-methylazetidin-3-yl)-1,3,4-oxadiazol-2-yl]-1,1-dioxo-2,3-dihydro-1λ⁶,5-benzothiazepin-4-one | 1-tert-Butoxycar bonyl-3-methyl- azetidine-3-carboxylic acid **7a, 8a, 5, 6d** | 506.2 [M+H]⁺ |
| | | (*) | | |
| 103 | 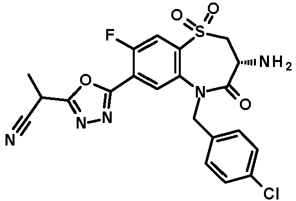 | 2-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]propanenitrile | 2-Cyanoprop ionic acid **7a, 8a, 5, 6a** | 490.2 [M+H]⁺ |
| | | (*) | | |
| 104 | 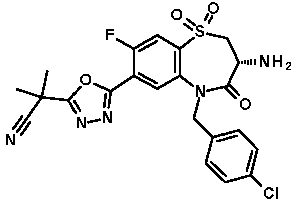 | 2-methyl-2-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]propanenitrile | 2-Cyano-2-methyl-propionic acid **7a, 8a, 5, 6a** | 504.2 [M+H]⁺ |
| | | (*) | | |
| 105 | 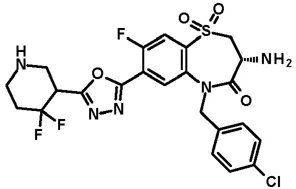 | (3R)-3-amino-5-[(4-chlorophenyl)methyl]-7-[5-(4,4-difluoro-3-piperidyl)-1,3,4-oxadiazol-2-yl]-8-fluoro-1,1-dioxo-2,3-dihydro-1λ⁶,5-benzothiazepin-4-one | 1-tert-Butoxycar bonyl-4,4-difluoropiperidine-3-carboxylic acid **7c, 8a, 5, 6b** | 556.1 [M+H]⁺ |
| | | (*****) | | |
| 106 | 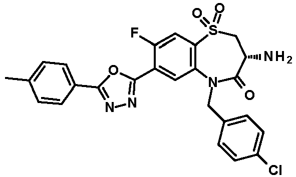 | (3R)-3-amino-5-[(4-chlorophenyl)methyl]-8-fluoro-1,1-dioxo-7-[5-(p-tolyl)-1,3,4-oxadiazol-2-yl]-2,3-dihydro-1λ⁶,5-benzothiazepin-4-one | p-Toluic acid **7c, 8a, 5, 6b** | 527.2 [M+H]⁺ |
| | | (******) | | |
| 107 | 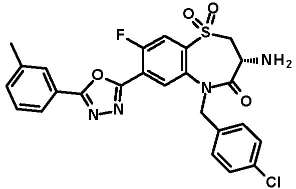 | (3R)-3-amino-5-[(4-chlorophenyl)methyl]-8-fluoro-7-[5-(m-tolyl)-1,3,4-oxadiazol-2-yl]-1,1-dioxo-2,3-dihydro-1λ⁶,5-benzothiazepin-4-one | m-Toluic acid, **7c 8a, 5, 6b** | 527.1 [M+H]⁺ |
| | | (*****) | | |
| 108 | 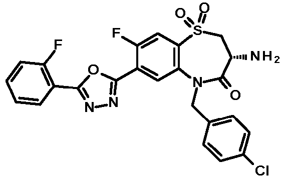 | (3R)-3-amino-5-[(4-chlorophenyl)methyl]-8-fluoro-7-[5-(2-fluorophenyl)-1,3,4-oxadiazol-2-yl]-1,1-dioxo-2,3-dihydro-1λ⁶,5-benzothiazepin-4-one | 2-Fluorobenz oic acid **7c 8a, 5, 6b** | 530.8 [M+H]⁺ |
| | | (*****) | | |
| 109 | 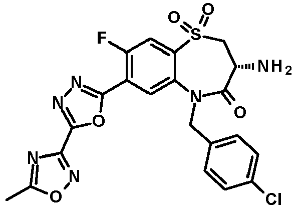 | (3R)-3-amino-5-[(4-chlorophenyl)methyl]-8-fluoro-7-[5-(5-methyl-1,2,4-oxadiazol-3-yl)-1,3,4-oxadiazol-2-yl]-1,1-dioxo-2,3-dihydro-1λ⁶,5-benzothiazepin-4-one | 5-Methyl-1,2,4-oxadiazole -3-carbonyl chloride (DCE, pyridine, 0 C), **8a, 5, 6d** | 519.2 [M+H]⁺ |
| | | (*) | | |
| 110 | 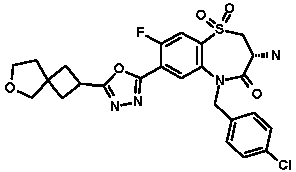 | (3R)-3-amino-5-[(4-chlorophenyl)methyl]-8-fluoro-7-[5-(6-oxaspiro[3.4]octan-2-yl)-1,3,4-oxadiazol-2-yl]-1,1-dioxo-2,3-dihydro-1λ⁶,5-benzothiazepin-4-one | 6-Oxaspiro[3 .4]octane-2-carboxylic acid, **7a, 8a, 5, 6d** | 547.2 [M+H]⁺ |
| | | (*****) | | |
| 111 | 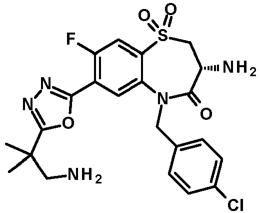 | (3R)-3-amino-7-[5-(2-amino-1,1-dimethyl-ethyl)-1,3,4-oxadiazol-2-yl]-5-[(4-chlorophenyl)methyl]-8-fluoro-1,1-dioxo-2,3-dihydro-1λ⁶,5-benzothiazepin-4-one | {[(tert-Butoxy)car bonyl]ami no}-2,2-dimethylpr opanoic acid, **7a, 8a, 5, 6b** | 508.2 [M+H]⁺ |
| | | (*****) | | |
| 112 | 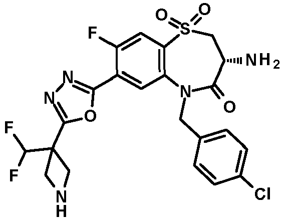 | (3R)-3-amino-5-[(4-chlorophenyl)methyl]-7-[5-[3-(difluoromethyl)azetidin-3-yl]-1,3,4-oxadiazol-2-yl]-8-fluoro-1,1-dioxo-2,3-dihydro-1λ⁶,5-benzothiazepin-4-one | 1-tert-Butoxycar bonyl-3-(difluorom ethyl)azeti dine-3-carboxylic acid, **7a, 8a, 5, 7d** | 586.2 [M+CO OH]⁻ |
| | | (***) | | |
| 113 | 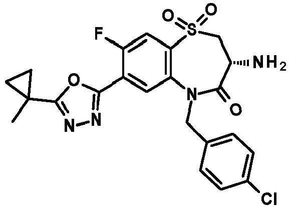 | (3R)-3-amino-5-[(4-chlorophenyl)methyl]-8-fluoro-7-[5-(1-methylcyclopropyl)-1,3,4-oxadiazol-2-yl]-1,1-dioxo-2,3-dihydro-1λ⁶,5-benzothiazepin-4-one | 1-Methylcycl opropanec arboxylic acid, **7a, 8a, 5, 6d** | 491.2 [M+H]⁺ |
| | | (***) | | |
| 114 | 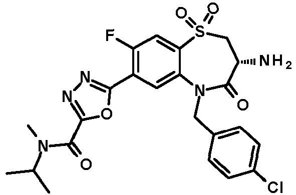 | N-isopropyl-N-methyl-5- [(3 R)-3-amino-5-[(4-chlorophenyl)methyl]-8-fluoro-1,1,4-trioxo-2,3-dihydro-1λ⁶,5-benzothiazepin-7-yl]-1,3,4-oxadiazole-2-carboxamide | 2-[Isopropyl( methyl)am ino]-2-oxo-acetic acid **7a, 8a, 5, 6b** | 536.4 [M+H]⁺ |
| | | (*****) | | |
| 115 | 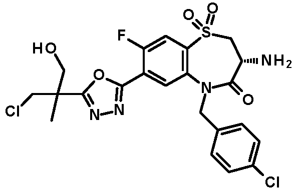 | (3R)-3-amino-7-[5-[1-(chloromethyl)-2-hydroxy-1-methyl-ethyl]-1,3,4-oxadiazol-2-yl]-5-[(4-chlorophenyl)methyl]-8-fluoro-1,1-dioxo-2,3-dihydro-1λ⁶,5-benzothiazepin-4-one | 3-Methyloxe tane-3-carboxylic acid, **7a, 8a, 5, 7d** | 543.2 [M+H]⁺ |
| | | (*) | | |
| 116 | 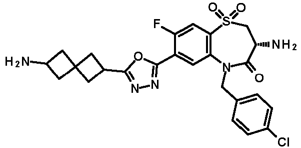 | (3R)-3-amino-7-[5-(2-aminospiro[3.3]heptan-6-yl)-1,3,4-oxadiazol-2-yl]-5-[(4-chlorophenyl)methyl]-8-fluoro-1,1-dioxo-2,3-dihydro-1λ⁶,5-benzothiazepin-4-one | 2-(tert-Butoxycar bonylamin o)spiro[3.3 ]heptane-6-carboxylic acid **7a, 8a, 5, 6d** | 590.4 [M+HC OO]⁻ |
| | | (*) | | |
| 117 | 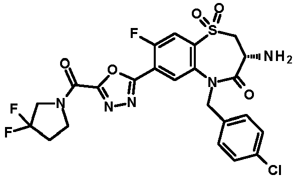 | (3R)-3-amino-5-[(4-chlorophenyl)methyl]-7-[5-(3,3-difluoropyrrolidine-1-carbonyl)-1,3,4-oxadiazol-2-yl]-8-fluoro-1,1-dioxo-2,3-dihydro-1λ⁶,5-benzothiazepin-4-one | 2-(3,3-Difluoropy rrolidin-1-yl)-2-oxo-acetic acid **7a, 8a, 5, 6b** | 570.4 [M+H]⁺ |
| | | (*****) | | |
| 118 | 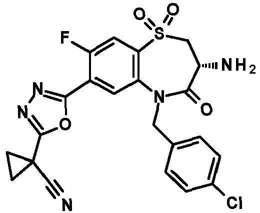 | 1-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]cyclopropanecarbonitrile | 1-Cyanocyclo propaneca rboxylic acid, **7a, 8a, 5, 6a** | 502.2 [M+H]⁺ |
| | | (*) | | |
| 119 | 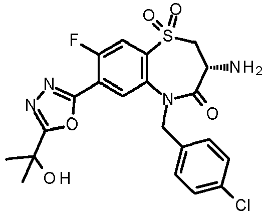 | (3R)-3-amino-5-[(4-chlorophenyl)methyl]-8-fluoro-7-[5-(1-hydroxy-1-methyl-ethyl)-1,3,4-oxadiazol-2-yl]-1,1-dioxo-2,3-dihydro-1λ⁶,5-benzothiazepin-4-one | 2-Hydroxyiso butyric acid **7a, TBDMS protection , 8a, 5, 6e** | 495.0 [M+H]⁺ |
| | | (******) | | |
| Inter medi ate 120 | 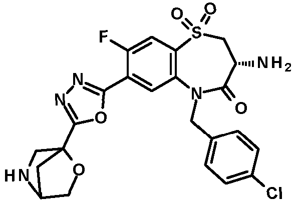 | (3R)-3-amino-5-[(4-chlorophenyl)methyl]-8-fluoro-7-[5-(2-oxa-5-azabicyclo[2.2.1]heptan-1-yl)-1,3,4-oxadiazol-2-yl]-1,1-dioxo-2,3-dihydro-1λ⁶,5-benzothiazepin-4-one | 4-tert-Butoxycar bonyl-2-ethyl-morpholin e-2-carboxylic acid, **7a, 8a, 5, 6d** | 534.3 [M+H]⁺ |
| | | (*) | | |
| 121 | 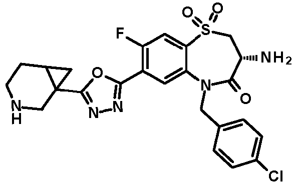 | (3R)-3-amino-7-[5-(3-azabicyclo[4.1.0]heptan-1-yl)-1,3,4-oxadiazol-2-yl]-5-[(4-chlorophenyl)methyl]-8-fluoro-1,1-dioxo-2,3-dihydro-1λ⁶,5-benzothiazepin-4-one | 3-tert-Butoxycar bonyl-3-azabicyclo[ 4.1.0]hept ane-1-carboxylic acid **7a, 8a, 5, 6e** | 532.1 [M+H]⁺ |
| | | (*****) | | |
| 122 | 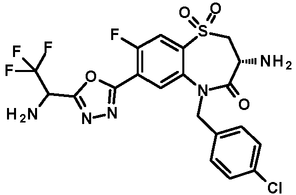 | (3R)-3-amino-7-[5-(1-amino-2,2,2-trifluoro-ethyl)-1,3,4-oxadiazol-2-yl]-5-[(4-chlorophenyl)methyl]-8-fluoro-1,1-dioxo-2,3-dihydro-1λ⁶,5-benzothiazepin-4-one (Epimers 1:1) | 2-(tert-Butoxycar bonylamin o)-3,3,3-trifluoro-propionic acid | 534.1 [M+H]⁺ |
| | | (*) | **7a, 8a, 5, 6D** | |

| | | | | |
|---|---|---|---|---|
| * as a hydrochloride salt ** as a 1,1,1,3,3,3-hexafluoropropan-2-ol adduct *** as a hydrochloride salt and 1,1,1,3,3,3-hexafluoropropan-2-ol adduct **** as a formic acid salt ***** as free base after prep HPLC ****** TFA salt after prep HPLC | | | | |

### Debenzylation example 65

### tert-butyl N-[(3R)-5-[(4-chlorophenyl)methyl]-8-fluoro-7-[5-[1-(hydroxymethyl)cyclopropyl]-1,3,4-oxadiazol-2-yl]-1,1,4-trioxo-2,3-dihydro-1λ⁶,5-benzothiazepin-3-yl]carbamate

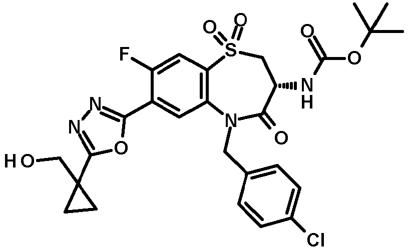

To a solution of tert-butyl N-[(3R)-7-[5-[1-(benzyloxymethyl)cyclopropyl]-1,3,4-oxadiazol-2-yl]-5-[(4-chlorophenyl)methyl]-8-fluoro-1,1,4-trioxo-2,3-dihydro-1λ6,5-benzothiazepin-3-yl]carbamate (450mg, 0.65 mmol) in MeOH (5 mL) and DCM (5 mL) was added Pd/C (300 mg) under N₂ at 25 °C. The reaction was stirred at 25 °C for 1 h under H₂, filtered, concentrated under vacuum and purified by prep-HPLC to provide the title compound (160 mg, 0.26 mmol, 40% yield) as white solid. MS (ESI): 607.2 [M+H]⁺.

### TBDMS protection example 119

### tert-butyl N-[(3R)-7-[[[2-[tert-butyl(dimethyl)silylloxy-2-methylpropanoyl]amino]carbamoyl]-5-[(4-chlorophenyl)methyl]-8-fluoro-4-oxo-2,3-dihydro-1,5-benzothiazepin-3-yl]carbamate

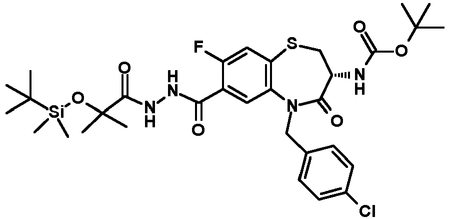

To a solution of tert-butyl N-[(3R)-5-[(4-chlorophenyl)methyl]-8-fluoro-7-[[(2-hydroxy-2-methyl-propanoyl)amino]carbamoyl]-4-oxo-2,3-dihydro-1,5-benzothiazepin-3-yl]carbamate (290 mg, 0.5 mmol) in DMF (12 mL) was added 4-dimethylaminopyridine (487 mg, 4.0 mmol, 8 eq) and tert-butyldimethylchlorosilane (188 mg, 1.25 mmol, 2.5 eq) at 25 °C and stirred at 25 °C for 16 h. The mixture was diluted with EtOAc (20 mL), washed with brine (20 mL x 3). The organic layer was dried with Na₂SO₄, filtered, concentrated under reduced pressure and purified by column chromatography on silica gel (PE:EtOAc 9:1 to 3:1) to afford the title compound (320 mg, 0.46 mmol, 91% yield) as a light yellow solid. MS (ESI): 639.4 [M-isobutene+H]⁺.

### Example 120

### methyl 1-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]-2-oxa-5-azabicyclo[2.2.1]heptane-5-carboxylate

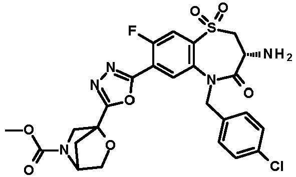

(3R)-3-amino-5-[(4-chlorophenyl)methyl]-8-fluoro-7-[5-(2-oxa-5-azabicyclo[2.2.1]heptan-1-yl)-1,3,4-oxadiazol-2-yl]-1,1-dioxo-2,3-dihydro-1λ⁶,5-benzothiazepin-4-one 1:2 hydrochloride salt (Intermediate 120, 12 mg, 0.019 mmol) was stirred with methyl chloroformate (1.81 mg, 1.49 uL, 0.019 mmol, 1 eq) and DIPEA (7.44 mg, 10.05 uL, 0.058 mmol, 3 eq) in DCM at room temperature for 1.5h. The solvent was evaporated and submitted to prep-HPLC affording the title compound (4 mg, 32%) as white solid. MS (ESI): 592.4 [M+H]⁺.

### Example 124

### N,2-dimethyl-2-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]propanamide

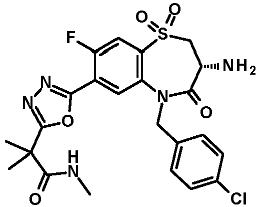

### Step a) ethyl 2-methyl-2-[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]propanoate

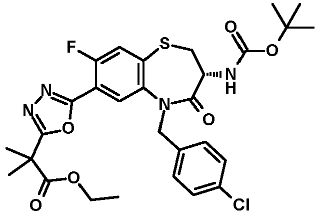

The title compound was prepared according to general procedure **7a** and **8a** from N-[(3R)-7-carbazoyl-5-(4-chlorobenzyl)-8-fluoro-4-keto-2,3-dihydro-1,5-benzothiazepin-3-yl]carbamic acid tert-butyl ester (400 mg, 0.727 mmol) and 3-ethoxy-3-keto-2,2-dimethyl-propionic acid (128 mg, 114 uL, 0.80 mmol, 1.1 eq) in one pot and was obtained as a yellow oil (320 mg, 71% yield). MS (ESI): 619.3 [M-isobutene+H]⁺.

### Step b) 2-methyl-2-[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]propanoic acid

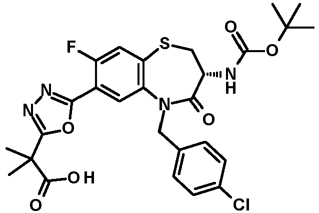

ethyl 2-methyl-2-[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]propanoate (320 mg, 0.517 mmol) was dissolved in THF (2.6 mL) and 1M aqueous NaOH (620 uL, 0.62 mmol, 1.2 eq) was added and the reaction was stirred at RT over night. 1 M aqueous HCl (620 uL, 0.620 mmol, 1.2 eq) was added until pH = 1. The reaction was extracted with EtOAc. The organic layers were combined, dried over magnesium sulfate, filtered and evaporated affording the title compound (140 mg, 38%) as white foam. MS (ESI): 591.4 (M+H).

### Step c) tert-butyl N-[(3R)-5-[(4-chlorophenyl)methyl]- 7-[5-[1,1-dimethyl-2-(methylamino)-2-oxo-ethyl]-1,3,4-oxadiazol-2-yl]-8-fluoro-4-oxo-2,3-dihydro-1,5-benzothiazepin-3-yl]carbamate

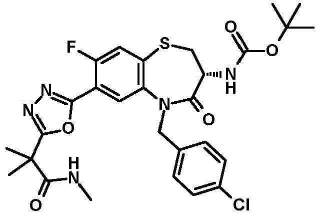

The title compound was prepared according to general procedure **7a** from 2-methyl-2-[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]propanoic acid (70 mg, 0.101 mmol) and methylamine (15 mg, 20 uL, 0.2 mmol, 2 eq) and was obtained after chromatography on silica gel (heptane:EtOAc = 7:3 to 0:1) as a colorless solid (30 mg, 42% yield). MS (ESI): 604.2 (M+H), [M+H]⁺.

### Step d) tert-butyl N-[(3R)-5-[(4-chlorophenyl)methyl]-7-[5-[1,1-dimethyl-2-(methylamino)-2-oxo-ethyl]-1,3,4-oxadiazol-2-yl]-8-fluoro-1,1,4-trioxo-2,3-dihydro-1λ⁶,5-benzothiazepin-3-yl]carbamate

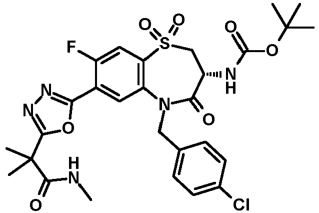

The title compound was prepared according to general procedure **5** from tert-butyl N-[(3R)-5-[(4-chlorophenyl)methyl]-7-[5-[1,1-dimethyl-2-(methylamino)-2-oxo-ethyl]-1,3,4-oxadiazol-2-yl]-8-fluoro-4-oxo-2,3-dihydro-1,5-benzothiazepin-3-yl]carbamate (30 mg, 0.05 mmol) and was obtained after column chromatography on silica gel (heptane:EtOAc = 1:0 to 1:1) as a colorless oil (12 mg, 32% yield). MS (ESI): 591.4 [M-isobutene+H]⁺.

### Step e) N, 2-dimethyl-2-[5-[(3R)-3-amino-5-[(4-chlorophenyl)methyl]-8-fluoro-1,1,4-trioxo-2, 3-dihydro-1λ⁶,5-benzothiazepin-7-yl]-1,3,4-oxadiazol-2yl]propanamide

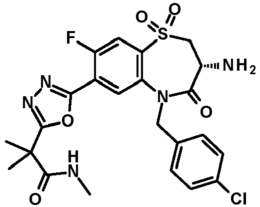

The title compound was prepared according to general procedure **6d** from tert-butyl N-[(3R)-5-[(4-chlorophenyl)methyl]-7-[5-[1,1-dimethyl-2-(methylamino)-2-oxo-ethyl]-1,3,4-oxadiazol-2-yl]-8-fluoro-1,1,4-trioxo-2,3-dihydro-1λ⁶,5-benzothiazepin-3-yl]carbamate (12 mg, 0.019 mmol) and was obtained as off-white powder (5 mg, 45% yield). MS (ESI): 536.3 [M+H]⁺.

Example 125 of the following table were prepared in analogy to Example 124, steps c to e), using the appropriate amine building block.

| **Ex.** | **Structure** | **Systematic Name** | **Building Block deprotection Method** | **MS, ESI: m/z** |
|---|---|---|---|---|
| 125 | 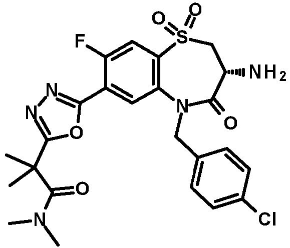 | N,N,2-trimethyl-2-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]propanamide | Dimethyla mine, **6d** | 550.2 [M+H]⁺ |
| | | (*) | | |

| | | | | |
|---|---|---|---|---|
| * as a hydrochloride salt and 1,1,1,3,3,3-hexafluoropropan-2-ol adduct | | | | |

### Example 126

### (3R)-3-amino-5-[(4-chlorophenyl)methyl]-8-fluoro-1,1-dioxo-7-[5-[1-(trifluoromethyl)cyclopropyl]-1,3,4-oxadiazol-2-yl]-2,3-dihydro-1λ⁶,5-benzothiazepin-4-one

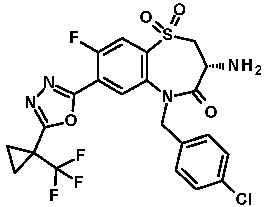

### Step a) methyl (3R)-3-(tert-butoxycarbonylamino)-5-[(4-chlorophenyl)methyl]-8-fluoro-1,1, 4-trioxo-2, 3-dihydro-1λ⁶,5-benzothiazepine-7-carboxylate

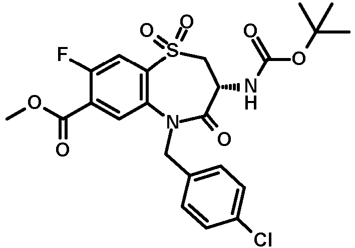

The title compound was prepared according to general method **5** from methyl (R)-3-((tert-butoxycarbonyl)amino)-5-(4-chlorobenzyl)-8-fluoro-4-oxo-2,3,4,5-tetrahydrobenzo[b][1,4]thiazepine-7-carboxylate (1.0 g, 2.0 mmol) and was obtained as a light yellow solid (1.14 g, 96% yield). MS (ESI): 471.3 [M-isobutene+H]⁺.

### Step b) (3R)-3-(tert-butoxycarbonylamino)-5-[(4-chlorophenyl)methyl]-8fluoro-1,1,4-trioxo-2,3-dihydro-1λ⁶,5-benzothiazepine-7-carboxylic acid

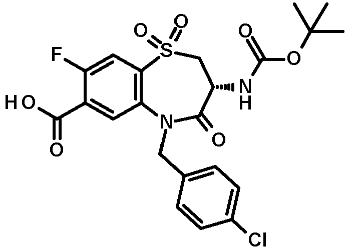

To a solution of methyl (3R)-3-(tert-butoxycarbonylamino)-5-[(4-chlorophenyl)methyl]-8-fluoro-1,1,4-trioxo-2,3-dihydro-1λ⁶,5-benzothiazepine-7-carboxylate (1 g, 1.9 mmol, 1) in THF (10 mL) was added lithium hydroxide hydrate (106 mg, 2.52 mmol, 1.33 eq) in water (8 mL) at 0 °C. The mixture was stirred at 0 °C for 0.5 h. To the reaction was added dropwise 0.5 M aqueous HCl (6 mL). The mixture was extracted with EtOAc (8 mL), DCM (5 mL). The organic layer was washed with brine (15 mL x 3), dried over anhydrous Na₂SO₄, filtered and concentrated under vacuum to give the crude title compound (1020 mg, 1.9 mmol, 103% yield) as a light yellow solid. MS (ESI): 457.0 [M-isobutene+H]⁺.

### Step c) tert-butyl N-[(3R)-5-[(4-chlorophenyl)methyl]-8-fluoro-7-(hydrazinecarbonyl)-1,1,4-trioxo-2,3-dihydro-1λ⁶,5-benzothiazepin-3-yl]carbamate

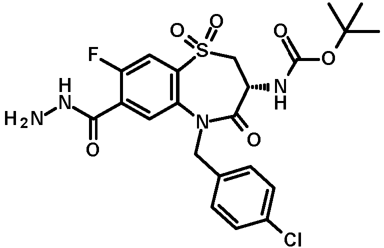

The title compound was prepared according to general method **7b** from (3R)-3-(tert-butoxycarbonylamino)-5-[(4-chlorophenyl)methyl]-8-fluoro-1,1,4-trioxo-2,3-dihydro-1λ⁶,5-benzothiazepine-7-carboxylic acid (819 mg, 1.6 mmol) and hydrazine hydrate (374 mg, 363 uL, 4.79 mmol, 3 eq) and was obtained as a light yellow solid (659 mg, 78% yield). MS (ESI): 471.2 [M-isobutene+H]⁺.

### Step d) tert-butyl N-[(3R)-5-[(4-chlorophenyl)methyl]-8-fluoro-1,1,4-trioxo-7-[[[1-(trifluoromethyl)cyclopropanecarbonyl]amino]carbamoyl]-2,3-dihydro-1λ⁶,5-benzothiazepin-3-yl]carbamate

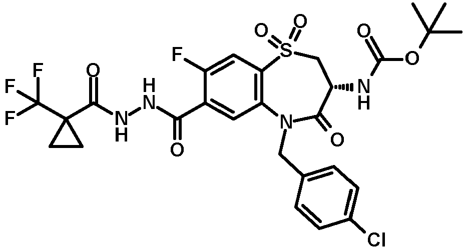

The title compound was prepared according to general procedure **7a** from tert-butyl N-[(3R)-5-[(4-chlorophenyl)methyl]-8-fluoro-7-(hydrazinecarbonyl)-1,1,4-trioxo-2,3-dihydro-1λ⁶,5-benzothiazepin-3-yl]carbamate (30 mg, 0.057 mmol) and 1-(trifluoromethyl)cyclopropanecarboxylic acid ((9.65 mg, 0.063 mmol, 1.1 eq) and was obtained as a yellow oil (48.8 mg, 108% yield). MS (ESI): 607.3 [M-isobutene+H]⁺.

### Step e) tert-butyl N-[(3R)-5-[(4-chlorophenyl)methyl]-8-fluoro-1,1,4-trioxo-7-[5-[1-(trifluoromethyl)cyclopropyl]-1,3,4-oxadiazol-2-yl]-2,3-dihydro-1λ⁶, 5-benzothiazepin-3-yl]carbamate

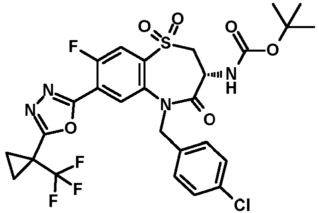

The title compound was prepared according to general procedure **8b** from tert-butyl N-[(3R)-5-[(4-chlorophenyl)methyl]-8-fluoro-1,1,4-trioxo-7-[[[1-(trifluoromethyl)cyclopropanecarbonyl]amino]carbamoyl]-2,3-dihydro-1λ⁶,5-benzothiazepin-3-yl]carbamate (48.8 mg, 0.074 mmol) and was obtained as an off-white solid (24.9 mg, 51% yield). MS (ESI): 589.3 [M-isobutene+H]⁺.

### Step f) (3R)-3-amino-5-[(4-chlorophenyl)methyl]-8-fluoro-1,1-dioxo-7-[5-[1-(trifluoromethyl)cyclopropyl]-1,3,4-oxadiazol-2-yl]-2,3-dihydro-1λ⁶,5-benzothiazepin-4-one

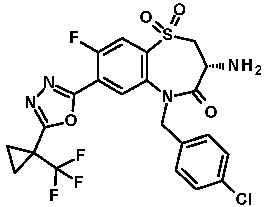

The title compound was prepared in analogy to the method used in **6d** from tert-butyl N-[(3R)-5-[(4-chlorophenyl)methyl]-8-fluoro-1,1,4-trioxo-7-[5-[1-(trifluoromethyl)cyclopropyl]-1,3,4-oxadiazol-2-yl]-2,3-dihydro-1λ⁶,5-benzothiazepin-3-yl]carbamate (24.9 mg, 0.038 mmol) and was obtained as a light yellow solid as a hydrochloride salt (18.6 mg, 83% yield). MS (ESI): 545.3 [M+H]⁺.

Examples 127 to 135 of the following table were prepared in analogy to Example 126 step d to f), using the appropriate carboxylic acid building block.

| **Ex.** | **Structure** | **Systematic Name** | **Building Block** | **MS, ESI: m/z** |
|---|---|---|---|---|
| | | | **General Procedures** | |
| 127 | 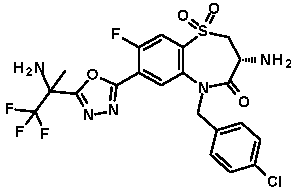 | (3R)-3-amino-7-[5-(1-amino-2,2,2-trifluoro-1-methylethyl)-1,3,4-oxadiazol-2-yl]-5-[(4-chlorophenyl)methyl]-8-fluoro-1,1-dioxo-2,3-dihydro-1λ⁶,5-benzothiazepin-4-one | 2-(tert-Butoxycar bonylamin o)-3,3,3-trifluoro-2-methyl-propionic acid, **7a, 8b, 6a** | 548.3 [M+H]⁺ |
| | | (*) | | |
| 128 | 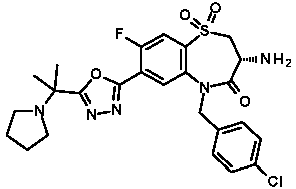 | (3R)-3-amino-5-[(4-chlorophenyl)methyl]-8-fluoro-7-[5-(1-methyl-1-pyrrolidin-1-yl-ethyl)-1,3,4-oxadiazol-2-yl]-1,1-dioxo-2,3-dihydro-1λ⁶,5-benzothiazepin-4-one | 2-Methyl-2-pyrrolidino -propionic acid, **7a, 8a, 6d** | 274.9 [M/2+ H]⁺ |
| | | (*) | | |
| 129 | 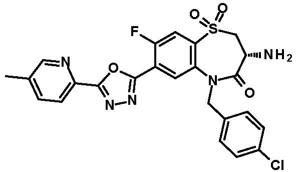 | (3R)-3-amino-5-[(4-chlorophenyl)methyl]-8-fluoro-7-[5-(5-methyl-2-pyridyl)-1,3,4-oxadiazol-2-yl]-1,1-dioxo-2,3-dihydro-1λ⁶,5-benzothiazepin-4-one | 5-Methylpic olinic acid, **7a, 8a, 6d** | 528.3 [M+H]⁺ |
| | | (*) | | |
| 130 | 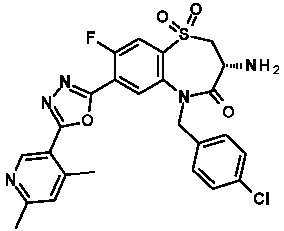 | (3R)-3-amino-5-[(4-chlorophenyl)methyl]-7-[5-(4,6-dimethyl-3-pyridyl)-1,3,4-oxadiazol-2-yl]-8-fluoro-1,1-dioxo-2,3-dihydro-1λ⁶,5-benzothiazepin-4-one | 4,6-Dimethylni cotinic acid, **7a, 8a, 6d** | 586.4 [M+HC OO]⁻ |
| | | (*) | | |
| 131 | 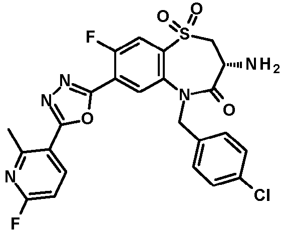 | (3R)-3-amino-5-[(4-chlorophenyl)methyl]-8-fluoro-7-[5-(6-fluoro-2-methyl-3-pyridyl)-1,3,4-oxadiazol-2-yl]-1,1-dioxo-2,3-dihydro-1λ⁶,5-benzothiazepin-4- one | 6-Fluoro-2-methyl-nicotinic acid, **7a, 8a, 6d** | 546.3 [M+H]⁺ |
| | | (*) | | |
| 132 | 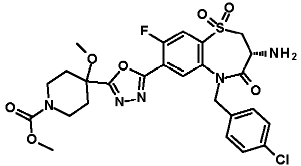 | methyl 4-methoxy-4-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]piperidine-1-carboxylate | 1-Carbometh oxy-4-methoxy-isonipecotic acid, **7a, 8a, 6b** | 608.4 [M+H]⁺ |
| 133 | 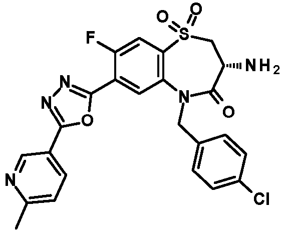 | (3R)-3-amino-5-[(4-chlorophenyl)methyl]-8-fluoro-7-[5-(6-methyl-3-pyridyl)-1,3,4-oxadiazol-2-yl]-1,1-dioxo-2,3-dihydro-1λ⁶,5-benzothiazepin-4-one | 6-Methylnic otinic acid, **7c, 8a, 6d** | 528.3 [M+H]⁺ |
| | | (*) | | |
| 134 | 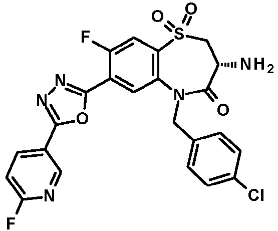 | (3R)-3-amino-5-[(4-chlorophenyl)methyl]-8-fluoro-7-[5-(6-fluoro-3-pyridyl)-1,3,4-oxadiazol-2-yl]-1,1-dioxo-2,3-dihydro-1λ⁶,5-benzothiazepin-4-one | 6-Fluoronico tinic acid, **7a, 8a, 6d** | 532.1 [M+H]⁺ |
| | | (*) | | |
| 135 | 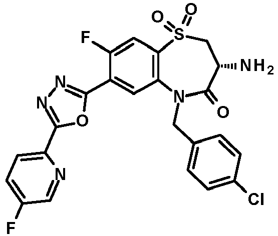 | (3R)-3-amino-5-[(4-chlorophenyl)methyl]-8-fluoro-7-[5-(5-fluoro-2-pyridyl)-1,3,4-oxadiazol-2-yl]-1,1-dioxo-2,3-dihydro-1λ⁶,5-benzothiazepin-4-one | Fluoropicol inic acid, **7a, 8a, 6d** | 532.2 [M+H]⁺ |
| | | (*) | | |

| | | | | |
|---|---|---|---|---|
| * as a hydrochloride salt | | | | |

### Example 136

### (3R)-3-amino-7-[5-(2-amino-3,3,3-trifluoro-1,1-dimethyl-propyl)-1,3,4-oxadiazol-2-yl]-5-[(4-chlorophenyl)methyl]-8-fluoro-1,1-dioxo-2,3-dihydro-1λ⁶,5-benzothiazepin-4-one

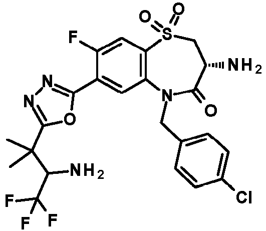

### Step a) methyl 3-(allyloxycarbonylamino)-4, 4, 4-trifluoro-2, 2-dimethyl-butanoate and methyl 4,4,4-trifluoro-3-(methoxycarbonylamino)-2,2-dimethyl-butanoate

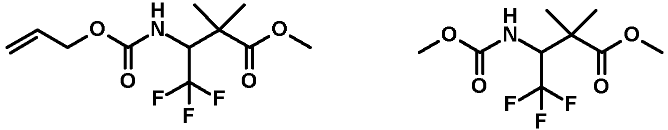

To 3-amino-4,4,4-trifluoro-2,2-dimethyl-butyric acid methyl ester hydrogen chloride (116 mg, 0.49 mmol) in DMF (2 mL) was added NEt₃ (149 mg, 205 uL, 1.48 mmol, 3 eq) and allyl chloroformate (71 mg, 63 uL, 0.59 mmol, 1.2 eq; it was a 1:1 mixture of allyl and methyl chloroformate) carefully, keeping the reaction mixture at 20-25°C and stirred vigorously for 1 h. Allyl chloroformate (71 mg, 63 uL, 0.59 mmol, 1.2 eq) and NEt₃ (149 mg, 205 uL, 1.48 mmol, 3 eq ) were carefully added and the reaction mixture was stirred at RT for 1 h. The reaction mixture was diluted with DCM and extracted with 1N aqueous HCl. The layers were separated, and the aqueous layer was extracted three times with DCM. The combined organic layers were washed with brine (20 ml) and dried over MgSO₄, filtered and concentrated, purified by column chromatography on silica gel (heptane:EtOAc =1:0 to 1:1) to yield the title compound (59.4 mg, 38% yield) as white semisolid. MS (ESI): 284.2 [M+H]⁺, MS (ESI): 244.1 [M+H]⁺.

### Step b) 3-(allyloxycarbonylamino)-4,4,4-trifluoro-2, 2-dimethyl-butanoic acid and 3-(methoxycarbonylamino)-4, 4, 4-trifluoro-2, 2-dimethyl-butanoic acid

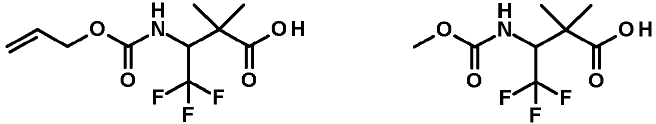

3-(allyloxy/methoxycarbonylamino)-4,4,4-trifluoro-2,2-dimethyl-butyric acid methyl ester (59.4 mg, 0.19 mmol) was dissolved in THF (0.5 mL), MeOH (0.5 mL) and water (0.3 mL). LiOH monohydrate (16 mg, 0.38 mmol, 2 eq) was added and the reaction mixture was stirred at RT for 2 hours. The reaction mixture was extracted with EtOAc (2 x 20ml). The aq. phase was acidified with 1N aqueous HCl and extracted with EtOAc (3 x 20 ml)). The combined organic phase was dried with MgSO₄, filtered and concentrated to the title compound (46.8 mg, 46% yield) as light brown solid. MS (ESI): 270.1 [M+H]⁺. MS (ESI): 258.2 [M+H]⁺.

### Step c) allyl N-[2, 2-dimethyl-3-oxo-3-[2-[(3R)-3-(tert-butoxycarbonylamino)-5-[(4-chlorophenyl)methyl]-8-fluoro-1,1,4-trioxo-2,3-dihydro-1λ⁶,5-benzothiazepine-7-carbonyl]hydrazino]-1-(trifluoromethyl)propyl]carbamate and methyl N-[2,2-dimethyl-3-oxo-3-[2-[(3R)-3-(tert-butoxycarbonylamino)-5-[(4-chlorophenyl)methyl]-8-fluoro-1,1, 4-trioxo-2, 3-dihydro-1λ⁶,5-benzothiazepine-7-carbonyl]hydrazino]-1-(trifluoromethyl)propyl]carbamate

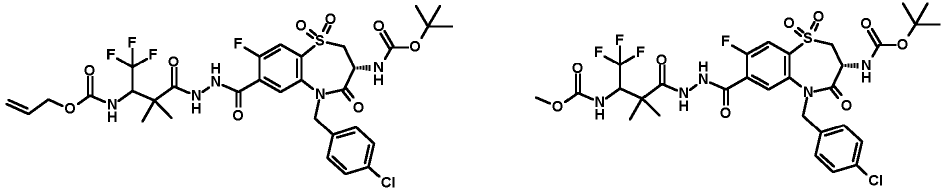

The title compound was prepared in analog of general procedure **7a** from 3-(allyl/methyloxycarbonylamino)-4,4,4-trifluoro-2,2-dimethyl-butyric acid (45 mg, 0.17 mmol, 1.1 eq) to yield the crude title compound (161 mg, 27% yield ) as yellow gum. MS (ESI): 776.4 [M+H]⁺. MS (ESI): 750.4 [M+H]⁺.

### Step d) allyl N-[2-methyl-2-[5-[(3R)-3-(tert-butoxycarbonylamino)-5-[(4-chlorophenyl)methyl]-8-fluoro-1,1,4-trioxo-2, 3-dihydro-1λ⁶,5-benzothiazepin-7-yl]-1,3,4-oxadiazol-2-yl]-1-(trifluoromethyl)propyl]carbamate and methyl N-[2-methyl-2-[5-[(3R)-3-(tert-butoxycarbonylamino)-5-[(4-chlorophenyl)methyl]-8-fluoro-1,1,4-trioxo-2, 3-dihydro-1λ⁶,5-benzothiazepin-7-yl]-1,3,4-oxadiazol-2-yl]-1-(trifluoromethyl)propyl]carbamate

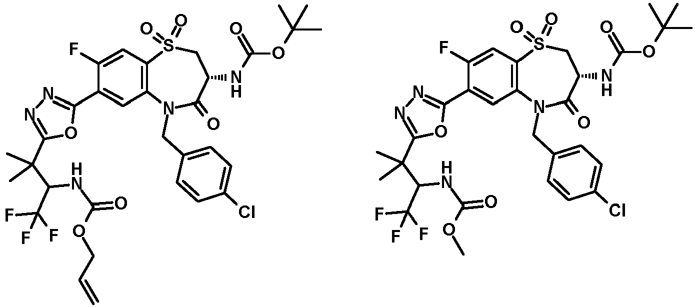

The title compound was prepared in analog of general procedure **8a** from allyl N-[2,2-dimethyl-3-oxo-3-[2-[(3R)-3-(tert-butoxycarbonylamino)-5-[(4-chlorophenyl)methyl]-8-fluoro-1,1,4-trioxo-2,3-dihydro-1λ⁶,5-benzothiazepine-7-carbonyl]hydrazino]-1-(trifluoromethyl)propyl]carbamate and methyl N-[2,2-dimethyl-3-oxo-3-[2-[(3R)-3-(tert-butoxycarbonylamino)-5-[(4-chlorophenyl)methyl]-8-fluoro-1,1,4-trioxo-2,3-dihydro-1λ⁶,5-benzothiazepine-7-carbonyl]hydrazino]-1-(trifluoromethyl)propyl]carbamate (161 mg, 0.042 mmol) to yield after column chromatography on silica gel (PE:EtOAc =1:0 to 1:1) the title compound (30 mg) as yellow solid and the second title compound (49.6 mg) as a light yellow solid. MS (ESI): 758.4 [M-H]⁻. MS (ESI): 732.4 [M-H]⁻.

### Step e) tert-butyl N-[(3R)-7-[5-(2-amino-3,3,3-trifluoro-1,1-dimethyl-propyl)-1,3, 4-oxadiazol-2-yl]-5-[(4-chlorophenyl)methyl]-8-fluoro-1,1,4-trioxo-2,3-dihydro-1λ⁶,5-benzothiazepin-3-yl]carbamate

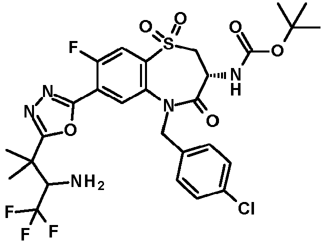

A solution of tetrakis(triphenylphosphine) palladium (2.17 mg, 0.002 mmol, 0.05 eq) and N-[(3R)-7-[5-[2-(allyl/methoxycarbonylamino)-3,3,3-trifluoro-1,1-dimethyl-propyl]-1,3,4-oxadiazol-2-yl]-5-(4-chlorobenzyl)-8-fluoro-1,1,4-triketo-2,3-dihydro-1λ6,5-benzothiazepin-3-yl]carbamic acid tert-butyl ester (30 mg, 0.037 mmol) in DCM (0.5 mL) was degassed 3 times with argon and phenylsilan (20.9 mg, 23.8 uL, 0.19 mmol, 5 eq) was added. The reaction was stirred at RT for 2 h, diluted with water and DCM. Aq. NaHCO₃ and 1M aqueous NaOH solution was added to the aqueous layer until pH 13. The aqueous layer was then extracted with DCM (2 x 15 ml). The combined organic layers were dried over Na₂SO₄, filtered and concentrated in vacuo to afford the title compound (11.2 mg, 44% yield) as a white solid. MS (ESI): 620.3 [M-isobutene+H]⁺.

### Step f) (3R)-3-amino-7-[5-(2-amino-3, 3, 3-trifluoro-1,1-dimethyl-propyl)-1,3,4-oxadiazol-2-yl]-5-[(4-chlorophenyl)methyl]-8-fluoro-1,1-dioxo-2,3-dihydro-1λ⁶,5-benzothiazepin-4-one

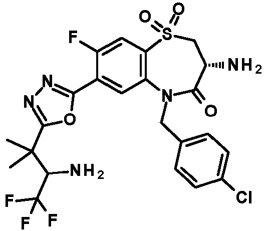

The title compound was prepared in analogy to the method used in **6d** from tert-butyl N-[(3R)-7-[5-(2-amino-3,3,3-trifluoro-1,1-dimethyl-propyl)-1,3,4-oxadiazol-2-yl]-5-[(4-chlorophenyl)methyl]-8-fluoro-1,1,4-trioxo-2,3-dihydro-1λ⁶,5-benzothiazepin-3-yl]carbamate (11.2 mg, 0.017 mmol) and was obtained as a light yellow solid as a hydrochloride salt (9.3 mg, 86% yield). MS (ESI): 620.3 [M+HCOO]⁻.

### Example 137

### methyl N-[2-methyl-2-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]-1-(trifluoromethyl)propyl]carbamate

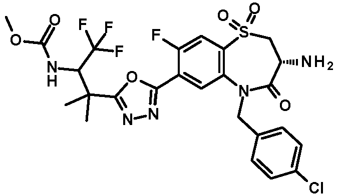

The title compound was prepared in analogy to the general method **6d** from N-[2-[5-[(3R)-3-(tert-butoxycarbonylamino)-5-(4-chlorobenzyl)-8-fluoro-1,1,4-triketo-2,3-dihydro-1λ6,5-benzothiazepin-7-yl]-1,3,4-oxadiazol-2-yl]-2-methyl-1-(trifluoromethyl)propyl]carbamic acid methyl ester (49.6 mg, 0.066) and was obtained as a light yellow solid as a hydrochloride salt (28.6 mg, 61% yield). MS (ESI): 634.3 [M+H]⁺.

### Example 138

### (3R)-3-amino-5-[(4-chlorophenyl)methyl]-7-[5-[2-(1,1-dioxo-1,4-thiazinan-4-yl)-1,1-dimethyl-ethyl]-1,3,4-oxadiazol-2-yl]-8-fluoro-1,1-dioxo-2,3-dihydro-1λ⁶,5-benzothiazepin-4-one

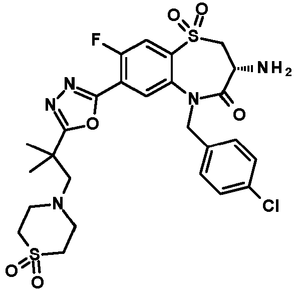

### Step a) benzyl 2,2-dimethyl-3-thiomorpholino-propanoate

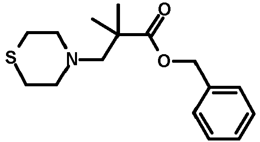

To a solution of benzyl 2,2-dimethyl-3-oxo-propanoate (500 mg, 2.42 mmol) in DCE (25 mL) was added thiomorpholine (0.39 mL, 3.88 mmol, 1.6 eq) and stirred at 25 °C for 1 h. sodium triacetoxyborohydride (925 mg, 4.36 mmol, 1.8 eq) at 0 °C was added and stirred at 25 °C for 16 hrs. The reaction mixture was cooled to 0 °C and added dropwise aqueous NH₄Cl (30 mL) to quench NaBH(OAc)₃. The mixture was extracted with EtOAc (30 mL), the organic phase was washed with brine (10 mL x 3), dried over Na₂SO₄, filtered, the organic phase was concentrated, diluted with MeOH (6 mL) and purified by prep-HPLC then freeze-dried to obtain the title compound (180 mg, 0.61 mmol, 25% yield) as a light yellow oil. MS (ESI): 294.4 [M+H]⁺.

### Step b) benzyl 2,2-dimethyl-3-(4-oxido-1,1-dioxo-1,4-thiazinan-4-ium-4-yl)propanoate

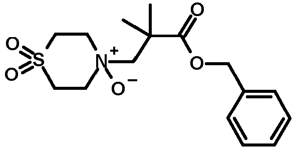

The title compound was prepared according to general method 5 from benzyl 2,2-dimethyl-3-thiomorpholino-propanoate (180 mg, 0.61 mmol) using 4.5 eq MCPBA and was obtained as a light yellow solid after prep-TLC (EtOAc) (140 mg, 0.41 mmol, 54% yield). MS (ESI): 342.1 [M+H]⁺.

### Step c) 3-(1,1-dioxo-1,4-thiazinan-4-yl)-2,2-dimethyl-propanoic acid

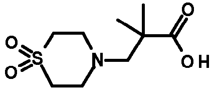

To a solution of benzyl 2,2-dimethyl-3-(4-oxido-1,1-dioxo-1,4-thiazinan-4-ium-4-yl)propanoate (120 mg, 0.30 mmol) in MeOH (4 mL) was added Pd/C (37.4 mg, 0.04 mmol, 0.1 eq) under N₂ atmosphere and degassed 3 times with H₂. The reaction was stirred at 25 °C for 16 h, filtered with diatomite, concentrated to yield the title compound (100 mg, 0.42 mmol, 99% yield) as a light yellow solid. MS (ESI): 236.1 [M+H]⁺.

### Step d) tert-butyl N-[(3R)-5-[(4-chlorophenyl)methyl]-7-[[[3-(1,1-dioxo-1,4-thiazinan-4-yl)-2,2-dimethyl-propanoyl]amino]carbamoyl]-8-fluoro-4-oxo-2,3-dihydro-1,5-benzothiazepin-3-yl]carbamate

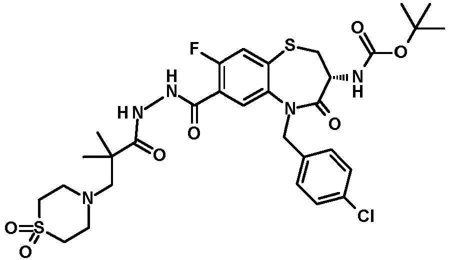

The title compound was prepared according to general procedure **7a** from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (180 mg, 0.36 mmol) and 3-(1,1-dioxo-1,4-thiazinan-4-yl)-2,2-dimethyl-propanoic acid (100 mg, 0.42 mmol, 1.2 eq) and was obtained after prep-TLC (PE:EA = 1:3) as a light yellow solid (170 mg, 0.24 mmol, 59% yield). MS (ESI): 712.2 [M+H]⁺.

### Step e) tert-butyl N-[(3R)-5-[(4-chlorophenyl)methyl]-7-[5-[2-(1,1-dioxo-1,4-thiazinan-4-yl)-1,1-dimethyl-ethyl]-1,3,4-oxadiazol-2-yl]-8-fluoro-4-oxo-2,3-dihydro-1,5-benzothiazepin-3-yl]carbamate

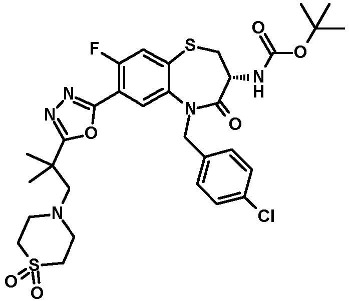

The title compound was prepared according to general procedure **8a** from tert-butyl N-[(3R)-5-[(4-chlorophenyl)methyl]-7-[[[3-(1,1-dioxo-1,4-thiazinan-4-yl)-2,2-dimethylpropanoyl]amino]carbamoyl]-8-fluoro-4-oxo-2,3-dihydro-1,5-benzothiazepin-3-yl]carbamate (170 mg, 0.24 mmol) and was obtained as a white solid (155 mg, 0.22 mmol, 93% yield). MS (ESI): 638.1 [M-isobutene+H]⁺.

### Step f) tert-butyl N-[(3R)-5-[(4-chlorophenyl)methyl]-7-[5-[1,1-dimethyl-2-(4-oxido-1,1-dioxo-1,4-thiazinan-4-ium-4-yl)ethyl]-1,3, 4-oxadiazol-2-yl]-8-fluoro-1,1,4-trioxo-2, 3-dihydro-1λ⁶,5-benzothiazepin-3-yl]carbamate

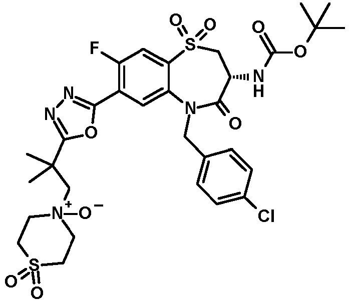

The title compound was prepared according to general procedure **5** from tert-butyl N-[(3R)-5-[(4-chlorophenyl)methyl]-7-[5-[2-(1, 1-dioxo-1,4-thiazinan-4-yl)-1, 1-dimethyl-ethyl]-1,3,4-oxadiazol-2-yl]-8-fluoro-4-oxo-2,3-dihydro-1,5-benzothiazepin-3-yl]carbamate (155 mg, 0.22 mmol) using 5.5 eq MCPBA and was obtained as a light yellow solid (360 mg, 0.49 mmol, 50% yield). MS (ESI): 742.2 [M+H]⁺.

### Step g) tert-butyl N-[(3R)-5-[(4-chlorophenyl)methyl]-7-[5-[2-(1,1-dioxo-1,4-thiazinan-4-yl)-1,1-dimethyl-ethyl]-1,3,4-oxadiazol-2-yl]-8-fluoro-1,1,4-trioxo-2,3-dihydro-1λ⁶,5-benzothiazepin-3-yl]carbamate

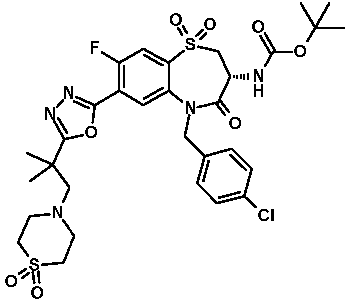

To the solution of tert-butyl N-[(3R)-5-[(4-chlorophenyl)methyl]-7-[5-[1,1-dimethyl-2-(4-oxido-1,1-dioxo-1,4-thiazinan-4-ium-4-yl)ethyl]-1,3,4-oxadiazol-2-yl]-8-fluoro-1,1,4-trioxo-2,3-dihydro-1λ⁶,5-benzothiazepin-3-yl]carbamate (360 mg, 0.49 mmol) in 1,2-dichloroethane (8 mL) was added phenylboronic acid (147 mg, 1.2 mmol, 2.5 eq) at 25 °C. The mixture was stirred at 80 °C for 0.5 h. The reaction mixture was diluted with EtOAc (10 mL), washed with water (3 x 15 mL) followed by brine (15 mL). The organic layer was dried with Na₂SO₄, filtered, oncentrated in vacuo and purified by prep-TLC (PE:EA=1:1) to obtain the title compound (140 mg, 0.19 mmol, 29% yield) as a light yellow solid. MS (ESI): 670.1 [M-isobutene+H]⁺.

### Step h) (3R)-3-amino-5-[(4-chlorophenyl)methyl]-7-[5-[2-(1,1-dioxo-1,4-thiazinan-4-yl)-1,1-dimethyl-ethyl]-1,3, 4-oxadiazol-2-yl]-8-fluoro-1,1-dioxo-2,3-dihydro-1λ⁶,5-benzothiazepin-4-one

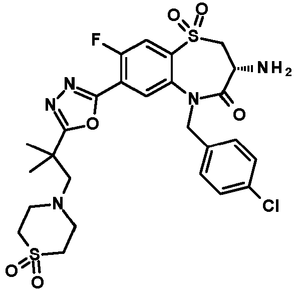

The title compound was prepared in analogy to the method used in **6b** from tert-butyl N-[(3R)-5-[(4-chlorophenyl)methyl]-7-[5-[2-(1,1-dioxo-1,4-thiazinan-4-yl)-1,1-dimethyl-ethyl]-1,3,4-oxadiazol-2-yl]-8-fluoro-1,1,4-trioxo-2,3-dihydro-1λ⁶,5-benzothiazepin-3-yl]carbamate (140 mg, 0.19 mmol) and was obtained as a white solid after prep-HPLC (52.7 mg, 43% yield). MS (ESI): 626.2 [M+H]⁺.

### Starting material for example 139

### 3-[2-[tert-butyl(dimethyl)silyl]oxyethyl-methyl-amino]-2,2-dimethyl-propanoic acid

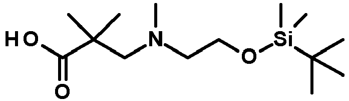

The title compound was prepared in analogy to starting material for example 64 step b and c) from benzyl 2,2-dimethyl-3-oxo-propanoate (500 mg, 2.42 mmol) and 2-[tert-butyl(dimethyl)silyl]oxy-N-methyl-ethanamine (734.56 mg, 3.88 mmol, 1.6 eq) and was obtained as a light yellow solid. MS (ESI): 290.1 [M-isobutene+H]⁺.

### Starting material for example 140

### 4-(dimethylamino)-2,2-dimethyl-butanoic acid

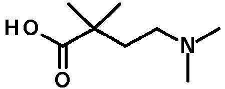

To a solution of 4-amino-2,2-dimethyl-butanoic acid;hydrochloride (example 149, step b)) 400 mg, 2.39 mmol) and formaldeyde (0.85 mL, 7.16 mmol, 3 eq) in MeOH (20 mL) was added NaBH₃CN (599 mg, 9.54 mmol, 4 eq) at 25 °C. The mixture was degassed with N₂ and stirred for 16 h. Then additional NaBH₃CN (599 mg, 9.54 mmol, 4 eq) and formaldeyde (0.85 mL, 7.16 mmol, 3 eq) was added into the reaction mixture and stirred for another 16 h. The reaction was diluted with water (10 mL), acidified by 1N aqueous HCl to pH =6 and concentrated under vacuum, extracted with EtOAc (10 mL x 3). The organic phase was concentrated and freeze dried to give the crude title compound as a hygroscopic white solid (110 mg, 0.69 mmol, 29% yield). MS (ESI): 160.2 [M+H]⁺.

### Starting material for example 141

### 2,2-dimethyl-3-(1-piperidyl)propanoic acid

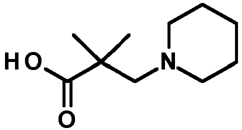

### Step a) methyl 2, 2-dimethyl-3-(1-piperidyl)propanoate

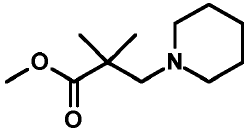

To a solution of tert-butyl-(1-methoxy-2-methyl-prop-1-enoxy)-dimethyl-silane (500 mg, 2.31 mmol, 1 eq) in DCE (6 mL) was added 2,6-ditert-butyl-4-methylpyridine (0.36 mL, 2.57 mmol, 1.1 eq). After cooled to 0 °C, Tf₂O (0.43 mL, 2.57 mmol, 1.1 eq) was added into the mixture rapidly and stirred for 2 mins. Then N-formylpiperidine (0.26 mL, 2.31 mmol, 1 eq) was added and the mixture was heated to 60 °C for 16 h. The reaction was cooled to 0 °C and NaBH₃CN (871 mg, 13.8 mmol, 6 eq) was added. The reaction was stirred for 16 h, diluted with EtOAc (10 mL) and washed with brine (10 mL x 2), dried over anhydrous Na₂SO₄, concentrated and was purified by column chromatography on silica gel (PE:EA=1:1 to 0:1) to give crude title compound (500 mg, 2.51 mmol, 108% yield) as a colorless gum. MS (ESI): 200.2 [M+H]⁺.

### Step a) 2,2-dimethyl-3-(1-piperidyl)propanoic acid

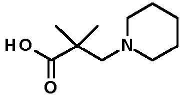

To a solution of methyl 2,2-dimethyl-3-(1-piperidyl)propanoate (400 mg, 2.0 mmol,) in THF (6 mL) and water (6 mL) was added LiOH (63.9 mg, 2.67 mmol, 1.3 eq). The resulting mixture was heated to 50 °C and stirred for 22 h. After cooled to ambient temperature, the mixture was basified with 1N aqueous NaOH to pH=8., extracted with EtOAc (5 mL), the aqueous layer was acidified to pH=5 with 1N aqueous HCl. The aqueous phase was freeze dried to give crude product which was washed with EtOAc (10 mL x 4), the organic phase was concentrated to give the title compound (250 mg, 1.35 mmol, 67% yield) as a colorless gum. MS (ESI): 186.1 [M+H]⁺.

### Starting material for example 142

### 3-(4,4-difluoro-1-piperidyl)-2,2-dimethyl-propanoic acid

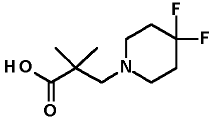

The title compound was prepared in analogy to starting material for Example 64, in 2 steps from benzyl 2,2-dimethyl-3-oxo-propanoate (500 mg, 2.42 mmol) and 4,4-difluoropiperidine (0.3 mL, 2.67 mmol, 1.1 eq) and was obtained as a light yellow solid. MS (ESI): 222.1 [M+H]⁺.

### Starting material for example 143

### 5-(dimethylamino)-2,2-dimethyl-pentanoic acid

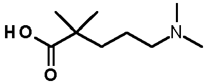

The title compound was prepared in analogy to starting material for Example 140 from 5-amino-2,2-dimethyl-pentanoic acid hydrochloride (Example 149, step b)) (500 mg, 2.75 mmol) and formaldehyde (0.98 mL, 8.26 mmol, 3 eq) and was obtained after prep-HPLC as a light brown gum (330 mg, 1.9 mmol, 69% yield). MS (ESI): 174.2 [M+H]⁺.

### Starting material for example 148

### 2,2-dimethyl-3-(4-methylpiperazin-1-yl)propanoic acid

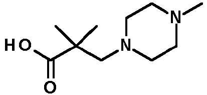

The title compound was prepared in analogy to starting material for Example 64, step b and c) in from benzyl 2,2-dimethyl-3-oxo-propanoate (500 mg, 2.42 mmol) and 1-methylpiperazine (0.43 mL, 3.88 mmol, 1.6 eq) and was obtained as a light yellow solid (100 mg). MS (ESI): 201.1 [M+H]⁺.

Examples 139 to 148 of the following table were prepared in analogy to Example 138 step d to h), using the appropriate carboxylic acid building block.

| **Ex.** | **Structure** | **Systematic Name** | **Building Block** | **MS, ESI: m/z** |
|---|---|---|---|---|
| | | | **General Procedure** | |
| 139 | 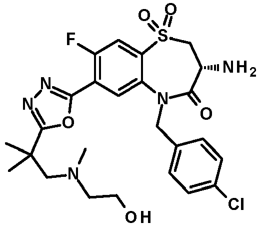 | (3R)-3-amino-5-[(4-chlorophenyl)methyl]-8-fluoro-7-[5-[2-[2-hydroxyethyl(methyl)amino]-1,1-dimethyl-ethyl]-1,3,4-oxadiazol-2-yl]-1,1-dioxo-2,3-dihydro-1λ⁶,5-benzothiazepin-4-one | 3-[2-[tert-Butyl(dime thyl)silyl]o xyethyl-methyl-amino]-2,2-dimethyl-propanoic acid, **7a, 8a, 5, 6b** | 566.3 [M+H]⁺ |
| | | (**) | | |
| 140 | 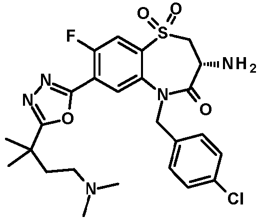 | (3R)-3-amino-5-[(4-chlorophenyl)methyl]-7-[5-[3-(dimethylamino)-1,1-dimethyl-propyl]-1,3,4-oxadiazol-2-yl]-8-fluoro-1,1-dioxo-2,3-dihydro-1λ⁶,5-benzothiazepin-4-one | 4-(Dimethyla mino)-2,2-dimethyl-butanoic acid, **7c, 8a, 5, 6b** | 550.1 [M+H]⁺ |
| | | (*) | | |
| 141 | 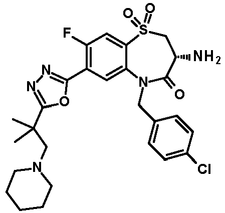 | (3R)-3-amino-5-[(4-chlorophenyl)methyl]-7-[5-[1,1-dimethyl-2-(1-piperidyl)ethyl]-1,3,4-oxadiazol-2-yl]-8-fluoro-1,1-dioxo-2,3-dihydro-1λ⁶,5-benzothiazepin-4-one | 2,2-Dimethyl-3-(1-piperidyl)p ropanoic acid, **7a, 8a, 5, 6b** | 576.2 [M+H]⁺ |
| | | (**) | | |
| 142 | 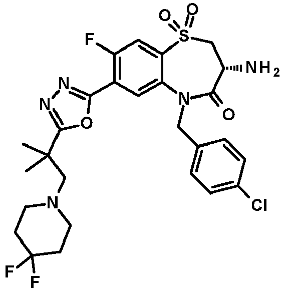 | (3R)-3-amino-5-[(4-chlorophenyl)methyl]-7-[5-[2-(4,4-difluoro-1-piperidyl)-1,1-dimethyl-ethyl]-1,3,4-oxadiazol-2-yl]-8-fluoro-1,1-dioxo-2,3-dihydro-1λ⁶,5-benzothiazepin-4-one | 3-(4,4-Difluoro-1-piperidyl)-2,2-dimethyl-propanoic acid, **7a, 8a, 5, 6b** | 612.1 [M+H]⁺ |
| | | (**) | | |
| 143 | 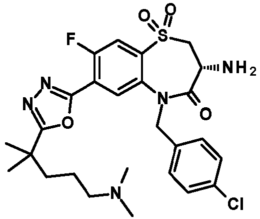 | (3R)-3-amino-5-[(4-chlorophenyl)methyl]-7-[5-[4-(dimethylamino)-1,1-dimethyl-butyl]-1,3,4-oxadiazol-2-yl]-8-fluoro-1,1-dioxo-2,3-dihydro-1λ⁶,5-benzothiazepin-4-one | 5-(Dimethyla mino)-2,2-dimethyl-pentanoic acid, **7a, 8a, 5, 6b** | 564.2 [M+H]⁺ |
| | | (*) | | |
| 144 | 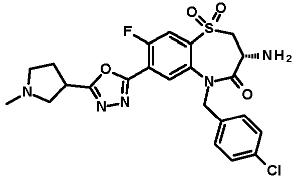 | (3R)-3-amino-5-[(4-chlorophenyl)methyl]-8-fluoro-7-[5-(1-methylpyrrolidin-3-yl)-1,3,4-oxadiazol-2-yl]-1,1-dioxo-2,3-dihydro-1λ⁶,5-benzothiazepin-4-one | 1-Methylpyrr olidine-3-carboxylic acid **7c 8a, 5, 6b** | 520.2 [M+H]⁺ |
| | | (**) | | |
| 145 | 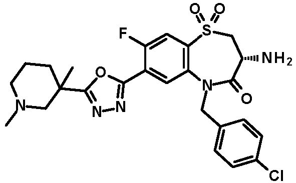 | (3R)-3-amino-5-[(4-chlorophenyl)methyl]-7-[5-(1,3-dimethyl-3-piperidyl)-1,3,4-oxadiazol-2-yl]-8-fluoro-1,1-dioxo-2,3-dihydro-1λ⁶,5-benzothiazepin-4-one | 1,3-Dimethylni pecotic acid, **7a, 8a, 5, 6d** | 548.2 [M+H]⁺ |
| 146 | 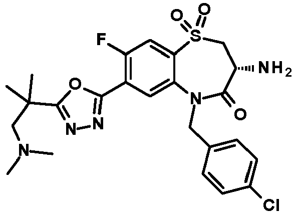 | (3R)-3-amino-5-[(4-chlorophenyl)methyl]-7-[5-[2-(dimethylamino)-1,1-dimethyl-ethyl]-1,3,4-oxadiazol-2-yl]-8-fluoro-1,1-dioxo-2,3-dihydro-1λ⁶,5-benzothiazepin-4-one | 3-(Dimethyla mino)-2,2-dimethyl-propanoic acid, **7a, 8a, 5, 6b** | 536.0 [M+H]⁺ |
| | | (**) | | |
| 147 | 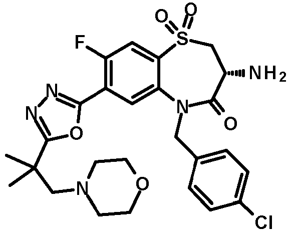 | (3R)-3-amino-5-[(4-chlorophenyl)methyl]-7-[5-(1,1-dimethyl-2-morpholinoethyl)-1,3,4-oxadiazol-2-yl]-8-fluoro-1,1-dioxo-2,3-dihydro-1λ⁶,5-benzothiazepin-4-one | 2,2-Dimethyl-3-morpholin o-propanoic acid, **7a, 8a, 5, 6b** | 578.1 [M+H]⁺ |
| | | (**) | | |
| 148 | 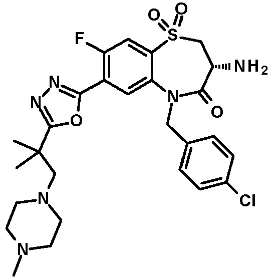 | (3R)-3-amino-5-[(4-chlorophenyl)methyl]-7-[5-[1,1-dimethyl-2-(4-methylpiperazin-1-yl)ethyl]-1,3,4-oxadiazol-2-yl]-8-fluoro-1,1-dioxo-2,3-dihydro-1λ⁶,5-benzothiazepin-4-one | 2,2-Dimethyl-3-(4-methylpip erazin-1-yl)propanoic acid, **7a, 8a, 5, 6b** | 591.2 [M+H]⁺ |
| | | (**) | | |

| | | | | |
|---|---|---|---|---|
| * as a hydrochloride salt ** as free base after prep HPLC | | | | |

### Example 149

### (3R)-3-amino-7-[5-(4-amino-1,1-dimethyl-butyl)-1,3,4-oxadiazol-2-yl]-5-[(4-chlorophenyl)methyl]-8-fluoro-1,1-dioxo-2,3-dihydro-1λ⁶,5-benzothiazepin-4-one

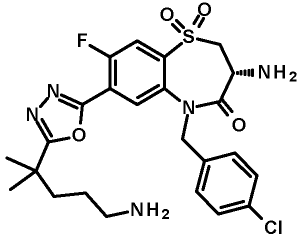

### Step a) tert-butyl 3,3-dimethyl-2-oxo-piperidine-1-carboxylate

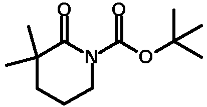

To a solution of LiHMDS (13.8 mL, 13.8 mmol, 2.75 eq) in THF (14 mL) was added a solution of 1-Boc-2-piperidone (1g, 5.0 mmol, 1 eq) in THF (6 mL) dropwise at -70 °C under N₂, then the mixture was warmed up to 25 °C and stirred for 1 h. The mixture was re-cooled to -70 °C and MeI (1.56 mL, 25 mmol, 5 eq) was added dropwise. The resulting mixture was warmed up to 25°C and stirred for 2 h. The reaction was quenched with water (20 mL) under 0 °C. After separated, the aqueous phase was extracted with EtOAc (10 mL x 3), the combined organic phase was washed with brine (30 mL x 3), dried over anhydrous Na₂SO₄, concentrated and purified by column chromatography on silica gel (PE:EA=15:1 to 5:1), concentrated under vacuum to give the title compound (470 mg, 2.1 mmol, 33% yield) as a light brown oil. MS (ESI): 172.2 [M-isobutene+H]⁺.

### Step b) 5-amino-2,2-dimethyl-pentanoic acid

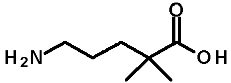

tert-butyl 3,3-dimethyl-2-oxo-piperidine-1-carboxylate (370 mg, 1.63 mmol) was dissolved in 6N aqueous HCl (7.4 mL, 44.4 mmol, 27 eq) and refluxed for 16 hours. The mixture was cooled to room temperature, concentrated under vacuum twice with THF to give the crude title compound as a hydrochloride salt (220 mg, 1.2 mmol, 74% yield) as a brown solid. MS (ESI): 146.1 [M+H]+.

### Step c) 5-(benzyloxycarbonylamino)-2,2-dimethyl-pentanoic acid

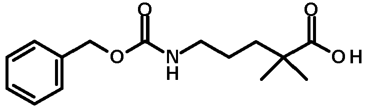

To a solution of 5-amino-2,2-dimethyl-pentanoic acid hydrochloride (220 mg, 1.2 mmol) in 1,4-dioxane (2.5 mL) was added dropwise a solution of 1N aqueous NaOH (5.45 mL, 5.45 mmol, 4.5 eq) at 0°C. The mixture was stirred 0.25 h at 0 °C. To the solution was added benzyl chloroformate (413 mg, 2.42 mmol, 2 eq) at 0 °C, and stirred for 5 h at 25 °C. The mixture was diluted with water (3 mL), extracted with PE:EtOAc (4:1, 5 mL*3). The water layer was acidified by 1N aqueous HCl to pH ~5, extracted with EtOAc (10 mL*2). The combined organic phase was dried over anhydrous Na₂SO₄, concentrated in vacuo to obtain the crude title compound (380 mg, 1.36 mmol, 70% yield) as brown oil confirmed. MS (ESI): 280.3 [M+H]⁺.

### Step d) benzyl N-[4,4-dimethyl-5-oxo-5-[2-[(3R)-3-(tert-butoxycarbonylamino)-5-[(4-chlorophenyl)methyl]-8-fluoro-4-oxo-2,3-dihydro-1,5-benzothiazepine-7-carbonyl]hydrazino]pentyl]carbamate

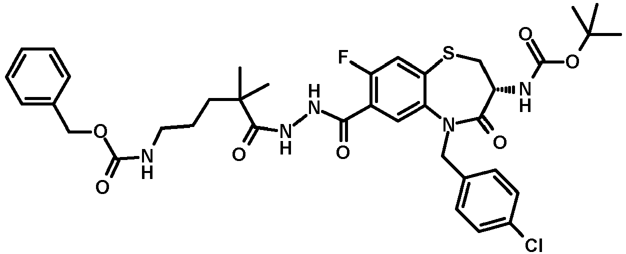

The title compound was prepared according to general procedure **7a** from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (290 mg, 0.59 mmol) and 5-(benzyloxycarbonylamino)-2,2-dimethyl-pentanoic acid (280 mg, 0.63 mmol, 1.1 eq) and was obtained after column chromatography on silica gel (PE:EA = 5:1 to 2:1)as a white solid (450 mg, 0.6 mmol, 100% yield). MS (ESI): 756.3 [M+H]⁺.

### Step e) benzyl N-[4-methyl-4-[5-[(3R)-3-(tert-butoxycarbonylamino)-5-[(4-chlorophenyl)methyl]-8-fluoro-4-oxo-2, 3-dihydro-1, 5-benzothiazepin-7-yl]-1,3, 4-oxadiazol-2-ylJpentylJcarbamate

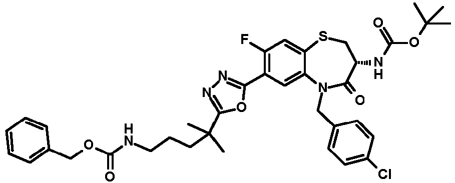

The title compound was prepared according to general procedure **8a** from benzyl N-[4,4-dimethyl-5-oxo-5-[2-[(3R)-3-(tert-butoxycarbonylamino)-5-[(4-chlorophenyl)methyl]-8-fluoro-4-oxo-2,3-dihydro-1,5-benzothiazepine-7-carbonyl]hydrazino]pentyl]carbamate (300 mg, 0.4 mmol) and was obtained as a light brown gum (400 mg, 0.54 mmol, 132% yield). MS (ESI): 738.3 [M+H]⁺.

### Step f) benzyl N-[4-methyl-4-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,3, 4-oxadiazol-2-yl]pentyl]carbamate

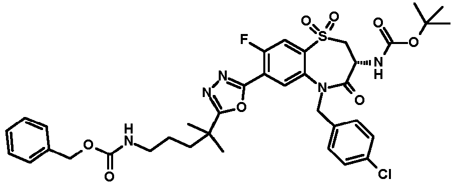

The title compound was prepared according to general procedure **5** from benzyl N-[4-methyl-4-[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]pentyl]carbamate (300 mg, 0.41 mmol) was obtained as a light yellow solid (210 mg, 0.27 mmol, 61% yield). MS (ESI): 770.3 [M+H]⁺.

### Step g) tert-butyl N-[(3R)-5-[(4-chlorophenyl)methyl]-7-[5-(5, 5-difluoro-3-piperidyl)-1, 3, 4-oxadiazol-2-yl]-8-fluoro-1,1,4-trioxo-2,3-dihydro-1λ⁶,5-benzothiazepin-3-yl]carbamate

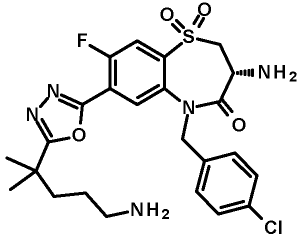

To solution of benzyl N-[4-methyl-4-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,3,4-oxadiazol-2-yl]pentyl]carbamate (160 mg, 0.21 mmol) in methanol (6 mL) was added EtOAc\HCl (1.04 mL, 4.15 mmol, 20 eq) and Pd/C (44 mg, 0.42 mmol, 2 eq), the reaction mixture was stirred at 25 °C for 0.5 h under a bolloon with hydrogen. Then EtOAc\HCl (1.56 mL, 6.23 mmol, 30 eq) was added into the mixture and stirred for 1 h. After filtered by diatomite, the filtrate was blow-dried by N₂ flow to 2 mL, the residue was purified by prep-HPLC and freeze dried to give the title compound as the bis hydrochloride salt (68 mg, 0.11 mmol, 51% yield) as a white solid. MS (ESI): 536.2 [M+H]⁺.

### Example 150

### (3R)-3-amino-5-[(4-chlorophenyl)methyl]-7-[5-(1-ethyl-5,5-difluoro-3-piperidyl)-1,3,4-oxadiazol-2-yl]-8-fluoro-1,1-dioxo-2,3-dihydro-1λ⁶,5-benzothiazepin-4-one

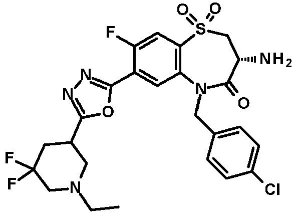

### Step a) benzyl 3,3-difluoro-5-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoyl]piperidine-1-carboxylate

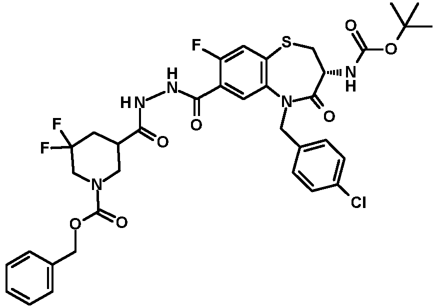

The title compound was prepared according to general procedure **7a** from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (2.9 g, 5.86 mmol) and 1-benzyloxycarbonyl-5,5-difluoro-piperidine-3-carboxylic acid (2.1 g, 7.03 mmol, 1.2 eq) and was obtained after chromatography on silica gel (PE:EA = 10:1 to 1:1) as a white solid (4.77 g, 6.14 mmol, 105% yield). MS (ESI): 776.3 [M+H]⁺.

### Step b) benzyl 3,3-difluoro-5-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoyl]piperidine-1-carboxylate

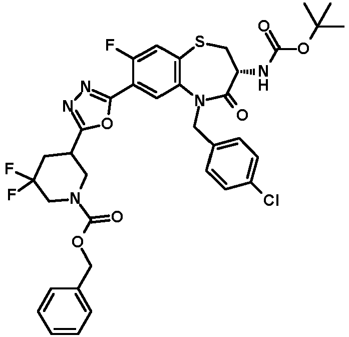

The title compound was prepared according to general procedure **8a** from benzyl 3,3-difluoro-5-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoyl]piperidine-1-carboxylate (4.0 g, 5.15 mmol) and was obtained after chromatography on silica gel (PE:EA = 10:1 to 1:1) as a white solid (2.66 g, 3.51 mmol, 68% yield). MS (ESI): 758.2 [M+H]⁺.

### Step c) benzyl 3,3-difluoro-5-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,3, 4-oxadiazol-2-yl]piperidine-1-carboxylate

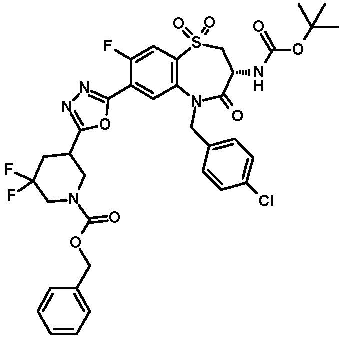

The title compound was prepared according to general procedure **5** from benzyl 3,3-difluoro-5-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoyl]piperidine-1-carboxylate (42.6 g, 3.43 mmol) and was obtained after chromatography on silica gel as a white solid (2.6 g, 3.29 mmol, 94% yield). MS (ESI): 734.3 [M-isobutene+H]⁺.

### Step d) tert-butyl N-[(3R)-5-[(4-chlorophenyl)methyl]-7-[5-(5, 5-difluoro-3-piperidyl)-1,3,4-oxadiazol-2-yl]-8-fluoro-1,1,4-trioxo-2,3-dihydro-1λ⁶,5-benzothiazepin-3-yl]carbamate

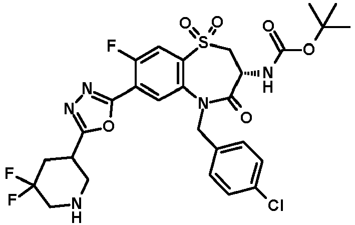

To a solution of benzyl 3,3-difluoro-5-[5-[(3R)-3-(tert-butoxycarbonylamino)-5-[(4-chlorophenyl)methyl]-8-fluoro-1,1,4-trioxo-2,3-dihydro-1λ6,5-benzothiazepin-7-yl]-1,3,4-oxadiazol-2-yl]piperidine-1-carboxylate (400 mg, 0.51 mmol) in MeOH (5 mL) was added Pd/C/H₂O (100 mg) .Tthe mixture was stirred at 25 °C for 0.5 h under H₂. The mixture was filtered and purified by prep-HPLC to afford the title compound (800 mg, 1.22 mmol, 240% yield) as white solid. MS (ESI): 656.3 [M+H]⁺.

### Step e) tert-butyl N-[(3R)-5-[(4-chlorophenyl)methyl]-7-[5-(1-ethyl-5,5-difluoro-3-piperidyl)-1,3,4-oxadiazol-2-yl]-8-fluoro-1,1,4-trioxo-2,3-dihydro-1λ⁶,5-benzothiazepin-3-yl]carbamate

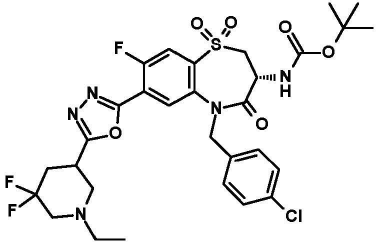

To a solution of acetaldehyde (0.1 mL, 1.79 mmol, 14 eq) in MeOH (3 mL) was added tert-butyl N-[(3R)-5-[(4-chlorophenyl)methyl]-7-[5-(5,5-difluoro-3-piperidyl)-1,3,4-oxadiazol-2-yl]-8-fluoro-1,1,4-trioxo-2,3-dihydro-1λ⁶,5-benzothiazepin-3-yl]carbamate (80.0 mg, 0.12 mmol). After the mixture was stirred for 30 min, sodium triacetoxyborohydride (259 mg, 1.2 mmol, 10 eq) was added. The mixture was stirred at 25 °C for 1 h, poured to water (10 mL) and extracted by DCM (10 mL x 2). The organic layers were washed by brine (10 mL x 2), dried over Na₂SO₄, concentrated in vacuo and purified by column chromatography on silica gel (PE:EA=10:1 to 1:1) to afford the title compound (70 mg, 0.1 mmol, 83% yield) as white solid. MS (ESI): 684.2 [M+H]⁺.

### Step f) (3R)-3-amino-5-[(4-chlorophenyl)methyl]-7-[5-(1-ethyl-5,5-difluoro-3-piperidyl)-1,3,4-oxadiazol-2-yl]-8-fluoro-1,1-dioxo-2,3-dihydro-1λ⁶,5-benzothiazepin-4-one

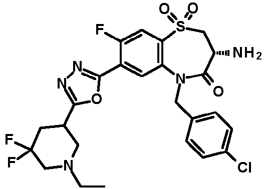

The title compound was prepared according to general procedure **6b** from tert-butyl N-[(3R)-5-[(4-chlorophenyl)methyl]-7-[5-(1-ethyl-5,5-difluoro-3-piperidyl)-1,3,4-oxadiazol-2-yl]-8-fluoro-1,1,4-trioxo-2,3-dihydro-1λ⁶,5-benzothiazepin-3-yl]carbamate (70.0 mg, 0.1 mmol) and was obtained after prep-HPLC as a white solid (35.4 mg, 0.06 mmol, 37% yield). MS (ESI): 584.1 [M+H]⁺.

Examples 151 to 153 of the following table were prepared in analogy to Example 150 step e-f), using the appropriate aldehyde building block.

| **Ex.** | **Structure** | **Systematic Name** | **Building Block deprotection Method** | **MS, ESI: m/z** |
|---|---|---|---|---|
| 151 | 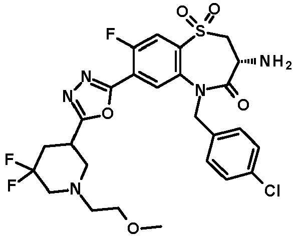 | (3R)-3-amino-5-[(4-chlorophenyl)methyl]-7-[5-[5,5-difluoro-1-(2-methoxyethyl)-3-piperidyl]-1,3,4-oxadiazol-2-yl]-8-fluoro-1,1-dioxo-2,3-dihydro-1λ⁶,5-benzothiazepin-4-one (*) | Methoxy acetaldehy de, **6a** | 614.2 [M+H]⁺ |
| 152 | 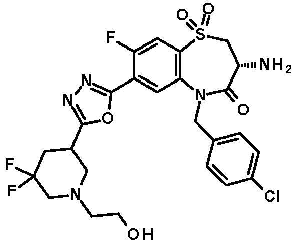 | (3R)-3-amino-5-[(4-chlorophenyl)methyl]-7-[5-[5,5-difluoro-1-(2-hydroxyethyl)-3-piperidyl]-1,3,4-oxadiazol-2-yl]-8-fluoro-1,1-dioxo-2,3-dihydro-1λ⁶,5-benzothiazepin-4-one (*) | (tert-Butyldimet hylsilyloxy) acetaldehy de,**6b** | 600.2 [M+H]⁺ |
| 153 | 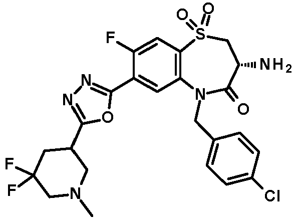 | (3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1λ⁶,5-benzothiazepin-4-one (*) | Formaldeh yde, **6b** | 570.4 [M+H]⁺ |

| | | | | |
|---|---|---|---|---|
| * as free base after prep HPLC | | | | |

Examples 154 to 157 of the following table were prepared in analogy to Example 150 step a-f), using the appropriate acid and aldehyde building block.

| **Ex.** | **Structure** | **Systematic Name** | **Building Block General Procedure s** | **MS, ESI: m/z** |
|---|---|---|---|---|
| 154 | 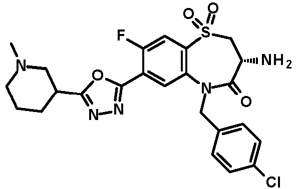 | (3R)-3-amino-5-[(4-chlorophenyl)methyl]-8-fluoro-7-[5-(1-methyl-3-piperidyl)-1,3,4-oxadiazol-2-yl]-1,1-dioxo-2,3-dihydro-1λ⁶,5-benzothiazepin-4-one (**) | 1-[(Benzylox y)carbonyl] piperidine-3-carboxylic acid, formaldeh yde **7c, 8a, 5, 6b** | 534.4 [M+H]⁺ |
| 155 | 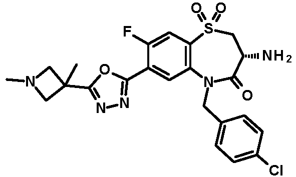 | (3R)-3-amino-5-[(4-chlorophenyl)methyl]-7-[5-(1,3-dimethylazetidin-3-yl)-1,3,4-oxadiazol-2-yl]-8-fluoro-1,1-dioxo-2,3-dihydro-1λ⁶,5-benzothiazepin-4-one (*) | 1-Carbobenz oxy-3-methylazetidine-3-carboxylic acid, formaldeh yde **7a, 8a, 5, 6d** | 520.2 [M+H]⁺ |
| 156 | 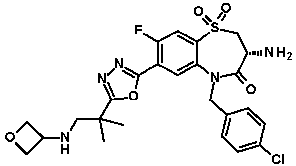 | (3R)-3-amino-5-[(4-chlorophenyl)methyl]-7-[5-[1,1-dimethyl-2-(oxetan-3-ylamino)ethyl]-1,3,4-oxadiazol-2-yl]-8-fluoro-1,1-dioxo-2,3-dihydro-1lambda6,5-benzothiazepin-4-one (**) | 3-(Benzyloxy carbonyla mino)-2,2-dimethylpropanoic acid, 3-oxetanone , **7a, 8a, 5, 6e** | 564.4 [M+H]⁺ |
| 157 | 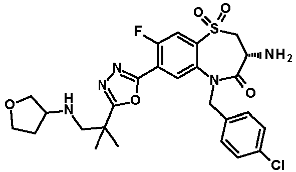 | (3R)-3-amino-5-[(4-chlorophenyl)methyl]-7-[5-[1,1-dimethyl-2-(tetrahydrofuran-3-ylamino)ethyl]-1,3,4-oxadiazol-2-yl]-8-fluoro-1,1-dioxo-2,3-dihydro-1λ⁶,5-benzothiazepin-4-one (**) | 3-(Benzyloxy carbonyla mino)-2,2-dimethyl-propanoic acid, dihydrofur an-3(2H)-one, **7a, 8a, 5, 6e** | 578.4 [M+H]⁺ |

| | | | | |
|---|---|---|---|---|
| * as a hydrochloride salt ** as free base after prep-HPLC | | | | |

### Example 158

### (3R)-3-amino-5-[(4-chlorophenyl)methyl]-7-[5-(4,4-difluoro-1-methyl-3-piperidyl)-1,3,4-oxadiazol-2-yl]-8-fluoro-1,1-dioxo-2,3-dihydro-1λ⁶,5-benzothiazepin-4-one

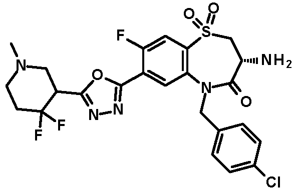

### Step a) tert-butyl N-[(3R)-7-[[(1-benzyl-4,4-difluoro-piperidine-3-carbonyl)amino]carbamoyl]-5-[(4-chlorophenyl)methyl]-8-fluoro-4-oxo-2,3-dihydro-1,5-benzothiazepin-3-yl]carbamate

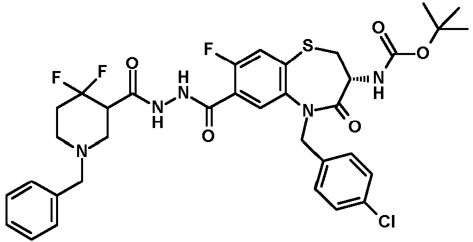

The title compound was prepared according to general procedure 7b from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (1.0 g, 2.02 mmol) and 1-benzyl-4,4-difluoro-piperidine-3-carboxylic acid (860.0 mg, 3.37 mmol, 1.67 eq ) and was obtained after prep HPLC as a white solid (1.24 g, 1.69 mmol, 83% yield).

### Step b) tert-butyl N-[(3R)-7-[5-(1-benzyl-4, 4-difluoro-3-piperidyl)-1,3, 4-oxadiazol-2-yl]-5-[(4-chlorophenyl)methyl]-8-fluoro-4-oxo-2,3-dihydro-1,5-benzothiazepin-3-yl]carbamate

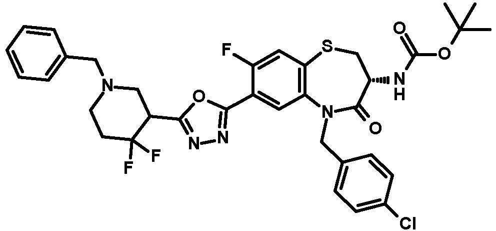

The title compound was prepared according to general procedure **8a** from tert-butyl N-[(3R)-7-[[(1-benzyl-4,4-difluoro-piperidine-3-carbonyl)amino]carbamoyl]-5-[(4-chlorophenyl)methyl]-8-fluoro-4-oxo-2,3-dihydro-1,5-benzothiazepin-3-yl]carbamate (0.9 g, 1.23 mmol) and was obtained after prep-HPLC as a white solid (430 mg, 0.6 mmol, 48% yield). MS (ESI): 714.2 [M+H]⁺.

### Step c) tert-butyl N-[(3R)-5-[(4-chlorophenyl)methyl]-7-[5-(4,4-difluoro-1-methyl-3-piperidyl)-1,3,4-oxadiazol-2-yl]-8-fluoro-4-oxo-2,3-dihydro-1,5-benzothiazepin-3-yl]carbamate

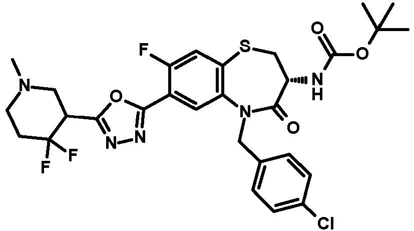

To a solution of tert-butyl N-[(3R)-7-[5-(1-benzyl-4,4-difluoro-3-piperidyl)-1,3,4-oxadiazol-2-yl]-5-[(4-chlorophenyl)methyl]-8-fluoro-4-oxo-2,3-dihydro-1,5-benzothiazepin-3-yl]carbamate (210 mg, 0.29 mmol) in MeOH (3 mL) was added formaldehyde (0.02 g, 0.29 mmol, 1 eq) and Pd/C/H₂O (100) under nitrogen. The mixture was stirred at 25 °C for 1 h under H₂, filtered and purified by prep-TLC (PE:EA = 1:1) to afford the title compound (120 mg, 0.19 mmol, 63% yield) as white solid. MS (ESI): 638.2 [M+H]⁺.

### Step d) tert-butyl N-[(3R)-5-[(4-chlorophenyl)methyl]-7-[5-(4,4-difluoro-1-methyl-1-oxido-piperidin-1-ium-3-yl)-1,3,4-oxadiazol-2-yl]-8-fluoro-1,1,4-trioxo-2,3-dihydro-1λ⁶,5-benzothiazepin-3-yl]carbamate

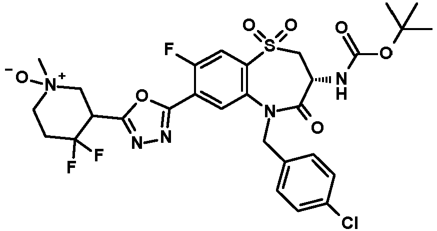

The title compound was prepared according to general procedure 5 from tert-butyl N-[(3R)-5-[(4-chlorophenyl)methyl]-7-[5-(4,4-difluoro-1-methyl-3-piperidyl)-1,3,4-oxadiazol-2-yl]-8-fluoro-4-oxo-2,3-dihydro-1,5-benzothiazepin-3-yl]carbamate (120 mg, 0.19 mmol) and was obtained as a yellow solid (120 mg, 0.17 mmol, 93% yield). MS (ESI): 686.3 [M+H]⁺.

### Step e) tert-butylN-[(3R)-5-[(4-chlorophenyl)methyl]-7-[5-(4,4-difluoro-1-methyl-3-piperidyl)-1,3,4-oxadiazol-2-yl]-8-fluoro-1,1,4-trioxo-2,3-dihydro-1λ⁶,5-benzothiazepin-3-yl]carbamate

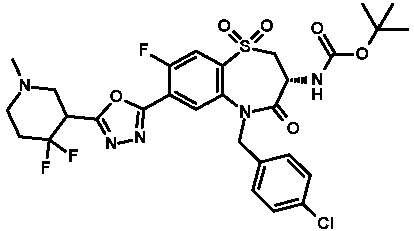

The title compound was prepared according to Example 138, step g) from tert-butyl N-[(3R)-5-[(4-chlorophenyl)methyl]-7-[5-(4,4-difluoro-1-methyl-1-oxido-piperidin-1-ium-3-yl)-1,3,4-oxadiazol-2-yl]-8-fluoro-1,1,4-trioxo-2,3-dihydro-1λ⁶,5-benzothiazepin-3-yl]carbamate (90 mg, 0.13 mmol) and was obtained after-prep-TLC (PE:EA = 1:1) as a white solid (40 mg, 0.060 mmol, 45% yield). MS (ESI): 670.4 [M+H]⁺.

### Step f) (3R)-3-amino-5-[(4-chlorophenyl)methyl]-7-[5-(4,4-difluoro-1-methyl-3-piperidyl)-1,3, 4-oxadiazol-2-yl]-8-fluoro-1,1-dioxo-2,3-dihydro-1λ⁶,5-benzothiazepin-4-one

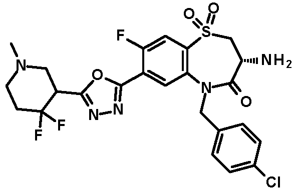

The title compound was prepared according to general procedure **6b** from tert-butyl N-[(3R)-5-[(4-chlorophenyl)methyl]-7-[5-(4,4-difluoro-1-methyl-3-piperidyl)-1,3,4-oxadiazol-2-yl]-8-fluoro-1,1,4-trioxo-2,3-dihydro-1λ⁶,5-benzothiazepin-3-yl]carbamate (35.0 mg, 0.05 mmol) and was obtained as a white solid as a hydrochloride salt (27 mg, 0.040 mmol, 88% yield). MS (ESI): 570.2 [M+H]⁺.

### Example 159

### (3R)-3-amino-5-[(4-chlorophenyl)methyl]-7-[5-(1-cyclopropyl-5,5-difluoro-3-piperidyl)-1,3,4-oxadiazol-2-yl]-8-fluoro-1,1-dioxo-2,3-dihydro-1λ⁶,5-benzothiazepin-4-one

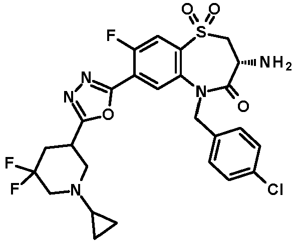

### Step a) tert-butyl N-[(3R)-5-[(4-chlorophenyl)methyl]-7-[5-(1-cyclopropyl-5, 5-difluoro-3-piperidyl)-1,3,4-oxadiazol-2-yl]-8-fluoro-1, 1, 4-trioxo-2,3-dihydro-1λ⁶,5-benzothiazepin-3-yl]carbamate

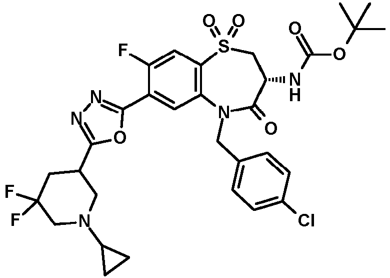

To a solution of N-[(3R)-5-(4-chlorobenzyl)-7-[5-(5,5-difluoro-3-piperidyl)-1,3,4-oxadiazol-2-yl]-8-fluoro-1,1,4-triketo-2,3-dihydro-1λ6,5-benzothiazepin-3-yl]carbamic acid tert-butyl ester (16 mg, 0.023 mmol, 1 eq) in THF (0.12 mL) and MeOH (0.12 mL) was added (1-ethoxycyclopropoxy)trimethylsilane (8.16 mg, 9.38 uL, 0.047 mmol, 2 eq), NaBH₃CN (2.21 mg, 0.035 mmol, 1.5 eq), and acetic acid (2.39 mg, 2.28 uL, 0.04 mmol, 1.7 eq). The reaction mixture was kept at 60 °C and stirred overnight. The reaction was diluted with DCM and sat. aq. NaHCO3 was added. Phases were separated and the aq. phase was washed twice with DCM. The combined organic phases were dried over Na₂SO₄, filtered, concentrated in vacuo, purified using flash column chromatography (heptane:EtOAC = 1:0 to 1:1) to afford the title compound as a white solid (5 mg, 28% yield). MS (ESI): 696.3 [M+H]⁺.

### Step b) (3R)-3-amino-5-[(4-chlorophenyl)methyl]-7-[5-(1-cyclopropyl-5,5-difluoro-3-piperidyl)-1,3, 4-oxadiazol-2-yl]-8-fluoro-1,1-dioxo-2,3-dihydro-1λ⁶,5-benzothiazepin-4-one

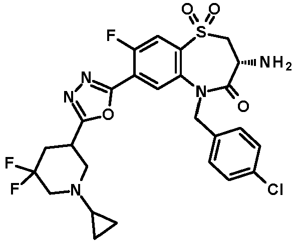

The title compound was prepared according to general procedure **6d** from tert-butyl N-[(3R)-5-[(4-chlorophenyl)methyl]-7-[5-(1-cyclopropyl-5,5-difluoro-3-piperidyl)-1,3,4-oxadiazol-2-yl]-8-fluoro-1,1,4-trioxo-2,3-dihydro-1λ⁶,5-benzothiazepin-3-yl]carbamate (6 mg, 0.009 mmol) and was obtained as a white solid as a hydrochloride salt (35 mg, 87% yield). MS (ESI): 596.2 [M+H]⁺.

### Example 160

### (3R)-3-amino-5-[(4-chlorophenyl)methyl]-7-[5-(5,5-difluoro-3-piperidyl)-1,3,4-oxadiazol-2-yl]-8-fluoro-1,1-dioxo-2,3-dihydro-1λ⁶,5-benzothiazepin-4-one

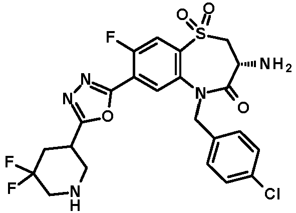

The title compound was prepared according to general procedure **6d** from tert-butyl N-[(3R)-5-[(4-chlorophenyl)methyl]-7-[5-(5,5-difluoro-3-piperidyl)-1,3,4-oxadiazol-2-yl]-8-fluoro-1,1,4-trioxo-2,3-dihydro-1λ⁶,5-benzothiazepin-3-yl]carbamate (50.0 mg, 0.08 mmol) and was obtained after prep-HPLC as a white solid (25.9 mg, 0.05 mmol, 60% yield). MS (ESI): 556.3 [M+H]⁺.

Example 161 of the following table were prepared in analogy to Example 160 using the appropriate Boc-protected building block.

| **Ex.** | **Structure** | **Systematic Name** | **Building Block deprotection Method** | **MS, ESI: m/z** |
|---|---|---|---|---|
| 161 | 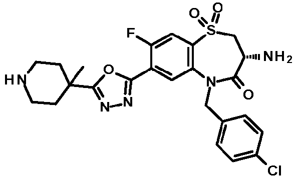 | (3R)-3-amino-5-[(4-chlorophenyl)methyl]-8-fluoro-1-[5-(4-methyl-4-piperidyl)-1,3,4-oxadiazol-2-yl]-1,1-dioxo-2,3-dihydro-1λ⁶,5-benzothiazepin-4-one (*) | Example 161, step d), **6b** | 534.1 [M+H]⁺ |

| | | | | |
|---|---|---|---|---|
| * as a hydrochloride salt | | | | |

### Example 162

### (3R)-7-[5-(1-acetyl-5,5-difluoro-3-piperidyl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one

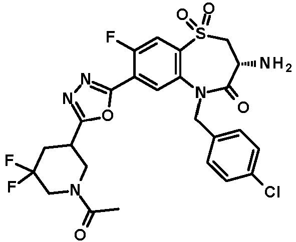

### Step a) tert-butyl N-[(3R)-7-[5-(1-acetyl-5,5-difluoro-3-piperidyl)-1,3, 4-oxadiazol-2-yl]-5-[(4-chlorophenyl)methyl]-8-fluoro-1,1, 4-trioxo-2, 3-dihydro-1λ⁶,5-benzothiazepin-3-yl]carbamate

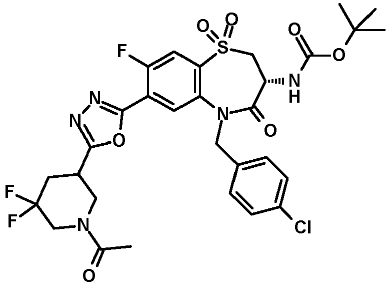

To a solution of tert-butyl N-[(3R)-5-[(4-chlorophenyl)methyl]-7-[5-(5,5-difluoro-3-piperidyl)-1,3,4-oxadiazol-2-yl]-8-fluoro-1,1,4-trioxo-2,3-dihydro-1λ⁶,5-benzothiazepin-3-yl]carbamate (80.0 mg, 0.12 mmol) in DCM (4 mL) was added acetic anhydride (25.0 mg, 0.24 mmol, 2.0 eq), DIPEA (0.07 mL, 0.37 mmol, 3.0 eq).Then the mixture was stirred at 2 °C for 1 h. The reaction was poured onto water (10ml), extracted with DCM (10 mL x 3), washed with brine (10 mL x 3), dried over Na₂SO₄,concentrated in vacuo and purified by column chromatography on silica gel (PE:EA=10:1 to 2:1) to afford the title compound (90 mg, 0.13 mmol, 105% yield) as white solid. MS (ESI): 698.4 [M+H]⁺.

### Step b) (3R)-7-[5-(1-acetyl-5,5-difluoro-3-piperidyl)-1,3, 4-oxadiazol-2-yl]-3-amino-5-[(4-chlorophenyl)methyl]-8-fluoro-1,1-dioxo-2,3-dihydro-1λ⁶,5-benzothiazepin-4-one

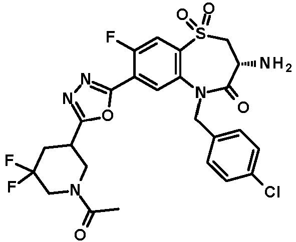

The title compound was prepared according to general procedure **6b** from tert-butyl N-[(3R)-7-[5-(1-acetyl-5,5-difluoro-3-piperidyl)-1,3,4-oxadiazol-2-yl]-5-[(4-chlorophenyl)methyl]-8-fluoro-1,1,4-trioxo-2,3-dihydro-1λ⁶,5-benzothiazepin-3-yl]carbamate (90.0 mg, 0.130 mmol) and was obtained as a white solid (42.2 mg, 0.070 mmol, 53% yield) as the hydrochloric acid salt. MS (ESI): 598.3 [M+H]⁺.

Examples 163 and 164of the following table were prepared in analogy to Example 162 using the appropriate acylating building block.

| **Ex.** | **Structure** | **Systematic Name** | **Building Block deprotection Method** | **MS, ESI: m/z** |
|---|---|---|---|---|
| 163 | 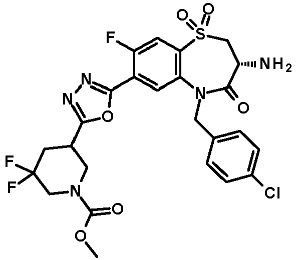 | methyl 3,3-difluoro-5-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]piperidine-1-carboxylate (*) | Methyl chloroform ate procedure, **6b** | 614.1 [M+H]⁺ |
| 164 | 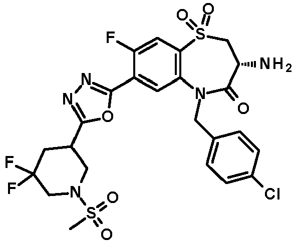 | (3R)-3-amino-5-[(4-chlorophenyl)methyl]-7-[5-(5,5-difluoro-1-methylsulfonyl-3-piperidyl)-1,3,4-oxadiazol-2-yl]-8-fluoro-1,1-dioxo-2,3-dihydro-1λ⁶,5-benzothiazepin-4-one (*) | Methanes ulfonyl chloride, **6b** | 634.1 [M+H]⁺ |

| | | | | |
|---|---|---|---|---|
| * as free base after prep HPLC | | | | |

Examples 165 to 168 of the following table were prepared in analogy to Example 150, step a to d) and to Example 162 a,b), using the appropriate acid and acylating building block.

| **Ex.** | **Structure** | **Systematic Name** | **Building Block General Method** | **MS, ESI: m/z** |
|---|---|---|---|---|
| 165 | 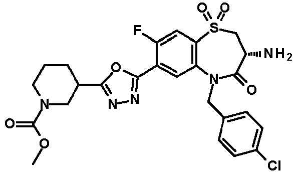 | methyl 3-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]piperidine-1-carboxylate (*) | 1-[(Benzylox y)carbonyl] piperidine-3-carboxylic acid, methyl chloroform ate **7c, 8a, 5, 6b** | 578.4 [M+H]⁺ |
| 166 | 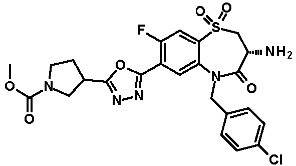 | methyl 3-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]pyrrolidine-1-carboxylate (**) | 1-Benzyloxyc arbonylpyr rolidine-3-carboxylic acid, methyl chloroform ate **7a, 8a, 5, 6b** | 564.4 [M+H]⁺ |
| 167 | 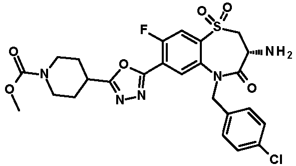 | methyl 4-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]piperidine-1-carboxylate (**) | 1-[(Benzylox y)carbonyl] piperidine-4-carboxylic acid, methyl chloroform ate, **7a, 8a, 5, 6b** | 578.2 [M+H]⁺ |
| 168 | 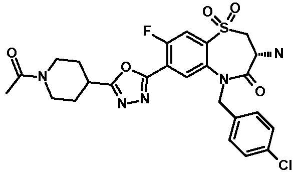 | (3R)-7-[5-(1-acetyl-4-piperidyl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one (**) | 1-[(Benzylox y)carbonyl] piperidine-4-carboxylic acid, aetic acid anhydride, **7a, 8a, 5, 6b** | 562.2 [M+H]⁺ |

| | | | | |
|---|---|---|---|---|
| * as a hydrochloride salt ** as free base after prep HPLC | | | | |

### Example 169

### (3R)-7-[5-(1-acetyl-4-methyl-4-piperidyl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one

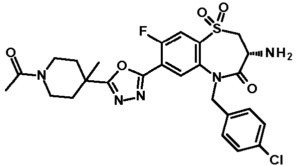

### Step a) tert-butyl N-[(3R)-7-[5-(1-acetyl-4-methyl-4-piperidyl)-1,3,4-oxadiazol-2-yl]-5-[(4-chlorophenyl)methyl]-8-fluoro-1,1,4-trioxo-2,3-dihydro-1λ⁶,5-benzothiazepin-3-yl]carbamate

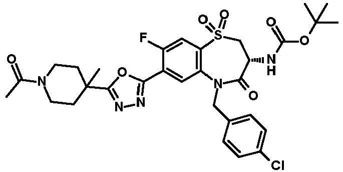

To a solution of tert-butyl N-[(3R)-5-[(4-chlorophenyl)methyl]-8-fluoro-7-[5-(4-methyl-4-piperidyl)-1,3,4-oxadiazol-2-yl]-1,1,4-trioxo-2,3-dihydro-1λ6,5-benzothiazepin-3-yl]carbamate (Example 161, step d)) (50 mg, 0.08 mmol) in DCM (3 mL) was added acetyl chloride (0.01 mL, 0.120 mmol, 1.5 eq) under N₂.Then the mixture was stirred at 25 °C for 2 h, concentrated in vacuo to afford the title compound (40 mg, 0.06 mmol, 75% yield) as colorless solid. MS (ESI): 676.2 [M+H]⁺.

### Step b) (3R)-7-[5-(1-acetyl-4-methyl-4-piperidyl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one

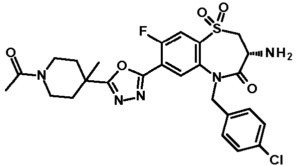

The title compound was prepared according to general procedure **6b** from tert-butyl N-[(3R)-7-[5-(1-acetyl-4-methyl-4-piperidyl)-1,3,4-oxadiazol-2-yl]-5-[(4-chlorophenyl)methyl]-8-fluoro-1,1,4-trioxo-2,3-dihydro-1λ⁶,5-benzothiazepin-3-yl]carbamate (40 mg, 0.06 mmol) and was obtained after chromatography on silica gel as a white solid as a hydrochloride salt (22.3 mg, 0.04 mmol, 60% yield). MS (ESI): 576.2 [M+H]⁺.

Examples 170 to 172 of the following table were prepared in analogy to Example 169 using the appropriate amine and acylating building block.

| **Ex.** | **Structure** | **Systematic Name** | **Building Block deprotection Method** | **MS, ESI: m/z** |
|---|---|---|---|---|
| 170 | 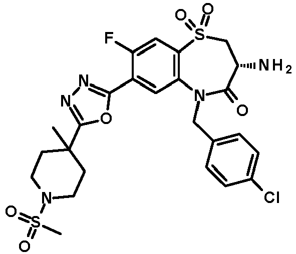 | (3R)-3-amino-5-[(4-chlorophenyl)methyl]-8-fluoro-1-[5-(4-methyl-1-methylsulfonyl-4-piperidyl)-1,3,4-oxadiazol-2-yl]-1,1-dioxo-2,3-dihydro-1λ⁶,5-benzothiazepin-4-one (***) | Example 161, step d), Methanes ulfonyl chloride, **6b** | 612.1 [M+H]⁺ |
| 171 | 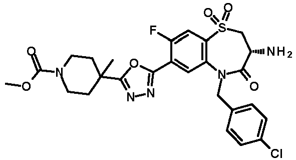 | methyl 4-methyl-4-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]piperidine-1-carboxylate (*) | Example 161, step d) Methyl chloroform ate, **6b** | 592.3 [M+H]⁺ |
| 172 | 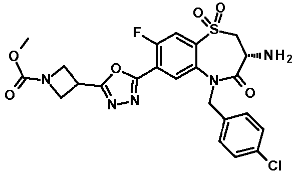 | methyl 3-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]azetidine-1-carboxylate (**) | Example 155, step d), Methyl chloroform ate **6e** | 550.1 [M+H]⁺ |

| | | | | |
|---|---|---|---|---|
| * as a hydrochloride salt ** as a trifluoroacetic acid salt *** as free base after prep HPLC | | | | |

### Example 173

### (3R)-7-[5-(3-acetyl-3-azabicyclo[3.1.1]heptan-1-yl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one

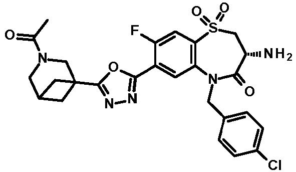

### Step a) tert-butyl 1-[[[(3R)-3-(tert-butoxycarbonylamino)-5-[(4-chlorophenyl)methyl]-8-fluoro-4-oxo-2,3-dihydro-1,5-henzothiazepine-7-carbonyl]amino]carbamoyl]-3-azabicyclo[3.1.1]heptane-3-carboxylate

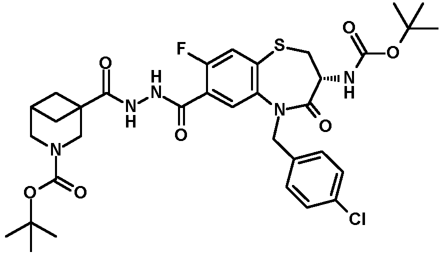

The title compound was prepared according to general procedure **7a** from tert-butyl N-[(3R)-7-[5-(1-acetyl-4-methyl-4-piperidyl)-1,3,4-oxadiazol-2-yl]-5-[(4-chlorophenyl)methyl]-8-fluoro-1,1,4-trioxo-2,3-dihydro-1λ⁶,5-benzothiazepin-3-yl]carbamate (500.0 mg, 1.0 mmol) and 3-tert-butoxycarbonyl-3-azabicyclo[3.1.1]heptane-1-carboxylic acid (243 mg, 1.0 mmol) and was obtained after chromatography on silica gel as a light yellow solid (730 mg, 1.0 mmol, 100% yield). MS (ESI): 618.3 [M-isobutene-CO₂+H]⁺.

### Step b) tert-butyl 1-[5-[(3R)-3-(tert-butoxycarbonylamino)-5-[(4-chlorophenyl)methyl]-8-fluoro-4-oxo-2, 3-dihydro-1,5-benzothiazepin-7-yl]-1,3,4-oxadiazol-2-yl]-3-azabicyclo[3.1.1]heptane-3-carboxylate

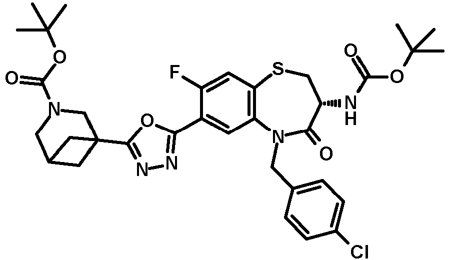

The title compound was prepared according to general procedure **8a** from tert-butyl 1-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoyl]-3-azabicyclo[3.1.1]heptane-3-carboxylate (730 mg, 1.0 mmol) in dioxane and was obtained after column chromatography on silica gel (PE:EA = 3:1 to 1:2) as a light yellow solid (570 mg, 0.810 mmol, 80% yield). MS (ESI): 700.3 [M+H]⁺.

### Step c) tert-butyl 1-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,3,4-oxadiazol-2-yl]-3-azabicyclo[3.1.1]heptane-3-carboxylate

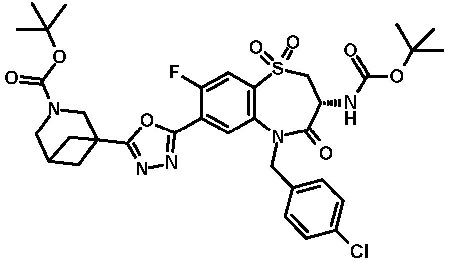

The title compound was prepared according to general procedure **5** tert-butyl 1-[5-[(3R)-3-(tertbutoxycarbonylamino)-5-[(4-chlorophenyl)methyl]-8-fluoro-4-oxo-2,3-dihydro-1,5-benzothiazepin-7-yl]-1,3,4-oxadiazol-2-yl]-3-azabicyclo[3.1.1]heptane-3-carboxylate (370.0 mg, 0.53 mmol) and was obtained as a light yellow solid (310 mg, 0.42 mmol, 80% yield). MS (ESI): 732.3 [M+H]⁺.

### Step d) (3R)-3-amino-7-[5-(3-azabicyclo[3.1.1]heptan-1-yl)-1,3,4-oxadiazol-2-yl]-5-[(4-chlorophenyl)methyl]-8-fluoro-1,1-dioxo-2,3-dihydro-1λ⁶,5-benzothiazepin-4-one

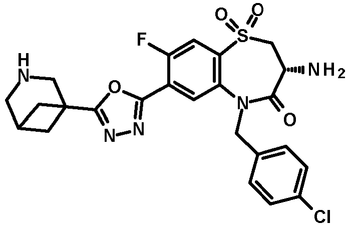

The title compound was prepared according to general procedure **6b** tert-butyl 1-[5-[(3R)-3-(tertbutoxycarbonylamino)-5-[(4-chlorophenyl)methyl]-8-fluoro-1,1,4-trioxo-2,3-dihydro-1λ⁶,5-benzothiazepin-7-yl]-1,3,4-oxadiazol-2-yl]-3-azabicyclo[3.1.1]heptane-3-carboxylate (150 mg, 0.2 mmol) and was obtained after prep-HPLC as a white solid (35.1 mg, 0.07 mmol, 31% yield). MS (ESI): 532.2 [M+H]⁺.

### Step e) (3R)-7-[5-(3-acetyl-3-azabicyclo[3.1.1]heptan-1-yl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one

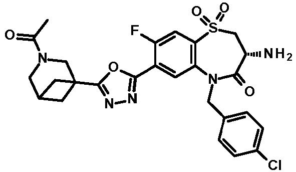

To a solution of (3R)-3-amino-7-[5-(3-azabicyclo[3.1.1]heptan-1-yl)-1,3,4-oxadiazol-2-yl]-5-[(4-chlorophenyl)methyl]-8-fluoro-1,1-dioxo-2,3-dihydro-1λ6,5-benzothiazepin-4-one (50 mg, 0.09 mmol) in DCM (1 mL) was added DIPEA (24 mg, 0.19 mmol, 2 eq). The mixture was purged with nitrogen and cooled to 0 °C, then acetic anhydride (9.6 mg, 0.09 mmol, 1 eq) (dilute with 1ml DCM) was added dropwise. The reaction was stirred at 20 °C for 12 h, poured into water (5 ml), extracted with EtOAc (5 ml x 3). The combined organic layer was dried over Na₂SO₄ and concentrated in vacuum and purified with prep-HPLC and lyophilized to get the title compound (24.7 mg, 0.04 mmol, 40% yield) as withe solid. MS (ESI): 574.2 [M+H]⁺.

Examples 174 to 184 of the following table were prepared in analogy to Example 173 using the appropriate carboxylic acid and acylating building block.

| **Ex.** | **Structure** | **Systematic Name** | **Building Block General Procedure** | **MS, ESI: m/z** |
|---|---|---|---|---|
| 174 | 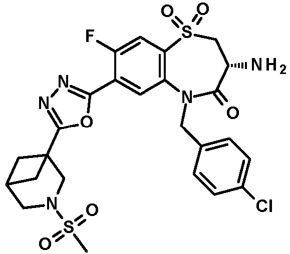 | (3R)-3-amino-5-[(4-chlorophenyl)methyl]-8-fluoro-7-[5-(3-methylsulfonyl-3-azabicyclo[3.1.1]heptan-1-yl)-1,3,4-oxadiazol-2-yl]-1,1-dioxo-2,3-dihydro-1λ⁶,5-benzothiazepin-4-one | 3-tert-Butoxycar bonyl-3-azabicyclo[ 3.1.1]hept ane-1-carboxylic acid. methanesu lfonyl chloride, **7a, 8a, 5, 6b** | 610.0 [M+H]⁺ |
| | | (**) | | |
| 175 | 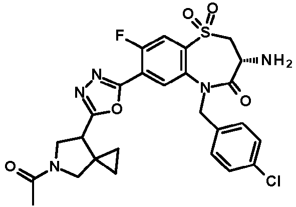 | (3R)-7-[5-(5-acetyl-5-azaspiro[2.4]heptan-7-yl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one | 5-tert-Butoxycar bonyl-5-azaspiro[2. 4]heptane-7-carboxylic acid, Ac₂O | 574.1 [M+H]⁺ |
| | | (**) | **7c, 8a, 5, 6b** | |
| 176 | 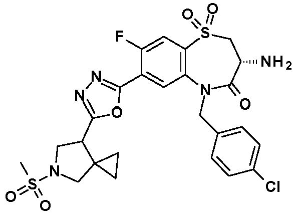 | (3R)-3-amino-5-[(4-chlorophenyl)methyl]-8-fluoro-7-[5-(5-methylsulfonyl-5-azaspiro[2.4]heptan-7-yl)-1,3,4-oxadiazol-2-yl]-1,1-dioxo-2,3-dihydro-1λ⁶,5-benzothiazepin-4-one | 5-tert-Butoxycar bonyl-5-azaspiro[2. 4]heptane-7-carboxylic acid, methanesulfonyl chloride | 610.2 [M+H]⁺ |
| | | (*) | **7c, 8a, 5, 6b** | |
| 177 | 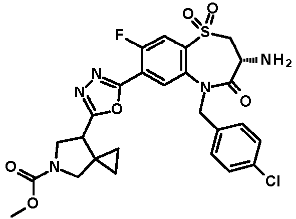 | methyl 7-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]-5-azaspiro[2.4]heptane-5-carboxylate | 5-tert-Butoxycar bonyl-5-azaspiro[2. 4]heptane-7-carboxylic acid, methyl chloroform ate | 590.0 [M+H]⁺ |
| | | (*) | | |
| | | | **7c, 8a, 5, 6b** | |
| 178 | 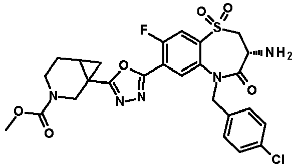 | methyl 1-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]-3-azabicyclo[4.1.0]heptane-3-carboxylate | 3-tert-Butoxycar bonyl-3-azabicyclo[ 4.1.0]hept ane-1-carboxylic acid, methyl chloroform ate,**7c, 8a, 5, 6e** | 590.0 [M+H]⁺ |
| | | (**) | | |
| 179 | 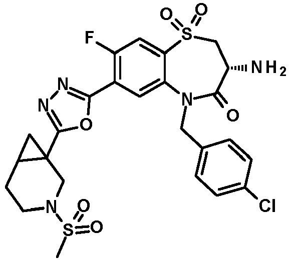 | (3R)-3-amino-5-[(4-chlorophenyl)methyl]-8-fluoro-7-[5-(3-methylsulfonyl-3-azabicyclo[4.1.0]heptan-1-yl)-1,3,4-oxadiazol-2-yl]-1,1-dioxo-2,3-dihydro-1λ⁶,5-benzothiazepin-4-one | 3-tert-Butoxycar bonyl-3-azabicyclo[ 4.1.0]hept ane-1-carboxylic acid, methanesu lfonyl chloride,**7c , 8a, 5, 6e** | 610.0 [M+H]⁺ |
| | | (*) | | |
| 180 | 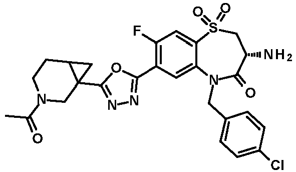 | (3R)-7-[5-(3-acetyl-3-azabicyclo[4.1.0]heptan-1-yl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one | 3-tert-Butoxycar bonyl-3-azabicyclo[ 4.1.0]hept ane-1-carboxylic acid, acetic anhydride, **7c, 8a, 5, 6e** | 574.2 [M+H]⁺ |
| | | (*) | | |
| 181 | 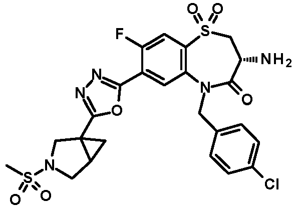 | (3R)-3-amino-5-[(4-chlorophenyl)methyl]-8-fluoro-7-[5-(3-methylsulfonyl-3-azabicyclo[3.1.0]hexan-1-yl)-1,3,4-oxadiazol-2-yl]-1,1-dioxo-2,3-dihydro-1λ⁶,5-benzothiazepin-4-one | 3-tert-Butoxycar bonyl-3-azabicyclo[ 3.1.0]hexane -1-carboxylic acid methanesulfonyl chloride, **7a, 8a, 5, 6b** | 596.3 [M+H]⁺ |
| | | (**) | | |
| 182 | 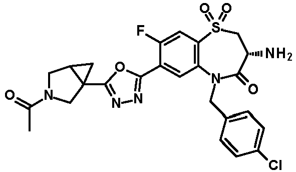 | (3R)-7-[5-(3-acetyl-3-azabicyclo[3.1.0]hexan-1-yl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one | 3-tert-Butoxycar bonyl-3-azabicyclo[ 3.1.0]hexa ne-1-carboxylic acid, acetic anhydride, **7a, 8a, 5, 6b** | 560.2 [M+H]⁺ |
| | | (**) | | |
| 183 | 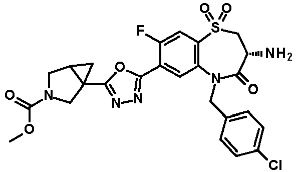 | methyl 1-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]-3-azabicyclo[3.1.0]hexane-3-carboxylate | 3-tert-Butoxycar bonyl-3-azabicyclo[ 3.1.0]hexa ne-1-carboxylic acid methyl chloroform ate, **7a, 8a, 5, 6b** | 576.3 [M+H]⁺ |
| | | (**) | | |
| 184 | 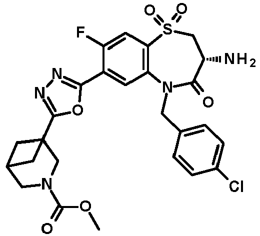 | methyl 1-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]-3-azabicyclo[3.1.1]heptane-3-carboxylate | 3-tert-Butoxycar bonyl-3-azabicyclo[ 3.1.1]hept ane-1-carboxylic acid. Methyl chloroform iate, **7a, 8a, 5, 6b** | 590.4 [M+H]⁺ |
| | | (**) | | |

| | | | | |
|---|---|---|---|---|
| * as a hydrochloride salt ** as free base after prep HPLC | | | | |

### Example 185

### (3R)-3-amino-5-[(4-chlorophenyl)methyl]-8-fluoro-1,1-dioxo-7-[5-(1,1,2,2,2-pentafluoroethyl)-1,3,4-oxadiazol-2-yl]-2,3-dihydro-1λ⁶,5-benzothiazepin-4-one

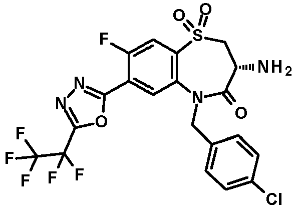

### Step a) tert-butyl N-[(3R)-8-fluoro-4-oxo-7-[5-(1, 1,2, 2, 2-pentafluoroethyl)-1, 3, 4-oxadiazol-2-yl]-3,5-dihydro-2H-1,5-benzothiazepin-3-yl]carbamate

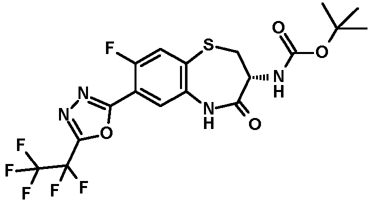

To a solution of tert-butyl N-[(3R)-8-fluoro-7-(hydrazinecarbonyl)-4-oxo-3,5-dihydro-2H-1,5-benzothiazepin-3-yl]carbamate (Intermediate 219b) (800 mg, 2.16 mmol) and NEt₃ (2.4 mL, 17.2 mmol, 8 eq) in DCM (16 mL) was added 2,2,3,3,3-pentafluoropropanoyl 2,2,3,3,3-pentafluoropropanoate (5357 mg, 17.2 mmol, 8 eq) at 0 °C. The reaction was stirred for 16 h at 25 °C, concentrated under vacuum, diluted with EtOAc (10 mL), washed with brine (10 mL x 2), dried over anhydrous Na₂SO₄, concentrated and purified by column chromatography on silica gel (PE:EA = 3:1 to 1:1) and by prep-HPLC to provide the title compound (130 mg, 0.26 mmol, 9% yield) as a white solid. MS (ESI): 443.0 [M-isobutene+H]⁺.

### Step b) tert-butyl N-[(3R)-8-fluoro-1,1,4-trioxo-7-[5-(1,1,2, 2, 2-pentafluoroethyl)-1, 3, 4-oxadiazol-2-yl]-3,5-dihydro-2H-1λ⁶,5-benzothiazepin-3-yl]carbamate

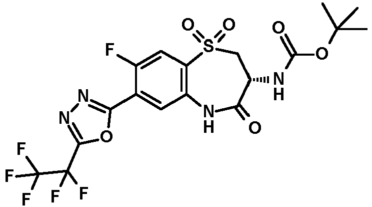

The title compound was prepared according to general procedure **5** from tert-butyl N-[(3R)-8-fluoro-4-oxo-7-[5-(1,1,2,2,2-pentafluoroethyl)-1,3,4-oxadiazol-2-yl]-3,5-dihydro-2H-1,5-benzothiazepin-3-yl]carbamate (130 mg, 0.26 mmol) and was obtained as a white solid (140 mg, 0.260 mmol, 95% yield). MS (ESI): 475.0 [M-isobutene+H]⁺.

### Step c) tert-butyl N-[(3R)-5-[(4-chlorophenyl)methyl]-8-fluoro-1,1,4-trioxo-7-[5-(1,1,2,2,2-pentafluoroethyl)-1, 3, 4-oxadiazol-2-yl]-2,3-dihydro-1λ⁶,5-benzothiazepin-3-yl]carbamate

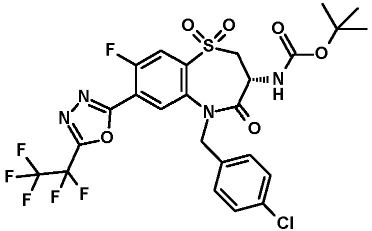

The title compound was prepared according to general procedure **4** from tert-butyl N-[(3R)-8-fluoro-1,1,4-trioxo-7-[5-(1,1,2,2,2-pentafluoroethyl)-1,3,4-oxadiazol-2-yl]-3,5-dihydro-2H-1λ⁶,5-benzothiazepin-3-yl]carbamate (48 mg, 0.09 mmol) and 4-chlorobenzyl bromide (15.81 mg, 0.080 mmol, 0.85 eq) and was obtained after prep-TLC (PE:EtOAc = 2: 1) as a white solid (40 mg, 0.06 mmol, 62% yield). MS (ESI): 598.9 [M-isobutene+H]⁺.

### Step d) (3R)-3-amino-5-[(4-chlorophenyl)methyl]-8-fluoro-1,1-dioxo-7-[5-(1,1,2,2,2-pentafluoroethyl)-1,3, 4-oxadiazol-2-yl]-2,3-dihydro-1λ⁶,5-benzothiazepin-4-one

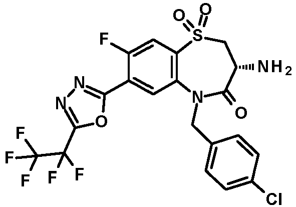

The title compound was prepared according to general procedure **6b** from tert-butyl N-[(3R)-5-[(4-chlorophenyl)methyl]-8-fluoro-1,1,4-trioxo-7-[5-(1,1,2,2,2-pentafluoroethyl)-1,3,4-oxadiazol-2-yl]-2,3-dihydro-1λ⁶,5-benzothiazepin-3-yl]carbamate (40 mg, 0.06 mmol) and was obtained after prep-HPLC as a white solid (2.7 mg, 7% yield). MS (ESI): 555.0 [M+H]⁺.

### Example 186

### (3R)-3-amino-7-(5-tert-butyl-1,3,4-oxadiazol-2-yl)-5-[(4-chlorophenyl)methyl]-8-fluoro-1-oxo-2,3-dihydro-1λ⁴,5-benzothiazepin-4-one

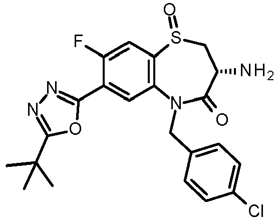

### Step a) tert-butyl N-[(3R)-7-(5-tert-butyl-1,3,4-oxadiazol-2-yl)-5-[(4-chlorophenyl)methyl]-8-fluoro-1,4-dioxo-2,3-dihydro-1λ⁴,5-benzothiazepin-3-yl]carbamate (Epimer A) and tert-butyl N-[(3R)-7-(5-tert-butyl-1,3,4-oxadiazol-2-yl]-5-[(4-chlorophenyl)methyl]-8-fluoro-1,4-dioxo-2,3-dihydro-1λ⁴,5-benzothiazepin-3-yl]carbamate (Epimer B)

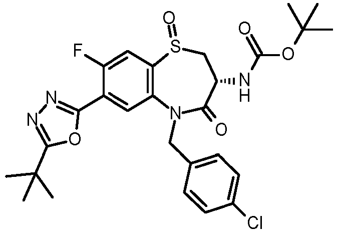

To a solution of tert-butyl N-[(3R)-7-(5-tert-butyl-1,3,4-oxadiazol-2-yl)-5-[(4-chlorophenyl)methyl]-8-fluoro-4-oxo-2,3-dihydro-1,5-benzothiazepin-3-yl]carbamate (Example 63, step b) (280 mg, 0.5 mmol) in DCM (5 mL) was added a solution of m-CPBA (53.8 mg, 0.25 mmol, 0.5 eq). The mixture was stirred at 20 °C for 16 h., diluted with Na₂SO₃ (25 mL), Na₂CO₃ (25 mL) and DCM (10 mL), washed with water (15 mL), brine (10 mL), dried over Na₂SO₄, filtered, concentrated and purified by column chromatography (EtOAc:PE = 0: 1 to 1:4) to afford the title compound (Epimer B) (120 mg, 0.21 mmol, 41% yield) and epimer A (140 mg, 0.24 mmol, 48% yield) as colorless gum. MS (ESI): 521.2 [M-isobuteneH]⁺.

### Step b) (3R)-3-amino-7-(5-tert-butyl-1,3,4-oxadiazol-2-yl)-5-[(4-chlorophenyl)methyl]-8-fluoro-1-oxo-2, 3-dihydro-1λ⁴,5-benzothiazepin-4-one (Epimer A)

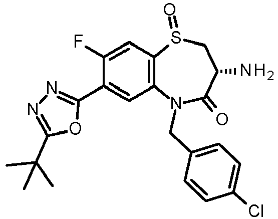

The title compound was prepared in analogy to general procedure **6b** from tert-butyl N-[(3R)-7-(5-tert-butyl-1,3,4-oxadiazol-2-yl)-5-[(4-chlorophenyl)methyl]-8-fluoro-1,4-dioxo-2,3-dihydro-1λ⁴,5-benzothiazepin-3-yl]carbamate (Epimer A) (120 mg, 0.2 mmol) and was obtained as a white solid (54.5 mg, 0.11 mmol, 54% yield). MS (ESI): 477.0 [M+H] ⁺.

Examples 187 of the following table was prepared in analogy to Example 186

| **Ex.** | **Structure** | **Systematic Name** | **Epimer** | **MS, ESI: m/z** |
|---|---|---|---|---|
| 187 | 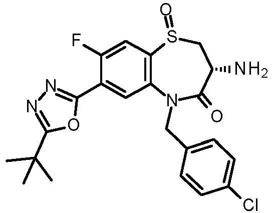 | (3R)-3-amino-1-(5-tert-butyl-1,3,4-oxadiazol-2-yl)-5-[(4-chlorophenyl)methyl]-8-fluoro-1-oxo-2,3-dihydro-1λ⁴,5-benzothiazepin-4-one (Epimer B) | B | 477.1 [M+H]⁺ |
| | | (*) | | |

| | | | | |
|---|---|---|---|---|
| * as a hydrochloride salt | | | | |

### Example 188

### 4-[[(3R)-3-amino-8-fluoro-7-[5-(2-hydroxy-1,1-dimethyl-ethyl)-1,3,4-oxadiazol-2-yl]-1,1,4-trioxo-2,3-dihydro-1λ⁶,5-benzothiazepin-5-yl]methyl] benzonitrile

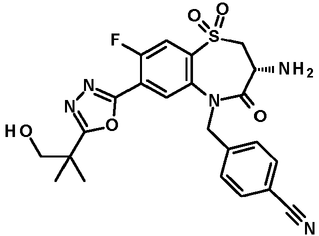

### Step a) methyl (3R)-3-(tert-butoxycarbonylamino)-5-[(4-cyanophenyl)methyl]-8-fluoro-4-oxo-2,3-dihydro-1,5-benzothiazepine-7-carboxylate

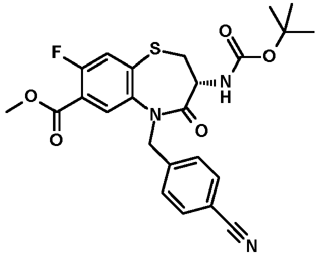

The title compound was prepared in analogy to general procedure **4** from 4-(bromomethyl)benzonitrile (291 mg, 1.48 mmol, 1.1 eq) and (3R)-3-(tert-butoxycarbonylamino)-8-fluoro-4-keto-3,5-dihydro-2H-1,5-benzothiazepine-7-carboxylic acid methyl ester (500 mg, 1.35 mmol) after column chromatography on silica gel (heptane:EtOAc = 1:0 to 0:1) as a light yellow solid (616 mg, 93% yield). MS (ESI): 430.3 [M-isobutene+H]⁺.

### Step b) (3R)-3-(tert-butoxycarbonylamino)-5-[(4-cyanophenyl)methyl]-8-fluoro-4-oxo-2,3-dihydro-1,5-benzothiazepine-7-carboxylic acid

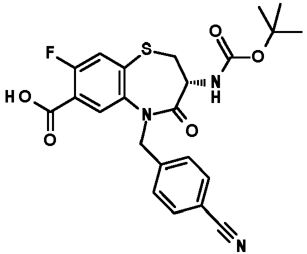

The title compound was prepared in analogy to general procedure **13** from (3R)-3-(tert-butoxycarbonylamino)-5-(4-cyanobenzyl)-8-fluoro-4-keto-2,3-dihydro-1,5-benzothiazepine-7-carboxylic acid methyl ester (616 mg, 1.27 mmol) as a light yellow solid (617 mg, 99% yield). MS (ESI): 470.4 [M-H]⁻.

### Step c) tert-butyl N-[(3R)-5-[(4-cyanophenyl)methyl]-8-fluoro-7-(hydrazinecarbonyl)-4-oxo-2,3-dihydro-1, 5-benzothiazepin-3-yl]carbamate

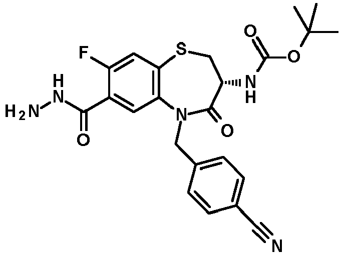

(3R)-3-(tert-butoxycarbonylamino)-5-(4-cyanobenzyl)-8-fluoro-4-keto-2,3-dihydro-1,5-benzothiazepine-7-carboxylic acid (567 mg, 1.15 mmol) and CDI (243 mg, 1.5 mmol, 1.3 eq) in THF(5 mL) was stirred at r.t. for 45 min. This solution was then slowly added to a second solution of hydrazine monohydrate (168 mg, 164 uL, 3.46 mmol, 3 eq) in THF (1.68 mL) and the reaction was stirred at r.t. for 30 min, diluted with EtOAc, washed with water and brine. The organic layer was dried over magnesium sulfate, filtered and concentrated in vacuo to the title compound (578 mg, 99% yield) as light yellow solid. MS (ESI): 430.3 [M-isobutene+H]⁺.

### Step d) tert-butyl N-[(3R)-5-[(4-cyanophenyl)methyl]-8-fluoro-7-[[(3-hydroxy-2,2-dimethylpropanoyl)amino]carbamoyl]-4-oxo-2,3-dihydro-1,5-benzothiazepin-3-yl]carbamate

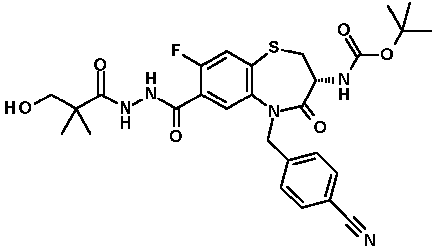

The title compound was prepared in analogy to general procedure **7a** from 3-hydroxy-2,2-dimethyl-propionic acid (40.1 mg, 0.34 mmol, 1.1 eq) and N-[(3R)-7-carbazoyl-5-(4-cyanobenzyl)-8-fluoro-4-keto-2,3-dihydro-1,5-benzothiazepin-3-yl]carbamic acid tert-butyl ester (150 mg, 0.31 mmol) as a light yellow solid (209 mg, 98% yield). MS (ESI): 584.5 [M-H]⁻.

### Step e) tert-butyl N-[(3R)-7-[[[3-[tert-butyl(dimethyl)silyl]oxy-2, 2-dimethylpropanoyl]amino]carbamoyl]-5-[(4-cyanophenyl)methyl]-8-fluoro-4-oxo-2,3-dihydro-1,5-benzothiazepin-3-yl]carbamate

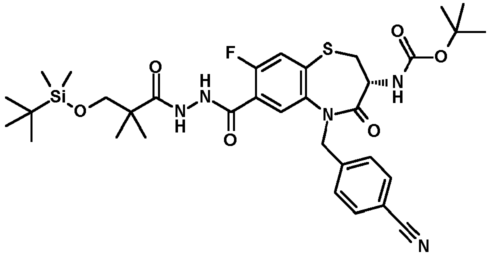

N-[(3R)-5-(4-cyanobenzyl)-8-fluoro-7-[[(3-hydroxy-2,2-dimethyl-propanoyl)amino]carbamoyl]-4-keto-2,3-dihydro-1,5-benzothiazepin-3-yl]carbamic acid tert-butyl ester (209 mg, 0.3 mmol), TBDMS-Cl (59 mg, 0.39 mmol, 1.3 eq) and imidazole (51.6 mg, 0.75 mmol, 2.5 eq) were in THF (3 mL) was stirred at RT for 12 h. Water was added and the reaction was dilluted with EtOAc. The organic layer was washed with water and brine, dried using magnesium sulfate, concentrated in vacuo an purified by column chromatography on silica gel (EtOAc in heptane, 0-100%) to yield the title compound (135 mg, 63%yield) as awhite solid MS (ESI): 498.6 [M-H]⁻.

### Step f) 4-[[(3R)-3-amino-8-fluoro-7-[5-(2-hydroxy-1,1-dimethyl-ethyl)-1, 3, 4-oxadiazol-2-yl]-1,1,4-trioxo-2,3-dihydro-1λ⁶,5-benzothiazepin-5-yl]methyl]benzonitrile

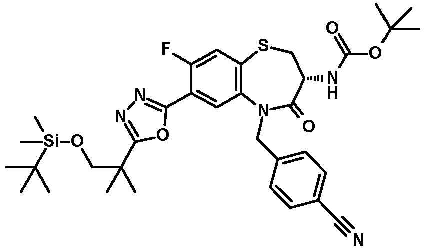

The title compound was prepared in analogy to general procedure **8a** from tert-butyl N-[(3R)-7-[[[3-[tert-butyl(dimethyl)silyl]oxy-2,2-dimethyl-propanoyl]amino]carbamoyl]-5-[(4-cyanophenyl)methyl]-8-fluoro-4-oxo-2,3-dihydro-1,5-benzothiazepin-3-yl]carbamate (135 mg, 0.19 mmol) as a light yellow solid (150 mg, 96% yield). MS (ESI): 682.5 [M+H]⁺.

### Step g) tert-butyl N-[(3R)-7-[5-[2-[tert-butyl(dimethyl)silyl]oxy-1,1-dimethyl-ethyl]-1,3,4-oxadiazol-2-yl]-5-[(4-cyanophenyl)methyl]-8-fluoro-1,1,4-trioxo-2,3-dihydro-1λ⁶,5-benzothiazepin-3-yl]carbamate

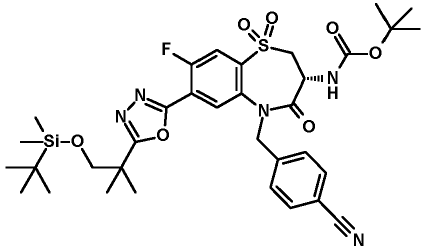

The title compound was prepared in analogy to general procedure **5** from tert-butyl N-[(3R)-7-[5-[2-[tert-butyl(dimethyl)silyl]oxy-1,1-dimethyl-ethyl]-1,3,4-oxadiazol-2-yl]-5-[(4-cyanophenyl)methyl]-8-fluoro-4-oxo-2,3-dihydro-1,5-benzothiazepin-3-yl]carbamate (150 mg, 0.187 mmol) as a white solid (42.4 mg, 31% yield). MS (ESI): 714.5 [[M+H]⁺.

### Step h) 4-[[(3R)-3-amino-8-fluoro-7-[5-(1-hydroxy-1-methyl-ethyl)-1,3,4-oxadiazol-2-yl]-1,1,4-trioxo-2,3-dihydro-1λ⁶,5-benzothiazepin-5-yl]methyl]benzonitrile

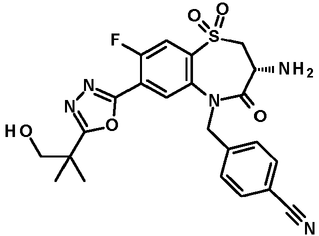

The title compound was prepared in analogy to general procedure **6d** from tert-butyl N-[(3R)-7-[5-[2-[tert-butyl(dimethyl)silyl]oxy-1,1-dimethyl-ethyl]-1,3,4-oxadiazol-2-yl]-5-[(4-cyanophenyl)methyl]-8-fluoro-1,1,4-trioxo-2,3-dihydro-1λ⁶,5-benzothiazepin-3-yl]carbamate (35 mg, 0.049 mmol) as a light yellow solid as a hydrogen chloride salt (10 mg, 34% yield). MS (ESI): 500.3 [[M+H]⁺.

### Example 189

### 3-[2,2-difluoro-12-(1H-pyrrol-2-yl)-1-aza-3-azonia-2-boranuidatricyclo[7.3.0.03,7]dodeca-3,5,7,9,11-pentaen-5-yl]-N-[11-[4-[[(3R)-3-amino-7-(5-tert-butyl-1,3,4-oxadiazol-2-yl)-8-fluoro-1,1,4-trioxo-2,3-dihydro-1λ⁶,5-benzothiazepin-5-yl]methyl]phenoxy]undecyl]propanamide

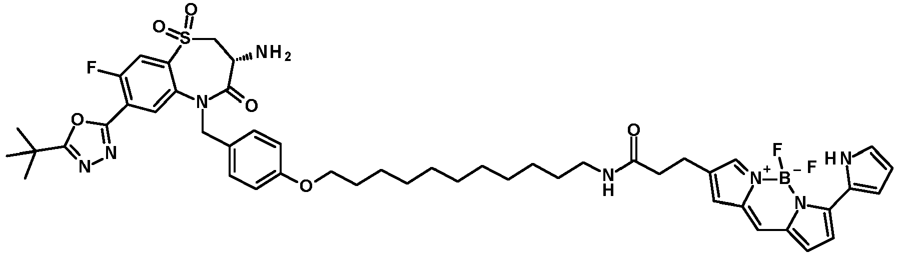

### Step a) benzyl N-[11-[4-(hydroxymethyl)phenoxy]undecyl]carbamate

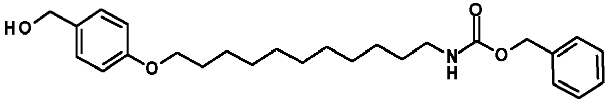

4-hydroxybenzyl alcohol (150 mg, 1.2 mmol, 1 eq), benzyl N-(11-bromoundecyl)carbamate (510 mg, 1.33 mmol, 1.1 eq) and potassium carbonate (360 mg, 2.6 mmol, 2.16 eq) in MeCN (8 mL) was stirred for 6 h at 80 °C. The mixture was poured into water (60 mL), and extracted with EtOAc (50 mL x 2), the combined organic layers were washed with brine (40 mL), dried over Na₂SO₄, filtered and concentrated in vacuo. The residue was purified by column chromatography on silica gel (PE:EA = 1:0 to 3:1) to afford the title compound (410 mg, 0.96 mmol, 76% yield) as a light yellow solid. MS (ESI): 410.2 [M-OH]⁺.

### Step b) [4-[11-(benzyloxycarbonylamino)undecoxy]phenyl]methyl methanesulfonate

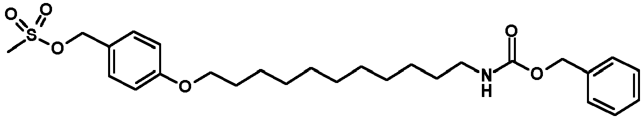

To a mixture of benzyl N-[11-[4-(hydroxymethyl)phenoxy]undecyl]carbamate (300 mg, 0.7 mmol) and DIPEA (0.3 mL, 1.72 mmol, 2.45 eq) in DCM (8 mL) was added methanesulfonyl chloride (0.2 mL, 2.62 mmol, 3.73 eq) at 0 °C, and then the mixture was stirred at 25 °C for 1 h. DCM (40 mL) was added to the mixture. The organic layer was washed water (20 mL), brine (20 mL), dired over Na₂SO₄, filtered and concentrated in vacuum to give the crude title compound (280 mg, 0.55 mmol, 78% yield) as yellow oil. MS (ESI): 410.2 [M-MeSO₃]⁺.

### Step c) tert-butyl N-[(3R)-5-[[4-[11-(benzyloxycarbonylamino)undecoxy]phenyl]methyl]-7-(5-tert-butyl-1,3,4-oxadiazol-2-yl)-8-fluoro-4-oxo-2,3-dihydro-1,5-benzothiazepin-3-yl]carbamate

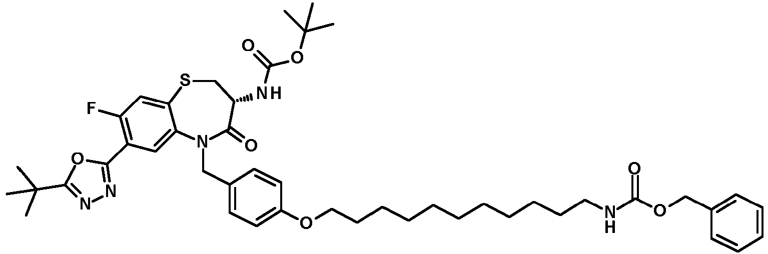

The title compound was prepared in analogy to general procedure **4** from tert-butyl N-[(3R)-7-(5-tert-butyl-1,3,4-oxadiazol-2-yl)-8-fluoro-4-oxo-3,5-dihydro-2H-1,5-benzothiazepin-3-yl]carbamate (120 mg, 0.27 mmol) and [4-[11-(benzyloxycarbonylamino)undecoxy]phenyl]methyl methanesulfonate (280 mg, 0.55 mmol, 2. eq) after prep-TLC (PE:EA= 2:1) as a colorless oil (160 mg, 0.19 mmol, 63% yield). MS (ESI): 846.4 [M+H]⁺.

### Step d) tert-butyl N-[(3R)-5-[[4-[11-(benzyloxycarbonylamino)undecoxy]phenyl]methyl]-7-(5-tert-butyl-1, 3, 4-oxadiazol-2-yl)-8-fluoro-11,1,4-trioxo-2,3-dihydro-1λ⁶,5-benzothiazepin-3-yl]carbamate

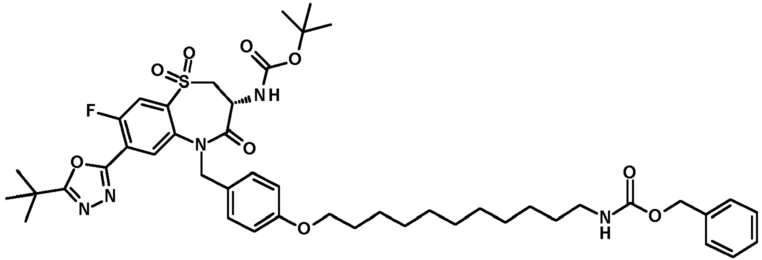

The title compound was prepared in analogy to general procedure **5** from tert-butyl N-[(3R)-5-[[4-[11-(benzyloxycarbonylamino)undecoxy]phenyl]methyl]-7-(5-tert-butyl-1,3,4-oxadiazol-2-yl)-8-fluoro-4-oxo-2,3-dihydro-1,5-benzothiazepin-3-yl]carbamate (160 mg, 0.19 mmol) after prep-TLC (PE:EA= 3:1) as a colorless oil (150 mg, 0.170 mmol, 88% yield). MS (ESI): 878.3 [M+H]⁺.

### Step e) tert-butyl N-[(3R)-5-[[4-(11-aminoundecoxy)phenyl]methyl]-7-(5-tert-butyl-1,3,4-oxadiazol-2-yl)-8-fluoro-1,1,4-trioxo-2,3-dihydro-1λ⁶,5-benzothiazepin-3-yl]carbamate

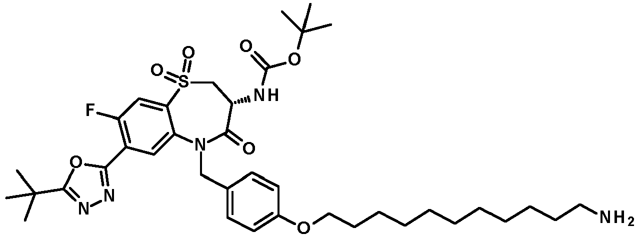

tert-butyl N-[(3R)-5-[[4-[11-(benzyloxycarbonylamino)undecoxy]phenyl]methyl]-7-(5-tert-butyl-1,3,4-oxadiazol-2-yl)-8-fluoro-1,1,4-trioxo-2,3-dihydro-1λ6,5-benzothiazepin-3-yl]carbamate (120 mg, 0.14 mmol) and Pd/C (10 mg) in MeOH (4 mL) was strried at 25 °C for 2 h under H₂ (15.0 Psi) atmosphere. The mixture was filtered and the filtrate was concentrated in vacuo. The residue was purified by prep-HPLC. Lyophilization afforded the title compound as a hydrochloride salt (18.4 mg, 0.02 mmol, 17% yield) as a white solid. MS (ESI): 744.3 [M+H]⁺.

### Step f) (3R)-3-amino-5-[[4-(11-aminoundecoxy)phenyl]methyl]-7-(5-tert-butyl-1,3,4-oxadiazol-2-yl)-8-fluoro-1,1-dioxo-2,3-dihydro-1λ⁶,5-benzothiazepin-4-one

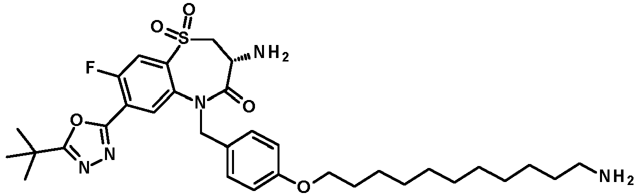

The title compound was prepared in analogy to general procedure **7d** from N-[(3R)-5-[4-(11-aminoundecoxy)benzyl]-7-(5-tert-butyl-1,3,4-oxadiazol-2-yl)-8-fluoro-1,1,4-triketo-2,3-dihydro-1λ6,5-benzothiazepin-3-yl]carbamic acid tert-butyl ester hydrochloride salt (5 mg, 0.006 mmol) after prep-HPLC as a white solid (5.2 mg, 126% yield). MS (ESI): 644.5 [M+H]⁺.

### Step g) 3-[2,2-difluoro-12-(1H-pyrrol-2-yl)-1-aza-3-azonia-2-boranuidatricyclo[7.3.0.03,7]dodeca-3,5,7,9,11-pentaen-5-yl]-N-[11-[4-[[(3R)-3-amino-7-(5-tert-butyl-1, 3, 4-oxadiazol-2-yl)-8-fluoro-11,1,4-trioxo-2, 3-dihydro-1λ⁶,5-benzothiazepin-5-yl]methyl]phenoxy]undecyl]propanamide

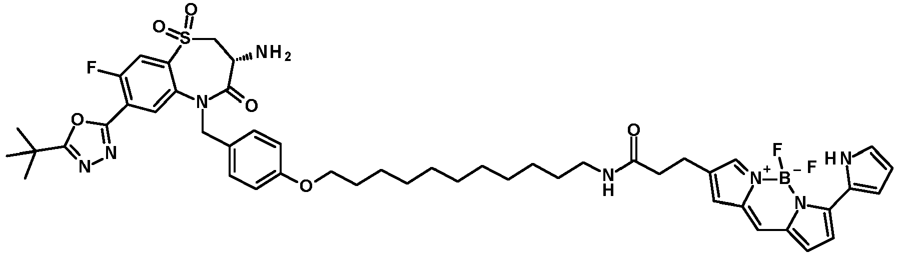

To (3R)-3-amino-5-[4-(11-aminoundecoxy)benzyl]-7-(5-tert-butyl-1,3,4-oxadiazol-2-yl)-8-fluoro-1,1-diketo-2,3-dihydro-1λ6,5-benzothiazepin-4-one (1.3 mg, 0.002 mmol) and 3-[2,2-difluoro-12-(1H-pyrrol-2-yl)-1-aza-3-azonia-2-boranuidatricyclo[7.3.0.03,7]dodeca-3,5,7,9,11-pentaen-5-yl]propionic acid succinimido ester (1.2 mg, 0.003 mmol, 1.39 eq) was added DMF (0.05 mL). The reaction was stirred overnight at room temperature. The reaction was purified by prep-HPLC affording the title compound (0.66 mg, 23%) as purple solid. MS (ESI): 955.6 [M+H]⁺.

### Example 190

### (2R,3R)-3-amino-7-(5-tert-butyl-1,3,4-oxadiazol-2-yl)-5-[(4-chlorophenyl)methyl]-8-fluoro-2-methyl-1,1-dioxo-2,3-dihydro-1λ⁶,5-benzothiazepin-4-one

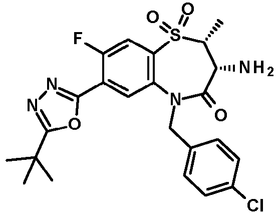

### Step a) tert-butyl N-[(2R,3R)-5-[(4-chlorophenyl)methyl]-8-fluoro-7-(hydrazinecarbonyl)-2-methyl-4-oxo-2,3-dihydro-1,5-benzothiazepin-3-yl]carbamate

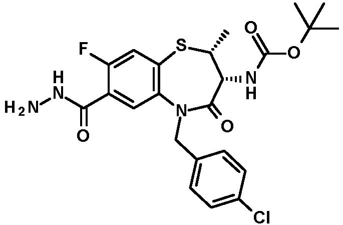

The title compound was prepared in analogy to general procedure **14** from (2R,3R)-3-(tert-butoxycarbonylamino)-5-[(4-chlorophenyl)methyl]-8-fluoro-2-methyl-4-oxo-2,3-dihydro-1,5-benzothiazepine-7-carboxylic acid (CAS 2002451-02-5) (258 mg, 0.52 mmol) and was obtained as a light yellow foam (269 mg, 99% yield). MS (ESI): 453.1 [M-isobutene+H]⁺.

### Step b) tert-butyl N-[(2R,3R)-5-[(4-chlorophenyl)methyl]-7-[(2, 2-dimethylpropanoylamino)carbamoyl]-8-fluoro-2-methyl-4-oxo-2,3-dihydro-1,5-benzothiazepin-3-yl]carbamate

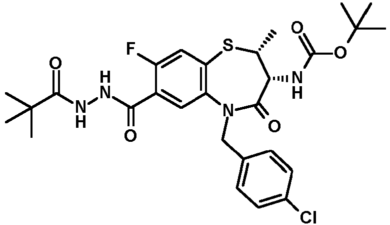

The title compound was prepared in analogy to general procedure **7a** from *tert-butyl N-[(2R,*3R)-*5-[(4-chlorophenyl)methyl]-8-fluoro-7-(hydrazinecarbonyl)-2-methyl-4-oxo-2,3-dihydro-1,5-benzothiazepin-3-yl]carbamate* (50 mg, 0.096 mmol) using pivalic acid (CAS 75-989) (10.8 mg, 0.106 mmol) and was obtained as a white solid (47 mg, 77% yield) MS (ESI): 493.2 [M-isobutene-CO₂+H]⁺.

### Step c) tert-butyl N-[(2R,3R)-7-(5-tert-butyl-1, 3, 4-oxadiazol-2-yl)-5-[(4-chlorophenyl)methyl]-8-fluoro-2-methyl-4-oxo-2, 3-dihydro-1, 5-benzothiazepin-3-yl]carbamate

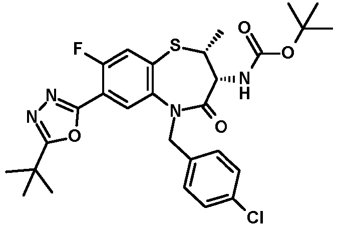

The title compound was prepared in analogy to general procedure **8a** from *tert-butyl N-[(2R,*3R)-*5-[(4-chlorophenyl)methyl]-7-[(2,2-dimethylpropanoylamino)carbamoyl]-8-fluoro-2-methyl-4-oxo-2,3-dihydro-1,5-benzothiazepin-3-yl]carbamate* (50 mg, 0.078 mmol) and was obtained as a white solid (32 mg, 70% yield). MS (ESI): 575.3 [M+H]⁺.

### Step d) tert-butyl N-[(2R, 3R)-7-(5-tert-butyl-1,3, 4-oxadiazol-2yl)-5-[(4-chlorophenyl)methyl]-8-fluoro-2-methyl-1,1, 4-trioxo-2, 3-dihydro-1λ⁶,5-benzothiazepin-3-yl]carbamate

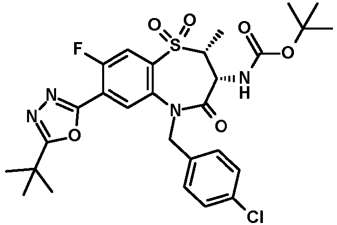

The title compound was prepared in analogy to general procedure **9b** from tert-butyl N-[(2R,3R)-7-(5-tert-butyl-1,3,4-oxadiazol-2-yl)-5-[(4-chlorophenyl)methyl]-8-fluoro-2-methyl-4-oxo-2,3-dihydro-1,5-benzothiazepin-3-yl]carbamate (32 mg, 0.056 mmol) and was obtained as a white solid (28 mg, 81% yield). MS (ESI): 551.3 [M-isobutene+H]⁺.

### Step e) (2R, 3R)-3-amino-7-(5-tert-butyl-1, 3, 4-oxadiazol-2-yl)-5-[(4-chlorophenyl)methyl]-8-fluoro-2-methyl-1,1-dioxo-2,3-dihydro-1λ⁶,5-benzothiazepin-4-one

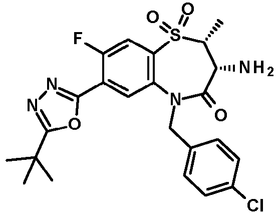

The title compound was prepared in analogy to general procedure **6d** from *tert-butyl N-[(2R, 3R)-7-(5-tert-butyl-1,3,4-oxadiazol-2-yl)-5-[(4-chlorophenyl)methyl]-8-fluoro-2-methyl-1,1,4-trioxo-2,3-dihydro-1λ⁶,5*-benzothiazepin-3-yl]carbamate (28 mg, 0.045 mmol) and was obtained as a white solid (25 mg, 78% yield) as a hydrochloride salt and 1,1,1,3,3,3-hexafluoropropan-2-ol adduct. MS (ESI): 507.3 [M+H]⁺.

### Example 191

### (3R)-3-amino-7-[5-(azetidin-1-yl)-1,3,4-oxadiazol-2-yl]-5-[(4-chlorophenyl)methyl]-8-fluoro-1,1-dioxo-2,3-dihydro-1λ⁶,5-benzothiazepin-4-one

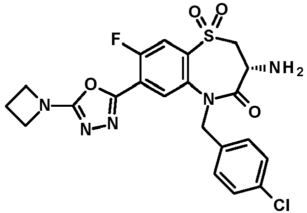

### Step a) tert-butyl N-[(3R)-5-[(4-chlorophenyl)methyl]-8-fluoro-7-(5-methylsulfanyl-1,3,4-oxadiazol-2 yl)-4-oxo-2,3-dihydro-1,5-benzothiazepin-3-yl]carbamate

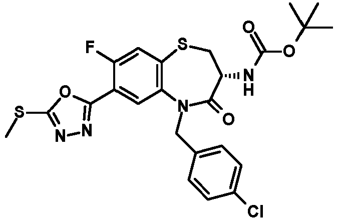

To a solution of tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (Example 63, step a)) (1000.0 mg, 2.02 mmol, 1 eq) in DMF (20 mL) was added CS₂ (0.37 mL, 6.06 mmol, 3 eq) at 25°C. The reaction mixture was stirred for 15 min under an atmosphere of nitrogen at 25°C and then heated to 70 °C for 4 h. After the mixture was cooled to 25°C, TEA (1.12 mL, 8.08 mmol, 4 eq) and MeI (0.15 mL, 2.42 mmol, 1.2 eq) were added and the reaction mixture was stirred for 16 h at 25 °C. The mixture was diluted with EtOAc (25 mL) and washed with brine (3 x 25 mL), dried over anhydrous Na₂SO₄ and concentrated to give crude product (1 g) which was purified by column chromatography on silica gel (PE:EtOAc 5:1 to 2:1) to give the title compound (650 mg, 1.18 mmol, 58% yield) as a light yellow solid. MS (ESI): 495.1 [M-isobutene+H]⁺.

### Step b) tert-butyl N-[(3R)-5-[(4-chlorophenyl)methyl]-8-fluoro-7-(5-methylsulfonyl-1,3,4-oxadiazol-2-yl)-1,1,4-trioxo-2,3-dihydro-1λ⁶,5-benzothiazepin-3-yl]carbamate

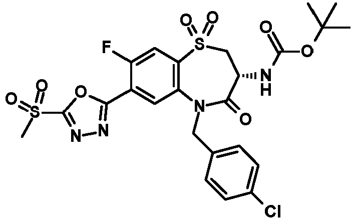

A solution of KMnO₄ (476.67 mg, 3.02 mmol, 5.04 eq) in water (2.02 mL) was added dropwise to a solution of tert-butyl N-[(3R)-5-[(4-chlorophenyl)methyl]-8-fluoro-7-(5-methylsulfanyl-1,3,4-oxadiazol-2-yl)-4-oxo-2,3-dihydro-1,5-benzothiazepin-3-yl]carbamate (330.0 mg, 0.600 mmol, 1 eq) in a mixture of acetic acid (6.72 mL) and water (4.03 mL) at 0°C under an atmosphere of nitrogen. The mixture was stirred for 2 h, keeping the internal temperature below 5°C. The reaction mixture was poured into a cold saturated aqueous Na₂SO₃ solution (50 mL). Then the mixture was extracted with EtOAc (2 x 20 mL) and the combined organic layers were washed with brine (2 x 40 mL), dried over anhydrous Na₂SO₄ and concentrated to give a crude product (400 mg) which was purified by prep-HPLC. The organic solvent was removed under vacuum and the title compound was obtained after freeze drying (140 mg, 0.230 mmol, 34% yield) as a white solid. MS (ESI): 559.2 [M-isobutene+H]⁺.

### Step c) tert-butyl N-[(3R)-7-[5-(azetidin-1-yl)-1, 3, 4-oxadiazol-2yl]-5-[(4-chlorophenyl)methyl]-8-fluoro-1,1,4-trioxo-2,3-dihydro-1λ⁶,5-benzothiazepin-3-yl]carbamate

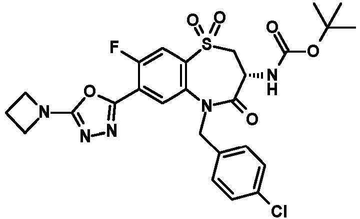

A mixture of tert-butyl N-[(3R)-5-[(4-chlorophenyl)methyl]-8-fluoro-7-(5-methylsulfonyl-1,3,4-oxadiazol-2-yl)-1,1,4-trioxo-2,3-dihydro-1λ⁶,5-benzothiazepin-3-yl]carbamate (120.0 mg, 0.200 mmol, 1 eq), azetidine (0.02 mL, 0.23 mmol, 1.2 eq) and K₂CO₃ (32.36 mg, 0.23 mmol, 1.2 eq) in DMF (12 mL) was stirred for 1 h at 25°C. The reaction mixture was diluted with EtOAc (15 mL), washed with brine (3 x 10 mL), dried over anhydrous Na₂SO₄ and concentrated to give crude product (150 mg) which was purified by prep-HPLC. The organic solvent was removed under vacuum and the title compound was obtained after freeze drying as a white solid (30 mg, 0.050 mmol, 26% yield). MS (ESI): 592.3 [M+H]⁺.

### Step d) (3R)-3-amino-7-[5-(azetidin-1-yl)-1,3,4-oxadiazol-2-yl]-5-[(4-chlorophenyl)methyl]-8-fluoro-1,1-dioxo-2,3-dihydro-1λ⁶,5-benzothiazepin-4-one

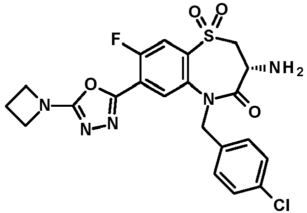

To a solution of tert-butyl N-[(3R)-7-[5-(azetidin-1-yl)-1,3,4-oxadiazol-2-yl]-5-[(4-chlorophenyl)methyl]-8-fluoro-1,1,4-trioxo-2,3-dihydro-1λ⁶,5-benzothiazepin-3-yl]carbamate (30.0 mg, 0.050 mmol, 1 eq) in DCM (1 mL) were added TMSOTf (0.03 mL, 0.150 mmol, 3 eq) and 2,6-lutidine (0.01 mL, 0.130 mmol, 2.5 eq) and the mixture was stirred for 2 h at 25°C. The mixture was concentrated under vacuum and the remaining residue was purified by prep-HPLC (column: Phenomenex Gemini NX-C18<75*30mm*3um>; mobile phase:[water<10mM NH₄HCO₃ >-MeCN];B%: 22%-52%,8min) followed by freeze drying to give the title compound (13.5 mg, 0.030 mmol, 52% yield) as a white solid. MS (ESI): 492.2 [M+H]⁺.

### Example 192

### (3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1,1-dioxo-2,3-dihydro-1λ⁶,5-benzothiazepin-4-one

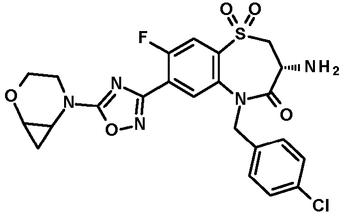

### Step a) tert-butyl N-[(3R)-5-[(4-chlorophenyl)methyl]-8-fluoro-4-oxo-7-[(Z)-N'-hydroxycarbamimidoyl]-2, 3-dihydro-1, 5-benzothiazepin-3yl]carbamate

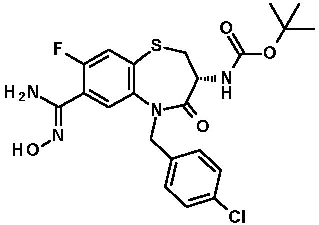

To a solution of N-[(3R)-5-(4-chlorobenzyl)-7-cyano-8-fluoro-4-keto-2,3-dihydro-1,5-benzothiazepin-3-yl]carbamic acid tert-butyl ester (419 mg, 0.83 mmol, 1 eq) in MeOH (4.17 mL) was added hydroxylamine hydrochloride (89.6 mg, 1.25 mmol, 1.5 eq) followed by NaHCO₃ (350.5 mg, 4.17 mmol, 5 eq). The reaction mixture was heated to reflux for 90 min, cooled to room temperature, filtered and the filter cake was further washed with DCM. The filtrate was then concentrated in vacuo and the residue was taken up in DCM and washed with water, brine, dried over Na₂SO₄, filtered and concentrated in vacuo to afford the title compound (215 mg, 98% yield) as white solid (purity 89%). MS (ESI): 539.3 [M-H+HCO₂H]⁻.

### Step b) tert-butyl N-[(3R)-5-[(4-chlorophenyl)methyl]-8-fluoro-4-oxo-7-(5-oxo-4H-1,2,4-oxadiazol-3-yl)-2,3-dihydro-1,5-benzothiazepin-3-yl]carbamate

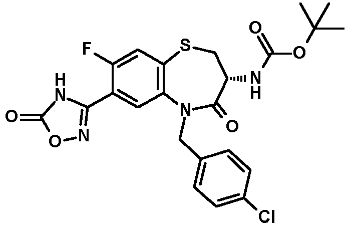

To a solution of tert-butyl N-[(3R)-5-[(4-chlorophenyl)methyl]-8-fluoro-4-oxo-7-[(Z)-N'-hydroxycarbamimidoyl]-2,3-dihydro-1,5-benzothiazepin-3-yl]carbamate (100 mg, 0.18 mmol, 1 eq) in THF (1.86 mL) was added CDI (31.2 mg, 0.19 mmol, 1.06 eq) and triethylamine (19.5 mg, 26.7 uL, 0.19 mmol, 1.06 eq) at room temperature. The reaction mixture was heated to 70 °C and stirred for 3 hours and after cooling taken up in EtOAc (30mL), washed with 1N aqueous HCl solution (20ml) and brine (50ml), dried over sodium sulfate, filtered and evaporated to afford the title compound (117 mg, 92% yield) as light brown solid (purity 75%). MS (ESI): 519.4 [M-H]⁻.

### Step c) tert-butyl N-[(3R)-5-[(4-chlorophenyl)methyl]-8-fluoro-7-[5-(2-oxa-5-azabicyclo[4.1.0]heptan-5-yl)-1,2,4-oxadiazol-3-yl]-4-oxo-2,3-dihydro-1,5-benzothiazepin-3-yl]carbamate

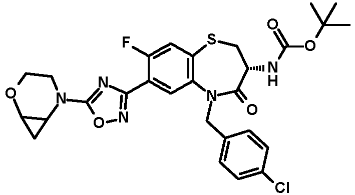

To a solution of tert-butyl N-[(3R)-5-[(4-chlorophenyl)methyl]-8-fluoro-4-oxo-7-(5-oxo-4H-1,2,4-oxadiazol-3-yl)-2,3-dihydro-1,5-benzothiazepin-3-yl]carbamate (33 mg, 0.06 mmol, 1 eq) in 1,4-dioxane (1.03 mL) was added 2-oxa-5-azabicyclo[4.1.0]heptane hydrochloride (19.5 mg, 0.14 mmol, 2 eq), DIPEA (37.2 mg, 50.2 uL, 0.29 mmol, 4 eq) and bromotripyrrolidinophosphonium hexafluorophosphate (42.4 mg, 0.086 mmol, 1.2 eq). The mixture was heated to 50 °C for 90 min. The reaction was cooled to RT and diluted with EtOAc and water and stirred vigorously. The phases were separated and the aqueous phase washed twice with EtOAc. The combined organic phases were dried over Na₂SO₄, filtered, and concentrated in vacuo. The crude mixture was purified by column chromatography on silica gel (EtOAc:heptanes gradient) to afford the desired title compound (36 mg, 76% yield) as white solid (purity 91%). MS (ESI): 546.3 [M+H-isobutene]⁺.

### Step d) tert-butyl N-[(3R)-5-[(4-chlorophenyl)methyl]-8-fluoro-7-[5-(2-oxa-5-azabicyclo[4.1.0]heptan-5-yl)-1, 2, 4-oxadiazol-3-yl]-1,1,4-trioxo-2, 3-dihydro-1λ⁶,5-benzothiazepin-3-yl]carbamate

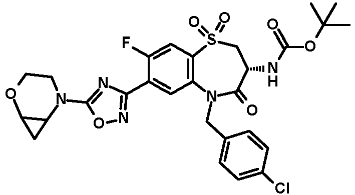

tert-butyl N-[(3R)-5-[(4-chlorophenyl)methyl]-8-fluoro-7-[5-(2-oxa-5-azabicyclo[4.1.0]heptan-5-yl)-1,2,4-oxadiazol-3-yl]-4-oxo-2,3-dihydro-1,5-benzothiazepin-3-yl]carbamate (36 mg, 0.054 mmol) was stirred with 3-chloroperoxybenzoic acid (36.5 mg, 0.16 mmol, 3 eq) in DCM (1.09 mL) at room temperature for 2.5 hours. The reaction solution was then diluted with DCM and 1N aqueous NaOH was added (washed 2x with DCM). The organic layers were washed with water, brine, dried over Na₂SO₄, filtered and evaporated to give crude product, which was purified by column chromatography on silica gel (EtOAc:heptanes gradient) to afford the desired title compound (28 mg, 81% yield) as white solid (purity 100%). MS (ESI): 632.5 [M-H]⁻.

### Step e) (3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1,1-dioxo-2,3-dihydro-1λ⁶,5-benzothiazepin-4-one

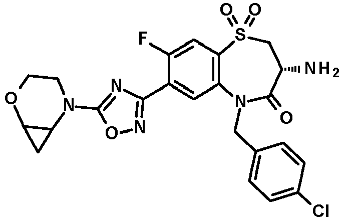

2 M HCl in Et₂O (39.4 uL, 0.079 mmol, 2 eq) was stirred tert-butyl N-[(3R)-5-[(4-chlorophenyl)methyl]-8-fluoro-7-[5-(2-oxa-5-azabicyclo[4.1.0]heptan-5-yl)-1,2,4-oxadiazol-3-yl]-1,1,4-trioxo-2,3-dihydro-1λ⁶,5-benzothiazepin-3-yl]carbamate (25 mg, 0.039 mmol, 1 eq) in 1,1,1,3,3,3-hexafluoro-2-propanol (1.31 mL) at room temperature for 40 min. The solvent was evaporated in vacuo. The solid was suspended in DCM, sonicated for 30 seconds and again concentrated. This process was repeated twice then dried in vacuo to afford the title compound (21 mg, 91% yield) as white solid (purity 100%). MS (ESI): 534.3 [M+H]⁺.

Example 193 to 199 of the following table was prepared in analogy to Example 192 in three steps, using the appropriate amine building block.

| **Ex.** | **Structure** | **Systematic Name** | **Building Block** | **MS, ESI: m/z** |
|---|---|---|---|---|
| 193 | 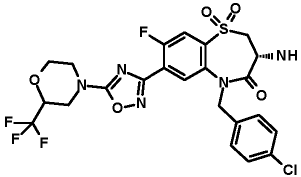 | (3R)-3-amino-5-[(4-chlorophenyl)methyl]-8-fluoro-1,1-dioxo-7-[5-[2-(trifluoromethyl)morpholin-4-yl]-1,2,4-oxadiazol-3-yl]-2,3-dihydro-1λ⁶,5-benzothiazepin-4-one (*) [EPIMERS 1:1] | 2-(Trifluoro methyl)mo rpholine hydrochloride) | 590.3 [M+H]⁺ |
| 194 | 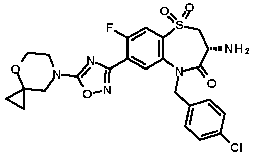 | (3R)-3-amino-5-[(4-chlorophenyl)methyl]-8-fluoro-7-[5-(4-oxa-7-azaspiro[2.5]octan-7-yl)-1,2,4-oxadiazol-3-yl]-1,1-dioxo-2,3-dihydro-1λ⁶,5-benzothiazepin-4-one (*) | 4-Oxa-7-azaspiro[2. 5]octane hydrochloride | 548.1 [M+H]⁺ |
| 195 | 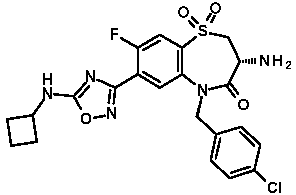 | (3R)-3-amino-5-[(4-chlorophenyl)methyl]-7-[5-(cyclobutylamino)-1,2,4-oxadiazol-3-yl]-8-fluoro-1,1-dioxo-2,3-dihydro-1λ⁶,5-benzothiazepin-4-one (*) | Cyclobutylamine | 506.1 [M+H]⁺ |
| 196 | 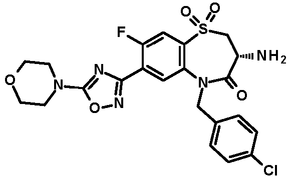 | (3R)-3-amino-5-[(4-chlorophenyl)methyl]-8-fluoro-7-(5-morpholino-1,2,4-oxadiazol-3-yl)-1,1-dioxo-2,3-dihydro-1λ⁶,5-benzothiazepin-4-one (*) | Morpholine | 522.1 [M+H]⁺ |
| 197 | 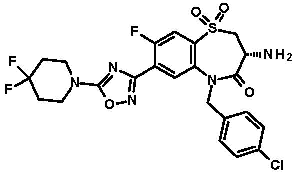 | (3R)-3-amino-5-[(4-chlorophenyl)methyl]-7-[5-(4,4-difluoro-1-piperidyl)-1,2,4-oxadiazol-3-yl]-8-fluoro-1,1-dioxo-2,3-dihydro-1λ⁶,5-benzothiazepin-4-one (*) | 4,4-Difluoropiperi dine hydrochloride | 556.1 [M+H]⁺ |
| 198 | 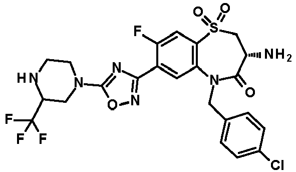 | (3R)-3-amino-5-[(4-chlorophenyl)methyl]-8-fluoro-1,1-dioxo-7-[5-[3-(trifluoromethyl)piperazin-1-yl]-1,2,4-oxadiazol-3-yl]- 2,3-dihydro-1λ⁶,5-benzothiazepin-4-one (**) | tert-Butyl 2-(trifluoromet hyl)piperazine -1-carboxylate hydrochloride | 589.1 [M+H]⁺ |
| 199 | 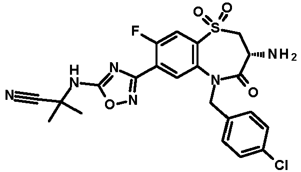 | 2-methyl-2-[[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]amino]propanenitrile | 2-Amino-2-methylpropa nenitrile | 519.1 [M+H]⁺ |

| | | | | |
|---|---|---|---|---|
| * obtained as a hydrochloride salt. ** obtained as TFA salt since it was purified by prep HPLC and the eluent contained 0.1 % TFA. | | | | |

### Example 200

### (3R)-3-amino-5-[(4-chlorophenyl)methyl]-8-fluoro-1,1-dioxo-7-[5-[(1R,5S)-3-oxa-8-azabicyclo[3.2.1]octan-8-yl]-1,2,4-oxadiazol-3-yl]-2,3-dihydro-1λ⁶,5-benzothiazepin-4-one

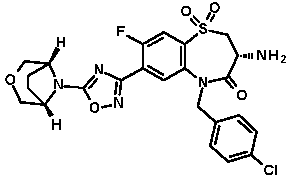

### Step a) tert-butyl N-[(3R)-5-[(4-chlorophenyl)methyl]-8-fluoro-1,1, 4-trioxo-7-[(Z)-N'-hydroxycarbamimidoyl]-2, 3-dihydro-1λ⁶,5-benzothiazepin-3-yl]carbamate

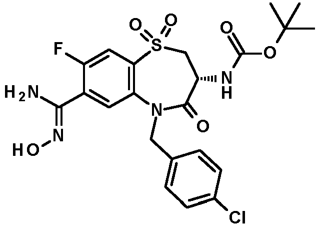

The title compound was prepared in analogy to example 192, step a) from tert-butyl N-[(3R)-5-[(4-chlorophenyl)methyl]-7-cyano-8-fluoro-1,1,4-trioxo-2,3-dihydro-1λ⁶,5-benzothiazepin-3-yl]carbamate (832 mg, 1.68 mmol) as a yellow solid (563 mg, 63% yield). MS (ESI): 471.1 [M-isobutene+H]⁺.

### Step b) tert-butyl N-[(3R)-5-[(4-chlorophenyl)methyl]-8-fluoro-1,1,4-trioxo-7-(5-oxo-4H-1,2,4-oxadiazol-3-yl)-2,3-dihydro-1λ⁶,5-benzothiazepin-3-yl]carbamate

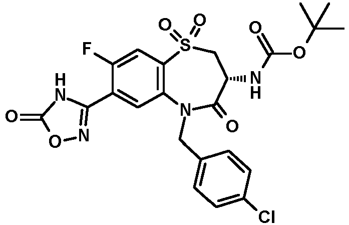

The title compound was prepared in analogy to example 192, step b) from tert-butyl N-[(3R)-5-[(4-chlorophenyl)methyl]-8-fluoro-1,1,4-trioxo-7-[(Z)-N'-hydroxycarbamimidoyl]-2,3-dihydro-1λ⁶,5-benzothiazepin-3-yl]carbamate (100 mg, 0.19 mmol) as an orange solid (80 mg, 72% yield, 94% purity). MS (ESI): 497.0 [M-isobutene+H]⁺.

### Step c) tert-butyl N-[(3R)-5-[(4-chlorophenyl)methyl]-8-fluoro-1,1,4-trioxo-7-[5-[rac-(1S,SR)-3-oxa-8-azabicyclo[3.2.1]octan-8-yl]-1,2,4-oxadiazol-3-yl]-2,3-dihydro-1λ⁶,5-benzothiazepin-3-yl]carbamate

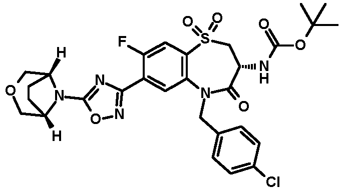

The title compound was prepared in analogy to example 192, step c) from tert-butyl N-[(3R)-5-[(4-chlorophenyl)methyl]-8-fluoro-1,1,4-trioxo-7-(5-oxo-4H-1,2,4-oxadiazol-3-yl)-2,3-dihydro-1λ⁶,5-benzothiazepin-3-yl]carbamate (45 mg, 0.069 mmol) as an orange solid (3.4 mg, 7% yield, 95% purity). MS (ESI): 592.3 [M-isobutene+H]⁺.

### Step d) (3R)-3-amino-5-[(4-chlorophenyl)methyl]-8-fluoro-1,1-dioxo-7-[5-[rac-(1R,5S)-3-oxa-8-azabicyclo[3.2.1]octan-8-yl]-1,2,4-oxadiazol-3-yl]-2,3-dihydro-1λ⁶,5-benzothiazepin-4-one

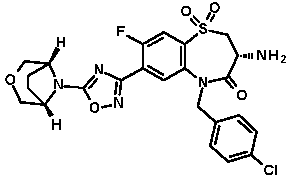

The title compound was prepared in analogy to example 192, step e) from tert-butyl N-[(3R)-5-[(4-chlorophenyl)methyl]-8-fluoro-1,1,4-trioxo-7-[5-[rac-(1S,5R)-3-oxa-8-azabicyclo[3.2.1]octan-8-yl]-1,2,4-oxadiazol-3-yl]-2,3-dihydro-1λ⁶,5-benzothiazepin-3-yl]carbamate (3 mg, 0.005 mmol) as a light yellow solid, as a hydrochloride salt (3 mg, 100% yield, 93% purity). MS (ESI): 548.3 [M+H]⁺.

Example 201 to 206 of the following table was prepared in analogy to Example 200 in two steps, using the appropriate amine building block.

| **Ex.** | **Structure** | **Systematic Name** | **Building Block** | **MS, ESI: m/z** |
|---|---|---|---|---|
| 201 | 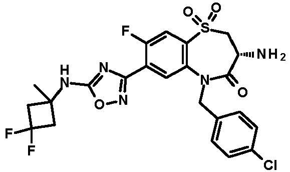 | (3R)-3-amino-5-[(4-chlorophenyl)methyl]-7-[5-[(3,3-difluoro-1-methylcyclobutyl)amino]-1,2,4-oxadiazol-3-yl]-8-fluoro-1,1-dioxo-2,3-dihydro-1λ⁶,5-benzothiazepin-4-one (*,**) [EPIMERS 1:1] | 3,3-Difluoro-1-methylcyclobutan amine hydrochlor ide | 556.0 [M+H]⁺ |
| 202 | 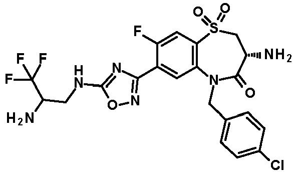 | (3R)-3-amino-7-[5-[(2-amino-3,3,3-trifluoro-propyl)amino]-1,2,4-oxadiazol-3-yl]-5-[(4-chlorophenyl)methyl]-8-fluoro-1,1-dioxo-2,3-dihydro-1λ⁶,5-benzothiazepin-4-one (*) [EPIMERS 1:1] | 3,3,3-Trifluoropr opane-1,2-diamine dihydrochl oride | 561.1 [M+H]⁺ |
| 203 | 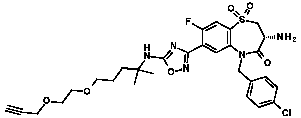 | (3R)-3-amino-5-[(4-chlorophenyl)methyl]-7-[5-[[1,1-dimethyl-4-(2-prop-2-ynoxyethoxy)butyl]amino]-1,2,4-oxadiazol-3-yl]-8-fluoro-1,1-dioxo-2,3-dihydro-1λ⁶,5-benzothiazepin-4-one (**) | 2-Methyl-5-(2-prop-2-ynoxyethoxy) pentan-2-amine | 634.2 [M+H]⁺ |
| 204 | 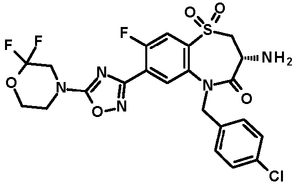 | (3R)-3-amino-5-[(4-chlorophenyl)methyl]-7-[5-(2,2-difluoromorpholin-4-yl)-1,2,4-oxadiazol-3-yl]-8-fluoro-1, 1-dioxo-2,3-dihydro-1λ⁶,5-benzothiazepin-4-one | 2,2-Difluoromorp holine;hydroc hloride | 558.0 [M+H]+ |
| 205 | 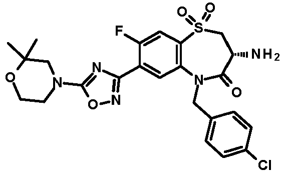 | (3R)-3-amino-5-[(4-chlorophenyl)methyl]-7-[5-(2,2-dimethylmorpholin-4-yl)-1,2,4-oxadiazol-3-yl]-8-fluoro-1,1-dioxo-2,3-dihydro-1λ⁶,5-benzothiazepin-4-one | 2,2-Dimethylm orpholine | 550.1 [M+H]⁺ |
| 206 | 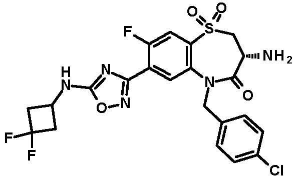 | (3R)-3-amino-5-[(4-chlorophenyl)methyl]-7-[5-[(3,3-difluorocyclobutyl)amino]-1,2,4-oxadiazol-3-yl]-8-fluoro-1,1-dioxo-2,3-dihydro-1λ⁶,5-benzothiazepin-4-one (**) | 3,3-Difluorocy clobutana mine hydrochlor ide | 542.0 [M+H]⁺ |

| | | | | |
|---|---|---|---|---|
| * obtained as a hydrochloride salt. ** Deprotection was carried out in EtOAc with HCl in EtOAc at rt | | | | |

### Precursor amine for Example 203: 2-methyl-5-(2-prop-2-ynoxyethoxy)pentan-2-amine

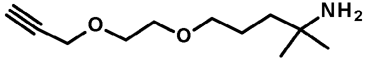

To a solution of 4-methyl-4-nitro-1-(2-prop-2-ynoxyethoxy)pentane (350.0 mg, 1.53 mmol) in MeOH (3.5 mL) and aqueous hydrogenchloride (3.5 mL, 42 mmol, 27.51 eq) was added Zinc (598.8 mg, 9.16 mmol, 6 eq) portionwise at 0 °C. After finished, the mixture was allowed to warm to room temperature and stirred for 3 h, concentrated under vacuum to remove most of the solvent, and the residue was added into saturated aqueous NaHCO₃ (10 mL) dropwise, the suspension was then extracted with EtOAc (10 mL), filtered through celite to separated the preciptated Zn salt. The water phase was extracted with EtOAc (10 mL) again. The combined organic phase was dried over anhydrous Na₂SO₄, concentrated under vacuum to give crude title compound (250 mg, 1.25 mmol, 82% yield) as a light brown oil.

### Example 207

### (3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1λ⁴,5-benzothiazepin-4-one (Epimer A)

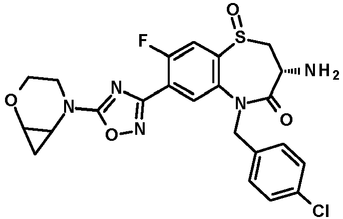

### Step a) tert-butyl N-[(3R)-5-[(4-chlorophenyl)methyl]-8-fluoro-7-[5-(2-oxa-5-azabicyclo[4.1.0]heptan-5-yl)-1,2,4-oxadiazol-3-yl]-4-oxo-2,3-dihydro-1,5-benzothiazepin-3-yl]carbamate

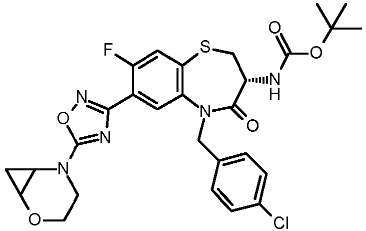

The title compound was prepared in analogy to general procedure 16 from tert-butyl N-[(3R)-5-[(4-chlorophenyl)methyl]-8-fluoro-4-oxo-7-(5-oxo-4H-1,2,4-oxadiazol-3-yl)-2,3-dihydro-1,5-benzothiazepin-3-yl]carbamate (Example 192, step b) (100 mg, 0.154 mmol, 1 eq) and 2-oxa-5-azabicyclo[4.1.0]heptane (CAS 1354952-28-5) (20.82 mg, 0.154 mmol, 1 eq) and was obtained as a white crystalline (63.3 mg, 65% yield). MS (ESI): 546.2 [M-isobutene+H]⁺.

### Step b) tert-butyl N-[(3R)-5-[(4-chlorophenyl)methyl]-8-fluoro-7-[5-(2-oxa-5-azabicyclo[4.1.0]heptan-5-yl)-1,2, 4-oxadiazol-3-yl]-1,4-dioxo-2, 3-dihydro-1^{λ4},5-benzothiazepin-3-yl]carbamate (Epimer A) and tert-butyl N-[(3R)-5-[(4-chlorophenyl)methyl]-8-fluoro-7-[5-(2-oxa-5-azabicyclo[4.1.0]heptan-5-yl)-1,2,4-oxadiazol-3-yl]-1,4-dioxo-2,3-dihydro-1λ⁴,5-benzothiazepin-3-yl]carbamate (Epimer B)

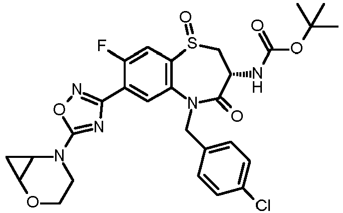

tert-butyl N-[(3R)-5-[(4-chlorophenyl)methyl]-8-fluoro-7-[5-(2-oxa-5-azabicyclo[4.1.0]heptan-5-yl)-1,2,4-oxadiazol-3-yl]-4-oxo-2,3-dihydro-1,5-benzothiazepin-3-yl]carbamate (63.3 mg, 0.1 mmol) was stirred with 3-chloroperoxybenzoic acid (22.4 mg, 0.1 mmol, 1 eq) in DCM (2 mL) at RT for 1:5 hours. The reaction solution was diluted with DCM and 1N aqueous NaOH. The aqueous layer was extracted twice with DCM. The organic layers were washed with water, brine, dried over Na₂SO₄, filtered and evaporated and purified by column chromatography on silica gel (heptane:EtOAc = 1:0 to 1:2) to afford the title compound (epimer A) (24.7 mg, 40%) as white powder (MS (ESI): 616.3 [M+HCOOH-H]) and epimer B (18.3 mg, 29.6%) as white powder (MS (ESI): 616.3 [M+HCOOH-H]⁻).

### Step c) (3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1λ⁴,5-benzothiazepin-4-one (Epimer A)

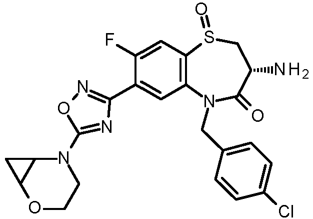

The title compound was prepared in analogy to general procedure 6d from tert-butyl N-[(3R)-5-[(4-chlorophenyl)methyl]-8-fluoro-7-[5-(2-oxa-5-azabicyclo[4.1.0]heptan-5-yl)-1,2,4-oxadiazol-3-yl]-1,4-dioxo-2,3-dihydro-1λ⁴,5-benzothiazepin-3-yl]carbamate (Epimer A) (24.7 mg, 40 µmol) and was obtained (15.3 mg, 67% yield) as a white powder as a hydrochloride salt. MS (ESI): 518.1 [M+H]⁺.

Examples 208 to 213 of the following table were prepared in analogy to Example 207.

| **Ex.** | **Structure** | **Systematic Name** | **Epimer** | **MS, ESI: m/z** |
|---|---|---|---|---|
| 208 | 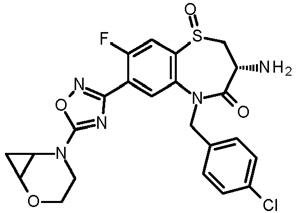 | (3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1λ⁴,5-benzothiazepin-4-one (Epimer B) (*) | B | 518.1 [M+H]⁺ |
| 209 | 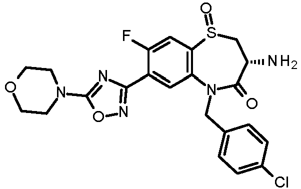 | (3R)-3-amino-5-[(4-chlorophenyl)methyl]-8-fluoro-7-(5-morpholino-1,2,4-oxadiazol-3-yl)-1-oxo-2,3-dihydro-1λ⁴,5-benzothiazepin-4-one (Epimer A) (*) | A | 506.1 [M+H]⁺ |
| 210 | 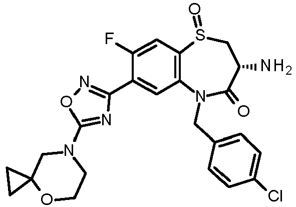 | (3R)-3-amino-5-[(4-chlorophenyl)methyl]-8-fluoro-7-[5-(4-oxa-7-azaspiro[2.5]octan-7-yl)-1,2,4-oxadiazol-3-yl]-1-oxo-2,3-dihydro-1λ⁴,5-benzothiazepin-4-one (Epimer B) (*) | B | 532.1 [M+H]⁺ |
| 211 | 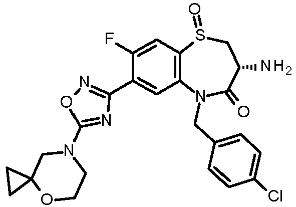 | (3R)-3-amino-5-[(4-chlorophenyl)methyl]-8-fluoro-7-[5-(4-oxa-7-azaspiro[2.5]octan-7-yl)-1,2,4-oxadiazol-3-yl]-1-oxo-2,3-dihydro-1λ⁴,5-benzothiazepin-4-one (Epimer A) (*) | A | 532.2 [M+H]⁺ |
| 212 | 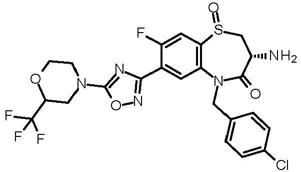 | (3R)-3-amino-5-[(4-chlorophenyl)methyl]-8-fluoro-1-oxo-7-[5-[2-(trifluoromethyl)morpholin-4-yl]-1,2,4-oxadiazol-3-yl]-2,3-dihydro-1λ⁴,5-benzothiazepin-4-one (Epimer B) (*) | B | 574.1 [M+H]⁺ |
| 213 | 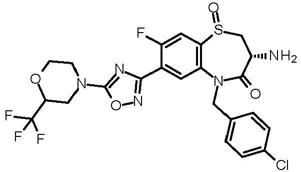 | (3R)-3-amino-5-[(4-chlorophenyl)methyl]-8-fluoro-1-oxo-7-[5-[2-(trifluoromethyl)morpholin-4-yl]-1,2,4-oxadiazol-3-yl]-2,3-dihydro-1λ⁴,5-benzothiazepin-4-one (Epimer A) (*) | A | 574.2 [M+H]⁺ |

| | | | | |
|---|---|---|---|---|
| * as a hydrochloride salt | | | | |

### Example 214

### (3R)-3-amino-8-fluoro-5-[(6-isopropoxy-3-pyridyl)methyl]-7-[5-(4-oxa-7-azaspiro[2.5]octan-7-yl)-1,2,4-oxadiazol-3-yl]-1,1-dioxo-2,3-dihydro-1λ⁶,5-benzothiazepin-4-one

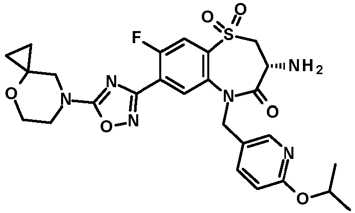

### Step a) tert-butyl N-[(3R)-7-cyano-8-fluoro-1,1, 4-trioxo-3, 5-dihydro-2H-1λ⁶,5-benzothiazepin-3-yl]carbamate

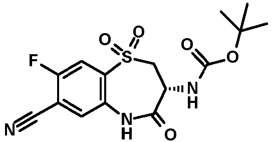

The title compound was prepared in analogy to general procedure 5 from Example 321, step c) (7 g, 20.75 mmol) and was obtained as a white solid (7.4 g, 96% yield). MS (ESI): 314.1 [M-isobutene+H]⁺.

### Step b) tert-butyl N-[(3R)-7-cyano-8-fluoro-5-[(6-isopropoxy-3-pyridyl)methyl]-1,1, 4-trioxo-2, 3-dihydro-1λ⁶,5-benzothiazepin-3-yl]carbamate

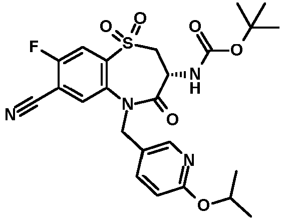

To a mixture of tert-butyl N-[(3R)-7-cyano-8-fluoro-1,1,4-trioxo-3,5-dihydro-2H-1λ⁶,5-benzothiazepin-3-yl]carbamate (500 mg, 1.35 mmol, 1 eq), KI (226 mg, 1.35 mmol, 1 eq), 1M aqueous Na₂CO₃ solution (0.42 ml, 4.03 mmol, 3 eq) in DMF (10 mL) was added 5-(bromomethyl)-2-(1-methylethoxy)pyridine (CAS: 1382866-91-2 ) (398 mg, 1.5 mmol, 1.1 eq) and stirred at room temperature for 16 h. The reaction mixture was poured into ethyl acetate (20 mL) and the organic layer was washed with brine (3 x 20 mL), dried over anhydrous Na₂SO₄ and concentrated under vacuum. The crude product was purified by column chromatography on silica gel (PE:EA = 5:1 to 1:1) affording the title compound (640 mg, 1.23 mmol, 66% yield) as a yellow solid. MS (ESI): 519.2 [M+H]+.

### Step c) tert-butyl N-[(3R)-8-fluoro-5-[(6-isopropoxy-3-pyridyl)methyl]-1,1,4-trioxo-7-[(Z)-N'-hydroxycarbamimidoyl]-2,3-dihydro-1λ⁶,5-benzothiazepin-3-yl]carbamate

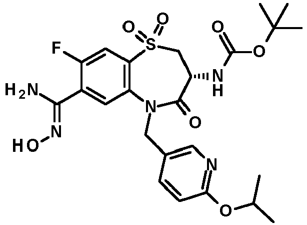

The title compound was prepared in analogy to general procedure **12** from tert-butyl N-[(3R)-7-cyano-8-fluoro-5-[(6-isopropoxy-3-pyridyl)methyl]-1,1,4-trioxo-2,3-dihydro-1λ⁶,5-benzothiazepin-3-yl]carbamate (640 mg, 1.23 mmol) and was obtained as a yellow solid (220 mg, 29% yield). MS (ESI): 552.3 [M+H]⁺.

### Step d) tert-butyl N-[(3R)-8-fluoro-5-[(6-isopropoxy-3-pyridyl)methyl]-1,1,4-trioxo-7-(5-oxo-4H-1,2,4-oxadiazol-3-yl)-2, 3-dihydro-1λ⁶,5-benzothiazepin-3-yl]carbamate

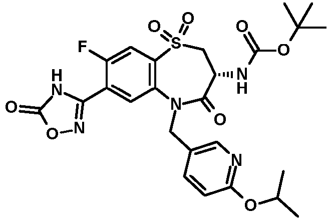

To a mixture of tert-butyl N-[(3R)-8-fluoro-5-[(6-isopropoxy-3-pyridyl)methyl]-1,1,4-trioxo-7-[(Z)-N'-hydroxycarbamimidoyl]-2,3-dihydro-1λ⁶,5-benzothiazepin-3-yl]carbamate (220 mg, 0.4 mmol) and triethylamine (0.11 ml, 0.8 mmol, 2eq) in DCM ( 5mL) was added N.N'-carbonyldiimidazole (97 mg, 0.6 mmol, 1.5 eq) at room temperature. The reaction mixture was stirred for 3h. The reaction mixture was diluted with EtOAc (10 mL) and the layer was washed with brine (2 x 10 mL), dried over anhydrous Na₂SO₄ and concentrated under vacuum. The crude product was purified by column chromatography on silica gel (PE:EA = 2:1 to 0:1) affording the title compound (185 mg, 0.32mmol, 69% yield) as an off-white solid. MS (ESI): 578.3[M+H]+.

### Step e) tert-butyl N-[(3R)-8-fluoro-5-[(6-isopropoxy-3-pyridyl)methyl]-7-[5-(4-oxa-7-azaspiro[2.5]octan-7-yl)-1,2, 4-oxadiazol-3-yl]-1,1, 4-trioxo-2,3-dihydro-1^{λ6},5-benzothiazepin-3-yl]carbamate

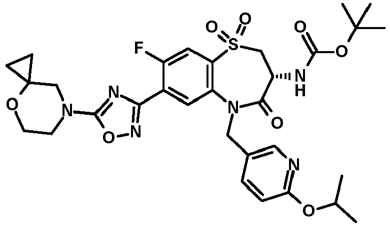

The title compound was prepared in analogy to general procedure 16 from tert-butyl N-[(3R)-8-fluoro-5-[(6-isopropoxy-3-pyridyl)methyl]-1,1,4-trioxo-7-(5-oxo-4H-1,2,4-oxadiazol-3-yl)-2,3-dihydro-1λ⁶,5-benzothiazepin-3-yl]carbamate (100 mg, 0.17 mmol) and was obtained as a white solid (160 mg, 100% yield). MS (ESI): 673.3 [M+H]⁺.

### Step f) (3R)-3-amino-8-fluoro-5-[(6-isopropoxy-3-pyridyl)methyl]-7-[5-(4-oxa-7-azaspiro[2.5]octan-7-yl)-1,2,4-oxadiazol-3-yl]-1,1-dioxo-2,3-dihydro-1λ⁶,5-benzothiazepin-4-one

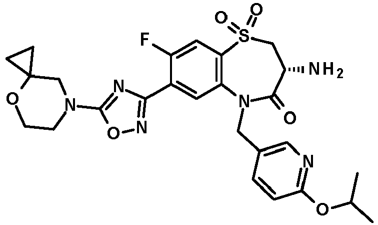

The title compound was prepared in analogy to general procedure **6b** from tert-butyl N-[(3R)-8-fluoro-5-[(6-isopropoxy-3-pyridyl)methyl]-7-[5-(4-oxa-7-azaspiro[2.5]octan-7-yl)-1,2,4-oxadiazol-3-yl]-1,1,4-trioxo-2,3-dihydro-1λ⁶,5-benzothiazepin-3-yl]carbamate (160 mg, 0.24 mmol) further purified by prep-HPLC (column: Waters Xbridge 150*25mm* 5um; mobile phase:water(10mM NH₄HCO₃)- MeCN;B%: 36%-66%,8 min). The eluent was concentrated under vacuum to remove MeCN the residue was freeze dried and the title compound was obtained as a white solid (31.2 mg, 61% yield). MS (ESI): 573.3 [M+H]⁺.

### Example 215

### (3R)-3-amino-8-fluoro-5-[(5-isopropoxy-2-pyridyl)methyl]-7-[5-(4-oxa-7-azaspiro[2.5]octan-7-yl)-1,2,4-oxadiazol-3-yl]-1,1-dioxo-2,3-dihydro-1λ⁶,5-benzothiazepin-4-one

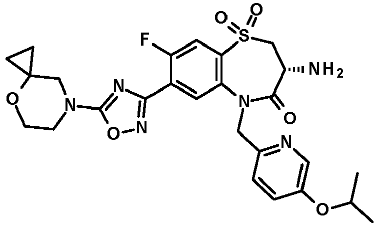

### Step a) tert-butyl N-[(3R)-7-cyano-8-fluoro-1,1, 4-trioxo-3, 5-dihydro-2H-1λ⁶,5-benzothiazepin-3-yl]carbamate

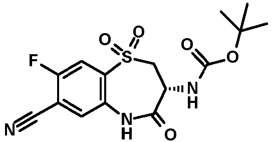

The title compound was prepared in analogy to general procedure **5** from Example 321, step c) (7 g, 20.7 mmol) and was obtained as a white solid (7.4 g, 96% yield). MS (ESI): 314.1 [M-isobutene+H]⁺.

### Step b) tert-butyl N-[(3R)-7-cyano-8-fluoro-5-[(5-isopropoxy-2-pyridyl)methyl]-1,1,4-trioxo-2, 3-dihydro-1λ⁶,5-benzothiazepin-3-yl]carbamate

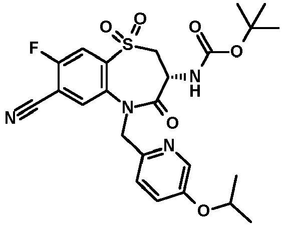

To a mixture of tert-butyl N-[(3R)-7-cyano-8-fluoro-1,1,4-trioxo-3,5-dihydro-2H-1λ⁶,5-benzothiazepin-3-yl]carbamate (500 mg, 1.35 mmol), 5-(1-methylethoxy)-2-pyridinemethanol (CAS:1198166-00-5) (249 mg, 1.49 mmol, 1.1 eq), Ph₃P (710 mg, 2.71 mml, 2 eq) in toluene (10 mL) was added DIAD (0.53 mL, 2.71 mmol, 2 eq) at 0 °C. The reaction mixture was diluted with ethyl acetate (10 mL) and the organic layer was washed with three portions of brine (10 mL), dried over anhydrous Na₂SO₄ and concentrated under vacuum. The crude material was purified by prep-HPLC (column: Waters Xbridge 150*50mm* 10um; mobile phase:water(10mM NH₄HCO₃)-MeCN;B%: 40%-70%,10min). The eluent was concentrated under vacuum to remove MeCN, the residue was freeze dried, affording the title compound (260 mg, 0.5 mmol, 36% yield) as a light brown solid. MS (ESI): 518.9 [M+H]+.

### Step c) tert-butyl N-[(3R)-8-fluoro-5-[(6-isopropoxy-2-pyridyl)methyl]-1,1,4-trioxo-7-[(Z)-N'-hydroxycarbamimidoyl]-2, 3-dihydro-1λ⁶,5-benzothiazepin-3-yl]carbamate

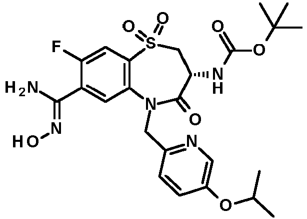

The title compound was prepared in analogy to general procedure **12** from tert-butyl N-[(3R)-7-cyano-8-fluoro-5-[(6-isopropoxy-2-pyridyl)methyl]-1,1,4-trioxo-2,3-dihydro-1λ⁶,5-benzothiazepin-3-yl]carbamate (86 mg, 0.16 mmol, 1 eq) and was obtained as a light brown solid (80 mg, 76.2% yield). MS (ESI): 552.3 [M+H]⁺.

### Step d) tert-butyl N-[(3R)-8-fluoro-5-[(6-isopropoxy-2-pyridyl)methyl]-1,1,4-trioxo-7-(5-oxo-4H-1,2,4-oxadiazol-3-yl)-2,3-dihydro-1λ⁶,5-benzothiazepin-3-yl]carbamate

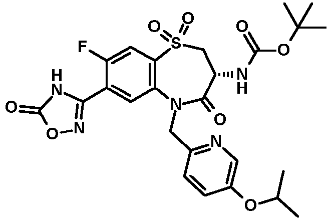

To a mixture of tert-butyl N-[(3R)-8-fluoro-5-[(6-isopropoxy-2-pyridyl)methyl]-1,1,4-trioxo-7-[(Z)-N'-hydroxycarbamimidoyl]-2,3-dihydro-1λ⁶,5-benzothiazepin-3-yl]carbamate (86 mg, 0.16 mmol) and NEt₃ (0.04 ml, 0.31 mmol, 2eq) in DCM (2 mL) was added N.N'-carbonyldiimidazole (37.9 mg, 0.23 mmol, 1.5 eq) at room temperature. The reaction mixture was stirred for 3h. The reaction mixture was poured into water (5 mL). The aqueous layer was extracted with three portion of EtOAc (5 mL). The combined organic layers were washed with brine (2 x 10 mL), dried over anhydrous Na₂SO₄ and concentrated under vacuum. The crude product was purified by column chromatography on silica gel (PE:EA = 1:1 to 0:1) affording the title compound (80 mg, 0.14 mmol, 76% yield) as a light brown solid. MS (ESI): 578.3[M+H]+.

### Step e) tert-butyl N-[(3R)-8-fluoro-5-[(6-isopropoxy-2-pyridyl)methyl]-7-[5-(4-oxa-7-azaspiro[2.5]octan-7-yl)-1,2, 4-oxadiazol-3-yl]-1,1, 4-trioxo-2,3-dihydro-1λ⁶,5-benzothiazepin-3-yl]carbamate

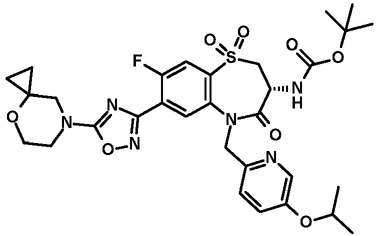

The title compound was prepared in analogy to general procedure 16 from tert-butyl N-[(3R)-8-fluoro-5-[(6-isopropoxy-2-pyridyl)methyl]-1,1,4-trioxo-7-(5-oxo-4H-1,2,4-oxadiazol-3-yl)-2,3-dihydro-1λ⁶,5-benzothiazepin-3-yl]carbamate (60 mg, 0.1 mmol) and was obtained as a light brown solid (60 mg, 80% yield). MS (ESI): 673.4 [M+H]⁺.

### Step f) (3R)-3-amino-8-fluoro-5-[(6-isopropoxy-2-pyridyl)methyl]-7-[5-(4-oxa-7-azaspiro[2.5]octan-7-yl)-1,2,4-oxadiazol-3-yl]-1,1-dioxo-2,3-dihydro-1λ⁶,5-benzothiazepin-4-one

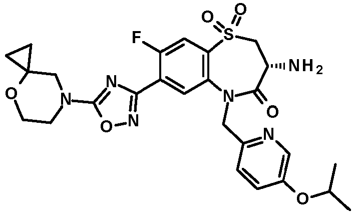

The title compound was prepared in analogy to general procedure **6b** from tert-butyl N-[(3R)-8-fluoro-5-[(6-isopropoxy-2-pyridyl)methyl]-7-[5-(4-oxa-7-azaspiro[2.5]octan-7-yl)-1,2,4-oxadiazol-3-yl]-1,1,4-trioxo-2,3-dihydro-1λ⁶,5-benzothiazepin-3-yl]carbamate (60 mg, 0.09 mmol) and purified by prep-HPLC (column: 3_Phenomenex Luna C18 75*30mm*3um; mobile phase: [water(0.05%HCl)-MeCN];B%: 24%-44%,6min) The eluent was concentrated under vacuum to remove MeCN. The residue was freeze dried and the title compound was obtained as a light brown solid (11.2 mg, 19% yield). MS (ESI): 573.3 [M+H]⁺.

### Example 216

### (3R)-3-amino-5-[(4-chlorophenyl)methyl]-7-(4-ethyloxazol-2-yl)-8-fluoro-1,1-dioxo-2,3-dihydro-1λ⁶,5-benzothiazepin-4-one

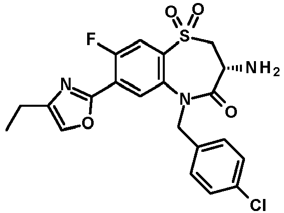

### Step a) tert-butyl N-[(3R)-5-[(4-chlorocyclohexa-2,4-dien-1-yl)methyl]-8-fluoro-7-[1-(hydroxymethyl)propylcarbamoyl]-4-oxo-2,3-dihydro-1,5-benzothiazepin-3-yl]carbamate

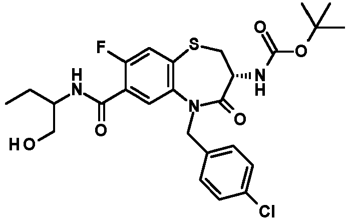

(R)-3-((tert-butoxycarbonyl)amino)-5-(4-chlorobenzyl)-8-fluoro-4-oxo-2,3,4,5-tetrahydrobenzo[b][1,4]thiazepine-7-carboxylic acid (CAS:2002449-40-1) (70 mg, 146 µmol, Eq: 1) was dissolved in DMF (0.5 mL) and DIPEA (75.2 mg, 102 µl, 582 µmol, Eq: 4) was added to the reaction mixture. The reaction mixture was cooled to 0°C and HATU (166 mg, 437 µmol, Eq: 3) was added and the mixture was stirred for 30 minutes. The reaction mixture was then warmed to RT and 2-aminobutan-1-ol (19.5 mg, 20.6 µl, 218 µmol, Eq: 1.5) was added and the mixture was stirred at RT for 1 h. The reaction mixture was adsorbed on silica gel and purified by flash chromatography on silica gel (0-50% EtOAc in heptane), affording the title compound (28 mg, 49.7 µmol, 34% yield) as a orange solid. MS (ESI): 496.3 [M-isobutene+H]⁺.

### Step b) tert-butyl N-[(3R)-5-[(4-chlorophenyl)methyl]-7-(4-ethyl-4,5-dihydrooxazol-2-yl)-8-fluoro-4-oxo-2, 3-dihydro-1,5-benzothiazepin-3-yl]carbamate

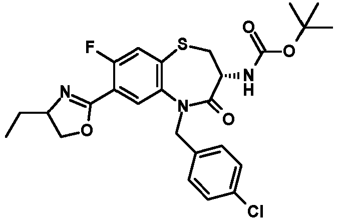

tert-butyl N-[(3R)-5-[(4-chlorocyclohexa-2,4-dien-1-yl)methyl]-8-fluoro-7-[1-(hydroxymethyl)propylcarbamoyl]-4-oxo-2,3-dihydro-1,5-benzothiazepin-3-yl]carbamate (25 mg, 45.3 µmol, Eq: 1) was dissolved in THF (0.4 mL). Burgess reagent (50.8 mg, 226 µmol, Eq: 5) was added and the reaction mixture was stirred at 60°C for 2 h. The solvent was evaporated and the remaining residue was purified by chromatography on silica gel (0-60% EtOAc in heptane), affording the title compound (8.6 mg, 16.1 µmol, 36% yield) as a white soild. MS (ESI): 534.4 [M+H]⁺.

### Step c) tert-butyl N-[(3R)-5-[(4-chlorophenyl)methyl]-7-(4-ethyloxazol-2-yl)-8-fluoro-4-oxo-2, 3-dihydro-1,5-benzothiazepin-3-yl]carbamate

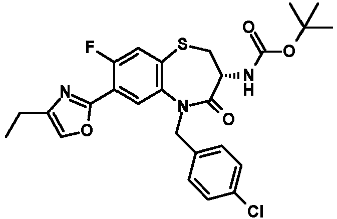

tert-butyl-N-[(3R)-5-[(4-chlorophenyl)methyl]-7-(4-ethyl-4,5-dihydrooxazol-2-yl)-8-fluoro-4-oxo-2,3-dihydro-1,5-benzothiazepin-3-yl]carbamate (8 mg, 15 µmol, Eq: 1) was dissolved in toluene (0.5 mL). DDQ (3.4 mg, 15 µmol, Eq: 1) was added and the reaction mixture was stirred at 50°C for 30 minutes and then stirred at 80°C over night. DDQ (3.4 mg, 15 µmol, Eq: 1) was added to the reaction mixture and it was stirred for two days at 110°C. The reaction mixture was adsorbed on silica gel and purified by chromatography on silica gel (0-50% EtOAc in heptane), affording the title compound (2.3 mg, 4.32 µmol, 29% yield) as a white solid. MS (ESI): 532.4 [M+H]⁺.

### Step d) tert-butyl N-[(3R)-5-[(4-chlorophenyl)methyl]-7-(4-ethyloxazol-2-yl)-8-fluoro-1,1,4-trioxo-2, 3-dihydro-1λ⁶,5-benzothiazepin-3-yl]carbamate

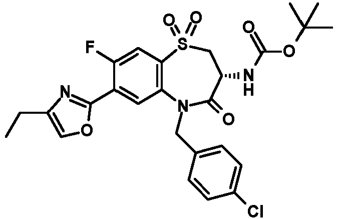

The title compound was prepared according to general method 5 from tert-butyl N-[(3R)-5-[(4-chlorophenyl)methyl]-7-(4-ethyloxazol-2-yl)-8-fluoro-4-oxo-2,3-dihydro-1,5-benzothiazepin-3-yl]carbamate (Example 216, step c) (2.1 mg, 3.95 µmol) and was obtained as a white solid (1.25 mg, 2.22 µmol, 56% yield). MS (ESI): 564.4 [M+H]⁺.

### Step e) (3R)-3-amino-5-[(4-chlorophenyl)methyl]-7-(4-ethyloxazol-2-yl)-8-fluoro-1,1-dioxo-2,3-dihydro-1λ⁶,5-benzothiazepin-4-one

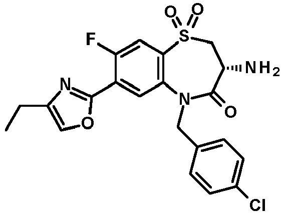

The title compound was prepared in analogy to method **6b** from tert-butyl N-[(3R)-5-[(4-chlorophenyl)methyl]-7-(4-ethyloxazol-2-yl)-8-fluoro-1,1,4-trioxo-2,3-dihydro-1λ⁶,5-benzothiazepin-3-yl]carbamate (1.25 mg, 2.22 µmol) and was obtained as a white solid, as a hydrochloride salt (1 mg, 1.68 µmol, 76% yield). MS (ESI): 464.3 [M+H]⁺.

### Example 217

### (3R)-3-amino-5-[(4-chlorophenyl)methyl]-6-fluoro-1,1-dioxo-7-[5-(2,2,2-trifluoroethyl)-1,3,4-oxadiazol-2-yl]-2,3-dihydro-1λ⁶,5-benzothiazepin-4-one

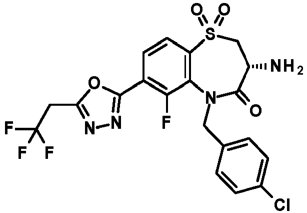

### Step a) (2R)-2-(tert-butoxycarbonylamino)-3-(3-fluoro-4-methoxycarbonyl-2-nitrophenyl)sulfanyl-propanoic acid

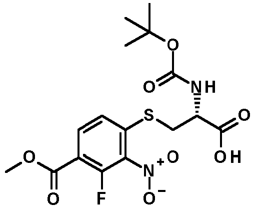

The title compound was prepared in analogy to general method **1b** from methyl 2,4-difluoro-3-nitrobenzoate (600 mg, 2.76 mmol, Eq: 1) and was obtained as an off-white solid (1.16 g, 2.77 mmol, 100% yield). MS (ESI): 319.0 [M-isobutene-CO₂+H]⁺.

### Step b) (2R)-3-(2-amino-3-fluoro-4-methoxycarbonyl-phenyl)sulfanyl-2-(tert-butoxycarbonylamino)propanoic acid

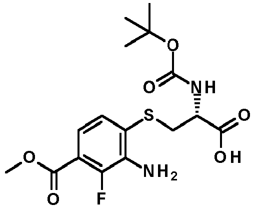

The title compound was prepared in analogy to general method 2 from (2R)-2-(tert-butoxycarbonylamino)-3-(3-fluoro-4-methoxycarbonyl-2-nitro-phenyl)sulfanyl-propanoic acid (1.15 g, 2.75 mmol, Eq: 1) and was obtained as a dark brown amorphous solid (854 mg, 1.93 mmol, 70% yield). MS (ESI): 387.2 [M-H]⁻.

### Step c) methyl (3R)-3-(tert-butoxycarbonylamino)-6-fluoro-4-oxo-3,5-dihydro-2H-1,5-benzothiazepine-7-carboxylate

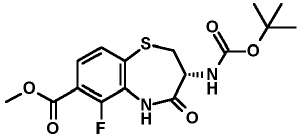

The title compound was prepared in analogy to general method 3 from (2R)-3-(2-amino-3-fluoro-4-methoxycarbonyl-phenyl)sulfanyl-2-(tert-butoxycarbonylamino)propanoic acid (400 mg, 1.03 mmol) and was obtained as a light yellow oil (222 mg, 599 µmol, 58% yield). MS (ESI): 369.2 [M-H]⁻.

### Step d) methyl (3R)-3-(tert-butoxycarbonylamino)-5-[(4-chlorophenyl)methyl]-6-fluoro-4-oxo-2,3-dihydro-1,5-benzothiazepine-7-carboxylate

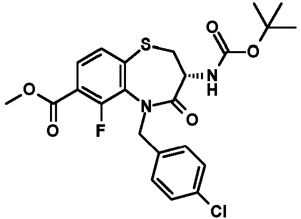

The title compound was prepared in analogy to general method **4** from methyl (3R)-3-(tert-butoxycarbonylamino)-6-fluoro-4-oxo-3,5-dihydro-2H-1,5-benzothiazepine-7-carboxylate (220 mg, 594 µmol) and was obtained as a light yellow solid (201 mg, 191 µmol, 32% yield). MS (ESI): 439.0 [M-isobutene+H]⁺.

### Step e) (3R)-3-(tert-butoxycarbonylamino)-5-[(4-chlorophenyl)methyl]-6-fluoro-4-oxo-2,3-dihydro-1,5-benzothiazepine-7-carboxylic acid

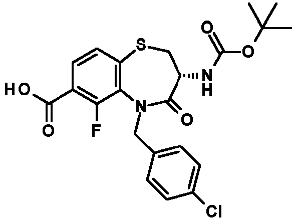

Methyl-(3R)-3-(tert-butoxycarbonylamino)-5-[(4-chlorophenyl)methyl]-6-fluoro-4-oxo-2,3-dihydro-1,5-benzothiazepine-7-carboxylate (195 mg, 394 µmol, Eq: 1) was dissolved in a mixture of THF (3.5 mL), MeOH (500 µl) and water (1 mL). Lithium hydroxide hydrate (33.1 mg, 788 µmol, Eq: 2) was added and the reaction mixture was stirred at RT for 1 h. The solvent was evaporated and the remaining residue was dissolved in EtOAc and the mixture was washed with 1N aq. HCl, water and brine. The organic layer was dried over magnesium sulfate, filtered and evaporated to afford the title compound (195 mg, 215 µmol, 55% yield) as a yellow solid. MS (ESI): 479.1 [M-H]⁻.

### Step f) tert-butyl N-[(3R)-5-[(4-chlorophenyl)methyl]-6-fluoro-4-oxo-7-[5-(2,2,2-trifluoroethyl)-1,3,4-oxadiazol-2-yl]-2,3-dihydro-1,5-benzothiazepin-3-yl]carbamate

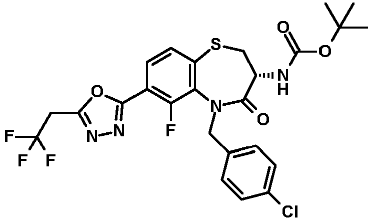

A mixture of (3R)-3-(tert-butoxycarbonylamino)-5-[(4-chlorophenyl)methyl]-6-fluoro-4-oxo-2,3-dihydro-1,5-benzothiazepine-7-carboxylic acid (40 mg, 83.2 µmol, Eq: 1), 3,3,3-trifluoropropanehydrazide (11.8 mg, 83.2 µmol, Eq: 1), HATU (31.6 mg, 83.2 µmol, Eq: 1) and DIPEA (21.5 mg, 29.1 µl, 166 µmol, Eq: 2) in THF (800 µl) was stirred at RT. After 30 min. Burgess reagent (59.5 mg, 250 µmol, Eq: 3) was added and stirring was continued at RT overnight. The reaction mixture was adsorbed on silica gel and purified by column chromatography on silica gel (0-50% EtOAc in heptane) to afford the title compound (29 mg, 34.1 µmol, 41% yield) as a light yellow oil. MS (ESI): 531.1 [M-isobutene+H]⁺.

### Step g) tert-butyl N-[(3R)-5-[(4-chlorophenyl)methyl]-6-fluoro-1,1, 4-trioxo-7-[5-(2, 2,2-trifluoroethyl)-1,3,4-oxadiazol-2-yl]-2,3-dihydro-1λ⁶,5-benzothiazepin-3-yl]carbamate

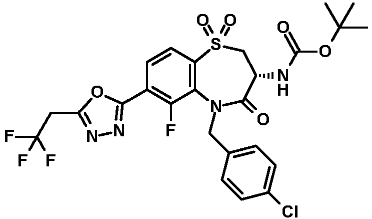

The title compound was prepared in analogy to general method 5 from tert-butyl N-[(3R)-5-[(4-chlorophenyl)methyl]-6-fluoro-4-oxo-7-[5-(2,2,2-trifluoroethyl)-1,3,4-oxadiazol-2-yl]-2,3-dihydro-1,5-benzothiazepin-3-yl]carbamate (27 mg, 46 µmol) and was obtained (18 mg, 29.1 µmol, 63% yield) as an off-white solid. MS (ESI): 563.1 [M-isobutene+H]⁺.

### Step h) (3R)-3-amino-5-[(4-chlorophenyl)methyl]-6-fluoro-1,1-dioxo-7-[5-(2,2,2-trifluoroethyl)-1,3, 4-oxadiazol-2-yl]-2,3-dihydro-1λ⁶,5-benzothiazepin-4-one

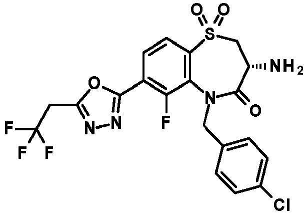

The title compound was prepared in analogy to general method **6c** from tert-butyl N-[(3R)-5-[(4-chlorophenyl)methyl]-6-fluoro-1,1,4-trioxo-7-[5-(2,2,2-trifluoroethyl)-1,3,4-oxadiazol-2-yl]-2,3-dihydro-1λ⁶,5-benzothiazepin-3-yl]carbamate (16 mg, 25.8 µmol) with addition of 4 drops of HCl (4M in dioxane) and was obtained (5.2 mg, 10 µmol, 39% yield) as a white solid. MS (ESI): 519.0 [M+H]⁺.

### Example 218

### (3R)-3-amino-7-(5-tert-butyl-1,3,4-oxadiazol-2-yl)-5-[(4-chlorophenyl)methyl]-8-fluoro-9-methyl-1,1-dioxo-2,3-dihydro-1λ⁶,5-benzothiazepin-4-one

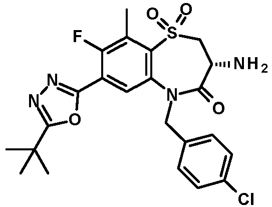

### Step a) methyl 2, 4-difluoro-3-methyl-5-nitro-benzoate

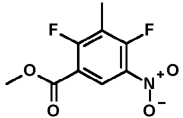

Concentrated nitric acid (2.49 mL, 56.09 mmol, 3.73 eq) was added dropwise to a mixture of methyl 2,4-difluoro-3-methyl-benzoate (CAS 1206675-31-1) (2.8 g, 15.04 mmol, 1 eq) in concentrated sulfuric acid (3.11 mL, 58.05 mmol, 3.86 eq) at 0°C. The reaction mixture was stirred at 0°C for 2 h and then poured onto ice. The mixture was extracted with EtOAc(3 x 20 mL) and the combined organic layers were washed with water (20 mL) and brine (20 mL), dried over sodium sulfate and concentrated in vacuum to get a residue which was purified by column chromatography on silica gel (PE/EA = 20:1 to 5:1) to obtain the title compound (540 mg, 2.34 mmol, 16% yield) as light yellow oil. ¹H-NMR (CDCl₃, 400 MHz) δ = 8.60 (t, J = 7.8 Hz, 1H), 4.12 (s, 1H), 3.99 (s, 3H), 2.35 (t, J = 2.3 Hz, 3H).

### Step b) (2R)-2-(tert-butoxycarbonylamino)-3-(3-fluoro-4-methoxycarbonyl-2-methyl-6-nitrophenyl)sulfanyl-propanoic acid

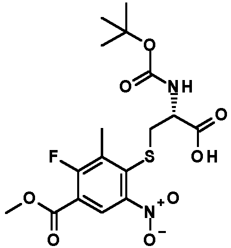

The title compound was prepared from methyl 2,4-difluoro-3-methyl-5-nitro-benzoate (2000.0 mg, 8.65 mmol, 1 eq) in analogy to general procedure **1b** and was obtained as yellow oil (3700 mg, 8.56 mmol, 99% yield). MS (ESI): 431.1 [M-H]⁻.

### Step c) (2R)-3-(6-amino-3-fluoro-4-methoxycarbonyl-2-methyl-phenyl)sulfanyl-2-(tert-butoxycarbonylamino)propanoic acid

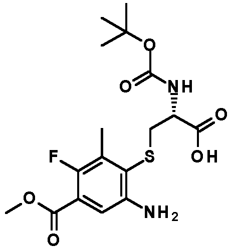

To a suspension of (2R)-2-(tert-butoxycarbonylamino)-3-(3-fluoro-4-methoxycarbonyl-2-methyl-6-nitro-phenyl)sulfanyl-propanoic acid (1.0 g, 2.31 mmol, 1 eq) in MeOH (10 mL) was added Pd/C (100.0 mg, 2.31 mmol, 1 eq) and the mixture was stirred under an atmosphere of hydrogen at 20°C for 12 h. The reaction mixture was filtered and filtrate was concentrated in vacuum to get the crude title compound (700 mg, 1.74 mmol, 75% yield) as light yellow oil which was used in the next reaction step without further purification.

### Step d) methyl (3R)-3-(tert-butoxycarbonylamino)-8-fluoro-9-methyl-4-oxo-3,5-dihydro-2H-1,5-benzothiazepine-7-carboxylate

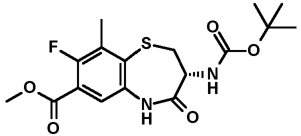

The title compound was prepared from (2R)-3-(6-amino-3-fluoro-4-methoxycarbonyl-2-methylphenyl)sulfanyl-2-(tert-butoxycarbonylamino)propanoic acid (2100.0 mg, 5.22 mmol, 1 eq) in analogy to general procedure **3** and was obtained as brown solid (350 mg, 0.910 mmol, 17% yield). MS (ESI): 383.1 [M-H]⁻.

### Step e) methyl (3R)-3-(tert-butoxycarbonylamino)-5-[(4-chlorophenyl)methyl]-8-fluoro-9-methyl-4-oxo-2,3-dihydro-1,5-benzothiazepine-7-carboxylate

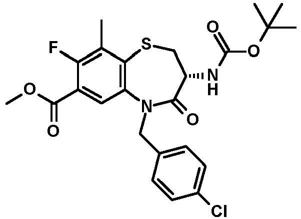

The title compound was prepared from methyl (3R)-3-(tert-butoxycarbonylamino)-8-fluoro-9-methyl-4-oxo-3,5-dihydro-2H-1,5-benzothiazepine-7-carboxylate (320.0 mg, 0.830 mmol, 1 eq) in analogy to general procedure **4** (DMF instead of DMSO as solvent) and was obtained as light yellow oil (300 mg, 0.590 mmol, 71% yield). MS (ESI): 453.3 [M-isobuten+H]⁺.

### Step f) (3R)-3-(tert-butoxycarbonylamino)-5-[(4-chlorophenyl)methyl]-8-fluoro-9-methyl-4-oxo-2,3-dihydro-1,5-benzothiazepine-7-carboxylic acid

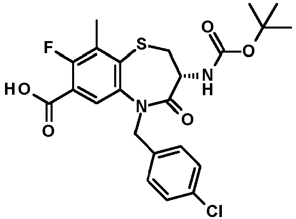

To a solution of methyl (3R)-3-(tert-butoxycarbonylamino)-5-[(4-chlorophenyl)methyl]-8-fluoro-9-methyl-4-oxo-2,3-dihydro-1,5-benzothiazepine-7-carboxylate (200.0 mg, 0.390 mmol, 1 eq) in a solvent mixture of MeOH (1 mL), THF (1 mL) and water (1 mL) was added LiOH (56.63 mg, 2.36 mmol, 6 eq) and the mixture was stirred at 20°C for 12 h. The reaction mixture was concentrated in vacuum to remove all solvents, the remaining residue was re-dissolved in water (10 mL) and the pH of the solution was adjusted to 6 by addition of 1M HCl (10 mL). The mixture was extracted with EtOAc (3 x 10 mL) and the combined organic layers were washed with water (10 mL) and brine(10 mL), dried over sodium sulfate and concentrated in vacuum to afford a light yellow oil (250 mg) containing the title compound which was used in the next reaction step without further purification. MS (ESI): 439.3 [M-isobuten+H]⁺.

### Step g) tert-butyl N-[(3R)-5-[(4-chlorophenyl)methyl]-8-fluoro-7-(hydrazinecarbonyl)-9-methyl-4-oxo-2,3-dihydro-1,5-benzothiazepin-3-yl]carbamate

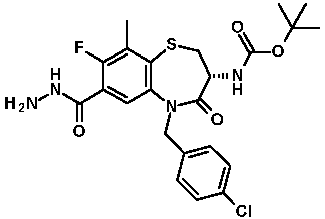

The title compound was prepared from (3R)-3-(tert-butoxycarbonylamino)-5-[(4-chlorophenyl)methyl]-8-fluoro-9-methyl-4-oxo-2,3-dihydro-1,5-benzothiazepine-7-carboxylic acid (200.0 mg, 0.400 mmol, 1 eq) in analogy to general procedure **14** and was obtained as light yellow oil. MS (ESI): 453.3 [M-isobuten+H]⁺.

### Step h) tert-butyl N-[(3R)-5-[(4-chlorophenyl)methyl]-7-[(2,2-dimethylpropanoylamino)carbamoyl]-8-fluoro-9-methyl-4-oxo-2,3-dihydro-1,5-benzothiazepin-3-yl]carbamate

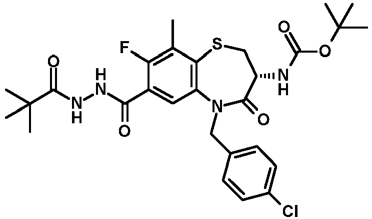

The title compound was prepared from tert-butyl N-[(3R)-5-[(4-chlorophenyl)methyl]-8-fluoro-7-(hydrazinecarbonyl)-9-methyl-4-oxo-2,3-dihydro-1,5-benzothiazepin-3-yl]carbamate (230.0 mg, 0.450 mmol, 1 eq) in analogy to general procedure **7a** and was obtained as light yellow oil. MS (ESI): 593.4 [M+H]⁺.

### Step i) tert-butyl N-[(3R)-7-(5-tert-butyl-1, 3, 4-oxadiazol-2-yl)-5-[(4-chlorophenyl)methyl]-8-fluoro-9-methyl-4-oxo-2,3-dihydro-1,5-benzothiazepin-3-yl]carbamate

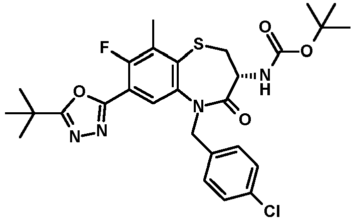

The title compound was prepared from tert-butyl N-[(3R)-5-[(4-chlorophenyl)methyl]-7-[(2,2-dimethylpropanoylamino)carbamoyl]-8-fluoro-9-methyl-4-oxo-2,3-dihydro-1,5-benzothiazepin-3-yl]carbamate (230.0 mg, 0.390 mmol, 1 eq) in analogy to general procedure **8a** and was obtained as light yellow oil (200 mg, 0.350 mmol, 90% yield). MS (ESI): 519.3 [M-isobuten+H]⁺.

### Step j) tert-butyl N-[(3R)-7-(5-tert-butyl-1, 3, 4-oxadiazol-2-yl)-5-[(4-chlorophenyl)methyl]-8-fluoro-9-methyl-1,1, 4-trioxo-2,3-dihydro-1λ⁶,5-benzothiazepin-3-yl]carbamate

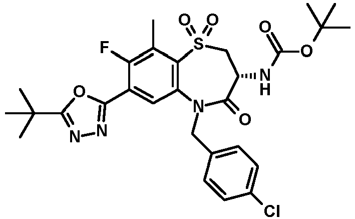

The title compound was prepared from tert-butyl N-[(3R)-7-(5-tert-butyl-1,3,4-oxadiazol-2-yl)-5-[(4-chlorophenyl)methyl]-8-fluoro-9-methyl-4-oxo-2,3-dihydro-1,5-benzothiazepin-3-yl]carbamate (200.0 mg, 0.350 mmol, 1 eq) in analogy to general procedure **5** and was obtained as light yellow oil (200 mg, 0.330 mmol, 95% yield). MS (ESI): 607.4 [M+H]⁺.

### Step k) (3R)-3-amino-7-(5-tert-butyl-1,3, 4-oxadiazol-2-yl)-5-[(4-chlorophenyl)methyl]-8-fluoro-9-methyl-1,1-dioxo-2,3-dihydro-1λ⁶,5-benzothiazepin-4-one

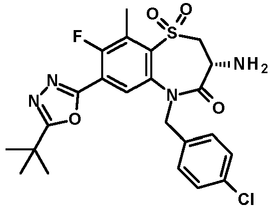

The title compound was prepared from tert-butyl N-[(3R)-7-(5-tert-butyl-1,3,4-oxadiazol-2-yl)-5-[(4-chlorophenyl)methyl]-8-fluoro-9-methyl-1,1,4-trioxo-2,3-dihydro-1λ⁶,5-benzothiazepin-3-yl]carbamate (200.0 mg, 0.330 mmol, 1 eq) in analogy to general procedure **6b** and was obtained as white solid (42.7 mg, 0.080 mmol, 25% yield). MS (ESI): 507.1 [M+H]⁺.

### Example 219

### (3R)-3-amino-7-(5-tert-butyl-1,3,4-oxadiazol-2-yl)-5-[(3-chlorophenyl)methyl]-8-fluoro-1,1-dioxo-2,3-dihydro-1λ⁶,5-benzothiazepin-4-one

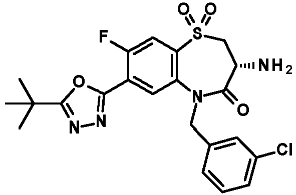

### Step a) (3R)-3-(tert-butoxycarbonylamino)-8-fluoro-4-oxo-3,5-dihydro-2H-1,5-benzothiazepine-7-carboxylic acid

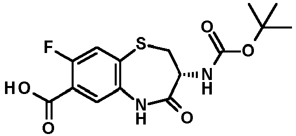

Methyl-(R)-3-((tert-butoxycarbonyl)amino)-8-fluoro-4-oxo-2,3,4,5-tetrahydrobenzo[b][1,4]thiazepine-7-carboxylate (CAS 2002449-38-7) (3.1 g, 8.37 mmol, Eq: 1) was dissolved in a mixture of THF (60 mL) and water (18 mL). Sodium hydroxide (445 mg, 11.1 mmol, Eq: 1.33) was added to the reaction mixture and it was stirred at RT for 7 h. The reaction was quenched by addition of HCl aq. 1N (11.1 ml, 11.1 mmol, Eq: 1.33) and the mixture was extracted with EtOAc. The organic layer was washed with water and brine, dried over magnesium sulfate, filtered and evaporated to afford the title compound (3.13 g, 6.76 mmol, 81% yield) as a yellow solid. MS (ESI): 301.0 [M-isobutene+H]⁺.

### Step b) tert-butyl N-[(3R)-8-fluoro-7-(hydrazinecarbonyl)-4-oxo-3,5-dihydro-2H-1,5-benzothiazepin-3-yl]carbamate

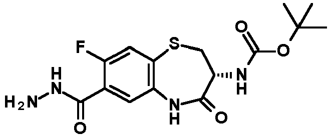

(3R)-3-(tert-butoxycarbonylamino)-8-fluoro-4-oxo-3,5-dihydro-2H-1,5-benzothiazepine-7-carboxylic acid (3.13 g, 6.76 mmol, Eq: 1) was dissolved in THF (30 mL) and CDI (1.43 g, 8.79 mmol, Eq: 1.3) was added and the yellow solution was stirred for 30 min. This first solution was then dropwise added to a second solution of hydrazine hydrate (1.02 g, 984 µl, 20.3 mmol, Eq: 3) in THF (10 mL) at RT. The reaction mixture was poured on water and was extracted with EtOAc three times. The combined organic layers were washed with brine, dried over magnesium sulfate, filtered and evaporated to afford the title compound (3.06 g, 6.2 mmol, 92% yield) as a yellow solid. MS (ESI): 315.0 [M-isobutene+H]⁺.

### Step c) tert-butyl N-[(3R)-7-[(2,2-dimethylpropanoylamino)carbamoyl]-8-fluoro-4-oxo-3,5-dihydro-2H-1,5-benzothiazepin-3-yl]carbamate

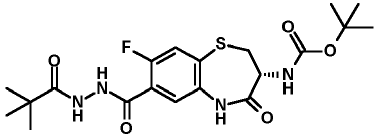

To a solution of tert-butyl N-[(3R)-8-fluoro-7-(hydrazinecarbonyl)-4-oxo-3,5-dihydro-2H-1,5-benzothiazepin-3-yl]carbamate (2.98 g, 6.03 mmol, Eq: 1) in THF (60 mL) were added pivalic acid (678 mg, 6.64 mmol, Eq: 1.1), HATU (2.52 g, 6.64 mmol, Eq: 1.1) and DIPEA (1.56 g, 2.11 ml, 12.1 mmol, Eq: 2) and the reaction mixture was stirred at RT for 2 h. The solvent was evaporated and the remaining residue was purified by chromatography on silica gel (50-100% EtOAc in heptane), affording the title compound (2.69 g, 5.92 mmol, 98% yield) as a light yellow solid. MS (ESI): 399.1 [M-isobutene+H]⁺.

### Step d) tert-butyl N-[(3R)-7-(5-tert-butyl-1,3,4-oxadiazol-2-yl)-8-fluoro-4-oxo-3, 5-dihydro-2H-1,5-benzothiazepin-3-yl]carbamate

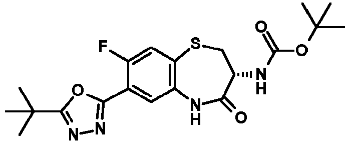

Burgess reagent (7.05 g, 29.6 mmol, Eq: 5) was added to a yellow solution of tert-butyl (R)-(8-fluoro-4-oxo-7-(2-pivaloylhydrazine-1-carbonyl)-2,3,4,5-tetrahydrobenzo[b][1,4]thiazepin-3-yl)carbamate (2.69 g, 5.92 mmol, Eq: 1) in THF (50 mL) and the reaction mixture was stirred at RT for 3 h. The solvent was evaporated and the crude residue was purified by chromatography on silica gel (0-10% MeOH in DCM) to obtain the title compound (2.57 g, 5.36 mmol, 91% yield) as a light yellow solid. MS (ESI): 381.1[M-isobutene+H]⁺.

### Step e) tert-butyl N-[(3R)-7-(5-tert-butyl-1,3, 4-oxadiazol-2-yl)-8-fluoro-1,1,4-trioxo-3, 5-dihydro-2H-1λ⁶,5-benzothiazepin-3-yl]carbamate

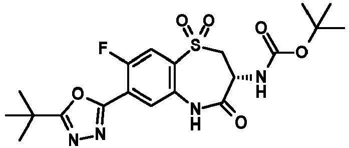

m-CPBA (117 mg, 521 µmol, Eq: 2.5) was added to a colorless solution of tert-butyl N-[(3R)-7-(5-tert-butyl-1,3,4-oxadiazol-2-yl)-8-fluoro-4-oxo-3,5-dihydro-2H-1,5-benzothiazepin-3-yl]carbamate (100 mg, 208 µmol, Eq: 1) in DCM (2 mL) and the reaction mixture was stirred at RT over night. 1N aqueous NaOH solution was added and the mixture was extracted with DCM. The organic layer was washed with water and brine, dried over magnesium sulfate, filtered and evaporated. The crude residue was purified by chromatography on silica gel (0-50% EtOAc in heptane), affording the title compound (65 mg, 135 µmol, 65% yield) as a white solid. MS (ESI): 413.0[M-isobutene+H]⁺.

### Step f) tert-butyl N-[(3R)-7-(5-tert-butyl-1,3, 4-oxadiazol-2-yl)-5-[(3-chlorophenyl)methyl]-8-fluoro-1,1, 4-trioxo-2,3-dihydro-1λ⁶,5-benzothiazepin-3-yl]carbamate

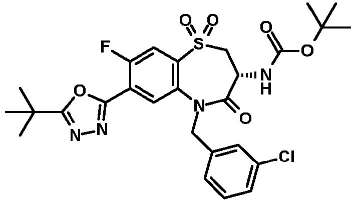

tert-butyl N-[(3R)-7-(5-tert-butyl-1,3,4-oxadiazol-2-yl)-8-fluoro-1,1,4-trioxo-3,5-dihydro-2H-1λ⁶,5-benzothiazepin-3-yl]carbamate (60 mg, 128 µmol, Eq: 1) was combined with 1-(bromomethyl)-3-chlorobenzene (28.9 mg, 18.5 µl, 141 µmol, Eq: 1.1), K₂CO₃ (53.1 mg, 384 µmol, Eq: 3) and KI (10.6 mg, 64 µmol, Eq: 0.5) in DMSO (0.6 mL) and the reaction mixture was stirred at RT for 1 h. Water was added and the mixture was extracted with EtOAc. The organic layer was washed with brine, dried over magnesium sulfate, filtered and evaporated. The crude residue was purified by flash chromatography on silica gel (0-50% EtOAc in heptane) to afford the title compound (53 mg, 89.4 µmol, 70% yield) as a white solid. MS (ESI): 537.1 [M-isobutene+H]⁺.

### Step g) (3R)-3-amino-7-(5-tert-butyl-1,3, 4-oxadiazol-2-yl)-5-[(3-chlorophenyl)methyl]-8-fluoro-1,1-dioxo-2, 3-dihydro-1λ⁶,5-benzothiazepin-4-one

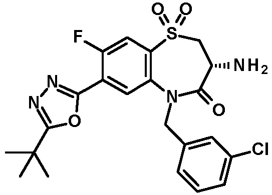

tert-butyl N-[(3R)-7-(5-tert-butyl-1,3,4-oxadiazol-2-yl)-5-[(3-chlorophenyl)methyl]-8-fluoro-1,1,4-trioxo-2,3-dihydro-1λ⁶,5-benzothiazepin-3-yl]carbamate (50 mg, 84.3 µmol, Eq: 1) was dissolved in HFIP (14.2 mg, 2 ml, 84.3 µmol, Eq: 1) and a few drops of HCl (4N in dioxane) were added. The reaction mixture was heated to 70°C and was stirred for 5 h. Saturated aqueous NaHCO₃ solution was added and the mixture was extracted with EtOAc. The organic layer was washed with water and brine, dried over magnesium sulfate, filtered and evaporated, affording the title compound (42 mg, 84 µmol, 100% yield) as a white solid. MS (ESI): 493.0[M+H]⁺.

### Intermediate 11

### 3-(chloromethyl)-5-[(4-chlorophenyl)methyl]pyridine, hydrochloride

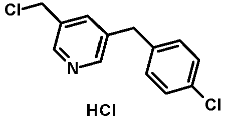

### Step a) 3-bromo-5-[(4-chlorophenyl)methyl]pyridine

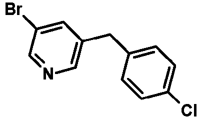

A mixture of N-[(E)-(5-bromo-3-pyridyl)methyleneamino]-4-methyl-benzenesulfonamide (CAS 2415435-97-9) (2000.0 mg, 5.65 mmol, 1 eq), 4-chlorophenylboronic acid (1324.35 mg, 8.47 mmol, 1.5 eq) and K₂CO₃ (2205.92 mg, 16.94 mmol, 3 eq) in 1,4-dioxane (40 mL) was heated to 110°C and stirred for 1 h under an atmosphere of nitrogen. The mixture was cooled to RT and filtered and the filter cake was washed with EtOAc (10 mL). The filtrate was washed with brine (2 x 20 mL), dried over anhydrous Na₂SO₄ and concentrated under vacuum to give a crude product which was purified by column chromatography on silica gel (PE/EA = 9:1 to 4:1) to obtain the title compound (1540 mg, 5.45 mmol, 94% yield) as a colorless oil. MS (ESI): 283.9 [M+H]⁺.

### Step b) methyl 5-[(4-chlorophenyl)methyl]pyridine-3-carboxylate

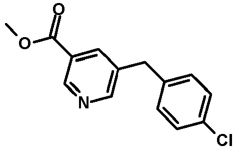

A solution of 3-bromo-5-[(4-chlorophenyl)methyl]pyridine (840.0 mg, 2.97 mmol, 1 eq), TEA (0.82 mL, 5.95 mmol, 2 eq) and Pd(dppf)Cl₂ (217.52 mg, 0.300 mmol, 0.100 eq) in a solvent mixture of MeOH (10 mL) and DMF (10 mL) was degassed with argon three times and then purged with CO three times. The mixture was heated to 80°C and stirred under CO (50 psi) for 24 h. The mixture was filtered, the filter cake was washed with MeOH (2 x 5 mL) and the filtrate was concentrated to remove all MeOH. EtOAc (10 mL) was added to the residue and the mixture was washed with brine (3 x 10 mL), dried over anhydrous Na₂SO₄ and concentrated under vacuum to give residue which was purified by column chromatography on silica gel (PE/EA = 9:1 to 4:1) to obtain the title compound (570 mg, 2.18 mmol, 70% yield) as a colorless oil. MS (ESI): 262.1 [M+H]⁺.

### Step c) [5-[(4-chlorophenyl)methyl]-3-pyridyl]methanol

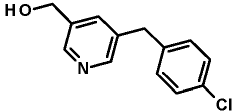

To a suspension of LiAlH₄ (79.76 mg, 2.1 mmol, 1.1 eq) in THF (10 mL) was added a solution of methyl 5-[(4-chlorophenyl)methyl]pyridine-3-carboxylate (500.0 mg, 1.91 mmol, 1 eq) in THF (2 mL) dropwise at 0°C under an atmosphere of nitrogen. The mixture was stirred for 10 min at 0°C and then warmed to 25°C and stirred for 2 h. The reaction was quenched by addition of water (0.013 mL) at 0°C and then 15% NaOH aqueous solution (0.013 mL) and water (0.039 mL) were added. Then the suspension was filterted through a pad of celite and the filter pad was washed with EtOAc (2 x 5 mL). The filtrate was washed with brine (2 x 10 mL), dried over anhydrous Na₂SO₄ and concentrated under vacuum to give the crude title compound (340 mg, 1.45 mmol, 34% yield) as a colorless oil which was unsed in the next reaction step without any further purification. MS (ESI): 234.1 [M+H]⁺.

### Step d) 3-(chloromethyl)-5-[(4-chlorophenyl)methyl]pyridine, hydrochloride

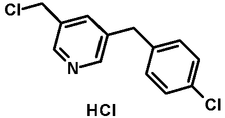

To a solution of [5-[(4-chlorophenyl)methyl]-3-pyridyl]methanol (50.0 mg, 0.210 mmol, 1 eq) in DCM (1 mL) was added thionyl chloride (0.09 mL, 1.28 mmol, 6 eq) at 0°C. The mixture was warmed to 25°C and stirred for 3 h. The reaction mixture was concentrated under vacuum, THF (3 mL) was added and then evaporated again to give the crude title compound (65 mg, 0.230 mmol, 76% yield) as a light brown solid which was used in the next reaction step without further purification. MS (ESI): 252.0 [M+H]⁺.

### Intermediate 12

### (6-isopropoxy-3-pyridyl)methyl methanesulfonate

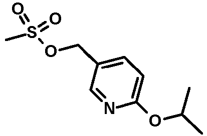

To a solution of (6-isopropoxy-3-pyridyl)methanol (CAS 1104461-69-9) (50 mg, 0.299 mmol, 1 eq) in DCM (1.25 mL) was added TEA (30.26 mg, 41.68 uL, 0.299 mmol, 1 eq). The reaction mixture was purged with argon and cooled to 0°C. Then mesyl chloride (34.25 mg, 23.3 uL, 0.299 mmol, 1 eq) was added dropwise and the mixture was stirred for 1.5 h. The reaction mixture was diluted with water and extracted with DCM three times. The combined organic layers were dried with MgSO₄ and filtered. The solvent was evaporated to obtain a colorless oil (126 mg) containing the title compound. This oil was used in the next reaction step without further purification.

### Intermediate 13

### [4-(11-acetamidoundecoxy)phenyl]methyl methanesulfonate

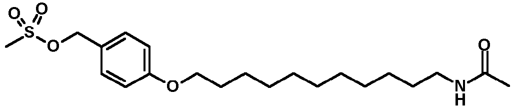

### Step a) [4-(11-aminoundecoxy)phenyl]methanol, hydrochloride

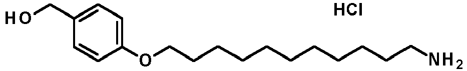

A mixture of tert-butyl N-tert-butoxycarbonyl-N-[11-[4-(hydroxymethyl)phenoxy]undecyl]carbamate (Example 257, step b) (500.0 mg, 1.01 mmol, 1 eq) in hydrochloric acid in EtOAc (15.0 mL, 60 mmol, 59.24 eq) was stirred at 20°C for 2 h. The mixture was concentrated in vacuum to give the crude title compound (330 mg, 1 mmol, 99% yield) as white solid which was used for next reaction step without further purification. MS (ESI): 276.3 [M+H-H₂O]⁺.

### Step b) N-[11-[4-(hydroxymethyl)phenoxy]undecyl]acetamide

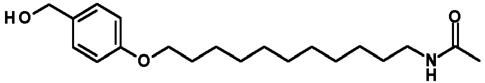

To a mixture of [4-(11-aminoundecoxy)phenyl]methanol, hydrochloride (200.0 mg, 0.610 mmol, 1 eq) and TEA (0.42 mL, 3.03 mmol, 5 eq) in DCM (8 mL) was added acetic anhydride (0.07 mL, 0.760 mmol, 1.26 eq) at 0°C under an atmosphere of nitrogen and the mixture was stirred at 0°C for 10 min and then at 20°C for 50 min. The mixture was poured into water (20 mL) and then extracted with DCM (20 mL). The combined organic layers were washed with brine (20 mL), dried over (Na₂SO₄), filtered and concentrated in vacuum to give a residue which was purified by column chromatography on silica gel (PE/EA = 10:1 to 1:1) to afford the title compound (106 mg, 0.320 mmol, 52% yield) as a white solid. MS (ESI): 318.3 [M+H-H₂O]⁺.

### Step c) [4-(11-acetamidoundecoxy)phenyl]methyl methanesulfonate

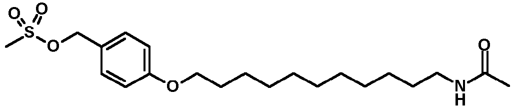

To a mixture of N-[11-[4-(hydroxymethyl)phenoxy]undecyl]acetamide (100.0 mg, 0.300 mmol, 1 eq) and TEA (0.15 mL, 1.09 mmol, 3.65 eq) and in DCM (4 mL) was added methanesulfonyl chloride (0.05 mL, 0.610 mmol, 2.05 eq) at 0°C and then the mixture was stirred at 20°C for 2 h. The mixture was concentrated in vacuum to give a yellow semisolid (300 mg) containing the title compound which was used as such in the next reaction step.

### Intermediate 14

### [4-(1,1,2,2-tetrafluoroethoxy)phenyl]methyl methanesulfonate

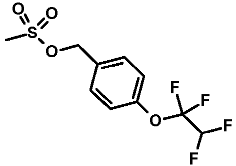

The intermediate was prepared in analogy to intermediate 12 from [4-(1,1,2,2-tetrafluoroethoxy)phenyl]methanol (CAS 773868-39-6) and was obtained as yellow liquid. Examples 220 to 251of the following table were prepared in analogy to Example 219, using the appropriate benzyl halide or benzyl sulfonate building block.

| **Ex.** | **Structure** | **Systematic Name** | **Building Block** | **MS, ESI: m/z** |
|---|---|---|---|---|
| 220 | 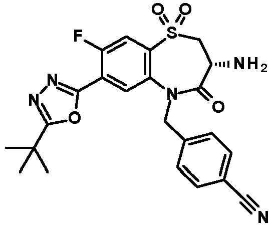 | 4-[[(3R)-3-amino-7-(5-tert-butyl-1,3,4-oxadiazol-2-yl)-8-fluoro-1,1,4-trioxo-2,3-dihydro-1λ⁶,5-benzothiazepin-5-yl]methyl]benzonitrile (***) | 4-(Bromome thyl)benzo nitrile | 484.4 [M+H]⁺ |
| | | | (CAS 17201-43-3) | |
| 221 | 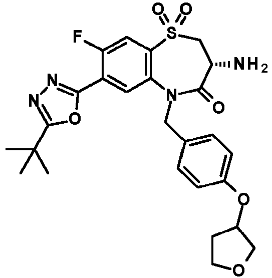 | (3R)-3-amino-7-(5-tert-butyl-1,3,4-oxadiazol-2-yl)-8-fluoro-1,1-dioxo-5-[(4-tetrahydrofuran-3-yloxyphenyl)methyl]-2,3-dihydro-1λ⁶,5-benzothiazepin-4-one (***) | 3-[4-(Chlorome thyl)pheno xy]tetrahy drofuran | 545.3 [M+H]⁺ |
| | | | (CAS 126049-18-1) | |
| 222 | 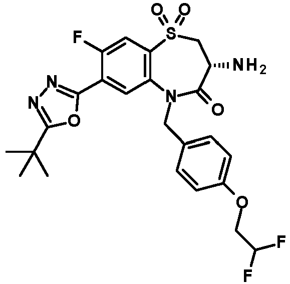 | (3R)-3-amino-7-(5-tert-butyl-1,3,4-oxadiazol-2-yl)-5-[[4-(2,2-difluoroethoxy)phenyl]methyl ]-8-fluoro-1,1-dioxo-2,3-dihydro-1λ⁶,5-benzothiazepin-4-one (*) | 1-(Chlorome thyl)-4-(2,2-difluoroeth oxy)benze ne | 539.2 [M+H]⁺ |
| | | | (CAS 1178393-11-7) | |
| 223 | 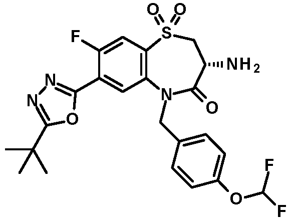 | (3R)-3-amino-7-(5-tert-butyl-1,3,4-oxadiazol-2-yl)-5-[[4-(difluoromethoxy)phenyl]met hyl]-8-fluoro-1,1-dioxo-2,3-dihydro-1λ⁶,5-benzothiazepin-4-one (***) | 1-(Bromome thyl)-4-(difluorom ethoxy)be nzene | 525.3 [M+H]⁺ |
| | | | (CAS 3447-53-8) | |
| 224 | 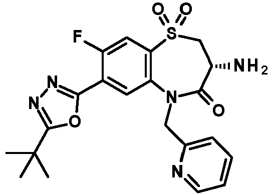 | (3R)-3-amino-7-(5-tert-butyl-1,3,4-oxadiazol-2-yl)-8-fluoro-1,1-dioxo-5-(2-pyridylmethyl)-2,3-dihydro-1λ⁶,5-benzothiazepin-4-one | 2-(Chlorome thyl)pyridi ne | 460.2 [M+H]⁺ |
| | | | (CAS 4377-33-7) | |
| 225 | 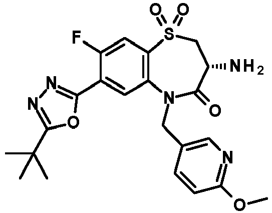 | (3R)-3-amino-7-(5-tert-butyl-1,3,4-oxadiazol-2-yl)-8-fluoro-5-[(6-methoxy-3-pyridyl)methyl]-1,1-dioxo-2,3-dihydro-1λ⁶,5-benzothiazepin-4-one (*) | 5-(Chlorome thyl)-2-methoxypyridine | 490.1 [M+H]⁺ |
| | | | (CAS 101990-70-9) | |
| 226 | 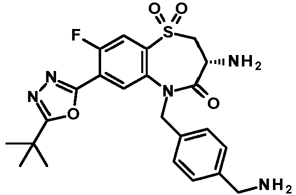 | (3R)-3-amino-5-[[4-(aminomethyl)phenyl]methyl] -7-(5-tert-butyl-1,3,4-oxadiazol-2-yl)-8-fluoro-1,1-dioxo-2,3-dihydro-1λ⁶,5-benzothiazepin-4-one | tert-Butyl N-[[4-(bromome thyl)pheny I]methyl]c arbamate | 488.1 [M+H]⁺ |
| | | | (CAS 187283-17-6) | |
| 227 | 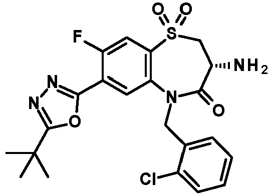 | (3R)-3-amino-7-(5-tert-butyl-1,3,4-oxadiazol-2-yl)-5-[(2-chlorophenyl)methyl]-8-fluoro-1,1-dioxo-2,3-dihydro-1λ⁶,5-benzothiazepin-4-one | 1-(Bromome thyl)-2-chlorobenzene | 493.1 [M+H]⁺ |
| | | | (CAS 611-17-6) | |
| 228 | 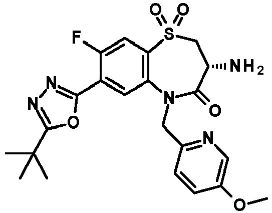 | (3R)-3-amino-7-(5-tert-butyl-1,3,4-oxadiazol-2-yl)-8-fluoro-5-[(5-methoxy-2-pyridyl)methyl]-1,1-dioxo-2,3-dihydro-1λ⁶,5-benzothiazepin-4-one (***) | 2-(Chlorome thyl)-5-methoxypyridine | 490.1 [M+H]⁺ |
| | | | (CAS 75342-33-5) | |
| 229 | 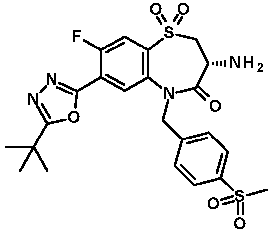 | (3R)-3-amino-7-(5-tert-butyl-1,3,4-oxadiazol-2-yl)-8-fluoro-5-[(4-methylsulfonylphenyl)methyl] -1,1-dioxo-2,3-dihydro-1λ⁶,5-benzothiazepin-4-one (***) | 1-(Chlorome thyl)-4-methylsulf onylbenzene | 537.2 [M+H]⁺ |
| | | | (CAS 40517-43-9) | |
| 230 | 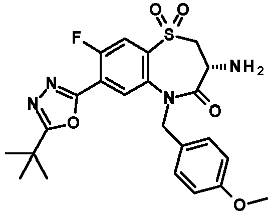 | (3R)-3-amino-7-(5-tert-butyl-1,3,4-oxadiazol-2-yl)-8-fluoro-5-[(4-methoxyphenyl)methyl]-1,1-dioxo-2,3-dihydro-1λ⁶,5-benzothiazepin-4-one (***) | 1-(Chlorome thyl)-4-methoxybenzene | 489.4 [M+H]⁺ |
| | | | (CAS 824-94-2) | |
| 231 | 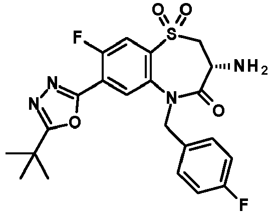 | (3R)-3-amino-7-(5-tert-butyl-1,3,4-oxadiazol-2-yl)-8-fluoro-5-[(4-fluorophenyl)methyl]-1,1-dioxo-2,3-dihydro-1λ⁶,5-benzothiazepin-4-one (***) | 1-(Chlorome thyl)-4-fluorobenzene | 477.4 [M+H]⁺ |
| | | | (CAS 352-11-4) | |
| 232 | 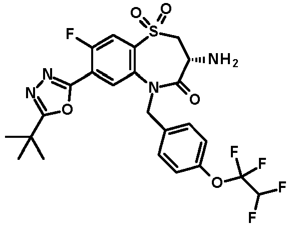 | (3R)-3-amino-7-(5-tert-butyl-1,3,4-oxadiazol-2-yl)-8-fluoro-1,1-dioxo-5-[[4-(1,1,2,2-tetrafluoroethoxy)phenyl]met hyl]-2,3-dihydro-1λ⁶,5-benzothiazepin-4-one (*) | [4-(1,1,2,2-Tetrafluor oethoxy)p henyl]met hyl methanesu Ifonate | 575.2 [M+H]⁺ |
| | | | (Intermedi ate 14) | |
| 233 | 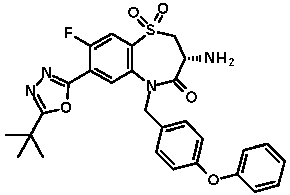 | (3R)-3-amino-7-(5-tert-butyl-1,3,4-oxadiazol-2-yl)-8-fluoro-1,1-dioxo-5-[(4-phenoxyphenyl)methyl]-2,3-dihydro-1λ⁶,5-benzothiazepin-4-one (*) | 1-(Bromome thyl)-4-phenoxybenzene | 551.3 [M+H]⁺ |
| | | | (CAS 36881-42-2) | |
| 234 | 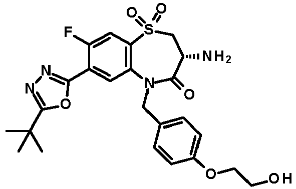 | (3R)-3-amino-7-(5-tert-butyl-1,3,4-oxadiazol-2-yl)-8-fluoro-5-[[4-(2-hydroxyethoxy)phenyl]methyl ]-1,1-dioxo-2,3-dihydro-1λ⁶,5-benzothiazepin-4-one (*****) | [4-[2-[tert-Butyl(dime thyl)silyl]o xyethoxy]p henyl]met hyl methanesu lfonate | 519.2 [M+H]⁺ |
| | | | (CAS 2244238-45-5) | |
| 235 | 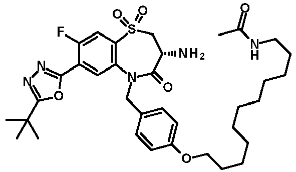 | N-[11-[4-[[(3R)-3-amino-7-(5-tert-butyl-1,3,4-oxadiazol-2-yl)-8-fluoro-1,1,4-trioxo-2,3-dihydro-1 λ⁶,5-benzothiazepin-5-yl]methyl]phenoxy]undecyl] acetamide (*) | 4-(11-Acetamido undecoxy) phenyl]me thyl methanesu lfonate | 686.6 [M+H]⁺ |
| | | | (Intermedi ate 13) | |
| 236 | 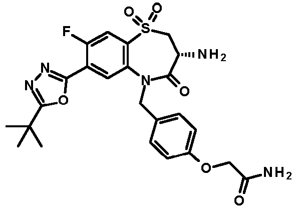 | 2-[4-[[(3R)-3-amino-7-(5-tert-butyl-1,3,4-oxadiazol-2-yl)-8-fluoro-1,1,4-trioxo-2,3-dihydro-1 λ⁶,5-benzothiazepin-5-yl]methyl]phenoxy]acetamide (***) | 2-[4-(Chlorome thyl)pheno xy]acetami de | 532.3 [M+H]⁺ |
| | | | (CAS 1012-20-0) | |
| 237 | 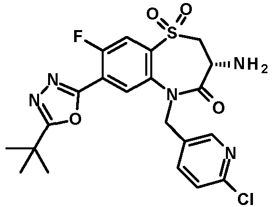 | (3R)-3-amino-7-(5-tert-butyl-1,3,4-oxadiazol-2-yl)-5-[(6-chloro-3-pyridyl)methyl]-8-fluoro-1,1-dioxo-2,3-dihydro-1λ⁶,5-benzothiazepin-4-one (***) | 5-(Bromome thyl)-2-chloropyridine | 494.2 [M+H]⁺ |
| | | | (CAS 182924-36-3) | |
| 238 | 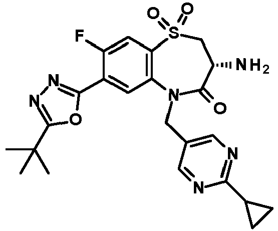 | (3R)-3-amino-7-(5-tert-butyl-1,3,4-oxadiazol-2-yl)-5-[(2-cyclopropylpyrimidin-5-yl)methyl]-8-fluoro-1,1-dioxo-2,3-dihydro-1λ⁶,5-benzothiazepin-4-one (***) | 5-(Bromome thyl)-2-cyclopropy l-pyrimidine | 501.2 [M+H]⁺ |
| | | | (CAS 1823028-59-6) | |
| 239 | 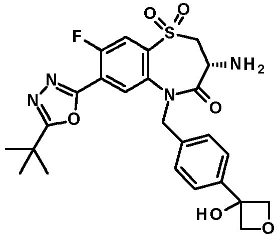 | (3R)-3-amino-7-(5-tert-butyl-1,3,4-oxadiazol-2-yl)-8-fluoro-5-[[4-(3-hydroxyoxetan-3-yl)phenyl]methyl]-1,1-dioxo-2,3-dihydro-1 λ⁶,5-benzothiazepin-4-one | 3-[4-(Chlorome thyl)pheny l]oxetan-3-ol | 531.2 [M+H]⁺ |
| | | | (CAS 2231294-32-7) | |
| 240 | 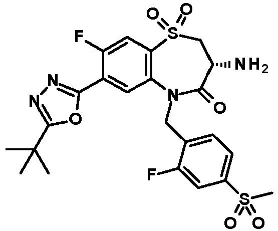 | (3R)-3-amino-7-(5-tert-butyl-1,3,4-oxadiazol-2-yl)-8-fluoro-5-[(2-fluoro-4-methylsulfonylphenyl)methyl]-1,1-dioxo-2,3-dihydro-1λ⁶,5-benzothiazepin-4-one (***) | 1-(Bromome thyl)-2-fluoro-4-methylsulf onylbenzene | 555.3 [M+H]⁺ |
| | | | (CAS 1401796-84-6) | |
| 241 | 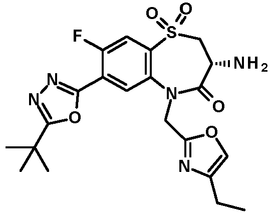 | (3R)-3-amino-7-(5-tert-butyl-1,3,4-oxadiazol-2-yl)-5-[(4-ethyloxazol-2-yl)methyl]-8-fluoro-1,1-dioxo-2,3-dihydro-1λ⁶,5-benzothiazepin-4-one | 2-(Chlorome thyl)-4-ethyl-oxazole | 478.2 [M+H]⁺ |
| | | | (CAS 224441-76-3) | |
| 242 | 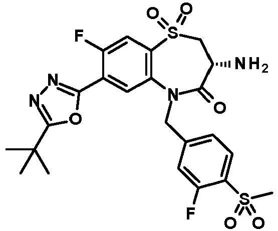 | (3R)-3-amino-7-(5-tert-butyl-1,3,4-oxadiazol-2-yl)-8-fluoro-5-[(3-fluoro-4-methylsulfonyl-phenyl)methyl]-1,1-dioxo-2,3-dihydro-1λ⁶,5-benzothiazepin-4-one (*) | 4-(Bromome thyl)-2-fluoro-1-methylsulf onyl-benzene | 555.4 [M+H]⁺ |
| | | | (CAS 942199-49-7) | |
| 243 | 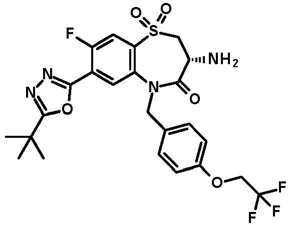 | (3R)-3-amino-7-(5-tert-butyl-1,3,4-oxadiazol-2-yl)-8-fluoro-1,1-dioxo-5-[[4-(2,2,2-trifluoroethoxy)phenyl]methyl ]-2,3-dihydro-1λ⁶,5-benzothiazepin-4-one | 1-(Bromome thyl)-4-(2,2,2-trifluoroet hoxy)benz ene | 557.1 [M+H]⁺ |
| | | | (CAS 126300-83-2) | |
| 244 | 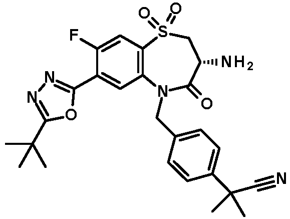 | 2-methyl-2-[4-[[(3R)-3-amino-7-(5-tert-butyl-1,3,4-oxadiazol-2-yl)-8-fluoro-1,1,4-trioxo-2,3-dihydro-1λ⁶,5-benzothiazepin-5-yl]methyl]phenyl]propanenitri le | 2-[4-(Bromome thyl)pheny l]-2-methyl-propanenit rile | 526.0 [M+H]⁺ |
| | | | (CAS 1201643-73-3) | |
| 245 | 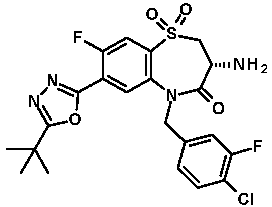 | (3R)-3-amino-7-(5-tert-butyl-1,3,4-oxadiazol-2-yl)-5-[(4-chloro-3-fluorophenyl)methyl]-8-fluoro-1,1-dioxo-2,3-dihydro-1λ⁶,5-benzothiazepin-4-one (*) | 4-(Bromome thyl)-1-chloro-2-fluorobenzene | 511.2 [M+H]⁺ |
| | | | (CAS 206362-80-3) | |
| 246 | 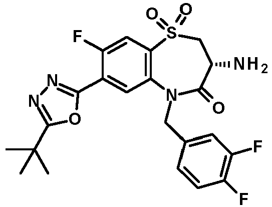 | (3R)-3-amino-7-(5-tert-butyl-1,3,4-oxadiazol-2-yl)-5-[(3,4-difluorophenyl)methyl]-8-fluoro-1,1-dioxo-2,3-dihydro-1λ⁶,5-benzothiazepin-4-one (*) | 4-(Bromome thyl)-1,2-difluorobenzene | 495.3 [M+H]⁺ |
| | | | (CAS 85118-01-0) | |
| 247 | 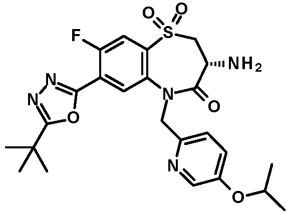 | (3R)-3-amino-7-(5-tert-butyl-1,3,4-oxadiazol-2-yl)-8-fluoro-5-[(5-isopropoxy-2-pyridyl)methyl]-1,1-dioxo-2,3-dihydro-1λ⁶,5-benzothiazepin-4-one (*) | 2-(Chlorome thyl)-5-isopropoxy -pyridine | 518.4 [M+H]⁺ |
| | | | (CAS 1315367-40-8) | |
| 248 | 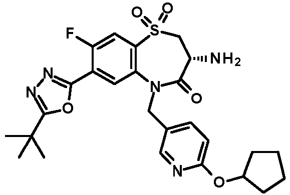 | (3R)-3-amino-7-(5-tert-butyl-1,3,4-oxadiazol-2-yl)-5-[[6-(cyclopentoxy)-3-pyridyl]methyl]-8-fluoro-1,1-dioxo-2,3-dihydro-1λ⁶,5-benzothiazepin-4-one | 5-(Chlorome thyl)-2-(cyclopent oxy)pyridin e | 544.3 [M+H]⁺ |
| | | | (CAS 250546-74-7) | |
| 249 | 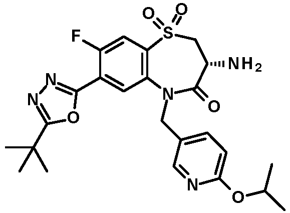 | (3R)-3-amino-7-(5-tert-butyl-1,3,4-oxadiazol-2-yl)-8-fluoro-5-[(6-isopropoxy-3-pyridyl)methyl]-1,1-dioxo-2,3-dihydro-1λ⁶,5-benzothiazepin-4-one (*) | (6-Isopropoxy -3-pyridyl)me thyl methanesu lfonate | 518.3 [M+H]⁺ |
| | | | (Intermedi ate 12) | |
| 251 | 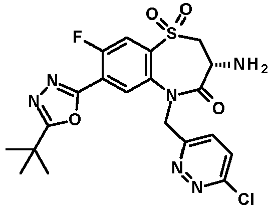 | (3R)-3-amino-7-(5-tert-butyl-1,3,4-oxadiazol-2-yl)-5-[(6-chloropyridazin-3-yl)methyl]-8-fluoro-1,1-dioxo-2,3-dihydro-1λ⁶,5-benzothiazepin-4-one | 3-Chloro-6-(chloromet hyl)pyridaz ine | 495.3 [M+H]⁺ |
| | | | (CAS 120276-59-7) | |

| | | | | |
|---|---|---|---|---|
| * as a hydrochloride salt ** as a 1,1,1,3,3,3-hexafluoropropan-2-ol adduct *** as a hydrochloride salt and 1,1,1,3,3,3-hexafluoropropan-2-ol adduct **** as a formic acid salt ***** as TFA salt | | | | |

### Example 252

### (3R)-3-amino-7-(5-tert-butyl-1,3,4-oxadiazol-2-yl)-5-[[5-[(4-chlorophenyl)methyl]-3-pyridyl]methyl]-8-fluoro-1,1-dioxo-2,3-dihydro-1λ⁶,5-benzothiazepin-4-one

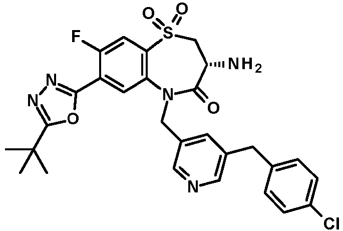

### Step a) tert-butylN-[(3R)-7-(5-tert-butyl-1,3, 4-oxadiazol-2-yl)-5-[[5-[(4-chlorophenyl)methyl]-3-pyridyl]methyl]-8-fluoro-4-oxo-2,3-dihydro-1,5-benzothiazepin-3-yl]carbamate

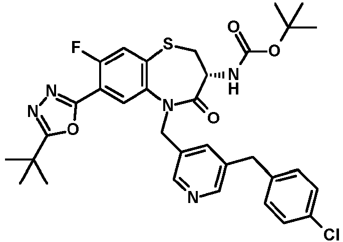

To a solution of tert-butyl N-[(3R)-7-(5-tert-butyl-1,3,4-oxadiazol-2-yl)-8-fluoro-4-oxo-3,5-dihydro-2H-1,5-benzothiazepin-3-yl]carbamate (Example 219, step d) (100.0 mg, 0.230 mmol, 1 eq) in DMF (3 mL) were added potassium carbonate (158.31 mg, 1.15 mmol, 5 eq), potassium iodide (19.02 mg, 0.110 mmol, 0.500 eq) and a solution of 3-(chloromethyl)-5-[(4-chlorophenyl)methyl]pyridine hydrochloride (Intermediate 11) (86.24 mg, 0.270 mmol, 1.2 eq) in DMF (1.5 mL) at 25°C and the reaction mixture was stirred at 25°C for 3 h. The mixture was diluted with EtOAc (5 mL), washed with brine (3 x 5 mL) and dried over anhydrous Na₂SO₄. After filtration, the organic layer was concentrated to dryness in vacuo to get the crude product which was purified by prep-TLC (PE:EA = 1:1) to obtain the title compound (110 mg, 0.170 mmol, 72% yield) as a white solid. MS (ESI): 652.3 [M+H]⁺.

### Step b) tert-butyl N-[(3R)-7-(5-tert-butyl-1,3, 4-oxadiazol-2-yl)-5-[[5-[(4-chlorophenyl)methyl]-1-oxido-pyridin-1-ium-3-yl]methyl]-8-fluoro-1,1, 4-trioxo-2,3-dihydro-1λ⁶,5-benzothiazepin-3-yl]carbamate

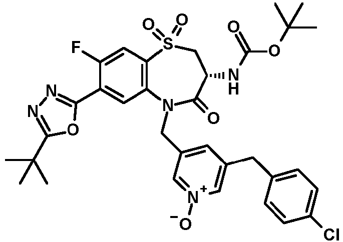

To a solution of tert-butyl N-[(3R)-7-(5-tert-butyl-1,3,4-oxadiazol-2-yl)-5-[[5-[(4-chlorophenyl)methyl]-3-pyridyl]methyl]-8-fluoro-4-oxo-2,3-dihydro-1,5-benzothiazepin-3-yl]carbamate (110.0 mg, 0.170 mmol, 1 eq) in DCM (4 mL) was added m-CPBA (90.95 mg, 0.420 mmol, 2.5 eq) and the mixture was stirred for 5 h at 25°C. The reaction mixture was diluted with DCM (10 mL) and then washed with saturated aqueous Na₂SO₃ solution (10 mL), saturated aqueous NaHCO₃ solution (2 x 15 mL) and brine (15 mL), dried over anhydrous Na₂SO₄ and concentrated to give a white solid containing the title compound (113 mg) which was used in the next reaction step without any further purification. MS (ESI): 700.3 [M+H]⁺.

### Step c) tert-butyl N-[(3R)-7-(5-tert-butyl-1,3, 4-oxadiazol-2-yl)-5-[[5-[(4-chlorophenyl)methyl]-3-pyridyl]methyl]-8-fluoro-1,1, 4-trioxo-2,3-dihydro-1λ⁶,5-benzothiazepin--3-yl]carbamate

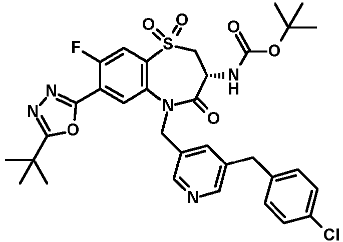

To a solution of tert-butyl N-[(3R)-7-(5-tert-butyl-1,3,4-oxadiazol-2-yl)-5-[[5-[(4-chlorophenyl)methyl]-1-oxido-pyridin-1-ium-3-yl]methyl]-8-fluoro-1,1,4-trioxo-2,3-dihydro-1λ⁶,5-benzothiazepin-3-yl]carbamate (118.41 mg, 0.170 mmol, 1 eq) in MeCN (4 mL) was added bis(pinacolato)diboron (42.94 mg, 0.170 mmol, 1 eq). The mixture was degassed with nitrogen for three times, stirred for 10 min at 25°C and then it was heated to 70°C and stirred for 7 h. The reaction mixture was concentrated under vacuum to give a brown oil containing the title compound (150 mg) which was used in the next step without any further purification. MS (ESI): 684.2 [M+H] ⁺.

### Step d) (3R)-3-amino-7-(5-tert-butyl-1,3,4-oxadiazol-2-yl)-5-[[5-[(4-chlorophenyl)methyl]-3-pyridyl]methyl]-8-fluoro-1,1-dioxo-2,3-dihydro-1λ⁶,5-benzothiazepin-4-one

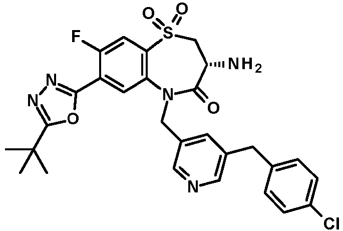

The title compound was prepared from tert-butyl N-[(3R)-7-(5-tert-butyl-1,3,4-oxadiazol-2-yl)-5-[[5-[(4-chlorophenyl)methyl]-3-pyridyl]methyl]-8-fluoro-1,1,4-trioxo-2,3-dihydro-1λ⁶,5-benzothiazepin--3-yl]carbamate (150.0 mg, 0.220 mmol, 1 eq) in analogy to general procedure 6b and was obtained as white solid as the hydrochloride salt (19.1 mg, 0.030 mmol, 14% yield). MS (ESI): 584.3 [M+H]⁺.

### Example 253

### (3R)-3-amino-7-(5-tert-butyl-1,3,4-oxadiazol-2-yl)-5-[[4-(cyclopentoxy)phenyl]methyl]-8-fluoro-1,1-dioxo-2,3-dihydro-1λ⁶,5-benzothiazepin-4-one

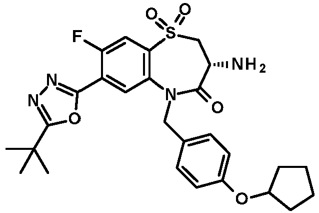

### Step a) tert-butyl N-[(3R)-7-(5-tert-butyl-1,3,4-oxadiazol-2-yl)-5-[[4-(cyclopentoxy)phenyl]methyl]-8-fluoro-4-oxo-2,3-dihydro-1,5-benzothiazepin-3-yl]carbamate

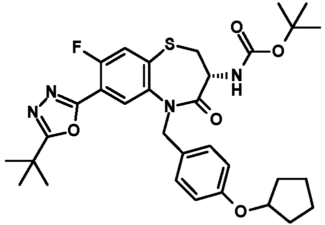

To a solution of tert-butyl N-[(3R)-7-(5-tert-butyl-1,3,4-oxadiazol-2-yl)-8-fluoro-4-oxo-3,5-dihydro-2H-1,5-benzothiazepin-3-yl]carbamate (Example 219, step d) (200.0 mg, 0.460 mmol, 1 eq) in DMF (3 mL) were added potassium carbonate (126.65 mg, 0.920 mmol, 2 eq), potassium iodide (38.03 mg, 0.230 mmol, 0.500 eq) and a solution of 1-(chloromethyl)-4-(cyclopentoxy)benzene (CAS 1041579-01-4) (193.08 mg, 0.920 mmol, 2 eq) in DMF (1.5 mL) at 25°C and the mixture was stirred for 3 h. The reaction mixture was diluted with EtOAc (5 mL), washed with brine (3 x 5 mL) and dried over anhydrous Na₂SO₄. After filtration, the organic layer was concentrated to dryness in vacuo to get the crude product which was purified by column chromatography on silica gel (PE/EtOAc = 9:1 to 3:2) to obtain the title compound (250 mg, 0.410 mmol, 60% yield) as a light yellow solid. MS (ESI): 611.3 [M+H]⁺.

### Step b) tert-butyl N-[(3R)-7-(5-tert-butyl-1,3,4-oxadiazol-2-yl)-5-[[4-(cyclopentoxy)phenyl]methyl]-8-fluoro-1,1,4-trioxo-2,3-dihydro-1λ⁶,5-benzothiazepin-3-yl]carbamate

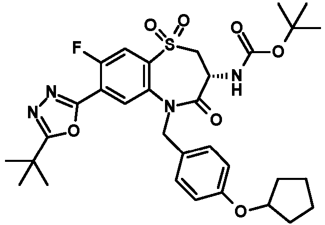

The title compound was prepared from tert-butyl N-[(3R)-7-(5-tert-butyl-1,3,4-oxadiazol-2-yl)-5-[[4-(cyclopentoxy)phenyl]methyl]-8-fluoro-4-oxo-2,3-dihydro-1,5-benzothiazepin-3-yl]carbamate (200.0 mg, 0.330 mmol, 1 eq) in analogy to general procedure 5 and was obtained as light yellow solid (220 mg, 0.340 mmol, 87% yield). MS (ESI): 665.1 [M+Na]⁺.

### Step c) (3R)-3-amino-7-(5-tert-butyl-1,3,4-oxadiazol-2-yl)-5-[[4-(cyclopentoxy)phenyl[methyl]-8-fluoro-1,1-dioxo-2,3-dihydro-1λ⁶,5-benzothiazepin-4-one

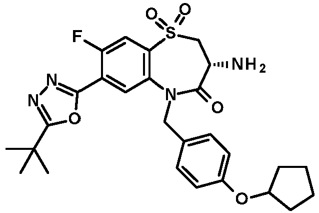

The title compound was prepared from tert-butyl N-[(3R)-7-(5-tert-butyl-1,3,4-oxadiazol-2-yl)-5-[[4-(cyclopentoxy)phenyl]methyl]-8-fluoro-1,1,4-trioxo-2,3-dihydro-1λ⁶,5-benzothiazepin-3-yl]carbamate (220.0 mg, 0.340 mmol, 1 eq) in analogy to general procedure **6b** and was obtained as white solid (81.4 mg, 0.140 mmol, 40% yield). MS (ESI): 543.3 [M+H]⁺.

Examples 254 and 255 of the following table were prepared in analogy to Example 253, using the appropriate benzyl halide or benzyl sulfonate building block.

| **Ex.** | **Structure** | **Systematic Name** | **Building Block** | **MS, ESI: m/z** |
|---|---|---|---|---|
| 254 | 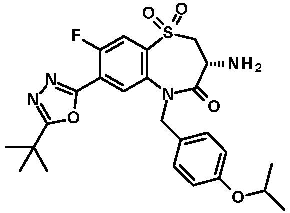 | (3R)-3-amino-7-(5-tert-butyl-1,3,4-oxadiazol-2-yl)-8-fluoro-5-[(4-isopropoxyphenyl)methyl]-1,1-dioxo-2,3-dihydro-1λ⁶,5-benzothiazepin-4-one (*) | 1-(Chlorome thyl)-4-isopropoxy -benzene | 517.3 [M+H]⁺ |
| | | | (CAS 40141-12-6) | |
| 255 | 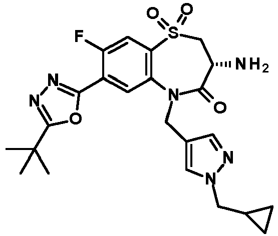 | (3R)-3-amino-7-(5-tert-butyl-1,3,4-oxadiazol-2-yl)-5-[[1-(cyclopropylmethyl)pyrazol-4-yl]methyl]-8-fluoro-1,1-dioxo-2,3-dihydro-1λ⁶,5-benzothiazepin-4-one | 4-(Chlorome thyl)-1-(cycloprop ylmethyl)p yrazole | 503.2 [M+H]⁺ |
| | | | (CAS 1339919-93-5) | |

| | | | | |
|---|---|---|---|---|
| * as a hydrochloride salt | | | | |

### Example 256

### (3R)-3-amino-5-[[2-(aminomethyl)-4-chloro-phenyl]methyl]-7-(5-tert-butyl-1,3,4-oxadiazol-2-yl)-8-fluoro-1,1-dioxo-2,3-dihydro-1λ⁶,5-benzothiazepin-4-one

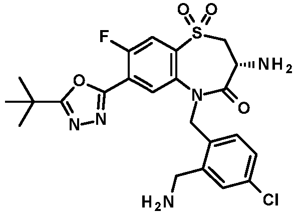

### Step a) tert-butyl N-[(3R)-7-(5-tert-butyl-1,3,4-oxadiazol-2-yl)-5-[(4-chloro-2-cyanophenyl)methyl]-8-fluoro-1,1,4-trioxo-2,3-dihydro-1λ⁶,5-benzothiazepin-3-yl]carbamate

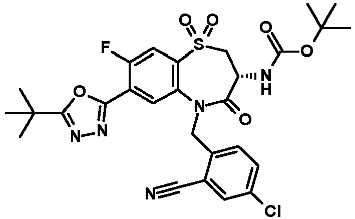

To a stirred mixture of tert-butyl N-[(3R)-7-(5-tert-butyl-1,3,4-oxadiazol-2-yl)-8-fluoro-1,1,4-trioxo-3,5-dihydro-2H-1λ⁶,5-benzothiazepin-3-yl]carbamate (Example 219, step e) (150.0 mg, 0.320 mmol, 1 eq), Na₂CO₃ (74.66 mg, 0.700 mmol, 2.2 eq) and KI (33.21 mg, 0.200 mmol, 0.620 eq) in DMF (4 mL) was added a solution of 2-(bromomethyl)-5-chloro-benzonitrile (88.56 mg, 0.380 mmol, 1.2 eq) in DMF (4 mL) within 4 h at 25 °C and then the mixture was stirred at 25 °C for 4 h. The reaction mixture was filtered and the filter cake was washed with EtOAc (10 mL). To the filtrate was added EtOAc (10 mL) and the resulting organic phase was washed with brine (15 mL x 3), dried over anhydrous Na₂SO₄ and concentrated under vacuum to afford the title compound (200 mg, 0.320 mmol, 79% yield) as a yellow solid. MS (ESI): 562.1 [M-isobutene+H]⁺.

### Step b) tert-butyl N-[(3R)-5-[[2-(aminomethyl)-4-chloro-phenyl]methyl]-7-(5-tert-butyl-1,3,4-oxadiazol-2-yl)-8-fluoro-1,1,4-trioxo-2,3-dihydro-1λ⁶5-benzothiazepin-3-yl]carbamate

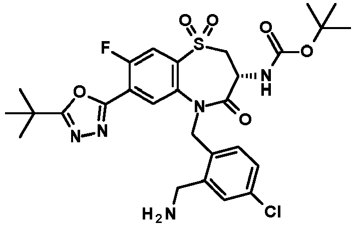

To a solution of tert-butyl N-[(3R)-7-(5-tert-butyl-1,3,4-oxadiazol-2-yl)-5-[(4-chloro-2-cyanophenyl)methyl]-8-fluoro-1,1,4-trioxo-2,3-dihydro-1λ⁶,5-benzothiazepin-3-yl]carbamate (160.0 mg, 0.260 mmol, 1 eq) in a mixture of MeOH (4 mL) and THF (8 mL) were added Raney-Ni (33.55 mg, 0.260 mmol, 1 eq) and ammonium hydroxide (0.4 mL, 3.2 mmol, 12.34 eq) and the mixture was stirred under an atmosphere of hydrogen at 25 °C for 16 h. The reaction mixture was carefully filtered with diatomite and the filter cake was washed with THF (5 mL). The filtrate was concentrated under vacuum affording the title compound (100 mg, 0.160 mmol, 53 % yield) as a white solid. MS (ESI): 622.2 [M+H]⁺.

### Step c) (3R)-3-amino-5-[[2-(aminomethyl)-4-chloro-phenyl]methyl]-7-(5-tert-butyl-1,3,4-oxadiazol-2-yl)-8-fluoro-1,1-dioxo-2,3-dihydro-1λ⁶,5-benzothiazepin-4-one

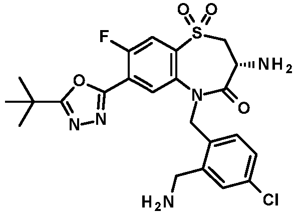

To a solution of tert-butyl N-[(3R)-5-[[2-(aminomethyl)-4-chloro-phenyl]methyl]-7-(5-tert-butyl-1,3,4-oxadiazol-2-yl)-8-fluoro-1,1,4-trioxo-2,3-dihydro-1λ⁶,5-benzothiazepin-3-yl]carbamate (100.0 mg, 0.160 mmol, 1 eq) in EtOAc (0.500 mL) was added EtOAc/HCl (2.0 mL, 8 mmol, 49.77 eq) at 0 °C. The mixture was stirred at 25 °C for 2 h. After the reaction mixture was concentrated under vacuum, the residue was purified by prep-HPLC affording the title compound (41.6 mg, 0.070 mmol, 45% yield) as a yellow solid as a hydrochloride salt. MS (ESI): 522.1 [M+H]⁺.

### Example 257

### (3R)-3-amino-5-[[4-(11-aminoundecoxy)phenyl]methyl]-7-(5-tert-butyl-1,3,4-oxadiazol-2-yl)-8-fluoro-1,1-dioxo-2,3-dihydro-1λ⁶,5-benzothiazepin-4-one

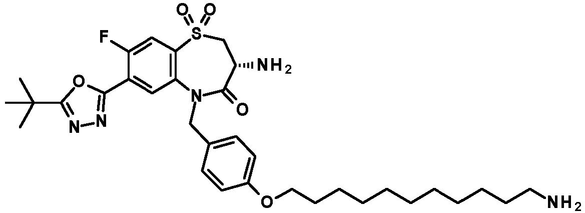

### Step a) tert-butyl N-(11-bromoundecyl)-N-tert-butoxycarbonyl-carbamate

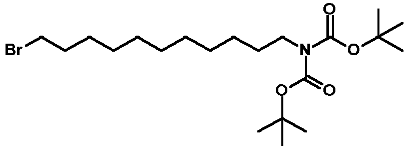

To a mixture of 1,11-dibromoundecane (8000.0 mg, 25.47 mmol, 1.84 eq) and cesium carbonate (9000.0 mg, 27.62 mmol, 2 eq) in MeCN (80 mL) was added di-tert-butyl iminodicarboxylate (3000.0 mg, 13.81 mmol, 1 eq) at 20°C and then the mixture was stirred at 70 °C for 8 h. The mixture was poured into water (600 mL) and was then extracted with EtOAc (300 mL x 2), the combined organic layers were washed with brine (200 mL), dried over Na₂SO₄, filtered and concentrated in vacuum to give a residue which was then purified by column chromatography (PE:EtOAc = 1:0 to 80:1) to afford the title compound (4000 mg, 8.88 mmol, 64% yield) as a colorless oil. MS (ESI): 474.2 [M+Na]⁺.

### Step b) tert-butyl N-tert-butoxycarbonyl-N-[11-[4-(hydroxymethyl)phenoxy]undecyl]carbamate

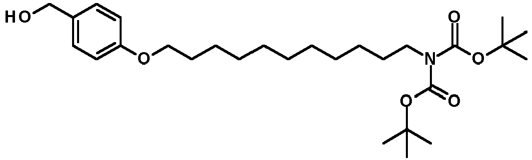

A mixture of 4-hydroxybenzyl alcohol (500.0 mg, 4.03 mmol, 1 eq), tert-butyl N-(11-bromoundecyl)-N-tert-butoxycarbonyl-carbamate (1905.0 mg, 4.23 mmol, 1.05 eq) and potassium carbonate (1200.0 mg, 8.68 mmol, 2.16 eq) in MeCN (40 mL) was stirred for 8 h at 80°C.The mixture was poured into water (300 mL) and was then extracted with EtOAc (250 mL x 2), the combined organic layers were washed with brine (200 mL), dried over Na₂SO₄, filtered and concentrated in vacuum to give a residue which was then purified by column chromatography (PE:EtOAc = 1:0 to 6:1) to afford the title compound (1490 mg, 3.02 mmol, 75% yield) as a colorless oil. MS (ESI): 516.4 [M+Na]⁺.

### Step c) [4-[11-[bis(tert-butoxycarbonyl)amino]undecoxy]phenyl]methyl methanesulfonate

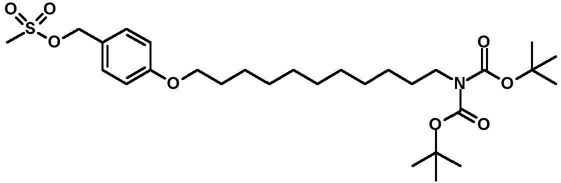

To a mixture of tert-butyl N-tert-butoxycarbonyl-N-[11-[4-(hydroxymethyl)phenoxy]undecyl]carbamate (100.0 mg, 0.200 mmol, 1 eq) and NEt₃ (0.1 mL, 0.720 mmol, 3.54 eq) in DCM (4 mL) was added methanesulfonyl chloride (0.03 mL, 0.440 mmol, 2.15 eq) at 0°C and then the mixture was stirred at 20°C for 3 h. The mixture was poured into water (20 mL) and was then extracted with DCM (25 mL). The organic layer was separated and washed with brine (30 mL), dried over Na₂SO₄, filtered and concentrated in vacuum affording a residue containing the title compound (150 mg, 0.260 mmol) as yellow oil.

### Step d) tert-butyl N-tert-butoxycarbonyl-N-[11-[4-[[(3R)-3-(tert-butoxycarbonylamino)-7-(5-tert-butyl-1,3,4-oxadiazol-2-yl)-8-fluoro-11,1,4-trioxo-2,3-dihydro-1λ⁶,5-benzothiazepin-5-yl]methyl]phenoxy]undecyl]carbamate

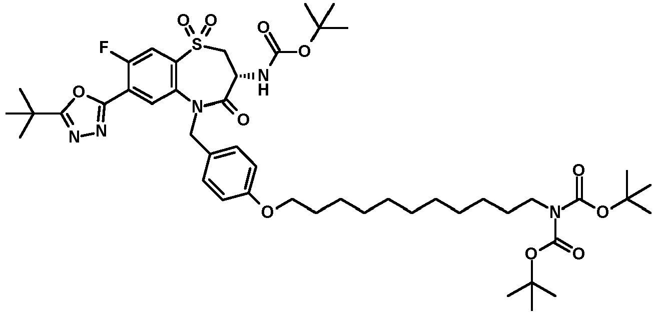

To a mixture of tert-butyl N-[(3R)-7-(5-tert-butyl-1,3,4-oxadiazol-2-yl)-8-fluoro-1,1,4-trioxo-3,5-dihydro-2H-1λ⁶,5-benzothiazepin-3-yl]carbamate (Example 219, step e) (80.0 mg, 0.580 mmol, 3.87 eq) and potassium iodide (20.0 mg, 0.120 mmol, 0.810 eq) in DMF (4 mL) was slowly added a solution of [4-[11-[bis(tert-butoxycarbonyl)amino]undecoxy]phenyl]methyl methanesulfonate (150.0 mg, 0.260 mmol, 1.76 eq) in DMF (1 mL) at 20°C and then the mixture was stirred at 20°C for 4 h. The mixture was poured into water (60 mL) and then extracted with EtOAc (40 mL*2). The combined organic layers were washed with brine (40 mL), dried over Na₂SO₄, filtered and concentrated in vacuum to give a residue which was purified by column chromatography (PE:EtOAc = 15:1 to 3:1) to afford the title compound (50 mg, 0.050 mmol, 35% yield) as a colorless oil. MS (ESI): 966.5 [M+Na] ⁺.

### Step e) (3R)-3-amino-5-[[4-(11-aminoundecoxy)phenyl]methyl]-7-(5-tert-butyl-1,3,4-oxadiazol-2-yl)-8-fluoro-1,1-dioxo-2,3-dihydro-1λ⁶,5-benzothiazepin-4-one

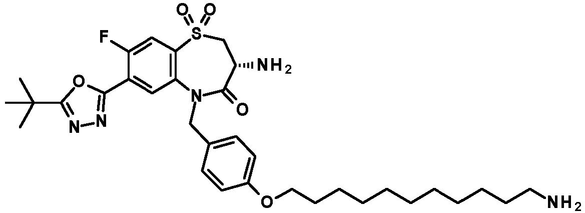

To a mixture of tert-butyl N-tert-butoxycarbonyl-N-[11-[4-[[(3R)-3-(tert-butoxycarbonylamino)-7-(5-tert-butyl-1,3,4-oxadiazol-2-yl)-8-fluoro-1,1,4-trioxo-2,3-dihydro-1λ⁶,5-benzothiazepin-5-yl]methyl]phenoxy]undecyl]carbamate (50.0 mg, 0.050 mmol, 1 eq) in EtOAc (1 mL) was added HCl in EtOAc (2.0 mL, 8 mmol, 151.07 eq) at 20°C, and then the mixture was stirred at 20°C for 1 h. The mixture was concentrated in vacuum to give a crude product. The crude was then purified by prep-HPLC affording the title compound (10.3 mg, 0.010 mmol, 26% yield) as a white solid, as a hydrochloride salt. MS (ESI): 644.4 [M+H]⁺.

### Example 258

### N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5,5-benzothiazepin-3-yl]acetamide

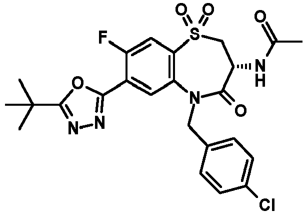

To a solution of acetic acid (0.01 mL, 0.120 mmol, 1.2 eq), DIPEA (0.05 mL, 0.290 mmol, 3 eq) and HATU (54.95 mg, 0.140 mmol, 1.5 eq) in THF (1 mL) was added (3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one (Example 63) (47.37 mg, 0.100 mmol, 1 eq) and the reaction mixture was stirred at 25 °C for 12 h. The mixture was quenched with water (2 mL), extracted with EtOAc (3 mL x 2) and purified by prep-HPLC affording the title compound (23.53 mg, 0.040 mmol, 45% yield) as a white powder. MS (ESI): 535.1 [M+H]⁺.

Examples 259 to 261 of the following table were prepared in analogy to Example 258, using the appropriate carboxylic acid building block.

| **Ex.** | **Structure** | **Systematic Name** | **Building Block** | **MS, ESI: m/z** |
|---|---|---|---|---|
| 259 | 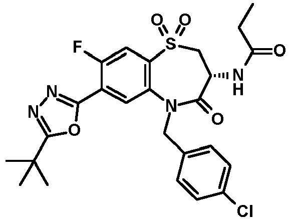 | N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide | Propionic acid | 549.1 [M+H]⁺ |
| | | | (CAS 79-09-4) | |
| 260 | 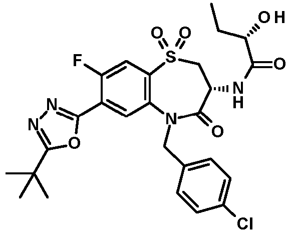 | (2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-hydroxy-butanamide | (2S)-2-Hydroxybu tanoic acid | 579.1 [M+H]⁺ |
| | | | (CAS 3347-90-8) | |
| 261 | 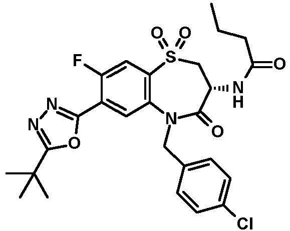 | N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide | Butyric acid | 563.1 [M+H]⁺ |
| | | | (CAS 107-92-6) | |

### Example 262

### (2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide

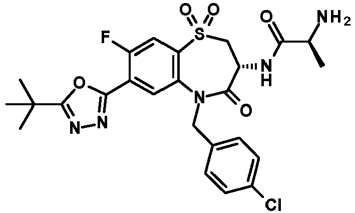

### Step a) tert-butyl N-[1S)-2-[[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]amino]-1-methyl-2-oxo-ethyl]carbamate

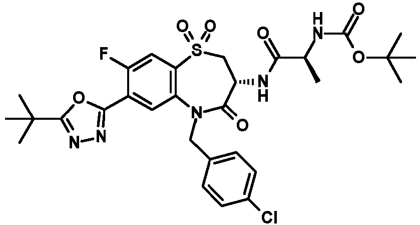

To a solution of (3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one (Example 63) (100 mg, 0.2 mmol, 1 eq), DIPEA (0.11 mL, 0.61 mmol, 3 eq) and HATU (116.01 mg, 0.3 mmol, 1.5 eq) in THF (1 mL) was added Boc-Ala-OH (0.04 mL, 0.240 mmol, 1.2 eq) and the reaction mixture was stirred at 25 °C for 12 h. The mixture was concentrated under vacuum to obtain the crude title compound (134 mg, 0.2 mmol, 79% yield) as a white solid. MS (ESI): 608.2 [M-isobuten+H]⁺.

### Step b) (2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶5-benzothiazepin-3-yl]propanamide

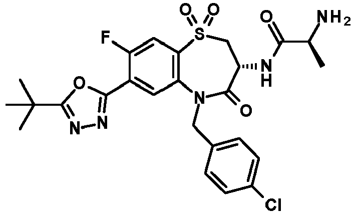

Tert-butyl N-[(1S)-2-[[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]amino]-1-methyl-2-oxoethyl]carbamate (134 mg, 0.2 mmol, 1 eq) was dissolved in HCl/EtOAc (4.0 mL, 16 mmol, 78.87 eq), the reaction mixture was stirred at 25 °C for 0.5 h. The mixture was concentrated under vacuum and purified by prep-HPLC affording the title compound (28 mg, 0.050 mmol, 23% yield) as a white powder, as a hydrochloride salt. MS (ESI): 564.2 [M+H]⁺.

Examples 263 to 267 of the following table were prepared in analogy to Example 262, using the appropriate carboxylic acid building block.

| **Ex.** | **Structure** | **Systematic Name** | **Building Block** | **MS, ESI: m/z** |
|---|---|---|---|---|
| 263 | 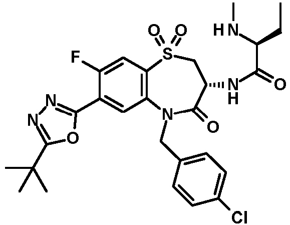 | (2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)butanamide | (2S)-2-[Tert-butoxycarb onyl(meth yl)amino]b utanoic acid | 592.1 [M+H]⁺ |
| | | | CAS (101759-74-4) | |
| 264 | 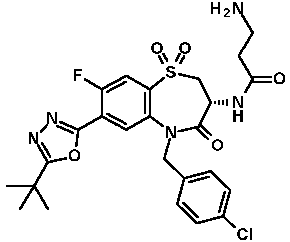 | 3-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide | 3-(Tert-butoxycarb onylamino )propanoic acid | 564.1 [M+H]⁺ |
| | | | (CAS 3303-84-2) | |
| 265 | 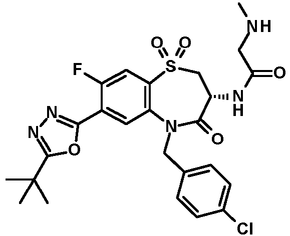 | N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)acetamide | 2-[Tert-butoxycarb onyl(meth yl)amino]a cetic acid | 564.1 [M+H]⁺ |
| | | | (CAS 13734-36-6) | |
| 266 | 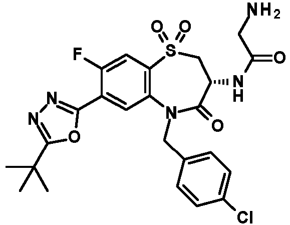 | 2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]acetamide | 2-(Tert-butoxycarb onylamino )acetic acid | 550.1 [M+H]⁺ |
| | | | (CAS 4530-20-5) | |
| 267 | 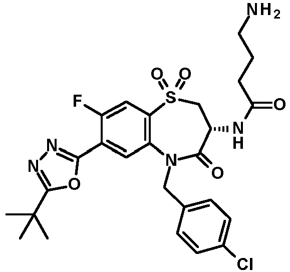 | 4-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide | 4-(Tert-butoxycarb onylamino )butanoic acid | 578.1 [M+H]⁺ |
| | | | (CAS 57294-38-9) | |

| | | | | |
|---|---|---|---|---|
| * as a hydrochloride salt | | | | |

### Example 268

### (2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)propanamide

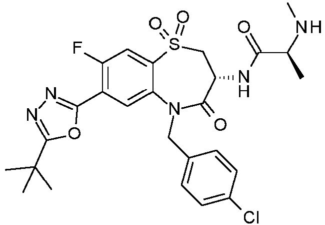

### Step a) tert-butyl N-[(1S)-2-[[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]amino]-1-methyl-2-oxo-ethyl]-N-methyl-carbamate

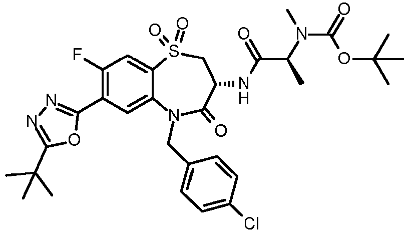

To a solution of (2S)-2-[tert-butoxycarbonyl(methyl)amino]propanoic acid (49.5 mg, 0.24 mmol, 1.2 eq), DIPEA (0.11 mL, 0.61 mmol, 3 eq) and HATU (116 mg, 0.3 mmol, 1.5 eq) in THF (1 mL) was added (3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one (Example 63, step d) (100 mg, 0.2 mmol) and the reaction mixture was stirred at 25 °C for 12 h, concentrated under vacuum to obtain the title compound (137 mg, 0.2 mmol, 85% yield) as white solid MS (ESI): 622.1 [M-isobuteneH]⁺.

### Step b) (2S)-N-[(3R)-7-(5-tert-butyl-1,3, 4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)propanamide

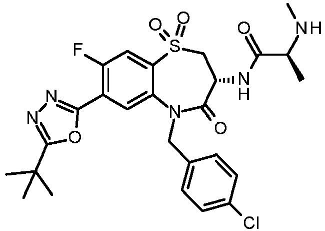

The title compound was prepared in analogy to general procedure 6b from tert-butyl N-[(1S)-2-[[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]amino]-1-methyl-2-oxo-ethyl]-N-methyl-carbamate (137 mg, 0.2 mmol, 1 eq) and was obtained as a withe powder (48 mg, 0.08 mmol, 39% yield). MS (ESI): 578.1 [M+H]⁺.

Examples 269 and 270 of the following table were prepared in analogy to Example 268, using the appropriate boc-protected amino acid building block.

| **Ex.** | **Structure** | **Systematic Name** | **Building Block** | **MS, ESI: m/z** |
|---|---|---|---|---|
| 269 | 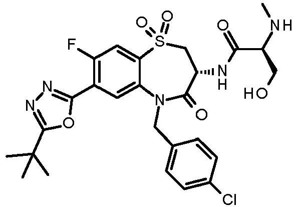 | (2S)-3-hydroxy-2-(methylamino)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide | (2S)-2-[tert-butoxycarb onyl(meth yl)amino]-3-hydroxy-propanoic acid | 594.1 [M+H]⁺ |
| | | | (CAS 101772-29-6) | |
| 270 | 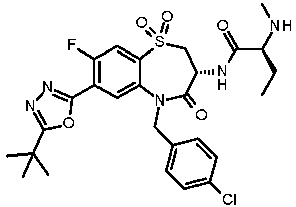 | (2S)-2-(methylamino)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide | (2S)-2-[tert-butoxycarb onyl(meth yl)amino]b utanoic acid | 592.1 [M+H]⁺ |
| | | | (CAS 101759-74-4) | |

### Example 271

### (3R)-3-amino-8-bromo-7-(5-tert-butyl-1,3,4-oxadiazol-2-yl)-5-[(4-chlorophenyl)methyl]-1,1-dioxo-2,3-dihydro-1λ⁶,5-benzothiazepin-4-one

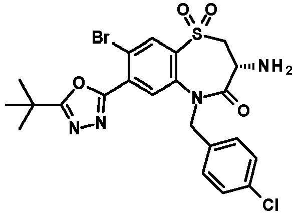

### Step a) (2R)-3-(5-bromo-4-methoxycarbonyl-2-nitro-phenyl)sulfanyl-2-(tert-butoxycarbonylamino)propanoic acid

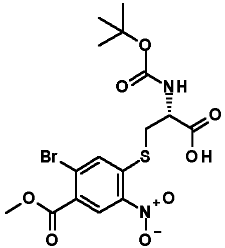

To a solution of methyl 2-bromo-4-fluoro-5-nitro-benzoate (28.5 g, 102.51 mmol, 1 eq) in DCE (300 mL) were added TEA (20706.4 mg, 205.01 mmol, 2 eq) and (2R)-2-(tert-butoxycarbonylamino)-3-sulfanyl-propanoic acid (34022.56 mg, 153.76 mmol, 1.5 eq) and the reaction mixture was stirred at 20°C for 12 h. The mixture was poured into water (150 ml) and the pH was adjusted to 4 by addition of 1M HCl. The mixture was then extracted with DCM (150 mL x 3) and the combined organic layers were washed with water (120 mL x 2) and brine (20 mL), dried over Na₂SO₄ and concentrated in vacuum to give the crude title compound (50 g) as a light yellow gum which was used in next step directly. MS (ESI): 381.0 [M-isobutene-CO₂+H]⁺.

### Step b) (2R)-3-(2-amino-5-bromo-4-methoxycarbonyl-phenyl)sulfanyl-2-(tert-butoxycarbonylamino)propanoic acid

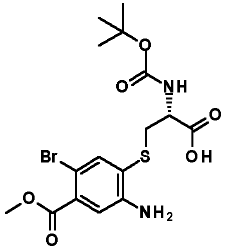

To a solution of (2R)-3-(5-bromo-4-methoxycarbonyl-2-nitro-phenyl)sulfanyl-2-(tert-butoxycarbonylamino)propanoic acid (50.0 g, 104.32 mmol, 1 eq) in MeOH (300 mL) was added NiCl₂ (26.66 g, 208.64 mmol, 2 eq). Then NaBH₄ (7.92 g, 208.64 mmol, 2 eq) was added in portions keeping the temperature at 0°C for 12 h. The reaction mixture was filtered and the filtrate was concentrated in vacuum to give the title compound (40 g, 89.02 mmol, 85% yield) as black oil which was used in next step directly. MS (ESI): 351.0 [M-isobutene+H]⁺.

### Step c) methyl (3R)-8-bromo-3-(tert-butoxycarbonylamino)-4-oxo-3,5-dihydro-2H-1,5-benzothiazepine-7-carboxylate

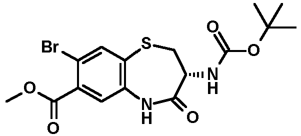

To a solution of (2R)-3-(2-amino-5-bromo-4-methoxycarbonyl-phenyl)sulfanyl-2-(tert-butoxycarbonylamino)propanoic acid (40 g, 89 mmol, 1 eq) in THF (500 mL) were added T3P (45.40 g, 178 mmol, 2 eq) and DIPEA (22.9 g, 178 mmol, 2 eq) and the reaction mixture was stirred at 25°C for 12 h. The mixture was concentrated in vacuum to remove the solvent and the remaining residue was purified with silica gel chromatography (PE:EtOAc = 10:1 to 1:1) to afford the title compound (5.6 g, 12.98 mmol, 15% yield) as light yellow solid. MS (ESI): 375.2 [M-isobutene+H]⁺.

### Step d) methyl (3R)-8-bromo-3-(tert-butoxycarbonylamino)-5-[(4-chlorophenyl)methyl]-4-oxo-2,3-dihydro-1,5-benzothiazepine-7-carboxylate

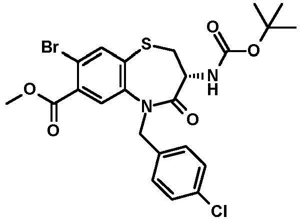

To a mixture of methyl (3R)-8-bromo-3-(tert-butoxycarbonylamino)-4-oxo-3,5-dihydro-2H-1,5-benzothiazepine-7-carboxylate (9.0 g, 20.87 mmol, 1 eq), potassium carbonate (6.02 g, 43.53 mmol, 2.09 eq) and potassium iodide (0.62 mL, 11.65 mmol, 0.560 eq) in DMF (85.94 mL) was added dropwise a solution of 4-chlorobenzyl bromide (4.73 g, 23 mmol, 1.1 eq) at 0°C. The mixture was stirred for 16 h at 25°C and then diluted with EtOAc (150 mL), washed with brine, dried over Na₂SO₄, filtered and concentrated under vacuum to afford a crude product which was then purified by silica gel chromatography (3% to 10% EtOAc in PE) to give the title compound (7 g, 12.59 mmol, 30% yield) as an orange solid. MS (ESI): 500.4 [M-isobutene+H]⁺.

### Step e) (3R)-8-bromo-3-(tert-butoxycarbonylamino)-5-[(4-chlorophenyl)methyl]-4-oxo-2,3-dihydro-1,5-benzothiazepine-7-carboxylic acid

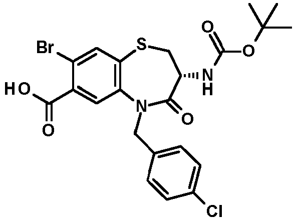

To a solution of methyl (3R)-8-bromo-3-(tert-butoxycarbonylamino)-5-[(4-chlorophenyl)methyl]-4-oxo-2,3-dihydro-1,5-benzothiazepine-7-carboxylate (7.0 g, 12.59 mmol, 1 eq) in a mixture of THF (25 mL), water (25 mL) and MeOH (25 mL) was added lithium hydroxide (0.71 mL, 75.56 mmol, 6 eq) and the reaction mixture was stirred at 25°C for 12 h. The mixture was diluted with EtOAc (150 mL) and washed with brine. The organic phase was dried over Na₂SO₄, filtered and concentrated under vacuum to give the title compound (7 g, 12.92 mmol, 62% yield) as orange oil. MS (ESI): 487.0 [M-isobutene+H]⁺.

### Step f) tert-butyl N-[(3R)-8-bromo-5-[(4-chlorophenyl)methyl]-7-(hydrazinecarbonyl)-4-oxo-2,3-dihydro-1,5-benzothiazepin-3-yl]carbamate

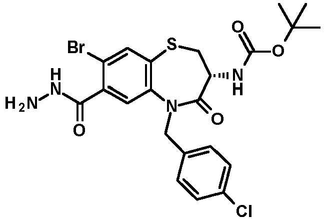

A mixture of (3R)-8-bromo-3-(tert-butoxycarbonylamino)-5-[(4-chlorophenyl)methyl]-4-oxo-2,3-dihydro-1,5-benzothiazepine-7-carboxylic acid (6.4 g, 11.81 mmol, 1 eq), NEt₃ (2.04 mL, 23.62 mmol, 2 eq) and ethyl chloroformate (1.46 mL, 15.36 mmol, 1.3 eq) in THF (90 mL) was stirred for 1.5 h at 0°C and then for 0.5 h at 25°C. Then the mixture was added into a solution of NH₂NH₂•H₂O (2.92 mL, 59.06 mmol, 5 eq) in THF (34 mL) slowly at 25 °C within 1 h. Then the reaction mixture was stirred for 20 h at 25°C. To the reaction mixture was added water (20 mL) at 0°C and the solution was stirred for 15 min. Then the mixture was concentrated in vacuo to obtain a crude product which was purified by prep-HPLC to obtain the title compound (2.38 g, 4.28 mmol, 36% yield) as a light brown solid. MS (ESI): 501.1 [M-isobutene+H]⁺.

### Step g) tert-butylN-[(3R)-8-bromo-5-[(4-chlorophenyl)methyl]-7-[(2,2-dimethylpropanoylamino)carbamoyl]-4-oxo-2,3-dihydro-1,5-benzothiazepin-3-yl]carbamate

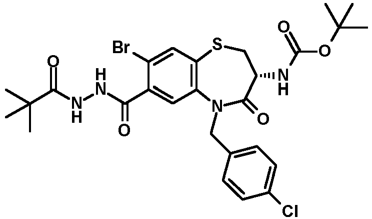

To a solution of tert-butyl N-[(3R)-8-bromo-5-[(4-chlorophenyl)methyl]-7-(hydrazinecarbonyl)-4-oxo-2,3-dihydro-1,5-benzothiazepin-3-yl]carbamate;2,2,2-trifluoroacetic acid (2.28 g, 3.4 mmol, 1 eq), pivalic acid (461.0 mg, 4.51 mmol, 1.33 eq) and HATU (1.87 g, 4.92 mmol, 1.45 eq) in THF (65 mL) was added DIPEA (2.14 mL, 12.29 mmol, 3.61 eq) and the mixture was stirred at 25°C for 2.5 h. The mixture was concentrated under vacuum to remove THF and the remaining residue was purified by silica gel chromatography (PE:EtOAc = 5:1 to 1:1), to obtain the title compound (2.0 g, 3.13 mmol, 91% yield) as a light yellow solid. MS (ESI): 541.2 [M-isubutene-CO₂+H]⁺.

### Step h) tert-butyl N-[(3R)-8-bromo-7-(5-tert-butyl-1,3, 4-oxadiazol-2-yl)-5-[(4-chlorophenyl)methyl]-4-oxo-2,3-dihydro-1,5-benzothiazepin-3-yl]carbamate

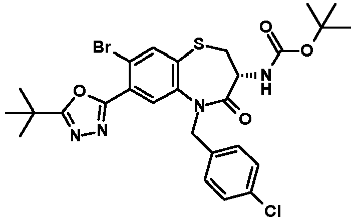

To a solution of tert-butyl N-[(3R)-8-bromo-5-[(4-chlorophenyl)methyl]-7-[(2,2-dimethylpropanoylamino)carbamoyl]-4-oxo-2,3-dihydro-1,5-benzothiazepin-3-yl]carbamate (1.8g, 2.81 mmol, 1 eq) in 1,4-dioxane (36 mL) was added Burgess reagent (2.68 g, 11.25 mmol, 4 eq) at 25°C and the mixture was heated to 80°C for 16 h. After cooling to ambient temperature, the mixture was concentrated under vacuum to remove the dioxane and the remaining residue was purified by column chromatography (PE:EtOAc = 5:1 to 1:1) to afford the title compound (2000 mg, 3.22 mmol, 99% yield) as an off-white solid. MS (ESI): 567.0 [M-isobuten+H]⁺.

### Step i) tert-butyl N-[(3R)-8-bromo-7-(5-tert-butyl-1,3, 4-oxadiazol-2-yl)-5-[(4-chlorophenyl)methyl]-1,1,4-trioxo-2,3-dihydro-1λ⁶5-benzothiazepin-3-yl]carbamate

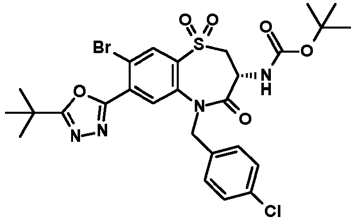

The title compound was prepared in analogy to general method 5 from tert-butyl N-[(3R)-8-bromo-7-(5-tert-butyl-1,3,4-oxadiazol-2-yl)-5-[(4-chlorophenyl)methyl]-4-oxo-2,3-dihydro-1,5-benzothiazepin-3-yl]carbamate (150.0 mg, 0.240 mmol) and was obtained (100 mg, 0.150 mmol, 47% yield) as a white solid. MS (ESI): 655.3 [M+H]⁺.

### Step j) (3R)-3-amino-8-bromo-7-(5-tert-butyl-1,3,4-oxadiazol-2yl)-5-[(4-chlorophenyl)methyl]-1,1-dioxo-2,3-dihydro-1λ⁶,5-benzothiazepin-4-one

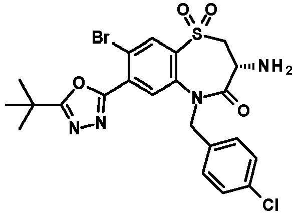

The title compound was prepared according to general method **6b** from tert-butyl N-[(3R)-8-bromo-7-(5-tert-butyl-1,3,4-oxadiazol-2-yl)-5-[(4-chlorophenyl)methyl]-1,1,4-trioxo-2,3-dihydro-1λ⁶,5-benzothiazepin-3-yl]carbamate (75.0 mg, 0.110 mmol) and was obtained (3.4 mg, 0.010 mmol, 5% yield) as a white solid, as a hydrochloride salt. MS (ESI): 555.1 [M+H]⁺.

### Example 272

### (3R)-3-amino-7-(5-tert-butyl-1,3,4-oxadiazol-2-yl)-5-[(4-chlorophenyl)methyl]-8-methyl-1,1-dioxo-2,3-dihydro-1λ⁶,5-benzothiazepin-4-one

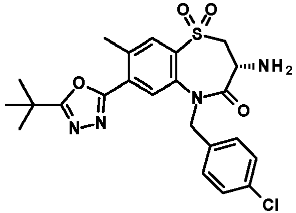

### Step a) tert-butyl N-[(3R)-7-(5-tert-butyl-1,3,4-oxadiazol-2-yl)-5-[(4-chlorophenyl)methyl]-8-methyl-4-oxo-2,3-dihydro-1,5-benzothiazepin-3-yl]carbamate

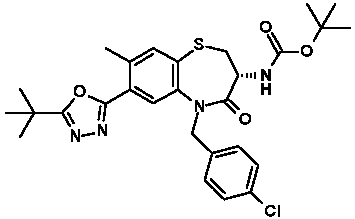

A suspension of tert-butyl N-[(3R)-8-bromo-7-(5-tert-butyl-1,3,4-oxadiazol-2-yl)-5-[(4-chlorophenyl)methyl]-4-oxo-2,3-dihydro-1,5-benzothiazepin-3-yl]carbamate (Example 271, step h) (200.0 mg, 0.320 mmol, 1 eq), trimethylboroxine (50% in EtOAc) (0.11 mL, 0.390 mmol, 1.2 eq), Cs₂CO₃ (209.54 mg, 0.640 mmol, 2 eq) and Pd(dppf)Cl₂ (35.29 mg, 0.050 mmol, 0.150 eq) in a mixture of 1,4-dioxane (2.8 mL) and water (0.400 mL) was degassed with nitrogen for three times and the resulting reaction mixture was heated to 110°C for 16 h. After cooling to ambient temperature, the reaction mixture was concentrated under vacuum to remove the solvent and to the remaining residue was added EtOAc (10 mL). The mixture was filtered and the filtrate was washed with brine (5 mL x 3), dried with anhydrous Na₂SO₄ and concentrated to give the residue which was purified by silica gel chromatography (PE:EtOAc = 10:1 to 5:1) to afford the title compound (80 mg, 0.140 mmol, 44% yield) as a white solid. MS (ESI): 501.1 [M-isobuten+H]⁺.

### Step b) tert-butyl N-[(3R)-7-(5-tert-butyl-1,3, 4-oxadiazol-2-yl)-5-[(4-chlorophenyl)methyl]-8-methyl-1,1,4-trioxo-2,3-dihydro-1λ⁶,5-benzothiazepin-3-yl]carbamate

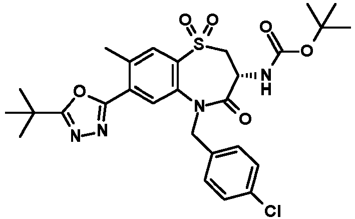

The title compound was prepared in analogy to general method **5** from tert-butyl N-[(3R)-7-(5-tert-butyl-1,3,4-oxadiazol-2-yl)-5-[(4-chlorophenyl)methyl]-8-methyl-4-oxo-2,3-dihydro-1,5-benzothiazepin-3-yl]carbamate (100.0 mg, 0.180 mmol) and was obtained (100 mg, 0.17 mmol, 92% yield) as a white solid. MS (ESI): 533.1 [M-isobuten+H]⁺.

### Step c) (3R)-3-amino-7-(5-tert-butyl-1,3,4-oxadiazol-2yl)-5-[(4-chlorophenyl)methyl]-8-methyl-1,1-dioxo-2,3-dihydro-1λ⁶,5-benzothiazepin-4-one

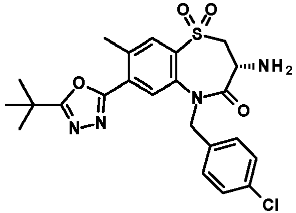

The title compound was prepared according to general method **6b** from tert-butyl N-[(3R)-7-(5-tert-butyl-1,3,4-oxadiazol-2-yl)-5-[(4-chlorophenyl)methyl]-8-methyl-1,1,4-trioxo-2,3-dihydro-1λ⁶,5-benzothiazepin-3-yl]carbamate (100.0 mg, 0.170 mmol) and was obtained (43.3 mg, 0.080 mmol, 48% yield) as a white solid, as a hydrochloride salt. MS (ESI): 489.1 [M+H]⁺.

### Example 273

### (3R)-3-amino-7-(5-tert-butyl-1,3,4-oxadiazol-2-yl)-5-[(4-chlorophenyl)methyl]-8-hydroxy-1,1-dioxo-2,3-dihydro-1λ⁶,5-benzothiazepin-4-one

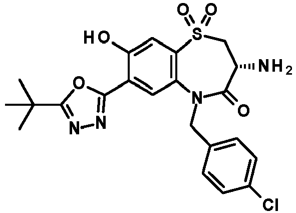

### Step a) tert-butyl N-[(3R)-7-(5-tert-butyl-1,3,4-oxadiazol-2-yl)-5-[(4-chlorophenyl)methyl]-8-hydroxy-4-oxo-2,3-dihydro-1,5-benzothiazepin-3-yl]carbamate

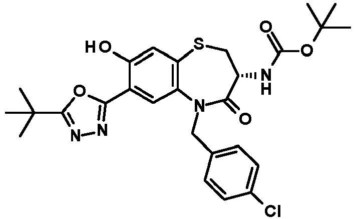

To a solution of tert-butyl N-[(3R)-8-bromo-7-(5-tert-butyl-1,3,4-oxadiazol-2-yl)-5-[(4-chlorophenyl)methyl]-4-oxo-2,3-dihydro-1,5-benzothiazepin-3-yl] (Example 271, step h) (130.0 mg, 0.210 mmol, 1 eq) in 1,4-dioxane (1 mL) were added tris(dibenzylideneacetone)dipalladium (0) (19.14 mg, 0.020 mmol, 0.100 eq), 2-di-tert-butylphosphino-2',4',6'-triisopropylbiphenyl (17.75 mg, 0.040 mmol, 0.200 eq) and potassium hydroxide (35.18 mg, 0.630 mmol, 3 eq) at 25°C and the reaction mixture was heated to 90°C for 16 h. The mixture was poured into water (1.5 mL). The aqueous phase was extracted with ethyl acetate (1.5 mL × 3). The combined organic phases were washed with brine (3 mL× 2), dried over anhydrous Na₂SO₄, filtered and concentrated under vacuum. The remaining residue was purified by prep-TLC (DCM/MeOH = 30:1) to afford the title compound (85 mg, 0.153 mmol, 60% yield) as a light red oil. MS (ESI): 559.3 [M+H]⁺.

### Step b) tert-butyl N-[(3R)-7-(S-tert-butyl-1,3,4-oxadiazol-2-yl)-5-[(4-chlorophenyl)methyl]-8-hydroxy-1,1,4-trioxo-2,3-dihydro-1λ⁶,5-benzothiazepin-3-yl]carbamate

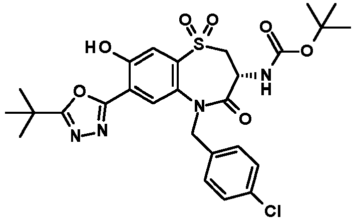

The title compound was prepared in analogy to general method **5** from tert-butyl N-[(3R)-7-(5-tert-butyl-1,3,4-oxadiazol-2-yl)-5-[(4-chlorophenyl)methyl]-8-hydroxy-4-oxo-2,3-dihydro-1,5-benzothiazepin-3-yl]carbamate (100.0 mg, 0.180 mmol) and was obtained (40 mg, 0.07 mmol, 34% yield) as a light red solid. MS (ESI): 535.2 [M-isobuten+H]⁺.

### Step b) (3R)-3-amino-7-(5-tert-butyl-1,3, 4-oxadiazol-2-yl)-5-[(4-chlorophenyl)methyl]-8-hydroxy-1,1-dioxo-2,3-dihydro-1λ⁶,5-benzothiazepin-4-one

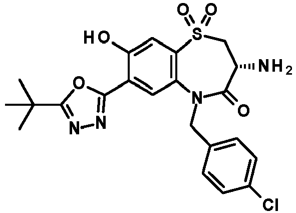

The title compound was prepared according to general method **6b** from tert-butyl N-[(3R)-7-(5-tert-butyl-1,3,4-oxadiazol-2-yl)-5-[(4-chlorophenyl)methyl]-8-hydroxy-1, 1,4-trioxo-2,3-dihydro-1λ⁶,5-benzothiazepin-3-yl]carbamate (40 mg, 0.07 mmol) and was obtained (15.4 mg, 0.030 mmol, 43% yield) as a light red solid. MS (ESI): 491.3 [M+H]⁺.

### Example 274

### (3R)-3-amino-7-(5-tert-butyl-1,3,4-oxadiazol-2-yl)-5-[(4-chlorophenyl)methyl]-8-(dimethylamino)-1,1-dioxo-2,3-dihydro-1λ⁶,5-benzothiazepin-4-one

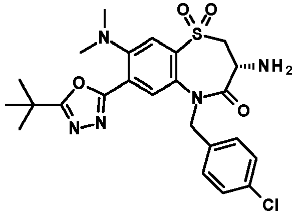

### Step a) tert-butyl N-[(3R)-7-(5-tert-butyl-1,3,4-oxadiazol-2-yl)-5-[(4-chlorophenyl)methyl]-8-(dimethylamino)-1,1,4-trioxo-2,3-dihydro-1λ⁶,5-benzothiazepin-3-ylcarbamate

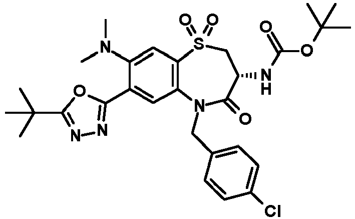

To tert-butyl N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]carbamate (Example 63) (60.0 mg, 0.100 mmol, 1 eq) was added dimethylamine (1.2 mL, 2.4 mmol, 23.72 eq) at 25°C and the mixture was heated to 45°C for 4 h. The mixture was poured into brine (2 mL). The aqueous phase was extracted with ethyl acetate (2 mL × 3). The combined organic phases were washed with brine (5 mL× 2), dried over anhydrous Na₂SO₄, filtered and concentrated under vacuum. The remaining residue was purified by prep-TLC (DCM:MeOH = 20:1) to afford the title compound (47 mg, 0.077 mmol, 72% yield) as a yellow oil. MS (ESI): 618.3 [M+H]⁺.

### Step b) (3R)-3-amino-7-(5-tert-butyl-1,3,4-oxadiazol-2-yl)-5-[(4-chlorophenyl)methyl]-8-(dimethylamino)-1,1-dioxo-2,3-dihydro-1λ⁶,5-benzothiazepin-4-one

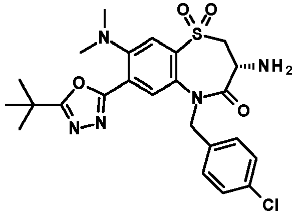

The title compound was prepared according to general method **6b** from tert-butyl N-[(3R)-7-(5-tert-butyl-1,3,4-oxadiazol-2-yl)-5-[(4-chlorophenyl)methyl]-8-(dimethylamino)-1,1,4-trioxo-2,3-dihydro-1λ⁶,5-benzothiazepin-3-yl]carbamate (40.0 mg, 0.060 mmol) and was obtained (15 mg, 0.030 mmol, 46% yield) as a light yellow solid, as a hydrochloride salt. MS (ESI): 518.3 [M+H]⁺.

### Example 275

### (3R)-3,8-diamino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one

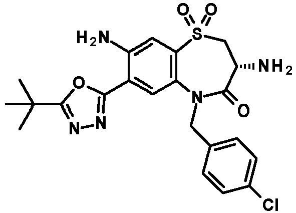

### Step a) tert-butyl N-[(3R)-3-(tert-butoxycarbonylamino)-7-(5-tert-butyl-1,3, 4-oxadiazol-2-yl)-5-[(4-chlorophenyl)methyl]-4-oxo-2,3-dihydro-1,5-benzothiazepin-8-yl]carbamate

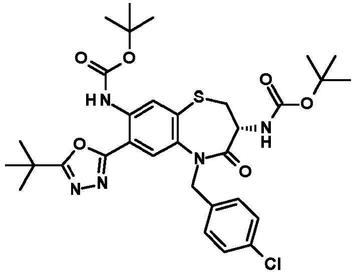

To a solution of tert-butyl N-[(3R)-8-bromo-7-(5-tert-butyl-1,3,4-oxadiazol-2-yl)-5-[(4-chlorophenyl)methyl]-4-oxo-2,3-dihydro-1,5-benzothiazepin-3-yl]carbamate (Example 271, step h) (100.0 mg, 0.16 mmol, 1 eq) in 1,4-dioxane (1.5 mL) were added 4,5-bis(diphenylphosphino)-9,9-dimethylxanthene (18.61 mg, 0.03 mmol, 0.2 eq), cesium carbonate (104.77 mg, 0.32 mmol, 2 eq), tert-butyl carbamate (37.67 mg, 0.32 mmol, 2 eq) and palladium (II) acetate (3.61 mg, 0.020 mmol, 0.100 eq) under an atmosphere of nitrogen at 25°C and the mixture was stirred for 16 h at 100°C. The mixture was poured into water (1.5 mL). The aqueous phase was extracted with ethyl acetate (2 mL × 3). The combined organic phases were washed with brine (3 mL × 2), dried over anhydrous Na₂SO₄, filtered and concentrated under vacuum. The remaining residue was purified by prep-TLC (PE:EtOAc = 2:1) to afford the title compound (33 mg, 0.050 mmol, 30% yield) as a white solid. MS (ESI): 658.4 [M+H]⁺.

### Step b) tert-butyl N-[(3R)-3-(tert-butoxycarbonylamino)-7-(5-tert-butyl-1, 3,4-oxadiazol-2-yl)-5-[(4-chlorophenyl)methyl]-1,1,4-trioxo-2,3-dihydro-1λ⁶,5-benzothiazepin-8-yl]carbamate

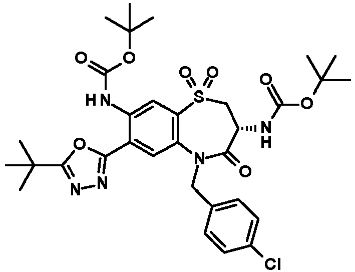

The title compound was prepared in analogy to general method **5** from tert-butyl N-[(3R)-3-(tert-butoxycarbonylamino)-7-(5-tert-butyl-1,3,4-oxadiazol-2-yl)-5-[(4-chlorophenyl)methyl]-4-oxo-2,3-dihydro-1,5-benzothiazepin-8-yl]carbamate (40.0 mg, 0.060 mmol) and was obtained (40 mg, 0.060 mmol, 64% yield) as a light red solid. MS (ESI): 578.3 [M-(2 x isobutene)+H]⁺.

### Step c) (3R)-3, 8-diamino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one

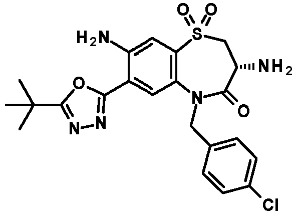

The title compound was prepared according to general method **6b** from tert-butyl N-[(3R)-3-(tert-butoxycarbonylamino)-7-(5-tert-butyl-1,3,4-oxadiazol-2-yl)-5-[(4-chlorophenyl)methyl]-1,1,4-trioxo-2,3-dihydro-1λ⁶,5-benzothiazepin-8-yl]carbamate (40.0 mg, 0.060 mmol) and was obtained (6.3 mg, 0.010 mmol, 20% yield) as an off-white solid, as a trifluoroacetic acid salt. MS (ESI): 490.2 [M+H]⁺.

### Example 276

### 2-[5-[(3R)-3-amino-5-[(4-chlorophenyl)methyl]-8-methyl-1,1,4-trioxo-2,3-dihydro-1λ⁶,5-benzothiazepin-7-yl]-1,3,4-oxadiazol-2-yl]-2-methyl-propanenitrile

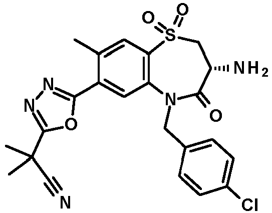

### Step a) (2R)-2-(tert-butoxycarbonylamino)-3-(4-methoxycarbonyl-5-methyl-2-nitrophenyl)sulfanyl-propanoic acid

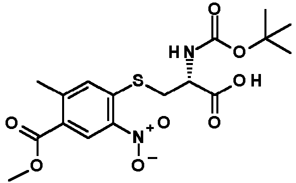

To a solution of methyl 4-fluoro-2-methyl-5-nitro-benzoate (CAS 1163287-01-1) in MeCN was added DIPEA (Eq. 3) and N-Boc-L-Cysteine (CAS 20887-95-0) (Eq 1). The mixture was stirred for 16 hours at 48°C. The obtained residue was purified by prep-HPLC to obtain the desired product in 35% yield.

### Step b) (2R)-3-(2-amino-4-methoxycarbonyl-5-methyl-phenyl)sulfanyl-2-(tert-butoxycarbonylamino)propanoic acid

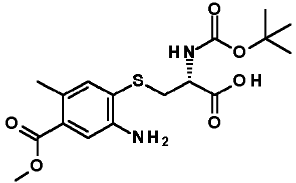

The title compound was prepared in analogy to the general procedure **2** from *(2R)-2-(tert-butoxycarbonylamino)-3-(4-methoxycarbonyl-5-methyl-2-nitro-phenyl)sulfanyl-propanoic acid in dioxane:water (4:1) and was obtained in 60% yield.*

### Step c) methyl (3R)-3-(tert-butoxycarbonylamino)-8-methyl-4-oxo-3,5-dihydro-2H-1,5-benzothiazepine-7-carboxylate

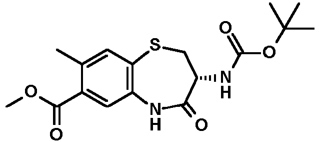

To a solution of *(2R)-3-(2-amino-4-methoxycarbonyl-5-methyl-phenyl)sulfanyl-2-(tert-butoxycarbonylamino)propanoic acid in DMF was added DIPEA (Eq. 1.1.) and HATU (Eq. 1). The mixture was stirred at 22°C for 16 hours. The mixture was extracted and the organic layer was washed with water. The combined organic layers were dried over sodium sulfate and evaporated in vacuo to afford the title compound in 50% yield.*

### Step d) methyl (3R)-3-(tert-butoxycarbonylamino)-5-[(4-chlorophenyl)methyl]-8-methyl-4-oxo-2,3-dihydro-1,5-benzothiazepine-7-carboxylate

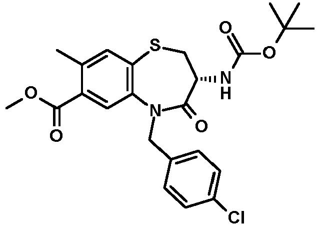

The title compound was prepared in analogy to general procedure **4** from methyl (3R)-3-(tert-butoxycarbonylamino)-8-methyl-4-oxo-3,5-dihydro-2H-1,5-benzothiazepine-7-carboxylate (2 g, 5.46 mmol) and 1-(bromomethyl)-4-chloro-benzene (1.79 g, 8.7 mmol, 1.6 eq) and was obtained as a yellow solid (2.37 g, 79% yield). MS (ESI): 435.2 [M-isobutene+H]⁺.

### Step e) (3R)-3-(tert-butoxycarbonylamino)-5-[(4-chlorophenyl)methyl]-8-methyl-4-oxo-2,3-dihydro-1,5-benzothiazepine-7-carboxylic acid

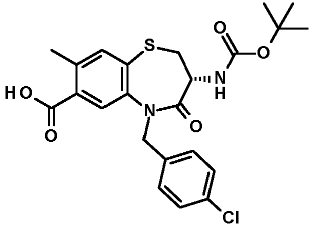

The title compound was prepared in analogy to general procedure **13** from *methyl (3R)-3-(tert-butoxycarbonylamino)-5-[(4-chlorophenyl)methyl]-8-methyl-4-oxo-2,3-dihydro-1,5-benzothiazepine-7-carboxylate* (2.37 g, 4.83 mmol) and was obtained as a yellow solid (2.29 g, 94% yield). MS (ESI): 475.3 [M-H]⁻.

### Step f) tert-butyl N-[(3R)-5-[(4-chlorophenyl)methyl]-7-(hydrazinecarbonyl)-8-methyl-4-oxo-2,3-dihydro-1,5-benzothiazepin-3-yl]carbamate

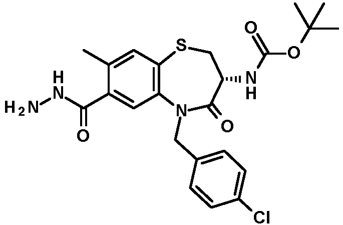

The title compound was prepared in analogy to general procedure **14** from (3R)-3-(tert-butoxycarbonylamino)-5-[(4-chlorophenyl)methyl]-8-methyl-4-oxo-2,3-dihydro-1,5-benzothiazepine-7-carboxylic acid (1.0 g, 2.1 mmol) and was obtained as a light yellow solid (1.08 g, 94% yield). MS (ESI): 435.2 [M-isobutene+H]⁺.

### Step g) tert-butyl N-[(3R)-5-[(4-chlorophenyl)methyl]-7-[[(2-cyano-2-methylpropanoyl)amino]carbamoyl]-8-methyl-4-oxo-2,3-dihydro-1,5-benzothiazepin-3-yl]carbamate

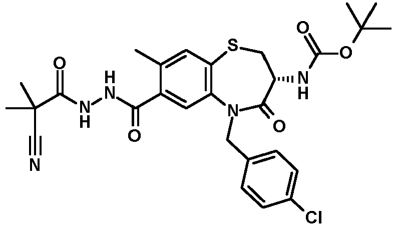

The title compound was prepared in analogy to general procedure **8a** from *tert-butyl N-[(3R)-5-[(4-chlorophenyl)methyl]-7-[[(2-cyano-2-methyl-propanoyl)amino]carbamoyl]-8-methyl-4-oxo-2,3-dihydro-1,5-benzothiazepin-3-yl]carbamate* (100 mg, 0.204 mmol) and 2-cyano-2-methyl-propionic acid (CAS 22426-30-8) (27.6 mg, 0.244 mmol) and was obtained as a colorless solid (97 mg, 77% yield). MS (ESI): 586.3 [M+H]⁺.

### Step h) tert-butyl N-[(3R)-5-[(4-chlorophenyl)methyl]-7-[5-(1-cyano-1-methyl-ethyl)-1,3,4-oxadiazol-2-yl]-8-methyl-4-oxo-2,3-dihydro-1,5-benzothiazepin-3-yl]carbamate

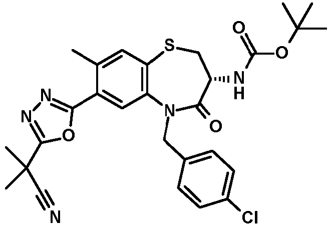

The title compound was prepared in analogy to general procedure **8a** from *tert-butyl N-[(3R)-5-[(4-chlorophenyl)methyl]-7-[[(2-cyano-2-methyl-propanoyl)amino]carbamoyl]-8-methyl-4-oxo-2,3-dihydro-1,5-benzothiazepin-3-yl]carbamate* (96 mg, 0.164 mmol) and was obtained as a white solid (39.2 mg, 40% yield). MS (ESI): 512.2 [M-isobutene+H]⁺.

### Step i) tert-butyl N-[(3R)-5-[(4-chlorophenyl)methyl]-7-[5-(1-cyano-1-methyl-ethyl)-1,3,4-oxadiazol-2-yl]-8-methyl-1,1,4-trioxo-2,3-dihydro-1λ⁶,5-benzothiazepin-3-yl]carbamate

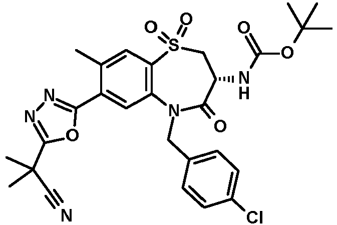

The title compound was prepared in analogy to general procedure **5** from tert-butyl N-[(3R)-5-[(4-chlorophenyl)methyl]-7-[5-(1-cyano-1-methyl-ethyl)-1,3,4-oxadiazol-2-yl]-8-methyl-4-oxo-2,3-dihydro-1,5-benzothiazepin-3-yl]carbamate (74.9 mg, 0.132 mmol) and was obtained as a white solid (76 mg, 91% yield). MS (ESI): 544.2 [M-isobutene+H]⁺.

### Step j) 2-[5-[(3R)-3-amino-5-[(4-chlorophenyl)methyl]-8-methyl-1,1,4-trioxo-2,3-dihydro-1λ⁶,5-benzothiazepin-7-yl]-1,3,4-oxadiazol-2-yl]-2-methyl-propanenitrile

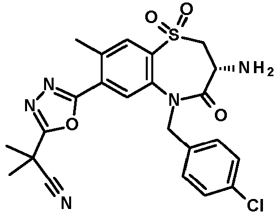

The title compound was prepared in analogy to general procedure **6d** *tert-butyl N-[(3R)-5-[(4-chlorophenyl)methyl]-7-[5-(1-cyano-1-methyl-ethyl)-1,3,4-oxadiazol-2-yl]-8-methyl-1,1,4-trioxo-2,3-dihydro-1λ⁶,5-benzothiazepin-3-yl]carbamate* (76 mg, 0.127 mmol) and was obtained as a white solid, as a hydrochloride salt (59.7 mg, 83% yield). MS (ESI): 500.2 [M +H]⁺.

Example 277 of the following table was prepared in analogy to Example 276, steps g-j), using the appropriate building block.

| **Ex.** | **Structure** | **Systematic Name** | **Building Blocks** | **MS, ESI: m/z** |
|---|---|---|---|---|
| 277 | 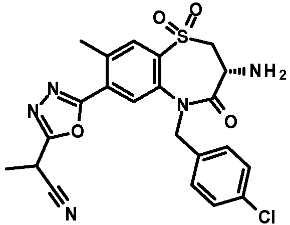 | 2-[5-[(3R)-3-amino-5-[(4-chlorophenyl)methyl]-8-methyl-1,1,4-trioxo-2,3-dihydro-1λ⁶,5-benzothiazepin-7-yl]-1,3,4-oxadiazol-2-yl]propanenitrile | 2-Cyanoprop anoic acid | 486.1 [M+H]⁺ |
| | | | (CAS 635-07-5) | |
| | | (*) | | |

| | | | | |
|---|---|---|---|---|
| * as hydrochloride salt | | | | |

### Example 278

### (3R)-3-amino-7-(1-tert-butylpyrazol-4-yl)-5-[(4-chlorophenyl)methyl]-8-fluoro-1,1-dioxo-2,3-dihydro-1λ⁶,5-benzothiazepin-4-one

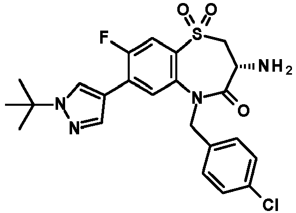

### Step a) tert-butyl N-[(3R)-7-(1-tert-butylpyrazol-4-yl)-5-[(4-chlorophenyl)methyl]-8-fluoro-4-oxo-2,3-dihydro-1,5-benzothiazepin-3-yl]carbamate

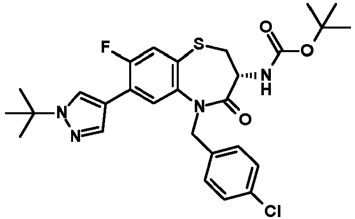

To a solution of tert-butyl N-[(3R)-7-bromo-8-fluoro-4-oxo-3,5-dihydro-2H-1,5-benzothiazepin-3-yl]carbamate (Example 6 step b) (102.8 mg, 0.2 mmol, 1.0 eq) and 1-tert-butyl-4-(4,4,5,5-tetramethyl-1,3,2-dioxaborolan-2-yl)pyrazole (CAS 1256359-15-5, 75.0 mg, 0.3 mmol, 1.5 eq) in a mixture of aqueous K₃PO₄ (2.0 M, 0.3 mL, 0.6 mmol, 3.0 eq) and dioxane (3.0 mL) was added Pd(dppf)Cl₂ (7.31 mg, 0.01 mmol, 0.05 eq) under an atmosphere of nitrogen. The reaction mixture was stirred at reflux for 3 h and then concentrated under reduced pressure to remove dioxane. The remaining residue was extracted with EtOAc and the combined organic layers were washed with water, dried over anhydrous Na₂SO₄, filtered and concentrated under reduced pressure to give a crude product which was purified by preparative TLC to give the title compound (89.3 mg, 0.16 mmol) as solid. MS (ESI): 559.2 [M+H]⁺.

### Step b) tert-butyl N-[(3R)-7-(1-tert-butylpyrazol-4-yl)-5-[(4-chlorophenyl)methyl]-8-fluoro-1,1,4-trioxo-2,3-dihydro-1λ⁶,5-benzothiazepin-3-yl]carbamate

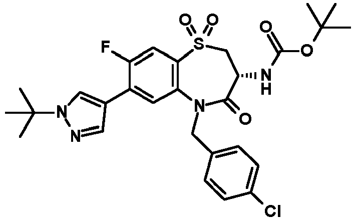

The title compound was prepared from tert-butyl N-[(3R)-7-(1-tert-butylpyrazol-4-yl)-5-[(4-chlorophenyl)methyl]-8-fluoro-4-oxo-2,3-dihydro-1,5-benzothiazepin-3-yl]carbamate (89.3 mg, 0.16 mmol) in analogy to general procedure **5** to obtain the crude title compound which was used in the next reaction step without further purification. MS (ESI): 591.3 [M+H]⁺.

### Step c) (3R)-3-amino-7-(1-tert-butylpyrazol-4-yl)-5-[(4-chlorophenyl)methyl]-8-fluoro-1,1-dioxo-2,3-dihydro-1λ⁶,5-benzothiazepin-4-one

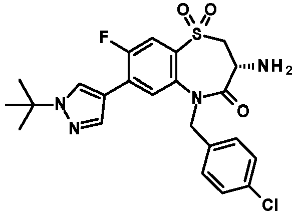

To a solution of crude tert-butyl N-[(3R)-7-(1-tert-butylpyrazol-4-yl)-5-[(4-chlorophenyl)methyl]-8-fluoro-1,1,4-trioxo-2,3-dihydro-1λ⁶,5-benzothiazepin-3-yl]carbamate in DCM (3.0 mL) was added TFA (1.0 mL) and the mixture was stirred at 30°C for 2 h. The mixture was concentrated under reduced pressure to give a residue, which was purified by prep-HPLC to give the desired title compound (46.5 mg, 0.095 mmol). MS (ESI): 491.1 [M+H]⁺.

Examples 279 to 280 of the following table were prepared in analogy to Example 278, using the appropriate boronic acid ester building blocks.

| **Ex.** | **Structure** | **Systematic Name** | **Building Blocks** | **MS, ESI: m/z** |
|---|---|---|---|---|
| 279 | 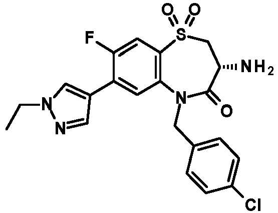 | ((3R)-3-amino-5-[(4-chlorophenyl)methyl]-7-(1-ethylpyrazol-4-yl)-8-fluoro-1,1-dioxo-2,3-dihydro-1λ⁶,5-benzothiazepin-4-one | 1-Ethyl-4-(4,4,5,5-tetrameth yl-1,3,2-dioxaborol an-2-yl)pyrazole | 463.1 [M+H]⁺ |
| | | | (CAS 847818-70-6) | |
| 280 | 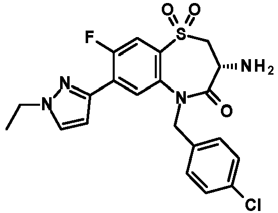 | (3R)-3-amino-5-[(4-chlorophenyl)methyl]-7-(1-ethylpyrazol-3-yl)-8-fluoro-1,1-dioxo-2,3-dihydro-1λ⁶,5-benzothiazepin-4-one | 1-Ethyl-3-(4,4,5,5-tetrameth yl-1,3,2-dioxaborol an-2-yl)pyrazole | 463.1 [M+H]⁺ |
| | | | (CAS 1233525-88-6) | |

### Example 281

### (3R)-3-amino-5-[(4-chlorophenyl)methyl]-7-[1-(5,5-difluoro-1-methyl-3-piperidyl)pyrazol-4-yl]-8-fluoro-1,1-dioxo-2,3-dihydro-1λ⁶,5-benzothiazepin-4-one

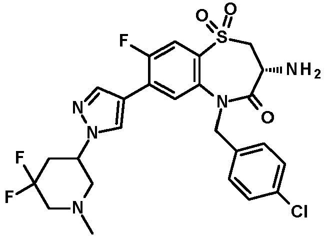

### Step a) 9H-fluoren-9-ylmethyl 3,3-difluoro-5-hydroxy-piperidine-1-carboxylate

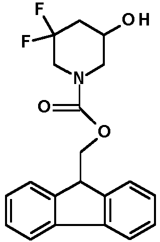

3,3-difluoro-5-hydroxy-piperidine-1-carboxylic acid tert-butyl ester (150 mg, 0.63 mmol) was stirred in 1,4-dioxane with 4 M HCl in dioxane (1 mL, 4 mmol, 6.33 eq). 9-fluorenylmethyl chloroformate (179 mg, 0.69 mmol, 1.1 eq) was added and stirred overnight at room temperature. The solvent was evaporated and the reaction diluted in DCM (5 mL). Et₃N (192 mg, 264 uL, 1.9 mmol, 3 eq) were added, the reaction cooled to 0°C and 9- fluorenylmethyl chloroformate (179 mg, 0.69 mmol, 1.1 eq) was added. The reaction mixture was stirred overnight at room temperature, partitioned between 50 ml DCM and 30 ml 1M aq. HCl. The phases were separated and the aqueous layer was extracted once with 30 ml DCM. The organic layers were combined, dried over Na₂SO₄ and filtered. The solvent was evaporated. The crude material was purified by column chromatography on silica gel (n-heptane:EtOAc = 100:0 to 70:30) to afford:the title compound (156 mg, 61% yield) as white solid. MS (ESI): 360.2 [M+H]⁺.

### Step b) 9H-fluoren-9-ylmethyl 3,3-difluoro-5-(p-tolylsulfonyloxy)piperidine-1-carboxylate

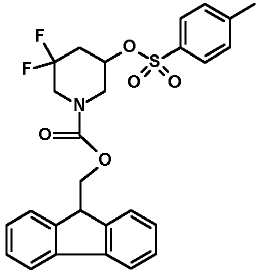

To a solution of 9H-fluoren-9-ylmethyl 3,3-difluoro-5-hydroxy-piperidine-1-carboxylate (495 mg, 1.1 mmol) in DCM (10 mL) was added p-toluenesulfonyl chloride (315 mg, 1.65 mmol, 1.5 eq), Et₃N (278 mg, 384 uL, 2.75 mmol, 2.5 eq) and 4-dimethylaminopyridine (134 mg, 1.1 mmol, 1 eq). The reaction was stirred at room temperature for 1 hour, partitioned between DCM (40 ml) and H₂O (40 ml). The layers were separated. The aqueous layer was extracted with two 40 ml portions of DCM. The combined organic layers were dried over anhydrous Na₂SO₄ and concentrated in vacuo. The crude material was purified by flash chromatography on silica gel (n-heptane:EtOAc = 100:0 to 70:30),to afford the title compound (416 mg, 66% yield) as white solid. MS (ESI): 514.2 [M+H]⁺.

### Step c) 5-(4-bromopyrazol-1-yl)-3,3-difluoro-piperidine

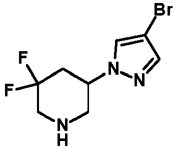

A suspension of 9H-fluoren-9-ylmethyl 3,3-difluoro-5-(p-tolylsulfonyloxy)piperidine-1-carboxylate (308 mg, 0.6 mmol, 1), 4-bromo-1H-pyrazole (99 mg, 0.66 mmol, 1.1 eq) and cesium carbonate (293 mg, 0.9 mmol, 1.5 eq) in DMF was heated at 60 °C for 1h. The reaction mixture was partitioned between EtOAc (40 ml) and 0.5 M aqueous HCl (40 ml). The layers were separated. The organic layer was extracted with one 20 ml portion of 1M aqueous HCl. The pH of the combined aq. layer was set basic and extracted with three 50 ml portions of EtOAc:THF (1:1). The combined organic layers were dried over anhydrous Na₂SO₄ and concentrated in vacuo. The crude material was purified by prep-HPLC to afford the title compound (31 mg, 15% yield) as an off-white solid. MS (ESI): 266.1/268.1 [M+H]⁺.

### Step d) 5-(4-bromopyrazol-1-yl)-3,3-difluoro-1-methyl-piperidine

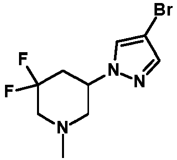

A suspension of 5-(4-bromopyrazol-1-yl)-3,3-difluoro-piperidine (31 mg, 0.12 mmol) and sodium acetate (9.56 mg, 0.12 mmol, 1 eq) in DCM (1 mL) was degased with argon for 5 min and stirred for 1h. Then formaldehyde (aq.) (12.6 mg, 11.6 uL, 0.15 mmol, 1.3 eq) was added at room temperature. Stirring was continued for 45 min. sodium triacetoxyborohydride (49 mg, 0.23 mmol, 2 eq) was added in one portion. The reaction mixture was stirred for 2 h, partitioned between EtOAc:THF (1:1) (50 ml) and sat. aqueous NaHCO₃ (40 ml). The layers were separated. The aqueous layer was extracted with two 40 ml portions of EtOAc:THF (1:1). The combined organic layers were dried over anhydrous Na₂SO₄ and concentrated in vacuo to afford the crude title compound (20 mg, 55% yield) as light yellow oil. MS (ESI): 279.1/281.8 [M+H]⁺.

### Step e) tert-butyl N-[(3R)-5-[(4-chlorophenyl)methyl]-7-[1-(5,5-difluoro-1-methyl-3-piperidyl)pyrazol-4-yl]-8-fluoro-4-oxo-2, 3-dihydro-1,5-benzothiazepin-3-yl carbamate

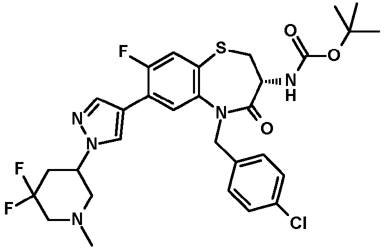

A suspension of N-[(3R)-5-(4-chlorobenzyl)-8-fluoro-4-keto-7-(4,4,5,5-tetramethyl-1,3,2-dioxaborolan-2-yl)-2,3-dihydro-1,5-benzothiazepin-3-yl]carbamic acid tert-butyl ester (90 mg, 0.11 mmol, 1.5 eq), 5-(4-bromopyrazol-1-yl)-3,3-difluoro-1-methyl-piperidine (21 mg, 0.075 mmol) and 2 M aq. tripotassium phosphate solution (112 uL, 0.225 mmol, 3 eq) in 1,4-dioxane (0.5 mL) was degased with argon for 5 min. 1,1'-bis(diphenylphosphino)ferrocenedichloro palladium(II) (8.23 mg, 0.011 mmol, 0.15 eq) was added. The reaction mixture was heated at 120 °C and stirred for 3h. The reaction mixture was partitioned between EtOAc (50 ml) and sat. aqueous NaHCO₃ (40 ml). The layers were separated. The aqueous layer was extracted with EtOAc (2 x 40 mL). The combined organic layers were washed with brine (40 mL), dried over anhydrous Na₂SO₄ and concentrated in vacuo. The crude material was purified by column chromatography on silica gel (n-heptane:(EtOAc:EtOH:ammonia 75:25:2) = 100:0 to 40:60) to afford the title compound (28.1 mg, 55% yield) as white solid. MS (ESI): 637.2 [M+H]⁺.

### Step f) tert-butyl N-[(3R)-5-[(4-chlorophenyl)methyl]-7-[1-(5,5-difluoro-1-methyl-1-oxido-piperidin-1-ium-3-yl)pyrazol-4-yl]-8-fluoro-1,1,4-trioxo-2,3-dihydro-1λ,⁶,5-benzothiazepin-3-yl]carbamate

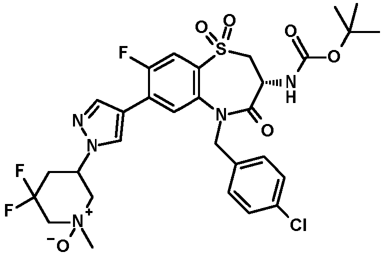

The title compound was prepared in analogy to general procedure **5** from tert-butyl N-[(3R)-5-[(4-chlorophenyl)methyl]-7-[1-(5,5-difluoro-1-methyl-3-piperidyl)pyrazol-4-yl]-8-fluoro-4-oxo-2,3-dihydro-1,5-benzothiazepin-3-yl]carbamate (28 mg, 0.044 mmol) as a white solid (36 mg, 100% yield). MS (ESI): 684.3 [M+H]⁺.

### Step g) tert-butyl N-[(3R)-5-[(4-chlorophenyl)methyl]-7-[1-(5,5-difluoro-1-methyl-3-piperidyl)pyrazol-4-yl]-8-fluoro-1,1,4-trioxo-2,3-dihydro-1λ⁶,5-benzothiazepin-3-yl]carbamate

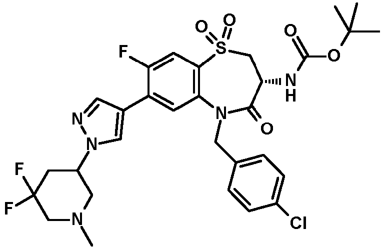

To a suspension of N-[(3R)-5-(4-chlorobenzyl)-7-[1-(5,5-difluoro-1-methyl-1-oxido-piperidin-1-ium-3-yl)pyrazol-4-yl]-8-fluoro-1,1,4-triketo-2,3-dihydro-1λ6,5-benzothiazepin-3-yl]carbamic acid tert-butyl ester (36 mg, 0.039 mmol) in 1,2-dichloroethane (0.75 mL) was added phenylboronic acid (5.29 mg, 0.043 mmol, 1.1 eq) at room temperature. The reaction mixture was heated to reflux for 2h. The reaction mixture was partitioned between DCM (40 ml) and 0.5M aqueous NaOH (40 ml). The layers were separated. The aqueous layer was extracted with EtOAc (2 x 40 mL). The combined organic layers were dried over anhydrous Na₂SO₄ and concentrated in vacuo. The crude material was purified by column chromatography on silica gel (DCM:MeOH = 100:0 to 95:5) to afford the title compound (12.5 mg, 45% yield) as white solid. MS (ESI): 668.4 [M+H]⁺.

### Step h) (3R)-3-amino-5-[(4-chlorophenyl)methyl]-7-[1-(5,5-difluoro-1-methyl-3-piperidyl)pyrazol-4-yl]-8-fluoro-1,1-dioxo-2,3-dihydro-1λ⁶,5-benzothiazepin-4-one

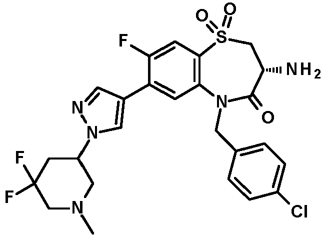

The title compound was prepared in analogy to general procedure **6d** from N-[(3R)-5-(4-chlorobenzyl)-7-[1-(5,5-difluoro-1-methyl-3-piperidyl)pyrazol-4-yl]-8-fluoro-1,1,4-triketo-2,3-dihydro-1λ6,5-benzothiazepin-3-yl]carbamic acid tert-butyl ester (11 mg, 0.016 mmol) after prep-HPLC as a yellow oil as a bis hydrochloride salt (10.2 mg, 91% yield). MS (ESI): 284.9 [M/2+H]⁺.

### Example 282

### (3R)-3-amino-7-(4-tert-butylpyrazol-1-yl)-5-[(4-chlorophenyl)methyl]-8-fluoro-1,1-dioxo-2,3-dihydro-1λ⁶,5-benzothiazepin-4-one

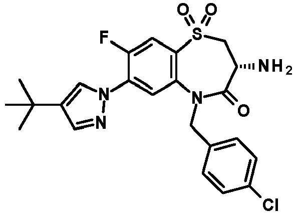

### Step a) tert-butyl N-[(3R)-7-(4-tert-butylpyrazol-1-yl)-5-[(4-chlorophenyl)methyl]-8-fluoro-4-oxo-2,3-dihydro-1,5-benzothiazepin-3-yl]carbamate

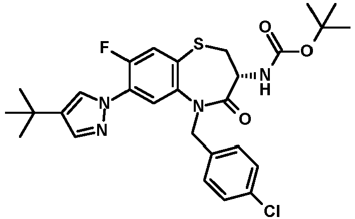

To a mixture of tert-butyl N-[(3R)-7-bromo-8-fluoro-4-oxo-3,5-dihydro-2H-1,5-benzothiazepin-3-yl]carbamate (Example 6 step b) (102.8 mg, 0.20 mmol, 1.0 eq), 4-tert-butyl-1H-pyrazole (CAS 105285-21-0, 37.2 mg, 0.3 mmol, 1.5 eq), K₂CO₃ (191.8 mg, 1.4 mmol, 7.0 eq) and CuI (56.7 mg, 0.3 mmol, 1.5 eq) in toluene (3.0 mL) was added trans-N, N'-dimethylcyclohexane-1,2-diamine (17.04 mg, 0.12 mmol, 0.6 eq) under an atmosphere of nitrogen and the mixture was stirred at reflux for 16 hrs. The reaction mixture was then concentrated under reduced pressure to remove toluene. To the residue was added water (5 mL) and then the mixture was extracted with EtOAc. The combined organic layers were dried over anhydrous Na₂SO₄, filtered and concentrated under reduced pressure to give a residue which was purified by silica gel chromatography (0 to 10% EtOAc in PE) to give the title compound (62.13mg, 0.11 mmol) as solid. MS (ESI): 559.3 [M+H]⁺.

### Step b) tert-butyl N-[(3R)-7-(4-tert-butylpyrazol-1-yl)-5-[(4-chlorophenyl)methyl]-8-fluoro-1,1,4-trioxo-2,3-dihydro-1λ⁶,5-benzothiazepin-3-yl]carbamate

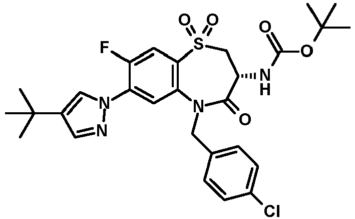

The title compound was prepared from tert-butyl N-[(3R)-7-(4-tert-butylpyrazol-1-yl)-5-[(4-chlorophenyl)methyl]-8-fluoro-4-oxo-2,3-dihydro-1,5-benzothiazepin-3-yl]carbamate (62.13mg, 0.11 mmol) in analogy to general procedure **5** to obtain the crude title compound which was used in the next reaction step without further purification. MS (ESI): 591.3 [M+H]⁺.

### Step c) (3R)-3-amino-7-(4-tert-butylpyrazol-1-yl)-5-[(4-chlorophenyl)methyl]-8-fluoro-1,1-dioxo-2,3-dihydro-1λ⁶,5-benzothiazepin-4-one

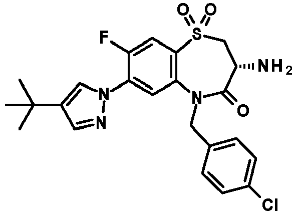

The title compound (22.6 mg, 0.046 mmol) was prepared in analogy to Example 278, step c) from crude tert-butyl N-[(3R)-7-(4-tert-butylpyrazol-1-yl)-5-[(4-chlorophenyl)methyl]-8-fluoro-1,1,4-trioxo-2,3-dihydro-1λ⁶,5-benzothiazepin-3-yl]carbamate. MS (ESI): 491.2 [M+H]⁺.

Examples 283 to 284 of the following table were prepared in analogy to Example 282, using the appropriate pyrazole building blocks.

| **Ex.** | **Structure** | **Systematic Name** | **Building Blocks** | **MS, ESI: m/z** |
|---|---|---|---|---|
| 283 | 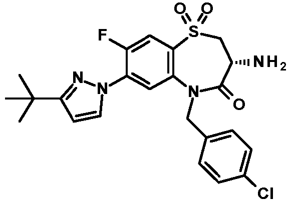 | (3R)-3-amino-7-(3-tert-butylpyrazol-1-yl)-5-[(4-chlorophenyl)methyl]-8-fluoro-1,1-dioxo-2,3-dihydro-1λ⁶,5-benzothiazepin-4-one | 3-tert-Butyl-1H-pyrazole | 491.1 [M+H]⁺ |
| | | | (CAS 15802-80-9) | |
| 284 | 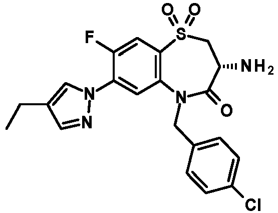 | (3R)-3-amino-5-[(4-chlorophenyl)methyl]-7-(4-ethylpyrazol-1-yl)-8-fluoro-1,1-dioxo-2,3-dihydro-1λ⁶,5-benzothiazepin-4-one | 3-Ethyl-1H-pyrazole | 463.1 [M+H]⁺ |
| | | | (CAS 17072-38-7) | |

### Example 285

### (3R)-3-amino-5-[(4-chlorophenyl)methyl]-7-(1-ethyl-1,2,4-triazol-3-yl)-8-fluoro-1,1-dioxo-2,3-dihydro-1λ⁶,5-benzothiazepin-4-one

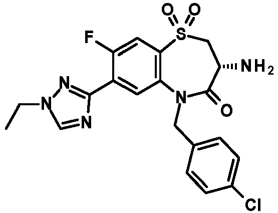

### Step a) tert-butyl N-[(3R)-5-[(4-chlorophenyl)methyl]-8-fluoro-4-oxo-7-(4, 4, 5, 5-tetramethyl-1,3,2-dioxaborolan-2-yl)-2,3-dihydro-1,5-benzothiazepin-3-yl]carbamate

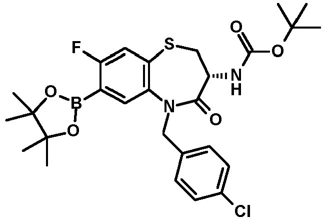

To a solution of tert-butyl N-[(3R)-7-bromo-8-fluoro-4-oxo-3,5-dihydro-2H-1,5-benzothiazepin-3-yl]carbamate (Example 6 step b) (2 g, 3.88 mmol, 1 eq) and 4,4,5,5-tetramethyl-2-(4,4,5,5-tetramethyl-1,3,2-dioxaborolan-2-yl)-1,3,2-dioxaborolane (1.97 g, 7.75 mmol, 2 eq) in dioxane (6 mL) were added KOAc (1.14 g, 11.63 mmol, 3 eq) and Pd(dppf)Cl₂•CH₂Cl₂ (316.63 mg, 388 µmol, 0.1 eq) under an atmosphere of nitrogen and the mixture was stirred at 80°C for 16 h. The reaction mixture was filtered and concentrated under reduced pressure to give a residue which was purified by silica gel chromatography (0 to 50% EtOAc in PE) to obtain a still crude title compound (2.3 g) as a yellow solid which was used as such in the next reaction step. MS (ESI): 507.2 [M-isobutene+H]⁺.

### Step b) tert-butyl N-[(3R)-5-[(4-chlorophenyl)methyl]-7-(1-ethyl-1,2,4-triazol-3-yl)-8-fluoro-4-oxo-2,3-dihydro-1,5-benzothiazepin-3-yl]carbamate

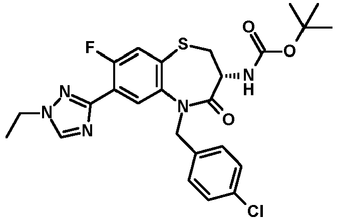

To a solution of tert-butyl N-[(3R)-5-[(4-chlorophenyl)methyl]-8-fluoro-4-oxo-7-(4,4,5,5-tetramethyl-1,3,2-dioxaborolan-2-yl)-2,3-dihydro-1,5-benzothiazepin-3-yl]carbamate (140.8 mg, 0.25 mmol, 1.0 eq) and 3-bromo-1-ethyl-1,2,4-triazole (57.2 mg, 0.325 mmol, 1.3 eq) in a mixture of aqueous K₃PO₄ (2.0 M, 0.375 mL, 0.75 mmol, 3.0 eq) and dioxane (4.0 mL) was added Pd(dppf)Cl₂ (27.4 mg, 0.04 mmol, 0.15 eq) under an atmosphere of nitrogen and the reaction mixture was stirred at reflux for 3 h. Then water (4 mL) was added and the mixture was extracted with EtOAc. The combined organic layers were dried over anhydrous Na₂SO₄, filtered and concentrated under reduced pressure to give the crude title compound, which was used in the next reaction step as such. MS (ESI): 532.2 [M+H]⁺.

### Step c) tert-butyl N-[(3R)-5-[(4-chlorophenyl)methyl]-7-(1-ethyl-1,2,4-triazol-3-yl)-8-fluoro-1,1,4-trioxo-2,3-dihydro-1λ⁶,5-benzothiazepin-3-yl]carbamate

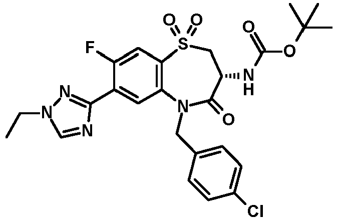

The title compound was prepared from crude tert-butyl N-[(3R)-5-[(4-chlorophenyl)methyl]-7-(1-ethyl-1,2,4-triazol-3-yl)-8-fluoro-4-oxo-2,3-dihydro-1,5-benzothiazepin-3-yl]carbamate in analogy to general procedure **5** to obtain the crude title compound which was used in the next reaction step without further purification. MS (ESI): 508.1 [M-isobutene+H]⁺.

### Step d) (3R)-3-amino-5-[(4-chlorophenyl)methyl]-7-(1-ethyl-1,2, 4-triazol-3-yl)-8-fluoro-1,1-dioxo-2,3-dihydro-1λ⁶,5-benzothiazepin-4-one

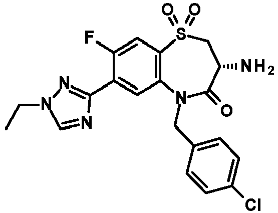

The title compound (6.68 mg, 0.014 mmol) was prepared in analogy to Example 278, step c) from crude tert-butyl N-[(3R)-5-[(4-chlorophenyl)methyl]-7-(1-ethyl-1,2,4-triazol-3-yl)-8-fluoro-1,1,4-trioxo-2,3-dihydro-1λ⁶,5-benzothiazepin-3-yl]carbamate. MS (ESI): 464.1 [M+H]⁺.

Examples 286 to 287 of the following table were prepared in analogy to Example 285, using the appropriate bromo pyrazole or bromo triazole building blocks.

| **Ex.** | **Structure** | **Systematic Name** | **Building Blocks** | **MS, ESI: m/z** |
|---|---|---|---|---|
| 286 | 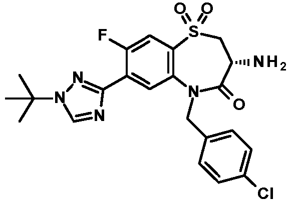 | (3R)-3-amino-7-(1-tert-butyl-1,2,4-triazol-3-yl)-5-[(4-chlorophenyl)methyl]-8-fluoro-1,1-dioxo-2,3-dihydro-1λ⁶,5-benzothiazepin-4-one | 3-Bromo-1-tert-butyl-1,2,4-triazole | 492.1 [M+H]⁺ |
| 287 | 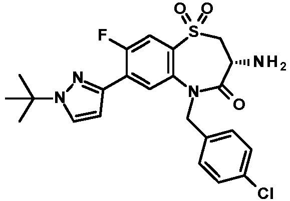 | (3R)-3-amino-7-(1-tert-butylpyrazol-3-yl)-5-[(4-chlorophenyl)methyl]-8-fluoro-1,1-dioxo-2,3-dihydro-1λ⁶,5-benzothiazepin-4-one | 3-Bromo-1-tert-butyl-pyrazole | 491.1 [M+H]⁺ |
| | | | (CAS 1779857-30-5) | |

### Intermediate 15

### 2-bromo-8-(trifluoromethyl)-[1,2,4]triazolo[1,5-a]pyrazine

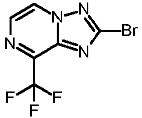

### Step a) ethyl N-[[3-(trifluoromethyl)pyrazin-2-yl]carbamothioyl]carbamate

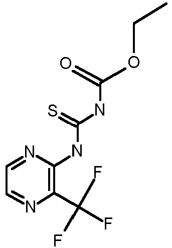

[3-(trifluoromethyl)pyrazin-2-yl]amine (1 g, 6.13 mmol) was combined with N-(thioxomethylene)carbamic acid ethyl ester (964 mg, 869 uL, 7.36 mmol, 1.2 eq) in 1,4-dioxane (10 mL) and the reaction was stirred at room temperature over night. N-(thioxomethylene)carbamic acid ethyl ester (964 mg, 869 uL, 7.36 mmol, 1.2 eq) was added again and stirring at room temperature was continued for another night. The reaction was heated to 60 °C and stirring was continued overnight. The solvent was evaporated, the mixture was purified by column chromatography on silica gel (heptane;EtOAc = 1:0 to 1:1) affording the title compound (1.87 g, 92% yield) as light brown oil. MS (ESI): 295.0 [M+H]⁺.

### Step b) 8-(trifluoromethyl)-[1,2,4]triazolo[1,5-a]pyrazin-2-amine

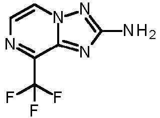

Ethyl N-[[3-(trifluoromethyl)pyrazin-2-yl]carbamothioyl]carbamate (1.87 g, 5.66 mmol) was combined with hydroxylamine hydrochloride (707 mg, 10.2 mmol, 1.8 eq) and DIPEA (2.19 g, 2.96 mL, 16.97 mmol, 3 eq) in EtOH (14 mL) and MeOH (14 mL). The reaction was heated to 65 °C and stirred overnight. The solvent was partly evaporated, the precipitate was filtered off and washed with DCM/MeOH (98:2). The white solid was dried in vacuo affording the title compound (1.04 g, 90% yield) as grey solid. MS (ESI): 204.0 [M+H]⁺.

### Step c) 2-bromo-8-(trifluoromethyl)-[1,2,4]triazolo[1,5-a]pyrazine

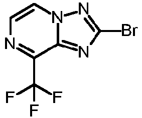

tert-butyl nitrite (380 mg, 439 uL, 3.69 mmol, 1.5 eq) and copper(I) bromide (529 mg, 3.69 mmol, 1.5 eq) were combined in MeCN (12.5 mL) and the dark green mixture was heated to 60 °C. [8-(trifluoromethyl)-[1,2,4]triazolo[1,5-a]pyrazin-2-yl]amine (500 mg, 2.46 mmol) was added, the reaction was heated to 80 °C and stirred overnight. Copper(II) bromide (412 mg, 1.85 mmol, 0.75 eq) was added and stirring was continued for 1h at 80°C. The solvent was evaporated. The crude residue was purified by column chromatography on silica gel (heptane;EtOAc = 1:0 to 1:1) affording the title compound (426 mg, 60% yield) as a white solid. MS (ESI): 268.9 [M+H]⁺.

### Example 288

### (3R)-3-amino-8-fluoro-1,1-dioxo-5-[[4-(trifluoromethoxy)phenyl]methyl]-7-[8-(trifluoromethyl)-5,6,7,8-tetrahydro-[1,2,4]triazolo[1,5-a]pyrazin-2-yl]-2,3-dihydro-1λ⁶,5-benzothiazepin-4-one

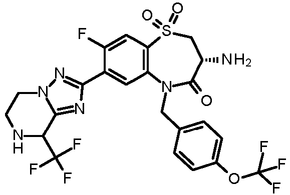

Step a) *tert-butyl N-[(3R)-7-bromo-8-fluoro-4-oxo-5-[[4-(trifluoromethoxy)phenyl]methyl]-2, 3-dihydro-1,5-benzothiazepin-3-yl]carbamate*

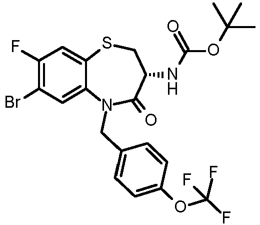

tert-butyl N-[(3R)-7-bromo-8-fluoro-4-oxo-3,5-dihydro-2H-1,5-benzothiazepin-3-yl]carbamate (300 mg, 0.77 mmol) was stirred with 1-(bromomethyl)-4-(trifluoromethoxy)benzene (215 mg, 134 uL, 0.843 mmol, 1.1 eq), potassium carbonate (318 mg, 2.3 mmol, 3 eq) and potassium iodide (63.64 mg, 0.383 mmol, 0.5 eq) in DMSO (6 mL) at RT overnight. 0.5eq of 1-(bromomethyl)-4-(trifluoromethoxy)benzene was added again and stirring was continued for 2h. The crude material was purified by column chromatography (heptane;EtOAc = 1:0 to 0:1) affording the title compound (433 mg, 99%) as colorless foam. MS (ESI): 510.9 [M-isobutene+H]⁺.

### Step b) tert-butyl N-[(3R)-7-bromo-8-fluoro-1,1,4-trioxo-5-[[4-(trifluoromethoxy)phenyl]methyl]-2,3-dihydro-1λ⁶,5-benzothiazepin-3-yl]carbamate

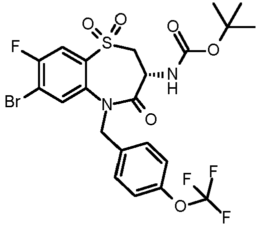

tert-butyl N-[(3R)-7-bromo-8-fluoro-4-oxo-5-[[4-(trifluoromethoxy)phenyl]methyl]-2,3-dihydro-1,5-benzothiazepin-3-yl]carbamate (430 mg, 0.76 mmol) was stirred with 3-chloroperoxybenzoic acid (426 mg, 1.9 mmol, 2.5 eq) in DCM (7 mL) at RT overnight. The crude material was purified by column chromatography (heptane;EtOAc = 1:0 to 0:1) affording the title compound (510 mg, 88% yield) as a white solid. MS (ESI): 543.0 [M+H]⁺.

### Step c) [(3R)-3-(tert-butoxycarbonylamino)-8-fluoro-1,1,4-trioxo-5-[[4-(trifluoromethoxy)phenylmethyl]-2,3-dihydro-1λ⁶,5-benzothiazepin-7-yl]boronic acid

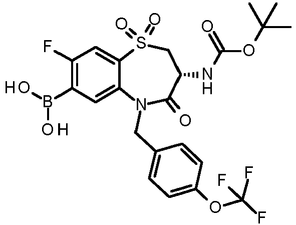

tert-butyl N-[(3R)-7-bromo-8-fluoro-1,1,4-trioxo-5-[[4-(trifluoromethoxy)phenyl]methyl]-2,3-dihydro-1λ⁶,5-benzothiazepin-3-yl]carbamate (250 mg, 0.33 mmol) was stirred with bis(pinacolato)diboron (184.7 mg, 0.727 mmol, 2.2 eq), 1,1'-bis(diphenylphosphino)ferrocene-palladium(II)dichloride dichloromethane adduct (31 mg, 0.038 mmol, 0.115 eq) and potassium acetate (97.3 mg, 0.99 mmol, 3 eq) in 1,4-dioxane (4.5 mL) at 100°C for 1h in a closed vial. Water was added and extracted three times with EtOAc. The organic layer was dried over magnesium sulfate, filtered and evaporated, affording the crude title compound (510 mg) as light brown solid. MS (ESI): 507.1 [M-isobutene+H]⁺.

### Step d) tert-butyl N-[(3R)-8-fluoro-1,1,4-trioxo-5-[[4-(trifluoromethoxy)phenyl]methyl]-7-[8-(trifluoromethyl)-[1,2,4]triazolo[1,5-a]pyrazin-2-yl]-2,3-dihydro-1λ⁶,5-benzothiazepin-3-yl]carbamate

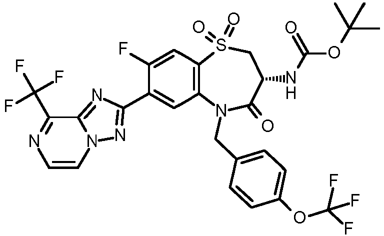

[(3R)-3-(tert-butoxycarbonylamino)-8-fluoro-1,1,4-trioxo-5-[[4-(trifluoromethoxy)phenyl]methyl]-2,3-dihydro-1λ⁶,5-benzothiazepin-7-yl]boronic acid (315 mg, 0.56 mmol), 2-bromo-8-(trifluoromethyl)-[1,2,4]triazolo[1,5-a]pyrazine (Intermediate 15) (149mg, 0.560 mmol), Na₂CO₃ (178 mg, 1.68 mmol, 3 eq) and [1,1'-bis(diphenylphosphino)ferrocene]dichloropalladium(II) complex with dichloromethane (68.6 mg, 0.084 mmol, 0.15 eq) in 1,4-dioxane (11.6 mL) and water (2.32 mL) in a sealed tube was heated to 100 °C for 3.5h. The solvent was evaporated, the crude residue purified by column chromatography on silica gel (heptane;EtOAc = 1:0 to 1:1) affording the title compound (75 mg, 18% yield) as light yellow solid. MS (ESI): 649.2 [M-isobutene+H]⁺.

### Step e) tert-butyl N-[(3R)-8-fluoro-1,1,4-trioxo-5-[[4-(trifluoromethoxy)phenyl]methyl]-7-[8-(trifluoromethyl)-5,6,7,8-tetrahydro-[1,2,4]triazolo[1,5-a]pyrazin-2-yl]-2,3-dihydro-1λ⁶,5-benzothiazepin-3-yl]carbamate

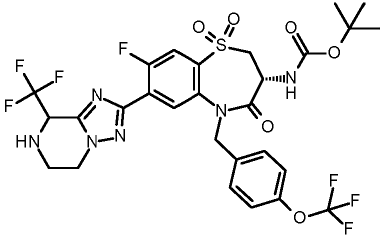

tert-butyl N-[(3R)-8-fluoro-1,1,4-trioxo-5-[[4-(trifluoromethoxy)phenyl]methyl]-7-[8-(trifluoromethyl)-[1,2,4]triazolo[1,5-a]pyrazin-2-yl]-2,3-dihydro-1λ⁶,5-benzothiazepin-3-yl]carbamate (65 mg, 0.093 mmol) was hydrogenated under H₂ at 50bar using 5% Pt/C as catalyst. The reaction was shaken overnight at 80°C. The catalyst was filtered off, washed and the solvent was evaporated and dried under high vacuum affording the title compound (49 mg, 59% yield) as light yellow solid. MS (ESI): 653.1 [M-isobutene+H]⁺.

### Step f) (3R)-3-amino-8-fluoro-1,1-dioxo-5-[[4-(trifluoromethoxy)phenyl]methyl]-7-[8-(trifluoromethyl)-5,6,7,8-tetrahydro-[1,2,4]triazolo[1,5-a]pyrazin-2-yl]-2,3-dihydro-1λ⁶,5-benzothiazepin-4-one

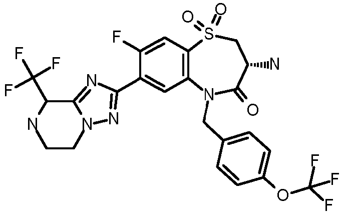

The title compound was prepared in analogy to general procedure 6d from tert-butyl N-[(3R)-8-fluoro-1,1,4-trioxo-5-[[4-(trifluoromethoxy)phenyl]methyl]-7-[8-(trifluoromethyl)-5,6,7,8-tetrahydro-[1,2,4]triazolo[1,5-a]pyrazin-2-yl]-2,3-dihydro-1λ⁶,5-benzothiazepin-3-yl]carbamate (47 mg, 0.066 mmol) and was obtained as a white solid (20 mg, 49% yield). MS (ESI): 609.3 [M+H]⁺.

### Example 289

### (3R)-3-amino-7-(5-tert-butyl-1,2,4-oxadiazol-3-yl)-5-[(4-chlorophenyl)methyl]-8-fluoro-1,1-dioxo-2,3-dihydro-1λ⁶,5-benzothiazepin-4-one

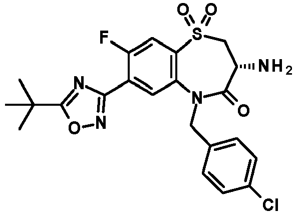

### Step a) tert-Butyl N-[(3R)-5-[(4-chlorophenyl)methyl]-8-fluoro-7-[(Z)-N'-hydroxycarbamimidoyl] -4-oxo-2,3-dihydro-1,5-benzothiazepin-3-yl]carbamate

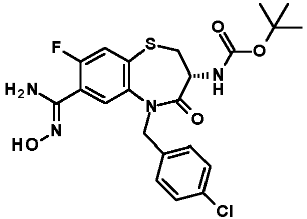

A mixture of tert-butyl N-[(3R)-5-[(4-chlorophenyl)methyl]-7-cyano-8-fluoro-4-oxo-2,3-dihydro-1,5-benzothiazepin-3-yl]carbamate (Example 7 step a) (200.0 mg, 0.430 mmol, 1 eq), K₂CO₃ (155.58 mg, 1.13 mmol, 2.6 eq) and hydroxylamine hydrochloride (78.22 mg, 1.13 mmol, 2.6 eq) in DMF (5 mL) was stirred at 80°C for 16 h. The reaction mixture was cooled to room temperature and EtOAc (10 mL) and H₂O (10 mL) were added. The layers were separated and the organic layer was washed with brine (10 mL x 3) and dried over Na₂SO₄, filtered and concentrated. The remaining residue was purified by silica gel chromatography (0 to 20% EtOAc in PE) to afford the crude product (150 mg) as a light yellow solid, which was then further purified by prep-TLC (PE.EtOAc = 1:1) to afford the title compound (40 mg, 0.080 mmol, 18% yield) as a light yellow solid. MS (ESI): 439.0 [M-isubutene+H]⁺.

### Step b) tert-butyl N-[(3R)-7-(5-tert-butyl-1,2,4-oxadiazol-3-yl)-5-[(4-chlorophenyl)methyl]-8-fluoro-4-oxo-2,3-dihydro-1,5-benzothiazepin-3-yl[carbamate

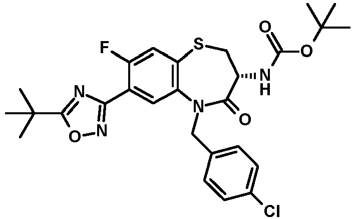

To a solution of pivalic acid (39.62 mg, 0.390 mmol, 1.2 eq) in MeCN (4 mL) were added EDCI (80.56 mg, 0.420 mmol, 1.3 eq) and HOBT (64.35 mg, 0.420 mmol, 1.3 eq) at 25°C. After the mixture stirred for 0.5 h at 25°C, DIPEA (0.11 mL, 0.650 mmol, 2 eq) and tert-butyl N-[(3R)-5-[(4-chlorophenyl)methyl]-8-fluoro-4-oxo-7-[(Z)-N'-hydroxycarbamimidoyl]-2,3-dihydro-1,5-benzothiazepin-3-yl]carbamate (160 mg, 0.323 mmol) were added and the mixture was stirred for 3 h at 25°C. After that, the mixture was heated to 80°C and stirred for another 24 h. The reaction mixture was concentrated under vacuum, the residue was diluted with EtOAc (5 mL), washed with brine (5 mL x 3), dried over anhydrous Na₂SO₄ and concentrated under vacuum. The remaining residue was purified by column chromatography on silica gel (PE:EtOAc=10:1 to 2:1) to obtain the title compound (160 mg, 0.290 mmol, 88% yield) as a light brown gum. MS (ESI): 505.1 [M-isubutene+H]⁺.

### Step c) tert-butyl N-[(3R)-7-(5-tert-butyl-1,2,4-oxadiazol-3yl)-5-[(4-chlorophenyl)methyl]-8-fluoro-1,1,4-trioxo-2,3-dihydro-1λ⁶,5-benzothiazepin-3-yl]carbamate

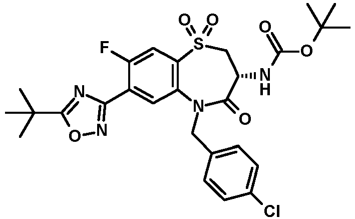

The title compound was prepared from tert-butyl N-[(3R)-7-(5-tert-butyl-1,2,4-oxadiazol-3-yl)-5-[(4-chlorophenyl)methyl]-8-fluoro-4-oxo-2,3-dihydro-1,5-benzothiazepin-3-yl]carbamate (160 mg, 0.285 mmol) in analogy to general procedure 5 and was obtained as light yellow solid (150 mg, 86%). MS (ESI): 537.3 [M-isobutene+H]⁺.

### Step d) (3R)-3-amino-7-(5-tert-butyl-1,2,4-oxadiazol-3-yl)-5-[(4-chlorophenyl)methyl]-8-fluoro-1,1-dioxo-2,3-dihydro-1λ⁶,5-benzothiazepin-4-one

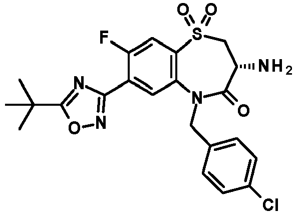

The title compound was prepared in analogy to general procedure **6b** from tert-butyl N-[(3R)-7-(5-tert-butyl-1,2,4-oxadiazol-3-yl)-5-[(4-chlorophenyl)methyl]-8-fluoro-1,1,4-trioxo-2,3-dihydro-1λ⁶,5-benzothiazepin-3-yl]carbamate (150 mg, 0.253 mmol) and was obtained as a white solid (37 mg, 29% yield). MS (ESI): 493.1 [M+H]⁺.

Examples 290 to 303 of the following table was prepared in analogy to Example 289, using the appropriate carboxylic acid building block.

| **Ex.** | **Structure** | **Systematic Name** | **Building Block, General procedure** | **MS, ESI: m/z** |
|---|---|---|---|---|
| 290 | 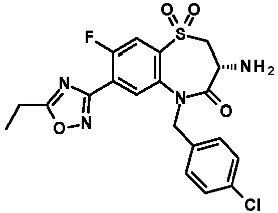 | (3R)-3-amino-5-[(4-chlorophenyl)methyl]-7-(5-ethyl-1,2,4-oxadiazol-3-yl)-8-fluoro-1,1-dioxo-2,3-dihydro-1λ⁶,5-benzothiazepin-4-one | Propionic acid | 465.1 [M+H]⁺ |
| | | | (CAS 79-09-4) | |
| | | (*) | | |
| 291 | 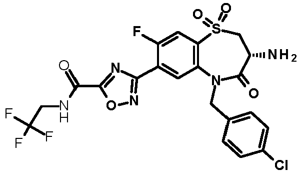 | 3-[(3R)-3-amino-5-[(4-chlorophenyl)methyl]-8-fluoro-1,1,4-trioxo-2,3-dihydro-1λ⁶,5-benzothiazepin-7-yl]-N-(2,2,2-trifluoroethyl)-1,2,4-oxadiazole-5-carboxamide | 2-Oxo-2-(2,2,2-trifluoroet hylamino) acetic acid | 562.1 [M+H]⁺ |
| | | | (CAS 1156726-64-5) | |
| | | (*) | **9b, 6b** | |
| 292 | 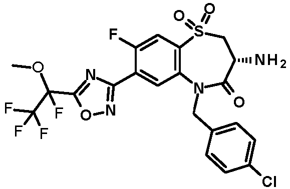 | (3R)-3-amino-5-[(4-chlorophenyl)methyl]-8-fluoro-1,1-dioxo-7-[5-(1,2,2,2-tetrafluoro-1-methoxy-ethyl)-1,2,4-oxadiazol-3-yl]-2,3-dihydro-1λ⁶,5-benzothiazepin-4-one | (2,3,3,3-Tetrafluor o-2-methoxy-propanoic acid | 567.1 [M+H]⁺ |
| | | | (CAS 10186-64-8) | |
| | | (*) | **10a, 11a, 6d** | |
| 293 | 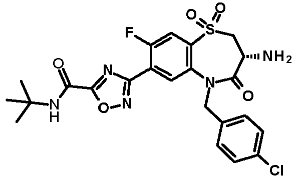 | 3-[(3R)-3-amino-5-[(4-chlorophenyl)methyl]-8-fluoro-1,1,4-trioxo-2,3-dihydro-1λ⁶,5-benzothiazepin-7-yl]-N-tert-butyl-1,2,4-oxadiazole-5-carboxamide | 2-(tert-Butylamin o)-2-oxo-acetic acid | 536.0 [M+H]⁺ |
| | | | (CAS 169772-25-2) | |
| | | | **9c, 6b** | |
| 294 | 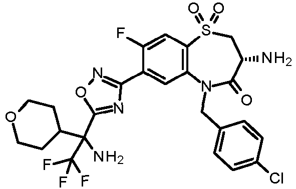 | (3R)-3-amino-7-[5-(1-amino-2,2,2-trifluoro-1-tetrahydropyran-4-yl-ethyl)-1,2,4-oxadiazol-3-yl]-5-[(4-chlorophenyl)methyl]-8-fluoro-1,1-dioxo-2,3-dihydro-1λ⁶,5-benzothiazepin-4-one | 2-(tert-Butoxycar bonylamin o)-3,3,3-trifluoro-2-tetrahydro pyran-4-yl-propanoic acid | 618.3 [M+H]⁺ |
| | | (**) | **10b, 11a, 6d** | |
| 295 | 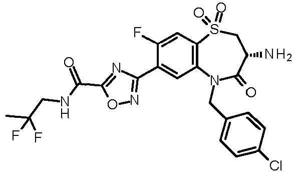 | 3-[(3R)-3-amino-5-[(4-chlorophenyl)methyl]-8-fluoro-1,1,4-trioxo-2,3-dihydro-1λ⁶,5-benzothiazepin-7-yl]-N-(2,2-difluoropropyl)-1,2,4-oxadiazole-5-carboxamide (*) | 2-(2,2-Difluoropr opylamino )-2-oxo-acetic acid | 558.1 [M+H]⁺ |
| | | | (CAS 2002648-57-7) | |
| | | | **9b, 6b** | |
| 296 | 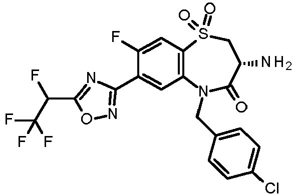 | (3R)-3-amino-5-[(4-chlorophenyl)methyl]-8-fluoro-1,1-dioxo-7-[5-(1,2,2,2-tetrafluoroethyl)-1,2,4-oxadiazol-3-yl]-2,3-dihydro-1λ⁶,5-benzothiazepin-4-one (*) | 2,3,3,3-Tetrafluor opropanoic acid | 537.1 [M+H]⁺ |
| | | | (CAS 359-49-9) | |
| | | | **10c, 11b, 6d** | |
| 297 | 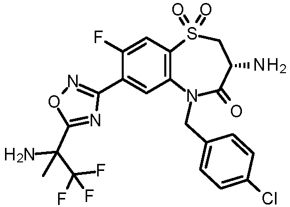 | (3R)-3-amino-7-[5-(1-amino-2,2,2-trifluoro-1-methylethyl)-1,2,4-oxadiazol-3-yl]-5-[(4-chlorophenyl)methyl]-8-fluoro-1,1-dioxo-2,3-dihydro-1λ⁶,5-benzothiazepin-4-one | 2-(tert-Butoxycar bonylamin o)-3,3,3-trifluoro-2-methylpropanoic acid | 548.1 [M+H]⁺ |
| | | (**) | (CAS 1900683-56-8) | |
| | | | **10c, 11b, 6d** | |
| 298 | 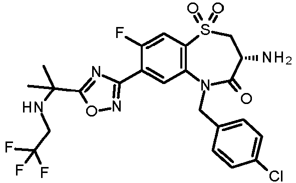 | (3R)-3-amino-5-[(4-chlorophenyl)methyl]-8-fluoro-7-[5-[1-methyl-1-(2,2,2-trifluoroethylamino)ethyl]-1,2,4-oxadiazol-3-yl]-1,1-dioxo-2,3-dihydro-1λ⁶,5-benzothiazepin-4-one | 2-Methyl-2-(2,2,2-trifluoroet hylamino) propanoic acid | 576.2 [M+H]⁺ |
| | | | (CAS 1179284-80-0) | |
| | | (**) | **9a, 6d** | |
| 299 | 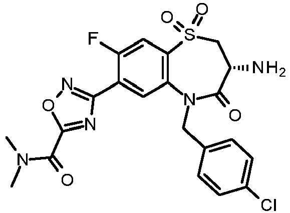 | 3-[(3R)-3-amino-5-[(4-chlorophenyl)methyl]-8-fluoro-1,1,4-trioxo-2,3-dihydro-1λ⁶,5-benzothiazepin-7-yl]-N,N-dimethyl-1,2,4-oxadiazole-5-carboxamide | 2-(Dimethyla mino)-2-oxo-acetic acid (CAS 32833-96-8) | 508.1 [M+H]⁺ |
| | | (*) | **9c, 6d** | |
| 300 | 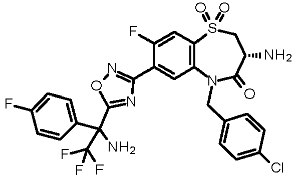 | (3R)-3-amino-7-[5-[1-amino-2,2,2-trifluoro-1-(4-fluorophenyl)ethyl]-1,2,4-oxadiazol-3-yl]-5-[(4-chlorophenyl)methyl]-8-fluoro-1,1-dioxo-2,3-dihydro-1λ⁶,5-benzothiazepin-4-one | 2-(tert-Butoxycar bonylamin o)-3,3,3-trifluoro-2-(4-fluorophen yl)propanoic acid | 628.4 [M+H]⁺ |
| | | (*) | **10b, 11a, 6d** | |
| 301 | 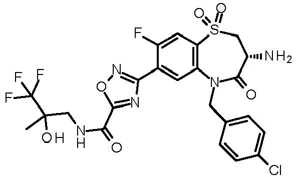 | 3-[(3R)-3-amino-5-[(4-chlorophenyl)methyl]-8-fluoro-1,1,4-trioxo-2,3-dihydro-1λ⁶,5-benzothiazepin-7-yl]-N-(3,3,3-trifluoro-2-hydroxy-2-methyl-propyl)-1,2,4-oxadiazole-5-carboxamide | 2-Oxo-2-[(3,3,3-trifluoro-2-hydroxy-2-methylpropyl)ami no]acetic acid | 606.1 [M+H]⁺ |
| | | (*) | (CAS18646 30-88-5) | |
| | | | **9b, 6b** | |
| 302 | 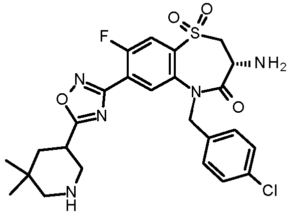 | (3R)-3-amino-5-[(4-chlorophenyl)methyl]-7-[5-(5,5-dimethyl-3-piperidyl)-1,2,4-oxadiazol-3-yl]-8-fluoro-1,1-dioxo-2,3-dihydro-1λ⁶,5- benzothiazepin-4-one | 1-tert-Butoxycar bonyl-5,5-dimethylpiperidine-3-carboxylic acid | 548.3 [M+H]⁺ |
| | | (*) | (CAS 1781347-99-6) | |
| | | | **10c, 11b, 6d** | |
| 303 | 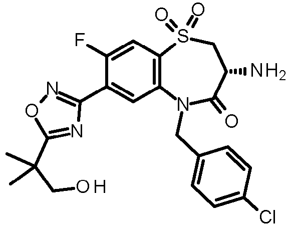 | (3R)-3-amino-5-[(4-chlorophenyl)methyl]-8-fluoro-7-[5-(2-hydroxy-1,1-dimethyl-ethyl)-1,2,4-oxadiazol-3-yl]-1,1-dioxo-2,3-dihydro-1λ⁶,5-benzothiazepin-4-one | 3-Hydroxy-2,2-dimethyl-propanoic acid | 509.2 [M+H]⁺ |
| | | | (CAS 4835-90-9) | |
| | | | **9a, 6d** | |

| | | | | |
|---|---|---|---|---|
| * as a hydrochloride salt ** as dihydrochloride salt | | | | |

### Example 304

### 4-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-N,4-dimethyl-piperidine-1-carboxamide

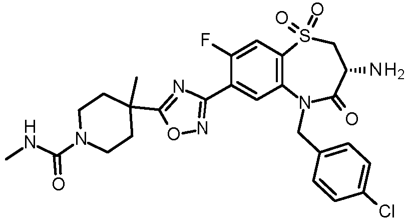

### Step a) benzyl 4-[3-[(3R)-3-(tert-butoxycarbonylamino)-5-[(4-chlorophenyl)methyl]-8-fluoro-4-oxo-2,3-dihydro-1,5-benzothiazepin-7-yl]-1,2,4-oxadiazol-5-yl]-4-methyl-piperidine-1-carboxylate

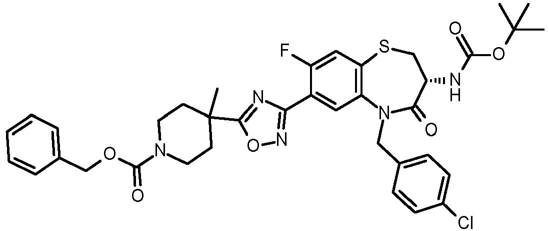

The title compound was prepared in analogy to general procedure 9a from tert-butyl N-[(3R)-5-[(4-chlorophenyl)methyl]-8-fluoro-7-[(Z)-N'-hydroxycarbamimidoyl]-4-oxo-2,3-dihydro-1,5-benzothiazepin-3-yl]carbamate (Example 289, step a)) (703 mg, 1.21 mmol, 1 eq) and 1-benzyloxycarbonyl-4-methyl-piperidine-4-carboxylic acid (CAS 203522-12-7) and was obtained as a white solid (764 mg, 82% yield). MS (ESI): 636.2 [M-isobutene-CO₂+H]⁺.

### Step b) benzyl 4-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-4-methyl-piperidine-1-carboxylate

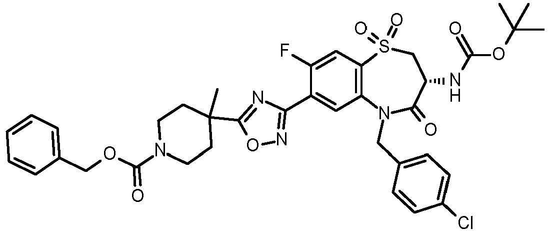

The title compound was prepared in analogy to general procedure 5 from benzyl 4-[3-[(3R)-3-(tert-butoxycarbonylamino)-5-[(4-chlorophenyl)methyl]-8-fluoro-4-oxo-2,3-dihydro-1,5-benzothiazepin-7-yl]-1,2,4-oxadiazol-5-yl]-4-methyl-piperidine-1-carboxylate (764 mg, 1.04 mmol, 1 eq) and was obtained as a white solid (596 mg, 70% yield). MS (ESI): 668.3 [M-isobutene-CO₂+H]⁺.

### Step c) tert-butylN-[(3R)-5-[(4-chlorophenyl)methyl]-8-fluoro-7-[5-(4-methyl-4-piperidyl)-1,2,4-oxadiazol-3-yl]-1,1,4-trioxo-2,3-dihydro-1λ⁶,5-benzothiazepin-3-yl]carbamate

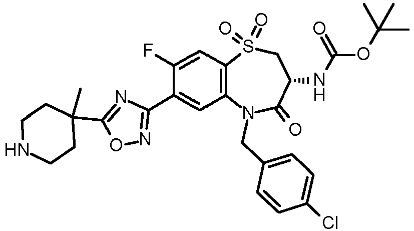

A solution of benzyl 4-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-4-methylpiperidine-1-carboxylate (596 mg, 0.729 mmol, 1 eq) in MeOH (15 mL) was purged with argon three times. Then Pd/C (38.8 mg, 0.036 mmol, 0.05 eq) was added and the mixture was stirred for 60 min under an atmosphere of hydrogen gas. The mixture was filtered and the filter cake was washed with MeOH. The filtrate was concentrated in vacuo and the remaining was purified by column chromatography on silica gel (DCM/MeOH 100:0 to 90:10) to afford the title compound (317 mg, 67%) as white solid. MS (ESI): 634.3 [M+H]⁺.

### Step d) tert-butyl N-[(3R)-5-[(4-chlorophenyl)methyl]-8-fluoro-7-[5-[4-methyl-1-(methylcarbamoyl)-4-piperidyl]-1,2,4-oxadiazol-3-yl]-1,1,4-trioxo-2,3-dihydro-1λ⁶,5-benzothiazepin-3-yl]carbamate

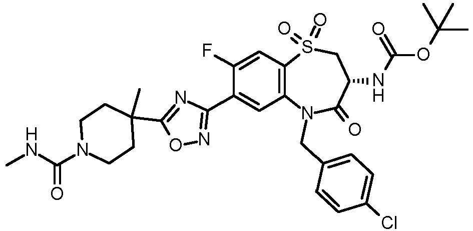

To a solution of tert-butyl N-[(3R)-5-[(4-chlorophenyl)methyl]-8-fluoro-7-[5-(4-methyl-4-piperidyl)-1,2,4-oxadiazol-3-yl]-1,1,4-trioxo-2,3-dihydro-1λ⁶,5-benzothiazepin-3-yl]carbamate (50 mg, 0.079 mmol, 1 eq) and pyridine (18.71 mg, 19.13 µL, 0.237 mmol, 3 eq) in 1,2-dichloroethane (1.25 mL) was added triphosgene (16.38 mg, 0.055 mmol, 0.700 eq) at 0°C in one portion. The cooling bath was removed and stirring was continued for 2 h. Methylamine (2M in THF, 118.27 µL, 0.237 mmol, 3 eq) was added and the mixture was stirred for 1 h. The reaction mixture was partitioned between DCM (50 mL) and water (25 mL) and the layers were separated. The aqueous layer was extracted with two 20 mL portions of DCM. The combined organic layers were dried over anhydrous sodium sulfate and concentrated in vacuo. The remaining material was purified by column chromatography on silica gel (DCM/MeOH 100:0 to 95:5) to afford the title compound (6.5 mg, 11% yield) as white solid as a hydrochloride salt. MS (ESI): 691.3 [M+H]⁺.

### Step e) 4-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-N,4-dimethyl-piperidine-1-carboxamide

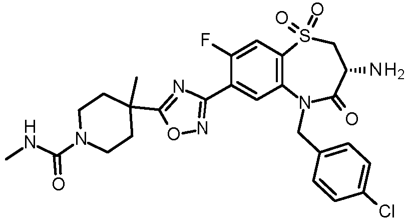

The title compound was prepared in analogy to general procedure 6d from tert-butyl N-[(3R)-5-[(4-chlorophenyl)methyl]-8-fluoro-7-[5-[4-methyl-1-(methylcarbamoyl)-4-piperidyl]-1,2,4-oxadiazol-3-yl]-1,1,4-trioxo-2,3-dihydro-1λ⁶,5-benzothiazepin-3-yl]carbamate (6.5 mg, 0.009 mmol, 1 eq) and was obtained as a white solid (4.5 mg, 76% yield). MS (ESI): 591.2 [M+H]⁺.

Example 305 of the following table was prepared in analogy to Example 304, using the appropriate amine building block.

| **Ex.** | **Structure** | **Systematic Name** | **Building Block** | **MS, ESI: m/z** |
|---|---|---|---|---|
| | 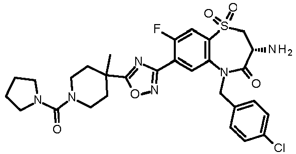 | (3R)-3-amino-5-[(4-chlorophenyl)methyl]-8-fluoro-7-[5-[4-methyl-1-(pyrrolidine-1-carbonyl)-4-piperidyl]-1,2,4-oxadiazol-3-yl]-1,1-dioxo-2,3-dihydro-1λ⁶,5-benzothiazepin-4-one (*) | Pyrrolidine | 631.3 [M+H]⁺ |
| 305 | | | (CAS123-75-1) | |

| | | | | |
|---|---|---|---|---|
| * as hydrochloride salt | | | | |

### Example 306

### (3R)-3-amino-5-[(4-chlorophenyl)methyl]-7-[5-(1-ethyl-4-methyl-4-piperidyl)-1,2,4-oxadiazol-3-yl]-8-fluoro-1,1-dioxo-2,3-dihydro-1λ⁶,5-benzothiazepin-4-one

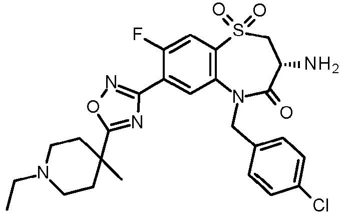

### Step a) tert-butyl N-[(3R)-5-[(4-chlorophenyl)methyl]-7-[5-(1-ethyl-4-methyl-4-piperidyl)-1,2,4-oxadiazol-3-yl]-8-fluoro-1,1,4-trioxo-2,3-dihydro-1λ⁶,5-benzothiazepin-3-yl]carbamate

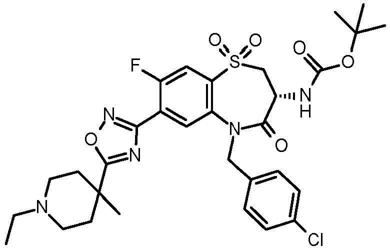

To a solution of tert-butyl N-[(3R)-5-[(4-chlorophenyl)methyl]-8-fluoro-7-[5-(4-methyl-4-piperidyl)-1,2,4-oxadiazol-3-yl]-1,1,4-trioxo-2,3-dihydro-1λ⁶,5-benzothiazepin-3-yl]carbamate (Example 304, step c)) (20 mg, 0.032 mmol, 1 eq) in MeOH (0.297 mL) were added acetaldehyde (13.89 mg, 17.81 µL, 0.315 mmol, 10 eq) and sodium triacetoxyborohydride (66.85 mg, 0.315 mmol, 10 eq). The reaction mixture was stirred at room temperature for 1 hour. The reaction mixture was poured into water and the mixture was extracted with DCM (3 x 10mL). The combined organic extracts were washed with brine (10mL), dried over MgSO₄, filtered and evaporated in vacuo. The crude material was purified by column chromatography on silica gel (EtOAc in heptane, 0 to 100% EtOAc) to afford the title compound (12.1 mg, 56% yield) as orange solid. MS (ESI): 662.4 [M+H]⁺.

### Step b) (3R)-3-amino-5-[(4-chlorophenyl)methyl]-7-[5-(1-ethyl-4-methyl-4-piperidyl)-1,2,4-oxadiazol-3-yl]-8-fluoro-1,1-dioxo-2,3-dihydro-1λ⁶,5-benzothiazepin-4-one

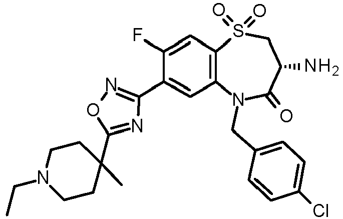

The title compound was prepared in analogy to general procedure **6d** from tert-butyl N-[(3R)-5-[(4-chlorophenyl)methyl]-7-[5-(1-ethyl-4-methyl-4-piperidyl)-1,2,4-oxadiazol-3-yl]-8-fluoro-1,1,4-trioxo-2,3-dihydro-1λ⁶,5-benzothiazepin-3-yl]carbamate (12.1 mg, 0.018 mmol, 1 eq) and was obtained as a hydrochloride salt as a brown solid (10 mg, 85% yield). MS (ESI): 562.2 [M+H]⁺.

### Example 307

### methyl 4-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-4-methyl-piperidine-1-carboxylate

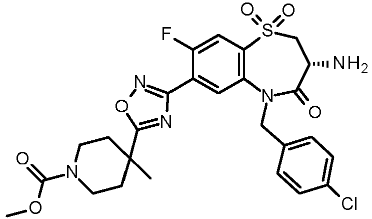

### Step a) methyl 4-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-4-methyl-piperidine-1-carboxylate

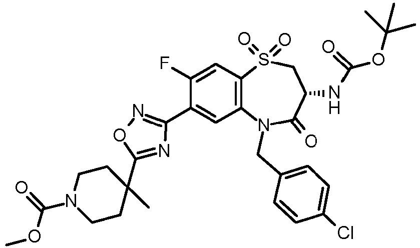

To a solution of tert-butyl N-[(3R)-5-[(4-chlorophenyl)methyl]-8-fluoro-7-[5-(4-methyl-4-piperidyl)-1,2,4-oxadiazol-3-yl]-1,1,4-trioxo-2,3-dihydro-1λ⁶,5-benzothiazepin-3-yl]carbamate (Example 304, step c) (20 mg, 0.032 mmol, 1 eq) in DCM (0.631 mL) were added methyl chloroformate (3.28 mg, 2.69 µL, 0.035 mmol, 1.1 eq) and DIPEA (10.1 mg, 13.77 µL, 0.079 mmol, 2.5 eq) and the reaction mixture was stirred at room temperature for 1 h. The reaction mixture was poured on water and extracted with DCM. The combined organic extracts were washed with brine (10 mL), dried over MgSO₄, filtered and evaporated in vacuo affording the title compound (22.5 mg, 100% yield) as white solid. MS (ESI): 592.2 [M- isobutene-CO₂+H]⁺.

### Step b) methyl 4-[3-[(3R)-3-amino-5-[(4-chlorophenyl)methyl]-8-fluoro-1,1,4-trioxo-2, 3-dihydro-1λ⁶,5-benzothiazepin-7-yl]-1,2,4-oxadiazol-5-yl]-4-methyl-piperidine-1-carboxylate

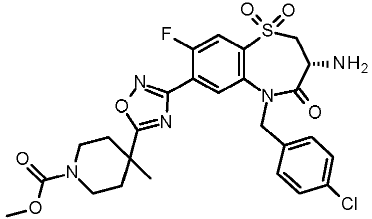

The title compound was prepared in analogy to general procedure **6d** from methyl 4-[3-[(3R)-3-(tert-butoxycarbonylamino)-5-[(4-chlorophenyl)methyl]-8-fluoro-1,1,4-trioxo-2,3-dihydro-1λ⁶ ,5-benzothiazepin-7-yl]-1,2,4-oxadiazol-5-yl]-4-methyl-piperidine-1-carboxylate (22.5 mg, 0.033 mmol, 1 eq) and was obtained as a hydrochloride salt as an off-white solid (18.2 mg, 89% yield). MS (ESI): 592.3 [M+H]⁺.

### Example 308

### (3R)-3-amino-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1-oxo-2,3-dihydro- 1λ⁴ ,5-benzothiazepin-4-one

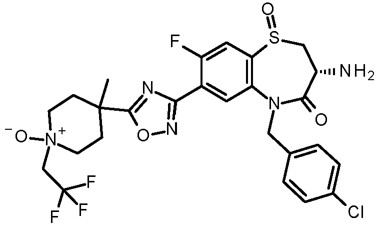

### Step a) [(Z)-[amino-[(3R)-3-(tert-butoxycarbonylamino)-5-[(4-chlorophenyl)methyl]-8-fluoro-4-oxo-2,3-dihydro-1,5-benzothiazepin-7-yl]methylene]amino] 4-methyl-1-(2,2,2-trifluoroethyl)piperidine-4-carboxylate

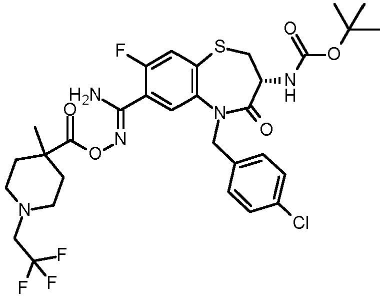

The title compound was prepared in analogy to general procedure **10a** from tert-butyl N-[(3R)-5-[(4-chlorophenyl)methyl]-8-fluoro-7-[(Z)-N'-hydroxycarbamimidoyl]-4-oxo-2,3-dihydro-1,5-benzothiazepin-3-yl]carbamate (Example 289, step a)) (150 mg, 0.270 mmol, 1 eq) and 4-methyl-1-(2,2,2-trifluoroethyl)piperidine-4-carboxylic acid (CAS 1340224-92-1) and was obtained as a light red solid (96 mg, 51% yield). MS (ESI): 703.3 [M+H]⁺.

### Step b) tert-butylN-[(3R)-5-[(4-chlorophenyl)methyl]-8-fluoro-7-[5-[4-methyl-1-(2,2,2-trifluoroethyl)-4-piperidyl]-1,2,4-oxadiazol-3-yl]-4-oxo-2,3-dihydro-1,5-benzothiazepin-3-yl]carbamate

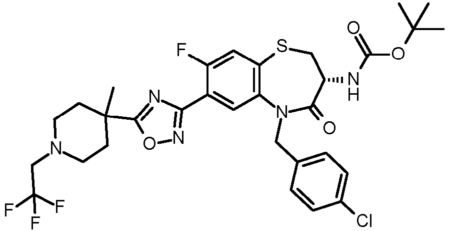

The title compound was prepared in analogy to general procedure **11b** from [(Z)-[amino-[(3R)-3-(tert-butoxycarbonylamino)-5-[(4-chlorophenyl)methyl]-8-fluoro-4-oxo-2,3-dihydro-1,5-benzothiazepin-7-yl]methylene]amino] 4-methyl-1-(2,2,2-trifluoroethyl)piperidine-4-carboxylate (96 mg, 0.137 mmol, 1 eq) and was obtained as a red solid (81.1 mg, 86% yield). MS (ESI): 684.3 [M+H]⁺.

### Step c) tert-butyl N-[(3R)-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1,4-dioxo-2,3-dihydro-1λ⁴,5-benzothiazepin-3-yl]carbamate

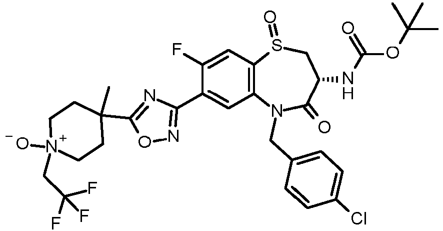

The title compound was prepared in analogy to general procedure **5** from tert-butyl N-[(3R)-5-[(4-chlorophenyl)methyl]-8-fluoro-7-[5-[4-methyl-1-(2,2,2-trifluoroethyl)-4-piperidyl]-1,2,4-oxadiazol-3-yl]-4-oxo-2,3-dihydro-1,5-benzothiazepin-3-yl]carbamate (81.1 mg, 0.117 mmol, 1 eq) and was obtained as a light yellow solid (12.7 mg, 15% yield). MS (ESI): 716.4 [M+H]⁺.

### Step d) (3R)-3-amino-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1-oxo-2,3-dihydro-1λ⁴,5-benzothiazepin-4-one

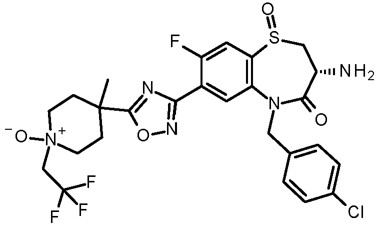

The title compound was prepared in analogy to general procedure **6d** from tert-butyl N-[(3R)-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1,4-dioxo-2,3-dihydro-1λ⁴,5-benzothiazepin-3-yl]carbamate (12 mg, 0.017 mmol, 1 eq) and was obtained as a hydrochloride salt as an light yellow solid (18.2 mg, 89% yield). MS (ESI): 616.3 [M+H]⁺.

### Example 309

### (3R)-3-amino-5-[(4-chlorophenyl)methyl]-8-fluoro-7-[5-[4-methyl-1-(2,2,2-trifluoroethyl)-4-piperidyl]-1,2,4-oxadiazol-3-yl]-1,1-dioxo-2,3-dihydro-1λ⁶,5-benzothiazepin-4-one

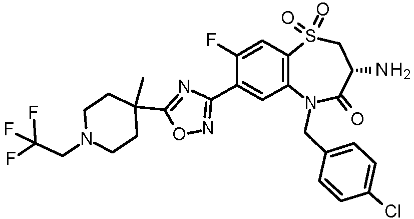

### Step a) tert-butyl N-[(3R)-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1,1,4-trioxo-2,3-dihydro-1λ⁶,5-benzothiazepin-3-yl]carbamate

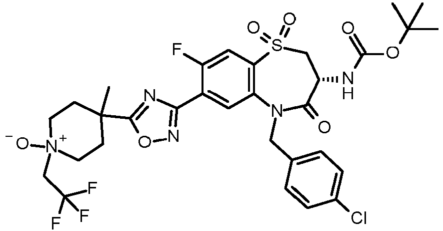

The title compound was prepared in analogy to general procedure **5** from tert-butyl N-[(3R)-5-[(4-chlorophenyl)methyl]-8-fluoro-7-[5-[4-methyl-1-(2,2,2-trifluoroethyl)-4-piperidyl]-1,2,4-oxadiazol-3-yl]-4-oxo-2,3-dihydro-1,5-benzothiazepin-3-yl]carbamate (Example 308, step b) (81.1 mg, 0.117 mmol, 1 eq) and was obtained as a light yellow solid (8.1 mg, 9.4% yield). MS (ESI): 732.3 [M+H]⁺.

### Step b) tert-butyl N-[(3R)-5-[(4-chlorophenyl)methyl]-8-fluoro-7-[5-[4-methyl-1-(2,2,2-trifluoroethyl)-4-piperidyl]-1,2,4-oxadiazol-3-yl]-1,1,4-trioxo-2,3-dihydro-1λ⁶,5-benzothiazepin-3-yl]carbamate

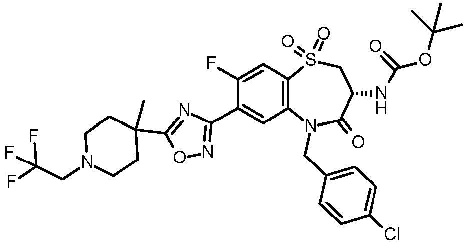

To a solution of tert-butyl N-[(3R)-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1,1,4-trioxo-2,3-dihydro-1λ⁶,5-benzothiazepin-3-yl]carbamate (8 mg, 0.011 mmol, 1 eq) in 1,2-dichloroethane (0.320 mL) was added phenylboronic acid (1.47 mg, 0.012 mmol, 1.1 eq) and the reaction mixture was heated to 85°C for 1 h. The solution was concentrated and the remaining residue was purified by preparative TLC (heptane/EtOAc 1:1) affording the title compound (7.7 mg, 98%) as white solid. MS (ESI): 716.4 [M+H]⁺.

### Step c) (3R)-3-amino-5-[(4-chlorophenyl)methyl]-8-fluoro-7-[5-[4-methyl-1-(2,2,2-trifluoroethyl)-4-piperidyl]-1,2,4-oxadiazol-3yl]-1,1-dioxo-2,3-dihydro-1λ⁶,5-benzothiazepin-4-one

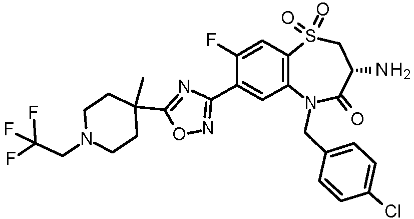

The title compound was prepared in analogy to general procedure **6d** from tert-butyl N-[(3R)-5-[(4-chlorophenyl)methyl]-8-fluoro-7-[5-[4-methyl-1-(2,2,2-trifluoroethyl)-4-piperidyl]-1,2,4-oxadiazol-3-yl]-1,1,4-trioxo-2,3-dihydro-1λ⁶,5-benzothiazepin-3-yl]carbamate (7.7 mg, 0.011 mmol, 1 eq) and was obtained as a hydrochloride salt as a white solid (5.7 mg, 79% yield). MS (ESI): 616.3 [M+H]⁺.

### Example 310

### (3R)-3-amino-5-[(4-chlorophenyl)methyl]-8-fluoro-7-[5-(4-methyl-3-pyridyl)-1,2,4-oxadiazol-3-yl]-1,1-dioxo-2,3-dihydro-1λ⁶,5-benzothiazepin-4-one

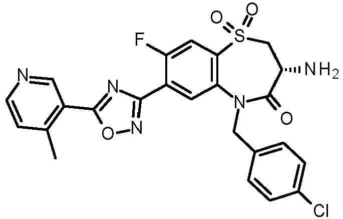

### Step a) tert-butyl N-[(3R)-5-[(4-chlorophenyl)methyl]-8-fluoro-7-[(Z)-N'-hydroxycarbamimidoyl]-1,1,4-trioxo-2,3-dihydro-1λ⁶,5-benzothiazepin-3-yl]carbamate

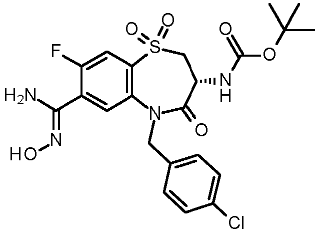

The title compound was prepared in analogy to general procedure **12** from tert-butyl N-[(3R)-5-[(4-chlorophenyl)methyl]-7-cyano-8-fluoro-1,1,4-trioxo-2,3-dihydro-1λ⁶,5-benzothiazepin-3-yl]carbamate (Example 8 step a) (2.05 g, 3.24 mmol, 1 eq) and was obtained as a yellow foam (1.47 g, 76% yield). MS (ESI): 471.2 [M-isobutene+H]⁺.

### Step b) [(Z)-[amino-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]methylene]amino] 4-methylpyridine-3-carboxylate

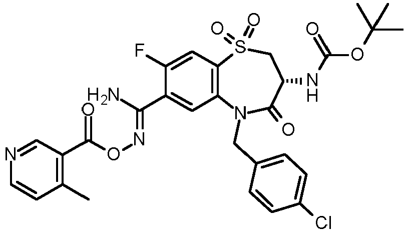

The title compound was prepared in analogy to general procedure **10a** from tert-butyl N-[(3R)-5-[(4-chlorophenyl)methyl]-8-fluoro-7-[(Z)-N'-hydroxycarbamimidoyl]-1,1,4-trioxo-2,3-dihydro-1λ⁶,5-benzothiazepin-3-yl]carbamate (50 mg, 0.083 mmol, 1 eq) and 4-methylpyridine-3-carboxylic acid (CAS 3222-50-2) and was obtained as a yellow oil (1.47 g, 76% yield). MS (ESI): 646.15 [M+H]⁺.

### Step c) tert-butyl N-[(3R)-5-[(4-chlorophenyl)methyl]-8-fluoro-7-[5-(4-methyl-3-pyridyl)-1,2,4-oxadiazol-3-yl]-1,1,4-trioxo-2,3-dihydro-1λ⁶,5-benzothiazepin-3-yl]carbamate

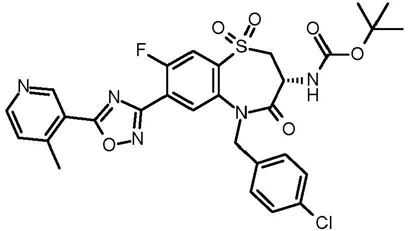

To a solution of [(Z)-[amino-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]methylene]amino] 4-methylpyridine-3-carboxylate (80.6 mg, 0.110 mmol, 1 eq) in THF (1.82 mL) was added TBAF (1M in THF, 164.7 µL, 0.165 mmol, 1.5 eq) at room temperature and the mixture was stirred for 2 h at room temperature. Then water and EtOAc were added and the layers were separated. The aqueous layer was extracted twice with EtOAc and the combined organic layers were washed with brine, dried over MgSO₄ and concentrated. The remaining residue was purified by column chromatography on silica gel (EtOAc in heptane 0 to 70%) to afford the title compound (31.3 mg, 45%) as white solid. MS (ESI): 628.3 [M+H]⁺.

### Step d) (3R)-3-amino-5-[(4-chlorophenyl)methyl]-8-fluoro-7-[5-(4-methyl-3-pyridyl)-1,2,4-oxadiazol-3-yl]-1,1-dioxo-2,3-dihydro-1λ⁶,5-benzothiazepin-4-one

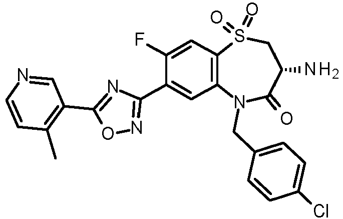

The title compound was prepared in analogy to general procedure **6d** from tert-butyl N-[(3R)-5-[(4-chlorophenyl)methyl]-8-fluoro-7-[5-(4-methyl-3-pyridyl)-1,2,4-oxadiazol-3-yl]-1,1,4-trioxo-2,3-dihydro-1λ⁶,5-benzothiazepin-3-yl]carbamate (31.3 mg, 0.050 mmol, 1 eq) and was obtained as a hydrochloride salt as a white solid (21.3 mg, 74% yield). MS (ESI): 528.1 [M+H]⁺.

The examples 311 to 314 of the following table were prepared in analogy to Example 310, using the appropriate carboxylic acid building block.

| **Ex.** | **Structure** | **Systematic Name** | **Building Block** | **MS, ESI: m/z** |
|---|---|---|---|---|
| 311 | 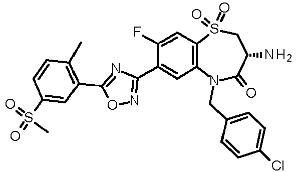 | (3R)-3-amino-5-[(4-chlorophenyl)methyl]-8-fluoro-7-[5-(2-methyl-5-methylsulfonyl-phenyl)-1,2,4-oxadiazol-3-yl]-1,1-dioxo-2,3-dihydro-1λ⁶,5-benzothiazepin-4-one | 2-Methyl-5-methylsulf onylbenzoic acid (CAS15110 4-37-9) | 605.1 [M+H]⁺ |
| | | (**) | | |
| 312 | 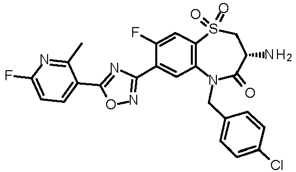 | (3R)-3-amino-5-[(4-chlorophenyl)methyl]-8-fluoro-7-[5-(6-fluoro-2-methyl-3-pyridyl)-1,2,4-oxadiazol-3-yl]-1,1-dioxo-2,3-dihydro-1λ⁶,5-benzothiazepin-4-one | 6-Fluoro-2-methylpyridine-3-carboxylic acid | 546.2 [M+H]⁺ |
| | | | (CAS 884494-97-7) | |
| 313 | 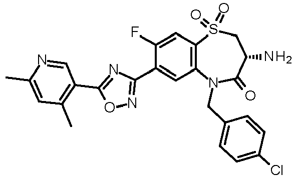 | (3R)-3-amino-5-[(4-chlorophenyl)methyl]-7-[5-(4,6-dimethyl-3-pyridyl)-1,2,4-oxadiazol-3-yl]-8-fluoro-1,1-dioxo-2,3-dihydro-1λ⁶,5-benzothiazepin-4-one | 4,6-Dimethylp yridine-3-carboxylic acid | 540.1 [M-H]⁻ |
| | | | (CAS 22047-86-5) | |
| | | (*) | | |
| 314 | 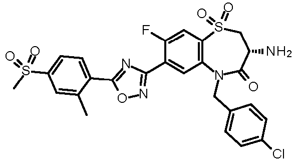 | (3R)-3-amino-5-[(4-chlorophenyl)methyl]-8-fluoro-7-[5-(2-methyl-4-methylsulfonyl-phenyl)-1,2,4-oxadiazol-3-yl]-1,1-dioxo-2,3-dihydro-1λ⁶,5-benzothiazepin-4-one | 2-Methyl-4-methylsulf onylbenzoic acid (CAS 118939-09-6) | 605.3 [M+H]⁺ |
| | | (*) | | |

| | | | | |
|---|---|---|---|---|
| * as a hydrochloride salt ** as a hydrochloride salt and 1,1,1,3,3,3-hexafluoropropan-2-ol adduct | | | | |

### Example 315

### ethyl 1-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3-azabicyclo[3.1.1]heptane-3-carboxylate

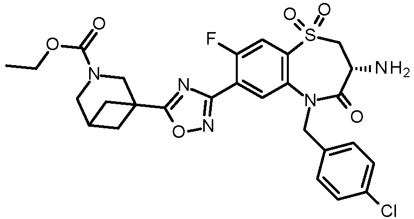

### Step a) tert-butyl 1-[3-[(3R)-3-(tert-butoxycarbonylamino)-5-[(4-chlorophenyl)methyl]-8-fluoro-4-oxo-2,3-dihydro-1,5-benzothiazepin-7-yl]-1,2,4-oxadiazol-5-yl]-3-azabicyclo[3.1.1]heptane-3-carboxylate

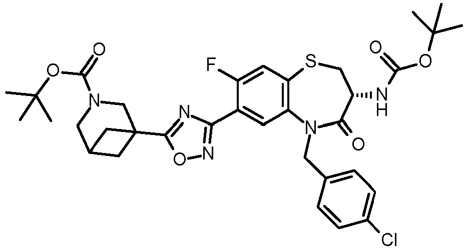

The title compound was prepared in analogy to general procedure **9a** from tert-butyl N-[(3R)-5-[(4-chlorophenyl)methyl]-8-fluoro-7-[(Z)-N'-hydroxycarbamimidoyl]-4-oxo-2,3-dihydro-1,5-benzothiazepin-3-yl]carbamate (Example 289, step a)) (150 mg, 0.3 mmol, 1 eq) and 3-tert-butoxycarbonyl-3-azabicyclo[3.1.1]heptane-1-carboxylic acid (CAS 1000931-22-5) and was obtained as a white solid (120 mg, 57% yield). MS (ESI): 722.3 [M+Na]⁺.

### Step b) tert-butyl 1-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3-azabicyclo[3.1.1]heptane-3-carboxylate

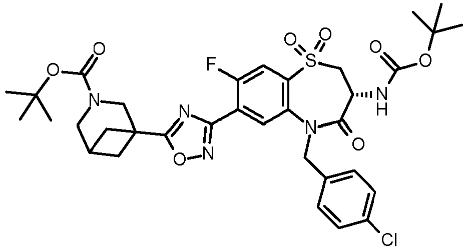

The title compound was prepared in analogy to general procedure **5** from tert-butyl 1-[3-[(3R)-3-(tert-butoxycarbonylamino)-5-[(4-chlorophenyl)methyl]-8-fluoro-4-oxo-2,3-dihydro-1,5-benzothiazepin-7-yl]-1,2,4-oxadiazol-5-yl]-3-azabicyclo[3.1.1]heptane-3-carboxylate (120 mg, 0.170 mmol, 1 eq) and was obtained as a white solid (125 mg, 98% yield). MS (ESI): 754.3 [M+Na]⁺.

### Step c) (3R)-3-amino-7-[5-(3-azabicyclo[3.1.1]heptan-1-yl)-1,2,4-oxadiazol-3-yl]-5-[(4-chlorophenyl)methyl]-8-fluoro-1,1-dioxo-2,3-dihydro-1λ⁶,5-benzothiazepin-4-one

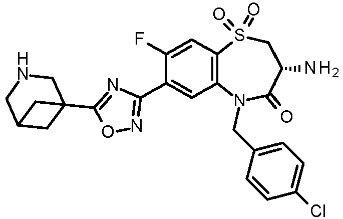

The title compound was prepared in analogy to general procedure **6b** from tert-butyl 1-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3-azabicyclo[3.1.1]heptane-3-carboxylate (125 mg, 0.170 mmol, 1 eq) and was obtained as a dihydrochloride salt as a white solid (100 mg, 97% yield). MS (ESI): 532.2 [M+H]⁺.

### Step d) ethyl 1-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3-azabicyclo[3.1.1]heptane-3-carboxylate

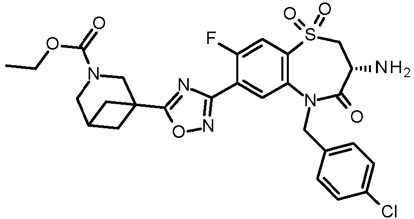

To a solution of (3R)-3-amino-7-[5-(3-azabicyclo[3.1.1]heptan-1-yl)-1,2,4-oxadiazol-3-yl]-5-[(4-chlorophenyl)methyl]-8-fluoro-1,1-dioxo-2,3-dihydro-1λ⁶,5-benzothiazepin-4-one;dihydrochloride (70.0 mg, 0.120 mmol, 1 eq) in DCM (5 mL) were added DIPEA (49.0 mg, 0.380 mmol, 3.28 eq) and ethyl chloroformate (12.0 mg, 0.110 mmol, 0.960 eq) and the mixture was stirred at 20°C for 1 h. The mixture was concentrated in vacuo and the remaining residue was purified by prep. HPLC affording the title compound (26.2 mg, 0.040 mmol, 37% yield) as white solid. MS (ESI): 604.2 [M+H]⁺.

The examples 316 and 317 of the following table were prepared in analogy to Example 315, using the appropriate chloroformate building block.

| **Ex.** | **Structure** | **Systematic Name** | **Building Block** | **MS, ESI: m/z** |
|---|---|---|---|---|
| 316 | 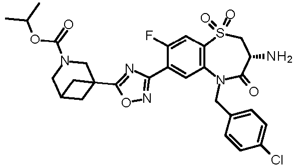 | isopropyl 1-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3-azabicyclo[3.1.1]heptane-3-carboxylate | Isopropyl carbonochl oridate | 618.1 [M+H]⁺ |
| | | | (CAS 108-23-6) | |
| 317 | 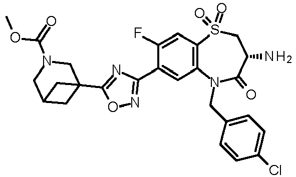 | methyl 1-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3-azabicyclo[3.1.1]heptane-3-carboxylate | Methyl carbonochl oridate | 590.2 [M+H]⁺ |
| | | | (CAS 79-22-1) | |

### Example 318

### (3R)-3-amino-5-[(4-chlorophenyl)methyl]-8-fluoro-1,1-dioxo-7-[5-[1-(trifluoromethyl)cyclopropyl]-1,2,4-oxadiazol-3-yl]-2,3-dihydro-1λ⁶,5-benzothiazepin-4-one

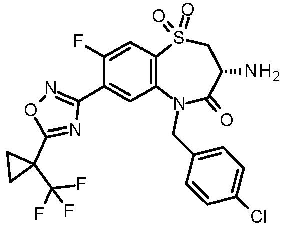

### Step a) [(Z)-[amino-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]methylene]amino] 1-(trifluoromethyl)cyclopropanecarboxylate

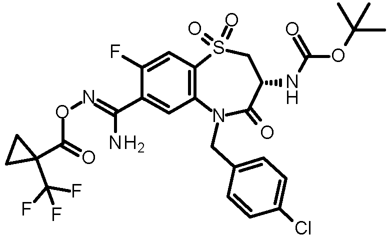

The title compound was prepared in analogy to general procedure **10a** from tert-butyl N-[(3R)-5-[(4-chlorophenyl)methyl]-8-fluoro-7-[(Z)-N'-hydroxycarbamimidoyl]-1,1,4-trioxo-2,3-dihydro-1λ⁶,5-benzothiazepin-3-yl]carbamate (Example 310, step a) (80 mg, 0.152 mmol, 1 eq) and 1-(trifluoromethyl)cyclopropanecarboxylic acid (CAS 277756-46-4) and was obtained as a yellow oil (120 mg, 115.6% yield). MS (ESI): 607.0 [M-isobutene+H]⁺.

### Step b) tert-butyl N-[(3R)-5-[(4-chlorophenyl)methyl]-8-fluoro-1,1,4-trioxo-7-[5-[]-(trifluoromethyl)cyclopropyl]-1,2,4-oxadiazol-3-yl]-2,3-dihydro-1λ⁶,5-benzothiazepin-3-yl]carbamate

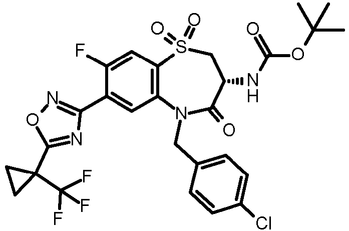

The title compound was prepared in analogy to general procedure **11a** [(Z)-[amino-[(3R)-3-(tertbutoxycarbonylamino)-5-[(4-chlorophenyl)methyl]-8-fluoro-1,1,4-trioxo-2,3-dihydro-1λ⁶,5-benzothiazepin-7-yl]methylene]amino] 1-(trifluoromethyl)cyclopropanecarboxylate (100 mg, 0.150 mmol, 1 eq) and was obtained as a yellow oil (80 mg, 64% yield). MS (ESI): 589.0 [M-isobutene+H]⁺.

### Step c) (3R)-3-amino-5-[(4-chlorophenyl)methyl]-8-fluoro-1,1-dioxo-7-[5-[1-(trifluoromethyl)cyclopropyl]-1,2,4-oxadiazol-3-yl]-2,3-dihydro-1λ⁶,5-benzothiazepin-4-one

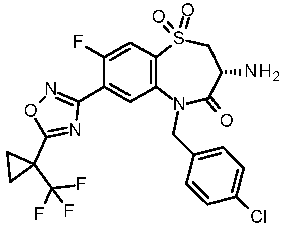

The title compound was prepared in analogy to general procedure 6b from tert-butyl N-[(3R)-5-[(4-chlorophenyl)methyl]-8-fluoro-1,1,4-trioxo-7-[5-[1-(trifluoromethyl)cyclopropyl]-1,2,4-oxadiazol-3-yl]-2,3-dihydro-1λ⁶,5-benzothiazepin-3-yl]carbamate (80 mg, 0.120 mmol, 1 eq) and was obtained as a hydrochloride salt as a white solid (42.5 mg, 59% yield). MS (ESI): 545.0 [M+H]⁺.

The examples 319 and 320 of the following table were prepared in analogy to Example 318, using the appropriate carboxylic acid building block.

| **Ex.** | **Structure** | **Systematic Name** | **Building Block** | **MS, ESI: m/z** |
|---|---|---|---|---|
| 319 | 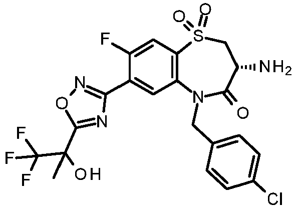 | (3R)-3-amino-5-[(4-chlorophenyl)methyl]-8-fluoro-1,1-dioxo-7-[5-(2,2,2-trifluoro-1-hydroxy-1-methylethyl)-1,2,4-oxadiazol-3-yl]-2,3-dihydro-1λ⁶,5-benzothiazepin-4-one | 3,3,3-Trifluoro-2-hydroxy-2-methylpropanoic acid | 549.2 [M+H]⁺ |
| | | | (CAS 374-35-6) | |
| 320 | 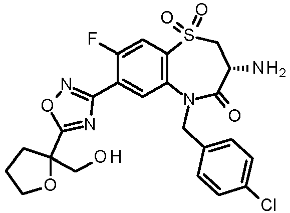 | (3R)-3-amino-5-[(4-chlorophenyl)methyl]-8-fluoro-7-[5-[2-(hydroxymethyl)tetrahydrofur an-2-yl]-1,2,4-oxadiazol-3-yl]-1,1-dioxo-2,3-dihydro-1λ⁶,5-benzothiazepin-4-one | 2-(Hydroxym ethyl)tetra hydrofuran -2-carboxylic acid | 537.3 [M+H]⁺ |
| | | | (CAS 442877-01-2) | |

### Example 321

### 2-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-2-methyl-propanenitrile

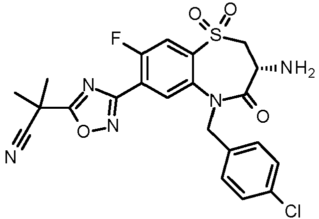

### Step a) (2R)-2-(tert-butoxycarbonylamino)-3-(4-cyano-5-fluoro-2-nitro-phenyl)sulfanyl-propanoic acid

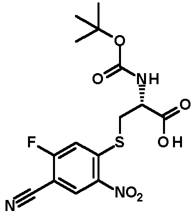

To a solution of 2,4-difluoro-5-nitro-benzonitrile (9.4 g, 50 mmol) and (tert-butoxycarbonyl)-L-cysteine (11.07 g, 50 mmol) in DCM (157 mL) was added DIPEA (17.48 mL, 100 mmol, Eq: 2).

The reaction mixture was stirred for 24 hours at 22°C, diluted with DCM (40 mL) and washed once with 1N aqueous HCl solution and extracted twice with DCM. The combined organic layers were washed with brine solution, dried over sodium sulfate, filtrated and concentrated in vacuo to yield the title compound as a yellow solid (23.5 g, 118 % yield). MS (ESI): 286.1 [M-CO₂-isobutene+H]⁺.

### Step b) (2R)-3-(2-amino-4-cyano-5-fluoro-phenyl)sulfanyl-2-(tert-butoxycarbonylamino)propanoic acid

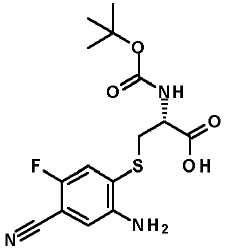

The title compound was prepared in analogy to general procedure **2** from (2R)-2-(tert-butoxycarbonylamino)-3-(4-cyano-5-fluoro-2-nitro-phenyl)sulfanyl-propanoic acid (23 g, 57.9 mmol) and was obtained as a black solid (23 g, 48.1 mmol, 80% yield). MS (ESI): 300.1 [M-isobutene+H]⁺.

### Step c) tert-butyl N-[(3R)-7-cyano-8-fluoro-4-oxo-3,5-dihydro-2H-1, 5-benzothiazepin-3-yl]carbamate

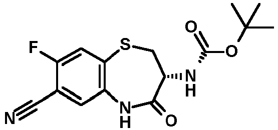

The title compound was prepared in analogy to general procedure **3** from (2R)-3-(2-amino-4-cyano-5-fluoro-phenyl)sulfanyl-2-(tert-butoxycarbonylamino)propanoic acid (23 g, 48.1 mmol) and was obtained as a light yellow solid (8.4 g, 24.9 mmol, 48% yield). MS (ESI): 282.1 [M-isobutene+H]⁺.

### Step d) tert-butyl N-[(3R)-8-fluoro-7-[(Z)-N'-hydroxycarbamimidoyl]-4-oxo-3, 5-dihydro-2H-1,5-benzothiazepin-3-yl]carbamate

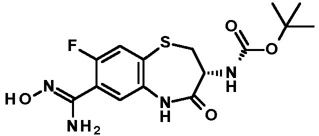

To a solution of tert-butyl N-[(3R)-7-cyano-8-fluoro-4-oxo-3,5-dihydro-2H-1,5-benzothiazepin-3-yl]carbamate(2R)-3-(2-amino-4-cyano-5-fluoro-phenyl)sulfanyl-2-(tertbutoxycarbonylamino)propanoic acid (200 mg, 0.59 mmol) in MeOH (2 mL) were added hydroxylamine hydrochloride (63.7 mg, 0.89 mmol, Eq: 1.5) and sodium bicarbonate (249 mg, 2.96 mmol, Eq: 5). The mixture was stirred for 16 hours at 70°C, cooled to 22°C, filtered and the filter cake was washed with DCM. The combined filtrates were concentrated in vacuo. The reaction was diluted with DCM and washed with water and brine. The organic layer was then dried over Na2SO4, filtered and concentrated in vacuo to afford the title compound (444 mg, 1.19 mmol, 74% yield) as yellow solid. MS (ESI): 315.1 [M-isobutene+H]⁺.

### Step e) [(Z)-[amino-[(3R)-3-(tert-butoxycarbonylamino)-8-fluoro-4-oxo-3,5-dihydro-2H-1,5-benzothiazepin-7-yl]methylene]amino] 2-cyano-2-methyl-propanoate

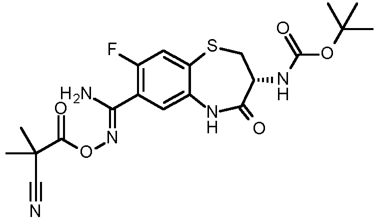

The title compound was prepared in analogy to general procedure **10c** from tert-butyl N-[(3R)-8-fluoro-7-[(Z)-N'-hydroxycarbamimidoyl]-4-oxo-3,5-dihydro-2H-1,5-benzothiazepin-3-yl]carbamate (Example 321, step d)) (100 mg, 0.270 mmol, 1 eq) and 2-cyano-2-methyl-propanoic acid (CAS 22426-30-8) and was obtained as a white solid (13.2 mg, 11% yield). MS (ESI): 410.1 [M-isobutene+H]⁺.

### Step f) tert-butyl N-[(3R)-7-[5-(1-cyano-1-methyl-ethyl)-1,2,4-oxadiazol-3-yl]-8-fluoro-4-oxo-3,5-dihydro-2H-1,5-benzothiazepin-3-yl]carbamate

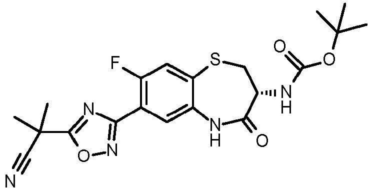

The title compound was prepared in analogy to general procedure **11b** from [(Z)-[amino-[(3R)-3-(tert-butoxycarbonylamino)-8-fluoro-4-oxo-3,5-dihydro-2H-1,5-benzothiazepin-7-yl]methylene]amino] 2-cyano-2-methyl-propanoate (13 mg, 0.028 mmol, 1 eq) and was obtained as a light yellow solid (9.4 mg, 72% yield). MS (ESI): 392.1 [M-isobutene+H]⁺.

### Step g) tert-butyl N-[(3R)-5-[(4-chlorophenyl)methyl]-7-[5-(1-cyano-1-methyl-ethyl)-1,2,4-oxadiazol-3-yl]-8-fluoro-4-oxo-2,3-dihydro-1,5-benzothiazepin-3-yl]carbamate

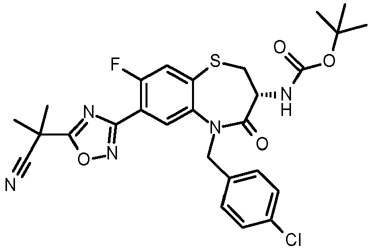

The title compound was prepared in analogy to general procedure **4** from tert-butyl N-[(3R)-7-[5-(1-cyano-1-methyl-ethyl)-1,2,4-oxadiazol-3-yl]-8-fluoro-4-oxo-3,5-dihydro-2H-1,5-benzothiazepin-3-yl]carbamate (9.4 mg, 0.02 mmol, 1 eq) and was obtained as a white solid (7 mg, 55% yield). MS (ESI): 516.1 [M-isobutene+H]⁺.

### Step h) tert-butyl N-[(3R)-5-[(4-chlorophenyl)methyl]-7-[5-(1-cyano-1-methyl-ethyl)-1,2,4-oxadiazol-3-yl]-8-fluoro-1,1,4-trioxo-2,3-dihydro-1λ⁶,5-benzothiazepin-3-yl]carbamate

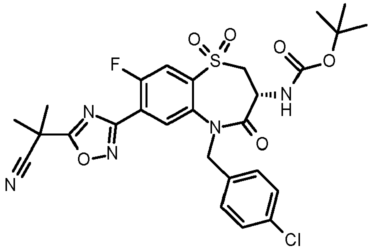

The title compound was prepared in analogy to general procedure **5** from tert-butyl N-[(3R)-5-[(4-chlorophenyl)methyl]-7-[5-(1-cyano-1-methyl-ethyl)-1,2,4-oxadiazol-3-yl]-8-fluoro-4-oxo-2,3-dihydro-1,5-benzothiazepin-3-yl]carbamate (7 mg, 0.011 mmol, 1 eq) and was obtained as a white solid (5 mg, 64% yield). MS (ESI): 548.1 [M-isobutene+H]⁺.

### Step i) 2-[3-[(3R)-3-amino-5-[(4-chlorophenyl)methyl]-8-fluoro-1,1,4-trioxo-2,3-dihydro-1λ⁶,5-benzothiazepin-7-yl]-1,2,4-oxadiazol-5-yl]-2-methyl-propanenitrile

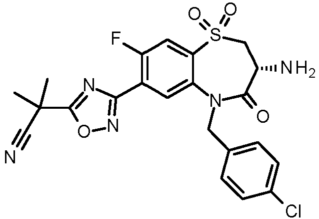

The title compound was prepared in analogy to general procedure **6d** from tert-butyl N-[(3R)-5-[(4-chlorophenyl)methyl]-7-[5-(1-cyano-1-methyl-ethyl)-1,2,4-oxadiazol-3-yl]-8-fluoro-1,1,4-trioxo-2,3-dihydro-1λ⁶,5-benzothiazepin-3-yl]carbamate (5 mg, 0.007 mmol, 1 eq) and was obtained as a hydrochloride salt as a white solid (5 mg, 118% yield). MS (ESI): 504.2 [M+H]⁺.

### Example 322

### (3R)-3-amino-5-[(4-chlorophenyl)methyl]-8-fluoro-7-[5-(5-methyl-1,3,4-oxadiazol-2-yl)-1,2,4-oxadiazol-3-yl]-1,1-dioxo-2,3-dihydro-1λ⁶,5-benzothiazepin-4-one

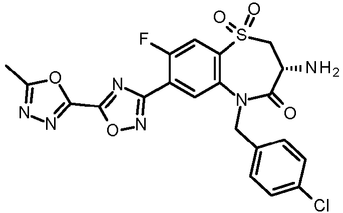

### Step a) [(Z)-[amino-[(3R)-3-(tert-butoxycarbonylamino)-5-[(4-chlorophenyl)methyl]-8-fluoro-4-oxo-2,3-dihydro-1,5-benzothiazepin-7-yl]methylene]amino] 5-methyl-1,3,4-oxadiazole-2-carboxylate

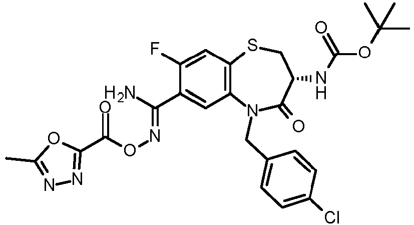

To a solution of tert-butyl N-[(3R)-5-[(4-chlorophenyl)methyl]-8-fluoro-7-[(Z)-N'-hydroxycarbamimidoyl]-4-oxo-2,3-dihydro-1,5-benzothiazepin-3-yl]carbamate (Example 289, step a)) (223 mg, 0.266 mmol, 1 eq) in 1,2-dichloroethane (4 mL) was added 5-methyl-1,3,4-oxadiazole-2-carbonyl chloride (CAS 889131-28-6) (77.9 mg, 0.53 mmol, 2 eq) as a solution in1,2-dichloroethane (4 mL) at 0-5°C. The ice bath was removed and the reaction mixture was stirred for 3h. The reaction mixture was partitioned between DCM and sat. NaHCO₃ solution and the layers were separated. The aqueous layer was extracted with EtOAc and the combined organic layers were washed with brine, dried over anhydrous sodium sulfate and concentrated in vacuo. The remaining residue was purified by column chromatography on silica gel (n-heptane/EtOAc 100:0 to 20:80) affording the title compound (65.4 mg, 40% yield) as light yellow solid. MS (ESI): 603.4 [M-H]⁻.

### Step b) tert-butyl N-[(3R)-5-[(4-chlorophenyl)methyl]-8-fluoro-7-[5-(5-methyl-1, 3, 4-oxadiazol-2-yl)-1,2,4-oxadiazol-3-yl]-4-oxo-2,3-dihydro-1,5-benzothiazepin-3-yl]carbamate

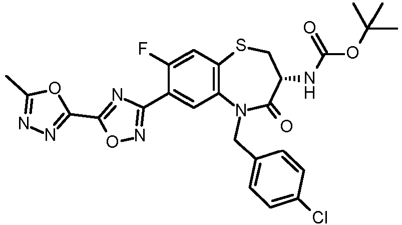

The title compound was prepared in analogy to general procedure **11a** from [(Z)-[amino-[(3R)-3-(tert-butoxycarbonylamino)-5-[(4-chlorophenyl)methyl]-8-fluoro-4-oxo-2,3-dihydro-1,5-benzothiazepin-7-yl]methylene]amino] 5-methyl-1,3,4-oxadiazole-2-carboxylate (65.4 mg, 0.107 mmol, 1 eq) and was obtained as a white solid (43.3 mg, 38% yield). MS (ESI): 531.2 [M-isobutene+H]⁺.

### Step c) tert-butyl N-[(3R)-5-[(4-chlorophenyl)methyl]-8-fluoro-7-[5-(5-methyl-1, 3, 4-oxadiazol-2-yl)-1,2,4-oxadiazol-3-yl]-1,1,4-trioxo-2,3-dihydro-1λ⁶,5-benzothiazepin-3-yl]carbamate

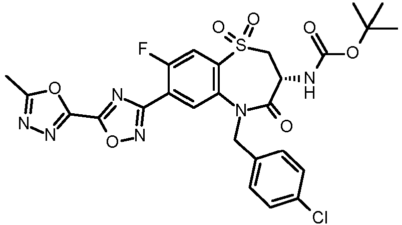

The title compound was prepared in analogy to general procedure **5** from tert-butyl N-[(3R)-5-[(4-chlorophenyl)methyl]-8-fluoro-7-[5-(5-methyl-1,3,4-oxadiazol-2-yl)-1,2,4-oxadiazol-3-yl]-4-oxo-2,3-dihydro-1,5-benzothiazepin-3-yl]carbamate (43.3 mg, 0.074 mmol, 1 eq) and was obtained as a white solid (16.9 mg, 37% yield). MS (ESI): 617.3 [M-H]⁻.

### Step d) (3R)-3-amino-5-[(4-chlorophenyl)methyl]-8-fluoro-7-[5-(5-methyl-1,3,4-oxadiazol-2-yl)-1,2,4-oxadiazol-3-yl]-1,1-dioxo-2,3-dihydro-1λ⁶,5-benzothiazepin-4-one

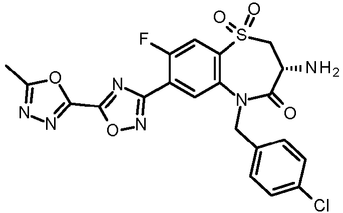

The title compound was prepared in analogy to general procedure 6d from tert-butyl N-[(3R)-5-[(4-chlorophenyl)methyl]-8-fluoro-7-[5-(5-methyl-1,3,4-oxadiazol-2-yl)-1,2,4-oxadiazol-3-yl]-1,1,4-trioxo-2,3-dihydro-1λ⁶,5-benzothiazepin-3-yl]carbamate (16.9 mg, 0.027 mmol, 1 eq) and was obtained as hydrochloride as a white solid (12.5 mg, 78% yield). MS (ESI): 519.2 [M+H]⁺.

### Example 323

### (3R)-3-amino-5-[(4-chlorophenyl)methyl]-7-[5-(5,5-difluoro-1-methyl-3-piperidyl)-1,2,4-oxadiazol-3-yl]-8-fluoro-1,1-dioxo-2,3-dihydro-1λ⁶,5-benzothiazepin-4-one

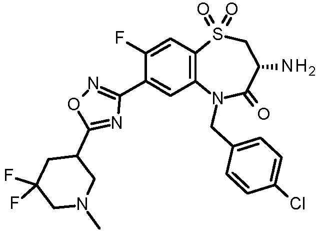

### Step a) O3-[(Z)-[amino-[(3R)-3-(tert-butoxycarbonylamino)-5-[(4-chlorophenyl)methyl]-8-fluoro-4-oxo-2,3-dihydro-1,5-benzothiazepin-7-yl]methylene]amino] O1-benzyl 5,5-difluoropiperidine-1,3-dicarboxylate

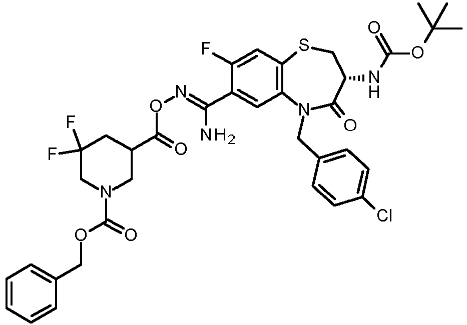

The title compound was prepared in analogy to general procedure **10c** from tert-butyl N-[(3R)-5-[(4-chlorophenyl)methyl]-8-fluoro-7-[(Z)-N'-hydroxycarbamimidoyl]-4-oxo-2,3-dihydro-1,5-benzothiazepin-3-yl]carbamate (Example 289, step a)) (80 mg, 0.158 mmol) and 1-benzyloxycarbonyl-5,5-difluoro-piperidine-3-carboxylic acid (CAS 1356338-81-2) and was obtained as a white solid (105 mg, 79% yield). MS (ESI): 776.3 [M+H]⁺.

### Step b) benzyl 5-[3-[(3R)-3-(tert-butoxycarbonylamino)-5-[(4-chlorophenyl)methyl]-8-fluoro-4-oxo-2,3-dihydro-1,5-benzothiazepin-7-yl]-1,2,4-oxadiazol-5-yl]-3,3-difluoro-piperidine-1-carboxylate

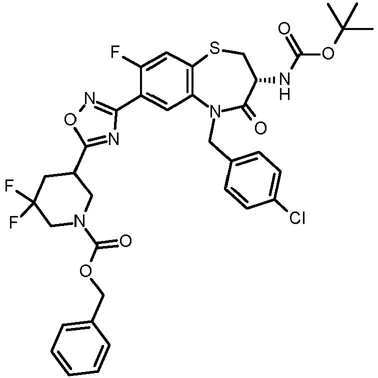

The title compound was prepared in analogy to general procedure **11b** from O3-[(Z)-[amino-[(3R)-3-(tert-butoxycarbonylamino)-5-[(4-chlorophenyl)methyl]-8-fluoro-4-oxo-2,3-dihydro-1,5-benzothiazepin-7-yl]methylene]amino] O1-benzyl 5,5-difluoropiperidine-1,3-dicarboxylate (100 mg, 0.120 mmol, 1 eq) and was obtained as a white solid (58 mg, 63% yield). MS (ESI): 658.2 [M-isobutene-CO₂+H]⁺.

### Step c) benzyl 5-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3,3-difluoro-piperidine-1-carboxylate

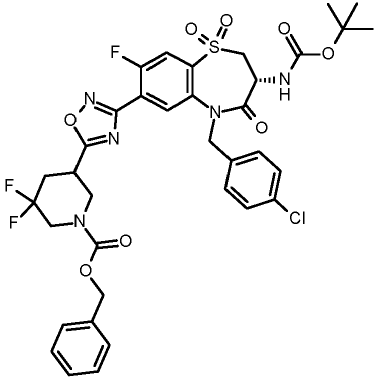

The title compound was prepared in analogy to general procedure **5** from benzyl 5-[3-[(3R)-3-(tert-butoxycarbonylamino)-5-[(4-chlorophenyl)methyl]-8-fluoro-4-oxo-2,3-dihydro-1,5-benzothiazepin-7-yl]-1,2,4-oxadiazol-5-yl]-3,3 -difluoro-piperidine-1-carboxylate (58 mg, 0.075 mmol, 1 eq) and was obtained as an off-white solid (57 mg, 83% yield). MS (ESI): 690.2 [-isobutene-CO₂+H]⁺.

### Step d) tert-butyl N-[(3R)-5-[(4-chlorophenyl)methyl]-7-[5-(5,5-difluoro-3-piperidyl)-1,2,4-oxadiazol-3-yl]-8-fluoro-1,1,4-trioxo-2,3-dihydro-1λ⁶,5-benzothiazepin-3-yl]carbamate

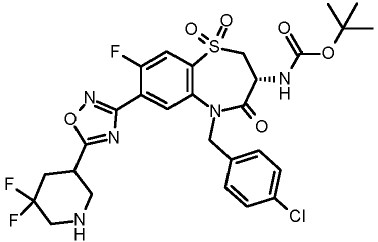

To a solution of benzyl 5-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-3,3-difluoro-piperidine-1-carboxylate (57 mg, 0.072 mmol, 1 eq) in MeOH (0.721 mL) was added Pd/C (16 mg). The suspension was carefully degassed and then recharged with argon (3 times). The same procedure was repeated but with recharging with hydrogen and the reaction was stirred for 4 h under an atmosphere of hydrogen. The reaction mixture was filtered through a plug of celite and the celite plug was washed with MeOH. The filtrate was concentrated and the remaining residue was purified by column chromatography on silica gel (0 to 100% EtOAc in heptane) affording the title compound (21 mg, 37% yield) as white solid. MS (ESI): 656.2 [M+H]⁺.

### Step e) tert-butylN-[(3R)-5-[(4-chlorophenyl)methyl]-7-[5-(5, 5-difluoro-1-methyl-3-piperidyl)-1,2,4-oxadiazol-3-yl]-8-fluoro-1,1,4-trioxo-2,3-dihydro-1λ⁶,5-benzothiazepin-3-yl]carbamate

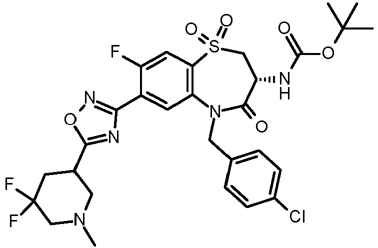

To a solution of tert-butyl N-[(3R)-5-[(4-chlorophenyl)methyl]-7-[5-(5,5-difluoro-3-piperidyl)-1,2,4-oxadiazol-3-yl]-8-fluoro-1,1,4-trioxo-2,3-dihydro-1λ⁶,5-benzothiazepin-3-yl]carbamate (21 mg, 0.027 mmol, 1 eq) in MeOH (0.672 mL) were added formaldehyde aqueous solution (37%) (21.82 mg, 26.78 µL, 0.269 mmol, 10 eq) and sodium triacetoxyborohydride (56.9 mg, 0.269 mmol, 10 eq) and the mixture was stirred at room temperature for 1 h. The reaction mixture was poured into water and the mixture was extracted with DCM (3 times). The combined organic extracts were dried over Na₂SO₄, filtered and concentrated in vacuo and the remaining residue was purified by column chromatography on silica gel (EtOAc in heptane) to afford the title compound (14 mg, 72% yield) as white solid. MS (ESI): 670.2 [M+H]⁺.

### Step f) (3R)-3-amino-5-[(4-chlorophenyl)methyl]-7-[5-(5,5-difluoro-1-methyl-3-piperidyl)-1,2,4-oxadiazol-3-yl]-8-fluoro-1,1-dioxo-2,3-dihydro-1λ⁶,5-benzothiazepin-4-one

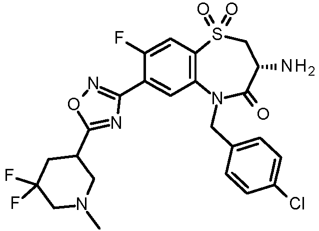

The title compound was prepared in analogy to general procedure **6d** from tert-butyl N-[(3R)-5-[(4-chlorophenyl)methyl]-7-[5-(5,5-difluoro-1-methyl-3-piperidyl)-1,2,4-oxadiazol-3-yl]-8-fluoro-1,1,4-trioxo-2,3-dihydro-1λ⁶,5-benzothiazepin-3-yl]carbamate (14 mg, 0.019 mmol, 1 eq) and was obtained as a white solid (6 mg, 54% yield). MS (ESI): 570.2 [M+H]⁺.

The example 324 of the following table were prepared in analogy to Example 323, using the appropriate carboxylic acid building block.

| **Ex.** | **Structure** | **Systematic Name** | **Building Block** | **MS, ESI: m/z** |
|---|---|---|---|---|
| 324 | 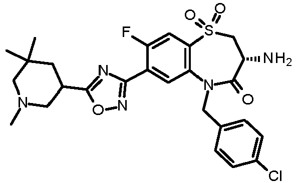 | (3R)-3-amino-5-[(4-chlorophenyl)methyl]-8-fluoro-1,1-dioxo-7-[5-(1,5,5-trimethyl-3-piperidyl)-1,2,4-oxadiazol-3-yl]-2,3-dihydro-1λ⁶,5-benzothiazepin-4-one | 1-Benzyloxyc arbonyl-5,5-dimethylpiperidine-3-carboxylic acid | 562.2 [M+H]⁺ |
| | | (*) | (CAS 2580202-96-4) | |

| | | | | |
|---|---|---|---|---|
| * as hydrochloride salt | | | | |

### Example 325

### (3R)-3-amino-5-[(4-chlorophenyl)methyl]-7-[5-[(dimethylamino)methyl]-1,2,4-oxadiazol-3-yl]-8-fluoro-1,1-dioxo-2,3-dihydro-1λ⁶,5-benzothiazepin-4-one

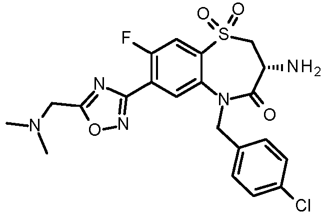

### Step a) tert-butyl N-[(3R)-5-[(4-chlorophenyl)methyl]-7-[5-[(dimethylamino)methyl]-1,2,4-oxadiazol-3-yl]-8-fluoro-4-oxo-2,3-dihydro-1,5-benzothiazepin-3-yl]carbamate

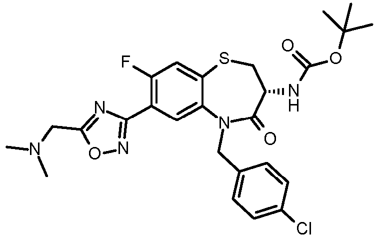

The title compound was prepared in analogy to general procedure **9c** from tert-butyl N-[(3R)-5-[(4-chlorophenyl)methyl]-8-fluoro-7-[(Z)-N'-hydroxycarbamimidoyl]-4-oxo-2,3-dihydro-1,5-benzothiazepin-3-yl]carbamate (Example 289, step a)) (159 mg, 0.321 mmol, 1 eq) and 2-(dimethylamino)acetic acid (CAS 1118-68-9) and was obtained as a light yellow oil (140 mg, 78% yield). MS (ESI): 562.1 [M+H]⁺.

### Sep b) 1-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-N,N-dimethyl-methanamine oxide

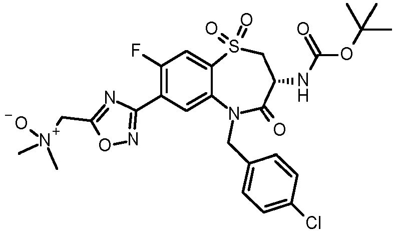

The title compound was prepared in analogy to general procedure **5** from tert-butyl N-[(3R)-5-[(4-chlorophenyl)methyl]-7-[5-[(dimethylamino)methyl]-1,2,4-oxadiazol-3-yl]-8-fluoro-4-oxo-2,3-dihydro-1,5-benzothiazepin-3-yl]carbamate (120 mg, 0.214 mmol, 1 eq). The compound was obtained as a crude product (light yellow solid, 268 mg) and was used as such in the next reaction step. MS (ESI): 610.4 [M+H]⁺.

### Step c) tert-butyl N-[(3R)-5-[(4-chlorophenyl)methyl]-7-[5-[(dimethylamino)methyl]-1,2,4-oxadiazol-3-yl]-8-fluoro-1,1,4-trioxo-2,3-dihydro-1λ⁶,5-benzothiazepin-3-yl]carbamate

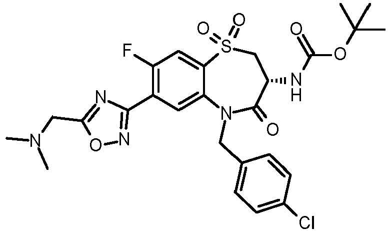

To the solution of 1-[3-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,2,4-oxadiazol-5-yl]-N,N-dimethyl-methanamine oxide (300 mg, 0.49 mmol, 1 eq) in 1,2-dichloroethane (3 mL) was added phenylboronic acid (149.9 mg, 1.23 mmol, 2.5 eq) at 25°C and the mixture was heated to 80°C for 0.5 h. The reaction mixture was concentrated under vacuum and the remaining residue was purified by column chromatography on silica gel (40% to 80% EtOAc in PE) to afford the title compound (34 mg, 0.06 mmol, 12% yield) as a white solid. MS (ESI): 594.4 [M+H]⁺.

### Step d) (3R)-3-amino-5-[(4-chlorophenyl)methyl]-7-[5-[(dimethylamino)methyl]-1,2,4-oxadiazol-3-yl]-8-fluoro-1,1-dioxo-2,3-dihydro-1λ⁶,5-benzothiazepin-4-one

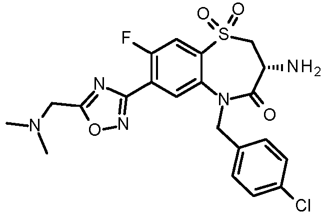

The title compound was prepared in analogy to general procedure **6b** from tert-butyl N-[(3R)-5-[(4-chlorophenyl)methyl]-7-[5-[(dimethylamino)methyl]-1,2,4-oxadiazol-3-yl]-8-fluoro-1,1,4-trioxo-2,3-dihydro-1λ⁶,5-benzothiazepin-3-yl]carbamate (34 mg, 0.057 mmol, 1 eq) and was obtained as a white solid (11.3 mg, 40% yield). MS (ESI): 494.0 [M+H]⁺.

### Example 326

### (3R)-3-amino-5-[(4-chlorophenyl)methyl]-8-fluoro-1,1-dioxo-7-[5-(1,1,2,2,2-pentafluoroethyl)-1,2,4-oxadiazol-3-yl]-2,3-dihydro-1λ⁶,5-benzothiazepin-4-one

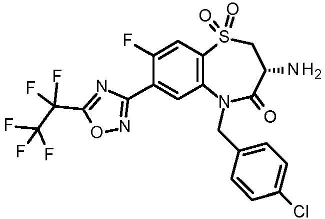

### Step a) tert-butyl N-[(3R)-5-[(4-chlorophenyl)methyl]-8-fluoro-1,1,4-trioxo-7-[5-(1,1,2,2,2-pentafluoroethyl)-1,2,4-oxadiazol-3-yl]-2,3-dihydro-1λ⁶,5-benzothiazepin-3-yl]carbamate

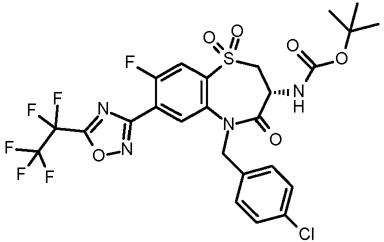

To a solution of tert-butyl N-[(3R)-5-[(4-chlorophenyl)methyl]-8-fluoro-7-[(Z)-N'-hydroxycarbamimidoyl]-1,1,4-trioxo-2,3-dihydro-1λ⁶,5-benzothiazepin-3-yl]carbamate (Example 310, step a)) (100.0 mg, 0.190 mmol, 1 eq) in DCM (2 mL) were added 2,2,3,3,3-pentafluoropropanoyl 2,2,3,3,3-pentafluoropropanoate (CAS 356-42-3) (0.19 mL, 0.950 mmol, 5 eq) and TEA (0.08 mL, 0.570 mmol, 3 eq) at 0°C and the mixture was stirred for 16 h at 20°C. The mixture was concentrated under vacuum to remove DCM, then diluted with water (3 mL) and extracted with EtOAc (2 mL x 3). The organic extracts were dried over Na₂SO₄ and concentrated and the remaining residue was purified by column chromatography on silica gel (4% to 40% EtOAc in PE) to afford the title compound (60 mg, 40% yield) as colorless oil. MS (ESI): 599.0 [M-isobutene+H]⁺.

### Step b) (3R)-3-amino-5-[(4-chlorophenyl)methyl]-8-fluoro-1,1-dioxo-7-[5-(1,1,2,2,2-pentafluoroethyl)-1,2,4-oxadiazol-3-yl]-2,3-dihydro-1λ⁶,5-benzothiazepin-4-one

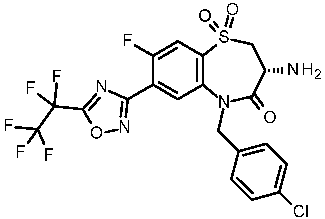

The title compound was prepared in analogy to general procedure **6b** from tert-butyl N-[(3R)-5-[(4-chlorophenyl)methyl]-8-fluoro-1,1,4-trioxo-7-[5-(1,1,2,2,2-pentafluoroethyl)-1,2,4-oxadiazol-3-yl]-2,3-dihydro-1λ⁶,5-benzothiazepin-3-yl]carbamate (60 mg, 0.092 mmol, 1 eq) and was obtained as hydrochloride salt as a white solid (17.3 mg, 34% yield). MS (ESI): 555.0 [M+H]⁺.

### Example 327

### (3R)-3-amino-5-[(4-chlorophenyl)methyl]-7-[5-[1,1-dimethyl-4-[2-(2-prop-2-ynoxyethoxy)ethoxy]butyl]-1,2,4-oxadiazol-3-yl]-8-fluoro-1,1-dioxo-2,3-dihydro-1λ⁶,5-benzothiazepin-4-one

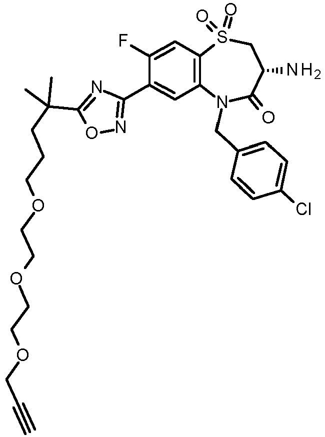

### Step a) ethyl 2, 2-dimethyl-5-[2-(2-prop-2-ynoxyethoxy)ethoxy]pentanoate

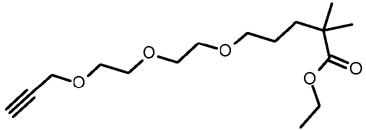

To a solution of 2-(2-prop-2-ynoxyethoxy)ethanol (CAS 7218-43-1) (1.0 g, 6.94 mmol, 1 eq) in THF (40 mL) was added NaH (60% in mineral oil, 305.2 mg, 7.6 mmol, 1.1 eq) at 0°C and the mixture was stirred for 0.5 h at 0°C. Then ethyl 5-bromo-2,2-dimethyl-pentanoate (CAS 77858-42-5) (1.64 g, 6.94 mmol, 1 eq) and NaI (103.97 mg, 0.690 mmol, 0.1 eq) were added at 0°C. The reaction mixture was stirred at 20°C for 0.5 h and then at 70°C for 16 h. The mixture was diluted in water (10 mL) and extracted with EtOAc (10 ml x 3). The combined extracts were dried over Na₂SO₄ and concentrated and the remaining residue was purified by column chromatography on silica gel (4% to 80% EtOAc in PE) to afford the title compound (450 mg, 19% yield) as yellow oil. ¹H NMR (400 MHz, CDCl₃) δ = 4.21 (d, *J =* 2.3 Hz, 2H), 4.11 (q, *J =* 7.1 Hz, 2H), 3.72 - 3.68 (m, 4H), 3.66 - 3.62 (m, 2H), 3.60 - 3.56 (m, 2H), 3.43 (t, *J =* 5.8 Hz, 2H), 2.43 (t, *J =* 2.3 Hz, 1H), 1.57 - 1.51 (m, 4H), 1.24 (t, *J =* 7.2 Hz, 3H), 1.16 (s, 6H).

### Step b) 2,2-dimethyl-5-[2-(2-prop-2-ynoxyethoxy)ethoxy]pentanoic acid

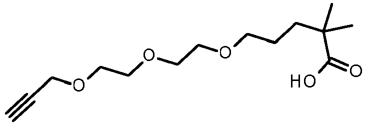

To a mixture of ethyl 2,2-dimethyl-5-[2-(2-prop-2-ynoxyethoxy)ethoxy]pentanoate (200 mg, 0.67 mmol, 1 eq) in MeOH (4 mL) was added a solution of KOH (149.41 mg, 2.66 mmol, 4 eq) in water (2 mL) under an atmosphere of nitrogen at 15°C and the mixture was stirred for 16 h at 20°C. The mixture was concentrated under vacuum to remove the MeOH and the pH was adjusted to 2-3 by addition of 1N HCl. The mixture was then extracted with EtOAc (5 mL x 3) and the combined extracts were dried over Na₂SO₄ and concentrated under vacuum to afford the title compound (190 mg, 94% yield) as yellow oil. MS (ESI): 271.0 [M-H]⁻.

### Step c) [(Z)-[amino-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]methylene]amino] 2,2-dimethyl-5-[2-(2-prop-2-ynoxyethoxy)ethoxy]pentanoate

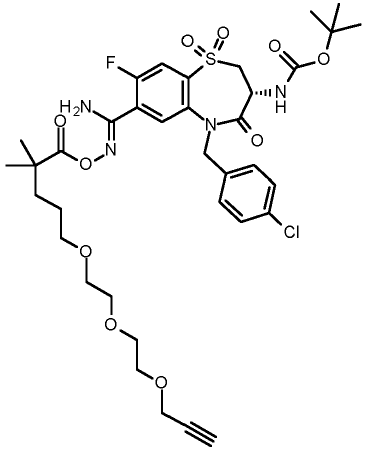

The title compound was prepared in analogy to general procedure **10b** from tert-butyl N-[(3R)-5-[(4-chlorophenyl)methyl]-8-fluoro-7-[(Z)-N'-hydroxycarbamimidoyl]-1,1,4-trioxo-2,3-dihydro-1λ⁶,5-benzothiazepin-3-yl]carbamate (Example 310, step a)) (250 mg, 0.474 mmol, 1 eq) and 2,2-dimethyl-5-[2-(2-prop-2-ynoxyethoxy)ethoxy]pentanoic acid (167.96 mg, 0.620 mmol, 1.3 eq) (Example 327, step b)) and was obtained as a red solid (140 mg, 78% yield). MS (ESI): 781.1 [M+H]⁺.

### Step d) tert-butyl N-[(3R)-5-[(4-chlorophenyl)methyl]-7-[5-[1,1-dimethyl-4-[2-(2-prop-2-ynoxyethoxy)ethoxy]butyl]-1,2,4-oxadiazol-3-yl]-8-fluoro-1,1,4-trioxo-2,3-dihydro-1λ⁶,5-benzothiazepin-3-yl]carbamate

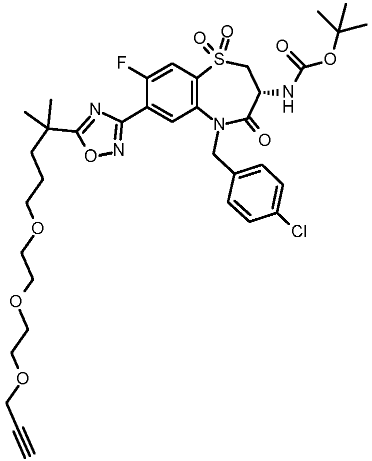

The title compound was prepared in analogy to general procedure **11a** from [(Z)-[amino-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]methylene]amino] 2,2-dimethyl-5-[2-(2-prop-2-ynoxyethoxy)ethoxy]pentanoate (100 mg, 0.128 mmol, 1 eq) and was obtained as a yellow oil (40 mg, 36% yield). MS (ESI): 707.0 [M-isobutene+H]⁺.

### Step e) (3R)-3-amino-5-[(4-chlorophenyl)methyl]-7-[5-[1,1-dimethyl-4-[2-(2-prop-2-ynoxyethoxy)ethoxy]butyl]-1,2,4-oxadiazol-3-yl]-8-fluoro-1,1-dioxo-2,3-dihydro-1λ⁶,5-benzothiazepin-4-one

The title compound was prepared in analogy to general procedure **6b** from tert-butyl N-[(3R)-5-[(4-chlorophenyl)methyl]-7-[5-[1,1-dimethyl-4-[2-(2-prop-2-ynoxyethoxy)ethoxy]butyl]-1,2,4-oxadiazol-3-yl]-8-fluoro-1,1,4-trioxo-2,3-dihydro-1λ⁶,5-benzothiazepin-3-yl]carbamate (150 mg, 0.197 mmol, 1 eq) and was obtained as hydrochloride salt as a white solid (72 mg, 52% yield). MS (ESI): 663.0 [M+H]⁺.

### Example 328

### 3-[(3R)-3-amino-8-fluoro-1,1,4-trioxo-5-[[4-(trifluoromethoxy)phenyl]methyl]-2,3-dihydro-1λ⁶,5-benzothiazepin-7-yl]-N,N-dimethyl-1,2,4-oxadiazole-5-carboxamide

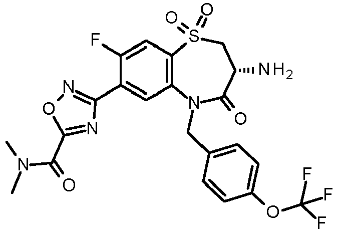

### Step a) tert-butyl N-[(3R)-8-fluoro-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate

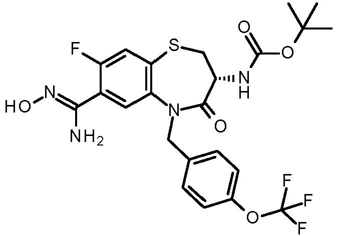

The title compound was prepared in analogy to general procedure 12 from tert-butyl N-[(3R)-7-cyano-8-fluoro-4-oxo-5-[[4-(trifluoromethoxy)phenyl]methyl]-2,3-dihydro-1,5-benzothiazepin-3-yl]carbamate (Example 30, step a)) (324 mg, 0.633 mmol, 1 eq) and was obtained as a yellow solid (300 mg, 65% yield). MS (ESI): 545.2 [M+H]⁺.

### Step b) tert-butyl N-[(3R)-7-[5-(dimethylcarbamoyl)-1, 2, 4-oxadiazol-3-yl]-8-fluoro-4-oxo-5-[[4-(trifluoromethoxy)phenyl]methyl]-2,3-dihydro-1,5-benzothiazepin-3-yl]carbamate

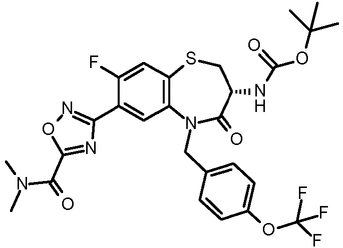

The title compound was prepared in analogy to general procedure **9c** from tert-butyl N-[(3R)-8-fluoro-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate (100 mg, 0.138 mmol, 1 eq) and 2-(dimethylamino)-2-keto-acetic acid (19.4 mg, 0.165 mmol, 1.2 eq) and was obtained as an orange solid (20.3 mg, 20% yield). MS (ESI): 624.2 [M-H]⁻.

### Step c) tert-butylN-[(3R)-7-[5-(dimethylcarbamoyl)-1,2,4-oxadiazol-3-yl]-8-fluoro-1,1,4-trioxo-5-[[4-(trifluoromethoxy)phenyl]methyl]-2,3-dihydro-1λ⁶,5-benzothiazepin-3-yl]carbamate

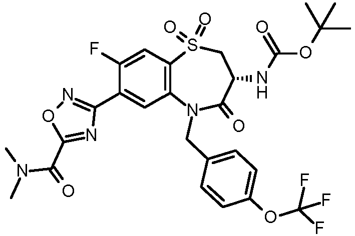

The title compound was prepared in analogy to general procedure **5** from tert-butyl N-[(3R)-7-[5-(dimethylcarbamoyl)-1,2,4-oxadiazol-3-yl]-8-fluoro-4-oxo-5-[[4-(trifluoromethoxy)phenyl]methyl]-2,3-dihydro-1,5-benzothiazepin-3-yl]carbamate (20.2 mg, 0.032 mmol, 1 eq) and was obtained as a white solid (20.3 mg, 20% yield). MS (ESI): 656.2 [M-H]⁻.

### Step d) 3-[(3R)-3-amino-8-fluoro-1,1,4-trioxo-5-[[4-(trifluoromethoxy)phenyl]methyl]-2,3-dihydro-1λ⁶,5-benzothiazepin-7-yl]-N,N-dimethyl-1,2,4-oxadiazole-5-carboxamide

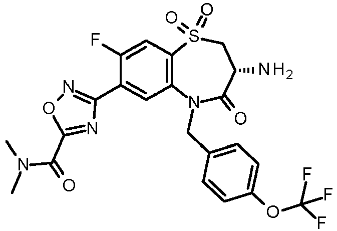

The title compound was prepared in analogy to general procedure **6d** from tert-butyl N-[(3R)-7-[5-(dimethylcarbamoyl)-1,2,4-oxadiazol-3-yl]-8-fluoro-1,1,4-trioxo-5-[[4-(trifluoromethoxy)phenyl]methyl]-2,3-dihydro-1λ⁶,5-benzothiazepin-3-yl]carbamate (12.3 mg, 0.019 mmol, 1 eq) and was obtained as hydrochloride salt as a white solid (9.4 mg, 79% yield). MS (ESI): 558.3 [M+H]⁺.

The examples 329 and 330 of the following table were prepared in analogy to Example 328, using the appropriate carboxylic acid building block.

| **Ex.** | **Structure** | **Systematic Name** | **Building Block, General procedure** | **MS, ESI: m/z** |
|---|---|---|---|---|
| 329 | 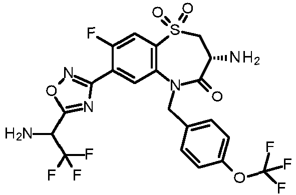 | (3R)-3-amino-7-[5-(1-amino-2,2,2-trifluoro-ethyl)-1,2,4-oxadiazol-3-yl]-8-fluoro-1,1-dioxo-5-[[4-(trifluoromethoxy)phenyl]met hyl]-2,3-dihydro-1λ⁶,5-benzothiazepin-4-one | 2-(tert-Butoxycar bonylamin 0)-3,3,3-trifluoro-propanoic acid | 584.2 [M+H]⁺ |
| | | | (CAS 188030-43-5) | |
| | | (*) | | |
| | | | **10b, 11a, 6b** | |
| 330 | 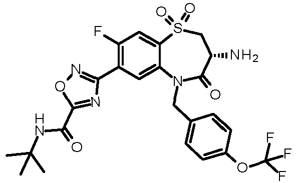 | 3-[(3R)-3-amino-8-fluoro-1,1,4-trioxo-5-[[4-(trifluoromethoxy)phenyl]met hyl]-2,3-dihydro-1λ⁶,5-benzothiazepin-7-yl]-N-tert-butyl-1,2,4-oxadiazole-5-carboxamide | 2-(tert-Butylamin o)-2-oxo-acetic acid | 586.0 [M+H]⁺ |
| | | | (CAS 169772-25-2) | |
| | | | **9c, 6b** | |

| | | | | |
|---|---|---|---|---|
| * as a dihydrochloride salt | | | | |

### Example 331

### (3R)-3-amino-7-[5-(1-amino-2,2,2-trifluoro-1-methyl-ethyl)-1,2,4-oxadiazol-3-yl]-8-fluoro-1,1-dioxo-5-[[4-(trifluoromethoxy)phenyl]methyl]-2,3-dihydro-1λ⁶,5-benzothiazepin-4-one

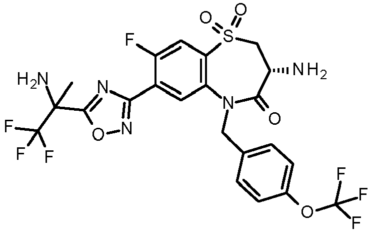

### Step a) tert-butyl N-[(3R)-8-fluoro-7-[(Z)-N'-hydroxycarbamimidoyl]-1,1,4-trioxo-5-[[4-(trifluoromethoxy)phenyl]methyl]-2,3-dihydro-1λ⁶,5-benzothiazepin-3-yl]carbamate

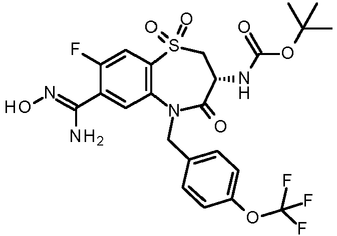

The title compound was prepared in analogy to general procedure 12 from tert-butyl N-[(3R)-7-cyano-8-fluoro-1,1,4-trioxo-5-[[4-(trifluoromethoxy)phenyl]methyl]-2,3-dihydro-1λ⁶,5-benzothiazepin-3-yl]carbamate (Example 30, step b)) (766 mg, 1.41 mmol, 1 eq) and was obtained as a light yellow foam (479.1 mg, 59% yield). MS (ESI): 577.3 [M+H]⁺.

### Step b) [(Z)-[amino-[(3R)-3-(tert-butoxycarbonylamino)-8-fluoro-1,1,4-trioxo-5-[[4-(trifluoromethoxy)phenyl]methyl]-2,3-dihydro-1λ⁶,5-benzothiazepin-7-yl]methylene]amino] 2-(tert-butoxycarbonylamino)-3,3,3-trifluoro-2-methyl-propanoate

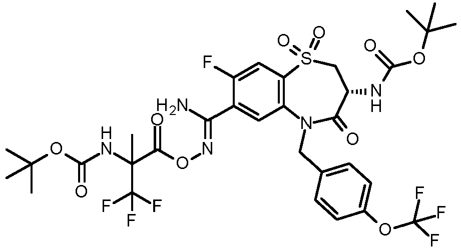

The title compound was prepared in analogy to general procedure **10a** from tert-butyl N-[(3R)-8-fluoro-7-[(Z)-N'-hydroxycarbamimidoyl]-1,1,4-trioxo-5-[[4-(trifluoromethoxy)phenyl]methyl]-2,3-dihydro-1λ⁶,5-benzothiazepin-3-yl]carbamate (250 mg, 0.434 mmol, 1 eq) and 2-(tert-butoxycarbonylamino)-3,3,3-trifluoro-2-methyl-propanoic acid (CAS 1900683-56-8). The compound was obtained as a crude product as a light brown oil (546.4 mg) and was used as such in the next reaction step. MS (ESI): 816.5 [M+H]⁺.

### Step c) tert-butylN-[1-[3-[(3R)-3-(tert-butoxycarbonylamino)-8-fluoro-1,1,4-trioxo-5-[[4-(trifluoromethoxy)phenyl]methyl]-2,3-dihydro-1λ⁶,5-benzothiazepin-7-yl]-1,2,4-oxadiazol-5-yl]-2,2,2-trifluoro-1-methyl-ethyl]carbamate

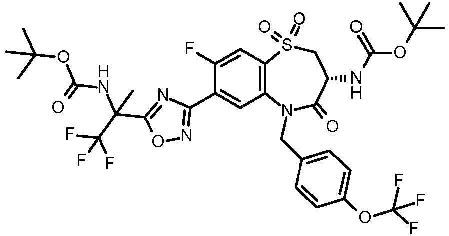

The title compound was prepared in analogy to general procedure **11a** from [(Z)-[amino-[(3R)-3-(tert-butoxycarbonylamino)-8-fluoro-1,1,4-trioxo-5-[[4-(trifluoromethoxy)phenyl]methyl]-2,3-dihydro-1λ⁶,5-benzothiazepin-7-yl]methylene]amino]2-(tert-butoxycarbonylamino)-3,3,3-trifluoro-2-methyl-propanoate (540 mg, 0.662 mmol, 1 eq) and was obtained as a light brown oil (111.7 mg, 21% yield). MS (ESI): 796.4 [M-H]⁻.

### Step d) (3R)-3-amino-7-[5-(1-amino-2,2,2-trifluoro-1-methyl-ethyl)-1,2,4-oxadiazol-3-yl]-8-fluoro-1,1-dioxo-5-[[4-(trifluoromethoxy)phenyl]methyl]-2,3-dihydro-1λ⁶,5-benzothiazepin-4-one

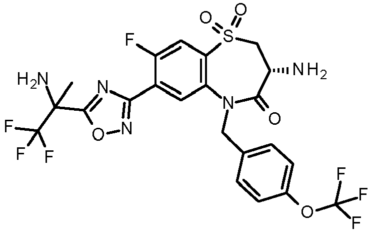

The title compound was prepared in analogy to general procedure **6d** from tert-butyl N-[1-[3-[(3R)-3-(tert-butoxycarbonylamino)-8-fluoro-1,1,4-trioxo-5-[[4-(trifluoromethoxy)phenyl]methyl]-2,3-dihydro-1λ⁶,5-benzothiazepin-7-yl]-1,2,4-oxadiazol-5-yl]-2,2,2-trifluoro-1-methyl-ethyl]carbamate (112 mg, 0.140 mmol, 1 eq) and was obtained as dihydrochloride salt as a white solid (69.6 mg, 74% yield). MS (ESI): 598.3 [M+H]⁺.

The examples 332 and 333 of the following table were prepared in analogy to Example 331, using the appropriate carboxylic acid building block.

| **Ex.** | **Structure** | **Systematic Name** | **Building Block** | **MS, ESI: m/z** |
|---|---|---|---|---|
| 332 | 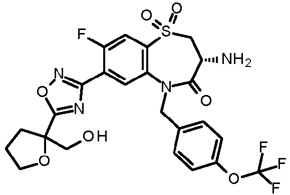 | (3R)-3-amino-8-fluoro-7-[5-[2-(hydroxymethyl)tetrahydrofur an-2-yl]-1,2,4-oxadiazol-3-yl]-1,1-dioxo-5-[[4-(trifluoromethoxy)phenyl]met hyl]-2,3-dihydro-1λ⁶,5-benzothiazepin-4-one | 2-(Hydroxym ethyl)tetra hydrofuran -2-carboxylic acid | 587.4 [M+H]⁺ |
| | | | (CAS 442877-01-2) | |
| | | | **10a, 11a, 6c** | |
| 333 | 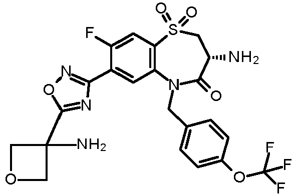 | (3R)-3-amino-7-[5-(3-aminooxetan-3-yl)-1,2,4-oxadiazol-3-yl]-8-fluoro-1,1-dioxo-5-[[4-(trifluoromethoxy)phenyl]met hyl]-2,3-dihydro-1lambda6,5-benzothiazepin-4-one | 3-(tert-Butoxycar bonylamin o)oxetane-3-carboxylic acid (CAS 1159736-25-0) **10c, 11a, 6c** | 558.3 [M+H]⁺ |

### Example 334

### (3R)-3-amino-8-fluoro-7-[5-(5-methyl-1,3,4-oxadiazol-2-yl)-1,2,4-oxadiazol-3-yl]-1,1-dioxo-5-[[4-(trifluoromethoxy)phenyl]methyl]-2,3-dihydro-1λ⁶,5-benzothiazepin-4-one

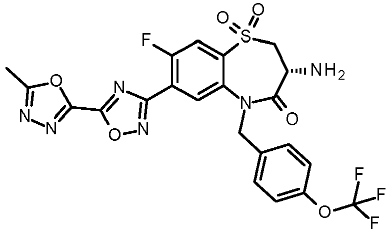

### Step a) [(Z)-[amino-[(3R)-3-(tert-butoxycarbonylamino)-8-fluoro-4-oxo-5-[[4-(trifluoromethoxy)phenyl]methyl]-2,3-dihydro-1,5-benzothiazepin-7-yl]methylene]amino] 5-methyl-1,3,4-oxadiazole-2-carboxylate

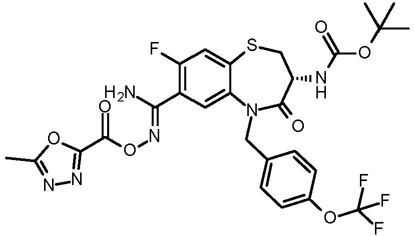

To a solution of tert-butyl N-[(3R)-8-fluoro-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate (Example 328, step a)) (103 mg, 0.189 mmol, 1 eq) and pyridine (44.99 mg, 46.01 µL, 0.569 mmol, 3.01 eq) in 1,2-dichloroethane (3.54 mL) was added 5-methyl-1,3,4-oxadiazole-2-carbonyl chloride (CAS 889131-28-6) (41.58 mg, 0.284 mmol, 1.5 eq) as a solution in 1,2-dichloroethane (3.54 mL) at 0-5°C. The ice bath was removed and the reaction mixture was stirred for 2h. The mixture was partitioned between DCM and sat. NaHCO₃ solution. The layers were separated and the aqueous layer was extracted with EtOAc. The combined organic layers were washed with brine, dried over anhydrous sodium sulfate and concentrated in vacuo affording the title compound (36 mg, 28%) as light yellow solid. MS (ESI): 653.4 [M-H]⁻.

### Step b) tert-butyl N-[(3R)-8-fluoro-7-[5-(5-methyl-1,3,4-oxadiazol-2-yl)-1,2,4-oxadiazol-3 -yl]-4-oxo-5-[[4-(trifluoromethoxy)phenyl]methyl]-2,3-dihydro-1,5-benzothiazepin-3-yl]carbamate

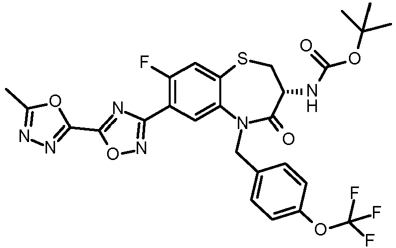

The title compound was prepared in analogy to general procedure **11a** from [(Z)-[amino-[(3R)-3-(tert-butoxycarbonylamino)-8-fluoro-4-oxo-5-[[4-(trifluoromethoxy)phenyl]methyl]-2,3-dihydro-1,5-benzothiazepin-7-yl]methylene]amino] 5-methyl-1,3,4-oxadiazole-2-carboxylate (36 mg, 0.052 mmol, 1 eq) and was obtained as a white solid (16 mg, 48% yield). MS (ESI): 635.5 [M-H]⁻.

### Step c) tert-butyl N-[(3R)-8-fluoro-7-[5-(5-methyl-1,3,4-oxadiazol-2-yl)-1,2,4-oxadiazol-3-yl]-1,1,4-trioxo-5-[[4-(trifluoromethoxy)phenyl]methyl]-2,3-dihydro-1λ⁶,5-benzothiazepin-3-yl]carbamate

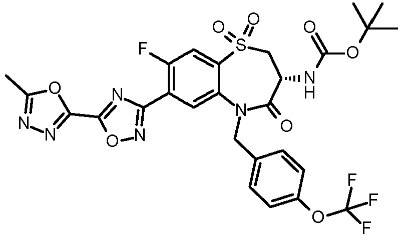

The title compound was prepared in analogy to general procedure 5 from tert-butyl N-[(3R)-8-fluoro-7-[5-(5-methyl-1,3,4-oxadiazol-2-yl)-1,2,4-oxadiazol-3-yl]-4-oxo-5-[[4-(trifluoromethoxy)phenyl]methyl]-2,3-dihydro-1,5-benzothiazepin-3-yl]carbamate (6 mg, 0.025 mmol, 1 eq) and was obtained as a white solid (12.7 mg, 73% yield). MS (ESI): 667.3 [M-H]⁻.

### Step d) (3R)-3-amino-8-fluoro-7-[5-(5-methyl-1,3,4-oxadiazol-2-yl)-1,2,4-oxadiazol-3-yl]-1,1-dioxo-5-[[4-(trifluoromethoxy)phenyl]methyl]-2, 3-dihydro-1λ⁶,5-benzothiazepin-4-one

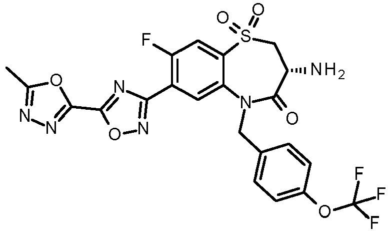

The title compound was prepared in analogy to general procedure **6d** from tert-butyl N-[(3R)-8-fluoro-7-[5-(5-methyl-1,3,4-oxadiazol-2-yl)-1,2,4-oxadiazol-3-yl]-1,1,4-trioxo-5-[[4-(trifluoromethoxy)phenyl]methyl]-2,3-dihydro-1λ⁶, 5-benzothiazepin-3-yl]carbamate (12.7 mg, 0.018 mmol, 1 eq) and was obtained as hydrochloride salt as a light brown solid (10.8 mg, 91% yield). MS (ESI): 569.6 [M+H]⁺.

### Example 335

### methyl 1-[3-[(3R)-3-amino-8-fluoro-1,1,4-trioxo-5-[[4-(trifluoromethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate

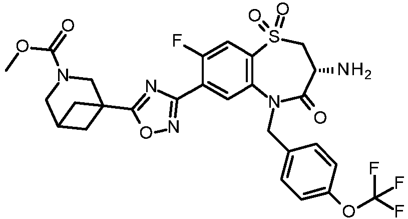

### Step a) O1-[(Z)-[amino-[(3R)-3-(tert-butoxycarbonylamino)-8-fluoro-4-oxo-5-[[4-(trifluoromethoxy)phenyl]methyl]-2,3-dihydro-1,5-benzothiazepin-7-yl]methylene]amino] O3-tert-butyl 3-azabicyclo[3.1.1]heptane-1,3-dicarboxylate

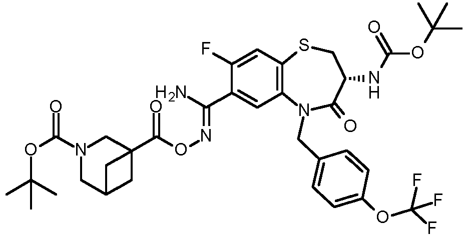

The title compound was prepared in analogy to general procedure **10a** from tert-butyl N-[(3R)-8-fluoro-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate (Example 328, step a)) (90 mg, 0.17 mmol, 1 eq) and 3-tert-butoxycarbonyl-3-azabicyclo[3.1.1]heptane-1-carboxylic acid (CAS 1000931-22-5) and was obtained as a yellow oil (120 mg, 95% yield). MS (ESI): 768.3 [M+H]⁺.

### Step b) tert-butyl 1-[3-[(3R)-3-(tert-butoxycarbonylamino)-8-fluoro-4-oxo-5-[[4-(trifluoromethoxy)phenyl]methyl]-2,3-dihydro-1,5-benzothiazepin-7-yl]-1,2,4-oxadiazol-5-yl]-3-azabicyclo[3.1.1]heptane-3-carboxylate

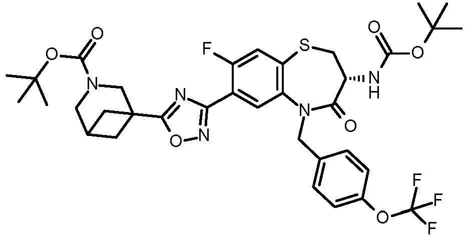

The title compound was prepared in analogy to general procedure **11a** from O1-[(Z)-[amino-[(3R)-3-(tert-butoxycarbonylamino)-8-fluoro-4-oxo-5-[[4-(trifluoromethoxy)phenyl]methyl]-2,3-dihydro-1,5-benzothiazepin-7-yl]methylene]amino] O3-tert-butyl 3-azabicyclo[3.1.1]heptane-1,3-dicarboxylate (110 mg, 0.150 mmol, 1 eq) and was obtained as a yellow oil (110 mg, 94% yield). MS (ESI): 650 [M-isobutene-CO₂+H]⁺.

### Step c) tert-butyl 1-[3-[(3R)-3-(tert-butoxycarbonylamino)-8-fluoro-1,1,4-trioxo-5-[[4-(trifluoromethoxy)phenylmethyl]-2,3-dihydro-1λ⁶,5-benzothiazepin-7-yl]-1,2,4-oxadiazol-5-yl]-3-azabicyclo[3.1.1]heptane-3-carboxylate

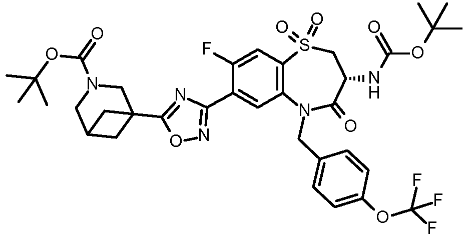

The title compound was prepared in analogy to general procedure 5 from tert-butyl 1-[3-[(3R)-3-(tert-butoxycarbonylamino)-8-fluoro-4-oxo-5-[[4-(trifluoromethoxy)phenyl]methyl]-2,3-dihydro-1,5-benzothiazepin-7-yl]-1,2,4-oxadiazol-5-yl]-3-azabicyclo[3.1.1]heptane-3-carboxylate (150 mg, 0.2 mmol, 1 eq) and was obtained as a white solid (90 mg, 58% yield). MS (ESI): 626 [M-isobutene-CO₂+H]⁺.

### Step d) (3R)-3-amino-7-[5-(3-azabicyclo[3.1.1]heptan-1-yl)-1,2,4-oxadiazol-3-yl]-8-fluoro-1,1-dioxo-5-[[4-(trifluoromethoxy)phenyl]methyl]-2,3-dihydro-1λ⁶,5-benzothiazepin-4-one

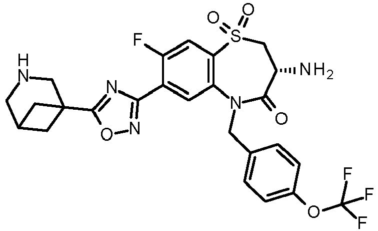

The title compound was prepared in analogy to general procedure **6b** from tert-butyl 1-[3-[(3R)-3-(tert-butoxycarbonylamino)-8-fluoro-1,1,4-trioxo-5-[[4-(trifluoromethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate (80 mg, 0.100 mmol, 1 eq) and was obtained as dihydrochloride salt as a white solid (50 mg, 75% yield). MS (ESI): 582.1 [M+H]⁺.

### Step e) methyl 1-[3-[(3R)-3-amino-8-fluoro-1,1,4-trioxo-5-[[4-(trifluoromethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate

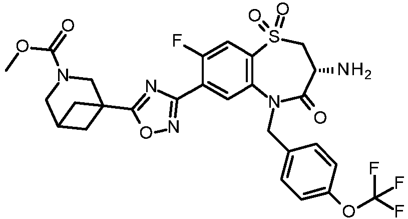

To a solution of (3R)-3-amino-7-[5-(3-azabicyclo[3.1.1]heptan-1-yl)-1,2,4-oxadiazol-3-yl]-8-fluoro-1,1-dioxo-5-[[4-(trifluoromethoxy)phenyl]methyl]-2,3-dihydro-1λ6,5-benzothiazepin-4-one; dihydrochloride (40.0 mg, 0.060 mmol, 1 eq) in DCM (3 mL) were added DIPEA (24.0 mg, 0.190 mmol, 3.04 eq) and methyl chloroformate (6.0 mg, 0.060 mmol, 1.04 eq) and the mixture was stirred at 20°C for 1 h. The solvent was evaporated and the crude product was purified by prep. HPLC affording the title compound (19.7 mg, 0.030 mmol, 50% yield) as a white solid. MS (ESI): 640.1 [M+H]⁺.

### Example 336

### methyl 1-[3-[(3R)-3-amino-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer A]

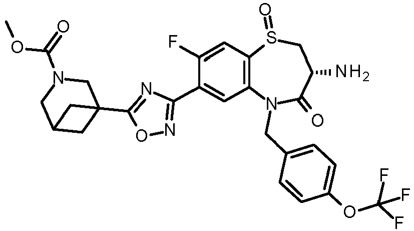

### Step a) tert-butyl 1-[3-[(3R)-3-(tert-butoxycarbonylamino)-8-fluoro-1,4-dioxo-5-[[4-(trifluoromethoxy)phenyl]methyl]-2,3-dihydro-1λ⁴,5-henzothiazepin-7-yl]-1,2,4-oxadiazol-5-yl]-3-azahicyclo[3.1.1]heptane-3-carboxylate [Epimer A] and tert-butyl 1-[3-[(3R)-3-(tert-butoxycarbonylamino)-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer B]

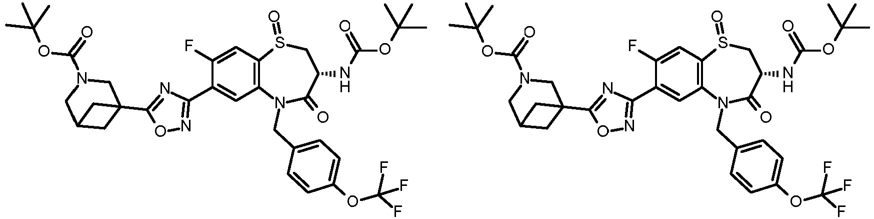

To a solution of tert-butyl 1-[3-[(3R)-3-(tert-butoxycarbonylamino)-8-fluoro-4-oxo-5-[[4-(trifluoromethoxy)phenyl]methyl]-2,3-dihydro-1,5-benzothiazepin-7-yl]-1,2,4-oxadiazol-5-yl]-3-azabicyclo[3.1.1]heptane-3-carboxylate (Example 328, step b)) (150.0 mg, 0.200 mmol, 1 eq) in DCM (2 mL) was added m-CPBA (33.0 mg, 0.160 mmol, 0.810 eq) and the solution was stirred at 20°C for 12 h. The mixture was poured into water (20 mL) and extracted with DCM (60 mL). The organic phase was washed with brine (50 mL), dried (Na₂SO₄) and concentrated on vacuum. The remaining residue was purified by preparative TLC(PE/EA = 1:1) to afford tert-butyl 1-[3-[(3R)-3-(tert-butoxycarbonylamino)-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer A] (60 mg, 39% yield) as a white solid (MS (ESI): 610.0 [M-Boc-isobutene+H]⁺) and tert-butyl 1-[3-[(3R)-3-(tert-butoxycarbonylamino)-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer B] (30 mg, 19% yield) as a light yellow solid (MS (ESI): 610.0 [M-Boc-isobutene+H]⁺).

### Step b) (3R)-3-amino-7-[5-(3-azabicyclo[3.1.1]heptan-1-yl)-1,2,4-oxadiazol-3-yl]-8-fluoro-1-oxo-5-[[4-(trifluoromethoxy)phenyl]methyl]-2,3-dihydro-1λ⁴,5-benzothiazepin-4-one [Epimer A]

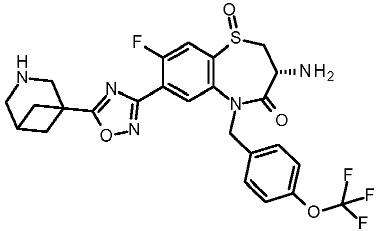

A solution of tert-butyl 1-[3-[(3R)-3-(tert-butoxycarbonylamino)-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer A] (55.0 mg, 0.070 mmol, 1 eq) and trifluoroacetic acid (1.0 mL, 12.98 mmol, 180.72 eq) in DCM (10 mL) was stirred at 20°C for 1 h. The solvent was evaporated to afford the title compound (60 mg, 97% yield) as a TFA salt as a white solid. MS (ESI): 566.1 [M+H]⁺.

### Step c) methyl 1-[3-[(3R)-3-amino-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer A]

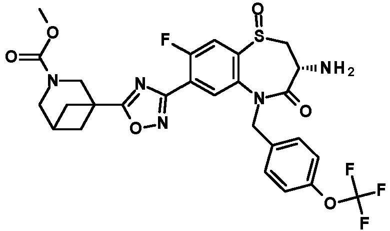

To a solution of (3R)-3-amino-7-[5-(3-azabicyclo[3.1.1]heptan-1-yl)-1,2,4-oxadiazol-3-yl]-8-fluoro-1-oxo-5-[[4-(trifluoromethoxy)phenyl]methyl]-2,3-dihydro-1λ⁴,5-benzothiazepin-4-one [Epimer A] (60.0 mg, 0.070 mmol, 1 eq) and DIPEA (26.38 mg, 0.200 mmol, 3 eq) in DCM (5 mL) was added dropwise a solution of methyl chloroformate (6.43 mg, 0.070 mmol, 1 eq) in DCM (0.500 mL) at 20°C and the mixture was stirred at 20°C for 1 h. The reaction mixture was concentrated under vacuum and the remaining residue was purified by prep. HPLC to afford the title compound (24.7 mg, 58% yield) as a white solid. MS (ESI): 624.1 [M+H]⁺.

### Example 337

### methyl 1-[3-[(3R)-3-amino-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer B]

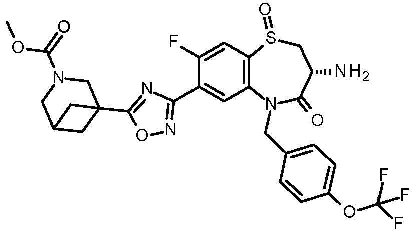

### Step a) (3R)-3-amino-7-[5-(3-azabicyclo[3.1.1]heptan-1-yl)-1,2,4-oxadiazol-3-yl]-8-fluoro-1-oxo-5-[[4-(trifluoromethoxy)phenyl]methyl]-2, 3-dihydro-1λ⁴,5-benzothiazepin-4-one [Epimer B]

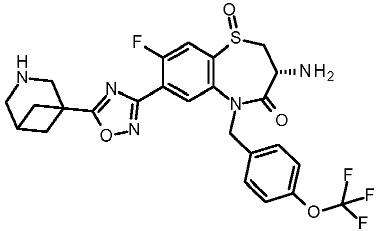

A solution of tert-butyl 1-[3-[(3R)-3-(tert-butoxycarbonylamino)-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer B] (Example 336, step a)) (25.0 mg, 0.030 mmol, 1 eq) and trifluoroacetic acid (0.5 mL, 6.49 mmol, 198 eq) in DCM (5 mL) was stirred at 20 °C for 1 h. The solvent was evaporated to afford the title compound (25 mg, 90% yield) as a TFA salt as a white solid. MS (ESI): 566.0 [M+H]⁺.

### Step b) methyl 1-[3-[(3R)-3-amino-8-fluoro-1,4-dioxo-5-[[4-(trifluoromethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate [Epimer B]

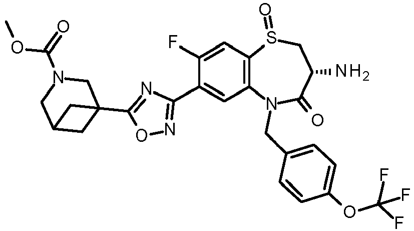

To a solution of (3R)-3-amino-7-[5-(3-azabicyclo[3.1.1]heptan-1-yl)-1,2,4-oxadiazol-3-yl]-8-fluoro-1-oxo-5-[[4-(trifluoromethoxy)phenyl]methyl]-2,3-dihydro-1λ⁴,5-benzothiazepin-4-one [Epimer B] (25.0 mg, 0.030 mmol, 1 eq) and DIPEA (11.36 mg, 0.09 mmol, 3 eq) in DCM (5 mL) was added dropwise a solution of methyl chloroformate (2.77 mg, 0.03 mmol, 1 eq) in DCM (0.5 mL) at 20 °C and the mixture was stirred at 20°C for 1 h. The reaction mixture was concentrated under vacuum and the remaining residue was purified by prep. HPLC to afford the title compound (12.1 mg, 0.020 mmol, 64% yield) as a light yellow solid. MS (ESI): 624.1 [M+H]⁺.

### Example 338

### (3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1λ⁴,5-benzothiazepin-4-one [Epimer A at S]

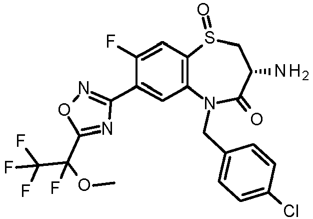

### Step a) tert-butylN-[(3R)-5-[(4-chlorophenyl)methyl]-8-fluoro-4-oxo-7-[5-(1,2,2,2-tetrafluoro-1-methoxy-ethyl)-1,2,4-oxadiazol-3-yl]-2,3-dihydro-1,5-benzothiazepin-3-yl]carbamate

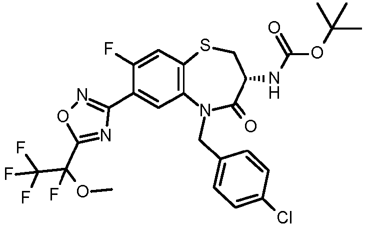

The title compound was prepared in analogy to general procedure **9a** from tert-butyl N-[(3R)-8-fluoro-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate (Example 289, step a)) (120 mg, 0.175 mmol, 1 eq) and 2,3,3,3-tetrafluoro-2-methoxy-propanoic acid (CAS 10186-64-8) and was obtained as a light yellow solid (23 mg, 20% yield). MS (ESI): 579.1 [M-isobutene+H]⁺.

### Step b) tert-butyl N-[(3R)-5-[(4-chlorophenyl)methyl]-8-fluoro-1,4-dioxo-7-[5-(1,2,2,2-tetrafluoro-1-methoxy-ethyl)-1,2,4-oxadiazol-3-yl]-2,3-dihydro-1λ⁴,5-benzothiazepin-3-yl]carbamate [Epimer A at S] and tert-butyl N-[(3R)-5-[(4-chlorophenyl)methyl]-8-fluoro-1,4-dioxo-7-[5-(1,2,2,2-tetrafluoro-1-methoxy-ethyl)-1,2,4-oxadiazol-3-yl]-2, 3-dihydro-1λ⁴,5-benzothiazepin-3-yl]carbamate [Epimer B at S]

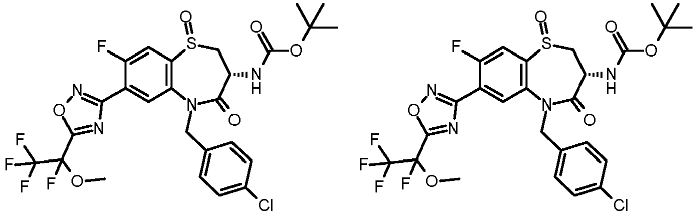

tert-butyl N-[(3R)-5-[(4-chlorophenyl)methyl]-8-fluoro-4-oxo-7-[5-(1,2,2,2-tetrafluoro-1-methoxy-ethyl)-1,2,4-oxadiazol-3-yl]-2,3-dihydro-1,5-benzothiazepin-3-yl]carbamate (23 mg, 0.034 mmol, 1 eq) was stirred with 3-chloroperoxybenzoic acid (7.63 mg, 0.034 mmol, 1 eq) in DCM (0.681 mL) at room temperature for 30 min. The reaction solution was diluted with DCM and 1N NaOH and was extracted with DCM twice. The combined organic layers were washed with water and brine, dried over Na₂SO₄ and evaporated and the remaining residue was purified by column chromatography on silica gel (0% to 50% EtOAc in heptane) to afford tert-butyl N-[(3R)-5-[(4-chlorophenyl)methyl]-8-fluoro-1,4-dioxo-7-[5-(1,2,2,2-tetrafluoro-1-methoxyethyl)-1,2,4-oxadiazol-3-yl]-2,3-dihydro-1λ⁴,5-benzothiazepin-3-yl]carbamate [Epimer A at S] (9 mg, 39%) as off-white solid (MS (ESI): 551.0 [M-BOC+H]⁺) and tert-butyl N-[(3R)-5-[(4-chlorophenyl)methyl]-8-fluoro-1,4-dioxo-7-[5-(1,2,2,2-tetrafluoro-1-methoxy-ethyl)-1,2,4-oxadiazol-3-yl]-2,3-dihydro-1λ⁴,5-benzothiazepin-3-yl]carbamate [Epimer *B* at S] (6 mg, 26%) as off-white solid (MS (ESI): 651.3 [M+H]⁺).

### Step c) (3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1λ⁴,5-benzothiazepin-4-one [Epimer A at S]

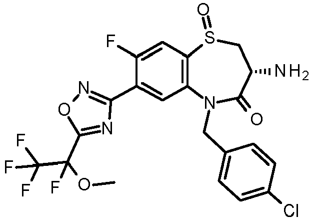

The title compound was prepared in analogy to general procedure **6d** from tert-butyl N-[(3R)-5-[(4-chlorophenyl)methyl]-8-fluoro-1,4-dioxo-7-[5-(1,2,2,2-tetrafluoro-1-methoxy-ethyl)-1,2,4-oxadiazol-3-yl]-2,3-dihydro-1λ⁴,5-benzothiazepin-3-yl]carbamate [Epimer A at S] (9 mg, 0.014 mmol, 1 eq) and was obtained as hydrochloride salt as a off-white solid (8 mg, 95% yield). MS (ESI): 551.1 [M+H]⁺.

### Example 339

### (3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1λ⁴,5-benzothiazepin-4-one [Epimer B at S]

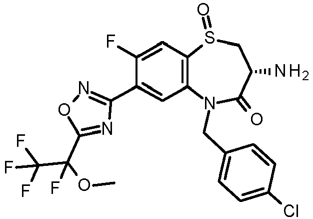

The title compound was prepared in analogy to general procedure **6d** from tert-butyl N-[(3R)-5-[(4-chlorophenyl)methyl]-8-fluoro-1,4-dioxo-7-[5-(1,2,2,2-tetrafluoro-1-methoxy-ethyl)-1,2,4-oxadiazol-3-yl]-2,3-dihydro-1λ⁴,5-benzothiazepin-3-yl]carbamate [Epimer B at S] (Example 338, step b)) (6 mg, 0.009 mmol, 1 eq) and was obtained as hydrochloride salt as a light green solid (5 mg, 98% yield). MS (ESI): 551.1 [M+H]⁺.

### Example 340

### (3R)-3-amino-7-(5-tert-butyl-1,2,4-oxadiazol-3-yl)-5-[(4-chlorophenyl)methyl]-8-fluoro-1-oxo-2,3-dihydro-1λ⁴,5-benzothiazepin-4-one [Epimer A]

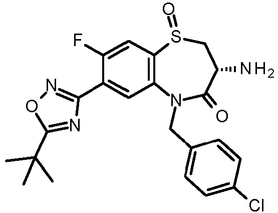

### Step a) tert-butyl N-[(3R)-7-(5-tert-butyl-1,2,4-oxadiazol-3-yl)-5-[(4-chlorophenyl)methyl]-8-fluoro-4-oxo-2,3-dihydro-1,5-benzothiazepin-3-yl]carbamate

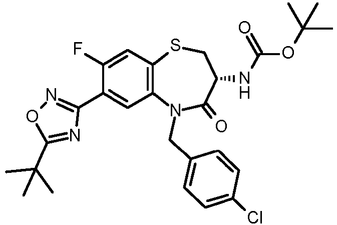

The title compound was prepared in analogy to general procedure **9a** from tert-butyl N-[(3R)-8-fluoro-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate (Example 289, step a)) (210 mg, 0.424 mmol, 1 eq) and 2,2-dimethylpropanoic acid (CAS 75-98-9) and was obtained as a white solid (202 mg, 85% yield). MS (ESI): 505.1 [M-isobutene+H]⁺.

### Step b) tert-butyl N-[(3R)-7-(5-tert-butyl-1,2,4-oxadiazol-3-yl)-5-[(4-chlorophenyl)methyl]-8-fluoro-1,4-dioxo-2,3-dihydro-1λ⁴,5-benzothiazepin-3-yl]carbamate [Epimer A] and tert-butyl N-[(3R)-7-(5-tert-butyl-1,2,4-oxadiazol-3-yl)-5-[(4-chlorophenyl)methyl]-8-fluoro-1,4-dioxo-2,3-dihydro-1λ⁴,5-benzothiazepin-3-yl]carbamate [Epimer B]

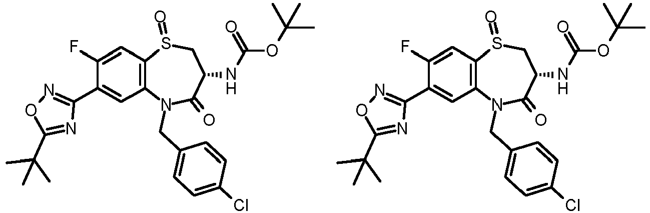

tert-butyl N-[(3R)-7-(5-tert-butyl-1,2,4-oxadiazol-3-yl)-5-[(4-chlorophenyl)methyl]-8-fluoro-4-oxo-2,3-dihydro-1,5-benzothiazepin-3-yl]carbamate (30 mg, 0.053 mmol, 1 eq) was stirred with 3-chloroperoxybenzoic acid (11.98 mg, 0.053 mmol, 1 eq) in DCM (1.07 mL) at room temperature for 3 h. The reaction solution was diluted with DCM and 1N NaOH and was extracted with DCM twice. The combined organic layers were washed with water and brine, dried over Na₂SO₄ and evaporated and the remaining residue was purified by column chromatography on silica gel (0% to 80% EtOAc in heptane) to afford tert-butyl N-[(3R)-7-(5-tert-butyl-1,2,4-oxadiazol-3-yl)-5-[(4-chlorophenyl)methyl]-8-fluoro-1,4-dioxo-2,3-dihydro-1λ⁴,5-benzothiazepin-3-yl]carbamate [Epimer A] (17 mg, 55%) as white solid (MS (ESI): 521.1 [M-isobutene+H]⁺) and tert-butyl N-[(3R)-7-(5-tert-butyl-1,2,4-oxadiazol-3-yl)-5-[(4-chlorophenyl)methyl]-8-fluoro-1,4-dioxo-2,3-dihydro-1λ⁴,5-benzothiazepin-3-yl]carbamate [Epimer B] (9 mg, 29%) as white solid (MS (ESI): 521.1 [M-isobutene+H]⁺).

### Step c) (3R)-3-amino-7-(5-tert-butyl-1,2,4-oxadiazol-3-yl)-5-[(4-chlorophenyl)methyl]-8-fluoro-1-oxo-2,3-dihydro-1λ⁴,5-benzothiazepin-4-one [Epimer A]

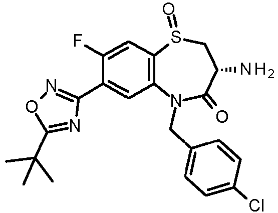

The title compound was prepared in analogy to general procedure **6d** from tert-butyl N-[(3R)-7-(5-tert-butyl-1,2,4-oxadiazol-3-yl)-5-[(4-chlorophenyl)methyl]-8-fluoro-1,4-dioxo-2,3-dihydro-1λ⁴,5-benzothiazepin-3-yl]carbamate [Epimer A] (17 mg, 0.029 mmol, 1 eq) and was obtained as hydrochloride salt as a white solid (14 mg, 93% yield). MS (ESI): 477.1 [M+H]⁺.

### Example 341

### (3R)-3-amino-7-(5-tert-butyl-1,2,4-oxadiazol-3-yl)-5-[(4-chlorophenyl)methyl]-8-fluoro-1-oxo-2,3-dihydro-1λ⁴,5-benzothiazepin-4-one [Epimer B]

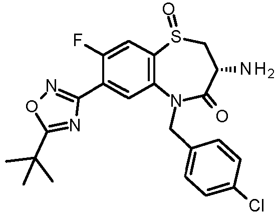

The title compound was prepared in analogy to general procedure **6d** from from tert-butyl N-[(3R)-7-(5-tert-butyl-1,2,4-oxadiazol-3-yl)-5-[(4-chlorophenyl)methyl]-8-fluoro-1,4-dioxo-2,3-dihydro-1λ⁴,5-benzothiazepin-3-yl]carbamate [Epimer B] (9 mg, 0.016 mmol, 1 eq) and was obtained as hydrochloride salt as a white solid (8 mg, 100% yield). MS (ESI): 477.1 [M+H]⁺.

### Example 342 and Example 343

### (3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1λ⁴,5-benzothiazepin-4-one [Epimer A at S] and (3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1λ⁴,5-benzothiazepin-4-one [Epimer B at S]

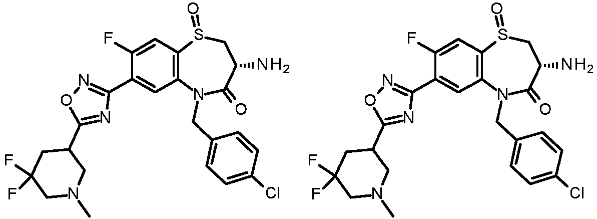

### Step a) benzyl 5-[3-[(3R)-3-(tert-butoxycarbonylamino)-5-[(4-chlorophenyl)methyl]-8-fluoro-1,4-dioxo-2,3-dihydro-1λ⁴,5-benzothiazepin-7-yl]-1,2,4-oxadiazol-5-yl]-3,3-difluoro-piperidine-1-carboxylate

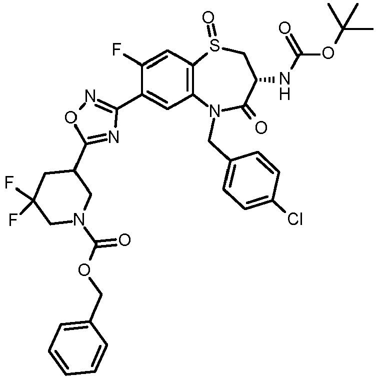

benzyl 5-[3-[(3R)-3-(tert-butoxycarbonylamino)-5-[(4-chlorophenyl)methyl]-8-fluoro-4-oxo-2,3-dihydro-1,5-benzothiazepin-7-yl]-1,2,4-oxadiazol-5-yl]-3,3-difluoro-piperidine-1-carboxylate (Example 323, step b)) (426 mg, 0.562 mmol, 1 eq) was stirred with 3-chloroperoxybenzoic acid (125.92 mg, 0.562 mmol, 1 eq) in DCM (6 mL) at room temperature for 1.5 h. The mixture was concentrated and the remaining residue was purified by column chromatography on silica gel (0% to 100% EtOAc in heptane) to afford the title compund (356 mg, 72% yield) as white foam. MS (ESI): 674.3 [M-isobutene-CO₂+H]⁺.

### Step b) tert-butylN-[(3R)-5-[(4-chlorophenyl)methyl]-7-[5-(5,5-difluoro-3-piperidyl)-1,2,4-oxadiazol-3-yl]-8-fluoro-1,4-dioxo-2, 3-dihydro-1λ⁴,5-benzothiazepin-3-yl]carbamate

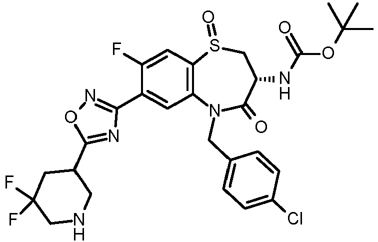

A mixture of benzyl 5-[3-[(3R)-3-(tert-butoxycarbonylamino)-5-[(4-chlorophenyl)methyl]-8-fluoro-1,4-dioxo-2,3-dihydro-1λ⁴,5-benzothiazepin-7-yl]-1,2,4-oxadiazol-5-yl]-3,3-difluoro-piperidine-1-carboxylate (356 mg, 0.46 mmol, 1 eq) and Pd/C 10% (48.9 mg, 0.046 mmol, 0.1 eq) in MeOH (10 mL) was stirred under an atmosphere of hydrogen for 2 days at room temperature. The catalyst was filtered off, the filtrate was concentrated and the remaining residue was purified by column chromatography on silica gel (first 0% to 100% EtOAc in heptane and then 0% to 10% MeOH in EtOAc) to afford the title compound (92 mg, 28% yield) as colorless oil. MS (ESI): 640.3 [M+H]⁺.

### Step c) tert-butylN-[(3R)-5-[(4-chlorophenyl)methyl]-7-[5-(5, 5-difluoro-1-methyl-3-piperidyl)-1,2,4-oxadiazol-3-yl]-8-fluoro-1,4-dioxo-2,3-dihydro-1λ⁴,5-benzothiazepin-3-yl]carbamate

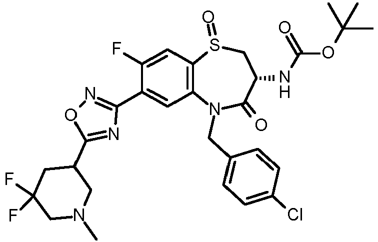

A mixture of tert-butyl N-[(3R)-5-[(4-chlorophenyl)methyl]-7-[5-(5,5-difluoro-3-piperidyl)-1,2,4-oxadiazol-3-yl]-8-fluoro-1,4-dioxo-2,3-dihydro-1λ⁴,5-benzothiazepin-3-yl]carbamate (92 mg, 0.144 mmol, 1 eq), formaldehyde (37% in water, 116.66 mg, 107.03 µL, 1.44 mmol, 10 eq) and sodium triacetoxyborohydride (304.63 mg, 1.44 mmol, 10 eq) in MeOH (3 mL) was stirred for 1 h at room temperature. A small amount of water was added and the material was absorped on silica gel and then purified by column chromatography on silica gel (0% to 100% EtOAc in heptane) to afford the title compound (68 mg, 67% yield) as colorless oil. MS (ESI): 654.3 [M+H]⁺.

### Step d) (3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1λ ⁴,5-benzothiazepin-4-one [Epimer A at S] and (3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1λ ⁴,5-benzothiazepin-4-one [Epimer B at S]

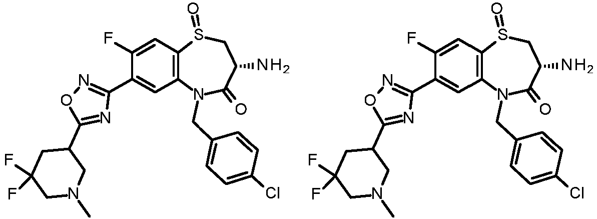

A mixture of tert-butyl N-[(3R)-5-[(4-chlorophenyl)methyl]-7-[5-(5,5-difluoro-1-methyl-3-piperidyl)-1,2,4-oxadiazol-3-yl]-8-fluoro-1,4-dioxo-2,3-dihydro-1λ⁴,5-benzothiazepin-3-yl]carbamate (25 mg, 0.038 mmol, 1 eq) and 4M HCl in dioxane (5 drops) was stirred in 1,1,1,3,3,3-hexafluoro-2-propanol (1 mL) at room temperature for 30 min. The solvent was evaporated and the crude material submitted to preparative HPLC affording (3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1λ⁴,5-benzothiazepin-4-one [Epimer A at S] (7.3 mg, 35% yield) as white solid (MS (ESI): 554.3 [M+H]⁺) and (3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1λ⁴,5-benzothiazepin-4-one [Epimer B at S] (9.3 mg, 44% yield) as white solid (MS (ESI): 554.3 [M+H]⁺).

### Example 344

### (3R)-3-amino-8-fluoro-1,1-dioxo-5-[[4-(trifluoromethoxy)phenyl]methyl]-7-[5-[1-(trifluoromethyl)cyclopropyl]-1,2,4-oxadiazol-3-yl]-2,3-dihydro-1λ⁶,5-benzothiazepin-4-one

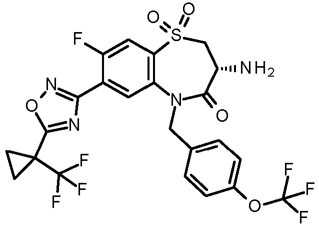

### Step a) [(Z)-[amino-[(3R)-3-(tert-butoxycarbonylamino)-8-fluoro-1,1,4-trioxo-5-[[4-(trifluoromethoxy)phenyl]methyl]-2,3-dihydro-1λ ⁶,5-benzothiazepin-7-yl]methylene]amino]1-(trifluoromethyl)cyclopropanecarboxylate

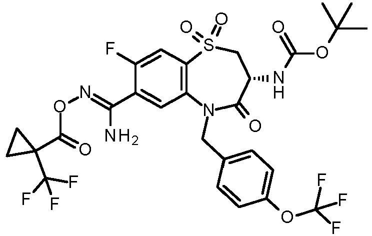

The title compound was prepared in analogy to general procedure **10a** from tert-butyl N-[(3R)-8-fluoro-7-[(Z)-N'-hydroxycarbamimidoyl]-1,1,4-trioxo-5-[[4-(trifluoromethoxy)phenyl]methyl]-2,3-dihydro-1λ⁶,5-benzothiazepin-3-yl]carbamate (Example 331, step a)) (30 mg, 0.052 mmol, 1 eq) and 1-(trifluoromethyl)cyclopropanecarboxylic acid (CAS 277756-46-4) and was obtained as a yellow solid (59.1 mg, 96% yield). MS (ESI): 657.3 [M-isobutene+H]⁺.

### Step b) (3R)-3-amino-8-fluoro-1,1-dioxo-5-[[4-(trifluoromethoxy)phenyl]methyl]-7-[5-[1-(trifluoromethyl)cyclopropyl]-1,2,4-oxadiazol-3-yl]-2,3-dihydro-1λ ⁶,5-benzothiazepin-4-one

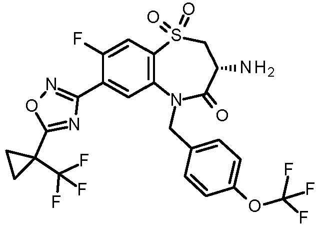

[(Z)-[amino-[(3R)-3-(tert-butoxycarbonylamino)-8-fluoro-1,1,4-trioxo-5-[[4-(trifluoromethoxy)phenyl]methyl]-2,3-dihydro-1λ⁶, 5-benzothiazepin-7-yl]methylene]amino] 1-(trifluoromethyl)cyclopropanecarboxylate (59 mg, 0.083 mmol, 1 eq) was dissolved in a solvent mixture of toluene (3 mL) and THF (0.5 mL) and the mixture was heated to 100°C and stirred for 16 h. The solution was concentrated under vacuum and the remaining residue was purified by preparative HPLC affording the title compound (48.8 mg, 99.15%) as white solid. MS (ESI): 595.3 [M+H]⁺.

### Example 345

### (3R)-3-amino-7-[5-(5,5-difluoro-1-methyl-3-piperidyl)-1,2,4-oxadiazol-3-yl]-8-fluoro-5-[(4-isopropoxyphenyl)methyl]-1,1-dioxo-2,3-dihydro-1λ⁶,5-benzothiazepin-4-one

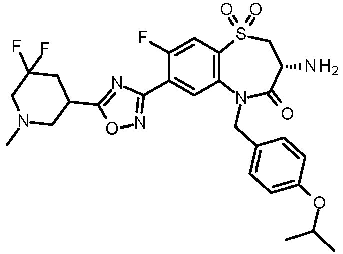

### Step a) benzyl 5-[3-[(3R)-3-(tert-butoxycarbonylamino)-8-fluoro-4-oxo-3, 5-dihydro-2H-1,5-benzothiazepin-7-yl]-1,2,4-oxadiazol-5-yl]-3,3-difluoro-piperidine-1-carboxylate

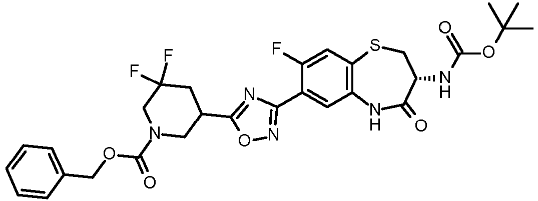

The title compound was prepared in analogy to general procedure **9a** from tert-butyl N-[(3R)-8-fluoro-7-[(Z)-N'-hydroxycarbamimidoyl]-4-oxo-3,5-dihydro-2H-1,5-benzothiazepin-3-yl]carbamate (Example 321, step d)) (268 mg, 0.615 mmol, 1 eq) and 1-benzyloxycarbonyl-5,5-difluoro-piperidine-3-carboxylic acid (CAS 1356338-81-2) and was obtained as a light yellow solid (256 mg, 92% yield). MS (ESI): 578.2 [M-isobutene+H]⁺.

### Step b) benzyl 5-[3-[(3R)-3-(tert-butoxycarbonylamino)-8-fluoro-1,1,4-trioxo-3,5-dihydro-2H-1λ ⁶,5-benzothiazepin-7-yl]-1,2,4-oxadiazol-5-yl]-3,3-difluoro-piperidine-1-carboxylate

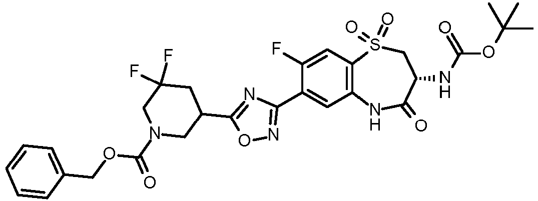

The title compound was prepared in analogy to general procedure **5** from benzyl 5-[3-[(3R)-3-(tert-butoxycarbonylamino)-8-fluoro-4-oxo-3,5-dihydro-2H-1,5-benzothiazepin-7-yl]-1,2,4-oxadiazol-5-yl]-3,3-difluoro-piperidine-1-carboxylate (254 mg, 0.381 mmol, 1 eq) and was obtained as an off-white solid (219 mg, 86% yield). MS (ESI): 610.3 [M-isobutene+H]⁺.

### Step c) tert-butyl N-[(3R)-7-[5-(5,5-difluoro-3-piperidyl)-1,2,4-oxadiazol-3-yl]-8-fluoro-1,1,4-trioxo-3,5-dihydro-2H-1λ ⁶,5-benzothiazepin-3-yl]carbamate

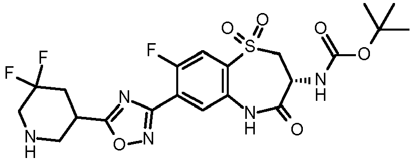

benzyl 5-[3-[(3R)-3-(tert-butoxycarbonylamino)-8-fluoro-1,1,4-trioxo-3,5-dihydro-2H-1λ⁶,5-benzothiazepin-7-yl]-1,2,4-oxadiazol-5-yl]-3,3 -difluoro-piperidine-1-carboxylate (219 mg, 0.329 mmol, 1 eq) was dissolved in MeOH (10 mL) at room temperature. Pd/C (50 mg, 0.047 mmol, 0.143 eq) was added and the reaction mixture was stirred under an atmosphere of hydrogen for 2 h. The mixture was filtered over a pad of celite and the celite pad was washed throroughly with MeOH. The filtrate was concentrated to afford the title compound (179 mg, 60% yield) as white solid. MS (ESI): 476.1 [M-isobutene+H]⁺.

### Step d) tert-butyl N-[(3R)-7-[5-(5,5-difluoro-1-methyl-3-piperidyl)-1,2,4-oxadiazol-3-yl]-8-fluoro-1,1,4-trioxo-3, 5-dihydro-2H-1λ⁶,5-benzothiazepin-3-yl]carbamate

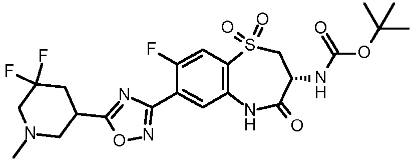

A mixture of tert-butyl N-[(3R)-7-[5-(5,5-difluoro-3-piperidyl)-1,2,4-oxadiazol-3-yl]-8-fluoro-1,1,4-trioxo-3,5-dihydro-2H-1λ⁶,5-benzothiazepin-3-yl]carbamate (175 mg, 0.194 mmol, 1 eq), formaldehyde (37% in water, 157.66 mg, 144.65 µL, 1.94 mmol, 10 eq) and sodium triacetoxyborohydride (411.71 mg, 1.94 mmol, 10 eq) in MeOH (4 mL) was stirred for 1h at room temperature. A small amount of water was added and the material was absorped on silica gel and then purified by column chromatography on silica gel (50% to100% EtOAc in heptane) to afford the title compound (131 mg, 96% yield) as white solid. MS (ESI): 546.3 [M+H]⁺.

### Step e) tert-butyl N-[(3R)-7-[5-(5,5-difluoro-1-methyl-3-piperidyl)-1,2,4-oxadiazol-3-yl]-8-fluoro-5-[(4-isopropoxyphenyl)methyl]-1,1,4-trioxo-2, 3-dihydro-1λ⁶,5-benzothiazepin-3-yl]carbamate

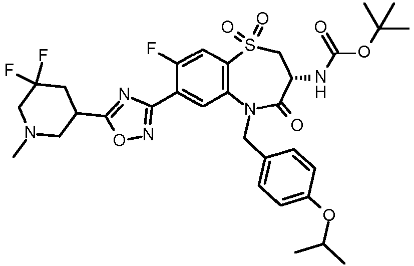

The title compound was prepared in analogy to general procedure **4** from tert-butyl N-[(3R)-7-[5-(5,5-difluoro-1-methyl-3-piperidyl)-1,2,4-oxadiazol-3-yl]-8-fluoro-1,1,4-trioxo-3,5-dihydro-2H-1λ⁶,5-benzothiazepin-3-yl]carbamate (40 mg, 0.085 mmol, 1 eq) and (4-isopropoxyphenyl)methyl methanesulfonate (CAS 329045-48-9) and was obtained as a white solid (19 mg, 48% yield). MS (ESI): 694.6 [M+H]⁺.

### Step f) (3R)-3-amino-7-[5-(5,5-difluoro-1-methyl-3-piperidyl)-1,2,4-oxadiazol-3-yl]-8-fluoro-5-[(4-isopropoxyphenyl)methyl]-1,1-dioxo-2,3-dihydro-1λ⁶,5-benzothiazepin-4-one

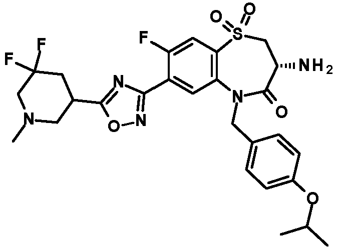

The title compound was prepared in analogy to general procedure **6d** from tert-butyl N-[(3R)-7-[5-(5,5-difluoro-1-methyl-3-piperidyl)-1,2,4-oxadiazol-3-yl]-8-fluoro-5-[(4-isopropoxyphenyl)methyl]-1,1,4-trioxo-2,3-dihydro-1λ⁶,5-benzothiazepin-3-yl]carbamate (19 mg, 0.027 mmol, 1 eq) and was obtained as a hydrochloride salt as a white solid (12.5 mg, 72% yield). MS (ESI): 594.2 [M+H]⁺.

The examples 346 and 347 of the following table were prepared in analogy to Example 345, using the appropriate benzyl halide building block.

| **Ex.** | **Structure** | **Systematic Name** | **Building Block** | **MS, ESI: m/z** |
|---|---|---|---|---|
| 346 | 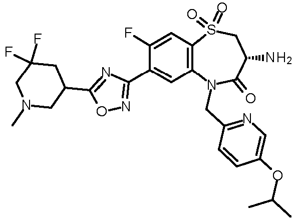 | (3R)-3-amino-7-[5-(5,5-difluoro-1-methyl-3-piperidyl)-1,2,4-oxadiazol-3-yl]-8-fluoro-5-[(5-isopropoxy-2-pyridyl)methyl]-1,1-dioxo-2,3-dihydro-1λ⁶,5-benzothiazepin-4-one | 2-(Chlorome thyl)-5-isopropoxy -pyridine | 595.3 [M+H]⁺ |
| | | | (CAS 329045-48-9) | |
| | | (**) | | |
| 347 | 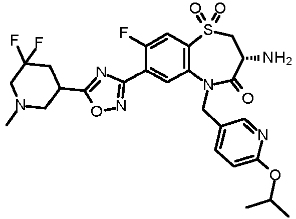 | (3R)-3-amino-7-[5-(5,5-difluoro-1-methyl-3-piperidyl)-1,2,4-oxadiazol-3-yl]-8-fluoro-5-[(6-isopropoxy-3-pyridyl)methyl]-1,1-dioxo-2,3-dihydro-1λ⁶,5-benzothiazepin-4-one | 5-(Chlorome thyl)-2-isopropoxy -pyridine | 595.2 [M+H]⁺ |
| | | | (CAS 1247496-01-0) | |
| | | (*) | | |

| | | | | |
|---|---|---|---|---|
| * as hydrochloride salt ** as dihydrochloride salt | | | | |

### Example 348

### methyl 3-[3-[(3R)-3-amino-5-[[4-(cyclopentoxy)phenyl]methyl]-8-fluoro-1,1,4-trioxo-2,3-dihydro-1λ⁶,5-benzothiazepin-7-yl]-1,2,4-oxadiazol-5-yl]pyrrolidine-1-carboxylate

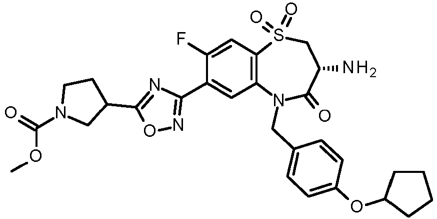

### Step a) tert-butyl N-[(3R)-7-cyano-5-[[4-(cyclopentoxy)phenyl[methyl]-8-fluoro-4-oxo-2,3-dihydro-1,5-benzothiazepin-3-yl]carbamate

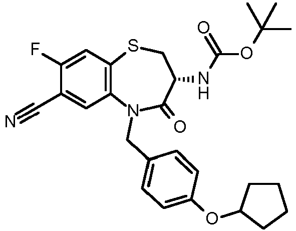

The title compound was prepared in analogy to general procedure 4 from tert-butyl N-[(3R)-7-cyano-8-fluoro-4-oxo-3,5-dihydro-2H-1,5-benzothiazepin-3-yl]carbamate (Example 321, step c)) (180 mg, 0.534 mmol, 1 eq) and 1-(chloromethyl)-4-(cyclopentoxy)benzene (CAS 1041579-01-4) and was obtained as a white solid (172 mg, 57% yield). MS (ESI): 510.4 [M-H]⁻.

### Step b) tert-butyl N-[(3R)-5-[[4-(cyclopentoxy)phenyl]methyl]-8-fluoro-7-(N-hydroxycarbamimidoyl)-4-oxo-2,3-dihydro-1,5-benzothiazepin-3-yl]carbamate

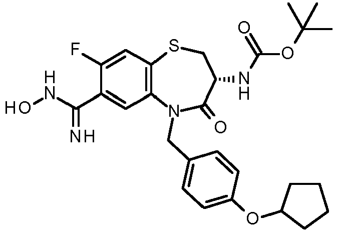

The title compound was prepared in analogy to general procedure **12** from tert-butyl N-[(3R)-7-cyano-5-[[4-(cyclopentoxy)phenyl]methyl]-8-fluoro-4-oxo-2,3-dihydro-1,5-benzothiazepin-3-yl]carbamate (172 mg, 0.306 mmol, 1 eq) and was obtained as a white solid (182 mg, 96% yield). MS (ESI): 545.5 [M+H]⁺.

### Step c) benzyl 3-[3-[(3R)-3-(tert-butoxycarbonylamino)-5-[[4-(cyclopentoxy)phenyl]methyl]-8-fluoro-4-oxo-2,3-dihydro-1,5-benzothiazepin-7-yl]-1,2,4-oxadiazol-5-yl]pyrrolidine-1-carboxylate

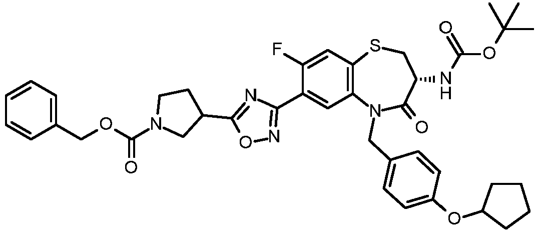

The title compound was prepared in analogy to general procedure **9a** from tert-butyl N-[(3R)-5-[[4-(cyclopentoxy)phenyl]methyl]-8-fluoro-7-(N-hydroxycarbamimidoyl)-4-oxo-2,3-dihydro-1,5-benzothiazepin-3-yl]carbamate (60 mg, 0.097 mmol, 1 eq) and 1-benzyloxycarbonylpyrrolidine-3-carboxylic acid (CAS 188527-21-1) and was obtained as a white foam (53 mg, 71% yield). MS (ESI): 658.5 [M-isobutene-CO₂+H]⁺.

### Step d) benzyl 3-[3-[(3R)-3-(tert-butoxycarbonylamino)-5-[[4-(cyclopentoxy)phenyl]methyl]-8-fluoro-1,1,4-trioxo-2, 3-dihydro-1λ ⁶,5-benzothiazepin-7-yl]-1,2, 4-oxadiazol-5-yl]pyrrolidine-1-carboxylate

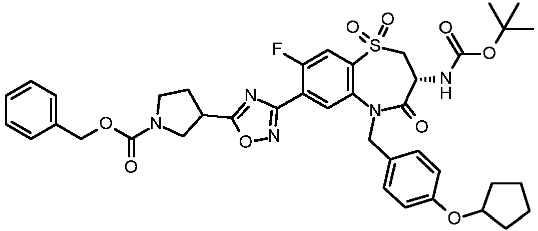

The title compound was prepared in analogy to general procedure **5** from benzyl 3-[3-[(3R)-3-(tert-butoxycarbonylamino)-5-[[4-(cyclopentoxy)phenyl]methyl]-8-fluoro-4-oxo-2,3-dihydro-1,5-benzothiazepin-7-yl]-1,2,4-oxadiazol-5-yl]pyrrolidine-1-carboxylate (53 mg, 0.069 mmol, 1 eq) and was obtained as a white solid (30 mg, 53% yield). MS (ESI): 690.5 [M-isobutene-CO₂+H]⁺.

### Step e) tert-butyl N-[(3R)-5-[[4-(cyclopentoxy)phenyl]methyl]-8-fluoro-1,1,4-trioxo-7-(5-pyrrolidin-3-yl-1,2,4-oxadiazol-3-yl)-2,3-dihydro-1λ⁶,5-benzothiazepin-3-yl]carbamate

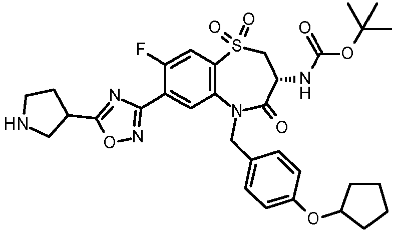

benzyl 3-[3-[(3R)-3-(tert-butoxycarbonylamino)-5-[[4-(cyclopentoxy)phenyl]methyl]-8-fluoro-1,1,4-trioxo-2,3-dihydro-1λ⁶,5-benzothiazepin-7-yl]-1,2,4-oxadiazol-5-yl]pyrrolidine-1-carboxylate (30 mg, 0.036 mmol, 1 eq) was suspended in MeOH (3 mL) and argon was bubbled through the mixture for 5 min. Then Pd/C (3.88 mg, 0.004 mmol, 0.1 eq) was added and the reaction mixture was stirred under an atmosphere of hydrogen at room temperature for 2 h. The reaction mixture was filtered over Dicalite and the Dicalite was washed thoroughly with MeOH. The filtrate was concentrated to afford a light yellow solid (31 mg) containing the title compound. MS (ESI): 656.6 [M+H]⁺.

### Step f) methyl 3-[3-[(3R)-3-(tert-butoxycarbonylamino)-5-[[4-(cyclopentoxy)phenyl]methyl]-8-fluoro-1,1,4-trioxo-2, 3-dihydro-1λ ⁶,5-benzothiazepin-7-yl]-1,2, 4-oxadiazol-5-yl]pyrrolidine-1-carboxylate

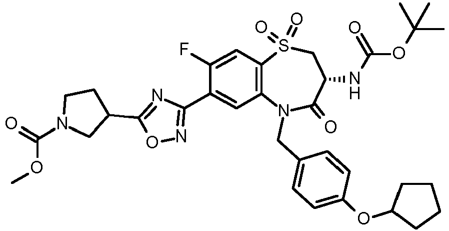

A mixture of tert-butyl N-[(3R)-5-[[4-(cyclopentoxy)phenyl]methyl]-8-fluoro-1,1,4-trioxo-7-(5-pyrrolidin-3-yl-1,2,4-oxadiazol-3-yl)-2,3-dihydro-1λ⁶, 5-benzothiazepin-3-yl]carbamate (31 mg, 0.038 mmol, 1 eq), methyl chloroformate (3.93 mg, 3.22 µL, 0.042 mmol, 1.1 eq) and DIPEA (12.22 mg, 16.51 µL, 0.095 mmol, 2.5 eq) in DCM (1 mL) was stirred at room temperature for 2 h. The solvent was evaporated and the remaining residue was purified by column chromatography on silica gel (0% to100% EtOAc in heptane) to afford the title compound (15 mg, 53%) as white solid. MS (ESI): 614.4 [M-isobutene-CO₂+H]⁺.

### Step g) methyl 3-[3-[(3R)-3-amino-5-[[4-(cyclopentoxy)phenyl]methyl]-8-fluoro-1,1,4-trioxo-2,3-dihydro-1λ⁶,5-benzothiazepin-7-yl]-1,2,4-oxadiazol-5-yl]pyrrolidine-1-carboxylate

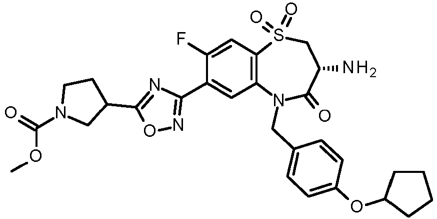

The title compound was prepared in analogy to general procedure 6d from methyl 3-[3-[(3R)-3-(tert-butoxycarbonylamino)-5-[[4-(cyclopentoxy)phenyl]methyl]-8-fluoro-1,1,4-trioxo-2,3-dihydro-1λ⁶,5-benzothiazepin-7-yl]-1,2,4-oxadiazol-5-yl]pyrrolidine-1-carboxylate (15 mg, 0.020 mmol, 1 eq) and was obtained as a white solid (8 mg, 64% yield). MS (ESI): 614.5 [M+H]⁺.

The example 349 of the following table was prepared in analogy to Example 348, using the appropriate benzyl mesylate building block.

| **Ex.** | **Structure** | **Systematic Name** | **Building Block** | **MS, ESI: m/z** |
|---|---|---|---|---|
| 349 | 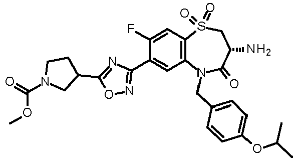 | methyl 3-[3-[(3R)-3-amino-8-fluoro-5-[(4-isopropoxyphenyl)methyl]-1,1,4-trioxo-2,3-dihydro-1λ⁶,5-benzothiazepin-7-yl]-1,2,4-oxadiazol-5-yl]pyrrolidine-1-carboxylate (*) | (4-Isopropoxy phenyl)me thyl methanesu lfonate (CAS 329045-48-9) | 588.6 [M+H]⁺ |

| | | | | |
|---|---|---|---|---|
| * as hydrochloride salt | | | | |

### Example 350

### (3R)-3-amino-7-(5-tert-butyl-1,2,4-oxadiazol-3-yl)-5-[[4-(cyclopentoxy)phenyl]methyl]-8-fluoro-1,1-dioxo-2,3-dihydro-1λ⁶,5-benzothiazepin-4-one

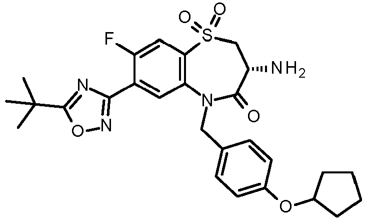

### Step a) tert-butyl N-[(3R)-7-(5-tert-butyl-1,2,4-oxadiazol-3-yl)-5-[[4-(cyclopentoxy)phenyl]methyl]-8-fluoro-4-oxo-2,3-dihydro-1,5-benzothiazepin-3-yl]carbamate

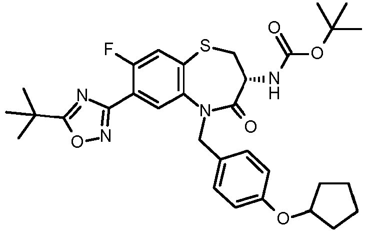

The title compound was prepared in analogy to general procedure **9a** from tert-butyl N-[(3R)-5-[[4-(cyclopentoxy)phenyl]methyl]-8-fluoro-7-(N-hydroxycarbamimidoyl)-4-oxo-2,3-dihydro-1,5-benzothiazepin-3-yl]carbamate (Example 348, step b)) (50 mg, 0.081 mmol, 1 eq) and 2,2-dimethylpropanoic acid (CAS 75-98-9) and was obtained as a white foam (41 mg, 74% yield). MS (ESI): 555.5 [M-isobutene+H]⁺.

### Step b) tert-butylN-[(3R)-7-(5-tert-butyl-1,2,4-oxadiazol-3-yl)-5-[[4-(cyclopentoxy)phenyl]methyl]-8-fluoro-1,1,4-trioxo-2,3-dihydro-1λ ⁶,5-benzothiazepin-3-yl]carbamate

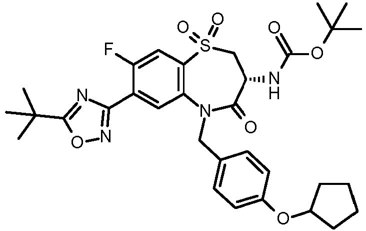

The title compound was prepared in analogy to general procedure **5** from tert-butyl N-[(3R)-7-(5-tert-butyl-1,2,4-oxadiazol-3-yl)-5-[[4-(cyclopentoxy)phenyl]methyl]-8-fluoro-4-oxo-2,3-dihydro-1,5-benzothiazepin-3-yl]carbamate (41 mg, 0.060 mmol, 1 eq) and was obtained as a white solid (32 mg, 75% yield). MS (ESI): 641.4 [M-H]⁻.

### Step c) (3R)-3-amino-7-(5-tert-butyl-1,2,4-oxadiazol-3-yl)-5-[[4-(cyclopentoxy)phenyl]methyl]-8-fluoro-1,1-dioxo-2, 3-dihydro-1λ ⁶,5-benzothiazepin-4-one

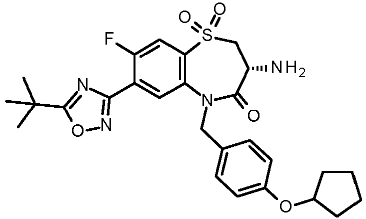

The title compound was prepared in analogy to general procedure **6d** from tert-butyl N-[(3R)-7-(5-tert-butyl-1,2,4-oxadiazol-3-yl)-5-[[4-(cyclopentoxy)phenyl]methyl]-8-fluoro-1,1,4-trioxo-2,3-dihydro-1λ⁶,5-benzothiazepin-3-yl]carbamate (32 mg, 0.045 mmol, 1 eq) and was obtained as hydrochloride salt as a white solid (29 mg). MS (ESI): 543.5 [M+H]⁺.

### Example 351

### (3R)-3-amino-5-benzyl-8-fluoro-1,1-dioxo-7-[5-(1,2,2,2-tetrafluoro-1-methoxy-ethyl)-1,2,4-oxadiazol-3-yl]-2,3-dihydro-1λ⁶,5-benzothiazepin-4-one

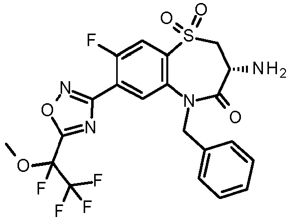

### Step a) tert-butyl N-[(3R)-5-benzyl-7-cyano-8-fluoro-4-oxo-2,3-dihydro-1,5-benzothiazepin-3-yl]carbamate

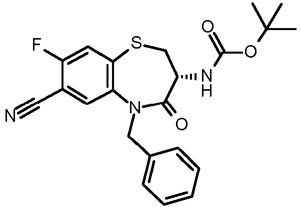

The title compound was prepared in analogy to general procedure **4** from tert-butyl N-[(3R)-7-cyano-8-fluoro-4-oxo-3,5-dihydro-2H-1,5-benzothiazepin-3-yl]carbamate (Example 321, step c)) (250 mg, 0.741 mmol, 1 eq) and bromomethylbenzene (CAS 100-39-0) and was obtained as a light yellow solid (289 mg, 77% yield). MS (ESI): 472.2 [M-isobutene+H]⁺.

### Step b) tert-butyl N-[(3R)-5-benzyl-8-fluoro-7-(N-hydroxycarbamimidoyl)-4-oxo-2,3-dihydro-1,5-benzothiazepin-3-yl]carbamate

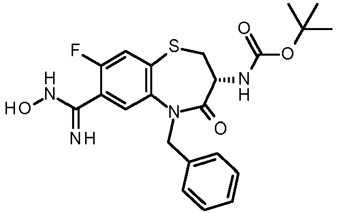

The title compound was prepared in analogy to general procedure **12** from tert-butyl N-[(3R)-5-benzyl-7-cyano-8-fluoro-4-oxo-2,3-dihydro-1,5-benzothiazepin-3-yl]carbamate (289 mg, 0.676 mmol, 1 eq) and was obtained as a light yellow solid (266 mg, 76% yield). MS (ESI): 461.3 [M+H]⁺.

### Step c) tert-butyl N-[(3R)-5-benzyl-8-fluoro-4-oxo-7-[5-(1,2,2,2-tetrafluoro-1-methoxy-ethyl)-1,2,4-oxadiazol-3-yl]-2,3-dihydro-1,5-benzothiazepin-3-yl]carbamate

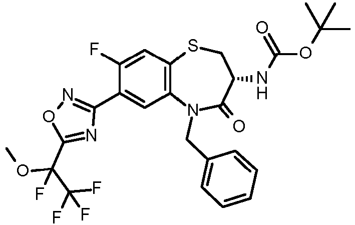

The title compound was prepared in analogy to general procedure 9a from tert-butyl N-[(3R)-5-benzyl-8-fluoro-7-(N-hydroxycarbamimidoyl)-4-oxo-2,3-dihydro-1,5-benzothiazepin-3-yl]carbamate (201 mg, 0.436 mmol, 1 eq) and 2,3,3,3-tetrafluoro-2-methoxy-propanoic acid (CAS 10186-64-8) and was obtained as a white solid (26 mg, 8% yield). MS (ESI): 545.3 [M-isobutene+H]⁺.

### Step d) tert-butyl N-[(3R)-5-benzyl-8-fluoro-1,1,4-trioxo-7-[5-(1,2,2,2-tetrafluoro-1-methoxyethyl)-1,2,4-oxadiazol-3-yl]-2, 3-dihydro-1λ ⁶, 5-benzothiazepin-3-yl]carbamate

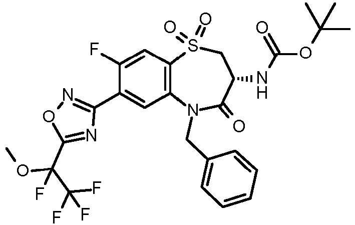

The title compound was prepared in analogy to general procedure **5** from tert-butyl N-[(3R)-5-benzyl-8-fluoro-4-oxo-7-[5-(1,2,2,2-tetrafluoro-1-methoxy-ethyl)-1,2,4-oxadiazol-3-yl]-2,3-dihydro-1,5-benzothiazepin-3-yl]carbamate (26 mg, 0.043 mmol, 1 eq) and was obtained as a white solid (19 mg, 53 % yield). MS (ESI): 631.3 [M-H]⁻.

### Step e) (3R)-3-amino-5-benzyl-8-fluoro-1,1-dioxo-7-[5-(1,2,2,2-tetrafluoro-1-methoxy-ethyl)-1,2,4-oxadiazol-3-yl]-2,3-dihydro-1λ ⁶,5-benzothiazepin-4-one

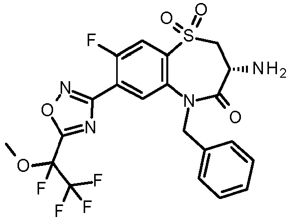

The title compound was prepared in analogy to general procedure 6d from tert-butyl N-[(3R)-5-benzyl-8-fluoro-1,1,4-trioxo-7-[5-(1,2,2,2-tetrafluoro-1-methoxy-ethyl)-1,2,4-oxadiazol-3-yl]-2,3-dihydro-1λ⁶,5-benzothiazepin-3-yl]carbamate (14 mg, 0.022 mmol, 1 eq) and was obtained as hydrochloride salt as a white solid (8.6 mg, 62 % yield). MS (ESI): 533.3 [M+H]⁺.

### Example 352

### 3-[(3R)-3-amino-8-fluoro-1,1,4-trioxo-5-[[4-(1,1,2,2-tetrafluoroethoxy)phenyl]methyl]-2,3-dihydro-1λ⁶,5-benzothiazepin-7-yl]-N,N-dimethyl-1,2,4-oxadiazole-5-carboxamide

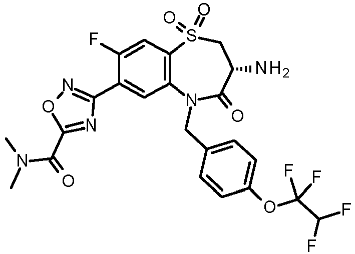

### Step a) tert-butyl N-[(3R)-7-cyano-8-fluoro-4-oxo-5-[[4-(1,1,2,2-tetrafluoroethoxy)phenyl]methyl]-2,3-dihydro-1,5-benzothiazepin-3-yl]carbamate

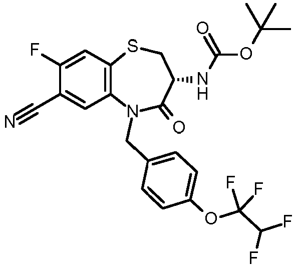

The title compound was prepared in analogy to general procedure **4** from tert-butyl N-[(3R)-7-cyano-8-fluoro-4-oxo-3,5-dihydro-2H-1,5-benzothiazepin-3-yl]carbamate (Example 321, step c)) (200 mg, 0.593 mmol, 1 eq) and 1-(bromomethyl)-4-(1,1,2,2-tetrafluoroethoxy)benzene (CAS 67033-41-4) and was obtained as a white solid (261 mg, 77% yield). MS (ESI): 542.1 [M-H]⁻.

### Step b) tert-butyl N-[(3R)-8-fluoro-7-(hydrazinecarboximidoyl)-4-oxo-5-[[4-(1,1,2,2-tetrafluoroethoxy)phenyl]methyl]-2,3-dihydro-1,5-benzothiazepin-3-yl]carbamate

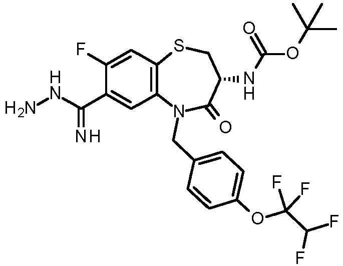

The title compound was prepared in analogy to general procedure **12** from tert-butyl N-[(3R)-7-cyano-8-fluoro-4-oxo-5-[[4-(1,1,2,2-tetrafluoroethoxy)phenyl]methyl]-2,3-dihydro-1,5-benzothiazepin-3-yl]carbamate (261 mg, 0.480 mmol, 1 eq) and was obtained as a white solid (284 mg, 92% yield). MS (ESI): 577.4 [M+H]⁺.

### Step c) tert-butylN-[(3R)-7-[5-(dimethylcarbamoyl)-1,2,4-oxadiazol-3yl]-8-fluoro-4-oxo-5-[[4-(1,1,2,2-tetrafluoroethoxy)phenyl]methyl]-2,3-dihydro-1,5-benzothiazepin-3-yl]carbamate

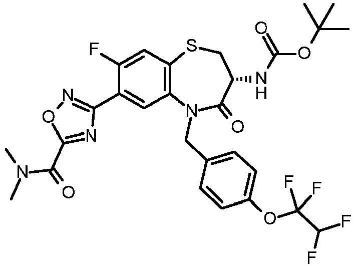

The title compound was prepared in analogy to general procedure **9c** from tert-butyl N-[(3R)-8-fluoro-7-(hydrazinecarboximidoyl)-4-oxo-5-[[4-(1,1,2,2-tetrafluoroethoxy)phenyl]methyl]-2,3-dihydro-1,5-benzothiazepin-3-yl]carbamate (125 mg, 0.217 mmol, 1 eq) and 2-(dimethylamino)-2-oxo-acetic acid (CAS 32833-96-8) and was obtained as a light yellow solid (22.7 mg, 15% yield). MS (ESI): 558.2 [M-isobutene-CO₂+H]⁺.

### Step d) tert-butyl N-[(3R)-7-[5-(dimethylcarbamoyl)-1,2,4-oxadiazol-3-yl]-8-fluoro-1,1,4-trioxo-5-[[4-(1,1,2,2-tetrafluoroethoxy)phenyl]methyl]-2,3-dihydro-1λ ⁶,5-benzothiazepin-3-yl]carbamate

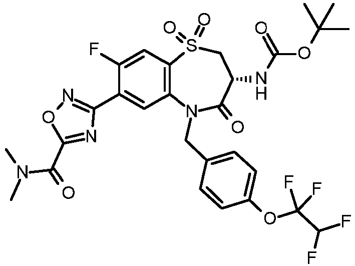

The title compound was prepared in analogy to general procedure **5** from tert-butyl N-[(3R)-7-[5-(dimethylcarbamoyl)-1,2,4-oxadiazol-3-yl]-8-fluoro-4-oxo-5-[[4-(1,1,2,2-tetrafluoroethoxy)phenyl]methyl]-2,3-dihydro-1,5-benzothiazepin-3-yl]carbamate (22.7 mg, 0.035 mmol, 1 eq) and was obtained as a white solid (17.4 mg, 72% yield). MS (ESI): 688.2 [M-H]⁻.

### Step e) 3-[(3R)-3-amino-8-fluoro-1,1,4-trioxo-5-[[4-(1,1,2,2-tetrafluoroethoxy)phenyl]methyl]-2, 3-dihydro-1λ ⁶,5-benzothiazepin-7-yl]-N,N-dimethyl-1,2,4-oxadiazole-5-carboxamide

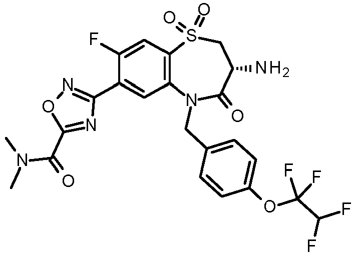

The title compound was prepared in analogy to general procedure **6d** from tert-butyl N-[(3R)-7-[5-(dimethylcarbamoyl)-1,2,4-oxadiazol-3-yl]-8-fluoro-1,1,4-trioxo-5-[[4-(1,1,2,2-tetrafluoroethoxy)phenyl]methyl]-2,3-dihydro-1λ⁶,5-benzothiazepin-3-yl]carbamate (17.4 mg, 0.025 mmol, 1 eq) and was obtained as hydrochloride salt as an off-white solid (13 mg, 82% yield). MS (ESI): 590.3 [M+H]⁺.

### Example 353

### (3R)-3-amino-7-(5-tert-butyl-1,2,4-oxadiazol-3-yl)-8-fluoro-1,1-dioxo-5-[[4-(tetrahydropyran-4-ylmethoxy)phenyl]methyl]-2,3-dihydro-1λ⁶,5-benzothiazepin-4-one

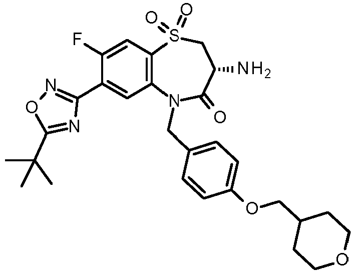

### Step a) tert-butyl N-[(3R)-7-cyano-8-fluoro-4-oxo-5-[[4-(tetrahydropyran-4-ylmethoxy)phenyl]methyl]-2,3-dihydro-1,5-benzothiazepin-3-yl]carbamate

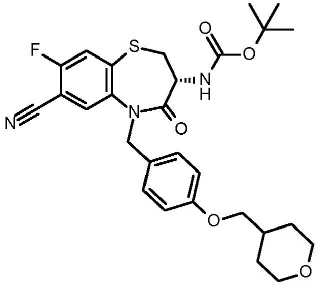

The title compound was prepared in analogy to general procedure **4** from tert-butyl N-[(3R)-7-cyano-8-fluoro-4-oxo-3,5-dihydro-2H-1,5-benzothiazepin-3-yl]carbamate (Example 321, step c)) (200 mg, 0.593 mmol, 1 eq) and 4-[[4-(chloromethyl)phenoxy]methyl]tetrahydropyran (CAS 1248950-37-9) and was obtained as a white solid (290 mg, 87% yield). MS (ESI): 564.1 [M+Na]⁺.

### Step b) tert-butyl N-[(3R)-8-fluoro-7-(N-hydroxycarbamimidoyl)-4-oxo-5-[[4-(tetrahydropyran-4-ylmethoxy)phenyl]methyl]-2,3-dihydro-1,5-benzothiazepin-3-yl]carbamate

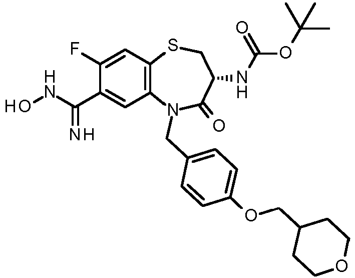

The title compound was prepared in analogy to general procedure 12 from tert-butyl N-[(3R)-7-cyano-8-fluoro-4-oxo-5-[[4-(tetrahydropyran-4-ylmethoxy)phenyl]methyl]-2,3-dihydro-1,5-benzothiazepin-3-yl]carbamate (220 mg, 0.408 mmol, 1 eq) and was obtained as a light yellow oil (230 mg, 99% yield). MS (ESI): 575.1 [M+H]⁺.

### Step c) tert-butyl N-[(3R)-7-(5-tert-butyl-1,2,4-oxadiazol-3-yl)-8-fluoro-4-oxo-5-[[4-(tetrahydropyran-4-ylmethoxy)phenyl]methyl]-2,3-dihydro-1,5-benzothiazepin-3-yl]carbamate

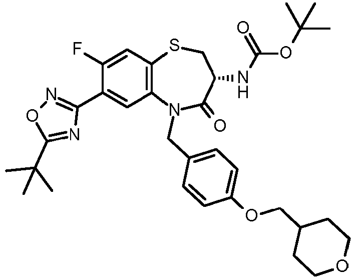

To a mixture of tert-butyl N-[(3R)-8-fluoro-7-(N-hydroxycarbamimidoyl)-4-oxo-5-[[4-(tetrahydropyran-4-ylmethoxy)phenyl]methyl]-2,3-dihydro-1,5-benzothiazepin-3-yl]carbamate (180.0 mg, 0.310 mmol, 1 eq) in toluene (5 mL) was added 2,2-dimethylpropanoyl 2,2-dimethylpropanoate (175 mg, 0.94 mmol, 3 eq) at 25 °C. The reaction was heated to 100 °C and was stirred for 12 h. The solvent was evaporated and the remaining residue was purified by preparative TLC (PE/EtOAc = 3:1) to afford the title compound (100 mg, 50% yield) as a white solid. MS (ESI): 641.3 [M+H]⁺.

### Step d) tert-butyl N-[(3R)-7-(5-tert-butyl-1,2,4-oxadiazol-3-yl)-8-fluoro-1,1,4-trioxo-5-[[4-(tetrahydropyran-4-ylmethoxy)phenyl]methyl]-2, 3-dihydro-1λ ⁶,5-benzothiazepin-3-yl]carbamate

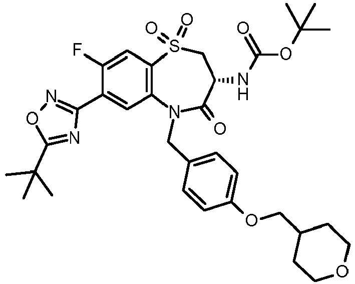

The title compound was prepared in analogy to general procedure 5 from tert-butyl N-[(3R)-7-(5-tert-butyl-1,2,4-oxadiazol-3-yl)-8-fluoro-4-oxo-5-[[4-(tetrahydropyran-4-ylmethoxy)phenyl]methyl]-2,3-dihydro-1,5-benzothiazepin-3-yl]carbamate (100 mg, 0.156 mmol, 1 eq) and was obtained as a white solid (80 mg, 76% yield). MS (ESI): 695.3 [M+Na]⁺.

### Step e) (3R)-3-amino-7-(5-tert-butyl-1,2,4-oxadiazol-3-yl)-8-fluoro-1,1-dioxo-5-[[4-(tetrahydropyran-4-ylmethoxy)phenyl]methyl]-2,3-dihydro-1λ ⁶,5-benzothiazepin-4-one

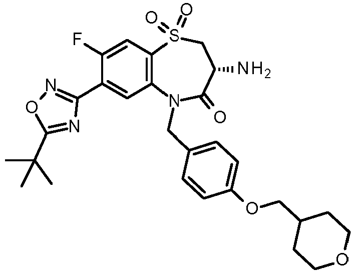

The title compound was prepared in analogy to general procedure **6b** from tert-butyl N-[(3R)-7-(5-tert-butyl-1,2,4-oxadiazol-3-yl)-8-fluoro-1,1,4-trioxo-5-[[4-(tetrahydropyran-4-ylmethoxy)phenyl]methyl]-2,3-dihydro-1λ⁶,5-benzothiazepin-3-yl]carbamate (80 mg, 0.119 mmol, 1 eq) and was obtained as a white solid (41.7 mg, 61% yield). MS (ESI): 573.3 [M+H]⁺.

### Example 354

### 3-[(3R)-3-amino-5-[(4-chlorophenyl)methyl]-1,1,4-trioxo-2,3-dihydro-1λ⁶,5-benzothiazepin-7-yl]-N-tert-butyl-1,2,4-oxadiazole-5-carboxamide

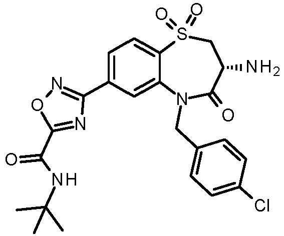

### Step a) tert-butyl N-[(3R)-5-[(4-chlorophenyl)methyl]-7-[(Z)-N'-hydroxycarbamimidoyl]-4-oxo-2,3-dihydro-1,5-benzothiazepin-3-yl]carbamate

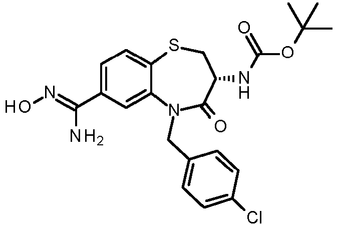

The title compound was prepared in analogy to general procedure **12** from tert-butyl N-[(3R)-5-[(4-chlorophenyl)methyl]-7-cyano-4-oxo-2,3-dihydro-1,5-benzothiazepin-3-yl]carbamate (Example 36, step d)) (400 mg, 0.901 mmol, 1 eq) and was obtained as a white foam (368 mg, 86% yield). MS (ESI): 477.4 [M+H]⁺.

### Step b) tert-butyl N-[(3R)-7-[5-(tert-butylcarbamoyl)-1,2,4-oxadiazol-3-yl]-5-[(4-chlorophenyl)methyl]-4-oxo-2,3-dihydro-1,5-benzothiazepin-3-yl]carbamate

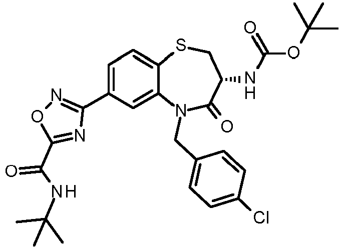

The title compound was prepared in analogy to general procedure 9c from tert-butyl N-[(3R)-5-[(4-chlorophenyl)methyl]-7-[(Z)-N'-hydroxycarbamimidoyl]-4-oxo-2,3-dihydro-1,5-benzothiazepin-3-yl]carbamate (100 mg, 0.210 mmol, 1 eq) and 2-(tert-butylamino)-2-oxo-acetic acid (CAS 169772-25-2) and was obtained as a white foam (110 mg, 90% yield). MS (ESI): 586.2 [M+H]⁺.

### Step c) tert-butyl N-[(3R)-7-[5-(tert-butylcarbamoyl)-1,2,4-oxadiazol-3-yl]-5-[(4-chlorophenyl)methyl]-1,1,4-trioxo-2,3-dihydro-1λ ⁶,5-benzothiazepin-3-yl]carbamate

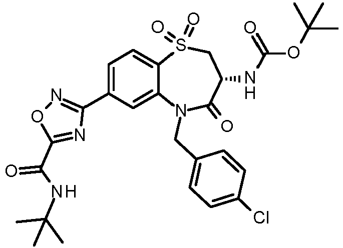

The title compound was prepared in analogy to general procedure **5** from tert-butyl N-[(3R)-7-[5-(tert-butylcarbamoyl)-1,2,4-oxadiazol-3-yl]-5-[(4-chlorophenyl)methyl]-4-oxo-2,3-dihydro-1,5-benzothiazepin-3-yl]carbamate (100 mg, 0.170 mmol, 1 eq) and was obtained as a white solid (76 mg, 72% yield). MS (ESI): 562.0 [M-isobutene+H]⁺.

### Step d) 3-[(3R)-3-amino-5-[(4-chlorophenyl)methyl]-1,1,4-trioxo-2,3-dihydro-1λ ⁶,5-benzothiazepin-7-yl]-N-tert-butyl-1,2,4-oxadiazole-5-carboxamide

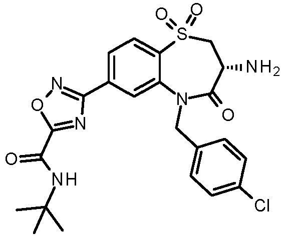

The title compound was prepared in analogy to general procedure **6b** from tert-butyl N-[(3R)-7-[5-(tert-butylcarbamoyl)-1,2,4-oxadiazol-3-yl]-5-[(4-chlorophenyl)methyl]-1,1,4-trioxo-2,3-dihydro-1λ⁶,5-benzothiazepin-3-yl]carbamate (66 mg, 0.110 mmol, 1 eq) and was obtained as a white solid (31.9 mg, 58% yield). MS (ESI): 518.1 [M+H]⁺.

### Example 355

### 3-[(3R)-3-amino-1,1,4-trioxo-5-[[4-(trifluoromethoxy)phenyl]methyl]-2,3-dihydro-1λ⁶,5-benzothiazepin-7-yl]-N-tert-butyl-1,2,4-oxadiazole-5-carboxamide

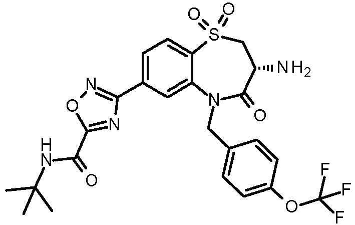

### Step a) tert-butyl N-[(3R)-7-cyano-4-oxo-5-[[4-(trifluoromethoxy)phenyl]methyl]-2,3-dihydro-1,5-benzothiazepin-3-yl]carbamate

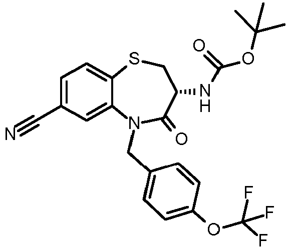

The title compound was prepared in analogy to general procedure 4 from tert-butyl N-[(3R)-7-cyano-4-oxo-3,5-dihydro-2H-1,5-benzothiazepin-3-yl]carbamate (Example 36, step c)) (150 mg, 0.470 mmol, 1 eq) and 1-(bromomethyl)-4-(trifluoromethoxy)benzene (CAS 50824-05-5). A colorless oil (273 mg) containing the title compound was obtaind which was used in the next reaction step without further purification. MS (ESI): 438.0 [M-isobutene+H]⁺.

### Step b) tert-butyl N-[(3R)-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate

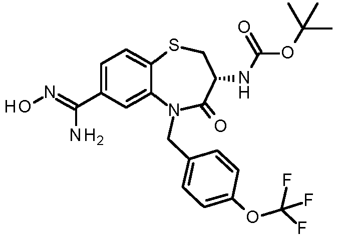

The title compound was prepared in analogy to general procedure **12** from tert-butyl N-[(3R)-7-cyano-4-oxo-5-[[4-(trifluoromethoxy)phenyl]methyl]-2,3-dihydro-1,5-benzothiazepin-3-yl]carbamate (273 mg, 0.553 mmol, 1 eq) and was obtained as a white foam (237 mg, 81% yield). MS (ESI): 471.1 [M+H]⁺.

### Step c) tert-butyl N-[(3R)-7-[5-(tert-butylcarbamoyl)-1,2,4-oxadiazol-3-yl]-4-oxo-5-[[4-(trifluoromethoxy)phenyl]methyl]-2,3-dihydro-1,5-benzothiazepin-3-yl]carbamate

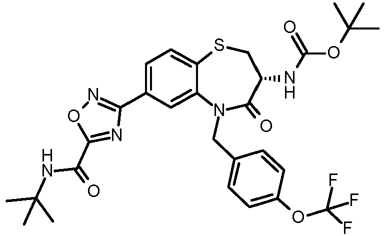

The title compound was prepared in analogy to general procedure **9c** from tert-butyl N-[(3R)-7-[(Z)-N'-hydroxycarbamimidoyl]-4-oxo-5-[[4-(trifluoromethoxy)phenyl]methyl]-2,3-dihydro-1,5-benzothiazepin-3-yl]carbamate (100 mg, 0.190 mmol, 1 eq) and 2-(tert-butylamino)-2-oxo-acetic acid (CAS 169772-25-2) and was obtained as a light yellow oil (111 mg, 93% yield). MS (ESI): 580.4 [M-isobutene+H]⁺.

### Step d) tert-butyl N-[(3R)-7-[5-(tert-butylcarbamoyl)-1,2, 4-oxadiazol-3-yl]-1,1, 4-trioxo-5-[[4-(trifluoromethoxy)phenyl]methyl]-2,3-dihydro-1λ ⁶,5-benzothiazepin-3-yl]carbamate

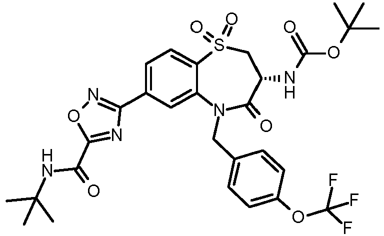

The title compound was prepared in analogy to general procedure 5 from tert-butyl N-[(3R)-7-[5-(tert-butylcarbamoyl)-1,2,4-oxadiazol-3-yl]-4-oxo-5-[[4-(trifluoromethoxy)phenyl]methyl]-2,3-dihydro-1,5-benzothiazepin-3-yl]carbamate (107 mg, 0.170 mmol, 1 eq). A colorless oil (119 mg) containing the title compound was obtaind which was used in the next reaction step without further purification. MS (ESI): 612.0 [M-isobutene+H]⁺.

### Step e) 3-[(3R)-3-amino-1,1,4-trioxo-5-[[4-(trifluoromethoxy)phenyl]methyl]-2,3-dihydro-1λ ⁶,5-benzothiazepin-7-yl]-N-tert-butyl-1,2,4-oxadiazole-5-carboxamide

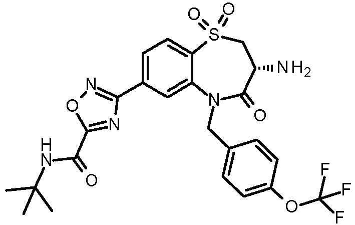

The title compound was prepared in analogy to general procedure **6b** from tert-butyl N-[(3R)-7-[5-(tert-butylcarbamoyl)-1,2,4-oxadiazol-3-yl]-1,1,4-trioxo-5-[[4-(trifluoromethoxy)phenyl]methyl]-2,3-dihydro-1λ⁶,5-benzothiazepin-3-yl]carbamate (119 mg, 0.180 mmol, 1 eq) and was obtained as a white solid (48.1 mg, 48% yield). MS (ESI): 568.0 [M+H]⁺.

Example 357 of the following table was prepared in analogy to Example 289, using the appropriate benzylhalogenide building block.

| **Ex.** | **Structure** | **Systematic Name** | **Building Block** | **MS, ESI: m/z** |
|---|---|---|---|---|
| 357 | 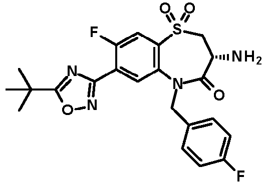 | (3R)-3-amino-7-(5-tert-butyl-1,2,4-oxadiazol-3-yl)-8-fluoro-5-[(4-fluorophenyl)methyl]-1,1-dioxo-2,3-dihydro-1λ⁶,5-benzothiazepin-4-one (*) | 4-Fluorobenz yl bromide | 477.1 [M+H]⁺ |
| | | | (CAS 459-46-1) | |

| | | | | |
|---|---|---|---|---|
| * as a hydrochloride salt | | | | |

Example 358 of the following table was prepared in analogy to Example 357, using the appropriate carboxylic acid building block.

| **Ex.** | **Structure** | **Systematic Name** | **Building Block** | **MS, ESI: m/z** |
|---|---|---|---|---|
| 358 | 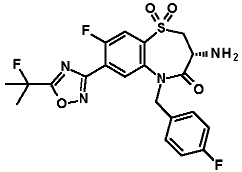 | (3R)-3-amino-8-fluoro-7-[5-(1-fluoro-1-methyl-ethyl)-1,2,4-oxadiazol-3-yl]-5-[(4-fluorophenyl)methyl]-1,1-dioxo-2,3-dihydro-1λ⁶,5-benzothiazepin-4-one | 2-fluoro-2-methylpro panoic acid | 480.9 [M+H]⁺ |
| | | | (CAS 63812-15-7) | |
| | | (*) | | |

| | | | | |
|---|---|---|---|---|
| * as a hydrochloride salt | | | | |

### Example 359

### (3R)-3-amino-7-[5-(1,1-dimethylbut-3-ynyl)-1,3,4-oxadiazol-2-yl]-8-fluoro-1,1-dioxo-5-[[4-[3-(trifluoromethyl)diazirin-3-yl]phenyl]methyl]-2,3-dihydro-1λ⁶,5-benzothiazepin-4-one

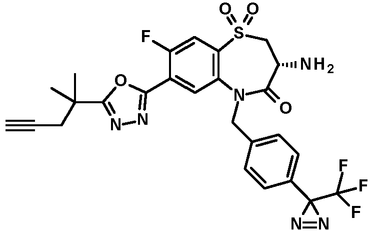

### Step a) tert-butyl N-[(3R)-7-[5-(1,1-dimethylbut-3-ynyl)-1,3,4-oxadiazol-2-yl]-8-fluoro-4-oxo-3,5-dihydro-2H-1,5-benzothiazepin-3-yl]carbamate

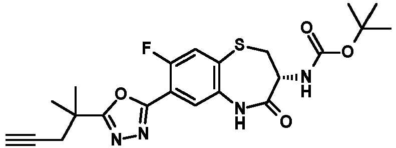

In a 25 mL round-bottomed flask, tert-butyl (R)-(8-fluoro-7-(hydrazinecarbonyl)-4-oxo-2,3,4,5-tetrahydrobenzo[b][1,4]thiazepin-3-yl)carbamate (200 mg, 540 µmol, Eq: 1), 2,2-dimethylpent-4-ynoic acid (74.9 mg, 594 µmol, Eq: 1.1), DIPEA (140 mg, 189 µl, 1.08 mmol, Eq: 2) and HATU (411 mg, 1.08 mmol, Eq: 2) were combined with THF (5.35 ml) to give a colorless solution. The reaction mixture was stirred at RT for 2 h. Burgess reagent (643 mg, 2.7 mmol, Eq: 5) was added and the reaction mixture was stirred at RT for 5h. The mixture was concentrated and the remaining residue was purified by silica gel chromatography (0% to 70% EtOAc in heptane) to yield the title compound (157 mg, 63 % yield) as a white solid. MS (ESI): 461.2 [M+H]⁺.

### Step b) tert-butyl N-[(3R)-7-[5-(1,1-dimethylbut-3-ynyl)-1,3,4-oxadiazol-2-yl]-8-fluoro-4-oxo-5-[[4-[3-(trifluoromethyl)diazirin-3-yl]phenyl]methyl]-2,3-dihydro-1,5-benzothiazepin-3-yl]carbamate

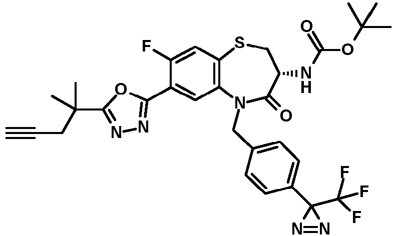

The title compound was prepared in analogy to general method **4** from tert-butyl N-[(3R)-7-[5-(1,1-dimethylbut-3-ynyl)-1,3,4-oxadiazol-2-yl]-8-fluoro-4-oxo-3,5-dihydro-2H-1,5-benzothiazepin-3-yl]carbamate (66 mg, 143 µmol) and was obtained as a light yellow oil (115 mg). MS (ESI): 659.3 [M+H]⁺.

### Step c) tert-butyl N-[(3R)-7-[5-(1,1-dimethylbut- 3-ynyl)-1,3,4-oxadiazol-2-yl]-8-fluoro-1,1,4-trioxo-5-[[4-[3-(trifluoromethyl)diazirin-3-yl]phenyl]methyl]-2, 3-dihydro-1λ ⁶,5-benzothiazepin-3-yl]carbamate

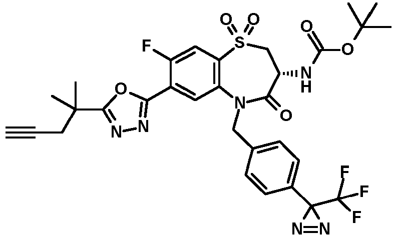

The title compound was prepared according to general method **5** from tert-butyl N-[(3R)-7-[5-(1,1-dimethylbut-3-ynyl)-1,3,4-oxadiazol-2-yl]-8-fluoro-4-oxo-5-[[4-[3-(trifluoromethyl)diazirin-3-yl]phenyl]methyl]-2,3-dihydro-1,5-benzothiazepin-3-yl]carbamate (110 mg, 167 µmol) and was obtained as white solid (57.5 mg, 83.3 µmol, 50% yield). MS (ESI): 689.3 [M-H]⁻.

### Step d) (3R)-3-amino-7-[5-(1,1-dimethylbut-3-ynyl)-1,3,4-oxadiazol-2-yl]-8-fluoro-1,1-dioxo-5-[[4-[3-(trifluoromethyl)diazirin-3-yl]phenyl]methyl]-2,3-dihydro-1λ⁶,5-benzothiazepin-4-one

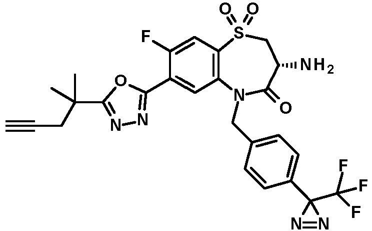

The title compound was prepared in analogy to method 6c from tert-butyl N-[(3R)-7-[5-(1,1-dimethylbut-3-ynyl)-1,3,4-oxadiazol-2-yl]-8-fluoro-1,1,4-trioxo-5-[[4-[3-(trifluoromethyl)diazirin-3-yl]phenyl]methyl]-2,3-dihydro-1λ⁶,5-benzothiazepin-3-yl]carbamate (55 mg, 79.6 µmol) with additional 4 drops of HCl (4M in dioxane) and was obtained as a white solid as hydrochloride salt and HFIP adduct (68.8 mg). MS (ESI): 591.2 [M+H]⁺.

### Example 360

### (3R)-3-amino-7-(5-tert-butyl-1,3,4-oxadiazol-2-yl)-1,1-dioxo-5-[[4-(trifluoromethoxy)phenyl]methyl]-2,3-dihydro-1λ⁶,5-benzothiazepin-4-one

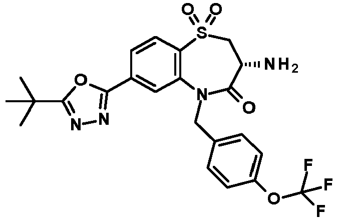

### Step a) tert-butyl N-[(3R)-7-(hydrazinecarbonyl)-4-oxo-5-[[4-(trifluoromethoxy)phenyl]methyl]-2,3-dihydro-1,5-benzothiazepin-3-yl]carbamate

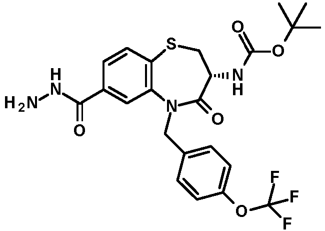

A mixture of (3R)-3-(tert-butoxycarbonylamino)-4-oxo-5-[[4-(trifluoromethoxy)phenyl]methyl]-2,3-dihydro-1,5-benzothiazepine-7-carboxylic acid (CAS:2002450-47-5) (580.0 mg, 1.13 mmol, 1 eq) and CDI (238.56 mg, 1.47 mmol, 1.3 eq) in THF (10 mL) was stirred for 1 h at 40°C. Then a solution of hydrazine hydrate (169.96 mg, 3.4 mmol, 3 eq) in THF (5 mL) was slowly added and the reaction mixture was stirred for another 4 h at 40°C.The mixture was concentrated under vacuum to remove most of the THF and the remaining residue was diluted with EtOAc. The mixture was washed with brine, dried over anhydrous Na₂SO₄, filtered and concentrated under vacuum to give the crude product which was purified by column chromatography on silica gel to afford the title compound (550 mg, 1.04 mmol, 88% yield) as a yellow solid. MS (ESI): 471.0 [M-isobutene+H] ⁺.

### Step b) tert-butyl N-[(3R)-7-[(2,2-dimethylpropanoylamino)carbamoyl]-4-oxo-5-[[4-(trifluoromethoxy)phenyl]methyl]-2,3-dihydro-1,5-benzothiazepin-3-yl]carbamate

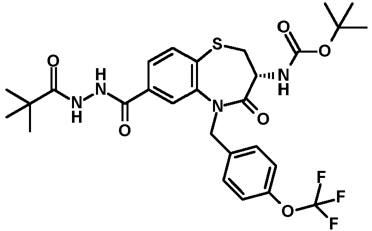

To a mixture of tert-butyl N-[(3R)-7-(hydrazinecarbonyl)-4-oxo-5-[[4-(trifluoromethoxy)phenyl]methyl]-2,3-dihydro-1,5-benzothiazepin-3-yl]carbamate (550.0 mg, 1.04 mmol, 1 eq), pivalic acid (117.35 mg, 1.15 mmol, 1.1 eq) and HATU (476.61 mg, 1.25 mmol, 1.2 eq) in THF (6 mL) was added DIPEA (0.55 mL, 3.13 mmol, 3 eq) at 25°C and the reaction mixture was stirred at 25°C for 2.5 h. The mixture was concentrated under vacuum to remove THF and the remaining residue was purified by silica gel column chromatography (PE:EtOAc = 5:1 to 1:1) to give the title compound (650 mg, 1.06 mmol, 89% yield) as a yellow liquid. MS (ESI): 555.1 [M-isobutene+H]⁺.

### Step c) tert-butyl N-[(3R)-7-(5-tert-butyl-1,3,4-oxadiazol-2-yl)-4-oxo-5-[[4-(trifluoromethoxy)phenyl]methyl]-2,3-dihydro-1,5-benzothiazepin-3-yl]carbamate

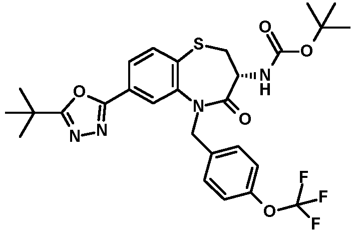

To a solution of tert-butyl N-[(3R)-7-[(2,2-dimethylpropanoylamino)carbamoyl]-4-oxo-5-[[4-(trifluoromethoxy)phenyl]methyl]-2,3-dihydro-1,5-benzothiazepin-3-yl]carbamate (630.0 mg, 1.03 mmol, 1 eq) in 1,4-dioxane (8 mL) was added Burgess reagent (983.4 mg, 4.13 mmol, 4 eq) at 25°C and then the reaction mixture was stirred at 80°C for 16 h. After cooling to ambient temperature, the mixture was concentrated under vacuum to remove dioxane and the remaining residue was purified by silica gel column chromatography (PE:EtOAc = 10:1 to 5:1) to give the title compound (570 mg, 0.960 mmol, 93% yield) as a yellow liquid. MS (ESI): 537.1 [M+H]⁺.

### Step d) tert-butyl N-[(3R)-7-(5-tert-butyl-1,3,4-oxadiazol-2-yl)-1,1,4-trioxo-5-[[4-(trifluoromethoxy)phenyl]methyl]-2,3-dihydro-1λ⁶,5-benzothiazepin-3-yl]carbamate

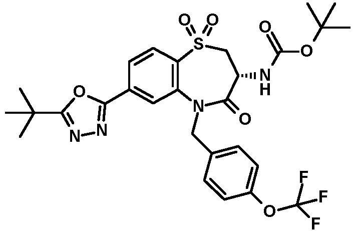

The title compound was prepared in analogy to general method **5** from tert-butyl N-[(3R)-7-(5-tert-butyl-1,3,4-oxadiazol-2-yl)-4-oxo-5-[[4-(trifluoromethoxy)phenyl]methyl]-2,3-dihydro-1,5-benzothiazepin-3-yl]carbamate (250.0 mg, 0.420 mmol) and was obtained (180 mg, 0.290 mmol, 55% yield) as a yellow oil. MS (ESI): 569.0 [M-isobutene+H]⁺.

### Step e) (3R)-3-amino-7-(5-tert-butyl-1,3,4-oxadiazol-2-yl)-1,1-dioxo-5-[[4-(trifluoromethoxy)phenyl]methyl]-2,3-dihydro-1λ⁶,5-benzothiazepin-4-one

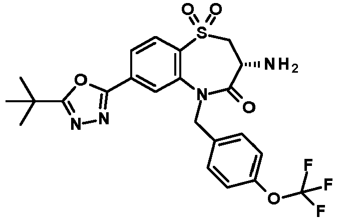

The title compound was prepared according to general method **6b** from tert-butyl N-[(3R)-7-(5-tert-butyl-1,3,4-oxadiazol-2-yl)-1,1,4-trioxo-5-[[4-(trifluoromethoxy)phenyl]methyl]-2,3-dihydro-1λ⁶,5-benzothiazepin-3-yl]carbamate (180.0 mg, 0.290 mmol) and was obtained as a white solid (20 mg, 0.040 mmol, 13% yield). MS (ESI): 525.0 [M+H]⁺.

Example 361 of the following table was prepared in analogy to Example 360, using the appropriate benzylbromid building block.

| **Ex.** | **Structure** | **Systematic Name** | **Building Block** | **MS, ESI: m/z** |
|---|---|---|---|---|
| 3 6 1 | 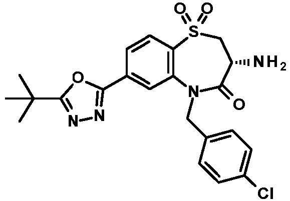 | (3R)-3-amino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one | 4-Chloroben zyl bromide | 475.1 [M+H]⁺ |
| | | | CAS (622-95-7) | |
| | | (*) | | |

| | | | | |
|---|---|---|---|---|
| * as a hydrochloride salt | | | | |

### Example 362

### (3R)-3-amino-7-[5-(1-amino-2,2,2-trifluoro-1-methyl-ethyl)-1,3,4-oxadiazol-2-yl]-5-[(4-chlorophenyl)methyl]-1,1-dioxo-2,3-dihydro-1λ⁶,5-benzothiazepin-4-one

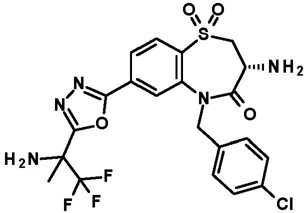

### Step a) methyl (3R)-3-(tert-butoxycarbonylamino)-5-[(4-chlorophenyl)methyl]-4-oxo-2, 3-dihydro-1,5-benzothiazepine-7-carboxylate

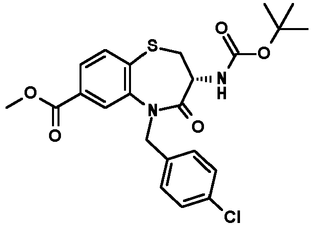

To a solution of methyl (3R)-3-(tert-butoxycarbonylamino)-4-oxo-3,5-dihydro-2H-1,5-benzothiazepine-7-carboxylate (5 g, 14.19 mmol, Eq: 1) (CAS: 2089150-62-7) in DMSO (55 mL) was added 1-(bromomethyl)-4-chlorobenzene (4.37 g, 21.28 mmol, Eq: 1.5), potassium carbonate (5.88 g, 42.56 mmol, Eq: 3) and potassium iodide (1.18 g, 7.09 mmol, Eq: 0.5) at RT. The reaction mixture was stirred overnight and then partitioned between EtOAc (300 ml) and water (200 ml). The layers were separated and the aqueous layer was extracted with EtOAc (2x200 ml). The combined organic layers were washed with water (100 ml) and sat. NH₄Cl (100 ml), dried over sodium sulfate and concentrated in vacuo. The crude residue was purified by column chromatography on silica gel (0-30% EtAOc in heptane) to afford methyl (3R)-3-(tertbutoxycarbonylamino)-5-[(4-chlorophenyl)methyl]-4-oxo-2,3-dihydro-1,5-benzothiazepine-7-carboxylate (5.88 g, 86% yield) as off-white solid. MS (ESI): 421.1 [M-isoubtene+H]⁺

### Step b) (3R)-3-(tert-butoxycarbonylamino)-5-[(4-chlorophenyl)methyl]-4-oxo-2,3-dihydro-1,5-benzothiazepine-7-carboxylic acid

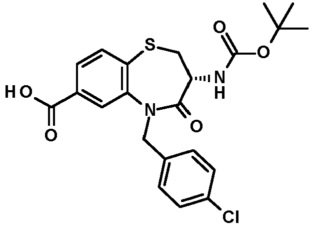

Methyl (3R)-3-(tert-butoxycarbonylamino)-5-[(4-chlorophenyl)methyl]-4-oxo-2,3-dihydro-1,5-benzothiazepine-7-carboxylate (1.99 g, 4.17 mmol, Eq: 1) was stirred with lithium hydroxide hydrate (350.13 mg, 8.34 mml, Eq: 2) in THF (18 mL), MeOH (3 mL) and water (6 mL) at RT overnight. 1N HCl was added and extracted with EtOAc (3x). The combined organic layers were dried over MgSO4, filtered and concentrated in vacuo to afford (3R)-3-(tert-butoxycarbonylamino)-5-(4-chlorobenzyl)-4-keto-2,3-dihydro-1,5-benzothiazepine-7-carboxylic acid (2.01 g, 98% yield) as light yellow oil. MS (ESI): 461.2 [M-H]⁻.

### Step c) tert-butyl N-[(3R)-5-[(4-chlorophenyl)methyl]-7-(hydrazinecarbonyl)-4-oxo-2, 3-dihydro-1,5-benzothiazepin-3-yl]carbamate

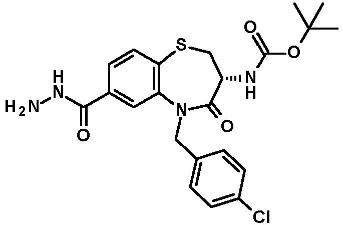

(3R)-3-(tert-butoxycarbonylamino)-5-(4-chlorobenzyl)-4-keto-2,3-dihydro-1,5-benzothiazepine-7-carboxylic acid (2.11 g, 4.57 mmol, Eq: 1) was dissolved in THF (19.7 mL). Then CDI (940 mg, 5.8 mmol, Eq: 1.27) was added in one portion and the light yellow solution was stirred for 90 min. A solution of hydrazine hydrate (691.44 mg, 670 uL, 13.81 mmol, Eq: 3) in THF (3.3 mL) was then added via syringe and the reaction stirred overnight at RT. The reaction mixture was partitioned between EtOAc (100 ml) and water (80 ml). 10 ml of brine were added to help with phase separation. The layers were separated and the aqueous layer was extracted with EtOAc (2x100 ml). The combined organic layers were washed with one 80 ml portion of brine, dried over anhydrous sodium sulfate and concentrated in vacuo to afford tert-butyl N-[(3R)-5-[(4-chlorophenyl)methyl]-7-(hydrazinecarbonyl)-4-oxo-2,3-dihydro-1,5-benzothiazepin-3-yl]carbamate (2.29 g, 86% yield) as yellow waxy solid. MS (ESI): 521.1 [M+H]⁺.

### Step d) tert-butyl N-[(3R)-7-[[[2-(tert-butoxycarbonylamino)-3, 3, 3-trifluoro-2-methylpropanoyl]amino]carbamoyl]-5-[(4-chlorophenyl)methyl]-4-oxo-2,3-dihydro-1, 5-benzothiazepin-3-yl]carbamate

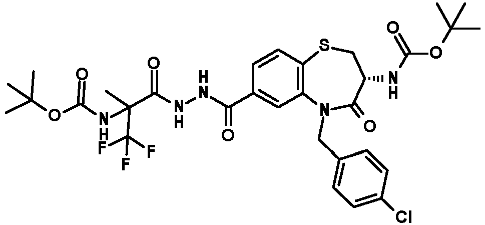

tert-Butyl N-[(3R)-5-[(4-chlorophenyl)methyl]-7-(hydrazinecarbonyl)-4-oxo-2,3-dihydro-1,5-benzothiazepin-3-yl]carbamate (100 mg, 0.193 mmol, Eq: 1) was stirred with 2-(tert-butoxycarbonylamino)-3,3,3-trifluoro-2-methyl-propionic acid (CAS 170462-68-7) (74.42 mg, 0.289 mmol, Eq: 1.5) and DIPEA (49.86 mg, 66.03 uL, 0.386 mmol, Eq: 2) in THF (1.59 mL) at RT. HATU (110.01 mg, 0.289 mmol, Eq: 1.5) was added and stirring was continued for 4 hours. The reaction solution was diluted with EtOAc and water. The layers were separated and the aqueous phase extracted EtOAc (3x). The combined organic layers were dried over sodium sulfate, filtered and concentrated. The crude was purified by column chromatography on silica gel (0-100% EtOAc in heptane) to give tert-butyl N-[1-[2-[(3R)-3-(tert-butoxycarbonylamino)-5-[(4-chlorophenyl)methyl]-4-oxo-2,3-dihydro-1,5-benzothiazepine-7-carbonyl]hydrazino]-2,2,2-trifluoro-1-methyl-ethyl]carbamate (100 mg, 68% yield) as white solid. MS (ESI): 604.1 [M-2isobutene+H]⁺.

### Step e) tert-butyl N-[1-[5-[(3R)-3-(tert-butoxycarbonylamino)-5-[(4-chlorophenyl)methyl]-4-oxo-2,3-dihydro-1,5-benzothiazepin-7-yl]-1,3,4-oxadiazol-2-yl]-2,2,2-trifluoro-1-methylethyl]carbamate

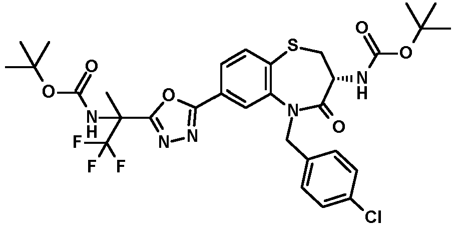

tert-butyl N-[(3R)-7-[[[2-(tert-butoxycarbonylamino)-3,3,3-trifluoro-2-methylpropanoyl]amino]carbamoyl]-5-[(4-chlorophenyl)methyl]-4-oxo-2,3-dihydro-1,5-benzothiazepin-3-yl]carbamate (100 mg, 0.131 mmol, Eq: 1) was stirred with Burgess Reagent (93.83 mg, 0.394 mmol, Eq: 3) in THF (2.63 mL) for 2 hours at RT. The reaction was then diluted with EtOAc and washed with water and then brine. The organic phase was dried over sodium sulfate, filtered and concentrated in vacuo. The crude material was purified using column chromatography on silica gel (0-35% EtOAc in heptane) to afford tert-butyl N-[1-[5-[(3R)-3-(tert-butoxycarbonylamino)-5-[(4-chlorophenyl)methyl]-4-oxo-2,3-dihydro-1,5-benzothiazepin-7-yl]-1,3,4-oxadiazol-2-yl]-2,2,2-trifluoro-1-methyl-ethyl]carbamate (61 mg, 67% yield) as white solid. MS (ESI): 586.1 [M-2isobutene+H]⁺.

### Step f) tert-butyl N-[1-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-2,2,2-trifluoro-1-methylethyl]carbamate

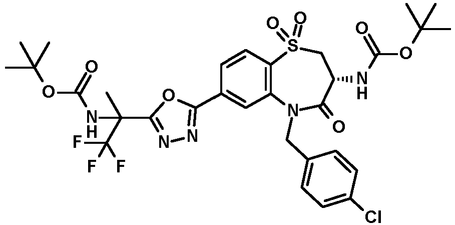

The title compound was prepared from tert-butyl N-[1-[5-[(3R)-3-(tert-butoxycarbonylamino)-5-[(4-chlorophenyl)methyl]-4-oxo-2,3-dihydro-1,5-benzothiazepin-7-yl]-1,3,4-oxadiazol-2-yl]-2,2,2-trifluoro-1-methyl-ethyl]carbamate (61 mg, 0.087 mmol) in analogy to general procedure **5** and was obtained as a white solid (34 mg, 54% yield). MS (ESI): 618.1 [M-2isobutene+H]⁺, 728.3 [M-H]⁻.

### Step g) (3R)-3-amino-7-[5-(1-amino-2,2,2-trifluoro-1-methyl-ethyl)-1,3, 4-oxadiazol-2-yl]-5-[(4-chlorophenyl)methyl]-1,1-dioxo-2,3-dihydro-1λ⁶,5-benzothiazepin-4-one

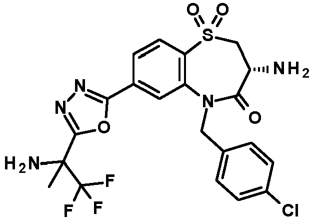

4 M HCl in dioxane (23.28 uL, 0.093 mmol, Eq: 2) was added to a solution of tert-butyl N-[1-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-2,2,2-trifluoro-1-methyl-ethyl]carbamate (34 mg, 0.047 mmol, Eq: 1) in 1,1,1,3,3,3-hexafluoro-2-propanol (2.3 mL) at RT and stirred for 1 hour. The solvent was evaporated and the crude dissolved in DCM and concentrated again to remove trace HFIP. This process was repeated three times. The so obtained white powder was dried in vacuo to afford (3R)-3-amino-7-[5-(1-amino-2,2,2-trifluoro-1-methyl-ethyl)-1,3,4-oxadiazol-2-yl]-5-[(4-chlorophenyl)methyl]-1,1-dioxo-2,3-dihydro-1λ⁶,5-benzothiazepin-4-one as white solid, as hydrochloride salt (24 mg, 91% yield). MS (ESI): 530.1 [M+H]⁺.

The examples 363 to 381 of the following table were prepared in analogy to Example 362, using the appropriate carboxylic acid building block.

| **Ex.** | **Structure** | **Systematic Name** | **Building Block** | **MS, ESI: m/z** |
|---|---|---|---|---|
| 363 | 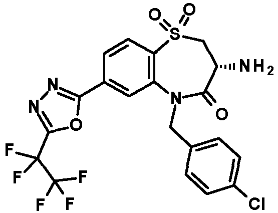 | (3R)-3-amino-5-[(4-chlorophenyl)methyl]-1,1-dioxo-7-[5-(1,1,2,2,2-pentafluoroethyl)-1,3,4-oxadiazol-2-yl]-2,3-dihydro-1λ⁶,5-benzothiazepin-4-one (*) | 2,2,3,3,3-Pentafluor opropanoic acid | 537.1 [M+H]⁺ |
| | | | (CAS 422-64-0) | |
| 364 | 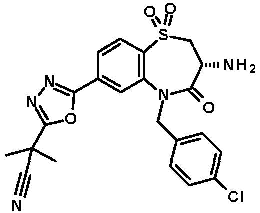 | 2-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-2-methylpropanenitrile (*) | 2-Cyano-2-methylpro panoic acid | 486.2 [M+H]⁺ |
| | | | (CAS 22426-30-8) | |
| 365 | 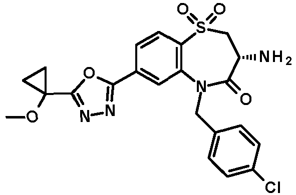 | (3R)-3-amino-5-[(4-chlorophenyl)methyl]-7-[5-(1-methoxycyclopropyl)-1,3,4-oxadiazol-2-yl]-1,1-dioxo-2,3-dihydro-1λ⁶,5-benzothiazepin-4-one (*) | 1-Methoxy-cyclopropa necarboxyl ic acid | 489.3 [M+H]⁺ |
| | | | (CAS 100683-08-7 | |
| 366 | 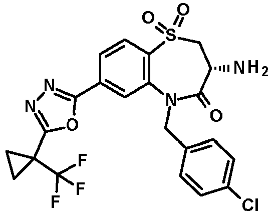 | (3R)-3-amino-5-[(4-chlorophenyl)methyl]-1,1-dioxo-7-[5-[1-(trifluoromethyl)cyclopropyl]-1,3,4-oxadiazol-2-yl]-2,3-dihydro-1λ⁶,5-benzothiazepin-4-one (*) | 1-(Trifluoro methyl)cyc lopropane-1-carboxylic acid | 527.3 [M+H]⁺ |
| | | | (CAS 277756-46-4) | |
| 367 | 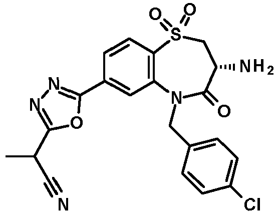 | 2-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]propanenitrile (*) | 2-Cyanoprop anoic acid | 472.2 [M+H]⁺ |
| | | | (CAS 632-07-5) | |
| 368 | 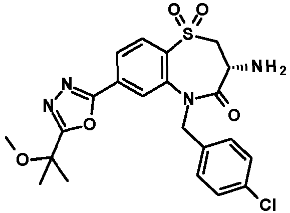 | 2-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]propanenitrile (*) | 2-Methoxy-2-methylpro panoic acid | 491.2 [M+H]⁺ |
| | | | (CAS 13836-62-9) | |
| 369 | 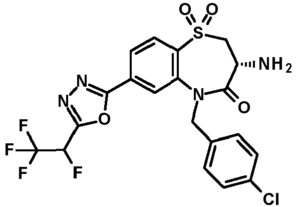 | (3R)-3-amino-5-[(4-chlorophenyl)methyl]-1,1-dioxo-7-[5-(1,2,2,2-tetrafluoroethyl)-1,3,4-oxadiazol-2-yl]-2,3-dihydro-1λ⁶,5-benzothiazepin-4-one (*) | 2,3,3,3-Tetrafluor opropanoic acid | 519.1 [M+H]⁺ |
| | | | (CAS 659-49-9) | |
| 370 | 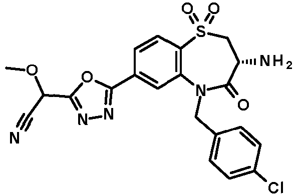 | 2-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-2-methoxyacetonitrile (*) | 2-Cyano-2-methoxyac etic acid | 488.0 [M+H]⁺ |
| | | | (CAS 59026-41-4) | |
| 371 | 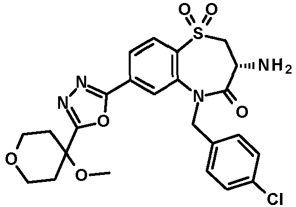 | (3R)-3-amino-5-[(4-chlorophenyl)methyl]-7-[5-(4-methoxytetrahydropyran-4-yl)-1,3,4-oxadiazol-2-yl]-1,1-dioxo-2,3-dihydro-1λ⁶,5-benzothiazepin-4-one (*) | 4-Methoxyte trahydro-2H-pyran-4-carboxylic acid | 533.3 [M+H]⁺ |
| | | | (CAS 101836-49-3) | |
| 372 | 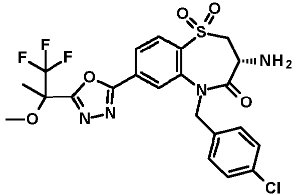 | (3R)-3-amino-5-[(4-chlorophenyl)methyl]-1,1-dioxo-7-[5-(2,2,2-trifluoro-1-methoxy-1-methyl-ethyl)-1,3,4-oxadiazol-2-yl]-2,3-dihydro-1λ⁶,5-benzothiazepin-4-one (*) | 3,3,3-Trifluoro-2-methoxy-2-methylpropanoic acid | 545.3 [M+H]⁺ |
| | | | (CAS 56135-02-5) | |
| 373 | 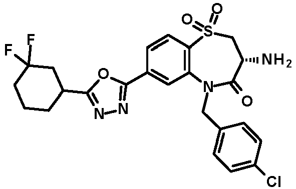 | (3R)-3-amino-5-[(4-chlorophenyl)methyl]-7-[5-(3,3-difluorocyclohexyl)-1,3,4-oxadiazol-2-yl]-1,1-dioxo-2,3-dihydro-1λ⁶,5-benzothiazepin-4-one | 3,3-Difluorocy clohexane-1-carboxylic acid | 537.1 [M+H]⁺ |
| | | | (CAS 849669-20-1) | |
| 374 | 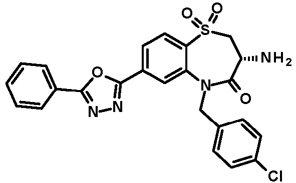 | (3R)-3-amino-5-[(4-chlorophenyl)methyl]-1,1-dioxo-7-(5-phenyl-1,3,4-oxadiazol-2-yl)-2,3-dihydro-1λ⁶,5-benzothiazepin-4-one | Benzoic acid | 495.1 [M+H]⁺ |
| | | | (CAS 65-85-0) | |
| 375 | 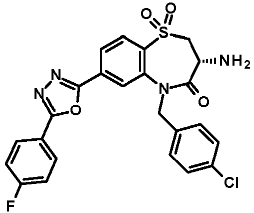 | (3R)-3-amino-5-[(4-chlorophenyl)methyl]-7-[5-(4-fluorophenyl)-1,3,4-oxadiazol-2-yl]-1,1-dioxo-2,3-dihydro-1λ⁶,5-benzothiazepin-4-one (*) | 4-Fluorobenz oic acid | 512.8 [M+H]⁺ |
| | | | (CAS 456-22-4) | |
| 376 | 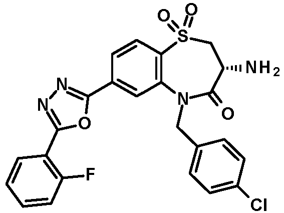 | (3R)-3-amino-5-[(4-chlorophenyl)methyl]-7-[5-(2-fluorophenyl)-1,3,4-oxadiazol-2-yl]-1,1-dioxo-2,3-dihydro-1λ⁶,5-benzothiazepin-4-one (*) | 2-Fluorobenz oic acid | 512.8 [M+H]⁺ |
| | | | (CAS 445-29-4) | |
| 377 | 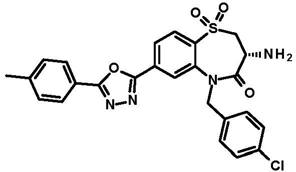 | (3R)-3-amino-5-[(4-chlorophenyl)methyl]-1,1-dioxo-7-[5-(p-tolyl)-1,3,4-oxadiazol-2-yl]-2,3-dihydro-1λ⁶,5-benzothiazepin-4-one (*) | 4-Methylben zoic acid | 509.2 [M+H]⁺ |
| | | | (CAS 99-94-5) | |
| 378 | 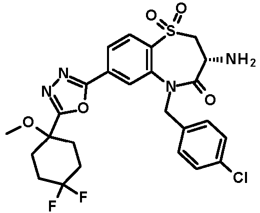 | (3R)-3-amino-5-[(4-chlorophenyl)methyl]-7-[5-(4,4-difluoro-1-methoxy-cyclohexyl)-1,3,4-oxadiazol-2-yl]-1,1-dioxo-2,3-dihydro-1λ⁶,5-benzothiazepin-4-one (*) | 4,4-Difluoro-1-methoxycy clohexane-1-carboxylic acid | 608.4 [M+H]⁺ |
| | | | (CAS 1447942-28-0) | |
| 379 | 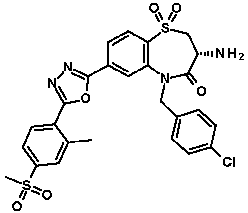 | (3R)-3-amino-5-[(4-chlorophenyl)methyl]-7-[5-(2-methyl-4-methylsulfonyl-phenyl)-1,3,4-oxadiazol-2-yl]-1,1-dioxo-2,3-dihydro-1λ⁶,5-benzothiazepin-4-one (*) | 2-Methyl-5-(methylsul fonyl)benz oic acid | 587.3 [M+H]⁺ |
| | | | (CAS 151104-37-9) | |
| 380 | 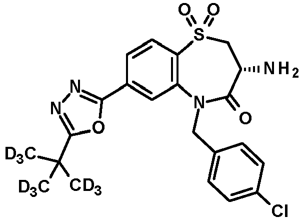 | (3R)-3-amino-5-[(4-chlorophenyl)methyl]-1,1-dioxo-7-[5-[2,2,2-trideuterio-1,1-bis(trideuteriomethyl)ethyl]-1,3,4-oxadiazol-2-yl]-2,3-dihydro-1λ⁶,5-benzothiazepin-4-one (*) | Pivalic acid-D9 | 484.4 [M+H]⁺ |
| | | | (CAS 42983-07-3) | |
| 381 | 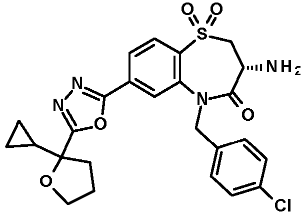 | (3R)-3-amino-5-[(4-chlorophenyl)methyl]-7-[5-(2-cyclopropyltetrahydrofuran-2-yl)-1,3,4-oxadiazol-2-yl]-1,1-dioxo-2,3-dihydro-1λ⁶,5-benzothiazepin-4-one (*) | 2-Cyclopropy loxolane-2-carboxylic acid | 529.1 [M+H]⁺ |
| | | | (CAS: 1936371-80-0) | |

| | | | | |
|---|---|---|---|---|
| (*) as hydrochloride salt | | | | |

### Example 382

### 2,2,2-trifluoroethyl 4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-4-methyl-piperidine-1-carboxylate

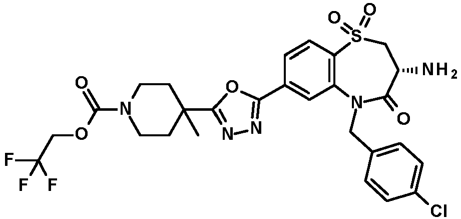

### Step a) benzyl 4-[[[(3R)-3-(tert-butoxycarbonylamino)-5-[(4-chlorophenyl)methyl]-4-oxo-2, 3-dihydro-1,5-benzothiazepine-7-carbonyl]amino]carbamoyl]-4-methyl-piperidine-1-carboxylate

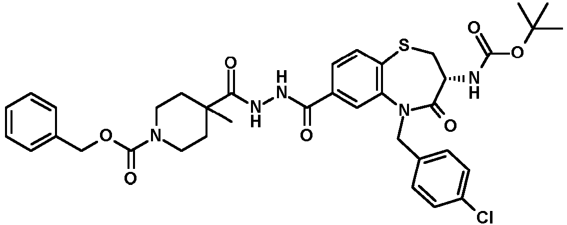

The title compound was prepared from N-[(3R)-7-carbazoyl-5-(4-chlorobenzyl)-4-keto-2,3-dihydro-1,5-benzothiazepin-3-yl]carbamic acid tert-butyl ester (350 mg, 0.734 mmol) in analogy to general procedure 7a using 1-carbobenzoxy-4-methyl-isonipecotic acid (348.84 mg, 1.26 mmol, 1.71 eq) and was obtained as light yellow solid (420 mg, 72% yield). MS (ESI): 734.3 [M-H]⁻.

### Step b) benzyl 4-[5-[(3R)-3-(tert-butoxycarbonylamino)-5-[(4-chlorophenyl)methyl]-4-oxo-2, 3-dihydro-1,5-benzothiazepin-7-yl]-1,3,4-oxadiazol-2-yl]-4-methyl-piperidine-1-carboxylate

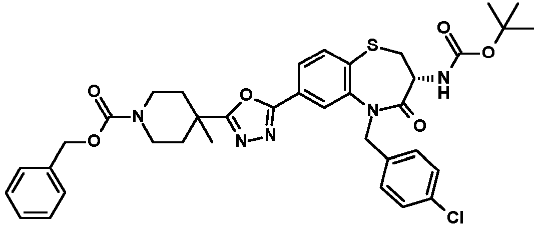

The title compound was prepared from benzyl 4-[[[(3R)-3-(tert-butoxycarbonylamino)-5-[(4-chlorophenyl)methyl]-4-oxo-2,3-dihydro-1,5-benzothiazepine-7-carbonyl]amino]carbamoyl]-4-methyl-piperidine-1-carboxylate (420 mg, 0.570 mmol) in analogy to general procedure **8b** and was obtained as white solid (325 mg, 65% yield) as white solid. MS (ESI): 618.3 [M+H-Boc]⁺.

### Step c) benzyl 4-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3, 4-oxadiazol-2-yl]-4-methyl-piperidine-1-carboxylate

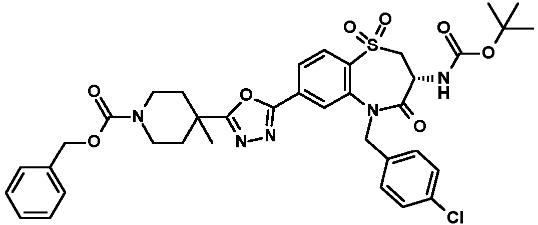

The title compound was prepared from benzyl 4-[5-[(3R)-3-(tert-butoxycarbonylamino)-5-[(4-chlorophenyl)methyl]-4-oxo-2,3-dihydro-1,5-benzothiazepin-7-yl]-1,3,4-oxadiazol-2-yl]-4-methyl-piperidine-1-carboxylate (325 mg, 0.452 mmol) in analogy to general procedure 5 and was obtained as colourless waxy (332 mg, 97% yield) as colorless waxy solid. MS (ESI): 694.2 [M+H-isobutene]⁺.

### Step d) tert-butyl N-[(3R)-5-[(4-chlorophenyl)methyl]-7-[5-(4-methyl-4-piperidyl)-1,3,4-oxadiazol-2-yl]-1,1,4-trioxo-2, 3-dihydro-1λ⁶,5-benzothiazepin-3-yl]carbamate

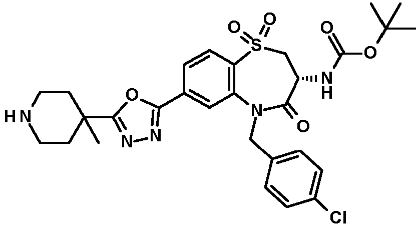

To a solution of benzyl 4-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-4-methyl-piperidine-1-carboxylate (145 mg, 0.193 mmol, 1 eq) in MeOH (3.6 mL) under Ar atmosphere was added Pd/C (20.6 mg) and the mixture was stirred under an atmosphere of hydrogen for 60 min. The mixture was filtered through a plug of celite, which was washed with MeOH and THF. The filtrate was concentrated and the remaining crude was purified using column chromatography on silica gel (0-50% EtOAc in heptane) to give tert-butyl N-[(3R)-5-[(4-chlorophenyl)methyl]-7-[5-(4-methyl-4-piperidyl)-1,3,4-oxadiazol-2-yl]-1,1,4-trioxo-2,3-dihydro-1λ⁶,5-benzothiazepin-3-yl]carbamate (112 mg, 88% yield) as white solid. MS (ESI): 616.4 [M+H]⁺.

### Step e) 2,2,2-trifluoroethyl 4-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3, 4-oxadiazol-2-yl]-4-methyl-piperidine-1-carboxylate

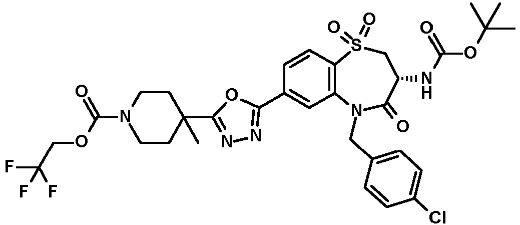

To a solution of tert-butyl N-[(3R)-5-[(4-chlorophenyl)methyl]-7-[5-(4-methyl-4-piperidyl)-1,3,4-oxadiazol-2-yl]-1,1,4-trioxo-2,3-dihydro-1λ⁶,5-benzothiazepin-3-yl]carbamate (22 mg, 0.036 mmol, 1 eq) in DCM (0.73 mL) was added DIPEA (11.54 mg, 15.59 uL, 0.089 mmol, 2.5 eq) and 2,2,2-trifluoroethyl chloroformate (6.38 mg, 4.28 uL, 0.039 mmol, 1.1 eq) at RT and stirred for 3 h. The crude material was concentrated and directly purified by column chromatography on silica gel (0-50% EtOAc in heptane) to afford 2,2,2-trifluoroethyl 4-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-4-methyl-piperidine-1-carboxylate (12 mg, 41% yield) as colorless solid. MS (ESI): 642.1 [M+H-Boc]⁺.

### Step f) 2,2, 2-trifluoroethyl 4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-4-methyl-piperidine-1-carboxylate

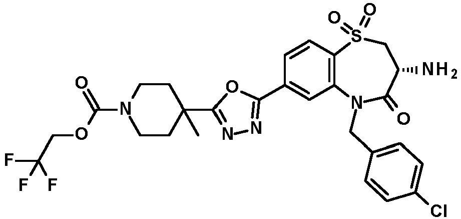

The title compound was prepared from 2,2,2-trifluoroethyl 4-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-4-methyl-piperidine-1-carboxylate (21.6 mg, 0.029 mmol, 1 eq) in analogy to general procedure **6d** and was obtained as white solid, as hydrochloride salt (10 mg, 48% yield). MS (ESI): 642.2 [M+H]⁺.

Example 383 of the following table were prepared in analogy to Example 382, using the appropriate carboxylic acid building block.

| **Ex.** | **Structure** | **Systematic Name** | **Building Block** | **MS, ESI: m/z** |
|---|---|---|---|---|
| 383 | 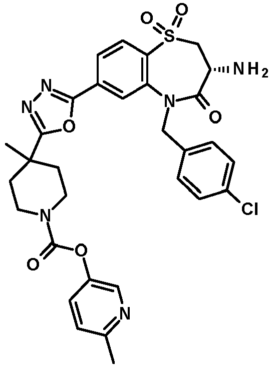 | (6-methyl-3-pyridyl) 4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-4-methylpiperidine-1-carboxylate (*) | 6-Methylpyri dine-3-yl chloroform ate | 695.3 [M-H+HCO ₂H]⁻ |
| | | | (CAS 1057393-35-7) | |

| | | | | |
|---|---|---|---|---|
| (*) as hydrochloride salt | | | | |

### Example 384

### (3R)-3-amino-5-[(4-chlorophenyl)methyl]-7-[5-(3-fluoro-1-methyl-3-piperidyl)-1,3,4-oxadiazol-2-yl]-1,1-dioxo-2,3-dihydro-1λ⁶,5-benzothiazepin-4-one

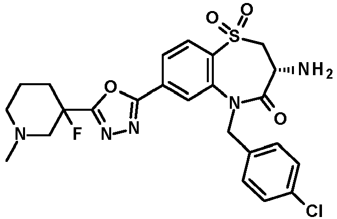

### Step a) tert-butyl N-[(3R)-5-[(4-chlorophenyl)methyl]-7-[[(3-fluoro-1-methyl-piperidine-3-carbonyl)amino]carbamoyl]-4-oxo-2,3-dihydro-1,5-benzothiazepin-3-yl]carbamate

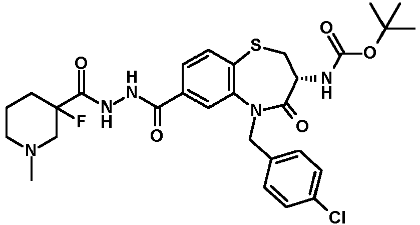

The title compound was prepared from tert-butyl N-[(3R)-5-[(4-chlorophenyl)methyl]-7-(hydrazinecarbonyl)-4-oxo-2,3-dihydro-1,5-benzothiazepin-3-yl]carbamate (100 mg, 0.210 mmol, 1 eq, Example 362, step c) and 3-fluoro-1-methyl-nipecotic acid hydrochloride (62.15 mg, 0.314 mmol, 1.5 eq) in analogy to general procedure **7a.** The title compound was obtained as colourless amporphous solid (55.5 mg, 40% yield). MS (ESI): 620.5 [M+H]⁺.

### Step b) tert-butyl N-[(3R)-5-[(4-chlorophenyl)methyl]-7-[5-(3-fluoro-1-methyl-3-piperidyl)-1,3,4-oxadiazol-2-yl]-4-oxo-2,3-dihydro-1,5-benzothiazepin-3-yl]carbamate

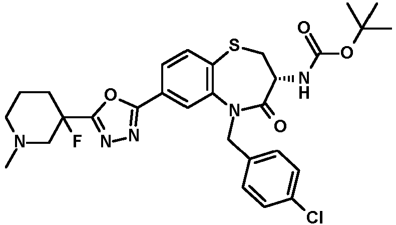

The title compound was prepared from tert-butyl N-[(3R)-5-[(4-chlorophenyl)methyl]-7-[[(3-fluoro-1-methyl-piperidine-3-carbonyl)amino]carbamoyl]-4-oxo-2,3-dihydro-1,5-benzothiazepin-3-yl]carbamate (53 mg, 0.085 mmol) in analogy to general procedure **8a** and was obtained as colourless amorphous solid (50 mg, 91% yield). MS (ESI): 602.3 [M+H]⁺.

### Step c) tert-butyl N-[(3R)-5-[(4-chlorophenyl)methyl]-7-[5-(3-fluoro-1-methyl-1-oxido-piperidin-1-ium-3-yl)-1,3,4-oxadiazol-2-yl]-1,1,4-trioxo-2,3-dihydro-1λ⁶,5-benzothiazepin-3-yl]carbamate

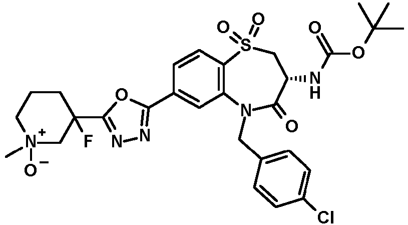

The title compound was prepared from tert-butyl N-[(3R)-5-[(4-chlorophenyl)methyl]-7-[5-(3-fluoro-1-methyl-3-piperidyl)-1,3,4-oxadiazol-2-yl]-4-oxo-2,3-dihydro-1,5-benzothiazepin-3-yl]carbamate (50 mg, 0.083 mmol, 1 eq in analogy to general procedure 5 and was obtained as light yellow amorphous solid (17 mg, 32% yield). MS (ESI): 650.3 [M+H]⁺.

### Step d) tert-butyl N-[(3R)-5-[(4-chlorophenyl)methyl]-7-[5-(3-fluoro-1-methyl-3-piperidyl)-1,3,4-oxadiazol-2-yl]-1,1,4-trioxo-2,3-dihydro-1λ⁶,5-benzothiazepin-3-yl]carbamate

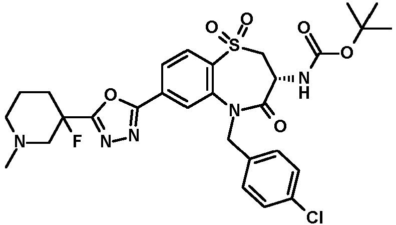

tert-butyl N-[(3R)-5-[(4-chlorophenyl)methyl]-7-[5-(3-fluoro-1-methyl-1-oxido-piperidin-1-ium-3-yl)-1,3,4-oxadiazol-2-yl]-1,1,4-trioxo-2,3-dihydro-1λ⁶,5-benzothiazepin-3-yl]carbamate (15 mg, 0.023 mmol, 1 eq) was dissolved in 1,2-dichloroethane (0.500 mL) and phenylboronic acid (3.09 mg, 0.025 mmol, 1.1 eq) was added in one portion. The mixture was heated to 85°C for one hour and then concentrated. The crude material was purified by column chromatography on silica gel (100% EtOAc) to afford tert-butyl N-[(3R)-5-[(4-chlorophenyl)methyl]-7-[5-(3-fluoro-1-methyl-3-piperidyl)-1,3,4-oxadiazol-2-yl]-1,1,4-trioxo-2,3-dihydro-1λ^{6,}5-benzothiazepin-3-yl]carbamate (11 mg, 75% yield) as white solid. MS (ESI): 643.3 [M+H]⁺.

### Step e) (3R)-3-amino-5-[(4-chlorophenyl)methyl]-7-[5-(3-fluoro-1-methyl-3-piperidyl)-1,3,4-oxadiazol-2-yl]-1,1-dioxo-2,3-dihydro-1λ⁶,5-benzothiazepin-4-one

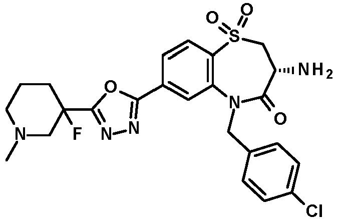

The title compound was prepared from tert-butyl N-[(3R)-5-[(4-chlorophenyl)methyl]-7-[5-(3-fluoro-1-methyl-3-piperidyl)-1,3,4-oxadiazol-2-yl]-1,1,4-trioxo-2,3-dihydro-1λ⁶,5-benzothiazepin-3-yl]carbamate (11 mg, 0.017 mmol) in analogy to general procedure **6d** and was obtained as white solid, as hydrochloride salt (9 mg, 89% yield) as white solid. MS (ESI): 534.2 [M+H]⁺.

### Example 385

### (3R)-3-amino-5-[(4-chlorophenyl)methyl]-7-[5-(5,5-difluoro-1-methyl-3-piperidyl)-1,3,4-oxadiazol-2-yl]-1,1-dioxo-2,3-dihydro-1λ⁶,5-benzothiazepin-4-one

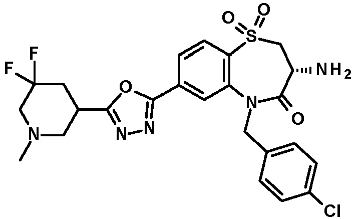

### Step a) benzyl 5-[[[(3R)-3-(tert-butoxycarbonylamino)-5-[(4-chlorophenyl)methyl]-4-oxo-2,3-dihydro-1,5-benzothiazepine-7-carbonyl]amino]carbamoyl]-3,3-difluoro-piperidine-1-carboxylate

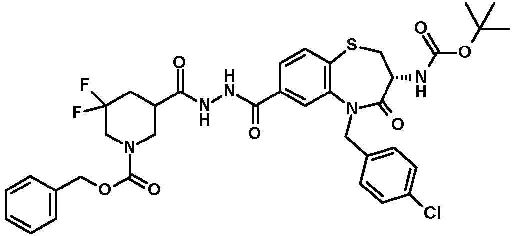

N-[(3R)-7-carbazoyl-5-(4-chlorobenzyl)-4-keto-2,3-dihydro-1,5-benzothiazepin-3-yl]carbamic acid tert-butyl ester (250 mg, 0.524 mmol, 1 eq, Example 362, step c) was stirred in THF (5 ml) with 1-carbobenzoxy-5,5-difluoro-nipecotic acid (156.86 mg, 0.524 mmol, 1 eq), T₃P (50% in EtOAc, 1 g, 926.49 uL, 1.57 mmol, 3 eq) and DIPEA (203.22 mg, 274.62 uL, 1.57 mmol, 3 eq) at 60°C for 2 h. Water (10 ml) was added and extracted with EtOAc (3x 20 ml). The combined organic layers were dired over magnesium sulfate, filtered and concentrated. The crude material was purified by perp-HPLC. The organic solvent was removed under vacuum and the title compound obtained after freeze drying (164 mg, 41% yield) as white solid. MS (ESI): 756.3 [M-H]⁻.

### Step b) benzyl 5-[5-[(3R)-3-(tert-butoxycarbonylamino)-5-[(4-chlorophenyl)methyl]-4-oxo-2, 3-dihydro-1,5-benzothiazepin-7-yl]-1,3,4-oxadiazol-2-yl]-3,3-difluoro-piperidine-1-carboxylate

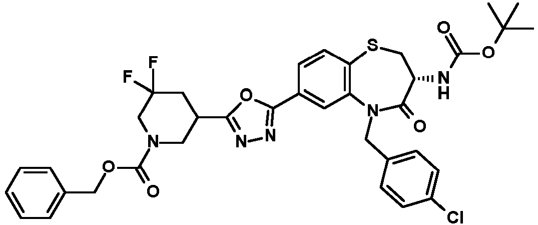

To a solution of benzyl 5-[[[(3R)-3-(tert-butoxycarbonylamino)-5-[(4-chlorophenyl)methyl]-4-oxo-2,3-dihydro-1,5-benzothiazepine-7-carbonyl]amino]carbamoyl]-3,3-difluoro-piperidine-1-carboxylate (173 mg, 0.228 mmol, 1 eq) in toluene (2 ml) was added Burgess reagent (271.86 mg, 1.14 mmol, 5 eq) and heated to 110°C for 1 h. Water (5 ml) was added and extracted with EtOAc (3x 10 ml). The combined organic layers were dried over magnesium sulfate, filtered and concentrated to afford a orange oil (284 mg) containing the title compound which was used in the next step without further purification. MS (ESI): 740.3 [M+H]⁺.

### Step c) benzyl 5-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-3,3-difluoro-piperidine-1-carboxylate

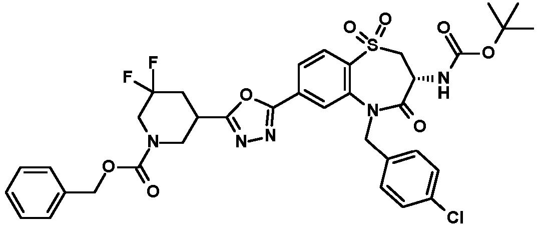

The title compound was prepared from benzyl 5-[5-[(3R)-3-(tert-butoxycarbonylamino)-5-[(4-chlorophenyl)methyl]-4-oxo-2,3-dihydro-1,5-benzothiazepin-7-yl]-1,3,4-oxadiazol-2-yl]-3,3-difluoro-piperidine-1-carboxylate (284 mg, 0.341 mmol) in analogy to general procedure 5 and was obtained as light yellow oil (190 mg, 67% yield). MS (ESI): 716.3 [M+H-isobutene]⁺.

### Step d) tert-butyl N-[(3R)-5-[(4-chlorophenyl)methyl]-7-[5-(5,5-difluoro-3-piperidyl)-1,3, 4-oxadiazol-2-yl]-1,1,4-trioxo-2,3-dihydro-1λ⁶,5-benzothiazepin-3-yl]carbamate

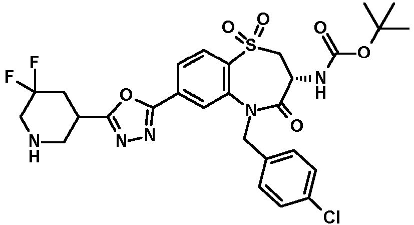

To a solution of benzyl 5-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-3,3-difluoro-piperidine-1-carboxylate (181 mg, 0.234 mmol, 1 eq) in MeOH (2.5 mL) under Ar atmosphere was added Pd/C (20 mg) and the mixture was stirred under an atmosphere of hydrogen for 30 min. The mixture was filtered through a plug of celite and the filtrate concentrated to give tert-butyl N-[(3R)-5-[(4-chlorophenyl)methyl]-7-[5-(5,5-difluoro-3-piperidyl)-1,3,4-oxadiazol-2-yl]-1,1,4-trioxo-2,3-dihydro-1λ⁶,5-benzothiazepin-3-yl]carbamate (104 mg, 62% yield) as light yellow oil. MS (ESI): 638.3 [M+H]⁺.

### Step e) tert-butylN-[(3R)-5-[(4-chlorophenyl)methyl]-7-[5-(5, 5-difluoro-1-methyl-3-piperidyl)-1,3,4-oxadiazol-2-yl]-1,1,4-trioxo-2,3-dihydro-1λ⁶,5-benzothiazepin-3-yl]carbamate

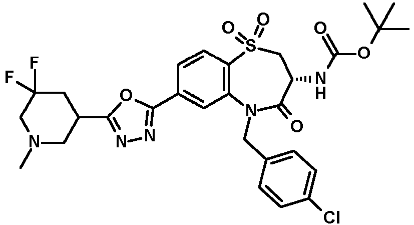

tert-butyl N-[(3R)-5-[(4-chlorophenyl)methyl]-7-[5-(5,5-difluoro-3-piperidyl)-1,3,4-oxadiazol-2-yl]-1,1,4-trioxo-2,3-dihydro-1λ⁶,5-benzothiazepin-3-yl]carbamate (30 mg, 0.047 mmol, 1 eq) was stirred with formalin (37% in H₂O, 12.95 uL, 0.470 mmol, 10 eq) and sodium triacetoxyborohydride (269.31 mg, 0.470 mmol, 10 eq) for 1 h at RT. A small amount of water was added and the crude material was applied on silica gel and purified by column chromatography using on silica gel (0-100% EtOAc in Heptane) to afford tert-butyl N-[(3R)-5-[(4-chlorophenyl)methyl]-7-[5-(5,5-difluoro-1-methyl-3-piperidyl)-1,3,4-oxadiazol-2-yl]-1,1,4-trioxo-2,3-dihydro-1λ⁶,5-benzothiazepin-3-yl]carbamate (24 mg, 70% yield) as white solid. MS (ESI): 652.4 [M+H]⁺.

### Step f) (3R)-3-amino-5-[(4-chlorophenyl)methyl]-7-[5-(5, 5-difluoro-1-methyl-3-piperidyl)-1,3,4-oxadiazol-2-yl]-1,1-dioxo-2,3-dihydro-1λ⁶,5-benzothiazepin-4-one

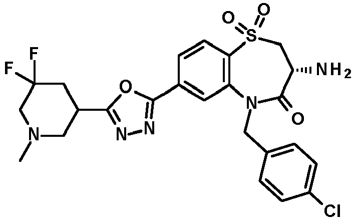

The title compound was prepared from tert-butyl N-[(3R)-5-[(4-chlorophenyl)methyl]-7-[5-(5,5-difluoro-1-methyl-3-piperidyl)-1,3,4-oxadiazol-2-yl]-1,1,4-trioxo-2,3-dihydro-1λ⁶,5-benzothiazepin-3-yl]carbamate (24 mg, 0.037 mmol) in analogy to general procedure **6b** and was obtained as white solid (11 mg, 54% yield) as white solid. MS (ESI): 552.2 [M+H]⁺.

Example 386 of the following table were prepared in analogy to Example 385, using the appropriate carboxylic acid building block and the indicated method.

| **Ex.** | **Structure** | **Systematic Name** | **Building Block** | **MS, ESI: m/z** |
|---|---|---|---|---|
| 386 | 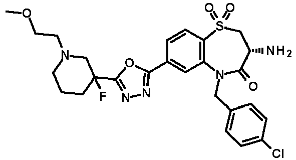 | (3R)-3-amino-5-[(4-chlorophenyl)methyl]-7-[5-[3-fluoro-1-(2-methoxyethyl)-3-piperidyl]-1,3,4-oxadiazol-2-yl]-1,1-dioxo-2,3-dihydro-1λ⁶,5-benzothiazepin-4-one (*) | 1-(Phenylme thyl)-3-fluoro-1,3-piperidinec arboxylate (CAS 1363166-38-4) and methoxyac etaldehyde (CAS 10312-83-1) | 578.3 [M+H]⁺ |

| | | | | |
|---|---|---|---|---|
| (*) as hydrochloride salt | | | | |

### Example 387

### (3R)-3-amino-5-[(4-chlorophenyl)methyl]-7-[5-(2-hydroxyspiro[3.3]heptan-6-yl)-1,3,4-oxadiazol-2-yl]-1,1-dioxo-2,3-dihydro-1λ⁶,5-benzothiazepin-4-one

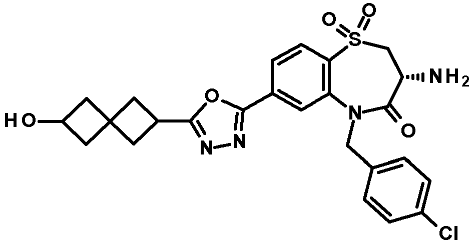

### Step a) tert-butyl N-[(3R)-5-[(4-chlorophenyl)methyl]-7-[[(2-hydroxyspiro[3.3]heptane-6-carbonyl)amino]carbamoyl]-4-oxo-2,3-dihydro-1,5-benzothiazepin-3-yl]carbamate

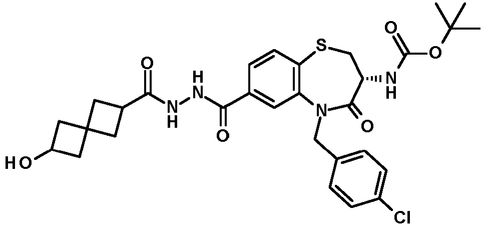

To a solution of tert-butyl N-[(3R)-5-[(4-chlorophenyl)methyl]-7-(hydrazinecarbonyl)-4-oxo-2,3-dihydro-1,5-benzothiazepin-3-yl]carbamate (100 mg, 0.210 mmol, 1 eq, Example 362, step c) in THF (1.5 ml) was added 2-hydroxyspiro[3.3]heptane-6-carboxylic acid (36.02 mg, 0.231 mmol, 1.1 eq), ethyl(diisopropyl)amine (54.19 mg, 71.78 uL, 0.419 mmol, 2 eq), and HATU (87.69 mg, 0.231 mmol, 1.1 eq). The reaction was stirred at RT overnight. Water (10 ml) and EtOAc (10 ml) were added. The layers were separated and the aqueous phase extracted with EtOAC (2x). The combined organic layers were dried over magnesium sulfate, filtered and concentrated to a white foam (220 mg) containing the title compound which was used in the next step without further purification. MS (ESI): 559.2 [M+H-isobutene]⁺.

### Step b) tert-butyl N-[(3R)-7-[[[2-[tert-butyl(dimethyl)silyl]oxyspiro[3.3]heptane-6-carbonyl]amino]carbamoyl]-5-[(4-chlorophenyl)methyl]-4-oxo-2,3-dihydro-1,5-benzothiazepin-3-yl]carbamate

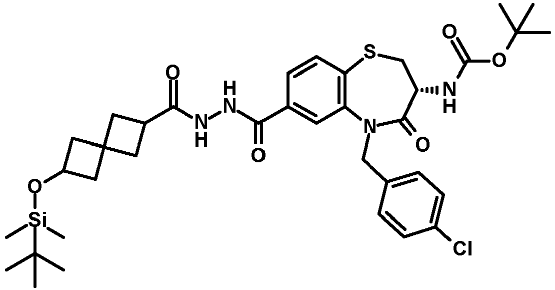

tert-butyl N-[(3R)-5-[(4-chlorophenyl)methyl]-7-[[(2-hydroxyspiro[3.3]heptane-6-carbonyl)amino]carbamoyl]-4-oxo-2,3-dihydro-1,5-benzothiazepin-3-yl]carbamate (220 mg, 0.210 mmol, 1 eq) was stirred with TBDMS-Cl (37.92 mg, 0.252 mmol, 1.2 eq) and imidazole (35.68 mg, 0.524 mmol, 2.5 eq) in THF (1.5 mL) at RT for 1 h. The reaction was concentrated and purified by column chromatography on silica gel (0-100% EtOAc in heptanes) to give tert-butyl N-[(3R)-7-[[[2-[tert-butyl(dimethyl)silyl]oxyspiro[3.3]heptane-6-carbonyl]amino]carbamoyl]-5-[(4-chlorophenyl)methyl]-4-oxo-2,3-dihydro-1,5-benzothiazepin-3-yl]carbamate (112 mg, 70% yield) as colourless amorphous solid. MS (ESI): 629.3.1 [M+H-Boc]⁺.

### Step c) tert-butyl N-[(3R)-7-[5-[2-[tert-butyl(dimethyl)silyl]oxyspiro[3.3]heptan-6-yl]-1,3, 4-oxadiazol-2-yl]-5-[(4-chlorophenyl)methyl]-4-oxo-2,3-dihydro-1,5-benzothiazepin-3-yl]carbamate

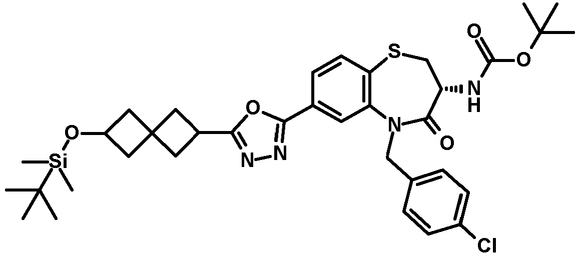

The title compound was prepared from tert-butyl N-[(3R)-7-[[[2-[tert-butyl(dimethyl)silyl]oxyspiro[3.3]heptane-6-carbonyl]amino]carbamoyl]-5-[(4-chlorophenyl)methyl]-4-oxo-2,3-dihydro-1,5-benzothiazepin-3-yl]carbamate (111 mg, 0.152 mmol) in analogy to general procedure **8a** and was obtained as colourless amorphous solid (51 mg, 47% yield). MS (ESI): 611.1 [M+H-Boc]⁺.

### Step d) tert-butyl N-[(3R)-7-[5-[2-[tert-butyl(dimethyl)silyl]oxyspiro[3.3]heptan-6-yl]-1,3,4-oxadiazol-2-yl]-5-[(4-chlorophenyl)methyl]-1,1,4-trioxo-2,3-dihydro-1λ⁶,5-benzothiazepin-3-yl]carbamate

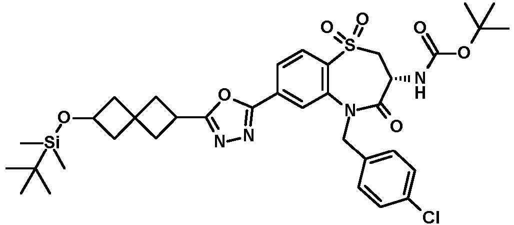

The title compound was prepared from tert-butyl N-[(3R)-7-[5-[2-[tert-butyl(dimethyl)silyl]oxyspiro[3.3]heptan-6-yl]-1,3,4-oxadiazol-2-yl]-5-[(4-chlorophenyl)methyl]-4-oxo-2,3-dihydro-1,5-benzothiazepin-3-yl]carbamate (49 mg, 0.069 mmol) in analogy to general procedure 5 and was obtained as light yellow oil (44 mg, 82% yield). MS (ESI): 643.3 [M+H-Boc]⁺.

### Step e) (3R)-3-amino-5-[(4-chlorophenyl)methyl]-7-[5-(2-hydroxyspiro[3.3]heptan-6-yl)-1,3,4-oxadiazol-2-yl]-1,1-dioxo-2,3-dihydro-1λ⁶,5-benzothiazepin-4-one

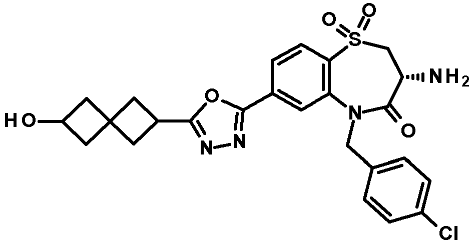

The title compound was prepared from tert-butyl N-[(3R)-7-[5-[2-[tert-butyl(dimethyl)silyl]oxyspiro[3.3]heptan-6-yl]-1,3,4-oxadiazol-2-yl]-5-[(4-chlorophenyl)methyl]-1,1,4-trioxo-2,3-dihydro-1λ⁶,5-benzothiazepin-3-yl]carbamate (42 mg, 0.056 mmol) in analogy to general procedure **6b** and was obtained as white solid (21.3 mg, 71%). MS (ESI): 529.1 [M+H]⁺.

### Example 388

### (3R)-3-amino-5-[(4-chlorophenyl)methyl]-1,1-dioxo-7-[5-(1,2,2,2-tetrafluoro-1-methoxyethyl)-1,3,4-oxadiazol-2-yl]-2,3-dihydro-1λ⁶,5-benzothiazepin-4-one

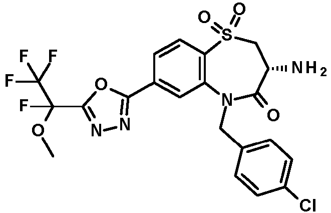

### Step a) 2,3, 3,3-tetrafluoro-2-methoxy-propanehydrazide

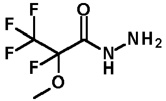

2,3,3,3-tetrafluoro-2-methoxy-propionic acid methyl ester (0.500 g, 2.63 mmol, 1 eq) was dissolved in EtOH (2.63 ml). Hydrazine monohydrate (65%, 155.63 mg, 1.2 eq) was added and the reaction heated to 80 °C for 8 hours. The mixture was concentrated to afford 2,3,3,3-tetrafluoro-2-methoxy-propanehydrazide (437 mg, 85%) as white solid. MS (ESI): 191.0 [M+H]⁺.

### Step b) tert-butyl N-[(3R)-5-[(4-chlorophenyl)methyl]-4-oxo-7-[[(2, 3, 3, 3-tetrafluoro-2-methoxy-propanoyl)amino[carbamoyl]-2,3-dihydro-1,5-benzothiazepin-3-yl]carbamate

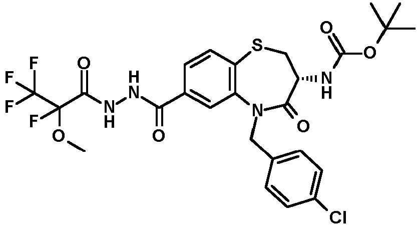

The title compound was prepared from (3R)-3-(tert-butoxycarbonylamino)-5-(4-chlorobenzyl)-4-keto-2,3-dihydro-1,5-benzothiazepine-7-carboxylic acid (100 mg, 0.212 mmol, 1 eq, Example 362, step c) and 2,3,3,3-tetrafluoro-2-methoxy-propionohydrazide (53.93 mg, 0.275 mmol, 1.3 eq) in analogy to general procedure **7a** and was obtained as white solid (101 mg, 75% yield). MS (ESI): 633.3 [M-H]⁻.

### Step c) tert-butyl N-[(3R)-5-[(4-chlorophenyl)methyl]-4-oxo-7-[5-(1,2,2,2-tetrafluoro-1-methoxy-ethyl)-1,3,4-oxadiazol-2-yl]-2,3-dihydro-1,5-benzothiazepin-3-yl]carbamate

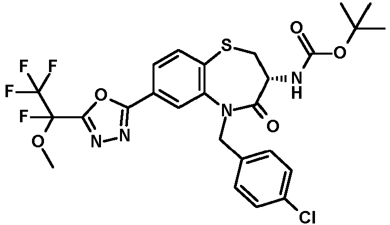

The titel compound was prepared from tert-butyl N-[(3R)-5-[(4-chlorophenyl)methyl]-4-oxo-7-[[(2,3,3,3-tetrafluoro-2-methoxy-propanoyl)amino]carbamoyl]-2,3-dihydro-1,5-benzothiazepin-3-yl]carbamate (100 mg, 0.157 mmol, 1 eq) in analogy to general procedure **8a** and was obtained as white solid (65 mg, 67% yield). MS (ESI): 561.1 [M+H-isobutene]⁺.

### Step d) tert-butyl N-[(3R)-5-[(4-chlorophenyl)methyl]-1,1,4-trioxo-7-[5-(1,2,2, 2-tetrafluoro-1-methoxy-ethyl)-1,3,4-oxadiazol-2-yl]-2,3-dihydro-1λ⁶,5-benzothiazepin-3-yl]carbamate

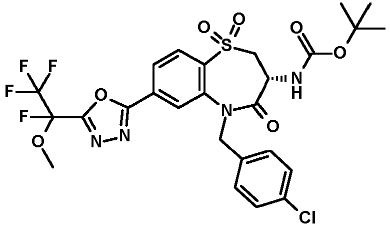

The title compound was prepared from tert-butyl N-[(3R)-5-[(4-chlorophenyl)methyl]-4-oxo-7-[5-(1,2,2,2-tetrafluoro-1-methoxy-ethyl)-1,3,4-oxadiazol-2-yl]-2,3-dihydro-1,5-benzothiazepin-3-yl]carbamate (60 mg, 0.097 mmol) in analogy to general procedure **5** and was obtained as white solid (45 mg, 71% yield). MS (ESI): 593.1 [M+H-isobutene]⁺.

### Step e) (3R)-3-amino-5-[(4-chlorophenyl)methyl]-1,1-dioxo-7-[5-(1,2,2,2-tetrafluoro-1-methoxy-ethyl)-1,3,4-oxadiazol-2-yl]-2,3-dihydro-1λ⁶,5-benzothiazepin-4-one

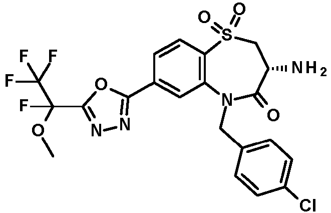

The title compound was prepared from tert-butyl N-[(3R)-5-[(4-chlorophenyl)methyl]-1,1,4-trioxo-7-[5-(1,2,2,2-tetrafluoro-1-methoxy-ethyl)-1,3,4-oxadiazol-2-yl]-2,3-dihydro-1λ⁶,5-benzothiazepin-3-yl]carbamate (15 mg, 0.023 mmol) in analogy to general procedure **6d** and was obtained as white solid (12 mg, 87% yield). MS (ESI): 549.0 [M+H]⁺.

### Example 389

### methyl 4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-4-cyano-piperidine-1-carboxylate

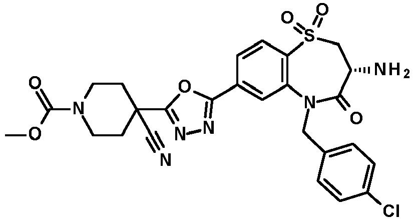

### Step a) tert-butyl 4-[5-[(3R)-3-(tert-butoxycarbonylamino)-5-[(4-chlorophenyl)methyl]-4-oxo-2,3-dihydro-1,5-benzothiazepin-7-yl]-1,3,4-oxadiazol-2-yl]-4-cyano-piperidine-1-carhoxylate

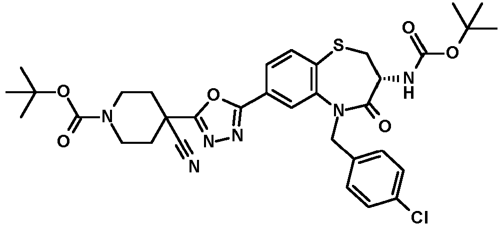

To a solution of (3R)-3-(tert-butoxycarbonylamino)-5-(4-chlorobenzyl)-4-keto-2,3-dihydro-1,5-benzothiazepine-7-carboxylic acid (200 mg, 0.419 mmol, 1 eq, Example 362, step c) in THF (3.76 ml) was added 1-tert-butoxycarbonyl-4-cyano-isonipecotic acid (117.28 mg, 0.461 mmol, 1.1 eq), HATU (175.38 mg, 0.461 mmol, 1.1 eq) and DIPEA (108.38 mg, 146.07 uL, 0.839 mmol, 2 eq). The mixture was stirred for 2 h at RT. The reaction was then concentrated in vacuo. The crude was dissolved in THF (3.76 ml) and Burgess reagent (499.6 mg, 2.1 mmol, 5 eq) was added and stirred for 16 h at RT. The reaction was concentrated and directly purified by column chromatography on silica gel (0-50% EtOAc in heptane) to afford tert-butyl 4-[5-[(3R)-3-(tert-butoxycarbonylamino)-5-[(4-chlorophenyl)methyl]-4-oxo-2,3-dihydro-1,5-benzothiazepin-7-yl]-1,3,4-oxadiazol-2-yl]-4-cyano-piperidine-1-carboxylate. MS (ESI): 539.2 [M+H-Boc-isobutene]⁺.

### Step b) tert-butyl 4-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-4-cyano-piperidine-1-carboxylate

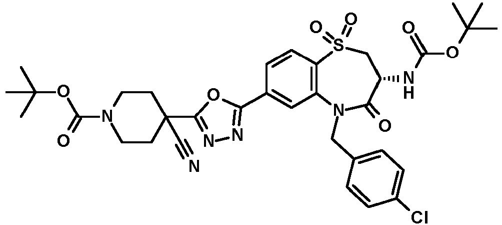

The title compound was prepared from tert-butyl 4-[5-[(3R)-3-(tert-butoxycarbonylamino)-5-[(4-chlorophenyl)methyl]-4-oxo-2,3-dihydro-1, 5-benzothiazepin-7-yl]-1,3, 4-oxadiazol-2-yl]-4-cyano-piperidine-1-carboxylate (110 mg, 0.158 mmol, 1 eq) in analogy to general procedure **5** and was obtained as light yellow solid (85 mg, 75% yield). MS (ESI): 725.3 [M-H]⁻.

### Step c) 4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]piperidine-4-carbonitrile

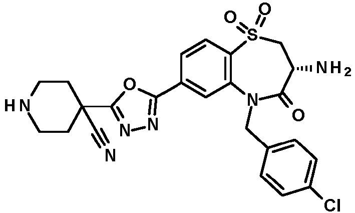

The title compound was prepared from tert-butyl 4-[5-[(3R)-3-(tert-butoxycarbonylamino)-5-[(4-chlorophenyl)methyl]-1,1,4-trioxo-2,3-dihydro-1λ⁶,5-benzothiazepin-7-yl]-1,3,4-oxadiazol-2-yl]-4-cyano-piperidine-1-carboxylate (86 mg, 0.118 mmol, 1 eq) in analogy to general procedure **6d** and was obtained as light yellow solid, as dihydrochloride salt (76 mg, 102% yield). MS (ESI): 571.1 [M-H+HCO₂H]⁺.

### Step d) methyl 4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-4-cyano-piperidine-1-carboxylate

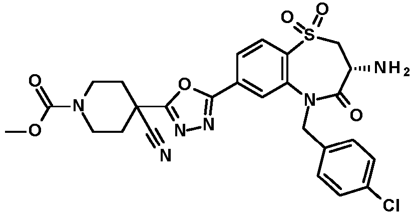

4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]piperidine-4-carbonitrile (40 mg, 0.067 mmol, 1 eq) was suspended in DCM (433.71 uL) and DIPEA (25.85 mg, 34.84 uL, 0.200 mmol, 3 eq) was added at 0°C. The mixture was stirred for 5 min and methyl chloroformate (5.67 mg, 4.64 uL, 0.060 mmol, 0.9 eq) was added. After stirring for 1 hour, water (1 ml) was added. The aqueous phase was extracted with DCM (3x, 10 ml). The combined organic layers were dried over sodium sulfate, filtered and concnentrated. The crude was purified by prep-HPLC to afford methyl 4-[5-[(3R)-3-amino-5-[(4-chlorophenyl)methyl]-1,1,4-trioxo-2,3-dihydro-1λ⁶,5-benzothiazepin-7-yl]-1,3,4-oxadiazol-2-yl]-4-cyano-piperidine-1-carboxylate (1.8 mg, 4% yield) as white solid. MS (ESI): 585.2 [M+H]⁺.

### Example 390

### (3R)-3-amino-5-[(4-chlorophenyl)methyl]-7-[5-(6-methyl-3-pyridyl)-1,3,4-oxadiazol-2-yl]-1,1-dioxo-2,3-dihydro-1λ⁶,5-benzothiazepin-4-one

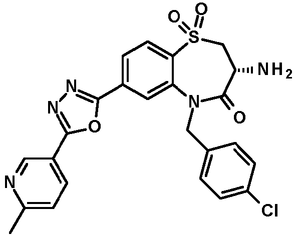

### Step a) (3R)-3-(tert-butoxycarbonylamino)-5-[(4-chlorophenyl)methyl]-1,1,4-trioxo-2,3-dihydro-1λ⁶,5-benzothiazepine-7-carboxylic acid

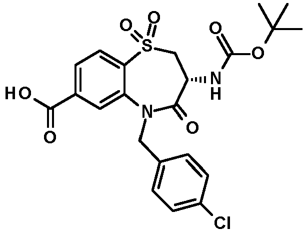

The title compound was preapared from (3R)-3-(tert-butoxycarbonylamino)-5-[(4-chlorophenyl)methyl]-4-oxo-2,3-dihydro-1,5-benzothiazepine-7-carboxylic acid (1.36 g, 2.94 mmol, 1 eq, Example 362, step b) in analogy to general procedure **5** and was obtained as white solid (0.943 g, 64% yield). MS (ESI): 439.1 [M+H-isobutene]⁺.

### Step b) tert-butyl N-[(3R)-5-[(4-chlorophenyl)methyl]-7-(hydrazinecarbonyl)-1,1,4-trioxo-2, 3-dihydro-1λ⁶,5-benzothiazepin-3-yl]carbamate

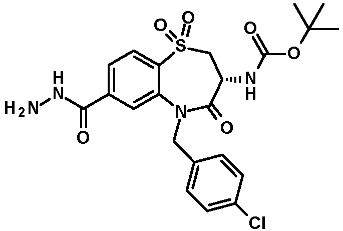

To a solution of (3R)-3-(tert-butoxycarbonylamino)-5-[(4-chlorophenyl)methyl]-1,1,4-trioxo-2,3-dihydro-1λ⁶,5-benzothiazepine-7-carboxylic acid (0.940 g, 1.9 mmol, 1 eq) in THF (8 mL)at RT was added CDI (369.54 mg, 2.28 mmol, 1.2 eq) in one portion. The solution was then stirred for 45 min. To this solution was added a mixture of hydrazine hydrate (445.66 mg, 431.84 uL, 5.7 mmol, 3 eq) in THF (1.5 mL) and stirred for 30 min. The reaction mixture was diluted with EtOAc and brine and the layers were separated. The aqueous layer was extracted twice more with EtOAc. The combined organic layers were dried over anhydrous sodium sulfate, filtered and concentrated in vacuo. The crude product was then purified using column chromatography using on silica gel (0-5% MeOH in EtOAc) to give the title compound as light yellow solid (357 mg, 37% yield). MS (ESI): 453.1 [M+H-isobutene]⁺.

### Step c) tert-butyl N-[(3R)-5-[(4-chlorophenyl)methyl]-7-[[(6-methylpyridine-3-carbonyl)amino]carbamoyl]-1,1,4-trioxo-2,3-dihydro-1λ⁶,5-benzothiazepin-3-yl]carbamate

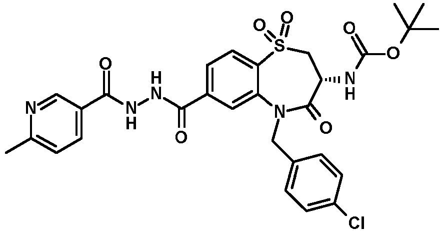

The title compound was prepared from tert-butyl N-[(3R)-5-[(4-chlorophenyl)methyl]-7-(hydrazinecarbonyl)-1,1,4-trioxo-2,3-dihydro-1λ⁶,5-benzothiazepin-3-yl]carbamate (50 mg, 0.098 mmol, 1 eq) and 6-methylnicotinic acid (14.82 mg, 0.108 mmol, 1.1 eq) in analogy to general procedure 7a to afford a yellow oil (96 mg) containing the title product, which was used in the next step without further purification. MS (ESI): 628.3 [M+H]⁺.

### Step d) tert-butyl N-[(3R)-5-[(4-chlorophenyl)methyl]-7-[5-(6-methyl-3-pyridyl)-1,3,4-oxadiazol-2-yl]-1,1,4-trioxo-2,3-dihydro-1λ⁶,5-benzothiazepin-3-yl]carbamate

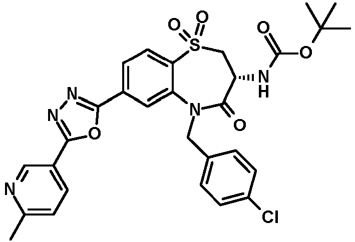

The title compound was prepared from tert-butyl N-[(3R)-5-[(4-chlorophenyl)methyl]-7-[[(6-methylpyridine-3-carbonyl)amino]carbamoyl]-1,1,4-trioxo-2,3-dihydro-1λ⁶,5-benzothiazepin-3-yl]carbamate (96 mg, 0.118 mmol, 1 eq) in analogy to general procedure **8b** and was obtained as white solid (27.6 mg, 37% yield). MS (ESI): 610.3 [M+H]⁺.

### Step e) (3R)-3-amino-5-[(4-chlorophenyl)methyl]-7-[5-(6-methyl-3-pyridyl)-1,3,4-oxadiazol-2-yl]-1,1-dioxo-2,3-dihydro-1λ⁶,5-benzothiazepin-4-one

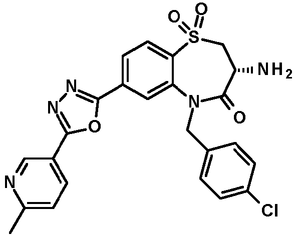

The title compound was prepared from tert-butyl N-[(3R)-5-[(4-chlorophenyl)methyl]-7-[5-(6-methyl-3-pyridyl)-1,3,4-oxadiazol-2-yl]-1,1,4-trioxo-2,3-dihydro-1λ⁶,5-benzothiazepin-3-yl]carbamate (27.6 mg, 0.044 mmol, 1 eq) in analogy to general procedure **6d** and was obtained as white solid, as hydrochloride salt (17.9 mg, 75%). MS (ESI): 554.2 [M-H+HCO₂H]⁻.

### Example 391

### (3R)-3-amino-5-[(4-chlorophenyl)methyl]-7-[5-(2,2-difluoromorpholin-4-yl)-1,3,4-oxadiazol-2-yl]-1,1-dioxo-2,3-dihydro-1λ⁶,5-benzothiazepin-4-one

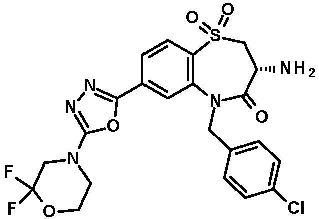

### Step a) tert-butyl N-[(3R)-5-[(4-chlorophenyl)methyl]-7-[[(2,2-difluoromorpholine-4-carbonyl)amino[carbamoyl]-4-oxo-2,3-dihydro-1,5-benzothiazepin-3-yl]carbamate

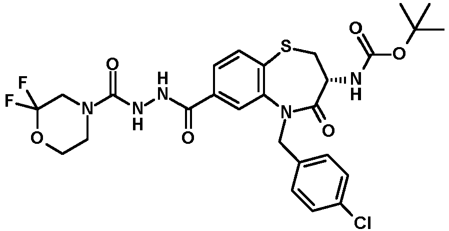

To a solution of 2,2-difluoromorpholine hydrochloride (CAS 1820647-38-8, 150 mg, 0.940 mmol, Eq: 1) and pyridine (223.08 mg, 228.1 uL, 2.82 mmol, Eq: 3) in 1,2-dichloroethane (4 mL) was added triphosgene (278.97 mg, 0.940 mmol, Eq: 1) at 0 °C in one portion. The cooling bath was removed and stirring was continued for 2 h. The reaction mixture was then partitioned between EtOAc (50 ml) and 1M HCl (25 ml). The layers were separated. The aqueous layer was extracted with two 20 ml portions of EtOAc and the combined organic layers were dried over anhydrous sodium sulfate and concentrated in vacuo. The residue was dissolved in 1,2-DCE (4 mL) and tert-butyl N-[(3R)-5-[(4-chlorophenyl)methyl]-7-(hydrazinecarbonyl)-4-oxo-2,3-dihydro-1,5-benzothiazepin-3-yl]carbamate (224.2 mg, 0.470 mmol, 0.5 Eq: 1) was added and the reaction mixture was stirred for 4 h at RT. The reaction mixture was partitioned between EtOAc (50 ml) and water (25 ml). The layers were separated and the aqueous layer was extracted with two 20 ml portions of EtOAc. The combined organic layers were dried over anhydrous sodium sulfate and concentrated in vacuo to afford a yellow solid (365 mg) containing the title compound, which was used in the next step without further purification. MS (ESI): 624.2 [M-H]⁻.

### Step b) tert-butyl N-[(3R)-5-[(4-chlorophenyl)methyl]-7-[5-(2,2-difluoromorpholin-4-yl)-1,3, 4-oxadiazol-2-yl]-4-oxo-2,3-dihydro-1,5-benzothiazepin-3-yl]carbamate

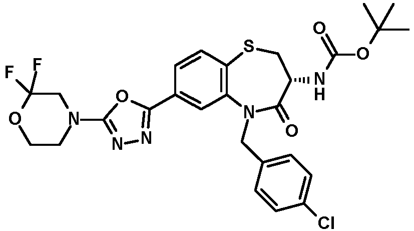

tert-butyl N-[(3R)-5-[(4-chlorophenyl)methyl]-7-[[(2,2-difluoromorpholine-4-carbonyl)amino]carbamoyl]-4-oxo-2,3-dihydro-1,5-benzothiazepin-3-yl]carbamate (365 mg, 0.583 mmol, Eq: 1) in acetonitrile (10 mL) was added p-toluenesulfonyl chloride (333.45 mg, 1.75 mmol, Eq: 3) and DIPEA (150.69 mg, 203.64 uL, 1.17 mmol, Eq: 2). The reaction mixture was stirred at RT for 4 h and then partitioned between EtOAc (50 ml) and water (40 ml). The layers were separated and the aqueous layer was extracted with two 40 ml portions of EtOAc. The combined organic layers were washed with one 20 ml portion of brine, dried over anhydrous sodium sulfate and concentrated in vacuo. The crude material was purified by column chromatography on siliga gel (0-50% EtOAc in heptane) to afford tert-butyl N-[(3R)-5-[(4-chlorophenyl)methyl]-7-[5-(2,2-difluoromorpholin-4-yl)-1,3,4-oxadiazol-2-yl]-4-oxo-2,3-dihydro-1,5-benzothiazepin-3-yl]carbamate (155 mg, 35% yield) as yellow solid. MS (ESI): 608.2 [M+H]⁺.

### Step c) tert-butyl N-[(3R)-5-[(4-chlorophenyl)methyl]-7-[5-(2,2-difluoromorpholin-4-yl)-1,3,4-oxadiazol-2-yl]-1,1,4-trioxo-2,3-dihydro-1λ⁶,5-benzothiazepin-3-yl]carbamate

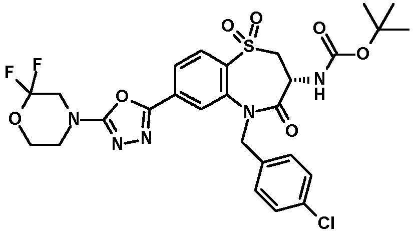

The title compound was prepared from tert-butyl N-[(3R)-5-[(4-chlorophenyl)methyl]-7-[5-(2,2-difluoromorpholin-4-yl)-1,3,4-oxadiazol-2-yl]-4-oxo-2,3-dihydro-1,5-benzothiazepin-3-yl]carbamate (155 mg, 0.255 mmol, Eq: 1) in analogy to general procedure **5** and was obtained as white solid (97.8 mg, 57% yield). MS (ESI): 640.2 [M+H]⁺.

### Step d) (3R)-3-amino-5-[(4-chlorophenyl)methyl]-7-[5-(2, 2-difluoromorpholin-4-yl)-1,3,4-oxadiazol-2-yl]-1,1-dioxo-2,3-dihydro-1λ⁶,5-benzothiazepin-4-one

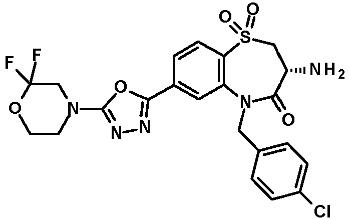

To a solution of tert-butyl N-[(3R)-5-[(4-chlorophenyl)methyl]-7-[5-(2,2-difluoromorpholin-4-yl)-1,3,4-oxadiazol-2-yl]-1,1,4-trioxo-2,3-dihydro-1λ⁶,5-benzothiazepin-3-yl]carbamate (97.8 mg, 0.145 mmol, 1 eq) in 1,1,1,3,3,3-hexafluoro-2-propanol (1 mL) was added 4M HCl in dioxane (5.29 mg, 4.41 uL, 0.145 mmol, Eq: 1) at RT. The reaction mixture was stirred for 3 h. The solvent was evaporated and the crude dissolved in DCM and concentrated again to remove trace HFIP. This process was repeated three times to afford (3R)-3-amino-5-[(4-chlorophenyl)methyl]-7-[5-(2,2-difluoromorpholin-4-yl)-1,3,4-oxadiazol-2-yl]-1,1-dioxo-2,3-dihydro-1λ⁶,5-benzothiazepin-4-one (55.4 mg, 66% yield) as white solid, as hydrochloride salt. MS (ESI): 540.1 [M+H]⁺.

Example 392 to 394 of the following table were prepared in analogy to Example 391, using the appropriate amine building block.

| **Ex.** | **Structure** | **Systematic Name** | **Building Block** | **MS, ESI: m/z** |
|---|---|---|---|---|
| 392 | 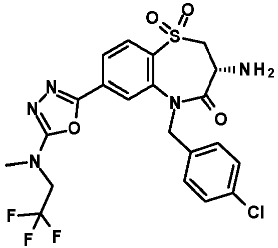 | (3R)-3-amino-5-[(4-chlorophenyl)methyl]-7-[5-[methyl(2,2,2-trifluoroethyl)amino]-1,3,4-oxadiazol-2-yl]-1,1-dioxo-2,3-dihydro-1λ⁶,5-benzothiazepin-4-one (*) | Methyl(2,2 ,2-trifluoroet hyl)amine | 530.2 [M+H]⁺ |
| | | | (CAS 2730-67-8) | |
| 393 | 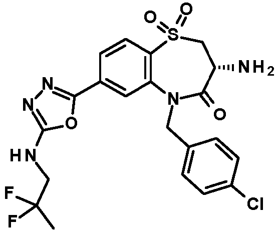 | (3R)-3-amino-5-[(4-chlorophenyl)methyl]-7-[5-(2,2-difluoropropylamino)-1,3,4-oxadiazol-2-yl]-1,1-dioxo-2,3-dihydro-1λ⁶,5-benzothiazepin-4-one (*) | 2,2,2-Trifluoroet hylamine | 512.2 [M+H]⁺ |
| | | | (CAS 753-90-2) | |
| 394 | 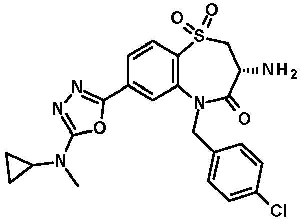 | (3R)-3-amino-5-[(4-chlorophenyl)methyl]-7-[5-[cyclopropyl(methyl)amino]-1,3,4-oxadiazol-2-yl]-1,1-dioxo-2,3-dihydro-1λ⁶,5-benzothiazepin-4-one (*) | Cyclopropy Imethylamine | 488.2 [M+H]⁺ |
| | | | (CAS 5163-20-2) | |

| | | | | |
|---|---|---|---|---|
| (*) as hydrochloride salt | | | | |

### Example 395

### (3R)-3-amino-5-[(4-chlorophenyl)methyl]-7-[5-(2,2-dimethylmorpholin-4-yl)-1,3,4-oxadiazol-2-yl]-1,1-dioxo-2,3-dihydro-1λ⁶,5-benzothiazepin-4-one

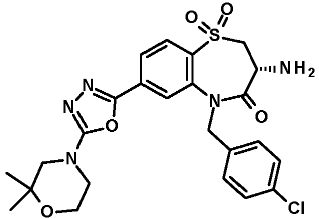

### Step a) tert-butyl N-[(3R)-5-[(4-chlorophenyl)methyl]-4-oxo-7-(2-oxo-3H-1,3,4-oxadiazol-5-yl)-2,3-dihydro-1,5-benzothiazepin-3-yl]carbamate

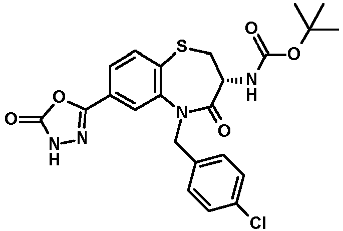

A solution of N-[(3R)-7-carbazoyl-5-(4-chlorobenzyl)-4-keto-2,3-dihydro-1,5-benzothiazepin-3-yl]carbamic acid tert-butyl ester (2 g, 4.19 mmol, Eq: 1) in THF (42.79 mL) was degassed with argon for 10 min. Then CDI (720.7 mg, 4.44 mmol, Eq: 1.05) and NEt₃ (449.75 mg, 616.1 uL, 4.44 mmol, Eq: 1.05) were added at RT. The reaction mixture was stirred for 2 h. The reaction mixture was diluted with EtOAc (150ml) and then washed with 1N HCl solution (50ml), sat. aq. NaHCO₃ solution (50ml) and brine (50ml), dried over sodium sulfate, filtered and evaporated to afford tert-butyl N-[(3R)-5-[(4-chlorophenyl)methyl]-4-oxo-7-(2-oxo-3H-1,3,4-oxadiazol-5-yl)-2,3-dihydro-1,5-benzothiazepin-3-yl]carbamate (1.79 g, 80% yield) as light yellow solid. MS (ESI): 501.2 [M-H]⁻.

### Step b) tert-butyl N-[(3R)-5-[(4-chlorophenyl)methyl]-1,1,4-trioxo-7-(2-oxo-3H-1,3,4-oxadiazol-5-yl)-2,3-dihydro-1λ⁶,5-benzothiazepin-3-yl]carbamate

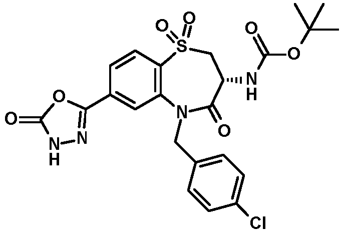

The title compound was prepared from tert-butyl N-[(3R)-5-[(4-chlorophenyl)methyl]-4-oxo-7-(2-oxo-3H-1,3,4-oxadiazol-5-yl)-2,3-dihydro-1,5-benzothiazepin-3-yl]carbamate (1.79 g, 3.56 mmol) in analogy to general procedure 5 and was obtained as white solid (1.31 g, 65% yield). MS (ESI): 533.1 [M-H]⁻.

### Step c) tert-butyl N-[(3R)-5-[(4-chlorophenyl)methyl]-7-[5-(2,2-dimethylmorpholin-4-yl)-1,3,4-oxadiazol-2-yl]-1,1,4-trioxo-2,3-dihydro-1λ⁶,5-henzothiazepin-3-yl]carbamate

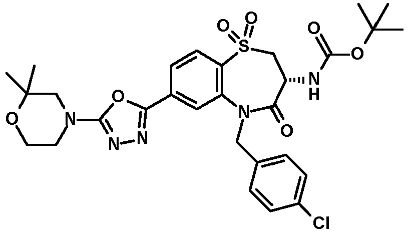

To a colorless solution of tert-butyl N-[(3R)-5-[(4-chlorophenyl)methyl]-1,1,4-trioxo-7-(2-oxo-3H-1,3,4-oxadiazol-5-yl)-2,3-dihydro-1λ⁶,5-benzothiazepin-3-yl]carbamate (50 mg, 0.093 mmol, Eq: 1) in DMF (1 mL) was added DIPEA (48.32 mg, 63.58 uL, 0.374 mmol, Eq: 4) and BOP (51.66 mg, 0.117 mmol, Eq: 1.25) and a yellow solution resulted. After 5 min, 2,2-dimethylmorpholine (CAS: 147688-58-2, 12.92 mg, 0.112 mmol, Eq: 1) was added in one portion and the reaction mixture was stirred at RT for 3 h. The reaction was diluted with EtOAc and water. The layers were separated and the aqueous phase extracted twice with EtOAc. The combined organic layers were dried over sodium sulfate, filtered and concentrated. The crude was purified by column chromatography on silica gel (0-50% EtOAc in heptane) to afford tert-butyl N-[(3R)-5-[(4-chlorophenyl)methyl]-7-[5-(2,2-dimethylmorpholin-4-yl)-1,3,4-oxadiazol-2-yl]-1,1,4-trioxo-2,3-dihydro-1λ⁶,5-benzothiazepin-3-yl]carbamate (33.1 mg, 56% yield) as white solid. MS (ESI): 632.2 [M+H]⁺.

### Step d) (3R)-3-amino-5-[(4-chlorophenyl)methyl]-7-[5-(2,2-dimethylmorpholin-4-yl)-1,3,4-oxadiazol-2-yl]-1,1-dioxo-2,3-dihydro-1λ⁶,5-benzothiazepin-4-one

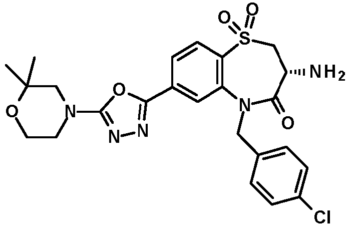

To a solution of tert-butyl N-[(3R)-5-[(4-chlorophenyl)methyl]-7-[5-(2,2-dimethylmorpholin-4-yl)-1,3,4-oxadiazol-2-yl]-1,1,4-trioxo-2,3-dihydro-1λ⁶,5-benzothiazepin-3-yl]carbamate (33.1 mg, 0.052 mmol, 1 eq) in 1,1,1,3,3,3-hexafluoro-2-propanol (1 mL) was added 4M HCl in dioxane (8 drops) at RT. The reaction mixture was stirred for 3 h. The solvent was evaporated and the crude dissolved in DCM and concentrated again to remove trace HFIP. This process was repeated three times to afford (3R)-3-amino-5-[(4-chlorophenyl)methyl]-7-[5-(2,2-dimethylmorpholin-4-yl)-1,3,4-oxadiazol-2-yl]-1,1-dioxo-2,3-dihydro-1λ⁶,5-benzothiazepin-4-one (25.1 mg, 84.32%) as white solid, as hydrochloride salt. MS (ESI): 532.2 [M+H]⁺.

The examples 396 to 412 of the following table were prepared in analogy to Example 395, using the appropriate amine building block.

| **Ex.** | **Structure** | **Systematic Name** | **Building Block** | **MS, ESI: m/z** |
|---|---|---|---|---|
| 396 | 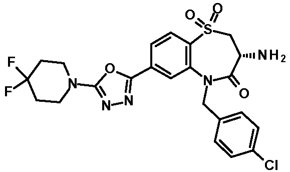 | (3R)-3-amino-5-[(4-chlorophenyl)methyl]-7-[5-(4,4-difluoro-1-piperidyl)-1,3,4-oxadiazol-2-yl]-1,1-dioxo-2,3-dihydro-1λ⁶,5-benzothiazepin-4-one (*) | 4,4-Difluoropi peridine | 538.1 [M+H]⁺ |
| | | | (CAS 21987-29-1) | |
| 397 | 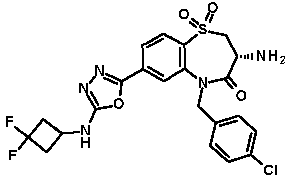 | (3R)-3-amino-5-[(4-chlorophenyl)methyl]-7-[5-[(3,3-difluorocyclobutyl)amino]-1,3,4-oxadiazol-2-yl]-1,1-dioxo-2,3-dihydro-1λ⁶,5-benzothiazepin-4-one (*) | 3,3-Difluorocy clobutan-1-amine hydrochlor ide | 524.1 [M+H]⁺ |
| | | | (CAS 637031-93-7) | |
| 398 | 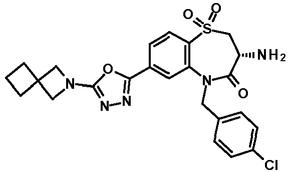 | (3R)-3-amino-7-[5-(2-azaspiro[3.3]heptan-2-yl)-1,3,4-oxadiazol-2-yl]-5-[(4-chlorophenyl)methyl]-1,1-dioxo-2,3-dihydro-1λ⁶,5-benzothiazepin-4-one (*) | 2-Azaspiro[3. 3]heptane | 514.2 [M+H]⁺ |
| | | | (CAS 665-04-3) | |
| 399 | 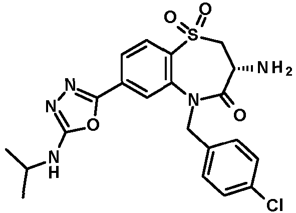 | (3R)-3-amino-5-[(4-chlorophenyl)methyl]-7-[5-(isopropylamino)-1,3,4-oxadiazol-2-yl]-1,1-dioxo-2,3-dihydro-1λ⁶,5-benzothiazepin-4-one (*) | Isopropyla mine | 476.2 [M+H]⁺ |
| | | | (CAS 75-31-0) | |
| 400 | 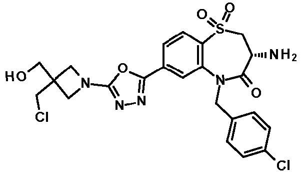 | (3R)-3-amino-7-[5-[3-(chloromethyl)-3-(hydroxymethyl)azetidin-1-yl]-1,3,4-oxadiazol-2-yl]-5-[(4-chlorophenyl)methyl]-1,1-dioxo-2,3-dihydro-1λ⁶,5-benzothiazepin-4-one (*) | 2-Oxa-6-azapsiro[3. 3]heptane | 552.1 [M+H]⁺ |
| | | | (CAS 174-78-7) | |
| 401 | 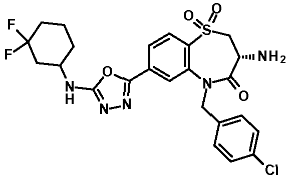 | (3R)-3-amino-5-[(4-chlorophenyl)methyl]-7-[5-[(3,3-difluorocyclohexyl)amino]-1,3,4-oxadiazol-2-yl]-1,1-dioxo-2,3-dihydro-1λ⁶,5-benzothiazepin-4-one (*) | 3,3,-Difluorocy clohexana mine hydrochlor ide | 552.2 [M+H]⁺ |
| | | | (CAS 921602-77-9) | |
| 402 | 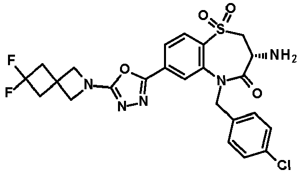 | (3R)-3-amino-5-[(4-chlorophenyl)methyl]-7-[5-(6,6-difluoro-2-azaspiro[3.3]heptan-2-yl)-1,3,4-oxadiazol-2-yl]-1,1-dioxo-2,3-dihydro-1λ⁶,5-benzothiazepin-4-one (*) | 6,6,-Difluoro-2-azaspiro[3. 3]heptane trifluoroac etate | 550.2 [M+H]⁺ |
| | | | (CAS 1427367-47-2) | |
| 403 | 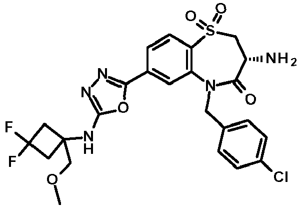 | (3R)-3-amino-5-[(4-chlorophenyl)methyl]-7-[5-[[3,3-difluoro-1-(methoxymethyl)cyclobutyl]a mino]-1,3,4-oxadiazol-2-yl]-1,1-dioxo-2,3-dihydro-1λ⁶,5-benzothiazepin-4-one (*) | 3,3-Difluoro-1-(methoxy methyl)cyc lobutane-1-amine | 568.2 [M+H]⁺ |
| | | | (CAS 1408076-03-8) | |
| 404 | 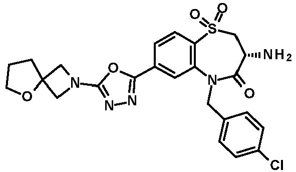 | (3R)-3-amino-5-[(4-chlorophenyl)methyl]-7-[5-(5-oxa-2-azaspiro[3.4]octan-2-yl)-1,3,4-oxadiazol-2-yl]-1,1-dioxo-2,3-dihydro-1λ⁶,5-benzothiazepin-4-one (*) | 5-Oxa-2-azaspiro[3. 4]octane | 530.2 [M+H]⁺ |
| | | | (CAS 145309-24-6) | |
| 405 | 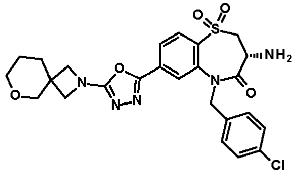 | (3R)-3-amino-5-[(4-chlorophenyl)methyl]-7-[5-(6-oxa-2-azaspiro[3.5]nonan-2-yl)-1,3,4-oxadiazol-2-yl]-1,1-dioxo-2,3-dihydro-1λ⁶,5-benzothiazepin-4-one (*) | 5-Oxa-2-azaspiro[3. 5]nonane | 544.2 [M+H]⁺ |
| | | | (CAS 138387-19-6) | |
| 406 | 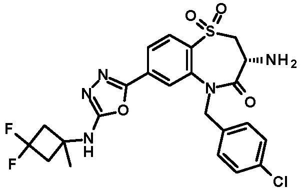 | (3R)-3-amino-5-[(4-chlorophenyl)methyl]-7-[5-[(3,3-difluoro-1-methylcyclobutyl)amino]-1,3,4-oxadiazol-2-yl]-1,1-dioxo-2,3-dihydro-1λ⁶,5-benzothiazepin-4-one (*) | 3,3-Difluoro-1-methylcycl obutane-1-amine hydrochlor ide | 538.1 [M+H]⁺ |
| | | | (CAS 1408076-03-8) | |
| 407 | 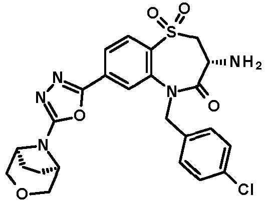 | (3R)-3-amino-5-[(4-chlorophenyl)methyl]-7-[5-[(1R,5S)-3-oxa-8-azabicyclo[3.2.1]octan-8-yl]-1,3,4-oxadiazol-2-yl]-1,1-dioxo-2,3-dihydro-1λ⁶,5-benzothiazepin-4-one | 3-Oxa-8-azabicyclo[ 3.2.1]octa ne hydrochlor ide | 530.4 [M+H]⁺ |
| | | | (CAS 280-07-9) | |
| 408 | 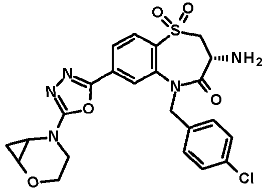 | (3R)-3-amino-5-[(4-chlorophenyl)methyl]-7-[5-(2-oxa-5-azabicyclo[4.1.0]heptan-5-yl)-1,3,4-oxadiazol-2-yl]-1,1-dioxo-2,3-dihydro-1λ⁶,5-benzothiazepin-4-one | 2-Oxa-5-azabicyclo[ 4.1.0]hept ane hydrochlor ide | 516.3 [M+H]⁺ |
| | | | (CAS 1354952-28-5) | |
| 409 | 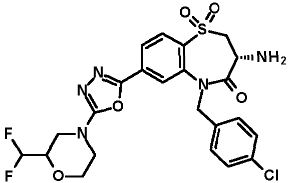 | (3R)-3-amino-5-[(4-chlorophenyl)methyl]-7-[5-[2-(difluoromethyl)morpholin-4-yl]-1,3,4-oxadiazol-2-yl]-1,1-dioxo-2,3-dihydro-1λ⁶,5-benzothiazepin-4-one | 2-(Difluorom ethyl)morp holine hydrochlor ide | 554.2 [M+H]⁺ |
| | | | (CAS 2095408-94-7) | |
| 410 | 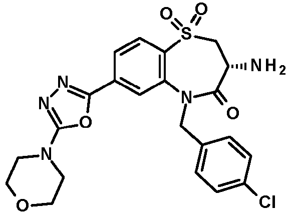 | (3R)-3-amino-5-[(4-chlorophenyl)methyl]-7-(5-morpholino-1,3,4-oxadiazol-2-yl)-1,1-dioxo-2,3-dihydro-1λ⁶,5-benzothiazepin-4-one | Morpholin e | 504.2 [M+H]⁺ |
| | | | (CAS 110-91-8) | |
| 411 | 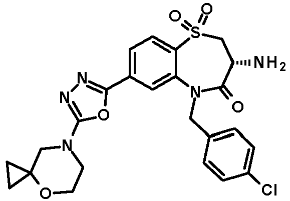 | (3R)-3-amino-5-[(4-chlorophenyl)methyl]-7-[5-(4-oxa-7-azaspiro[2.5]octan-7-yl)-1,3,4-oxadiazol-2-yl]-1,1-dioxo-2,3-dihydro-1λ⁶,5-benzothiazepin-4-one (*) | 4-Oxa-7-azasprio[2. 5]octane hydrochlor ide | 530.3 [M+H]⁺ |
| | | | (CAS 1427195-23-0) | |
| 412 | 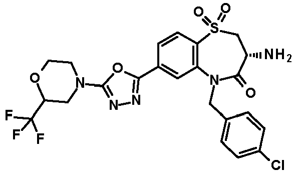 | (3R)-3-amino-5-[(4-chlorophenyl)methyl]-1,1-dioxo-7-[5-[2-(trifluoromethyl)morpholin-4-yl]-1,3,4-oxadiazol-2-yl]-2,3-dihydro-1λ⁶,5-benzothiazepin-4-one | 2-(Trifluoro methyl)mo rpholine hydrochlor ide | 572.2 [M+H]⁺ |
| | | | (CAS 1196152-51-8) | |

| | | | | |
|---|---|---|---|---|
| (*) as hydrochloride salt | | | | |

### Example 413

### (3R)-3-amino-5-[(4-chlorophenyl)methyl]-7-[5-(2,2-difluoromorpholin-4-yl)-1,3,4-oxadiazol-2-yl]-1,1-dioxo-2,3-dihydro-1λ⁶,5-benzothiazepin-4-one

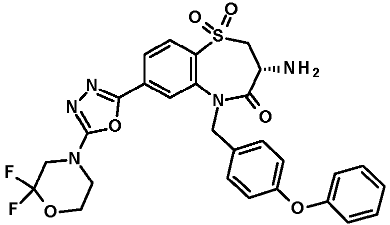

### Step a) methyl (3R)-3-(tert-butoxycarbonylamino)-4-oxo-5-[(4-phenoxyphenyl)methyl]-2,3-dihydro-1,5-benzothiazepine-7-carboxylate

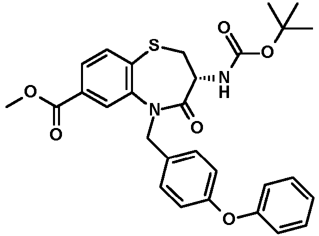

To a solution methyl (3R)-3-(tert-butoxycarbonylamino)-4-oxo-3,5-dihydro-2H-1,5-benzothiazepine-7-carboxylate (300 mg, 0.851 mmol, 1 eq) (CAS: 2089150-62-7) in DMSO (3.54 mL) was added 1-(bromomethyl)-4-phenoxy-benzene (336. mg, 1.28 mmol, 1.5 eq), potassium carbonate (352.95 mg, 2.55 mmol, 3 eq) and potassium iodide (70.66 mg, 0.426 mmol, 0.5 eq) at RT and the reaction was stirred for 24 h. The mixture was partitioned between EtOAc (75ml) and sat. NaHCO₃ (50ml). The layers were separated and the aqueous layer was extracted with one 50 ml portions of EtOAc. The combined organic layers were washed with one 25 ml portion of water and one 25 ml portion of sat. NH₄Cl, dried over sodium sulfate and concentrated. The crude material was purified by column chromatography on silica gel (0-30% EtOAc in heptane) to afford methyl (3R)-3-(tert-butoxycarbonylamino)-4-oxo-5-[(4-phenoxyphenyl)methyl]-2,3-dihydro-1,5-benzothiazepine-7-carboxylate (250 mg, 22% yield) as brown oil. MS (ESI): 535.4 [M+H]⁺.

### Step b) (3R)-3-(tert-butoxycarbonylamino)-4-oxo-5-[(4-phenoxyphenyl)methyl]-2,3-dihydro-1,5-benzothiazepine-7-carboxylic acid

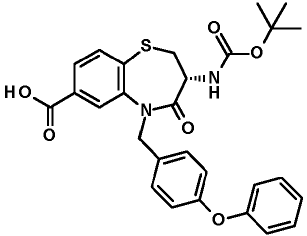

To a yellow suspension of methyl (3R)-3-(tert-butoxycarbonylamino)-4-oxo-5-[(4-phenoxyphenyl)methyl]-2,3-dihydro-1,5-benzothiazepine-7-carboxylate (250 mg, 0.187 mmol, 1 eq) in THF (1 mL) and water (0.5 mL) was added 1 M LiOH (196.4 uL, 0.196 mmol, 1.05 eq) at RT and the reaction mixture was stirred for 2 h. The reaction mixture was concentrated to remove most of THF and to the remaining solution was added EtOAc (50 ml) and 1M HCl (30 ml). The layers were separated and the aqueous layer was extracted with two 50 ml portions of EtOAc. The combined organic layers were washed with one 50 ml portion of brine, dried over sodium sulfate and concentrated in vacuo. The crude material was purified by column chromatography on silica gel (0-20% EtOAc in heptane) to afford (3R)-3-(tert-butoxycarbonylamino)-4-oxo-5-[(4-phenoxyphenyl)methyl]-2,3-dihydro-1,5-benzothiazepine-7-carboxylic acid (31 mg, 26% yield) as white solid. MS (ESI): 519.3 [M-H]⁻.

### Step c) tert-butyl N-[(3R)-7-(hydrazinecarbonyl)-4-oxo-5-[(4-phenoxyphenyl)methyl]-2,3-dihydro-1,5-benzothiazepin-3-yl]carbamate

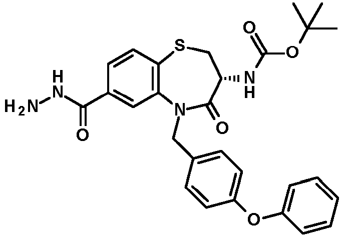

(3R)-3-(tert-butoxycarbonylamino)-4-oxo-5-[(4-phenoxyphenyl)methyl]-2,3-dihydro-1,5-benzothiazepine-7-carboxylic acid (31 mg, 0.057 mmol, 1 eq) was dissolved in THF (0.245 mL) and CDI (13.9 mg, 0.086 mmol, 1.5 eq) was added in one portion to give a yellow solution which was stirred for 90 min at RT. To this solution, a mixture of hydrazine hydrate (13.41 mg, 13. uL, 0.171 mmol, 3 eq) in THF (0.041 mL) was added and stirred for 90 min. The reaction mixture was partitioned between EtOAc and water. The layers were separated and the aqueous layer was extracted with two portions of EtOAc. The combined organic layers were washed with brine, dried over sodium sulfate and concentrated in vacuo to afford tert-butyl N-[(3R)-7-(hydrazinecarbonyl)-4-oxo-5-[(4-phenoxyphenyl)methyl]-2,3-dihydro-1,5-benzothiazepin-3-yl]carbamate (44 mg, 101%) as light yellow solid. MS (ESI): 479.3 [M+H-isobutene]⁺.

### Step a) tert-butylN-[(3R)-7-[[(2,2-difluoromorpholine-4-carbonyl)amino]carbamoyl]-4-oxo-5-[(4-phenoxyphenyl)methyl]-2,3-dihydro-1,5-benzothiazepin-3-yl]carbamate

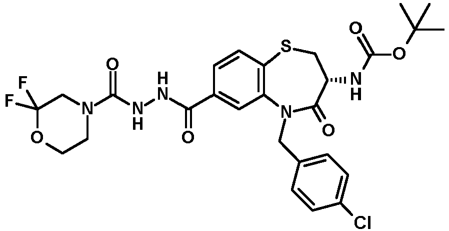

To a solution of 2,2-difluoromorpholine hydrochloride (CAS 1820647-38-8, 150 mg, 0.940 mmol, Eq: 1) and pyridine (223.08 mg, 228.1 uL, 2.82 mmol, Eq: 3) in 1,2-dichloroethane (4 mL) was added triphosgene (278.97 mg, 0.940 mmol, Eq: 1) at 0 °C in one portion. The cooling bath was removed and stirring was continued for 2 h. The reaction mixture was then partitioned between DCM (50 ml) and 1M HCl (25 ml). The layers were separated. The aqueous layer was extracted with two 20 ml portions of DCM and the combined organic layers were dried over anhydrous sodium sulfate and concentrated in vacuo. The residue was dissolved in 1,2-dichloroethane (4 mL) and tert-butyl N-[(3R)-7-(hydrazinecarbonyl)-4-oxo-5-[(4-phenoxyphenyl)methyl]-2,3-dihydro-1,5-benzothiazepin-3-yl]carbamate (66 mg, 0.123 mmol, Eq: 0.13) was added and the reaction mixture was stirred overnight. The reaction mixture was partitioned between EtOAc (50 ml) and water (25 ml). The layers were separated and the aqueous layer was extracted with two 20 ml portions of EtOAc. The combined organic layers were dried over anhydrous sodium sulfate and concentrated in vacuo to afford a light yellow oil (224 mg) containing the title compound, which was used in the next step without further purification. MS (ESI): 682.4 [M-H]⁻.

### Step b) tert-butyl N-[(3R)-7-[5-(2,2-difluoromorpholin-4-yl)-1,3,4-oxadiazol-2-yl]-4-oxo-5-[(4-phenoxyphenyl)methyl]-2,3-dihydro-1,5-benzothiazepin-3-yl]carbamate

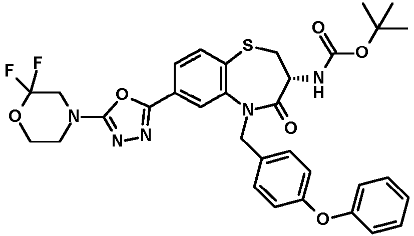

tert-butyl N-[(3R)-7-[[(2,2-difluoromorpholine-4-carbonyl)amino]carbamoyl]-4-oxo-5-[(4-phenoxyphenyl)methyl]-2,3-dihydro-1,5-benzothiazepin-3-yl]carbamate (224 mg, 0.229 mmol, Eq: 1) in acetonitrile (3.93 mL) was added p-toluenesulfonyl chloride (131.17 mg, 0.688 mmol, Eq: 3) and DIPEA (59.28 mg, 0.459 mmol, Eq: 2). The reaction mixture was stirred at RT for 4 h and then partitioned between EtOAc (50 ml) and water (40 ml). The layers were separated and the aqueous layer was extracted with two 40 ml portions of EtOAc. The combined organic layers were washed with one 20 ml portion of brine, dried over anhydrous sodium sulfate and concentrated in vacuo. The crude material was purified by column chromatography on silica gel (0-50% EtOAc in heptane) to afford tert-butyl N-[(3R)-7-[5-(2,2-difluoromorpholin-4-yl)-1,3,4-oxadiazol-2-yl]-4-oxo-5-[(4-phenoxyphenyl)methyl]-2,3-dihydro-1,5-benzothiazepin-3-yl]carbamate (86.4 mg, 56% yield) as white solid. MS (ESI): 666.4 [M+H]⁺.

### Step c) tert-butyl N-[(3R)-7-[5-(2,2-difluoromorpholin-4-yl)-1,3,4-oxadiazol-2-yl]-1,1,4-trioxo-5-[(4-phenoxyphenyl)methyl]-2,3-dihydro-1λ⁶,5-benzothiazepin-3-yl]carbamate

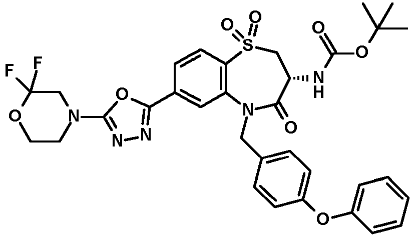

The title compound was prepared tert-butyl N-[(3R)-7-[5-(2,2-difluoromorpholin-4-yl)-1,3,4-oxadiazol-2-yl]-4-oxo-5-[(4-phenoxyphenyl)methyl]-2,3-dihydro-1,5-benzothiazepin-3-yl]carbamate (86.4 mg, 0.128 mmol) in analogy to general procedure 5 and was obtained as white solid (88 mg, 93% yield). MS (ESI): 642.3 [M+H-isobutene]⁺.

### Step d) (3R)-3-amino-7-[5-(2, 2-difluoromorpholin-4-yl)-1,3, 4-oxadiazol-2-yl]-1, 1-dioxo-5-[(4-phenoxyphenyl)methyl]-2,3-dihydro-1λ⁶,5-benzothiazepin-4-one

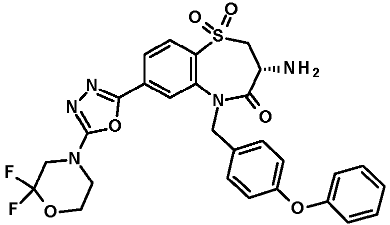

The title compound was prepared from tert-butyl N-[(3R)-7-[5-(2,2-difluoromorpholin-4-yl)-1,3,4-oxadiazol-2-yl]-1,1,4-trioxo-5-[(4-phenoxyphenyl)methyl]-2,3-dihydro-1λ⁶,5-benzothiazepin-3-yl]carbamate (88 mg, 0.126 mmol) in analogy to general procedure 6d and was obtained as white solid, as hydrochloride salt (58.9 mg, 71% yield). MS (ESI): 598.3 [M+H]⁺.

### Example 414

### (3R)-3-amino-7-[5-(1-aminocyclopentyl)-1,3,4-oxadiazol-2-yl]-1,1-dioxo-5-[[4-(trifluoromethoxy)phenyl]methyl]-2,3-dihydro-1λ⁶,5-benzothiazepin-4-one

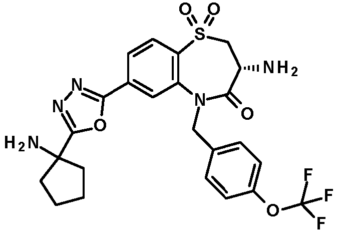

### Step a) methyl (3R)-3-(tert-butoxycarbonylamino)-4-oxo-5-[[4-(trifluoromethoxy)phenyl]methyl]-2, 3-dihydro-1, 5-benzothiazepine-7-carboxylate

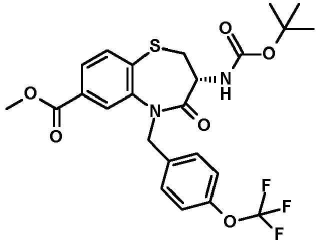

To a solution of methyl (3R)-3-(tert-butoxycarbonylamino)-4-oxo-3,5-dihydro-2H-1,5-benzothiazepine-7-carboxylate (5 g, 14.19 mmol, 1 eq) (CAS: 2089150-62-7) in DMSO (55 mL) was added 1-(bromomethyl)-4-(trifluoromethoxy)benzene (5.43 g, 21.28 mmol, 1.5 eq), potassium carbonate (5.88 g, 42.56 mmol, 3 eq) and potassium iodide (1.18 g, 7.09 mmol, 0.500 eq) at RT and the reaction mixture was stirred overnight. Water (200 ml) and EtOAc (200 ml) were added and the layers were separated. The aqueous layer was extracted with EtOAc (2x100 ml). The combined organic layers were washed with water (100 ml), aq. sat. NH₄Cl (100 ml), dried over anhydrous sodium sulfate and concentrated in vacuo. The crude material was purified by column chromatography on silica gel (0-30% EtOAc in heptane) to afford methyl (3R)-3-(tert-butoxycarbonylamino)-4-oxo-5-[[4-(trifluoromethoxy)phenyl]methyl]-2,3-dihydro-1,5-benzothiazepine-7-carboxylate (7.1 g, 93% yield) as orange waxy solid. MS (ESI): 471.2 [M+H-isobutene]⁺.

### Step b) (3R)-3-(tert-butoxycarbonylamino)-4-oxo-5-[[4-(trifluoromethoxy)phenyl]methyl]-2,3-dihydro-1,5-benzothiazepine-7-carboxylic acid

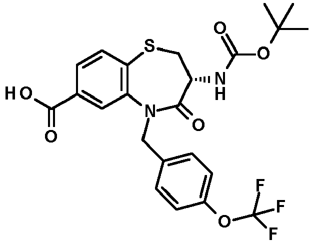

To a solution of methyl (3R)-3-(tert-butoxycarbonylamino)-4-oxo-5-[[4-(trifluoromethoxy)phenyl]methyl]-2,3-dihydro-1,5-benzothiazepine-7-carboxylate (7.1 g, 13.48 mmol, 1 eq) in THF (66 mL), MeOH (11 mL) and water (18 mL) was added lithium hydroxide hydrate (647 mg, 27.01 mmol, 2 eq) and the mixture was stirred at RT for 3 hr. Organic solvent was evaporated and 1M HCl (100 ml) and EtOAc (100 ml) was added. The layers were separated and the aqueous layer extracted with EtOAc (2x). The combined organic layers were dried over sodium sulfate, filtered and concentrated in vacuo to yield (3R)-3-(tert-butoxycarbonylamino)-4-oxo-5-[[4-(trifluoromethoxy)phenyl]methyl]-2,3-dihydro-1,5-benzothiazepine-7-carboxylic acid (6.79 g, 96% yield) as off-white solid. MS (ESI): 457.1 [M+H-isobutene]⁺.

### Step c) tert-butyl N-[(3R)-7-(hydrazinecarbonyl)-4-oxo-5-[[4-(trifluoromethoxy)phenyl]methyl]-2,3-dihydro-1,5-benzothiazepin-3-yl]carbamate

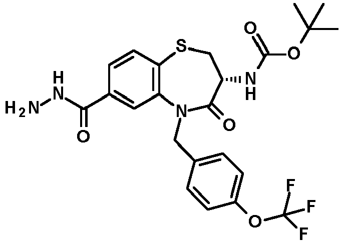

To a solution of (3R)-3-(tert-butoxycarbonylamino)-4-oxo-5-[[4-(trifluoromethoxy)phenyl]methyl]-2,3-dihydro-1,5-benzothiazepine-7-carboxylic acid (160 mg, 3.12 mmol, 1 eq) in THF (10 mL) was added CDI (758.87 mg, 4.68 mmol, 1.5 eq) and the mixture was stirred at 40 °C for 1 h. After that, the solution was added dropwise to a stirring solution of hydrazine monohydrate (318.89 mg, 6.24 mmol, 2 eq) in THF (10 mL). The resulting mixture was stirred at RT for 1 h. The reaction was poured into water (30 ml) and extracted with EtOAc (3x30 ml). The combined organic layers were washed with water (2x20 ml) and brine (20 ml), dried over sodium sulfate and concentrated in vacuum to afford tert-butyl N-[(3R)-7-(hydrazinecarbonyl)-4-oxo-5-[[4-(trifluoromethoxy)phenyl]methyl]-2,3-dihydro-1,5-benzothiazepin-3-yl]carbamate (1.4 g, 2.66 mmol, 85% yield) as light yellow oil. MS (ESI): 471.1 [M+H]⁺.

### Step d) tert-butyl N-[(3R)-7-[[[1-(tert-butoxycarbonylamino)cyclopentanecarbonyl]amino]carbamoyl]-4-oxo-5-[[4-(trifluoromethoxy)phenyl]methyl]-2, 3-dihydro-1, 5-benzothiazepin-3-yl]carbamate

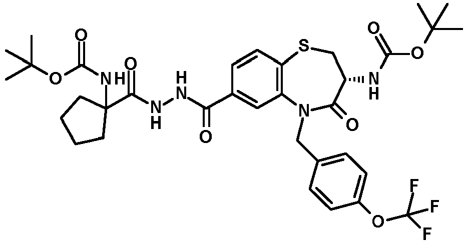

The title compound was prepared from tert-butyl N-[(3R)-7-(hydrazinecarbonyl)-4-oxo-5-[[4-(trifluoromethoxy)phenyl]methyl]-2,3-dihydro-1,5-benzothiazepin-3-yl]carbamate (200.0 mg, 0.380 mmol) and Boc-cycloleucine (95.8 mg, 0.420 mmol, 1.1 eq) in analogy to general procedure 7a and was obtained as light yellow oil (230 mg, 0.310 mmol, 82% yield) as light yellow oil. MS (ESI): 738.3 [M+H]⁺.

### Step e) tert-butyl N-[(3R)-7-[5-[1-(tert-butoxycarbonylamino)cyclopentyl]-1, 3, 4-oxadiazol-2-yl]-4-oxo-5-[[4-(trifluoromethoxy)phenyl]methyl]-2,3-dihydro-1, 5-benzothiazepin-3-yl]carbamate

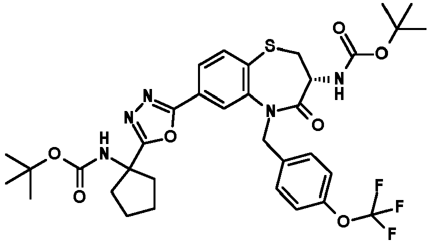

The title compound was prepared from tert-butyl N-[(3R)-7-[[[1-(tert-butoxycarbonylamino)cyclopentanecarbonyl]amino]carbamoyl]-4-oxo-5-[[4-(trifluoromethoxy)phenyl]methyl]-2,3-dihydro-1,5-benzothiazepin-3-yl]carbamate (220 mg, 0.3 mmol) in analogy to general procedure 8a and was obtained as light yellow oil (200 mg, 0.28 mmol, 93% yield). MS (ESI): 720.3 [M+H]⁺.

### Step f) tert-butyl N-[(3R)-7-[5-[1-(tert-butoxycarbonylamino)cyclopentyl]-1, 3,4-oxadiazol-2-yl]-1,1,4-trioxo-5-[[4-(trifluoromethoxy)phenyl]methyl]-2, 3-dihydro-1λ⁶,5-benzothiazepin-3-yl]carbamate

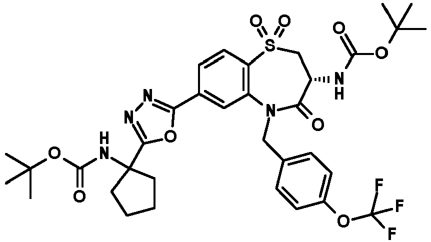

The title compound was prepared form tert-butyl N-[(3R)-7-[5-[1-(tert-butoxycarbonylamino)cyclopentyl]-1,3,4-oxadiazol-2-yl]-4-oxo-5-[[4-(trifluoromethoxy)phenyl]methyl]-2,3-dihydro-1,5-benzothiazepin-3-yl]carbamate (200.0 mg, 0.280 mmol) in analogy to general procedure 5 and was obtained as light yellow solid (160 mg, 0.210 mmol, 76.59% yield). MS (ESI): 752.6 [M+H]⁺.

### Step g) (3R)-3-amino-7-[5-(1-aminocyclopentyl)-1,3, 4-oxadiazol-2 -yl]-1,1-dioxo-5-[[4-(trifluoromethoxy)phenyl]methyl]-2, 3-dihydro-1λ⁶,5-benzothiazepin-4-one

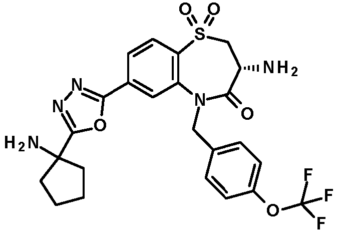

The title compound was prepared from tert-butyl N-[(3R)-7-[5-[1-(tert-butoxycarbonylamino)cyclopentyl]-1,3,4-oxadiazol-2-yl]-1,1,4-trioxo-5-[[4-(trifluoromethoxy)phenyl]methyl]-2,3-dihydro-1λ⁶,5-benzothiazepin-3-yl]carbamate (150.0 mg, 0.200 mmol) in analogy to general procedure 6b and was obtained as white solid (63.5 mg, 0.120 mmol, 57% yield). MS (ESI): 552.2 [M+H]⁺.

The examples 415 to 420 of the following table were prepared in analogy to Example 414, using the appropriate carboxylic acid building block.

| Ex. | Structure | Systematic Name | Building **Block** | MS, ESI: m/z |
|---|---|---|---|---|
| 415 | 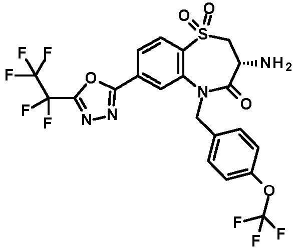 | (3R)-3-amino-1,1-dioxo-7-[5-(1,1,2,2,2-pentafluoroethyl)-1,3,4-oxadiazol-2-yl]-5-[[4-(trifluoromethoxy)phenyl]met hyl]-2,3-dihydro-1λ⁶,5-benzothiazepin-4-one (*) | Pentafluor opropionic acid | 587.1 [M+H]⁺ . |
| | | | (CAS 422-64-0) | |
| 416 | 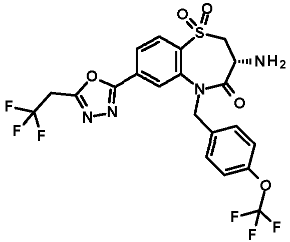 | (3R)-3-amino-1,1-dioxo-7-[5-(2,2,2-trifluoroethyl)-1,3,4-oxadiazol-2-yl]-5-[[4-(trifluoromethoxy)phenyl]met hyl]-2,3-dihydro-1λ⁶,5-benzothiazepin-4-one (*) | 3,3,3-Trifluoropr opionic acid | 551.1 [M+H]⁺ |
| | | | (CAS 2516-99-6) | |
| 417 | 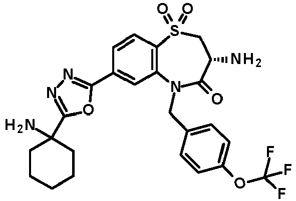 | (3R)-3-amino-7-[5-(1-aminocyclohexyl)-1,3,4-oxadiazol-2-yl]-1,1-dioxo-5-[[4-(trifluoromethoxy)phenyl]met hyl]-2,3-dihydro-1λ⁶,5-benzothiazepin-4-one | 1-(Boc-amino) cyclohexan ecarboxylic acid | 566.4 [M+H]⁺ |
| | | | (CAS 115951-16-1) | |
| 418 | 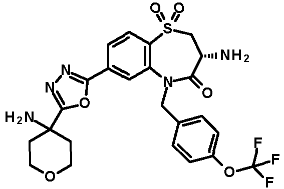 | (3R)-3-amino-7-[5-(4-aminotetrahydropyran-4-yl)-1,3,4-oxadiazol-2-yl]-1,1-dioxo-5-[[4-(trifluoromethoxy)phenyl]met hyl]-2,3-dihydro-1λ⁶,5-benzothiazepin-4-one | 4-((tert-Butoxycab onyl)amin o)tetrahyd ro-2H-pyran-4-carboxylic acid | 568.4 [M+H]⁺ |
| | | | (CAS 172843-97-9) | |
| 419 | 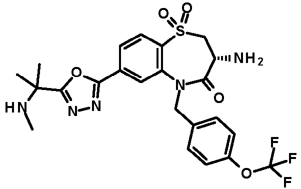 | (3R)-3-amino-7-[5-[1-methyl-1-(methylamino)ethyl]-1,3,4-oxadiazol-2-yl]-1,1-dioxo-5-[[4-(trifluoromethoxy)phenyl]met hyl]-2,3-dihydro-1λ⁶,5-benzothiazepin-4-one | 2-Methyl-2-(methylam ino)propan oic acid | 568.4 [M+H]⁺ |
| | | | (CAS 2566-34-9) | |
| 420 | 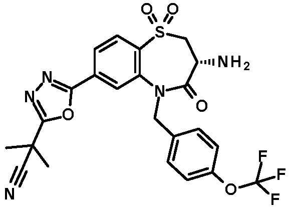 | 2-[5-[(3R)-3-amino-1,1,4-trioxo-5-[[4-(trifluoromethoxy)phenyl)met hyl]-2,3-dihydro-1λ⁶,5-benzothiazepin-7-yl]-1,3,4-oxadiazol-2-yl]-2-methylpropanenitrile (*) | 2-Cyano-2-methylpro panoic acid | 631.3 [M+H]⁺ |
| | | | (CAS 22426-30-8) | |

| | | | | |
|---|---|---|---|---|
| (*) as hydrochloride salt | | | | |

### Example 421

### (3R)-3-amino-7-[5-(5,5-difluoro-1-methyl-3-piperidyl)-1,3,4-oxadiazol-2-yl]-1,1-dioxo-5-[[4-(trifluoromethoxy)phenyl]methyl]-2,3-dihydro-1λ⁶,5-benzothiazepin-4-one

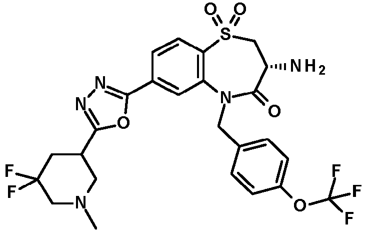

### Step a) benzyl 5-[5-[(3R)-3-(tert-butoxycarbonylamino)-4-oxo-5-[[4-(trifluoromethoxy)phenyl]methyl]-2,3-dihydro-1,5-benzothiazepin-7-yl]-1,3,4-oxadiazol-2-yl]-3,3-difluoro-piperidine-1-carboxylate

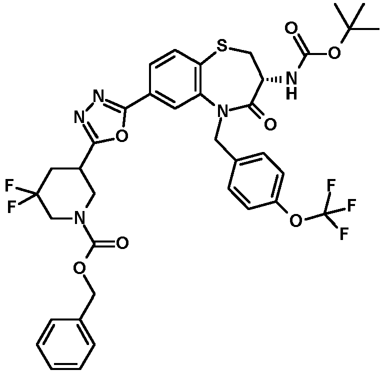

tert-butyl N-[(3R)-7(hydrazinecarbonyl)-4-oxo-5-[[4-(trifluoromethoxy)phenyl]methyl]-2,3-dihydro-1,5-benzothiazepin-3-yl]carbamate (200 mg, 0.380 mmol, 1 eq, Example 414, step c) was dissolved in THF (3.76 mL) and 1-carbobenzoxy-5,5-difluoro-nipecotic acid (125.04 mg, 0.418 mmol, 1.1 eq), HATU (158.87 mg, 0.418 mmol, 1.1 eq) and DIPEA (98.18 mg, 132.68 uL, 0.760 mmol, 2 eq) were added to give a yellow solution. The mixture was stirred for 45 minutes at RT. Burgess reagent (452.59 mg, 1.9 mmol, 5 eq) was added and the reaction mixture stirred for 60 min at RT. The solution was concentrated and the crude purified by column chromatography on silica gel (0-100% EtOAc in heptane) to give benzyl 5-[5-[(3R)-3-(tert-butoxycarbonylamino)-4-oxo-5-[[4-(trifluoromethoxy)phenyl]methyl]-2,3-dihydro-1,5-benzothiazepin-7-yl]-1,3,4-oxadiazol-2-yl]-3,3-difluoro-piperidine-1-carboxylate (217 mg, 65% yield) as light yellow solid. MS (ESI): 790.4 [M+H]⁺.

### Step b) benzyl 3, 3-difluoro-5-[5-[(3R)-3-(tert-butoxycarbonylamino)-1,1, 4-trioxo-5-[[4-(trifluoromethoxy)phenyl]methyl]-2, 3-dihydro-1λ⁶,5-benzothiazepin-7-yl]-1,3, 4-oxadiazol-2-yl]piperidine-1-carboxylate

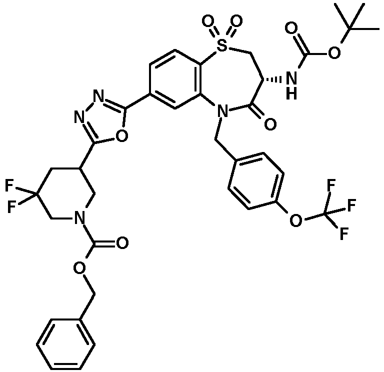

The title compound was prepared from benzyl 5-[5-[(3R)-3-(tert-butoxycarbonylamino)-4-oxo-5-[[4-(trifluoromethoxy)phenyl]methyl]-2,3-dihydro-1,5-benzothiazepin-7-yl]-1,3,4-oxadiazol-2-yl]-3,3-difluoro-piperidine-1-carboxylate (217 mg, 0.275 mmol) in analogy to general procedure 5 and was obtained as light yellow solid (161 mg, 64% yield). MS (ESI): 822.3 [M+H]⁺.

### Step c) tert-butyl N-[(3R)-7-[5-(5,5-difluoro-3-piperidyl)-1,3,4-oxadiazol-2-yl]-1,1, 4-trioxo-5-[[4-(trifluoromethoxy)phenyl]methyl]-2, 3-dihydro-1λ⁶,5-benzothiazepin-3-yl]carbamate

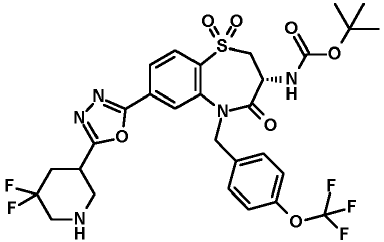

To a solution of benzyl 3,3-difluoro-5-[5-[(3R)-3-(tert-butoxycarbonylamino)-1,1,4-trioxo-5-[[4-(trifluoromethoxy)phenyl]methyl]-2,3-dihydro-1λ⁶,5-benzothiazepin-7-yl]-1,3,4-oxadiazol-2-yl]piperidine-1-carboxylate (185 mg, 0.225 mmol, 1 eq) in MeOH (6 mL) under Ar atmosphere was added Pd/C (47.92 mg, 0.023 mmol) and stirred under hydrogen atmosphere for 2 h. Themixture was filtered through celite and the filtrate concentrated to afford tert-butyl N-[(3R)-7-[5-(5,5-difluoro-3-piperidyl)-1,3,4-oxadiazol-2-yl]-1,1,4-trioxo-5-[[4-(trifluoromethoxy)phenyl]methyl]-2,3-dihydro-1λ⁶,5-benzothiazepin-3-yl]carbamate (78 mg, 46% yield) as light yellow solid. MS (ESI): 688.3 [M+H]⁺.

### Step d) tert-butyl N-[(3R)-7-[5-(5,5-difluoro-1-methyl-3-piperidyl)-1,3, 4-oxadiazol-2-yl]-1,1,4-trioxo-5-[[4-(trifluoromethoxy)phenyl]methyl]-2, 3-dihydro-1λ⁶,5-benzothiazepin-3-yl]carbamate

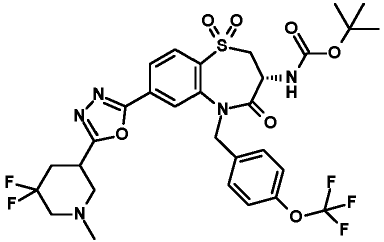

Tert-butyl N-[(3R)-7-[5-(5,5-difluoro-3-piperidyl)-1,3,4-oxadiazol-2-yl]-1,1,4-trioxo-5-[[4-(trifluoromethoxy)phenyl]methyl]-2,3-dihydro-1λ⁶,5-benzothiazepin-3-yl]carbamate (78 mg, 0.113 mmol, 1 eq) was dissolved in MeOH (1.07 mL) and formalin (35% in H₂O, 92.05 mg, 84.45 uL, 1.13 mmol, 10 eq) and sodium triacetoxyborohydride (240.41 mg, 1.13 mmol, 10 eq) were added. Gas formation was observed and the mixture was kept stirring at 22°CRT or 1 h. The solution was poured onto water (20 ml) and extracted with DCM (3x 30 ml). The combined organic layers were dried over sodium sulfate, filtered and concentrated. The crude was purified by column chromatography on silica geld (0-60% EtOAc in heptane) to afford tert-butyl N-[(3R)-7-[5-(5,5-difluoro-1-methyl-3-piperidyl)-1,3,4-oxadiazol-2-yl]-1,1,4-trioxo-5-[[4-(trifluoromethoxy)phenyl]methyl]-2,3-dihydro-1λ⁶,5-benzothiazepin-3-yl]carbamate (29 mg, 35% yield) as white solid. MS (ESI): 702.5 [M+H]⁺.

### Step e) (3R)-3-amino-7-[5-(5, 5-difluoro-1-methyl-3-piperidyl)-1, 3, 4-oxadiazol-2-yl]-1,1-dioxo-5-[[4-(trifluoromethoxy)phenyl]methyl]-2, 3-dihydro-1λ⁶,5-benzothiazepin-4-one

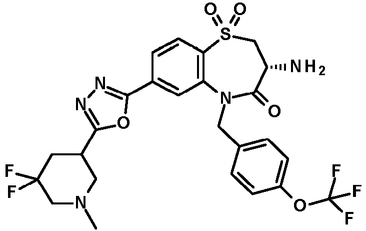

The title compound was prepared from tert-butyl N-[(3R)-7-[5-(5,5-difluoro-1-methyl-3-piperidyl)-1,3,4-oxadiazol-2-yl]-1,1,4-trioxo-5-[[4-(trifluoromethoxy)phenyl]methyl]-2,3-dihydro-1λ⁶,5-benzothiazepin-3-yl]carbamate (29 mg, 0.041 mmol) in analogy to general procedure 6d and was obtaines as white solid, as hydrochloride salt (25 mg, 90%). MS (ESI): 602.2 [M+H]⁺.

### Example 422

### Methyl 1-[5-[(3R)-3-amino-1,1,4-trioxo-5-[[4-(trifluoromethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate

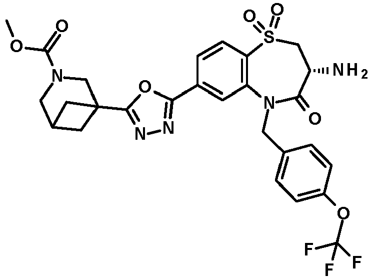

### Step a) tert-butyl 1-[[[(3R)-3-(tert-butoxycarbonylamino)-1, 1,4-trioxo-5-[[4-(trifluoromethoxy)phenyl]methyl]-2, 3-dihydro-1λ⁶,5-benzothiazepine-7-carbonyl]amino]carbamoyl]-3-azabicyclo[3.1. 1 Jheptane-3-carboxylate

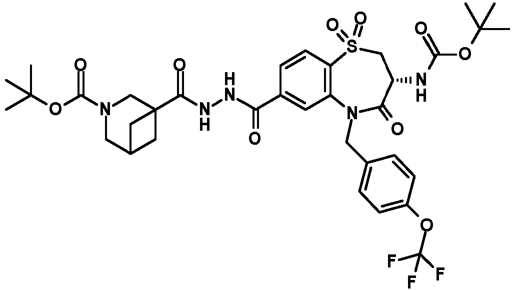

To a solution of tert-butyl N-[(3R)-7-(hydrazinecarbonyl)-4-oxo-5-[[4-(trifluoromethoxy)phenyl]methyl]-2,3-dihydro-1,5-benzothiazepin-3-yl]carbamate (170.0 mg, 0.320 mmol, 1 eq, Example 414, step c) in pyridine (4 mL) was added 3-tert-butoxycarbonyl-3-azabicyclo[3.1.1]heptane-1-carboxylic acid (95.0 mg, 0.390 mmol, 1.22 eq) and EDCI (198.55 mg, 1.04 mmol, 3.21 eq). The reaction mixture was stirred at RT for 1 h. The mixture was poured into water (20 mL) and extracted with EtOAc (2x20 mL). The combined orgnic layers were washed with brine (20 mL), dried over sodium sulfate, and concentrated in vacuo. The crude yellow residue was purified by column chromatography on silica gel (10-50% EtOAc in petroleum ether) to afford tert-butyl 1-[[[(3R)-3-(tert-butoxycarbonylamino)-1,1,4-trioxo-5-[[4-(trifluoromethoxy)phenyl]methyl]-2,3-dihydro-1λ⁶,5-benzothiazepine-7-carbonyl]amino]carbamoyl]-3-azabicyclo[3.1.1]heptane-3-carboxylate (250 mg, 0.330 mmol, 92% yield) as white solid. MS (ESI): 750.1 [M+H]⁺.

### Step b) tert-butyl 1-[5-[(3R)-3-(tert-butoxycarbonylamino)-4-oxo-5-[[4-(trifluoromethoxy)phenyl]methyl]-2,3-dihydro-1, 5-benzothiazepin-7-yl]-1,3,4-oxadiazol-2-yl]-3-azabicyclo[3.1.1]heptane-3-carboxylate

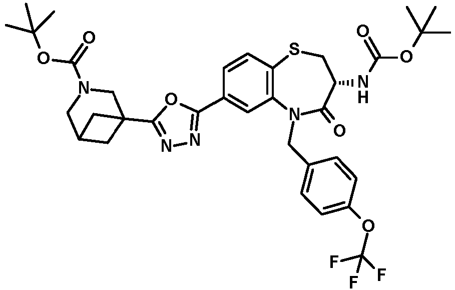

The title compound was prepared from tert-butyl 1-[[[(3R)-3-(tert-butoxycarbonylamino)-1,1,4-trioxo-5-[[4-(trifluoromethoxy)phenyl]methyl]-2,3-dihydro-1λ⁶,5-benzothiazepine-7-carbonyl]amino]carbamoyl]-3-azabicyclo[3.1.1]heptane-3-carboxylate (250.0 mg, 0.330 mmol) in analogy to general procedure 8a and was obtained as yellow oil (170 mg, 0.230 mmol, 70% yield). MS (ESI): 732.4 [M+H]⁺.

### Step c) tert-butyl 1-[5-[(3R)-3-(tert-butoxycarbonylamino)-1,1, 4-trioxo-5-[[4-(trifluoromethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate

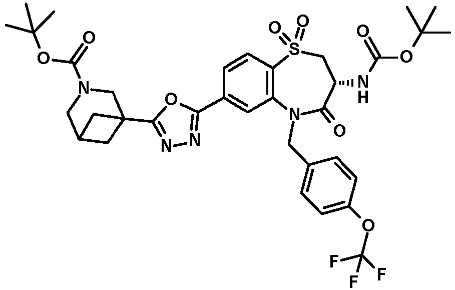

The title compound was prepared from tert-butyl 1-[5-[(3R)-3-(tert-butoxycarbonylamino)-4-oxo-5-[[4-(trifluoromethoxy)phenyl]methyl]-2,3-dihydro-1,5-benzothiazepin-7-yl]-1,3,4-oxadiazol-2-yl]-3-azabicyclo[3.1.1]heptane-3-carboxylate (150.0 mg, 0.200 mmol) in analogy to general procedure 5 and was obtained as white solid (137 mg, 0.180 mmol, 88% yield). MS (ESI): 664.0 [M+H-Boc]⁺.

### Step d) (3R)-3-amino-7-[5-(3-azabicyclo[3.1.1]heptan-1-yl)-1,3, 4-oxadiazol-2-yl]-1,1-dioxo-5-[[4-(trifluoromethoxy)phenyl]methyl]-2, 3-dihydro-1λ⁶,5-benzothiazepin-4-one

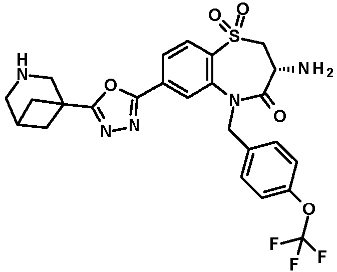

The titel compound was prepared from tert-butyl 1-[5-[(3R)-3-(tert-butoxycarbonylamino)-1,1,4-trioxo-5-[[4-(trifluoromethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate (120.0 mg, 0.160 mmol) in analogy to general procedure 6b and was obtained as white solid, as dihydrochloride salt (75 mg, 0.120 mmol, 85% yield). MS (ESI): 564.2 [M+H]⁺.

### Step e) methyl 1-[5-[(3R)-3-amino-1,1,4-trioxo-5-[[4-(trifluoromethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate

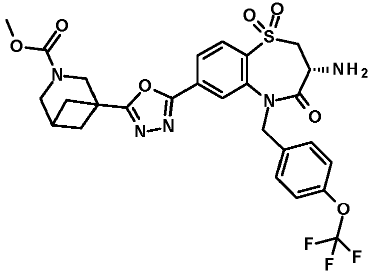

To a solution of (3R)-3-amino-7-[5-(3-azabicyclo[3.1.1]heptan-1-yl)-1,3,4-oxadiazol-2-yl]-1,1-dioxo-5-[[4-(trifluoromethoxy)phenyl]methyl]-2,3-dihydro-1λ⁶,5-benzothiazepin-4-one (35 mg, 0.05 mmol, 1 eq) in DCM (2 mL) was added DIPEA (25 mg, 0.19 mmol, 3.5 eq) and methyl chloroformate (6 mg, 0.06 mmol, 1.15 eq). The reaction was stirred at RT for 1 h. The reaction mixture was concentrated in vacuo to afford the crude as white solid which was purified by prep-HPLC to afford methyl 1-[5-[(3R)-3-amino-1,1,4-trioxo-5-[[4-(trifluoromethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate (18.7 mg, 0.03 mmol, 55% yield) as white solid. MS (ESI): 622.2 [M+H]⁺.

Example 423 of the following table was prepared in analogy to Example 422, using the appropriate carboxylic acid building block.

| **Ex.** | **Structure** | **Systematic Name** | **Building Block** | **MS, ESI: m/z** |
|---|---|---|---|---|
| 423 | 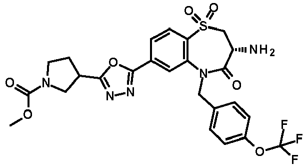 | methyl 3-[5-[(3R)-3-amino-1,1,4-trioxo-5-[[4-(trifluoromethoxy)phenyl)met hyl]-2,3-dihydro-1λ⁶,5-benzothiazepin-7-yl]-1,3,4-oxadiazol-2-yl]pyrrolidine-1-carboxylate | 1-(tert-butoxycarb onyl)pyrrol idine-3-carboxylic acid | 596.4 [M+H]⁺ . |
| | | | (CAS 59378-75-5) | |
| | | (*) | | |

| | | | | |
|---|---|---|---|---|
| (*) as hydrochloride salt | | | | |

### Example 424

### (3R)-3-amino-1,1-dioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1λ⁶,5-benzothiazepin-4-one

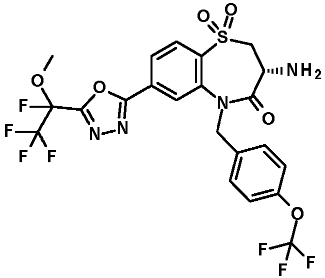

### Step a) tert-butyl N-[(3R)-4-oxo-7-[[(2, 3,3,3-tetrafluoro-2-methoxy-propanoyl)amino[carbamoyl[-5-[[4-(trifluoromethoxy)phenyl[methyl]-2,3-dihydro-1,5-benzothiazepin-3-yl[carbamate

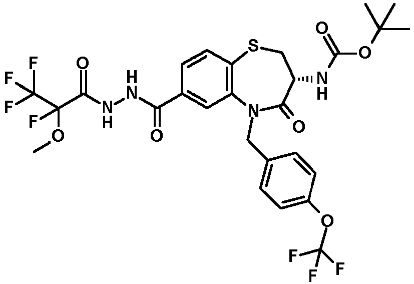

(3R)-3-(tert-butoxycarbonylamino)-4-oxo-5-[[4-(trifluoromethoxy)phenyl]methyl]-2,3-dihydro-1,5-benzothiazepine-7-carboxylic acid (100 mg, 0.191 mmol, 1 eq, Example 414, step b) was stirred with 2,3,3,3-tetrafluoro-2-methoxy-propanehydrazide (48.72 mg, 0.249 mmol, 1.3 eq, Example 388, step a) and DIPEA (49.43 mg, 65.47 uL, 0.38 mmol, 2 eq) in THF (1.58 mL) at RT. HATU (109.06 mg, 0.287 mmol, 1.5 eq) was added and stirring was continued for 16 h. The crude material was diluted with EtOAc and water and the layers were separated. The aqueous phase was extracted with EtOAc (2x). The combined organic layers were dried over sodium sulfate, filtered and concentrated. The crude was purified by column chromatography on silica gel (0%-100% EtOAc in heptane) to give tert-butyl N-[(3R)-4-oxo-7-[[(2,3,3,3-tetrafluoro-2-methoxy-propanoyl)amino]carbamoyl]-5-[[4-(trifluoromethoxy)phenyl]methyl]-2,3-dihydro-1,5-benzothiazepin-3-yl]carbamate (94 mg, 72% yield) as white solid. MS (ESI): 683.3 [M-H]⁻.

### Step b) tert-butyl N-[(3R)-4-oxo-7-[5-(1,2,2, 2-tetrafluoro-1-methoxy-ethyl)-1,3,4-oxadiazol-2-yl]-5-[[4-(trifluoromethoxy)phenylmethyl]-2,3-dihydro-1,5-benzothiazepin-3-yl[carbamate

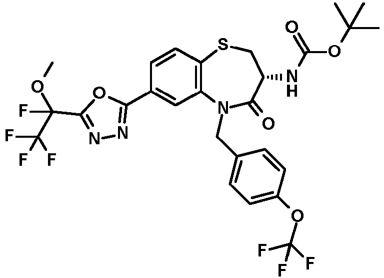

The title compound was prepared from tert-butyl N-[(3R)-4-oxo-7-[[(2,3,3,3-tetrafluoro-2-methoxy-propanoyl)amino]carbamoyl]-5-[[4-(trifluoromethoxy)phenyl]methyl]-2,3-dihydro-1,5-benzothiazepin-3-yl]carbamate (100 mg, 0.146 mmol) in analogy to general procedure 8a and was obtained as colourless foam (65 mg, 67% yield). MS (ESI): 611.3 [M+H-isobutene]⁺.

### Step c) tert-butyl N-[(3R)-1,1, 4-trioxo-7-[5-(1, 2,2, 2-tetrafluoro-1-methoxy-ethyl)-1, 3,4-oxadiazol-2-yl]-5-[[4-(trifluoromethoxy)phenyl]methyl]-2,3-dihydro-1λ⁶,5-benzothiazepin-3-yl]carbamate

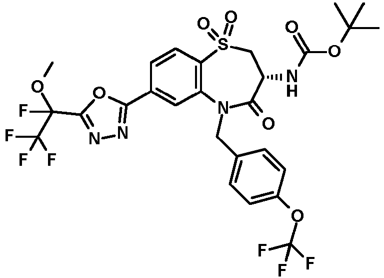

The title compound was prepared from tert-butyl N-[(3R)-4-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1,5-benzothiazepin-3-yl]carbamate (52 mg, 0.078 mmol) in analogy to general procedure 5 and was obtained as white solid (45 mg, 83% yield). MS (ESI): 643.1 [M+H-isobutene]⁺.

### Step d) (3R)-3-amino-1,1-dioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1λ⁶,5-benzothiazepin-4-one

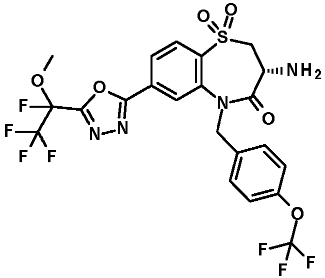

The title compound was prepared from tert-butyl N-[(3R)-1,1,4-trioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1λ⁶,5-benzothiazepin-3-yl]carbamate (39 mg, 0.056 mmol) in analogy to general procedure 6d and was obtained as white solid, as hydrochloride salt (33 mg, 93% yield). MS (ESI): 599.1 [M+H]⁺.

### Example 425 and 426

### (3R)-3-amino-1-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1λ⁴,5-benzothiazepin-4-one [epimer A at S] and (3R)-3-amino-1-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1λ⁴,5-benzothiazepin-4-one [epimer B at S]

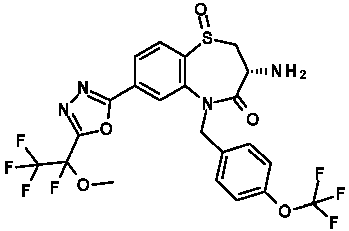

### Step a) tert-butyl N-[(3R)-1, 4-dioxo-7-[5-(1,2, 2, 2-tetrafluoro-1-methoxy-ethyl)-1, 3, 4-oxadiazol-2-yl]-5-[[4-(trifluoromethoxy)phenyl]methyl]-2, 3-dihydro-1λ⁴,5-benzothiazepin-3-yl]carbamate [epimer A at S] and tert-butyl N-[(3R)-1, 4-dioxo-7-[5-(1, 2, 2, 2-tetrafluoro-1-methoxy-ethyl)-1,3, 4-oxadiazol-2-yl]-5-[[4-(trifluoromethoxy)phenyl]methyl]-2,3-dihydro-1λ⁴,5-benzothiazepin-3-yl]carbamate [epimer B at S]

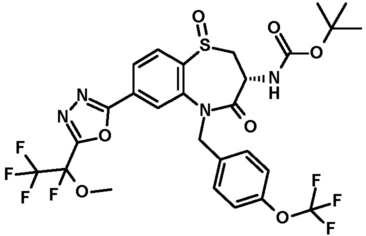

The title compounds were prepared from tert-butyl N-[(3R)-4-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1,5-benzothiazepin-3-yl]carbamate (20 mg, 0.03 mmol, Example 424, step b) in analogy to general procedure 5 using m-CPBA (6.72 mg, 0.03 mmol, 1 eq). The two epimers at S were separated and obtained as white solid (10 mg, 49% yield, epimer 1 at S) and white solid (8 mg, 39% yield, epimer 2 at S). MS (ESI): 627.1 [M+H-isobutene]⁺ for both products.

### Step b1) (3R)-3-amino-1-oxo-7-[5-(1, 2,2, 2-tetrafluoro-1-methoxy-ethyl)-1, 3,4-oxadiazol-2-yl]-5-[[4-(trifluoromethoxy)phenyl]methyl]-2,3-dihydro-1λ⁴,5-benzothiazepin-4-one [epimer A at S]

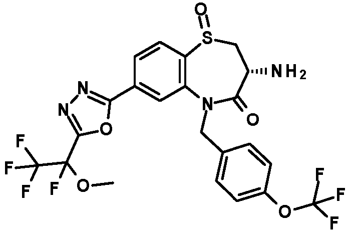

The title compound was prepared from tert-butyl N-[(3R)-1,4-dioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1λ⁴,5-benzothiazepin-3-yl]carbamate [epimer 1 at S] (10 mg, 0.015 mmol) in analogy to general procedure 6d and was obtained as light yellow solid, as hydrochloride salt (8 mg, 86% yield). MS (ESI): 583.4 [M+H]⁺.

### Step b2) (3R)-3-amino-1-oxo-7-[5-(1, 2,2, 2-tetrafluoro-1-methoxy-ethyl)-1, 3, 4-oxadiazol-2-yl]-5-[[4-(trifluoromethoxy)phenyl]methyl]-2,3-dihydro-1λ⁴,5-benzothiazepin-4-one [epimer B at S]

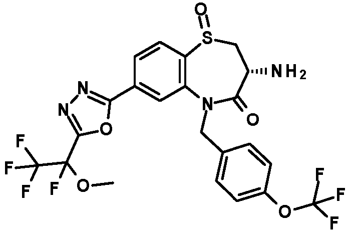

The title compound was prepared from tert-butyl N-[(3R)-1,4-dioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1λ⁴,5-benzothiazepin-3-yl]carbamate [epimer 2 at S] (7 mg, 0.01 mmol) in analogy to general procedure 6d and was obtained as light yellow solid, as hydrochloride salt (5 mg, 80%). MS (ESI): 583.2 [M+H]+.

### Example 427 and 428

### (3R)-3-amino-1-oxido-7-[5-(2,2,2-trifluoroethyl)-1,3,4-oxadiazol-2-yl]-5-[[4-(trifluoromethoxy)phenyl]methyl]-2,3-dihydro-1,5-benzothiazepin-1-ium-4-one [epimer A] and (3R)-3-amino-1-oxido-7-[5-(2,2,2-trifluoroethyl)-1,3,4-oxadiazol-2-yl]-5-[[4-(trifluoromethoxy)phenyl]methyl]-2,3-dihydro-1,5-benzothiazepin-1-ium-4-one [epimer B]

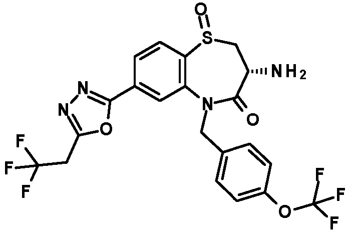

### Step a) tert-butyl N-[(3R)-4-oxo-5-[[4-(trifluoromethoxy)phenyl]methyl]-7-[(3, 3, 3-trifluoropropanoylamino)carbamoyl]-2,3-dihydro-1,5-benzothiazepin-3-yl]carbamate

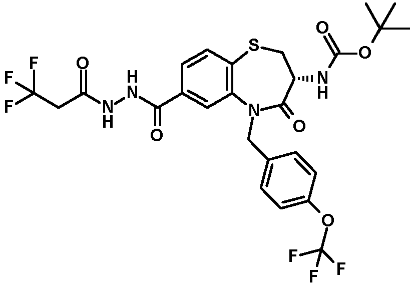

The title compound was prepared from (3R)-3-(tert-butoxycarbonylamino)-4-oxo-5-[[4-(trifluoromethoxy)phenyl]methyl]-2,3-dihydro-1,5-benzothiazepine-7-carboxylic acid (300 mg, 0.57 mmol, 1 eq, Example 414, step b) and 3,3,3-trifluoropropionic acid (102 mg, 0.8 mmol, 1.4 eq) in analogy to general procedure 7a and was obtained as light yellow solid (280 mg, 0.44 mmol, 77% yield). MS (ESI): 581.1 [M+H-isobutene]⁺.

### Step b) tert-butyl N-[(3R)-4-oxo-7-[5-(2,2,2-trifluoroethyl)-1, 3, 4-oxadiazol-2-yl]-5-[[4-(trifluoromethoxy)phenyl]methyl]-2, 3-dihydro-1, 5-benzothiazepin-3-yl]carbamate

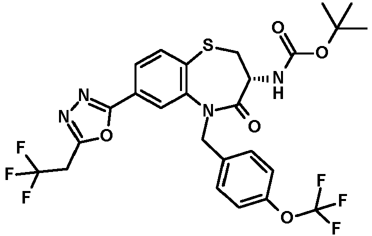

The title compound was prepared from tert-butyl N-[(3R)-4-oxo-5-[[4-(trifluoromethoxy)phenyl]methyl]-7-[(3,3,3-trifluoropropanoylamino)carbamoyl]-2,3-dihydro-1,5-benzothiazepin-3-yl]carbamate (260 mg, 0.41 mmol) in analogy to general procedure 8a and was obtained as white solid (180 mg, 0.29 mmol, 71% yield). MS (ESI): 563.3 [M+H-isobutene]⁺.

### Step c) tert-butyl N-[(3R)-1, 4-dioxo-7-[5-(2, 2, 2-trifluoroethyl)-1, 3,4-oxadiazol-2-yl]-5-[[4-(trifluoromethoxy)phenyl]methyl]-2,3-dihydro-1λ⁴,5-benzothiazepin-3-yl]carbamate [epimer A] and tert-butyl N-[(3R)-1, 4-dioxo-7-[5-(2,2, 2-trifluoroethyl)-1, 3, 4-oxadiazol-2-yl]-5-[[4-(trifluoromethoxy)phenyl]methyl]-2,3-dihydro-1λ⁴,5-benzothiazepin-3-yl]carbamate [epimer B]

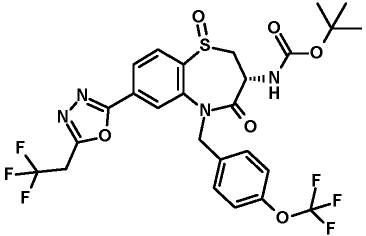

The title compounds were prepared from tert-butyl N-[(3R)-4-oxo-7-[5-(2,2,2-trifluoroethyl)-1,3,4-oxadiazol-2-yl]-5-[[4-(trifluoromethoxy)phenyl]methyl]-2,3-dihydro-1,5-benzothiazepin-3-yl]carbamate (20 mg, 0.03 mmol) in analogy to general procedure 5 using m-CPBA (7.17 mg, 0.032 mmol, 1 eq). The two epimers were separated and obtained as white solid (8 mg, 38% yield, epimer A) and white solid (3 mg, 14% yield, epimer B). MS (ESI): 579.2 [M+H-isobutene]⁺ for both products.

### Step d1) (3R)-3-amino-1-oxo-7-[5-(2, 2, 2-trifluoroethyl)-1, 3, 4-oxadiazol-2-yl]-5-[[4-(trifluoromethoxy)phenyl]methyl]-2,3-dihydro-1λ⁴,5-benzothiazepin-4-one [epimer A]

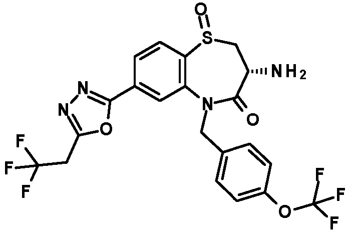

The title compound was prepared from tert-butyl N-[(3R)-1,4-dioxo-7-[5-(2,2,2-trifluoroethyl)-1,3,4-oxadiazol-2-yl]-5-[[4-(trifluoromethoxy)phenyl]methyl]-2,3-dihydro-1λ⁴,5-benzothiazepin-3-yl]carbamate [epimer A] (7 mg, 0.011 mmol) in analogy to general procedure 6d and was obtained as light yellow solid, as hydrochloride salt (5 mg, 81% yield). MS (ESI): 535.1 [M+H]⁺.

### Step d2) (3R)-3-amino-1-oxo-7-[5-(2, 2, 2-trifluoroethyl)-1, 3, 4-oxadiazol-2-yl]-5-[[4-(trifluoromethoxy)phenyl]methyl]-2,3-dihydro-1λ⁴,5-benzothiazepin-4-one [epimer A]

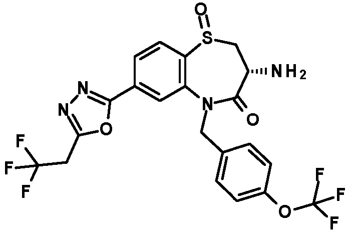

The title compound was prepared from tert-butyl N-[(3R)-1,4-dioxo-7-[5-(2,2,2-trifluoroethyl)-1,3,4-oxadiazol-2-yl]-5-[[4-(trifluoromethoxy)phenyl]methyl]-2,3-dihydro-1λ⁴,5-benzothiazepin-3-yl]carbamate [epimer B] (3 mg, 0.005 mmol) in analogy to general procedure 6d and was obtained as light yellow solid, as hydrochloride salt (2.5 mg, 97% yield). MS (ESI): 535.1 [M+H]⁺.

### Example 429

### (3R)-3-amino-7-(5-tert-butyl-1,3,4-oxadiazol-2-yl)-1,1-dioxo-5-[[4-(2,2,2-trifluoroethoxy)phenyl]methyl]-2,3-dihydro-1λ⁶,5-benzothiazepin-4-one

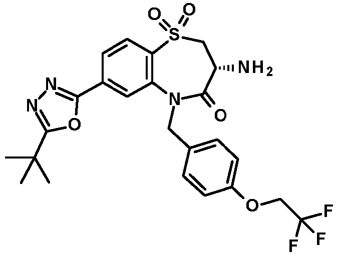

### Step a) (3R)-3-(tert-butoxycarbonylamino)-4-oxo-3, 5-dihydro-2H-1, 5-benzothiazepine-7-carboxylic acid

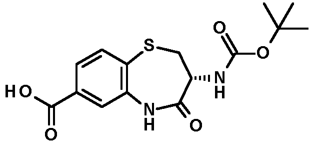

To a solution of methyl (3R)-3-(tert-butoxycarbonylamino)-4-oxo-3,5-dihydro-2H-1,5-benzothiazepine-7-carboxylate (30.0 g, 85.13 mmol, 1 eq) (CAS: 2089150-62-7) in THF (300 mL) was added a solution of NaOH (5.11 g, 127.69 mmol, 1.5 eq) in water (300 mL) at 0 °C. The mixture was stirred at RT for 7 h. At 0 °C, 0.5 M HCl was added to the reaction mixture which was then allowed to warm to RT. EtOAc was added and the layers were separated. The aqueous phase was extracted with EtOAc (2x 200 mL). The combined organic layer were washed with brine (3x200 mL), dried over sodium sulfate, filtered and concentrated to afford (3R)-3-(tert-butoxycarbonylamino)-4-oxo-3,5-dihydro-2H-1,5-benzothiazepine-7-carboxylic acid (29.4 g, 86.88 mmol, 91% yield) as yellow solid. MS (ESI): 283.0 [M+H-isobutene]⁺.

### Step b) tert-butyl N-[(3R)-7-(hydrazinecarbonyl)-4-oxo-3,5-dihydro-2H-1,5-benzothiazepin-3-yl]carbamate

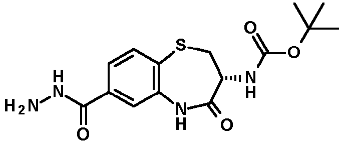

To a solution of (3R)-3-(tert-butoxycarbonylamino)-4-oxo-3,5-dihydro-2H-1,5-benzothiazepine-7-carboxylic acid (29.4 g, 86.88 mmol, 1 eq) and DIPEA (45.4 mL, 260.65 mmol, 3 eq) in DCM (400 mL) was added T₃P (50 wt% in EtOAc, 66.88 mL, 130.33 mmol, 1.5 eq) at RT and the reaction was stirred for 30 min. The reaction mixture was then transferred via dropping funnel to a solution of hydrazine hydrate (17.2 mL, 347.54 mmol, 4 eq) in DCM (190 mL). The reaction mixture was stirred for 3 h at RT and a yellow precipitate formed. The mixture was concentrated and the crude purified by prep-HPLC to afford tert-butyl N-[(3R)-7-(hydrazinecarbonyl)-4-oxo-3,5-dihydro-2H-1,5-benzothiazepin-3-yl]carbamate (6 g, 17.03 mmol, 19% yield) as white solid. MS (ESI): 297.1 [M+H-isobutene]⁺.

### Step c) tert-butyl N-[(3R)-7-[(2,2-dimethylpropanoylamino)carbamoyl]-4-oxo-3,5-dihydro-2H-1,5-benzothiazepin-3-yl]carbamate

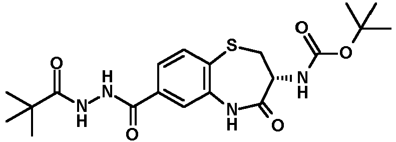

The title compound was prepared from tert-butyl N-[(3R)-7-(hydrazinecarbonyl)-4-oxo-3,5-dihydro-2H-1,5-benzothiazepin-3-yl]carbamate (4.5 g, 7.39 mmol, 1 eq) and pivalic acid (889.0 mg, 8.7 mmol, 1.18 eq) in analogy to general procedure 7a and was obtained as light yellow solid (2.95 g, 6.76 mmol, 84% yield). MS (ESI): 381.1 [M+H-isobutene]⁺.

### Step d) tert-butylN-[(3R)-7-(5-tert-butyl-1, 3, 4-oxadiazol-2-yl)-4-oxo-3, 5-dihydro-2H-1,5-benzothiazepin-3-yl]carbamate

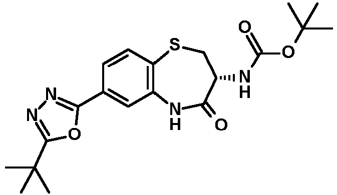

The title compound was prepared from tert-butyl N-[(3R)-7-[(2,2-dimethylpropanoylamino)carbamoyl]-4-oxo-3,5-dihydro-2H-1,5-benzothiazepin-3-yl]carbamate (2.9 g, 6.64 mmol) in analogy to general procedure 8a and was obtained as light yellow solid (1.6 g, 3.82 mmol, 54% yield). MS (ESI): 363.1 [M+H-isobutene]⁺.

### Step e) tert-butyl N-[(3R)-7-(5-tert-butyl-1, 3, 4-oxadiazol-2-yl)-1,1, 4-trioxo-3, 5-dihydro-2H-1λ⁶,5-henzothiazepin-3-yl]carbamate

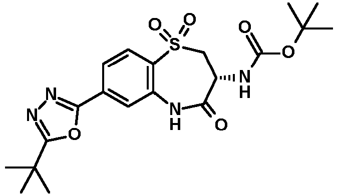

The title compound was prepared from tert-butyl N-[(3R)-7-(5-tert-butyl-1,3,4-oxadiazol-2-yl)-4-oxo-3,5-dihydro-2H-1,5-benzothiazepin-3-yl]carbamate (241.0 mg, 0.580 mmol) in analogy to general procedure 5 and was obtained as white solid (270 mg, 0.600 mmol, 94.5% yield). MS (ESI): 395.2 [M+H-isobutene]⁺.

### Step f) tert-butyl N-[(3R)-7-(5-tert-butyl-1, 3, 4-oxadiazol-2-yl)-1,1, 4-trioxo-5-[[4-(2,2, 2-trifluoroethoxy)phenyl]methyl]-2, 3-dihydro-1λ⁶,5-benzothiazepin-3-yl]carbamate

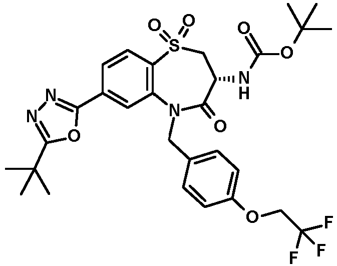

To a solution of tert-butyl N-[(3R)-7-(5-tert-butyl-1,3,4-oxadiazol-2-yl)-1,1,4-trioxo-3,5-dihydro-2H-1λ⁶,5-benzothiazepin-3-yl]carbamate (50.0 mg, 0.11 mmol, 1 eq) in DMF (1 mL) was added potassium carbonate (33.7 mg, 0.24 mmol, 2.2 eq) and KI (3.68 mg, 0.02 mmol, 0.2 eq) at RT and stirred for 5 min. To this mixture was then added dropwise 1-(bromomethyl)-4-(2,2,2-trifluoroethoxy)benzene (29.8 mg, 0.11 mmol, 1 eq) in DMF (1 mL) via syringe. The mixture was stirred for 16 h at RT. The mixture was then poured into water (40 mL) and extracted with EtOAc (2 x 30 mL). The combined organic layers were washed with brine (30 mL), dried over sodium sulfate, filtered and concentrated in vacuum. The remaining residue was purified by preparative TLC (PE/EtOAc= 1:1) to afford desired title compound (50 mg, 0.08 mmol, 52% yield) as a light yellow solid. MS (ESI): 583.1 [M+H-isobutene]⁺.

### Step g) (3R)-3-amino-7-(5-tert-butyl-1, 3, 4-oxadiazol-2-yl)-1,1-dioxo-5-[[4-(2,2,2-trifluoroethoxy)phenyl]methyl]-2, 3-dihydro-1λ⁶,5-benzothiazepin-4-one

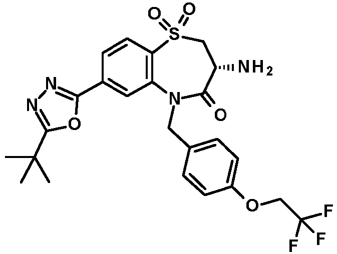

The title compound was prepared from tert-butyl N-[(3R)-7-(5-tert-butyl-1,3,4-oxadiazol-2-yl)-1,1,4-trioxo-5-[[4-(2,2,2-trifluoroethoxy)phenyl]methyl]-2,3-dihydro-1λ⁶,5-benzothiazepin-3-yl]carbamate (50.0 mg, 0.080 mmol) in analogy to general procedure 6b and was obtained as white solid (20.8 mg, 0.04 mmol, 48% yield). MS (ESI): 539.1 [M+H]⁺.

The examples 123 and 430 to 440 of the following table were prepared in analogy to Example 362, using the appropriate benzyl halide building block.

| **Ex.** | **Structure** | **Systematic Name** | **Building Block** | **MS, ESI: m/z** |
|---|---|---|---|---|
| 123 | 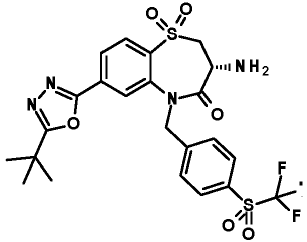 | (3R)-3-amino-7-(5-tert-butyl-1,3,4-oxadiazol-2-yl)-1,1-dioxo-5-[[4-(trifluoromethylsulfonyl)phen yl]methyl]-2,3-dihydro-1λ⁶,5-benzothiazepin-4-one | 1-(Bromome thyl)-4-[(trifluoro methyl)sul fonyl]benz ene | 573.1 [M+H]⁺ |
| | | | (CAS 118089-89-7) | |
| 430 | 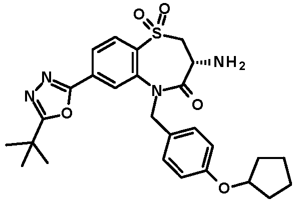 | (3R)-3-amino-7-(5-tert-butyl-1,3,4-oxadiazol-2-yl)-5-[[4-(cyclopentoxy)phenyl]methyl] -1,1-dioxo-2,3-dihydro-1λ⁶,5-benzothiazepin-4-one | 1-(Chlorome thyl)-4-(cyclopent yloxy)benz ene | 525.5 [M+H]⁺ |
| | | | (CAS 1041579-01-4) | |
| 431 | 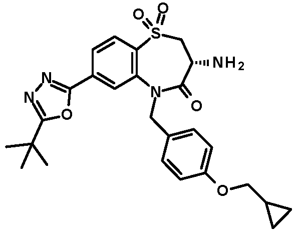 | (3R)-3-amino-7-(5-tert-butyl-1,3,4-oxadiazol-2-yl)-5-[[4-(cyclopropylmethoxy)phenyl] methyl]-1,1-dioxo-2,3-dihydro-1λ⁶,5-benzothiazepin-4-one | 1-Cchlorome thyl)-4-(cycloprop ylmethoxy) benzene | 511.0 [M+H]⁺ |
| | | | (CAS 183994-41-4) | |
| 432 | 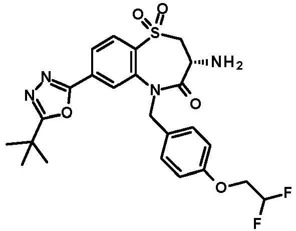 | (3R)-3-amino-7-(5-tert-butyl-1,3,4-oxadiazol-2-yl)-5-[[4-(2,2-difluoroethoxy)phenyl]methyl ]-1,1-dioxo-2,3-dihydro-1λ⁶,5-benzothiazepin-4-one | 1-(Chlorome thyl)-4-(2,2-difluoroeth oxy)benze ne | 521.2 [M+H]⁺ |
| | | | (CAS 1178393-11-7) | |
| 433 | 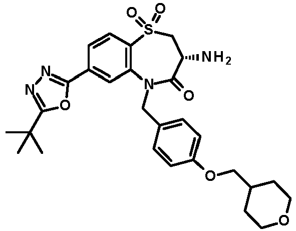 | (3R)-3-amino-7-(5-tert-butyl-1,3,4-oxadiazol-2-yl)-1,1-dioxo-5-[[4-(tetrahydropyran-4-ylmethoxy)phenyl]methyl]-2,3-dihydro-1λ⁶,5-benzothiazepin-4-one | 4-[[4-(Chlorome thyl)pheno xy]methyl] tetrahydro -2H-pyran | 555.1 [M+H]⁺ |
| | | | (CAS 1248950-37-9) | |
| 434 | 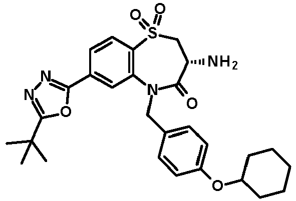 | (3R)-3-amino-7-(5-tert-butyl-1,3,4-oxadiazol-2-yl)-5-[[4-(cyclohexoxy)phenyl]methyl]-1,1-dioxo-2,3-dihydro-1λ⁶,5-benzothiazepin-4-one | 1-(Chlorome thyl)-4-(cyclohexyl oxy)benzene | 539.3 [M+H]⁺ |
| | | | (CAS 13485-83-1) | |
| 435 | 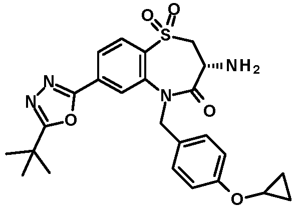 | (3R)-3-amino-7-(5-tert-butyl-1,3,4-oxadiazol-2-yl)-1,1-dioxo-5-[(4-tetrahydropyran-4-yloxyphenyl)methyl]-2,3-dihydro-1λ⁶,5-benzothiazepin-4-one | 1-(Chlormet hyl)-4-(cycloprop yloxy)benzene | 497.2 [M+H]⁺ |
| | | | (CAS 1690434-36-6) | |
| 436 | 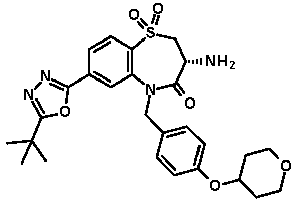 | (3R)-3-amino-7-(5-tert-butyl-1,3,4-oxadiazol-2-yl)-1,1-dioxo-5-[(4-tetrahydropyran-4-yloxyphenyl)methyl]-2,3-dihydro-1λ⁶,5-benzothiazepin-4-one | 4-[4-(Chlorome thyl)pheno xy]tetrahy dro-2H-pyran | 541.3 [M+H]⁺ |
| | | | (CAS 1342540-72-0) | |
| 437 | 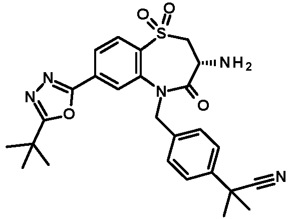 | 2-[4-[[(3R)-3-amino-7-(5-tert-butyl-1,3,4-oxadiazol-2-yl)-1,1,4-trioxo-2,3-dihydro-1λ⁶,5-benzothiazepin-5-yl]methyl]phenyl]-2-methylpropanenitrile | 4-(Bromome thyl)-α,α-dimethylb enzeneace tonitrile | 508.1 [M+H]⁺ |
| | | | (CAS 1201643-73-3) | |
| 438 | 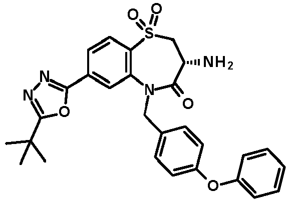 | (3R)-3-amino-7-(5-tert-butyl-1,3,4-oxadiazol-2-yl)-1,1-dioxo-5-[(4-phenoxyphenyl)methyl]-2,3-dihydro-1λ⁶,5-benzothiazepin-4-one | 1-(Bromome thyl)-4-phenoxybe nzene | 532.9 [M+H]⁺ |
| | | | (CAS 36881-42-2) | |
| 439 | 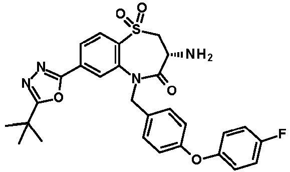 | (3R)-3-amino-7-(5-tert-butyl-1,3,4-oxadiazol-2-yl)-5-[[4-(4-fluorophenoxy)phenyl]methyl ]-1,1-dioxo-2,3-dihydro-1λ⁶,5-benzothiazepin-4-one | 1-(Chlorome thyl)-4-(4-fluorophen oxy)benze ne | 551.2 [M+H]⁺ |
| | | | (CAS 167091-97-6) | |
| 440 | 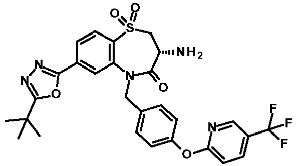 | (3R)-3-amino-7-(5-tert-butyl-1,3,4-oxadiazol-2-yl)-1,1-dioxo-5-[[4-[[5-(trifluoromethyl)-2-pyridyl]oxy]phenyl]methyl]-2,3-dihydro-1λ⁶,5-benzothiazepin-4-one | 2-[(4-(Cloromet hyl)phenox y]-5-(trifluorom ethyl)pyrid ine | 602.2 [M+H]⁺ |
| | | | (CAS 1637306-15-0) | |

### Example 441

### (3R)-3-amino-7-(5-tert-butyl-1,3,4-oxadiazol-2-yl)-5-[(4-but-2-ynoxyphenyl)methyl]-1,1-dioxo-2,3-dihydro-1λ⁶,5-benzothiazepin-4-one

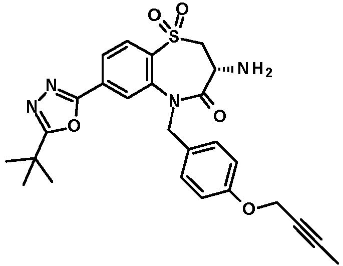

### Step a) (4-but-2-ynoxyphenyl)methanol

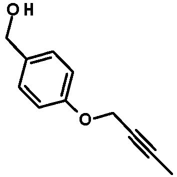

To a solution of 4-hydroxybenzyl alcohol (1 g, 8.06 mmol, 1 eq) in DMF (8 mL) was added potassium carbonate (2226 mg, 16.1 mmol, 2 eq), KI (0.04 mL, 0.810 mmol, 0.1 eq) and 1-bromo-2-butyne (1339 mg, 10 mmol, 1.25 eq). The mixture was stirred at 80 °C for 16 h. The reaction mixture was diluted with EtOAc (20 mL) and filtered through celite. The filtrate was washed with brine (3x 25 mL) and the organic phase was dried over sodium sulfate, filtered and concentrated. The resulting oil was purified by column chromatography on silica gel (8%-40% EtOAc in PE) to afford (4-but-2-ynoxyphenyl)methanol (950 mg, 5.39 mmol, 48% yield) as a brown oil. MS (ESI): 159.0 [M+H-H₂O]⁺.

### Step b) tert-butyl N-[(3R)-7-(5-tert-butyl-1,3, 4-oxadiazol-2-yl)-5-[(4-but-2-ynoxyphenyl)methyl]-1,1,4-trioxo-2, 3-dihydro-1λ⁶,5-benzothiazepin-3-yl]carbamate

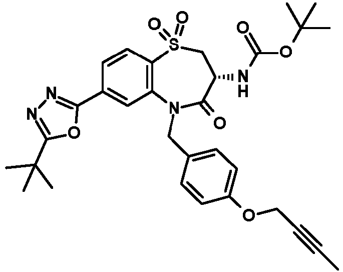

To a solution of tert-butyl N-[(3R)-7-(5-tert-butyl-1,3,4-oxadiazol-2-yl)-1,1,4-trioxo-3,5-dihydro-2H-1λ⁶,5-benzothiazepin-3-yl]carbamate (50.0 mg, 0.110 mmol, 1 eq) in toluene (1.5 mL) was degassed by bubbling through N₂ for 5 min. To this solution was then added (4-but-2-ynoxyphenyl)methanol (20.0 mg, 0.110 mmol, 1.02 eq), Ph₃P (0.05 mL, 0.220 mmol, 2 eq) and DIAD (0.04 mL, 0.220 mmol, 2 eq) and the reaction was stirred at 50 °C for 4 h. The reaction mixture was diluted with EtOAc (5 mL) and washed with brine (3x10 mL). The organic phase was dried over sodium sulfate, filtered and concentrated. The remaining residue was purified by preparative TLC (PE/EtOAc=2:1) to obtain the title compound (50 mg, 0.08 mmol, 74% yield) as light yellow solid, MS (ESI): 553.2 [M+H-isobutene]⁺.

### Step c) (3R)-3-amino-7-(5-tert-butyl-1,3,4-oxadiazol-2-yl)-5-[(4-but-2-ynoxyphenyl)methyl]-1,1-dioxo-2, 3-dihydro-1λ⁶,5-benzothiazepin-4-one

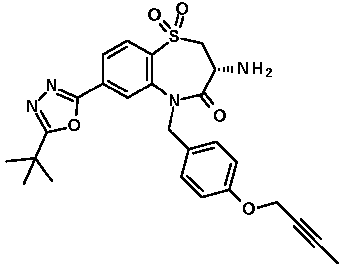

The title compound was prepared from tert-butyl N-[(3R)-7-(5-tert-butyl-1,3,4-oxadiazol-2-yl)-5-[(4-but-2-ynoxyphenyl)methyl]-1,1,4-trioxo-2,3-dihydro-1λ⁶,5-benzothiazepin-3-yl]carbamate (45.0 mg, 0.070 mmol) in analogy to general procedure 6b and was obtained as white solid (14.3 mg, 0.030 mmol, 38% yield). MS (ESI): 509.1 [M+H]⁺.

### Example 442

### (3R)-3-amino-7-(5-tert-butyl-1,3,4-oxadiazol-2-yl)-1,1-dioxo-5-[[4-(1,1,2,2-tetrafluoroethoxy)phenyl]methyl]-2,3-dihydro-1λ⁶,5-benzothiazepin-4-one

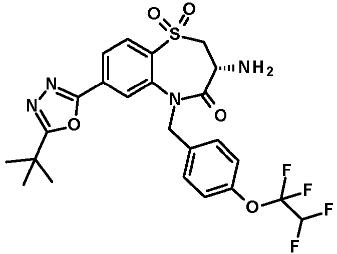

### Step a) methyl (3R)-3-(tert-butoxycarbonylamino)-4-oxo-5-[[4-(1, 1, 2, 2-tetrafluoroethoxy)phenyl]methyl]-2,3-dihydro-1, 5-benzothiazepine-7-carboxylate

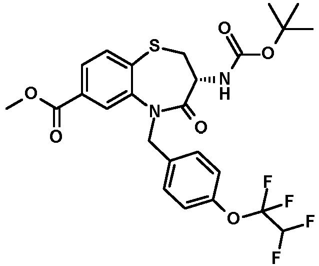

To a solution of methyl (3R)-3-(tert-butoxycarbonylamino)-4-oxo-3,5-dihydro-2H-1,5-benzothiazepine-7-carboxylate (250 mg, 0.71 mmol, 1 eq) (CAS: 2089150-62-7) in DMSO (3 mL) was added 1-(bromomethyl)-4-(1,1,2,2-tetrafluoroethoxy)benzene (300 mg, 0.99 mmol, 1.4 eq, CAS 67033-41-4), potassium carbonate (294.13 mg, 2.13 mmol, 3 eq) and potassium iodide (58.8 mg, 0.355 mmol, 0.5 eq) at RT. The reaction mixture was stirred for 2.5 h at RT. The reaction mixture was partitioned between EtOAc (100 ml) and water (100 ml). The layers were separated and the aqueous layer was extracted with two 100 ml portions of EtOAc. The combined organic layers were washed with one 50 ml portion of water and one 50 ml portion of sat. NH₄Cl, dried over anhydrous sodium sulfate and concentrated in vacuo. The crude material was purified by column chromatography on silica gel (0-30% EtOAc in heptane) to afford the title compound (394 mg, 98% yield) as yellow oil. MS (ESI): 503.2 [M+H-isobutene]⁺.

### Step b) (3R)-3-(tert-butoxycarbonylamino)-4-oxo-5-[[4-(1,1,2,2-tetrafluoroethoxy)phenyl]methyl]-2,3-dihydro-1, 5-benzothiazepine-7-carboxylic acid

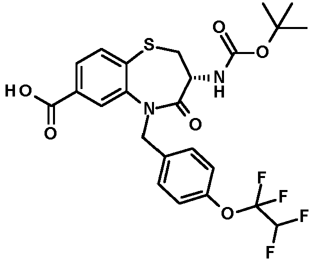

To a solution of methyl (3R)-3-(tert-butoxycarbonylamino)-4-oxo-5-[[4-(1,1,2,2-tetrafluoroethoxy)phenyl]methyl]-2,3-dihydro-1,5-benzothiazepine-7-carboxylate (394 mg, 0.7 mmol, 1 eq) in THF (3.5 mL), MeOH (0.58 mL) and water (0.93 mL) was added 1 M lithium hydroxide (1M in H₂O) (1.41 mL, 1.41 mmol, 2 eq). The mixture was stirred at RT for 4 hr. The reaction mixture was diluted with 100 ml EtOAc and washed once with 50 ml 1M HCl. The layers were separated and the aqueous layer was extracted with two 70 ml portions of EtOAc. The combined organic layers were dried over sodium sulfate, filtered and concentrated in vacuo to give (3R)-3-(tert-butoxycarbonylamino)-4-oxo-5-[[4-(1,1,2,2-tetrafluoroethoxy)phenyl]methyl]-2,3-dihydro-1,5-benzothiazepine-7-carboxylic acid (400 mg, 99% yield) as light yellow oil. MS (ESI): 543.2 [M-H]⁻.

### Step c) tert-butyl N-[(3R)-7-(hydrazinecarbonyl)-4-oxo-5-[[4-(1,1,2,2-tetrafluoroethoxy)phenyl]methyl]-2,3-dihydro-1, 5-benzothiazepin-3yl]carbamate

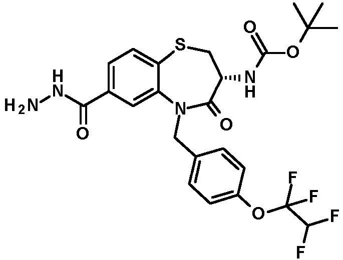

(3R)-3-(tert-butoxycarbonylamino)-4-oxo-5-[[4-(1,1,2,2-tetrafluoroethoxy)phenyl]methyl]-2,3-dihydro-1,5-benzothiazepine-7-carboxylic acid (0.4 g, 0.73 mmol, 1 eq) was dissolved in THF (3.18 mL). Then CDI (151 mg, 0.93 mmol, 1.27 eq) was added in one portion and the resulting light yellow solution was stirred for 90 min. To this solution was added a mixture of hydrazine hydrate (45.9 mg, 44.5 uL, 0.918 mmol, 1.25 eq) in THF (0.535 mL) and stirred for 90 min. The reaction mixture was partitioned between EtOAc (100 ml) and water (80 ml) and 5 ml of brine were added to help with phase separation. The layers were separated and the aqueous layer was extracted with two 100 ml portions of EtOAc. The combined organic layers were washed with one 80 ml portion of brine, dried over anhydrous sodium sulfate and concentrated in vacuo to afford the title compound (422 mg, 93% yield) as yellow oil. MS (ESI): 559.2 [M+H]⁺.

### Step d) tert-butylN-[(3R)-7-[(2, 2-dimethylpropanoylamino)carbamoyl]-4-oxo-5-[[4-(1,1,2,2-tetrafluoroethoxy)phenyl]methyl]-2,3-dihydro-1, 5-benzothiazepin-3-yl]carbamate

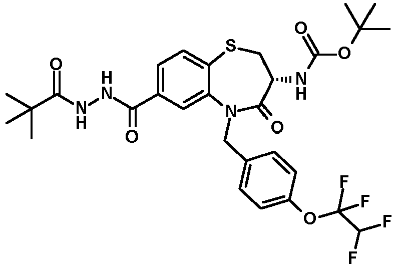

The title compound was prepared from tert-butyl N-[(3R)-7-(hydrazinecarbonyl)-4-oxo-5-[[4-(1,1,2,2-tetrafluoroethoxy)phenyl]methyl]-2,3-dihydro-1,5-benzothiazepin-3-yl]carbamate (200 mg, 0.36 mmol) and pivalic acid (36.57 mg, 0.36 mmol, 1 eq) in analogy to general procedure 7b and was obtained as light red foam (181 mg, 0.28 mmol, 76% yield). MS (ESI): 543.1 [M+H-Boc]⁺.

### Step e) tert-butyl N-[(3R)-7-(5-tert-butyl-1, 3, 4-oxadiazol-2-yl)-4-oxo-5-[[4-(1,1,2, 2-tetrafluoroethoxy)phenyl]methyl]-2,3-dihydro-1,5-benzothiazepin-3-yl]carbamate

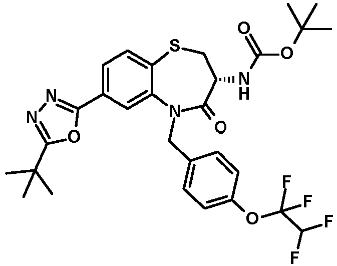

The title compound was prepared from tert-butyl N-[(3R)-7-[(2,2-dimethylpropanoylamino)carbamoyl]-4-oxo-5-[[4-(1,1,2,2-tetrafluoroethoxy)phenyl]methyl]-2,3-dihydro-1,5-benzothiazepin-3-yl]carbamate (181 mg, 0.28 mmol) in analogy to general procuedre 8a and was obtained as colourless oil (170 mg, 0.27 mmol, 97% yield). MS (ESI): 625.2 [M+H]⁺.

### Step f) tert-butyl N-[(3R)-7-(5-tert-butyl-1, 3, 4-oxadiazol-2-yl)-1,1, 4-trioxo-5-[[4-(1,1, 2,2-tetrafluoroethoxy)phenyl]methyl]-2,3-dihydro-1λ⁶,5-benzothiazepin-3-yl]carbamate

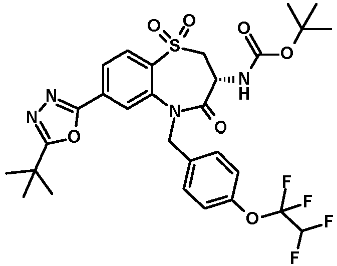

The title compound as prepared from tert-butyl N-[(3R)-7-(5-tert-butyl-1,3,4-oxadiazol-2-yl)-4-oxo-5-[[4-(1,1,2,2-tetrafluoroethoxy)phenyl]methyl]-2,3-dihydro-1,5-benzothiazepin-3-yl]carbamate (170.0 mg, 0.270 mmol) in analogy to general procedure 5 and was obtained as white foam (60 mg, 0.09 mmol, 34% yield). MS (ESI): 657.5 [M+H-isobutene]⁺.

### Step g) (3R)-3-amino-7-(5-tert-butyl-1, 3, 4-oxadiazol-2-yl)-1,1-dioxo-5-[[4-(1,1,2,2-tetrafluoroethoxy)phenyl]methyl]-2,3-dihydro-1λ⁶,5-benzothiazepin-4-one

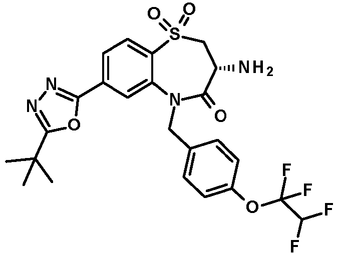

The title compound was prepared from tert-butyl N-[(3R)-7-(5-tert-butyl-1,3,4-oxadiazol-2-yl)-1,1,4-trioxo-5-[[4-(1,1,2,2-tetrafluoroethoxy)phenyl]methyl]-2,3-dihydro-1λ⁶,5-benzothiazepin-3-yl]carbamate (75 mg, 0.11 mmol) in analogy to general procedure 6b and was obtained as white solid (46.5 mg, 0.08 mmol, 73% yield). MS (ESI): 557.2 [M+H]⁺.

### Example 443

### Methyl 1-[5-[(3R)-3-amino-1,1,4-trioxo-5-[[4-(1,1,2,2-tetrafluoroethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate

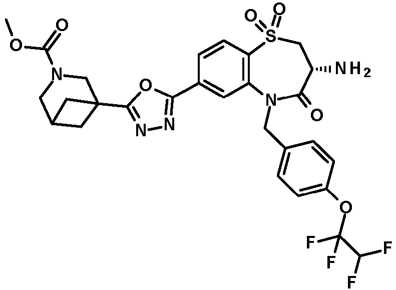

### Step a) tert-butyl 1-[[[(3R)-3-(tert-butoxycarbonylamino)-4-oxo-5-[[4-(1,1,2,2-tetrafluoroethoxy)phenyl]methyl]-2,3-dihydro-1, 5-benzothiazepine-7-carbonyl]amino]carbamoyl]-3-azabicyclo[3.1. 1 Jheptane-3-carboxylate

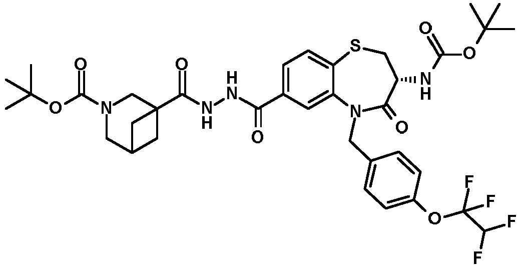

The title compound was prepared from tert-butyl N-[(3R)-7-(hydrazinecarbonyl)-4-oxo-5-[[4-(1,1,2,2-tetrafluoroethoxy)phenyl]methyl]-2,3-dihydro-1,5-benzothiazepin-3-yl]carbamate (110 mg, 0.2 mmol, 1 eq) and 3-tert-butoxycarbonyl-3-azabicyclo[3.1.1]heptane-1-carboxylic acid (55.0 mg, 0.23 mmol, 1.16 eq) in analogy to general procedure 7a and was obtained as yellow solid (120 mg, 0.15 mmol, 78% yield). MS (ESI): 782.3 [M+H]⁺.

### Step b) tert-butyl 1-[5-[(3R)-3-(tert-butoxycarbonylamino)-4-oxo-5-[[4-(1,1,2,2-tetrafluoroethoxy)phenyl]methyl]-2,3-dihydro-1,5-benzothiazepin-7-yl]-1,3,4-oxadiazol-2-yl]-3-azabicyclo[3.1.1]heptane-3-carboxylate

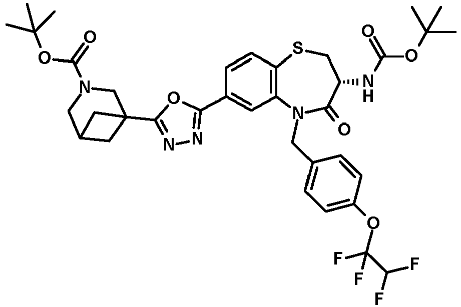

The title compound was prepared from tert-butyl 1-[[[(3R)-3-(tert-butoxycarbonylamino)-4-oxo-5-[[4-(1,1,2,2-tetrafluoroethoxy)phenyl]methyl]-2,3-dihydro-1,5-benzothiazepine-7-carbonyl]amino]carbamoyl]-3-azabicyclo[3.1.1]heptane-3-carboxylate (120 mg, 0.15 mmol) in analogy to general procedure 8a and was obtained as white solid (85 mg, 0.11 mmol, 73% yield) as white solid. MS (ESI): 708.2 [M+H-isobutene]⁺.

### Step c) tert-butyl 1-[5-[(3R)-3-(tert-butoxycarbonylamino)-1,1,4-trioxo-5-[[4-(1,1,2, 2-tetrafluoroethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate

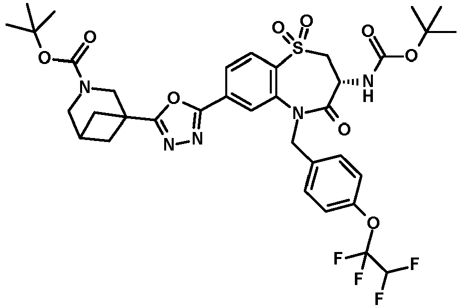

The title compound was prepared from tert-butyl 1-[5-[(3R)-3-(tert-butoxycarbonylamino)-4-oxo-5-[[4-(1,1,2,2-tetrafluoroethoxy)phenyl]methyl]-2,3-dihydro-1,5-benzothiazepin-7-yl]-1,3,4-oxadiazol-2-yl]-3-azabicyclo[3.1.1]heptane-3-carboxylate (85 mg, 0.11 mmol) in analogy to general procedure 5 and was obtained as white solid (50 mg, 57% yield). MS (ESI): 740.3 [M+H-isobutene]⁺.

### Step d) (3R)-3-amino-7-[5-(3-azabicyclo[3.1.1]heptan-1-yl)-1,3, 4-oxadiazol-2-yl]-1,1-dioxo-5-[[4-(1,1,2,2-tetrafluoroethoxy)phenyl]methyl]-2,3-dihydro-1λ⁶,5-benzothiazepin-4-one

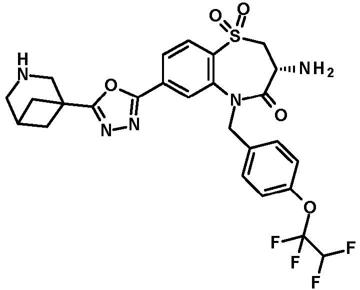

The title compound was prepared from tert-butyl 1-[5-[(3R)-3-(tert-butoxycarbonylamino)-1,1,4-trioxo-5-[[4-(1,1,2,2-tetrafluoroethoxy)phenyl]methyl]-2,3-dihydro-1λ⁶,5-benzothiazepin-7-yl]-1,3,4-oxadiazol-2-yl]-3-azabicyclo[3.1.1]heptane-3-carboxylate (50 mg, 0.07 mmol) in analogy to general procedure 6b and was obtained as white solid (27 mg, 0.040 mmol, 69% yield). MS (ESI): 596.2 [M+H]⁺.

### Step e) methyl 1-[5-[(3R)-3-amino-1,1, 4-trioxo-5-[[4-(1,1, 2, 2-tetrafluoroethoxy)phenyl]methyl]-2,3-dihydro-1λ ⁶,5-benzothiazepin-7-yl]-1,3,4-oxadiazol-2-yl]-3-azabicyclo[3.1.1]heptane-3-carboxylate

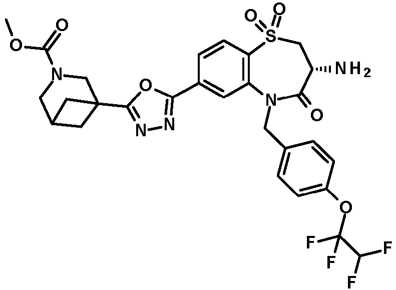

To a solution of (3R)-3-amino-7-[5-(3-azabicyclo[3.1.1]heptan-1-yl)-1,3,4-oxadiazol-2-yl]-1,1-dioxo-5-[[4-(1,1,2,2-tetrafluoroethoxy)phenyl]methyl]-2,3-dihydro-1λ⁶,5-benzothiazepin-4-one (22 mg, 0.03 mmol, 1 eq) in DCM (2 mL) was added DIPEA (17 mg, 0.13 mmol, 4 eq) and methyl chloroformate (4 mg, 0.04 mmol, 1.29 eq). The mixture was stirred at RT for 1 h and then concentrated in vacuo. The remaining crude was purified by prep-HPLC to afford the title compound (5.5 mg, 0.01 mmol, 23% yield) as white solid. MS (ESI): 654.2 [M+H]⁺.

### Example 444

### methyl 3-[5-[(3R)-3-amino-5-[[4-(cyclopentoxy)phenyl]methyl]-1,1,4-trioxo-2,3-dihydro-1λ⁶,5-benzothiazepin-7-yl]-1,3,4-oxadiazol-2-yl]pyrrolidine-1-carboxylate

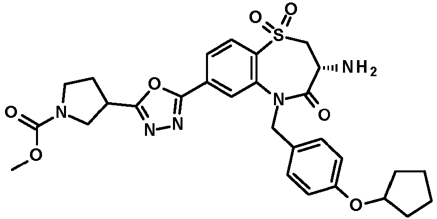

### Step a) benzyl 3-(hydrazinecarbonyl)pyrrolidine-1-carboxylate

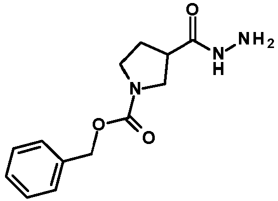

Ethyl chloroformate (87.0 mg, 77.06 uL, 0.8 mmol, 1 eq) was added to a solution 1-benzyloxycarbonylpyrrolidine-3-carboxylic acid (200 mg, 0.8 mmol, 1 eq) (CAS 188527-21-1) and NEt₃ (85.2 mg, 117 uL, 0.84 mmol, 1.05 eq) in THF (2 mL) at 0 °C and the reaction was stirred for 1 hour. The resulting white suspension was filtered and the filtrate was directly added to a solution of hydrazine hydrate (123 mg, 120 uL, 1.6 mmol, 2 eq) in MeOH (2 mL) at 0 °C. The reaction was allowed to warm up to RT and stirred for 5 h. The solution was diluted with EtOAc and washed once with water and once with brine. The organic layer was then dried over magnesium sulfate, filtered and concentrated to afford the title compound (138 mg, 59% yield) as white solid. MS (ESI): 264.2 [M+H]⁺.

### Step b) methyl (3R)-3-(tert-butoxycarbonylamino)-5-[[4-(cyclopentoxy)phenyl]methyl]-4-oxo-2,3-dihydro-1,5-benzothiazepine-7-carboxylate

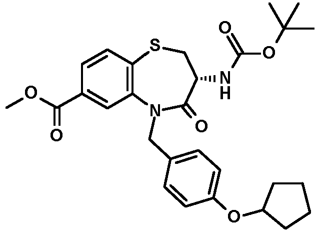

To a solution of methyl (3R)-3-(tert-butoxycarbonylamino)-4-oxo-3,5-dihydro-2H-1,5-benzothiazepine-7-carboxylate (220 mg, 0.624 mmol, 1 eq) (CAS: 2089150-62-7) in DMSO (2 ml) was added sequentially 1-(chloromethyl)-4-(cyclopentoxy)benzene (144 mg, 0.687 mmol, 1.1 eq), KI (51.81 mg, 0.312 mmol, 0.5 eq) and K₂CO₃ (258.84 mg, 1.87 mmol, 3 eq). The reaction was stirred at RT overnight. The reaction was diluted with EtOAc and washed once with water and once with brine. The organic layer was dried over magnesium sulfate, filtered and concentrated. The remaining residue was purified by column chromatography on silica gel (0-30% EtOAc in heptane to give the title compound (213 mg, 62% yield) as light yellow solid. MS (ESI): 471.3 [M+H-isobutene]⁺.

### Step c) (3R)-3-(tert-butoxycarbonylamino)-5-[[4-(cyclopentoxy)phenyl]methyl]-4-oxo-2,3-dihydro-1,5-benzothiazepine-7-carboxylic acid

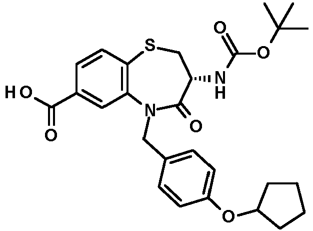

Lithium hydroxide monohydrate (32.58 mg, 0.777 mmol, 2 eq) was added to a yellow solution of methyl (3R)-3-(tert-butoxycarbonylamino)-5-[[4-(cyclopentoxy)phenyl]methyl]-4-oxo-2,3-dihydro-1,5-benzothiazepine-7-carboxylate (213 mg, 0.388 mmol, 1 eq) in THF (1.4 mL), MeOH (0.2 mL) and water (0.4 mL) and the reaction was stirred at RT for 2 hours. 1N aq. HCl was added to give a white suspension and the mixture was extracted with EtOAc (3x). The combined organic layers were washed with brine, dried over magnesium sulfate, filtered and concentrated to give the title compound as light yellow solid (193 mg, 93% yield). MS (ESI): 457.3 [M+H-isobutene]⁺.

### Step d) benzyl 3-[[[(3R)-3-(tert-butoxycarbonylamino)-5-[[4-(cyclopentoxy)phenyl[methyl]-4-oxo-2,3-dihydro-1,5-benzothiazepine-7-carbonyl]amino]carbamoyl]pyrrolidine-1-carboxylate

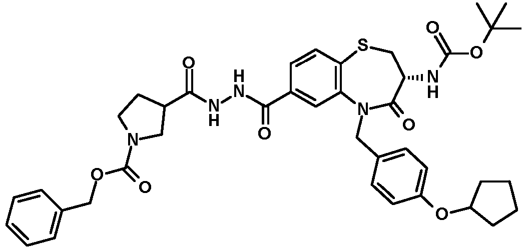

The title compound was prepared from (3R)-3-(tert-butoxycarbonylamino)-5-[[4-(cyclopentoxy)phenyl]methyl]-4-oxo-2,3-dihydro-1,5-benzothiazepine-7-carboxylic acid (40 mg, 0.075 mmol, 1 eq) and benzyl 3-(hydrazinecarbonyl)pyrrolidine-1-carboxylate (29.5 mg, 0.11 mmol, 1.5 eq) in analogy to general procedure 7b and was obtained as white solid (35 mg, 62% yield). MS (ESI): 758.5 [M+H]⁺.

### Step e) benzyl 3-[5-[(3R)-3-(tert-butoxycarbonylamino)-5-[[4-(cyclopentoxy)phenyl]methyl]-4-oxo-2,3-dihydro-1,5-benzothiazepin-7-yl]-1,3,4-oxadiazol-2-yl]pyrrolidine-1-carboxylate

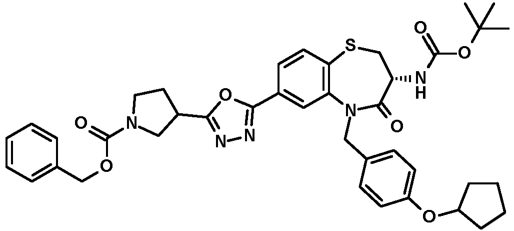

The title compound was prepared from benzyl 3-[[[(3R)-3-(tert-butoxycarbonylamino)-5-[[4-(cyclopentoxy)phenyl]methyl]-4-oxo-2,3-dihydro-1,5-benzothiazepine-7-carbonyl]amino]carbamoyl]pyrrolidine-1-carboxylate (35 mg, 0.046 mmol) in analogy to general procedure 8a and was obtained as white solid (23 mg, 67%). MS (ESI): 640.4 [M+H-Boc]⁺.

### Step f) benzyl 3-[5-[(3R)-3-(tert-butoxycarbonylamino)-5-[[4-(cyclopentoxy)phenyl]methyl]-1,1,4-trioxo-2, 3-dihydro-1λ⁶,5-benzothiazepin-7-yl]-1,3, 4-oxadiazol-2-yl]pyrrolidine-1-carboxylate

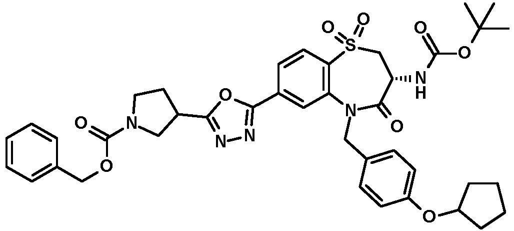

The title compound was prepared from benzyl 3-[5-[(3R)-3-(tert-butoxycarbonylamino)-5-[[4-(cyclopentoxy)phenyl]methyl]-4-oxo-2,3-dihydro-1,5-benzothiazepin-7-yl]-1,3,4-oxadiazol-2-yl]pyrrolidine-1-carboxylate (23 mg, 0.031 mmol) in analogy to general procedure 5 and was obtained as white solid (21 mg, 84%). MS (ESI): 672.4 [M+H-Boc]⁺.

### Step g) tert-butyl N-[(3R)-5-[[4-(cyclopentoxy)phenyl]methyl]-1,1, 4-trioxo-7-(5-pyrrolidin-3-yl-1,3, 4-oxadiazol-2-yl)-2, 3-dihydro-1λ⁶,5-benzothiazepin-3-yl]carbamate

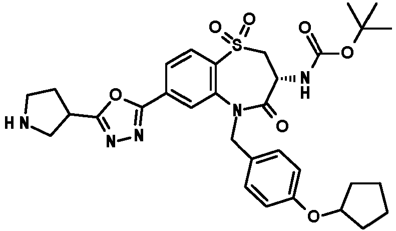

Benzyl 3-[5-[(3R)-3-(tert-butoxycarbonylamino)-5-[[4-(cyclopentoxy)phenyl]methyl]-1,1,4-trioxo-2,3-dihydro-1λ⁶,5-benzothiazepin-7-yl]-1,3,4-oxadiazol-2-yl]pyrrolidine-1-carboxylate (21 mg, 0.026 mmol, 1 eq) was suspended in MeOH (0.67 mL). Argon was bubbled through the suspension for 5 min. Pd/C (2.78 mg, 0.003 mmol, 0.1 eq) was added in one portion and the resulting black suspension was stirred under hydrogen atmosphere at RT for 2 h. The reaction mixture was filtered through a plug of celite. The filter cake was washed thoroughly with MeOH and the filtrate concentrated in vacuo to afford the title compound as yellow solid (14 mg, 81%). MS (ESI): 638.5 [M+H]⁺.

### Step h) methyl 3-[5-[(3R)-3-(tert-butoxycarbonylamino)-5-[[4-(cyclopentoxy)phenyl]methyl]-1,1, 4-trioxo-2, 3-dihydro-1λ⁶,5-benzothiazepin-7yl]-1,3,4-oxadiazol-2-yl]pyrrolidine-1-carboxylate

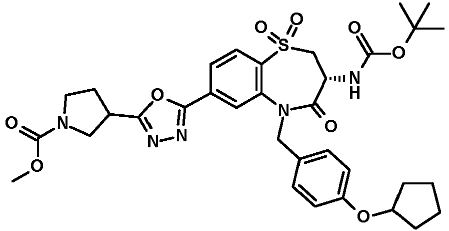

To a solution of tert-butyl N-[(3R)-5-[[4-(cyclopentoxy)phenyl]methyl]-1,1,4-trioxo-7-(5-pyrrolidin-3-yl-1,3,4-oxadiazol-2-yl)-2,3-dihydro-1λ⁶,5-benzothiazepin-3-yl]carbamate (14 mg, 0.021 mmol, 1 eq) in DCM (0.5 ml) was added methyl chloroformate (2.19 mg, 1.8 uL, 0.023 mmol, 1.1 eq) and DIPEA (6.81 mg, 9.2 uL, 0.053 mmol, 2.5 eq). The reaction was stirred at RT for 2 h. The solvent was evaporated and the remaining residue purified by column chromatography on silica gel (0-100% EtOAc in heptane) to afford a white solid (18 mg) containing the title compound, which was used in the next step without further purification. MS (ESI): 596.4 [M+H-Boc]⁺.

### Step i) methyl 3-[5-[(3R)-3-amino-5-[[4-(cyclopentoxy)phenyl]methyl]-1,1, 4-trioxo-2, 3-dihydro-1λ⁶,5-benzothiazepin-7-yl]-1,3,4-oxadiazol-2-yl]pyrrolidine-1-carboxylate

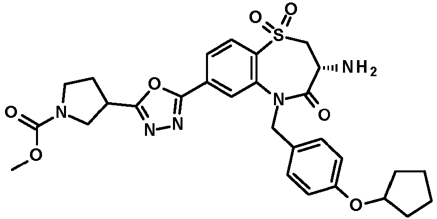

The title compound was prepared from methyl 3-[5-[(3R)-3-(tert-butoxycarbonylamino)-5-[[4-(cyclopentoxy)phenyl]methyl]-1,1,4-trioxo-2,3-dihydro-1λ⁶,5-benzothiazepin-7-yl]-1,3,4-oxadiazol-2-yl]pyrrolidine-1-carboxylate (18 mg, 0.025 mmol) in analogy to general procedure 6d and was obtained as off-white solid, as hydrochloride salt (16 mg, 92%). MS (ESI): 596.5 [M+H]⁺.

### Example 445

### (3R)-3-amino-7-(5-tert-butyl-1,3,4-oxadiazol-2-yl)-5-[[4-(4-cyclopropylpiperazin-1-yl)phenyl]methyl]-1,1-dioxo-2,3-dihydro-1λ⁶,5-benzothiazepin-4-one

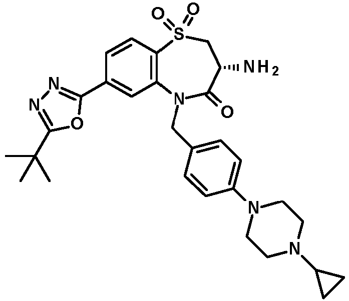

### Step a) tert-butylN-[(3R)-7-(5-tert-butyl-1, 3, 4-oxadiazol-2-yl)-5-[[4-(4-cyclopropylpiperazin-1-yl)phenyl]methyl]-1, 1, 4-trioxo-2, 3-dihydro-1λ⁶,5-benzothiazepin-3-yl]carbamate

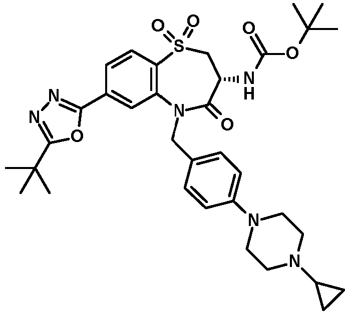

To a solution of tert-butyl N-[(3R)-7-(5-tert-butyl-1,3,4-oxadiazol-2-yl)-1,1,4-trioxo-3,5-dihydro-2H-1λ⁶,5-benzothiazepin-3-yl]carbamate (100 mg, 0.22 mmol, 1 eq, Example 429, step e)) in toluene (1.5 mL) was added [4-(4-cyclopropylpiperazin-1-yl)phenyl]methanol (61.88 mg, 0.27 mmol, 1.2 eq, CAS 1303477-83-9), Ph₃P (116.44 mg, 0.440 mmol, 2 eq) and di-tert-butyl azodicarboxylate (102.22 mg, 0.44 mmol, 2 eq). The mixture was degassed with N₂ and stirred at 50 °C for 3 h. The reaction was diluted with water (5 mL ) and EtOAc (5 ml). The layers were separated and the aqueous phase extracted with EtOAc (3x5 mL). The combined organic extracts were dried over sodium sulfate, filtered and concentrated. The remaining crude was purified using column chromatography on silica gel (5-80% EtOAc in PE) to obtain the title compound (50 mg, 0.08 mmol, 28% yield) as yellow oil. MS (ESI): 665.3 [M+H]⁺

### Step b) (3R)-3-amino-7-(5-tert-butyl-1, 3, 4-oxadiazol-2-yl)-5-[[4-(4-cyclopropylpiperazin-1-yl)phenyl]methyl]-1,1-dioxo-2,3-dihydro-1λ⁶,5-benzothiazepin-4-one

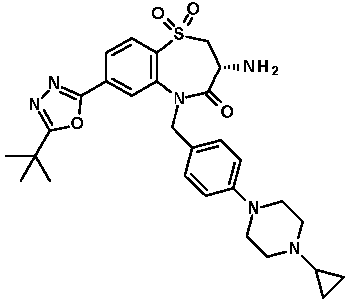

The title compound was prepared from tert-butyl N-[(3R)-7-(5-tert-butyl-1,3,4-oxadiazol-2-yl)-5-[[4-(4-cyclopropylpiperazin-1-yl)phenyl]methyl]-1,1,4-trioxo-2,3-dihydro-1λ⁶,5-benzothiazepin-3-yl]carbamate (50 mg, 0.08 mmol) in analogy to general procedure 6b and was obtained as white powder (6.5 mg, 0.01 mmol, 15% yield). MS (ESI): 565.2 [M+H]⁺

Example 446 to 448 of the following table were prepared in analogy to Example 445, using the appropriate benzylic alcohol building block.

| **Ex.** | **Structure** | **Systematic Name** | **Building Block** | **MS, ESI: m/z** |
|---|---|---|---|---|
| 446 | 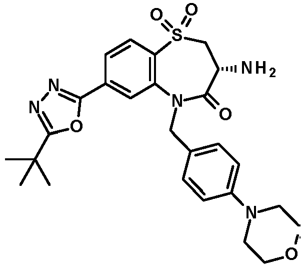 | (3R)-3-amino-7-(5-tert-butyl-1,3,4-oxadiazol-2-yl)-5-[(4-morpholinophenyl)methyl]-1,1-dioxo-2,3-dihydro-1λ⁶,5-benzothiazepin-4-one | 4-(4-Morpholin yl)benzene methanol | 526.1 [M+H]⁺ |
| | | | (CAS 280556-71-0) | |
| 447 | 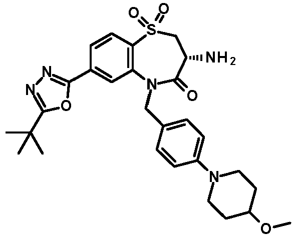 | (3R)-3-amino-7-(5-tert-butyl-1,3,4-oxadiazol-2-yl)-5-[[4-(4-methoxy-1-piperidyl)phenyl]methyl]-1,1-dioxo-2,3-dihydro-1λ⁶,5-benzothiazepin-4-one | 4-(4-Methoxy-1-piperidinyl )benzenem ethanol | 554.2 [M+H]⁺ |
| | | | (CAS 1096813-20-5) | |
| 448 | 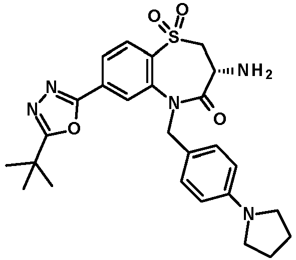 | (3R)-3-amino-7-(5-tert-butyl-1,3,4-oxadiazol-2-yl)-1,1-dioxo-5-[(4-pyrrolidin-1-ylphenyl)methyl]-2,3-dihydro-1λ⁶,5-benzothiazepin-4-one | 4-(1-Pyrrolidiny l)benzene methanol | 510.2 [M+H]⁺ |
| | | | (CAS 676245-12-8) | |

### Example 450

### (3R)-3-amino-7-[5-(1,1-dimethylbut-3-ynyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one

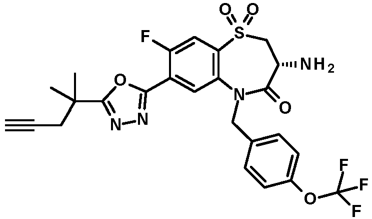

### Step a) tert-butyl N-[(3R)-7-[5-(1,1-dimethylbut-3-ynyl)-1,3,4-oxadiazol-2-yl]-8-fluoro-4-oxo-5-[[4-(trifluoromethoxy)phenyl]methyl]-2,3-dihydro-1, 5-benzothiazepin-3-yl]carbamate

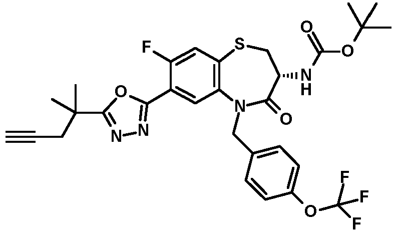

The title compound was prepared according to general method 4 from tert-butyl N-[(3R)-7-[5-(1,1-dimethylbut-3-ynyl)-1,3,4-oxadiazol-2-yl]-8-fluoro-4-oxo-3,5-dihydro-2H-1,5-benzothiazepin-3-yl]carbamate (Example 359, step a)) (80 mg, 174 µmol) and was obtained (111.9 mg) as a light brown viscous oil. MS (ESI): 635.2 [M+H]⁺.

### Step b) tert-butyl N-[(3R)-7-[5-(1,1-dimethylbut-3-ynyl)-1,3, 4-oxadiazol-2-yl]-8-fluoro-1, 1,4-trioxo-5-[[4-(trifluoromethoxy)phenyl]methyl]-2, 3-dihydro-1λ⁶,5-benzothiazepin-3-yl]carbamate

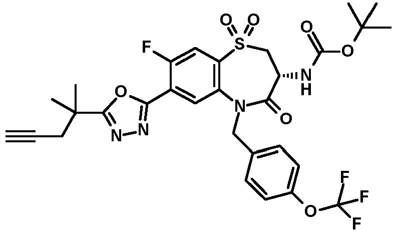

The title compound was prepared in analogy to general method 5 from tert-butyl N-[(3R)-7-[5-(1,1-dimethylbut-3-ynyl)-1,3,4-oxadiazol-2-yl]-8-fluoro-4-oxo-5-[[4-(trifluoromethoxy)phenyl]methyl]-2,3-dihydro-1,5-benzothiazepin-3-yl]carbamate (39.5 mg, 62.2 µmol) and was obtained (51 mg) as a white solid. MS (ESI): 611.1 [M-isobutene+H]⁺.

### Step c) (3R)-3-amino-7-[5-(1,1-dimethylbut-3-ynyl)-1,3,4-oxadiazol-2-yl]-8-fluoro-1,1-dioxo-5-[[4-(trifluoromethoxy)phenyl]methyl]-2, 3-dihydro-1λ⁶,5-benzothiazepin-4-one

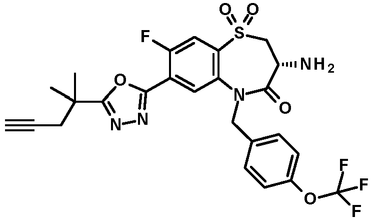

The title compound was prepared according to general method 6b from tert-butyl N-[(3R)-7-[5-(1,1-dimethylbut-3-ynyl)-1,3,4-oxadiazol-2-yl]-8-fluoro-1,1,4-trioxo-5-[[4-(trifluoromethoxy)phenyl]methyl]-2,3-dihydro-1λ⁶,5-benzothiazepin-3-yl]carbamate (50.0 mg, 75 µmol) and was obtained (8.9 mg, 15.68 µmol, 21% yield) as a white waxy solid. MS (ESI): 567.2 [M+H]⁺.

### Example 451

### (3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1λ⁶,5-benzothiazepin-4-one

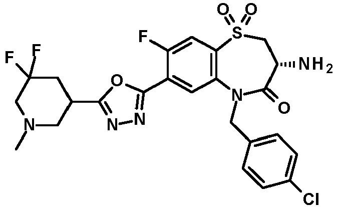

### Step a) benzyl 5-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoyl]-3,3-difluoro-piperidine-1-carboxylate

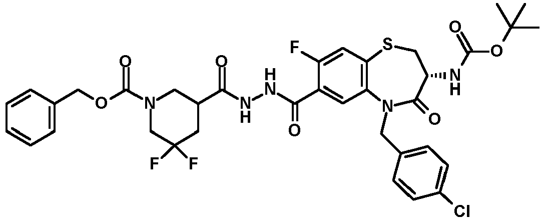

To a solution of 1-benzyloxycarbonyl-5,5-difluoro-piperidine-3-carboxylic acid (328.0 mg, 1.1 mmol, 1.21 eq) in DMF (8 mL) were added [dimethylamino(triazolo[4,5-b]pyridin-3-yloxy)methylidene]-dimethylazanium; hexafluorophosphate (519.0 mg, 1.36 mmol, 1.5 eq) and DIPEA (0.48 mL, 2.75 mmol, 3.02 eq) and the mixture was stirred for 30 min. To the mixture was added tert-butyl N-[ (3R)-5-[(4-chlorophenyl)methyl]-8-fluoro-7-(hydrazinecarbonyl)-4-oxo-2,3-dihydro-1,5-benzothiazepin-3-yl]carbamate (Example 63, step a) (450.0 mg, 0.910 mmol, 1 eq) in DMF (2 mL) and the mixture was stirred at 25°C for 30 min. The mixture was poured into water and was extracted with EtOAc. The organic layers were combined and washed with brine, dried over Na₂SO₄ and concentrated in vacuo. The remaining residue was purified by column chromatography affording the title compund (350 mg, 0.450 mmol, 49% yield) as a yellow solid. MS (ESI): 776.2 [M+H]⁺.

### Step b) benzyl 5-[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]-3,3-difluoro-piperidine-1-carboxylate

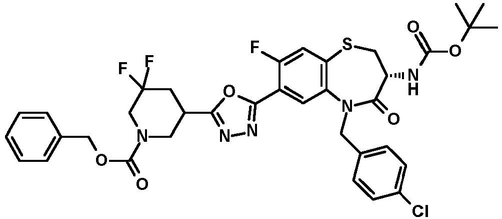

To a solution of benzyl 3,3-difluoro-5-[[[ (3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoyl]piperidine-1-carboxylate (100 mg, 0.12 mmol, 1 eq) in 1,4-dioxane (6 mL) was added Burgess reagent (120.0 mg, 0.94 mmol, 14.54 eq). The mixture was stirred at 115°C for 12 h under an atmosphere of nitrogen. The mixturewas poured into water and was extracted with EtOAc. The organic layers were combined and washed with brine, dried over Na₂SO₄ and concentrated in vacuo. The residue was purified by column chromatography affording the title compound (100 mg, 0.130 mmol) as yellow solid. MS (ESI): 758.2 [M+H]⁺.

### Step c) benzyl 5-[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,3,4-oxadiazol-2-yl]-3,3-difluoro-piperidine-1-carboxylate

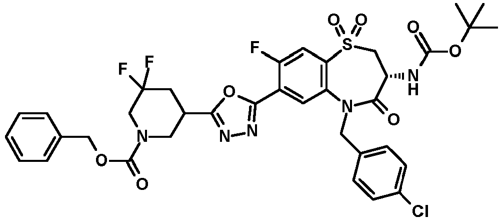

To a solution of benzyl 3,3-difluoro-5-[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]piperidine-1-carboxylate (90.0 mg, 0.120 mmol, 1 eq) in DCM (4 mL) was added m-CPBA (62.0 mg, 0.360 mmol, 3.03 eq). The mixture was stirred at 25°C for 12 h and then washed with aqueous NaHCO₃ and aqueous Na₂SO₃ solution. Then the mixture was extracted with EtOAc, the organic layers were combined and washed with brine, dried over Na₂SO₄ and concentrated in vacuo affording the title compound (74 mg, 0.090 mmol, 57% yield) as a white solid. MS (ESI): 790.3 [M+H]⁺.

### Step d) tert-butyl N-[(3R)-5-[(4-chlorophenyl)methyl]-7-[5-(5,5-difluoro-3-piperidyl)-1,3, 4-oxadiazol-2-yl]-8-fluoro-1,1,4-trioxo-2, 3-dihydro-1λ ⁶,5-benzothiazepin-3-yl]carbamate

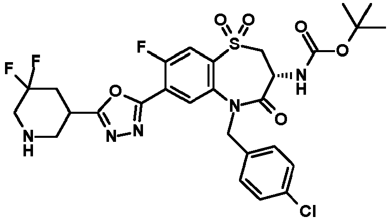

To a solutio of benzyl 3,3-difluoro-5-[5-[ (3R)-3-(tert-butoxycarbonylamino)-5-[(4-chlorophenyl)methyl]-8-fluoro-1,1,4-trioxo-2,3-dihydro-1λ6,5-benzothiazepin-7-yl]-1,3,4-oxadiazol-2-yl]piperidine-1-carboxylate (55.0 mg, 0.070 mmol, 1 eq) in MeOH (2 mL) was added Pd/C (40.0 mg) and the mixture was stirred at 25°C for 25 min under an atmosphere of hydrogen. The mxiture was filtered and purified by prep-HPLC affording the the title comound (30 mg, 0.050 mmol, 66% yield) as a white solid. MS (ESI): 656.1 [M+H]⁺.

### e) tert-butyl N-[(3R)-5-[(4-chlorophenyl)methyl]-7-[5-(5,5-difluoro-1-methyl-3-piperidyl)-1,3,4-oxadiazol-2-yl]-8-fluoro-1,1, 4-trioxo-2, 3-dihydro-1λ ⁶,5-benzothiazepin-3-yl]carbamate

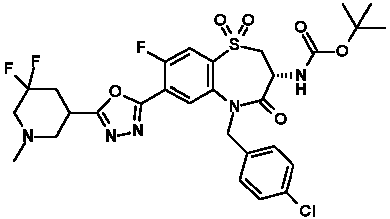

To a solution of tert-butyl N-[(3R)-5-[(4-chlorophenyl)methyl]-7-[5-(5,5-difluoro-3-piperidyl)-1,3,4-oxadiazol-2-yl]-8-fluoro-1,1,4-trioxo-2,3-dihydro-1λ⁶,5-benzothiazepin-3-yl]carbamate (20.0 mg, 0.030 mmol, 1 eq) in MeOH (2 mL) was added formaldehyde (33.0 mg, 0.410 mmol, 13.34 eq). After the mixture was stirred for 10 min sodiumtriacetoxyborohydride (66.0 mg, 0.310 mmol, 10.21 eq) was added. Then the mixture was stirred at 25°C for 1 h and then poured into water and extracted with DCM. The combined organic layers were washed with brine, dried over Na₂SO₄ and concentrated in vacuo. The remaining residue was purified by prep-HPLC affording the title compound (11 mg, 0.020 mmol, 43% yield) as a white solid. MS (ESI): 670.2 [M+H]⁺.

### f) (3R)-3-amino-5-[(4-chlorophenyl)methyl]-7-[5-(5, 5-difluoro-1-methyl-3-piperidyl)-1,3,4-oxadiazol-2-yl]-8-fluoro-1,1-dioxo-2,3-dihydro-1λ ⁶,5-benzothiazepin-4-one

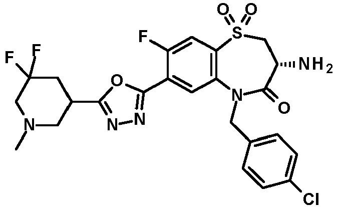

To a solution of tert-butyl N-[(3R)-5-[(4-chlorophenyl)methyl]-7-[5-(5,5-difluoro-1-methyl-3-piperidyl)-1,3,4-oxadiazol-2-yl]-8-fluoro-1,1,4-trioxo-2,3-dihydro-1λ6,5-benzothiazepin-3-yl]carbamate (11.0 mg, 0.020 mmol, 1 eq) in EtOAc (2 mL) was added HCl in EtOAc (2.0 mL, 8 mmol, 487.35 eq). The mixture was stirred at 25°C for 1 h and then concentrated in vacuo. The remaining residue was purified by prep-HPLC and after lyophilisation the title compound (4.6 mg, 0.010 mmol, 45% yield) was obtained as white solid as a formic acid salt. MS (ESI): 570.2 [M+H]⁺.

### Example 452

### (3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one (Epimer A)

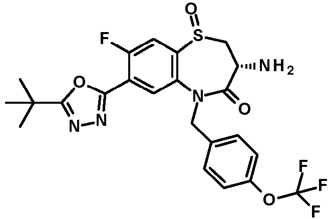

### Step a) tert-butyl N-[(3R)-7-(5-tert-butyl-1, 3, 4-oxadiazol-2-yl)-8-fluoro-1,4-dioxo-5-[[4-(trifluoromethoxy)phenyl]methyl]-2,3-dihydro-1λ ⁴,5-benzothiazepin-3-yl]carbamate epimer A and epimer B

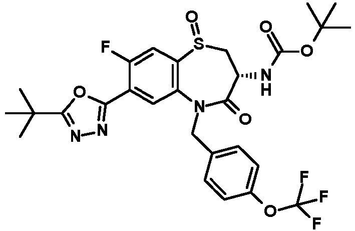

To a solution of tert-butyl N-[(3R)-7-(5-tert-butyl-1,3,4-oxadiazol-2-yl)-8-fluoro-4-oxo-5-[[4-(trifluoromethoxy)phenyl]methyl]-2,3-dihydro-1,5-benzothiazepin-3-yl]carbamate (Example 54, step b)) (230.0 mg, 0.380 mmol, 1 eq) in DCM (10 mL) was added m-CPBA (40.63 mg, 0.190 mmol, 0.500 eq), the mixture was stirred at 20°C for 16 h. The mixture was diluted with aqueous Na₂SO₃ sol., aqueous Na₂CO₃ sol. and DCM, washed with H₂O and brine, dried over Na₂SO₄, filtered and concentrated to give a residue which was purified by column chromatography, affording the title compound epimer A (120 mg, 0.190 mmol, 51% yield) as colorless gum (MS (ESI): 571.1 [M-isobutene+H]⁺) and tert-butyl N-[(3R)-7-(5-tert-butyl-1,3,4-oxadiazol-2-yl)-8-fluoro-1,4-dioxo-5-[[4-(trifluoromethoxy)phenyl]methyl]-2,3-dihydro-1λ⁴,5-benzothiazepin-3-yl]carbamate (epimer B) (100 mg, 0.160 mmol, 42% yield) as colorless gum. MS (ESI): 571.1 [M-isobutene+H]⁺.

### Step b) (3R)-3-amino-7-(5-tert-butyl-1, 3, 4-oxadiazol-2-yl)-8-fluoro-1-oxo-5-[[4-(trifluoromethoxy)phenyl]methyl]-2,3-dihydro-1λ ⁴,5-benzothiazepin-4-one (epimer A)

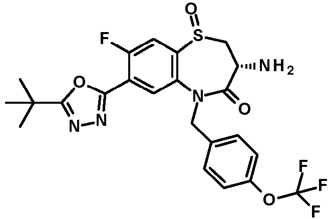

A solution of tert-butyl N-[(3R)-7-(5-tert-butyl-1,3,4-oxadiazol-2-yl)-8-fluoro-1,4-dioxo-5-[[4-(trifluoromethoxy)phenyl]methyl]-2,3-dihydro-1λ4,5-benzothiazepin-3-yl]carbamate (epimer A) (100.0 mg, 0.160 mmol, 1 eq) in HCl/EtOAc (10.0 mL, 40 mmol, 250.65 eq) was stirred at 20°C for 1 h. The mixture was concentrated to give a residue, which was purified by prep-HPLC (HCl). The elution was lyophilized and the residue was again purified by prep-HPLC. The elution was lyophilized affording the title compound (29.6 mg, 0.050 mmol, 33% yield) as a white solid as a hydrochloride salt. MS (ESI): 527.0 [M+H]⁺.

Example 453 of the following table was prepared in analogy to Example 452.

| **Ex.** | **Structure** | **Systematic Name** | **MS, ESI: m/z** |
|---|---|---|---|
| 453 | 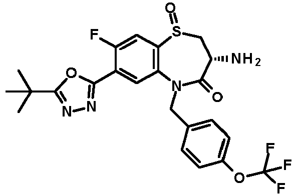 | (3R)-3-amino-7-(5-tert-butyl-1,3,4-oxadiazol-2-yl)-8-fluoro-1-oxo-5-[[4-(trifluoromethoxy)phenyl]met hyl]-2,3-dihydro-1λ⁴,5-benzothiazepin-4-one (Epimer B) | 527.1 [M+H]⁺ |

### Example 454

### (3R)-3-amino-5-[(4-chlorophenyl)methyl]-8-fluoro-7-(5-morpholino-1,3,4-oxadiazol-2-yl)-1,1-dioxo-2,3-dihydro-1λ⁶,5-benzothiazepin-4-one

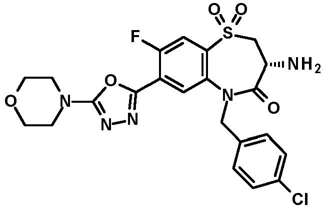

### Step a) tert-butylN-[(3R)-5-[(4-chlorophenyl)methyl]-8-fluoro-4-oxo-7-(2-oxo-3H-1,3,4-oxadiazol-5-yl)-2,3-dihydro-1,5-benzothiazepin-3-yl]carbamate

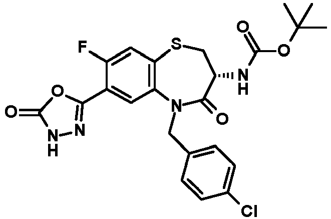

To a solution of tert-butyl (R)-(5-(4-chlorobenzyl)-8-fluoro-7-(hydrazinecarbonyl)-4-oxo-2,3,4,5-tetrahydrobenzo[b][1,4]thiazepin-3-yl)carbamate (Example 63, step a) (462 mg, 0.905 mmol, 1 eq) in dry tetrahydrofuran (9.24 mL) were added NEt₃ (97.11 mg, 133 uL, 0.96 mmol, 1.06 eq) and CDI (155.62 mg, 0.960 mmol, 1.06 eq) under an atmosphere of nitrogen. The light yellow solution was stirred at room temperature for 2 h. The reaction mixture was concentrated under vacuo and the residue was taken up in EtOAc, washed with aqueous 1N HCl solution, aqueous sat. NaHCO₃ solution and brine, dried over magnesium sulfate, filtered and evaporated to afford the title compound (406 mg, 84 %) as a light yellow foam. MS (ESI): 465.2 [M-isobutene+H]⁺.

### Step b) tert-butyl N-[(3R)-5-[(4-chlorophenyl)methyl]-8-fluoro-7-(5-morpholino-1,3,4-oxadiazol-2-yl)-4-oxo-2,3-dihydro-1,5-benzothiazepin-3-yl]carbamate

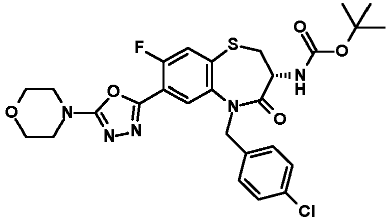

To a colorless solution of N-[(3R)-5-(4-chlorobenzyl)-8-fluoro-4-keto-7-(2-keto-3H-1,3,4-oxadiazol-5-yl)-2,3-dihydro-1,5-benzothiazepin-3-yl]carbamic acid tert-butyl ester (40 mg, 0.075 mmol, 1 eq) in dry DMF (1 mL) were added DIPEA (19.45 mg, 25.59 uL, 0.150 mmol, 2 eq) and morpholine (13.11 mg, 13.02 uL, 0.150 mmol, 2 eq). BOP (36.6 mg, 0.083 mmol, 1.1 eq) was added after stirring for 5 min. The reaction mixture was stirred at room temperature for 2 h. The reaction mixture was poured on ice-water and the resulting mixture was extracted with EtOAc three times. The organic layers were combined and washed with brine, dried over MgSO₄, filtered and concentrated under vacuum to obtain the crude product as light yellow oil. The crude residue was purified by column chromatography on silica gel (heptane:EtOAc 1:0 to 1:1) affording the title compound (32 mg, 72% yield) as a white solid. MS (ESI): 590.4 [M+H]⁺.

### Step c) tert-butyl N-[(3R)-5-[(4-chlorophenyl)methyl]-8-fluoro-7-(5-morpholino-1,3,4-oxadiazol-2-yl)-1,1,4-trioxo-2,3-dihydro-1λ ⁶,5-henzothiazepin-3-yl]carbamate

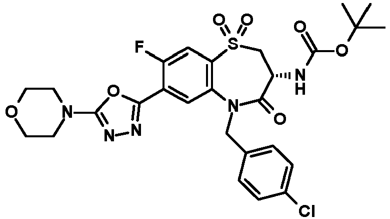

To a solution of N-[(3R)-5-(4-chlorobenzyl)-8-fluoro-4-keto-7-(5-morpholino-1,3,4-oxadiazol-2-yl)-2,3-dihydro-1,5-benzothiazepin-3-yl]carbamic acid tert-butyl ester (29 mg, 0.049 mmol, 1 eq) in tetrahydrofuran, extra dry (0.500 mL) was added m-CPBA (27.54 mg, 0.123 mmol, 2.5 eq). The reaction mixture was stirring at room temperature for 6 h. NaOH 1N aq. was added and the mixture was extracted with EtOAc three times. The combined organic layers were washed with water and brine, dried over magnesium sulfate, filtered and evaporated. The crude residue was purified by column chromatography on silica gel (heptane:EtOAc 1:0 to 1:1) affording the title compound (20 mg, 66%) as a white solid. MS (ESI): 622.4 [M+H]⁺.

### Step d) (3R)-3-amino-5-[(4-chlorophenyl)methyl]-8-fluoro-7-(5-morpholino-1, 3, 4-oxadiazol-2-yl)-1,1-dioxo-2, 3-dihydro-1λ ⁶,5-benzothiazepin-4-one

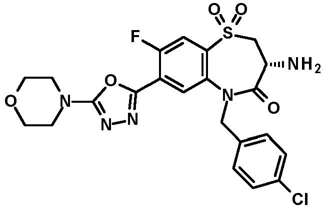

The title compound was prepared in analogy to method 6c from N-[(3R)-5-(4-chlorobenzyl)-8-fluoro-1,1,4-triketo-7-(5-morpholino-1,3,4-oxadiazol-2-yl)-2,3-dihydro-1λ6,5-benzothiazepin-3-yl]carbamic acid tert-butyl ester (17 mg, 0.027 mmol) with additional 4 drops of HCl (4M in dioxane) and was obtained as a white solid, as a hydrochloric salt and HFIP adduct (13.2 mg, 67%). MS (ESI): 522.3 [M+H]⁺.

### Example 455

### (3R)-3-amino-5-[(4-chlorophenyl)methyl]-7-[5-(cyclopentylamino)-1,3,4-oxadiazol-2-yl]-8-fluoro-1,1-dioxo-2,3-dihydro-1λ⁶,5-benzothiazepin-4-one

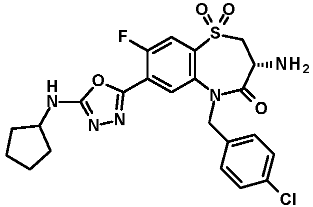

### Step a) tert-butyl N-[(3R)-5-[(4-chlorophenyl)methyl]-7-[5-(cyclopentylamino)-1,3, 4-oxadiazol-2-yl]-8-fluoro-1,1, 4-trioxo-2, 3-dihydro-1λ ⁶,5-henzothiazepin-3-yl]carbamate

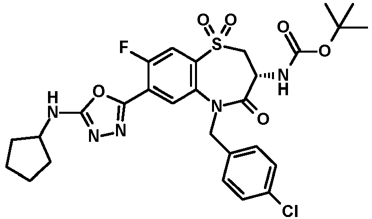

To a colorless solution of N-[(3R)-5-(4-chlorobenzyl)-8-fluoro-1,1,4-triketo-7-(2-keto-3H-1,3,4-oxadiazol-5-yl)-2,3-dihydro-1λ⁶,5-benzothiazepin-3-yl]carbamic acid tert-butyl ester (Example 454, step a)) (50 mg, 0.081 mmol, 1 eq) in dry DMF (0.909 mL) were added DIPEA (21.04 mg, 28.43 uL, 0.163 mmol, 2 eq) and cyclopentylamine (13.86 mg, 16.58 uL, 0.163 mmol, 2 eq). Then BOP (39.58 mg, 0.090 mmol, 1.1 eq) was added and the mixture was stirred over night. The reaction mixture was poured on ice-water and extracted with EtOAc three times. The organic layers were combined and washed with brine, dried over Na₂SO₄, filtered and concentrated in vacuo. The crude residue was purified by column chromatography on silica gel to afford the title compound (24.5 mg, 46%) as an off-white solid. MS (ESI): 620.4 [M+H]⁺.

### Step b) tert-butyl N-[(3R)-5-[(4-chlorophenyl)methyl]-7-[5-(cyclopentylamino)-1, 3,4-oxadiazol-2-yl]-8-fluoro-1,1,4-trioxo-2,3-dihydro-1λ⁶,5-benzothiazepin-3-yl]carbamate

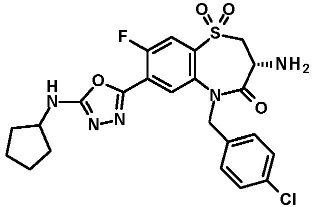

The title compound was prepared in analogy to method 6c from N-[(3R)-5-(4-chlorobenzyl)-7-[5-(cyclopentylamino)-1,3,4-oxadiazol-2-yl]-8-fluoro-1,1,4-triketo-2,3-dihydro-1λ6,5-benzothiazepin-3-yl]carbamic acid tert-butyl ester (20.19 mg, 0.031 mmol) with additional 4 drops of HCl (4M in dioxane) and was obtained as a light yellow solid, as a hydrochloric salt (16.9 mg, 97%). MS (ESI): 520.3 [M+H]⁺.

The examples 456 to 482 of the following table were prepared in analogy to Example 455, using the appropriate amine building block:

| **Ex.** | **Structure** | **Systematic Name** | **Building Block** | **MS, El: m/z** |
|---|---|---|---|---|
| 456 | 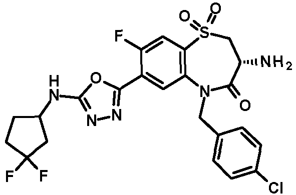 | (3R)-3-amino-5-[(4-chlorophenyl)methyl]-7-[5-[(3,3-difluorocyclopentyl)amino]-1,3,4-oxadiazol-2-yl]-8-fluoro-1,1-dioxo-2,3-dihydro-1λ⁶,5-benzothiazepin-4-one | 3,3-Difluorocy clopentan-1-amine | 556.2 [M+H]⁺ |
| | | | (CAS 939525-61-8) | |
| | | (*) | | |
| 457 | 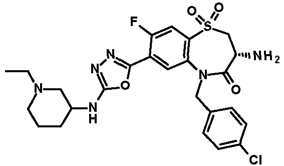 | (3R)-3-amino-5-[(4-chlorophenyl)methyl]-7-[5-[(1-ethyl-3-piperidyl)amino]-1,3,4-oxadiazol-2-yl]-8-fluoro-1,1-dioxo-2,3-dihydro-1λ⁶,5-benzothiazepin-4-one | (1-Ethyl-3-piperidyl) amine | 663.5 [M+H]⁺ |
| | | | (CAS 6789-94-2) | |
| 458 | 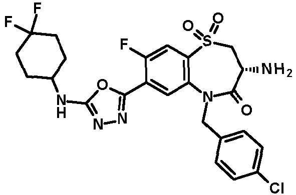 | (3R)-3-amino-5-[(4-chlorophenyl)methyl]-7-[5-[(4,4-difluorocyclohexyl)amino]-1,3,4-oxadiazol-2-yl]-8-fluoro-1,1-dioxo-2,3-dihydro-1λ⁶,5-benzothiazepin-4-one | (4,4-Difluorocy clohexyl) amine hydrochloride | 570.2 [M+H]⁺ |
| | | (*) | (CAS 675112-70-6) | |
| 459 | 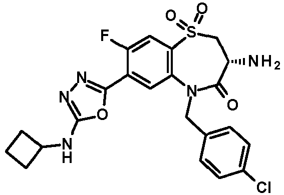 | (3R)-3-amino-5-[(4-chlorophenyl)methyl]-7-[5-(cyclobutylamino)-1,3,4-oxadiazol-2-yl]-8-fluoro-1,1-dioxo-2,3-dihydro-1λ⁶,5-benzothiazepin-4-one | Cyclobutyl amine | 506.2 [M+H]⁺ |
| | | | (CAS 2516-34-9) | |
| | | (*) | | |
| 460 | 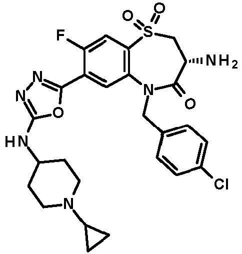 | (3R)-3-amino-5-[(4-chlorophenyl)methyl]-7-[5-[(1-cyclopropyl-4-piperidyl)amino]-1,3,4-oxadiazol-2-yl]-8-fluoro-1,1-dioxo-2,3-dihydro-1λ⁶,5-benzothiazepin-4-one | (1-Cyclopropyl -4-piperidyl)a mine | 573.3 [M-H] |
| | | | (CAS 62813-02-9) | |
| | | (*) | | |
| 461 | 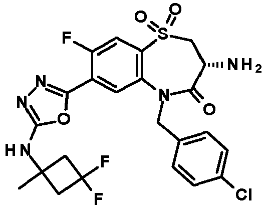 | (3R)-3-amino-5-[(4-chlorophenyl)methyl]-7-[5-[(3,3-difluoro-1-methylcyclobutyl)amino]-1,3,4-oxadiazol-2-yl]-8-fluoro-1,1-dioxo-2,3-dihydro-1λ⁶,5-benzothiazepin-4-one | (3,3-Difluoro-1-methylcyclobutyl) amine;hyd rochloride | 556.2 [M+H]⁺ |
| | | | (CAS 1408076-03-8) | |
| | | (*) | | |
| 462 | 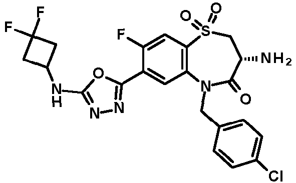 | (3R)-3-amino-5-[(4-chlorophenyl)methyl]-7-[5-[(3,3-difluorocyclobutyl)amino]-1,3,4-oxadiazol-2-yl]-8-fluoro-1,1-dioxo-2,3-dihydro-1λ⁶,5-benzothiazepin-4-one | (3,3-Difluorocy clobutyl)a mine;hydr ochloride | 542.2 [M+H]⁺ |
| | | | (CAS 637031-93-7) | |
| | | (*) | | |
| 463 | 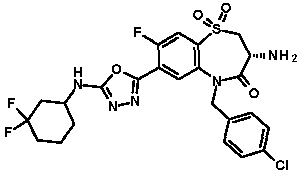 | (3R)-3-amino-5-[(4-chlorophenyl)methyl]-7-[5-[(3,3-difluorocyclohexyl)amino]-1,3,4-oxadiazol-2-yl]-8-fluoro-1,1-dioxo-2,3-dihydro-1λ⁶,5-benzothiazepin-4-one | (3,3-Difluorocy clohexyl)a mine;hydr ochloride | 542.2 [M+H]⁺ |
| | | | (CAS 921602-77-9) | |
| | | (*) | | |
| 464 | 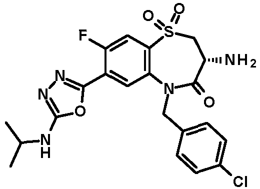 | (3R)-3-amino-5-[(4-chlorophenyl)methyl]-8-fluoro-7-[5-(isopropylamino)-1,3,4-oxadiazol-2-yl]-1,1-dioxo-2,3-dihydro-1λ⁶,5-benzothiazepin-4-one | 2-Azaspiro[3. 3]heptane | 556.3 [M+H]⁺ |
| | | | (CAS 665-04-3) | |
| | | (*) | | |
| 465 | 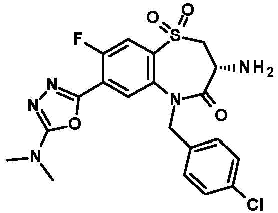 | (3R)-3-amino-5-[(4-chlorophenyl)methyl]-7-[5-(dimethylamino)-1,3,4-oxadiazol-2-yl]-8-fluoro-1,1-dioxo-2,3-dihydro-1λ⁶,5-benzothiazepin-4-one | Dimethyla mine hydrochlor ide | 542.2 [M+H]⁺ |
| | | | (CAS 506-59-2) | |
| | | (**) | | |
| 466 | 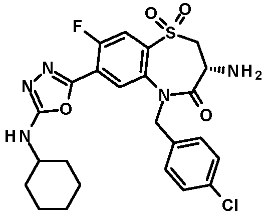 | (3R)-3-amino-5-[(4-chlorophenyl)methyl]-7-[5-(cyclohexylamino)-1,3,4-oxadiazol-2-yl]-8-fluoro-1,1-dioxo-2,3-dihydro-1λ⁶,5-benzothiazepin-4-one | Cyclohexyl amine | 534.3 [M+H]⁺ |
| | | | (CAS 108-91-8) | |
| | | (**) | | |
| 467 | 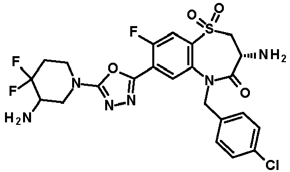 | (3R)-3-amino-7-[5-(3-amino-4,4-difluoro-1-piperidyl)-1,3,4-oxadiazol-2-yl]-5-[(4-chlorophenyl)methyl]-8-fluoro-1,1-dioxo-2,3-dihydro-1λ⁶,5-benzothiazepin-4-one | N-(4,4-Difluoro-3-piperidyl)c arbamic acid tert-butyl ester | 571.3 [M+H]⁺ |
| | | | (CAS 1052713-53-7) | |
| | | (*) | | |
| 468 | 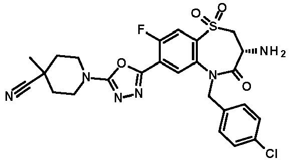 | 4-methyl-1-[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]piperidine-4-carbonitrile | 4-Methylison ipecotonitr ile hydrochlor ide | 559.2 [M+H]⁺ |
| | | (*) | (CAS 948894-26-6) | |
| 469 | 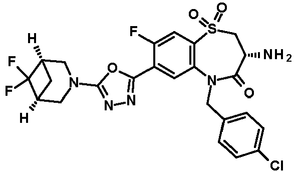 | (3R)-3-amino-5-[(4-chlorophenyl)methyl]-8-fluoro-1,1-dioxo-7-[5-[(1R,5S)-6,6-difluoro-3-azabicyclo[3.1.1]heptan-3-yl]-1,3,4-oxadiazol-2-yl]-2,3-dihydro-1λ⁶,5-benzothiazepin-4-one | (1R,5S)-6,6-Difluoro-3-azabicyclo[ 3.1.1]hept ane hydrochlor ide | 568.2 [M+H]⁺ |
| | | (*) | (CAS 1788054-81-8 ) | |
| 470 | 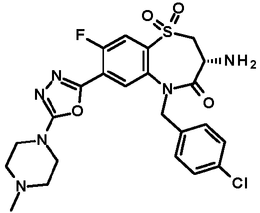 | (3R)-3-amino-5-[(4-chlorophenyl)methyl]-8-fluoro-7-[5-(4-methylpiperazin-1-yl)-1,3,4-oxadiazol-2-yl]-1,1-dioxo-2,3-dihydro-1λ⁶,5-benzothiazepin-4-one (*) | 1-Methylpip erazine | 268.5 [M+H]^{2 +} |
| | | | (CAS 109-01-3) | |
| 471 | 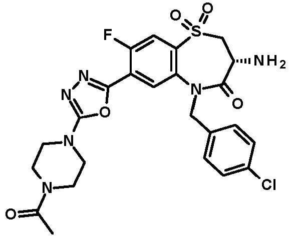 | (3R)-7-[5-(4-acetylpiperazin-1-yl)-1,3,4-oxadiazol-2-yl]-3-amino-5-[(4-chlorophenyl)methyl]-8-fluoro-1,1-dioxo-2,3-dihydro-1λ⁶,5-benzothiazepin-4-one | 1-Acetylpipe razine | 563.3 [M+H]⁺ |
| | | | (CAS 13889-98-0) | |
| | | (*) | | |
| 472 | 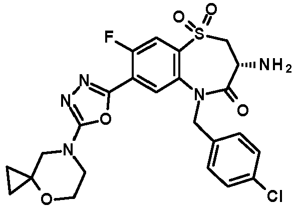 | (3R)-3-amino-5-[(4-chlorophenyl)methyl]-8-fluoro-7-[5-(4-oxa-7-azaspiro[2.5]octan-7-yl)-1,3,4-oxadiazol-2-yl]-1,1-dioxo-2,3-dihydro-1λ⁶,5-benzothiazepin-4-one (*) | 4-Oxa-7-azaspiro[2. 5]octane | 548.3 [M+H]⁺ |
| | | | (CAS 220291-92-9) | |
| 473 | 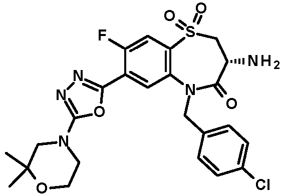 | (3R)-3-amino-5-[(4-chlorophenyl)methyl]-7-[5-(2,2-dimethylmorpholin-4-yl)-1,3,4-oxadiazol-2-yl]-8-fluoro-1,1-dioxo-2,3-dihydro-1λ⁶,5-benzothiazepin-4-one | 2,2-Dimethylm orpholine | 550.3 [M+H]⁺ |
| | | | (CAS 147688-58-2) | |
| | | (*) | | |
| 474 | 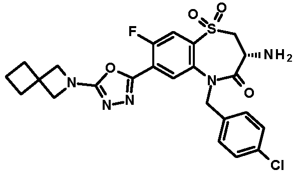 | 3R)-3-amino-7-[5-(2-azaspiro[3.3]heptan-2-yl)-1,3,4-oxadiazol-2-yl]-5-[(4-chlorophenyl)methyl]-8-fluoro-1,1-dioxo-2,3-dihydro-1λ⁶,5-benzothiazepin-4-one | 2-Azaspiro[3. 3]heptane | 532.3 [M+H]⁺ |
| | | | (CAS 665-04-3) | |
| | | (*) | | |
| 475 | 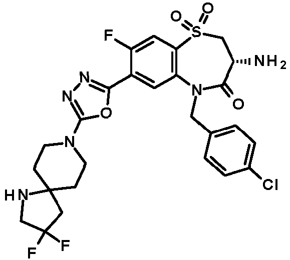 | (3R)-3-amino-5-[(4-chlorophenyl)methyl]-7-[5-(3,3-difluoro-1,8-diazaspiro[4.5]decan-8-yl)-1,3,4-oxadiazol-2-yl]-8-fluoro-1,1-dioxo-2,3-dihydro-1λ⁶,5-benzothiazepin-4-one;hydrochloride | 3,3-Difluoro-1,8-diazaspiro[ 4.5]decane dihydrochl oride | 611.3 [M+H]⁺ |
| | | | (CAS 1895392-87-6) | |
| | | (*) | | |
| 476 | 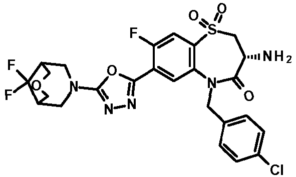 | (3R)-3-amino-5-[(4-chlorophenyl)methyl]-7-[5-(9,9-difluoro-3-oxa-7-azabicyclo[3.3.1]nonan-7-yl)-1,3,4-oxadiazol-2-yl]-8-fluoro-1,1-dioxo-2,3-dihydro-1λ⁶,5-benzothiazepin-4-one | 9,9-Difluoro-3-oxa-7-azabicyclo[ 3.3.1]nona ne hydrochlor ide | 598.3 [M+H]⁺ |
| | | (*) | (CAS 1783765-41-2) | |
| 477 | 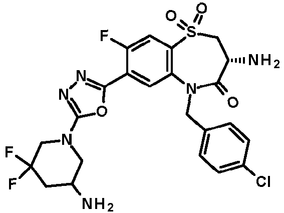 | (3R)-3-amino-7-[5-(5-amino-3,3-difluoro-1-piperidyl)-1,3,4-oxadiazol-2-yl]-5-[(4-chlorophenyl)methyl]-8-fluoro-1,1-dioxo-2,3-dihydro-1λ⁶,5-benzothiazepin-4-one | N-(5,5-Difluoro-3-piperidyl)c arbamic acid tert-butyl ester | 571.3 [M+H]⁺ |
| | | (*) | (CAS 1303973-94-5) | |
| 478 | 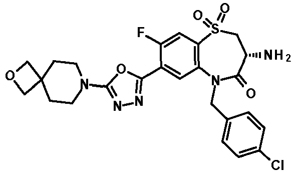 | (3R)-3-amino-5-[(4-chlorophenyl)methyl]-8-fluoro-7-[5-(2-oxa-7-azaspiro[3.5]nonan-7-yl)-1,3,4-oxadiazol-2-yl]-1,1-dioxo-2,3-dihydro-1λ⁶,5-benzothiazepin-4-one (*) | 2-Oxa-7-azaspiro[3. 5]nonane | 562.2 [M+H]⁺ |
| | | | (CAS 241820-91-7) | |
| 479 | 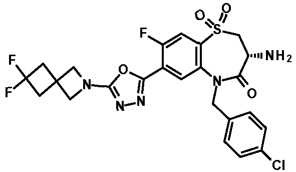 | (3R)-3-amino-5-[(4-chlorophenyl)methyl]-7-[5-(6,6-difluoro-2-azaspiro[3.3]heptan-2-yl)-1,3,4-oxadiazol-2-yl]-8-fluoro-1,1-dioxo-2,3-dihydro-1λ⁶,5-benzothiazepin-4-one | 6,6-Difluoro-2-azaspiro[3. 3]heptane; 2,2,2-trifluoroac etic acid | 568.3 [M+H]⁺ |
| | | (***) | (CAS 1427367-47-2) | |
| 480 | 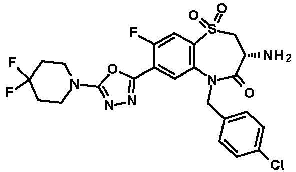 | (3R)-3-amino-5-[(4-chlorophenyl)methyl]-7-[5-(4,4-difluoro-1-piperidyl)-1,3,4-oxadiazol-2-yl]-8-fluoro-1,1-dioxo-2,3-dihydro-1λ⁶,5-benzothiazepin-4-one | 4,4-Difluoropi peridine | 556.3 [M+H]⁺ |
| | | | (CAS 21987-29-1) | |
| | | (***) | | |
| 481 | 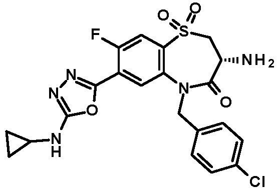 | (3R)-3-amino-5-[(4-chlorophenyl)methyl]-7-[5-(cyclopropylamino)-1,3,4-oxadiazol-2-yl]-8-fluoro-1,1-dioxo-2,3-dihydro-1λ⁶,5-benzothiazepin-4-one | Cyclopropy lamine | 492.3 [M+H]⁺ |
| | | | (CAS 765-30-0) | |
| | | (***) | | |
| 482 | 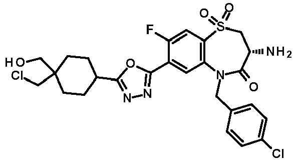 | (3R)-3-amino-7-[5-[4-(chloromethyl)-4-(hydroxymethyl)cyclohexyl]-1,3,4-oxadiazol-2-yl]-5-[(4-chlorophenyl)methyl]-8-fluoro-1,1-dioxo-2,3-dihydro-1λ⁶,5-benzothiazepin-4-one | 2-Oxa-7-azaspiro[3. 5]nonane | 598.1 [M+H]⁺ |
| | | | (CAS 241820-91-7) | |
| | | (*) | | |

| | | | | |
|---|---|---|---|---|
| * as a hydrochloride salt ** as a 1,1,1,3,3,3-hexafluoropropan-2-ol adduct *** as a hydrochloride salt and 1,1,1,3,3,3-hexafluoropropan-2-ol adduct **** as a formic acid salt | | | | |

### Example 483

### (3R)-3-amino-5-[(4-chlorophenyl)methyl]-8-fluoro-7-[5-[[1-(2-hydroxyethyl)cyclohexyl]amino]-1,3,4-oxadiazol-2-yl]-1,1-dioxo-2,3-dihydro-1λ⁶,5-benzothiazepin-4-one

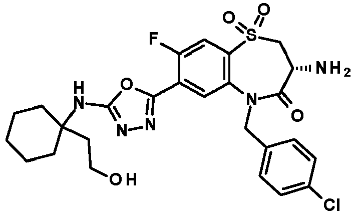

### Step a) 1-[2-[tert-butyl (dimethyl) silyl]oxyethyl]cyclohexanaminee

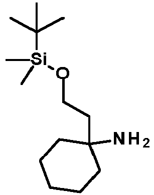

To a solution of 2-(1-aminocyclohexyl) ethanol (CAS 24682-53-9) (50 mg, 0.349 mmol, 1 eq) in THF (3.49 ml) was added tert-butyldimethylsilyl chloride (68.4 mg, 0.454 mmol, 1.3 eq) and imidazole (59.41 mg, 0.873 mmol, 2.5 eq). The light yellow solution was stirred at RT for 1 d. Water and EtAOc were added to the reaction mixture. The layers were separated and the organic layer was washed with water and brine, dried over magnesium sulfate and concentrated in vacuo. The crude product was purified by flash chromatography on silica gel (EtOAc in heptane, 0-100%) to afford the title compound (52.3 mg, 58%) as light yellow oil. MS (ESI): 258.4 [M+H]⁺.

### Step b) tert-butyl N-[(3R)-7-[[[1-[2-[tert-butyl(dimethyl)silyl]oxyethyl]cyclohexyl]carbamoylamino]carbamoyl]-5-[(4-chlorophenyl)methyl]-8-fluoro-1,1, 4-trioxo-2, 3-dihydro-1λ ⁶,5-benzothiazepin-3-yl]carbamate

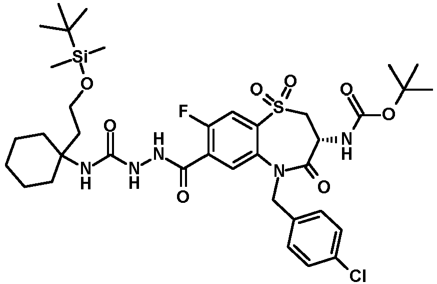

The title compound was prepared from tert-butyl N-[ (3R)-5-[(4-chlorophenyl)methyl]-8-fluoro-7-(hydrazinecarbonyl)-1,1,4-trioxo-2,3-dihydro-1λ⁶,5-benzothiazepin-3-yl]carbamate (20 mg, 0.038 mmol) in analogy to general procedure 15 and was obtained as light yellow solid (29.6 mg, 96% yield). MS (ESI): 808.6 [M-H]⁺.

### Step c) tert-butyl N-[(3R)-7-[5-[[1-[2-[tert-butyl(dimethyl)silyl]oxyethyl]cyclohexyl]amino]-1,3,4-oxadiazol-2-yl]-5-[(4-chlorophenyl)methyl]-8-fluoro-1,1, 4-trioxo-2, 3-dihydro-1λ ⁶,5-benzothiazepin-3yl]carbamate

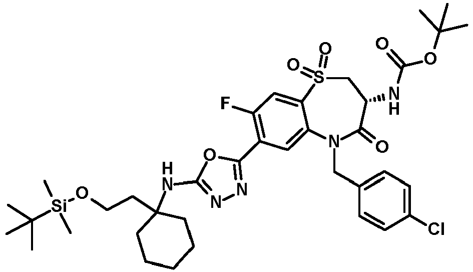

The title compound was prepared from tert-butyl N-[(3R)-7-[[[1-[2-[tert-butyl(dimethyl)silyl]oxyethyl]cyclohexyl]carbamoylamino]carbamoyl]-5-[(4-chlorophenyl)methyl]-8-fluoro-1,1,4-trioxo-2,3-dihydro-1λ⁶,5-benzothiazepin-3-yl]carbamate (29.6 mg, 0.037 mmol) in analogy to general procedure 8b and was obtained as white solid (17.4 mg, 60% yield). MS (ESI) 792.6 [M+H]⁺.

### Step d) (3R)-3-amino-5-[(4-chlorophenyl)methyl]-8-fluoro-7-[5-[[1-(2-hydroxyethyl)cyclohexyl]amino]-1,3,4-oxadiazol-2-yl]-1,1-dioxo-2, 3-dihydro-1λ ⁶,5-benzothiazepin-4-one

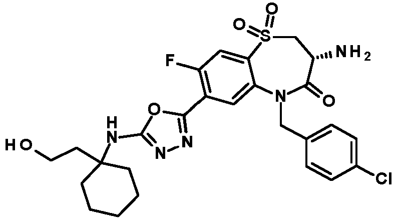

The title compound was prepared from tert-butyl acetate-(3R)-3-amino-7-[5-[[1-[2-[tert-butyl(dimethyl)silyl]oxyethyl]cyclohexyl]amino]-1,3,4-oxadiazol-2-yl]-5-[(4-hlorophenyl)methyl]-8-fluoro-1,1-dioxo-2,3-dihydro-1lambda6,5-benzothiazepin-4-one (17.4 mg, 0.022 mmol) in analogy to general procedure 6d and was obtained as a light yellow solid (12.3 mg, 90% yield), as hydrochloride salt. MS (ESI): 576.3 [M-H]⁻.

### Example 484

### (3R)-3-amino-5-[(4-chlorophenyl)methyl]-8-fluoro-1,1-dioxo-7-[5-[[(3,3,3-trifluoro-2-hydroxy-2-methyl-propyl)amino]-1,3,4-oxadiazol-2-yl]-2,3-dihydro-1λ⁶,5-benzothiazepin-4-one

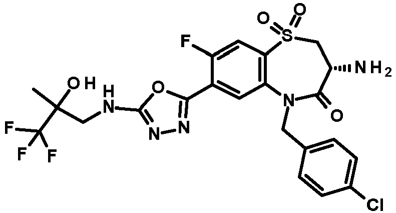

### Step a) 2-[tert-butyl (dimethyl) silyl]oxy-3,3,3-trifluoro-2-methyl-propan-1-amine

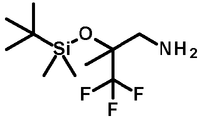

The title compound was prepared from 3-amino-1,1,1-trifluoro-2-methyl-propan-2-ol (CAS 354-68-7) (50mg,0.349 mmol) in analogy to Example 483, step a) and was obtained as light yellow liquid (67.9 mg, 76% yield). MS (ESI): 258.2 [M+H]⁺

### Step b) tert-butyl N-[(3R)-7-[[[2-[tert-butyl(dimethyl)silyl]oxy-3, 3, 3-trifluoro-2-methylpropyl]carbamoylamino]carbamoyl]-5-[(4-chlorophenyl)methyl]-8-fluoro-1,1,4-trioxo-2,3-dihydro-1λ⁶,5-benzothiazepin-3-yl]carbamate

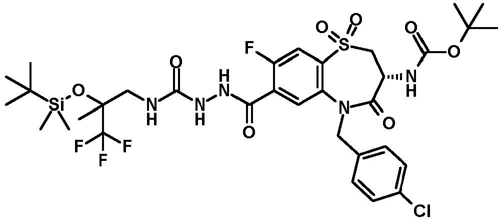

The title compound was prepared from tert-butyl N-[(3R)-5-[(4-chlorophenyl ) methyl]-8-fluoro-7-(hydrazinecarbonyl)-1,1,4-trioxo-3,5-dihydro-2H-1λ⁶-benzothiepin-3-yl]carbamate (20 mg, 0.038 mmol) in analogy to general procedure 15 and was obtained as light yellow solid (33.7 mg, 93% yield). MS (ESI): 808.5 [M-H]⁻.

### Step c) tert-butyl N-[(3R)-7-[5-[[[2-[tert-butyl(dimethyl)silyl]oxy-3,3,3-trifluoro-2-methylpropyl]amino]-1, 3, 4-oxadiazol-2yl]-5-[(4-chlorophenyl)methyl]-8-fluoro-1, 1, 4-trioxo-2, 3-dihydro-1λ⁶,5-benzothiazepin-3-yl]carbamate

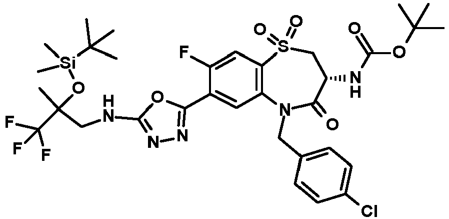

The title compound was prepared from tert-butyl N-[(3R)-7-[[[2-[tert-butyl(dimethyl)silyl]oxy-3,3,3-trifluoro-2-methyl-propyl]carbamoylamino]carbamoyl]-5-[(4-chlorophenyl)methyl]-8-fluoro-1,1,4-trioxo-3,5-dihydro-2H-1λ⁶-benzothiepin-3-yl]carbamate (31.1 mg, 0.033 mmol) in analogy to general procedure **8b** and was obtained as light yellow solid (10.7 mg, 41% yield). MS (ESI): 792.5 [M+H]⁺.

### Step d) (3R)-3-amino-7-[5-[[[2-[tert-butyl(dimethyl)silyl]oxy-3,3,3-trifluoro-2-methylpropyl]amino]-1,3,4-oxadiazol-2-yl]-5-[(4-chlorophenyl)methyl]-8-fluoro-1,1-dioxo-2,3-dihydro-1λ⁶,5-benzothiazepin-4-one

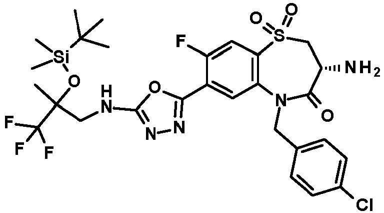

The title compound was prepared from tert-butyl N-[(3R)-7-[5-[[[2-[tert-butyl(dimethyl)silyl]oxy-3,3,3-trifluoro-2-methyl-propyl]amino]-1,3,4-oxadiazol-2-yl]-5-[(4-chlorophenyl)methyl]-8-fluoro-1,1,4-trioxo-3,5-dihydro-2H-1λ⁶-benzothiepin-3-yl]carbamate (10.7 mg, 0.014 mmol) in analogy to general procedure **6d** and was obtained as an off-white solid (10.0 mg, 99% yield). MS (ESI): 690.4 [M-H]⁻.

### Step e) (3R)-3-amino-5-[(4-chlorophenyl)methyl]-8-fluoro-1,1-dioxo-7-[5-[[(3,3,3-trifluoro-2-hydroxy-2-methyl-propyl)amino]-1, 3,4-oxadiazol-2-yl]-2, 3-dihydro-1λ ⁶,5-benzothiazepin-4-one

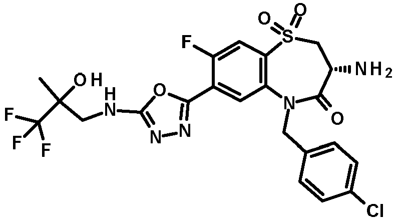

To a colorless solution of (3R)-3-amino-7-[5-[[[2-[tert-butyl(dimethyl)silyl]oxy-3,3,3-trifluoro-2-methyl-propyl]amino]-1,3,4-oxadiazol-2-yl]-5-[(4-chlorophenyl)methyl]-8-fluoro-1,1-dioxo-3,5-dihydro-2H-1λ⁶-benzothiepin-4-one (10 mg, 0.014 mmol) in dry THF (0.144 mL) was slowly added TBAF (1M in THF) (17.34 uL,0.017 mmol, 1.2 eq). The reaction mixture was stirred at RT for 1 hour. Then the mixture was quenched with water and extracted twice with EtOAc. The combined organic layer was washed with 1N HCL and brine, dried over magnesium sulfate, filtered and concentrated under recued pressure to obtain the title compound as brown solid (8 mg, 96% yield). MS (ESI): 578.3 [M+H]⁺.

### Example 485

### (3R)-3-amino-5-[(4-chlorophenyl) methyl]-7-[5-[4,4-difluoro-3-(hydroxymethyl)-1-piperidyl]-1,3,4-oxadiazol-2-yl]-8-fluoro-1,1-dioxo-2,3-dihydro-1λ⁶,5-benzothiazepin-4-one

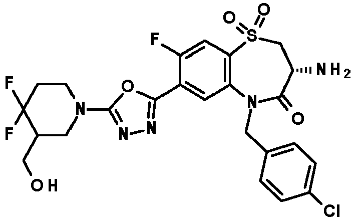

### Step a) tert-butyl-[(4,4-difluoro-3-piperidyl)methoxy]-dimethyl-silane

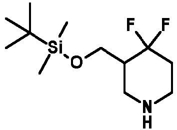

To a suspension of NaH (52.93 mg, 1.32 mmol, 1 eq) in THF (2.83 mL) under Ar atmosphere at 5 °C was added (4,4-difluoro-3-piperidyl)methanol (CAS 1331823-62-1) (200 mg, 1.32 mmol, 1 eq). The reaction was stirred at RT for 1 hour. Then, a solution of TBDMS-Cl (199.43 mg, 1.32 mmol, 1 eq ) in THF (0.472 mL) was added dropwise at 0 °C and the mixture stirred at RT overnight. The reaction mixture was quenched with 10 ml of MeOH and concentrated. Heptane was added and the mixture washed with water, brine, dried over MgSO4, filtered and concentrated to afford the title compound (335 mg, 86% yield) as colorless oil. MS (ESI): 266.2 [M+H]⁺.

### Step b) tert-butyl N-[(3R)-7-[5-[3-[[tert-butyl(dimethyl)silyl]oxymethyl]-4,4-difluoro-1-piperidyl]-1,3,4-oxadiazol-2-yl]-5-[(4-chlorophenyl)methyl]-8-fluoro-1,1,4-trioxo-2, 3-dihydro-1λ ⁶,5-benzothiazepin-3-yl]carbamate

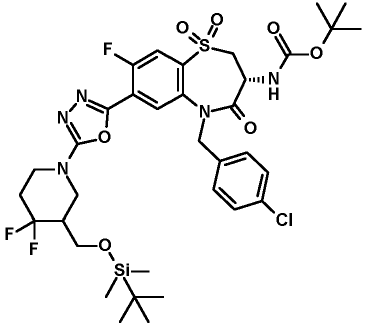

The title compound was prepared from tert-butyl N-[(3R)-5-[(4-chlorophenyl)methyl]-8-fluoro-1,1,4-trioxo-7-(2-oxo-3H-1,3,4-oxadiazol-5-yl)-2,3-dihydro-1λ⁶,5-benzothiazepin-3-yl]carbamate (30 mg, 0.054 mmol) in analogy to Example 455, step a) and was obtained as white solid (30 mg, 69% yield). MS (ESI) 800.5 [M+H]⁺.

### Step c) (3R)-3-amino-5-[(4-chlorophenyl) methyl]-7-[5-[4,4-difluoro-3-(hydroxymethyl)-1-piperidyl]-1,3,4-oxadiazol-2-yl]-8-fluoro-1,1-dioxo-2,3-dihydro-1λ ⁶,5-benzothiazepin-4-one

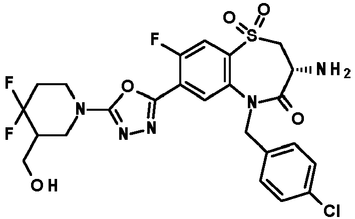

The title compound was prepared from tert-butyl N-[(3R)-7-[5-[3-[[tert-butyl(dimethyl)silyl]oxymethyl]-4,4-difluoro-1-piperidyl]-1,3,4-oxadiazol-2-yl]-5-[(4-chlorophenyl)methyl]-8-fluoro-1,1,4-trioxo-2,3-dihydro-1λ⁶,5-benzothiazepin-3-yl]carbamate (30 mg, 0.037 mmol) in analogy to general procedure 6d and was obtained as white solid (15 mg, 68% yield). MS (ESI): 586.2 [M+H]⁺.

### Example 486

### 2-methyl-2-[[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]amino]propanenitrile

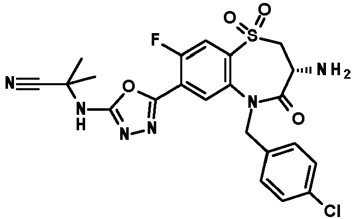

### Step a) tert-butyl N-[(3R)-5-[(4-chlorophenyl)methyl]-7-[[(1-cyano-1-methylethyl)carbamoylamino]carbamoyl]-8-fluoro-4-oxo-2,3-dihydro-1,5-benzothiazepin-3-yl]carbamate

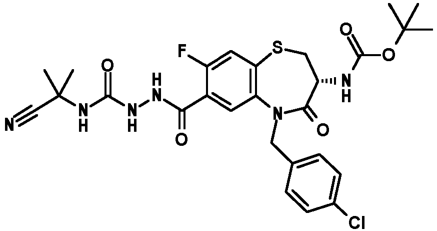

Tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (100 mg, 0.202 mmol, Example 63, step a) was stirred in THF (1 mL) at RT. 2-isocyanato-2-methyl-propanenitrile (CAS 52161-43-0) (22.25 mg, 0.202 mmol) was added as a solution in THF (1 mL) via syringe and stirred at RT for 15 min. Then, a solution of 2-isocyanato-2-methyl-propanenitrile (4.45 mg, 0.040 mmol) in THF (0.5 ml) was added and stirring was continued for 1 hour. The reaction was concentrated under reduced pressure and the crude material purified by column chromatography on silica gel (0-100% EtOAc in heptane) to afford the title compound (110 mg, 84% yield) as white solid. MS (ESI): 605.3 [M+H]⁺.

### Step b) tert-butyl N-[(3R)-5-[(4-chlorophenyl)methyl]-7-[5-[(1-cyano-1-methyl-ethyl)amino]-1,3,4-oxadiazol-2-yl]-8-fluoro-4-oxo-2,3-dihydro-1,5-benzothiazepin-3-yl]carbamate

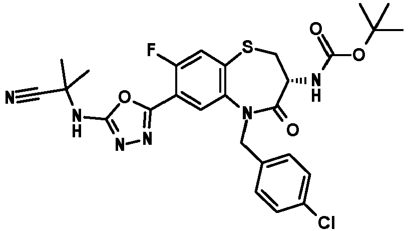

The title compound was prepared from tert-butyl N-[(3R)-5-[(4-chlorophenyl)methyl]-7-[[(1-cyano-1-methyl-ethyl)carbamoylamino]carbamoyl]-8-fluoro-4-oxo-2,3-dihydro-1,5-benzothiazepin-3-yl]carbamate (110 mg, 0.182 mmol) in analogy to general procedure 8a and was obtained as colorless oil (30.5 mg, 27% yield). MS (ESI): 587.3[M+H]⁺.

### Step c) tert-butyl N-[(3R)-5-[(4-chlorophenyl)methyl]-7-[5-[(1-cyano-1-methyl-ethyl)amino]-1,3, 4-oxadiazol-2-yl]-8-fluoro-1,1, 4-trioxo-2, 3-dihydro-1λ ⁶,5-benzothiazepin-3-yl]carbamate

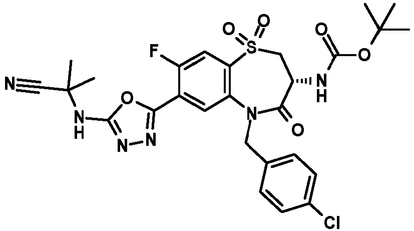

The title compound was prepared from tert-butyl N-[(3R)-5-[(4-chlorophenyl)methyl]-7-[5-[(1-cyano-1-methyl-ethyl)amino]-1,3,4-oxadiazol-2-yl]-8-fluoro-4-oxo-2,3-dihydro-1,5-benzothiazepin-3-yl]carbamate (30.5 mg, 0.052 mmol) in analogy to general procedure 5 and was obtained as white solid (21 mg, 65% yield). MS (ESI): 563.2 [M+H-isobutene]⁺.

### Step d) 2-methyl-2-[[5-[(3R)-3-amino-5-[(4-chlorophenyl)methyl]-8-fluoro-1,1,4-trioxo-2, 3-dihydro-1λ⁶,5-benzothiazepin-7-yl]-1,3, 4-oxadiazol-2-yl]amino]propanenitrile

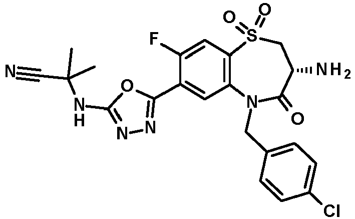

The title compound was prepared from tert-butyl N-[(3R)-5-[(4-chlorophenyl)methyl]-7-[5-[(1-cyano-1-methyl-ethyl)amino]-1,3,4-oxadiazol-2-yl]-8-fluoro-1,1,4-trioxo-2,3-dihydro-1λ⁶,5-benzothiazepin-3-yl]carbamate (21 mg, 0.034 mmol) in analogy to general procedure 6d and was obtained as white solid (18.2 mg, 97% yield), as hydrochloride salt. MS (ESI): 519.3 [M+H]⁺.

### Example 487

### (3R)-3-amino-5-[(4-chlorophenyl)methyl]-8-fluoro-1,1-dioxo-7-[5-(2,2,2-trifluoroethylamino)-1,3,4-oxadiazol-2-yl]-2,3-dihydro-1λ⁶,5-benzothiazepin-4-one

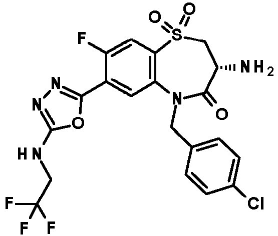

### Step a) tert-butyl N-[(3R)-5-[(4-chlorophenyl)methyl]-8-fluoro-4-oxo-7-[5-(2,2,2-trifluoroethylamino)-1,3, 4-oxadiazol-2-yl]-2,3-dihydro-1, 5-benzothiazepin-3-yl]carbamate

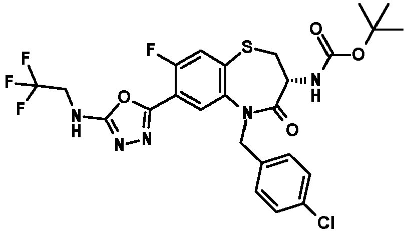

To a solution of tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (Example 63, step a)) (50 mg, 0.098 mmol, 1 eq) in THF (0.653 mL) was added NEt₃ (29.75 mg, 40.97 uL, 0.294 mmol, 3 eq) and 1,1,1-trifluoro-2-isocyanato-ethane (CAS 371-92-6) (14.7 mg, 10.6 uL, 0.118 mmol, 1.2 eq). The solution was stirred at RT for 2 hours. To this solution was added p-toluenesulfonyl chloride (56.04 mg, 0.294 mmol, 3 eq) in one portion and stirred for 2 h. The reaction mixture was concentrated and the remaining crude purified by column chromatography on silica gel (0-50% EtOAc in heptane) to afford the title compound (41 mg, 63% yield) as white solid. MS (ESI): 546.1 [M+H-isobutene]⁺.

### Step b) tert-butyl N-[(3R)-5-[(4-chlorophenyl)methyl]-8-fluoro-1, 1, 4-trioxo-7-[5-(2, 2,2-trifluoroethylamino)-1,3, 4-oxadiazol-2-yl]-2,3-dihydro-1λ ⁶,5-benzothiazepin-3-yl]carbamate

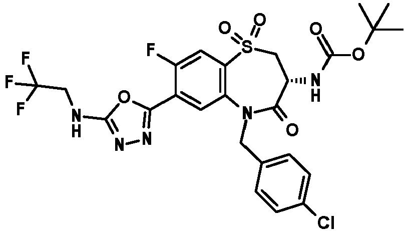

The title compound was prepared from tert-butyl N-[(3R)-5-[(4-chlorophenyl)methyl]-8-fluoro-4-oxo-7-[5-(2,2,2-trifluoroethylamino)-1,3,4-oxadiazol-2-yl]-2,3-dihydro-1,5-benzothiazepin-3-yl]carbamate (38 mg, 0.057 mmol) in analogy to general procedure 5 and was obtained as a white solid (26 mg, 72% yield). MS (ESI): 578.1 [M+H-isobutene]⁺.

### Step c) (3R)-3-amino-5-[(4-chlorophenyl)methyl]-8-fluoro-1,1-dioxo-7-[5-(2,2,2-trifluoroethylamino)-1,3, 4-oxadiazol-2-yl]-2,3-dihydro-1λ ⁶,5-benzothiazepin-4-one

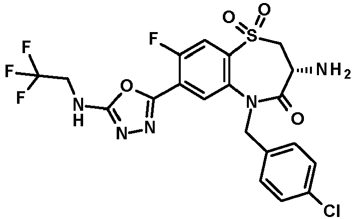

The title compound was prepared from tert-butyl N-[(3R)-5-[(4-chlorophenyl)methyl]-8-fluoro-1,1,4-trioxo-7-[5-(2,2,2-trifluoroethylamino)-1,3,4-oxadiazol-2-yl]-2,3-dihydro-1λ⁶,5-benzothiazepin-3-yl]carbamate (25 mg, 0.039 mmol) in analogy to general procedure **6d** and was obtained as a white solid (21 mg, 90% yield), as hydrochloride salt. MS (ESI): 534.1 [M+H]⁺.

Example 488 of the following table was prepared in analogy to Example 487 using the appropriate isocyanate building block.

| **Ex.** | **Structure** | **Systematic Name** | **Building Block** | **MS, ESI: m/z** |
|---|---|---|---|---|
| 488 | 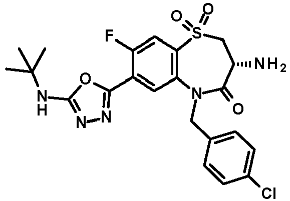 | (3R)-3-amino-7-[5-(tert-butylamino)-1,3,4-oxadiazol-2-yl]-5-[(4-chlorophenyl)methyl]-8-fluoro-1,1-dioxo-2,3-dihydro-1λ⁶,5-benzothiazepin-4-one | 2-Isocyanato -2-methylpropane | 508.3 [M+H]⁺ |
| | | | (CAS 1609-86-5) | |
| | | (*) | | |

| | | | | |
|---|---|---|---|---|
| * as hydrochloride salt | | | | |

### Example 489

### (3R)-3-amino-5-[(4-chlorophenyl)methyl]-7-[5-[(5,5-difluoro-3-piperidyl)amino]-1,3,4-oxadiazol-2-yl]-8-fluoro-1,1-dioxo-2,3-dihydro-1λ⁶,5-benzothiazepin-4-one

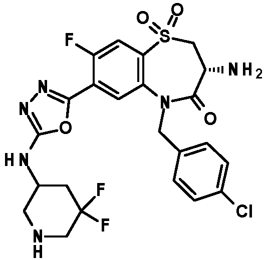

### Step a) 9H-fluoren-9-ylmethyl 5-(tert-butoxycarbonylamino)-3,3-difluoro-piperidine-1-carboxylate

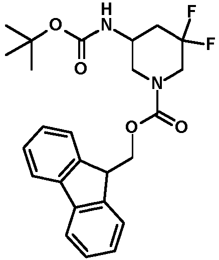

To a solution of tert-butyl N-(5,5-difluoro-3-piperidyl)carbamate (500 mg, 2.01 mmol, 1 eq) in THF (5 ml) at 0 °C was added DIPEA (259 mg, 351 uL, 2.01 mmol, 1 eq) and 9H-fluoren-9-ylmethyl carbonochloridate (520 mg, 2.01 mmol, 1 eq). The reaction mixture was allowed to warm to RT and was overnight. The reaction mixture was partitioned between EtOAc and 0.5M NaOH. The layers were separated and the organic layer washed with 1M HCl and brine, dried over anhydrous sodium sulfate and concentrated in vacuo. The remaining crude material was purified by column chromatography on silica gel (0-50% EtOAc in heptane) to afford the title compound (806 mg, 74% yield) as a white solid. MS (ESI): 359.2 ([M-isobutene-CO₂+H]⁺.

### Step b) 9H-fluoren-9-ylmethyl 5-amino-3,3-difluoro-piperidine-1-carboxylate

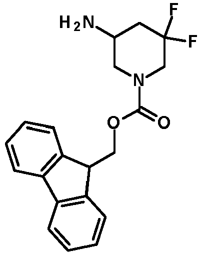

The title compound was prepared from 9H-fluoren-9-ylmethyl 5-(tert-butoxycarbonylamino)-3,3-difluoro-piperidine-1-carboxylate (672.6 mg, 1.47 mmol, 1 eq) in analogy to general procedure **6a** and was obtained as a white solid (814 mg, 100%), as hydrochloride salt. MS (ESI): 359.4 [M+H]⁺.

### Step c) 9H-fluoren-9-ylmethyl 3, 3-difluoro-5-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoylamino]piperidine-1-carboxylate

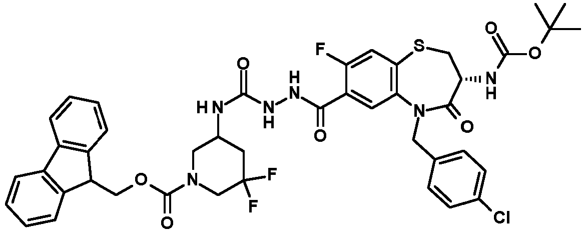

The title compound was prepared from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (218 mg, 0.354 mmol, Example 63, step a)) and 9H-fluoren-9-ylmethyl 5-amino-3,3-difluoro-piperidine-1-carboxylate (250 mg, 0.443 mmol) in analogy to general procedure 15 and was obtained as a white solid (194 mg, 38% yield). MS (ESI): 877.2 [M-H]⁻.

### Step c) 9H-fluoren-9-ylmethyl 3, 3-difluoro-5-[[5-[(3R)-3-(tert-butoxycarbonylamino)-5-[(4-chlorophenyl)methyl]-8-fluoro-4-oxo-2,3-dihydro-1,5-benzothiazepin-7-yl]-1,3, 4-oxadiazol-2-yl]amino]piperidine-1-carboxylate

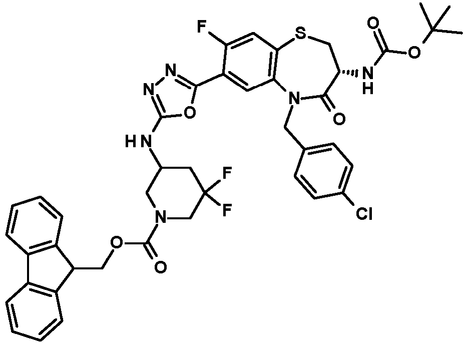

The title compound was prepared from 9H-fluoren-9-ylmethyl 3,3-difluoro-5-[[[(3R)-3-(tert-butoxycarbonylamino)-5-[(4-chlorophenyl)methyl]-8-fluoro-4-oxo-2,3-dihydro-1,5-benzothiazepine-7-carbonyl]amino]carbamoylamino]piperidine-1-carboxylate (244 mg, 0.208 mmol) in analogy to general procedure **8b** and was obtained as a white solid (156 mg, 78% yield). MS (ESI) 861.4 [M+H]⁺.

### Step d) 9H-fluoren-9-ylmethyl 3,3-difluoro-5-[[5-[(3R)-3-(tert-butoxycarbonylamino)-5-[(4-chlorophenyl)methyl]-8-fluoro-1,1, 4-trioxo-2, 3-dihydro-1λ ⁶,5-benzothiazepin-7-yl]-1,3, 4-oxadiazol-2-yl]amino]piperidine-1-carboxylate

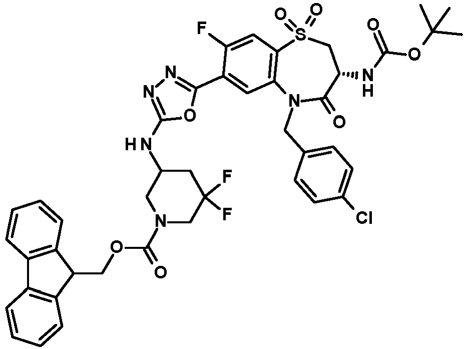

The title compound was prepared from 9H-fluoren-9-ylmethyl 3,3-difluoro-5-[[5-[(3R)-3-(tert-butoxycarbonylamino)-5-[(4-chlorophenyl)methyl]-8-fluoro-4-oxo-2,3-dihydro-1,5-benzothiazepin-7-yl]-1,3,4-oxadiazol-2-yl]amino]piperidine-1-carboxylate (156 mg, 0.163 mmol) in analogy to general procedure **5** and was obtained as a white solid (127 mg, 83% yield). MS (ESI): 893.4 [M+H]⁺.

### Step e) tert-butyl N-[(3R)-5-[(4-chlorophenyl)methyl]-7-[5-[(5, 5-difluoro-3-piperidyl)amino]-1,3, 4-oxadiazol-2-yl]-8-fluoro-1,1, 4-trioxo-2, 3-dihydro-1λ ⁶,5-benzothiazepin-3-yl]carbamate

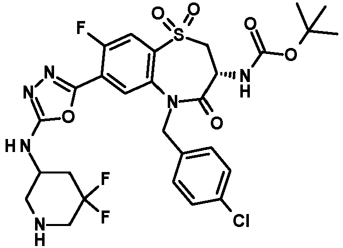

To a solution of 9H-fluoren-9-ylmethyl 3,3-difluoro-5-[[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,3,4-oxadiazol-2-yl]amino]piperidine-1-carboxylate (127 mg, 0.135 mmol, 1 eq) in DCM (1 mL) was added 4-methylpiperidine (107.15 mg, 127.86 uL, 1.08 mmol, 8 eq) at RT and stirred for 2 h. The reaction mixture was partitioned between EtOAc and sat. NaHCO₃. The layers were separated and the aqueous layer extracted twice with EtOAc. The combined organic layers were washed with brine, dried over anhydrous sodium sulfate and concentrated in vacuo. The remaining residue was purified by column chromatography on silica gel (0-5% MeOH in DCM) to give the title compound (34.5 mg, 36% yield) as a white solid. MS (ESI) 671.4 [M+H]⁺.

### Step f) (3R)-3-amino-5-[(4-chlorophenyl)methyl]-7-[5-[(5,5-difluoro-3-piperidyl)amino]-1,3,4-oxadiazol-2-yl]-8-fluoro-1,1-dioxo-2, 3-dihydro-1λ ⁶,5-benzothiazepin-4-one

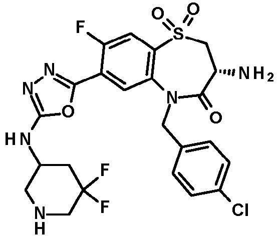

The title compound was prepared from tert-butyl N-[(3R)-5-[(4-chlorophenyl)methyl]-7-[5-[(5,5-difluoro-3-piperidyl)amino]-1,3,4-oxadiazol-2-yl]-8-fluoro-1,1,4-trioxo-2,3-dihydro-1λ⁶,5-benzothiazepin-3-yl]carbamate (10 mg, 0.015 mmol) in analogy to general procedure 6d and was obtained as a white solid (7.6 mg, 78% yield), as hydrochloride salt. MS (ESI): 569.2 [M-H]⁻.

### Example 490

### methyl 3,3-difluoro-5-[[5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-yl]amino]piperidine-1-carboxylate

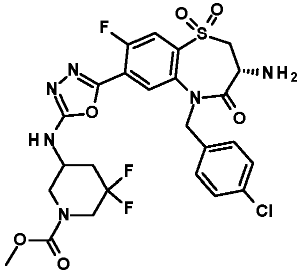

### Step a) methyl 3, 3-difluoro-5-[[5-[(3R)-3-(tert-butoxycarbonylamino)-5-[(4-chlorophenyl)methyl]-8-fluoro-1,1, 4-trioxo-2, 3-dihydro-1λ ⁶,5-benzothiazepin-7-yl]-1,3, 4-oxadiazol-2-yl]amino]piperidine-1-carboxylate

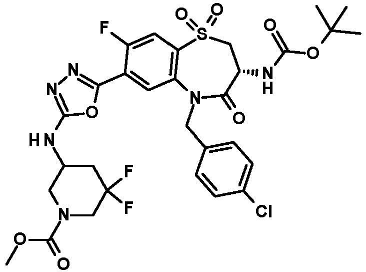

To a solution of tert-butyl N-[(3R)-5-[(4-chlorophenyl)methyl]-7-[5-[(5,5-difluoro-3-piperidyl)amino]-1,3,4-oxadiazol-2-yl]-8-fluoro-1,1,4-trioxo-2,3-dihydro-1λ⁶,5-benzothiazepin-3-yl]carbamate (Example 489, step e) (22.5 mg, 0.032 mmol, 1 eq) in DCM (0.652 mL) was added DIPEA (10.29 mg, 13.91 uL, 0.080 mmol, 2.5 eq) and methyl chloroformate (3.31 mg, 2.71 uL, 0.035 mmol, 1.1 eq) at RT and the reaction mixture was stirred for 3 h. The reaction was concentrated and purified by column chromatography on silica gel (0-3% MeOH in DCM) to afford the title compound (15.7 mg, 67% yield) as a white solid. MS (ESI) 729.3 [M+H]⁺.

### Step b) methyl 3,3-difluoro-5-[[5-[(3R)-3-amino-5-[(4-chlorophenyl)methyl]-8-fluoro-1,1,4-trioxo-2,3-dihydro-1λ ⁶,5-benzothiazepin-7-yl]-1,3, 4-oxadiazol-2-yl]amino]piperidine-1-carboxylate

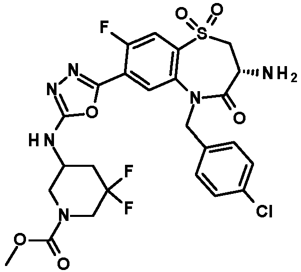

The title compound was prepared from methyl 3,3-difluoro-5-[[5-[(3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]-1,3,4-oxadiazol-2-yl]amino]piperidine-1-carboxylate (15.7 mg, 0.022 mmol, 1eq) in analogy to general procedure **6d** and was obtained as a white solid (8.4 mg, 58% yield), as hydrochloride salt. MS (ESI): 630.0 [M+H]⁺.

### Example 491

### (3R)-3-amino-5-[(4-chlorophenyl)methyl]-7-[5-(3,3-dimethylmorpholin-4-yl)-1,3,4-oxadiazol-2-yl]-8-fluoro-1,1-dioxo-2,3-dihydro-1λ⁶,5-benzothiazepin-4-one

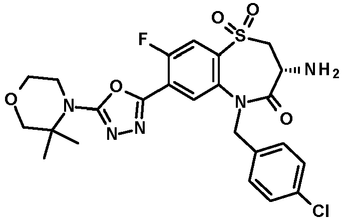

### Step a) tert-butyl N-[(3R)-7-(5-amino-1,3,4-oxadiazol-2-yl)-5-[(4-chlorophenyl)methyl]-8-fluoro-4-oxo-2,3-dihydro-1,5-benzothiazepin-3-yl[carbamate

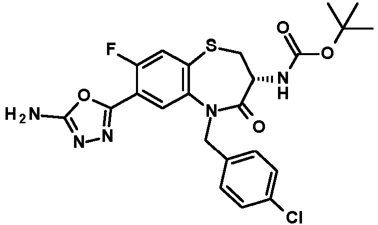

Tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (250 mg, 0.505 mmol, 1 eq) was stirred with sodium bicarbonate (127 mg, 1.52 mmol, 3 eq) and cyanogen bromide (CAS 506-68-3) (160 mg, 1.52 mmol, 3 eq) in 1,4-dioxane (8 mL) and water (5 mL) at RT for 45 min. Water was added and the mixture extracted three times with EtOAc. The combined organic layers were dried over magnesium sulfate, filtered and concentrated to afford the title compound (283 mg, 99% yield) as a light yellow solid. MS (ESI): 520.2 [M+H]⁺.

### Step b) tert-butyl N-[(3R)-7-(5-bromo-1,3,4-oxadiazol-2-yl)-5-[(4-chlorophenyl)methyl]-8-fluoro-4-oxo-2,3-dihydro-1,5-benzothiazepin-3-yl]carbamate

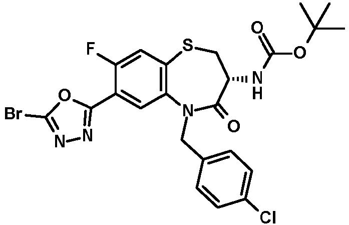

tert-butyl N-[(3R)-7-(5-amino-1,3,4-oxadiazol-2-yl)-5-[(4-chlorophenyl)methyl]-8-fluoro-4-oxo-2,3-dihydro-1,5-benzothiazepin-3-yl]carbamate (282 mg, 0.542 mmol, 1 eq) was dissolved in acetonitrile (2.5 mL) and CuBr₂ (127.19 mg, 0.569 mmol, 1.05 eq) was added at RT. To the resulting green mixture was added isopentyl nitrite (CAS 110-46-3) (127.07 mg, 146.05 mmol, 2 eq) in one portion and the reaction mixture was stirred at RT overnight. The reaction was concentrated and the remaining crude directly purified by column chromatography on silica gel (0-80% EtOAc in heptane) to give the title compound (60 mg, 19% yield) as a colorless oil. MS (ESI): 529.5 [M+H-isobutene]⁺.

### Step c) tert-butyl N-[(3R)-7-(5-bromo-1,3,4-oxadiazol-2-yl)-5-[(4-chlorophenyl)methyl]-8-fluoro-1,1, 4-trioxo-2, 3-dihydro-1λ ⁶,5-benzothiazepin-3-yl]carbamate

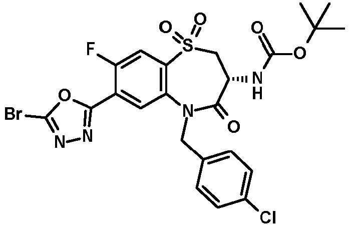

The title compound was prepared from tert-butyl N-[(3R)-7-(5-bromo-1,3,4-oxadiazol-2-yl)-5-[(4-chlorophenyl)methyl]-8-fluoro-4-oxo-2,3-dihydro-1,5-benzothiazepin-3-yl]carbamate (30 mg, 0.051 mmol) in analogy to general procedure **5** and was obtained as a white solid (22 mg, 67% yield). MS (ESI): 615.2 [M+H]⁺.

### Step d) tert-butyl N-[(3R)-5-[(4-chlorophenyl)methyl]-7-[5-(3,3-dimethylmorpholin-4-yl)-1,3,4-oxadiazol-2-yl]-8-fluoro-1,1,4-trioxo-2, 3-dihydro-1λ ⁶,5-benzothiazepin-3-yl]carbamate

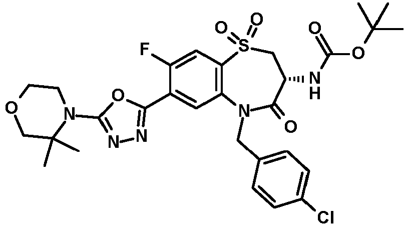

Tert-butyl N-[(3R)-7-(5-bromo-1,3,4-oxadiazol-2-yl)-5-[(4-chlorophenyl)methyl]-8-fluoro-1,1,4-trioxo-2,3-dihydro-1λ⁶,5-benzothiazepin-3-yl]carbamate (22 mg, 0.036 mmol, 1 eq) was stirred with 3,3-dimethylmorpholine (4.94 mg, 5.28 uL, 0.043 mmol, 1.2 eq) and sodium carbonate (3.79 mg, 0.036 mmol, 1 eq) in DMF (100 uL) at RT overnight. The crude material was concentrated and the remaining crude directly purified by column chromatography on silica gel (0-100% EtOAc in heptane) to give the title compound (12.5 mg, 44% yield) as light yellow solid. MS (ESI): 650.3 [M+H]⁺.

### Step e) (3R)-3-amino-5-[(4-chlorophenyl)methyl]-7-[5-(3,3-dimethylmorpholin-4-yl)-1,3,4-oxadiazol-2-yl]-8-fluoro-1,1-dioxo-2,3-dihydro-1λ ⁶,5-benzothiazepin-4-one

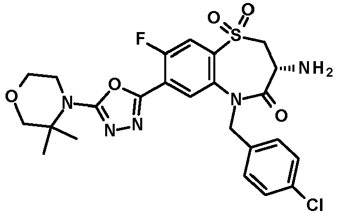

The title compound was prepared from tert-butyl N-[(3R)-5-[(4-chlorophenyl)methyl]-7-[5-(3,3-dimethylmorpholin-4-yl)-1,3,4-oxadiazol-2-yl]-8-fluoro-1,1,4-trioxo-2,3-dihydro-1λ⁶,5-benzothiazepin-3-yl]carbamate (12.5 mg, 0.016 mmol) in analogy to general procedure 6d and was obtained as a white solid ( 6.1 mg, 71% yield). MS (ESI): 550.3 [M+H]⁺.

### Example 492

### (3R)-3-amino-5-[(4-chlorophenyl) methyl]-8-fluoro-1,1-dioxo-7-[5-[[1-(2,2,2-trifluoroethyl)-3-piperidyl]amino]-1,3,4-oxadiazol-2-yl]-2,3-dihydro-1λ⁶,5-benzothiazepin-4-one

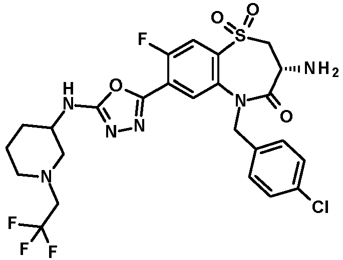

### Step a) tert-butyl N-[(3R)-5-[(4-chlorophenyl) methyl]-8-fluoro-4-oxo-7-[[[1-(2,2,2-trifluoroethyl)-3-piperidyl]carbamoylamino]carbamoyl]-2,3-dihydro-1,5-benzothiazepin-3-yl]carbamate

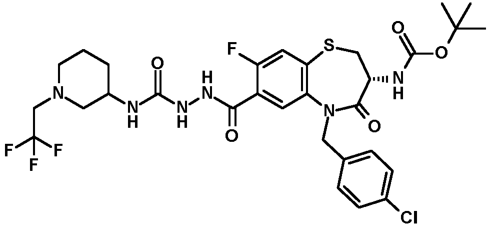

The title compound was prepared from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (80 mg, 0.129 mmol, 1 eq, Example 63, step a) and 1-(2,2,2-trifluoroethyl)piperidin-3-amine dihydrochloride (CAS 1803583-68-7) (51.18 mg, 0.201 mmol, 1.55 eq) in analogy to general procedure 15 and was obtained as light yellow solid (122 mg, 59% yield). MS (ESI): 703.4 [M+H]⁺.

### Step b) tert-butyl N-[(3R)-5-[(4-chlorophenyl)methyl]-8-fluoro-4-oxo-7-[5-[[1-(2,2,2-trifluoroethyl)-3-piperidyl]amino]-1,3,4-oxadiazol-2-yl]-2,3-dihydro-1,5-benzothiazepin-3-yl]carbamate

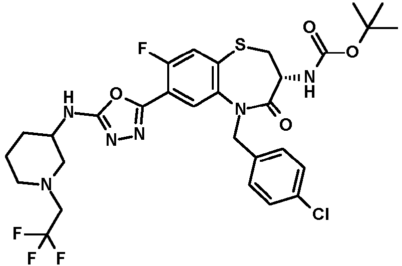

The title compound was prepared from tert-butyl N-[(3R)-5-[(4-chlorophenyl)methyl]-8-fluoro-4-oxo-7-[[[1-(2,2,2-trifluoroethyl)-3-piperidyl]carbamoylamino]carbamoyl]-2,3-dihydro-1,5-benzothiazepin-3-yl]carbamate (122 mg, 0.076 mmol) in analogy to general procedure **8b** and was obtained as light yellow solid (40 mg, 74% yield). MS (ESI): 685.4 [M+H]⁺.

### Step c) tert-butyl N-[(3R)-5-[(4-chlorophenyl)methyl]-8-fluoro-7-[5-[[1-oxido-1-(2,2,2-trifluoroethyl)piperidin-1-ium-3-yl]amino]-1,3,4-oxadiazol-2-yl]-1,1,4-trioxo-2, 3-dihydro-1λ ⁶,5-benzothiazepin-3yl]carbamate

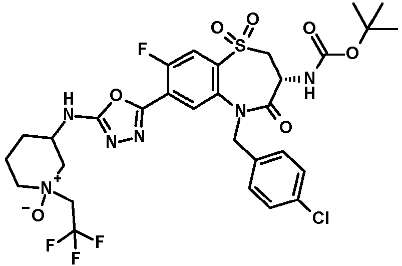

The title compound was prepared from tert-butyl N-[(3R)-5-[(4-chlorophenyl)methyl]-8-fluoro-4-oxo-7-[5-[[1-(2,2,2-trifluoroethyl)-3-piperidyl]amino]-1,3,4-oxadiazol-2-yl]-2,3-dihydro-1,5-benzothiazepin-3-yl]carbamate (40 mg, 0.058 mmol, 1 eq) in analogy to general procedure 5 using *m-CPBA* (39 mg, 3.75 eq) to give an off-white solid (77 mg) containing the title compound. MS (ESI): 733.3 [M+H]⁺.

### Step d) tert-butyl N-[(3R)-5-[(4-chlorophenyl)methyl]-8-fluoro-1,1,4-trioxo-7-[5-[[1-(2,2, 2-trifluoroethyl)-3-piperidyl]amino]-1,3, 4-oxadiazol-2-yl]-2,3-dihydro-1λ ⁶,5-benzothiazepin-3-yl]carbamate

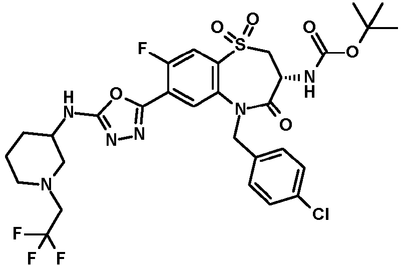

tert-butyl N-[(3R)-5-[(4-chlorophenyl)methyl]-8-fluoro-7-[5-[[1-oxido-1-(2,2,2-trifluoroethyl)piperidin-1-ium-3-yl]amino]-1,3,4-oxadiazol-2-yl]-1,1,4-trioxo-2,3-dihydro-1λ⁶,5-benzothiazepin-3-yl]carbamate (77 mg, 0.105 mmol, 1 eq) was dissolved in 1,2 dichloroethane (2 mL) and phenylboronic acid (14.09 mg, 0.116 mmol, 1.1 eq) was added in one portion and the resulting mixture heated to 85°C for one hour. The reaction was allowed to cool to RT and concentrated under reduced pressure. The crude material was purified by column chromatography on silica gel (100% EtOAc) to afford the title compound (20 mg, 26% yield) as a white solid. MS (ESI): 717.4 [M+H]⁺.

### Step e) (3R)-3-amino-5-[(4-chlorophenyl)methyl]-8-fluoro-1,1-dioxo-7-[5-[[1-(2,2,2-trifluoroethyl)-3-piperidyl]amino]-1,3,4-oxadiazol-2-yl]-2,3-dihydro-1λ ⁶,5-benzothiazepin-4-one

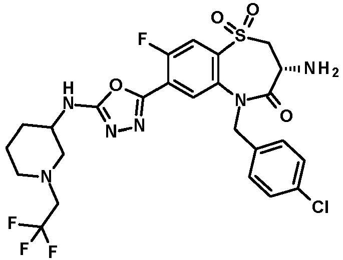

The title compound was prepared from tert-butyl N-[(3R)-5-[(4-chlorophenyl)methyl]-8-fluoro-1,1,4-trioxo-7-[5-[[1-(2,2,2-trifluoroethyl)-3-piperidyl]amino]-1,3,4-oxadiazol-2-yl]-2,3-dihydro-1λ⁶,5-benzothiazepin-3-yl]carbamate (20 mg, 0.028 mmol) in analogy to general procedure 6d and was obtained as white solid (12 mg, 70% yield). MS (ESI): 617.2 [M+H]⁺.

### Example 493

### (3R)-3-amino-5-[(4-chlorophenyl)methyl]-7-[5-[1-(3,3-difluorocyclobutyl)ethylamino]-1,3,4-oxadiazol-2-yl]-8-fluoro-1,1-dioxo-2,3-dihydro-1λ⁶,5-benzothiazepin-4-one

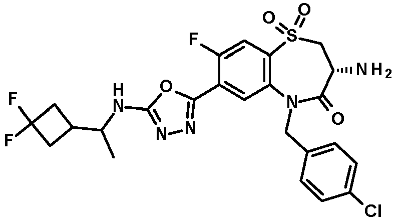

### Step a) tert-butyl N-[(3R)-5-[(4-chlorophenyl)methyl]-7-[[1-(3,3-difluorocyclobutyl)ethylcarbamoylamino]carbamoyl]-8-fluoro-4-oxo-2,3-dihydro-1,5-benzothiazepin-3yl]carbamate

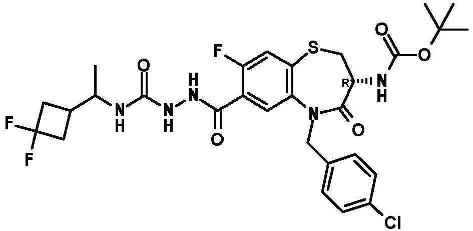

The title compound was prepared from tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (Example 63, step a)), 80 mg, 0.129 mmol) and 1-(3,3-difluorocyclobutyl)ethylamine hydrochloride *(*CAS 1780822-89-0) (34.43 mg, 0.201 mmol) in analogy to general procedure 15 and was obtained as yellow solid (115 mg, 95% yield). MS (ESI): 556.2 [M+H-Boc]⁺.

### Step b) tert-butyl N-[(3R)-5-[(4-chlorophenyl)methyl]-7-[5-[1-(3,3-difluorocyclobutyl)ethylamino]-1,3,4-oxadiazol-2-yl]-8-fluoro-4-oxo-2,3-dihydro-1,5-benzothiazepin-3yl]carbamate

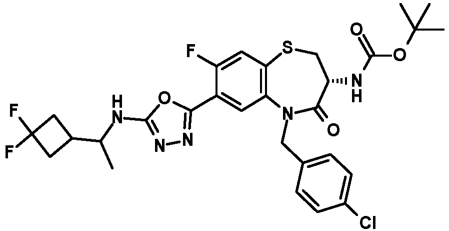

The title compound was prepared from tert-butyl N-[(3R)-5-[(4-chlorophenyl)methyl]-7-[[1-(3,3-difluorocyclobutyl)ethylcarbamoylamino]carbamoyl]-8-fluoro-1,1,4-trioxo-2,3-dihydro-1λ⁶,5-benzothiazepin-3-yl]carbamate (110 mg, 0.117 mmol) in analogy to general procedure **8b** *and was obtained as white solid (41 mg, 53% yield). MS (ESI):* 638.2 [M+H]⁺.

### Step c) tert-butyl N-[(3R)-5-[(4-chlorophenyl)methyl]-7-[5-[1-(3, 3-difluorocyclobutyl)ethylamino]-1,3,4-oxadiazol-2-yl]-8-fluoro-1,1,4-trioxo-2, 3-dihydro-1λ ⁶,5-benzothiazepin-3yl]carbamate

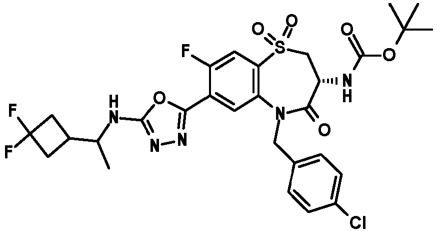

The title compound was prepared from tert-butyl N-[(3R)-5-[(4-chlorophenyl)methyl]-7-[5-[1-(3,3-difluorocyclobutyl)ethylamino]-1,3,4-oxadiazol-2-yl]-8-fluoro-4-oxo-2,3-dihydro-1,5-benzothiazepin-3-yl]carbamate (36 mg, 0.054 mmol) in analogy to general procedure 5 and was obtained as white solid (25 mg, 69% yield). *MS (ESI):* 670.2 [M+H]⁺.

### Step d) (3R)-3-amino-5-[(4-chlorophenyl)methyl]-7-[5-[1-(3,3-difluorocyclobutyl)ethylamino]-1,3,4-oxadiazol-2-yl]-8-fluoro-1,1-dioxo-2, 3-dihydro-1λ ⁶,5-benzothiazepin-4-one

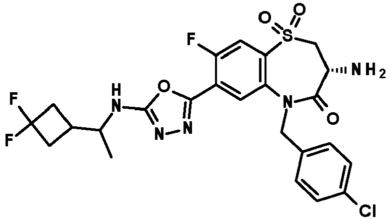

The title compound was prepared from tert-butyl N-[(3R)-5-[(4-chlorophenyl)methyl]-7-[5-[1-(3,3-difluorocyclobutyl)ethylamino]-1,3,4-oxadiazol-2-yl]-8-fluoro-1,1,4-trioxo-2,3-dihydro-1λ⁶,5-benzothiazepin-3-yl]carbamate (20 mg, 0.03 mmol) in analogy to general procedure 6d and was obtained as white solid (18 mg, 99% yield). *MS (ESI):* 570.2 [M+H]⁺.

The examples 494 to 506 of the following table were prepared in analogy to Example 493, using the appropriate amine building block.

| **Ex.** | **Structure** | **Systematic Name** | **Building Block** | **MS, ESI: m/z** |
|---|---|---|---|---|
| 494 | 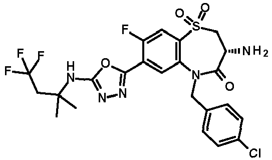 | (3R)-3-amino-5-[(4-chlorophenyl)methyl]-8-fluoro-1,1-dioxo-7-[5-[(3,3,3-trifluoro-1,1-dimethylpropyl)amino]-1,3,4-oxadiazol-2-yl]-2,3-dihydro-1λ⁶,5-benzothiazepin-4-one | 4,4,4-Trifluoro-2-methyl-butan-2-amine hydrochloride | 576.2 [M+H]⁺ |
| | | (*) | (CAS 93339-72-1) | |
| 495 | 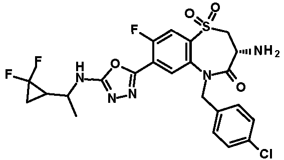 | (3R)-3-amino-5-[(4-chlorophenyl)methyl]-7-[5-[1-(2,2-difluorocyclopropyl)ethylamin o]-1,3,4-oxadiazol-2-yl]-8-fluoro-1,1-dioxo-2,3-dihydro-1λ⁶,5-benzothiazepin-4-one | 1-(2,2-Difluorocycl opropyl)eth ylamine hydrochloride | 556.1 [M+H]⁺ |
| | | | (CAS 1909336-12-4) | |
| | | (*) Diastereomer A | | |
| 496 | 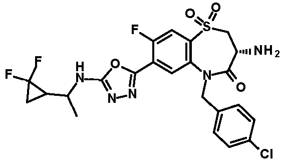 | (3R)-3-amino-5-[(4-chlorophenyl)methyl]-7-[5-[1-(2,2-difluorocyclopropyl)ethylamin o]-1,3,4-oxadiazol-2-yl]-8-fluoro-1,1-dioxo-2,3-dihydro-1λ⁶,5-benzothiazepin-4-one | 1-(2,2-Difluorocycl opropyl)eth ylamine hydrochloride | 556.1 [M+H]⁺ |
| | | | (CAS 1909336-12-4) | |
| | | (*) Diastereomer B | | |
| 497 | 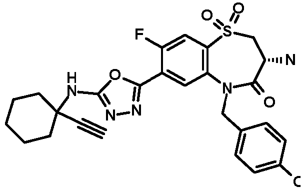 | (3R)-3-amino-5-[(4-chlorophenyl)methyl]-7-[5-[(1-ethynylcyclohexyl)amino]-1,3,4-oxadiazol-2-yl]-8-fluoro-1,1-dioxo-2,3-dihydro-1λ⁶,5-benzothiazepin-4-one | (1-Ethynylcyclo hexyl)amine | 558.3 [M+H]⁺ |
| | | | (CAS 30389-18-5) | |
| | | (*) | | |
| 498 | 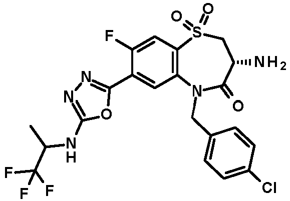 | (3R)-3-amino-5-[(4-chlorophenyl)methyl]-8-fluoro-1,1-dioxo-7-[5-[(2,2,2-trifluoro-1-methylethyl)amino]-1,3,4-oxadiazol-2-yl]-2,3-dihydro-1λ⁶,5-benzothiazepin-4-one | (2,2,2-Trifluoro-1-methylethyl)amine hydrochloride | 548.1 [M+H]⁺ |
| | | | (CAS 2968-32-3) | |
| | | (*) | | |
| 499 | 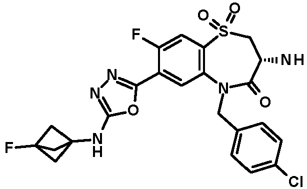 | (3R)-3-amino-5-[(4-chlorophenyl)methyl]-8-fluoro-7-[5-[(3-fluoro-1-bicyclo[1.1.1]pentanyl)amino] -1,3,4-oxadiazol-2-yl]-1,1-dioxo-2,3-dihydro-1λ⁶,5-benzothiazepin-4-one | (3-Fluoro-1-bicyclo[1.1. 1]pentanyl) amine hydrochloride | 536.1 [M+H]⁺ |
| | | | (CAS 1826900-79-1) | |
| | | (*) | | |
| 500 | 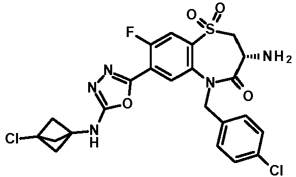 | (3R)-3-amino-7-[5-[(3-chloro-1-bicyclo[1.1.1]pentanyl)amino] -1,3,4-oxadiazol-2-yl]-5-[(4-chlorophenyl)methyl]-8-fluoro-1,1-dioxo-2,3-dihydro-1λ⁶,5-benzothiazepin-4-one | (3-Chloro-1-bicyclo[ 1.1. 1] pentanyl) amine hydrochloride | 552.1 [M+H]⁺ |
| | | | (CAS 1886967-25-4) | |
| | | (*) | | |
| 501 | 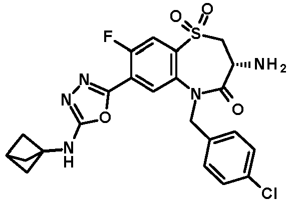 | (3R)-3-amino-7-[5-(1-bicyclo[1.1.1]pentanylamino)-1,3,4-oxadiazol-2-yl]-5-[(4-chlorophenyl)methyl]-8-fluoro-1,1-dioxo-2,3-dihydro-1λ⁶,5-benzothiazepin-4-one | 1-Bicyclo[1.1. 1]pentanyla mine hydrochloride | 518.2 [M+H]⁺ |
| | | (*) | (CAS 22287-35-0) | |
| 502 | 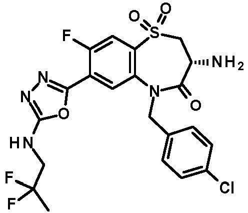 | (3R)-3-amino-5-[(4-chlorophenyl)methyl]-7-[5-(2,2-difluoropropylamino)-1,3,4-oxadiazol-2-yl]-8-fluoro-1,1-dioxo-2,3-dihydro-1λ⁶,5-benzothiazepin-4-one | 2,2-Difluoropro pylamine hydrochloride | 530.2 [M+H]⁺ |
| | | (*) | (CAS 421-00-1) | |
| 503 | 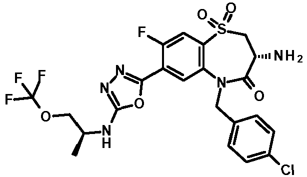 | (3R)-3-amino-5-[(4-chlorophenyl)methyl]-8-fluoro-1,1-dioxo-7-[5-[[(1S)-1-methyl-2-(trifluoromethoxy)ethyl]amin o]-1,3,4-oxadiazol-2-yl]-2,3-dihydro-1λ⁶,5-benzothiazepin-4-one | [(1S)-1-Methyl-2-(trifluorome thoxy)ethyl] amine hydrochloride | 578.2 [M+H]⁺ |
| | | | (CAS 1932194-99-4) | |
| | | (*) | | |
| 504 | 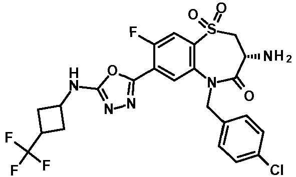 | (3R)-3-amino-5-[(4-chlorophenyl)methyl]-8-fluoro-1,1-dioxo-7-[5-[[3-(trifluoromethyl)cyclobutyl]a mino]-1,3,4-oxadiazol-2-yl]-2,3-dihydro-1λ⁶,5-benzothiazepin-4-one | [3-(Trifluorome thyl)cyclobu tyl]amine | 574.1 [M+H]⁺ |
| | | | (CAS 1803601-06-0) | |
| | | (***) | | |
| 505 | 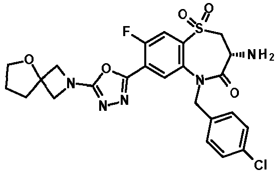 | (3R)-3-amino-5-[(4-chlorophenyl)methyl]-8-fluoro-7-[5-(5-oxa-2-azaspiro[3.4]octan-2-yl)-1,3,4-oxadiazol-2-yl]-1,1-dioxo-2,3-dihydro-1λ⁶,5-benzothiazepin-4-one | 5-Oxa-2-azaspiro[3.4 ]octane;oxalic acid | 548.2 [M+H]⁺ |
| | | | (CAS 1523618-29-2) | |
| | | (*) | | |
| 506 | 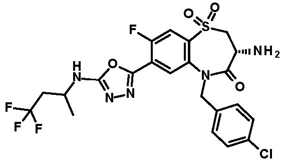 | (3R)-3-amino-5-[(4-chlorophenyl)methyl]-8-fluoro-1,1-dioxo-7-[5-[(3,3,3-trifluoro-1-methylpropyl)amino]-1,3,4-oxadiazol-2-yl]-2,3-dihydro-1λ⁶,5-benzothiazepin-4-one | (3,3,3-Trifluoro-1-methylpropyl)amine hydrochloride | 562.1 [M+H]⁺ |
| | | (***) | (CAS 93339-76-5) | |

| | | | | |
|---|---|---|---|---|
| * as a hydrochloride salt ** as a 1,1,1,3,3,3-hexafluoropropan-2-ol adduct *** as a hydrochloride salt and 1,1,1,3,3,3-hexafluoropropan-2-ol adduct **** as a formic acid salt | | | | |

### Example 507

### (3R)-3-amino-5-[(4-chlorophenyl)methyl]-8-fluoro-7-[5-[(3-methylazetidin-1-yl)methyl]-1,3,4-oxadiazol-2-yl]-2,3-dihydro-1,5-benzothiazepin-4-one

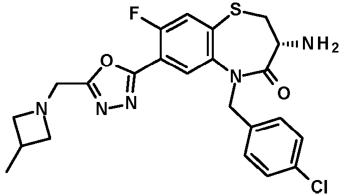

### Step a) tert-butyl N-[(3R)-7-[[(2-bromoacetyl)amino]carbamoyl]-5-[(4-chlorophenyl)methyl]-8-fluoro-4-oxo-2, 3-dihydro-1,5-benzothiazepin-3-yl]carbamate

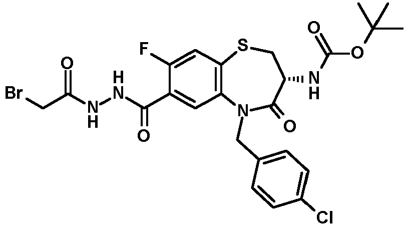

To a solution of tert-butyl N-[(3R)-5-[(4-chlorophenyl)methyl]-8-fluoro-7-(hydrazinecarbonyl)-4-oxo-2,3-dihydro-1,5-benzothiazepin-3-yl]carbamate (Example 63, step a)) (200 mg, 0.392 mmol, 1 eq) in DCM (2 mL) was added NEt₃ ( 39.66 mg, 54.63 uL, 0.392 mmol, 1 eq) and the mixture was cooled down to 0°C. 2-bromoacetyl bromide (126 mg, 54.5 uL, 0.627 mmol, 1.6 eq) was added dropwise. Upon complete addition, the reaction was allowed to warm to RT and was stirred for 1 hour. The reaction mixture was poured on water and extracted three times with DCM. The combined organic layers were washed with brine, dried over magnesium sulfate, filtered and concentrated under reduced pressure. The remaining crude residue was purified by column chromatography on silica gel (0-50% EtOAc in heptane) to afford the title compound (135 mg, 48% yield) as a white solid. MS (ESI): 615.3 [M-H]⁻.

### Step b) tert-butyl N-[(3R)-7-[5-(bromomethyl)-1,3,4-oxadiazol-2-yl]-5-[(4-chlorophenyl)methyl]-8-fluoro-4-oxo-2,3-dihydro-1,5-benzothiazepin-3-yl]carbamate

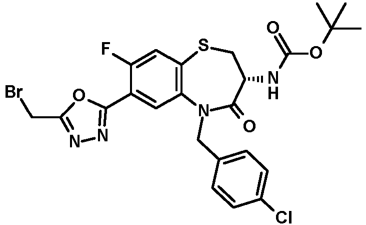

The title compound was prepared from tert-butyl N-[(3R)-7-[5-(bromomethyl)-1,3,4-oxadiazol-2-yl]-5-[(4-chlorophenyl)methyl]-8-fluoro-4-oxo-2,3 -dihydro-1,5-benzothiazepin-3-yl]carbamate (85 mg, 0.138 mmol, 1 eq) in analogy to general procedure **8a** and was obtained as a colorless gum (22.5 mg, 20% yield). MS (ESI): 595.3 [M-H]⁻.

### Step c) tert-butyl N-[(3R)-5-[(4-chlorophenyl)methyl]-8-fluoro-7-[5-[(3-methylazetidin-1-ylmethyl]-1,3,4-oxadiazol-2-yl]-4-oxo-2,3-dihydro-1,5-benzothiazepin-3-yl]carbamate

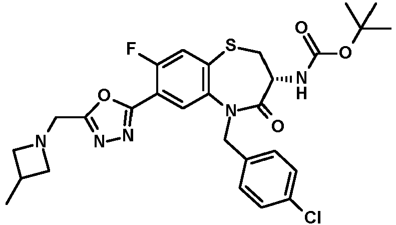

To a solution of tert-butyl N-[(3R)-7-[5-(bromomethyl)-1,3,4-oxadiazol-2-yl]-5-[(4-chlorophenyl)methyl]-8-fluoro-4-oxo-2,3-dihydro-1,5-benzothiazepin-3-yl]carbamate (30 mg, 0.05 mmol, 1 eq) in DCM (0.5 mL) was added DIPEA (16.21 mg, 21.91 uL, 0.125 mmol, 2.5 eq) and 3-methylazetidine hydrochloride (CAS 935669-28-6) (11.88 mg, 0.11 mmol, 2.2 eq). The reaction mixture was stirred at RT overnight. The mixture was poured on water and extracted three times with DCM. The combined organic layers were washed with brine, dried over magnesium sulfate, filtered and concentrated. The crude residue was purified by column chromatography on silica gel (0-100% EtOAc in heptane) to afford the title compound (17.2 mg, 55% yield) as a colorless oil. MS (ESI): 588.3 [M+H]⁺.

### Step d) (3R)-3-amino-5-[(4-chlorophenyl)methyl]-8-fluoro-7-[5-[(3-methylazetidin-1-yl)methyl]-1,3,4-oxadiazol-2-yl]-2,3-dihydro-1,5-benzothiazepin-4-one

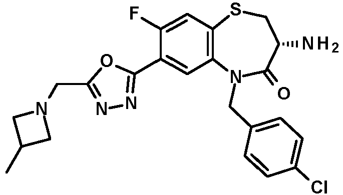

The title compound was prepared from tert-butyl N-[(3R)-5-[(4-chlorophenyl)methyl]-8-fluoro-7-[5-[(3-methylazetidin-1-yl)methyl]-1,3,4-oxadiazol-2-yl]-4-oxo-2,3-dihydro-1,5-benzothiazepin-3-yl]carbamate (17.2 mg, 0.028 mmol) in analogy to general procedure **6d** and was obtained as off-white solid (13.3 mg, 66% yield), as hydrochloride salt and 1, 1, 1,3,3,3-hexafluoropropan-2-ol adduct. MS (ESI): 244.7 [M+H]²⁺.

### Example 508

### (3R)-3-amino-7-(5-tert-butyl-1,3,4-thiadiazol-2-yl)-5-[(4-chlorophenyl)methyl]-8-fluoro-1,1-dioxo-2,3-dihydro-1λ⁶,5-benzothiazepin-4-one

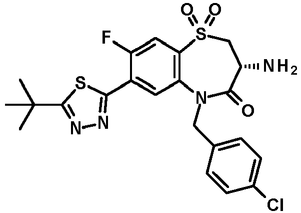

### Step a) [(3R)-3-(tert-butoxycarbonylamino)-5-[(4-chlorophenyl)methyl]-8-fluoro-1,1, 4-trioxo-2,3-dihydro-1λ ⁶,5-benzothiazepin-7-yl]boronic acid

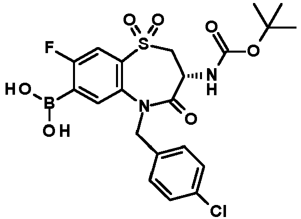

N-[(3R)-7-bromo-5-(4-chlorobenzyl)-8-fluoro-1,1,4-triketo-2,3-dihydro-1λ6,5-benzothiazepin-3-yl]carbamic acid tert-butyl ester (Example 37, step a)) (66 mg, 0.120 mmol, 1 eq) was stirred with bis(pinacolato)diboron (67.31 mg, 0.265 mmol, 2.2 eq), 1,1'-bis(diphenylphosphino)ferrocene-palladium(II)dichloride dichloromethane adduct (11.31 mg, 0.014 mmol, 0.115 eq) and potassium acetate (35.47 mg, 0.361 mmol, 3 eq) in dry 1,4-dioxane (1.2 mL) at 100°C for 1h in a closed vial. Water was added and the mixture was extracted three times with EtOAc. The combined organic extracts were dried over magnesium sulfate, filtered and evaporated to afford the crude title compound (143 mg) as a light brown oil. MS (ESI): 457.1 [M-isobutene+H]⁺.

### Step b) tert-butyl N-[(3R)-7-(5-tert-butyl-1,3,4-thiadiazol-2-yl)-5-[(4-chlorophenyl)methyl]-8-fluoro-1,1,4-trioxo-2,3-dihydro-1λ ⁶,5-benzothiazepin-3-yl]carbamate

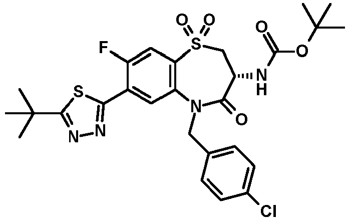

[(3R)-3-(tert-butoxycarbonylamino)-5-(4-chlorobenzyl)-8-fluoro-1,1,4-triketo-2,3-dihydro-1λ⁶,5-benzothiazepin-7-yl]boronic acid (61 mg, 0.119 mmol, 1 eq) was stirred with 2-bromo-5-tert-butyl-1,3,4-thiadiazole (26.31 mg, 0.119 mmol, 1 eq), 1,1'-bis(diphenylphosphino)ferrocene-palladium(II)dichloride dichloromethane adduct (14.57 mg, 0.018 mmol, 0.15 eq) and Na₂CO₃ (37.83 mg, 0.357 mmol, 3 eq) in a mixture of 1,4-dioxane (2.5 mL) and water (0.5 mL) at 100°C for 2h in a closed vial. The crude material was purified by column chromatography on silica gel (heptane:EtOAc 1:0 to 0:1) affording the title compound (6.4 mg, 9%) as a white solid. MS (ESI): 609.3 [M+H]⁺.

### Step c) (3R)-3-amino-7-(5-tert-butyl-1,3,4-thiadiazol-2-yl)-5-[(4-chlorophenyl)methyl]-8-fluoro-1,1-dioxo-2, 3-dihydro-1λ ⁶,5-benzothiazepin-4-one

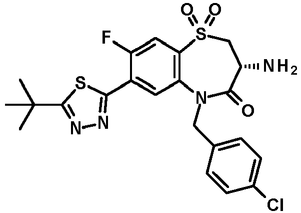

The title compound was prepared in analogy to method **6c** from N-[(3R)-7-(5-tert-butyl-1,3,4-thiadiazol-2-yl)-5-(4-chlorobenzyl)-8-fluoro-1,1,4-triketo-2,3-dihydro-1λ⁶,5-benzothiazepin-3-yl]carbamic acid tert-butyl ester (6.4 mg, 0.011 mmol) with additional 4 drops of HCl (4M in dioxane) and was obtained as a white solid as a formic acid salt (3.8 mg, 65%). MS (ESI): 509.2 [M+H]⁺.

### Example 509

### (3R)-3-amino-5-[(4-chlorophenyl)methyl]-7-(5-ethyltetrazol-2-yl)-8-fluoro-1,1-dioxo-2,3-dihydro-1λ⁶,5-benzothiazepin-4-one

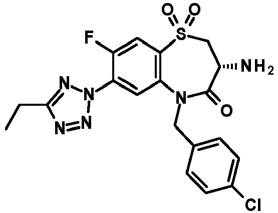

### Step a) ethyl (2E)-2-chloro-2-[(2,4-difluorophenyl)hydrazono]acetate

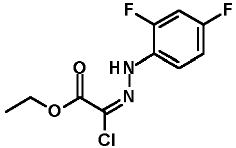

To a solution of 2,4-difluoroaniline (40 g, 309 mmol) in HCl (6N, 309.81 mL, 1859 mmol, 6 eq) was added NaNO₂ (23.51 g, 340 mmol, 1.1 eq) while the temperature was kept at 0°C. After stirring at this temperature for 30 min, the mixture was added to a stirred solution of NaOAc (33.03 g, 402.76 mmol, 1.3 eq) and ethyl 2-chloroacetoacetate (56.09 g, 340.79 mmol, 1.1 eq) in EtOH (400 mL) while the temperature was kept below 5°C and then the reaction mixture was stirred at 0°C for another 3 h. The reaction mixture was filtered and the filter cake was washed with EtOH and water. The solid material was recrystallized from EtOH, affording the desired product (48 g, 182 mmol, 59% yield) as light yellow solid.

### Step b) ethyl (2E)-2-amino-2-[(2,4-difluorophenyl)hydrazono]acetate

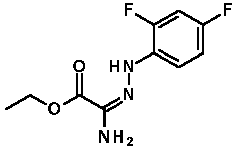

To a solution of ethyl (2E)-2-chloro-2-[(2,4-difluorophenyl)hydrazono]acetate (48.0 g, 182 mmol, 1 eq) in THF (182 mL) was added NH₃•H₂O (136.0 mL, 182 mmol, 1 eq) dropwise and the mixture was stirred at 20°C for 12 h. The reaction mixture was diluted with petroleum ether and EtOAc. After 10 min of stirring the two layers were separated and the aqueous layer was extracted with EtOAc three times. The combined organic layers were washed with brine, dried over Na₂SO₄ and concentrated in vacuum to afford the title compound (43.3 g, 178 mmol, 97% yield) as light yellow solid.

### Step c) ethyl 2-(2,4-difluorophenyl)tetrazole-5-carboxylate

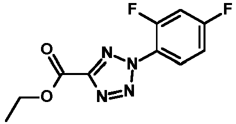

Acetic acid (42.47 g, 707.21 mmol, 4 eq) was added to a stirred solution of ethyl (2E)-2-amino-2-[(2,4-difluorophenyl)hydrazono]acetate (43.0 g, 176 mmol, 1 eq) in THF (400 mL) and the mixture was heated to 85°C. Then NaNO₂ (14.64 g, 212 mmol, 1.2 eq) in water (40 mL) was added to the reaction mixture dropwise over 30 min. The reaction mixture was stirred at 85°C for 11h 30min. The mixture was concentrated in vacuum to remove the solvent and the remaining residue was re-dissolved in EtOAc. The EtOAc solution was washed with saturated NaHCO₃ solution, water and brine, dried over Na₂SO₄ and concentrated in vacuum to afford the title compound (29.3 g, 115 mmol, 65% yield) as light yellow solid.

### Step d) 1-[2-(2,4-difluorophenyl)tetrazol-5-yl]ethanone

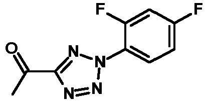

MeMgBr (13.11 mL, 39.3 mmol, 2 eq) and NEt₃ (9933 mg, 98.35 mmol, 5 eq) were added to a stirred solution of ethyl 2-(2,4-difluorophenyl)tetrazole-5-carboxylate (5000 mg, 19.6 mmol, 1 eq) in toluene (20 mL) at -10°C for 3 h. To the mixture was added saturated NH₄Cl solution (20 ml) and the temperature was kept at 0°C. The mixture was then extracted with EtOAc two times. The combined organic layers were washed with water and brine, dried over Na₂SO₄, filtered and concentrated in vacuum to afford the title compound (2.2 g, 9.81 mmol, 50% yield) as yellow solid.

### Step e) 1-[2-(2,4-difluorophenyl)tetrazol-5-yl]ethanol

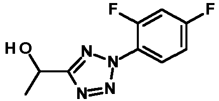

To a solution of 1-[2-(2,4-difluorophenyl)tetrazol-5-yl]ethanone (2.2 g, 9.81 mmol, 1 eq) in MeOH (30 mL) was added NaBH₄ (597.1 mg, 15.7 mmol, 1.6 eq) and the temperature was kept at 0°C. The reaction mixture was stirred at this temperature for 1h. The reaction mixture was treated with 1M aqueous HCl and extracted with EtOAc two times. The combined organic layers were washed with water and brine, dried over Na₂SO₄ and concentrated in vacuumm to afford the title compound (2 g, 8.84 mmol, 90% yield) as light yellow oil.

### Step f) 5-(1-chloroethyl)-2-(2,4-difluorophenyl)tetrazole

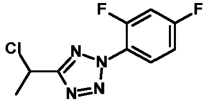

To a solution of 1-[2-(2,4-difluorophenyl)tetrazol-5-yl]ethanol (2 g, 8.84 mmol, 1 eq) in MeCN (10 mL) was added SOCl₂ (5212.66 mg, 44.21 mmol, 5 eq) while the temperature was kept at 0°C for 1 h and then the mixture was stirred at 25°C for 11 h. The reaction mixture was poured into sat. NaHCO₃ solution and this mixture was extracted with EtOAc three times. The combined organic layers were washed with water and brine, dried over Na₂SO₄ and concentrated in vacuum to get crude product, which was purified by column chromatography to afford the title compound (1.7 g, 6.95 mmol, 79% yield) as light yellow oil.

### Step g) 2-(2,4-difluorophenyl)-5-ethyl-tetrazole

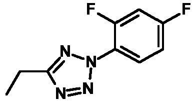

To a suspension of Raney Ni (170.0 mg) in MeOH (10 mL) was added 5-(1-chloroethyl)-2-(2,4-difluorophenyl)tetrazole (1.7 g, 6.95 mmol, 1 eq) and the mixture was stirred under an atmosphere of hydrogen at 25°C for 12 h. The reaction mixture was filtered and concentrated in vacuum, affording the title compound (1.0 g, 4.76 mmol, 69% yield) as light yellow oil.

### Step h) 2-(2,4-difluoro-5-nitro-phenyl)-5-ethyl-tetrazole

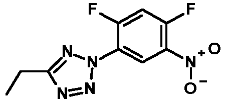

To a solution of 2-(2,4-difluorophenyl)-5-ethyl-tetrazole (750 mg, 3.57 mmol, 1 eq) in sulfuric acid (370 mg, 3.78 mmol, 1.06 eq) was added nitric acid (247 mg, 3.93 mmol, 1.1 eq) while the temperaure was kept at 0°C and the mixture was stirred at 0°C for 3 h. The reaction mixture was poured into ice water and the resulting mixture was extracted with DCM three times. The combined organic layers were washed with water and brine, dried over Na₂SO₄ and concentrated in vacuum to afford the title compound (800 mg, 3.14 mmol, 88% yield) in 10 ml DCM as a light yellow liquid.

### Step i) (2R)-2-(tert-butoxycarbonylamino)-3-[4-(5-ethyltetrazol-2-yl)-5-fluoro-2-nitrophenyl]sulfanyl-propanoic acid

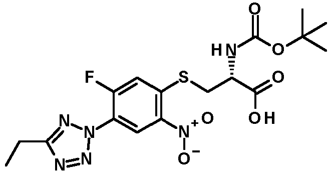

To a solution of 2-(2,4-difluoro-5-nitro-phenyl)-5-ethyl-tetrazole (480.0 mg, 1.88 mmol, 1 eq) in DCM (10 mL) were added NEt₃ (379 mg, 3.76 mmol, 2 eq) and (2R)-2-(tert-butoxycarbonylamino)-3-sulfanyl-propanoic acid (499 mg, 2.26 mmol, 1.2 eq) and the mixture was stirred at 25°C for 12 h. The reaction mixture was diluted with EtOH (10 mL) and then concentrated at 20°C under reduced pressure to remove the DCM. The liquid was used directly for the next step. MS (ESI): 357.3 [M-isobutene-CO₂+H]⁺.

### Step j) (2R)-3-[2-amino-4-(5-ethyltetrazol-2-yl)-5-fluoro-phenyl]sulfanyl-2-(tert-butoxycarbonylamino)propanoic acid

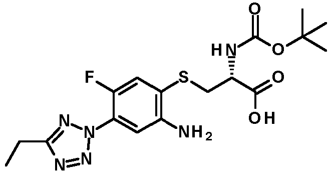

To a solution of (2R)-2-(tert-butoxycarbonylamino)-3-[4-(5-ethyltetrazol-2-yl)-5-fluoro-2-nitrophenyl]sulfanyl-propanoic acid (700 mg, 1.53 mmol, 1 eq) and ammonium chloride (246 mg, 4.6 mmol, 3 eq) in a mixture of ethanol (30 mL) and water (10 mL) was added iron powder (257 mg, 4.6 mmol, 3 eq) and the mixture was stirred at 20°C for 12 h. The reaction mixture was filtered and filtrate was concentrated in vacuum to afford a residue containing the title compound (700 mg) as light yellow oil. MS (ESI): 371.2 [M-isobutene+H] ⁺.

### Step k) tert-butylN-[(3R)-7-(5-ethyltetrazol-2-yl)-8-fluoro-4-oxo-3,5-dihydro-2H-1,5-benzothiazepin-3-yl]carbamate

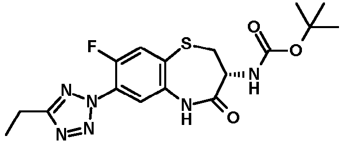

To a solution of (2R)-3-[2-amino-4-(5-ethyltetrazol-2-yl)-5-fluoro-phenyl]sulfanyl-2-(tert-butoxycarbonylamino)propanoic acid (700 mg, 1.64 mmol, 1 eq) and DIPEA (423 mg, 3.28 mmol, 2 eq) in THF (10 mL) was added T3P (1.56 g, 2.46 mmol, 1.5 eq) and the mixture was stirred at 20°C for 12 h. The reaction mixture was concentrated in vacuum to obtain a yellow residue which was purified by column chromatography on silica gel, affording the title compound (100 mg, 0.24 mmol, 15% yield) as light yellow solid. MS (ESI): 353.3 [M-isobutene+H] ⁺.

### Step 1) tert-butyl N-[(3R)-5-[(4-chlorophenyl)methyl]-7-(5-ethyltetrazol-2-yl)-8-fluoro-4-oxo-2,3-dihydro-1,5-benzothiazepin-3-yl]carbamate

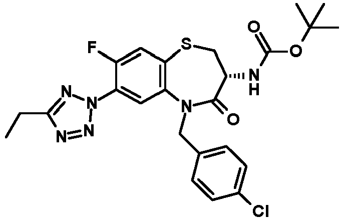

To a mixture of tert-butyl N-[(3R)-7-(5-ethyltetrazol-2-yl)-8-fluoro-4-oxo-3,5-dihydro-2H-1,5-benzothiazepin-3-yl]carbamate (95.0 mg, 0.230 mmol, 1 eq) and potassium carbonate (64.3 mg, 0.47 mmol, 2 eq) in DMF (1 mL) were added 1-(bromomethyl)-4-chlorobenzene (56.9 mg, 0.28 mmol, 1.2 eq) and potassium iodide (0.02 mmol, 0.1 eq) and the mixture was stirred at 20°C for 12 h. The reaction mixture was poured into water and the resulting mixture was extracted with EtOAc three times. The combined organic layers were washed with water (twice) and brine, dried over Na₂SO₄ and concentrated in vacuum to obtain a crude product which was purified by column chromatographyon silica gel to afford the title compound (110 mg, 0.210 mmol, 89% yield) as light yellow oil. MS (ESI): 477.3 [M-isobutene+H] ⁺.

### Step m) tert-butyl N-[(3R)-5-[(4-chlorophenyl)methyl]-7-(5-ethyltetrazol-2-yl)-8-fluoro-1,1,4-trioxo-2,3-dihydro-1λ⁶,5-benzothiazepin-3-yl]carbamate

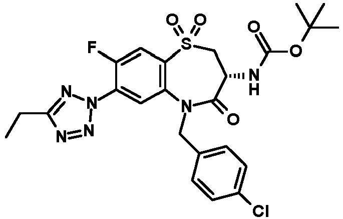

The title compound was prepared in analogy to general method 5 from N-[(3R)-5-[(4-chlorophenyl)methyl]-7-(5-ethyltetrazol-2-yl)-8-fluoro-4-oxo-2,3-dihydro-1,5-benzothiazepin-3-yl]carbamate (22.0 mg, 0.040 mmol, 1 eq) and was obtained (10 mg, 0.020 mmol, 43% yield) as a yellow solid. MS (ESI): 481.3 [M-isobutene-N₂+H]⁺.

### Step n) (3R)-3-amino-5-[(4-chlorophenyl)methyl]-7-(5-ethyltetrazol-2-yl)-8-fluoro-1,1-dioxo-2,3-dihydro-1λ⁶,5-benzothiazepin-4-one

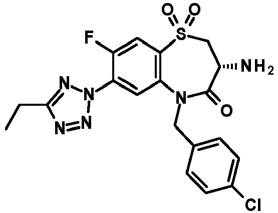

The title compound was prepared according to general method 6b from tert-butyl N-[(3R)-5-[(4-chlorophenyl)methyl]-7-(5-ethyltetrazol-2-yl)-8-fluoro-1,1,4-trioxo-2,3-dihydro-1λ⁶,5-benzothiazepin-3-yl]carbamate (88.0 mg, 0.160 mmol, 1 eq) and was obtained as a white solid (17.3 mg, 0.040 mmol, 23% yield). MS (ESI): 437.1 [M-N₂+H]⁺.

### Example 510

### 3-tert-butyl-5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-one

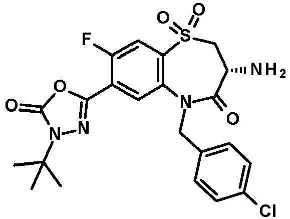

### Step a) tert-butyl N-[(3R)-7-[(tert-butylamino)carbamoyl]-5-[(4-chlorophenyl)methyl]-8-fluoro-4-oxo-2, 3-dihydro-1, 5-benzothiazepin-3-yl]carbamate

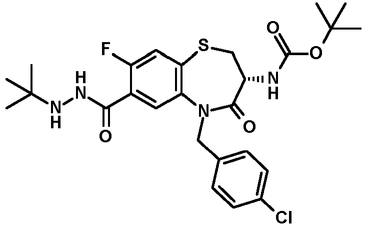

(3R)-3-(tert-butoxycarbonylamino)-5-[(4-chlorophenyl )methyl]-8-fluoro-4-oxo-2,3-dihydro-1,5-benzothiazepine-7-carboxylic acid (CAS 2002449-40-1) (200 mg, 0.416 mmol, 1 eq) was dissolved in THF (1.8 mL). CDI (87.66 mg, 0.541 mmol, 1.3 eq) was added and the light yellow solution was stirred at RT for 30 min. This solution was then transferred via syringe to a separate solution of tert-butylhydrazine hydrochloride (51.82 mg, 0.416 mmol, 1 eq) in THF (0.6 mL) at RT and stirred overnight. The reaction mixture was poured on water and was extracted three times with EtOAc. The organic layers were combined, washed with brine, dried over magnesium sulfate, filtered and concentrated. The remaining crude material was purified by column chromatography on silica gel (0-100% EtOAc in heptane) to afford the title compound (114 mg, 50% yield) as white solid. MS (ESI): 551.3 [M+H]⁺.

### Step b) tert-butyl N-[(3R)-7-(4-tert-butyl-5-oxo-1,3,4-oxadiazol-2-yl)-5-[(4-chlorophenyl)methyl]-8-fluoro-4-oxo-2,3-dihydro-1,5-benzothiazepin-3-yl]carbamate

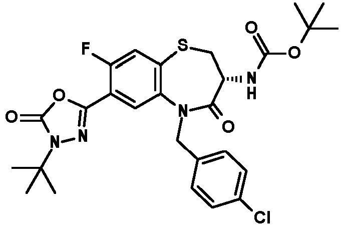

To a colorless solution of tert-butyl N-[(3R)-7-[(tert-butylamino)carbamoyl]-5-[(4-chlorophenyl)methyl]-8-fluoro-4-oxo-2,3-dihydro-1,5-benzothiazepin-3-yl]carbamate (100 mg, 0.181 mmol, 1 eq) in THF (2 mL) was added pyridine (43.06 mg, 44.03 uL, 0.544 mmol, 3 eq) and the solution was cooled 0°C. A solution of trichloromethyl carbonochloridate (CAS 503-38-8) (39.49 mg, 24.08 uL, 0.200 mmol, 1.1 eq) in THF (2 mL) was added dropwise over a time of 5 min and stirred for further 10 min. The ice-bath was removed and the reaction mixture was stirred at RT for 90 min. The reaction was quenched by slow addition of aqueous ammonia. The mixture was then extracted three times with EtOAc. The combined organic layers washed with water and 0.1 N HCl, dried over magnesium sulfate and concentrated under reduced pressure to afford the title compound (63.6 mg, 61% yield) as white solid. MS (ESI): 521.2 [M+H-isobutene]⁺.

### Step c) tert-butyl N-[(3R)-7-(4-tert-butyl-5-oxo-1,3,4-oxadiazol-2-yl)-5-[(4-chlorophenyl)methyl]-8-fluoro-1,1,4-trioxo-2,3-dihydro-1λ⁶,5-benzothiazepin-3-yl]carbamate

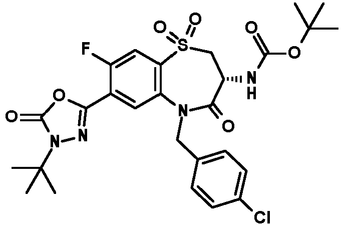

The title compound was prepared from tert-butyl N-[(3R)-7-(4-tert-butyl-5-oxo-1,3,4-oxadiazol-2-yl)-5-[(4-chlorophenyl)methyl]-8-fluoro-4-oxo-2,3-dihydro-1,5-benzothiazepin-3-yl]carbamate (63 mg, 0.109 mmol) in analogy to general procedure **5** and was obtained as white solid (61.8 mg, 93% yield). MS (ESI): 553.2 [M+H-isobutene]⁺.

### Step d) 3-tert-butyl-5-[(3R)-3-amino-5-[(4-chlorophenyl)methyl]-8-fluoro-1,1,4-trioxo-2,3-dihydro-1λ⁶,5-benzothiazepin-7-yl]-1,3,4-oxadiazol-2-one

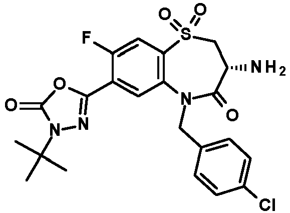

The title compound was prepared from tert-butyl N-[(3R)-7-(4-tert-butyl-5-oxo-1,3,4-oxadiazol-2-yl)-5-[(4-chlorophenyl)methyl]-8-fluoro-1,1,4-trioxo-2,3-dihydro-1λ⁶,5-benzothiazepin-3-yl]carbamate (61.8 mg, 0.101 mmol) in analogy to general procedure 6d and was obtained as a white solid (47.5 mg, 85% yield), as hydrochloride salt. MS (ESI): 509.2 [M+H]⁺.

### Example 511

### methyl 1-[3-[(3R)-3-amino-8-fluoro-1,4-dioxo-5-[[4-(1,1,2,2-tetrafluoroethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate

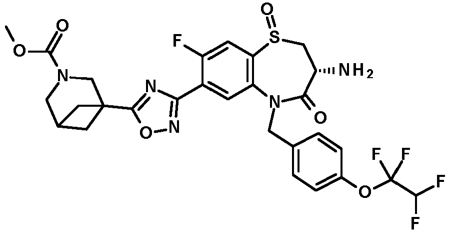

### Step a) tert-butyl N-[(3R)-7-cyano-8-fluoro-4-oxo-5-[[4-(1,1,2,2-tetrafluoroethoxy)phenyl]methyl]-2,3-dihydro-1,5-benzothiazepin-3-yl]carbamate

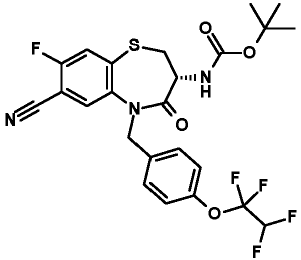

The title compound was prepared in analogy to general procedure **4** from tert-butyl N-[(3R)-7-cyano-8-fluoro-4-oxo-3,5-dihydro-2H-1,5-benzothiazepin-3-yl]carbamate (Example 321, Step c) (450 mg, 1.33 mmol) using 1-(bromomethyl)-4-(1,1,2,2-tetrafluoroethoxy)benzene (CAS 67033-41-4) (459 mg, 1.6 mmol) and was obtained as a light yellow solid (680 mg, 94 % yield). MS (ESI): 488.0 [M-isobutene+H]⁺.

### Step b) tert-butyl N-[(3R)-8-fluoro-7-[(Z)-N'-hydroxycarbamimidoyl]-4-oxo-5-[[4-(1,1,2,2-tetrafluoroethoxy)phenyl]methyl]-2,3-dihydro-1, 5-benzothiazepin-3-yl]carbamate

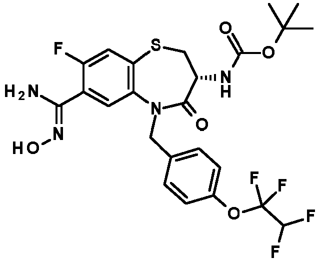

The title compound was prepared in analogy to general procedure **12** from *tert-butyl N-[(3R)-7-cyano-8-fluoro-4-oxo-5-[[4-(1,1,2,2-tetrafluoroethoxy)phenyl]methyl]-2,3-dihydro-1,5-benzothiazepin-3-yl]carbamate* (700 mg, 1.29 mmol) and was obtained as a light yellow solid (660 mg, 89% yield). MS (ESI): 521.1 [M-isobutene+H]⁺.

### Step c) O1-[(Z)-[amino-[(3R)-3-(tert-butoxycarbonylamino)-8-fluoro-4-oxo-5-[[4-(1,1,2,2-tetrafluoroethoxy)phenyl]methyl]-2,3-dihydro-1,5-benzothiazepin-7-yl]methylene[amino] O3-tert-butyl 3-azabicyclo[3.1.1]heptane-1,3-dicarboxylate

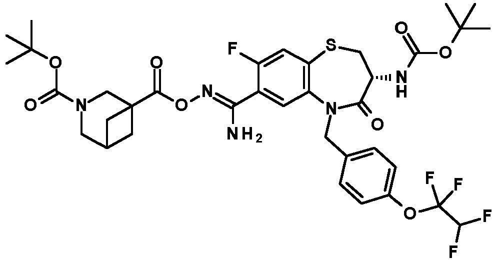

The title compound was prepared in analogy to general procedure **10a** from *tert-butyl N-[(3R)-8-fluoro-7-[(Z)-N'-hydroxycarbamimidoyl]-4-oxo-5-[[4-(1,1,2,2-tetrafluoroethoxy)phenyl]methyl]-2,3-dihydro-1,5-benzothiazepin-3-yl]carbamate* (400 mg, 0.69 mmol) using 3-tert-butoxycarbonyl-3-azabicyclo[3.1.1]heptane-1-carboxylic acid (CAS 1250995-41-5) (200 mg, 0.83 mmol) and was obtained as a light yellow solid (500 mg, 90% yield). MS (ESI): 644.2 [M-2isobutene-CO₂+H]⁺.

### Step d) tert-butyl 1-[3-[(3R)-3-(tert-butoxycarbonylamino)-8-fluoro-4-oxo-5-[[4-(1,1,2,2-tetrafluoroethoxy)phenyl]methyl]-2,3-dihydro-1,5-benzothiazepin-7-yl]-1,2,4-oxadiazol-5-yl]-3-azabicyclo[3.1.1]heptane-3-carboxylate

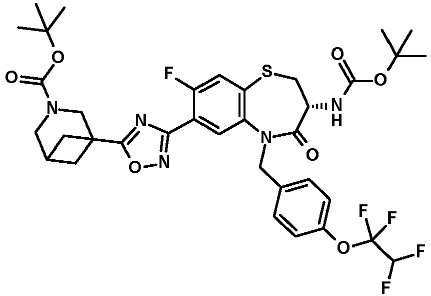

The title compound was prepared in analogy to general procedure **11a** from O1-[(Z)-[amino-[(3R)-3-(tert-butoxycarbonylamino)-8-fluoro-4-oxo-5-[[4-(1,1,2,2-tetrafluoroethoxy)phenyl]methyl]-2,3-dihydro-1,5-benzothiazepin-7-yl methylene]amino] O3-tert-butyl 3-azabicyclo[3.1.1]heptane-1,3-dicarboxylate (500 mg, 0.625 mmol) and was obtained as a white solid (380 mg, 77% yield). MS (ESI): 626.1 [M-CO₂-2isobutene+H]⁺.

### Step e) tert-butyl 1-[3-[(3R)-3-(tert-butoxycarbonylamino)-8-fluoro-1,4-dioxo-5-[[4-(1,1,2,2-tetrafluoroethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate (Epimer A) and tert-butyl 1-[3-[(3R)-3-(tert-butoxycarbonylamino)-8-fluoro-1,4-dioxo-5-[[4-(1,1,2,2-tetrafluoroethoxy)phenyl]methyl]-2,3-dihydro-1λ⁴,5-henzothiazepin-7-yl]-1,2,4-oxadiazol-5-yl]-3-azahicyclo[3.1.1]heptane-3-carboxylate (Epimer B)

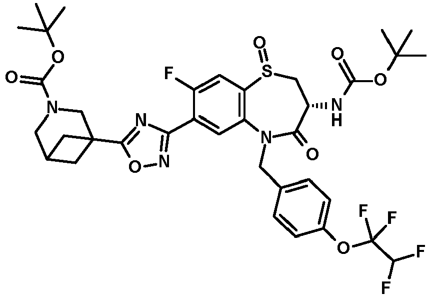

The title compound was prepared in analogy to general procedure **5** from *tert-butyl* 1-[3-[(3R)-3-*(tert-butoxycarbonylamino)-8-fluoro-4-oxo-5-[[4-(1,1,2,2-tetrafluoroethoxy)phenyl]methyl]-2, 3-dihydro-1,5-benzothiazepin-7-yl]-1,2,4-oxadiazol-5-yl]-3-azabicyclo[3.1.1]heptane-3-carboxylate* (170 mg, 0.217 mmol) and were obtained as a colorless oil (Epimer A, 95 mg, 49% yield) (MS (ESI): 624.1 [M-CO₂-2isobutene+H]⁺) and as a colorless oil (Epimer B, 65 mg, 30% yield) (MS (ESI): 624.1 [M-CO₂-2isobutene+H]⁺).

### Step f) (3R)-3-amino-7-[5-(3-azabicyclo[3.1.1]heptan-1-yl)-1,2,4-oxadiazol-3-yl]-8-fluoro-1-oxo-5-[[4-(1,1,2,2-tetrafluoroethoxy)phenyl]methyl]-2,3-dihydro-1λ⁴,5-benzothiazepin-4-one (Epimer A)

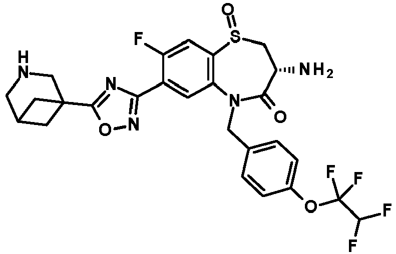

The title compound was prepared in analogy to general procedure **6e** from tert-butyl 1-[3-[(3R)-3-(tert-butoxycarbonylamino)-8-fluoro-1,4-dioxo-5-[[4-(1,1,2,2-tetrafluoroethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate (Epimer A) (85 mg, 0.107 mmol). and was obtained as a light yellow solid (73 mg, 75% yield). MS (ESI): 598.2 [M+H]⁺.

### Step g) methyl 1-[3-[(3R)-3-amino-8-fluoro-1,4-dioxo-5-[[4-(1,1,2,2-tetrafluoroethoxy)phenyl]methyl]-2,3-dihydro-1λ⁴,5-benzothiazepin- 7-yl]-1,2, 4-oxadiazol-5-yl]-3-azabicyclo[3.1.1]heptane-3-carboxylate (Epimer A)

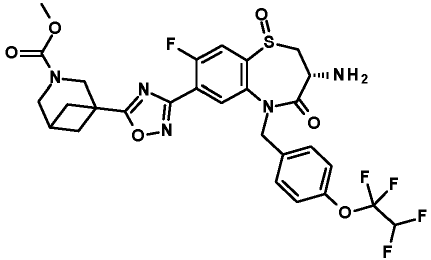

To a solution of tert-butyl 1-[3-[(3R)-3-(tert-butoxycarbonylamino)-8-fluoro-1,4-dioxo-5-[[4-(1,1,2,2-tetrafluoroethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate (Epimer A) (73 mg, 0.088 mmol) and DIPEA (34.328 mg, 0.265 mmol) in DCM (2mL) was added a solution of methyl chloroformate (8.36 mg, 0.088mmol) in DCM (0.5 mL) at RT. The mixture was stirred for 1 hour at RT. The mixture was evaporated to dryness to obtain a light yellow oil which was purified by prep-HPLC affording the title compound (23.6 mg, 41% yield). MS (ESI): 656.2 [M+H]⁺.

### Example 512

### methyl 1-[3-[(3R)-3-amino-8-fluoro-1,1,4-trioxo-5-[[4-(1,1,2,2-tetrafluoroethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate

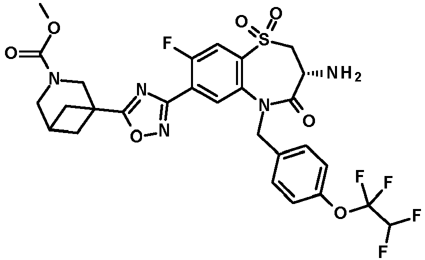

### Step a) tert-butyl 1-[3-[(3R)-3-(tert-butoxycarbonylamino)-8-fluoro-1,1,4-trioxo-5-[[4-(1,1,2,2-tetrafluoroethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate

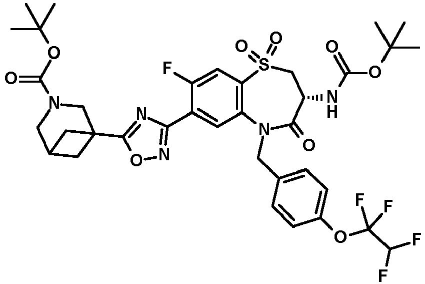

The title compound was prepared in analogy to general procedure **5** from *tert-butyl* 1-[3-[(3R)-3-*(tert-butoxycarbonylamino)-8-fluoro-4-oxo-5-[[4-(1,1,2,2-tetrafluoroethoxy)phenyl]methyl]-2,3-dihydro-1,5-benzothiazepin-7yl]-1,2,4-oxadiazol-5yl]-3-azabicyclo[3.1.1]heptane-3-carboxylate* (Example 511, Step d) (150 mg, 0.192 mmol) and was obtained as a white solid (165 mg, 106% yield). MS (ESI): 714.3 [M-Boc+H]⁺.

### Step b) methyl 1-[3-[(3R)-3-amino-8-fluoro-1,1,4-trioxo-5-[[4-(1,1,2,2-tetrafluoroethoxy)phenyl]methyl]-2,3-dihydro-1λ⁴,5-benzothiazepin-7-yl]-1,2,4-oxadiazol-5-yl]-3-azabicyclo[3.1.1]heptane-3-carboxylate

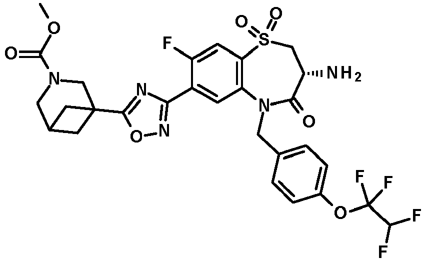

The title compound was prepared in analogy to Example 511, steps f to g) from *tert-butyl 1-[3-[(3R)-3-(tert-butoxycarbonylamino)-8-fluoro-1,1,4-trioxo-5-[[4-(1,1,2,2-tetrafluoroethoxy)phenyl]methyl]-2,3-dihydro-1λ⁴,⁶,5-benzothiazepin-7-yl]-1,2,4-oxadiazol-5-yl]-3-azabicyclo[3.1.1]heptane-3-carboxylate* (160 mg, 0.037 mmol) and was obtained as a white solid (47.3 mg, 39% yield). MS (ESI): 672.2 [M+H]⁺.

### Example 513

### (3R)-3-amino-8-fluoro-5-[(4-fluorophenyl)methyl]-1,1-dioxo-7-[5-(1,2,2,2-tetrafluoro-1-methoxy-ethyl)-1,2,4-oxadiazol-3-yl]-2,3-dihydro-1λ⁶,5-benzothiazepin-4-one

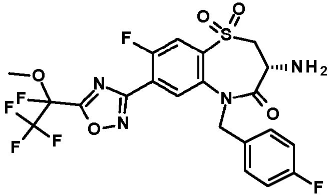

### Step a) tert-butyl N-[(3R)-8-fluoro-4-oxo-7-[5-(1,2,2,-ethyl)-1,2,4-oxadiazol-3-yl]-3,5-dihydro-2H-1,5-benzothiazepin-3-yl]carbamate

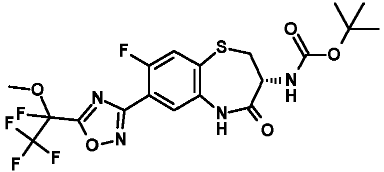

The title compound was prepared in analogy to general procedure **9a** from tert-butyl N-[(3R)-8-fluoro-7-[(Z)-N'-hydroxycarbamimidoyl]-4-oxo-3,5-dihydro-2H-1,5-benzothiazepin-3-yl]carbamate (Example 321, Step d) (94 mg, 0.535 mmol) and was obtained as a white solid (14 mg, 5 % yield). MS (ESI): 509.1 [M-H]⁻.

### Step b) tert-butyl N-[(3R)-8-fluoro-5-[(4-fluorophenyl)methyl]-4-oxo-7-[5-(1,2,2,2-tetrafluoro-1-methoxy-ethyl)-1,2,4-oxadiazol-3-yl]-2, 3-dihydro-1,5-benzothiazepin-3-yl]carbamate

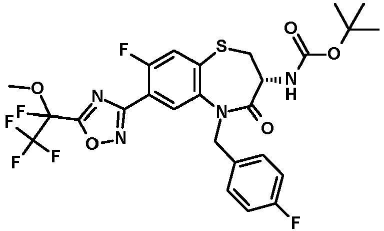

The title compound was prepared in analogy to general procedure 4 from *tert-butyl N-[(3R)-8-fluoro-4-oxo-7-[5-(1,2,2,2-tetrafluoro-1-methoxy-ethyl)-1,2,4-oxadiazol-3-yl]-3,5-dihydro-2H-1,5-benzothiazepin-3-yl]carbamate* (14 mg, 0.027 mmol) and 4-fluorobenzyl bromide (7.78 mg, 5.08 uL, 0.041 mmol, 1.5 eq) and was obtained as a brown solid (18.3 mg, 86% yield). MS (ESI): 563.0 [M-isobutene+H]⁺.

### Step c) tert-butyl N-[(3R)-8-fluoro-5-[(4-fluorophenyl)methyl]-1,1,4-trioxo-7-[5-(1,2,2,2-tetrafluoro-1-methoxy-ethyl)-1,2,4-oxadiazol-3-yl]-2,3-dihydro-1λ⁴,5-benzothiazepin-3-yl]carbamate

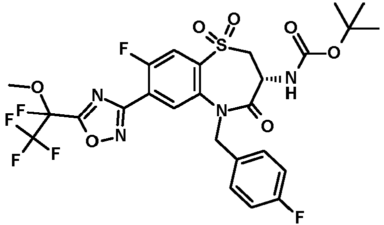

The title compound was prepared in analogy to general procedure **5** from *tert-butyl N-[(3R)-8-fluoro-5-[(4-fluorophenyl)methyl]-4-oxo-7-[5-(1,2,2,2-tetrafluoro-1-methoxy-ethyl)-],2,4-oxadiazol-3-yl]-2,3-dihydro-1,5-benzothiazepin-3-yl]carbamate* (18.3 mg, 0.024 mmol) and was obtained as a white solid (7.3 mg, 45% yield). MS (ESI): 595.1 [M-isobutene+H]⁺.

### Step d) (3R)-3-amino-8-fluoro-5-[(4-fluorophenyl)methyl]-1,1-dioxo-7-[5-(1,2,2,2-tetrafluoro-1-methoxy-ethyl)-1,2,4-oxadiazol-3-yl]-2,3-dihydro-1λ⁶,5-benzothiazepin-4-one

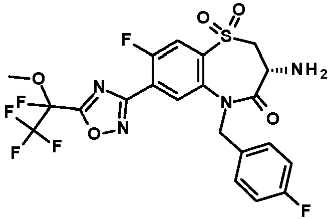

The title compound was prepared in analogy to general procedure **6d** from *tert-butyl N-[(3R)-8-fluoro-5-[(4-fluorophenyl)methyl]-1,1,4-trioxo-7-[5-(1,2,2,2-tetrafluoro-1-methoxy-ethyl)-1,2,4-oxadiazol-3-yl]-2,3-dihydro-1λ⁶,5-benzothiazepin-3-yl]carbamate* (7.3 mg, 0.011 mmol) and was obtained as a white solid (5.7 mg, 87% yield). MS (ESI): 551.1 [M+H]⁺.

### Example 514

### (3R)-3-amino-8-fluoro-5-[(4-fluorophenyl)methyl]-1-oxo-7-[5-(1,2,2,2-tetrafluoro-1-methoxy-ethyl)-1,2,4-oxadiazol-3-yl] -2,3-dihydro-1λ⁴,5-benzothiazepin-4-one

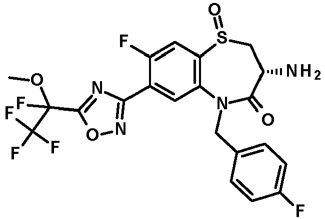

### Step a) tert-butyl N-[(3R)-8-fluoro-5-[(4-fluorophenyl)methyl]-1,4-dioxo-7-[5-(1,2,2,2-tetrafluoro-1-methoxy-ethyl)-1, 2, 4-oxadiazol-3-yl]-2,3-dihydro-1λ⁴,5-benzothiazepin-3-yl]carbamate

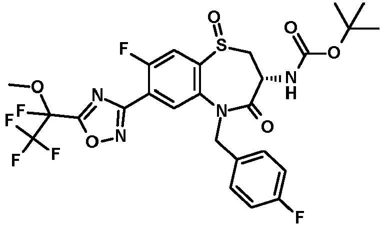

The title compound was prepared in analogy to general procedure **5** from *tert-butyl N-[(3R)-8-fluoro-5-[(4-fluorophenyl)methyl]-4-oxo-7-[5-(1,2,2,2-tetrafluoro-1-methoxy-ethyl)-1,2,4-oxadiazol-3-yl]-2,3-dihydro-1,5-benzothiazepin-3-yl]carbamate* (Example 513, Step b) (18.3 mg, 0.024 mmol) and was obtained as a white solid (1.5 mg, 9% yield). MS (ESI): 633.3 [M-H]⁻.

### Step b) (3R)-3-amino-8-fluoro-5-[(4-fluorophenyl)methyl]-1-oxo-7-[5-(1,2,2,2-tetrafluoro-1-methoxy-ethyl)-1,2,4-oxadiazol3-yl]-2,3-dihydro-1λ⁴,5-benzothiazepin-4-one

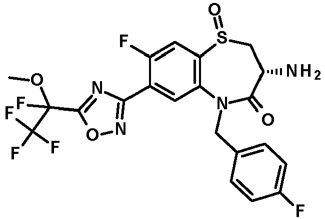

The title compound was prepared in analogy to general procedure **6d** from *tert-butyl N-[(3R)-8-fluoro-5-[(4-fluorophenyl)methyl]-1,4-dioxo-7-[5-(1,2,2,2-tetrafluoro-1-methoxy-ethyl)-1,2,4-oxadiazol-3-yl]-2,3-dihydro-1λ⁴,5-benzothiazepin-3-yl]carbamate* (1.5 mg, 0.002 mmol) and was obtained as a colorless solid (1 mg, 63% yield). MS (ESI): 535.1 [M+H]⁺.

### Example 515

### (3R)-3-amino-5-[(4-chlorophenyl)methyl]-8-fluoro-1-imino-1-oxo-7-[5-(1,2,2,2-tetrafluoro-1-methoxy-ethyl)-1,2,4-oxadiazol-3-yl]-2,3-dihydro-1λ⁶,5-benzothiazepin-4-one

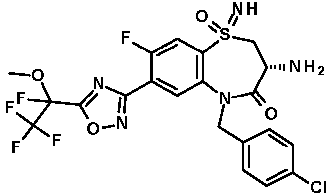

### Step a) tert-butylN-[(3R)-5-[(4-chlorophenyl)methyl]-8-fluoro-1-imino-1,4-dioxo-7-[5-(1,2,2,2-tetrafluoro-1-methoxy-ethyl)-1, 2, 4-oxadiazol-3-yl]-2,3-dihydro-1λ⁶,5-benzothiazepin-3-yl]carbamate

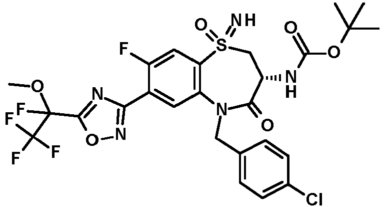

To a solution of tert-butyl N-[(3R)-5-[(4-chlorophenyl)methyl]-8-fluoro-4-oxo-7-[5-(1,2,2,2-tetrafluoro-1-methoxy-ethyl)-1,2,4-oxadiazol-3-yl]-2,3-dihydro-1,5-benzothiazepin-3-yl]carbamate (Example 338, step a) (21 mg, 0.033 mmol) in MeOH (0.165 mL) were added ammonium carbamate (6.45 mg, 0.083 mmol) and iodobenzene diacetate (31.96 mg, 0.099 mmol) and the reaction was stirred at RT for 30 mins.. Isolute was added and the reaction was concentrated in vacuo. The crude product was purified by column chromatography on silica gel (EtOAc:heptane = 0:10 to 8:2) to afford the desired title compound as an off-white solid (7.0 mg, 30% yield). MS (ESI): 610.0 [M-isobutene+H] ⁺.

### Step b) (3R)-3-amino-5-[(4-chlorophenyl)methyl]-8-fluoro-1-imino-1-oxo-7-[5-(1,2,2,2-tetrafluoro-1-methoxy-ethyl)-1,2,4-oxadiazol-3-yl]-2,3-dihydro-1λ⁶,5-benzothiazepin-4-one

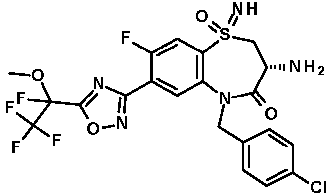

The title compound was prepared in analogy to general procedure **6d** from *tert-butyl N-[(3R)-5-[(4-chlorophenyl)methyl]-8-fluoro-1-imino-1,4-dioxo-7-[5-(1,2,2,2-tetrafluoro-1-methoxyethyl)-1, 2, 4-oxadiazol-3-yl]-2,3-dihydro-1λ⁶,5-benzothiazepin-3-yl]carbamate* (7 mg, 0.01 mmol) and was obtained as a light yellow solid, as a hydrochloric salt (6 mg, 89% yield). MS (ESI): 566.0 [M+H]⁺.

Example 516 of the following table was prepared in analogy to Example 515, steps a to b), using the appropriate building block.

| **Ex.** | **Structure** | **Systematic Name** | **Building Blocks** | **MS, ESI: m/z** |
|---|---|---|---|---|
| 516 | 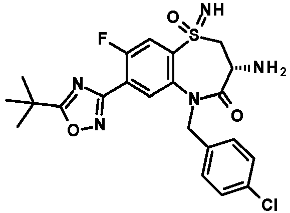 | (3R)-3-amino-7-(5-tert-butyl-1,2,4-oxadiazol-3-yl)-5-[(4-chlorophenyl)methyl]-8-fluoro-1-imino-1-oxo-2,3-dihydro-1λ⁶,5-benzothiazepin-4-one | tert-Butyl N-[(3R)-5-[(4-chlorophe nyl)methyl ]-8-fluoro-7-[(Z)-N'-hydroxycar bamimidoy l]-4-oxo-2,3-dihydro-1,5-benzothiaz epin-3-yl]carbamate | 492.2 [M+H]⁺ |
| | | (*) | (Example 289 step a) | |

| | | | | |
|---|---|---|---|---|
| * As a hydrochloric salt | | | | |

### Example 517and Example 518

### (3R)-3-amino-7-(5-tert-butyl-1,3,4-oxadiazol-2-yl)-8-fluoro-1-imino-1-oxo-5-[[4-(trifluoromethoxy)phenyl]methyl]-2,3-dihydro-1λ⁶,5-benzothiazepin-4-one

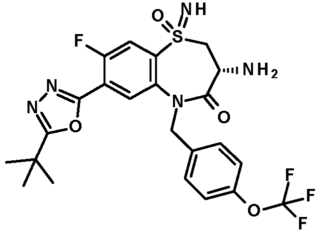

### Step a) tert-butyl N-[(3R)-7-[(2,2-dimethylpropanoylamino)carbamoyl]-8-fluoro-4-oxo-5-[[4-(trifluoromethoxy)phenyl]methyl]-2, 3-dihydro-1, 5-benzothiazepin-3-yl]carbamate

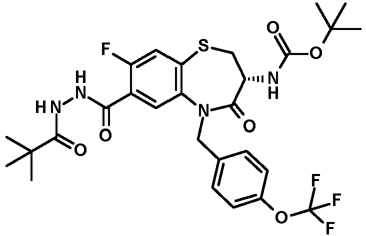

The title compound was prepared in analogy to general procedure **7a** from (3R)-3-amino-8-fluoro-4-oxo-5-[[4-(trifluoromethoxy)phenyl]methyl]-2,3-dihydro-1,5-benzothiazepine-7-carboxylic acid (Example 53, Step b) (1000 mg, 1.89 mmol) and was obtained as a light yellow solid (470 mg, 36 % yield). MS (ESI): 651.2 [M+Na]⁺.

### Step b) tert-butyl N-[(3R)-7-(5-tert-butyl-1, 3, 4-oxadiazol-2 -yl)-8-fluoro-4-oxo-5-[[4-(trifluoromethoxy)phenyl]methyl]-2, 3-dihydro-1, 5-benzothiazepin-3-yl]carbamate

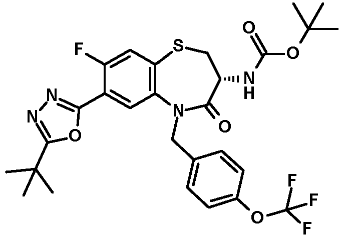

The title compound was prepared in analogy to general procedure **8a** from *tert-butyl N-[(3R)-7-[(2,2-dimethylpropanoylamino)carbamoyl]-8-fluoro-4-oxo-5-[[4-(trifluoromethoxy)phenyl]methyl]-2,3-dihydro-1,5-benzothiazepin-3-yl]carbamate* (470 mg, 0.748 mmol) and was obtained as a light yellow gum (250 mg, 55% yield). MS (ESI): 611.4 [M+H]⁺.

### Step c) tert-butyl N-[(3R)-7-(5-tert-butyl-1,3,4-oxadiazol-2-yl)-8-fluoro-1-imino-1,4-dioxo-5-[[4-(trifluoromethoxy)phenyl]methyl]-2,3-dihydro-1λ⁶,5-benzothiazepin-3-yl]carbamate

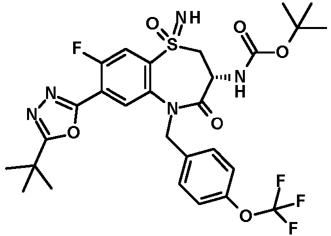

To a solution of *tert-butyl N-[(3R)-7-(5-tert-butyl-1,3,4-oxadiazol-2-yl)-8-fluoro-4-oxo-5-[[4-(trifluoromethoxy)phenyl]methyl]-2, 3-dihydro-1, 5-benzothiazepin-3-yl]carbamate* (250 mg, 0.409 mmol) in MeOH (2 mL) were added ammonium carbamate (103.2 mg, 1.64 mmol) and iodobenzene diacetate (395 mg, 1.23 mmol) and the reaction was stirred at 15°C for 16 hours. The crude was evaporated to dryness and purified by column chromatography on silica gel (PE:EtOAc = 9:1 to 1:4) and the title compound obtained as a light yellow gum (88 mg, 30% yield) MS (ESI): 642.2 [M+H]⁺.

### Step d) (3R)-3-amino-7-(5-tert-butyl-1,3,4-oxadiazol-2-yl)-8-fluoro-1-imino-1-oxo-5-[[4-(trifluoromethoxy)phenyl]methyl]-2,3-dihydro-1λ⁶,5-benzothiazepin-4-one (Epimer A) and (3R)-3-amino-7-(5-tert-butyl-1,3,4-oxadiazol-2 yl)-8-fluoro-1-imino-1-oxo-5-[[4-(trifluoromethoxy)phenyl]methyl]-2,3-dihydro-1λ⁶,5-benzothiazepin-4-one (Epimer B)

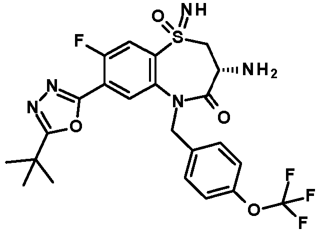

The title compound was prepared in analogy to general procedure **6d** from *tert-butyl N-[(3R)-7-(5-tert-butyl-1,3,4-oxadiazol-2-yl)-8-fluoro-1-imino-1,4-dioxo-5-[[4-(trifluoromethoxy)phenyl]methyl]-2,3-dihydro-1λ⁶,5-benzothiazepin-3-yl]carbamate* (88 mg, 0.137 mmol) and the epimers were separated by prep-HPLC. They were obtained as white solids (Epimer A, 12.5 mg, 15% yield) as a hydrochloric salt (MS (ESI): 542.1 [M+H]⁺) and (Epimer B, 24.1 mg, 30% yield) as a hydrochloric salt (MS (ESI): 542.1 [M+H]⁺).

### Example 519

### (3R)-3-amino-7-(5-tert-butyl-1,3,4-oxadiazol-2-yl)-8-fluoro-1-methylimino-1-oxo-5-[[4-(trifluoromethoxy)phenyl]methyl]-2,3-dihydro-1λ⁶,5-benzothiazepin-4-one

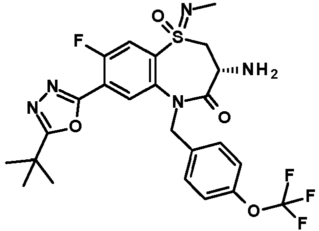

### Step a) tert-butyl N-[(3R)-7-(5-tert-butyl-1,3,4-oxadiazol-2-yl)-8-fluoro-1-methylimino-1,4-dioxo-5-[[4-(trifluoromethoxy)phenyl]methyl]-2,3-dihydro-1λ⁶,5-benzothiazepin-3-yl]carbamate

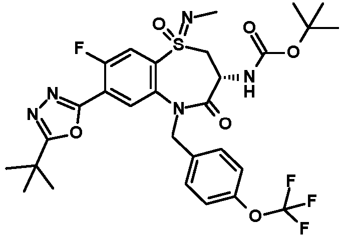

To a solution of *tert-butyl N-[(3R)-7-(5-tert-butyl-1,3,4-oxadiazol-2yl)-8-fluoro-1-imino-1,4-dioxo-5-[[4-(trifluoromethoxy)phenyl]methyl]-2,3-dihydro-1λ⁶,5-benzothiazepin-3-yl]carbamate* (Example 517, Step c) (98 mg, 0.153 mmol) in 1,4-dioxane (5 mL) were added methylboronic acid (36.5 mg, 0.61 mmol), copper(II) acetate (41.1 mg, 0.23 mmol) and pyridine (29 mg, 0.37 mmol) and the reaction was stirred at 90°C for 3 hours. The solvent was evaporated to dryness and purified by column chromatography (PE:EtOAc = 9:1 to 1:4). The title compound was obtained as a white solid (58 mg, 53% yield) MS (ESI): 656.3 [M+H]⁺.

### Step b) (3R)-3-amino-7-(5-tert-butyl-1,3,4-oxadiazol-2-yl)-8-fluoro-1-methylimino-1-oxo-5-[[4-(trifluoromethoxy)phenyl]methyl]-2,3-dihydro-1λ⁶,5-benzothiazepin-4-one (Epimer A) and (3R)-3-amino-7-(5-tert-butyl-1,3,4-oxadiazol-2 yl)-8-fluoro-1-methylimino-1-oxo-5-[[4-(trifluoromethoxy)phenyl]methyl]-2,3-dihydro-1λ⁶,5-benzothiazepin-4-one (Epimer B)

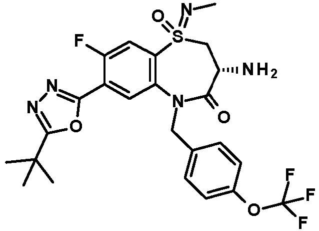

The title compound was prepared in analogy to general procedure **6d** from tert-butyl N-[(3R)-7-(5-tert-butyl-1,3,4-oxadiazol-2-yl)-8-fluoro-1-methylimino-1,4-dioxo-5-[[4-(trifluoromethoxy)phenyl]methyl]-2,3-dihydro-1λ⁶,5-benzothiazepin-3-yl]carbamate (83.7 mg, 0.128 mmol). and the epimers were separated by prep-HPLC. They were obtained as white solids (Epimer A, 7.4 mg, 10% yield) as a hydrochloric salt (MS (ESI): 556.3 [M+H]⁺) and (Epimer B, 22.3 mg, 26 % yield) as a hydrochloric salt (MS (ESI): 556.3 [M+H]⁺).

### Example 520

### methyl 1-[3-[(3R)-3-amino-8-fluoro-1-imino-1,4-dioxo-5-[[4-(1,1,2,2-tetrafluoroethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate

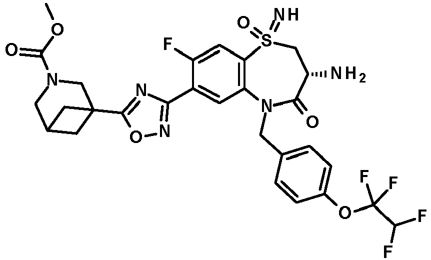

### Step a) tert-butyl 1-[3-[(3R)-3-(tert-butoxycarbonylamino)-8-fluoro-1-imino-1,4-dioxo-5-[[4-(1,1,2,2-tetrafluoroethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate (EpimerA) and tert-butyl 1-[3-[(3R)-3-(tert-butoxycarbonylamino)-8-fluoro-1-imino-1,4-dioxo-5-[[4-(1,1,2,2-tetrafluoroethoxy)phenyl]methyl]-2,3-dihydro-1λ⁶,5-benzothiazepin-7-yl]-1,2,4-oxadiazol-5-yl]-3-azahicyclo[3. 1.1]heptane-3-carboxylate (Epimer B)

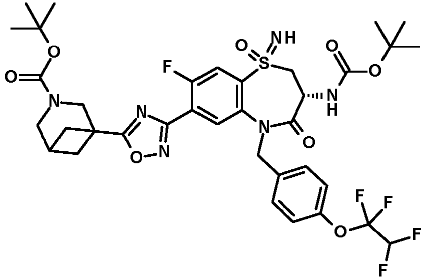

To a solution of *tert-butyl 1-[3-[(3R)-3-(tert-butoxycarbonylamino)-8-fluoro-4-oxo-5-[[4-(1,1,2,2-tetrafluoroethoxy)phenyl]methyl]-2,3-dihydro-1,5-benzothiazepin-7-yl]-1,2,4-oxadiazol-5-yl]-3-azabicyclo[3.1.1]heptane-3-carboxylate* (Example 511, Step d) (190 mg, 0.243 mmol) in MeOH (5 mL) were added ammonium carbamate (76.6 mg, 1.22 mmol) and iodobenzene diacetate (469 mg, 1.46 mmol) and the reaction was stirred at 40°C for 12 hours. The solvent was evaporated to dryness and purified by prep TLC ((PE:EtOAc = 1:1) and prep-SFC and the two epimers were obtained as a white solid (Epimer A, 13 mg, 7% yield) (MS (ESI): 813.3 [M-BOC-isobutene+H]⁺) and as a white solid (Epimer B, 36 mg, 17% yield) (MS (ESI): 624.1 [M+H]⁺).

### Step b) methyl 1-[3-[(3R)-3-amino-8-fluoro-1-imino-1,4-dioxo-5-[[4-(1,1,2, 2-tetrafluoroethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate (Epimer B)

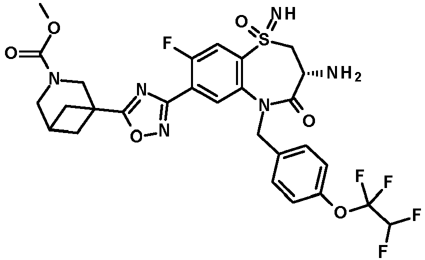

The title compound was prepared in analogy to Example 511, steps f to g) from tert-butyl 1-[3-[(3R)-3-(tert-butoxycarbonylamino)-8-fluoro-1-imino-1,4-dioxo-5-[[4-(1,1,2,2-tetrafluoroethoxy)phenyl]methyl]-2,3-dihydro-1λ⁶,5-benzothiazepin-7-yl]-1,2,4-oxadiazol-5-yl]-3-azabicyclo[3.1.1]heptane-3-carboxylate (Epimer B) (30 mg, 0.037 mmol) and was obtained as a light yellow solid (11 mg, 40% yield). MS (ESI): 671.2 [M+H]⁺.

### Example 521

### (3R)-3-amino-8-fluoro-7-[5-[2-(hydroxymethyl)tetrahydrofuran-2-yl]-1,2,4-oxadiazol-3-yl]-1,1-dioxo-5-[(4-phenoxyphenyl)methyl]-2,3-dihydro-1λ⁶,5-benzothiazepin-4-one

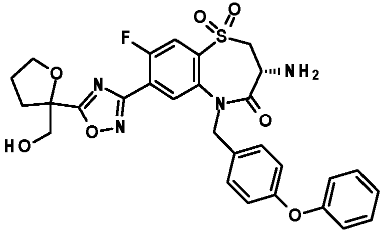

### Step a) tert-butyl N-[(3R)-7-cyano-8-fluoro-4-oxo-5-[(4-phenoxyphenyl)methyl]-2,3-dihydro-1,5-benzothiazepin-3-yl]carbamate

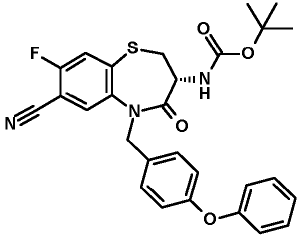

The title compound was prepared in analogy to general procedure **4** from tert-butyl N-[(3R)-7-cyano-8-fluoro-4-oxo-3,5-dihydro-2H-1,5-benzothiazepin-3-yl]carbamate (Example 321, step c) (150 mg, 0.445 mmol) and was obtained as a white solid (213 mg, 81% yield) MS (ESI): 464.4 [M-isobutene+H]⁺.

### Step b) tert-butyl N-[(3R) -8-fluoro-7-[(Z)-N'-hydroxycarbamimidoyl]-4-oxo-5-[(4-phenoxyphenyl)methyl]-2,3-dihydro-1,5-benzothiazepin-3-yl]carbamate

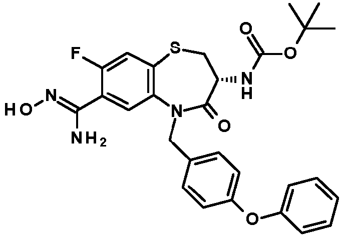

The title compound was prepared in analogy to general procedure **12** from *tert-butyl N-[(3R)-7-cyano-8-fluoro-4-oxo-5-[(4-phenoxyphenyl)methyl]-2,3-dihydro-1,5-benzothiazepin-3-yl]carbamate* (200 mg, 0.339 mmol) and was obtained as a white solid (210 mg, 95% yield) MS (ESI): 553.4 [M+H]⁺.

### Step c) [(Z)-[amino-[(3R)-3-(tert-butoxycarbonylamino)-8-fluoro-4-oxo-5-[(4-phenoxyphenyl)methyl]-2, 3-dihydro-1, 5-benzothiazepin-7-yl]methylene]amino] 2-(hydroxymethyl)tetrahydrofuran-2-carboxylate

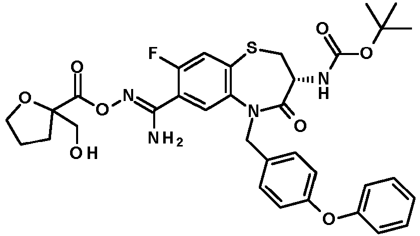

The title compound was prepared in analogy to general procedure 7a from *tert-butyl N-[(3R)-8-fluoro-7-[(Z)-N'-hydroxycarbamimidoyl]-4-oxo-5-[(4-phenoxyphenyl)methyl]-2,3-dihydro-1,5-benzothiazepin-3-yl]carbamate* (100 mg, 0.154 mmol) using tetrahydro-2-(hydroxymethyl)-2-furancarboxylic acid (CAS 442877-01-2) (26.9 mg, 0.185 mmol) and was obtained as a light yellow solid (78.9 mg, 60% yield) MS (ESI): 681.4 [M+H]⁺.

### Step d) tert-butyl N-[(3R)-8-fluoro-7-[5-[2-(hydroxymethyl)tetrahydrofuran-2-yl]-1,2,4-oxadiazol-3-yl]-4-oxo-5-[(4-phenoxyphenyl)methyl]-2,3-dihydro-1,5-benzothiazepin-3-yl]carbamate

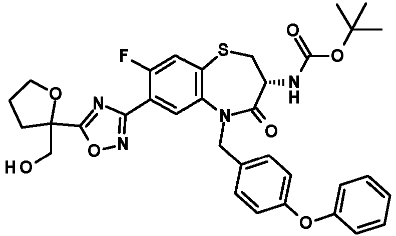

The title compound was prepared in analogy to general procedure **11a** from *[(Z)-[amino-[(3R)-3-(tert-butoxycarbonylamino)-8-fluoro-4-oxo-5-[(4-phenoxyphenyl)methyl]-2,3-dihydro-1,5-benzothiazepin-7yl]methylene]amino] 2-(hydroxymethyl)tetrahydrofuran-2-carboxylate* (78.9 mg, 0.093 mmol) and was obtained as a white powder (41 mg, 67% yield) MS (ESI): 607.3 [M-isobutene+H]⁺.

### Step e) tert-butyl N-[(3R)-8-fluoro-7-[5-[2-(hydroxymethyl)tetrahydrofuran-2-yl]-1,2,4-oxadiazol-3-yl]-1,1,4-trioxo-5-[(4-phenoxyphenyl)methyl]-2,3-dihydro-1λ⁶,5-benzothiazepin-3-yl]carbamate; 5-(3-fluoro-1-methyl-3-piperidyl)-1,2,4-oxadiazole

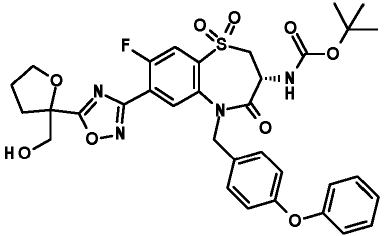

The title compound was prepared in analogy to general procedure **5** from *tert-butyl N-[(3R)-8-fluoro-7-[5-[2-(hydroxymethyl)tetrahydrofuran-2-yl]-1,2,4-oxadiazol-3-yl]-4-oxo-5-[(4-phenoxyphenyl)methyl]-2,3-dihydro-1,5-benzothiazepin-3-yl]carbamate* (41 mg, 0.049 mmol) and was obtained as a white solid (26.3 mg, 76% yield) MS (ESI): 639.2 [M-isobutene+H]⁺.

### Step f) (3R)-3-amino-8-fluoro-7-[5-[2-(hydroxymethyl)tetrahydrofuran-2-yl]-1,2,4-oxadiazol-3-yl]-1,1-dioxo-5-[(4-phenoxyphenyl)methyl]-2, 3-dihydro-1λ⁶,5-benzothiazepin-4-one

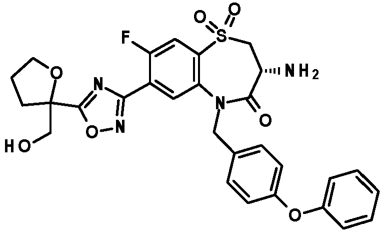

The title compound was prepared in analogy to general procedure **6d** from *tert-butyl N-[(3R)-8-fluoro-7-[5-[2-(hydroxymethyl)tetrahydrofuran-2-yl]-1,2,4-oxadiazol-3-yl]-1,1,4-trioxo-5-[(4-phenoxyphenyl)methyl]-2,3-dihydro-1λ⁶,5-benzothiazepin-3-yl]carbamate;5-(3-fluoro-1-methyl-3-piperidyl)-1,2,4-oxadiazole* (26.3 mg, 0.038 mmol) and was obtained as a white powder, as a hydrochloride salt (23.3 mg, 97% yield) MS (ESI): 595.3 [M +H]⁺.

Examples 522 of the following table was prepared in analogy to Example 521, step a to f), using the appropriate building block.

| **Ex.** | **Structure** | **Systematic Name** | **Building Blocks** | **MS, ESI: m/z** |
|---|---|---|---|---|
| 522 | 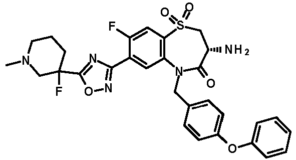 | (3R)-3-amino-8-fluoro-7-[5-(3-fluoro-1-methyl-3-piperidyl)-1,2,4-oxadiazol-3-yl]-1,1-dioxo-5-[(4-phenoxyphenyl)methyl]-2,3-dihydro-1λ⁶,5-benzothiazepin-4-one | 3-Fluoro-1-methylpiperidine-3-carboxylic acid | 610.4 [M+H]⁺ |
| | | | (CAS 2551117-83-8) | |

### Example 523

### (3R)-3-amino-7-(5-tert-butyl-1,3,4-oxadiazol-2-yl)-8-chloro-5-[(4-chlorophenyl)methyl]-1,1-dioxo-2,3-dihydro-1λ⁶,5-benzothiazepin-4-one

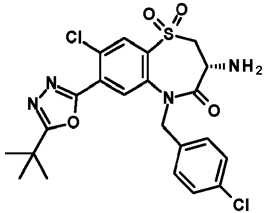

### Step a) (2R)-2-(tert-butoxycarbonylamino)-3-(5-chloro-4-methoxycarbonyl-2-nitrophenyl)sulfanyl-propanoic acid

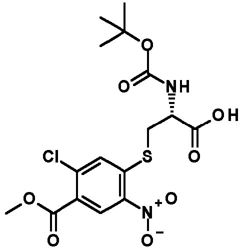

Methyl 2-chloro-4-fluoro-5-nitrobenzoate (CAS 85953-30-6) (2 g, 8.56 mmol, Eq: 1) was dissolved in EtOH (35 mL) and (tert-butoxycarbonyl)-L-cysteine (2.08 g, 9.42 mmol, Eq: 1.1) in water (8.75 ml) was added to the reaction mixture. Then sodium bicarbonate (2.16 g, 25.7 mmol, Eq: 3) was added and the reaction mixture was stirred at 80°C for 2.5 hours. The solvent was evaporated, EtOAc was added and the mixture was extracted with water. The combined aqueous layers were acidified by addition of 1N aq. HCl and they were extracted with EtOAc. The combined extracts were washed with brine, dried over magnesium sulfate, filtered and evaporated to afford the title compound (4.349 g, 6.2 mmol, 72 % yield) as a yellow solid which was used in the next reaction step without further purification.

### Step b) (2R)-3-(2-amino-5-chloro-4-methoxycarbonyl-phenyl)sulfanyl-2-(tert-butoxycarbonylamino)propanoic acid

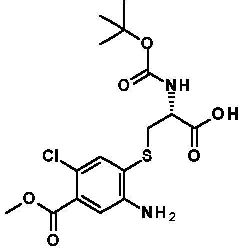

To a solution of (2R)-2-(tert-butoxycarbonylamino)-3-(5-chloro-4-methoxycarbonyl-2-nitrophenyl)sulfanyl-propanoic acid (5.0 g, 11.5 mmol, 1 eq) in EtOAc (50 mL) was added Pd/C (500.0 mg, 0.470 mmol, 0.040 eq). The mixture was degassed with nitrogen three times, then purged with hydrogen three times and then stirred at 25°C for 16 h under an atmosphere of hydrogen. The reaction mixture was filtered and the filter cake was washed with EtOAc (3 x 50 mL). The filtrate was concentrated under vacuum to obtain a brown oil (5.52 g) containing the title compound which was used in the next reaction step without further purification.

### Step c) methyl (3R)-3-(tert-butoxycarbonylamino)-8-chloro-4-oxo-3,5-dihydro-2H-1,5-benzothiazepine-7-carboxylate

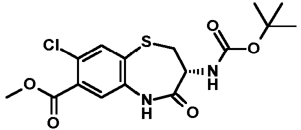

The title compound was prepared from (2R)-3-(2-amino-5-chloro-4-methoxycarbonylphenyl)sulfanyl-2-(tert-butoxycarbonylamino)propanoic acid (5.52 g) in analogy to general procedure **3** and was obtained as a light yellow solid (566 mg, 1.46 mmol, 11% yield). MS (ESI): 408.9 [M+Na]⁺.

### Step d) methyl (3R)-3-(tert-butoxycarbonylamino)-8-chloro-5-[(4-chlorophenyl)methyl]-4-oxo-2,3-dihydro-1,5-benzothiazepine-7-carboxylate

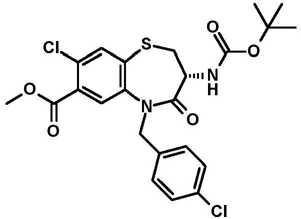

The title compound was prepared from methyl (3R)-3-(tert-butoxycarbonylamino)-8-chloro-4-oxo-3,5-dihydro-2H-1,5-benzothiazepine-7-carboxylate (566.0 mg, 1.46 mmol, 1 eq) in analogy to general procedure **4** (DMF instead of DMSO as solvent) and was obtained as light yellow oil (714 mg, 1.4 mmol, 95% yield). MS (ESI): 455.3 [M-isobuten+H]⁺.

### Step e) (3R)-3-(tert-butoxycarbonylamino)-8-chloro-5-[(4-chlorophenyl)methyl]-4-oxo-2,3-dihydro-1,5-benzothiazepine-7-carboxylic acid

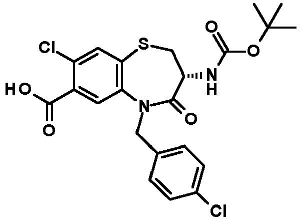

The title compound was prepared from methyl (3R)-3-(tert-butoxycarbonylamino)-8-chloro-5-[(4-chlorophenyl)methyl]-4-oxo-2,3-dihydro-1,5-benzothiazepine-7-carboxylate (714.0 mg, 1.4 mmol, 1 eq) in analogy to Example 218, step f) and was obtained as yellow oil (600 mg, 1.21 mmol, 86% yield). MS (ESI): 441.0 [M-isobuten+H]⁺.

### Step f) tert-butyl N-[(3R)-8-chloro-5-[(4-chlorophenyl)methyl]-7-[(2,2-dimethylpropanoylamino)carbamoyl]-4-oxo-2,3-dihydro-1,5-benzothiazepin-3-yl]carbamate

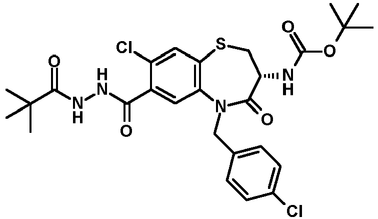

To a solution of (3R)-3-(tert-butoxycarbonylamino)-8-chloro-5-[(4-chlorophenyl)methyl]-4-oxo-2,3-dihydro-1,5-benzothiazepine-7-carboxylic acid (600.0 mg, 1.21 mmol), pivalic acid hydrazide (280.42 mg, 2.41 mmol) and HATU (688.0 mg, 1.81 mmol) in DMF (11 mL) was added DIPEA (0.43 mL, 2.41 mmol) at 25 °C and the mixture was stirred at 25°C for 4.5 h. The reaction mixture was poured into water (10 mL) and the mixture was extracted with ethyl acetate (3 x 10 mL). The combined extracts were washed with brine (3 x 15 mL), dried over anhydrous sodium sulfate, filtered and concentrated under vacuum. The remaining residue was purified by column chromatography on silica gel (PE/EtOAc = 5:1 to 1:1) to afford the title compound (300 mg, 0.500 mmol) as a yellow solid. MS (ESI): 539.1 [M-isobuten+H]⁺.

### Step g) tert-butyl N-[(3R)-7-(5-tert-butyl-1,3,4-oxadiazol-2-yl)-8-chloro-5-[(4-chlorophenyl)methyl]-4-oxo-2,3-dihydro-1,5-benzothiazepin-3-yl]carbamate

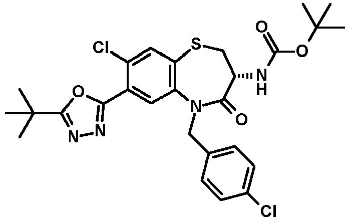

The title compound was prepared from tert-butyl N-[(3R)-8-chloro-5-[(4-chlorophenyl)methyl]-7-[(2,2-dimethylpropanoylamino)carbamoyl]-4-oxo-2,3-dihydro-1,5-benzothiazepin-3-yl]carbamate (300.0 mg, 0.500 mmol, 1 eq) in analogy to general procedure **8a** and was obtained as light yellow solid (260 mg, 0.450 mmol, 72% yield). MS (ESI): 521.2 [M-isobuten+H]⁺.

### Step h) tert-butyl N-[(3R)-7-(5-tert-butyl-1,3,4-oxadiazol-2-yl)-8-chloro-5-[(4-chlorophenyl)methyl]-1,1,4-trioxo-2,3-dihydro-1λ⁶,5-benzothiazepin-3-yl]carbamate

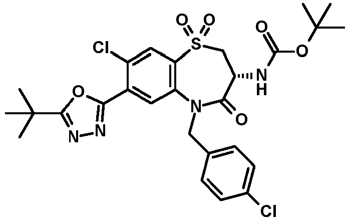

The title compound was prepared from tert-butyl N-[(3R)-7-(5-tert-butyl-1,3,4-oxadiazol-2-yl)-8-chloro-5-[(4-chlorophenyl)methyl]-4-oxo-2,3-dihydro-1,5-benzothiazepin-3-yl]carbamate (260.0 mg, 0.450 mmol, 1 eq) in analogy to general procedure **5** and was obtained as light yellow solid (170 mg, 0.280 mmol, 39% yield). MS (ESI): 553.1 [M-isobuten+H]⁺.

### Step i) (3R)-3-amino-7-(5-tert-butyl-1,3,4-oxadiazol-2yl)-8-chloro-5-[(4-chlorophenyl)methyl]-1,1-dioxo-2,3-dihydro-1λ⁶,5-benzothiazepin-4-one

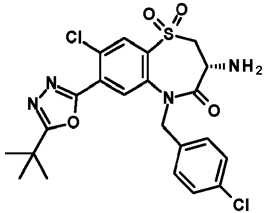

The title compound was prepared from tert-butyl N-[(3R)-7-(5-tert-butyl-1,3,4-oxadiazol-2-yl)-8-chloro-5-[(4-chlorophenyl)methyl]-1,1,4-trioxo-2,3-dihydro-1λ⁶,5-benzothiazepin-3-yl]carbamate (170.0 mg, 0.280 mmol, 1 eq) in analogy to general procedure **6b** and was obtained as white solid (13 mg, 0.020 mmol, 9% yield) as the hydrochloride salt. MS (ESI): 509.1 [M+H]⁺.

### Example 524

### (3R)-3-amino-5-[(4-chlorophenyl)methyl]-7-[5-(5,5-difluoro-1-methyl-3-piperidyl)-1,3,4-oxadiazol-2-yl]-8-methyl-1,1-dioxo-2,3-dihydro-1λ⁶,5-benzothiazepin-4-one

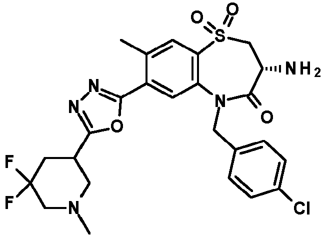

### Step a) benzyl 5-[[[(3R)-3-(tert-butoxycarbonylamino)-5-[(4-chlorophenyl)methyl]-8-methyl-4-oxo-2,3-dihydro-1,5-benzothiazepine-7-carbonyl]amino]carbamoyl]-3,3-difluoro-piperidine-1-carboxylate

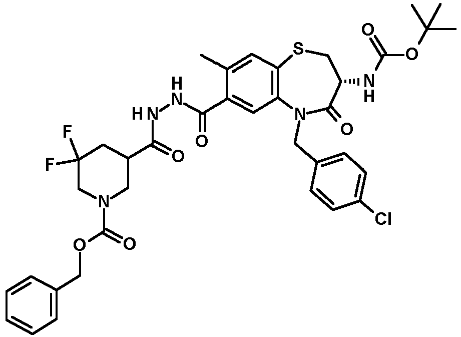

The title compound was prepared in analogy to general procedure 7b from tert-butyl N-[(3R)-5-[(4-chlorophenyl)methyl]-7-(hydrazinecarbonyl)-8-methyl-4-oxo-2,3-dihydro-1,5-benzothiazepin-3-yl]carbamate (Example 276, Step c) (250 mg, 0.509 mmol) and 1-(phenylmethyl) 5,5-difluoro-1,3-piperidinedicarboxylate (CAS 1356338-81-2) (152 mg, 0.509 mmol) and was obtained as a white solid (200 mg, 51% yield). MS (ESI): 772.4 [M+H]⁺.

### Step b) benzyl 5-[5-[(3R)-3-(tert-butoxycarbonylamino)-5-[(4-chlorophenyl)methyl]-8-methyl-4-oxo-2,3-dihydro-1,5-benzothiazepin-7-yl]-1,3,4-oxadiazol-2-yl]-3,3-difluoro-piperidine-1-carboxylate

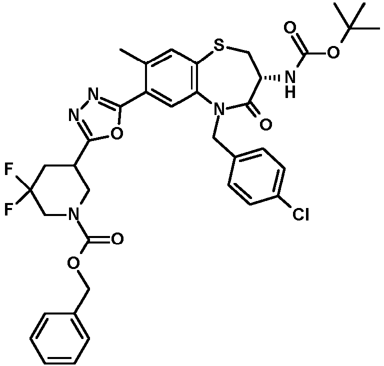

The title compound was prepared in analogy to general procedure **8a** from benzyl 5-[[[(3R)-3-(tert-butoxycarbonylamino)-5-[(4-chlorophenyl)methyl]-8-methyl-4-oxo-2,3-dihydro-1,5-benzothiazepine-7-carbonyl]amino]carbamoyl]-3,3-difluoro-piperidine-1-carboxylate (200 mg, 0.256 mmol) and was obtained as a light yellow foam containing the desired product (333 mg, 156% yield). MS (ESI): 654.3 [M-isobutene-CO₂+H]⁺.

### Step c) benzyl 5-[5-[(3R)-3-(tert-butoxycarbonylamino)-5-[(4-chlorophenyl)methyl]-8-methyl-1,1,4-trioxo-2,3-dihydro-1λ⁶,5-benzothiazepin-7-yl]-1,3,4-oxadiazol-2-yl]-3,3-difluoro-piperidine-1-carboxylate

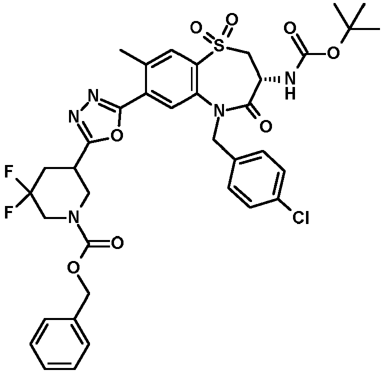

The title compound was prepared in analogy to general procedure **5** benzyl 5-[5-[(3R)-3-(tert-butoxycarbonylamino)-5-[(4-chlorophenyl)methyl]-8-methyl-4-oxo-2,3-dihydro-1,5-benzothiazepin-7-yl]-1,3,4-oxadiazol-2-yl]-3,3 -difluoro-piperidine-1-carboxylate (333 mg, 0.406 mmol) and was obtained as a light yellow oil (140 mg, 41% yield). MS (ESI): 730.3 [M-isobutene+H]⁺.

### Step d) tert-butyl N-[(3R)-5-[(4-chlorophenyl)methyl]-7-[5-(5,5-difluoro-3-piperidyl)-1,3,4-oxadiazol-2-yl]-8-methyl-1,1,4-trioxo-2,3-dihydro-1λ⁶,5-benzothiazepin-3-yl]carbamate

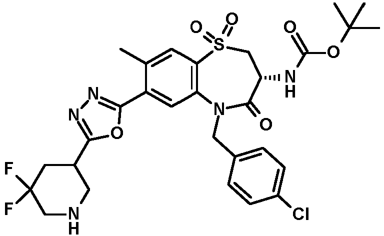

To a solution of benzyl 5-[5-[(3R)-3-(tert-butoxycarbonylamino)-5-[(4-chlorophenyl)methyl]-8-methyl-1,1,4-trioxo-2,3-dihydro-1λ⁶,5-benzothiazepin-7-yl]-1,3,4-oxadiazol-2-yl]-3,3-difluoropiperidine-1-carboxylate (131 mg, 0.167 mmol) in MeOH (2.5 mL) was added Pd/C 10% (CAS 7440-05-3) and the mixture was stirred under a hydrogen atmosphere at RT for 30 min. The mixture was filtered off, the solvent evaporated to afford the title compound as a colourless amorphous solid (92.2 mg, 73% yield), MS (ESI): 652.4 [M+H]⁺.

### Step e) tert-butylN-[(3R)-5-[(4-chlorophenyl)methyl]-7-[5-(5, 5-difluoro-1-methyl-3-piperidyl)-1,3,4-oxadiazol-2-yl]-8-methyl-1,1,4-trioxo-2,3-dihydro-1λ⁶,5-benzothiazepin-3-yl]carbamate

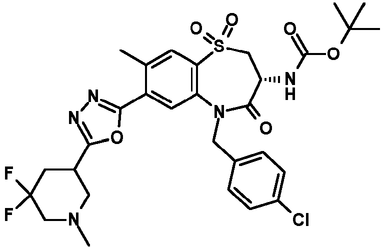

To a solution of tert-butyl N-[(3R)-5-[(4-chlorophenyl)methyl]-7-[5-(5,5-difluoro-3-piperidyl)-1,3,4-oxadiazol-2-yl]-8-methyl-1,1,4-trioxo-2,3-dihydro-1λ⁶,5-benzothiazepin-3-yl]carbamate (30 mg, 0.046 mmol) in MeOH (0.978 mL) were added formaldehyde in water (37% solution, CAS 50-00-0) (13.8 mg, 0.46 mmol) and sodium triacetoxyborohydride (CAS 56553-60-7) (263 mg, 0.46 mmol) and the reaction was stirred at RT for 1 h. Few drops of water were added to the reaction mixture and the mixture was purified by column chromatography on silica gel (heptane:EtOAc = 1:0 to 0:1) affording the title compound as a white solid (17.2 mg, 53% yield), MS (ESI): 666.4 [M+H]⁺.

### Step f) (3R)-3-amino-5-[(4-chlorophenyl)methyl]-7-[5-(5,5-difluoro-1-methyl-3-piperidyl)-1,3,4-oxadiazol-2-yl]-8-methyl-1,1-dioxo-2,3-dihydro-1λ⁶,5-benzothiazepin-4-one

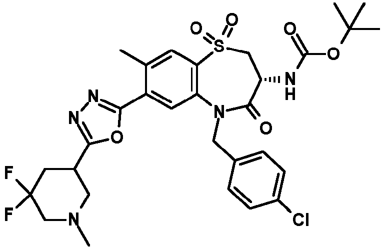

The title compound was prepared in analogy to general procedure **6d** tert- tert-butyl N-[(3R)-5-[(4-chlorophenyl)methyl]-7-[5-(5,5-difluoro-1-methyl-3-piperidyl)-1,3,4-oxadiazol-2-yl]-8-methyl-1,1,4-trioxo-2,3-dihydro-1λ⁶,5-benzothiazepin-3-yl]carbamate (17.2 mg, 0.026 mmol) and was obtained as a white solid (12 mg, 82% yield). MS (ESI): 566.2 [M +H]⁺.

### Example 525

### (3R)-3-amino-5-[(4-chlorophenyl)methyl]-7-(3-ethyl-1,2,4-triazol-1-yl)-8-fluoro-1,1-dioxo-2,3-dihydro-1λ⁶,5-benzothiazepin-4-one

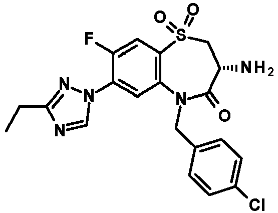

### Step a) (3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]boronic acid

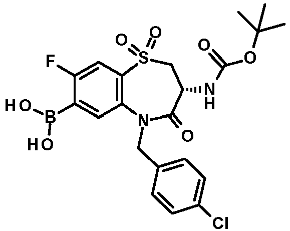

To a solution of (3R)-3-amino-7-bromo-5-[(4-chlorophenyl)methyl]-8-fluoro-1,1-dioxo-2,3-dihydro-1λ⁶,5-benzothiazepin-4-one (2.2 g, 4.0 mmol, 1 eq) in 1,4-Dioxane (50 mL) was added bis(pinacolato)diboron (2.44 g, 9.63 mmol, 2.4 eq), potassium acetate (1.22 g, 12.45 mmol, 3.1 eq) and [1,1'-bis(diphenylphosphino)ferrocene]dichloropalladium(II) (0.34 g, 0.470 mmol, 0.12 eq) under Ar atmosphere and stirred at 120 °C for 3 h. The mixture was poured into water and extracted three times with EOAc. The combined organic layers were washed with brine, dried over anhdydrous sodium sulfate and the solvent evaporated under reduced pressure. The remaining crude was purified by reverse phase prep-HPLC to afford (3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]boronic acid (1.01 g, 42% yield) as light yellow solid. MS (ESI): 457.2 [M-isobutene+H]⁺.

### Step b) tert-butyl N-[(3R)-5-[(4-chlorophenyl)methyl]-7-(3-ethyl-1,2, 4-triazol-1-yl)-8-fluoro-1,1,4-trioxo-2,3-dihydro-1λ⁶,5-benzothiazepin-3-yl]carbamate

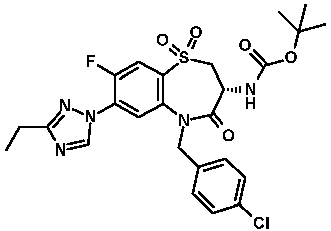

To a mixture of cupric acetate (187.52 mg, 1.03 mmol, 2.65 eq), NEt₃ (0.26 mL, 1.85 mmol, 4.75 eq) and 3-ethyl-1H-1,2,4-triazole (CAS:7411-16-7) (189 mg, 1.95 mmol, 5 eq) in MeCN (5 mL) was added a solution of (3R)-3-(tert-butoxycarbonylamino)-5-[(4-chlorophenyl)methyl]-8-fluoro-1,1,4-trioxo-2,3-dihydro-1λ⁶,5-benzothiazepin-7-yl]boronic acid (200 mg, 0.39 mmol) inMeCN (10 mL) in five portions. The reaction mixture was stirred at 20°C for 12 h, poured into water (10 mL), extracted with EtOAc (50 mL) and the combined organic layers were washed with brine (50 mL), dried over anhydrous Na₂SO₄ and concentrated under vacuum. The crude product was purified by prep-TLC (PE:EA = 2:1) affording the title compound (30 mg, 0.05 mmol, 9.9% yield) as a yellow solid. MS (ESI): 564.1 [M+H]⁺.

### Step c) (3R)-3-amino-5-[(4-chlorophenyl)methyl]-7-(3-ethyl-1,2,4-triazol-1-yl)-8-fluoro-1,1-dioxo-2,3-dihydro-1λ⁶,5-benzothiazepin-4-one

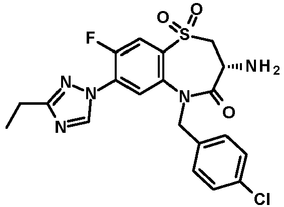

The title compound was prepared in analogy to general procedure **6f** (using HCl/EtOH instead of HCl/EtOAc) from tert-butyl N-[(3R)-5-[(4-chlorophenyl)methyl]-7-(3-ethyl-1,2,4-triazol-1-yl)-8-fluoro-1,1,4-trioxo-2,3-dihydro-1λ⁶,5-benzothiazepin-3-yl]carbamate (30 mg, 0.053 mmol) and was obtained as a white powder (2.9 mg, 11% yield). MS (ESI): 464.3 [M+H]⁺.

### Example 526

### (3R)-3-amino-7-(3-tert-butyl-1,2,4-triazol-1-yl)-5-[(4-chlorophenyl)methyl]-8-fluoro-1,1-dioxo-2,3-dihydro-1λ⁶,5-benzothiazepin-4-one

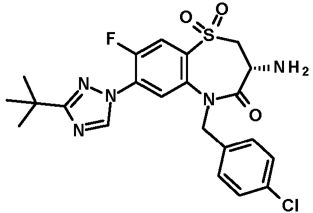

### Step a) (2S)-3-(2-acetamido-4,5-difluoro-phenyl)sulfanyl-2-(tert-butoxycarbonylamino)propanoic acid

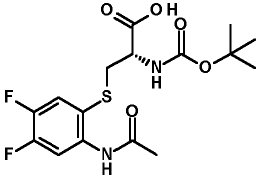

To a mixture of N-(2-bromo-4,5-difluoro-phenyl)acetamide (CAS:64695-81-4) (5.0 g, 20 mmol) and tBu₃P-Pd-G2 (CAS 1375325-71-5) (2.05 g, 4 mmol, 0.2 eq) in tert-amyl alcohol (70 mL) were added (2R)-2-(tert-butoxycarbonylamino)-3-sulfanyl-propanoic acid (CAS 20887-95-0) (4.87 g, 22 mmol, 1.1 eq) and cesium carbonate (9.77 g, 30 mmol, 1.5 eq). The reaction mixture was heated at 110°C and stirred for 12h under an atmosphere of nitrogen. The reaction mixture was poured into water (300 mL) and the pH was adjusted to 4 by addition of 1M aq HCl. The mixture was extracted with EtOAc (300 mL) and the organic phase was washed with brine (300 mL), dried over anhydrous Na₂SO₄ and concentrated in vacuum. The crude product was purified by prep-HPLC. The eluent was extracted with ethyl acetate (500 mL) affording the title compound (4.4 g, 49% yield) as a yellow solid. MS (ESI): 291.2 [M-isobutene-CO₂+H]⁺.

### Step b) (2S)-2-amino-3-(2-amino-4,5-difluorophenyl)sulfanyl-propanoic acid

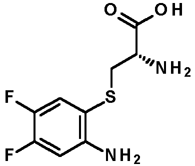

To a solution of (2S)-3-(2-acetamido-4,5-difluoro-phenyl)sulfanyl-2-(tert-butoxycarbonylamino)propanoic acid (4.3 g, 11.01 mmol, 1eq) in a solvent mixture of THF /EtOH (80 mL, 1:1) was added 2M aqueous HCl (200 mL, 400 mmol, 36 eq). The reaction mixture was heated to 80°C and stirred for 8h. The solvent was removed to afford the title compound (3 g, 98% yield) as a yellow solid, which was used without further purification in the next reaction step. MS (ESI): 249.1 [M+H]⁺.

### Step c) (2S)-3-(2-amino-4,5-difluorophenyl)sulfanyl-2-(tert-butoxycarbonylamino)propanoic acid

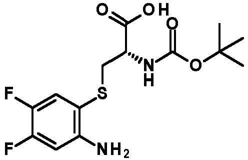

A mixture of (2S)-2-amino-3-(2-amino-4,5-difluoro-phenyl)sulfanyl-propanoic acid (3.0 g, 10.7 mmol, 1 eq), sodium hydrogen carbonate (2.46 g, 29.3 mmol, 2.73 eq) and di-tert-butyldicarbonate (2.55 g, 11.7 mmol, 1.09 eq) in THF (30 mL) and water (15 mL) was stirred at 20°C for 1h. The solution was poured into water (100 mL) and the mixture was extracted with EtOAc (200 mL). The layers were separated and the organic phase was washed with brine (100 mL), dried over anhydrous Na₂SO₄ and concentrated to afford the title compound (2.6 g, 53% yield) as a yellow solid, which was used without further purification in the next reaction step. MS (ESI): 293.0 [M-isobutene+H]⁺.

### Step d) tert-butyl N-[(3R)-7,8-difluoro-4-oxo-3,5-dihydro-2H-1,5-benzothiazepin-3-yl]carbamate

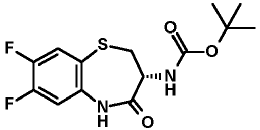

The title compound was prepared in analogy to general procedure **3** (in THF instead of DMF) from (2S)-3-(2-amino-4,5-difluoro-phenyl)sulfanyl-2-(tert-butoxycarbonylamino)propanoic acid (2.6 g, 5.97 mmol) and was obtained as a light yellow solid (1.02 g, 39% yield). MS (ESI): 275.0 [M-isobutene+H]⁺.

### Step e) tert-butylN-[(3R)-5-[(4-chlorophenyl)methyl]-7, 8-difluoro-4-oxo-2, 3-dihydro-1, 5-benzothiazepin-3yl]carbamate

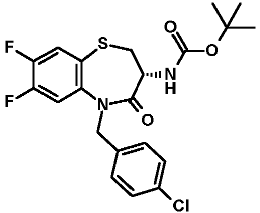

The title compound was prepared in analogy to general procedure **4** (in DMF instead of DMSO) from tert-butyl N-[(3R)-7,8-difluoro-4-oxo-3,5-dihydro-2H-1,5-benzothiazepin-3-yl]carbamate (1.0 g, 3.03 mmol) and was obtained as a light yellow solid (1.01 g, 63% yield). MS (ESI): 399.3 [M-isobutene+H]⁺.

### Step f) tert-butyl N-[(3R)-5-[(4-chlorophenyl)methyl]-7,8-difluoro-1,1,4-trioxo-2,3-dihydro-1λ⁶,5-benzothiazepin-3-yl]carbamate

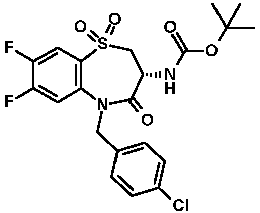

The title compound was prepared in analogy to general procedure **5** from tert-butyl N-[(3R)-5-[(4-chlorophenyl)methyl]-7,8-difluoro-4-oxo-2,3-dihydro-1,5-benzothiazepin-3-yl]carbamate (1.0 g, 2.2 mmol, 1 eq) and was obtained as a light yellow solid (1.01 g, 83% yield). MS (ESI): 431.0 [M-isobutene+H]⁺.

### Step g) tert-butyl N-[(3R)-7-(3-tert-butyl-1, 2, 4-triazol-1-yl)-5-[(4-chlorophenyl)methyl]-8-fluoro-1,1,4-trioxo-2,3-dihydro-1λ⁶,5-benzothiazepin-3-yl]carbamate

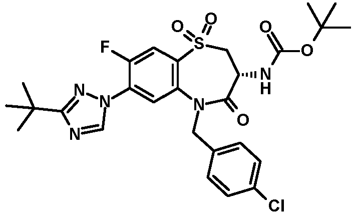

To a suspension of *tert-butyl* N-[(3R)-5-[(4-chlorophenyl)methyl]-7,8-difluoro-1,1,4-trioxo-2,3-dihydro-1λ⁶,5-benzothiazepin-3-yl]carbamate (100 mg, 0.21 mmol) and potassium carbonate (120 mg, 0.87 mmol, 4.23 eq) in DMSO (1 mL) was added 3-tert-butyl-4H-1,2,4-triazole (CAS:96440-78-7) (110 mg, 0.88 mmol, 4.28 eq) at 20°C and the mixture was stirred for 3h. The solution was poured into water (20 mL) and the mixture was extracted with EtOAc (50 mL). The layers were separated and the organic phase was washed with brine (50 mL), dried over anhydrous Na₂SO₄ and concentrated in vacuum. The crude product was purified by prep-TLC (PE:EA = 2:1) affording the title compound (51 mg, 42% yield) as a white solid. MS (ESI): 592.0 [M+H]⁺.

### Step h) (3R)-3-amino-7-(3-tert-butyl-1,2,4-triazol-1-yl)-5-[(4-chlorophenyl)methyl]-8-fluoro-1,1-dioxo-2,3-dihydro-1λ⁶,5-benzothiazepin-4-one

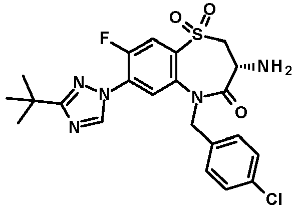

The title compound was prepared in analogy to general procedure **6b** from tert-butyl N-[(3R)-7-(3-tert-butyl-1,2,4-triazol-1-yl)-5-[(4-chlorophenyl)methyl]-8-fluoro-1,1,4-trioxo-2,3-dihydro-1λ⁶,5-benzothiazepin-3-yl]carbamate (40 mg, 0.07 mmol) and was obtained as a white solid (21.9 mg, 64% yield). MS (ESI): 492.1 [M+H]⁺.

Example 527 of the following table was prepared in analogy to Example 526, step g,h) using the appropriate imidazole building block.

| **Ex.** | **Structure** | **Systematic Name** | **Building Blocks** | **MS, ESI: m/z** |
|---|---|---|---|---|
| 527 | 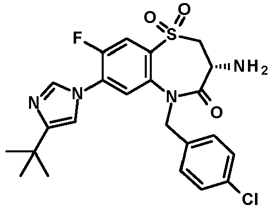 | (3R)-3-amino-7-(4-tert-butylimidazol-1-yl)-5-[(4-chlorophenyl)methyl]-8-fluoro-1,1-dioxo-2,3-dihydro-1λ⁶,5-benzothiazepin-4-one | 5-tert-Butyl-1H-imidazole (CAS 21149-98-4) | 491.3 [M+H]⁺ |
| | | (*) | | |

| | | | | |
|---|---|---|---|---|
| * as hydrochloride salt | | | | |

### 2) Biological examples

### 2.1) In vitro DGK Inhibition Assays

DGK α and ζ kinase use ATP to phosphorylate the substrate 1,2-dilauroyl-sn-glycerol (DLG, incorporated in the liposomes). ATP is converted to ADP as a result of this enzymatic reaction. After the kinase reaction, an ATP-depletion reagent is added to terminate the kinase reaction and deplete any remaining ATP, leaving only ADP. Second, a detection reagent is added to simultaneously convert ADP to ATP and allow the newly synthesized ATP to be converted to light using a coupled luciferase/luciferin reaction.

### Reagents and Material

### Buffer Ingredients (solutions & salts)

| **Chemicals** | **Vendor** | **Cat. Nr.:** |
|---|---|---|
| DTT | AppliChem | Cat# A3668 |
| BSA | Sigma - Aldrich | Cat# A2153-10G |
| MOPS | Sigma - Aldrich | Cat# M1254 |
| Sodium chloride (NaCl) | Sigma - Aldrich | Cat# S7663-1KG |
| Magnesium chloride (MgCl₂) | MERCK | Cat# 1.05833.0250 |
| Calcium chloride (CaCl₂) | Sigma - Aldrich | Cat# C4901-500G |

### Protein / Substrates / Tracer

| **Proteins / Substrate / Tracer** | **Vendor** | **Lot#; Probe#** |
|---|---|---|
| DGKA | In house production | hDGKalpha(1-735)-N-His |
| DGKZ | In house production | hDGKzeta(1-928)-N-His |
| 18:1 PS (DOPS) | Sigma - Aldrich | Cat# 840035C |
| 16:0-18:1 PC | Sigma - Aldrich | Cat# 850457C |
| 1,2-dilauroyl-sn-glycerol (DLG) | Sigma - Aldrich | Cat# 800812C |
| Ultra Pure ATP | Promega | Cat# V9101 Part No. V915A |
| ADP-Glo Reagent | Promega | Cat# V9101 Part No. V912A |
| Kinase Detection Substr. | Promega | Cat# V9101 Part No. V914A |
| Kinase Detection Buffer | Promega | Cat# V9101 Part No. V913A |

Full length DGKA and Z were expressed in Sf21 insect cells by infecting the cells with the baculovirus stock at MOI of 2. Purification of both enzymes was performed as previously described by Takahashi et al., PeerJ, 2018 (Takahashi, D.; Sakane, F. Expression and purification of human diacylglycerol kinase alpha from baculovirus-infected insect cells for structural studies. PeerJ 2018, 6, No. e5449*).*

### Hardware

| **Material** | **Vendor** | **Cat. Nr. / Volurne range** |
|---|---|---|
| 384 multiwell plate, white | Greiner | Cat# 781904 |
| 384 multiwell plate, white | Corning | Cat# CLS3574 |
| Matrix Multichannel Pipette | Thermo Fisher SCIENTIFIC | 2 -125 µl |
| Multidrop Combi | Thermo Fisher SCIENTIFIC | 0.5 - 50 µl/well |
| Envision plate reader | Perkin Elmer | |

### Assay Buffer (30ml)

| **Chemical** | **initial concentration (Stock)** | | **Working Solution (1time)** | | | **final AssaY concentrtion** | |
|---|---|---|---|---|---|---|---|
| | conc. | [mM, %....] | conc. | [mM, %....] | Volume [µl] | conc. | [mM,%... ,] |
| MOPS (pH 7.5) | 1000 | mM | 50 | mM | 1500 | 50 | mM |
| NaCl | 5000 | mM | 100 | mM | 600 | 100 | mM |
| MgCl₂ | 1000 | mM | 10 | mM | 300 | 10 | mM |
| CaCl₂ | 1 | mM | 0.001 | mM | 30 | 0.001 | mM |
| DTT | 1000 | mM | 1 | mM | 30 | 1 | mM |
| BSA | 10 | % | 0.01 | % | 30 | 0.01 | % |
| | | | | | | | |
| | | | | | | | |
| filled up with: | | | ddWater: | | 27510 | | |

### Assay procedure

A concentrated liposome solution was prepared in assay buffer without DTT and BSA: 2mM of DLG in 21 mM of total liposome (2 mM DLG / 8 mM PS / 11 mM PC). The reaction mixtures contain the assay buffer with a final DLG concentration of 125uM ATP concentrations of 25µM (for DGKA assay) or 50 µM (for DGKZ assay). The reactions were started by addition of DGK α and ζ kinases at 4 nM and 2 nM final concentrations, respectively. After 1 hour reaction, the amount of ADP formed was detected with the ADP-Glo kinase assay (Promega) according to the manufacturer instructions. Compounds were added in 11-points dose response, starting at 10mM, 1:3 dilutions, with a final DMSO concentration of 2%. The multidrop combi was used as a liquid handler and luminescence was read with 0.5 s by the envision reader (PE).

### Results

| **Example** | **DGKalpha ADP Glo assay IC50 (µM)** | **DGKzeta ADP Glo assay IC50 (µM)** |
|---|---|---|
| **8** | 0.284 | 0.224 |
| **28** | 0.513 | 0.672 |
| **29** | >62.50 | 33.089 |
| **43** | 0.275 | 0.344 |
| **47** | 0.943 | 0.062 |
| **48** | 0.403 | 0.114 |
| **54** | 1.993 | 4.093 |
| **57** | 0.21 | 0.254 |
| **62** | 0.452 | 0.622 |
| **63** | 0.2 | 0.126 |
| **86** | 0.643 | 0.685 |
| **87** | 3.06 | 3.666 |
| **90** | 2.977 | 4.459 |
| **184** | 0.244 | 0.29 |
| **196** | 0.216 | 0.231 |
| **204** | 0.068 | 0.07 |
| **205** | 0.299 | 0.147 |
| **207** | >62.50 | 0.193 |
| **208** | 2.641 | 0.147 |
| **209** | 1.487 | 0.408 |
| **210** | 1.619 | 0.36 |
| **211** | 1.29 | 0.33 |
| **212** | 0.975 | 0.046 |
| **213** | 2.136 | 0.052 |
| **214** | 0.214 | 0.1 |
| **215** | 0.662 | 0.886 |
| **244** | 0.836 | 1.101 |
| **253** | 0.198 | 0.105 |
| **266** | 54.48 | 40.085 |
| **289** | 0.587 | 0.482 |
| **292** | 0.528 | 0.494 |
| **293** | 1.153 | 1.203 |
| **297** | 0.373 | 0.357 |
| **300** | 3.935 | 0.481 |
| **303** | 0.301 | 0.251 |
| **310** | 2.766 | 0.098 |
| **311** | 43.696 | 0.069 |
| **312** | 0.454 | 0.029 |
| **313** | 1.659 | 0.13 |
| **314** | 6.146 | 0.15 |
| **322** | 17.831 | 0.933 |
| **331** | 0.857 | 0.176 |
| **345** | 0.343 | 0.207 |
| **348** | 2.662 | 0.374 |
| **350** | 1.613 | 0.235 |
| **357** | 0.403 | 0.114 |
| **358** | 2.699 | 0.122 |
| **361** | 0.876 | 0.933 |
| **373** | 1.405 | 0.644 |
| **378** | 4.057 | 1.436 |
| **379** | 42.677 | 1.305 |
| **381** | 3.309 | 0.658 |
| **384** | 4.579 | 2.252 |
| **386** | 3.569 | 0.778 |
| **390** | 38.547 | 2.628 |
| **391** | 0.773 | 0.993 |
| **406** | 5.018 | 13.982 |
| **407** | 1.419 | 2.661 |
| **408** | 4.767 | 0.881 |
| **409** | 2.871 | 0.795 |
| **411** | 2.089 | 0.746 |
| **412** | 2.693 | 0.484 |
| **413** | 1.547 | 1.981 |
| **416** | 1.448 | 1.676 |
| **417** | 6.406 | 2.114 |
| **420** | 0.806 | 0.454 |
| **430** | 0.467 | 0.594 |
| **431** | 0.42 | 0.683 |
| **437** | 0.528 | 3.679 |
| **438** | 0.797 | 1.345 |
| **442** | 1.387 | 0.285 |
| **444** | 6.112 | 1.906 |
| **452** | 0.340 | 0.400 |
| **459** | 0.625 | 0.632 |
| **466** | 2.091 | 1.032 |
| **500** | 1.734 | 1.827 |
| **517** | 0.272 | 0.316 |
| **518** | 0.442 | 0.639 |
| **521** | 3.002 | 0.338 |
| **522** | 4.07 | 0.529 |
| **523** | 1.294 | 1.512 |

### 2.2) IL2 secretion measurements

As readouts for T-cell activation, IL2 secretion after 24 hours and proliferation after 5 days was measured. Increases in IL2 secretion and proliferation upon compound treatment were assessed as the % of the maximum of reference compound **A1.** WO 2016/139181 discloses reference compound **A1** as example 70. As a counter screen and to make sure that no unwanted TCR-independent activation was triggered, PBS conditions were run for all compounds.

### Reagents and Material

| **Reagents** | **Information** | **Stock conc.** | **End conc:** |
|---|---|---|---|
| Compound dilution Buffer (CDB) DMSO | Sigma, #D2650 | >99% | 0.2% DMSO |
| RPMI + GlutaMAX | Gibco, #61870-010 | | |
| Human serum (HS) | Sigma, #H3667-100ML | 100% | 5% |
| Sodium Pyruvate (100 mM) | Gibco, #11360-039 | 100x | 1x |
| 2-Mercaptoethanol | Gibco, #31350-010 | 50m M | 50µM |
| Penicillin-Streptomycin (10,000 U/mL) | Life Technologies, #15140122 | 100X | 1X |
| Primary T Lymphocyte cells enriched from isolated Peripheral blood mononuclear cells (PBMCs) | Buffy coats for PBMCs ordered from Blutspendezentrum Basel-Stadt, RNCB ID: CL008438 | | 312'500 cells/cm2 in 96-well (100k/well) |
| Activator Control Ref cpd | reference compound A1, stock in DMSO, 0.2% DMSO final | 10m M | 20µM |
| Neutral Control DMSO | Sigma, #D2650 | 100% | 0.2% DMSO |

| Compounds | Stock in DMSO | 10m M | DR starting at 20µM; Dil. Factor 3.333; 5 steps |
|---|---|---|---|
| No stimulation Control PBS++ | PBS++, Gibco #14040-091 | 100% | 100% |
| Stimulation Control CD3 | CD3 Monoclonal Antibody (OKT3), Thermo Fisher #16-0037-81 | 1mg/ ml | Depending on donor (0.1 - 1.0ug/ml) |
| 96-well cell culture plates, Poly-D lysine coated | Corning, #354640 | | |
| Coating buffer for stim control CD3 | PBS++, Gibco #14040-091 | | |
| Washing buffer for coated plates and ELISA | PBS--, Gibco # 14190-094 | | |
| IL-2 Human ProQuantum Immunoassay Kit | Invitrogen, #A35603 | | |
| MicroAmp^{™} EnduraPlate^{™} Optical 384-Well for immunoassay | Applied Biosystems, #4483273 | | |
| LightCycler^{®} 480 Sealing Foil for immunoassay | Roche, #04729757001 | | |
| IL-2 Human Uncoated ELISA Kit | Thermo Fisher Scientific, #88-7025-88 | | |
| Tween 10% for ELISA | Bio-Rad, #161-0781 | 10% | 0.05% |
| Nunc MaxiSorp plates for ELISA | Thermo Fisher Scientific, #439454 | | |
| V-bottom plates for ELISA dilutions | Greiner Bio-One, #651201 | | |
| MACS filter (cell strainer) | Miltenyi Biotech, #130-041-407 | | |
| TopSeal-A Plus (sealing for sn collection) | Perkin Elmer, #6050185 | | |
| U-bottom plates for IL-2 / supernatant harvest | Costar, #3799 | | |
| LightCycler^{®} 480 Multiwell Plate 96 white, ProQuantum working plate | Roche, #04729692001 | | |

### Cell Culture

Expanded primary human T-cells were thawed and cultured in RPMI 1640 (Gibco, #61870-010) + 5% human serum (HS, Sigma, #H3667) + 1mM Sodium Pyruvate (Gibco, #11360-039) + 50µM 2-mercaptoethanol (Gibco, #31350-010) and 1x Pen-Strep (Life Technologies, #15140122) medium at density of 2 Mio/ml for 3 hours in 5% CO2, 37°C and 95% humidity. For coating of plates, PBS++ with PBS-- or PBS++ with CD3 antibody (concentration depending on donor and determined by CD3 titrations) was added 100µl/well to Poly-D Lysine coated 96-well plates. Plates were sealed and incubated at room temperature for 3 hours on a table-top rocking platform. After incubation, plates were washed once with PBS-- and filled with 40µl/well culture medium only. Compounds were then added (see next section) to medium only plates. After 3 hours of culturing the T-cells, cells were filtered through a cell strainer (Miltenyi Biotech, #130-041-407), counted again and concentration was adjusted to 1.25 Mio/ml. Cells were then seeded 80µl/well to the 40µl/well including dispensed compounds according to plate layout. By adding cells, compounds were further diluted 1:3, and resulting in 100k cells/120µl/well. After 24 hours 40µl of supernatant was collected carefully from the top while not disturbing the cells and transferred into a round bottom 96well plate. Collected and frozen supernatant was used for detection of IL2 using the IL-2 Human ProQuantum Immunoassay Kit (Invitrogen) or using the Human IL-2 ELISA Kit (Thermo Fisher).

### Compound treatment

Compounds were added in a 5 or 6pt dose response with the Tecan D300e Digital Dispenser, all conditions 3 times more concentrated than the end-concentration, since cells are added afterwards (80µl cells to 40µl prepared medium with treatment). The DR was starting at 20µM or 10µM final top concentration and a dilution factor of 3.333. The positive control was the reference compound A1 that was added in a dose response as well, additionally to 3 wells of only 20µM representing the positive stimulator control. All wells were normalized with DMSO to a final concentration of 0.6% (0.2% end-concentration).

### IL2 ProQuantum Immunoassay

The immunoassay is done following the manufacturer's manual (Invitrogen, #A35603). Additional information: For the immunoassay, MicroAmp^{™} EnduraPlate^{™} Optical 384-Well plates are used. Frozen supernatant is thawed and centrifuged for 5 minutes at 1000xg, both steps at 4°C. After centrifugation, required sample amount is taken from the top, and in a separate LightCycler V-bottom plate (working plate) diluted with assay dilution buffer, dilution factor depending on the PBS or CD3 condition but at least 1:3. IL-2 standard and blanks are prepared in the same V-bottom plate, standard with a range of 0.0128-5000pg/ml (extended version). After preparation, 5µl of sample dilutions or standard/blanks are transferred to the optical 384-well plate (assay plate) and the 10µl reaction protocol is being followed. For measurement, the QuantStudio 12K Flex system is used. Raw data is extracted and IL-2 concentrations are calculated with the Thermo Fisher online app (apps.thermofisher.com/apps/proquantum).

### IL2 Elisa

ELISA is done following the manufacturer's manual (Thermo Fisher Scientific, #88-7025-88). Additional information: For the ELISA Nunc MaxiSorp 96well plates are used. Frozen supernatant is thawed and centrifuged for 5 minutes at 1000xg, both steps at 4°C. After that, required sample amount is taken from the top, and in a separate V-bottom plate diluted with ELISA diluent, dilution factor depending on the PBS or CD3 condition. IL-2 standard and blanks are prepared in the same V-bottom plate. After preparation, 50µl of sample dilutions and 100µl of standard or blanks are transferred to the Nunc plates.

### Calculations and data reporting

CD3 and PBS plates were analysed separately in Genedata Screener using Roche Normalization PCT_POS_CTRL with DMSO set as Neutral Control and 20µM of the reference compound **A1** set as Stimulator Control/100%.

For CD3 conditions EC50 and Emax of the fitted sigmoidal curve were reported. If no curve could be fitted, the EC50 was reported as blank field and the Emax was based on individual data points. The Emax did not always correspond to the highest concentration tested (20 µM), which is why the concentration at which the maximum activation was achieved was also reported ("Concentration @ Max IL2 activation"). Compounds which activate unstimulated cells or compounds which negatively affected viability (see proliferation assay) were flagged.

### Results

| **Example** | **Tcell IL2 CD3 EC50 (nM)** | **Emax IL2 (% activation)** | **Concentration @ Max IL2 activation (nM)** |
|---|---|---|---|
| **1** | | 12 | 20,000 |
| **2** | | 41 | 1,800 |
| **3** | 11,959.5 | 65 | 20,000 |
| **4** | | 52 | 20,000 |
| **5** | 991.2 | 180 | 6,000 |
| **6** | >20,000.0 | 40 | 20,000 |
| **7** | | 144 | 20,000 |
| **8** | 93.8 | 110 | 6,000 |
| **9** | 1,316.80 | 75 | |
| **10** | 3,096.40 | 92 | |
| **11** | 1,301.40 | 95 | |
| **12** | 967 | 79 | |
| **13** | >10,000.0 | 76 | 10,000 |
| **14** | 917.1 | 86 | |
| **15** | 832.3 | 150 | |
| **16** | 2,825.50 | 102 | |
| **17** | 262.4 | 149 | |
| **18** | 910 | 88 | |
| **19** | 1,165.10 | 97 | |
| **20** | 845.5 | 81 | |
| **21** | 665.5 | 174 | |
| **22** | >10,000.0 | 42 | 10,000 |
| **23** | 3,786.10 | 109 | |
| **24** | 6,624.10 | 64 | |
| **25** | 1,319.20 | 185 | |
| **26** | >10,000.0 | 23 | 10,000 |
| **27** | 1,231.70 | 94 | |
| **28** | 1,109.00 | 74 | |
| **29** | 854.6 | 88 | |
| **30** | 39.8 | 93 | |
| **31** | 343.6 | 111 | |
| **32** | 7,627.90 | 150 | |
| **33** | 5,281.50 | 101 | |
| **34** | 5,866.50 | 66 | |
| **35** | 7,129.00 | 152 | |
| **36** | 472.5 | 74 | |
| **37** | >20,000.0 | 35 | 20,000 |
| **38** | >20,000.0 | 55 | 20,000 |
| **39** | 842.3 | 150 | 20,000 |
| **40** | | 55 | 6,000 |
| **41** | | 67 | 6,000 |
| **42** | | 75 | 6,000 |
| **43** | 2,319.30 | 131 | 20,000 |
| **44** | | 140 | 20,000 |
| **45** | | 118 | 20,000 |
| **46** | 686.4 | 71 | |
| **47** | 2,858.10 | 137 | |
| **48** | 2,327.9 | 151 | |
| **49** | 2,180.20 | 117 | 20,000 |
| **50** | | 35 | 20,000 |
| **51** | | 40 | 20,000 |
| **52** | 974.5 | 161 | 6,000 |
| **53** | <162.0 | 111 | 160 |
| **54** | | 85 | 6,000 |
| **55** | 53.2 | 135 | 1,800 |
| **56** | | 156 | 6,000 |
| **57** | 36.7 | 232 | 6,000 |
| **58** | 219.9 | 78 | |
| **59** | 79.9 | 64 | |
| **60** | 535.2 | 182 | |
| **61** | 1912.2 | 27 | 6,000 |
| **62** | 148.9 | 89 | |
| **63** | 67.1 | 178 | 160 |
| **64** | 4,345.20 | 52 | |
| **65** | 3,848.10 | 64 | |
| **66** | 1,333.30 | 137 | |
| **67** | 199.1 | 153 | 1,600 |
| **68** | >20,000.0 | 36 | 20,000 |
| **69** | 1,673.80 | 206 | 20,000 |
| **70** | 74.6 | 167 | 540 |
| **71** | >20,000.0 | 47 | 540 |
| **72** | 3,237.30 | 73 | 20,000 |
| **73** | 50.2 | 164 | 1,800 |
| **74** | 55.2 | 237 | 6,000 |
| **75** | 113.8 | 97 | 10,000 |
| **76** | 748.8 | 127 | 1,800 |
| **77** | 340.9 | 135 | 20,000 |
| **78** | 2,351.20 | 127 | 20,000 |
| **79** | | 95 | 20,000 |
| **80** | 176.4 | 212 | 1,800 |
| **81** | 1,272.20 | 128 | 20,000 |
| **82** | 631.4 | 171 | 20,000 |
| **83** | 469.9 | 62 | 1,800 |
| **84** | 450.5 | 245 | 6,000 |
| **85** | 470.3 | 89 | 6,000 |
| **86** | 4180.8 | 94 | 20,000 |
| **87** | 1,526.20 | 66 | |
| **88** | 688.9 | 64 | |
| **89** | 117.1 | 83 | |
| **90** | 818.6 | 94 | |
| **91** | 34.1 | 201 | |
| **92** | 144.9 | 89 | |
| **93** | 659.7 | 105 | |
| **94** | 161.2 | 88 | |
| **95** | 5,360.70 | 70 | |
| **96** | 603.8 | 61 | |
| **97** | 1,038.70 | 167 | |
| **98** | 71.2 | 205 | |
| **99** | 105.3 | 153 | |
| **100** | 484.3 | 153 | |
| **101** | 72.2 | 78 | |
| **102** | >10,000.0 | 27 | 10,000.00 |
| **103** | 583.2 | 63 | |
| **104** | 277.4 | 110 | |
| **105** | 1,173.10 | 112 | |
| **106** | 783.7 | 173 | |
| **107** | 188.3 | 274 | |
| **108** | 498.2 | 93 | |
| **109** | 1,498.40 | 78 | |
| **110** | 231.6 | 81 | |
| **111** | 3,132.60 | 60 | |
| **112** | 3,226.40 | 82 | |
| **113** | 100.2 | 91 | |
| **114** | 756.4 | 91 | |
| **115** | 2,214.20 | 135 | |
| **116** | 7,344.20 | 57 | |
| **117** | 3,571.60 | 67 | |
| **118** | 227.6 | 81 | |
| **119** | 1,214.00 | 79 | |
| **120** | 256 | 101 | |
| **121** | 6,373.20 | 98 | |
| **122** | 238.3 | 76 | |
| **123** | 1,650.10 | 89 | |
| **124** | 1,666.80 | 74 | |
| **125** | 1,436.10 | 86 | |
| **126** | 62.4 | 148 | |
| **127** | 231.7 | 136 | |
| **128** | 1,240.90 | 94 | |
| **129** | 1,903.20 | 50 | |
| **130** | 283.8 | 163 | |
| **131** | 376.4 | 150 | |
| **132** | 1,954.90 | 78 | |
| **133** | 1,185.10 | 84 | |
| **134** | 1,360.50 | 115 | |
| **135** | 2,903.70 | 97 | |
| **136** | 187 | 67 | |
| **137** | 486.7 | 99 | |
| **138** | 3,474.50 | 82 | |
| **139** | 3,998.50 | 77 | |
| **140** | 3,884.70 | 65 | |
| **141** | 254.6 | 59 | |
| **142** | 223.6 | 78 | |
| **143** | 1,271.10 | 74 | |
| **144** | 1,823.50 | 93 | |
| **145** | 404 | 93 | |
| **146** | 259.8 | 93 | |
| **147** | 348.4 | 77 | |
| **148** | 987.5 | 99 | |
| **149** | 4,229.80 | 85 | |
| **150** | 358 | 120 | |
| **151** | 93.4 | 149 | |
| **152** | 476.9 | 126 | |
| **153** | 245.9 | 141 | |
| **154** | 890.3 | 78 | |
| **155** | 778.1 | 66 | |
| **156** | 3,343.90 | 71 | |
| **157** | 2,102.20 | 102 | |
| **158** | 193.8 | 98 | |
| **159** | 218.4 | 127 | |
| **160** | 499.5 | 132 | |
| **161** | 1,150.20 | 46 | |
| **162** | 382.7 | 76 | |
| **163** | 99.7 | 55 | |
| **164** | 708.4 | 108 | |
| **165** | 457.7 | 74 | |
| **166** | 566.8 | 70 | |
| **167** | 121.8 | 71 | |
| **168** | 1,587.10 | 60 | |
| **169** | 3,344.40 | 84 | |
| **170** | 405.2 | 50 | |
| **171** | 75.7 | 54 | |
| **172** | 1,671.90 | 147 | |
| **173** | 2,179.70 | 95 | |
| **174** | 2,193.30 | 99 | |
| **175** | >10,000.0 | 48 | 10,000.00 |
| **176** | 2,505.10 | 79 | |
| **177** | 1,442.90 | 123 | |
| **178** | 333.4 | 98 | |
| **179** | 334.6 | 101 | |
| **180** | 2,379.30 | 107 | |
| **181** | 1,989.10 | 76 | |
| **182** | 1,594.10 | 75 | |
| **183** | 43 | 73 | |
| **184** | 108.7 | 84 | |
| **185** | 4,930.80 | 57 | |
| **186** | 157.8 | 66 | |
| **187** | 594.4 | 81 | |
| **188** | 3,183.60 | 73 | |
| **189** | >2,727.3 | 64 | 2,720 |
| **190** | 1,643.30 | 75 | |
| **191** | 11,389.60 | 85 | 20,000 |
| **192** | 124.6 | 183 | |
| **193** | 38.7 | 435 | |
| **194** | 40.7 | 135 | |
| **195** | 167 | 97 | |
| **196** | 36.1 | 105 | |
| **197** | 226.1 | 122 | |
| **198** | 562.1 | 96 | |
| **199** | >10,000.0 | 25 | 10,000 |
| **200** | 141.5 | 84 | |
| **201** | 827.9 | 155 | |
| **202** | 1,304.20 | 77 | |
| **203** | 1,581.10 | 167 | |
| **204** | 78.5 | 142 | |
| **205** | 1,052.40 | 112 | |
| **206** | 541.7 | 116 | |
| **207** | 4,379.2 | 131 | |
| **208** | 5,251.0 | 92 | |
| **209** | 5,813.3 | 84 | |
| **210** | 7,166.0 | 93 | |
| **211** | 793.1 | 203 | |
| **212** | 2,947.1 | 116 | |
| **213** | 3,271.5 | 203 | |
| **214** | 1,498.4 | 175 | |
| **215** | 5,360.30 | 174 | |
| **216** | 16,351.50 | 63 | 20,000 |
| **217** | 779.8 | 168 | 6,000 |
| **218** | 7,864.30 | 57 | |
| **219** | 3,212.50 | 90 | 20,000 |
| **220** | 237.7 | 220 | 20,000 |
| **221** | 382.2 | 46 | 20,000 |
| **222** | 534.8 | 61 | 6,000 |
| **223** | >20,000.0 | 65 | 6,000 |
| **224** | >20,000.0 | 34 | 20,000 |
| **225** | 4,374.50 | 85 | 20,000 |
| **226** | >20,000.0 | 33 | 20,000 |
| **227** | >20,000.0 | 48 | 20,000 |
| **228** | 5,203.50 | 96 | 20,000 |
| **229** | 2,442.70 | 110 | 20,000 |
| **230** | 104.6 | 119 | 20,000 |
| **231** | 295 | 109 | 20,000 |
| **232** | 37 | 93 | |
| **233** | 95 | 80 | |
| **234** | 5,337.10 | 72 | |
| **235** | 170 | 135 | |
| **236** | 3,228.90 | 88 | |
| **237** | 526.1 | 90 | |
| **238** | 769 | 27 | |
| **239** | 5,577.60 | 63 | |
| **240** | 6,389.70 | 65 | |
| **241** | >10,000.0 | 39 | 10,000 |
| **242** | 4,799.90 | 74 | |
| **243** | 918.3 | 257 | |
| **244** | 235.9 | 161 | |
| **245** | 92.1 | 99 | |
| **246** | 342 | 100 | |
| **247** | 320.5 | 48 | |
| **248** | 139.7 | 168 | |
| **249** | >10,000.0 | 41 | 740 |
| **251** | >10,000.0 | 10 | 2,720 |
| **252** | 1,155.60 | 49 | |
| **253** | <15.1 | 161 | |
| **254** | 59.5 | 134 | |
| **255** | 848.8 | 23 | |
| **256** | >20,000.0 | 44 | 20,000 |
| **257** | >20,000.0 | 76 | 160 |
| **258** | 10,765.10 | 57 | 20,000 |
| **259** | 2,099.30 | 61 | 6,000 |
| **260** | >20,000.0 | 25 | 20,000 |
| **261** | 2,687.80 | 108 | 20,000 |
| **262** | <162.0 | 62 | 1,800 |
| **263** | 1,396.70 | 26 | 6,000 |
| **264** | 8,103.00 | 35 | 20,000 |
| **265** | >20,000.0 | 25 | 20,000 |
| **266** | 214 | 120 | 1,800 |
| **267** | 5,442.00 | 86 | 20,000 |
| **268** | 7,825.00 | 57 | |
| **269** | >10,000.0 | 3 | 740 |
| **270** | 57.4 | 68 | |
| **271** | 4,814.70 | 65 | 20,000 |
| **272** | 1,703.00 | 73 | 20,000 |
| **273** | 7,206.10 | 103 | 20,000 |
| **274** | >20,000.0 | 69 | 20,000 |
| **275** | 9,150.30 | 55 | 20,000 |
| **276** | 1,062.60 | 58 | |
| **277** | 6,937.80 | 60 | |
| **278** | 176.8 | 135 | 1,800 |
| **279** | 470 | 63 | 1,800 |
| **280** | 852.5 | 56 | 1,800 |
| **281** | >10,000.0 | 50 | 10,000.00 |
| **282** | 3,714.60 | 103 | 20,000 |
| **283** | 1,009.40 | 81 | 6,000 |
| **284** | >20,000.0 | 30 | 20,000 |
| **285** | 9,365.80 | 56 | 20,000 |
| **286** | >20,000.0 | 46 | 20,000 |
| **287** | 19,263.30 | 47 | 20,000 |
| **288** | >10,000.0 | 16 | 10,000.00 |
| **289** | 158 | 99 | 6,000 |
| **290** | | 31 | 1,800 |
| **291** | 1,701.10 | 165 | |
| **292** | 125.5 | 154 | |
| **293** | 3,308.70 | 71 | |
| **294** | 373.5 | 176 | |
| **295** | 885 | 104 | |
| **296** | 709.6 | 112 | |
| **297** | 169 | 193 | |
| **298** | | 122 | 2,720 |
| **299** | 2,496.10 | 85 | |
| **300** | 243.2 | 182 | |
| **301** | 6,139.00 | 74 | |
| **302** | >10,000.0 | 37 | 2,720.00 |
| **303** | 1,073.80 | 101 | |
| **304** | 8,138.80 | 57 | |
| **305** | 1,811.70 | 115 | |
| **306** | 570.8 | 67 | |
| **307** | | 172 | 10,000 |
| **308** | >10,000.0 | 25 | 10,000 |
| **309** | 443.3 | 204 | |
| **310** | 3,237.90 | 109 | |
| **311** | 5,037.00 | 75 | |
| **312** | 225.6 | 437 | |
| **313** | 1,120.50 | 255 | |
| **314** | 234.3 | 115 | |
| **315** | 1,026.10 | 120 | |
| **316** | 678.9 | 273 | |
| **317** | 2,234.80 | 246 | |
| **318** | 550.2 | 116 | |
| **319** | 458.3 | 122 | |
| **320** | 3,205.80 | 125 | |
| **321** | 596.7 | 200 | |
| **322** | 740 | 77 | |
| **323** | 402.7 | 76 | |
| **324** | 4,372.90 | 132 | |
| **325** | 454.9 | 84 | |
| **326** | 703.3 | 93 | |
| **327** | 2,330.00 | 151 | |
| **328** | 1,078.10 | 116 | |
| **329** | 197.5 | 67 | |
| **330** | 1,825.60 | 108 | |
| **331** | 74.6 | 295 | |
| **332** | 746.5 | 142 | |
| **333** | 713.9 | 80 | |
| **334** | 239.4 | 50 | |
| **335** | 818.7 | 241 | |
| **336** | 7,020.30 | 140 | |
| **337** | 5,951.50 | 58 | |
| **338** | 1,576.40 | 158 | |
| **339** | 1,298.90 | 130 | |
| **340** | 3,078.50 | 119 | |
| **341** | >10,000.0 | 63 | 10,000 |
| **342** | >10,000.0 | 35 | 10,000 |
| **343** | 4,827.80 | 58 | |
| **344** | 472.7 | 175 | |
| **345** | | 230 | 2,720 |
| **346** | 5,725.00 | 119 | |
| **347** | 3,498.50 | 60 | |
| **348** | 1,711.30 | 142 | |
| **349** | 1,329.30 | 201 | |
| **350** | 311.6 | 354 | |
| **351** | 287.8 | 188 | |
| **352** | 5,743.30 | 175 | |
| **353** | 3,245.20 | 112 | |
| **354** | >10,000.0 | 38 | 10,000 |
| **355** | 6,048.50 | 71 | |
| **357** | 2,997.9 | 101 | |
| **358** | 9,453.50 | 122 | |
| **359** | 208.4 | 142 | 6,000 |
| **360** | 922.1 | 65 | 20,000 |
| **361** | 525.2 | 83 | 20,000 |
| **362** | 1,730.60 | 99 | |
| **363** | 2,173.60 | 80 | |
| **364** | 981.1 | 91 | |
| **365** | 956.8 | 65 | |
| **366** | 584.1 | 88 | |
| **367** | 576 | 49 | |
| **368** | 5,552.90 | 66 | |
| **369** | 1,699.70 | 85 | |
| **370** | 5,099.40 | 77 | |
| **371** | >10,000.0 | 43 | 10,000 |
| **372** | 1,289.60 | 128 | |
| **373** | 1,162.40 | 130 | |
| **374** | 15,079.60 | 65 | |
| **375** | 461 | 54 | |
| **376** | 3,198.30 | 89 | |
| **377** | 1,720.40 | 51 | |
| **378** | >10,000.0 | 37 | |
| **379** | 2,090.10 | 64 | |
| **380** | 819.3 | 137 | |
| **381** | 2,402.8 | 140 | |
| **382** | 1,683.70 | 101 | |
| **383** | 857.1 | 40 | |
| **384** | 791.9 | 86 | |
| **385** | 895 | 50 | |
| **386** | 4,874.8 | 72 | |
| **387** | 2,281.40 | 190 | |
| **388** | 331.3 | 115 | |
| **389** | >2,727.3 | 24 | 2,720 |
| **390** | >10,000.0 | 28 | 10,000 |
| **391** | 284.3 | 121 | |
| **392** | 567.5 | 99 | |
| **393** | 4,011.60 | 71 | |
| **394** | 3,085.50 | 74 | |
| **395** | 1,116.00 | 66 | |
| **396** | 936.6 | 128 | |
| **397** | 649.4 | 75 | |
| **398** | 878.9 | 117 | |
| **399** | 3,582.80 | 75 | |
| **400** | 4,052.60 | 75 | |
| **401** | 446.3 | 69 | |
| **402** | 1,636.80 | 124 | |
| **403** | 3,678.40 | 71 | |
| **404** | 8,110.80 | 117 | |
| **405** | 1,318.40 | 118 | |
| **406** | 2,506.60 | 76 | |
| **407** | >10,000.0 | 40 | 10,000 |
| **408** | >10,000.0 | 34 | 10,000 |
| **409** | | 65 | 10,000 |
| **410** | 7,127.90 | 62 | |
| **411** | >10,000.0 | 32 | 2,720 |
| **412** | 2,356.20 | 124 | |
| **413** | 1,079.10 | 160 | |
| **414** | 4,386.90 | 62 | |
| **415** | 443.4 | 77 | |
| **416** | 369.4 | 65 | |
| **417** | 327.5 | 74 | |
| **418** | 5,736.70 | 63 | |
| **419** | 1,508.60 | 127 | |
| **420** | 1,352.30 | 135 | |
| **421** | 3,244.40 | 82 | |
| **422** | 835.2 | 89 | |
| **423** | 1,498.80 | 74 | |
| **424** | 69.4 | 66 | |
| **425** | >10,000.0 | 36 | 2,720 |
| **426** | 1,290.70 | 113 | |
| **427** | >10,000.0 | 46 | 10,000 |
| **428** | 1,575.50 | 71 | |
| **429** | 959.9 | 96 | |
| **430** | 24.2 | 83 | |
| **431** | 4,083.40 | 82 | |
| **432** | 2,702.20 | 46 | |
| **433** | 5,603.60 | 50 | |
| **434** | 6,162.30 | 162 | |
| **435** | 3,280.70 | 138 | |
| **436** | 4,225.10 | 91 | |
| **437** | 1,401.50 | 125 | |
| **438** | 700.5 | 111 | |
| **439** | 2,103.90 | 260 | |
| **440** | 2,505.60 | 111 | |
| **441** | 3,850.80 | 144 | |
| **442** | 148.1 | 186 | |
| **443** | >10,000.0 | 111 | 10,000 |
| **444** | >10,000.0 | 50 | 10,000 |
| **445** | 9,559.30 | 54 | |
| **446** | 7,566.60 | 72 | |
| **447** | 2,539.50 | 52 | |
| **448** | 4,624.90 | 138 | |
| **450** | 73.1 | 122 | 540 |
| **451** | 152.9 | 160 | 10,000 |
| **452** | 121.3 | 102 | 740 |
| **453** | 178.1 | 87 | 2,720 |
| **454** | 97.7 | 111 | 740 |
| **455** | 225.2 | 101 | 2,720 |
| **456** | 606.8 | 100 | |
| **457** | 4,876.90 | 63 | |
| **458** | 543 | 131 | |
| **459** | 507.9 | 122 | |
| **460** | 508.6 | 49 | |
| **461** | 455.3 | 122 | |
| **462** | 986 | 99 | |
| **463** | 577.3 | 92 | |
| **464** | 1,132.00 | 115 | |
| **465** | 1,172.90 | 82 | |
| **466** | 458 | 152 | |
| **467** | 474.5 | 90 | |
| **468** | 3,781.70 | 89 | |
| **469** | 2,551.80 | 155 | |
| **470** | 551.3 | 55 | |
| **471** | >10,000.0 | 31 | 10,000 |
| **472** | 108.8 | 122 | |
| **473** | 2,607.40 | 132 | |
| **474** | 251.3 | 165 | |
| **475** | 4,243.10 | 74 | |
| **476** | 8,679.00 | 105 | |
| **477** | 2,703.00 | 95 | |
| **478** | 831.6 | 95 | |
| **479** | 109.7 | 81 | |
| **480** | <15.1 | 91 | |
| **481** | 1,882.80 | 78 | |
| **482** | 1,247.50 | 106 | |
| **483** | 2, 601.10 | 85 | |
| **484** | 1,885.90 | 70 | |
| **485** | 8,129.20 | 129 | |
| **486** | 6,687.50 | 66 | |
| **487** | 8,604.00 | 97 | |
| **488** | 254.4 | 103 | |
| **489** | 4,057.70 | 66 | |
| **490** | 3,760.40 | 82 | |
| **491** | 443.2 | 150 | |
| **492** | 638.3 | 76 | |
| **493** | 1,279.70 | 89 | |
| **494** | 1,195.60 | 134 | |
| **495** | 798.7 | 171 | |
| **496** | >10,000.0 | 17 | 2,720 |
| **497** | 1,721.60 | 139 | |
| **498** | 724.3 | 91 | |
| **499** | 2,227.30 | 102 | |
| **500** | 911.3 | 150 | |
| **501** | 3,119.50 | 113 | |
| **502** | >10,000.0 | 29 | |
| **503** | 3,089.10 | 84 | |
| **504** | 1,226.60 | 104 | |
| **505** | 190.9 | 111 | |
| **506** | 1,321.00 | 95 | |
| **507** | 8,042.90 | 58 | |
| **508** | 458.8 | 65 | 10,000 |
| **509** | 290.2 | 123 | 2,720 |
| **510** | 91.7 | 102 | |
| **511** | 7,822.80 | 135 | |
| **512** | 992.8 | 267 | |
| **513** | 306.7 | 156 | |
| **514** | 1,921.40 | 91 | |
| **515** | 4,072.70 | 160 | |
| **516** | 2,738.90 | 97 | |
| **517** | 299.4 | 78 | |
| **518** | 134.8 | 114 | |
| **519** | 5,527.10 | 61 | |
| **520** | 1,150.50 | 146 | |
| **521** | 783.1 | 149 | |
| **522** | 619.4 | 135 | |
| **523** | 5,277.70 | 26 | |
| **524** | >10,000.0 | 31 | |
| **525** | 3,512.80 | 80 | |
| **526** | | 165 | 10,000 |
| **527** | 292.3 | 74 | |

### 2.1) Proliferation assay

### Reagents and Material

| **Reagents** | **Information** | **Stock a conc.** | **End conc.** |
|---|---|---|---|
| Compound dilution Buffer (CDB) DMSO | Sigma, #D2650 | >99% | 0.2% DMSO |
| RPMI + GlutaMAX | Gibco, #61870-010 | | |
| Human serum (HS) heat inactivated | Sigma, #H3667-100ML | | 5% |
| Sodium Pyruvate (100 mM) | Gibco, #11360-039 | 100x | 1x |
| 2-Mercaptoethanol | Gibco, #31350-010 | 50m M | 50µM |
| Penicillin-Streptomycin (10,000 U/mL) | Life Technologies, #15140122 | 100X | 1X |
| Primary T Lymphocyte cells enriched from isolated Peripheral blood mononuclear cells (PBMCs) | Buffy coats for PBMCs ordered from Blutspendezentrum Basel-Stadt, RNCB ID: CL008438 | | 312'500 cells/cm2 in 96-well (100k/well) |
| Activator Control Ref cpd | reference compound A1, stock in DMSO, 0.2% DMSO final | 10m M | 20µM |
| Neutral Control DMSO | Sigma, #D2650 | | 0.2% DMSO |
| Compounds | Stock in DMSO | 10m M | DR starting at 20µM; Dil. Factor 3.333; 5 steps |
| No stimulation Control PBS++ | PBS++, Gibco #14040-091 | 100% | 100% |
| Stimulation Control CD3 | CD3 Monoclonal Antibody (OKT3), Thermo Fisher #16-0037-81 | 1mg/ ml | Depending on donor (0.1-1.0ug/ml) |
| 96-well cell culture plates, Poly-D lysine coated | Corning, #354640 | | |
| Coating buffer for stim control CD3 | PBS++, Gibco #14040-091 | | |
| Washing buffer for coated plates | PBS--, Gibco # 14190-094 | | |
| CellTiter-Glo^{®} Luminescent Cell Viability Assay | Promega, #G7572 | 2x | 1x |
| MACS filter (cell strainer) | Miltenyi Biotech, #130-041-407 | | |
| TopSeal-A Plus (sealing for coating) | Perkin Elmer, #6050185 | | |
| Backing tape (for measurement) | Perkin Elmer, #6005199 | | |

Expanded primary human T-cells are thawed and cultured in RPMI 1640 (Gibco, #61870-010) + 5% human serum (HS, Sigma, #H3667) + 1mM Sodium Pyruvate (Gibco, #11360-039) + 50µM 2-mercaptoethanol (Gibco, #31350-010) and 1x Pen-Strep (Life Technologies, #15140122) medium at density of 2 Mio/ml for 3 hours in 5% CO2, 37°C and 95% humidity. For coating of plates, PBS++ only or PBS++ with CD3 antibody (concentration depending on donor and determined by CD3 titrations) is added 100µl/well to Poly-D Lysine coated 96-well plates. Plates are sealed and incubated at room temperature for 3 hours on a table-top rocking platform. After incubation, plates are washed once with PBS-- and filled with 40µl/well culture medium only. Compounds are then added (see next section) to medium only plates. After 3 hours of culturing the T-cells, cells are filtered through a cell strainer (Miltenyi Biotech, #130-041-407), counted again and concentration is adjusted to 1.25 Mio/ml. Cells are then seeded 80µl/well to the 40µl/well including dispensed compounds according to plate layout. By adding cells, compounds are further diluted 1:3, and resulting in 100k cells/120µl/well. After 48 hours 40µl of supernatant is collected carefully from the top while not disturbing the cells. Cells are assessed for proliferation 5 days later by measuring ATP consumption using CellTiterGlo (Promega).

### Compound treatment

Compounds were added in a 5 or 6pt dose response with the Tecan D300e Digital Dispenser, all conditions 3 times more concentrated than the end-concentration, since cells are added afterwards (80µl cells to 40µl prepared medium with treatment). The DR was starting at 20µM or 10µM final top concentration and a dilution factor of 3.333. The positive control was the reference compound **A1** that was added in a dose response as well, additionally to 3 wells of only 20µM representing the positive stimulator control. All wells were normalized with DMSO to a final concentration of 0.6% (0.2% end-concentration).

### Cell Titer Glo Measurements

After 5 days, for detection of ATP which is directly proportional to the number of cells present per well, the CellTiter-Glo^{®} 2.0 Reagent is used. After visual control for toxicity or precipitations of the tested compounds, the plates are equilibrated to room temperature for 45 minutes. CellTiter-Glo^{®} 2.0 Reagent is equilibrated to room temperature as well. After equilibration, an equal amount of CellTiter-Glo reagent is added to the cells (80µl/well) with an electronic multichannel pipette. Plates are placed on a rocking platform for 15 minutes at room temperature. After incubation, the bottom of the plates is sealed with backing tape. Luminescence is measured with PHERAstar FSX (interval time 0.5sec, gain 3000, focal height 15mm) and exported as CSV file for analysis in Genedata screener.

### Calculations and data reporting

CD3 and PBS plates were analysed separately in Genedata Screener using Roche Normalization PCT_POS_CTRL with DMSO set as Neutral Control and 20µM of the reference compound **A1** set as Stimulator Control/100%. For CD3 conditions EC50 and Emax of the fitted sigmoidal curve were reported. If no curve could be fitted, the EC50 was reported as blank field and the Emax was based on individual data points. The Emax did not always correspond to the highest concentration tested (20µM), which is why the concentration at which the maximum proliferation was achieved was also reported ("Concentration @ Max proliferation"). Compounds which activate unstimulated cells (see IL2 measurements) or compounds which negatively affected viability were flagged.

### Results

| **Example** | **Tcell Proliferation CD3 EC50 (nM)** | **Emax proliferation (% activation)** | **Concentration @ Max proliferation (nM)** |
|---|---|---|---|
| **1** | >20,000.0 | 33 | 20,000 |
| **2** | >20,000.0 | 34 | 1,800 |
| **3** | 4,299.4 | 68 | 20,000 |
| **4** | 1,050.1 | 168 | 6,000 |
| **5** | 2,141.7 | 154 | 20,000 |
| **6** | >20,000.0 | 39 | 20,000 |
| **7** | 2,748.6 | 264 | 20,000 |
| **8** | 84.5 | 130 | |
| **9** | 4,662.90 | 63 | |
| **10** | 4,035.00 | 78 | |
| **11** | 1,356.20 | 107 | |
| **12** | 1,016.80 | 84 | |
| **13** | 3,509.10 | 87 | |
| **14** | 810 | 131 | |
| **15** | 2,436.90 | 109 | |
| **16** | 3,658.20 | 65 | |
| **17** | 166.6 | 239 | |
| **18** | 295.5 | 136 | |
| **19** | 1,292.20 | 58 | |
| **20** | 567.1 | 111 | |
| **21** | 292.9 | 127 | |
| **22** | >10,000.0 | 74 | |
| **23** | 588.2 | 161 | |
| **24** | 2,017.80 | 95 | |
| **25** | 248.8 | 132 | |
| **26** | 6,392.10 | 64 | |
| **27** | 2,053.30 | 103 | |
| **28** | 1,261.30 | 129 | |
| **29** | 93.6 | 172 | |
| **30** | 43.5 | 117 | |
| **31** | 275.6 | 97 | |
| **32** | 808.5 | 101 | |
| **33** | 1,901.40 | 104 | |
| **34** | 2,802.90 | 100 | |
| **35** | 1,241.40 | 125 | |
| **36** | 403.7 | 86 | |
| **37** | 15,303.2 | 57 | 20,000 |
| **38** | >20,000.0 | 50 | 20,000 |
| **39** | 485.7 | 161 | 20,000 |
| **40** | >20,000.0 | 34 | 20,000 |
| **41** | 9,356.4 | 61 | 20,000 |
| **42** | 1,308.1 | 80 | 20,000 |
| **43** | 265.2 | 143 | 6,000 |
| **44** | 701.7 | 122 | 20,000 |
| **45** | 1969.9 | 80 | 20,000 |
| **46** | 1,819.40 | 94 | |
| **47** | 561.1 | 142 | |
| **48** | 3,189.0 | 248 | 10,000 |
| **49** | 585.3 | 112 | 6,000 |
| **50** | >20,000.0 | 40 | 20,000 |
| **51** | 2,691.6 | 94 | 20,000 |
| **52** | 1,094.9 | 133 | 60,000 |
| **53** | <162.0 | 243 | 6,000 |
| **54** | 1,120.6 | 116 | 6,000 |
| **55** | 50.7 | 282 | 1,800 |
| **56** | 414.4 | 136 | 20,000 |
| **57** | 32.7 | 231 | 1,800 |
| **58** | 684.5 | 126 | |
| **59** | 54.5 | 94 | |
| **60** | 320.1 | 110 | |
| **61** | 624.7 | 144 | 20,000 |
| **62** | 721.3 | 88 | |
| **63** | 48.8 | 141 | |
| **64** | >10,000.0 | 36 | 10,000 |
| **65** | 1,055.80 | 77 | |
| **66** | 2,069.60 | 112 | |
| **67** | 112 | 165 | 20,000 |
| **68** | 5,140.0 | 91 | 20,000 |
| **69** | 4,767.7 | 171 | 20,000 |
| **70** | 213.5 | 255 | 1,800 |
| **71** | <162.0 | 71 | 1,800 |
| **72** | 1,138.7 | 124 | 20,000 |
| **73** | 125.6 | 194 | 6,000 |
| **74** | 48.7 | 180 | 6,000 |
| **75** | 35.1 | 161 | 20,000 |
| **76** | 1,079.8 | 173 | 20,000 |
| **77** | | 183 | 20,000 |
| **78** | 1,108.4 | 86 | 20,000 |
| **79** | 172.9 | 154 | 20,000 |
| **80** | 81.5 | 278 | 6,000 |
| **81** | 520.7 | 185 | 6,000 |
| **82** | 728.1 | 148 | 20,000 |
| **83** | >20,000.0 | 27 | 6,000 |
| **84** | 226.5 | 191 | 1,800 |
| **85** | 236.6 | 100 | 1,800 |
| **86** | 777.3 | 95 | 6,000 |
| **87** | 2,737.00 | 82 | |
| **88** | 242 | 97 | |
| **89** | 615.2 | 104 | |
| **90** | 2,342.40 | 96 | |
| **91** | 124.6 | 139 | |
| **92** | 195.9 | 141 | |
| **93** | 1,753.10 | 100 | |
| **94** | 18.1 | 85 | |
| **95** | 6,155.70 | 67 | |
| **96** | >2,727.3 | 46 | 2,720 |
| **97** | 519.9 | 115 | |
| **98** | 64.6 | 112 | |
| **99** | 82.2 | 112 | |
| **100** | 49.6 | 89 | |
| **101** | 49.1 | 121 | |
| **102** | 7,691.80 | 65 | |
| **103** | 213.4 | 102 | |
| **104** | 149.8 | 112 | |
| **105** | 4,163.80 | 31 | |
| **106** | 253.9 | 111 | |
| **107** | 955.4 | 134 | |
| **108** | 1,526.30 | 117 | |
| **109** | 1,744.40 | 86 | |
| **110** | 125.1 | 131 | |
| **111** | 556 | 97 | |
| **112** | 1,048.60 | 124 | |
| **113** | 107.3 | 106 | |
| **114** | >10,000.0 | 65 | 10,000 |
| **115** | 495.2 | 127 | |
| **116** | 4,642.70 | 84 | |
| **117** | 5,017.80 | 68 | |
| **118** | 668.8 | 99 | |
| **119** | 730 | 97 | |
| **120** | 128.7 | 96 | |
| **121** | 4,759.80 | 72 | |
| **122** | 781.5 | 92 | |
| **123** | >2,727.3 | 82 | 2,720 |
| **124** | 3,228.50 | 98 | |
| **125** | 586.6 | 112 | |
| **126** | 113.2 | 135 | |
| **127** | 181.3 | 110 | |
| **128** | 533.6 | 99 | |
| **129** | 1,419.40 | 106 | |
| **130** | 159 | 114 | |
| **131** | 347.4 | 192 | |
| **132** | 149.8 | 97 | |
| **133** | 324 | 144 | |
| **134** | >10,000.0 | 34 | 10,000 |
| **135** | 1,045.20 | 93 | |
| **136** | 114.1 | 99 | |
| **137** | 309.4 | 90 | |
| **138** | 1,513.50 | 120 | |
| **139** | 3,402.30 | 102 | |
| **140** | 5,351.60 | 70 | |
| **141** | 767.7 | 91 | |
| **142** | 137.9 | 66 | |
| **143** | 2,389.60 | 77 | |
| **144** | 6,154.40 | 106 | |
| **145** | 428.2 | 107 | |
| **146** | 188.6 | 90 | |
| **147** | 343 | 117 | |
| **148** | >10,000.0 | 48 | 10,000 |
| **149** | 2,559.00 | 57 | |
| **150** | 75.7 | 128 | |
| **151** | 45.9 | 112 | |
| **152** | 549.4 | 78 | |
| **153** | 111.9 | 150 | |
| **154** | 1,718.00 | 86 | |
| **155** | 4,179.30 | 72 | |
| **156** | 8,132.00 | 135 | |
| **157** | 1,009.40 | 106 | |
| **158** | 2,423.80 | 141 | |
| **159** | 183.8 | 121 | |
| **160** | 381.8 | 96 | |
| **161** | 5,480.20 | 73 | |
| **162** | 627.8 | 84 | |
| **163** | 51.4 | 118 | |
| **164** | 291.7 | 82 | |
| **165** | 308.8 | 85 | |
| **166** | 904.5 | 104 | |
| **167** | 208.3 | 100 | |
| **168** | 4,951.20 | 63 | |
| **169** | 2,468.80 | 95 | |
| **170** | 1,498.40 | 97 | |
| **171** | 55.5 | 93 | |
| **172** | 2,942.40 | 190 | |
| **173** | 595.6 | 89 | |
| **174** | 4,463.00 | 93 | |
| **175** | 7,035.80 | 62 | |
| **176** | 4,279.50 | 70 | |
| **177** | 788.4 | 68 | |
| **178** | 206 | 105 | |
| **179** | 1,581.60 | 80 | |
| **180** | 5,706.90 | 103 | |
| **181** | 7,834.30 | 107 | |
| **182** | >10,000.0 | 38 | 2,720 |
| **183** | 336 | 131 | |
| **184** | 24.2 | 77 | |
| **185** | >10,000.0 | 13 | 10,000 |
| **186** | 710.4 | 94 | |
| **187** | 1,538.50 | 92 | |
| **188** | >10,000.0 | 29 | 10,000 |
| **189** | 679.7 | 134 | |
| **190** | 1,161.40 | 114 | |
| **191** | 1,278.3 | 184 | 20,000 |
| **192** | 100.3 | 110 | |
| **193** | 21.3 | 108 | |
| **194** | 15.2 | 79 | |
| **195** | 105.5 | 108 | |
| **196** | 16.6 | 81 | |
| **197** | 117.9 | 112 | |
| **198** | 331.6 | 112 | |
| **199** | 2,956.20 | 71 | |
| **200** | 37.9 | 88 | |
| **201** | 228.6 | 156 | |
| **202** | 1,425.40 | 57 | |
| **203** | 232.8 | 99 | |
| **204** | 34.8 | 154 | |
| **205** | 2,121.70 | 119 | |
| **206** | 157 | 168 | |
| **207** | 1,859.9 | 280 | |
| **208** | 5,308.30 | 106 | |
| **209** | 4,096.40 | 92 | |
| **210** | 1,554.20 | 89 | |
| **211** | 326.3 | 162 | |
| **212** | 337.7 | 178 | |
| **213** | 1,386.4 | 222 | |
| **214** | 2,135.7 | 275 | |
| **215** | 2,166.30 | 153 | |
| **216** | >20,000.0 | 41 | 20,000 |
| **217** | 2,114.4 | 221 | 20,000 |
| **218** | 3,886.40 | 80 | |
| **219** | 2,740.9 | 105 | 20,000 |
| **220** | 564.4 | 102 | 540 |
| **221** | 254.8 | 107 | 6,000 |
| **222** | 234.8 | 119 | 1,800 |
| **223** | <162.0 | 128 | 1,800 |
| **224** | >20,000.0 | 31 | 20,000 |
| **225** | 1,887.2 | 109 | 6,000 |
| **226** | >20,000.0 | 25 | 20,000 |
| **227** | >20,000.0 | 39 | 20,000 |
| **228** | 1,735.3 | 112 | 6,000 |
| **229** | 1,677.8 | 116 | 6,000 |
| **230** | 89.3 | 119 | 160 |
| **231** | 257.2 | 114 | 540 |
| **232** | 57.6 | 127 | |
| **233** | 86.6 | 127 | |
| **234** | 1,196.30 | 130 | |
| **235** | 46.6 | 108 | |
| **236** | 3,235.60 | 98 | |
| **237** | 1,300.40 | 106 | |
| **238** | 3,073.80 | 102 | |
| **239** | 5,098.50 | 69 | |
| **240** | 5,552.70 | 74 | |
| **241** | 2,940.10 | 73 | |
| **242** | 3,396.40 | 92 | |
| **243** | 246.4 | 129 | |
| **244** | 137.3 | 102 | |
| **245** | 102.3 | 127 | |
| **246** | 441.6 | 110 | |
| **247** | 760.8 | 79 | |
| **248** | 440 | 157 | |
| **249** | 1,238.60 | 161 | |
| **251** | 5,302.00 | 66 | |
| **252** | 4,818.80 | 72 | |
| **253** | 40.3 | 116 | |
| **254** | <15.1 | 95 | |
| **255** | 4,834.20 | 78 | |
| **256** | >20,000.0 | 3 | 20,000 |
| **257** | <162.0 | 142 | 1,800 |
| **258** | 585.9 | 124 | 1,800 |
| **259** | 345 | 159 | 1,800 |
| **260** | 1,822.6 | 82 | 20,000 |
| **261** | 427.8 | 115 | 6,000 |
| **262** | <162.0 | 101 | 20,000 |
| **263** | <162.0 | 185 | 6,000 |
| **264** | 2,703.8 | 106 | 20,000 |
| **265** | 884 | 143 | 6,000 |
| **266** | 77.5 | 137 | 740 |
| **267** | 3,071.6 | 74 | 20,000 |
| **268** | 248.7 | 95 | |
| **269** | 5,835.50 | 67 | |
| **270** | 39.7 | 111 | |
| **271** | 3,189.4 | 91 | 20,000 |
| **272** | 790.1 | 102 | 6,000 |
| **273** | 3,189.4 | 61 | 6,000 |
| **274** | >20,000.0 | 10 | 20,000 |
| **275** | 5,492.9 | 108 | 20,000 |
| **276** | 2,731.70 | 96 | |
| **277** | 8,219.70 | 59 | |
| **278** | 1,077.4 | 102 | 6,000 |
| **279** | 5,337.7 | 90 | 20,000 |
| **280** | 19,489.1 | 52 | 20,000 |
| **281** | 1,039.80 | 89 | |
| **282** | 5,492.9 | 96 | 20,000 |
| **283** | 5,492.9 | 67 | 20,000 |
| **284** | 5,492.9 | 30 | 6,000 |
| **285** | 5,492.9 | 60 | 20,000 |
| **286** | 337.5 | 93 | 6,000 |
| **287** | 2210.7 | 66 | 20,000 |
| **288** | 4,313.50 | 71 | |
| **289** | 187.4 | 124 | 540 |
| **290** | 743.8 | 139 | 20,000 |
| **291** | 445.7 | 104 | |
| **292** | 31.9 | 179 | |
| **293** | 2,069.70 | 92 | |
| **294** | 261.2 | 143 | |
| **295** | 1,393.30 | 97 | |
| **296** | 687.2 | 66 | |
| **297** | 219 | 157 | |
| **298** | | 162 | 2,720 |
| **299** | 5,006.90 | 111 | |
| **300** | 126.6 | 193 | |
| **301** | 2,440.90 | 93 | |
| **302** | 990.5 | 76 | |
| **303** | 605.3 | 140 | |
| **304** | 7,494.30 | 61 | |
| **305** | 719.6 | 84 | |
| **306** | 3,484.30 | 79 | |
| **307** | | 143 | 2,720 |
| **308** | 7,634.20 | 61 | |
| **309** | | 202 | 10,000 |
| **310** | 2,705.20 | 90 | |
| **311** | 1,087.90 | 67 | |
| **312** | 80.6 | 163 | |
| **313** | 375.1 | 191 | |
| **314** | 155.1 | 78 | |
| **315** | | 131 | 2,720 |
| **316** | | 112 | 2,720 |
| **317** | | 142 | 10,000 |
| **318** | 254.6 | 140 | |
| **319** | 243.5 | 113 | |
| **320** | 3,060.10 | 107 | |
| **321** | 249.5 | 130 | |
| **322** | 750.6 | 110 | |
| **323** | 228.6 | 109 | |
| **324** | 340.7 | 107 | |
| **325** | 869.2 | 96 | |
| **326** | 908.3 | 129 | |
| **327** | 721.6 | 104 | |
| **328** | 1,955.00 | 120 | |
| **329** | 5,329.40 | 70 | |
| **330** | 291.4 | 132 | |
| **331** | 58.4 | 126 | |
| **332** | 814.5 | 128 | |
| **333** | 1,220.30 | 94 | |
| **334** | 345.3 | 80 | |
| **335** | | 161 | 2,720 |
| **336** | 1,101.80 | 114 | |
| **337** | 2,274.90 | 92 | |
| **338** | 282.1 | 137 | |
| **339** | 420.9 | 141 | |
| **340** | 1,348.20 | 121 | |
| **341** | 3,006.30 | 105 | |
| **342** | 4,852.50 | 80 | |
| **343** | 2,624.90 | 97 | |
| **344** | 69.7 | 120 | |
| **345** | | 152 | 740 |
| **346** | 5,654.10 | 100 | |
| **347** | 977.2 | 151 | |
| **348** | 661.7 | 141 | |
| **349** | 399.4 | 124 | |
| **350** | 207.8 | 256 | |
| **351** | 112.7 | 107 | |
| **352** | 768.3 | 100 | |
| **353** | 7,240.20 | 108 | |
| **354** | 4,705.70 | 77 | |
| **355** | 1,615.40 | 65 | |
| **357** | 1,136.9 | 386 | |
| **358** | 1,860.80 | 127 | |
| **359** | 81.7 | 115 | 2,720 |
| **360** | 383.6 | 103 | 1,800 |
| **361** | 360.6 | 100 | 10,000 |
| **362** | 3,724.00 | 84 | |
| **363** | >10,000.0 | 50 | 10,000 |
| **364** | 927.9 | 108 | |
| **365** | 686.4 | 142 | |
| **366** | 676.8 | 106 | |
| **367** | 3,688.70 | 87 | |
| **368** | 6,555.40 | 103 | |
| **369** | 466.3 | 114 | |
| **370** | >10,000.0 | 31 | 10,000 |
| **371** | 2,756.70 | 84 | |
| **372** | 367.4 | 118 | |
| **373** | 555.9 | 100 | |
| **374** | >10,000.0 | 52 | 10,000 |
| **375** | 817.3 | 62 | |
| **376** | 4,460.10 | 70 | |
| **377** | 319 | 38 | |
| **378** | 446.9 | 74 | |
| **379** | 4,849.50 | 69 | |
| **380** | 613.2 | 132 | |
| **381** | 2,418.80 | 119 | |
| **382** | 893.1 | 100 | |
| **383** | 1,324.30 | 94 | |
| **384** | 698.5 | 112 | |
| **385** | 1,494.50 | 106 | |
| **386** | 2,268.80 | 108 | |
| **387** | 1,117.00 | 157 | |
| **388** | 165.7 | 129 | |
| **389** | 1,993.20 | 80 | |
| **390** | 4,513.00 | 69 | |
| **391** | 207.3 | 157 | |
| **392** | 947.6 | 96 | |
| **393** | 2,821.20 | 51 | |
| **394** | 1,770.80 | 98 | |
| **395** | 516.7 | 88 | |
| **396** | 525.6 | 87 | |
| **397** | 2,187.30 | 82 | |
| **398** | 3,479.10 | 115 | |
| **399** | 3,459.80 | 73 | |
| **400** | 5,221.10 | 72 | |
| **401** | 1,133.20 | 69 | |
| **402** | 928.2 | 93 | |
| **403** | 1,146.20 | 83 | |
| **404** | 2,346.00 | 101 | |
| **405** | 1,077.60 | 86 | |
| **406** | 1,856.90 | 79 | |
| **407** | 2,812.80 | 96 | |
| **408** | 4,891.70 | 88 | |
| **409** | 3,882.9 | 95 | |
| **410** | 1,164.20 | 109 | |
| **411** | 454.4 | 78 | |
| **412** | 176 | 126 | |
| **413** | 193.4 | 160 | |
| **414** | 2,592.50 | 83 | |
| **415** | 6,226.10 | 64 | |
| **416** | 402 | 58 | |
| **417** | 336.6 | 70 | |
| **418** | 9,502.80 | 52 | |
| **419** | 7,272.50 | 62 | |
| **420** | 1,591.00 | 167 | |
| **421** | 2,482.40 | 123 | |
| **422** | 1,832.70 | 102 | |
| **423** | 1,720.40 | 78 | |
| **424** | 72.3 | 88 | |
| **425** | 1,880.50 | 81 | |
| **426** | 1,397.10 | 109 | |
| **427** | >10,000.0 | 26 | |
| **428** | 3,280.50 | 38 | |
| **429** | 4,854.90 | 103 | |
| **430** | 97 | 110 | |
| **431** | 1,933.80 | 110 | |
| **432** | 1,440.70 | 103 | |
| **433** | 4,790.30 | 105 | |
| **434** | | 130 | 10,000 |
| **435** | 671.4 | 117 | |
| **436** | 940.3 | 66 | |
| **437** | 698 | 112 | |
| **438** | 245.6 | 78 | |
| **439** | 298.6 | 138 | |
| **440** | 2,516.10 | 110 | |
| **441** | 1,025.00 | 128 | |
| **442** | 239.8 | 134 | |
| **443** | 3,852.90 | 97 | |
| **444** | 2,374.80 | 99 | |
| **445** | 4,250.00 | 88 | |
| **446** | 9,898.30 | 51 | |
| **447** | >10,000.0 | 113 | 10,000 |
| **448** | 1,961.10 | 94 | |
| **450** | 71.5 | 114 | 10,000 |
| **451** | 82.1 | 200 | 10,000 |
| **452** | 467.8 | 165 | 10,000 |
| **453** | 443.8 | 136 | 10,000 |
| **454** | 308.7 | 129 | 10,000 |
| **455** | 338.6 | 115 | 10,000 |
| **456** | 1,261.20 | 99 | |
| **457** | 1,135.10 | 50 | |
| **458** | 490.7 | 116 | |
| **459** | 590.7 | 91 | |
| **460** | 5,701.60 | 55 | |
| **461** | 395.3 | 106 | |
| **462** | 561.6 | 121 | |
| **463** | 519.3 | 104 | |
| **464** | 1,038.60 | 111 | |
| **465** | 170.8 | 100 | |
| **466** | 453.7 | 103 | |
| **467** | 879.9 | 102 | |
| **468** | 2,670.50 | 127 | |
| **469** | 170.4 | 105 | |
| **470** | 1,412.00 | 101 | |
| **471** | 8,029.50 | 65 | |
| **472** | 116.2 | 115 | |
| **473** | 403 | 126 | |
| **474** | 135.4 | 138 | |
| **475** | 941 | 85 | |
| **476** | 455.3 | 95 | |
| **477** | 3,494.60 | 77 | |
| **478** | 1,322.20 | 87 | |
| **479** | 439.1 | 127 | |
| **480** | 162.5 | 152 | |
| **481** | 3,045.80 | 89 | |
| **482** | 786.2 | 106 | |
| **483** | 1,536.50 | 79 | |
| **484** | 1,063.20 | 51 | |
| **485** | 867.9 | 98 | |
| **486** | 2,684.20 | 106 | |
| **487** | 1,193.50 | 112 | |
| **488** | 351.4 | 105 | |
| **489** | 7,243.30 | 55 | |
| **490** | 6,051.90 | 72 | |
| **491** | 141.6 | 56 | |
| **492** | 671.3 | 88 | |
| **493** | 982.5 | 102 | |
| **494** | 464.1 | 118 | |
| **495** | 262.6 | 93 | |
| **496** | 1,147.10 | 91 | |
| **497** | 873.7 | 125 | |
| **498** | 1,075.40 | 103 | |
| **499** | 768.3 | 68 | |
| **500** | 1,230.40 | 97 | |
| **501** | 472.9 | 92 | |
| **502** | 4,455.40 | 72 | |
| **503** | 371 | 135 | |
| **504** | 1,113.70 | 103 | |
| **505** | 181.5 | 94 | |
| **506** | 243.3 | 88 | |
| **507** | 2,234.40 | 74 | |
| **508** | 395.5 | 110 | 2,720 |
| **509** | 158.1 | 122 | 2,720 |
| **510** | 403 | 129 | |
| **511** | 6,620.70 | 104 | |
| **512** | | 162 | 2,720 |
| **513** | 285.4 | 157 | |
| **514** | 1,948.90 | 105 | |
| **515** | 4,906.80 | 63 | |
| **516** | 8,333.90 | 51 | |
| **517** | 2,927.90 | 90 | |
| **518** | 9,069.20 | 58 | |
| **519** | 4,372.80 | 72 | |
| **520** | 267.4 | 42 | |
| **521** | 622.1 | 189 | |
| **522** | 782.1 | 161 | |
| **523** | 461.5 | 45 | |
| **524** | 4,269.80 | 80 | |
| **525** | 935.1 | 67 | |
| **526** | | 125 | 10,000 |
| **527** | 1,283.50 | 69 | |

### 2.2) T-cell - TCB - MV3 killing assays

### Reagents and Material

| **Reagents** | **Information** | **Stock conc.** | **End conc.** |
|---|---|---|---|
| Compound dilution Buffer (CDB) DMSO | Sigma, #D2650 | >99% | 0.2% DMSO |

| MV-3 RFP medium | | | |
|---|---|---|---|
| DMEM + GlutaMAX | Gibco, #31966-021 | | |
| Fetal Bovine Serum (FBS) | VWR, #97068-085 | | |
| Penicillin-Streptomycin (PenStrep) (10,000 U/mL) | Life Technologies, #15140122 | 100X | 1X |
| Puromycin dihydrochloride | Sigma, #P9620-10M | | |

### T-cell medium

| | | | |
|---|---|---|---|
| RPMI + GlutaMAX | Gibco, #61870-010 | | |
| Human serum (HS) heat inactivated | Sigma, #H3667-100ML | | 5% |
| Sodium Pyruvate (100 mM) | Gibco, #11360-039 | 100x | 1x |
| 2-Mercaptoethanol | Gibco, #31350-010 | 50mM | 50µM |
| Penicillin-Streptomycin (PenStrep) (10,000 U/mL) | Life Technologies, #15140122 | 100X | 1X |
| Primary T Lymphocyte cells enriched from isolated Peripheral blood mononuclear cells (PBMCs) | PBMCs ordered from stemcell technologies, #70025 | | 312'500 cells/cm2 in 96-well (100k/well) |
| MV-3 RFP (nuclear expression), clone 22 | RNCB Specimen ID: | | 31'250 cells/cm2 in 96-well (10k/well) |
| Activator Control Ref cpd | reference compound A1, stock in DMSO, 0.2% DMSO final | 10mM | 20µM |
| Trypsin 0.05% | Gibco, #25300-054 | | |
| T-flask | Falcon, #353133 | | |
| Neutral Control DMSO | Sigma, #D2650 | | 0.2% DMSO |
| Compounds | Stock in DMSO | 10mM | DR starting at 20µM ; Dil. Factor 3.333; 8 steps |
| No stimulation Control PBS-- | PBS--, Gibco # 14190-094 | 100% | Depending on TCB concentration |
| Stimulation Control MCSP-TCB | In-house generated TCB, Concept ID P1AD8773-001, WO 2014/131711 A1 | Lot dependent | 1.5 pM - 5 pM (depending on donor sensitivity) |
| 96-well cell culture plates, low evaporation | TTP, #92696 | | |
| Round bottom plate | Costar, #3799 | | |
| 6-well cell culture plate | Corning, #3506 | | |
| MACS filter (cell strainer) | Miltenyi Biotech, #130-041-407 | | |

### Cell Culture

All culturing steps are executed at 5% CO2, 37°C and 95% humidity.

MV-3 RFP cells are cultured in MV-3 medium (DMEM + 10% FBS, 1x PenStrep and 0.5 µg/mL Puromycin) for at least 3 weeks. Cultured MV-3 cells at 80% confluency are washed once with PBS-- and trypsinized until detached. Cells are then counted and resuspended to 1*105 cells/mL in T-cell medium (RPMI 1640 + 5% human serum + 1mM Sodium Pyruvate + 50µM 2-mercaptoethanol and 1x Pen-Strep). Cells are seeded with 100 µL/well into a 96-well plate (TTP, #92696), and placed for 40 minutes without moving at room temperature in order to achieve evenly distributed attachment of cells. Plates are then incubated until further use.

On the next day, expanded primary human T-cells are thawed and resuspended in T-cell medium to 4* 106 cells/mL. For 3 hours, they are cultured in a 6-well plate with 6 mL per well at maximum. After culturing the T-cells, they are filtered through a cell strainer (Miltenyi Biotech, #130-041-407), counted again, and cell concentration is adjusted to 2* 106 cells/mL.

### Compound treatment

MCSP-TCB or PBS are pre-diluted in T-cell medium (concentration depending on T-cell donor), 4 times more concentrated than the end-concentration. 60 µL/well of pre-dilutions are then distributed into a round bottom plate (Costar, #3799) according to plate layout. Compounds are added in a 9pt dose response with the Tecan D300e Digital Dispenser, as well 4 times more concentrated than the end-concentration. DMSO concentration of all wells is adjusted to 0.8 %, resulting in 0.2 % as final concentration.

60 µL per well of T-cell suspension are added to the prepared round bottom plate and resuspended with a manual multichannel. 100 µL/well of the resuspended T-cell suspension including treatments are then transferred cautiously to the overnight cultured MV-3 cells according to plate layout. 100 µL T-cell medium only is added to the outer MV-3 wells only. Final compound DR is starting at 20 µM with a dilution factor of 3.333. Final TCB concentration is between 1.5 pM to 5 pM and was determined for each T-cell donor individually by running TCB titrations. For each donor, a TCB concentration was chosen which corresponds to 10-20% of MV3 baseline cell killing in the absence of compound treatment. Positive control is the reference compound A1 which is added in a DR, as well as additional wells with only 20 µM. 20 µM of reference compound A1 represent the positive stimulator control, TCB only (DMSO wells) the neutral control.

### Calculations

After transfer of T-cells with treatment pre-dilutions, MV-3 cells are imaged by time-lapse microscopy using IncucyteZOOM^{™} (Essen BioScience, MI, USA). Imaging is performed every 3 hours for a total of 120 hours (10X objective, phase and red image channels, acquisition time 400 ms, Green/Red 4614 optical module). RFP object count per well is analysed in the IncucyteZOOM^{™} Software (Version 2019B Rev2) with a mask that was previously created and optimized for MV-3 cells. Raw data is exported as object count/well and values are normalized as % TCL compared to wells with MV-3 only, representing 100% growth and therefore 0% TCL.

### RFP measurements

Calculated % TCL values are analysed in Genedata Screener using Roche Normalization PCT_POS_CTRL with MCSP-TCB only set as Neutral Control and 20 µM of the reference compound A1 set as Stimulator Control/100%.

EC50 and Emax values were provided in the table below.

Induced TCL by compounds without TCB treatment or toxicity (observed in the PBS condition) were be flagged.

### Results

| Example | MSCP TCB killing MV-3 cells 5d EC50 (nM) | MSCP TCB killing MV-3 cells 5d Emax (%) |
|---|---|---|
| 3 | 1550 | 100 |
| 8 | 70 | 96 |
| 12 | 910 | 96 |
| 29 | 37 | 95 |
| 36 | 390 | 81 |
| 41 | 230 | 92 |
| 43 | 40 | 102 |
| 46 | 1040 | 83 |
| 49 | 170 | 100 |
| 54 | 300 | 123 |
| 56 | 110 | 123 |
| 57 | 50 | 100 |
| 62 | 260 | 81 |
| 63 | 20 | 93 |
| 72 | 310 | 115 |
| 74 | 20 | 97 |
| 86 | 520 | 100 |
| 91 | 20 | 93 |
| 122 | 510 | 86 |
| 171 | 40 | 100 |
| 184 | 90 | 79 |
| 186 | 130 | 100 |
| 187 | 1640 | 57 |
| 207 | 160 | 90 |
| 211 | 45 | 86 |
| 220 | 390 | 100 |
| 232 | 30 | 83 |
| 237 | 450 | 84 |
| 249 | 210 | 100 |
| 254 | 10 | 104 |
| 262 | 30 | 92 |
| 266 | 20 | 76 |
| 270 | 20 | 81 |
| 272 | 1090 | 100 |
| 278 | 830 | 100 |
| 282 | 880 | 74 |
| 286 | 80 | 100 |
| 289 | 120 | 97 |
| 303 | 400 | 92 |
| 323 | 90 | 100 |
| 331 | 30 | 116 |
| 361 | 560 | 100 |
| 381 | 160 | 101 |
| 384 | 990 | 110 |
| 391 | 70 | 100 |
| 392 | 210 | 92 |
| 397 | 1850 | 92 |
| 401 | 1540 | 92 |
| 406 | 1540 | 85 |
| 412 | 140 | 92 |
| 416 | 530 | 95 |
| 417 | 1860 | 182 |
| 430 | 60 | 96 |
| 437 | 340 | 88 |
| 451 | 160 | 88 |
| 452 | 230 | 81 |
| 454 | 70 | 100 |
| 455 | 370 | 91 |
| 456 | 770 | 94 |
| 458 | 590 | 95 |
| 459 | 330 | 91 |
| 461 | 310 | 91 |
| 462 | 350 | 100 |
| 463 | 540 | 91 |
| 464 | 460 | 89 |
| 466 | 240 | 95 |
| 488 | 300 | 93 |
| 509 | 190 | 94 |
| 517 | 330 | 79 |
| 518 | 960 | 78 |

## Claims

1. A compound of formula (I) 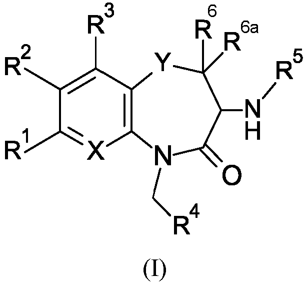 or a pharmaceutically acceptable salt thereof, wherein:
X is C(R⁷) or N;
Y is S, S(O), S(O)₂, S(O)N(R^{y}),
R¹ is 5-membered heteroaryl, wherein R¹ is optionally substituted with one or more R¹⁰ which can be the same or different;
R², R³ and R⁷ are each independently selected from hydrogen, halogen, C₁₋₆-alkyl, C₂₋₆-alkynyl, hydroxy, cyano, halo-C₁₋₆-alkyl, N(R⁸R^{8a}), C₁₋₆-alkoxy, C₃₋₇-cycloalkyl and 3-10 membered heterocyclyl;
R⁴ is selected from C₅₋₁₄-aryl and 5-14 membered heteroaryl, wherein R⁴ is optionally substituted with one or more R¹¹ which can be the same or different;
R⁵ is selected from hydrogen, C₁₋₆-alkyl, -C(O)(R⁹), amino, amino-C₁₋₆-alkyl and C₃₋₇-cycloalkyl,
wherein said C₃₋₇-cycloalkyl is optionally substituted with one or more C₁₋₆-alkyl, amino, amino-C₁₋₆-alkyl-;
R⁶ and R^{6a} are each independently selected from hydrogen and C₁₋₆-alkyl;
R⁸ and R^{8a} are each independently selected from hydrogen and C₁₋₆-alkyl;
R⁹ is selected from C₁₋₆-alkyl, hydroxy-C₁₋₆-alkyl, amino-C₁₋₆-alkyl- and -(C₁₋₆-alkyl)-N(R^{9a}R^{9b}), wherein said amino-C₁₋₆-alkyl is optionally substituted with one or more hydroxy, hydroxy-C₁₋₆-alkyl-;
R^{9a} and R^{9b} are each independently selected from hydrogen and C₁₋₆-alkyl, wherein said C₁₋₆-alkyl is optionally substituted with one or more hydroxy;
or R^{9a} and R^{9b}, taken together with the nitrogen atom to which they are attached, form a 3-10 membered heterocyclyl;
R¹⁰ is selected from:
i) C₁₋₁₀-alkyl, optionally substituted with one or more halogen, C₂₋₆-alkynyl, halo-C₁₋₆-alkyl, amino, hydroxy, 5-6 membered heteroaryl, C₁₋₆-haloalkoxy, C₁₋₆-alkoxy, 3-10 membered cycloalkyl, C₁₋₃-alkyl, -N(R^{10a}R^{10b}), -S(O)₂(C₁₋₆-alkyl), - S(O)2(C₁₋₆-cycloalkyl), 3-10 membered heterocyclyl, phenyl, cyano, - C(O)N(R^{10c}R^{10d}), wherein 3-10 membered heterocyclyl, 3-10 membered cycloalkyl, and phenyl are optionally substituted with one or more C₁₋₁₀-alkyl, C₁₋₁₀-alkoxy, -S(O)₂(C₁₋₆-alkyl), oxo, halogen, C₂₋₆-alkynyl, 3-10 membered cycloalkyl;
ii) C₃₋₁₀-cycloalkyl, optionally substituted with one or more halogen, C₁₋₁₀-alkyl, - S(O)₂(C₁₋₆-alkyl), cyano, C₁₋₆ haloalkyl, C₁₋₆-alkoxy, hydroxy, oxo, amino, - C(O)N(R^{10c}R^{10d}), =N(OH), hydroxy-C₁₋₆-alkyl;
iii) 3-10 membered heterocyclyl, optionally substituted with one or more halogen, C₁₋₁₀-alkyl, -S(O)₂(C₁₋₆-alkyl), -C₁₋₁₀-alkyl-C₁₋₄-alkoxy, amino, -C(O)N(R^{10h} R¹⁰ⁱ), C₁₋₆-haloalkyl, C₁₋₆-alkoxy, cyano, hydroxy-C₁₋₁₀-alkyl, oxo, -C(O)(C₁₋₆-alkyl), - C(O)O-(R^{10q}), C₃₋₁₀-cycloalkyl;
iv) phenyl, optionally substituted with one or more halogen, C₁₋₁₀-alkyl, -S(O)₂(C₁₋₆-alkyl);
v) -N(R^{10e}R^{10f});
vi) -OR^{10g};
vii)-C(O)NR^{10h}R¹⁰ⁱ;
viii) heteroaryl, optionally substituted with one or more C₁₋₁₀-alkyl, halogen, - SO₂(C₁₋₆-alkyl); and
ix) oxo;
R^{10a} and R^{10b} are each independently selected from hydrogen, C₁₋₆-alkyl, halo-C₁₋₆-alkyl, hydroxy-C₁₋₆-alkyl, C₁₋₆-alkoxy,-C(O)(R^{10j}), amino-C₁₋₆-alkyl-, 3-10 membered heterocyclyl, - SO₂(R^{10k}), -C₁₋₆-alkyl-SO₂(R^{10k}) and -N(R^{10l}R^{10m}), wherein 3-10 membered heterocyclyl is optionally substituted with C₁₋₆-alkyl;
or R^{10a} and R^{10b}, taken together with the nitrogen atom to which they are attached, form a 3-10 membered heterocyclyl which is optionally substituted with one or more halogen, C₁₋₆-alkyl;
R^{10c} and R^{10d} are each independently selected from hydrogen and C₁₋₆-alkyl;
R^{10e} and R^{10f} are each independently selected from:
i) hydrogen;
ii) C₁₋₆-alkyl, optionally substituted with one or more cyano, in particular one cyano, halogen, hydroxy;
iii) -C(O)R¹⁰ⁿ;
iv) -C₁₋₁₁₀alkyl((O-C₁₋₁₀alkyl)ₘ), wherein m is an integer between 1 to 5 (more particularly m is 2 or 3), optionally substituted with one or more C₂₋₆-alkynyl;
v) C₃₋₁₀-cycloalkyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, -C(O)O( R^{10o}), - C(O)(R^{10o}), -C(O)N(R^{10p})(R^{10p'}), -SO₂(C₁₋₆-alkyl), C2-6-alkynyl; and
vi) 3-10 membered heterocyclyl, optionally substituted with one or more halogen, hydroxy, oxo, C₁₋₁₀-alkyl, halo-C₁₋₁₀-alkyl, hydroxy-C₁₋₆-alkyl, C₁₋₆-alkoxy, - C(O)O(R^{10o}), -C(O)(R^{10o}), -C(O)N(R^{10p})(R^{10p'}), -SO₂(C₁₋₆-alkyl), 3-10 membered cycloalkyl;
R^{10g} is selected from halo-C₁₋₆-alkyl, cyano, -C₁₋₁₀-alkyl-phenyl -C₁₋₆-alkyl-C₃₋₇-cycloalkyl and -C₁₋₆-alkoxy-halo-C₁₋₆-alkyl;
R^{10h} and R¹⁰ⁱ are each independently selected from hydrogen and C₁₋₆-alkyl, C₁₋₆-haloalkyl, wherein C₁₋₆-alkyl and C₁₋₆-haloalkyl are optionally substituted with one or more hydroxy,
or R^{10h} and R¹⁰ⁱ, taken together with the nitrogen atom to which they are attached, form a 3-10 membered heterocyclyl which is optionally substituted with one or more halogen, hydroxy, C₁₋₆-alkyl;
R^{10j} is selected from C₁₋₆-alkyl, halo-C₁₋₆-alkyl-, hydroxy-C₁₋₆-alkyl- and amino-C₁₋₆-alkyl-;
R^{10k} is selected from hydrogen, C₁₋₆-alkyl and halo-C₁₋₆-alkyl;
R^{10l} and R^{10m} are each independently selected from hydrogen and C₁₋₆-alkyl;
R¹⁰ⁿ is selected from C₁₋₆-alkyl, amino-C₁₋₆-alkyl-, halo-C₁₋₆-alkyl, C₃₋₇-cycloalkyl and 3-10 membered heterocyclyl, wherein said C₃₋₇-cycloalkyl and 3-10 membered heterocyclyl are optionally substituted with one or more halogen, C₁₋₆-alkyl;
R^{10o} is selected from C₁₋₆-alkyl and halo-C₁₋₆-alkyl;
R^{10p} and R^{10p'} are independently selected from hydrogen, C₁₋₆-alkyl and halo-C₁₋₆-alkyl;
R^{10q} is C₁₋₁₀-alkyl, C₁₋₆-haloalkyl, aryl, heteroaryl, wherein aryl and heteroaryl are optionally substituted with one or more halogen, C₁₋₁₀-alkyl;
R¹¹ is selected from:
i) halogen;
ii) hydroxy;
iii) cyano;
iv) C₁₋₁₀-alkyl, optionally substituted with one or more cyano, halogen, hydroxyl, C₃₋₇-cycloalkyl, amino, aryl, -O-aryl, 5-6 membered heteroaryl, 3-7 membered heterocyclyl, wherein 3-7 membered heterocyclyl and aryl are optionally substituted with one or more C₁₋₆-alkyl, -SO₂(C₁₋₆-alkyl), hydroxy, halogen, cyano;
v) C₁₋₆-alkoxy, optionally substituted with one or more, particularly one, 3-10 membered heterocyclyl, halogen;
vi) C₃₋₇-cycloalkyl; vii)3-10 membered heterocyclyl, optionally substituted with one or more halo-C₁₋₆-
alkyl, hydroxy, C₁₋₆-alkyl, C₃₋₁₀-cycloalkyl, C₁₋₆-alkoxy, oxo;
viii) 5-6 membered heteroaryl, optionally substituted with one or more C₁₋₆-alkyl, 3-10 membered cycloalkyl, halogen, halo-C₁₋₆-alkyl, C₁₋₆-alkoxy, C₁₋₆-haloalkoxy;
ix) phenyl, optionally substituted with one or more halogen, cyano, C₁₋₆-alkoxy, C₁₋₆-haloalkyl, C₁₋₆-haloalkoxy, C₁₋₆-alkyl;
x) -O(R^{11a});
xi) -C(O)N(R^{11b}R^{11c});
xii)-SO₂(R^{11d});
xiii) -C(O)OR^{11e};
xiv) -C(O)R^{11f};
xv) oxo;
xvi) -N(R^{11g}R^{11h}); and
xvii) -S(R^{11k});
R^{11a}is selected from C₁₋₁₂-alkyl, halo-C₁₋₆-alkyl, amino-C₁₋₁₂-alkyl-, hydroxy-C₁₋₆-alkyl-, cyano, C₂₋₆-alkynyl, C₃₋₇-cycloalkyl, 3-10 membered heterocyclyl, 3-10 membered -(C₁₋₆-alkyl)heterocyclyl, 5-6 membered heteroaryl, phenyl, -C₁₋₆-alkyl-phenyl, -C₁₋₁₂-alkyl-C(O)N(R¹¹ⁱR^{11j}), -C₁₋₁₂-alkyl-NH-C(O)(C₁₋₆-alkyl), -C₁₋₁₂-alkoxy-NH-C(O)(C₁₋₆-alkyl), -C₁₋₆-alkyl-NH-C(O)(C₁₋₆-alkyl), -(CH₂CH₂O)ₙ-CH₂CH₂NH₂ and -(CH₂CH₂O)ₙ-CH₂CH₂₋NH-C(O)(C₁₋₆-alkyl);
wherein said C₁₋₁₂-alkyl, C₃₋₇-cycloalkyl, 3-10 membered heterocyclyl, 5-6 membered heteroaryl, phenyl and -C₁₋₆-alkyl-phenyl are optionally substituted with one or more halogen, C₁₋₆-alkyl, halo-C₁₋₆-alkyl, C₁₋₆-alkoxyl, C₁₋₆-haloalkoxyl, cyano;
n is an integer between 1 and 6, in particular n is two or three;
R^{11b} and R^{11c} are each independently selected from hydrogen, C₁₋₆-alkyl and halo-C₁₋₆-alkyl,
or R^{11b} and R^{11c}, taken together with the nitrogen atom to which they are attached, form a 3-10 membered heterocyclyl;
R^{11d} is selected from hydrogen, C₁₋₆-alkyl, -N(R^{11l}R^{11m}), halo-C₁₋₆-alkyl and phenyl;
R^{11e} is selected from hydrogen and C₁₋₆-alkyl;
R^{11f} is selected from hydrogen, C₁₋₆-alkyl and phenyl;
R^{11g} and R^{11h} are each independently selected from hydrogen, C₁₋₆-alkyl, -(C₁₋₆-alkyl)phenyl, halo-C₁₋₆-alkyl, -SO₂(C₁₋₆-alkyl), -SO₂(halo-C₁₋₆-alkyl) and -SO(C₁₋₆-alkyl)₂,
R¹¹ⁱR^{11j} are each independently selected from hydrogen, C₁₋₆-alkyl and halo-C₁₋₆-alkyl,
or R¹¹ⁱR^{11j}, taken together with the nitrogen atom to which they are attached, form a 3-10 membered heterocyclyl;
R^{11k} is selected from hydrogen, C₁₋₆-alkyl and halo-C₁₋₆-alkyl;
R^{11l} and R^{11m} are each independently selected from hydrogen and C₁₋₆-alkyl,
or R^{11l} and R^{11m}, taken together with the nitrogen atom to which they are attached, form a 3-10 membered heterocyclyl;
R^{y} is selected from hydrogen and C₁₋₆-alkyl.

2. The compound according to claim 1, or a pharmaceutically acceptable salt thereof, wherein the compound is a compound of formula (IA) 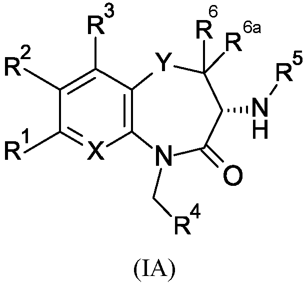 wherein X, Y, R¹, R², R³, R⁴, R⁵, R⁶, R^{6a} are as defined in any one of claims 1 to 3.

3. The compound according to claim 1 or 2, wherein R¹ is substituted with at least one R¹⁰, more particularly with one R¹⁰.

4. The compound according to any one of claims 1 to 3, or a pharmaceutically acceptable salt thereof, wherein X is N or C(R⁷), wherein R⁷ is hydrogen or halogen.

5. The compound according to any one of claims 1 to 4, or a pharmaceutically acceptable salt thereof, wherein Y is S(O) or S(O)₂.

6. The compound according to any one of claims 1 to 5, or a pharmaceutically acceptable salt thereof, wherein R¹⁰ is selected from:
i) C₁₋₁₀-alkyl, optionally substituted with one or more C₂₋₆-alkynyl, cyano;
ii) C₁₋₁₀-haloalkyl, optionally substituted with C₁₋₅-alkoxy, amino, phenyl, wherein phenyl is optionally substituted with one or more halogen;
iii) C₃₋₁₀-cycloalkyl, optionally substituted with one or more halogen;
iv) phenyl, optionally substituted with one or more halogen, C₁₋₁₀-alkyl;
v) heteroaryl, optionally substituted with one or more halogen, C₁₋₁₀-alkyl;
vi) 3-10 membered heterocyclyl, optionally substituted with one or more C₁₋₁₀-alkyl, C₃₋₁₀-cycloalkyl, C₁₋₆-haloalkyl, halogen, -C₁₋₁₀-alkyl-C₁₋₄-alkoxy, -C(O)O-C₁₋₅-alkyl;
vii)amino, optionally substituted with C₁₋₁₀-alkyl, C₁₋₁₀-haloalkyl, C₃₋₁₀-cycloalkyl, wherein C₃₋₁₀-cycloalkyl is optionally substituted with one or more C₁₋₅-alkyl, halogen.

7. The compound according to any one of claims 1 to 6, or a pharmaceutically acceptable salt thereof, wherein R¹ is selected from 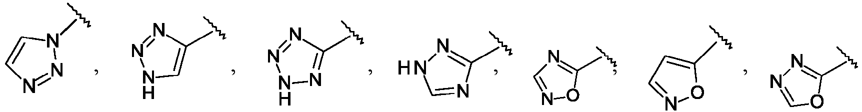 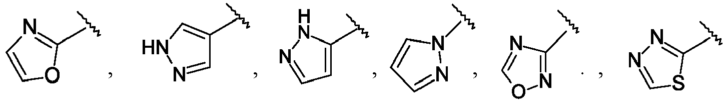 and 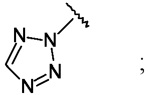 wherein R¹ is optionally substituted with one or more R¹⁰ which can be the same or different as defined in any one of claims 1 or 6 to 7.

8. The compound according to any one of claims 1 to 7, or a pharmaceutically acceptable salt thereof, wherein R² is hydrogen, halogen, C₁₋₆-alkyl, hydroxy or N(R⁸R^{8a}).

9. The compound according to any one of claims 1 to 8, or a pharmaceutically acceptable salt thereof, wherein R⁸ and R^{8a} are each independently selected from hydrogen and C₁₋₆-alkyl.

10. The compound according to any one of claims 1 to 9, or a pharmaceutically acceptable salt thereof, wherein R³ is hydrogen.

11. The compound according to any one of claims 1 to 10, or a pharmaceutically acceptable salt thereof, wherein R¹¹ is selected from halogen, C₁₋₆-haloalkoxy, C₁₋₆-alkoxy, -O-R^{11a}, C₁₋₆-alkyl, C₁₋₆-alkyl substituted with cyano.

12. The compound according to any one of claims 1 to 11, or a pharmaceutically acceptable salt thereof, wherein R^{11a} is selected from C₁₋₁₂-alkyl, halo-C₁₋₆-alkyl, 3-7 membered heterocycloalkyl and amino-C₁₋₁₂-alkyl.

13. The compound according to any one of claims 1 to 12, or a pharmaceutically acceptable salt thereof, wherein R⁴ is selected from 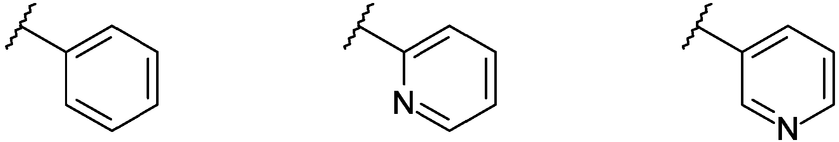 wherein R⁴ is optionally substituted with one or more R¹¹ as defined in any one of claims 1 or 11.

14. The compound according to any one of claims 1 to 13, or a pharmaceutically acceptable salt thereof, wherein R⁵ is hydrogen or -C(O)(R⁹).

15. The compound according to any one of claims 1 to 14, or a pharmaceutically acceptable salt thereof, wherein R⁶ and R^{6a} are hydrogen.

16. The compound according to any one of claims 1 to 15, or a pharmaceutically acceptable salt thereof, wherein
X is N or C(R⁷);
Y is S, SO or S(O)₂;
R¹ is selected from 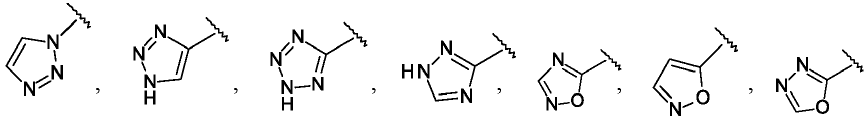 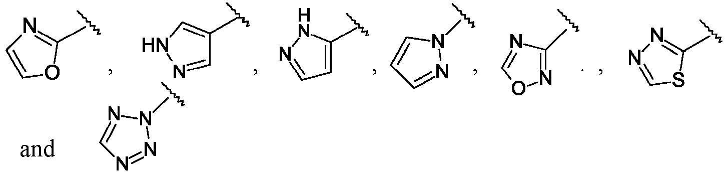
wherein R¹ is optionally substituted with one or more R¹⁰ which can be the same or different;
R² is hydrogen, halogen, C₁₋₆-alkyl, hydroxy or N(R⁸R^{8a});
R³ is hydrogen;
R⁴ is selected from 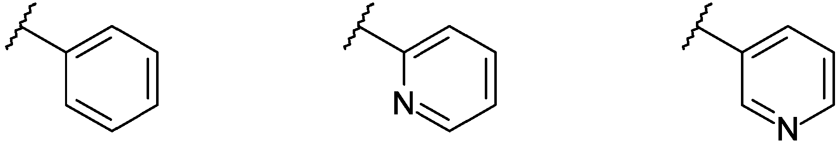
R⁵ is hydrogen or -C(O)(R⁹);
R⁶ and R^{6a} are hydrogen;
R⁷ is hydrogen or halogen;
R⁸ and R^{8a} are each independently selected from hydrogen and C₁₋₆-alkyl;
R⁹ is amino-C₁₋₆-alkyl;
R¹⁰ is selected from C₁₋₁₀-alkyl, C₁₋₁₀-alkyl substituted with C₂₋₆-alkynyl, C₁₋₁₀-haloalkyl, amino-C₁₋₁₀-alkyl, hydroxy-C₁₋₁₀-alkyl, C₃₋₁₀-cycloalkyl, C₃₋₁₀-cycloalkyl substituted with one or more halogen, phenyl substituted with one or more halogen, (C₁₋₁₀-alkyl)-phenyl substituted with one or more halogen, (alkoxy-C₁₋₁₀-alkyl)-phenyl, 3-10 membered heterocyclyl, 3-10 membered heterocyclyl substituted with one or more C₁₋₁₀-alkyl and halogen, heteroaryl, heteroaryl substituted with one or more C₁₋₁₀-alkyl, heteroaryl substituted with one or more halogen, and - N(R^{10e}R^{10f});
R^{10e} is hydrogen, R^{10f} is C₃₋₇-cycloalkyl;
R¹¹ is selected from halogen, -O(R^{11a}), cyano, amino-C₁₋₁₀-alkyl, C₁₋₆-haloalkoxy, - S(O₂)(C₁₋₆-alkyl) and 3-10 membered heterocyclyl substituted with one or more halo-C₁₋₆-alkyl;
R^{11a} is selected from C₁₋₁₂-alkyl, halo-C₁₋₆-alkyl, 3-7 membered heterocycloalkyl, amino-C₁₋₁₂-alkyl.

17. The compound according to any one of claims 1 to 16, or a pharmaceutically acceptable salt thereof, wherein
X is N or C(R⁷);
Y is S, SO or S(O)₂;
R¹ is selected from 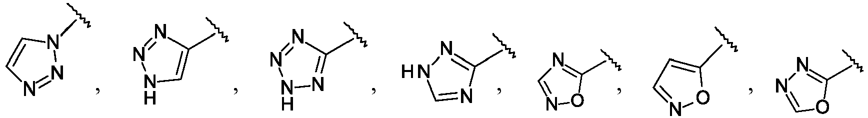 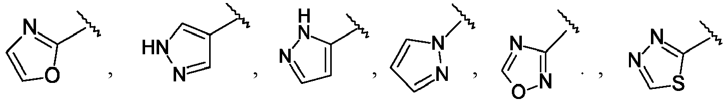 and 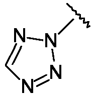
wherein R¹ is optionally substituted with one or more R¹⁰ which can be the same or different;
R² is hydrogen, halogen, C₁₋₆-alkyl, hydroxy or N(R⁸R^{8a});
R³ is hydrogen;
R⁴ is selected 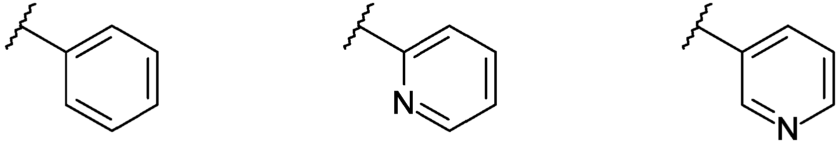
R⁵ is hydrogen or -C(O)(R⁹);
R⁶ and R^{6a} are hydrogen;
R⁷ is hydrogen or halogen;
R⁸ and R^{8a} are each independently selected from hydrogen and C₁₋₆-alkyl;
R⁹ is amino-C₁₋₆-alkyl;
R¹⁰ is selected from C₁₋₁₀-alkyl, C₁₋₁₀-alkyl substituted with C₂₋₆-alkynyl, C₁₋₁₀-haloalkyl, amino-C₁₋₁₀-alkyl, hydroxy-C₁₋₁₀-alkyl, C₃₋₁₀-cycloalkyl, C₃₋₁₀-cycloalkyl substituted with one or more halogen, phenyl substituted with one or more halogen, (C₁₋₁₀-alkyl)-phenyl substituted with one or more halogen, (alkoxy-C₁₋₁₀-alkyl)-phenyl, 3-10 membered heterocyclyl, 3-10 membered heterocyclyl substituted with one or more C₁₋₁₀-alkyl and halogen, -N(R^{10e}R^{10f});
R^{10e} is hydrogen, R^{10f} is C₃₋₇-cycloalkyl;
R¹¹ is selected from halogen, -O(R^{11a}), cyano, amino-C₁₋₁₀-alkyl, -S(O₂)(C₁₋₆-alkyl) and 3-10 membered heterocyclyl substituted with one or more halo-C₁₋₆-alkyl;
R^{11a} is selected from C₁₋₁₂-alkyl, halo-C₁₋₆-alkyl, 3-7 membered heterocycloalkyl, amino-C₁₋₁₂-alkyl.

18. The compound according to any one of claims 1 to 17, selected from
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1lambda4,5-benzothiazepin-4-one;
(3R)-3-amino-7-[5-(1-amino-2,2,2-trifluoro-ethyl)-1,2,4-oxadiazol-3-yl]-8-fluoro-5-[(4-isopropoxyphenyl)methyl]-1,1-dioxo-2,3-dihydro-1lambda6,5-benzothiazepin-4-one;
(3R)-3-amino-5-[(4-chlorophenyl)methyl]-7-[5-[3-fluoro-1-(2-methoxyethyl)-3-piperidyl]-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1-oxo-2,3-dihydro-1lambda4,5-benzothiazepin-4-one;
(3R)-3-amino-7-(5-tert-butyl-1,2,4-oxadiazol-3-yl)-8-fluoro-5-[(4-fluorophenyl)methyl]-1,1-dioxo-2,3-dihydro-1lambda6,5-benzothiazepin-4-one;
(3R)-3-amino-5-[(4-chlorophenyl)methyl]-8-fluoro-7-(5-morpholino-1,2,4-oxadiazol-3-yl)-1-oxo-2,3-dihydro-1lambda4,5-benzothiazepin-4-one;
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1-oxo-2,3-dihydro-1lambda4,5-benzothiazepin-4-one;
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1-oxo-2,3-dihydro-1lambda4,5-benzothiazepin-4-one;
(3R)-3-amino-8-fluoro-5-[(6-isopropoxy-3-pyridyl)methyl]-7-[5-(4-oxa-7-azaspiro[2.5]octan-7-yl)-1,2,4-oxadiazol-3-yl]-1,1-dioxo-2,3-dihydro-1lambda6,5-benzothiazepin-4-one
(3R)-3-amino-5-[(4-chlorophenyl)methyl]-7-[5-[2-(difluoromethyl)morpholin-4-yl]-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1-oxo-7-[5-[2-(trifluoromethyl)morpholin-4-yl]-1,2,4-oxadiazol-3-yl]-2,3-dihydro-1lambda4,5-benzothiazepin-4-one;
(3R)-3-amino-5-[(4-chlorophenyl)methyl]-7-(5-morpholino-1,3,4-oxadiazol-2-yl)-1,1-dioxo-2,3-dihydro-1lambda6,5-benzothiazepin-4-one
(3R)-3-amino-5-[(4-chlorophenyl)methyl]-8-fluoro-1-oxo-7-[5-[2-(trifluoromethyl)morpholin-4-yl]-1,2,4-oxadiazol-3-yl]-2,3-dihydro-1lambda4,5-benzothiazepin-4-one;
(3R)-3-amino-5-[(4-chlorophenyl)methyl]-7-[5-(4-oxa-7-azaspiro[2.5]octan-7-yl)-1,3,4-oxadiazol-2-yl]-1,1-dioxo-2,3-dihydro-1lambda6,5-benzothiazepin-4-one;
methyl 3-[5-[(3R)-3-amino-5-[[4-(cyclopentoxy)phenyl]methyl]-1,1,4-trioxo-2,3-dihydro-1lambda6,5-benzothiazepin-7-yl]-1,3,4-oxadiazol-2-yl]pyrrolidine-1-carboxylate;
(3R)-3-amino-5-[(4-chlorophenyl)methyl]-1,1-dioxo-7-[5-[2-(trifluoromethyl)morpholin-4-yl]-1,3,4-oxadiazol-2-yl]-2,3-dihydro-1lambda6,5-benzothiazepin-4-one
(3R)-3-amino-8-fluoro-5-[(5-isopropoxy-2-pyridyl)methyl]-7-[5-(4-oxa-7-azaspiro[2.5]octan-7-yl)-1,2,4-oxadiazol-3-yl]-1,1-dioxo-2,3-dihydro-1lambda6,5-benzothiazepin-4-one;
2-amino-N-[(3R)-7-(2-tert-butyltetrazol-5-yl)-5-[(4-chlorophenyl)methyl]-8-fluoro-1,1,4-trioxo-2,3-dihydro-1lambda6,5-benzothiazepin-3-yl]acetamide
(3R)-3-amino-5-[(4-chlorophenyl)methyl]-7-[5-(6-methyl-3-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4,6-dimethyl-3-pyridyl)-1,2,4-oxadiazol-3-yl]-8-fluoro-1,1-dioxo-2,3-dihydro-1lambda6,5-benzothiazepin-4-one;
(3R)-3-amino-8-fluoro-7-[5-[2-(hydroxymethyl)tetrahydrofuran-2-yl]-1,2,4-oxadiazol-3-yl]-1,1-dioxo-5-[(4-phenoxyphenyl)methyl]-2,3-dihydro-1lambda6,5-benzothiazepin-4-one;
2-[5-[(3R)-3-amino-1,1,4-trioxo-5-[[4-(trifluoromethoxy)phenyl]methyl]-2,3-dihydro-1lambda6,5-benzothiazepin-7-yl]-1,3,4-oxadiazol-2-yl]-2-methyl-propanenitrile;
(3R)-3-amino-5-[(4-chlorophenyl)methyl]-8-fluoro-7-[5-(1-fluoro-1-methyl-ethyl)-1,2,4-oxadiazol-3-yl]-1,1-dioxo-2,3-dihydro-1lambda6,5-benzothiazepin-4-one;
(3R)-3-amino-8-fluoro-7-[5-(1-fluoro-1-methyl-ethyl)-1,2,4-oxadiazol-3-yl]-5-[(4-fluorophenyl)methyl]-1,1-dioxo-2,3-dihydro-1lambda6,5-benzothiazepin-4-one;
(3R)-3-amino-7-[5-(2,2-difluoromorpholin-4-yl)-1,3,4-oxadiazol-2-yl]-1,1-dioxo-5-[(4-phenoxyphenyl)methyl]-2,3-dihydro-1lambda6,5-benzothiazepin-4-one;
(3R)-3-amino-5-[(4-chlorophenyl)methyl]-7-[5-(2-oxa-5-azabicyclo[4.1.0]heptan-5-yl)-1,3,4-oxadiazol-2-yl]-1,1-dioxo-2,3-dihydro-1lambda6,5-benzothiazepin-4-one
(3R)-3-amino-7-[5-(3-aminooxetan-3-yl)-1,2,4-oxadiazol-3-yl]-8-fluoro-5-[[4-(4-methoxyphenyl)phenyl]methyl]-1,1-dioxo-2,3-dihydro-1lambda6,5-benzothiazepin-4-one
(3R)-3-amino-5-[(4-chlorophenyl)methyl]-8-fluoro-7-[5-(6-fluoro-2-methyl-3-pyridyl)-1,2,4-oxadiazol-3-yl]-1,1-dioxo-2,3-dihydro-1lambda6,5-benzothiazepin-4-one
(3R)-3-amino-5-[(4-chlorophenyl)methyl]-8-fluoro-7-[5-(2-methyl-4-methylsulfonyl-phenyl)-1,2,4-oxadiazol-3-yl]-1,1-dioxo-2,3-dihydro-1lambda6,5-benzothiazepin-4-one;
(3R)-3-amino-5-[(4-chlorophenyl)methyl]-8-fluoro-7-[5-(2-methyl-5-methylsulfonyl-phenyl)-1,2,4-oxadiazol-3-yl]-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-8-fluoro-7-[5-(4-methyl-3-pyridyl)-1,2,4-oxadiazol-3-yl]-1,1-dioxo-2,3-dihydro-1lambda6,5-benzothiazepin-4-one;
(3R)-3-amino-5-[(4-chlorophenyl)methyl]-7-[5-[(1R,5S)-3-oxa-8-azabicyclo[3.2.1]octan-8-yl]-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(2,2-dimethylmorpholin-4-yl)-1,2,4-oxadiazol-3-yl]-8-fluoro-1,1-dioxo-2,3-dihydro-1lambda6,5-benzothiazepin-4-one
(3R)-3-amino-5-[(4-chlorophenyl)methyl]-7-[5-(2-methyl-4-methylsulfonyl-phenyl)-1,3,4-oxadiazol-2-yl]-1,1-dioxo-2,3-dihydro-1lambda6,5-benzothiazepin-4-one;
methyl 3-[3-[(3R)-3-amino-5-[[4-(cyclopentoxy)phenyl]methyl]-8-fluoro-1,1,4-trioxo-2,3-dihydro-1lambda6,5-benzothiazepin-7-yl]-1,2,4-oxadiazol-5-yl]pyrrolidine-1-carboxylate
(3R)-3-amino-5-[(4-chlorophenyl)methyl]-7-[5-(2,2-difluoromorpholin-4-yl)-1,2,4-oxadiazol-3-yl]-8-fluoro-1,1-dioxo-2,3-dihydro-1lambda6,5-benzothiazepin-4-one
(3R)-3-amino-7-(5-tert-butyl-1,2,4-oxadiazol-3-yl)-5-[[4-(cyclopentoxy)phenyl]methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(5-methyl-1,2,4-oxadiazol-3-yl)-1,3,4-oxadiazol-2-yl]-1,1-dioxo-2,3-dihydro-1lambda6,5-benzothiazepin-4-one;
(3R)-3-amino-8-fluoro-7-[5-(3-fluoro-1-methyl-3-piperidyl)-1,2,4-oxadiazol-3-yl]-1,1-dioxo-5-[(4-phenoxyphenyl)methyl]-2,3-dihydro-1lambda6,5-benzothiazepin-4-one
(3R)-3-amino-5-[(4-chlorophenyl)methyl]-8-fluoro-7-[5-(6-fluoro-2-methyl-3-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4,6-dimethyl-3-pyridyl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-(4,4-difluoro-1-methoxy-cyclohexyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(2-fluoro-6-methyl-phenyl)-1,3,4-oxadiazol-2-yl]-1,1-dioxo-2,3-dihydro-1lambda6,5-benzothiazepin-4-one;1,1,1,3,3,3-hexafluoropropan-2-ol;
methyl 4-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-4-methoxy-piperidine-1-carboxylate;
(3R)-3-amino-5-[(4-chlorophenyl)methyl]-8-fluoro-7-[5-(5-methyl-1,3,4-oxadiazol-2-yl)-1,2,4-oxadiazol-3-yl]-1,1-dioxo-2,3-dihydro-1lambda6,5-benzothiazepin-4-one;
methyl N-[2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-2-methyl-1-(trifluoromethyl)propyl carbamate;
(3R)-3-amino-7-[5-(2-amino-3,3,3-trifluoro-1,1-dimethyl-propyl)-1,3,4-oxadiazol-2-yl]-5-[(4-chlorophenyl)methyl]-8-fluoro-1,1-dioxo-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N-(3,3,3-trifluoro-2-hydroxy-2-methyl-propyl)-1,2,4-oxadiazole-5-carboxamide;
(3R)-3-amino-5-[(4-chlorophenyl)methyl]-8-fluoro-7-[5-[2-(hydroxymethyl)tetrahydrofuran-2-yl]-1,2,4-oxadiazol-3-yl]-1,1-dioxo-2,3-dihydro-1lambda6,5-benzothiazepin-4-one
(3R)-3-amino-8-fluoro-7-[5-(5-methyl-1,3,4-oxadiazol-2-yl)-1,2,4-oxadiazol-3-yl]-1,1-dioxo-5-[[4-(trifluoromethoxy)phenyl]methyl]-2,3-dihydro-1lambda6,5-benzothiazepin-4-one;
(3R)-3-amino-7-[5-[1-amino-2,2,2-trifluoro-1-(4-fluorophenyl)ethyl]-1,2,4-oxadiazol-3-yl]-5-[(4-chlorophenyl)methyl]-8-fluoro-1,1-dioxo-2,3-dihydro-1lambda6,5-benzothiazepin-4-one;
(3R)-3-amino-8-fluoro-7-[5-(5-methyl-1,3,4-oxadiazol-2-yl)-1,3,4-oxadiazol-2-yl]-1,1-dioxo-5-[[4-(trifluoromethoxy)phenyl]methyl]-2,3-dihydro-1lambda6,5-benzothiazepin-4-one;
(3R)-3-amino-5-[(4-chlorophenyl)methyl]-8-fluoro-7-[5-(3-oxa-8-azabicyclo[3.2.1]octan-8-yl)-1,2,4-oxadiazol-3-yl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[2-(trifluoromethyl)morpholin-4-yl]-1,2,4-oxadiazol-3-yl]-2,3-dihydro-1lambda6,5-benzothiazepin-4-one;
(3R)-3-amino-5-[(4-chlorophenyl)methyl]-8-fluoro-7-[5-(2-oxa-5-azabicyclo[4.1.0]heptan-5-yl)-1,2,4-oxadiazol-3-yl]-1,1-dioxo-2,3-dihydro-1lambda6,5-benzothiazepin-4-one;
(3R)-3-amino-8-fluoro-7-[5-[2-(hydroxymethyl)tetrahydrofuran-2-yl]-1,2,4-oxadiazol-3-yl]-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-7-[5-(2-fluorophenyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4-fluorophenyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-1,1-dioxo-7-[5-(p-tolyl)-1,3,4-oxadiazol-2-yl]-2,3-dihydro-1lambda6,5-benzothiazepin-4-one;
(3R)-3-amino-5-[(4-chlorophenyl)methyl]-8-fluoro-7-[5-(2-fluorophenyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-7-[5-(m-tolyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1,1-dioxo-7-[5-(p-tolyl)-1,3,4-oxadiazol-2-yl]-2,3-dihydro-1lambda6,5-benzothiazepin-4-one
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-2-oxa-5-azabicyclo[2.2.1]heptane-5-carboxylate
(3R)-3-amino-7-[5-(1-amino-2,2,2-trifluoro-1-tetrahydropyran-4-yl-ethyl)-1,2,4-oxadiazol-3-yl]-5-[(4-chlorophenyl)methyl]-8-fluoro-1,1-dioxo-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N-(2,2-difluoropropyl)-1,2,4-oxadiazole-5-carboxamide;
(3R)-3-amino-7-[5-(1-amino-2,2,2-trifluoro-1-methyl-ethyl)-1,2,4-oxadiazol-3-yl]-8-fluoro-1,1-dioxo-5-[[4-(trifluoromethoxy)phenyl]methyl]-2,3-dihydro-1lambda6,5-benzothiazepin-4-one;
(3R)-3-amino-8-fluoro-1,1-dioxo-5-[[4-(trifluoromethoxy)phenyl]methyl]-7-[5-[1-(trifluoromethyl)cyclopropyl]-1,2,4-oxadiazol-3-yl]-2,3-dihydro-1lambda6,5-benzothiazepin-4-one
(3R)-3-amino-7-[5-(1-amino-2,2,2-trifluoro-1-methyl-ethyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[1-(trifluoromethyl)cyclopropyl]-1,3,4-oxadiazol-2-yl]-2,3-dihydro-1lambda6,5-benzothiazepin-4-one;
(3R)-3-amino-5-[(4-chlorophenyl)methyl]-1,1-dioxo-7-[5-[1-(trifluoromethyl)cyclopropyl]-1,3,4-oxadiazol-2-yl]-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N-(2,2,2-trifluoroethyl)-1,2,4-oxadiazole-5-carboxamide;
(3R)-3-amino-5-[(4-chlorophenyl)methyl]-1,1-dioxo-7-(5-phenyl-1,3,4-oxadiazol-2-yl)-2,3-dihydro-1lambda6,5-benzothiazepin-4-one
(3R)-3-amino-5-[(4-chlorophenyl)methyl]-8-fluoro-7-[5-(4-fluorophenyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-7-[5-(o-tolyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1,1-dioxo-7-(5-phenyl-1,3,4-oxadiazol-2-yl)-2,3-dihydro-1lambda6,5-benzothiazepin-4-one;
methyl 3-[5-[(3R)-3-amino-1,1,4-trioxo-5-[[4-(trifluoromethoxy)phenyl]methyl]-2,3-dihydro-1lambda6,5-benzothiazepin-7-yl]-1,3,4-oxadiazol-2-yl]pyrrolidine-1-carboxylate;
(3R)-3-amino-5-[(4-chlorophenyl)methyl]-8-fluoro-7-[5-(5-methyl-2-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(6-methyl-3-pyridyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-8-fluoro-1,1-dioxo-7-[5-[(3,3,3-trifluoro-2-hydroxy-2-methyl-propyl)amino]-1,3,4-oxadiazol-2-yl]-2,3-dihydro-1lambda6,5-benzothiazepin-4-one
(3R)-3-amino-5-[(4-chlorophenyl)methyl]-8-fluoro-7-[5-[[1-(2-hydroxyethyl)cyclohexyl]amino]-1,3,4-oxadiazol-2-yl]-1,1-dioxo-2,3-dihydro-1lambda6,5-benzothiazepin-4-one;
(3R)-3-amino-5-benzyl-8-fluoro-1,1-dioxo-7-[5-(1,2,2,2-tetrafluoro-1-methoxy-ethyl)-1,2,4-oxadiazol-3-yl]-2,3-dihydro-1lambda6,5-benzothiazepin-4-one;
(3R)-3-amino-7-[5-(3-aminooxetan-3-yl)-1,2,4-oxadiazol-3-yl]-8-fluoro-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-8-fluoro-7-[5-(6-fluoro-3-pyridyl)-1,3,4-oxadiazol-2-yl]-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[(4-phenoxyphenyl)methyl]-2,3-dihydro-1lambda6,5-benzothiazepin-4-one
(3R)-3-amino-5-[(4-chlorophenyl)methyl]-8-fluoro-7-[5-(5-fluoro-2-pyridyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2,2-difluoropropylamino)-1,3,4-oxadiazol-2-yl]-1,1-dioxo-2,3-dihydro-1lambda6,5-benzothiazepin-4-one;
2-[[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]amino]-2-methyl-propanenitrile
(3R)-3-amino-5-[(4-chlorophenyl)methyl]-7-[5-(1-methoxycyclopropyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-1,1-dioxo-7-[5-(2,2,2-trifluoro-1-methoxy-1-methyl-ethyl)-1,3,4-oxadiazol-2-yl]-2,3-dihydro-1lambda6,5-benzothiazepin-4-one;
(3R)-3-amino-5-[(4-chlorophenyl)methyl]-7-[5-(4-methoxytetrahydropyran-4-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-(1,1,2,2,2-pentafluoroethyl)-1,3,4-oxadiazol-2-yl]-2,3-dihydro-1lambda6,5-benzothiazepin-4-one
N-[11-[4-[[(3R)-3-amino-7-(5-tert-butyl-1,3,4-oxadiazol-2-yl)-8-fluoro-1,1,4-trioxo-2,3-dihydro-1lambda6,5-benzothiazepin-5-yl]methyl]phenoxy]undecyl]-3-[2,2-difluoro-12-(1H-pyrrol-2-yl)-1-aza-3-azonia-2-boranuidatricyclo[7.3.0.03,7]dodeca-3,5,7,9,11-pent
(3R)-3-amino-5-[(4-chlorophenyl)methyl]-7-[5-(1-methoxy-1-methyl-ethyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[(1-ethynylcyclohexyl)amino]-1,3,4-oxadiazol-2-yl]-8-fluoro-1,1-dioxo-2,3-dihydro-1lambda6,5-benzothiazepin-4-one;
(3R)-3-amino-7-(5-tert-butyl-1,2,4-oxadiazol-3-yl)-5-[(4-chlorophenyl)methyl]-8-fluoro-1-imino-1-oxo-2,3-dihydro-1lambda6,5-benzothiazepin-4-one;
(3R)-3-amino-5-[(4-chlorophenyl)methyl]-7-[5-(9,9-difluoro-3-oxa-7-azabicyclo[3.3.1]nonan-7-yl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[4,4-difluoro-3-(hydroxymethyl)-1-piperidyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(3,3-dimethylmorpholin-4-yl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1lambda4,5-benzothiazepin-4-one
(3R)-3-amino-5-[(4-chlorophenyl)methyl]-7-[5-(5,5-difluoro-1-methyl-3-piperidyl)-1,2,4-oxadiazol-3-yl]-8-fluoro-1-oxo-2,3-dihydro-1lambda4,5-benzothiazepin-4-one
methyl 4-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-4-cyano-piperidine-1-carboxylate
(3R)-3-amino-5-[(4-chlorophenyl)methyl]-7-[5-(3,3-difluoro-1,8-diazaspiro[4.5]decan-8-yl)-1,3,4-oxadiazol-2-yl]-8-fluoro-1,1-dioxo-2,3-dihydro-1lambda6,5-benzothiazepin-4-one;
(3R)-3-amino-7-(5-tert-butyl-1,2,4-oxadiazol-3-yl)-5-[(4-chlorophenyl)methyl]-8-fluoro-1-oxo-2,3-dihydro-1lambda4,5-benzothiazepin-4-one;
(3R)-3-amino-7-(5-tert-butyl-1,2,4-oxadiazol-3-yl)-5-[(4-chlorophenyl)methyl]-8-fluoro-1-oxo-2,3-dihydro-1lambda4,5-benzothiazepin-4-one;
(3R)-3-amino-5-[(4-chlorophenyl)methyl]-8-fluoro-7-[5-(1-methyl-1-pyrrolidin-1-yl-ethyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-[4-methyl-1-oxido-1-(2,2,2-trifluoroethyl)piperidin-1-ium-4-yl]-1,2,4-oxadiazol-3-yl]-1-oxo-2,3-dihydro-1lambda4,5-benzothiazepin-4-one;
2-[4-[[(3R)-3-amino-7-(5-tert-butyl-1,3,4-oxadiazol-2-yl)-8-fluoro-1,1,4-trioxo-2,3-dihydro-1lambda6,5-benzothiazepin-5-yl]methyl]phenyl]-2-methyl-propanenitrile
(3R)-3-amino-5-[(4-chlorophenyl)methyl]-7-[5-[(1S,5R)-6,6-difluoro-3-azabicyclo[3.1.1]heptan-3-yl]-1,3,4-oxadiazol-2-yl]-8-fluoro-1,1-dioxo-2,3-dihydro-1lambda6,5-benzothiazepin-4-one;
1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-4-methyl-piperidine-4-carbonitrile;
(3R)-3-amino-7-(5-tert-butyl-1,3,4-oxadiazol-2-yl)-8-fluoro-1,1-dioxo-5-[[4-(2,2,2-trifluoroethoxy)phenyl]methyl]-2,3-dihydro-1lambda6,5-benzothiazepin-4-one
(3R)-3-amino-5-[(4-chlorophenyl)methyl]-7-[5-[[1,1-dimethyl-4-(2-prop-2-ynoxyethoxy)butyl]amino]-1,2,4-oxadiazol-3-yl]-8-fluoro-1,1-dioxo-2,3-dihydro-1lambda6,5-benzothiazepin-4-one
3-[(3R)-3-amino-8-fluoro-1,1,4-trioxo-5-[[4-(1,1,2,2-tetrafluoroethoxy)phenyl]methyl]-2,3-dihydro-1lambda6,5-benzothiazepin-7-yl]-N,N-dimethyl-1,2,4-oxadiazole-5-carboxamide;
(3R)-3-amino-7-(5-tert-butyl-1,3,4-oxadiazol-2-yl)-5-[(6-chloropyridazin-3-yl)methyl]-8-fluoro-1,1-dioxo-2,3-dihydro-1lambda6,5-benzothiazepin-4-one
(3R)-3-amino-5-[(4-chlorophenyl)methyl]-8-fluoro-1,1-dioxo-7-[5-(2,2,2-trifluoroethylamino)-1,3,4-oxadiazol-2-yl]-2,3-dihydro-1lambda6,5-benzothiazepin-4-one;
(3R)-3-amino-5-[(4-chlorophenyl)methyl]-7-[5-[1,1-dimethyl-4-[2-(2-prop-2-ynoxyethoxy)ethoxy]butyl]-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-7-[2-(5,5-difluoro-1-methylsulfonyl-3-piperidyl)tetrazol-5-yl]-8-fluoro-1,1-dioxo-2,3-dihydro-1lambda6,5-benzothiazepin-4-one
methyl 5-[5-[(3R)-3-amino-5-[(4-chlorophenyl)methyl]-8-fluoro-1,1,4-trioxo-2,3-dihydro-1lambda6,5-benzothiazepin-7-yl]tetrazol-2-yl]-3,3-difluoro-piperidine-1-carboxylate
2-[[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]amino]-2-methyl-propanenitrile;
(3R)-3-amino-5-[(4-chlorophenyl)methyl]-8-fluoro-7-[5-[4-methyl-1-(2,2,2-trifluoroethyl)-4-piperidyl]-1,2,4-oxadiazol-3-yl]-1,1-dioxo-2,3-dihydro-1lambda6,5-benzothiazepin-4-one;
(3R)-3-amino-5-[(4-chlorophenyl)methyl]-7-[5-(2,2-difluoropropylamino)-1,3,4-oxadiazol-2-yl]-8-fluoro-1,1-dioxo-2,3-dihydro-1lambda6,5-benzothiazepin-4-one;
(3R)-3-amino-7-[5-(1-bicyclo[1.1.1]pentanylamino)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[(3,3,3-trifluoro-1-methyl-propyl)amino]-1,3,4-oxadiazol-2-yl]-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-1,1-dioxo-5-[(4-tetrahydropyran-4-yloxyphenyl)methyl]-2,3-dihydro-1lambda6,5-benzothiazepin-4-one
methyl 1-[3-[(3R)-3-amino-8-fluoro-1-imino-1,4-dioxo-5-[[4-(1,1,2,2-tetrafluoroethoxy)phenyl]methyl]-2,3-dihydro-1lambda6,5-benzothiazepin-7-yl]-1,2,4-oxadiazol-5-yl]-3-azabicyclo[3.1.1]heptane-3-carboxylate
methyl 1-[3-[(3R)-3-amino-8-fluoro-1,4-dioxo-5-[[4-(trifluoromethoxy)phenyl]methyl]-2,3-dihydro-1lambda4,5-benzothiazepin-7-yl]-1,2,4-oxadiazol-5-yl]-3-azabicyclo[3.1.1]heptane-3-carboxylate
(3R)-3-amino-5-[(4-chlorophenyl)methyl]-8-fluoro-1-imino-1-oxo-7-[5-(1,2,2,2-tetrafluoro-1-methoxy-ethyl)-1,2,4-oxadiazol-3-yl]-2,3-dihydro-1lambda6,5-benzothiazepin-4-one;
(3R)-3-amino-7-(2-tert-butyltetrazol-5-yl)-8-fluoro-1,1-dioxo-5-[[4-(tetrahydropyran-4-ylmethoxy)phenyl]methyl]-2,3-dihydro-1lambda6,5-benzothiazepin-4-one
(3R)-3-amino-7-(5-tert-butyl-1,2,4-oxadiazol-3-yl)-8-fluoro-1,1-dioxo-5-[[4-(tetrahydropyran-4-ylmethoxy)phenyl]methyl]-2,3-dihydro-1lambda6,5-benzothiazepin-4-one
(3R)-3-amino-7-(5-tert-butyl-1,3,4-oxadiazol-2-yl)-5-[[4-(cyclopropoxy)phenyl]methyl]-1,1-dioxo-2,3-dihydro-1lambda6,5-benzothiazepin-4-one
methyl 1-[3-[(3R)-3-amino-8-fluoro-1,4-dioxo-5-[[4-(trifluoromethoxy)phenyl]methyl]-2,3-dihydro-1lambda4,5-benzothiazepin-7-yl]-1,2,4-oxadiazol-5-yl]-3-azabicyclo[3.1.1]heptane-3-carboxylate
(3R)-7-[2-(1-acetyl-5,5-difluoro-3-piperidyl)tetrazol-5-yl]-3-amino-5-[(4-chlorophenyl)methyl]-8-fluoro-1,1-dioxo-2,3-dihydro-1lambda6,5-benzothiazepin-4-one
N-[(3R)-5-[(4-chlorophenyl)methyl]-7-[2-(5,5-difluoro-3-piperidyl)tetrazol-5-yl]-8-fluoro-1,1,4-trioxo-2,3-dihydro-1lambda6,5-benzothiazepin-3-yl]acetamide;
(3R)-3-amino-5-[(4-chlorophenyl)methyl]-8-fluoro-1,1-dioxo-7-[5-(1,5,5-trimethyl-3-piperidyl)-1,2,4-oxadiazol-3-yl]-2,3-dihydro-1lambda6,5-benzothiazepin-4-one;
(3R)-3-amino-7-(5-tert-butyl-1,3,4-oxadiazol-2-yl)-5-[[4-(cyclohexoxy)phenyl]methyl]-1,1-dioxo-2,3-dihydro-1lambda6,5-benzothiazepin-4-one
methyl 1-[3-[(3R)-3-amino-8-fluoro-1,4-dioxo-5-[[4-(1,1,2,2-tetrafluoroethoxy)phenyl]methyl]-2,3-dihydro-1lambda4,5-benzothiazepin-7-yl]-1,2,4-oxadiazol-5-yl]-3-azabicyclo[3.1.1]heptane-3-carboxylate
(3R)-3-amino-7-(2-tert-butyltetrazol-5-yl)-5-[(4-chlorophenyl)methyl]-8-fluoro-1-oxo-2,3-dihydro-1lambda4,5-benzothiazepin-4-one
(3R)-3-amino-7-(2-tert-butyltetrazol-5-yl)-5-[(4-chlorophenyl)methyl]-8-fluoro-1-oxo-2,3-dihydro-1lambda4,5-benzothiazepin-4-one
(3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1lambda4,5-benzothiazepin-4-one;
(3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1lambda4,5-benzothiazepin-4-one
(3R)-3-amino-5-[(4-chlorophenyl)methyl]-8-fluoro-1-oxo-7-[5-(1,2,2,2-tetrafluoro-1-methoxy-ethyl)-1,2,4-oxadiazol-3-yl]-2,3-dihydro-1lambda4,5-benzothiazepin-4-one;
(3R)-3-amino-5-[(4-chlorophenyl)methyl]-8-fluoro-7-[5-[[(1S)-1-methyl-2-(trifluoromethoxy)ethyl]amino]-1,3,4-oxadiazol-2-yl]-1,1-dioxo-2,3-dihydro-1lambda6,5-benzothiazepin-4-one;
(3R)-3-amino-5-benzyl-7-[2-(5,5-difluoro-1-methyl-3-piperidyl)tetrazol-5-yl]-8-fluoro-1,1-dioxo-2,3-dihydro-1lambda6,5-benzothiazepin-4-one
(3R)-3-amino-5-[(4-chlorophenyl)methyl]-7-[2-(5,5-difluoro-1-methyl-3-piperidyl)tetrazol-5-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-1,1-dioxo-5-[[4-(tetrahydropyran-4-ylmethoxy)phenyl]methyl]-2,3-dihydro-1lambda6,5-benzothiazepin-4-one
(3R)-3-amino-8-fluoro-1,1-dioxo-5-[[4-(trifluoromethoxy)phenyl]methyl]-7-[8-(trifluoromethyl)-5,6,7,8-tetrahydro-[1,2,4]triazolo[1,5-a]pyrazin-2-yl]-2,3-dihydro-1lambda6,5-benzothiazepin-4-one
(3R)-3-amino-8-fluoro-5-[(4-fluorophenyl)methyl]-1-oxo-7-[5-(1,2,2,2-tetrafluoro-1-methoxy-ethyl)-1,2,4-oxadiazol-3-yl]-2,3-dihydro-1lambda4,5-benzothiazepin-4-one;
(3R)-3-amino-8-fluoro-5-[(4-fluorophenyl)methyl]-1,1-dioxo-7-[5-(1,2,2,2-tetrafluoro-1-methoxy-ethyl)-1,2,4-oxadiazol-3-yl]-2,3-dihydro-1lambda6,5-benzothiazepin-4-one;
(3R)-3-amino-5-[(4-chlorophenyl)methyl]-8-fluoro-1,1-dioxo-7-[5-(2,2,2-trifluoro-1-hydroxy-1-methyl-ethyl)-1,2,4-oxadiazol-3-yl]-2,3-dihydro-1lambda6,5-benzothiazepin-4-one
(3R)-3-amino-7-(5-tert-butyl-1,3,4-oxadiazol-2-yl)-1,1-dioxo-5-[[4-[[5-(trifluoromethyl)-2-pyridyl]oxy]phenyl]methyl]-2,3-dihydro-1lambda6,5-benzothiazepin-4-one
methyl 1-[3-[(3R)-3-amino-8-fluoro-1,1,4-trioxo-5-[[4-(1,1,2,2-tetrafluoroethoxy)phenyl]methyl]-2,3-dihydro-1lambda6,5-benzothiazepin-7-yl]-1,2,4-oxadiazol-5-yl]-3-azabicyclo[3.1.1]heptane-3-carboxylate
(3R)-3-amino-7-(5-tert-butyl-1,3,4-oxadiazol-2-yl)-5-[[4-(4-fluorophenoxy)phenyl]methyl]-1,1-dioxo-2,3-dihydro-1lambda6,5-benzothiazepin-4-one
methyl 1-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-3-azabicyclo[3.1.1]heptane-3-carboxylate
methyl 1-[3-[(3R)-3-amino-8-fluoro-1,1,4-trioxo-5-[[4-(trifluoromethoxy)phenyl]methyl]-2,3-dihydro-1lambda6,5-benzothiazepin-7-yl]-1,2,4-oxadiazol-5-yl]-3-azabicyclo[3.1.1]heptane-3-carboxylate
(3R)-3-amino-5-[(4-chlorophenyl)methyl]-8-fluoro-1,1-dioxo-7-[5-[1-(trifluoromethyl)cyclopropyl]-1,2,4-oxadiazol-3-yl]-2,3-dihydro-1lambda6,5-benzothiazepin-4-one;
(3R)-3-amino-7-(2-tert-butyltetrazol-5-yl)-8-fluoro-5-[(4-fluorophenyl)methyl]-1,1-dioxo-2,3-dihydro-1lambda6,5-benzothiazepin-4-one
(3R)-3-amino-1-oxo-7-[5-(2,2,2-trifluoroethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-1-oxo-7-[5-(2,2,2-trifluoroethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-1-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-1-oxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1lambda4,5-benzothiazepin-4-one;
(3R)-3-amino-7-[5-(1-amino-2,2,2-trifluoro-ethyl)-1,2,4-oxadiazol-3-yl]-8-fluoro-1,1-dioxo-5-[[4-(trifluoromethoxy)phenyl]methyl]-2,3-dihydro-1lambda6,5-benzothiazepin-4-one;
isopropyl 1-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-3-azabicyclo[3.1.1]heptane-3-carboxylate
ethyl 1-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-3-azabicyclo[3.1.1]heptane-3-carboxylate
(3R)-3-amino-5-[(4-chlorophenyl)methyl]-7-[5-[1-(2,2-difluorocyclopropyl)ethylamino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[1-(2,2-difluorocyclopropyl)ethylamino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[1-(3,3-difluorocyclobutyl)ethylamino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[(3,3,3-trifluoro-1,1-dimethyl-propyl)amino]-1,3,4-oxadiazol-2-yl]-2,3-dihydro-1lambda6,5-benzothiazepin-4-one;
(3R)-3-amino-5-[(4-chlorophenyl)methyl]-8-fluoro-7-[5-[1-methyl-1-(2,2,2-trifluoroethylamino)ethyl]-1,2,4-oxadiazol-3-yl]-1,1-dioxo-2,3-dihydro-1lambda6,5-benzothiazepin-4-one;
methyl 3-[3-[(3R)-3-amino-8-fluoro-5-[(4-isopropoxyphenyl)methyl]-1,1,4-trioxo-2,3-dihydro-1lambda6,5-benzothiazepin-7-yl]-1,2,4-oxadiazol-5-yl]pyrrolidine-1-carboxylate;
methyl 1-[5-[(3R)-3-amino-1,1,4-trioxo-5-[[4-(trifluoromethoxy)phenyl]methyl]-2,3-dihydro-1lambda6,5-benzothiazepin-7-yl]-1,3,4-oxadiazol-2-yl]-3-azabicyclo[3.1.1]heptane-3-carboxylate
(3R)-3-amino-7-(5-tert-butyl-1,3,4-oxadiazol-2-yl)-1,1-dioxo-5-[[4-(trifluoromethylsulfonyl)phenyl]methyl]-2,3-dihydro-1lambda6,5-benzothiazepin-4-one
(3R)-3-amino-7-[5-(5,5-difluoro-1-methyl-3-piperidyl)-1,2,4-oxadiazol-3-yl]-8-fluoro-5-[(4-isopropoxyphenyl)methyl]-1,1-dioxo-2,3-dihydro-1lambda6,5-benzothiazepin-4-one;
(3R)-3-amino-7-[5-(5,5-difluoro-1-methyl-3-piperidyl)-1,2,4-oxadiazol-3-yl]-8-fluoro-5-[(6-isopropoxy-3-pyridyl)methyl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-[[3-(trifluoromethyl)cyclobutyl]amino]-1,3,4-oxadiazol-2-yl]-2,3-dihydro-1lambda6,5-benzothiazepin-4-one;
(3R)-3-amino-5-[(4-chlorophenyl)methyl]-8-fluoro-1,1-dioxo-7-[5-(1,2,2,2-tetrafluoroethyl)-1,2,4-oxadiazol-3-yl]-2,3-dihydro-1lambda6,5-benzothiazepin-4-one;
methyl 1-[5-[(3R)-3-amino-1,1,4-trioxo-5-[[4-(1,1,2,2-tetrafluoroethoxy)phenyl]methyl] - 2,3-dihydro-1lambda6,5-benzothiazepin-7-yl]-1,3,4-oxadiazol-2-yl]-3-azabicyclo[3.1.1]heptane-3-carboxylate
(3R)-3-amino-7-[5-[(3-chloro-1-bicyclo[1.1.1]pentanyl)amino]-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-[(3-fluoro-1-bicyclo[1.1.1]pentanyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-(1,1,2,2,2-pentafluoroethyl)-1,2,4-oxadiazol-3-yl]-2,3-dihydro-1lambda6,5-benzothiazepin-4-one
(3R)-3-amino-5-[(4-chlorophenyl)methyl]-7-[5-[(3,3-difluoro-1-methylcyclobutyl)amino]-1,2,4-oxadiazol-3-yl]-8-fluoro-1,1-dioxo-2,3-dihydro-1lambda6,5-benzothiazepin-4-one
(3R)-3-amino-5-[(4-chlorophenyl)methyl]-8-fluoro-7-[2-[(3R)-1-methylpyrrolidin-3-yl]tetrazol-5-yl]-1,1-dioxo-2,3-dihydro-1lambda6,5-benzothiazepin-4-one;
(3R)-3-amino-5-[(4-chlorophenyl)methyl]-8-fluoro-7-[2-[(3S)-1-methylpyrrolidin-3-yl]tetrazol-5-yl]-1,1-dioxo-2,3-dihydro-1lambda6,5-benzothiazepin-4-one;
3-[(3R)-3-amino-5-[(4-chlorophenyl)methyl]-8-fluoro-1,1,4-trioxo-2,3-dihydro-1lambda6,5-benzothiazepin-7-yl]-N,N-dimethyl-1,2,4-oxadiazole-5-carboxamide;
3-[(3R)-3-amino-8-fluoro-1,1,4-trioxo-5-[[4-(trifluoromethoxy)phenyl]methyl]-2,3-dihydro-1lambda6,5-benzothiazepin-7-yl]-N,N-dimethyl-1,2,4-oxadiazole-5-carboxamide;
(3R)-3-amino-5-[(4-chlorophenyl)methyl]-7-[1-(5,5-difluoro-1-methyl-3-piperidyl)pyrazol-4-yl]-8-fluoro-1,1-dioxo-2,3-dihydro-1lambda6,5-benzothiazepin-4-one;
(3R)-3-amino-7-[5-[(2-amino-3,3,3-trifluoro-propyl)amino]-1,2,4-oxadiazol-3-yl]-5-[(4-chlorophenyl)methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[(4-pyrrolidin-1-ylphenyl)methyl]-2,3-dihydro-1lambda6,5-benzothiazepin-4-one
(6-methyl-3-pyridyl) 4-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-4-methyl-piperidine-1-carboxylate;
(3R)-3-amino-7-[5-(5,5-difluoro-1-methyl-3-piperidyl)-1,2,4-oxadiazol-3-yl]-8-fluoro-5-[(5-isopropoxy-2-pyridyl)methyl]-1,1-dioxo-2,3-dihydro-1lambda6,5-benzothiazepin-4-one;
(3R)-3-amino-5-[(4-chlorophenyl)methyl]-7-[5-(1-ethyl-4-methyl-4-piperidyl)-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-8-fluoro-1,1-dioxo-7-[5-(1,2,2,2-tetrafluoro-1-methoxy-ethyl)-1,2,4-oxadiazol-3-yl]-2,3-dihydro-1lambda6,5-benzothiazepin-4-one;
2-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-2-methyl-propanenitrile;
(3R)-3-amino-5-[(4-chlorophenyl)methyl]-7-[5-(5,5-dimethyl-3-piperidyl)-1,2,4-oxadiazol-3-yl]-8-fluoro-1,1-dioxo-2,3-dihydro-1lambda6,5-benzothiazepin-4-one;
2,2,2-trifluoroethyl 4-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-4-methyl-piperidine-1-carboxylate;
methyl 4-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-4-methyl-piperidine-1-carboxylate;
(3R)-3-amino-5-[(4-chlorophenyl)methyl]-8-fluoro-1,1-dioxo-7-[5-[[1-(2,2,2-trifluoroethyl)-3-piperidyl]amino]-1,3,4-oxadiazol-2-yl]-2,3-dihydro-1lambda6,5-benzothiazepin-4-one
(3R)-3-amino-7-[2-(2-amino-3,3,3-trifluoro-propyl)tetrazol-5-yl]-5-[(4-chlorophenyl)methyl]-8-fluoro-1,1-dioxo-2,3-dihydro-1lambda6,5-benzothiazepin-4-one;
4-[3-[(3R)-3-amino-5-[(4-chlorophenyl)methyl]-8-fluoro-1,1,4-trioxo-2,3-dihydro-1lambda6,5-benzothiazepin-7-yl]-1,2,4-oxadiazol-5-yl]-N,4-dimethyl-piperidine-1-carboxamide;
(3R)-3-amino-5-[(4-chlorophenyl)methyl]-8-fluoro-7-[5-[4-methyl-1-(pyrrolidine-1-carbonyl)-4-piperidyl]-1,2,4-oxadiazol-3-yl]-1,1-dioxo-2,3-dihydro-1lambda6,5-benzothiazepin-4-one;
(3R)-3-amino-7-[5-(5,5-difluoro-1-methyl-3-piperidyl)-1,3,4-oxadiazol-2-yl]-1,1-dioxo-5-[[4-(trifluoromethoxy)phenyl]methyl]-2,3-dihydro-1lambda6,5-benzothiazepin-4-one;
(3R)-3-amino-5-[(4-chlorophenyl)methyl]-8-fluoro-1,1-dioxo-7-[5-[(2,2,2-trifluoro-1-methyl-ethyl)amino]-1,3,4-oxadiazol-2-yl]-2,3-dihydro-1lambda6,5-benzothiazepin-4-one;
(3R)-3-amino-7-[5-(1-amino-2,2,2-trifluoro-1-methyl-ethyl)-1,2,4-oxadiazol-3-yl]-5-[(4-chlorophenyl)methyl]-8-fluoro-1,1-dioxo-2,3-dihydro-1lambda6,5-benzothiazepin-4-one;
methyl 3-[5-[(3R)-3-amino-5-[(4-chlorophenyl)methyl]-8-fluoro-1,1,4-trioxo-2,3-dihydro-1lambda6,5-benzothiazepin-7-yl]tetrazol-2-yl]pyrrolidine-1-carboxylate
(3R)-3-amino-5-[(4-chlorophenyl)methyl]-7-[5-[(3,3-difluorocyclobutyl)amino]-1,2,4-oxadiazol-3-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-5-[[4-(4-cyclopropylpiperazin-1-yl)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[[4-(4-methoxy-1-piperidyl)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[[4-(cyclopropylmethoxy)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[[4-(2,2-difluoroethoxy)phenyl]methyl]-1,1-dioxo-2,3-dihydro-1lambda6,5-benzothiazepin-4-one
(3R)-3-amino-7-(5-tert-butyl-1,3,4-oxadiazol-2-yl)-5-[(4-but-2-ynoxyphenyl)methyl]-1,1-dioxo-2,3-dihydro-1lambda6,5-benzothiazepin-4-one
(3R)-3-amino-7-[5-(1-amino-2,2,2-trifluoro-1-methyl-ethyl)-1,3,4-oxadiazol-2-yl]-5-[(4-chlorophenyl)methyl]-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[[4-(2,2,2-trifluoroethoxy)phenyl]methyl]-2,3-dihydro-1lambda6,5-benzothiazepin-4-one
(3R)-3-amino-7-(5-tert-butyl-1,3,4-oxadiazol-2-yl)-5-[(4-morpholinophenyl)methyl]-1,1-dioxo-2,3-dihydro-1lambda6,5-benzothiazepin-4-one
(3R)-3-amino-5-[(4-chlorophenyl)methyl]-8-fluoro-1,1-dioxo-7-[5-[3-(trifluoromethyl)piperazin-1-yl]-1,2,4-oxadiazol-3-yl]-2,3-dihydro-1lambda6,5-benzothiazepin-4-one;2,2,2-trifluoroacetic acid
(3R)-3-amino-1,1-dioxo-7-[5-(1,1,2,2,2-pentafluoroethyl)-1,3,4-oxadiazol-2-yl]-5-[[4-(trifluoromethoxy)phenyl]methyl]-2,3-dihydro-1lambda6,5-benzothiazepin-4-one;
(3R)-3-amino-5-[(4-chlorophenyl)methyl]-7-[5-[(3,3-difluorocyclohexyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(3,3-difluorocyclobutyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(3,3-difluoro-1-methylcyclobutyl)amino]-1,3,4-oxadiazol-2-yl]-8-fluoro-1,1-dioxo-2,3-dihydro-1lambda6,5-benzothiazepin-4-one;
2-[4-[[(3R)-3-amino-7-(5-tert-butyl-1,3,4-oxadiazol-2-yl)-1,1,4-trioxo-2,3-dihydro-1lambda6,5-benzothiazepin-5-yl]methyl]phenyl]-2-methyl-propanenitrile
(3R)-3-amino-5-[(4-chlorophenyl)methyl]-7-[5-(4,4-difluoro-1-piperidyl)-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-8-fluoro-7-(5-morpholino-1,2,4-oxadiazol-3-yl)-1,1-dioxo-2,3-dihydro-1lambda6,5-benzothiazepin-4-one;
(3R)-3-amino-5-[(4-chlorophenyl)methyl]-7-[5-(cyclobutylamino)-1,2,4-oxadiazol-3-yl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(4-oxa-7-azaspiro[2.5]octan-7-yl)-1,2,4-oxadiazol-3-yl]-1,1-dioxo-2,3-dihydro-1lambda6,5-benzothiazepin-4-one;
(3R)-7-[2-(1-acetyl-3-piperidyl)tetrazol-5-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[2-(1-methyl-3-piperidyl)tetrazol-5-yl]-1,1-dioxo-2,3-dihydro-1lambda6,5-benzothiazepin-4-one;
(3R)-3-amino-7-(3-tert-butyl-1,2,4-triazol-1-yl)-5-[(4-chlorophenyl)methyl]-8-fluoro-1,1-dioxo-2,3-dihydro-1lambda6,5-benzothiazepin-4-one;
(3R)-3-amino-7-[5-[1-methyl-1-(methylamino)ethyl]-1,3,4-oxadiazol-2-yl]-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-7-[5-[3-(dimethylamino)-1,1-dimethylpropyl]-1,3,4-oxadiazol-2-yl]-8-fluoro-1,1-dioxo-2,3-dihydro-1lambda6,5-benzothiazepin-4-one;
3-[(3R)-3-amino-1,1,4-trioxo-5-[[4-(trifluoromethoxy)phenyl]methyl]-2,3-dihydro-1lambda6,5-benzothiazepin-7-yl]-N-tert-butyl-1,2,4-oxadiazole-5-carboxamide
3-[(3R)-3-amino-8-fluoro-1,1,4-trioxo-5-[[4-(trifluoromethoxy)phenyl]methyl]-2,3-dihydro-1lambda6,5-benzothiazepin-7-yl]-N-tert-butyl-1,2,4-oxadiazole-5-carboxamide
3-[(3R)-3-amino-5-[(4-chlorophenyl)methyl]-8-fluoro-1,1,4-trioxo-2,3-dihydro-1lambda6,5-benzothiazepin-7-yl]-N-tert-butyl-1,2,4-oxadiazole-5-carboxamide
3-[(3R)-3-amino-5-[(4-chlorophenyl)methyl]-1,1,4-trioxo-2,3-dihydro-1lambda6,5-benzothiazepin-7-yl]-N-tert-butyl-1,2,4-oxadiazole-5-carboxamide
(3R)-3-amino-5-[(4-chlorophenyl)methyl]-7-[5-[(dimethylamino)methyl]-1,2,4-oxadiazol-3-yl]-8-fluoro-1,1-dioxo-2,3-dihydro-1lambda6,5-benzothiazepin-4-one
(3R)-3-amino-7-[5-(1-aminocyclopentyl)-1,3,4-oxadiazol-2-yl]-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-7-[5-(3,3-difluorocyclohexyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-[cyclopropyl(methyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[methyl(2,2,2-trifluoroethyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[[3,3-difluoro-1-(methoxymethyl)cyclobutyl]amino]-1,3,4-oxadiazol-2-yl]-1,1-dioxo-2,3-dihydro-1lambda6,5-benzothiazepin-4-one;
(3R)-3-amino-7-[5-(4-aminotetrahydropyran-4-yl)-1,3,4-oxadiazol-2-yl]-1,1-dioxo-5-[[4-(trifluoromethoxy)phenyl]methyl]-2,3-dihydro-1lambda6,5-benzothiazepin-4-one
(3R)-3-amino-7-[5-(1-aminocyclohexyl)-1,3,4-oxadiazol-2-yl]-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-8-fluoro-7-[5-(1-hydroxy-1-methyl-ethyl)-1,3,4-oxadiazol-2-yl]-1,1-dioxo-2,3-dihydro-1lambda6,5-benzothiazepin-4-one;2,2,2-trifluoroacetic acid
(3R)-3-amino-7-(2-tert-butyltetrazol-5-yl)-1,1-dioxo-5-[[4-(trifluoromethoxy)phenyl]methyl]-2,3-dihydro-1lambda6,5-benzothiazepin-4-one
(3R)-3-amino-7-(2-tert-butyltetrazol-5-yl)-8-fluoro-1,1-dioxo-5-[[4-(trifluoromethoxy)phenyl]methyl]-2,3-dihydro-1lambda6,5-benzothiazepin-4-one
(3R)-3-amino-5-[(4-chlorophenyl)methyl]-8-fluoro-7-[2-(1-methylsulfonyl-3-piperidyl)tetrazol-5-yl]-1,1-dioxo-2,3-dihydro-1lambda6,5-benzothiazepin-4-one;
(3R)-3-amino-5-[(4-chlorophenyl)methyl]-7-[5-[(3,3-difluoro-1-methylcyclobutyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[(3,3-difluorocyclobutyl)amino]-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-[(3,3-difluorocyclohexyl)amino]-1,3,4-oxadiazol-2-yl]-1,1-dioxo-2,3-dihydro-1lambda6,5-benzothiazepin-4-one;
(3R)-3-amino-7-(4-tert-butylimidazol-1-yl)-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(5-oxa-2-azaspiro[3.4]octan-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2-cyclopropyltetrahydrofuran-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2-cyclopropyltetrahydrofuran-2-yl)-1,3,4-oxadiazol-2-yl]-1,1-dioxo-2,3-dihydro-1lambda6,5-benzothiazepin-4-one
(3R)-3-amino-7-(2-tert-butyltetrazol-5-yl)-5-[(4-chlorophenyl)methyl]-1,1-dioxo-2,3-dihydro-1lambda6,5-benzothiazepin-4-one
(3R)-7-[5-(3-acetyl-3-azabicyclo[3.1.0]hexan-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
methyl 3-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]azetidine-1-carboxylate;2,2,2-trifluoroacetic acid
(3R)-3-amino-5-[(4-chlorophenyl)methyl]-7-[5-(2,2-difluoromorpholin-4-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-1,1-dioxo-7-[5-(1,2,2,2-tetrafluoroethyl)-1,3,4-oxadiazol-2-yl]-2,3-dihydro-1lambda6,5-benzothiazepin-4-one;
(3R)-3-amino-5-[(4-chlorophenyl)methyl]-8-fluoro-7-[5-(3-methylsulfonyl-3-azabicyclo[3.1.0]hexan-1-yl)-1,3,4-oxadiazol-2-yl]-1,1-dioxo-2,3-dihydro-1lambda6,5-benzothiazepin-4-one
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[3.1.0]hexane-3-carboxylate
(3R)-3-amino-5-[(4-chlorophenyl)methyl]-8-fluoro-7-[2-(1-methylsulfonylpyrrolidin-3-yl)tetrazol-5-yl]-1,1-dioxo-2,3-dihydro-1lambda6,5-benzothiazepin-4-one;
(3R)-3-amino-5-[(4-chlorophenyl)methyl]-7-[5-(2-hydroxyspiro[3.3]heptan-6-yl)-1,3,4-oxadiazol-2-yl]-1,1-dioxo-2,3-dihydro-1lambda6,5-benzothiazepin-4-one
(3R)-3-amino-7-[5-[3-(chloromethyl)-3-(hydroxymethyl)azetidin-1-yl]-1,3,4-oxadiazol-2-yl]-5-[(4-chlorophenyl)methyl]-1,1-dioxo-2,3-dihydro-1lambda6,5-benzothiazepin-4-one;
2-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-2-methoxy-acetonitrile;
(3R)-3-amino-1,1-dioxo-7-[5-(2,2,2-trifluoroethyl)-1,3,4-oxadiazol-2-yl]-5-[[4-(tiifluoromethoxy)phenyl]methyl]-2,3-dihydro-1lambda6,5-benzothiazepin-4-one;
(3R)-3-amino-5-[(4-chlorophenyl)methyl]-8-fluoro-7-[2-(1-methylsulfonyl-4-piperidyl)tetrazol-5-yl]-1,1-dioxo-2,3-dihydro-1lambda6,5-benzothiazepin-4-one;
(3R)-3-amino-7-(5-tert-butyl-1,3,4-oxadiazol-2-yl)-1,1-dioxo-5-[[4-(1,1,2,2-tetrafluoroethoxy)phenyl]methyl]-2,3-dihydro-1lambda6,5-benzothiazepin-4-one
(3R)-3-amino-5-[(4-chlorophenyl)methyl]-8-fluoro-7-[5-(5-oxa-2-azaspiro[3.4]octan-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(6-oxa-2-azaspiro[3.5]nonan-2-yl)-1,3,4-oxadiazol-2-yl]-1,1-dioxo-2,3-dihydro-1lambda6,5-benzothiazepin-4-one;
(3R)-3-amino-7-(5-tert-butyl-1,3,4-oxadiazol-2-yl)-5-[[4-(cyclopentoxy)phenyl]methyl]-1,1-dioxo-2,3-dihydro-1lambda6,5-benzothiazepin-4-one
(3R)-3-amino-5-[(4-chlorophenyl)methyl]-1,1-dioxo-7-[5-(1,1,2,2,2-pentafluoroethyl)-1,3,4-oxadiazol-2-yl]-2,3-dihydro-1lambda6,5-benzothiazepin-4-one;
methyl 7-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-5-azaspiro[2.4]heptane-5-carboxylate;
2-[5-[(3R)-3-amino-8-fluoro-1,1,4-trioxo-5-[[4-(trifluoromethoxy)phenyl]methyl]-2,3-dihydro-1lambda6,5-benzothiazepin-7-yl]-1,3,4-oxadiazol-2-yl]-2-methyl-propanenitrile;
(3R)-3-amino-1,1-dioxo-7-[5-(1,2,2,2-tetrafluoro-1-methoxy-ethyl)-1,3,4-oxadiazol-2-yl]-5-[[4-(trifluoromethoxy)phenyl]methyl]-2,3-dihydro-1lambda6,5-benzothiazepin-4-one;
(3R)-3-amino-5-[(4-chlorophenyl)methyl]-1,1-dioxo-7-[5-(1,2,2,2-tetrafluoro-1-methoxy-ethyl)-1,3,4-oxadiazol-2-yl]-2,3-dihydro-1lambda6,5-benzothiazepin-4-one;
(3R)-3-amino-7-[5-[(1R,5S)-8-azabicyclo[3.2.1]octane-8-carbonyl]-1,3,4-oxadiazol-2-yl]-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(5-methylsulfonyl-5-azaspiro[2.4]heptan-7-yl)-1,3,4-oxadiazol-2-yl]-1,1-dioxo-2,3-dihydro-1lambda6,5-benzothiazepin-4-one;
(3R)-7-[5-(1-acetyl-4-piperidyl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-8-fluoro-7-(2-methyltetrazol-5-yl)-1,1-dioxo-2,3-dihydro-1lambda6,5-benzothiazepin-4-one;
(3R)-3-amino-5-[(4-chlorophenyl)methyl]-8-fluoro-7-[2-(1-methylpyrrolidin-3-yl)tetrazol-5-yl]-1,1-dioxo-2,3-dihydro-1lambda6,5-benzothiazepin-4-one;
(3R)-7-[2-(1-acetyl-4-piperidyl)tetrazol-5-yl]-3-amino-5-[(4-chlorophenyl)methyl]-8-fluoro-1,1-dioxo-2,3-dihydro-1lambda6,5-benzothiazepin-4-one;
(3R)-7-[5-(3-acetyl-3-azabicyclo[4.1.0]heptan-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(3-methylsulfonyl-3-azabicyclo[4.1.0]heptan-1-yl)-1,3,4-oxadiazol-2-yl]-1,1-dioxo-2,3-dihydro-1lambda6,5-benzothiazepin-4-one;
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[4.1.0]heptane-3-carboxylate;
(3R)-3-amino-5-[(4-chlorophenyl)methyl]-7-[5-(6,6-difluoro-2-azaspiro[3.3]heptan-2-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(isopropylamino)-1,3,4-oxadiazol-2-yl]-1,1-dioxo-2,3-dihydro-1lambda6,5-benzothiazepin-4-one;
(3R)-3-amino-7-[5-(2-azaspiro[3.3]heptan-2-yl)-1,3,4-oxadiazol-2-yl]-5-[(4-chlorophenyl)methyl]-1,1-dioxo-2,3-dihydro-1lambda6,5-benzothiazepin-4-one;
methyl 5-[[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]amino]-3,3-difluoro-piperidine-1-carboxylate;
(3R)-3-amino-5-[(4-chlorophenyl)methyl]-7-[5-(4,4-difluoro-1-piperidyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(2,2-dimethylmorpholin-4-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(3-fluoro-1-methyl-3-piperidyl)-1,3,4-oxadiazol-2-yl]-1,1-dioxo-2,3-dihydro-1lambda6,5-benzothiazepin-4-one;
methyl 4-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]piperidine-1-carboxylate
(3R)-3-amino-7-[5-(1-amino-2,2,2-trifluoro-ethyl)-1,3,4-oxadiazol-2-yl]-8-fluoro-1,1-dioxo-5-[[4-(trifluoromethoxy)phenyl]methyl]-2,3-dihydro-1lambda6,5-benzothiazepin-4-one
methyl 3-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]pyrrolidine-1-carboxylate
(3R)-3-amino-5-[(4-chlorophenyl)methyl]-7-[5-(5,5-difluoro-1-methyl-3-piperidyl)-1,3,4-oxadiazol-2-yl]-8-methyl-1,1-dioxo-2,3-dihydro-1lambda6,5-benzothiazepin-4-one
(3R)-3-amino-5-[(4-chlorophenyl)methyl]-7-[5-(5,5-difluoro-1-methyl-3-piperidyl)-1,3,4-oxadiazol-2-yl]-1,1-dioxo-2,3-dihydro-1lambda6,5-benzothiazepin-4-one
2-[5-[(3R)-3-amino-5-[(4-chlorophenyl)methyl]-8-methyl-1,1,4-trioxo-2,3-dihydro-1lambda6,5-benzothiazepin-7-yl]-1,3,4-oxadiazol-2-yl]-2-methyl-propanenitrile;
(3R)-3-amino-5-[(4-chlorophenyl)methyl]-7-[5-[(5,5-difluoro-3-piperidyl)amino]-1,3,4-oxadiazol-2-yl]-8-fluoro-1,1-dioxo-2,3-dihydro-1lambda6,5-benzothiazepin-4-one;
(3R)-3-amino-7-[5-(3-azabicyclo[4.1.0]heptan-1-yl)-1,3,4-oxadiazol-2-yl]-5-[(4-chlorophenyl)methyl]-8-fluoro-1,1-dioxo-2,3-dihydro-1lambda6,5-benzothiazepin-4-one
(3R)-7-[5-(5-acetyl-5-azaspiro[2.4]heptan-7-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-7-[5-(3,3-difluoropiperidine-1-carbonyl)-1,3,4-oxadiazol-2-yl]-8-fluoro-1,1-dioxo-2,3-dihydro-1lambda6,5-benzothiazepin-4-one
2-[5-[(3R)-3-amino-5-[(4-chlorophenyl)methyl]-8-methyl-1,1,4-trioxo-2,3-dihydro-Ilambda6,5-benzothiazepin-7-yl]-1,3,4-oxadiazol-2-yl]propanenitrile;
(3R)-3-amino-5-[(4-chlorophenyl)methyl]-8-fluoro-7-[5-(3-methylsulfonyl-3-azabicyclo[3.1.1]heptan-1-yl)-1,3,4-oxadiazol-2-yl]-1,1-dioxo-2,3-dihydro-1lambda6,5-benzothiazepin-4-one
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[3.1.1]heptane-3-carboxylate;
methyl 1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3-azabicyclo[3.1.1]heptane-3-carboxylate
2-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]-2-methyl-propanenitrile;
(3R)-7-[5-(3-acetyl-3-azabicyclo[3.1.1]heptan-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one
(3R)-3-amino-7-[5-(1-amino-2,2,2-trifluoro-ethyl)-1,3,4-oxadiazol-2-yl]-5-[(4-chlorophenyl)methyl]-8-fluoro-1,1-dioxo-2,3-dihydro-1lambda6,5-benzothiazepin-4-one;2,2,2-trifluoroacetic acid
(3R)-3-amino-7-[5-(1-amino-2,2,2-trifluoro-ethyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-[4-(dimethylamino)-1,1-dimethyl-butyl]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(3,3-difluorocyclopentyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(4,4-difluorocyclohexyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-(1,3-dimethylazetidin-3-yl)-1,3,4-oxadiazol-2-yl]-8-fluoro-1,1-dioxo-2,3-dihydro-1lambda6,5-benzothiazepin-4-one;
(3R)-3-amino-7-[5-(tert-butylamino)-1,3,4-oxadiazol-2-yl]-5-[(4-chlorophenyl)methyl]-8-fluoro-1,1-dioxo-2,3-dihydro-1lambda6,5-benzothiazepin-4-one;
1-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]cyclopropanecarbonitrile;
2-[5-[(3R)-3-amino-5-[(4-chlorophenyl)methyl]-1,1,4-trioxo-2,3-dihydro-1lambda6,5-benzothiazepin-7-yl]-1,3,4-oxadiazol-2-yl]propanenitrile;
methyl 3-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]piperidine-1-carboxylate
(3R)-3-amino-5-[(4-chlorophenyl)methyl]-7-[5-(3,3-difluoropyrrolidine-1-carbonyl)-1,3,4-oxadiazol-2-yl]-8-fluoro-1,1-dioxo-2,3-dihydro-1lambda6,5-benzothiazepin-4-one
(3R)-3-amino-7-[5-(2-aminospiro[3.3]heptan-6-yl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(2-oxa-7-azaspiro[3.5]nonan-7-yl)-1,3,4-oxadiazol-2-yl]-1,1-dioxo-2,3-dihydro-1lambda6,5-benzothiazepin-4-one
4-[[(3R)-3-amino-8-fluoro-7-[5-(2-hydroxy-1,1-dimethyl-ethyl)-1,3,4-oxadiazol-2-yl]-1,1,4-trioxo-2,3-dihydro-1lambda6,5-benzothiazepin-5-yl]methyl]benzonitrile;
(3R)-3-amino-5-[(4-chlorophenyl)methyl]-7-[5-(5,5-difluoro-1-methyl-3-piperidyl)-1,2,4-oxadiazol-3-yl]-8-fluoro-1,1-dioxo-2,3-dihydro-1lambda6,5-benzothiazepin-4-one
(3R)-3-amino-7-[5-(5-amino-3,3-difluoro-1-piperidyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-7-[5-[1-(chloromethyl)-2-hydroxy-1-methyl-ethyl]-1,3,4-oxadiazol-2-yl]-5-[(4-chlorophenyl)methyl]-8-fluoro-1,1-dioxo-2,3-dihydro-1lambda6,5-benzothiazepin-4-one;
5-[(3R)-3-amino-5-[(4-chlorophenyl)methyl]-8-fluoro-1,1,4-trioxo-2,3-dihydro-1lambda6,5-benzothiazepin-7-yl]-N-isopropyl-N-methyl-1,3,4-oxadiazole-2-carboxamide
(3R)-3-amino-5-[(4-chlorophenyl)methyl]-7-[5-(1-cyclopropyl-5,5-difluoro-3-piperidyl)-1,3,4-oxadiazol-2-yl]-8-fluoro-1,1-dioxo-2,3-dihydro-1lambda6,5-benzothiazepin-4-one;
(3R)-3-amino-7-[5-[4-(chloromethyl)-4-(hydroxymethyl)-1-piperidyl]-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(4,4-difluoro-1-methyl-3-piperidyl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(2-methylsulfonylethyl)-1,3,4-oxadiazol-2-yl]-1,1-dioxo-2,3-dihydro-1lambda6,5-benzothiazepin-4-one;
5-[(3R)-3-amino-5-[(4-chlorophenyl)methyl]-8-fluoro-1,1,4-trioxo-2,3-dihydro-1lambda6,5-benzothiazepin-7-yl]-N-tert-butyl-1,3,4-oxadiazole-2-carboxamide
(3R)-3-amino-5-[(4-chlorophenyl)methyl]-8-fluoro-7-[5-(isopropoxymethyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-[5,5-difluoro-1-(2-methoxyethyl)-3-piperidyl]-1,3,4-oxadiazol-2-yl]-8-fluoro-1,1-dioxo-2,3-dihydro-1lambda6,5-benzothiazepin-4-one
5-[(3R)-3-amino-5-[(4-chlorophenyl)methyl]-8-fluoro-1,1,4-trioxo-2,3-dihydro-1lambda6,5-benzothiazepin-7-yl]-3-tert-butyl-1,3,4-oxadiazol-2-one;
(3R)-3-amino-5-[(4-chlorophenyl)methyl]-8-fluoro-7-[5-(2-methyltetrahydrofuran-2-yl)-1,3,4-oxadiazol-2-yl]-1,1-dioxo-2,3-dihydro-1lambda6,5-benzothiazepin-4-one;
(3R)-3-amino-7-[5-(4-amino-1,1-dimethyl-butyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(1,3-dimethyl-3-piperidyl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-[(1-ethyl-3-piperidyl)amino]-1,3,4-oxadiazol-2-yl]-8-fluoro-1,1-dioxo-2,3-dihydro-1lambda6,5-benzothiazepin-4-one
(3R)-3-amino-7-[5-(3-amino-4,4-difluoro-1-piperidyl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-[2-(1,1-dioxo-1,4-thiazinan-4-yl)-1,1-dimethyl-ethyl]-1,3,4-oxadiazol-2-yl]-8-fluoro-1,1-dioxo-2,3-dihydro-1lambda6,5-benzothiazepin-4-one
5-[(3R)-3-amino-5-[(4-chlorophenyl)methyl]-8-fluoro-1,1,4-trioxo-2,3-dihydro-1lambda6,5-benzothiazepin-7-yl]-N,N-dimethyl-1,3,4-oxadiazole-2-carboxamide
(3R)-3-amino-5-[(4-chlorophenyl)methyl]-7-[5-(1-ethyl-5,5-difluoro-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(1-methylpyrrolidin-3-yl)-1,3,4-oxadiazol-2-yl]-1,1-dioxo-2,3-dihydro-1lambda6,5-benzothiazepin-4-one
methyl 5-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-3,3-difluoro-piperidine-1-carboxylate
(3R)-3-amino-7-[5-(2-azaspiro[3.3]heptan-2-yl)-1,3,4-oxadiazol-2-yl]-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(isopropylamino)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(4,4-difluoro-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[(1-cyclopropyl-4-piperidyl)amino]-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-7-[5-(2,2-dimethylmorpholin-4-yl)-1,3,4-oxadiazol-2-yl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(4-oxa-7-azaspiro[2.5]octan-7-yl)-1,3,4-oxadiazol-2-yl]-1,1-dioxo-2,3-dihydro-1lambda6,5-benzothiazepin-4-one
(3R)-3-amino-7-(5-tert-butyl-1,3,4-oxadiazol-2-yl)-5-[[6-(cyclopentoxy)-3-pyridyl]methyl]-8-fluoro-1,1-dioxo-2,3-dihydro-1lambda6,5-benzothiazepin-4-one
(3R)-3-amino-5-[(4-chlorophenyl)methyl]-7-[5-[1,1-dimethyl-2-(2-oxo-1-piperidyl)ethyl]-1,3,4-oxadiazol-2-yl]-8-fluoro-1,1-dioxo-2,3-dihydro-1lambda6,5-benzothiazepin-4-one
2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-2-methyl-propanenitrile;
2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]propanenitrile;
(3R)-3-amino-5-[(4-chlorophenyl)methyl]-8-fluoro-7-[5-(3-methylazetidin-3-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-7-[5-(5,5-difluoro-1-methylsulfonyl-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[5,5-difluoro-1-(2-hydroxyethyl)-3-piperidyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[5-[1-(hydroxymethyl)cyclopropyl]-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(cyclobutylamino)-1,3,4-oxadiazol-2-yl]-8-fluoro-1,1-dioxo-2,3-dihydro-1lambda6,5-benzothiazepin-4-one;
(3R)-7-[5-(1-acetyl-5,5-difluoro-3-piperidyl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(5,5-difluoro-3-piperidyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-(3-ethyl-1,2,4-triazol-1-yl)-8-fluoro-1,1-dioxo-2,3-dihydro-1lambda6,5-benzothiazepin-4-one
(3R)-7-[5-(4-acetylpiperazin-1-yl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-5-[(4-chlorophenyl)methyl]-8-fluoro-7-(5-isobutyl-1,3,4-oxadiazol-2-yl)-1,1-dioxo-2,3-dihydro-1lambda6,5-benzothiazepin-4-one
(3R)-3-amino-5-[(4-chlorophenyl)methyl]-8-fluoro-7-[5-(4-methylpiperazin-1-yl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-[2-[2-hydroxyethyl(methyl)amino]-1,1-dimethyl-ethyl]-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-[1,1-dimethyl-2-(2-oxopyrrolidin-1-yl)ethyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(1-methyl-3-piperidyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-[1,1-dimethyl-2-(4-methylpiperazin-1-yl)ethyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[1,1-dimethyl-2-(tetrahydrofuran-3-ylamino)ethyl]-1,3,4-oxadiazol-2-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-5-[(5-isopropoxy-2-pyridyl)methyl]-1,1-dioxo-2,3-dihydro-1lambda6,5-benzothiazepin-4-one;
(3R)-3-amino-5-[(4-chlorophenyl)methyl]-7-[5-[1,1-dimethyl-2-(oxetan-3-ylamino)ethyl]-1,3,4-oxadiazol-2-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-5-[(3,4-difluorophenyl)methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-5-[(4-chloro-3-fluorophenyl)methyl]-8-fluoro-1,1-dioxo-2,3-dihydro-1lambda6,5-benzothiazepin-4-one;
(3R)-3-amino-5-[(4-chlorophenyl)methyl]-7-[5-(4,4-difluoro-1-piperidyl)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-7-[5-(1,1-dimethyl-2-morpholino-ethyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-[2-(dimethylamino)-1,1-dimethyl-ethyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-[5-(6,6-difluoro-2-azaspiro[3.3]heptan-2-yl)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-8-fluoro-5-[(6-isopropoxy-3-pyridyl)methyl]-1,1-dioxo-2,3-dihydro-1lambda6,5-benzothiazepin-4-one;
(3R)-3-amino-5-[(4-chlorophenyl)methyl]-7-[5-[2-(4,4-difluoro-1-piperidyl)-1,1-dimethyl-ethyl]-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-8-fluoro-7-[2-(2-hydroxy-1,1-dimethylethyl)tetrazol-5-yl]-1,1-dioxo-2,3-dihydro-1lambda6,5-benzothiazepin-4-one;
(3R)-3-amino-5-[(4-chlorophenyl)methyl]-8-fluoro-7-[2-(2-hydroxy-2-methylpropyl)tetrazol-5-yl]-1,1-dioxo-2,3-dihydro-1lambda6,5-benzothiazepin-4-one;
(3R)-3-amino-5-[(4-chlorophenyl)methyl]-7-[5-(cyclopropylamino)-1,3,4-oxadiazol-2-yl]-8-fluoro-1,1-dioxo-2,3-dihydro-llambda6,5-benzothiazepin-4-one;1,1,1,3,3,3-hexafluoropropan-2-ol;
(3R)-3-amino-5-[(4-chlorophenyl)methyl]-7-[5-[1,1-dimethyl-2-(1-piperidyl)ethyl]-1,3,4-oxadiazol-2-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-5-[(4-chlorophenyl)methyl]-8-fluoro-1-oxo-2,3-dihydro-1lambda4,5-benzothiazepin-4-one;
(3R)-3-amino-7-(5-tert-butyl-1,3,4-oxadiazol-2-yl)-5-[(4-chlorophenyl)methyl]-8-fluoro-1-oxo-2,3-dihydro-1lambda4,5-benzothiazepin-4-one
(3R)-3-amino-5-[(4-chlorophenyl)methyl]-7-[5-(2,2-difluorospiro[3.3]heptan-6-yl)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-7-[5-(dimethylamino)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-8-fluoro-7-[5-[(3-methylazetidin-1-yl)methyl]-1,3,4-oxadiazol-2-yl]-2,3-dihydro-1,5-benzothiazepin-4-one;1,1,1,3,3,3-hexafluoropropan-2-ol;
methyl 4-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-4-methyl-piperidine-1-carboxylate;
(3R)-3-amino-5-[(4-chlorophenyl)methyl]-8-fluoro-7-[5-(4-methyl-1-methylsulfonyl-4-piperidyl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(cyclohexylamino)-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-8-fluoro-7-[5-(1-methylcyclopropyl)-1,3,4-oxadiazol-2-yl]-1,1-dioxo-2,3-dihydro-1lambda6,5-benzothiazepin-4-one;1,1,1,3,3,3-hexafluoropropan-2-ol;
(3R)-3-amino-5-[(4-chlorophenyl)methyl]-7-[5-[3-(difluoromethyl)azetidin-3-yl]-1,3,4-oxadiazol-2-yl]-8-fluoro-1,1-dioxo-2,3-dihydro-1lambda6,5-benzothiazepin-4-one;1,1,1,3,3,3-hexafluoropropan-2-ol;
(3R)-3-amino-7-[5-(2-amino-1,1-dimethyl-ethyl)-1,3,4-oxadiazol-2-yl]-5-[(4-chlorophenyl)methyl]-8-fluoro-1,1-dioxo-2,3-dihydro-1lambda6,5-benzothiazepin-4-one
(3R)-3-amino-5-[(4-chlorophenyl)methyl]-8-fluoro-7-[5-(6-oxaspiro[3.4]octan-2-yl)-1,3,4-oxadiazol-2-yl]-1,1-dioxo-2,3-dihydro-1lambda6,5-benzothiazepin-4-one
(3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-5-[(3-fluoro-4-methylsulfonyl-phenyl)methyl]-1,1-dioxo-2,3-dihydro-1lambda6,5-benzothiazepin-4-one;
(3R)-3-amino-7-(5-tert-butyl-1,3,4-oxadiazol-2-yl)-5-[(4-ethyloxazol-2-yl)methyl]-8-fluoro-1,1-dioxo-2,3-dihydro-1lambda6,5-benzothiazepin-4-one
(3R)-3-amino-7-(5-tert-butyl-1,3,4-oxadiazol-2-yl)-8-fluoro-1-imino-1-oxo-5-[[4-(trifluoromethoxy)phenyl]methyl]-2,3-dihydro-1lambda6,5-benzothiazepin-4-one;
(3R)-3-amino-7-(5-tert-butyl-1,3,4-oxadiazol-2-yl)-8-fluoro-5-[(2-fluoro-4-methylsulfonyl-phenyl)methyl]-1,1-dioxo-2,3-dihydro-1lambda6,5-benzothiazepin-4-one;1,1,1,3,3,3-hexafluoropropan-2-ol;
2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-N,N,2-trimethyl-propanamide;1,1,1,3,3,3-hexafluoropropan-2-ol;
(3R)-3-amino-5-[(4-chlorophenyl)methyl]-8-fluoro-7-[3-(2-hydroxy-1,1-dimethyl-ethyl)-1,2,4-oxadiazol-5-yl]-1,1-dioxo-2,3-dihydro-1lambda6,5-benzothiazepin-4-one;
(3R)-3-amino-7-(5-tert-butyl-1,3,4-oxadiazol-2-yl)-8-fluoro-5-[[4-(3-hydroxyoxetan-3-yl)phenyl]methyl]-1,1-dioxo-2,3-dihydro-1lambda6,5-benzothiazepin-4-one
(3R)-3-amino-5-[(4-chlorophenyl)methyl]-8-fluoro-7-[5-(2-hydroxy-1,1-dimethyl-ethyl)-1,2,4-oxadiazol-3-yl]-1,1-dioxo-2,3-dihydro-1lambda6,5-benzothiazepin-4-one;2,2,2-trifluoroacetic acid
(3R)-3-amino-5-[(4-chlorophenyl)methyl]-8-fluoro-7-[5-(2-hydroxy-1,1-dimethyl-ethyl)-1,2,4-oxadiazol-3-yl]-1,1-dioxo-2,3-dihydro-1lambda6,5-benzothiazepin-4-one
(3R)-3-amino-7-(5-tert-butyl-1,3,4-oxadiazol-2-yl)-8-fluoro-1,1-dioxo-5-[(4-phenoxyphenyl)methyl]-2,3-dihydro-1lambda6,5-benzothiazepin-4-one;
(3R)-3-amino-7-(5-tert-butyl-1,3,4-oxadiazol-2-yl)-8-fluoro-1,1-dioxo-5-[[4-(1,1,2,2-tetrafluoroethoxy)phenyl]methyl]-2,3-dihydro-1lambda6,5-benzothiazepin-4-one;
(3R)-3-amino-7-(5-tert-butyl-1,3,4-oxadiazol-2-yl)-8-fluoro-5-[[4-(2-hydroxyethoxy)phenyl]methyl]-1,1-dioxo-2,3-dihydro-1lambda6,5-benzothiazepin-4-one;2,2,2-trifluoroacetic acid
(3R)-3-amino-7-(5-tert-butyl-1,3,4-oxadiazol-2-yl)-5-[(4-chlorophenyl)methyl]-8-fluoro-9-methyl-1,1-dioxo-2,3-dihydro-1lambda6,5-benzothiazepin-4-one
(3R)-3-amino-7-(5-tert-butyl-1,3,4-oxadiazol-2-yl)-5-[[1-(cyclopropylmethyl)pyrazol-4-yl]methyl]-8-fluoro-1,1-dioxo-2,3-dihydro-1lambda6,5-benzothiazepin-4-one;2,2,2-trifluoroacetic acid
(3R)-7-[5-(1-acetyl-4-methyl-4-piperidyl)-1,3,4-oxadiazol-2-yl]-3-amino-5-[(4-chlorophenyl)methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-8-fluoro-1-methylimino-1-oxo-5-[[4-(trifluoromethoxy)phenyl]methyl]-2,3-dihydro-1lambda6,5-benzothiazepin-4-one;
2-[5-[(3R)-3-amino-5-[(4-chlorophenyl)methyl]-8-fluoro-1,1,4-trioxo-2,3-dihydro-1lambda6,5-benzothiazepin-7-yl]-1,3,4-oxadiazol-2-yl]-N,2-dimethyl-propanamide;
(3R)-3-amino-7-(5-tert-butyl-1,3,4-oxadiazol-2-yl)-5-[(2-cyclopropylpyrimidin-5-yl)methyl]-8-fluoro-1,1-dioxo-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-5-[(6-chloro-3-pyridyl)methyl]-8-fluoro-1,1-dioxo-2,3-dihydro-1lambda6,5-benzothiazepin-4-one;1,1,1,3,3,3-hexafluoropropan-2-ol;
(2R,3R)-3-amino-7-(5-tert-butyl-1,3,4-oxadiazol-2-yl)-5-[(4-chlorophenyl)methyl]-8-fluoro-2-methyl-1,1-dioxo-2,3-dihydro-1lambda6,5-benzothiazepin-4-one;1,1,1,3,3,3-hexafluoropropan-2-ol;
(3R)-3-amino-7-(5-tert-butyl-1,3,4-oxadiazol-2-yl)-5-[[5-[(4-chlorophenyl)methyl]-3-pyridyl]methyl]-8-fluoro-1,1-dioxo-2,3-dihydro-1lambda6,5-benzothiazepin-4-one;
(3R)-3-amino-5-[(4-chlorophenyl)methyl]-8-fluoro-7-[5-(4-methyl-4-piperidyl)-1,3,4-oxadiazol-2-yl]-1,1-dioxo-2,3-dihydro-1lambda6,5-benzothiazepin-4-one;
(3R)-3-amino-5-[(4-chlorophenyl)methyl]-8-fluoro-7-[5-(4-methyltetrahydropyran-4-yl)-1,3,4-oxadiazol-2-yl]-1,1-dioxo-2,3-dihydro-1lambda6,5-benzothiazepin-4-one;2,2,2-trifluoroacetic acid
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1lambda4,5-benzothiazepin-4-one;
(3R)-3-amino-7-(5-tert-butyl-1,3,4-oxadiazol-2-yl)-8-fluoro-1-imino-1-oxo-5-[[4-(trifluoromethoxy)phenyl]methyl]-2,3-dihydro-1lambda6,5-benzothiazepin-4-one;
(3R)-3-amino-7-(5-tert-butyl-1,3,4-oxadiazol-2-yl)-8-chloro-5-[(4-chlorophenyl)methyl]-1,1-dioxo-2,3-dihydro-1lambda6,5-benzothiazepin-4-one;
2-[4-[[(3R)-3-amino-7-(5-tert-butyl-1,3,4-oxadiazol-2-yl)-8-fluoro-1,1,4-trioxo-2,3-dihydro-1lambda6,5-benzothiazepin-5-yl]methyl]phenoxy]acetamide;1,1,1,3,3,3-hexafluoropropan-2-ol;
N-[11-[4-[[(3R)-3-amino-7-(5-tert-butyl-1,3,4-oxadiazol-2-yl)-8-fluoro-1,1,4-trioxo-2,3-dihydro-1lambda6,5-benzothiazepin-5-yl]methyl]phenoxy]undecyl]acetamide;
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]-2-(methylamino)propanamide
(2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]butanamide
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1lambda6,5-benzothiazepin-3-yl]-3-hydroxy-2-(methylamino)propanamide
(3R)-3-amino-7-(5-tert-butyl-1,3,4-oxadiazol-2-yl)-5-[[4-(cyclopentoxy)phenyl]methyl]-8-fluoro-1,1-dioxo-2,3-dihydro-1lambda6,5-benzothiazepin-4-one;
(3R)-3-amino-7-(5-tert-butyl-1,3,4-oxadiazol-2-yl)-8-fluoro-5-[(4-isopropoxyphenyl)methyl]-1,1-dioxo-2,3-dihydro-1lambda6,5-benzothiazepin-4-one;
(3R)-3-amino-5-[(4-chlorophenyl)methyl]-1,1-dioxo-7-[5-[2,2,2-trideuterio-1,1-bis(trideuteriomethyl)ethyl]-1,3,4-oxadiazol-2-yl]-2,3-dihydro-1lambda6,5-benzothiazepin-4-one;
(3R)-3-amino-5-[(4-chlorophenyl)methyl]-7-[5-(3,3-difluorocyclohexyl)-1,3,4-oxadiazol-2-yl]-8-fluoro-1,1-dioxo-2,3-dihydro-1lambda6,5-benzothiazepin-4-one
(3R)-3-amino-5-[(4-chlorophenyl)methyl]-7-(4-ethyltriazol-1-yl)-8-fluoro-2,3-dihydro-1,5-benzothiazepin-4-one;
(3R)-3-amino-5-[(4-chlorophenyl)methyl]-7-[4-[(4-chlorophenyl)methyl]triazol-1-yl]-8-fluoro-2,3-dihydro-1,5-benzothiazepin-4-one;
(3R)-3-amino-5-[(4-chlorophenyl)methyl]-7-(4-ethyltriazol-1-yl)-8-fluoro-1,1-dioxo-2,3-dihydro-1λ⁶,5-benzothiazepin-4-one;
(3R)-3-amino-5-[(4-chlorophenyl)methyl]-7-[4-[(4-chlorophenyl)methyl]triazol-1-yl]-8-fluoro-1,1-dioxo-2,3-dihydro-1λ⁶,5-benzothiazepin-4-one;
(3R)-3-amino-7-(4-tert-butyltriazol-1-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(1-ethyltriazol-4-yl)-8-fluoro-1,1-dioxo-2,3-dihydro-1λ⁶,5-benzothiazepin-4-one;
(3R)-3-amino-5-[(4-chlorophenyl)methyl]-7-(2-ethyltetrazol-5-yl)-8-fluoro-1,1-dioxo-2,3-dihydro-1λ⁶,5-benzothiazepin-4-one;
(3R)-3-amino-7-(2-tert-butyltetrazol-5-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-8-fluoro-1,1-dioxo-7-(1H-pyrazol-5-yl)-2,3-dihydro-1λ⁶,5-benzothiazepin-4-one;
(3R)-3-amino-5-[(4-chlorophenyl)methyl]-7-(5-ethyl-1H-1,2, 4-triazol-3-yl)-8-fluoro-1,1-dioxo-2,3-dihydro-1λ⁶,5-benzothiazepin-4-one;
(3R)-3-amino-5-[(4-chlorophenyl)methyl]-1,1-dioxo-7-[3-(2,2,2-trifluoroethyl)-1,2,4-oxadiazol-5-yl]-2,3-dihydropyrido[3,2-b][1,4]thiazepin-4-one;
(3R)-3-amino-5-[(4-chlorophenyl)methyl]-7-(3-cyclopropyl-1,2,4-oxadiazol-5-yl)-8-fluoro-1,1-dioxo-2,3-dihydro-1λ⁶,5-benzothiazepin-4-one;
(3R)-3-Amino-5-[(4-chlorophenyl)methyl]-7-(3-ethyl-1,2,4-oxadiazol-5-yl)-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-(3-propyl-1,2,4-oxadiazol-5-yl)-2,3-dihydro-1λ⁶,5-benzothiazepin-4-one;
(3R)-3-Amino-7-(3-tert-butyl-1,2,4-oxadiazol-5-yl)-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-7-[3-[(4-chlorophenyl)methyl]-1,2,4-oxadiazol-5-yl]-8-fluoro-1,1-dioxo-2,3-dihydro-1λ⁶,5-benzothiazepin-4-one;
(3R)-3-Amino-7-[3-(benzyloxymethyl)-1,2,4-oxadiazol-5-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-8-fluoro-1,1-dioxo-7-[3-(2,2,2-trifluoroethyl)-1,2,4-oxadiazol-5-yl]-2,3-dihydro-1λ⁶,5-benzothiazepin-4-one;
(3R)-3-amino-7-[3-(aminomethyl)-1,2,4-oxadiazol-5-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(3-ethyl-1,2,4-oxadiazol-5-yl)-1,1-dioxo-2,3-dihydro-1λ⁶,5-benzothiazepin-4-one;
(3R)-3-amino-7-(3-tert-butylisoxazol-5-yl)-5-[(4-chlorophenyl)methyl]-8-fluoro-1,1-dioxo-2,3-dihydro-1λ⁶,5-benzothiazepin-4-one;
(3R)-3-amino-7-[5-(2,2-difluorocyclohexyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-amino-7-(5-tert-butyl-1,3,4-oxadiazol-2-yl)-8-fluoro-5-[[4-(trifluoromethoxy)phenyl]methyl]-2,3-dihydro-1,5-benzothiazepin-4-one;
(3R)-3-Amino-7-[5-(3,3-difluorocyclohexyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-Amino-8-fluoro-1,1-dioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-4-one;
(3R)-3-Amino-7-(5-tert-butyl-1,3,4-oxadiazol-2-yl)-8-fluoro-1,1-dioxo-5-[[4-(trifluoromethoxy)phenyl]methyl]-2,3-dihydro-1λ⁶,5-benzothiazepin-4-one;
(2S)-N-[(3R)-8-fluoro-1,1,4-trioxo-7-[5-(2,2,2-trifluoroethyl)-1,3,4-oxadiazol-2-yl]-5-[[4-(trifluoromethoxy)phenyl]methyl]-2,3-dihydro-1λ⁶,5-benzothiazepin-3-yl]-2-(methylamino)propanamide;
(3R)-3-amino-7-(5-tert-butyl-1,3,4-oxadiazol-2-yl)-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-[5-(3,3,3-trifluoropropyl)-1,3,4-oxadiazol-2-yl]-2,3-dihydro-1λ⁶,5-benzothiazepin-4-one;
(3R)-3-Amino-5-[(4-chlorophenyl)methyl]-8-fluoro-1,1-dioxo-7-[5-(2,2,2-trifluoroethyl)-1,3,4-oxadiazol-2-yl]-2,3-dihydro-1λ⁶,5-benzothiazepin-4-one;
(3R)-3-Amino-7-[5-(1-amino-1-methyl-ethyl)-1,3,4-oxadiazol-2-yl]-5-[(4-chlorophenyl)methyl]-8-fluoro-1,1-dioxo-2,3-dihydro-1λ⁶,5-benzothiazepin-4-one;
(3R)-3-Amino-5-[(4-chlorophenyl)methyl]-8-fluoro-7-[5-(1-fluoro-1-methyl-ethyl)-1,3,4-oxadiazol-2-yl]-1,1-dioxo-2,3-dihydro-1λ⁶,5-benzothiazepin-4-one;
(3R)-3-Amino-5-[(4-chlorophenyl)methyl]-7-[5-(2,2-difluoroethyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-(5-ethyl-1,3,4-oxadiazol-2-yl)-8-fluoro-1,1-dioxo-2,3-dihydro-1λ⁶,5-benzothiazepin-4-one;
(3R)-3-Amino-5-[(4-chlorophenyl)methyl]-7-[5-(4,4-difluorocyclohexyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclohexyl)-1,3,4-oxadiazol-2-yl]-8-fluoro-1,1-dioxo-2,3-dihydro-1λ⁶,5-benzothiazepin-4-one:
(3R)-3-Amino-5-[(4-chlorophenyl)methyl]-7-[5-(3,3-difluorocyclopentyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclopentyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-[(3,3-difluorocyclobutyl)methyl]-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-[5-(tetrahydropyran-4-ylmethyl)-1,3,4-oxadiazol-2-yl]-2,3-dihydro-1λ⁶,5-benzothiazepin-4-one;
(3R)-3-Amino-5-[(4-chlorophenyl)methyl]-8-fluoro-1,1-dioxo-7-[5-(4,4,4-trifluorobutyl)-1,3,4-oxadiazol-2-yl]-2,3-dihydro-1λ⁶,5-benzothiazepin-4-one;
(3R)-3-Amino-5-[(4-chlorophenyl)methyl]-7-[5-(3,3-difluorocyclobutyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-7-[5-(2,2-difluorocyclobutyl)-1,3,4-oxadiazol-2-yl]-8-fluoro-1,1-dioxo-2,3-dihydro-1λ⁶,5-benzothiazepin-4-one;
(3R)-3-Amino-5-[(4-chlorophenyl)methyl]-8-fluoro-1,1-dioxo-7-(5-tetrahydropyran-4-yl-1,3,4-oxadiazol-2-yl)-2,3-dihydro-1λ⁶,5-benzothiazepin-4-one;
(3R)-3-Amino-5-[(4-chlorophenyl)methyl]-8-fluoro-7-[5-(2-fluoroethyl)-1,3,4-oxadiazol-2-yl]-1,1-dioxo-2,3-dihydro-1λ⁶,5-benzothiazepin-4-one;
(3R)-3-Amino-5-[(4-chlorophenyl)methyl]-8-fluoro-7-(5-isopropyl-1,3,4-oxadiazol-2-yl)-1,1-dioxo-2,3-dihydro-1λ⁶,5-benzothiazepin-4-one;
(3R)-3-Amino-5-[(4-chlorophenyl)methyl]-7-(5-cyclopropyl-1,3,4-oxadiazol-2-yl)-8-fluoro-1,1-dioxo-2,3-dihydro-1λ⁶,5-benzothiazepin-4-one;
(3R)-3-amino-5-[(4-chlorophenyl)methyl]-8-fluoro-7-[5-(2-hydroxy-1,1-dimethyl-ethyl)-1,3,4-oxadiazol-2-yl]-1,1-dioxo-2,3-dihydro-1λ⁶,5-benzothiazepin-4-one;
(3R)-3-amino-7-[5-(azetidin-1-yl)-1,3,4-oxadiazol-2-yl]-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(4-ethyloxazol-2-yl)-8-fluoro-1,1-dioxo-2,3-dihydro-1λ⁶,5-benzothiazepin-4-one;
(3R)-3-amino-5-[(4-chlorophenyl)methyl]-6-fluoro-1,1-dioxo-7-[5-(2,2,2-trifluoroethyl)-1,3,4-oxadiazol-2-yl]-2,3-dihydro-1λ⁶,5-benzothiazepin-4-one;
(3R)-3-amino-7-(5-tert-butyl-1,3,4-oxadiazol-2-yl)-5-[(3-chlorophenyl)methyl]-8-fluoro-1,1-dioxo-2,3-dihydro-1λ⁶,5-benzothiazepin-4-one;
4-[[(3R)-3-amino-7-(5-tert-butyl-1,3,4-oxadiazol-2-yl)-8-fluoro-1,1,4-trioxo-2,3-dihydro-1λ⁶,5-benzothiazepin-5-yl]methyl]benzonitrile;
(3R)-3-amino-7-(5-tert-butyl-1,3,4-oxadiazol-2-yl)-8-fluoro-1,1-dioxo-5-[(4-tetrahydrofuran-3-yloxyphenyl)methyl]-2,3-dihydro-1λ⁶,5-benzothiazepin-4-one;
(3R)-3-amino-7-(5-tert-butyl-1,3,4-oxadiazol-2-yl)-5-[[4-(2,2-difluoroethoxy)phenyl]methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-5-[[4-(difluoromethoxy)phenyl]methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-1,1-dioxo-5-(2-pyridylmethyl)-2,3-dihydro-1λ⁶,5-benzothiazepin-4-one;
(3R)-3-amino-7-(5-tert-butyl-1,3,4-oxadiazol-2-yl)-8-fluoro-5-[(6-methoxy-3-pyridyl)methyl]-1,1-dioxo-2,3-dihydro-1λ⁶,5-benzothiazepin-4-one;
(3R)-3-amino-5-[[4-(aminomethyl)phenyl]methyl]-7-(5-tert-butyl-1,3,4-oxadiazol-2-yl)-8-fluoro-1,1-dioxo-2,3-dihydro-1λ⁶,5-benzothiazepin-4-one;
(3R)-3-amino-7-(5-tert-butyl-1,3,4-oxadiazol-2-yl)-5-[(2-chlorophenyl)methyl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(5-methoxy-2-pyridyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-methylsulfonylphenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-methoxyphenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-8-fluoro-5-[(4-fluorophenyl)methyl]-1,1-dioxo-2,3-dihydro-1λ⁶,5-benzothiazepin-4-one;
(3R)-3-amino-5-[[2-(aminomethyl)-4-chloro-phenyl]methyl]-7-(5-tert-butyl-1,3,4-oxadiazol-2-yl)-8-fluoro-1,1-dioxo-2,3-dihydro-1λ⁶,5-benzothiazepin-4-one;
(3R)-3-amino-5-[[4-(11-aminoundecoxy)phenyl]methyl]-7-(5-tert-butyl-1,3,4-oxadiazol-2-yl)-8-fluoro-1,1-dioxo-2,3-dihydro-1λ⁶,5-benzothiazepin-4-one;
N-[(3R)-7-(5-tert-butyl-1,3,4-oxadiazol-2-yl)-5-[(4-chlorophenyl)methyl]-8-fluoro-1,1,4-trioxo-2,3-dihydro-1 *λ⁶,*5,5-benzothiazepin-3-yl]acetamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-hydroxy-butanamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]butanamide;
(2S)-2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
(2S)-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)butanamide;
3-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]propanamide;
N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]-2-(methylamino)acetamide;
2-amino-N-[(3R)-7-(5-tert-butyl-1,3,4-oxadiazol-2-yl)-5-[(4-chlorophenyl)methyl]-8-fluoro-1,1,4-trioxo-2,3-dihydro-1λ⁶,5-benzothiazepin-3-yl]acetamide;
4-amino-N-[(3R)-7-(5-tert-butyl-1,3,4-oxadiazol-2-yl)-5-[(4-chlorophenyl)methyl]-8-fluoro-1, 1,4-trioxo-2,3-dihydro-1λ⁶,5-benzothiazepin-3-yl]butanamide;
(3R)-3-amino-8-bromo-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-methyl-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-hydroxy-1,1-dioxo-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-8-(dimethylamino)-1,1-dioxo-2,3-dihydro-1λ⁶,5-benzothiazepin-4-one;
(3R)-3,8-diamino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-(1-tert-butylpyrazol-4-yl)-5-[(4-chlorophenyl)methyl]-8-fluoro-1,1-dioxo-2,3-dihydro-1λ⁶,5-benzothiazepin-4-one;
((3R)-3-amino-5-[(4-chlorophenyl)methyl]-7-(1-ethylpyrazol-4-yl)-8-fluoro-1,1-dioxo-2,3-dihydro-1λ⁶,5-benzothiazepin-4-one;
(3R)-3-amino-5-[(4-chlorophenyl)methyl]-7-(1-ethylpyrazol-3-yl)-8-fluoro-1,1-dioxo-2,3-dihydro-1λ⁶,5-benzothiazepin-4-one;
(3R)-3-amino-7-(4-tert-butylpyrazol-1-yl)-5-[(4-chlorophenyl)methyl]-8-fluoro-1,1-dioxo-2,3-dihydro-1λ⁶,5-benzothiazepin-4-one;
(3R)-3-amino-7-(3-tert-butylpyrazol-1-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(4-ethylpyrazol-1-yl)-8-fluoro-1,1-dioxo-2,3-dihydro-1λ⁶,5-benzothiazepin-4-one;
(3R)-3-amino-5-[(4-chlorophenyl)methyl]-7-(1-ethyl-1,2,4-triazol-3-yl)-8-fluoro-1,1-dioxo-2,3-dihydro-1λ⁶,5-benzothiazepin-4-one;
(3R)-3-amino-7-(1-tert-butyl-1,2,4-triazol-3-yl)-5-[(4-chlorophenyl)methyl]-8-fluoro-1,1-dioxo-2,3-dihydro-1λ⁶,5-benzothiazepin-4-one;
(3R)-3-amino-7-(1-tert-butylpyrazol-3-yl)-5-[(4-chlorophenyl)methyl]-8-fluoro-1,1-dioxo-2,3-dihydro-1λ⁶,5-benzothiazepin-4-one;
(3R)-3-amino-7-(5-tert-butyl-1,2,4-oxadiazol-3-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(5-ethyl-1,2,4-oxadiazol-3-yl)-8-fluoro-1,1-dioxo-2,3-dihydro-1λ⁶,5-benzothiazepin-4-one;
(3R)-3-amino-7-[5-(1,1-dimethylbut-3-ynyl)-1,3,4-oxadiazol-2-yl]-8-fluoro-1,1-dioxo-5-[[4-[3-(trifluoromethyl)diazirin-3-yl]phenyl]methyl]-2,3-dihydro-1λ⁶,5-benzothiazepin-4-one;
(3R)-3-amino-7-(5-tert-butyl-1,3,4-oxadiazol-2-yl)-1,1-dioxo-5-[[4-(trifluoromethoxy)phenyl]methyl]-2,3-dihydro-1λ⁶,5-benzothiazepin-4-one;
(3R)-3-amino-7-(5-tert-butyl-1,3,4-oxadiazol-2-yl)-5-[(4-chlorophenyl)methyl]-1,1-dioxo-2,3-dihydro-1λ⁶,5-benzothiazepin-4-one;
(3R)-3-amino-7-[5-(1,1-dimethylbut-3-ynyl)-1,3,4-oxadiazol-2-yl]-8-fluoro-1,1-dioxo-5-[[4-(trifluoromethoxy)phenyl]methyl]-2,3-dihydro-1λ⁶,5-benzothiazepin-4-one;
(3R)-3-amino-5-[(4-chlorophenyl)methyl]-7-[5-(5,5-difluoro-1-methyl-3-piperidyl)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
(3R)-3-amino-7-(5-tert-butyl-1,3,4-oxadiazol-2-yl)-8-fluoro-1-oxo-5-[[4-(trifluoromethoxy)phenyl]methyl]-2,3-dihydro-1λ⁴,5-benzothiazepin-4-one;
3R)-3-amino-5-[(4-chlorophenyl)methyl]-8-fluoro-7-(5-morpholino-1,3,4-oxadiazol-2-yl)-1,1-dioxo-2,3-dihydro-1λ⁶,5-benzothiazepin-4-one;
(3R)-3-amino-5-[(4-chlorophenyl)methyl]-7-[5-(cyclopentylamino)-1,3,4-oxadiazol-2-yl]-8-fluoro-1,1-dioxo-2,3-dihydro-1λ⁶,5-benzothiazepin-4-one;
(3R)-3-amino-7-(5-tert-butyl-1,3,4-thiadiazol-2-yl)-5-[(4-chlorophenyl)methyl]-8-fluoro-1,1-dioxo-2,3-dihydro-1λ⁶,5-benzothiazepin-4-one;
(3R)-3-amino-5-[(4-chlorophenyl)methyl]-7-(5-ethyltetrazol-2-yl)-8-fluoro-1,1-dioxo-2,3-dihydro-1λ⁶,5-benzothiazepin-4-one;
or a pharmaceutically acceptable salt thereof.

19. A process for the preparation of a compound according to any one of claims 1 to 18, or a pharmaceutically acceptable salt thereof, comprising
reacting a compound of formula (IX) 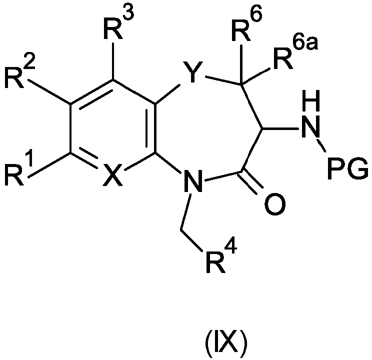
wherein X, Y, R¹, R², R³, R⁴, R⁶, R^{6a} are as defined in any one of claims 1 to 17 and PG is an amino protecting group,
with a suitable deprotection agent to form said compound of formula (I), or a pharmaceutically acceptable salt thereof, wherein X, Y, R¹, R², R³, R⁴, R⁶, R^{6a} are as defined in any one of claims 1 to 17 and R⁵ is hydrogen; or
reacting a compound of formula (I') 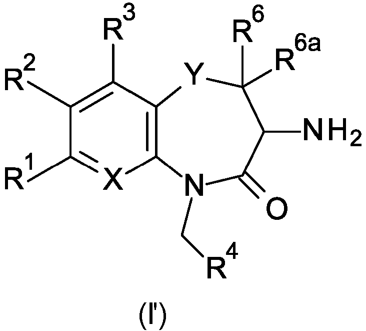
wherein X, Y, R¹, R², R³, R⁴, R⁶, R^{6a} are as defined in any one of claims 1 to 34 and R⁵ is hydrogen,
with a carboxylic acid derivative of formula R⁹CO₂H wherein R⁹ is as defined in any one of claims 1 to 15, in the presence of a base to form a compound of formula (I), or a pharmaceutically acceptable salt thereof, wherein R⁵ is -C(O)(R⁹).

20. The compound according to any one of claims 1 to 18, or a pharmaceutically acceptable salt thereof, for use as therapeutically active substance.

21. A pharmaceutical composition comprising a compound according to any one of claims 1 to 18, or a pharmaceutically acceptable salt thereof, and a pharmaceutically acceptable excipient.

22. The compound according to any one of claims 1 to 18, or a pharmaceutically acceptable salt thereof, for use in the treatment, prevention and/or delay of progression of cancer.

23. The compound for use according to claim 22, wherein the cancer is associated with aberrant diacylglycerol kinase signaling, wherein the diacylglycerol kinase is selected from DGKα and/or DGKζ.

24. The compound for use according to claim 22 or 23, wherein the cancer is selected from the group consisting of B-cell acute lymphoid leukemia, T-cell acute lymphoid leukemia, acute lymphoid leukemia, chronic myelogenous leukemia, chronic lymphocytic leukemia B-cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom's macroglobulinemia, preleukemia, sarcoma, carcinoma, melanoma, neuroblastoma, renal cell carcinoma, colon cancer, colorectal cancer, breast cancer, epithelial squamous cell cancer, melanoma, stomach cancer, brain cancer, lung cancer (e.g., NSCLC), pancreatic cancer, cervical cancer, ovarian cancer, liver cancer, bladder cancer, prostate cancer, testicular cancer, thyroid cancer, uterine cancer, adrenal cancer and head and neck cancer.

## Patentansprüche

1. Verbindung der Formel (I) 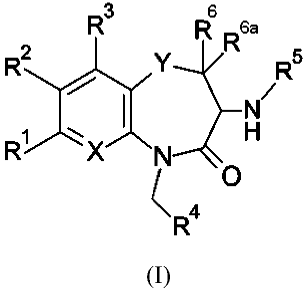 oder ein pharmazeutisch verträgliches Salz davon, wobei:
X C(R⁷) oder N ist;
Y S, S(O), S(O)₂, S(O)N(R^{y}) ist,
R¹ 5-gliedriges Heteroaryl ist, wobei R¹ gegebenenfalls mit einem oder mehreren R¹⁰ substituiert ist, die gleich oder verschieden sein können;
R², R³ und R⁷ jeweils unabhängig voneinander ausgewählt sind aus Wasserstoff, Halogen, C₁₋₆-Alkyl, C₂₋₆-Alkinyl, Hydroxy, Cyano, Halogen-C₁₋₆-alkyl, N(R⁸R^{8a}), C₁₋₆-Alkoxy, C₃₋₇-Cycloalkyl und 3-10-gliedrigem Heterocyclyl;
R⁴ ausgewählt ist aus C₅₋₁₄-Aryl und 5-14-gliedrigem Heteroaryl, wobei R⁴ gegebenenfalls mit einem oder mehreren R¹¹ substituiert ist, die gleich oder verschieden sein können;
R⁵ ausgewählt ist aus Wasserstoff, C₁₋₆-Alkyl, -C(O)(R⁹), Amino, Amino-C₁₋₆-alkyl und C₃₋₇-Cycloalkyl,
wobei das C₃₋₇-Cycloalkyl gegebenenfalls mit einem oder mehreren C₁₋₆-Alkyl, Amino, Amino-C₁₋₆-alkyl- substituiert ist;
R⁶ und R^{6a} jeweils unabhängig voneinander ausgewählt sind aus Wasserstoff und C₁₋₆-Alkyl;
R⁸ und R^{8a} jeweils unabhängig voneinander ausgewählt sind aus Wasserstoff und C₁₋₆-Alkyl;
R⁹ ausgewählt ist aus C₁₋₆-Alkyl, Hydroxy-C₁₋₆-alkyl, Amino-C₁₋₆-alkyl- und -(C₁₋₆-Alkyl)-N(R^{9a}R^{9b}), wobei das Amino-C₁₋₆-alkyl gegebenenfalls mit einem oder mehreren Hydroxy, Hydroxy-C₁₋₆-alkyl- substituiert ist;
R^{9a} und R^{9b} jeweils unabhängig voneinander ausgewählt sind aus Wasserstoff und C₁₋₆-Alkyl, wobei das C₁₋₆-Alkyl gegebenenfalls mit einem oder mehreren Hydroxy substituiert ist;
oder R^{9a} und R^{9b} zusammen mit dem Stickstoffatom, an das sie gebunden sind, ein 3-10-gliedriges Heterocyclyl bilden;
R¹⁰ ausgewählt ist aus:
i) C₁₋₁₀-Alkyl, gegebenenfalls substituiert mit einem oder mehreren Halogen, C₂₋₆-Alkinyl, Halogen-C₁₋₆-alkyl, Amino, Hydroxy, 5-6-gliedrigem Heteroaryl, C₁₋₆-Halogenalkoxy, C₁₋₆-Alkoxy, 3-10-gliedrigem Cycloalkyl, C₁₋₃-Alkyl, -N(R^{10a}R^{10b}), -S(O)₂(C₁₋₆-Alkyl), -S(O)₂(C₁₋₆-Cycloalkyl), 3-10-gliedrigem Heterocyclyl, Phenyl, Cyano, -C(O)N(R^{10c}R^{10d}), wobei 3-10-gliedriges Heterocyclyl, 3-10-gliedriges Cycloalkyl und Phenyl gegebenenfalls mit einem oder mehreren C₁₋₁₀-Alkyl, C₁₋₁₀-Alkoxy, -S(O)₂(C₁₋₆-Alkyl), Oxo, Halogen, C₂₋₆-Alkinyl, 3-10-gliedrigem Cycloalkyl substituiert sind;
ii) C₃₋₁₀-Cycloalkyl, gegebenenfalls substituiert mit einem oder mehreren Halogen, C₁₋₁₀-Alkyl, -S(O)₂(C₁₋₆-Alkyl), Cyano, C₁₋₆-Halogenalkyl, C₁₋₆-Alkoxy, Hydroxy, Oxo, Amino, -C(O)N(R^{10c}R^{10d}), =N(OH), Hydroxy-C₁₋₆-alkyl;
iii) 3-10-gliedrigem Heterocyclyl, gegebenenfalls substituiert mit einem oder mehreren Halogen, C₁₋₁₀-Alkyl, -S(O)₂(C₁₋₆-Alkyl), -C₁₋₁₀-Alkyl-C₁₋₄-alkoxy, Amino, -C(O)N(R^{10h}R¹⁰ⁱ), C₁₋₆-Halogenalkyl, C₁₋₆-Alkoxy, Cyano, Hydroxy-C₁₋₁₀-alkyl, Oxo, -C(O)(C₁₋₆-Alkyl), -C(O)O-(R^{10q}), C₃₋₁₀-Cycloalkyl;
iv) Phenyl, gegebenenfalls substituiert mit einem oder mehreren Halogen, C₁₋₁₀-Alkyl, -S(O)₂(C₁₋₆-Alkyl);
v) -N(R^{10e}R^{10f});
vi) -OR^{10g};
vii) -C(O)NR^{10h}R¹⁰ⁱ;
viii) Heteroaryl, gegebenenfalls substituiert mit einem oder mehreren C₁₋₁₀-Alkyl, Halogen, -SO₂(C₁₋₆-Alkyl); und
ix) Oxo;
R^{10a} und R^{10b} jeweils unabhängig voneinander ausgewählt sind aus Wasserstoff, C₁₋₆-Alkyl, Halogen-C₁₋₆-alkyl, Hydroxy-C₁₋₆-alkyl, C₁₋₆-Alkoxy, -C(O)(R^{10j}), Amino-C₁₋₆-alkyl-, 3-10-gliedrigem Heterocyclyl, -SO₂(R^{10k}), -C₁₋₆-Alkyl-SO₂(R^{10k}) und -N(R^{10l}R^{10m}), wobei 3-10-gliedriges Heterocyclyl gegebenenfalls mit C₁₋₆-Alkyl substituiert ist;
oder R^{10a} und R^{10b} zusammen mit dem Stickstoffatom, an das sie gebunden sind, ein 3-10-gliedriges Heterocyclyl bilden, das gegebenenfalls mit einem oder mehreren Halogen, C₁₋₆-Alkyl substituiert ist;
R^{10c} und R^{10d} jeweils unabhängig voneinander ausgewählt sind aus Wasserstoff und C₁₋₆-Alkyl;
R^{10e} und R^{10f} jeweils unabhängig voneinander ausgewählt sind aus:
i) Wasserstoff;
ii) C₁₋₆-Alkyl, gegebenenfalls substituiert mit einem oder mehreren Cyano, insbesondere einem Cyano, Halogen, Hydroxy;
iii) -C(O)R¹⁰ⁿ;
iv) -C₁₋₁₀-Alkyl((O-C₁₋₁₀-Alkyl)ₘ), wobei m eine ganze Zahl zwischen 1 und 5 ist (insbesondere ist m 2 oder 3), gegebenenfalls substituiert mit einem oder mehreren C₂₋₆-Alkinyl;
v) C₃₋₁₀-Cycloalkyl, gegebenenfalls substituiert mit einem oder mehreren Halogen, Hydroxy, Oxo, C₁₋₁₀-Alkyl, Halogen-C₁₋₁₀-alkyl, Hydroxy-C₁₋₆-alkyl, C₁₋₆-Alkoxy, -C(O)O(R^{10o}), -C(O)(R^{10o}), -C(O)N(R^{10p})(R^{10p'}), - SO₂(C₁₋₆-Alkyl), C₂₋₆-Alkinyl; und
vi) 3-10-gliedrigem Heterocyclyl, gegebenenfalls substituiert mit einem oder mehreren Halogen, Hydroxy, Oxo, C₁₋₁₀-Alkyl, Halogen-C₁₋₁₀-alkyl, Hydroxy-C₁₋₆-alkyl, C₁₋₆-Alkoxy, -C(O)O(R^{10o}), -C(O)(R^{10o}), - C(O)N(R^{10p})(R^{10p'}), -SO₂(C₁₋₆-Alkyl), 3-10-gliedrigem Cycloalkyl;
R^{10g} ausgewählt ist aus Halogen-C₁₋₆-alkyl, Cyano, -C₁₋₁₀-Alkyl-phenyl, -C₁₋₆-Alkyl-C₃₋₇-cycloalkyl und -C₁₋₆-Alkoxy-halogen-C₁₋₆-alkyl;
R^{10h} und R¹⁰ⁱ jeweils unabhängig voneinander ausgewählt sind aus Wasserstoff und C_{1- 6}-Alkyl, C₁₋₆-Halogenalkyl, wobei C₁₋₆-Alkyl und C₁₋₆-Halogenalkyl gegebenenfalls mit einem oder mehreren Hydroxy substituiert sind,
oder R^{10h} und R¹⁰ⁱ zusammen mit dem Stickstoffatom, an das sie gebunden sind, ein 3-10-gliedriges Heterocyclyl bilden, das gegebenenfalls mit einem oder mehreren Halogen, Hydroxy, C₁₋₆-Alkyl substituiert ist;
R^{10j} ausgewählt ist aus C₁₋₆-Alkyl, Halogen-C₁₋₆-alkyl-, Hydroxy-C₁₋₆-alkyl- und Amino-C₁₋₆-alkyl-;
R^{10k} ausgewählt ist aus Wasserstoff, C₁₋₆-Alkyl und Halogen-C₁₋₆-alkyl;
R^{10l} und R^{10m} jeweils unabhängig voneinander ausgewählt sind aus Wasserstoff und C₁₋₆-Alkyl;
R¹⁰ⁿ ausgewählt ist aus C₁₋₆-Alkyl, Amino-C₁₋₆-alkyl-, Halogen-C₁₋₆-alkyl, C₃₋₇-Cycloalkyl und 3-10-gliedrigem Heterocyclyl, wobei das C₃₋₇-Cycloalkyl und 3-10-gliedrige Heterocyclyl gegebenenfalls mit einem oder mehreren Halogen, C₁₋₆-Alkyl substituiert sind;
R^{10o} ausgewählt ist aus C₁₋₆-Alkyl und Halogen-C₁₋₆-alkyl;
R^{10p} und R^{10p'} unabhängig voneinander ausgewählt sind aus Wasserstoff, C₁₋₆-Alkyl und Halogen-C₁₋₆-alkyl;
R^{10q} C₁₋₁₀-Alkyl, C₁₋₆-Halogenalkyl, Aryl, Heteroaryl ist, wobei Aryl und Heteroaryl gegebenenfalls mit einem oder mehreren Halogen, C₁₋₁₀-Alkyl substituiert sind;
R¹¹ ausgewählt ist aus:
i) Halogen;
ii) Hydroxy;
iii) Cyano;
iv) C₁₋₁₀-Alkyl, gegebenenfalls substituiert mit einem oder mehreren Cyano, Halogen, Hydroxyl, C₃₋₇-Cycloalkyl, Amino, Aryl, -O-Aryl, 5-6-gliedrigem Heteroaryl, 3-7-gliedrigem Heterocyclyl, wobei 3-7-gliedriges Heterocyclyl und Aryl gegebenenfalls mit einem oder mehreren C₁₋₆-Alkyl, -SO₂(C₁₋₆-Alkyl), Hydroxy, Halogen, Cyano substituiert sind;
v) C₁₋₆-Alkoxy, gegebenenfalls substituiert mit einem oder mehreren, insbesondere einem, 3-10-gliedrigem Heterocyclyl, Halogen;
vi) C₃₋₇-Cycloalkyl;
vii) 3-10-gliedrigem Heterocyclyl, gegebenenfalls substituiert mit einem oder mehreren Halogen-C₁₋₆-alkyl, Hydroxy, C₁₋₆-Alkyl, C₃₋₁₀-Cycloalkyl, C₁₋₆-Alkoxy, Oxo;
viii) 5-6-gliedrigem Heteroaryl, gegebenenfalls substituiert mit einem oder mehreren C₁₋₆-Alkyl, 3-10-gliedrigem Cycloalkyl, Halogen, Halogen-C₁₋₆-alkyl, C₁₋₆-Alkoxy, C₁₋₆-Halogenalkoxy;
ix) Phenyl, gegebenenfalls substituiert mit einem oder mehreren Halogen, Cyano, C₁₋₆-Alkoxy, C₁₋₆-Halogenalkyl, C₁₋₆-Halogenalkoxy, C₁₋₆-Alkyl;
x) -O(R^{11a});
xi) -C(O)N(R^{11b}R^{11c});
xii) -SO₂(R^{11d});
xiii) -C(O)OR^{11e};
xiv) -C(O)R^{11f};
xv) Oxo;
xvi) -N(R^{11g}R^{11h}); und
xvii) -S(R^{11k});
R^{11a} ausgewählt ist aus C₁₋₁₂-Alkyl, Halogen-C₁₋₆-alkyl, Amino-C₁₋₁₂-alkyl-, Hydroxy-C₁₋₆-alkyl-, Cyano, C₂₋₆-Alkinyl, C₃₋₇-Cycloalkyl, 3-10-gliedrigem Heterocyclyl, 3-10-gliedrigem -(C₁₋₆-Alkyl)heterocyclyl, 5-6-gliedrigem Heteroaryl, Phenyl, -C₁₋₆-Alkyl-phenyl, -C₁₋₁₂-Alkyl-C(O)N(R¹¹ⁱR^{11j}), -C₁₋₁₂-Alkyl-NH-C(O)(C₁₋₆-alkyl), -C₁₋₁₂-Alkoxy-NH-C(O)(C₁₋₆-alkyl), -C₁₋₆-Alkyl-NH-C(O)(C₁₋₆-alkyl), -(CH₂CH₂O)ₙ-CH₂CH₂NH₂ und -(CH₂CH₂O)ₙ-CH₂CH₂-NH-C(O)(C₁₋₆-alkyl);
wobei das C₁₋₁₂-Alkyl, C₃₋₇-Cycloalkyl, 3-10-gliedrige Heterocyclyl, 5-6-gliedrige Heteroaryl, Phenyl und -C₁₋₆-Alkyl-phenyl gegebenenfalls mit einem oder mehreren Halogen, C₁₋₆-Alkyl, Halogen-C₁₋₆-alkyl, C₁₋₆-Alkoxyl, C₁₋₆-Halogenalkoxyl, Cyano substituiert sind;
n eine ganze Zahl zwischen 1 und 6 ist, insbesondere ist n zwei oder drei;
R^{11b} und R^{11c} jeweils unabhängig voneinander ausgewählt sind aus Wasserstoff, C₁₋₆-Alkyl und Halogen-C₁₋₆-alkyl,
oder R^{11b} und R^{11c} zusammen mit dem Stickstoffatom, an das sie gebunden sind, ein 3-10-gliedriges Heterocyclyl bilden;
R^{11d} ausgewählt ist aus Wasserstoff, C₁₋₆-Alkyl, -N(R^{11l}R^{11m}), Halogen-C₁₋₆-alkyl und Phenyl;
R^{11e} ausgewählt ist aus Wasserstoff und C₁₋₆-Alkyl;
R^{11f} ausgewählt ist aus Wasserstoff, C₁₋₆-Alkyl und Phenyl;
R^{11g} und R^{11h} jeweils unabhängig voneinander ausgewählt sind aus Wasserstoff, C₁₋₆-Alkyl, -(C₁₋₆-Alkyl)phenyl, Halogen-C₁₋₆-alkyl, -SO₂(C₁₋₆-Alkyl), -SO₂(Halogen-C₁₋₆-alkyl) und -SO(C₁₋₆-Alkyl)₂,
R¹¹ⁱR^{11j} jeweils unabhängig voneinander ausgewählt sind aus Wasserstoff, C₁₋₆-Alkyl und Halogen-C₁₋₆-alkyl,
oder R¹¹ⁱR^{11j} zusammen mit dem Stickstoffatom, an das sie gebunden sind, ein 3-10-gliedriges Heterocyclyl bilden;
R^{11k} ausgewählt ist aus Wasserstoff, C₁₋₆-Alkyl und Halogen-C₁₋₆-alkyl;
R^{11l} und R^{11m} jeweils unabhängig voneinander ausgewählt sind aus Wasserstoff und C₁₋₆-Alkyl,
oder R^{11l} und R^{11m} zusammen mit dem Stickstoffatom, an das sie gebunden sind, ein 3-10-gliedriges Heterocyclyl bilden;
R^{y} ausgewählt ist aus Wasserstoff und C₁₋₆-Alkyl.

2. Verbindung nach Anspruch 1 oder ein pharmazeutisch verträgliches Salz davon, wobei die Verbindung eine Verbindung der Formel (IA) ist 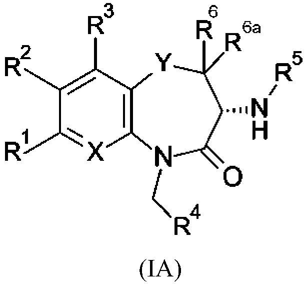 wobei X, Y, R¹, R², R³, R⁴, R⁵, R⁶, R^{6a} wie in einem der Ansprüche 1 bis 3 definiert sind.

3. Verbindung nach Anspruch 1 oder 2, wobei R¹ mit mindestens einem R¹⁰, insbesondere mit einem R¹⁰, substituiert ist.

4. Verbindung nach einem der Ansprüche 1 bis 3 oder ein pharmazeutisch verträgliches Salz davon, wobei X N oder C(R⁷) ist, wobei R⁷ Wasserstoff oder Halogen ist.

5. Verbindung nach einem der Ansprüche 1 bis 4 oder ein pharmazeutisch verträgliches Salz davon, wobei Y S(O) oder S(O)₂ ist.

6. Verbindung nach einem der Ansprüche 1 bis 5 oder ein pharmazeutisch verträgliches Salz davon, wobei R¹⁰ ausgewählt ist aus:
i) C₁₋₁₀-Alkyl, gegebenenfalls substituiert mit einem oder mehreren C₂₋₆-Alkinyl, Cyano;
ii) C₁₋₁₀-Halogenalkyl, gegebenenfalls substituiert mit C₁₋₅-Alkoxy, Amino, Phenyl, wobei Phenyl gegebenenfalls mit einem oder mehreren Halogen substituiert ist;
iii) C₃₋₁₀-Cycloalkyl, gegebenenfalls substituiert mit einem oder mehreren Halogen;
iv) Phenyl, gegebenenfalls substituiert mit einem oder mehreren Halogen, C₁₋₁₀-Alkyl;
v) Heteroaryl, gegebenenfalls substituiert mit einem oder mehreren Halogen, C₁₋₁₀-Alkyl;
vi) 3-10-gliedrigem Heterocyclyl, gegebenenfalls substituiert mit einem oder mehreren C₁₋₁₀-Alkyl, C₃₋₁₀-Cycloalkyl, C₁₋₆-Halogenalkyl, Halogen, -C₁₋₁₀-Alkyl-C₁₋₄-alkoxy, -C(O)O-C₁₋₅-Alkyl;
vii) Amino, gegebenenfalls substituiert mit C₁₋₁₀-Alkyl, C₁₋₁₀-Halogenalkyl, C₃₋₁₀-Cycloalkyl, wobei C₃₋₁₀-Cycloalkyl gegebenenfalls mit einem oder mehreren C₁₋₅-Alkyl, Halogen substituiert ist.

7. Verbindung nach einem der Ansprüche 1 bis 6 oder ein pharmazeutisch verträgliches Salz davon, wobei R¹ ausgewählt ist aus 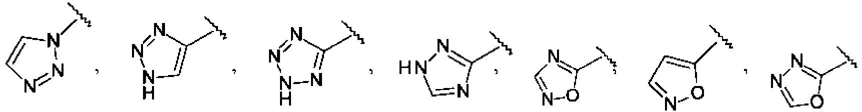 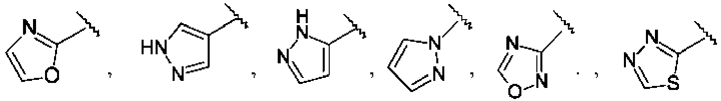 und 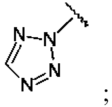 wobei R¹ gegebenenfalls mit einem oder mehreren R¹⁰ substituiert ist, die gleich oder verschieden sein können, wie in einem der Ansprüche 1 oder 6 bis 7 definiert.

8. Verbindung nach einem der Ansprüche 1 bis 7 oder ein pharmazeutisch verträgliches Salz davon, wobei R² Wasserstoff, Halogen, C₁₋₆-Alkyl, Hydroxy oder N(R⁸R^{8a}) ist.

9. Verbindung nach einem der Ansprüche 1 bis 8 oder ein pharmazeutisch verträgliches Salz davon, wobei R⁸ und R^{8a} jeweils unabhängig voneinander ausgewählt sind aus Wasserstoff und C₁₋₆-Alkyl.

10. Verbindung nach einem der Ansprüche 1 bis 9 oder ein pharmazeutisch verträgliches Salz davon, wobei R³ Wasserstoff ist.

11. Verbindung nach einem der Ansprüche 1 bis 10 oder ein pharmazeutisch verträgliches Salz davon, wobei R¹¹ ausgewählt ist aus Halogen, C₁₋₆-Halogenalkoxy, C₁₋₆-Alkoxy, -O-R^{11a}, C₁₋₆-Alkyl, mit Cyano substituiertem C₁₋₆-Alkyl.

12. Verbindung nach einem der Ansprüche 1 bis 11 oder ein pharmazeutisch verträgliches Salz davon, wobei R^{11a} ausgewählt ist aus C₁₋₁₂-Alkyl, Halogen-C₁₋₆-alkyl, 3-7-gliedrigem Heterocycloalkyl und Amino-C₁₋₁₂-alkyl.

13. Verbindung nach einem der Ansprüche 1 bis 12 oder ein pharmazeutisch verträgliches Salz davon, wobei R⁴ ausgewählt ist aus 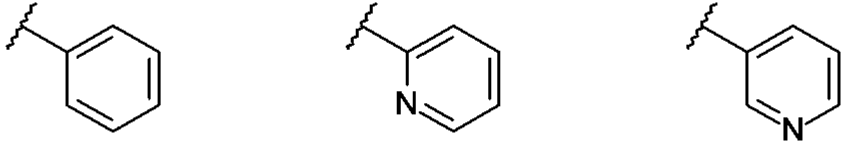 wobei R⁴ gegebenenfalls mit einem oder mehreren R¹¹ substituiert ist, wie in einem der Ansprüche 1 oder 11 definiert.

14. Verbindung nach einem der Ansprüche 1 bis 13 oder ein pharmazeutisch verträgliches Salz davon, wobei R⁵ Wasserstoff oder -C(O)(R⁹) ist.

15. Verbindung nach einem der Ansprüche 1 bis 14 oder ein pharmazeutisch verträgliches Salz davon, wobei R⁶ und R^{6a} Wasserstoff sind.

16. Verbindung nach einem der Ansprüche 1 bis 15 oder ein pharmazeutisch verträgliches Salz davon, wobei
X N oder C(R⁷) ist;
Y S, SO oder S(O)₂ ist_{;}
R¹ ausgewählt ist aus 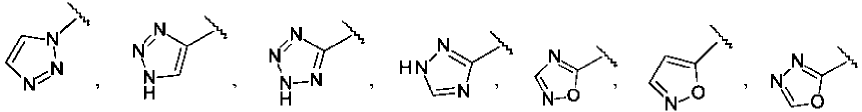 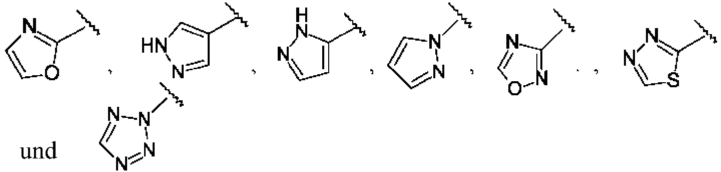
wobei R¹ gegebenenfalls mit einem oder mehreren R¹⁰ substituiert ist, die gleich oder verschieden sein können;
R² Wasserstoff, Halogen, C₁₋₆-Alkyl, Hydroxy oder N(R⁸R^{8a}) ist;
R³ Wasserstoff ist;
R⁴ ausgewählt ist aus 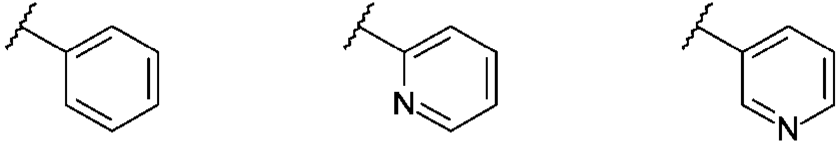
R⁵ Wasserstoff oder -C(O)(R⁹) ist;
R⁶ und R^{6a} Wasserstoff sind;
R⁷ Wasserstoff oder Halogen ist;
R⁸ und R^{8a} jeweils unabhängig voneinander ausgewählt sind aus Wasserstoff und C₁₋₆-Alkyl;
R⁹ Amino-C₁₋₆-alkyl ist;
R¹⁰ ausgewählt ist aus C₁₋₁₀-Alkyl, C₁₋₁₀-Alkyl, substituiert mit C₂₋₆-Alkinyl, C₁₋₁₀-Halogenalkyl, Amino-C₁₋₁₀-alkyl, Hydroxy-C₁₋₁₀-alkyl, C₃₋₁₀-Cycloalkyl, C₃₋₁₀-Cycloalkyl, substituiert mit einem oder mehreren Halogen, Phenyl, substituiert mit einem oder mehreren Halogen, (C₁₋₁₀-Alkyl)-phenyl, substituiert mit einem oder mehreren Halogen, (Alkoxy-C₁₋₁₀-alkyl)-phenyl, 3-10-gliedrigem Heterocyclyl, 3-10-gliedrigem Heterocyclyl, substituiert mit einem oder mehreren C₁₋₁₀-Alkyl und Halogen, Heteroaryl, Heteroaryl, substituiert mit einem oder mehreren C₁₋₁₀-Alkyl, Heteroaryl, substituiert mit einem oder mehreren Halogen, und -N(R^{10e}R^{10f});
R^{10e} Wasserstoff ist, R^{10f} C₃₋₇-Cycloalkyl ist;
R¹¹ ausgewählt ist aus Halogen, -O(R^{11a}), Cyano, Amino-C₁₋₁₀-alkyl, C₁₋₆-Halogenalkoxy, -S(O₂)(C₁₋₆-Alkyl) und 3-10-gliedrigem Heterocyclyl, substituiert mit einem oder mehreren Halogen-C₁₋₆-alkyl;
R^{11a} ausgewählt ist aus C₁₋₁₂-Alkyl, Halogen-C₁₋₆-alkyl, 3-7-gliedrigem Heterocycloalkyl, Amino-C₁₋₁₂-alkyl.

17. Verbindung nach einem der Ansprüche 1 bis 16 oder ein pharmazeutisch verträgliches Salz davon, wobei
X N oder C(R⁷) ist;
Y S, SO oder S(O)₂ ist_{;}
R¹ ausgewählt ist aus 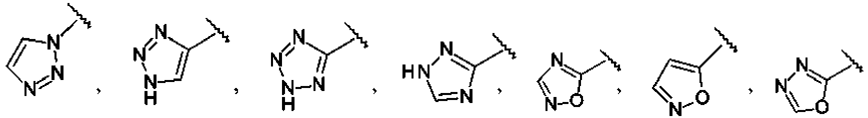 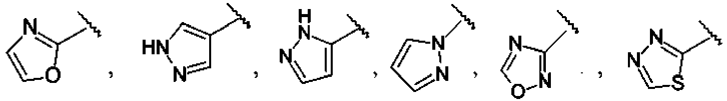 und 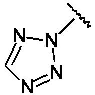
wobei R¹ gegebenenfalls mit einem oder mehreren R¹⁰ substituiert ist, die gleich oder verschieden sein können;
R² Wasserstoff, Halogen, C₁₋₆-Alkyl, Hydroxy oder N(R⁸R^{8a}) ist;
R³ Wasserstoff ist;
R⁴ ausgewählt ist aus 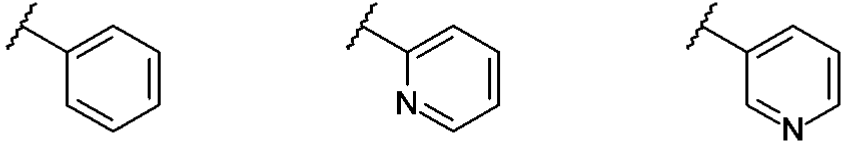
R⁵ Wasserstoff oder -C(O)(R⁹) ist;
R⁶ und R^{6a} Wasserstoff sind;
R⁷ Wasserstoff oder Halogen ist;
R⁸ und R^{8a} jeweils unabhängig voneinander ausgewählt sind aus Wasserstoff und C₁₋₆-Alkyl;
R⁹ Amino-C₁₋₆-alkyl ist;
R¹⁰ ausgewählt ist aus C₁₋₁₀-Alkyl, C₁₋₁₀-Alkyl, substituiert mit C₂₋₆-Alkinyl, C₁₋₁₀-Halogenalkyl, Amino-C₁₋₁₀-alkyl, Hydroxy-C₁₋₁₀-alkyl, C₃₋₁₀-Cycloalkyl, C₃₋₁₀-Cycloalkyl, substituiert mit einem oder mehreren Halogen, Phenyl, substituiert mit einem oder mehreren Halogen, (C₁₋₁₀-Alkyl)-phenyl, substituiert mit einem oder mehreren Halogen, (Alkoxy-C₁₋₁₀-alkyl)-phenyl, 3-10-gliedrigem Heterocyclyl, 3-10-gliedrigem Heterocyclyl, substituiert mit einem oder mehreren C₁₋₁₀-Alkyl und Halogen, -N(R^{10e}R^{10f});
R^{10e} Wasserstoff ist, R^{10f} C₃₋₇-Cycloalkyl ist;
R¹¹ ausgewählt ist aus Halogen, -O(R^{11a}), Cyano, Amino-C₁₋₁₀-alkyl, -S(O₂)(C₁₋₆-Alkyl) und 3-10-gliedrigem Heterocyclyl, substituiert mit einem oder mehreren Halogen-C₁₋₆-alkyl;
R^{11a} ausgewählt ist aus C₁₋₁₂-Alkyl, Halogen-C₁₋₆-alkyl, 3-7-gliedrigem Heterocycloalkyl, Amino-C₁₋₁₂-alkyl.

18. Verbindung nach einem der Ansprüche 1 bis 17, ausgewählt aus
(3R)-3-Amino-5-[(4-chlorphenyl)methyl]-8-fluor-7-[5-(2-oxa-5-azabicyclo[4.1.0]heptan-5-yl)-1,2,4-oxadiazol-3-yl]-1-oxo-2,3-dihydro-1lambda4,5-benzothiazepin-4-on;
(3R)-3-Amino-7-[5-(1-amino-2,2,2-trifluorethyl)-1,2,4-oxadiazol-3-yl]-8-fluor-5-[(4-isopropoxyphenyl)methyl]-1,1-dioxo-2,3-dihydro-1lambda6,5-benzothiazepin-4-on;
(3R)-3-Amino-5-[(4-chlorphenyl)methyl]-7-[5-[3-fluor-1-(2-methoxyethyl)-3-piperidyl]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-(2-oxa-5-azabicyclo[4.1.0]heptan-5-yl)-1,2,4-oxadiazol-3-yl]-1-oxo-2,3-dihydro-1lambda4,5-benzothiazepin-4-on;
(3R)-3-Amino-7-(5-tert-butyl-1,2,4-oxadiazol-3-yl)-8-fluor-5-[(4-fluorphenyl)methyl]-1,1-dioxo-2,3-dihydro-1lambda6,5-benzothiazepin-4-on;
(3R)-3-Amino-5-[(4-chlorphenyl)methyl]-8-fluor-7-(5-morpholino-1,2,4-oxadiazol-3-yl)-1-oxo-2,3-dihydro-1lambda4,5-benzothiazepin-4-on;
(3R)-3-Amino-5-[(4-chlorphenyl)methyl]-8-fluor-7-[5-(4-oxa-7-azaspiro[2.5]octan-7-yl)-1,2,4-oxadiazol-3-yl]-1-oxo-2,3-dihydro-1lambda4,5-benzothiazepin-4-on;
(3R)-3-Amino-5-[(4-chlorphenyl)methyl]-8-fluor-7-[5-(4-oxa-7-azaspiro[2.5]octan-7-yl)-1,2,4-oxadiazol-3-yl]-1-oxo-2,3-dihydro-1lambda4,5-benzothiazepin-4-on;
(3R)-3-Amino-8-fluor-5-[(6-isopropoxy-3-pyridyl)methyl]-7-[5-(4-oxa-7-azaspiro[2.5]octan-7-yl)-1,2,4-oxadiazol-3-yl]-1,1-dioxo-2,3-dihydro-1lambda6,5-benzothiazepin-4-on;
(3R)-3-Amino-5-[(4-chlorphenyl)methyl]-7-[5-[2-(difluormethyl)morpholin-4-yl]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-1-oxo-7-[5-[2-(trifluormethyl)morpholin-4-yl]-1,2,4-oxadiazol-3-yl]-2,3-dihydro-1lambda4,5-benzothiazepin-4-on;
(3R)-3-Amino-5-[(4-chlorphenyl)methyl]-7-(5-morpholino-1,3,4-oxadiazol-2-yl)-1,1-dioxo-2,3-dihydro-1lambda6,5-benzothiazepin-4-on;
(3R)-3-Amino-5-[(4-chlorphenyl)methyl]-8-fluor-1-oxo-7-[5-[2-(trifluormethyl)morpholin-4-yl]-1,2,4-oxadiazol-3-yl]-2,3-dihydro-1lambda4,5-benzothiazepin-4-on;
(3R)-3-Amino-5-[(4-chlorphenyl)methyl]-7-[5-(4-oxa-7-azaspiro[2.5]octan-7-yl)-1,3,4-oxadiazol-2-yl]-1,1-dioxo-2,3-dihydro-1lambda6,5-benzothiazepin-4-on;
Methyl-3-[5-[(3R)-3-amino-5-[[4-(cyclopentoxy)phenyl]methyl]-1,1,4-trioxo-2,3-dihydro-1lambda6,5-benzothiazepin-7-yl]-1,3,4-oxadiazol-2-yl]pyrrolidin-1-carboxylat;
(3R)-3-Amino-5-[(4-chlorphenyl)methyl]-1,1-dioxo-7-[5-[2-(trifluormethyl)morpholin-4-yl]-1,3,4-oxadiazol-2-yl]-2,3-dihydro-1lambda6,5-benzothiazepin-4-on;
(3R)-3-Amino-8-fluor-5-[(5-isopropoxy-2-pyridyl)methyl]-7-[5-(4-oxa-7-azaspiro[2.5]octan-7-yl)-1,2,4-oxadiazol-3-yl]-1,1-dioxo-2,3-dihydro-1lambda6,5-benzothiazepin-4-on;
2-Amino-N-[(3R)-7-(2-tert-butyltetrazol-5-yl)-5-[(4-chlorphenyl)methyl]-8-fluor-1,1,4-trioxo-2,3-dihydro-1lambda6,5-benzothiazepin-3-yl]acetamid;
(3R)-3-Amino-5-[(4-chlorphenyl)methyl]-7-[5-(6-methyl-3-pyridyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(4,6-dimethyl-3-pyridyl)-1,2,4-oxadiazol-3-yl]-8-fluor-1,1-dioxo-2,3-dihydro-1lambda6,5-benzothiazepin-4-on;
(3R)-3-Amino-8-fluor-7-[5-[2-(hydroxymethyl)tetrahydrofuran-2-yl]-1,2,4-oxadiazol-3-yl]-1,1-dioxo-5-[(4-phenoxyphenyl)methyl]-2,3-dihydro-1lambda6,5-benzothiazepin-4-on;
2-[5-[(3R)-3-Amino-1,1,4-trioxo-5-[[4-(trifluormethoxy)phenyl]methyl]-2,3-dihydro-1lambda6,5-benzothiazepin-7-yl]-1,3,4-oxadiazol-2-yl]-2-methylpropannitril;
(3R)-3-Amino-5-[(4-chlorphenyl)methyl]-8-fluor-7-[5-(1-fluor-1-methylethyl)-1,2,4-oxadiazol-3-yl]-1,1-dioxo-2,3-dihydro-1lambda6,5-benzothiazepin-4-on;
(3R)-3-Amino-8-fluor-7-[5-(1-fluor-1-methylethyl)-1,2,4-oxadiazol-3-yl]-5-[(4-fluorphenyl)methyl]-1,1-dioxo-2,3-dihydro-1lambda6,5-benzothiazepin-4-on;
(3R)-3-Amino-7-[5-(2,2-difluormorpholin-4-yl)-1,3,4-oxadiazol-2-yl]-1,1-dioxo-5-[(4-phenoxyphenyl)methyl]-2,3-dihydro-1lambda6,5-benzothiazepin-4-on;
(3R)-3-Amino-5-[(4-chlorphenyl)methyl]-7-[5-(2-oxa-5-azabicyclo[4.1.0]heptan-5-yl)-1,3,4-oxadiazol-2-yl]-1,1-dioxo-2,3-dihydro-1lambda6,5-benzothiazepin-4-on;
(3R)-3-Amino-7-[5-(3-aminooxetan-3-yl)-1,2,4-oxadiazol-3-yl]-8-fluor-5-[[4-(4-methoxyphenyl)phenyl]methyl]-1,1-dioxo-2,3-dihydro-1lambda6,5-benzothiazepin-4-on;
(3R)-3-Amino-5-[(4-chlorphenyl)methyl]-8-fluor-7-[5-(6-fluor-2-methyl-3-pyridyl)-1,2,4-oxadiazol-3-yl]-1,1-dioxo-2,3-dihydro-1lambda6,5-benzothiazepin-4-on;
(3R)-3-Amino-5-[(4-chlorphenyl)methyl]-8-fluor-7-[5-(2-methyl-4-methylsulfonylphenyl)-1,2,4-oxadiazol-3-yl]-1,1-dioxo-2,3-dihydro-1lambda6,5-benzothiazepin-4-on;
(3R)-3-Amino-5-[(4-chlorphenyl)methyl]-8-fluor-7-[5-(2-methyl-5-methylsulfonylphenyl)-1,2,4-oxadiazol-3-yl]-1,1-dioxo-2,3-dihydro-1lambda6,5-benzothiazepin-4-on;1,1,1,3,3,3-Hexafluorpropan-2-ol;
(3R)-3-Amino-5-[(4-chlorphenyl)methyl]-8-fluor-7-[5-(4-methyl-3-pyridyl)-1,2,4-oxadiazol-3-yl]-1,1-dioxo-2,3-dihydro-1lambda6,5-benzothiazepin-4-on;
(3R)-3-Amino-5-[(4-chlorphenyl)methyl]-7-[5-[(1R,5S)-3-oxa-8-azabicyclo[3.2.1]octan-8-yl]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(2,2-dimethylmorpholin-4-yl)-1,2,4-oxadiazol-3-yl]-8-fluor-1,1-dioxo-2,3-dihydro-1lambda6,5-benzothiazepin-4-on;
(3R)-3-Amino-5-[(4-chlorphenyl)methyl]-7-[5-(2-methyl-4-methylsulfonylphenyl)-1,3,4-oxadiazol-2-yl]-1,1-dioxo-2,3-dihydro-1lambda6,5-benzothiazepin-4-on;
Methyl-3-[3-[(3R)-3-amino-5-[[4-(cyclopentoxy)phenyl]methyl]-8-fluor-1,1,4-trioxo-2,3-dihydro-1lambda6,5-benzothiazepin-7-yl]-1,2,4-oxadiazol-5-yl]pyrrolidin-1-carboxylat;
(3R)-3-Amino-5-[(4-chlorphenyl)methyl]-7-[5-(2,2-difluormorpholin-4-yl)-1,2,4-oxadiazol-3-yl]-8-fluor-1,1-dioxo-2,3-dihydro-1lambda6,5-benzothiazepin-4-on;
(3R)-3-Amino-7-(5-tert-butyl-1,2,4-oxadiazol-3-yl)-5-[[4-(cyclopentoxy)phenyl]methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(5-methyl-1,2,4-oxadiazol-3-yl)-1,3,4-oxadiazol-2-yl]-1,1-dioxo-2,3-dihydro-1lambda6,5-benzothiazepin-4-on;
(3R)-3-Amino-8-fluor-7-[5-(3-fluor-1-methyl-3-piperidyl)-1,2,4-oxadiazol-3-yl]-1,1-dioxo-5-[(4-phenoxyphenyl)methyl]-2,3-dihydro-1lambda6,5-benzothiazepin-4-on;
(3R)-3-Amino-5-[(4-chlorphenyl)methyl]-8-fluor-7-[5-(6-fluor-2-methyl-3-pyridyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(4,6-dimethyl-3-pyridyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-(4,4-difluor-1-methoxycyclohexyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-(2-fluor-6-methylphenyl)-1,3,4-oxadiazol-2-yl]-1,1-dioxo-2,3-dihydro-1lambda6,5-benzothiazepin-4-on;1,1,1,3,3,3-Hexafluorpropan-2-ol;
Methyl-4-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-4-methoxypiperidin-1-carboxylat;
(3R)-3-Amino-5-[(4-chlorphenyl)methyl]-8-fluor-7-[5-(5-methyl-1,3,4-oxadiazol-2-yl)-1,2,4-oxadiazol-3-yl]-1,1-dioxo-2,3-dihydro-1lambda6,5-benzothiazepin-4-on;
Methyl-N-[2-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-2-methyl-1-(trifluormethyl)propyl]carbamat;
(3R)-3-Amino-7-[5-(2-amino-3,3,3-trifluor-1,1-dimethylpropyl)-1,3,4-oxadiazol-2-yl]-5-[(4-chlorphenyl)methyl]-8-fluor-1,1-dioxo-2,3-dihydro-1lambda6,5-benzothiazepin-4-on;
3-[(3R)-3-Amino-5-[(4-chlorphenyl)methyl]-8-fluor-1,1,4-trioxo-2,3-dihydro-1lambda6,5-benzothiazepin-7-yl]-N-(3,3,3-trifluor-2-hydroxy-2-methylpropyl)-1,2,4-oxadiazol-5-carboxamid;
(3R)-3-Amino-5-[(4-chlorphenyl)methyl]-8-fluor-7-[5-[2-(hydroxymethyl)tetrahydrofuran-2-yl]-1,2,4-oxadiazol-3-yl]-1,1-dioxo-2,3-dihydro-1lambda6,5-benzothiazepin-4-on;
(3R)-3-Amino-8-fluor-7-[5-(5-methyl-1,3,4-oxadiazol-2-yl)-1,2,4-oxadiazol-3-yl]-1,1-dioxo-5-[[4-(trifluormethoxy)phenyl]methyl]-2,3-dihydro-1lambda6,5-benzothiazepin-4-on;
(3R)-3-Amino-7-[5-[1-amino-2,2,2-trifluor-1-(4-fluorphenyl)ethyl]-1,2,4-oxadiazol-3-yl]-5-[(4-chlorphenyl)methyl]-8-fluor-1,1-dioxo-2,3-dihydro-1lambda6,5-benzothiazepin-4-on;
(3R)-3-Amino-8-fluor-7-[5-(5-methyl-1,3,4-oxadiazol-2-yl)-1,3,4-oxadiazol-2-yl]-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-8-fluor-7-[5-(3-oxa-8-azabicyclo[3.2.1]octan-8-yl)-1,2,4-oxadiazol-3-yl]-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-[2-(trifluormethyl)morpholin-4-yl]-1,2,4-oxadiazol-3-yl]-2,3-dihydro-1lambda6,5-benzothiazepin-4-on;
(3R)-3-Amino-5-[(4-chlorphenyl)methyl]-8-fluor-7-[5-(2-oxa-5-azabicyclo[4.1.0]heptan-5-yl)-1,2,4-oxadiazol-3-yl]-1,1-dioxo-2,3-dihydro-1lambda6,5-benzothiazepin-4-on;
(3R)-3-Amino-8-fluor-7-[5-[2-(hydroxymethyl)tetrahydrofuran-2-yl]-1,2,4-oxadiazol-3-yl]-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-7-[5-(2-fluorphenyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(4-fluorphenyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-1,1-dioxo-7-[5-(p-tolyl)-1,3,4-oxadiazol-2-yl]-2,3-dihydro-1lambda6,5-benzothiazepin-4-on;
(3R)-3-Amino-5-[(4-chlorphenyl)methyl]-8-fluor-7-[5-(2-fluorphenyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-(m-tolyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-(p-tolyl)-1,3,4-oxadiazol-2-yl]-2,3-dihydro-1lambda6,5-benzothiazepin-4-on;
Methyl-1-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-2-oxa-5-azabicyclo[2.2.1]heptan-5-carboxylat;
(3R)-3-Amino-7-[5-(1-amino-2,2,2-trifluor-1-tetrahydropyran-4-ylethyl)-1,2,4-oxadiazol-3-yl]-5-[(4-chlorphenyl)methyl]-8-fluor-1,1-dioxo-2,3-dihydro-1lambda6,5-benzothiazepin-4-on;
3-[(3R)-3-Amino-5-[(4-chlorphenyl)methyl]-8-fluor-1,1,4-trioxo-2,3-dihydro-1lambda6,5-benzothiazepin-7-yl]-N-(2,2-difluorpropyl)-1,2,4-oxadiazol-5-carboxamid;
(3R)-3-Amino-7-[5-(1-amino-2,2,2-trifluor-1-methylethyl)-1,2,4-oxadiazol-3-yl]-8-fluor-1,1-dioxo-5-[[4-(trifluormethoxy)phenyl]methyl]-2,3-dihydro-1lambda6,5-benzothiazepin-4-on;
(3R)-3-Amino-8-fluor-1,1-dioxo-5-[[4-(trifluormethoxy)phenyl]methyl]-7-[5-[1-(trifluormethyl)cyclopropyl]-1,2,4-oxadiazol-3-yl]-2,3-dihydro-1lambda6,5-benzothiazepin-4-on;
(3R)-3-Amino-7-[5-(1-amino-2,2,2-trifluor-1-methylethyl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-[1-(trifluormethyl)cyclopropyl]-1,3,4-oxadiazol-2-yl]-2,3-dihydro-1lambda6,5-benzothiazepin-4-on;
(3R)-3-Amino-5-[(4-chlorphenyl)methyl]-1,1-dioxo-7-[5-[1-(trifluormethyl)cyclopropyl]-1,3,4-oxadiazol-2-yl]-2,3-dihydro-1lambda6,5-benzothiazepin-4-on;
3-[(3R)-3-Amino-5-[(4-chlorphenyl)methyl]-8-fluor-1,1,4-trioxo-2,3-dihydro-1lambda6,5-benzothiazepin-7-yl]-N-(2,2,2-trifluorethyl)-1,2,4-oxadiazol-5-carboxamid;
(3R)-3-Amino-5-[(4-chlorphenyl)methyl]-1,1-dioxo-7-(5-phenyl-1,3,4-oxadiazol-2-yl)-2,3-dihydro-1lambda6,5-benzothiazepin-4-on;
(3R)-3-Amino-5-[(4-chlorphenyl)methyl]-8-fluor-7-[5-(4-fluorphenyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-(o-tolyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-1,1-dioxo-7-(5-phenyl-1,3,4-oxadiazol-2-yl)-2,3-dihydro-1lambda6,5-benzothiazepin-4-on;
Methyl-3-[5-[(3R)-3-amino-1,1,4-trioxo-5-[[4-(trifluormethoxy)phenyl]methyl]-2,3-dihydro-1lambda6,5-benzothiazepin-7-yl]-1,3,4-oxadiazol-2-yl]pyrrolidin-1-carboxylat;
(3R)-3-Amino-5-[(4-chlorphenyl)methyl]-8-fluor-7-[5-(5-methyl-2-pyridyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-(6-methyl-3-pyridyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-[(3,3,3-trifluor-2-hydroxy-2-methylpropyl)amino]-1,3,4-oxadiazol-2-yl]-2,3-dihydro-1lambda6,5-benzothiazepin-4-on;
(3R)-3-Amino-5-[(4-chlorphenyl)methyl]-8-fluor-7-[5-[[1-(2-hydroxyethyl)cyclohexyl]amino]-1,3,4-oxadiazol-2-yl]-1,1-dioxo-2,3-dihydro-1lambda6,5-benzothiazepin-4-on;
(3R)-3-Amino-5-benzyl-8-fluor-1,1-dioxo-7-[5-(1,2,2,2-tetrafluor-1-methoxyethyl)-1,2,4-oxadiazol-3-yl]-2,3-dihydro-1lambda6,5-benzothiazepin-4-on;
(3R)-3-Amino-7-[5-(3-aminooxetan-3-yl)-1,2,4-oxadiazol-3-yl]-8-fluor-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-8-fluor-7-[5-(6-fluor-3-pyridyl)-1,3,4-oxadiazol-2-yl]-1,1-dioxo-2,3-dihydro-1lambda6,5-benzothiazepin-4-on;
(3R)-3-Amino-7-(5-tert-butyl-1,3,4-oxadiazol-2-yl)-1,1-dioxo-5-[(4-phenoxyphenyl)methyl]-2,3-dihydro-1lambda6,5-benzothiazepin-4-on;
(3R)-3-Amino-5-[(4-chlorphenyl)methyl]-8-fluor-7-[5-(5-fluor-2-pyridyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(2,2-difluorpropylamino)-1,3,4-oxadiazol-2-yl]-1,1-dioxo-2,3-dihydro-1lambda6,5-benzothiazepin-4-on;
2-[[3-[(3R)-3-Amino-5-[(4-chlorphenyl)methyl]-8-fluor-1,1,4-trioxo-2,3-dihydro-1lambda6,5-benzothiazepin-7-yl]-1,2,4-oxadiazol-5-yl]amino]-2-methylpropannitril;
(3R)-3-Amino-5-[(4-chlorphenyl)methyl]-7-[5-(1-methoxycyclopropyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-1,1-dioxo-7-[5-(2,2,2-trifluor-1-methoxy-1-methylethyl)-1,3,4-oxadiazol-2-yl]-2,3-dihydro-1lambda6,5-benzothiazepin-4-on;
(3R)-3-Amino-5-[(4-chlorphenyl)methyl]-7-[5-(4-methoxytetrahydropyran-4-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-(1,1,2,2,2-pentafluorethyl)-1,3,4-oxadiazol-2-yl]-2,3-dihydro-1lambda6,5-benzothiazepin-4-on;
N-[11-[4-[[(3R)-3-Amino-7-(5-tert-butyl-1,3,4-oxadiazol-2-yl)-8-fluor-1,1,4-trioxo-2,3-dihydro-1lambda6,5-benzothiazepin-5-yl]methyl]phenoxy]undecyl]-3-[2,2-difluor-12-(1H-pyrrol-2-yl)-1-aza-3-azonia-2-boranuidatricyclo[7.3.0.03,7]dodeca-3,5,7,9,11-pent;
(3R)-3-Amino-5-[(4-chlorphenyl)methyl]-7-[5-(1-methoxy-1-methylethyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[(1-ethinylcyclohexyl)amino]-1,3,4-oxadiazol-2-yl]-8-fluor-1,1-dioxo-2,3-dihydro-1lambda6,5-benzothiazepin-4-on;
(3R)-3-Amino-7-(5-tert-butyl-1,2,4-oxadiazol-3-yl)-5-[(4-chlorphenyl)methyl]-8-fluor-1-imino-1-oxo-2,3-dihydro-1lambda6,5-benzothiazepin-4-on;
(3R)-3-Amino-5-[(4-chlorphenyl)methyl]-7-[5-(9,9-difluor-3-oxa-7-azabicyclo[3.3.1]nonan-7-yl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[4,4-difluor-3-(hydroxymethyl)-1-piperidyl]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-(3,3-dimethylmorpholin-4-yl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-(5,5-difluor-1-methyl-3-piperidyl)-1,2,4-oxadiazol-3-yl]-8-fluor-1-oxo-2,3-dihydro-1lambda4,5-benzothiazepin-4-on;
(3R)-3-Amino-5-[(4-chlorphenyl)methyl]-7-[5-(5,5-difluor-1-methyl-3-piperidyl)-1,2,4-oxadiazol-3-yl]-8-fluor-1-oxo-2,3-dihydro-1lambda4,5-benzothiazepin-4-on;
Methyl-4-[5-[(3R)-3-amino-5-[(4-chlorphenyl)methyl]-1,1,4-trioxo-2,3-dihydro-1lambda6,5-benzothiazepin-7-yl]-1,3,4-oxadiazol-2-yl]-4-cyanopiperidin-1-carboxylat;
(3R)-3-Amino-5-[(4-chlorphenyl)methyl]-7-[5-(3,3-difluor-1,8-diazaspiro[4.5]decan-8-yl)-1,3,4-oxadiazol-2-yl]-8-fluor-1,1-dioxo-2,3-dihydro-1lambda6,5-benzothiazepin-4-on;
(3R)-3-Amino-7-(5-tert-butyl-1,2,4-oxadiazol-3-yl)-5-[(4-chlorphenyl)methyl]-8-fluor-1-oxo-2,3-dihydro-1lambda4,5-benzothiazepin-4-on;
(3R)-3-Amino-7-(5-tert-butyl-1,2,4-oxadiazol-3-yl)-5-[(4-chlorphenyl)methyl]-8-fluor-1-oxo-2,3-dihydro-1lambda4,5-benzothiazepin-4-on;
(3R)-3-Amino-5-[(4-chlorphenyl)methyl]-8-fluor-7-[5-(1-methyl-1-pyrrolidin-1-ylethyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-[4-methyl-1-oxido-1-(2,2,2-trifluorethyl)piperidin-1-ium-4-yl]-1,2,4-oxadiazol-3-yl]-1-oxo-2,3-dihydro-1lambda4,5-benzothiazepin-4-on;
2-[4-[[(3R)-3-Amino-7-(5-tert-butyl-1,3,4-oxadiazol-2-yl)-8-fluor-1,1,4-trioxo-2,3-dihydro-1lambda6,5-benzothiazepin-5-yl]methyl]phenyl]-2-methylpropannitril;
(3R)-3-Amino-5-[(4-chlorphenyl)methyl]-7-[5-[(1S,5R)-6,6-difluor-3-azabicyclo[3.1.1]heptan-3-yl]-1,3,4-oxadiazol-2-yl]-8-fluor-1,1-dioxo-2,3-dihydro-1lambda6,5-benzothiazepin-4-on;
1-[5-[(3R)-3-Amino-5-[(4-chlorphenyl)methyl]-8-fluor-1,1,4-trioxo-2,3-dihydro-1lambda6,5-benzothiazepin-7-yl]-1,3,4-oxadiazol-2-yl]-4-methylpiperidin-4-carbonitril;
(3R)-3-Amino-7-(5-tert-butyl-1,3,4-oxadiazol-2-yl)-8-fluor-1,1-dioxo-5-[[4-(2,2,2-trifluorethoxy)phenyl]methyl]-2,3-dihydro-1lambda6,5-benzothiazepin-4-on;
(3R)-3-Amino-5-[(4-chlorphenyl)methyl]-7-[5-[[1,1-dimethyl-4-(2-prop-2-inoxyethoxy)butyl]amino]-1,2,4-oxadiazol-3-yl]-8-fluor-1,1-dioxo-2,3-dihydro-1lambda6,5-benzothiazepin-4-on;
3-[(3R)-3-Amino-8-fluor-1,1,4-trioxo-5-[[4-(1,1,2,2-tetrafluorethoxy)phenyl]methyl]-2,3-dihydro-1lambda6,5-benzothiazepin-7-yl]-N,N-dimethyl-1,2,4-oxadiazol-5-carboxamid;
(3R)-3-Amino-7-(5-tert-butyl-1,3,4-oxadiazol-2-yl)-5-[(6-chlorpyridazin-3-yl)methyl]-8-fluor-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-(2,2,2-trifluorethylamino)-1,3,4-oxadiazol-2-yl]-2,3-dihydro-1lambda6,5-benzothiazepin-4-on;
(3R)-3-Amino-5-[(4-chlorphenyl)methyl]-7-[5-[1,1-dimethyl-4-[2-(2-prop-2-inoxyethoxy)ethoxy]butyl]-1,2,4-oxadiazol-3-yl]-8-fluor-1,1-dioxo-2,3-dihydro-1lambda6,5-benzothiazepin-4-on;
(3R)-3-Amino-5-[(4-chlorphenyl)methyl]-7-[2-(5,5-difluor-1-methylsulfonyl-3-piperidyl)tetrazol-5-yl]-8-fluor-1,1-dioxo-2,3-dihydro-1lambda6,5-benzothiazepin-4-on;
Methyl-5-[5-[(3R)-3-amino-5-[(4-chlorphenyl)methyl]-8-fluor-1,1,4-trioxo-2,3-dihydro-1lambda6,5-benzothiazepin-7-yl]tetrazol-2-yl]-3,3-difluorpiperidin-1-carboxylat;
2-[[5-[(3R)-3-Amino-5-[(4-chlorphenyl)methyl]-8-fluor-1,1,4-trioxo-2,3-dihydro-1lambda6,5-benzothiazepin-7-yl]-1,3,4-oxadiazol-2-yl]amino]-2-methylpropannitril;
(3R)-3-Amino-5-[(4-chlorphenyl)methyl]-8-fluor-7-[5-[4-methyl-1-(2,2,2-trifluorethyl)-4-piperidyl]-1,2,4-oxadiazol-3-yl]-1,1-dioxo-2,3-dihydro-1lambda6,5-benzothiazepin-4-on;
(3R)-3-Amino-5-[(4-chlorphenyl)methyl]-7-[5-(2,2-difluorpropylamino)-1,3,4-oxadiazol-2-yl]-8-fluor-1,1-dioxo-2,3-dihydro-1lambda6,5-benzothiazepin-4-on;
(3R)-3-Amino-7-[5-(1-bicyclo[1.1.1]pentanylamino)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-[(3,3,3-trifluor-1-methylpropyl)amino]-1,3,4-oxadiazol-2-yl]-2,3-dihydro-1lambda6,5-benzothiazepin-4-on;1,1,1,3,3,3-Hexafluorpropan-2-ol;
(3R)-3-Amino-7-(5-tert-butyl-1,3,4-oxadiazol-2-yl)-1,1-dioxo-5-[(4-tetrahydropyran-4-yloxyphenyl)methyl]-2,3-dihydro-1lambda6,5-benzothiazepin-4-on;
Methyl-1-[3-[(3R)-3-amino-8-fluor-1-imino-1,4-dioxo-5-[[4-(1,1,2,2-tetrafluorethoxy)phenyl]methyl]-2,3-dihydro-1lambda6,5-benzothiazepin-7-yl]-1,2,4-oxadiazol-5-yl]-3-azabicyclo[3.1.1]heptan-3-carboxylat;
Methyl-1-[3-[(3R)-3-amino-8-fluor-1,4-dioxo-5-[[4-(trifluormethoxy)phenyl]methyl]-2,3-dihydro-1lambda4,5-benzothiazepin-7-yl]-1,2,4-oxadiazol-5-yl]-3-azabicyclo[3.1.1]heptan-3-carboxylat;
(3R)-3-Amino-5-[(4-chlorphenyl)methyl]-8-fluor-1-imino-1-oxo-7-[5-(1,2,2,2-tetrafluor-1-methoxyethyl)-1,2,4-oxadiazol-3-yl]-2,3-dihydro-1lambda6,5-benzothiazepin-4-on;
(3R)-3-Amino-7-(2-tert-butyltetrazol-5-yl)-8-fluor-1,1-dioxo-5-[[4-(tetrahydropyran-4-ylmethoxy)phenyl]methyl]-2,3-dihydro-1lambda6,5-benzothiazepin-4-on;
(3R)-3-Amino-7-(5-tert-butyl-1,2,4-oxadiazol-3-yl)-8-fluor-1,1-dioxo-5-[[4-(tetrahydropyran-4-ylmethoxy)phenyl]methyl]-2,3-dihydro-1lambda6,5-benzothiazepin-4-on;
(3R)-3-Amino-7-(5-tert-butyl-1,3,4-oxadiazol-2-yl)-5-[[4-(cyclopropoxy)phenyl]methyl]-1,1-dioxo-2,3-dihydro-1lambda6,5-benzothiazepin-4-on;
Methyl-1-[3-[(3R)-3-amino-8-fluor-1,4-dioxo-5-[[4-(trifluormethoxy)phenyl]methyl]-2,3-dihydro-1lambda4,5-benzothiazepin-7-yl]-1,2,4-oxadiazol-5-yl]-3-azabicyclo[3.1.1]heptan-3-carboxylat;
(3R)-7-[2-(1-Acetyl-5,5-difluor-3-piperidyl)tetrazol-5-yl]-3-amino-5-[(4-chlorphenyl)methyl]-8-fluor-1,1-dioxo-2,3-dihydro-1lambda6,5-benzothiazepin-4-on;
N-[(3R)-5-[(4-Chlorphenyl)methyl]-7-[2-(5,5-difluor-3-piperidyl)tetrazol-5-yl]-8-fluor-1,1,4-trioxo-2,3-dihydro-1lambda6,5-benzothiazepin-3-yl]acetamid;
(3R)-3-Amino-5-[(4-chlorphenyl)methyl]-8-fluor-1,1-dioxo-7-[5-(1,5,5-trimethyl-3-piperidyl)-1,2,4-oxadiazol-3-yl]-2,3-dihydro-1lambda6,5-benzothiazepin-4-on;
(3R)-3-Amino-7-(5-tert-butyl-1,3,4-oxadiazol-2-yl)-5-[[4-(cyclohexoxy)phenyl]methyl]-1,1-dioxo-2,3-dihydro-1lambda6,5-benzothiazepin-4-on;
Methyl-1-[3-[(3R)-3-amino-8-fluor-1,4-dioxo-5-[[4-(1,1,2,2-tetrafluorethoxy)phenyl]methyl]-2,3-dihydro-1lambda4,5-benzothiazepin-7-yl]-1,2,4-oxadiazol-5-yl]-3-azabicyclo[3.1.1]heptan-3-carboxylat;
(3R)-3-Amino-7-(2-tert-butyltetrazol-5-yl)-5-[(4-chlorphenyl)methyl]-8-fluor-1-oxo-2,3-dihydro-1lambda4,5-benzothiazepin-4-on;
(3R)-3-Amino-7-(2-tert-butyltetrazol-5-yl)-5-[(4-chlorphenyl)methyl]-8-fluor-1-oxo-2,3-dihydro-1lambda4,5-benzothiazepin-4-on;
(3R)-3-Amino-5-[(4-chlorphenyl)methyl]-8-fluor-1-oxo-7-[5-(1,2,2,2-tetrafluor-1-methoxyethyl)-1,2,4-oxadiazol-3-yl]-2,3-dihydro-1lambda4,5-benzothiazepin-4-on;
(3R)-3-Amino-5-[(4-chlorphenyl)methyl]-8-fluor-1-oxo-7-[5-(1,2,2,2-tetrafluor-1-methoxyethyl)-1,2,4-oxadiazol-3-yl]-2,3-dihydro-1lambda4,5-benzothiazepin-4-on;
(3R)-3-Amino-5-[(4-chlorphenyl)methyl]-8-fluor-1-oxo-7-[5-(1,2,2,2-tetrafluor-1-methoxyethyl)-1,2,4-oxadiazol-3-yl]-2,3-dihydro-1lambda4,5-benzothiazepin-4-on;
(3R)-3-Amino-5-[(4-chlorphenyl)methyl]-8-fluor-7-[5-[[(1S)-1-methyl-2-(trifluormethoxy)ethyl]amino]-1,3,4-oxadiazol-2-yl]-1,1-dioxo-2,3-dihydro-1lambda6,5-benzothiazepin-4-on;
(3R)-3-Amino-5-benzyl-7-[2-(5,5-difluor-1-methyl-3-piperidyl)tetrazol-5-yl]-8-fluor-1,1-dioxo-2,3-dihydro-1lambda6,5-benzothiazepin-4-on;
(3R)-3-Amino-5-[(4-chlorphenyl)methyl]-7-[2-(5,5-difluor-1-methyl-3-piperidyl)tetrazol-5-yl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-1,1-dioxo-5-[[4-(tetrahydropyran-4-ylmethoxy)phenyl]methyl]-2,3-dihydro-1lambda6,5-benzothiazepin-4-on;
(3R)-3-Amino-8-fluor-1,1-dioxo-5-[[4-(trifluormethoxy)phenyl]methyl]-7-[8-(trifluormethyl)-5,6,7,8-tetrahydro-[1,2,4]triazolo[1,5-a]pyrazin-2-yl]-2,3-dihydro-1lambda6,5-benzothiazepin-4-on;
(3R)-3-Amino-8-fluor-5-[(4-fluorphenyl)methyl]-1-oxo-7-[5-(1,2,2,2-tetrafluor-1-methoxyethyl)-1,2,4-oxadiazol-3-yl]-2,3-dihydro-1lambda4,5-benzothiazepin-4-on;
(3R)-3-Amino-8-fluor-5-[(4-fluorphenyl)methyl]-1,1-dioxo-7-[5-(1,2,2,2-tetrafluor-1-methoxyethyl)-1,2,4-oxadiazol-3-yl]-2,3-dihydro-1lambda6,5-benzothiazepin-4-on;
(3R)-3-Amino-5-[(4-chlorphenyl)methyl]-8-fluor-1,1-dioxo-7-[5-(2,2,2-trifluor-1-hydroxy-1-methylethyl)-1,2,4-oxadiazol-3-yl]-2,3-dihydro-1lambda6,5-benzothiazepin-4-on;
(3R)-3-Amino-7-(5-tert-butyl-1,3,4-oxadiazol-2-yl)-1,1-dioxo-5-[[4-[[5-(trifluormethyl)-2-pyridyl]oxy]phenyl]methyl]-2,3-dihydro-1lambda6,5-benzothiazepin-4-on;
Methyl-1-[3-[(3R)-3-amino-8-fluor-1,1,4-trioxo-5-[[4-(1,1,2,2-tetrafluorethoxy)phenyl]methyl]-2,3-dihydro-1lambda6,5-benzothiazepin-7-yl]-1,2,4-oxadiazol-5-yl]-3-azabicyclo[3.1.1]heptan-3-carboxylat;
(3R)-3-Amino-7-(5-tert-butyl-1,3,4-oxadiazol-2-yl)-5-[[4-(4-fluorphenoxy)phenyl]methyl]-1,1-dioxo-2,3-dihydro-1lambda6,5-benzothiazepin-4-on;
Methyl-1-[3-[(3R)-3-amino-5-[(4-chlorphenyl)methyl]-8-fluor-1,1,4-trioxo-2,3-dihydro-1lambda6,5-benzothiazepin-7-yl]-1,2,4-oxadiazol-5-yl]-3-azabicyclo[3.1.1]heptan-3-carboxylat;
Methyl-1-[3-[(3R)-3-amino-8-fluor-1,1,4-trioxo-5-[[4-(trifluormethoxy)phenyl]methyl]-2,3-dihydro-1lambda6,5-benzothiazepin-7-yl]-1,2,4-oxadiazol-5-yl]-3-azabicyclo[3.1.1]heptan-3-carboxylat;
(3R)-3-Amino-5-[(4-chlorphenyl)methyl]-8-fluor-1,1-dioxo-7-[5-[1-(trifluormethyl)cyclopropyl]-1,2,4-oxadiazol-3-yl]-2,3-dihydro-1lambda6,5-benzothiazepin-4-on;
(3R)-3-Amino-7-(2-tert-butyltetrazol-5-yl)-8-fluor-5-[(4-fluorphenyl)methyl]-1,1-dioxo-2,3-dihydro-1lambda6,5-benzothiazepin-4-on;
(3R)-3-Amino-1-oxo-7-[5-(2,2,2-trifluorethyl)-1,3,4-oxadiazol-2-yl]-5-[[4-(trifluormethoxy)phenyl]methyl]-2,3-dihydro-1lambda4,5-benzothiazepin-4-on;
(3R)-3-Amino-1-oxo-7-[5-(2,2,2-trifluorethyl)-1,3,4-oxadiazol-2-yl]-5-[[4-(trifluormethoxy)phenyl]methyl]-2,3-dihydro-1lambda4,5-benzothiazepin-4-on;
(3R)-3-Amino-1-oxo-7-[5-(1,2,2,2-tetrafluor-1-methoxyethyl)-1,3,4-oxadiazol-2-yl]-5-[[4-(trifluormethoxy)phenyl]methyl]-2,3-dihydro-1lambda4,5-benzothiazepin-4-on;
(3R)-3-Amino-1-oxo-7-[5-(1,2,2,2-tetrafluor-1-methoxyethyl)-1,3,4-oxadiazol-2-yl]-5-[[4-(trifluormethoxy)phenyl]methyl]-2,3-dihydro-1lambda4,5-benzothiazepin-4-on;
(3R)-3-Amino-7-[5-(1-amino-2,2,2-trifluorethyl)-1,2,4-oxadiazol-3-yl]-8-fluor-1,1-dioxo-5-[[4-(trifluormethoxy)phenyl]methyl]-2,3-dihydro-1lambda6,5-benzothiazepin-4-on;
Isopropyl-1-[3-[(3R)-3-amino-5-[(4-chlorphenyl)methyl]-8-fluor-1,1,4-trioxo-2,3-dihydro-1lambda6,5-benzothiazepin-7-yl]-1,2,4-oxadiazol-5-yl]-3-azabicyclo[3.1.1]heptan-3-carboxylat;
Ethyl-1-[3-[(3R)-3-amino-5-[(4-chlorphenyl)methyl]-8-fluor-1,1,4-trioxo-2,3-dihydro-1lambda6,5-benzothiazepin-7-yl]-1,2,4-oxadiazol-5-yl]-3-azabicyclo[3.1.1]heptan-3-carboxylat;
(3R)-3-Amino-5-[(4-chlorphenyl)methyl]-7-[5-[1-(2,2-difluorcyclopropyl)ethylamino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[1-(2,2-difluorcyclopropyl)ethylamino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[1-(3,3-difluorcyclobutyl)ethylamino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-[(3,3,3-trifluor-1,1-dimethylpropyl)amino]-1,3,4-oxadiazol-2-yl]-2,3-dihydro-1lambda6,5-benzothiazepin-4-on;
(3R)-3-Amino-5-[(4-chlorphenyl)methyl]-8-fluor-7-[5-[1-methyl-1-(2,2,2-trifluorethylamino)ethyl]-1,2,4-oxadiazol-3-yl]-1,1-dioxo-2,3-dihydro-1lambda6,5-benzothiazepin-4-on;
Methyl-3-[3-[(3R)-3-amino-8-fluor-5-[(4-isopropoxyphenyl)methyl]-1,1,4-trioxo-2,3-dihydro-1lambda6,5-benzothiazepin-7-yl]-1,2,4-oxadiazol-5-yl]pyrrolidin-1-carboxylat;
Methyl-1-[5-[(3R)-3-amino-1,1,4-trioxo-5-[[4-(trifluormethoxy)phenyl]methyl]-2,3-dihydro-1lambda6,5-benzothiazepin-7-yl]-1,3,4-oxadiazol-2-yl]-3-azabicyclo[3.1.1]heptan-3-carboxylat;
(3R)-3-Amino-7-(5-tert-butyl-1,3,4-oxadiazol-2-yl)-1,1-dioxo-5-[[4-(trifluormethylsulfonyl)phenyl]methyl]-2,3-dihydro-1lambda6,5-benzothiazepin-4-on;
(3R)-3-Amino-7-[5-(5,5-difluor-1-methyl-3-piperidyl)-1,2,4-oxadiazol-3-yl]-8-fluor-5-[(4-isopropoxyphenyl)methyl]-1,1-dioxo-2,3-dihydro-1lambda6,5-benzothiazepin-4-on;
(3R)-3-Amino-7-[5-(5,5-difluor-1-methyl-3-piperidyl)-1,2,4-oxadiazol-3-yl]-8-fluor-5-[(6-isopropoxy-3-pyridyl)methyl]-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-[[3-(trifluormethyl)cyclobutyl]amino]-1,3,4-oxadiazol-2-yl]-2,3-dihydro-1lambda6,5-benzothiazepin-4-on;
(3R)-3-Amino-5-[(4-chlorphenyl)methyl]-8-fluor-1,1-dioxo-7-[5-(1,2,2,2-tetrafluorethyl)-1,2,4-oxadiazol-3-yl]-2,3-dihydro-1lambda6,5-benzothiazepin-4-on;
Methyl-1-[5-[(3R)-3-amino-1,1,4-trioxo-5-[[4-(1,1,2,2-tetrafluorethoxy)phenyl]methyl]-2,3-dihydro-1lambda6,5-benzothiazepin-7-yl]-1,3,4-oxadiazol-2-yl]-3-azabicyclo[3.1.1]heptan-3-carboxylat;
(3R)-3-Amino-7-[5-[(3-chlor-1-bicyclo[1.1.1]pentanyl)amino]-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-[(3-fluor-1-bicyclo[1.1.1]pentanyl)amino]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-(1,1,2,2,2-pentafluorethyl)-1,2,4-oxadiazol-3-yl]-2,3-dihydro-1lambda6,5-benzothiazepin-4-on;
(3R)-3-Amino-5-[(4-chlorphenyl)methyl]-7-[5-[(3,3-difluor-1-methylcyclobutyl)amino]-1,2,4-oxadiazol-3-yl]-8-fluor-1,1-dioxo-2,3-dihydro-1lambda6,5-benzothiazepin-4-on;
(3R)-3-Amino-5-[(4-chlorphenyl)methyl]-8-fluor-7-[2-[(3R)-1-methylpyrrolidin-3-yl]tetrazol-5-yl]-1,1-dioxo-2,3-dihydro-1lambda6,5-benzothiazepin-4-on;
(3R)-3-Amino-5-[(4-chlorphenyl)methyl]-8-fluor-7-[2-[(3S)-1-methylpyrrolidin-3-yl]tetrazol-5-yl]-1,1-dioxo-2,3-dihydro-1lambda6,5-benzothiazepin-4-on;
3-[(3R)-3-Amino-5-[(4-chlorphenyl)methyl]-8-fluor-1,1,4-trioxo-2,3-dihydro-1lambda6,5-benzothiazepin-7-yl]-N,N-dimethyl-1,2,4-oxadiazol-5-carboxamid;
3-[(3R)-3-Amino-8-fluor-1,1,4-trioxo-5-[[4-(trifluormethoxy)phenyl]methyl]-2,3-dihydro-1lambda6,5-benzothiazepin-7-yl]-N,N-dimethyl-1,2,4-oxadiazol-5-carboxamid;
(3R)-3-Amino-5-[(4-chlorphenyl)methyl]-7-[1-(5,5-difluor-1-methyl-3-piperidyl)pyrazol-4-yl]-8-fluor-1,1-dioxo-2,3-dihydro-1lambda6,5-benzothiazepin-4-on;
(3R)-3-Amino-7-[5-[(2-amino-3,3,3-trifluorpropyl)amino]-1,2,4-oxadiazol-3-yl]-5-[(4-chlorphenyl)methyl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-1,1-dioxo-5-[(4-pyrrolidin-1-ylphenyl)methyl]-2,3-dihydro-1lambda6,5-benzothiazepin-4-on;
(6-Methyl-3-pyridyl)-4-[5-[(3R)-3-amino-5-[(4-chlorphenyl)methyl]-1,1,4-trioxo-2,3-dihydro-1lambda6,5-benzothiazepin-7-yl]-1,3,4-oxadiazol-2-yl]-4-methylpiperidin-1-carboxylat;
(3R)-3-Amino-7-[5-(5,5-difluor-1-methyl-3-piperidyl)-1,2,4-oxadiazol-3-yl]-8-fluor-5-[(5-isopropoxy-2-pyridyl)methyl]-1,1-dioxo-2,3-dihydro-1lambda6,5-benzothiazepin-4-on;
(3R)-3-Amino-5-[(4-chlorphenyl)methyl]-7-[5-(1-ethyl-4-methyl-4-piperidyl)-1,2,4-oxadiazol-3-yl]-8-fluor-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-(1,2,2,2-tetrafluor-1-methoxyethyl)-1,2,4-oxadiazol-3-yl]-2,3-dihydro-1lambda6,5-benzothiazepin-4-on;
2-[3-[(3R)-3-Amino-5-[(4-chlorphenyl)methyl]-8-fluor-1,1,4-trioxo-2,3-dihydro-1lambda6,5-benzothiazepin-7-yl]-1,2,4-oxadiazol-5-yl]-2-methylpropannitril;
(3R)-3-Amino-5-[(4-chlorphenyl)methyl]-7-[5-(5,5-dimethyl-3-piperidyl)-1,2,4-oxadiazol-3-yl]-8-fluor-1,1-dioxo-2,3-dihydro-1lambda6,5-benzothiazepin-4-on;
2,2,2-Trifluorethyl-4-[5-[(3R)-3-amino-5-[(4-chlorphenyl)methyl]-1,1,4-trioxo-2,3-dihydro-1lambda6,5-benzothiazepin-7-yl]-1,3,4-oxadiazol-2-yl]-4-methylpiperidin-1-carboxylat;
Methyl-4-[3-[(3R)-3-amino-5-[(4-chlorphenyl)methyl]-8-fluor-1,1,4-trioxo-2,3-dihydro-1lambda6,5-benzothiazepin-7-yl]-1,2,4-oxadiazol-5-yl]-4-methylpiperidin-1-carboxylat;
(3R)-3-Amino-5-[(4-chlorphenyl)methyl]-8-fluor-1,1-dioxo-7-[5-[[1-(2,2,2-trifluorethyl)-3-piperidyl]amino]-1,3,4-oxadiazol-2-yl]-2,3-dihydro-1lambda6,5-benzothiazepin-4-on;
(3R)-3-Amino-7-[2-(2-amino-3,3,3-trifluorpropyl)tetrazol-5-yl]-5-[(4-chlorphenyl)methyl]-8-fluor-1,1-dioxo-2,3-dihydro-1lambda6,5-benzothiazepin-4-on;
4-[3-[(3R)-3-Amino-5-[(4-chlorphenyl)methyl]-8-fluor-1,1,4-trioxo-2,3-dihydro-1lambda6,5-benzothiazepin-7-yl]-1,2,4-oxadiazol-5-yl]-N,4-dimethylpiperidin-1-carboxamid;
(3R)-3-Amino-5-[(4-chlorphenyl)methyl]-8-fluor-7-[5-[4-methyl-1-(pyrrolidin-1-carbonyl)-4-piperidyl]-1,2,4-oxadiazol-3-yl]-1,1-dioxo-2,3-dihydro-1lambda6,5-benzothiazepin-4-on;
(3R)-3-Amino-7-[5-(5,5-difluor-1-methyl-3-piperidyl)-1,3,4-oxadiazol-2-yl]-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-8-fluor-1,1-dioxo-7-[5-[(2,2,2-trifluor-1-methylethyl)amino]-1,3,4-oxadiazol-2-yl]-2,3-dihydro-1lambda6,5-benzothiazepin-4-on;
(3R)-3-Amino-7-[5-(1-amino-2,2,2-trifluor-1-methylethyl)-1,2,4-oxadiazol-3-yl]-5-[(4-chlorphenyl)methyl]-8-fluor-1,1-dioxo-2,3-dihydro-1lambda6,5-benzothiazepin-4-on;
Methyl-3-[5-[(3R)-3-amino-5-[(4-chlorphenyl)methyl]-8-fluor-1,1,4-trioxo-2,3-dihydro-1lambda6,5-benzothiazepin-7-yl]tetrazol-2-yl]pyrrolidin-1-carboxylat;
(3R)-3-Amino-5-[(4-chlorphenyl)methyl]-7-[5-[(3,3-difluorcyclobutyl)amino]-1,2,4-oxadiazol-3-yl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-5-[[4-(4-cyclopropylpiperazin-1-yl)phenyl]methyl]-1,1-dioxo-2,3-dihydro-1lambda6,5-benzothiazepin-4-on;
(3R)-3-Amino-7-(5-tert-butyl-1,3,4-oxadiazol-2-yl)-5-[[4-(4-methoxy-1-piperidyl)phenyl]methyl]-1,1-dioxo-2,3-dihydro-1lambda6,5-benzothiazepin-4-on;
(3R)-3-Amino-7-(5-tert-butyl-1,3,4-oxadiazol-2-yl)-5-[[4-(cyclopropylmethoxy)phenyl]methyl]-1,1-dioxo-2,3-dihydro-1lambda6,5-benzothiazepin-4-on;
(3R)-3-Amino-7-(5-tert-butyl-1,3,4-oxadiazol-2-yl)-5-[[4-(2,2-difluorethoxy)phenyl]methyl]-1,1-dioxo-2,3-dihydro-1lambda6,5-benzothiazepin-4-on;
(3R)-3-Amino-7-(5-tert-butyl-1,3,4-oxadiazol-2-yl)-5-[(4-but-2-inoxyphenyl)methyl]-1,1-dioxo-2,3-dihydro-1lambda6,5-benzothiazepin-4-on;
(3R)-3-Amino-7-[5-(1-amino-2,2,2-trifluor-1-methylethyl)-1,3,4-oxadiazol-2-yl]-5-[(4-chlorphenyl)methyl]-1,1-dioxo-2,3-dihydro-1lambda6,5-benzothiazepin-4-on;
(3R)-3-Amino-7-(5-tert-butyl-1,3,4-oxadiazol-2-yl)-1,1-dioxo-5-[[4-(2,2,2-trifluorethoxy)phenyl]methyl]-2,3-dihydro-1lambda6,5-benzothiazepin-4-on;
(3R)-3-Amino-7-(5-tert-butyl-1,3,4-oxadiazol-2-yl)-5-[(4-morpholinophenyl)methyl]-1,1-dioxo-2,3-dihydro-1lambda6,5-benzothiazepin-4-on;
(3R)-3-Amino-5-[(4-chlorphenyl)methyl]-8-fluor-1,1-dioxo-7-[5-[3-(trifluormethyl)piperazin-1-yl]-1,2,4-oxadiazol-3-yl]-2,3-dihydro-1lambda6,5-benzothiazepin-4-on;2,2,2-Trifluoressigsäure;
(3R)-3-Amino-1,1-dioxo-7-[5-(1,1,2,2,2-pentafluorethyl)-1,3,4-oxadiazol-2-yl]-5-[[4-(trifluormethoxy)phenyl]methyl]-2,3-dihydro-1lambda6,5-benzothiazepin-4-on;
(3R)-3-Amino-5-[(4-chlorphenyl)methyl]-7-[5-[(3,3-difluorcyclohexyl)amino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[(3,3-difluorcyclobutyl)amino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[(3,3-difluor-1-methylcyclobutyl)amino]-1,3,4-oxadiazol-2-yl]-8-fluor-1,1-dioxo-2,3-dihydro-1lambda6,5-benzothiazepin-4-on;
2-[4-[[(3R)-3-Amino-7-(5-tert-butyl-1,3,4-oxadiazol-2-yl)-1,1,4-trioxo-2,3-dihydro-1lambda6,5-benzothiazepin-5-yl]methyl]phenyl]-2-methylpropannitril;
(3R)-3-Amino-5-[(4-chlorphenyl)methyl]-7-[5-(4,4-difluor-1-piperidyl)-1,2,4-oxadiazol-3-yl]-8-fluor-1,1-dioxo-2,3-dihydro-1lambda6,5-benzothiazepin-4-on;
(3R)-3-Amino-5-[(4-chlorphenyl)methyl]-8-fluor-7-(5-morpholino-1,2,4-oxadiazol-3-yl)-1,1-dioxo-2,3-dihydro-1lambda6,5-benzothiazepin-4-on;
(3R)-3-Amino-5-[(4-chlorphenyl)methyl]-7-[5-(cyclobutylamino)-1,2,4-oxadiazol-3-yl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(4-oxa-7-azaspiro[2.5]octan-7-yl)-1,2,4-oxadiazol-3-yl]-1,1-dioxo-2,3-dihydro-1lambda6,5-benzothiazepin-4-on;
(3R)-7-[2-(1-Acetyl-3-piperidyl)tetrazol-5-yl]-3-amino-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-[2-(1-methyl-3-piperidyl)tetrazol-5-yl]-1,1-dioxo-2,3-dihydro-1lambda6,5-benzothiazepin-4-on;
(3R)-3-Amino-7-(3-tert-butyl-1,2,4-triazol-1-yl)-5-[(4-chlorphenyl)methyl]-8-fluor-1,1-dioxo-2,3-dihydro-1lambda6,5-benzothiazepin-4-on;
(3R)-3-Amino-7-[5-[1-methyl-1-(methylamino)ethyl]-1,3,4-oxadiazol-2-yl]-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-7-[5-[3-(dimethylamino)-1,1-dimethylpropyl]-1,3,4-oxadiazol-2-yl]-8-fluor-1,1-dioxo-2,3-dihydro-1lambda6,5-benzothiazepin-4-on;
3-[(3R)-3-Amino-1,1,4-trioxo-5-[[4-(trifluormethoxy)phenyl]methyl]-2,3-dihydro-1lambda6,5-benzothiazepin-7-yl]-N-tert-butyl-1,2,4-oxadiazol-5-carboxamid;
3-[(3R)-3-Amino-8-fluor-1,1,4-trioxo-5-[[4-(trifluormethoxy)phenyl]methyl]-2,3-dihydro-1lambda6,5-benzothiazepin-7-yl]-N-tert-butyl-1,2,4-oxadiazol-5-carboxamid;
3-[(3R)-3-Amino-5-[(4-chlorphenyl)methyl]-8-fluor-1,1,4-trioxo-2,3-dihydro-1lambda6,5-benzothiazepin-7-yl]-N-tert-butyl-1,2,4-oxadiazol-5-carboxamid;
3-[(3R)-3-Amino-5-[(4-chlorphenyl)methyl]-1,1,4-trioxo-2,3-dihydro-1lambda6,5-benzothiazepin-7-yl]-N-tert-butyl-1,2,4-oxadiazol-5-carboxamid;
(3R)-3-Amino-5-[(4-chlorphenyl)methyl]-7-[5-[(dimethylamino)methyl]-1,2,4-oxadiazol-3-yl]-8-fluor-1,1-dioxo-2,3-dihydro-1lambda6,5-benzothiazepin-4-on;
(3R)-3-Amino-7-[5-(1-aminocyclopentyl)-1,3,4-oxadiazol-2-yl]-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-7-[5-(3,3-difluorcyclohexyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[cyclopropyl(methyl)amino]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[methyl(2,2,2-trifluorethyl)amino]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[[3,3-difluor-1-(methoxymethyl)cyclobutyl]amino]-1,3,4-oxadiazol-2-yl]-1,1-dioxo-2,3-dihydro-1lambda6,5-benzothiazepin-4-on;
(3R)-3-Amino-7-[5-(4-aminotetrahydropyran-4-yl)-1,3,4-oxadiazol-2-yl]-1,1-dioxo-5-[[4-(trifluormethoxy)phenyl]methyl]-2,3-dihydro-1lambda6,5-benzothiazepin-4-on;
(3R)-3-Amino-7-[5-(1-aminocyclohexyl)-1,3,4-oxadiazol-2-yl]-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-8-fluor-7-[5-(1-hydroxy-1-methylethyl)-1,3,4-oxadiazol-2-yl]-1,1-dioxo-2,3-dihydro-1lambda6,5-benzothiazepin-4-on;2,2,2-Trifluoressigsäure;
(3R)-3-Amino-7-(2-tert-butyltetrazol-5-yl)-1,1-dioxo-5-[[4-(trifluormethoxy)phenyl]methyl]-2,3-dihydro-1lambda6,5-benzothiazepin-4-on;
(3R)-3-Amino-7-(2-tert-butyltetrazol-5-yl)-8-fluor-1,1-dioxo-5-[[4-(trifluormethoxy)phenyl]methyl]-2,3-dihydro-1lambda6,5-benzothiazepin-4-on;
(3R)-3-Amino-5-[(4-chlorphenyl)methyl]-8-fluor-7-[2-(1-methylsulfonyl-3-piperidyl)tetrazol-5-yl]-1,1-dioxo-2,3-dihydro-1lambda6,5-benzothiazepin-4-on;
(3R)-3-Amino-5-[(4-chlorphenyl)methyl]-7-[5-[(3,3-difluor-1-methylcyclobutyl)amino]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[(3,3-difluorcyclobutyl)amino]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[(3,3-difluorcyclohexyl)amino]-1,3,4-oxadiazol-2-yl]-1,1-dioxo-2,3-dihydro-1lambda6,5-benzothiazepin-4-on;
(3R)-3-Amino-7-(4-tert-butylimidazol-1-yl)-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-(5-oxa-2-azaspiro[3.4]octan-2-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(2-cyclopropyltetrahydrofuran-2-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(2-cyclopropyltetrahydrofuran-2-yl)-1,3,4-oxadiazol-2-yl]-1,1-dioxo-2,3-dihydro-1lambda6,5-benzothiazepin-4-on;
(3R)-3-Amino-7-(2-tert-butyltetrazol-5-yl)-5-[(4-chlorphenyl)methyl]-1,1-dioxo-2,3-dihydro-1lambda6,5-benzothiazepin-4-on;
(3R)-7-[5-(3-Acetyl-3-azabicyclo[3.1.0]hexan-1-yl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
Methyl-3-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]azetidin-1-carboxylat;2,2,2-Trifluoressigsäure;
(3R)-3-Amino-5-[(4-chlorphenyl)methyl]-7-[5-(2,2-difluormorpholin-4-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-1,1-dioxo-7-[5-(1,2,2,2-tetrafluorethyl)-1,3,4-oxadiazol-2-yl]-2,3-dihydro-1lambda6,5-benzothiazepin-4-on;
(3R)-3-Amino-5-[(4-chlorphenyl)methyl]-8-fluor-7-[5-(3-methylsulfonyl-3-azabicyclo[3.1.0]hexan-1-yl)-1,3,4-oxadiazol-2-yl]-1,1-dioxo-2,3-dihydro-1lambda6,5-benzothiazepin-4-on;
Methyl-1-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-3-azabicyclo[3.1.0]hexan-3-carboxylat;
(3R)-3-Amino-5-[(4-chlorphenyl)methyl]-8-fluor-7-[2-(1-methylsulfonylpyrrolidin-3-yl)tetrazol-5-yl]-1,1-dioxo-2,3-dihydro-1lambda6,5-benzothiazepin-4-on;
(3R)-3-Amino-5-[(4-chlorphenyl)methyl]-7-[5-(2-hydroxyspiro[3.3]heptan-6-yl)-1,3,4-oxadiazol-2-yl]-1,1-dioxo-2,3-dihydro-1lambda6,5-benzothiazepin-4-on;
(3R)-3-Amino-7-[5-[3-(chlormethyl)-3-(hydroxymethyl)azetidin-1-yl]-1,3,4-oxadiazol-2-yl]-5-[(4-chlorphenyl)methyl]-1,1-dioxo-2,3-dihydro-1lambda6,5-benzothiazepin-4-on;
2-[5-[(3R)-3-Amino-5-[(4-chlorphenyl)methyl]-1,1,4-trioxo-2,3-dihydro-1lambda6,5-benzothiazepin-7-yl]-1,3,4-oxadiazol-2-yl]-2-methoxyacetonitril;
(3R)-3-Amino-1,1-dioxo-7-[5-(2,2,2-trifluorethyl)-1,3,4-oxadiazol-2-yl]-5-[[4-(trifluormethoxy)phenyl]methyl]-2,3-dihydro-1lambda6,5-benzothiazepin-4-on;
(3R)-3-Amino-5-[(4-chlorphenyl)methyl]-8-fluor-7-[2-(1-methylsulfonyl-4-piperidyl)tetrazol-5-yl]-1,1-dioxo-2,3-dihydro-1lambda6,5-benzothiazepin-4-on;
(3R)-3-Amino-7-(5-tert-butyl-1,3,4-oxadiazol-2-yl)-1,1-dioxo-5-[[4-(1,1,2,2-tetrafluorethoxy)phenyl]methyl]-2,3-dihydro-1lambda6,5-benzothiazepin-4-on;
(3R)-3-Amino-5-[(4-chlorphenyl)methyl]-8-fluor-7-[5-(5-oxa-2-azaspiro[3.4]octan-2-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(6-oxa-2-azaspiro[3.5]nonan-2-yl)-1,3,4-oxadiazol-2-yl]-1,1-dioxo-2,3-dihydro-1lambda6,5-benzothiazepin-4-on;
(3R)-3-Amino-7-(5-tert-butyl-1,3,4-oxadiazol-2-yl)-5-[[4-(cyclopentoxy)phenyl]methyl]-1,1-dioxo-2,3-dihydro-1lambda6,5-benzothiazepin-4-on;
(3R)-3-Amino-5-[(4-chlorphenyl)methyl]-1,1-dioxo-7-[5-(1,1,2,2,2-pentafluorethyl)-1,3,4-oxadiazol-2-yl]-2,3-dihydro-1lambda6,5-benzothiazepin-4-on;
Methyl-7-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-5-azaspiro[2.4]heptan-5-carboxylat;
2-[5-[(3R)-3-Amino-8-fluor-1,1,4-trioxo-5-[[4-(trifluormethoxy)phenyl]methyl]-2,3-dihydro-1lambda6,5-benzothiazepin-7-yl]-1,3,4-oxadiazol-2-yl]-2-methylpropannitril;
(3R)-3-Amino-1,1-dioxo-7-[5-(1,2,2,2-tetrafluor-1-methoxyethyl)-1,3,4-oxadiazol-2-yl]-5-[[4-(trifluormethoxy)phenyl]methyl]-2,3-dihydro-1lambda6,5-benzothiazepin-4-on;
(3R)-3-Amino-5-[(4-chlorphenyl)methyl]-1,1-dioxo-7-[5-(1,2,2,2-tetrafluor-1-methoxyethyl)-1,3,4-oxadiazol-2-yl]-2,3-dihydro-1lambda6,5-benzothiazepin-4-on;
(3R)-3-Amino-7-[5-[(1R,5S)-8-azabicyclo[3.2.1]octan-8-carbonyl]-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(5-methylsulfonyl-5-azaspiro[2.4]heptan-7-yl)-1,3,4-oxadiazol-2-yl]-1,1-dioxo-2,3-dihydro-1lambda6,5-benzothiazepin-4-on;
(3R)-7-[5-(1-Acetyl-4-piperidyl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-(2-methyltetrazol-5-yl)-1,1-dioxo-2,3-dihydro-1lambda6,5-benzothiazepin-4-on;
(3R)-3-Amino-5-[(4-chlorphenyl)methyl]-8-fluor-7-[2-(1-methylpyrrolidin-3-yl)tetrazol-5-yl]-1,1-dioxo-2,3-dihydro-1lambda6,5-benzothiazepin-4-on;
(3R)-7-[2-(1-Acetyl-4-piperidyl)tetrazol-5-yl]-3-amino-5-[(4-chlorphenyl)methyl]-8-fluor-1,1-dioxo-2,3-dihydro-1lambda6,5-benzothiazepin-4-on;
(3R)-7-[5-(3-Acetyl-3-azabicyclo[4.1.0]heptan-1-yl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(3-methylsulfonyl-3-azabicyclo[4.1.0]heptan-l-yl)-1,3,4-oxadiazol-2-yl]-1,1-dioxo-2,3-dihydro-1lambda6,5-benzothiazepin-4-on;
Methyl-1-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-3-azabicyclo[4.1.0]heptan-3-carboxylat;
(3R)-3-Amino-5-[(4-chlorphenyl)methyl]-7-[5-(6,6-difluor-2-azaspiro[3.3]heptan-2-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(isopropylamino)-1,3,4-oxadiazol-2-yl]-1,1-dioxo-2,3-dihydro-1lambda6,5-benzothiazepin-4-on;
(3R)-3-Amino-7-[5-(2-azaspiro[3.3]heptan-2-yl)-1,3,4-oxadiazol-2-yl]-5-[(4-chlorphenyl)methyl]-1,1-dioxo-2,3-dihydro-1lambda6,5-benzothiazepin-4-on;
Methyl-5-[[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]amino]-3,3-difluorpiperidin-1-carboxylat;
(3R)-3-Amino-5-[(4-chlorphenyl)methyl]-7-[5-(4,4-difluor-1-piperidyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(2,2-dimethylmorpholin-4-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(3-fluor-1-methyl-3-piperidyl)-1,3,4-oxadiazol-2-yl]-1,1-dioxo-2,3-dihydro-1lambda6,5-benzothiazepin-4-on;
Methyl-4-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]piperidin-1-carboxylat;
(3R)-3-Amino-7-[5-(1-amino-2,2,2-trifluorethyl)-1,3,4-oxadiazol-2-yl]-8-fluor-1,1-dioxo-5-[[4-(trifluormethoxy)phenyl]methyl]-2,3-dihydro-1lambda6,5-benzothiazepin-4-on;
Methyl-3-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]pyrrolidin-l-carboxylat;
(3R)-3-Amino-5-[(4-chlorphenyl)methyl]-7-[5-(5,5-difluor-1-methyl-3-piperidyl)-1,3,4-oxadiazol-2-yl]-8-methyl-1,1-dioxo-2,3-dihydro-1lambda6,5-benzothiazepin-4-on;
(3R)-3-Amino-5-[(4-chlorphenyl)methyl]-7-[5-(5,5-difluor-1-methyl-3-piperidyl)-1,3,4-oxadiazol-2-yl]-1,1-dioxo-2,3-dihydro-1lambda6,5-benzothiazepin-4-on;
2-[5-[(3R)-3-Amino-5-[(4-chlorphenyl)methyl]-8-methyl-1,1,4-trioxo-2,3-dihydro-1lambda6,5-benzothiazepin-7-yl]-1,3,4-oxadiazol-2-yl]-2-methylpropannitril;
(3R)-3-Amino-5-[(4-chlorphenyl)methyl]-7-[5-[(5,5-difluor-3-piperidyl)amino]-1,3,4-oxadiazol-2-yl]-8-fluor-1,1-dioxo-2,3-dihydro-1lambda6,5-benzothiazepin-4-on;
(3R)-3-Amino-7-[5-(3-azabicyclo[4.1.0]heptan-l-yl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-7-[5-(5-Acetyl-5-azaspiro[2.4]heptan-7-yl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-(3,3-difluorpiperidin-1-carbonyl)-1,3,4-oxadiazol-2-yl]-8-fluor-1,1-dioxo-2,3-dihydro-1lambda6,5-benzothiazepin-4-on;
2-[5-[(3R)-3-Amino-5-[(4-chlorphenyl)methyl]-8-methyl-1,1,4-trioxo-2,3-dihydro-1lambda6,5-benzothiazepin-7-yl]-1,3,4-oxadiazol-2-yl]propannitril;
(3R)-3-Amino-5-[(4-chlorphenyl)methyl]-8-fluor-7-[5-(3-methylsulfonyl-3-azabicyclo[3.1.1]heptan-l-yl)-1,3,4-oxadiazol-2-yl]-1,1-dioxo-2,3-dihydro-1lambda6,5-benzothiazepin-4-on;
Methyl-1-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-3-azabicyclo[3.1.1]heptan-3-carboxylat;
Methyl-1-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-3-azabicyclo[3.1.1]heptan-3-carboxylat;
2-[5-[(3R)-3-Amino-5-[(4-chlorphenyl)methyl]-1,1,4-trioxo-2,3-dihydro-1lambda6,5-benzothiazepin-7-yl]-1,3,4-oxadiazol-2-yl]-2-methylpropannitril;
(3R)-7-[5-(3-Acetyl-3-azabicyclo[3.1.1]heptan-l-yl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
(3R)-3-Amino-7-[5-(1-amino-2,2,2-trifluorethyl)-1,3,4-oxadiazol-2-yl]-5-[(4-chlorphenyl)methyl]-8-fluor-1,1-dioxo-2,3-dihydro-1lambda6,5-benzothiazepin-4-on;2,2,2-Trifluoressigsäure;
(3R)-3-Amino-7-[5-(1-amino-2,2,2-trifluorethyl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-[4-(dimethylamino)-1,1-dimethylbutyl]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[(3,3-difluorcyclopentyl)amino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[(4,4-difluorcyclohexyl)amino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-(1,3-dimethylazetidin-3-yl)-1,3,4-oxadiazol-2-yl]-8-fluor-1,1-dioxo-2,3-dihydro-1lambda6,5-benzothiazepin-4-on;
(3R)-3-Amino-7-[5-(tert-butylamino)-1,3,4-oxadiazol-2-yl]-5-[(4-chlorphenyl)methyl]-8-fluor-1,1-dioxo-2,3-dihydro-1lambda6,5-benzothiazepin-4-on;
1-[5-[(3R)-3-Amino-5-[(4-chlorphenyl)methyl]-8-fluor-1,1,4-trioxo-2,3-dihydro-1lambda6,5-benzothiazepin-7-yl]-1,3,4-oxadiazol-2-yl]cyclopropancarbonitril;
2-[5-[(3R)-3-Amino-5-[(4-chlorphenyl)methyl]-1,1,4-trioxo-2,3-dihydro-1lambda6,5-benzothiazepin-7-yl]-1,3,4-oxadiazol-2-yl]propannitril;
Methyl-3-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]piperidin-1-carboxylat;
(3R)-3-Amino-5-[(4-chlorphenyl)methyl]-7-[5-(3,3-difluorpyrrolidin-1-carbonyl)-1,3,4-oxadiazol-2-yl]-8-fluor-1,1-dioxo-2,3-dihydro-1lambda6,5-benzothiazepin-4-on;
(3R)-3-Amino-7-[5-(2-aminospiro[3.3]heptan-6-yl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(2-oxa-7-azaspiro[3.5]nonan-7-yl)-1,3,4-oxadiazol-2-yl]-1,1-dioxo-2,3-dihydro-1lambda6,5-benzothiazepin-4-on;
4-[[(3R)-3-Amino-8-fluor-7-[5-(2-hydroxy-1,1-dimethylethyl)-1,3,4-oxadiazol-2-yl]-1,1,4-trioxo-2,3-dihydro-1lambda6,5-benzothiazepin-5-yl]methyl]benzonitril;
(3R)-3-Amino-5-[(4-chlorphenyl)methyl]-7-[5-(5,5-difluor-l-methyl-3-piperidyl)-1,2,4-oxadiazol-3-yl]-8-fluor-1,1-dioxo-2,3-dihydro-1lambda6,5-benzothiazepin-4-on;
(3R)-3-Amino-7-[5-(5-amino-3,3-difluor-1-piperidyl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-7-[5-[1-(chlormethyl)-2-hydroxy-l-methylethyl]-1,3,4-oxadiazol-2-yl]-5-[(4-chlorphenyl)methyl]-8-fluor-1,1-dioxo-2,3-dihydro-1lambda6,5-benzothiazepin-4-on;
5-[(3R)-3-Amino-5-[(4-chlorphenyl)methyl]-8-fluor-1,1,4-trioxo-2,3-dihydro-1lambda6,5-benzothiazepin-7-yl]-N-isopropyl-N-methyl-1,3,4-oxadiazol-2-carboxamid;
(3R)-3-Amino-5-[(4-chlorphenyl)methyl]-7-[5-(1-cyclopropyl-5,5-difluor-3-piperidyl)-1,3,4-oxadiazol-2-yl]-8-fluor-1,1-dioxo-2,3-dihydro-1lambda6,5-benzothiazepin-4-on;
(3R)-3-Amino-7-[5-[4-(chlormethyl)-4-(hydroxymethyl)-1-piperidyl]-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-(4,4-difluor-1-methyl-3-piperidyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(2-methylsulfonylethyl)-1,3,4-oxadiazol-2-yl]-1,1-dioxo-2,3-dihydro-1lambda6,5-benzothiazepin-4-on;
5-[(3R)-3-Amino-5-[(4-chlorphenyl)methyl]-8-fluor-1,1,4-trioxo-2,3-dihydro-1lambda6,5-benzothiazepin-7-yl]-N-tert-butyl-1,3,4-oxadiazol-2-carboxamid;
(3R)-3-Amino-5-[(4-chlorphenyl)methyl]-8-fluor-7-[5-(isopropoxymethyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[5,5-difluor-1-(2-methoxyethyl)-3-piperidyl]-1,3,4-oxadiazol-2-yl]-8-fluor-1,1-dioxo-2,3-dihydro-1lambda6,5-benzothiazepin-4-on;
5-[(3R)-3-Amino-5-[(4-chlorphenyl)methyl]-8-fluor-1,1,4-trioxo-2,3-dihydro-1lambda6,5-benzothiazepin-7-yl]-3-tert-butyl-1,3,4-oxadiazol-2-on;
(3R)-3-Amino-5-[(4-chlorphenyl)methyl]-8-fluor-7-[5-(2-methyltetrahydrofuran-2-yl)-1,3,4-oxadiazol-2-yl]-1,1-dioxo-2,3-dihydro-1lambda6,5-benzothiazepin-4-on;
(3R)-3-Amino-7-[5-(4-amino-1,1-dimethylbutyl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-(1,3-dimethyl-3-piperidyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[(1-ethyl-3-piperidyl)amino]-1,3,4-oxadiazol-2-yl]-8-fluor-1,1-dioxo-2,3-dihydro-1lambda6,5-benzothiazepin-4-on;
(3R)-3-Amino-7-[5-(3-amino-4,4-difluor-1-piperidyl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-[2-(1,1-dioxo-1,4-thiazinan-4-yl)-1,1-dimethylethyl]-1,3,4-oxadiazol-2-yl]-8-fluor-1,1-dioxo-2,3-dihydro-1lambda6,5-benzothiazepin-4-on;
5-[(3R)-3-Amino-5-[(4-chlorphenyl)methyl]-8-fluor-1,1,4-trioxo-2,3-dihydro-1lambda6,5-benzothiazepin-7-yl]-N,N-dimethyl-1,3,4-oxadiazol-2-carboxamid;
(3R)-3-Amino-5-[(4-chlorphenyl)methyl]-7-[5-(1-ethyl-5,5-difluor-3-piperidyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(1-methylpyrrolidin-3-yl)-1,3,4-oxadiazol-2-yl]-1,1-dioxo-2,3-dihydro-1lambda6,5-benzothiazepin-4-on;
Methyl-5-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-3,3-difluorpiperidin-1-carboxylat;
(3R)-3-Amino-7-[5-(2-azaspiro[3.3]heptan-2-yl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(isopropylamino)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(4,4-difluor-3-piperidyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[(1-cyclopropyl-4-piperidyl)amino]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-(2,2-dimethylmorpholin-4-yl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(4-oxa-7-azaspiro[2.5]octan-7-yl)-1,3,4-oxadiazol-2-yl]-1,1-dioxo-2,3-dihydro-1lambda6,5-benzothiazepin-4-on;
(3R)-3-Amino-7-(5-tert-butyl-1,3,4-oxadiazol-2-yl)-5-[[6-(cyclopentoxy)-3-pyridyl]methyl]-8-fluor-1,1-dioxo-2,3-dihydro-1lambda6,5-benzothiazepin-4-on;
(3R)-3-Amino-5-[(4-chlorphenyl)methyl]-7-[5-[1,1-dimethyl-2-(2-oxo-1-piperidyl)ethyl]-1,3,4-oxadiazol-2-yl]-8-fluor-1,1-dioxo-2,3-dihydro-1lambda6,5-benzothiazepin-4-on;
2-[5-[(3R)-3-Amino-5-[(4-chlorphenyl)methyl]-8-fluor-1,1,4-trioxo-2,3-dihydro-1lambda6,5-benzothiazepin-7-yl]-1,3,4-oxadiazol-2-yl]-2-methylpropannitril;
2-[5-[(3R)-3-Amino-5-[(4-chlorphenyl)methyl]-8-fluor-1,1,4-trioxo-2,3-dihydro-1lambda6,5-benzothiazepin-7-yl]-1,3,4-oxadiazol-2-yl]propannitril;
(3R)-3-Amino-5-[(4-chlorphenyl)methyl]-8-fluor-7-[5-(3-methylazetidin-3-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(5,5-difluor-l-methylsulfonyl-3-piperidyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[5,5-difluor-1-(2-hydroxyethyl)-3-piperidyl]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-8-fluor-7-[5-[1-(hydroxymethyl)cyclopropyl]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(cyclobutylamino)-1,3,4-oxadiazol-2-yl]-8-fluor-1,1-dioxo-2,3-dihydro-1lambda6,5-benzothiazepin-4-on;
(3R)-7-[5-(1-Acetyl-5,5-difluor-3-piperidyl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-(5,5-difluor-3-piperidyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-(3-ethyl-1,2,4-triazol-1-yl)-8-fluor-1,1-dioxo-2,3-dihydro-1lambda6,5-benzothiazepin-4-on;
(3R)-7-[5-(4-Acetylpiperazin-1-yl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-(5-isobutyl-1,3,4-oxadiazol-2-yl)-1,1-dioxo-2,3-dihydro-1lambda6,5-benzothiazepin-4-on;
(3R)-3-Amino-5-[(4-chlorphenyl)methyl]-8-fluor-7-[5-(4-methylpiperazin-1-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-[2-[2-hydroxyethyl(methyl)amino]-1,1-dimethylethyl]-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[1,1-dimethyl-2-(2-oxopyrrolidin-1-yl)ethyl]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(1-methyl-3-piperidyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-[1,1-dimethyl-2-(4-methylpiperazin-1-yl)ethyl]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[1,1-dimethyl-2-(tetrahydrofuran-3-ylamino)ethyl]-1,3,4-oxadiazol-2-yl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-8-fluor-5-[(5-isopropoxy-2-pyridyl)methyl]-1,1-dioxo-2,3-dihydro-1lambda6,5-benzothiazepin-4-on;
(3R)-3-Amino-5-[(4-chlorphenyl)methyl]-7-[5-[1,1-dimethyl-2-(oxetan-3-ylamino)ethyl]-1,3,4-oxadiazol-2-yl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-5-[(3,4-difluorphenyl)methyl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-5-[(4-chlor-3-fluorphenyl)methyl]-8-fluor-1,1-dioxo-2,3-dihydro-1lambda6,5-benzothiazepin-4-on;
(3R)-3-Amino-5-[(4-chlorphenyl)methyl]-7-[5-(4,4-difluor-1-piperidyl)-1,3,4-oxadiazol-2-yl]-8-fluor-1,1-dioxo-2,3-dihydro-1lambda6,5-benzothiazepin-4-on;1,1,1,3,3,3-Hexafluorpropan-2-ol;
(3R)-3-Amino-5-[(4-chlorphenyl)methyl]-7-[5-(1,1-dimethyl-2-morpholinoethyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-[2-(dimethylamino)-1,1-dimethylethyl]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-[5-(6,6-difluor-2-azaspiro[3.3]heptan-2-yl)-1,3,4-oxadiazol-2-yl]-8-fluor-1,1-dioxo-2,3-dihydro-1lambda6,5-benzothiazepin-4-on;1,1,1,3,3,3-Hexafluorpropan-2-ol;
(3R)-3-Amino-7-(5-tert-butyl-1,3,4-oxadiazol-2-yl)-8-fluor-5-[(6-isopropoxy-3-pyridyl)methyl]-1,1-dioxo-2,3-dihydro-1lambda6,5-benzothiazepin-4-on;
(3R)-3-Amino-5-[(4-chlorphenyl)methyl]-7-[5-[2-(4,4-difluor-1-piperidyl)-1,1-dimethylethyl]-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-8-fluor-7-[2-(2-hydroxy-1,1-dimethylethyl)tetrazol-5-yl]-1,1-dioxo-2,3-dihydro-1lambda6,5-benzothiazepin-4-on;
(3R)-3-Amino-5-[(4-chlorphenyl)methyl]-8-fluor-7-[2-(2-hydroxy-2-methylpropyl)tetrazol-5-yl]-1,1-dioxo-2,3-dihydro-1lambda6,5-benzothiazepin-4-on;
(3R)-3-Amino-5-[(4-chlorphenyl)methyl]-7-[5-(cyclopropylamino)-1,3,4-oxadiazol-2-yl]-8-fluor-1,1-dioxo-2,3-dihydro-1lambda6,5-benzothiazepin-4-on;1,1,1,3,3,3-Hexafluorpropan-2-ol;
(3R)-3-Amino-5-[(4-chlorphenyl)methyl]-7-[5-[1,1-dimethyl-2-(1-piperidyl)ethyl]-1,3,4-oxadiazol-2-yl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-5-[(4-chlorphenyl)methyl]-8-fluor-1-oxo-2,3-dihydro-1lambda4,5-benzothiazepin-4-on;
(3R)-3-Amino-7-(5-tert-butyl-1,3,4-oxadiazol-2-yl)-5-[(4-chlorphenyl)methyl]-8-fluor-1-oxo-2,3-dihydro-1lambda4,5-benzothiazepin-4-on;
(3R)-3-Amino-5-[(4-chlorphenyl)methyl]-7-[5-(2,2-difluorspiro[3.3]heptan-6-yl)-1,3,4-oxadiazol-2-yl]-8-fluor-1,1-dioxo-2,3-dihydro-1lambda6,5-benzothiazepin-4-on;1,1,1,3,3,3-Hexafluorpropan-2-ol;
(3R)-3-Amino-5-[(4-chlorphenyl)methyl]-7-[5-(dimethylamino)-1,3,4-oxadiazol-2-yl]-8-fluor-1,l-dioxo-2,3-dihydro-1lambda6,5-benzothiazepin-4-on;1,1,1,3,3,3-Hexafluorpropan-2-ol;
(3R)-3-Amino-5-[(4-chlorphenyl)methyl]-8-fluor-7-[5-[(3-methylazetidin-1-yl)methyl]-1,3,4-oxadiazol-2-yl]-2,3-dihydro-1,5-benzothiazepin-4-on;1,1,1,3,3,3-Hexafluorpropan-2-ol;
Methyl-4-[5-[(3R)-3-amino-5-[(4-chlorphenyl)methyl]-8-fluor-1,1,4-trioxo-2,3-dihydro-1lambda6,5-benzothiazepin-7-yl]-1,3,4-oxadiazol-2-yl]-4-methylpiperidin-1-carboxylat;
(3R)-3-Amino-5-[(4-chlorphenyl)methyl]-8-fluor-7-[5-(4-methyl-1-methylsulfonyl-4-piperidyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(cyclohexylamino)-1,3,4-oxadiazol-2-yl]-8-fluor-1,1-dioxo-2,3-dihydro-1lambda6,5-benzothiazepin-4-on;1,1,1,3,3,3-Hexafluorpropan-2-ol;
(3R)-3-Amino-5-[(4-chlorphenyl)methyl]-8-fluor-7-[5-(1-methylcyclopropyl)-1,3,4-oxadiazol-2-yl]-1,1-dioxo-2,3-dihydro-1lambda6,5-benzothiazepin-4-on;1,1,1,3,3,3-Hexafluorpropan-2-ol;
(3R)-3-Amino-5-[(4-chlorphenyl)methyl]-7-[5-[3-(difluormethyl)azetidin-3-yl]-1,3,4-oxadiazol-2-yl]-8-fluor-1,1-dioxo-2,3-dihydro-1lambda6,5-benzothiazepin-4-on;1,1,1,3,3,3-Hexafluorpropan-2-ol;
(3R)-3-Amino-7-[5-(2-amino-1,1-dimethylethyl)-1,3,4-oxadiazol-2-yl]-5-[(4-chlorphenyl)methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(6-oxaspiro[3.4]octan-2-yl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-7-[5-(5,5-difluor-1-methyl-3-piperidyl)-1,3,4-oxadiazol-2-yl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-8-fluor-5-[(3-fluor-4-methylsulfonylphenyl)methyl]-1,1-dioxo-2,3-dihydro-1lambda6,5-benzothiazepin-4-on;
(3R)-3-Amino-7-(5-tert-butyl-1,3,4-oxadiazol-2-yl)-5-[(4-ethyloxazol-2-yl)methyl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-8-fluor-1-imino-1-oxo-5-[[4-(trifluormethoxy)phenyl]methyl]-2,3-dihydro-1lambda6,5-benzothiazepin-4-on;
(3R)-3-Amino-7-(5-tert-butyl-1,3,4-oxadiazol-2-yl)-8-fluor-5-[(2-fluor-4-methylsulfonylphenyl)methyl]-1,1-dioxo-2,3-dihydro-1lambda6,5-benzothiazepin-4-on;1,1,1,3,3,3-Hexafluorpropan-2-ol;
2-[5-[(3R)-3-Amino-5-[(4-chlorphenyl)methyl]-8-fluor-1,1,4-trioxo-2,3-dihydro-1lambda6,5-benzothiazepin-7-yl]-1,3,4-oxadiazol-2-yl]-N,N,2-trimethylpropanamid;1,1,1,3,3,3-Hexafluorpropan-2-ol;
(3R)-3-Amino-5-[(4-chlorphenyl)methyl]-8-fluor-7-[3-(2-hydroxy-1,1-dimethylethyl)-1,2,4-oxadiazol-5-yl]-1,1-dioxo-2,3-dihydro-1lambda6,5-benzothiazepin-4-on;
(3R)-3-Amino-7-(5-tert-butyl-1,3,4-oxadiazol-2-yl)-8-fluor-5-[[4-(3-hydroxyoxetan-3-yl)phenyl]methyl]-1,1-dioxo-2,3-dihydro-1lambda6,5-benzothiazepin-4-on;
(3R)-3-Amino-5-[(4-chlorphenyl)methyl]-8-fluor-7-[5-(2-hydroxy-1,1-dimethylethyl)-1,2,4-oxadiazol-3-yl]-1,1-dioxo-2,3-dihydro-1lambda6,5-benzothiazepin-4-on;2,2,2-Trifluoressigsäure;
(3R)-3-Amino-5-[(4-chlorphenyl)methyl]-8-fluor-7-[5-(2-hydroxy-1,1-dimethylethyl)-1,2,4-oxadiazol-3-yl]-1,1-dioxo-2,3-dihydro-1lambda6,5-benzothiazepin-4-on;
(3R)-3-Amino-7-(5-tert-butyl-1,3,4-oxadiazol-2-yl)-8-fluor-1,1-dioxo-5-[(4-phenoxyphenyl)methyl]-2,3-dihydro-1lambda6,5-benzothiazepin-4-on;
(3R)-3-Amino-7-(5-tert-butyl-1,3,4-oxadiazol-2-yl)-8-fluor-1,1-dioxo-5-[[4-(1,1,2,2-tetrafluorethoxy)phenyl]methyl]-2,3-dihydro-1lambda6,5-benzothiazepin-4-on;
(3R)-3-Amino-7-(5-tert-butyl-1,3,4-oxadiazol-2-yl)-8-fluor-5-[[4-(2-hydroxyethoxy)phenyl]methyl]-1,1-dioxo-2,3-dihydro-1lambda6,5-benzothiazepin-4-on;2,2,2-Trifluoressigsäure;
(3R)-3-Amino-7-(5-tert-butyl-1,3,4-oxadiazol-2-yl)-5-[(4-chlorphenyl)methyl]-8-fluor-9-methyl-1,1-dioxo-2,3-dihydro-1lambda6,5-benzothiazepin-4-on;
(3R)-3-Amino-7-(5-tert-butyl-1,3,4-oxadiazol-2-yl)-5-[[1-(cyclopropylmethyl)pyrazol-4-yl]methyl]-8-fluor-1,1-dioxo-2,3-dihydro-1lambda6,5-benzothiazepin-4-on;2,2,2-Trifluoressigsäure;
(3R)-7-[5-(1-Acetyl-4-methyl-4-piperidyl)-1,3,4-oxadiazol-2-yl]-3-amino-5-[(4-chlorphenyl)methyl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-8-fluor-1-methylimino-1-oxo-5-[[4-(trifluormethoxy)phenyl]methyl]-2,3-dihydro-1lambda6,5-benzothiazepin-4-on;
2-[5-[(3R)-3-Amino-5-[(4-chlorphenyl)methyl]-8-fluor-1,1,4-trioxo-2,3-dihydro-1lambda6,5-benzothiazepin-7-yl]-1,3,4-oxadiazol-2-yl]-N,2-dimethylpropanamid;
(3R)-3-Amino-7-(5-tert-butyl-1,3,4-oxadiazol-2-yl)-5-[(2-cyclopropylpyrimidin-5-yl)methyl]-8-fluor-1,1-dioxo-2,3-dihydro-1lambda6,5-benzothiazepin-4-on;1,1,1,3,3,3-Hexafluorpropan-2-ol;
(3R)-3-Amino-7-(5-tert-butyl-1,3,4-oxadiazol-2-yl)-5-[(6-chlor-3-pyridyl)methyl]-8-fluor-1,1-dioxo-2,3-dihydro-1lambda6,5-benzothiazepin-4-on;1,1,1,3,3,3-Hexafluorpropan-2-ol;
(2R,3R)-3-Amino-7-(5-tert-butyl-1,3,4-oxadiazol-2-yl)-5-[(4-chlorphenyl)methyl]-8-fluor-2-methyl-1,1-dioxo-2,3-dihydro-1lambda6,5-benzothiazepin-4-on;1,1,1,3,3,3-Hexafluorpropan-2-ol;
(3R)-3-Amino-7-(5-tert-butyl-1,3,4-oxadiazol-2-yl)-5-[[5-[(4-chlorphenyl)methyl]-3-pyridyl]methyl]-8-fluor-1,1-dioxo-2,3-dihydro-1lambda6,5-benzothiazepin-4-on;
(3R)-3-Amino-5-[(4-chlorphenyl)methyl]-8-fluor-7-[5-(4-methyl-4-piperidyl)-1,3,4-oxadiazol-2-yl]-1,1-dioxo-2,3-dihydro-1lambda6,5-benzothiazepin-4-on;
(3R)-3-Amino-5-[(4-chlorphenyl)methyl]-8-fluor-7-[5-(4-methyltetrahydropyran-4-yl)-1,3,4-oxadiazol-2-yl]-1,1-dioxo-2,3-dihydro-1lambda6,5-benzothiazepin-4-on;2,2,2-Trifluoressigsäure;
(3R)-3-Amino-7-(5-tert-butyl-1,3,4-oxadiazol-2-yl)-8-fluor-l-oxo-5-[[4-(trifluormethoxy)phenyl]methyl]-2,3-dihydro-1lambda4,5-benzothiazepin-4-on;
(3R)-3-Amino-7-(5-tert-butyl-1,3,4-oxadiazol-2-yl)-8-fluor-1-imino-1-oxo-5-[[4-(trifluormethoxy)phenyl]methyl]-2,3-dihydro-1lambda6,5-benzothiazepin-4-on;
(3R)-3-Amino-7-(5-tert-butyl-1,3,4-oxadiazol-2-yl)-8-chlor-5-[(4-chlorphenyl)methyl]-1,1-dioxo-2,3-dihydro-1lambda6,5-benzothiazepin-4-on;
2-[4-[[(3R)-3-Amino-7-(5-tert-butyl-1,3,4-oxadiazol-2-yl)-8-fluor-1,1,4-trioxo-2,3-dihydro-1lambda6,5-benzothiazepin-5-yl]methyl]phenoxy]acetamid;1,1,1,3,3,3-Hexafluorpropan-2-ol;
N-[11-[4-[[(3R)-3-Amino-7-(5-tert-butyl-1,3,4-oxadiazol-2-yl)-8-fluor-1,1,4-trioxo-2,3-dihydro-1lambda6,5-benzothiazepin-5-yl]methyl]phenoxy]undecyl]acetamid;
(2S)-N-[(3R)-7-(5-tert-Butyl-1,3,4-oxadiazol-2-yl)-5-[(4-chlorphenyl)methyl]-8-fluor-1,1,4-trioxo-2,3-dihydro-1lambda6,5-benzothiazepin-3-yl]-2-(methylamino)propanamid;
(2S)-2-Amino-N-[(3R)-7-(5-tert-butyl-1,3,4-oxadiazol-2-yl)-5-[(4-chlorphenyl)methyl]-8-fluor-1,1,4-trioxo-2,3-dihydro-1lambda6,5-benzothiazepin-3-yl]butanamid;
(2S)-N-[(3R)-7-(5-tert-Butyl-1,3,4-oxadiazol-2-yl)-5-[(4-chlorphenyl)methyl]-8-fluor-1,1,4-trioxo-2,3-dihydro-1lambda6,5-benzothiazepin-3-yl]-3-hydroxy-2-(methylamino)propanamid;
(3R)-3-Amino-7-(5-tert-butyl-1,3,4-oxadiazol-2-yl)-5-[[4-(cyclopentoxy)phenyl]methyl]-8-fluor-1,1-dioxo-2,3-dihydro-1lambda6,5-benzothiazepin-4-on;
(3R)-3-Amino-7-(5-tert-butyl-1,3,4-oxadiazol-2-yl)-8-fluor-5-[(4-isopropoxyphenyl)methyl]-1,1-dioxo-2,3-dihydro-1lambda6,5-benzothiazepin-4-on;
(3R)-3-Amino-5-[(4-chlorphenyl)methyl]-1,1-dioxo-7-[5-[2,2,2-trideuterio-1,1-bis(trideuteriomethyl)ethyl]-1,3,4-oxadiazol-2-yl]-2,3-dihydro-1lambda6,5-benzothiazepin-4-on;
(3R)-3-Amino-5-[(4-chlorphenyl)methyl]-7-[5-(3,3-difluorcyclohexyl)-1,3,4-oxadiazol-2-yl]-8-fluor-1,1-dioxo-2,3-dihydro-1lambda6,5-benzothiazepin-4-on;
(3R)-3-Amino-5-[(4-chlorphenyl)methyl]-7-(4-ethyltriazol-1-yl)-8-fluor-2,3-dihydro-1,5-benzothiazepin-4-on;
(3R)-3-Amino-5-[(4-chlorphenyl)methyl]-7-[4-[(4-chlorphenyl)methyl]triazol-1-yl]-8-fluor-2,3-dihydro-1,5-benzothiazepin-4-on;
(3R)-3-Amino-5-[(4-chlorphenyl)methyl]-7-(4-ethyltriazol-1-yl)-8-fluor-1,1-dioxo-2,3-dihydro-1λ⁶,5-benzothiazepin-4-on;
(3R)-3-Amino-5-[(4-chlorphenyl)methyl]-7-[4-[(4-chlorphenyl)methyl]triazol-1-yl]-8-fluor-1,1-dioxo-2,3-dihydro-1λ⁶,5-benzothiazepin-4-on;
(3R)-3-Amino-7-(4-tert-butyltriazol-1-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-(1-ethyltriazol-4-yl)-8-fluor-1,1-dioxo-2,3-dihydro-1λ⁶,5-benzothiazepin-4-on;
(3R)-3-Amino-5-[(4-chlorphenyl)methyl]-7-(2-ethyltetrazol-5-yl)-8-fluor-1,1-dioxo-2,3-dihydro-1λ⁶,5-benzothiazepin-4-on;
(3R)-3-Amino-7-(2-tert-butyltetrazol-5-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-8-fluor-1,1-dioxo-7-(1H-pyrazol-5-yl)-2,3-dihydro-1λ⁶,5-benzothiazepin-4-on;
(3R)-3-Amino-5-[(4-chlorphenyl)methyl]-7-(5-ethyl-1H-1,2,4-triazol-3-yl)-8-fluor-1,1-dioxo-2,3-dihydro-1λ⁶,5-benzothiazepin-4-on;
(3R)-3-Amino-5-[(4-chlorphenyl)methyl]-1,1-dioxo-7-[3-(2,2,2-trifluorethyl)-1,2,4-oxadiazol-5-yl]-2,3-dihydropyrido[3,2-b][1,4]thiazepin-4-on;
(3R)-3-Amino-5-[(4-chlorphenyl)methyl]-7-(3-cyclopropyl-1,2,4-oxadiazol-5-yl)-8-fluor-1,1-dioxo-2,3-dihydro-1λ⁶,5-benzothiazepin-4-on;
(3R)-3-Amino-5-[(4-chlorphenyl)methyl]-7-(3-ethyl-1,2,4-oxadiazol-5-yl)-8-fluor-1,1-dioxo-2,3-dihydro-1λ⁶,5-benzothiazepin-4-on;
(3R)-3-Amino-5-[(4-chlorphenyl)methyl]-8-fluor-1,1-dioxo-7-(3-propyl-1,2,4-oxadiazol-5-yl)-2,3-dihydro-1λ⁶,5-benzothiazepin-4-on;
(3R)-3-Amino-7-(3-tert-butyl-1,2,4-oxadiazol-5-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-[3-[(4-chlorphenyl)methyl]-1,2,4-oxadiazol-5-yl]-8-fluor-1,1-dioxo-2,3-dihydro-1λ⁶,5-benzothiazepin-4-on;
(3R)-3-Amino-7-[3-(benzyloxymethyl)-1,2,4-oxadiazol-5-yl]-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-8-fluor-1,1-dioxo-7-[3-(2,2,2-trifluorethyl)-1,2,4-oxadiazol-5-yl]-2,3-dihydro-1λ⁶,5-benzothiazepin-4-on;
(3R)-3-Amino-7-[3-(aminomethyl)-1,2,4-oxadiazol-5-yl]-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-(3-ethyl-1,2,4-oxadiazol-5-yl)-1,1-dioxo-2,3-dihydro-1λ⁶,5-benzothiazepin-4-on;
(3R)-3-Amino-7-(3-tert-butylisoxazol-5-yl)-5-[(4-chlorphenyl)methyl]-8-fluor-1,1-dioxo-2,3-dihydro-1λ⁶,5-benzothiazepin-4-on;
(3R)-3-Amino-7-[5-(2,2-difluorcyclohexyl)-1,3,4-oxadiazol-2-yl]-8-fluor-1,1-dioxo-5-[[4-(trifluormethoxy)phenyl]methyl]-2,3-dihydro-1λ⁶,5-benzothiazepin-4-on;
(3R)-3-Amino-7-(5-tert-butyl-1,3,4-oxadiazol-2-yl)-8-fluor-5-[[4-(trifluormethoxy)phenyl]methyl]-2,3-dihydro-1,5-benzothiazepin-4-on;
(3R)-3-Amino-7-[5-(3,3-difluorcyclohexyl)-1,3,4-oxadiazol-2-yl]-8-fluor-1,1-dioxo-5-[[4-(trifluormethoxy)phenyl]methyl]-2,3-dihydro-1λ⁶,5-benzothiazepin-4-on;
(3R)-3-Amino-8-fluor-1,1-dioxo-7-[5-(2,2,2-trifluorethyl)-1,3,4-oxadiazol-2-yl]-5-[[4-(trifluormethoxy)phenyl]methyl]-2,3-dihydro-1λ⁶,5-benzothiazepin-4-on;
(3R)-3-Amino-7-(5-tert-butyl-1,3,4-oxadiazol-2-yl)-8-fluor-1,1-dioxo-5-[[4-(trifluormethoxy)phenyl]methyl]-2,3-dihydro-1λ⁶,5-benzothiazepin-4-on;
(2S)-N-[(3R)-8-Fluor-1,1,4-trioxo-7-[5-(2,2,2-trifluorethyl)-1,3,4-oxadiazol-2-yl]-5-[[4-(trifluormethoxy)phenyl]methyl]-2,3-dihydro-1λ⁶,5-benzothiazepin-3-yl]-2-(methylamino)propanamid;
(3R)-3-Amino-7-(5-tert-butyl-1,3,4-oxadiazol-2-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-8-fluor-1,1-dioxo-7-[5-(3,3,3-trifluorpropyl)-1,3,4-oxadiazol-2-yl]-2,3-dihydro-1λ⁶,5-benzothiazepin-4-on;
(3R)-3-Amino-5-[(4-chlorphenyl)methyl]-8-fluor-1,1-dioxo-7-[5-(2,2,2-trifluorethyl)-1,3,4-oxadiazol-2-yl]-2,3-dihydro-1λ⁶,5-benzothiazepin-4-on;
(3R)-3-Amino-7-[5-(1-amino-1-methylethyl)-1,3,4-oxadiazol-2-yl]-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-8-fluor-7-[5-(1-fluor-1-methylethyl)-1,3,4-oxadiazol-2-yl]-1,1-dioxo-2,3-dihydro-1λ⁶,5-benzothiazepin-4-on;
(3R)-3-Amino-5-[(4-chlorphenyl)methyl]-7-[5-(2,2-difluorethyl)-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-7-(5-ethyl-1,3,4-oxadiazol-2-yl)-8-fluor-1,1-dioxo-2,3-dihydro-1λ⁶,5-benzothiazepin-4-on;
(3R)-3-Amino-5-[(4-chlorphenyl)methyl]-7-[5-(4,4-difluorcyclohexyl)-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-7-[5-(2,2-difluorcyclohexyl)-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-7-[5-(3,3-difluorcyclopentyl)-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-7-[5-(2,2-difluorcyclopentyl)-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-7-[5-[(3,3-difluorcyclobutyl)methyl]-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-8-fluor-1,1-dioxo-7-[5-(tetrahydropyran-4-ylmethyl)-1,3,4-oxadiazol-2-yl]-2,3-dihydro-1λ⁶,5-benzothiazepin-4-on;
(3R)-3-Amino-5-[(4-chlorphenyl)methyl]-8-fluor-1,1-dioxo-7-[5-(4,4,4-trifluorbutyl)-1,3,4-oxadiazol-2-yl]-2,3-dihydro-1λ⁶,5-benzothiazepin-4-on;
(3R)-3-Amino-5-[(4-chlorphenyl)methyl]-7-[5-(3,3-difluorcyclobutyl)-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-7-[5-(2,2-difluorcyclobutyl)-1,3,4-oxadiazol-2-yl]-8-fluor-1,1-dioxo-2,3-dihydro-1λ⁶,5-benzothiazepin-4-on;
(3R)-3-Amino-5-[(4-chlorphenyl)methyl]-8-fluor-1,1-dioxo-7-(5-tetrahydropyran-4-yl-1,3,4-oxadiazol-2-yl)-2,3-dihydro-1λ⁶,5-benzothiazepin-4-on;
(3R)-3-Amino-5-[(4-chlorphenyl)methyl]-8-fluor-7-[5-(2-fluorethyl)-1,3,4-oxadiazol-2-yl]-1,1-dioxo-2,3-dihydro-1λ⁶,5-benzothiazepin-4-on;
(3R)-3-Amino-5-[(4-chlorphenyl)methyl]-8-fluor-7-(5-isopropyl-1,3,4-oxadiazol-2-yl)-1,1-dioxo-2,3-dihydro-1λ⁶,5-benzothiazepin-4-on;
(3R)-3-Amino-5-[(4-chlorphenyl)methyl]-7-(5-cyclopropyl-1,3,4-oxadiazol-2-yl)-8-fluor-1,1-dioxo-2,3-dihydro-1λ⁶,5-benzothiazepin-4-on;
(3R)-3-Amino-5-[(4-chlorphenyl)methyl]-8-fluor-7-[5-(2-hydroxy-1,1-dimethylethyl)-1,3,4-oxadiazol-2-yl]-1,1-dioxo-2,3-dihydro-1λ⁶,5-benzothiazepin-4-on;
(3R)-3-Amino-7-[5-(azetidin-1-yl)-1,3,4-oxadiazol-2-yl]-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-(4-ethyloxazol-2-yl)-8-fluor-1,1-dioxo-2,3-dihydro-1λ⁶,5-benzothiazepin-4-on;
(3R)-3-Amino-5-[(4-chlorphenyl)methyl]-6-fluor-1,1-dioxo-7-[5-(2,2,2-trifluorethyl)-1,3,4-oxadiazol-2-yl]-2,3-dihydro-1λ⁶,5-benzothiazepin-4-on;
(3R)-3-Amino-7-(5-tert-butyl-1,3,4-oxadiazol-2-yl)-5-[(3-chlorphenyl)methyl]-8-fluor-1,1-dioxo-2,3-dihydro-1λ⁶,5-benzothiazepin-4-on;
4-[[(3R)-3-Amino-7-(5-tert-butyl-1,3,4-oxadiazol-2-yl)-8-fluor-1,1,4-trioxo-2,3-dihydro-1λ⁶,5-benzothiazepin-5-yl]methyl]benzonitril;
(3R)-3-Amino-7-(5-tert-butyl-1,3,4-oxadiazol-2-yl)-8-fluor-1,1-dioxo-5-[(4-tetrahydrofuran-3-yloxyphenyl)methyl]-2,3-dihydro-1λ⁶,5-benzothiazepin-4-on;
(3R)-3-Amino-7-(5-tert-butyl-1,3,4-oxadiazol-2-yl)-5-[[4-(2,2-difluorethoxy)phenyl]methyl]-8-fluor-1,1-dioxo-2,3-dihydro-1λ⁶,5-benzothiazepin-4-on;
(3R)-3-Amino-7-(5-tert-butyl-1,3,4-oxadiazol-2-yl)-5-[[4-(difluormethoxy)phenyl]methyl]-8-fluor-1,1-dioxo-2,3-dihydro-1λ⁶,5-benzothiazepin-4-on;
(3R)-3-Amino-7-(5-tert-butyl-1,3,4-oxadiazol-2-yl)-8-fluor-1,1-dioxo-5-(2-pyridylmethyl)-2,3-dihydro-1λ⁶,5-benzothiazepin-4-on;
(3R)-3-Amino-7-(5-tert-butyl-1,3,4-oxadiazol-2-yl)-8-fluor-5-[(6-methoxy-3-pyridyl)methyl]-1,1-dioxo-2,3-dihydro-1λ⁶,5-benzothiazepin-4-on;
(3R)-3-Amino-5-[[4-(aminomethyl)phenyl]methyl]-7-(5-tert-butyl-1,3,4-oxadiazol-2-yl)-8-fluor-1,1-dioxo-2,3-dihydro-1λ⁶,5-benzothiazepin-4-on;
(3R)-3-Amino-7-(5-tert-butyl-1,3,4-oxadiazol-2-yl)-5-[(2-chlorphenyl)methyl]-8-fluor-1,1-dioxo-2,3-dihydro-1λ⁶,5-benzothiazepin-4-on;
(3R)-3-Amino-7-(5-tert-butyl-1,3,4-oxadiazol-2-yl)-8-fluor-5-[(5-methoxy-2-pyridyl)methyl]-1,1-dioxo-2,3-dihydro-1λ⁶,5-benzothiazepin-4-on;
(3R)-3-Amino-7-(5-tert-butyl-1,3,4-oxadiazol-2-yl)-8-fluor-5-[(4-methylsulfonylphenyl)methyl]-1,1-dioxo-2,3-dihydro-1λ⁶,5-benzothiazepin-4-on;
(3R)-3-Amino-7-(5-tert-butyl-1,3,4-oxadiazol-2-yl)-8-fluor-5-[(4-methoxyphenyl)methyl]-1,1-dioxo-2,3-dihydro-1λ⁶,5-benzothiazepin-4-on;
(3R)-3-Amino-7-(5-tert-butyl-1,3,4-oxadiazol-2-yl)-8-fluor-5-[(4-fluorphenyl)methyl]-1,1-dioxo-2,3-dihydro-1λ⁶,5-benzothiazepin-4-on;
(3R)-3-Amino-5-[[2-(aminomethyl)-4-chlorphenyl]methyl]-7-(5-tert-butyl-1,3,4-oxadiazol-2-yl)-8-fluor-1,1-dioxo-2,3-dihydro-1λ⁶,5-benzothiazepin-4-on;
(3R)-3-Amino-5-[[4-(11-aminoundecoxy)phenyl]methyl]-7-(5-tert-butyl-1,3,4-oxadiazol-2-yl)-8-fluor-1,1-dioxo-2,3-dihydro-1λ⁶,5-benzothiazepin-4-on;
N-[(3R)-7-(5-tert-Butyl-1,3,4-oxadiazol-2-yl)-5-[(4-chlorphenyl)methyl]-8-fluor-1,1,4-trioxo-2,3-dihydro-1λ⁶,5,5-benzothiazepin-3-yl]acetamid;
N-[(3R)-7-(5-tert-Butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]propanamid;
(2S)-N-[(3R)-7-(5-tert-Butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]-2-hydroxybutanamid;
N-[(3R)-7-(5-tert-Butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]butanamid;
(2S)-2-Amino-N-[(3R)-7-(5-tert-butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]propanamid;
(2S)-N-[(3R)-7-(5-tert-Butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]-2-(methylamino)butanamid;
3-Amino-N-[(3R)-7-(5-tert-butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]propanamid;
N-[(3R)-7-(5-tert-Butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]-2-(methylamino)acetamid;
2-Amino-N-[(3R)-7-(5-tert-butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]acetamid;
4-Amino-N-[(3R)-7-(5-tert-butyl-1,3,4-oxadiazol-2-yl)-5-[(4-chlorphenyl)methyl]-8-fluor-1,1,4-trioxo-2,3-dihydro-1λ⁶,5-benzothiazepin-3-yl]butanamid;
(3R)-3-Amino-8-brom-7-(5-tert-butyl-1,3,4-oxadiazol-2-yl)-5-[(4-chlorphenyl)methyl]-1,1-dioxo-2,3-dihydro-1λ⁶,5-benzothiazepin-4-on;
(3R)-3-Amino-7-(5-tert-butyl-1,3,4-oxadiazol-2-yl)-5-[(4-chlorphenyl)methyl]-8-methyl-1,1-dioxo-2,3-dihydro-1λ⁶,5-benzothiazepin-4-on;
(3R)-3-Amino-7-(5-tert-butyl-1,3,4-oxadiazol-2-yl)-5-[(4-chlorphenyl)methyl]-8-hydroxy-1,1-dioxo-2,3-dihydro-1λ⁶,5-benzothiazepin-4-on;
(3R)-3-Amino-7-(5-tert-butyl-1,3,4-oxadiazol-2-yl)-5-[(4-chlorphenyl)methyl]-8-(dimethylamino)-1,1-dioxo-2,3-dihydro-1λ⁶,5-benzothiazepin-4-on;
(3R)-3,8-Diamino-7-(5-tert-butyl-1,3,4-oxadiazol-2-yl)-5-[(4-chlorphenyl)methyl]-1,1-dioxo-2,3-dihydro-1λ⁶,5-benzothiazepin-4-on;
(3R)-3-Amino-7-(1-tert-butylpyrazol-4-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-(1-ethylpyrazol-4-yl)-8-fluor-1,1-dioxo-2,3-dihydro-1λ⁶,5-benzothiazepin-4-on;
(3R)-3-Amino-5-[(4-chlorphenyl)methyl]-7-(1-ethylpyrazol-3-yl)-8-fluor-1,1-dioxo-2,3-dihydro-1λ⁶,5-benzothiazepin-4-on;
(3R)-3-Amino-7-(4-tert-butylpyrazol-1-yl)-5-[(4-chlorphenyl)methyl]-8-fluor-1,1-dioxo-2,3-dihydro-1λ⁶,5-benzothiazepin-4-on;
(3R)-3-Amino-7-(3-tert-butylpyrazol-1-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-(4-ethylpyrazol-1-yl)-8-fluor-1,1-dioxo-2,3-dihydro-1λ⁶,5-benzothiazepin-4-on;
(3R)-3-Amino-5-[(4-chlorphenyl)methyl]-7-(1-ethyl-1,2,4-triazol-3-yl)-8-fluor-1,1-dioxo-2,3-dihydro-1λ⁶,5-benzothiazepin-4-on;
(3R)-3-Amino-7-(1-tert-butyl-1,2,4-triazol-3-yl)-5-[(4-chlorphenyl)methyl]-8-fluor-1,1-dioxo-2,3-dihydro-1λ⁶,5-benzothiazepin-4-on;
(3R)-3-Amino-7-(1-tert-butylpyrazol-3-yl)-5-[(4-chlorphenyl)methyl]-8-fluor-1,1-dioxo-2,3-dihydro-1λ⁶,5-benzothiazepin-4-on;
(3R)-3-Amino-7-(5-tert-butyl-1,2,4-oxadiazol-3-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-(5-ethyl-1,2,4-oxadiazol-3-yl)-8-fluor-1,1-dioxo-2,3-dihydro-1λ⁶,5-benzothiazepin-4-on;
(3R)-3-Amino-7-[5-(1,1-dimethylbut-3-inyl)-1,3,4-oxadiazol-2-yl]-8-fluor-1,1-dioxo-5-[[4-[3-(trifluormethyl)diazirin-3-yl]phenyl]methyl]-2,3-dihydro-1λ⁶,5-benzothiazepin-4-on;
(3R)-3-Amino-7-(5-tert-butyl-1,3,4-oxadiazol-2-yl)-1,1-dioxo-5-[[4-(trifluormethoxy)phenyl]methyl]-2,3-dihydro-1λ⁶,5-benzothiazepin-4-on;
(3R)-3-Amino-7-(5-tert-butyl-1,3,4-oxadiazol-2-yl)-5-[(4-chlorphenyl)methyl]-1,1-dioxo-2,3-dihydro-1λ⁶,5-benzothiazepin-4-on;
(3R)-3-Amino-7-[5-(1,1-dimethylbut-3-inyl)-1,3,4-oxadiazol-2-yl]-8-fluor-1,1-dioxo-5-[[4-(trifluormethoxy)phenyl]methyl]-2,3-dihydro-1λ⁶,5-benzothiazepin-4-on;
(3R)-3-Amino-5-[(4-chlorphenyl)methyl]-7-[5-(5,5-difluor-1-methyl-3-piperidyl)-1,3,4-oxadiazol-2-yl]-8-fluor-1,1-dioxo-2,3-dihydro-1λ⁶,5-benzothiazepin-4-on;
(3R)-3-Amino-7-(5-tert-butyl-1,3,4-oxadiazol-2-yl)-8-fluor-1-oxo-5-[[4-(trifluormethoxy)phenyl]methyl]-2,3-dihydro-1λ⁴,5-benzothiazepin-4-on;
(3R)-3-Amino-7-(5-tert-butyl-1,3,4-oxadiazol-2-yl)-8-fluor-1-oxo-5-[[4-(trifluormethoxy)phenyl]methyl]-2,3-dihydro-1λ⁴,5-benzothiazepin-4-on;
(3R)-3-Amino-5-[(4-chlorphenyl)methyl]-8-fluor-7-(5-morpholino-1,3,4-oxadiazol-2-yl)-1,1-dioxo-2,3-dihydro-1λ⁶,5-benzothiazepin-4-on;
(3R)-3-Amino-5-[(4-chlorphenyl)methyl]-7-[5-(cyclopentylamino)-1,3,4-oxadiazol-2-yl]-8-fluor-1,1-dioxo-2,3-dihydro-1λ⁶,5-benzothiazepin-4-on;
(3R)-3-Amino-7-(5-tert-butyl-1,3,4-thiadiazol-2-yl)-5-[(4-chlorphenyl)methyl]-8-fluor-1,1-dioxo-2,3-dihydro-1λ⁶,5-benzothiazepin-4-on;
(3R)-3-Amino-5-[(4-chlorphenyl)methyl]-7-(5-ethyltetrazol-2-yl)-8-fluor-1,1-dioxo-2,3-dihydro-1λ⁶,5-benzothiazepin-4-on;
oder ein pharmazeutisch verträgliches Salz davon.

19. Verfahren zur Herstellung einer Verbindung nach einem der Ansprüche 1 bis 18 oder eines pharmazeutisch verträglichen Salzes davon, umfassend
Umsetzen einer Verbindung der Formel (IX) 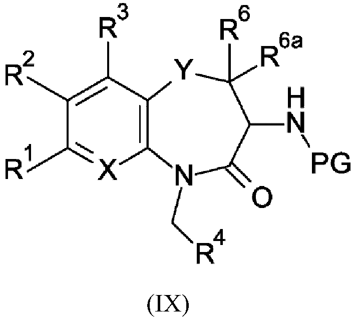
wobei X, Y, R¹, R², R³, R⁴, R⁶, R^{6a} wie in einem der Ansprüche 1 bis 17 definiert sind und PG eine Aminoschutzgruppe ist,
mit einem geeigneten Entschützungsmittel, um die Verbindung der Formel (I) oder ein pharmazeutisch verträgliches Salz davon zu bilden, wobei X, Y, R¹, R², R³, R⁴, R⁶, R^{6a} wie in einem der Ansprüche 1 bis 17 definiert sind und R⁵ Wasserstoff ist; oder
Umsetzen einer Verbindung der Formel (I') 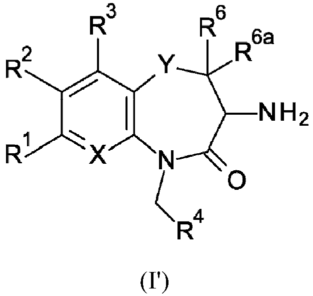
wobei X, Y, R¹, R², R³, R⁴, R⁶, R^{6a} wie in einem der Ansprüche 1 bis 34 definiert sind und R⁵ Wasserstoff ist,
mit einem Carbonsäurederivat der Formel R⁹CO₂H, wobei R⁹ wie in einem der Ansprüche 1 bis 15 definiert ist, in Gegenwart einer Base, um eine Verbindung der Formel (I) oder ein pharmazeutisch verträgliches Salz davon zu bilden, wobei R⁵ -C(O)(R⁹) ist.

20. Verbindung nach einem der Ansprüche 1 bis 18 oder ein pharmazeutisch verträgliches Salz davon zur Verwendung als therapeutisch wirksame Substanz.

21. Pharmazeutische Zusammensetzung, umfassend eine Verbindung nach einem der Ansprüche 1 bis 18 oder ein pharmazeutisch verträgliches Salz davon und einen pharmazeutisch verträglichen Hilfsstoff.

22. Verbindung nach einem der Ansprüche 1 bis 18 oder ein pharmazeutisch verträgliches Salz davon zur Verwendung bei der Behandlung, Prävention und/oder Verzögerung des Fortschreitens von Krebs.

23. Verbindung zur Verwendung nach Anspruch 22, wobei der Krebs mit anormaler Diacylglycerolkinase-Signalübertragung assoziiert ist, wobei die Diacylglycerolkinase aus DGKα und/oder DGKζ ausgewählt ist.

24. Verbindung zur Verwendung nach Anspruch 22 oder 23, wobei der Krebs ausgewählt ist aus der Gruppe bestehend aus akuter lymphatischer B-Zell-Leukämie, akuter lymphatischer T-Zell-Leukämie, akuter lymphatischer Leukämie, chronischer myeloischer Leukämie, chronischer lymphatischer Leukämie, prolymphatischer B-Zell-Leukämie, blastischem plasmazytoidem dendritischem Zellneoplasma, Burkitt-Lymphom, diffusem großzelligem B-Zell-Lymphom, follikulärem Lymphom, Haarzell-Leukämie, kleinzelligem oder großzelligem follikulärem Lymphom, malignen lymphoproliferativen Zuständen, MALT-Lymphom, Mantelzelllymphom, Marginalzonenlymphom, multiplem Myelom, Myelodysplasie und myelodysplastischem Syndrom, Non-Hodgkin-Lymphom, plasmablastischem Lymphom, plasmazytoidem dendritischem Zellneoplasma, Waldenström-Makroglobulinämie, Präleukämie, Sarkom, Karzinom, Melanom, Neuroblastom, Nierenzellkarzinom, Kolonkrebs, Kolorektalkrebs, Brustkrebs, epithelialem Plattenepithelkrebs, Melanom, Magenkrebs, Gehirnkrebs, Lungenkrebs (z. B. NSCLC), Bauchspeicheldrüsenkrebs, Gebärmutterhalskrebs, Eierstockkrebs, Leberkrebs, Blasenkrebs, Prostatakrebs, Hodenkrebs, Schilddrüsenkrebs, Gebärmutterkrebs, Nebennierenkrebs und Kopf- und Halskrebs.

## Revendications

1. Composé de formule (I) 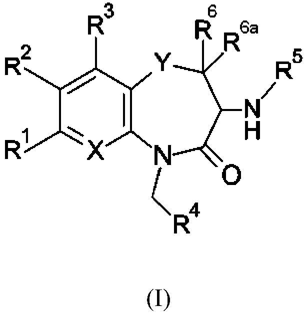 ou un sel pharmaceutiquement acceptable de celui-ci, dans lequel :
X représente C(R⁷) ou N ;
Y représente S, S(O), S(O)₂, S(O)N(R^{y}),
R¹ représente un hétéroaryle à 5 chaînons, dans lequel R¹ est éventuellement substitué par un ou plusieurs R¹⁰ qui peuvent être identiques ou différents ;
R², R³ et R⁷ sont chacun indépendamment choisis parmi un hydrogène, un halogène, un alkyle en C₁₋₆, un alcynyle en C₂₋₆, un hydroxy, un cyano, un halogénoalkyle en C₁₋₆, N(R⁸R^{8a}), un alcoxy en C₁₋₆, un cycloalkyle en C₃₋₇ et un hétérocyclyle à 3 à 10 chaînons ;
R⁴ est choisi parmi un aryle en C₅₋₁₄ et un hétéroaryle à 5 à 14 chaînons, dans lequel R⁴ est éventuellement substitué par un ou plusieurs R¹¹ qui peuvent être identiques ou différents ;
R⁵ est choisi parmi un hydrogène, un alkyle en C₁₋₆, -C(O)(R⁹), un amino, un aminoalkyle en C₁₋₆ et un cycloalkyle en C₃₋₇,
dans lequel ledit cycloalkyle en C₃₋₇ est éventuellement substitué par un ou plusieurs alkyle en C₁₋₆, amino, aminoalkyle en C₁₋₆ ;
R⁶ et R^{6a} sont chacun indépendamment choisis parmi un hydrogène et un alkyle en C₁₋₆ ;
R⁸ et R^{8a} sont chacun indépendamment choisis parmi un hydrogène et un alkyle en C₁₋₆ ;
R⁹ est choisi parmi un alkyle en C₁₋₆, un hydroxyalkyle en C₁₋₆, un aminoalkyle en C₁₋₆ et un -(alkyle en C₁₋₆)-N(R^{9a}R^{9b}), dans lequel ledit aminoalkyle en C₁₋₆ est éventuellement substitué par un ou plusieurs hydroxy, hydroxyalkyle en C₁₋₆ ;
R^{9a} et R^{9b} sont chacun indépendamment choisis parmi un hydrogène et un alkyle en C₁₋₆, dans lequel ledit alkyle en C₁₋₆ est éventuellement substitué par un ou plusieurs hydroxy ;
ou R^{9a} et R^{9b}, pris conjointement avec l'atome d'azote auquel ils sont liés, forment un hétérocyclyle à 3 à 10 chaînons ;
R¹⁰ est choisi parmi :
i) un alkyle en C₁₋₁₀, éventuellement substitué par un ou plusieurs halogènes, alcynyle en C₂₋₆, halogénoalkyle en C₁₋₆, amino, hydroxy, hétéroaryle à 5 à 6 chaînons, halogénoalcoxy en C₁₋₆, alcoxy en C₁₋₆, cycloalkyle à 3 à 10 chaînons, alkyle en C₁₋₃, -N(R^{10a}R^{10b}), -S(O)₂(alkyle en C₁₋₆), - S(O)₂(cycloalkyle en C₁₋₆), hétérocyclyle à 3 à 10 chaînons, phényle, cyano, -C(O)N(R^{10c}R^{10d}), dans lequel l'hétérocyclyle à 3 à 10 chaînons, le cycloalkyle à 3 à 10 chaînons et le phényle sont éventuellement substitués par un ou plusieurs alkyle en C₁₋₁₀, alcoxy en C₁₋₁₀, -S(O)₂(alkyle en C₁₋₆), oxo, halogènes, alcynyle en C₂₋₆, cycloalkyle à 3 à 10 chaînons ;
ii) un cycloalkyle en C₃₋₁₀, éventuellement substitué par un ou plusieurs halogènes, alkyle en C₁₋₁₀, -S(O)₂(alkyle en C₁₋₆), cyano, halogénoalkyle en C₁₋₆, alcoxy en C₁₋₆, hydroxy, oxo, amino, -C(O)N(R^{10c}R^{10d}), =N(OH), hydroxyalkyle en C₁₋₆ ;
iii) un hétérocyclyle à 3 à 10 chaînons, éventuellement substitué par un ou plusieurs halogènes, alkyle en C₁₋₁₀, -S(O)₂(alkyle en C₁₋₆), -alkyle en C₁₋₁₀-alcoxy en C₁₋₄, amino, -C(O)N(R^{10h}R¹⁰ⁱ), halogénoalkyle en C₁₋₆, alcoxy en C₁₋₆, cyano, hydroxyalkyle en C₁₋₁₀, oxo, -C(O)(alkyle en C₁₋₆), -C(O)O-(R^{10q}), cycloalkyle en C₃₋₁₀ ;
iv) un phényle, éventuellement substitué par un ou plusieurs halogènes, alkyle en C₁₋₁₀, -S(O)₂(alkyle en C₁₋₆) ;
v) -N(R^{10e}R^{10f}) ;
vi) -OR^{10g} ;
vii) -C(O)NR^{10h}R¹⁰ⁱ ;
viii) un hétéroaryle, éventuellement substitué par un ou plusieurs alkyle en C₁₋₁₀, halogènes, -SO₂(alkyle en C₁₋₆) ; et
ix) un oxo ;
R^{10a} et R^{10b} sont chacun indépendamment choisis parmi un hydrogène, un alkyle en C₁₋₆, un halogénoalkyle en C₁₋₆, un hydroxyalkyle en C₁₋₆, un alcoxy en C₁₋₆, -C(O)(R^{10j}), un aminoalkyle en C₁₋₆, un hétérocyclyle à 3 à 10 chaînons, -SO₂(R^{10k}), un -alkyle en C₁₋₆-SO₂(R^{10k}) et -N(R^{10l}R^{10m}), dans lequel l'hétérocyclyle à 3 à 10 chainons est éventuellement substitué par un alkyle en C₁₋₆ ;
ou R^{10a} et R^{10b}, pris conjointement avec l'atome d'azote auquel ils sont liés, forment un hétérocyclyle à 3 à 10 chaînons qui est éventuellement substitué par un ou plusieurs halogènes, alkyle en C₁₋₆ ;
R^{10c} et R^{10d} sont chacun indépendamment choisis parmi un hydrogène et un alkyle en C₁₋₆ ;
R^{10e} et R^{10f} sont chacun indépendamment choisis parmi :
i) un hydrogène ;
ii) un alkyle en C₁₋₆, éventuellement substitué par un ou plusieurs cyano, en particulier un cyano, halogène, hydroxy ;
iii) -C(O)R¹⁰ⁿ ;
iv) un -alkyle en C₁₋₁₀((O-alkyle en C₁₋₁₀)ₘ), dans lequel m représente un entier entre 1 et 5 (plus particulièrement m représente 2 ou 3), éventuellement substitué par un ou plusieurs alcynyle en C₂₋₆ ;
v) un cycloalkyle en C₃₋₁₀, éventuellement substitué par un ou plusieurs halogènes, hydroxy, oxo, alkyle en C₁₋₁₀, halogénoalkyle en C₁₋₁₀, hydroxyalkyle en C₁₋₆, alcoxy en C₁₋₆, -C(O)O(R^{10o}), -C(O)(R^{10o}), - C(O)N(R^{10p})(R^{10p'}), -SO₂(alkyle en C₁₋₆), alcynyle en C₂₋₆ ; et
vi) un hétérocyclyle à 3 à 10 chaînons, éventuellement substitué par un ou plusieurs halogènes, hydroxy, oxo, alkyle en C₁₋₁₀, halogénoalkyle en C₁₋₁₀, hydroxyalkyle en C₁₋₆, alcoxy en C₁₋₆, -C(O)O(R^{10o}), -C(O)(R^{10o}), - C(O)N(R^{10p})(R^{10p'}), -SO₂(alkyle en C₁₋₆), cycloalkyle à 3 à 10 chaînons ;
R^{10g} est choisi parmi un halogénoalkyle en C₁₋₆, un cyano, un -alkyle en C₁₋₁₀-phényle, un -alkyle en C₁₋₆-cycloalkyle en C₃₋₇ et un -alcoxy en C₁₋₆-halogénoalkyle en C₁₋₆ ;
R^{10h} et R¹⁰ⁱ sont chacun indépendamment choisis parmi un hydrogène et un alkyle en C₁₋₆, un halogénoalkyle en C₁₋₆, dans lequel l'alkyle en C₁₋₆ et l'halogénoalkyle en C₁₋₆ sont éventuellement substitués par un ou plusieurs hydroxy,
ou R^{10h} et R¹⁰ⁱ, pris conjointement avec l'atome d'azote auquel ils sont liés, forment un hétérocyclyle à 3 à 10 chaînons qui est éventuellement substitué par un ou plusieurs halogènes, hydroxy, alkyle en C₁₋₆ ;
R^{10j} est choisi parmi un alkyle en C₁₋₆, un halogénoalkyle en C₁₋₆, un hydroxyalkyle en C₁₋₆ et un aminoalkyle en C₁₋₆ ;
R^{10k} est choisi parmi un hydrogène, un alkyle en C₁₋₆ et un halogénoalkyle en C₁₋₆ ;
R^{10l} et R^{10m} sont chacun indépendamment choisis parmi un hydrogène et un alkyle en C₁₋₆ ;
R¹⁰ⁿ est choisi parmi un alkyle en C₁₋₆, un aminoalkyle en C₁₋₆, un halogénoalkyle en C₁₋₆, un cycloalkyle en C₃₋₇ et un hétérocyclyle à 3 à 10 chaînons, dans lequel lesdits cycloalkyle en C₃₋₇ et hétérocyclyle à 3 à 10 chaînons sont éventuellement substitués par un ou plusieurs halogènes, alkyle en C₁₋₆ ;
R^{10o} est choisi parmi un alkyle en C₁₋₆ et un halogénoalkyle en C₁₋₆ ;
R^{10p} et R^{10p'} sont indépendamment choisis parmi un hydrogène, un alkyle en C₁₋₆ et un halogénoalkyle en C₁₋₆ ;
R^{10q} représente un alkyle en C₁₋₁₀, un halogénoalkyle en C₁₋₆, un aryle, un hétéroaryle, dans lequel l'aryle et l'hétéroaryle sont éventuellement substitués par un ou plusieurs halogènes, alkyle en C₁₋₁₀ ;
R¹¹ est choisi parmi :
i) un halogène ;
ii) un hydroxy ;
iii) un cyano ;
iv) un alkyle en C₁₋₁₀, éventuellement substitué par un ou plusieurs cyano, halogènes, hydroxyle, cycloalkyle en C₃₋₇, amino, aryle, -O-aryle, hétéroaryle à 5 à 6 chaînons, hétérocyclyle à 3 à 7 chaînons, dans lequel l'hétérocyclyle à 3 à 7 chaînons et l'aryle sont éventuellement substitués par un ou plusieurs alkyle en C₁₋₆, -SO₂(alkyle en C₁₋₆), hydroxy, halogènes, cyano ;
v) un alcoxy en C₁₋₆, éventuellement substitué par un ou plusieurs, en particulier un, hétérocyclyle à 3 à 10 chaînons, halogène ;
vi) un cycloalkyle en C₃₋₇ ;
vii) un hétérocyclyle à 3 à 10 chaînons, éventuellement substitué par un ou plusieurs halogénoalkyle en C₁₋₆, hydroxy, alkyle en C₁₋₆, cycloalkyle en C₃₋₁₀, alcoxy en C₁₋₆, oxo ;
viii) un hétéroaryle à 5 à 6 chaînons, éventuellement substitué par un ou plusieurs alkyle en C₁₋₆, cycloalkyle à 3 à 10 chaînons, halogènes, halogénoalkyle en C₁₋₆, alcoxy en C₁₋₆, halogénoalcoxy en C₁₋₆ ;
ix) un phényle, éventuellement substitué par un ou plusieurs halogènes, cyano, alcoxy en C₁₋₆, halogénoalkyle en C₁₋₆, halogénoalcoxy en C₁₋₆, alkyle en C₁₋₆ ;
x) -O(R^{11a}) ;
xi) -C(O)N(R^{11b}R^{11c}) ;
xii) -SO₂(R^{11d}) ;
xiii) -C(O)OR^{11e} ;
xiv) -C(O)R^{11f} ;
xv) un oxo ;
xvi) -N(R^{11g}R^{11h}) ; et
xvii) -S(R^{11k}) ;
R^{11a} est choisi parmi un alkyle en C₁₋₁₂, un halogénoalkyle en C₁₋₆, un aminoalkyle en C₁₋₁₂, un hydroxyalkyle en C₁₋₆, un cyano, un alcynyle en C₂₋₆, un cycloalkyle en C₃₋₇, un hétérocyclyle à 3 à 10 chaînons, un -(alkyle en C₁₋₆)hétérocyclyle à 3 à 10 chaînons, un hétéroaryle à 5 à 6 chaînons, un phényle, un -alkyle en C₁₋₆-phényle, un -alkyle en C₁₋₁₂-C(O)N(R¹¹ⁱR^{11j}), un -alkyle en C₁₋₁₂-NH-C(O)(alkyle en C₁₋₆), un -alcoxy en C₁₋₁₂-NH-C(O)(alkyle en C₁₋₆), un -alkyle en C₁₋₆-NH-C(O)(alkyle en C₁₋₆), -(CH₂CH₂O)ₙ-CH₂CH₂NH₂ et -(CH₂CH₂O)ₙ-CH₂CH₂-NH-C(O)(alkyle en C₁₋₆) ;
dans lequel lesdits alkyle en C₁₋₁₂, cycloalkyle en C₃₋₇, hétérocyclyle à 3 à 10 chaînons, hétéroaryle à 5 à 6 chaînons, phényle et -alkyle en C₁₋₆-phényle sont éventuellement substitués par un ou plusieurs halogènes, alkyle en C₁₋₆, halogénoalkyle en C₁₋₆, alcoxyle en C₁₋₆, halogénoalcoxyle en C₁₋₆, cyano ;
n représente un entier entre 1 et 6, en particulier n représente deux ou trois ;
R^{11b} et R^{11c} sont chacun indépendamment choisis parmi un hydrogène, un alkyle en C₁₋₆ et un halogénoalkyle en C₁₋₆,
ou R^{11b} et R^{11c}, pris conjointement avec l'atome d'azote auquel ils sont liés, forment un hétérocyclyle à 3 à 10 chaînons ;
R^{11d} est choisi parmi un hydrogène, un alkyle en C₁₋₆, -N(R^{11l}R^{11m}), un halogénoalkyle en C₁₋₆ et un phényle ;
R^{11e} est choisi parmi un hydrogène et un alkyle en C₁₋₆ ;
R^{11f} est choisi parmi un hydrogène, un alkyle en C₁₋₆ et un phényle ;
R^{11g} et R^{11h} sont chacun indépendamment choisis parmi un hydrogène, un alkyle en C₁₋₆, un -(alkyle en C₁₋₆)phényle, un halogénoalkyle en C₁₋₆, un -SO₂(alkyle en C₁₋₆), un - SO₂(halogénoalkyle en C₁₋₆) et un -SO(alkyle en C₁₋₆)₂,
R¹¹ⁱ R^{11j} sont chacun indépendamment choisis parmi un hydrogène, un alkyle en C₁₋₆ et un halogénoalkyle en C₁₋₆,
ou R¹¹ⁱ R^{11j}, pris conjointement avec l'atome d'azote auquel ils sont liés, forment un hétérocyclyle à 3 à 10 chaînons ;
R^{11k} est choisi parmi un hydrogène, un alkyle en C₁₋₆ et un halogénoalkyle en C₁₋₆ ;
R^{11l} et R^{11m} sont chacun indépendamment choisis parmi un hydrogène et un alkyle en C₁₋₆,
ou R^{11l} et R^{11m}, pris conjointement avec l'atome d'azote auquel ils sont liés, forment un hétérocyclyle à 3 à 10 chaînons ;
R^{y} est choisi parmi un hydrogène et un alkyle en C₁₋₆.

2. Composé selon la revendication 1, ou un sel pharmaceutiquement acceptable de celui-ci, dans lequel le composé est un composé de formule (IA) 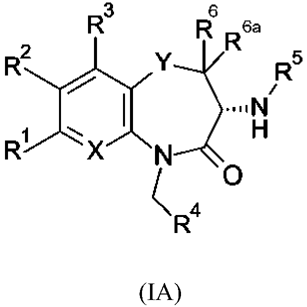 dans lequel X, Y, R¹, R², R³, R⁴, R⁵, R⁶, R^{6a} sont tels que définis dans l'une quelconque des revendications 1 à 3.

3. Composé selon la revendication 1 ou 2, dans lequel R¹ est substitué par au moins un R¹⁰, plus particulièrement par un R¹⁰.

4. Composé selon l'une quelconque des revendications 1 à 3, ou un sel pharmaceutiquement acceptable de celui-ci, dans lequel X représente N ou C(R⁷), dans lequel R⁷ représente un hydrogène ou un halogène.

5. Composé selon l'une quelconque des revendications 1 à 4, ou un sel pharmaceutiquement acceptable de celui-ci, dans lequel Y représente S(O) ou S(O)₂.

6. Composé selon l'une quelconque des revendications 1 à 5, ou un sel pharmaceutiquement acceptable de celui-ci, dans lequel R¹⁰ est choisi parmi :
i) un alkyle en C₁₋₁₀, éventuellement substitué par un ou plusieurs alcynyle en C₂₋₆, cyano ;
ii) un halogénoalkyle en C₁₋₁₀, éventuellement substitué par un alcoxy en C₁₋₅, un amino, un phényle, dans lequel le phényle est éventuellement substitué par un ou plusieurs halogènes ;
iii) un cycloalkyle en C₃₋₁₀, éventuellement substitué par un ou plusieurs halogènes ;
iv) un phényle, éventuellement substitué par un ou plusieurs halogènes, alkyle en C₁₋₁₀ ;
v) un hétéroaryle, éventuellement substitué par un ou plusieurs halogènes, alkyle en C₁₋₁₀ ;
vi) un hétérocyclyle à 3 à 10 chaînons, éventuellement substitué par un ou plusieurs alkyle en C₁₋₁₀, cycloalkyle en C₃₋₁₀, halogénoalkyle en C₁₋₆, halogènes, -alkyle en C₁₋₁₀-alcoxy en C₁₋₄, -C(O)O-alkyle en C₁₋₅ ;
vii) un amino, éventuellement substitué par un alkyle en C₁₋₁₀, un halogénoalkyle en C₁₋₁₀, un cycloalkyle en C₃₋₁₀, dans lequel le cycloalkyle en C₃₋₁₀ est éventuellement substitué par un ou plusieurs alkyle en C₁₋₅, halogènes.

7. Composé selon l'une quelconque des revendications 1 à 6, ou un sel pharmaceutiquement acceptable de celui-ci, dans lequel R¹ est choisi parmi 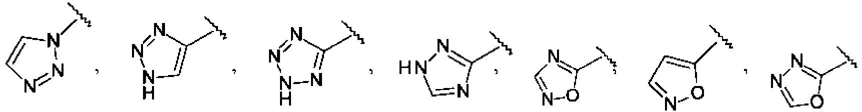 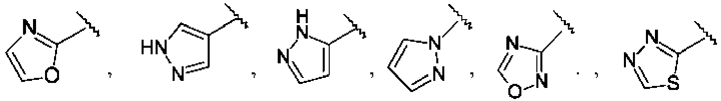 et 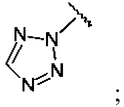 dans lequel R¹ est éventuellement substitué par un ou plusieurs R¹⁰ qui peuvent être identiques ou différents tels que définis dans l'une quelconque des revendications 1 ou 6 à 7.

8. Composé selon l'une quelconque des revendications 1 à 7, ou un sel pharmaceutiquement acceptable de celui-ci, dans lequel R² représente un hydrogène, un halogène, un alkyle en C₁₋₆, un hydroxy ou N(R⁸R^{8a}).

9. Composé selon l'une quelconque des revendications 1 à 8, ou un sel pharmaceutiquement acceptable de celui-ci, dans lequel R⁸ et R^{8a} sont chacun indépendamment choisis parmi un hydrogène et un alkyle en C₁₋₆.

10. Composé selon l'une quelconque des revendications 1 à 9, ou un sel pharmaceutiquement acceptable de celui-ci, dans lequel R³ représente un hydrogène.

11. Composé selon l'une quelconque des revendications 1 à 10, ou un sel pharmaceutiquement acceptable de celui-ci, dans lequel R¹¹ est choisi parmi un halogène, un halogénoalcoxy en C₁₋₆, un alcoxy en C₁₋₆, -O-R^{11a}, un alkyle en C₁₋₆, un alkyle en C₁₋₆ substitué par un cyano.

12. Composé selon l'une quelconque des revendications 1 à 11, ou un sel pharmaceutiquement acceptable de celui-ci, dans lequel R^{11a} est choisi parmi un alkyle en C₁₋₁₂, un halogénoalkyle en C₁₋₆, un hétérocycloalkyle à 3 à 7 chaînons et un aminoalkyle en C₁₋₁₂.

13. Composé selon l'une quelconque des revendications 1 à 12, ou un sel pharmaceutiquement acceptable de celui-ci, dans lequel R⁴ est choisi parmi 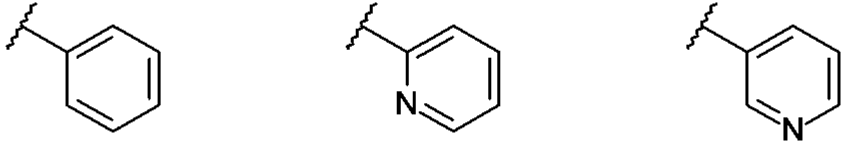 dans lequel R⁴ est éventuellement substitué par un ou plusieurs R¹¹ tels que définis dans l'une quelconque des revendications 1 ou 11.

14. Composé selon l'une quelconque des revendications 1 à 13, ou un sel pharmaceutiquement acceptable de celui-ci, dans lequel R⁵ représente un hydrogène ou -C(O)(R⁹).

15. Composé selon l'une quelconque des revendications 1 à 14, ou un sel pharmaceutiquement acceptable de celui-ci, dans lequel R⁶ et R^{6a} représentent un hydrogène.

16. Composé selon l'une quelconque des revendications 1 à 15, ou un sel pharmaceutiquement acceptable de celui-ci, dans lequel
X représente N ou C(R⁷) ;
Y représente S, SO ou S(O)₂ ;
R¹ est choisi parmi 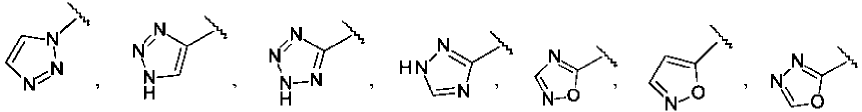 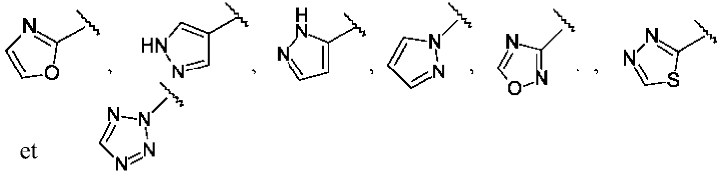
dans lequel R¹ est éventuellement substitué par un ou plusieurs R¹⁰ qui peuvent être identiques ou différents ;
R² représente un hydrogène, un halogène, un alkyle en C₁₋₆, un hydroxy ou N(R⁸R^{8a}) ;
R³ représente un hydrogène ;
R⁴ est choisi parmi 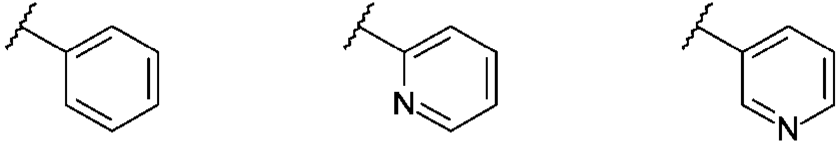
R⁵ représente un hydrogène ou -C(O)(R⁹) ;
R⁶ et R^{6a} représentent un hydrogène ;
R⁷ représente un hydrogène ou un halogène ;
R⁸ et R^{8a} sont chacun indépendamment choisis parmi un hydrogène et un alkyle en C₁₋₆ ;
R⁹ représente un aminoalkyle en C₁₋₆ ;
R¹⁰ est choisi parmi un alkyle en C₁₋₁₀, un alkyle en C₁₋₁₀ substitué par un alcynyle en C₂₋₆, un halogénoalkyle en C₁₋₁₀, un aminoalkyle en C₁₋₁₀, un hydroxyalkyle en C₁₋₁₀, un cycloalkyle en C₃₋₁₀, un cycloalkyle en C₃₋₁₀ substitué par un ou plusieurs halogènes, un phényle substitué par un ou plusieurs halogènes, un (alkyle en C₁₋₁₀)-phényle substitué par un ou plusieurs halogènes, un (alcoxyalkyle en C₁₋₁₀)-phényle, un hétérocyclyle à 3 à 10 chaînons, un hétérocyclyle à 3 à 10 chaînons substitué par un ou plusieurs alkyle en C₁₋₁₀ et halogènes, un hétéroaryle, un hétéroaryle substitué par un ou plusieurs alkyle en C₁₋₁₀, un hétéroaryle substitué par un ou plusieurs halogènes et -N(R^{10e}R^{10f}) ;
R^{10e} représente un hydrogène, R^{10f} représente un cycloalkyle en C₃₋₇ ;
R¹¹ est choisi parmi un halogène, -O(R^{11a}), un cyano, un aminoalkyle en C₁₋₁₀, un halogénoalkyle en C₁₋₆, un -S(O₂)(alkyle en C₁₋₆) et un hétérocyclyle à 3 à 10 chaînons substitué par un ou plusieurs halogénoalkyle en C₁₋₆ ;
R^{11a} est choisi parmi un alkyle en C₁₋₁₂, un halogénoalkyle en C₁₋₆, un hétérocycloalkyle à 3 à 7 chaînons, un aminoalkyle en C₁₋₁₂.

17. Composé selon l'une quelconque des revendications 1 à 16, ou un sel pharmaceutiquement acceptable de celui-ci, dans lequel
X représente N ou C(R⁷) ;
Y représente S, SO ou S(O)₂ ;
R¹ est choisi parmi 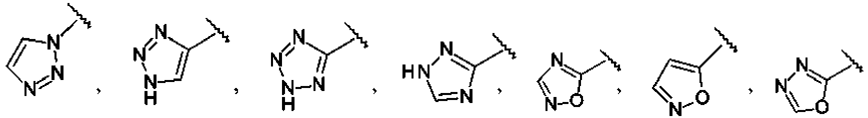 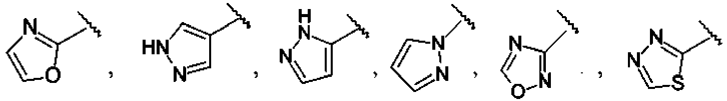 et 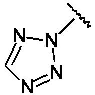
dans lequel R¹ est éventuellement substitué par un ou plusieurs R¹⁰ qui peuvent être identiques ou différents ;
R² représente un hydrogène, un halogène, un alkyle en C₁₋₆, un hydroxy ou N(R⁸R^{8a}) ;
R³ représente un hydrogène ;
R⁴ est choisi 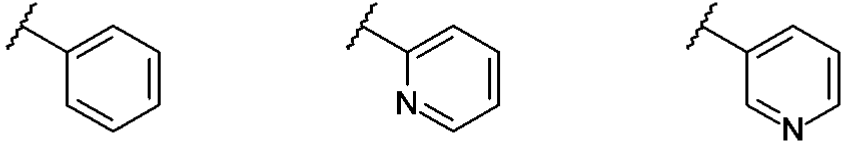
R⁵ représente un hydrogène ou -C(O)(R⁹) ;
R⁶ et R^{6a} représentent un hydrogène ;
R⁷ représente un hydrogène ou un halogène ;
R⁸ et R^{8a} sont chacun indépendamment choisis parmi un hydrogène et un alkyle en C₁₋₆ ;
R⁹ représente un aminoalkyle en C₁₋₆ ;
R¹⁰ est choisi parmi un alkyle en C₁₋₁₀, un alkyle en C₁₋₁₀ substitué par un alcynyle en C₂₋₆, un halogénoalkyle en C₁₋₁₀, un aminoalkyle en C₁₋₁₀, un hydroxyalkyle en C₁₋₁₀, un cycloalkyle en C₃₋₁₀, un cycloalkyle en C₃₋₁₀ substitué par un ou plusieurs halogènes, un phényle substitué par un ou plusieurs halogènes, un (alkyle en C₁₋₁₀)-phényle substitué par un ou plusieurs halogènes, un (alcoxyalkyle en C₁₋₁₀)-phényle, un hétérocyclyle à 3 à 10 chaînons, un hétérocyclyle à 3 à 10 chaînons substitué par un ou plusieurs alkyle en C₁₋₁₀ et halogènes, - N(R^{10e}R^{10f}) ;
R^{10e} représente un hydrogène, R^{10f} représente un cycloalkyle en C₃₋₇ ;
R¹¹ est choisi parmi un halogène, -O(R^{11a}), un cyano, un aminoalkyle en C₁₋₁₀, un - S(O₂)(alkyle en C₁₋₆) et un hétérocyclyle à 3 à 10 chaînons substitué par un ou plusieurs halogénoalkyle en C₁₋₆ ;
R^{11a} est choisi parmi un alkyle en C₁₋₁₂, un halogénoalkyle en C₁₋₆, un hétérocycloalkyle à 3 à 7 chaînons, un aminoalkyle en C₁₋₁₂.

18. Composé selon l'une quelconque des revendications 1 à 17, choisi parmi
la (3R)-3-amino-5-[(4-chlorophényl)méthyl]-8-fluoro-7-[5-(2-oxa-5-azabicyclo[4.1.0]heptan-5-yl)-1,2,4-oxadiazol-3-yl]-1-oxo-2,3-dihydro-1lambda4,5-benzothiazépin-4-one ;
la (3R)-3-amino-7-[5-(1-amino-2,2,2-trifluoro-éthyl)-1,2,4-oxadiazol-3-yl]-8-fluoro-5-[(4-isopropoxyphényl)méthyl]-1,1-dioxo-2,3-dihydro-1lambda6,5-benzothiazépin-4-one ;
la (3R)-3-amino-5-[(4-chlorophényl)méthyl]-7-[5-[3-fluoro-1-(2-méthoxyéthyl)-3-pipéridyl]-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-7-[5-(2-oxa-5-azabicyclo[4.1.0]heptan-5-yl)-1,2,4-oxadiazol-3-yl]-1-oxo-2,3-dihydro-1lambda4,5-benzothiazépin-4-one ;
la (3R)-3-amino-7-(5-tert-butyl-1,2,4-oxadiazol-3-yl)-8-fluoro-5-[(4-fluorophényl)méthyl]-1,1-dioxo-2,3-dihydro-1lambda6,5-benzothiazépin-4-one ;
la (3R)-3-amino-5-[(4-chlorophényl)méthyl]-8-fluoro-7-(5-morpholino-1,2,4-oxadiazol-3-yl)-1-oxo-2,3-dihydro-1lambda4,5-benzothiazépin-4-one ;
la (3R)-3-amino-5-[(4-chlorophényl)méthyl]-8-fluoro-7-[5-(4-oxa-7-azaspiro[2.5]octan-7-yl)-1,2,4-oxadiazol-3-yl]-1-oxo-2,3-dihydro-1lambda4,5-benzothiazépin-4-one ;
la (3R)-3-amino-5-[(4-chlorophényl)méthyl]-8-fluoro-7-[5-(4-oxa-7-azaspiro[2.5]octan-7-yl)-1,2,4-oxadiazol-3-yl]-1-oxo-2,3-dihydro-1lambda4,5-benzothiazépin-4-one ;
la (3R)-3-amino-8-fluoro-5-[(6-isopropoxy-3-pyridyl)méthyl]-7-[5-(4-oxa-7-azaspiro[2.5]octan-7-yl)-1,2,4-oxadiazol-3-yl]-1,1-dioxo-2,3-dihydro-1lambda6,5-benzothiazépin-4-one
la (3R)-3-amino-5-[(4-chlorophényl)méthyl]-7-[5-[2-(difluorométhyl)morpholin-4-yl]-1,3,4-oxadiazol-2-yl]-1,1-dioxo-2,3-dihydro-1lambda6,5-benzothiazépin-4-one
la (3R)-3-amino-5-[(4-chlorophényl)méthyl]-8-fluoro-1-oxo-7-[5-[2-(trifluorométhyl)morpholin-4-yl]-1,2,4-oxadiazol-3-yl]-2,3-dihydro-1lambda4,5-benzothiazépin-4-one ;
la (3R)-3-amino-5-[(4-chlorophényl)méthyl]-7-(5-morpholino-1,3,4-oxadiazol-2-yl)-1,1-dioxo-2,3-dihydro-1lambda6,5-benzothiazépin-4-one
la (3R)-3-amino-5-[(4-chlorophényl)méthyl]-8-fluoro-1-oxo-7-[5-[2-(trifluorométhyl)morpholin-4-yl]-1,2,4-oxadiazol-3-yl]-2,3-dihydro-1lambda4,5-benzothiazépin-4-one ;
la (3R)-3-amino-5-[(4-chlorophényl)méthyl]-7-[5-(4-oxa-7-azaspiro[2.5]octan-7-yl)-1,3,4-oxadiazol-2-yl]-1,1-dioxo-2,3-dihydro-1lambda6,5-benzothiazépin-4-one ;
le 3-[5-[(3R)-3-amino-5-[[4-(cyclopentoxy)phényl]méthyl]-1,1,4-trioxo-2,3-dihydro-1lambda6,5-benzothiazépin-7-yl]-1,3,4-oxadiazol-2-yl]pyrrolidine-1-carboxylate de méthyle ;
la (3R)-3-amino-5-[(4-chlorophényl)méthyl]-1,1-dioxo-7-[5-[2-(trifluorométhyl)morpholin-4-yl]-1,3,4-oxadiazol-2-yl]-2,3-dihydro-1lambda6,5-benzothiazépin-4-one
la (3R)-3-amino-8-fluoro-5-[(5-isopropoxy-2-pyridyl)méthyl]-7-[5-(4-oxa-7-azaspiro[2.5]octan-7-yl)-1,2,4-oxadiazol-3-yl]-1,1-dioxo-2,3-dihydro-1lambda6,5-benzothiazépin-4-one ;
le 2-amino-N-[(3R)-7-(2-tert-butyltétrazol-5-yl)-5-[(4-chlorophényl)méthyl]-8-fluoro-1,1,4-trioxo-2,3-dihydro-1lambda6,5-benzothiazépin-3-yl]acétamide
la (3R)-3-amino-5-[(4-chlorophényl)méthyl]-7-[5-(6-méthyl-3-pyridyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(4,6-diméthyl-3-pyridyl)-1,2,4-oxadiazol-3-yl]-8-fluoro-1,1-dioxo-2,3-dihydro-1lambda6,5-benzothiazépin-4-one ;
la (3R)-3-amino-8-fluoro-7-[5-[2-(hydroxyméthyl)tétrahydrofuran-2-yl]-1,2,4-oxadiazol-3-yl]-1,1-dioxo-5-[(4-phénoxyphényl)méthyl]-2,3-dihydro-1lambda6,5-benzothiazépin-4-one ;
le 2-[5-[(3R)-3-amino-1,1,4-trioxo-5-[[4-(trifluorométhoxy)phényl]méthyl]-2,3-dihydro-1lambda6,5-benzothiazépin-7-yl]-1,3,4-oxadiazol-2-yl]-2-méthyl-propanenitrile ;
la (3R)-3-amino-5-[(4-chlorophényl)méthyl]-8-fluoro-7-[5-(1-fluoro-1-méthyl-éthyl)-1,2,4-oxadiazol-3-yl]-1,1-dioxo-2,3-dihydro-1lambda6,5-benzothiazépin-4-one ;
la (3R)-3-amino-8-fluoro-7-[5-(1-fluoro-1-méthyl-éthyl)-1,2,4-oxadiazol-3-yl]-5-[(4-fluorophényl)méthyl]-1,1-dioxo-2,3-dihydro-1lambda6,5-benzothiazépin-4-one ;
la (3R)-3-amino-7-[5-(2,2-difluoromorpholin-4-yl)-1,3,4-oxadiazol-2-yl]-1,1-dioxo-5-[(4-phénoxyphényl)méthyl]-2,3-dihydro-1lambda6,5-benzothiazépin-4-one ;
la (3R)-3-amino-5-[(4-chlorophényl)méthyl]-7-[5-(2-oxa-5-azabicyclo[4.1.0]heptan-5-yl)-1,3,4-oxadiazol-2-yl]-1,1-dioxo-2,3-dihydro-1lambda6,5-benzothiazépin-4-one
la (3R)-3-amino-7-[5-(3-aminooxétan-3-yl)-1,2,4-oxadiazol-3-yl]-8-fluoro-5-[[4-(4-méthoxyphényl)phényl]méthyl]-1,1-dioxo-2,3-dihydro-1lambda6,5-benzothiazépin-4-one
la (3R)-3-amino-5-[(4-chlorophényl)méthyl]-8-fluoro-7-[5-(6-fluoro-2-méthyl-3-pyridyl)-1,2,4-oxadiazol-3-yl]-1,1-dioxo-2,3-dihydro-1lambda6,5-benzothiazépin-4-one
la (3R)-3-amino-5-[(4-chlorophényl)méthyl]-8-fluoro-7-[5-(2-méthyl-4-méthylsulfonyl-phényl)-1,2,4-oxadiazol-3-yl]-1,1-dioxo-2,3-dihydro-1lambda6,5-benzothiazépin-4-one ;
la (3R)-3-amino-5-[(4-chlorophényl)méthyl]-8-fluoro-7-[5-(2-méthyl-5-méthylsulfonyl-phényl)-1,2,4-oxadiazol-3-yl]-1,1-dioxo-2,3-dihydro-1lambda6,5-benzothiazépin-4-one ; 1,1,1,3,3,3-hexafluoropropan-2-ol ;
la (3R)-3-amino-5-[(4-chlorophényl)méthyl]-8-fluoro-7-[5-(4-méthyl-3-pyridyl)-1,2,4-oxadiazol-3-yl]-1,1-dioxo-2,3-dihydro-1lambda6,5-benzothiazépin-4-one ;
la (3R)-3-amino-5-[(4-chlorophényl)méthyl]-7-[5-[(1R,5S)-3-oxa-8-azabicyclo[3.2.1]octan-8-yl]-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-(2,2-diméthylmorpholin-4-yl)-1,2,4-oxadiazol-3-yl]-8-fluoro-1,1-dioxo-2,3-dihydro-1lambda6,5-benzothiazépin-4-one
la (3R)-3-amino-5-[(4-chlorophényl)méthyl]-7-[5-(2-méthyl-4-méthylsulfonyl-phényl)-1,3,4-oxadiazol-2-yl]-1,1-dioxo-2,3-dihydro-1lambda6,5-benzothiazépin-4-one ;
le 3-[3-[(3R)-3-amino-5-[[4-(cyclopentoxy)phényl]méthyl]-8-fluoro-1,1,4-trioxo-2,3-dihydro-1lambda6,5-benzothiazépin-7-yl]-1,2,4-oxadiazol-5-yl]pyrrolidine-1-carboxylate de méthyle
la (3R)-3-amino-5-[(4-chlorophényl)méthyl]-7-[5-(2,2-difluoromorpholin-4-yl)-1,2,4-oxadiazol-3-yl]-8-fluoro-1,1-dioxo-2,3-dihydro-1lambda6,5-benzothiazépin-4-one
la (3R)-3-amino-7-(5-tert-butyl-1,2,4-oxadiazol-3-yl)-5-[[4-(cyclopentoxy)phényl]méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(5-méthyl-1,2,4-oxadiazol-3-yl)-1,3,4-oxadiazol-2-yl]-1,1-dioxo-2,3-dihydro-1lambda6,5-benzothiazépin-4-one ;
la (3R)-3-amino-8-fluoro-7-[5-(3-fluoro-1-méthyl-3-pipéridyl)-1,2,4-oxadiazol-3-yl]-1,1-dioxo-5-[(4-phénoxyphényl)méthyl]-2,3-dihydro-1lambda6,5-benzothiazépin-4-one
la (3R)-3-amino-5-[(4-chlorophényl)méthyl]-8-fluoro-7-[5-(6-fluoro-2-méthyl-3-pyridyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(4,6-diméthyl-3-pyridyl)-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-(4,4-difluoro-1-méthoxy-cyclohexyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-7-[5-(2-fluoro-6-méthyl-phényl)-1,3,4-oxadiazol-2-yl]-1,1-dioxo-2,3-dihydro-1lambda6,5-benzothiazépin-4-one ; 1,1,1,3,3,3-hexafluoropropan-2-ol ;
le 4-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-4-méthoxy-pipéridine-1-carboxylate de méthyle ;
la (3R)-3-amino-5-[(4-chlorophényl)méthyl]-8-fluoro-7-[5-(5-méthyl-1,3,4-oxadiazol-2-yl)-1,2,4-oxadiazol-3-yl]-1,1-dioxo-2,3-dihydro-1lambda6,5-benzothiazépin-4-one ;
le N-[2-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-2-méthyl-1-(trifluorométhyl)propyl]carbamate de méthyle ;
la (3R)-3-amino-7-[5-(2-amino-3,3,3-trifluoro-1,1-diméthyl-propyl)-1,3,4-oxadiazol-2-yl]-5-[(4-chlorophényl)méthyl]-8-fluoro-1,1-dioxo-2,3-dihydro-1lambda6,5-benzothiazépin-4-one ;
le 3-[(3R)-3-amino-5-[(4-chlorophényl)méthyl]-8-fluoro-1,1,4-trioxo-2,3-dihydro-1lambda6,5-benzothiazépin-7-yl]-N-(3,3,3-trifluoro-2-hydroxy-2-méthyl-propyl)-1,2,4-oxadiazole-5-carboxamide ;
la (3R)-3-amino-5-[(4-chlorophényl)méthyl]-8-fluoro-7-[5-[2-(hydroxyméthyl)tétrahydrofuran-2-yl]-1,2,4-oxadiazol-3-yl]-1,1-dioxo-2,3-dihydro-1lambda6,5-benzothiazépin-4-one
la (3R)-3-amino-8-fluoro-7-[5-(5-méthyl-1,3,4-oxadiazol-2-yl)-1,2,4-oxadiazol-3-yl]-1,1-dioxo-5-[[4-(trifluorométhoxy)phényl]méthyl]-2,3-dihydro-1lambda6,5-benzothiazépin-4-one ;
la (3R)-3-amino-7-[5-[1-amino-2,2,2-trifluoro-1-(4-fluorophényl)éthyl]-1,2,4-oxadiazol-3-yl]-5-[(4-chlorophényl)méthyl]-8-fluoro-1,1-dioxo-2,3-dihydro-1lambda6,5-benzothiazépin-4-one ;
la (3R)-3-amino-8-fluoro-7-[5-(5-méthyl-1,3,4-oxadiazol-2-yl)-1,3,4-oxadiazol-2-yl]-1,1-dioxo-5-[[4-(trifluorométhoxy)phényl]méthyl]-2,3-dihydro-1lambda6,5-benzothiazépin-4-one ;
la (3R)-3-amino-5-[(4-chlorophényl)méthyl]-8-fluoro-7-[5-(3-oxa-8-azabicyclo[3.2.1]octan-8-yl)-1,2,4-oxadiazol-3-yl]-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-[2-(trifluorométhyl)morpholin-4-yl]-1,2,4-oxadiazol-3-yl]-2,3-dihydro-1lambda6,5-benzothiazépin-4-one ;
la (3R)-3-amino-5-[(4-chlorophényl)méthyl]-8-fluoro-7-[5-(2-oxa-5-azabicyclo[4.1.0]heptan-5-yl)-1,2,4-oxadiazol-3-yl]-1,1-dioxo-2,3-dihydro-1lambda6,5-benzothiazépin-4-one ;
la (3R)-3-amino-8-fluoro-7-[5-[2-(hydroxyméthyl)tétrahydrofuran-2-yl]-1,2,4-oxadiazol-3-yl]-1,1-dioxo-5-[[4-(trifluorométhoxy)phényl]méthyl]-2,3-dihydro-1lambda6,5-benzothiazépin-4-one
la (3R)-3-amino-5-[(4-chlorophényl)méthyl]-7-[5-(2-fluorophényl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(4-fluorophényl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-1,1-dioxo-7-[5-(p-tolyl)-1,3,4-oxadiazol-2-yl]-2,3-dihydro-1lambda6,5-benzothiazépin-4-one ;
la (3R)-3-amino-5-[(4-chlorophényl)méthyl]-8-fluoro-7-[5-(2-fluorophényl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-8-fluoro-7-[5-(m-tolyl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-8-fluoro-1,1-dioxo-7-[5-(p-tolyl)-1,3,4-oxadiazol-2-yl]-2,3-dihydro-1lambda6,5-benzothiazépin-4-one
le 1-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-2-oxa-5-azabicyclo[2.2.1]heptane-5-carboxylate de méthyle
la (3R)-3-amino-7-[5-(1-amino-2,2,2-trifluoro-1-tétrahydropyran-4-yl-éthyl)-1,2,4-oxadiazol-3-yl]-5-[(4-chlorophényl)méthyl]-8-fluoro-1,1-dioxo-2,3-dihydro-1lambda6,5-benzothiazépin-4-one ;
le 3-[(3R)-3-amino-5-[(4-chlorophényl)méthyl]-8-fluoro-1,1,4-trioxo-2,3-dihydro-1lambda6,5-benzothiazépin-7-yl]-N-(2,2-difluoropropyl)-1,2,4-oxadiazole-5-carboxamide ;
la (3R)-3-amino-7-[5-(1-amino-2,2,2-trifluoro-1-méthyl-éthyl)-1,2,4-oxadiazol-3-yl]-8-fluoro-1,1-dioxo-5-[[4-(trifluorométhoxy)phényl]méthyl]-2,3-dihydro-1lambda6,5-benzothiazépin-4-one ;
la (3R)-3-amino-8-fluoro-1,1-dioxo-5-[[4-(trifluorométhoxy)phényl]méthyl]-7-[5-[1-(trifluorométhyl)cyclopropyl]-1,2,4-oxadiazol-3-yl]-2,3-dihydro-1lambda6,5-benzothiazépin-4-one
la (3R)-3-amino-7-[5-(1-amino-2,2,2-trifluoro-1-méthyl-éthyl)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-[1-(trifluorométhyl)cyclopropyl]-1,3,4-oxadiazol-2-yl]-2,3-dihydro-1lambda6,5-benzothiazépin-4-one ;
la (3R)-3-amino-5-[(4-chlorophényl)méthyl]-1,1-dioxo-7-[5-[1-(trifluorométhyl)cyclopropyl]-1,3,4-oxadiazol-2-yl]-2,3-dihydro-1lambda6,5-benzothiazépin-4-one ;
le 3-[(3R)-3-amino-5-[(4-chlorophényl)méthyl]-8-fluoro-1,1,4-trioxo-2,3-dihydro-1lambda6,5-benzothiazépin-7-yl]-N-(2,2,2-trifluoroéthyl)-1,2,4-oxadiazole-5-carboxamide ;
la (3R)-3-amino-5-[(4-chlorophényl)méthyl]-1,1-dioxo-7-(5-phényl-1,3,4-oxadiazol-2-yl)-2,3-dihydro-1lambda6,5-benzothiazépin-4-one
la (3R)-3-amino-5-[(4-chlorophényl)méthyl]-8-fluoro-7-[5-(4-fluorophényl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-8-fluoro-7-[5-(o-tolyl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-8-fluoro-1,1-dioxo-7-(5-phényl-1,3,4-oxadiazol-2-yl)-2,3-dihydro-1lambda6,5-benzothiazépin-4-one ;
le 3-[5-[(3R)-3-amino-1,1,4-trioxo-5-[[4-(trifluorométhoxy)phényl]méthyl]-2,3-dihydro-1lambda6,5-benzothiazépin-7-yl]-1,3,4-oxadiazol-2-yl]pyrrolidine-1-carboxylate de méthyle ;
la (3R)-3-amino-5-[(4-chlorophényl)méthyl]-8-fluoro-7-[5-(5-méthyl-2-pyridyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-7-[5-(6-méthyl-3-pyridyl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-8-fluoro-1,1-dioxo-7-[5-[(3,3,3-trifluoro-2-hydroxy-2-méthyl-propyl)amino]-1,3,4-oxadiazol-2-yl]-2,3-dihydro-1lambda6,5-benzothiazépin-4-one
la (3R)-3-amino-5-[(4-chlorophényl)méthyl]-8-fluoro-7-[5-[[1-(2-hydroxyéthyl)cyclohexyl]amino]-1,3,4-oxadiazol-2-yl]-1,1-dioxo-2,3-dihydro-1lambda6,5-benzothiazépin-4-one ;
la (3R)-3-amino-5-benzyl-8-fluoro-1,1-dioxo-7-[5-(1,2,2,2-tétrafluoro-1-méthoxy-éthyl)-1,2,4-oxadiazol-3-yl]-2,3-dihydro-1lambda6,5-benzothiazépin-4-one ;
la (3R)-3-amino-7-[5-(3-aminooxétan-3-yl)-1,2,4-oxadiazol-3-yl]-8-fluoro-1,1-dioxo-5-[[4-(trifluorométhoxy)phényl]méthyl]-2,3-dihydro-1lambda6,5-benzothiazépin-4-one
la (3R)-3-amino-5-[(4-chlorophényl)méthyl]-8-fluoro-7-[5-(6-fluoro-3-pyridyl)-1,3,4-oxadiazol-2-yl]-1,1-dioxo-2,3-dihydro-1lambda6,5-benzothiazépin-4-one ;
la (3R)-3-amino-7-(5-tert-butyl-1,3,4-oxadiazol-2-yl)-1,1-dioxo-5-[(4-phénoxyphényl)méthyl]-2,3-dihydro-1lambda6,5-benzothiazépin-4-one
la (3R)-3-amino-5-[(4-chlorophényl)méthyl]-8-fluoro-7-[5-(5-fluoro-2-pyridyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(2,2-difluoropropylamino)-1,3,4-oxadiazol-2-yl]-1,1-dioxo-2,3-dihydro-1lambda6,5-benzothiazépin-4-one ;
le 2-[[3-[(3R)-3-amino-5-[(4-chlorophényl)méthyl]-8-fluoro-1,1,4-trioxo-2,3-dihydro-1lambda6,5-benzothiazépin-7-yl]-1,2,4-oxadiazol-5-yl]amino]-2-méthyl-propanenitrile
la (3R)-3-amino-5-[(4-chlorophényl)méthyl]-7-[5-(1-méthoxycyclopropyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-1,1-dioxo-7-[5-(2,2,2-trifluoro-1-méthoxy-1-méthyl-éthyl)-1,3,4-oxadiazol-2-yl]-2,3-dihydro-1lambda6,5-benzothiazépin-4-one ;
la (3R)-3-amino-5-[(4-chlorophényl)méthyl]-7-[5-(4-méthoxytétrahydropyran-4-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-(1,1,2,2,2-pentafluoroéthyl)-1,3,4-oxadiazol-2-yl]-2,3-dihydro-1lambda6,5-benzothiazépin-4-one
le N-[11-[4-[[(3R)-3-amino-7-(5-tert-butyl-1,3,4-oxadiazol-2-yl)-8-fluoro-1,1,4-trioxo-2,3-dihydro-1lambda6,5-benzothiazépin-5-yl]méthyl]phénoxy]undécyl]-3-[2,2-difluoro-12-(1H-pyrrol-2-yl)-1-aza-3-azonia-2-boranuidatricyclo[7.3.0.03,7]dodéca-3,5,7,9,11-pent
la (3R)-3-amino-5-[(4-chlorophényl)méthyl]-7-[5-(1-méthoxy-1-méthyl-éthyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-[(1-éthynylcyclohexyl)amino]-1,3,4-oxadiazol-2-yl]-8-fluoro-1,1-dioxo-2,3-dihydro-1lambda6,5-benzothiazépin-4-one ;
la (3R)-3-amino-7-(5-tert-butyl-1,2,4-oxadiazol-3-yl)-5-[(4-chlorophényl)méthyl]-8-fluoro-1-imino-1-oxo-2,3-dihydro-1lambda6,5-benzothiazépin-4-one ;
la (3R)-3-amino-5-[(4-chlorophényl)méthyl]-7-[5-(9,9-difluoro-3-oxa-7-azabicyclo[3.3.1]nonan-7-yl)-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[4,4-difluoro-3-(hydroxyméthyl)-1-pipéridyl]-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-[5-(3,3-diméthylmorpholin-4-yl)-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-[5-(5,5-difluoro-1-méthyl-3-pipéridyl)-1,2,4-oxadiazol-3-yl]-8-fluoro-1-oxo-2,3-dihydro-1lambda4,5-benzothiazépin-4-one
la (3R)-3-amino-5-[(4-chlorophényl)méthyl]-7-[5-(5,5-difluoro-1-méthyl-3-pipéridyl)-1,2,4-oxadiazol-3-yl]-8-fluoro-1-oxo-2,3-dihydro-1lambda4,5-benzothiazépin-4-one
le 4-[5-[(3R)-3-amino-5-[(4-chlorophényl)méthyl]-1,1,4-trioxo-2,3-dihydro-1lambda6,5-benzothiazépin-7-yl]-1,3,4-oxadiazol-2-yl]-4-cyano-pipéridine-1-carboxylate de méthyle
la (3R)-3-amino-5-[(4-chlorophényl)méthyl]-7-[5-(3,3-difluoro-1,8-diazaspiro[4.5]décan-8-yl)-1,3,4-oxadiazol-2-yl]-8-fluoro-1,1-dioxo-2,3-dihydro-1lambda6,5-benzothiazépin-4-one ;
la (3R)-3-amino-7-(5-tert-butyl-1,2,4-oxadiazol-3-yl)-5-[(4-chlorophényl)méthyl]-8-fluoro-1-oxo-2,3-dihydro-1lambda4,5-benzothiazépin-4-one ;
la (3R)-3-amino-7-(5-tert-butyl-1,2,4-oxadiazol-3-yl)-5-[(4-chlorophényl)méthyl]-8-fluoro-1-oxo-2,3-dihydro-1lambda4,5-benzothiazépin-4-one ;
la (3R)-3-amino-5-[(4-chlorophényl)méthyl]-8-fluoro-7-[5-(1-méthyl-1-pyrrolidin-1-yl-éthyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-7-[5-[4-méthyl-1-oxido-1-(2,2,2-trifluoroéthyl)pipéridin-1-ium-4-yl]-1,2,4-oxadiazol-3-yl]-1-oxo-2,3-dihydro-1lambda4,5-benzothiazépin-4-one ;
le 2-[4-[[(3R)-3-amino-7-(5-tert-butyl-1,3,4-oxadiazol-2-yl)-8-fluoro-1,1,4-trioxo-2,3-dihydro-1lambda6,5-benzothiazépin-5-yl]méthyl]phényl]-2-méthyl-propanenitrile
la (3R)-3-amino-5-[(4-chlorophényl)méthyl]-7-[5-[(1S,5R)-6,6-difluoro-3-azabicyclo[3.1.1]heptan-3-yl]-1,3,4-oxadiazol-2-yl]-8-fluoro-1,1-dioxo-2,3-dihydro-1lambda6,5-benzothiazépin-4-one ;
le 1-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-4-méthyl-pipéridine-4-carbonitrile ;
la (3R)-3-amino-7-(5-tert-butyl-1,3,4-oxadiazol-2-yl)-8-fluoro-1,1-dioxo-5-[[4-(2,2,2-trifluoroéthoxy)phényl]méthyl]-2,3-dihydro-1lambda6,5-benzothiazépin-4-one
la (3R)-3-amino-5-[(4-chlorophényl)méthyl]-7-[5-[[1,1-diméthyl-4-(2-prop-2-ynoxyéthoxy)butyl]amino]-1,2,4-oxadiazol-3-yl]-8-fluoro-1,1-dioxo-2,3-dihydro-1lambda6,5-benzothiazépin-4-one
le 3-[(3R)-3-amino-8-fluoro-1,1,4-trioxo-5-[[4-(1,1,2,2-tétrafluoroéthoxy)phényl]méthyl]-2,3-dihydro-1lambda6,5-benzothiazépin-7-yl]-N,N-diméthyl-1,2,4-oxadiazole-5-carboxamide ;
la (3R)-3-amino-7-(5-tert-butyl-1,3,4-oxadiazol-2-yl)-5-[(6-chloropyridazin-3-yl)méthyl]-8-fluoro-1,1-dioxo-2,3-dihydro-1lambda6,5-benzothiazépin-4-one
la (3R)-3-amino-5-[(4-chlorophényl)méthyl]-8-fluoro-1,1-dioxo-7-[5-(2,2,2-trifluoroéthylamino)-1,3,4-oxadiazol-2-yl]-2,3-dihydro-1lambda6,5-benzothiazépin-4-one ;
la (3R)-3-amino-5-[(4-chlorophényl)méthyl]-7-[5-[1,1-diméthyl-4-[2-(2-prop-2-ynoxyéthoxy)éthoxy]butyl]-1,2,4-oxadiazol-3-yl]-8-fluoro-1,1-dioxo-2,3-dihydro-1lambda6,5-benzothiazépin-4-one ;
la (3R)-3-amino-5-[(4-chlorophényl)méthyl]-7-[2-(5,5-difluoro-1-méthylsulfonyl-3-pipéridyl)tétrazol-5-yl]-8-fluoro-1,1-dioxo-2,3-dihydro-1lambda6,5-benzothiazépin-4-one
le 5-[5-[(3R)-3-amino-5-[(4-chlorophényl)méthyl]-8-fluoro-1,1,4-trioxo-2,3-dihydro-1lambda6,5-benzothiazépin-7-yl]tétrazol-2-yl]-3,3-difluoro-pipéridine-1-carboxylate de méthyle
le 2-[[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]amino]-2-méthyl-propanenitrile ;
la (3R)-3-amino-5-[(4-chlorophényl)méthyl]-8-fluoro-7-[5-[4-méthyl-1-(2,2,2-trifluoroéthyl)-4-pipéridyl]-1,2,4-oxadiazol-3-yl]-1,1-dioxo-2,3-dihydro-1lambda6,5-benzothiazépin-4-one ;
la (3R)-3-amino-5-[(4-chlorophényl)méthyl]-7-[5-(2,2-difluoropropylamino)-1,3,4-oxadiazol-2-yl]-8-fluoro-1,1-dioxo-2,3-dihydro-1lambda6,5-benzothiazépin-4-one ;
la (3R)-3-amino-7-[5-(1-bicyclo[1.1.1]pentanylamino)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-[(3,3,3-trifluoro-1-méthyl-propyl)amino]-1,3,4-oxadiazol-2-yl]-2,3-dihydro-1lambda6,5-benzothiazépin-4-one ; 1,1,1,3,3,3-hexafluoropropan-2-ol ;
la (3R)-3-amino-7-(5-tert-butyl-1,3,4-oxadiazol-2-yl)-1,1-dioxo-5-[(4-tétrahydropyran-4-yloxyphényl)méthyl]-2,3-dihydro-1lambda6,5-benzothiazépin-4-one
le 1-[3-[(3R)-3-amino-8-fluoro-1-imino-1,4-dioxo-5-[[4-(1,1,2,2-tétrafluoroéthoxy)phényl]méthyl]-2,3-dihydro-1lambda6,5-benzothiazépin-7-yl]-1,2,4-oxadiazol-5-yl]-3-azabicyclo[3.1.1]heptane-3-carboxylate de méthyle
le 1-[3-[(3R)-3-amino-8-fluoro-1,4-dioxo-5-[[4-(trifluorométhoxy)phényl]méthyl]-2,3-dihydro-1lambda4,5-benzothiazépin-7-yl]-1,2,4-oxadiazol-5-yl]-3-azabicyclo[3.1.1]heptane-3-carboxylate de méthyle
la (3R)-3-amino-5-[(4-chlorophényl)méthyl]-8-fluoro-1-imino-1-oxo-7-[5-(1,2,2,2-tétrafluoro-1-méthoxy-éthyl)-1,2,4-oxadiazol-3-yl]-2,3-dihydro-1lambda6,5-benzothiazépin-4-one ;
la (3R)-3-amino-7-(2-tert-butyltétrazol-5-yl)-8-fluoro-1,1-dioxo-5-[[4-(tétrahydropyran-4-ylméthoxy)phényl]méthyl]-2,3-dihydro-1lambda6,5-benzothiazépin-4-one
la (3R)-3-amino-7-(5-tert-butyl-1,2,4-oxadiazol-3-yl)-8-fluoro-1,1-dioxo-5-[[4-(tétrahydropyran-4-ylméthoxy)phényl]méthyl]-2,3-dihydro-1lambda6,5-benzothiazépin-4-one
la (3R)-3-amino-7-(5-tert-butyl-1,3,4-oxadiazol-2-yl)-5-[[4-(cyclopropoxy)phényl]méthyl]-1,1-dioxo-2,3-dihydro-1lambda6,5-benzothiazépin-4-one
le 1-[3-[(3R)-3-amino-8-fluoro-1,4-dioxo-5-[[4-(trifluorométhoxy)phényl]méthyl]-2,3-dihydro-1lambda4,5-benzothiazépin-7-yl]-1,2,4-oxadiazol-5-yl]-3-azabicyclo[3.1.1]heptane-3-carboxylate de méthyle
la (3R)-7-[2-(1-acétyl-5,5-difluoro-3-pipéridyl)tétrazol-5-yl]-3-amino-5-[(4-chiorophényl)méthyl]-8-fluoro-1,1-dioxo-2,3-dihydro-1lambda6,5-benzothiazépin-4-one
le N-[(3R)-5-[(4-chlorophényl)méthyl]-7-[2-(5,5-difluoro-3-pipéridyl)tétrazol-5-yl]-8-fluoro-1,1,4-trioxo-2,3-dihydro-1lambda6,5-benzothiazépin-3-yl]acétamide ;
la (3R)-3-amino-5-[(4-chlorophényl)méthyl]-8-fluoro-1,1-dioxo-7-[5-(1,5,5-triméthyl-3-pipéridyl)-1,2,4-oxadiazol-3-yl]-2,3-dihydro-1lambda6,5-benzothiazépin-4-one ;
la (3R)-3-amino-7-(5-tert-butyl-1,3,4-oxadiazol-2-yl)-5-[[4-(cyclohexoxy)phényl]méthyl]-1,1-dioxo-2,3-dihydro-1lambda6,5-benzothiazépin-4-one
le 1-[3-[(3R)-3-amino-8-fluoro-1,4-dioxo-5-[[4-(1,1,2,2-tétrafluoroéthoxy)phényl]méthyl]-2,3-dihydro-1lambda4,5-benzothiazépin-7-yl]-1,2,4-oxadiazol-5-yl]-3-azabicyclo[3.1.1]heptane-3-carboxylate de méthyle
la (3R)-3-amino-7-(2-tert-butyltétrazol-5-yl)-5-[(4-chlorophényl)méthyl]-8-fluoro-1-oxo-2,3-dihydro-1lambda4,5-benzothiazépin-4-one
la (3R)-3-amino-7-(2-tert-butyltétrazol-5-yl)-5-[(4-chlorophényl)méthyl]-8-fluoro-1-oxo-2,3-dihydro-1lambda4,5-benzothiazépin-4-one
la (3R)-3-amino-5-[(4-chlorophényl)méthyl]-8-fluoro-1-oxo-7-[5-(1,2,2,2-tétrafluoro-1-méthoxy-éthyl)-1,2,4-oxadiazol-3-yl]-2,3-dihydro-1lambda4,5-benzothiazépin-4-one ;
la (3R)-3-amino-5-[(4-chlorophényl)méthyl]-8-fluoro-1-oxo-7-[5-(1,2,2,2-tétrafluoro-1-méthoxy-éthyl)-1,2,4-oxadiazol-3-yl]-2,3-dihydro-1lambda4,5-benzothiazépin-4-one
la (3R)-3-amino-5-[(4-chlorophényl)méthyl]-8-fluoro-1-oxo-7-[5-(1,2,2,2-tétrafluoro-1-méthoxy-éthyl)-1,2,4-oxadiazol-3-yl]-2,3-dihydro-1lambda4,5-benzothiazépin-4-one ;
la (3R)-3-amino-5-[(4-chlorophényl)méthyl]-8-fluoro-7-[5-[[(1S)-1-méthyl-2-(trifluorométhoxy)éthyl]amino]-1,3,4-oxadiazol-2-yl]-1,1-dioxo-2,3-dihydro-1lambda6,5-benzothiazépin-4-one ;
la (3R)-3-amino-5-benzyl-7-[2-(5,5-difluoro-1-méthyl-3-pipéridyl)tétrazol-5-yl]-8-fluoro-1,1-dioxo-2,3-dihydro-1lambda6,5-benzothiazépin-4-one
la (3R)-3-amino-5-[(4-chlorophényl)méthyl]-7-[2-(5,5-difluoro-1-méthyl-3-pipéridyl)tétrazol-5-yl]-8-fluoro-1,1-dioxo-2,3-dihydro-1lambda6,5-benzothiazépin-4-one
la (3R)-3-amino-7-(5-tert-butyl-1,3,4-oxadiazol-2-yl)-1,1-dioxo-5-[[4-(tétrahydropyran-4-ylméthoxy)phényl]méthyl]-2,3-dihydro-1lambda6,5-benzothiazépin-4-one
la (3R)-3-amino-8-fluoro-1,1-dioxo-5-[[4-(trifluorométhoxy)phényl]méthyl]-7-[8-(trifluorométhyl)-5,6,7,8-tétrahydro-[1,2,4]triazolo[1,5-a]pyrazin-2-yl]-2,3-dihydro-1lambda6,5-benzothiazépin-4-one
la (3R)-3-amino-8-fluoro-5-[(4-fluorophényl)méthyl]-1-oxo-7-[5-(1,2,2,2-tétrafluoro-1-méthoxy-éthyl)-1,2,4-oxadiazol-3-yl]-2,3-dihydro-1lambda4,5-benzothiazépin-4-one ;
la (3R)-3-amino-8-fluoro-5-[(4-fluorophényl)méthyl]-1,1-dioxo-7-[5-(1,2,2,2-tétrafluoro-1-méthoxy-éthyl)-1,2,4-oxadiazol-3-yl]-2,3-dihydro-1lambda6,5-benzothiazépin-4-one ;
la (3R)-3-amino-5-[(4-chlorophényl)méthyl]-8-fluoro-1,1-dioxo-7-[5-(2,2,2-trifluoro-1-hydroxy-1-méthyl-éthyl)-1,2,4-oxadiazol-3-yl]-2,3-dihydro-1lambda6,5-benzothiazépin-4-one
la (3R)-3-amino-7-(5-tert-butyl-1,3,4-oxadiazol-2-yl)-1,1-dioxo-5-[[4-[[5-(trifluorométhyl)-2-pyridyl]oxy]phényl]méthyl]-2,3-dihydro-1lambda6,5-benzothiazépin-4-one
le 1-[3-[(3R)-3-amino-8-fluoro-1,1,4-trioxo-5-[[4-(1,1,2,2-tétrafluoroéthoxy)phényl]méthyl]-2,3-dihydro-1lambda6,5-benzothiazépin-7-yl]-1,2,4-oxadiazol-5-yl]-3-azabicyclo[3.1.1]heptane-3-carboxylate de méthyle
la (3R)-3-amino-7-(5-tert-butyl-1,3,4-oxadiazol-2-yl)-5-[[4-(4-fluorophénoxy)phényl]méthyl]-1,1-dioxo-2,3-dihydro-1lambda6,5-benzothiazépin-4-one
le 1-[3-[(3R)-3-amino-5-[(4-chlorophényl)méthyl]-8-fluoro-1,1,4-trioxo-2,3-dihydro-1lambda6,5-benzothiazépin-7-yl]-1,2,4-oxadiazol-5-yl]-3-azabicyclo[3.1.1]heptane-3-carboxylate de méthyle
le 1-[3-[(3R)-3-amino-8-fluoro-1,1,4-trioxo-5-[[4-(trifluorométhoxy)phényl]méthyl]-2,3-dihydro-1lambda6,5-benzothiazépin-7-yl]-1,2,4-oxadiazol-5-yl]-3-azabicyclo[3.1.1]heptane-3-carboxylate de méthyle
la (3R)-3-amino-5-[(4-chlorophényl)méthyl]-8-fluoro-1,1-dioxo-7-[5-[1-(trifluorométhyl)cyclopropyl]-1,2,4-oxadiazol-3-yl]-2,3-dihydro-1lambda6,5-benzothiazépin-4-one ;
la (3R)-3-amino-7-(2-tert-butyltétrazol-5-yl)-8-fluoro-5-[(4-fluorophényl)méthyl]-1,1-dioxo-2,3-dihydro-1lambda6,5-benzothiazépin-4-one
la (3R)-3-amino-1-oxo-7-[5-(2,2,2-trifluoroéthyl)-1,3,4-oxadiazol-2-yl]-5-[[4-(trifluorométhoxy)phényl]méthyl]-2,3-dihydro-1lambda4,5-benzothiazépin-4-one ;
la (3R)-3-amino-1-oxo-7-[5-(2,2,2-trifluoroéthyl)-1,3,4-oxadiazol-2-yl]-5-[[4-(trifluorométhoxy)phényl]méthyl]-2,3-dihydro-1lambda4,5-benzothiazépin-4-one ;
la (3R)-3-amino-1-oxo-7-[5-(1,2,2,2-tétrafluoro-1-méthoxy-éthyl)-1,3,4-oxadiazol-2-yl]-5-[[4-(trifluorométhoxy)phényl]méthyl]-2,3-dihydro-1lambda4,5-benzothiazépin-4-one ;
la (3R)-3-amino-1-oxo-7-[5-(1,2,2,2-tétrafluoro-1-méthoxy-éthyl)-1,3,4-oxadiazol-2-yl]-5-[[4-(trifluorométhoxy)phényl]méthyl]-2,3-dihydro-1lambda4,5-benzothiazépin-4-one ;
la (3R)-3-amino-7-[5-(1-amino-2,2,2-trifluoro-éthyl)-1,2,4-oxadiazol-3-yl]-8-fluoro-1,1-dioxo-5-[[4-(trifluorométhoxy)phényl]méthyl]-2,3-dihydro-1lambda6,5-benzothiazépin-4-one ;
le 1-[3-[(3R)-3-amino-5-[(4-chlorophényl)méthyl]-8-fluoro-1,1,4-trioxo-2,3-dihydro-1lambda6,5-benzothiazépin-7-yl]-1,2,4-oxadiazol-5-yl]-3-azabicyclo[3.1.1]heptane-3-carboxylate d'isopropyle
le 1-[3-[(3R)-3-amino-5-[(4-chlorophényl)méthyl]-8-fluoro-1,1,4-trioxo-2,3-dihydro-1lambda6,5-benzothiazépin-7-yl]-1,2,4-oxadiazol-5-yl]-3-azabicyclo[3.1.1]heptane-3-carboxylate d'éthyle
la (3R)-3-amino-5-[(4-chlorophényl)méthyl]-7-[5-[1-(2,2-difluorocyclopropyl)éthylamino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[1-(2,2-difluorocyclopropyl)éthylamino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[1-(3,3-difluorocyclobutyl)éthylamino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-[(3,3,3-trifluoro-1,1-diméthyl-propyl)amino]-1,3,4-oxadiazol-2-yl]-2,3-dihydro-1lambda6,5-benzothiazépin-4-one ;
la (3R)-3-amino-5-[(4-chlorophényl)méthyl]-8-fluoro-7-[5-[1-méthyl-1-(2,2,2-trifluoroéthylamino)éthyl]-1,2,4-oxadiazol-3-yl]-1,1-dioxo-2,3-dihydro-1lambda6,5-benzothiazépin-4-one ;
le 3-[3-[(3R)-3-amino-8-fluoro-5-[(4-isopropoxyphényl)méthyl]-1,1,4-trioxo-2,3-dihydro-1lambda6,5-benzothiazépin-7-yl]-1,2,4-oxadiazol-5-yl]pyrrolidine-1-carboxylate de méthyle ;
le 1-[5-[(3R)-3-amino-1,1,4-trioxo-5-[[4-(trifluorométhoxy)phényl]méthyl]-2,3-dihydro-1lambda6,5-benzothiazépin-7-yl]-1,3,4-oxadiazol-2-yl]-3-azabicyclo[3.1.1]heptane-3-carboxylate de méthyle
la (3R)-3-amino-7-(5-tert-butyl-1,3,4-oxadiazol-2-yl)-1,1-dioxo-5-[[4-(trifluorométhylsulfonyl)phényl]méthyl]-2,3-dihydro-1lambda6,5-benzothiazépin-4-one
la (3R)-3-amino-7-[5-(5,5-difluoro-1-méthyl-3-pipéridyl)-1,2,4-oxadiazol-3-yl]-8-fluoro-5-[(4-isopropoxyphényl)méthyl]-1,1-dioxo-2,3-dihydro-1lambda6,5-benzothiazépin-4-one ;
la (3R)-3-amino-7-[5-(5,5-difluoro-1-méthyl-3-pipéridyl)-1,2,4-oxadiazol-3-yl]-8-fluoro-5-[(6-isopropoxy-3-pyridyl)méthyl]-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-[[3-(trifluorométhyl)cyclobutyl]amino]-1,3,4-oxadiazol-2-yl]-2,3-dihydro-1lambda6,5-benzothiazépin-4-one ;
la (3R)-3-amino-5-[(4-chlorophényl)méthyl]-8-fluoro-1,1-dioxo-7-[5-(1,2,2,2-tétrafluoroéthyl)-1,2,4-oxadiazol-3-yl]-2,3-dihydro-1lambda6,5-benzothiazépin-4-one ;
le 1-[5-[(3R)-3-amino-1,1,4-trioxo-5-[[4-(1,1,2,2-tétrafluoroéthoxy)phényl]méthyl]-2,3-dihydro-1lambda6,5-benzothiazépin-7-yl]-1,3,4-oxadiazol-2-yl]-3-azabicyclo[3.1.1]heptane-3-carboxylate de méthyle
la (3R)-3-amino-7-[5-[(3-chloro-1-bicyclo[1.1.1]pentanyl)amino]-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-[(3-fluoro-1-bicyclo[1.1.1]pentanyl)amino]-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-(1,1,2,2,2-pentafluoroéthyl)-1,2,4-oxadiazol-3-yl]-2,3-dihydro-1lambda6,5-benzothiazépin-4-one
la (3R)-3-amino-5-[(4-chlorophényl)méthyl]-7-[5-[(3,3-difluoro-1-méthyl-cyclobutyl)amino]-1,2,4-oxadiazol-3-yl]-8-fluoro-1,1-dioxo-2,3-dihydro-1lambda6,5-benzothiazépin-4-one
la (3R)-3-amino-5-[(4-chlorophényl)méthyl]-8-fluoro-7-[2-[(3R)-1-méthylpyrrolidin-3-yl]tétrazol-5-yl]-1,1-dioxo-2,3-dihydro-1lambda6,5-benzothiazépin-4-one ;
la (3R)-3-amino-5-[(4-chlorophényl)méthyl]-8-fluoro-7-[2-[(3S)-1-méthylpyrrolidin-3-yl]tétrazol-5-yl]-1,1-dioxo-2,3-dihydro-1lambda6,5-benzothiazépin-4-one ;
le 3-[(3R)-3-amino-5-[(4-chlorophényl)méthyl]-8-fluoro-1,1,4-trioxo-2,3-dihydro-1lambda6,5-benzothiazépin-7-yl]-N,N-diméthyl-1,2,4-oxadiazole-5-carboxamide ;
le 3-[(3R)-3-amino-8-fluoro-1,1,4-trioxo-5-[[4-(trifluorométhoxy)phényl]méthyl]-2,3-dihydro-1lambda6,5-benzothiazépin-7-yl]-N,N-diméthyl-1,2,4-oxadiazole-5-carboxamide ;
la (3R)-3-amino-5-[(4-chlorophényl)méthyl]-7-[1-(5,5-difluoro-1-méthyl-3-pipéridyl)pyrazol-4-yl]-8-fluoro-1,1-dioxo-2,3-dihydro-1lambda6,5-benzothiazépin-4-one ;
la (3R)-3-amino-7-[5-[(2-amino-3,3,3-trifluoro-propyl)amino]-1,2,4-oxadiazol-3-yl]-5-[(4-chlorophényl)méthyl]-8-fluoro-1,1-dioxo-2,3-dihydro-1lambda6,5-benzothiazépin-4-one ;
la (3R)-3-amino-7-(5-tert-butyl-1,3,4-oxadiazol-2-yl)-1,1-dioxo-5-[(4-pyrrolidin-1-ylphényl)méthyl]-2,3-dihydro-1lambda6,5-benzothiazépin-4-one
le 4-[5-[(3R)-3-amino-5-[(4-chlorophényl)méthyl]-1,1,4-trioxo-2,3-dihydro-1lambda6,5-benzothiazépin-7-yl]-1,3,4-oxadiazol-2-yl]-4-méthyl-pipéridine-1-carboxylate de (6-méthyl-3-pyridyle) ;
la (3R)-3-amino-7-[5-(5,5-difluoro-1-méthyl-3-pipéridyl)-1,2,4-oxadiazol-3-yl]-8-fluoro-5-[(5-isopropoxy-2-pyridyl)méthyl]-1,1-dioxo-2,3-dihydro-1lambda6,5-benzothiazépin-4-one ;
la (3R)-3-amino-5-[(4-chlorophényl)méthyl]-7-[5-(1-éthyl-4-méthyl-4-pipéridyl)-1,2,4-oxadiazol-3-yl]-8-fluoro-1,1-dioxo-2,3-dihydro-1lambda6,5-benzothiazépin-4-one ;
la (3R)-3-amino-5-[(4-chlorophényl)méthyl]-8-fluoro-1,1-dioxo-7-[5-(1,2,2,2-tétrafluoro-1-méthoxy-éthyl)-1,2,4-oxadiazol-3-yl]-2,3-dihydro-1lambda6,5-benzothiazépin-4-one ;
le 2-[3-[(3R)-3-amino-5-[(4-chlorophényl)méthyl]-8-fluoro-1,1,4-trioxo-2,3-dihydro-1lambda6,5-benzothiazépin-7-yl]-1,2,4-oxadiazol-5-yl]-2-méthyl-propanenitrile ;
la (3R)-3-amino-5-[(4-chlorophényl)méthyl]-7-[5-(5,5-diméthyl-3-pipéridyl)-1,2,4-oxadiazol-3-yl]-8-fluoro-1,1-dioxo-2,3-dihydro-1lambda6,5-benzothiazépin-4-one ;
le 4-[5-[(3R)-3-amino-5-[(4-chlorophényl)méthyl]-1,1,4-trioxo-2,3-dihydro-1lambda6,5-benzothiazépin-7-yl]-1,3,4-oxadiazol-2-yl]-4-méthyl-pipéridine-1-carboxylate de 2,2,2-trifluoroéthyle ;
le 4-[3-[(3R)-3-amino-5-[(4-chlorophényl)méthyl]-8-fluoro-1,1,4-trioxo-2,3-dihydro-1lambda6,5-benzothiazépin-7-yl]-1,2,4-oxadiazol-5-yl]-4-méthyl-pipéridine-1-carboxylate de méthyle ;
la (3R)-3-amino-5-[(4-chlorophényl)méthyl]-8-fluoro-1,1-dioxo-7-[5-[[1-(2,2,2-trifluoroéthyl)-3-pipéridyl]amino]-1,3,4-oxadiazol-2-yl]-2,3-dihydro-1lambda6,5-benzothiazépin-4-one
la (3R)-3-amino-7-[2-(2-amino-3,3,3-trifluoro-propyl)tétrazol-5-yl]-5-[(4-chlorophényl)méthyl]-8-fluoro-1,1-dioxo-2,3-dihydro-1lambda6,5-benzothiazépin-4-one ;
le 4-[3-[(3R)-3-amino-5-[(4-chlorophényl)méthyl]-8-fluoro-1,1,4-trioxo-2,3-dihydro-1lambda6,5-benzothiazépin-7-yl]-1,2,4-oxadiazol-5-yl]-N,4-diméthyl-pipéridine-1-carboxamide ;
la (3R)-3-amino-5-[(4-chlorophényl)méthyl]-8-fluoro-7-[5-[4-méthyl-1-(pyrrolidine-1-carbonyl)-4-pipéridyl]-1,2,4-oxadiazol-3-yl]-1,1-dioxo-2,3-dihydro-1lambda6,5-benzothiazépin-4-one ;
la (3R)-3-amino-7-[5-(5,5-difluoro-1-méthyl-3-pipéridyl)-1,3,4-oxadiazol-2-yl]-1,1-dioxo-5-[[4-(trifluorométhoxy)phényl]méthyl]-2,3-dihydro-1lambda6,5-benzothiazépin-4-one ;
la (3R)-3-amino-5-[(4-chlorophényl)méthyl]-8-fluoro-1,1-dioxo-7-[5-[(2,2,2-trifluoro-1-méthyl-éthyl)amino]-1,3,4-oxadiazol-2-yl]-2,3-dihydro-1lambda6,5-benzothiazépin-4-one ;
la (3R)-3-amino-7-[5-(1-amino-2,2,2-trifluoro-1-méthyl-éthyl)-1,2,4-oxadiazol-3-yl]-5-[(4-chlorophényl)méthyl]-8-fluoro-1,1-dioxo-2,3-dihydro-1lambda6,5-benzothiazépin-4-one ;
le 3-[5-[(3R)-3-amino-5-[(4-chlorophényl)méthyl]-8-fluoro-1,1,4-trioxo-2,3-dihydro-1lambda6,5-benzothiazépin-7-yl]tétrazol-2-yl]pyrrolidine-1-carboxylate de méthyle
la (3R)-3-amino-5-[(4-chlorophényl)méthyl]-7-[5-[(3,3-difluorocyclobutyl)amino]-1,2,4-oxadiazol-3-yl]-8-fluoro-1,1-dioxo-2,3-dihydro-1lambda6,5-benzothiazépin-4-one
la (3R)-3-amino-7-(5-tert-butyl-1,3,4-oxadiazol-2-yl)-5-[[4-(4-cyclopropylpipérazin-1-yl)phényl]méthyl]-1,1-dioxo-2,3-dihydro-1lambda6,5-benzothiazépin-4-one
la (3R)-3-amino-7-(5-tert-butyl-1,3,4-oxadiazol-2-yl)-5-[[4-(4-méthoxy-1-pipéridyl)phényl]méthyl]-1,1-dioxo-2,3-dihydro-1lambda6,5-benzothiazépin-4-one
la (3R)-3-amino-7-(5-tert-butyl-1,3,4-oxadiazol-2-yl)-5-[[4-(cyclopropylméthoxy)phényl]méthyl]-1,1-dioxo-2,3-dihydro-1lambda6,5-benzothiazépin-4-one
la (3R)-3-amino-7-(5-tert-butyl-1,3,4-oxadiazol-2-yl)-5-[[4-(2,2-difluoroéthoxy)phényl]méthyl]-1,1-dioxo-2,3-dihydro-1lambda6,5-benzothiazépin-4-one
la (3R)-3-amino-7-(5-tert-butyl-1,3,4-oxadiazol-2-yl)-5-[(4-but-2-ynoxyphényl)méthyl]-1,1-dioxo-2,3-dihydro-1lambda6,5-benzothiazépin-4-one
la (3R)-3-amino-7-[5-(1-amino-2,2,2-trifluoro-1-méthyl-éthyl)-1,3,4-oxadiazol-2-yl]-5-[(4-chlorophényl)méthyl]-1,1-dioxo-2,3-dihydro-1lambda6,5-benzothiazépin-4-one ;
la (3R)-3-amino-7-(5-tert-butyl-1,3,4-oxadiazol-2-yl)-1,1-dioxo-5-[[4-(2,2,2-trifluoroéthoxy)phényl]méthyl]-2,3-dihydro-1lambda6,5-benzothiazépin-4-one
la (3R)-3-amino-7-(5-tert-butyl-1,3,4-oxadiazol-2-yl)-5-[(4-morpholinophényl)méthyl]-1,1-dioxo-2,3-dihydro-1lambda6,5-benzothiazépin-4-one
la (3R)-3-amino-5-[(4-chlorophényl)méthyl]-8-fluoro-1,1-dioxo-7-[5-[3-(trifluorométhyl)pipérazin-1-yl]-1,2,4-oxadiazol-3-yl]-2,3-dihydro-1lambda6,5-benzothiazépin-4-one ; acide 2,2,2-trifluoroacétique
la (3R)-3-amino-1,1-dioxo-7-[5-(1,1,2,2,2-pentafluoroéthyl)-1,3,4-oxadiazol-2-yl]-5-[[4-(trifluorométhoxy)phényl]méthyl]-2,3-dihydro-1lambda6,5-benzothiazépin-4-one ;
la (3R)-3-amino-5-[(4-chlorophényl)méthyl]-7-[5-[(3,3-difluorocyclohexyl)amino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[(3,3-difluorocyclobutyl)amino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[(3,3-difluoro-1-méthyl-cyclobutyl)amino]-1,3,4-oxadiazol-2-yl]-8-fluoro-1,1-dioxo-2,3-dihydro-1lambda6,5-benzothiazépin-4-one ;
le 2-[4-[[(3R)-3-amino-7-(5-tert-butyl-1,3,4-oxadiazol-2-yl)-1,1,4-trioxo-2,3-dihydro-1lambda6,5-benzothiazépin-5-yl]méthyl]phényl]-2-méthyl-propanenitrile
la (3R)-3-amino-5-[(4-chlorophényl)méthyl]-7-[5-(4,4-difluoro-1-pipéridyl)-1,2,4-oxadiazol-3-yl]-8-fluoro-1,1-dioxo-2,3-dihydro-1lambda6,5-benzothiazépin-4-one ;
la (3R)-3-amino-5-[(4-chlorophényl)méthyl]-8-fluoro-7-(5-morpholino-1,2,4-oxadiazol-3-yl)-1,1-dioxo-2,3-dihydro-1lambda6,5-benzothiazépin-4-one ;
la (3R)-3-amino-5-[(4-chlorophényl)méthyl]-7-[5-(cyclobutylamino)-1,2,4-oxadiazol-3-yl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(4-oxa-7-azaspiro[2.5]octan-7-yl)-1,2,4-oxadiazol-3-yl]-1,1-dioxo-2,3-dihydro-1lambda6,5-benzothiazépin-4-one ;
la (3R)-7-[2-(1-acétyl-3-pipéridyl)tétrazol-5-yl]-3-amino-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[2-(1-méthyl-3-pipéridyl)tétrazol-5-yl]-1,1-dioxo-2,3-dihydro-1lambda6,5-benzothiazépin-4-one ;
la (3R)-3-amino-7-(3-tert-butyl-1,2,4-triazol-1-yl)-5-[(4-chlorophényl)méthyl]-8-fluoro-1,1-dioxo-2,3-dihydro-1lambda6,5-benzothiazépin-4-one ;
la (3R)-3-amino-7-[5-[1-méthyl-1-(méthylamino)éthyl]-1,3,4-oxadiazol-2-yl]-1,1-dioxo-5-[[4-(trifluorométhoxy)phényl]méthyl]-2,3-dihydro-1lambda6,5-benzothiazépin-4-one
la (3R)-3-amino-5-[(4-chlorophényl)méthyl]-7-[5-[3-(diméthylamino)-1,1-diméthyl-propyl]-1,3,4-oxadiazol-2-yl]-8-fluoro-1,1-dioxo-2,3-dihydro-1lambda6,5-benzothiazépin-4-one ;
le 3-[(3R)-3-amino-1,1,4-trioxo-5-[[4-(trifluorométhoxy)phényl]méthyl]-2,3-dihydro-1lambda6,5-benzothiazépin-7-yl]-N-tert-butyl-1,2,4-oxadiazole-5-carboxamide
le 3-[(3R)-3-amino-8-fluoro-1,1,4-trioxo-5-[[4-(trifluorométhoxy)phényl]méthyl]-2,3-dihydro-1lambda6,5-benzothiazépin-7-yl]-N-tert-butyl-1,2,4-oxadiazole-5-carboxamide
le 3-[(3R)-3-amino-5-[(4-chlorophényl)méthyl]-8-fluoro-1,1,4-trioxo-2,3-dihydro-1lambda6,5-benzothiazépin-7-yl]-N-tert-butyl-1,2,4-oxadiazole-5-carboxamide
le 3-[(3R)-3-amino-5-[(4-chlorophényl)méthyl]-1,1,4-trioxo-2,3-dihydro-1lambda6,5-benzothiazépin-7-yl]-N-tert-butyl-1,2,4-oxadiazole-5-carboxamide
la (3R)-3-amino-5-[(4-chlorophényl)méthyl]-7-[5-[(diméthylamino)méthyl]-1,2,4-oxadiazol-3-yl]-8-fluoro-1,1-dioxo-2,3-dihydro-1lambda6,5-benzothiazépin-4-one
la (3R)-3-amino-7-[5-(1-aminocyclopentyl)-1,3,4-oxadiazol-2-yl]-1,1-dioxo-5-[[4-(trifluorométhoxy)phényl]méthyl]-2,3-dihydro-1lambda6,5-benzothiazépin-4-one
la (3R)-3-amino-5-[(4-chlorophényl)méthyl]-7-[5-(3,3-difluorocyclohexyl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-[cyclopropyl(méthyl)amino]-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-[méthyl(2,2,2-trifluoroéthyl)amino]-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-[[3,3-difluoro-1-(méthoxyméthyl)cyclobutyl]amino]-1,3,4-oxadiazol-2-yl]-1,1-dioxo-2,3-dihydro-1lambda6,5-benzothiazépin-4-one ;
la (3R)-3-amino-7-[5-(4-aminotétrahydropyran-4-yl)-1,3,4-oxadiazol-2-yl]-1,1-dioxo-5-[[4-(trifluorométhoxy)phényl]méthyl]-2,3-dihydro-1lambda6,5-benzothiazépin-4-one
la (3R)-3-amino-7-[5-(1-aminocyclohexyl)-1,3,4-oxadiazol-2-yl]-1,1-dioxo-5-[[4-(trifluorométhoxy)phényl]méthyl]-2,3-dihydro-1lambda6,5-benzothiazépin-4-one
la (3R)-3-amino-5-[(4-chlorophényl)méthyl]-8-fluoro-7-[5-(1-hydroxy-1-méthyl-éthyl)-1,3,4-oxadiazol-2-yl]-1,1-dioxo-2,3-dihydro-1lambda6,5-benzothiazépin-4-one ; acide 2,2,2-trifluoroacétique
la (3R)-3-amino-7-(2-tert-butyltétrazol-5-yl)-1,1-dioxo-5-[[4-(trifluorométhoxy)phényl]méthyl]-2,3-dihydro-1lambda6,5-benzothiazépin-4-one
la (3R)-3-amino-7-(2-tert-butyltétrazol-5-yl)-8-fluoro-1,1-dioxo-5-[[4-(trifluorométhoxy)phényl]méthyl]-2,3-dihydro-1lambda6,5-benzothiazépin-4-one
la (3R)-3-amino-5-[(4-chlorophényl)méthyl]-8-fluoro-7-[2-(1-méthylsulfonyl-3-pipéridyl)tétrazol-5-yl]-1,1-dioxo-2,3-dihydro-1lambda6,5-benzothiazépin-4-one ;
la (3R)-3-amino-5-[(4-chlorophényl)méthyl]-7-[5-[(3,3-difluoro-1-méthyl-cyclobutyl)amino]-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-[(3,3-difluorocyclobutyl)amino]-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-[(3,3-difluorocyclohexyl)amino]-1,3,4-oxadiazol-2-yl]-1,1-dioxo-2,3-dihydro-1lambda6,5-benzothiazépin-4-one ;
la (3R)-3-amino-7-(4-tert-butylimidazol-1-yl)-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-7-[5-(5-oxa-2-azaspiro[3.4]octan-2-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(2-cyclopropyltétrahydrofuran-2-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(2-cyclopropyltétrahydrofuran-2-yl)-1,3,4-oxadiazol-2-yl]-1,1-dioxo-2,3-dihydro-1lambda6,5-benzothiazépin-4-one
la (3R)-3-amino-7-(2-tert-butyltétrazol-5-yl)-5-[(4-chlorophényl)méthyl]-1,1-dioxo-2,3-dihydro-1lambda6,5-benzothiazépin-4-one
la (3R)-7-[5-(3-acétyl-3-azabicyclo[3.1.0]hexan-1-yl)-1,3,4-oxadiazol-2-yl]-3-amino-5-[(4-chlorophényl)méthyl]-8-fluoro-1,1-dioxo-2,3-dihydro-1lambda6,5-benzothiazépin-4-one
le 3-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]azétidine-1-carboxylate de méthyle ; acide 2,2,2-trifluoroacétique
la (3R)-3-amino-5-[(4-chlorophényl)méthyl]-7-[5-(2,2-difluoromorpholin-4-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-1,1-dioxo-7-[5-(1,2,2,2-tétrafluoroéthyl)-1,3,4-oxadiazol-2-yl]-2,3-dihydro-1lambda6,5-benzothiazépin-4-one ;
la (3R)-3-amino-5-[(4-chlorophényl)méthyl]-8-fluoro-7-[5-(3-méthylsulfonyl-3-azabicyclo[3.1.0]hexan-1-yl)-1,3,4-oxadiazol-2-yl]-1,1-dioxo-2,3-dihydro-1lambda6,5-benzothiazépin-4-one
le 1-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-3-azabicyclo[3.1.0]hexane-3-carboxylate de méthyle
la (3R)-3-amino-5-[(4-chlorophényl)méthyl]-8-fluoro-7-[2-(1-méthylsulfonylpyrrolidin-3-yl)tétrazol-5-yl]-1,1-dioxo-2,3-dihydro-1lambda6,5-benzothiazépin-4-one ;
la (3R)-3-amino-5-[(4-chlorophényl)méthyl]-7-[5-(2-hydroxyspiro[3.3]heptan-6-yl)-1,3,4-oxadiazol-2-yl]-1,1-dioxo-2,3-dihydro-1lambda6,5-benzothiazépin-4-one
la (3R)-3-amino-7-[5-[3-(chlorométhyl)-3-(hydroxyméthyl)azétidin-1-yl]-1,3,4-oxadiazol-2-yl]-5-[(4-chlorophényl)méthyl]-1,1-dioxo-2,3-dihydro-1lambda6,5-benzothiazépin-4-one ;
le 2-[5-[(3R)-3-amino-5-[(4-chlorophényl)méthyl]-1,1,4-trioxo-2,3-dihydro-1lambda6,5-benzothiazépin-7-yl]-1,3,4-oxadiazol-2-yl]-2-méthoxy-acétonitrile ;
la (3R)-3-amino-1,1-dioxo-7-[5-(2,2,2-trifluoroéthyl)-1,3,4-oxadiazol-2-yl]-5-[[4-(trifluorométhoxy)phényl]méthyl]-2,3-dihydro-1lambda6,5-benzothiazépin-4-one ;
la (3R)-3-amino-5-[(4-chlorophényl)méthyl]-8-fluoro-7-[2-(1-méthylsulfonyl-4-pipéridyl)tétrazol-5-yl]-1,1-dioxo-2,3-dihydro-1lambda6,5-benzothiazépin-4-one ;
la (3R)-3-amino-7-(5-tert-butyl-1,3,4-oxadiazol-2-yl)-1,1-dioxo-5-[[4-(1,1,2,2-tétrafluoroéthoxy)phényl]méthyl]-2,3-dihydro-1lambda6,5-benzothiazépin-4-one
la (3R)-3-amino-5-[(4-chlorophényl)méthyl]-8-fluoro-7-[5-(5-oxa-2-azaspiro[3.4]octan-2-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(6-oxa-2-azaspiro[3.5]nonan-2-yl)-1,3,4-oxadiazol-2-yl]-1,1-dioxo-2,3-dihydro-1lambda6,5-benzothiazépin-4-one ;
la (3R)-3-amino-7-(5-tert-butyl-1,3,4-oxadiazol-2-yl)-5-[[4-(cyclopentoxy)phényl]méthyl]-1,1-dioxo-2,3-dihydro-1lambda6,5-benzothiazépin-4-one
la (3R)-3-amino-5-[(4-chlorophényl)méthyl]-1,1-dioxo-7-[5-(1,1,2,2,2-pentafluoroéthyl)-1,3,4-oxadiazol-2-yl]-2,3-dihydro-1lambda6,5-benzothiazépin-4-one ;
le 7-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-5-azaspiro[2.4]heptane-5-carboxylate de méthyle ;
le 2-[5-[(3R)-3-amino-8-fluoro-1,1,4-trioxo-5-[[4-(trifluorométhoxy)phényl]méthyl]-2,3-dihydro-1lambda6,5-benzothiazépin-7-yl]-1,3,4-oxadiazol-2-yl]-2-méthyl-propanenitrile ;
la (3R)-3-amino-1,1-dioxo-7-[5-(1,2,2,2-tétrafluoro-1-méthoxy-éthyl)-1,3,4-oxadiazol-2-yl]-5-[[4-(trifluorométhoxy)phényl]méthyl]-2,3-dihydro-1lambda6,5-benzothiazépin-4-one ;
la (3R)-3-amino-5-[(4-chlorophényl)méthyl]-1,1-dioxo-7-[5-(1,2,2,2-tétrafluoro-1-méthoxy-éthyl)-1,3,4-oxadiazol-2-yl]-2,3-dihydro-1lambdab,5-benzothiazépin-4-one ;
la (3R)-3-amino-7-[5-[(1R,5S)-8-azabicyclo[3.2.1]octane-8-carbonyl]-1,3,4-oxadiazol-2-yl]-5-[(4-chlorophényl)méthyl]-8-fluoro-1,1-dioxo-2,3-dihydro-1lambda6,5-benzothiazépin-4-one
la (3R)-3-amino-5-[(4-chlorophényl)méthyl]-8-fluoro-7-[5-(5-méthylsulfonyl-5-azaspiro[2.4]heptan-7-yl)-1,3,4-oxadiazol-2-yl]-1,1-dioxo-2,3-dihydro-1lambda6,5-benzothiazépin-4-one ;
la (3R)-7-[5-(1-acétyl-4-pipéridyl)-1,3,4-oxadiazol-2-yl]-3-amino-5-[(4-chlorophényl)méthyl]-8-fluoro-1,1-dioxo-2,3-dihydro-1lambda6,5-benzothiazépin-4-one
la (3R)-3-amino-5-[(4-chlorophényl)méthyl]-8-fluoro-7-(2-méthyltétrazol-5-yl)-1,1-dioxo-2,3-dihydro-1lambda6,5-benzothiazépin-4-one ;
la (3R)-3-amino-5-[(4-chlorophényl)méthyl]-8-fluoro-7-[2-(1-méthylpyrrolidin-3-yl)tétrazol-5-yl]-1,1-dioxo-2,3-dihydro-1lambda6,5-benzothiazépin-4-one ;
la (3R)-7-[2-(1-acétyl-4-pipéridyl)tétrazol-5-yl]-3-amino-5-[(4-chlorophényl)méthyl]-8-fluoro-1,1-dioxo-2,3-dihydro-1lambda6,5-benzothiazépin-4-one ;
la (3R)-7-[5-(3-acétyl-3-azabicyclo[4.1.0]heptan-1-yl)-1,3,4-oxadiazol-2-yl]-3-amino-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(3-méthylsulfonyl-3-azabicyclo[4.1.0]heptan-1-yl)-1,3,4-oxadiazol-2-yl]-1,1-dioxo-2,3-dihydro-1lambda6,5-benzothiazépin-4-one ;
le 1-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-3-azabicyclo[4.1.0]heptane-3-carboxylate de méthyle ;
la (3R)-3-amino-5-[(4-chlorophényl)méthyl]-7-[5-(6,6-difluoro-2-azaspiro[3.3]heptan-2-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(isopropylamino)-1,3,4-oxadiazol-2-yl]-1,1-dioxo-2,3-dihydro-1lambda6,5-benzothiazépin-4-one ;
la (3R)-3-amino-7-[5-(2-azaspiro[3.3]heptan-2-yl)-1,3,4-oxadiazol-2-yl]-5-[(4-chlorophényl)méthyl]-1,1-dioxo-2,3-dihydro-1lambda6,5-benzothiazépin-4-one ;
le 5-[[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]amino]-3,3-difluoro-pipéridine-1-carboxylate de méthyle ;
la (3R)-3-amino-5-[(4-chlorophényl)méthyl]-7-[5-(4,4-difluoro-1-pipéridyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(2,2-diméthylmorpholin-4-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(3-fluoro-1-méthyl-3-pipéridyl)-1,3,4-oxadiazol-2-yl]-1,1-dioxo-2,3-dihydro-1lambda6,5-benzothiazépin-4-one ;
le 4-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]pipéridine-1-carboxylate de méthyle
la (3R)-3-amino-7-[5-(1-amino-2,2,2-trifluoro-éthyl)-1,3,4-oxadiazol-2-yl]-8-fluoro-1,1-dioxo-5-[[4-(trifluorométhoxy)phényl]méthyl]-2,3-dihydro-1lambda6,5-benzothiazépin-4-one
le 3-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]pyrrolidine-l-carboxylate de méthyle
la (3R)-3-amino-5-[(4-chlorophényl)méthyl]-7-[5-(5,5-difluoro-1-méthyl-3-pipéridyl)-1,3,4-oxadiazol-2-yl]-8-méthyl-1,1-dioxo-2,3-dihydro-1lambda6,5-benzothiazépin-4-one
la (3R)-3-amino-5-[(4-chlorophényl)méthyl]-7-[5-(5,5-difluoro-1-méthyl-3-pipéridyl)-1,3,4-oxadiazol-2-yl]-1,1-dioxo-2,3-dihydro-1lambda6,5-benzothiazépin-4-one
le 2-[5-[(3R)-3-amino-5-[(4-chlorophényl)méthyl]-8-méthyl-1,1,4-trioxo-2,3-dihydro-1lambda6,5-benzothiazépin-7-yl]-1,3,4-oxadiazol-2-yl]-2-méthyl-propanenitrile ;
la (3R)-3-amino-5-[(4-chlorophényl)méthyl]-7-[5-[(5,5-difluoro-3-pipéridyl)amino]-1,3,4-oxadiazol-2-yl]-8-fluoro-1,1-dioxo-2,3-dihydro-1lambda6,5-benzothiazépin-4-one ;
la (3R)-3-amino-7-[5-(3-azabicyclo[4.1.0]heptan-l-yl)-1,3,4-oxadiazol-2-yl]-5-[(4-chlorophényl)méthyl]-8-fluoro-1,1-dioxo-2,3-dihydro-1lambda6,5-benzothiazépin-4-one
la (3R)-7-[5-(5-acétyl-5-azaspiro[2.4]heptan-7-yl)-1,3,4-oxadiazol-2-yl]-3-amino-5-[(4-chlorophényl)méthyl]-8-fluoro-1,1-dioxo-2,3-dihydro-1lambda6,5-benzothiazépin-4-one
la (3R)-3-amino-5-[(4-chlorophényl)méthyl]-7-[5-(3,3-difluoropipéridine-1-carbonyl)-1,3,4-oxadiazol-2-yl]-8-fluoro-1,1-dioxo-2,3-dihydro-1lambda6,5-benzothiazépin-4-one
le 2-[5-[(3R)-3-amino-5-[(4-chlorophényl)méthyl]-8-méthyl-1,1,4-trioxo-2,3-dihydro-1lambda6,5-benzothiazépin-7-yl]-1,3,4-oxadiazol-2-yl]propanenitrile ;
la (3R)-3-amino-5-[(4-chlorophényl)méthyl]-8-fluoro-7-[5-(3-méthylsulfonyl-3-azabicyclo[3.1.1]heptan-l-yl)-1,3,4-oxadiazol-2-yl]-1,1-dioxo-2,3-dihydro-1lambda6,5-benzothiazépin-4-one
le 1-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-3-azabicyclo[3.1.1]heptane-3-carboxylate de méthyle ;
le 1-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-3-azabicyclo[3.1.1]heptane-3-carboxylate de méthyle
le 2-[5-[(3R)-3-amino-5-[(4-chlorophényl)méthyl]-1,1,4-trioxo-2,3-dihydro-1lambda6,5-benzothiazépin-7-yl]-1,3,4-oxadiazol-2-yl]-2-méthyl-propanenitrile ;
la (3R)-7-[5-(3-acétyl-3-azabicyclo[3.1.1]heptan-l-yl)-1,3,4-oxadiazol-2-yl]-3-amino-5-[(4-chlorophényl)méthyl]-8-fluoro-1,1-dioxo-2,3-dihydro-1lambda6,5-benzothiazépin-4-one
la (3R)-3-amino-7-[5-(1-amino-2,2,2-trifluoro-éthyl)-1,3,4-oxadiazol-2-yl]-5-[(4-chlorophényl)méthyl]-8-fluoro-1,1-dioxo-2,3-dihydro-1lambda6,5-benzothiazépin-4-one ; acide 2,2,2-trifluoroacétique
la (3R)-3-amino-7-[5-(1-amino-2,2,2-trifluoro-éthyl)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-7-[5-[4-(diméthylamino)-1,1-diméthyl-butyl]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[(3,3-difluorocyclopentyl)amino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[(4,4-difluorocyclohexyl)amino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-(1,3-diméthylazétidin-3-yl)-1,3,4-oxadiazol-2-yl]-8-fluoro-1,1-dioxo-2,3-dihydro-1lambda6,5-benzothiazépin-4-one ;
la (3R)-3-amino-7-[5-(tert-butylamino)-1,3,4-oxadiazol-2-yl]-5-[(4-chlorophényl)méthyl]-8-fluoro-1,1-dioxo-2,3-dihydro-1lambda6,5-benzothiazépin-4-one ;
le 1-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]cyclopropanecarbonitrile ;
le 2-[5-[(3R)-3-amino-5-[(4-chlorophényl)méthyl]-1,1,4-trioxo-2,3-dihydro-1lambda6,5-benzothiazépin-7-yl]-1,3,4-oxadiazol-2-yl]propanenitrile ;
le 3-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]pipéridine-1-carboxylate de méthyle
la (3R)-3-amino-5-[(4-chlorophényl)méthyl]-7-[5-(3,3-difluoropyrrolidine-1-carbonyl)-1,3,4-oxadiazol-2-yl]-8-fluoro-1,1-dioxo-2,3-dihydro-1lambda6,5-benzothiazépin-4-one
la (3R)-3-amino-7-[5-(2-aminospiro[3.3]heptan-6-yl)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(2-oxa-7-azaspiro[3.5]nonan-7-yl)-1,3,4-oxadiazol-2-yl]-1,1-dioxo-2,3-dihydro-1lambda6,5-benzothiazépin-4-one
le 4-[[(3R)-3-amino-8-fluoro-7-[5-(2-hydroxy-1,1-diméthyl-éthyl)-1,3,4-oxadiazol-2-yl]-1,1,4-trioxo-2,3-dihydro-1lambda6,5-benzothiazépin-5-yl]méthyl]benzonitrile ;
la (3R)-3-amino-5-[(4-chlorophényl)méthyl]-7-[5-(5,5-difluoro-l-méthyl-3-pipéridyl)-1,2,4-oxadiazol-3-yl]-8-fluoro-1,1-dioxo-2,3-dihydro-1lambda6,5-benzothiazépin-4-one
la (3R)-3-amino-7-[5-(5-amino-3,3-difluoro-1-pipéridyl)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-7-[5-[1-(chlorométhyl)-2-hydroxy-l-méthyl-éthyl]-1,3,4-oxadiazol-2-yl]-5-[(4-chlorophényl)méthyl]-8-fluoro-1,1-dioxo-2,3-dihydro-1lambda6,5-benzothiazépin-4-one ;
le 5-[(3R)-3-amino-5-[(4-chlorophényl)méthyl]-8-fluoro-1,1,4-trioxo-2,3-dihydro-1lambda6,5-benzothiazépin-7-yl]-N-isopropyl-N-méthyl-1,3,4-oxadiazole-2-carboxamide
la (3R)-3-amino-5-[(4-chlorophényl)méthyl]-7-[5-(1-cyclopropyl-5,5-difluoro-3-pipéridyl)-1,3,4-oxadiazol-2-yl]-8-fluoro-1,1-dioxo-2,3-dihydro-1lambda6,5-benzothiazépin-4-one ;
la (3R)-3-amino-7-[5-[4-(chlorométhyl)-4-(hydroxyméthyl)-1-pipéridyl]-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-7-[5-(4,4-difluoro-1-méthyl-3-pipéridyl)-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(2-méthylsulfonyléthyl)-1,3,4-oxadiazol-2-yl]-1,1-dioxo-2,3-dihydro-1lambda6,5-benzothiazépin-4-one ;
le 5-[(3R)-3-amino-5-[(4-chlorophényl)méthyl]-8-fluoro-1,1,4-trioxo-2,3-dihydro-1lambda6,5-benzothiazépin-7-yl]-N-tert-butyl-1,3,4-oxadiazole-2-carboxamide
la (3R)-3-amino-5-[(4-chlorophényl)méthyl]-8-fluoro-7-[5-(isopropoxyméthyl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-[5,5-difluoro-1-(2-méthoxyéthyl)-3-pipéridyl]-1,3,4-oxadiazol-2-yl]-8-fluoro-1,1-dioxo-2,3-dihydro-1lambda6,5-benzothiazépin-4-one
la 5-[(3R)-3-amino-5-[(4-chlorophényl)méthyl]-8-fluoro-1,1,4-trioxo-2,3-dihydro-1lambda6,5-benzothiazépin-7-yl]-3-tert-butyl-1,3,4-oxadiazol-2-one ;
la (3R)-3-amino-5-[(4-chlorophényl)méthyl]-8-fluoro-7-[5-(2-méthyltétrahydrofuran-2-yl)-1,3,4-oxadiazol-2-yl]-1,1-dioxo-2,3-dihydro-1lambda6,5-benzothiazépin-4-one ;
la (3R)-3-amino-7-[5-(4-amino-1,1-diméthyl-butyl)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-7-[5-(1,3-diméthyl-3-pipéridyl)-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-[(1-éthyl-3-pipéridyl)amino]-1,3,4-oxadiazol-2-yl]-8-fluoro-1,1-dioxo-2,3-dihydro-1lambda6,5-benzothiazépin-4-one
la (3R)-3-amino-7-[5-(3-amino-4,4-difluoro-1-pipéridyl)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-7-[5-[2-(1,1-dioxo-1,4-thiazinan-4-yl)-1,1-diméthyl-éthyl]-1,3,4-oxadiazol-2-yl]-8-fluoro-1,1-dioxo-2,3-dihydro-1lambda6,5-benzothiazépin-4-one
le 5-[(3R)-3-amino-5-[(4-chlorophényl)méthyl]-8-fluoro-1,1,4-trioxo-2,3-dihydro-1lambda6,5-benzothiazépin-7-yl]-N,N-diméthyl-1,3,4-oxadiazole-2-carboxamide
la (3R)-3-amino-5-[(4-chlorophényl)méthyl]-7-[5-(1-éthyl-5,5-difluoro-3-pipéridyl)-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-8-fluoro-7-[5-(1-méthylpyrrolidin-3-yl)-1,3,4-oxadiazol-2-yl]-1,1-dioxo-2,3-dihydro-1lambda6,5-benzothiazépin-4-one
le 5-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-3,3-difluoro-pipéridine-1-carboxylate de méthyle
la (3R)-3-amino-7-[5-(2-azaspiro[3.3]heptan-2-yl)-1,3,4-oxadiazol-2-yl]-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(isopropylamino)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(4,4-difluoro-3-pipéridyl)-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-[5-[(1-cyclopropyl-4-pipéridyl)amino]-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-7-[5-(2,2-diméthylmorpholin-4-yl)-1,3,4-oxadiazol-2-yl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(4-oxa-7-azaspiro[2.5]octan-7-yl)-1,3,4-oxadiazol-2-yl]-1,1-dioxo-2,3-dihydro-1lambda6,5-benzothiazépin-4-one
la (3R)-3-amino-7-(5-tert-butyl-1,3,4-oxadiazol-2-yl)-5-[[6-(cyclopentoxy)-3-pyridyl]méthyl]-8-fluoro-1,1-dioxo-2,3-dihydro-1lambda6,5-benzothiazépin-4-one
la (3R)-3-amino-5-[(4-chlorophényl)méthyl]-7-[5-[1,1-diméthyl-2-(2-oxo-1-pipéridyl)éthyl]-1,3,4-oxadiazol-2-yl]-8-fluoro-1,1-dioxo-2,3-dihydro-1lambda6,5-benzothiazépin-4-one
le 2-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-2-méthyl-propanenitrile ;
le 2-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]propanenitrile ;
la (3R)-3-amino-5-[(4-chlorophényl)méthyl]-8-fluoro-7-[5-(3-méthylazétidin-3-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-7-[5-(5,5-difluoro-l-méthylsulfonyl-3-pipéridyl)-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-[5-[5,5-difluoro-1-(2-hydroxyéthyl)-3-pipéridyl]-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-8-fluoro-7-[5-[1-(hydroxyméthyl)cyclopropyl]-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-(cyclobutylamino)-1,3,4-oxadiazol-2-yl]-8-fluoro-1,1-dioxo-2,3-dihydro-1lambda6,5-benzothiazépin-4-one ;
la (3R)-7-[5-(1-acétyl-5,5-difluoro-3-pipéridyl)-1,3,4-oxadiazol-2-yl]-3-amino-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-7-[5-(5,5-difluoro-3-pipéridyl)-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-(3-éthyl-1,2,4-triazol-1-yl)-8-fluoro-1,1-dioxo-2,3-dihydro-1lambda6,5-benzothiazépin-4-one
la (3R)-7-[5-(4-acétylpipérazin-1-yl)-1,3,4-oxadiazol-2-yl]-3-amino-5-[(4-chlorophényl)méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-(5-isobutyl-1,3,4-oxadiazol-2-yl)-1,1-dioxo-2,3-dihydro-1lambda6,5-benzothiazépin-4-one
la (3R)-3-amino-5-[(4-chlorophényl)méthyl]-8-fluoro-7-[5-(4-méthylpipérazin-1-yl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-7-[5-[2-[2-hydroxyéthyl(méthyl)amino]-1,1-diméthyl-éthyl]-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-[1,1-diméthyl-2-(2-oxopyrrolidin-1-yl)éthyl]-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-8-fluoro-7-[5-(1-méthyl-3-pipéridyl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-[1,1-diméthyl-2-(4-méthylpipérazin-1-yl)éthyl]-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-[5-[1,1-diméthyl-2-(tétrahydrofuran-3-ylamino)éthyl]-1,3,4-oxadiazol-2-yl]-8-fluoro-1,1-dioxo-2,3-dihydro-1lambda6,5-benzothiazépin-4-one
la (3R)-3-amino-7-(5-tert-butyl-1,3,4-oxadiazol-2-yl)-8-fluoro-5-[(5-isopropoxy-2-pyridyl)méthyl]-1,1-dioxo-2,3-dihydro-1lambda6,5-benzothiazépin-4-one ;
la (3R)-3-amino-5-[(4-chlorophényl)méthyl]-7-[5-[1,1-diméthyl-2-(oxétan-3-ylamino)éthyl]-1,3,4-oxadiazol-2-yl]-8-fluoro-1,1-dioxo-2,3-dihydro-1lambda6,5-benzothiazépin-4-one
la (3R)-3-amino-7-(5-tert-butyl-1,3,4-oxadiazol-2-yl)-5-[(3,4-difluorophényl)méthyl]-8-fluoro-1,1-dioxo-2,3-dihydro-1lambda6,5-benzothiazépin-4-one ;
la (3R)-3-amino-7-(5-tert-butyl-1,3,4-oxadiazol-2-yl)-5-[(4-chloro-3-fluoro-phényl)méthyl]-8-fluoro-1,1-dioxo-2,3-dihydro-1lambda6,5-benzothiazépin-4-one ;
la (3R)-3-amino-5-[(4-chlorophényl)méthyl]-7-[5-(4,4-difluoro-1-pipéridyl)-1,3,4-oxadiazol-2-yl]-8-fluoro-1,1-dioxo-2,3-dihydro-1lambda6,5-benzothiazépin-4-one ; 1,1,1,3,3,3-hexafluoropropan-2-ol ;
la (3R)-3-amino-5-[(4-chlorophényl)méthyl]-7-[5-(1,1-diméthyl-2-morpholino-éthyl)-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-[5-[2-(diméthylamino)-1,1-diméthyl-éthyl]-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-[5-(6,6-difluoro-2-azaspiro[3.3]heptan-2-yl)-1,3,4-oxadiazol-2-yl]-8-fluoro-1,1-dioxo-2,3-dihydro-1lambda6,5-benzothiazépin-4-one ; 1,1,1,3,3,3-hexafluoropropan-2-ol ;
la (3R)-3-amino-7-(5-tert-butyl-1,3,4-oxadiazol-2-yl)-8-fluoro-5-[(6-isopropoxy-3-pyridyl)méthyl]-1,1-dioxo-2,3-dihydro-1lambda6,5-benzothiazépin-4-one ;
la (3R)-3-amino-5-[(4-chlorophényl)méthyl]-7-[5-[2-(4,4-difluoro-1-pipéridyl)-1,1-diméthyl-éthyl]-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-8-fluoro-7-[2-(2-hydroxy-1,1-diméthyl-éthyl)tétrazol-5-yl]-1,1-dioxo-2,3-dihydro-1lambda6,5-benzothiazépin-4-one ;
la (3R)-3-amino-5-[(4-chlorophényl)méthyl]-8-fluoro-7-[2-(2-hydroxy-2-méthyl-propyl)tétrazol-5-yl]-1,1-dioxo-2,3-dihydro-1lambda6,5-benzothiazépin-4-one ;
la (3R)-3-amino-5-[(4-chlorophényl)méthyl]-7-[5-(cyclopropylamino)-1,3,4-oxadiazol-2-yl]-8-fluoro-1,1-dioxo-2,3-dihydro-1lambda6,5-benzothiazépin-4-one ; 1,1,1,3,3,3-hexafluoropropan-2-ol ;
la (3R)-3-amino-5-[(4-chlorophényl)méthyl]-7-[5-[1,1-diméthyl-2-(1-pipéridyl)éthyl]-1,3,4-oxadiazol-2-yl]-8-fluoro-1,1-dioxo-2,3-dihydro-1lambda6,5-benzothiazépin-4-one
la (3R)-3-amino-7-(5-tert-butyl-1,3,4-oxadiazol-2-yl)-5-[(4-chlorophényl)méthyl]-8-fluoro-1-oxo-2,3-dihydro-1lambda4,5-benzothiazépin-4-one ;
la (3R)-3-amino-7-(5-tert-butyl-1,3,4-oxadiazol-2-yl)-5-[(4-chlorophényl)méthyl]-8-fluoro-1-oxo-2,3-dihydro-1lambda4,5-benzothiazépin-4-one
la (3R)-3-amino-5-[(4-chlorophényl)méthyl]-7-[5-(2,2-difluorospiro[3.3]heptan-6-yl)-1,3,4-oxadiazol-2-yl]-8-fluoro-1,1-dioxo-2,3-dihydro-1lambda6,5-benzothiazépin-4-one ; 1,1,1,3,3,3-hexafluoropropan-2-ol ;
la (3R)-3-amino-5-[(4-chlorophényl)méthyl]-7-[5-(diméthylamino)-1,3,4-oxadiazol-2-yl]-8-fluoro-1,1-dioxo-2,3-dihydro-1lambda6,5-benzothiazépin-4-one ; 1,1,1,3,3,3-hexafluoropropan-2-ol ;
la (3R)-3-amino-5-[(4-chlorophényl)méthyl]-8-fluoro-7-[5-[(3-méthylazétidin-1-yl)méthyl]-1,3,4-oxadiazol-2-yl]-2,3-dihydro-1,5-benzothiazépin-4-one ; 1,1,1,3,3,3-hexafluoropropan-2-ol ;
le 4-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-4-méthyl-pipéridine-1-carboxylate de méthyle ;
la (3R)-3-amino-5-[(4-chlorophényl)méthyl]-8-fluoro-7-[5-(4-méthyl-1-méthylsulfonyl-4-pipéridyl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-(cyclohexylamino)-1,3,4-oxadiazol-2-yl]-8-fluoro-1,1-dioxo-2,3-dihydro-1lambda6,5-benzothiazépin-4-one ; 1,1,1,3,3,3-hexafluoropropan-2-ol ;
la (3R)-3-amino-5-[(4-chlorophényl)méthyl]-8-fluoro-7-[5-(1-méthylcyclopropyl)-1,3,4-oxadiazol-2-yl]-1,1-dioxo-2,3-dihydro-1lambda6,5-benzothiazépin-4-one ; 1,1,1,3,3,3-hexafluoropropan-2-ol ;
la (3R)-3-amino-5-[(4-chlorophényl)méthyl]-7-[5-[3-(difluorométhyl)azétidin-3-yl]-1,3,4-oxadiazol-2-yl]-8-fluoro-1,1-dioxo-2,3-dihydro-1lambda6,5-benzothiazépin-4-one ; 1,1,1,3,3,3-hexafluoropropan-2-ol ;
la (3R)-3-amino-7-[5-(2-amino-1,1-diméthyl-éthyl)-1,3,4-oxadiazol-2-yl]-5-[(4-chlorophényl)méthyl]-8-fluoro-1,1-dioxo-2,3-dihydro-1lambda6,5-benzothiazépin-4-one
la (3R)-3-amino-5-[(4-chlorophényl)méthyl]-8-fluoro-7-[5-(6-oxaspiro[3.4]octan-2-yl)-1,3,4-oxadiazol-2-yl]-1,1-dioxo-2,3-dihydro-1lambda6,5-benzothiazépin-4-one
la (3R)-3-amino-5-[(4-chlorophényl)méthyl]-7-[5-(5,5-difluoro-1-méthyl-3-pipéridyl)-1,3,4-oxadiazol-2-yl]-8-fluoro-1,1-dioxo-2,3-dihydro-1lambda6,5-benzothiazépin-4-one
la (3R)-3-amino-7-(5-tert-butyl-1,3,4-oxadiazol-2-yl)-8-fluoro-5-[(3-fluoro-4-méthylsulfonyl-phényl)méthyl]-1,1-dioxo-2,3-dihydro-1lambda6,5-benzothiazépin-4-one ;
la (3R)-3-amino-7-(5-tert-butyl-1,3,4-oxadiazol-2-yl)-5-[(4-éthyloxazol-2-yl)méthyl]-8-fluoro-1,1-dioxo-2,3-dihydro-1lambda6,5-benzothiazépin-4-one
la (3R)-3-amino-7-(5-tert-butyl-1,3,4-oxadiazol-2-yl)-8-fluoro-1-imino-1-oxo-5-[[4-(trifluorométhoxy)phényl]méthyl]-2,3-dihydro-1lambda6,5-benzothiazépin-4-one ;
la (3R)-3-amino-7-(5-tert-butyl-1,3,4-oxadiazol-2-yl)-8-fluoro-5-[(2-fluoro-4-méthylsulfonyl-phényl)méthyl]-1,1-dioxo-2,3-dihydro-1lambda6,5-benzothiazépin-4-one ; 1,1,1,3,3,3-hexafluoropropan-2-ol ;
le 2-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-N,N,2-triméthyl-propanamide ; 1,1,1,3,3,3-hexafluoropropan-2-ol ;
la (3R)-3-amino-5-[(4-chlorophényl)méthyl]-8-fluoro-7-[3-(2-hydroxy-1,1-diméthyl-éthyl)-1,2,4-oxadiazol-5-yl]-1,1-dioxo-2,3-dihydro-1lambda6,5-benzothiazépin-4-one ;
la (3R)-3-amino-7-(5-tert-butyl-1,3,4-oxadiazol-2-yl)-8-fluoro-5-[[4-(3-hydroxyoxétan-3-yl)phényl]méthyl]-1,1-dioxo-2,3-dihydro-1lambda6,5-benzothiazépin-4-one
la (3R)-3-amino-5-[(4-chlorophényl)méthyl]-8-fluoro-7-[5-(2-hydroxy-1,1-diméthyl-éthyl)-1,2,4-oxadiazol-3-yl]-1,1-dioxo-2,3-dihydro-1lambda6,5-benzothiazépin-4-one ; acide 2,2,2-trifluoroacétique
la (3R)-3-amino-5-[(4-chlorophényl)méthyl]-8-fluoro-7-[5-(2-hydroxy-1,1-diméthyl-éthyl)-1,2,4-oxadiazol-3-yl]-1,1-dioxo-2,3-dihydro-1lambda6,5-benzothiazépin-4-one
la (3R)-3-amino-7-(5-tert-butyl-1,3,4-oxadiazol-2-yl)-8-fluoro-1,1-dioxo-5-[(4-phénoxyphényl)méthyl]-2,3-dihydro-1lambda6,5-benzothiazépin-4-one ;
la (3R)-3-amino-7-(5-tert-butyl-1,3,4-oxadiazol-2-yl)-8-fluoro-1,1-dioxo-5-[[4-(1,1,2,2-tétrafluoroéthoxy)phényl]méthyl]-2,3-dihydro-1lambda6,5-benzothiazépin-4-one ;
la (3R)-3-amino-7-(5-tert-butyl-1,3,4-oxadiazol-2-yl)-8-fluoro-5-[[4-(2-hydroxyéthoxy)phényl]méthyl]-1,1-dioxo-2,3-dihydro-1lambda6,5-benzothiazépin-4-one ; acide 2,2,2-trifluoroacétique
la (3R)-3-amino-7-(5-tert-butyl-1,3,4-oxadiazol-2-yl)-5-[(4-chlorophényl)méthyl]-8-fluoro-9-méthyl-1,1-dioxo-2,3-dihydro-1lambda6,5-benzothiazépin-4-one
la (3R)-3-amino-7-(5-tert-butyl-1,3,4-oxadiazol-2-yl)-5-[[1-(cyclopropylméthyl)pyrazol-4-yl]méthyl]-8-fluoro-1,1-dioxo-2,3-dihydro-1lambda6,5-benzothiazépin-4-one ; acide 2,2,2-trifluoroacétique
la (3R)-7-[5-(1-acétyl-4-méthyl-4-pipéridyl)-1,3,4-oxadiazol-2-yl]-3-amino-5-[(4-chiorophényl)méthyl]-8-fluoro-1,1-dioxo-2,3-dihydro-1lambda6,5-benzothiazépin-4-one ;
la (3R)-3-amino-7-(5-tert-butyl-1,3,4-oxadiazol-2-yl)-8-fluoro-1-méthylimino-1-oxo-5-[[4-(trifluorométhoxy)phényl]méthyl]-2,3-dihydro-1lambda6,5-benzothiazépin-4-one ;
le 2-[5-[(3R)-3-amino-5-[(4-chlorophényl)méthyl]-8-fluoro-1,1,4-trioxo-2,3-dihydro-1lambda6,5-benzothiazépin-7-yl]-1,3,4-oxadiazol-2-yl]-N,2-diméthyl-propanamide ;
la (3R)-3-amino-7-(5-tert-butyl-1,3,4-oxadiazol-2-yl)-5-[(2-cyclopropylpyrimidin-5-yl)méthyl]-8-fluoro-1,1-dioxo-2,3-dihydro-1lambda6,5-benzothiazépin-4-one ; 1,1,1,3,3,3-hexafluoropropan-2-ol ;
la (3R)-3-amino-7-(5-tert-butyl-1,3,4-oxadiazol-2-yl)-5-[(6-chloro-3-pyridyl)méthyl]-8-fluoro-1,1-dioxo-2,3-dihydro-1lambda6,5-benzothiazépin-4-one ; 1,1,1,3,3,3-hexafluoropropan-2-ol ;
la (2R,3R)-3-amino-7-(5-tert-butyl-1,3,4-oxadiazol-2-yl)-5-[(4-chlorophényl)méthyl]-8-fluoro-2-méthyl-1,1-dioxo-2,3-dihydro-1lambda6,5-benzothiazépin-4-one ; 1,1,1,3,3,3-hexafluoropropan-2-ol ;
la (3R)-3-amino-7-(5-tert-butyl-1,3,4-oxadiazol-2-yl)-5-[[5-[(4-chlorophényl)méthyl]-3-pyridyl]méthyl]-8-fluoro-1,1-dioxo-2,3-dihydro-1lambda6,5-benzothiazépin-4-one ;
la (3R)-3-amino-5-[(4-chlorophényl)méthyl]-8-fluoro-7-[5-(4-méthyl-4-pipéridyl)-1,3,4-oxadiazol-2-yl]-1,1-dioxo-2,3-dihydro-1lambda6,5-benzothiazépin-4-one ;
la (3R)-3-amino-5-[(4-chlorophényl)méthyl]-8-fluoro-7-[5-(4-méthyltétrahydropyran-4-yl)-1,3,4-oxadiazol-2-yl]-1,1-dioxo-2,3-dihydro-1lambda6,5-benzothiazépin-4-one ; acide 2,2,2-trifluoroacétique
la (3R)-3-amino-7-(5-tert-butyl-1,3,4-oxadiazol-2-yl)-8-fluoro-l-oxo-5-[[4-(trifluorométhoxy)phényl]méthyl]-2,3-dihydro-1lambda4,5-benzothiazépin-4-one ;
la (3R)-3-amino-7-(5-tert-butyl-1,3,4-oxadiazol-2-yl)-8-fluoro-1-imino-1-oxo-5-[[4-(trifluorométhoxy)phényl]méthyl]-2,3-dihydro-1lambda6,5-benzothiazépin-4-one ;
la (3R)-3-amino-7-(5-tert-butyl-1,3,4-oxadiazol-2-yl)-8-chloro-5-[(4-chlorophényl)méthyl]-1,1-dioxo-2,3-dihydro-1lambda6,5-benzothiazépin-4-one ;
le 2-[4-[[(3R)-3-amino-7-(5-tert-butyl-1,3,4-oxadiazol-2-yl)-8-fluoro-1,1,4-trioxo-2,3-dihydro-1lambda6,5-benzothiazépin-5-yl]méthyl]phénoxy]acétamide ; 1,1,1,3,3,3-hexafluoropropan-2-ol ;
le N-[11-[4-[[(3R)-3-amino-7-(5-tert-butyl-1,3,4-oxadiazol-2-yl)-8-fluoro-1,1,4-trioxo-2,3-dihydro-1lambda6,5-benzothiazépin-5-yl]méthyl]phénoxy]undécyl]acétamide ;
le (2S)-N-[(3R)-7-(5-tert-butyl-1,3,4-oxadiazol-2-yl)-5-[(4-chlorophényl)méthyl]-8-fluoro-1,1,4-trioxo-2,3-dihydro-1lambda6,5-benzothiazépin-3-yl]-2-(méthylamino)propanamide
le (2S)-2-amino-N-[(3R)-7-(5-tert-butyl-1,3,4-oxadiazol-2-yl)-5-[(4-chlorophényl)méthyl]-8-fluoro-1,1,4-trioxo-2,3-dihydro-1lambda6,5-benzothiazépin-3-yl]butanamide
le (2S)-N-[(3R)-7-(5-tert-butyl-1,3,4-oxadiazol-2-yl)-5-[(4-chlorophényl)méthyl]-8-fluoro-1,1,4-trioxo-2,3-dihydro-1lambda6,5-benzothiazépin-3-yl]-3-hydroxy-2-(méthylamino)propanamide
la (3R)-3-amino-7-(5-tert-butyl-1,3,4-oxadiazol-2-yl)-5-[[4-(cyclopentoxy)phényl]méthyl]-8-fluoro-1,1-dioxo-2,3-dihydro-1lambda6,5-benzothiazépin-4-one ;
la (3R)-3-amino-7-(5-tert-butyl-1,3,4-oxadiazol-2-yl)-8-fluoro-5-[(4-isopropoxyphényl)méthyl]-1,1-dioxo-2,3-dihydro-1lambda6,5-benzothiazépin-4-one ;
la (3R)-3-amino-5-[(4-chlorophényl)méthyl]-1,1-dioxo-7-[5-[2,2,2-trideutério-1,1-bis(trideutériométhyl)éthyl]-1,3,4-oxadiazol-2-yl]-2,3-dihydro-1lambda6,5-benzothiazépin-4-one ;
la (3R)-3-amino-5-[(4-chlorophényl)méthyl]-7-[5-(3,3-difluorocyclohexyl)-1,3,4-oxadiazol-2-yl]-8-fluoro-1,1-dioxo-2,3-dihydro-1lambda6,5-benzothiazépin-4-one
la (3R)-3-amino-5-[(4-chlorophényl)méthyl]-7-(4-éthyltriazol-1-yl)-8-fluoro-2,3-dihydro-1,5-benzothiazépin-4-one ;
la (3R)-3-amino-5-[(4-chlorophényl)méthyl]-7-[4-[(4-chlorophényl)méthyl]triazol-1-yl]-8-fluoro-2,3-dihydro-1,5-benzothiazépin-4-one ;
la (3R)-3-amino-5-[(4-chlorophényl)méthyl]-7-(4-éthyltriazol-1-yl)-8-fluoro-1,1-dioxo-2,3-dihydro-1λ⁶,5-benzothiazépin-4-one ;
la (3R)-3-amino-5-[(4-chlorophényl)méthyl]-7-[4-[(4-chlorophényl)méthyl]triazol-1-yl]-8-fluoro-1,1-dioxo-2,3-dihydro-1λ⁶,5-benzothiazépin-4-one ;
la (3R)-3-amino-7-(4-tert-butyltriazol-1-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-(1-éthyltriazol-4-yl)-8-fluoro-1,1-dioxo-2,3-dihydro-1λ⁶,5-benzothiazépin-4-one ;
la (3R)-3-amino-5-[(4-chlorophényl)méthyl]-7-(2-éthyltétrazol-5-yl)-8-fluoro-1,1-dioxo-2,3-dihydro-1λ⁶,5-benzothiazépin-4-one ;
la (3R)-3-amino-7-(2-tert-butyltétrazol-5-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-8-fluoro-1,1-dioxo-7-(1H-pyrazol-5-yl)-2,3-dihydro-1λ⁶,5-benzothiazépin-4-one ;
la (3R)-3-amino-5-[(4-chlorophényl)méthyl]-7-(5-éthyl-1H-1,2,4-triazol-3-yl)-8-fluoro-1,1-dioxo-2,3-dihydro-1λ⁶,5-benzothiazépin-4-one ;
la (3R)-3-amino-5-[(4-chlorophényl)méthyl]-1,1-dioxo-7-[3-(2,2,2-trifluoroéthyl)-1,2,4-oxadiazol-5-yl]-2,3-dihydropyrido[3,2-b][1,4]thiazépin-4-one ;
la (3R)-3-amino-5-[(4-chlorophényl)méthyl]-7-(3-cyclopropyl-1,2,4-oxadiazol-5-yl)-8-fluoro-1,1-dioxo-2,3-dihydro-1λ⁶,5-benzothiazépin-4-one ;
la (3R)-3-amino-5-[(4-chlorophényl)méthyl]-7-(3-éthyl-1,2,4-oxadiazol-5-yl)-8-fluoro-1,1-dioxo-2,3-dihydro-1λ⁶,5-benzothiazépin-4-one ;
la (3R)-3-amino-5-[(4-chlorophényl)méthyl]-8-fluoro-1,1-dioxo-7-(3-propyl-1,2,4-oxadiazol-5-yl)-2,3-dihydro-1λ⁶,5-benzothiazépin-4-one ;
la (3R)-3-amino-7-(3-tert-butyl-1,2,4-oxadiazol-5-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-[3-[(4-chlorophényl)méthyl]-1,2,4-oxadiazol-5-yl]-8-fluoro-1,1-dioxo-2,3-dihydro-1λ⁶,5-benzothiazépin-4-one ;
la (3R)-3-amino-7-[3-(benzyloxyméthyl)-1,2,4-oxadiazol-5-yl]-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-8-fluoro-1,1-dioxo-7-[3-(2,2,2-trifluoroéthyl)-1,2,4-oxadiazol-5-yl]-2,3-dihydro-1λ⁶,5-benzothiazépin-4-one ;
la (3R)-3-amino-7-[3-(aminométhyl)-1,2,4-oxadiazol-5-yl]-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-(3-éthyl-1,2,4-oxadiazol-5-yl)-1,1-dioxo-2,3-dihydro-1λ⁶,5-benzothiazépin-4-one ;
la (3R)-3-amino-7-(3-tert-butylisoxazol-5-yl)-5-[(4-chlorophényl)méthyl]-8-fluoro-1,1-dioxo-2,3-dihydro-1λ⁶,5-benzothiazépin-4-one ;
la (3R)-3-amino-7-[5-(2,2-difluorocyclohexyl)-1,3,4-oxadiazol-2-yl]-8-fluoro-1,1-dioxo-5-[[4-(trifluorométhoxy)phényl]méthyl]-2,3-dihydro-1λ⁶,5-benzothiazépin-4-one ;
la (3R)-3-amino-7-(5-tert-butyl-1,3,4-oxadiazol-2-yl)-8-fluoro-5-[[4-(trifluorométhoxy)phényl]méthyl]-2,3-dihydro-1,5-benzothiazépin-4-one ;
la (3R)-3-amino-7-[5-(3,3-difluorocyclohexyl)-1,3,4-oxadiazol-2-yl]-8-fluoro-1,1-dioxo-5-[[4-(trifluorométhoxy)phényl]méthyl]-2,3-dihydro-1λ⁶,5-benzothiazépin-4-one ;
la (3R)-3-amino-8-fluoro-1,1-dioxo-7-[5-(2,2,2-trifluoroéthyl)-1,3,4-oxadiazol-2-yl]-5-[[4-(trifluorométhoxy)phényl]méthyl]-2,3-dihydro-1λ⁶,5-benzothiazépin-4-one ;
la (3R)-3-amino-7-(5-tert-butyl-1,3,4-oxadiazol-2-yl)-8-fluoro-1,1-dioxo-5-[[4-(trifluorométhoxy)phényl]méthyl]-2,3-dihydro-1λ⁶,5-benzothiazépin-4-one ;
le (2S)-N-[(3R)-8-fluoro-1,1,4-trioxo-7-[5-(2,2,2-trifluoroéthyl)-1,3,4-oxadiazol-2-yl]-5-[[4-(trifluorométhoxy)phényl]méthyl]-2,3-dihydro-1λ⁶,5-benzothiazépin-3-yl]-2-(méthylamino)propanamide ;
la (3R)-3-amino-7-(5-tert-butyl-1,3,4-oxadiazol-2-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-8-fluoro-1,1-dioxo-7-[5-(3,3,3-trifluoropropyl)-1,3,4-oxadiazol-2-yl]-2,3-dihydro-1λ⁶,5-benzothiazépin-4-one ;
la (3R)-3-amino-5-[(4-chlorophényl)méthyl]-8-fluoro-1,1-dioxo-7-[5-(2,2,2-trifluoroéthyl)-1,3,4-oxadiazol-2-yl]-2,3-dihydro-1λ⁶,5-benzothiazépin-4-one ;
la (3R)-3-amino-7-[5-(1-amino-1-méthyl-éthyl)-1,3,4-oxadiazol-2-yl]-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-8-fluoro-7-[5-(1-fluoro-1-méthyl-éthyl)-1,3,4-oxadiazol-2-yl]-1,1-dioxo-2,3-dihydro-1λ⁶,5-benzothiazépin-4-one ;
la (3R)-3-amino-5-[(4-chlorophényl)méthyl]-7-[5-(2,2-difluoroéthyl)-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-7-(5-éthyl-1,3,4-oxadiazol-2-yl)-8-fluoro-1,1-dioxo-2,3-dihydro-1λ⁶,5-benzothiazépin-4-one ;
la (3R)-3-amino-5-[(4-chlorophényl)méthyl]-7-[5-(4,4-difluorocyclohexyl)-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-7-[5-(2,2-difluorocyclohexyl)-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-7-[5-(3,3-difluorocyclopentyl)-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-7-[5-(2,2-difluorocyclopentyl)-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-7-[5-[(3,3-difluorocyclobutyl)méthyl]-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-8-fluoro-1,1-dioxo-7-[5-(tétrahydropyran-4-ylméthyl)-1,3,4-oxadiazol-2-yl]-2,3-dihydro-1λ⁶,5-benzothiazépin-4-one ;
la (3R)-3-amino-5-[(4-chlorophényl)méthyl]-8-fluoro-1,1-dioxo-7-[5-(4,4,4-trifluorobutyl)-1,3,4-oxadiazol-2-yl]-2,3-dihydro-1λ⁶,5-benzothiazépin-4-one ;
la (3R)-3-amino-5-[(4-chlorophényl)méthyl]-7-[5-(3,3-difluorocyclobutyl)-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-7-[5-(2,2-difluorocyclobutyl)-1,3,4-oxadiazol-2-yl]-8-fluoro-1,1-dioxo-2,3-dihydro-1λ⁶,5-benzothiazépin-4-one ;
la (3R)-3-amino-5-[(4-chlorophényl)méthyl]-8-fluoro-1,1-dioxo-7-(5-tétrahydropyran-4-yl-1,3,4-oxadiazol-2-yl)-2,3-dihydro-1λ⁶,5-benzothiazépin-4-one ;
la (3R)-3-amino-5-[(4-chlorophényl)méthyl]-8-fluoro-7-[5-(2-fluoroéthyl)-1,3,4-oxadiazol-2-yl]-1,1-dioxo-2,3-dihydro-1λ⁶,5-benzothiazépin-4-one ;
la (3R)-3-amino-5-[(4-chlorophényl)méthyl]-8-fluoro-7-(5-isopropyl-1,3,4-oxadiazol-2-yl)-1,1-dioxo-2,3-dihydro-1λ⁶,5-benzothiazépin-4-one ;
la (3R)-3-amino-5-[(4-chlorophényl)méthyl]-7-(5-cyclopropyl-1,3,4-oxadiazol-2-yl)-8-fluoro-1,1-dioxo-2,3-dihydro-1λ⁶,5-benzothiazépin-4-one ;
la (3R)-3-amino-5-[(4-chlorophényl)méthyl]-8-fluoro-7-[5-(2-hydroxy-1,1-diméthyl-éthyl)-1,3,4-oxadiazol-2-yl]-1,1-dioxo-2,3-dihydro-1λ⁶,5-benzothiazépin-4-one ;
la (3R)-3-amino-7-[5-(azétidin-1-yl)-1,3,4-oxadiazol-2-yl]-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-(4-éthyloxazol-2-yl)-8-fluoro-1,1-dioxo-2,3-dihydro-1λ⁶,5-benzothiazépin-4-one ;
la (3R)-3-amino-5-[(4-chlorophényl)méthyl]-6-fluoro-1,1-dioxo-7-[5-(2,2,2-trifluoroéthyl)-1,3,4-oxadiazol-2-yl]-2,3-dihydro-1λ⁶,5-benzothiazépin-4-one ;
la (3R)-3-amino-7-(5-tert-butyl-1,3,4-oxadiazol-2-yl)-5-[(3-chlorophényl)méthyl]-8-fluoro-1,1-dioxo-2,3-dihydro-1λ⁶,5-benzothiazépin-4-one ;
le 4-[[(3R)-3-amino-7-(5-tert-butyl-1,3,4-oxadiazol-2-yl)-8-fluoro-1,1,4-trioxo-2,3-dihydro-1λ⁶,5-benzothiazépin-5-yl]méthyl]benzonitrile ;
la (3R)-3-amino-7-(5-tert-butyl-1,3,4-oxadiazol-2-yl)-8-fluoro-1,1-dioxo-5-[(4-tétrahydrofuran-3-yloxyphényl)méthyl]-2,3-dihydro-1λ⁶,5-benzothiazépin-4-one ;
la (3R)-3-amino-7-(5-tert-butyl-1,3,4-oxadiazol-2-yl)-5-[[4-(2,2-difluoroéthoxy)phényl]méthyl]-8-fluoro-1,1-dioxo-2,3-dihydro-1λ⁶,5-benzothiazépin-4-one ;
la (3R)-3-amino-7-(5-tert-butyl-1,3,4-oxadiazol-2-yl)-5-[[4-(difluorométhoxy)phényl]méthyl]-8-fluoro-1,1-dioxo-2,3-dihydro-1λ⁶,5-benzothiazépin-4-one ;
la (3R)-3-amino-7-(5-tert-butyl-1,3,4-oxadiazol-2-yl)-8-fluoro-1,1-dioxo-5-(2-pyridylméthyl)-2,3-dihydro-1λ⁶,5-benzothiazépin-4-one ;
la (3R)-3-amino-7-(5-tert-butyl-1,3,4-oxadiazol-2-yl)-8-fluoro-5-[(6-méthoxy-3-pyridyl)méthyl]-1,1-dioxo-2,3-dihydro-1λ⁶,5-benzothiazépin-4-one ;
la (3R)-3-amino-5-[[4-(aminométhyl)phényl]méthyl]-7-(5-tert-butyl-1,3,4-oxadiazol-2-yl)-8-fluoro-1,1-dioxo-2,3-dihydro-1λ⁶,5-benzothiazépin-4-one ;
la (3R)-3-amino-7-(5-tert-butyl-1,3,4-oxadiazol-2-yl)-5-[(2-chlorophényl)méthyl]-8-fluoro-1,1-dioxo-2,3-dihydro-1λ⁶,5-benzothiazépin-4-one ;
la (3R)-3-amino-7-(5-tert-butyl-1,3,4-oxadiazol-2-yl)-8-fluoro-5-[(5-méthoxy-2-pyridyl)méthyl]-1,1-dioxo-2,3-dihydro-1λ⁶,5-benzothiazépin-4-one ;
la (3R)-3-amino-7-(5-tert-butyl-1,3,4-oxadiazol-2-yl)-8-fluoro-5-[(4-méthylsulfonylphényl)méthyl]-1,1-dioxo-2,3-dihydro-1λ⁶,5-benzothiazépin-4-one ;
la (3R)-3-amino-7-(5-tert-butyl-1,3,4-oxadiazol-2-yl)-8-fluoro-5-[(4-méthoxyphényl)méthyl]-1,1-dioxo-2,3-dihydro-1λ⁶,5-benzothiazépin-4-one ;
la (3R)-3-amino-7-(5-tert-butyl-1,3,4-oxadiazol-2-yl)-8-fluoro-5-[(4-fluorophényl)méthyl]-1,1-dioxo-2,3-dihydro-1λ⁶,5-benzothiazépin-4-one ;
la (3R)-3-amino-5-[[2-(aminométhyl)-4-chloro-phényl]méthyl]-7-(5-tert-butyl-1,3,4-oxadiazol-2-yl)-8-fluoro-1,1-dioxo-2,3-dihydro-1λ⁶,5-benzothiazépin-4-one ;
la (3R)-3-amino-5-[[4-(11-aminoundécoxy)phényl]méthyl]-7-(5-tert-butyl-1,3,4-oxadiazol-2-yl)-8-fluoro-1,1-dioxo-2,3-dihydro-1λ⁶,5-benzothiazépin-4-one ;
le N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5,5-benzothiazépin-3-yl]acétamide ;
le N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]propanamide ;
le (2S)-N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]-2-hydroxy-butanamide ;
le N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]butanamide ;
le (2S)-2-amino-N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]propanamide ;
le (2S)-N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]-2-(méthylamino)butanamide ;
le 3-amino-N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]propanamide ;
le N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]-2-(méthylamino)acétamide ;
le 2-amino-N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]acétamide ;
le 4-amino-N-[(3R)-7-(5-tert-butyl-1,3,4-oxadiazol-2-yl)-5-[(4-chlorophényl)méthyl]-8-fluoro-1,1,4-trioxo-2,3-dihydro-1λ⁶,5-benzothiazépin-3-yl]butanamide ;
la (3R)-3-amino-8-bromo-7-(5-tert-butyl-1,3,4-oxadiazol-2-yl)-5-[(4-chlorophényl)méthyl]-1,1-dioxo-2,3-dihydro-1λ⁶,5-benzothiazépin-4-one ;
la (3R)-3-amino-7-(5-tert-butyl-1,3,4-oxadiazol-2-yl)-5-[(4-chlorophényl)méthyl]-8-méthyl-1,1-dioxo-2,3-dihydro-1λ⁶,5-benzothiazépin-4-one ;
la (3R)-3-amino-7-(5-tert-butyl-1,3,4-oxadiazol-2-yl)-5-[(4-chlorophényl)méthyl]-8-hydroxy-1,1-dioxo-2,3-dihydro-1λ⁶,5-benzothiazépin-4-one ;
la (3R)-3-amino-7-(5-tert-butyl-1,3,4-oxadiazol-2-yl)-5-[(4-chlorophényl)méthyl]-8-(diméthylamino)-1,1-dioxo-2,3-dihydro-1λ⁶,5-benzothiazépin-4-one ;
la (3R)-3,8-diamino-7-(5-tert-butyl-1,3,4-oxadiazol-2-yl)-5-[(4-chlorophényl)méthyl]-1,1-dioxo-2,3-dihydro-1λ⁶,5-benzothiazépin-4-one ;
la (3R)-3-amino-7-(1-tert-butylpyrazol-4-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-(1-éthylpyrazol-4-yl)-8-fluoro-1,1-dioxo-2,3-dihydro-1λ⁶,5-benzothiazépin-4-one ;
la (3R)-3-amino-5-[(4-chlorophényl)méthyl]-7-(1-éthylpyrazol-3-yl)-8-fluoro-1,1-dioxo-2,3-dihydro-1λ⁶,5-benzothiazépin-4-one ;
la (3R)-3-amino-7-(4-tert-butylpyrazol-1-yl)-5-[(4-chlorophényl)méthyl]-8-fluoro-1,1-dioxo-2,3-dihydro-1λ⁶,5-benzothiazépin-4-one ;
la (3R)-3-amino-7-(3-tert-butylpyrazol-1-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-(4-éthylpyrazol-1-yl)-8-fluoro-1,1-dioxo-2,3-dihydro-1λ⁶,5-benzothiazépin-4-one ;
la (3R)-3-amino-5-[(4-chlorophényl)méthyl]-7-(1-éthyl-1,2,4-triazol-3-yl)-8-fluoro-1,1-dioxo-2,3-dihydro-1λ⁶,5-benzothiazépin-4-one ;
la (3R)-3-amino-7-(1-tert-butyl-1,2,4-triazol-3-yl)-5-[(4-chlorophényl)méthyl]-8-fluoro-1,1-dioxo-2,3-dihydro-1λ⁶,5-benzothiazépin-4-one ;
la (3R)-3-amino-7-(1-tert-butylpyrazol-3-yl)-5-[(4-chlorophényl)méthyl]-8-fluoro-1,1-dioxo-2,3-dihydro-1λ⁶,5-benzothiazépin-4-one ;
la (3R)-3-amino-7-(5-tert-butyl-1,2,4-oxadiazol-3-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-(5-éthyl-1,2,4-oxadiazol-3-yl)-8-fluoro-1,1-dioxo-2,3-dihydro-1λ⁶,5-benzothiazépin-4-one ;
la (3R)-3-amino-7-[5-(1,1-diméthylbut-3-ynyl)-1,3,4-oxadiazol-2-yl]-8-fluoro-1,1-dioxo-5-[[4-[3-(trifluorométhyl)diazirin-3-yl]phényl]méthyl]-2,3-dihydro-1λ⁶,5-benzothiazépin-4-one ;
la (3R)-3-amino-7-(5-tert-butyl-1,3,4-oxadiazol-2-yl)-1,1-dioxo-5-[[4-(trifluorométhoxy)phényl]méthyl]-2,3-dihydro-1λ⁶,5-benzothiazépin-4-one ;
la (3R)-3-amino-7-(5-tert-butyl-1,3,4-oxadiazol-2-yl)-5-[(4-chlorophényl)méthyl]-1,1-dioxo-2,3-dihydro-1λ⁶,5-benzothiazépin-4-one ;
la (3R)-3-amino-7-[5-(1,1-diméthylbut-3-ynyl)-1,3,4-oxadiazol-2-yl]-8-fluoro-1,1-dioxo-5-[[4-(trifluorométhoxy)phényl]méthyl]-2,3-dihydro-1λ⁶,5-benzothiazépin-4-one ;
la (3R)-3-amino-5-[(4-chlorophényl)méthyl]-7-[5-(5,5-difluoro-1-méthyl-3-pipéridyl)-1,3,4-oxadiazol-2-yl]-8-fluoro-1,1-dioxo-2,3-dihydro-1λ⁶,5-benzothiazépin-4-one ;
la (3R)-3-amino-7-(5-tert-butyl-1,3,4-oxadiazol-2-yl)-8-fluoro-1-oxo-5-[[4-(trifluorométhoxy)phényl]méthyl]-2,3-dihydro-1λ⁴,5-benzothiazépin-4-one ;
la (3R)-3-amino-7-(5-tert-butyl-1,3,4-oxadiazol-2-yl)-8-fluoro-1-oxo-5-[[4-(trifluorométhoxy)phényl]méthyl]-2,3-dihydro-1λ⁴,5-benzothiazépin-4-one ;
la (3R)-3-amino-5-[(4-chlorophényl)méthyl]-8-fluoro-7-(5-morpholino-1,3,4-oxadiazol-2-yl)-1,1-dioxo-2,3-dihydro-1λ⁶,5-benzothiazépin-4-one ;
la (3R)-3-amino-5-[(4-chlorophényl)méthyl]-7-[5-(cyclopentylamino)-1,3,4-oxadiazol-2-yl]-8-fluoro-1,1-dioxo-2,3-dihydro-1λ⁶,5-benzothiazépin-4-one ;
la (3R)-3-amino-7-(5-tert-butyl-1,3,4-thiadiazol-2-yl)-5-[(4-chlorophényl)méthyl]-8-fluoro-1,1-dioxo-2,3-dihydro-1λ⁶,5-benzothiazépin-4-one ;
la (3R)-3-amino-5-[(4-chlorophényl)méthyl]-7-(5-éthyltétrazol-2-yl)-8-fluoro-1,1-dioxo-2,3-dihydro-1λ⁶,5-benzothiazépin-4-one ;
ou un sel pharmaceutiquement acceptable de ceux-ci.

19. Procédé de préparation d'un composé selon l'une quelconque des revendications 1 à 18, ou d'un sel pharmaceutiquement acceptable de celui-ci, comprenant
la réaction d'un composé de formule (IX) 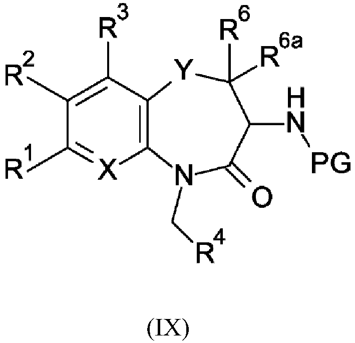
dans lequel X, Y, R¹, R², R³, R⁴, R⁶, R^{6a} sont tels que définis dans l'une quelconque des revendications 1 à 17 et PG représente un groupe protecteur d'amino,
avec un agent de déprotection approprié pour former ledit composé de formule (I), ou un sel pharmaceutiquement acceptable de celui-ci, dans lequel X, Y, R¹, R², R³, R⁴, R⁶, R^{6a} sont tels que définis dans l'une quelconque des revendications 1 à 17 et R⁵ représente un hydrogène ; ou
la réaction d'un composé de formule (I') 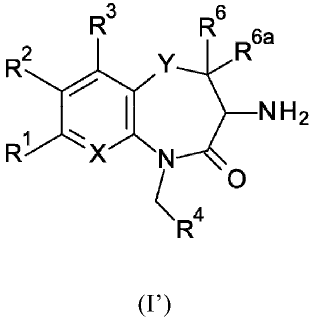
dans lequel X, Y, R¹, R², R³, R⁴, R⁶, R^{6a} sont tels que définis dans l'une quelconque des revendications 1 à 34 et R⁵ représente un hydrogène,
avec un dérivé d'acide carboxylique de formule R⁹CO₂H dans lequel R⁹ est tel que défini dans l'une quelconque des revendications 1 à 15, en présence d'une base pour former un composé de formule (I), ou un sel pharmaceutiquement acceptable de celui-ci, dans lequel R⁵ représente -C(O)(R⁹).

20. Composé selon l'une quelconque des revendications 1 à 18, ou un sel pharmaceutiquement acceptable de celui-ci, pour une utilisation comme substance thérapeutiquement active.

21. Composition pharmaceutique comprenant un composé selon l'une quelconque des revendications 1 à 18, ou un sel pharmaceutiquement acceptable de celui-ci, et un excipient pharmaceutiquement acceptable.

22. Composé selon l'une quelconque des revendications 1 à 18, ou un sel pharmaceutiquement acceptable de celui-ci, pour une utilisation dans le traitement, la prévention et/ou le ralentissement de la progression d'un cancer.

23. Composé pour une utilisation selon la revendication 22, dans lequel le cancer est associé à une signalisation aberrante de la diacylglycérol kinase, dans lequel la diacylglycérol kinase est choisie parmi DGKα et/ou DGKζ.

24. Composé pour une utilisation selon la revendication 22 ou 23, dans lequel le cancer est choisi dans le groupe constitué par une leucémie lymphoïde aiguë à cellules B, une leucémie lymphoïde aiguë à cellules T, une leucémie lymphoïde aiguë, une leucémie myéloïde chronique, une leucémie lymphocytaire chronique, une leucémie prolymphocytaire à cellules B, un néoplasme à cellules dendritiques plasmacytoïdes blastiques, un lymphome de Burkitt, un lymphome diffus à grandes cellules B, un lymphome folliculaire, une leucémie à tricholeucocytes, un lymphome folliculaire à petites cellules ou à grandes cellules, des affections lymphoprolifératives malignes, un lymphome de MALT, un lymphome à cellules du manteau, un lymphome de la zone marginale, un myélome multiple, une myélodysplasie et un syndrome myélodysplasique, un lymphome non hodgkinien, un lymphome plasmablastique, un néoplasme à cellules dendritiques plasmacytoïdes, une macroglobulinémie de Waldenström, une préleucémie, un sarcome, un carcinome, un mélanome, un neuroblastome, un carcinome à cellules rénales, un cancer du côlon, un cancer colorectal, un cancer du sein, un cancer épidermoïde à cellules squameuses, un mélanome, un cancer de l'estomac, un cancer du cerveau, un cancer du poumon (par exemple un CPNPC), un cancer du pancréas, un cancer du col de l'utérus, un cancer de l'ovaire, un cancer du foie, un cancer de la vessie, un cancer de la prostate, un cancer des testicules, un cancer de la thyroïde, un cancer de l'utérus, un cancer surrénalien et un cancer de la tête et du cou.
